(19) Europäisches Patentamt
European Patent Office
Office européen des brevets

(11) EP 4 745 135 A1

(12) EUROPEAN PATENT APPLICATION
published in accordance with Art. 153(4) EPC

(43) Date of publication:
**20.05.2026 Bulletin 2026/21**

(21) Application number: **24842342.8**

(22) Date of filing: **15.07.2024**

(51) International Patent Classification (IPC):
***C07D 401/14*** $^{(2006.01)}$ ***C07D 413/14*** $^{(2006.01)}$
***C07D 417/14*** $^{(2006.01)}$ ***C07D 407/14*** $^{(2006.01)}$
***C07D 409/14*** $^{(2006.01)}$ ***A61K 31/454*** $^{(2006.01)}$
***A61K 31/4545*** $^{(2006.01)}$ ***A61K 31/453*** $^{(2006.01)}$
***A61K 31/4535*** $^{(2006.01)}$ ***A61K 31/496*** $^{(2006.01)}$
***A61K 31/506*** $^{(2006.01)}$ ***A61P 35/00*** $^{(2006.01)}$
***A61P 35/02*** $^{(2006.01)}$ ***A61P 37/00*** $^{(2006.01)}$
***A61P 29/00*** $^{(2006.01)}$ ***A61P 9/00*** $^{(2006.01)}$
***A61P 3/10*** $^{(2006.01)}$

(52) Cooperative Patent Classification (CPC):
**A61K 31/453; A61K 31/4535; A61K 31/454; A61K 31/4545; A61K 31/496; A61K 31/506; A61P 3/10; A61P 9/00; A61P 29/00; A61P 35/00; A61P 35/02; A61P 37/00; C07D 401/14; C07D 407/14; C07D 409/14;** (Cont.)

(86) International application number:
**PCT/CN2024/105405**

(87) International publication number:
**WO 2025/016351 (23.01.2025 Gazette 2025/04)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **14.07.2023 CN 202310869035**

(71) Applicant: **Gluetacs Therapeutics (Shanghai) Co., Ltd.**
**Shanghai 201306 (CN)**

(72) Inventors:
- **YANG, Xiaobao**
  **Shanghai 201306 (CN)**
- **SUN, Renhong**
  **Shanghai 201306 (CN)**
- **REN, Chaowei**
  **Shanghai 201306 (CN)**
- **ZHAO, Baoyin**
  **Shanghai 201306 (CN)**
- **ZHOU, Yuedong**
  **Shanghai 201306 (CN)**
- **YANG, Sen**
  **Shanghai 201306 (CN)**
- **DENG, Meigui**
  **Shanghai 201306 (CN)**
- **REN, Yinbo**
  **Shanghai 201306 (CN)**

(74) Representative: **J A Kemp LLP**
**80 Turnmill Street**
**London EC1M 5QU (GB)**

# (54) COMPOUND BASED ON THIO-GLUTARIMIDO ISOINDOLINONE SKELETON AND USE THEREOF

(57) The present disclosure relates to compounds of Formula (I) or salts, enantiomers, stereoisomers, solvates, isotopically enriched analogs, prodrugs, or polymorphs thereof, and uses thereof. The present disclosure also relates to pharmaceutical compositions containing as an active ingredient a compound of Formula (I) or salts, enantiomers, stereoisomers, solvates, isotopically enriched analogs, prodrugs, or polymorphs thereof, and uses thereof. The series of compounds designed and synthesized in the present disclosure can effectively prevent or treat diseases or disorders related to cereblon protein, including tumors or cancers.



Processed by Luminess, 75001 PARIS (FR)

(Cont. next page)

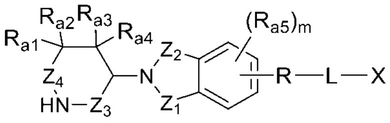

(I)

(52) Cooperative Patent Classification (CPC): (Cont.)
**C07D 413/14; C07D 417/14**

## Description

### Technical Field

The present disclosure relates to compounds of Formula (I), or salts, enantiomers, stereoisomers, solvates, polymorphs thereof, and uses thereof, particularly for the prevention or treatment of tumors or cancers.

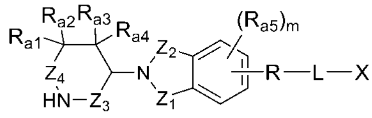

(I)

### Background

Immunomodulatory drugs (IMiDs) of the phthalimide class, such as thalidomide and lenalidomide, have shown remarkable efficacy in treating multiple myeloma and autoimmune diseases. However, it was not until 2010 that their direct binding target was identified as the E3 ubiquitin ligase cereblon (CRBN). Subsequent studies confirmed that upon binding to CRBN, these drugs act as molecular glues, recruiting substrate proteins (e.g., the transcription factors IKZF1/3) and inducing protein-protein interactions between CRBN and the substrates. This leads to the ubiquitination of the substrate proteins, which are then recognized and degraded by the proteasome, thereby eliciting pharmacological effects such as antitumor and immunomodulatory activities. Molecular glue degraders directly target and degrade specific proteins, offering potential advantages such as targeting "undruggable" proteins. Moreover, molecular glues typically exhibit low molecular weight and favorable drug-like properties, making their development highly challenging. Based on the CRBN E3 ubiquitin ligase and the molecular glue degradation mechanism, a series of compounds have been developed, including the approved pomalidomide and several candidates currently in clinical trials, such as Bristol Myers Squibb's CC-122, CC-220, CC-90009, CC-99282, and CC-92480; Novartis's DKY709; and C4 Therapeutics's CFT7455. The range of substrates degraded by these molecular glues has expanded from the initially identified transcription factors IKZF1/3 to include casein kinase 1$\alpha$ (CK1$\alpha$), zinc finger protein 91 (ZFP91), and the translation factor GSPT1. Degradation of these protein substrates enables molecular glues to exert immunomodulatory, anti-inflammatory, and antitumor activities. Currently, the structural novelty of designed molecular glue candidates remains quite limited, making the diversification of molecular scaffolds and the development of novel molecular glues a key research focus in the field.

Therefore, there is an urgent need for a series of novel CRBN E3 ubiquitin ligase ligand scaffolds to be applied in the development of effective molecular glue degraders for the treatment and/or prevention of diseases or disorders mediated by or associated with degradable proteins.

### Summary of Invention

In view of the foregoing, the objectvies of the present disclosure are to provide novel thio-glutarimido isoindolinone derivative protein degraders, their preparation methods, uses, and methods of administration thereof.

To achieve the above objectives and other related goals, in one aspect, the present disclosure provides a compound of Formula (I):

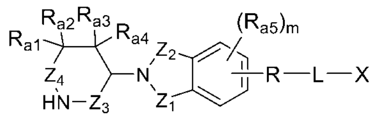

(I)

or salts, stereoisomers (including enantiomers and diastereoisomer), solvates, or polymorphs thereof,

wherein $Z_1$ represents C(O), C(S), $CH_2$ or $CD_2$; $Z_2$, $Z_3$, and $Z_4$ each independently represent C(O) or C(S), wherein at least one of $Z_1$, $Z_2$, $Z_3$, and $Z_4$ represents C(S);

$R_{a1}$, $R_{a2}$, $R_{a3}$, and $R_{a4}$ each independently represent hydrogen, deuterium, halogen, $C_{1-6}$ alkyl, halogenated $C_{1-6}$ alkyl, deuterated $C_{1-6}$ alkyl, $C_{1-6}$ alkoxy, deuterated $C_{1-6}$ alkoxy, or halogenated $C_{1-6}$ alkoxy;

$(R_{a5})_m$ indicates that the isoindoline ring to which it is attached is optionally substituted with m $R_{a5}$, with each $R_{a5}$ being identical or different and each independently representing deuterium, halogen, hydroxy, mercapto, nitro, amino,

cyano, $C_{1-6}$ alkyl, halogenated $C_{1-6}$ alkyl, deuterated $C_{1-6}$ alkyl, $C_{1-6}$ alkoxy, deuterated $C_{1-6}$ alkoxy, halogenated $C_{1-6}$ alkoxy, $C_{2-6}$ alkenyl, or $C_{2-6}$ alkynyl;

m represents an integer of 0, 1, 2 or 3;

R represents O, S, S(O), $S(O)_2$, alkenylene, alkynylene or $N(R_{a6})$, wherein $R_{a6}$ represents H or $C_{1-3}$ alkyl; or R represents a bond; or

R represents:

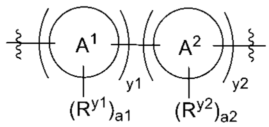

wherein each ring $A^1$ is identical or different and each independently represents nitrogen-containing heterocyclylene, arylene or heteroarylene; y1 represents an integer of 1 or 2; and $(R^{y1})_{a1}$ indicates that each ring $A^1$ is independently optionally substituted with a1 $R^{y1}$ groups, with each $R^{y1}$ being independently deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, $C_{2-6}$ alkynyl or $C_{2-6}$ alkenyl; and a1 represents an integer of 0-20;

each ring $A^2$ is identical or different and each independently represents heterocyclylene, cycloalkylene, arylene or heteroarylene; y2 represents an integer of 0 or 1; and $(R^{y2})_{a2}$ indicates that each ring $A^2$ is independently optionally substituted with a2 $R^{y2}$ groups, with each $R^{y2}$ being independently deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, $C_{2-6}$ alkynyl or $C_{2-6}$ alkenyl; and a2 represents an integer of 0-20;

L represents :

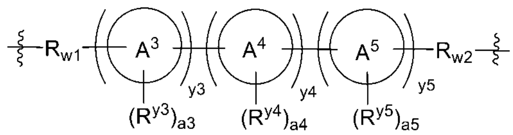

wherein $R_{w1}$ is linked to R, and $R_{w2}$ is linked to X;

$R_{w2}$ represents a bond, C(O), C(O)NH, NHC(O) or $N(R_{a6})$, wherein $R_{a6}$ represents H or $C_{1-3}$ alkyl;

each ring $A^3$ is identical or different and each independently represents heterocyclylene, cycloalkylene, arylene, or heteroarylene; y3 represents an integer of 0, 1, 2 or 3; and $(R^{y3})_{a3}$ indicates that each ring $A^3$ is independently optionally substituted with a3 $R^{y3}$ substituents, wherein each $R^{y3}$ independently represents deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, $C_{2-6}$ alkynyl or $C_{2-6}$ alkenyl; and a3 represents an integer of 0-20;

each ring $A^4$ is identical or different and each independently represents heterocyclylene, cycloalkylene, arylene, or heteroarylene; y4 represents an integer of 0, 1, 2 or 3; and $(R^{y4})_{a4}$ indicates that each ring $A^4$ is independently optionally substituted with a4 $R^{y4}$ substituents, wherein each $R^{y4}$ independently represents deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, $C_{2-6}$ alkynyl or $C_{2-6}$ alkenyl; and a4 represents an integer of 0-20;

each ring $A^5$ is identical or different and each independently represents heterocyclylene, cycloalkylene, arylene, or heteroarylene; y5 represents an integer of 0, 1, 2 or 3; and $(R^{y5})_{a5}$ indicates that each ring $A^5$ is independently optionally substituted with a5 $R^{y5}$ substituents, wherein each $R^{y5}$ independently represents deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, $C_{2-6}$ alkynyl or $C_{2-6}$ alkenyl; and a5 represents an integer of 0-20;

$R_{w1}$ represents a bond or an optionally substituted linear or branched $C_{1-20}$ alkylene, wherein any one or more methylene groups in the main carbon chain skeleton of the linear or branched $C_{1-20}$ alkylene are optionally replaced by one or more groups selected from $R_a$, $R_b$, and any combination thereof; wherein each $R_a$ is independently selected from the group consisting of: O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, wherein each $R_{a7}$ independently represents H or $C_{1-6}$ alkyl, and when any two or more methylene in the main carbon chain skeleton of the linear or branched $C_{1-20}$ alkylene are replaced by two or more $R_a$ groups, the $R_a$ groups are not directly connected to each other; and wherein each $R_b$ is independently selected from the group consisting of: optionally substituted cycloalkylene, optionally substituted arylene, optionally substituted heterocyclylene, optionally substituted heteroarylene, alkynylene, alkenylene, and any combination thereof;

X represents :

$NR_1R_2$, $C(O)NR_3R_4$, $NHC(O)R_5$, $NHC(O)NR_6R_7$, $OR_8$, $SR_9$, $S(O)_2NR_{10}R_{11}$ or $N(R_{12})S(O)_2R_{13}$, wherein $R_1$, $R_3$, $R_4$, $R_5$, $R_6$, $R_7$, $R_9$, $R_{10}$, $R_{11}$, and $R_{12}$ each independently represent H, optionally substituted linear or branched alkyl, optionally substituted cycloalkyl, optionally substituted aryl, optionally substituted heterocyclyl or optionally substituted heteroaryl, and wherein $R_2$, $R_8$, and $R_{13}$ each independently represent optionally substituted linear or branched alkyl, optionally substituted cycloalkyl, optionally substituted aryl, optionally substituted heterocyclyl or optionally substituted heteroaryl; or wherein $R_3$ and $R_4$, together with the nitrogen atom to which they are attached, form an optionally substituted 4- to 30-membered nitrogen-containing heterocyclyl; or

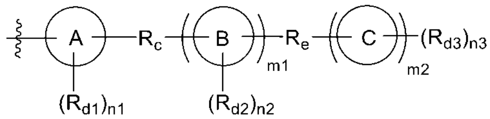

wherein ring A represents heterocyclylene, cycloalkylene, arylene or heteroarylene; and $(R_{d1})_{n1}$ indicates that ring A is optionally substituted with n1 $R_{d1}$ groups, with each $R_{d1}$ independently representing deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, oxo, $C_{2-6}$ alkynyl or $C_{2-6}$ alkenyl; and n1 represents an integer of 0-20;

$R_c$ represents $NR_{c1}$, C(O), $CR_{c2}R_{c3}$ or a bond, wherein $R_{c1}$ represents H, optionally substituted linear or branched alkyl, optionally substituted cycloalkyl, optionally substituted aryl, optionally substituted heterocyclyl or optionally substituted heteroaryl, and $R_{c2}$ and $R_{c3}$ each independently represent H, halogen, optionally substituted linear or branched alkyl, optionally substituted cycloalkyl, optionally substituted aryl, optionally substituted heterocyclyl or optionally substituted heteroaryl;

each ring B is identical or different and each independently represents heterocyclylene, cycloalkylene, arylene, or heteroarylene; ml represents an integer of 0, 1, 2 or 3;and $(R_{d2})_{n2}$ indicates that each ring B is independently optionally substituted with n2 $R_{d2}$ groups, with each $R_{d2}$ independently representing deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, oxo, $C_{2-6}$ alkynyl or $C_{2-6}$ alkenyl; and n2 represents an integer of 0-20;

$R_e$ represents S, O, $CR_{e1}R_{e2}$ or a bond, wherein $R_{e1}$ and $R_{e2}$ each independently represent H, halogen, optionally substituted linear or branched alkyl, optionally substituted cycloalkyl, optionally substituted aryl, optionally substituted heterocyclyl or optionally substituted heteroaryl; and

each ring C is identical or different and each independently represents heterocyclyl, cycloalkyl, aryl, or heteroaryl; m2 represents an integer of 0, 1, 2 or 3; and $(R_{d3})_{n3}$ indicates that each ring C is independently optionally substituted with n3 $R_{d3}$ groups, with each $R_{d3}$ independently representing deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, oxo, $C_{2-6}$ alkynyl or $C_{2-6}$ alkenyl; and n3 represents an integer of 0-20; or

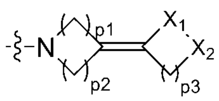

wherein $-X_1-X_2-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;

p1, p2, and p3 each independently represent an integer of 1, 2, 3 or 4; and

the ring containing $-X_1-X_2-$ is optionally substituted with one or more substituents selected from the group consisting of deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, oxo, $C_{2-6}$ alkynyl, $C_{2-6}$ alkenyl, and any combination thereof; or

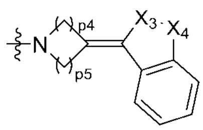

wherein $-X_3-X_4-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;

p4 and p5 each independently represent an integer of 1, 2, 3 or 4; and

the benzo-fused ring containing $-X_3-X_4-$ is optionally substituted with one or more substituents selected from the

group consisting of deuterium, $C_{1-6}$ alkyl, deuterated $C_{1-6}$ alkyl, halogenated $C_{1-6}$ alkyl, $C_{1-6}$ alkoxy, halogen, amino, hydroxy, mercapto, cyano, oxo, $C_{2-6}$ alkynyl, $C_{2-6}$ alkenyl, and any combination thereof.

In a further aspect, the present disclosure provides a pharmaceutical composition comprising the compound of Formula (I) or pharmaceutically acceptable salts, stereoisomers (including enantiomers and diastereomers), solvates, isotopically enriched analogs, prodrugs, or polymorphs thereof, and at least one pharmaceutically acceptable carrier.

In a further aspect, the present disclosure further provides a medicine kit or reagent kit comprising: the compound of Formula (I) or a pharmaceutically acceptable salt, or the pharmaceutical composition comprising the same.

In a further aspect, the present disclosure provides the compound of Formula (I) or pharmaceutically acceptable salts, enantiomers, diastereomers, solvates, prodrugs, or polymorphs thereof, for use as a medicament.

In a further aspect, the present disclosure provides the compound of Formula (I) or pharmaceutically acceptable salts, stereoisomers (including enantiomers and diastereomers), solvates, isotopically enriched analogs, prodrugs, or polymorphs thereof, or the pharmaceutical composition of the present disclosure, for use in the treatment or prevention of tumors or cancers.

In a further aspect, the present disclosure provides the use of the compound of Formula (I) or pharmaceutically acceptable salts, stereoisomers (including enantiomers and diastereomers), solvates, isotopically enriched analogs, prodrugs, or polymorphs thereof, or the pharmaceutical composition of the present disclosure, for the manufacture of a medicament for the prevention or treatment of tumors or cancers.

In a further aspect, the present disclosure provides a method for treating or preventing tumors or cancers in a subject, comprising administering to the subject a therapeutically effective amount of the compound of Formula (I) or pharmaceutically acceptable salts, stereoisomers (including enantiomers and diastereomers), solvates, isotopically enriched analogs, prodrugs, or polymorphs thereof, or the pharmaceutical composition of the present disclosure.

In a further aspect, the present disclosure provides a method for preparing a compound of Formula (II), comprising reacting a compound of Formula (M1) with a compound of Formula (M2) to prepare the compound of Formula (II):

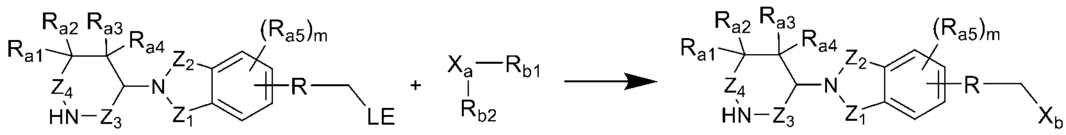

(M1) (M2) (II)

wherein the groups $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure; and

wherein

(i) the group LE represents Cl, Br, I, mesyloxy, tosyloxy, or ortho-nitrobenzenesulfonyl (ONs); $R_{b1}$-$X_a$-$R_{b2}$ of Formula (M2) is $R_1$-NH-$R_2$; and $X_b$ correspondingly represents $NR_1R_2$, wherein $R_1$ and $R_2$ are as defined in the compound of Formula (I) and its various sub-embodiments in the present disclosure; or

(ii) the group LE represents Cl, Br, I, OMs, OTs, or ONs; the group $X_a$ represents NH; and the group $R_{b1}$ and the group $R_{b2}$together with the nitrogen atom of $X_a$ to which they are attached form a nitrogen-containing heterocycle $A_a$ which is substituted by n1 substituent(s) $R_{d1}$, and a substituent represented by the formula of

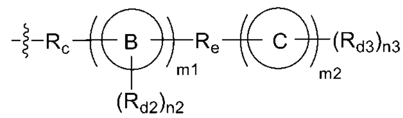

,

i.e., the compound of Formula (M2) has a structure represented by the following formula:

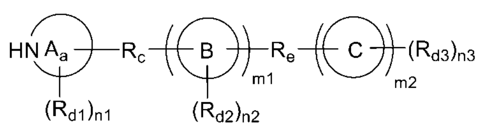

and $X_b$ correspondingly represents a structure represented by the following general formula:

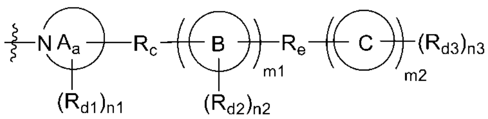

wherein the nitrogen-containing heterocycle $A_a$ represents 4- to 30-membered nitrogen-containing heterocyclyl (preferably, 4- to 20-membered nitrogen-containing heterocyclyl; more preferably, 4- to 15-membered nitrogen-containing heterocyclyl); and $(R_{d1})_{n1}$, n1, $R_c$, ring B, m1, $(R_{d2})_{n2}$, n2, $R_e$, ring C, m2, $(R_{d3})_{n3}$, and n3 are as defined in the compound of Formula (I) and its various sub-embodiments in the present disclosure; or

(iii) the group LE represents C(O)Cl, COOH or $S(O)_2Cl$; $R_{b1}$-$X_a$-$R_{b2}$ of Formula (M2) is $R_3$-NH-$R_4$ or $R_{10}$-NH-$R_{11}$; and $X_b$ correspondingly represents $C(O)NR_3R_4$ or $S(O)_2NR_{10}R_{11}$, wherein $R_3$, $R_4$, $R_{10}$, and $R_{11}$ are as defined in the compound of Formula (I) and its various sub-embodiments in the present disclosure; or

(iv) the group LE represents $NH_2$; $R_{b1}$-$X_a$-$R_{b2}$ of Formula (M2) represents: $R_5$-C(O)-OH, $R_5$-C(O)-Cl, $R_{13}$-$S(O)_2$-OH, or $R_{13}$-$S(O)_2$-Cl; and $X_b$ correspondingly represents $NHC(O)R_5$ or $NHS(O)_2R_{13}$, wherein $R_5$ and $R_{13}$ are as defined in the compound of Formula (I) and its various sub-embodiments in the present disclosure; or

(v) the group LE represents Cl, Br or I; $R_{b1}$-$X_a$-$R_{b2}$ of Formula (M2) represents: $R_8$-OH or $R_9$-SH; and $X_b$ correspondingly represents $OR_8$ or $SR_9$, wherein $R_8$ and $R_9$ are as defined in the compound of Formula (I) and its various sub-embodiments in the present disclosure.

In a further aspect, the present disclosure provides a method for preparing a compound of Formula (M1),

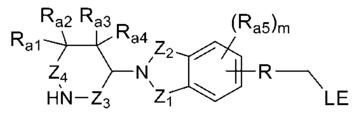

(M1)

wherein the group LE represents mesyloxy, and the method comprises reacting a compound of Formula (M3) with methanesulfonic anhydride or methanesulfonyl chloride to prepare the compound of Formula (M1),

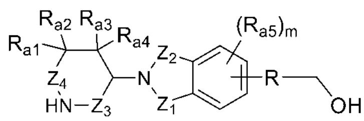

(M3)

wherein, in the compounds of Formula (M1) and Formula (M3), the groups $R_{a1}$, $R_{a2}$, $R_{a3}$, $R_{a4}$, R, $Z_1$, $Z_2$, $Z_3$, $Z_4$, and $(R_{a5})_m$ are as defined in the compound of Formula (I) and its various sub-embodiments in the present disclosure.

In a further aspect, the present disclosure provides a method for preparing a compound of Formula (M1),

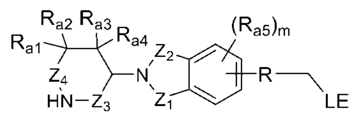

(M1)

wherein the group LE represents Cl, Br or I, and the method comprises preparing the compound of Formula (M1) by halogenation of a compound of Formula (M3) with a hydrohalic acid,

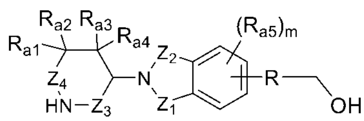

(M3)

wherein, in the compounds of Formula (M1) and Formula (M3), the groups $R_{a1}$, $R_{a2}$, $R_{a3}$, $R_{a4}$, R, $Z_1$, $Z_2$, $Z_3$, $Z_4$, and $(R_{a5})_m$

are as defined in the compound of Formula (I) and its various sub-embodiments in the present disclosure.

In a further aspect, the present disclosure provides a method for preparing a compound of Formula (M4), comprising preparing the thiocarbonyl compound of Formula (M4) by thionation of a carbonyl compound of Formula (M5) with a thionation reagent:

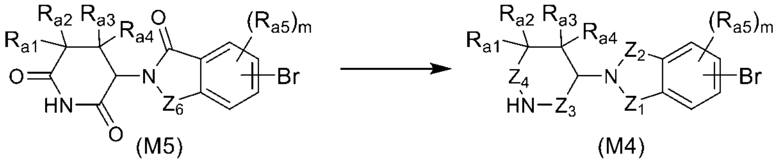

(M5) (M4)

wherein, in the carbonyl compound of Formula (M5), $Z_6$ represents C(O), $CH_2$ or $CD_2$; and the groups $R_{a1}$, $R_{a2}$, $R_{a3}$, $R_{a4}$, $Z_1$, $Z_2$, $Z_3$, $Z_4$, and $(R_{a5})_m$ are as defined in the compound of Formula (I) and its various sub-embodiments in the present disclosure.

In a further aspect, the present disclosure provides a method for preparing a compound of Formula (I), comprising preparing the compound of Formula (I) by thionation of a compound of Formula (A) with a thionation reagent,

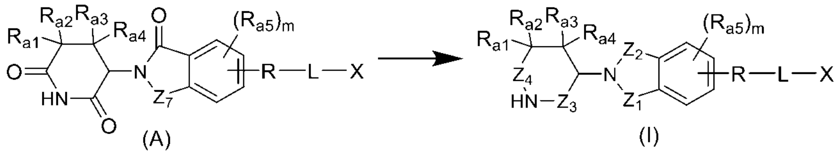

(A) (I)

wherein $Z_7$ of the compound of Formula (A) represents C(O), $CH_2$ or $CD_2$;

$Z_1$ of the compound of Formula (I) represents C(O), C(S), $CH_2$ or $CD_2$; $Z_2$, $Z_3$, and $Z_4$ each independently represent C(O) or C(S), wherein at least one of $Z_1$, $Z_2$, $Z_3$, and $Z_4$ represents C(S); and

wherein the groups $R_{a1}$, $R_{a2}$, $R_{a3}$, $R_{a4}$, $(R_{a5})_m$, R, L, and X are as defined in the compound of Formula (I) and its various sub-embodiments in the present disclosure.

## Detailed Description of the Invention

The following detailed description is provided as exemplary specific embodiments to assist those skilled in the art in understanding and practicing the present disclosure. It should be appreciated, however, that such description is not intended to limit the scope of the present disclosure, and that various modifications and changes may be made to the specific embodiments described in the present disclosure without departing from the spirit and scope of the present disclosure. Such changes and modifications are to be understood as being included within the scope of the present invention as defined by the appended claims.

## I. Compounds

### Compounds of Formula (I)

The present disclosure provides a compound of Formula (I) or salts (including pharmaceutically acceptable salts), stereoisomers (including enantiomers and diastereomers), solvates, isotopically enriched analogs, prodrugs, or polymorphs thereof:

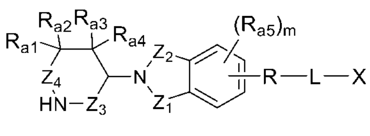

(I)

wherein $Z_1$, $Z_2$, $Z_3$, $Z_4$, $R_{a1}$, $R_{a2}$, $R_{a3}$, $R_{a4}$, $(R_{a5})_m$, R, L, and X are as defined in the compound of Formula (I) and its various sub-embodiments in the present disclosure.

The compounds of Formula (I) of the present disclosure, or salts thereof (including pharmaceutically acceptable salts), stereoisomers (including enantiomers), solvates, isotopically enriched analogs, prodrugs, or polymorphs thereof, exhibit binding affinity to CRBN, facilitate the recruitment of substrate proteins, and can act as molecular glues binding to

the CRBN E3 ubiquitin ligase. These compounds of Formula (I), or salts thereof (including pharmaceutically acceptable salts), stereoisomers (including enantiomers), solvates, isotopically enriched analogs, prodrugs, or polymorphs thereof, can also degrade substrate proteins (e.g., IKZF1/2/3/4 proteins, WEE1 protein, CK1α protein, GSPT1 protein, ZFP91 protein, etc.). Furthermore, the compounds of Formula (I) of the present disclosure, or salts thereof (including pharmaceutically acceptable salts), stereoisomers (including enantiomers), solvates, isotopically enriched analogs, prodrugs, or polymorphs thereof, possess anti-tumor activity and/or exhibit favorable pharmacokinetic properties, rendering them suitable for use as therapeutic agents for tumor patients.

In some embodiments of the present disclosure, $R_{a1}$, $R_{a2}$, $R_{a3}$, and $R_{a4}$ are identical or different and each independently represent H , deuterium (i.e., D), $C_{1-6}$ alkyl (e.g., $C_{1-3}$ alkyl, such as methyl, ethyl, or propyl), halogenated $C_{1-6}$ alkyl (e.g., halogenated $C_{1-4}$ alkyl, such as trifluoromethyl), deuterated $C_{1-6}$ alkyl, $C_{1-6}$ alkoxy (e.g., $C_{1-4}$ alkoxy, such as methoxy), deuterated $C_{1-6}$ alkoxy or halogenated $C_{1-6}$ alkoxy (e.g., halogenated $C_{1-4}$ alkoxy, such as trifluoromethoxy). In some sub-embodiments of the present disclosure, $R_{a1}$, $R_{a2}$, $R_{a3}$, and $R_{a4}$ each independently represent H.

In some embodiments of the present disclosure, at least one of $Z_1$, $Z_2$, $Z_3$, and $Z_4$ represents C(S).

In some embodiments of the present disclosure, at least two of $Z_1$, $Z_2$, $Z_3$, and $Z_4$ represent C(S).

In some embodiments of the present disclosure, at least three of $Z_1$, $Z_2$, $Z_3$, and $Z_4$ represent C(S).

In some embodiments of the present disclosure, $Z_1$, $Z_2$, $Z_3$, and $Z_4$ each represent C(S).

In some embodiments of the present disclosure, $Z_1$ represents C(S).

In some embodiments of the present disclosure, $Z_2$ represents C(S).

In some embodiments of the present disclosure, $Z_3$ represents C(S).

In some embodiments of the present disclosure, $Z_4$ represents C(S).

In some embodiments of the present disclosure, $Z_1$ and $Z_4$ represent C(S).

In some embodiments of the present disclosure, $Z_2$ and $Z_4$ represent C(S).

In some embodiments of the present disclosure, $Z_3$ and $Z_4$ represent C(S).

In some embodiments of the present disclosure, $Z_1$ and $Z_2$ represent C(S).

In some embodiments of the present disclosure, $Z_1$, $Z_3$, and $Z_4$ represent C(S).

In some embodiments of the present disclosure, $Z_2$, $Z_3$, and $Z_4$ represent C(S).

In some embodiments of the present disclosure, $Z_1$, $Z_2$, and $Z_3$ represent C(S).

In some embodiments of the present disclosure, $Z_1$, $Z_2$, and $Z_4$ represent C(S).

In some embodiments of the present disclosure, $Z_1$ represents $CH_2$, $Z_2$ represents C(S), and $Z_3$ and $Z_4$ represent C(O).

In some embodiments of the present disclosure, $Z_1$ represents $CH_2$, $Z_2$ represents C(S), $Z_3$ represents C(O), and $Z_4$ represents C(S).

In some embodiments of the present disclosure, $Z_1$ represents $CH_2$, $Z_2$ represents C(S), and $Z_3$ and $Z_4$ represent C(S).

In some embodiments of the present disclosure, $Z_1$ and $Z_2$ represent C(S), $Z_3$ represents C(O), and $Z_4$ represents C(S).

In some embodiments of the present disclosure, $Z_1$, $Z_2$, $Z_3$, and $Z_4$ represent C(S).

In some embodiments of the present disclosure, $(R_{a5})_m$ indicates that the isoindoline ring in Formula (I) to which it is attached is optionally substituted with m $R_{a5}$, wherein each $R_{a5}$ is identical or different and each independently represents deuterium, halogen (e.g., fluorine, chlorine, bromine, or iodine), hydroxy, mercapto, nitro, amino, cyano, $C_{1-6}$ alkyl (e.g., $C_{1-5}$ alkyl, $C_{1-4}$ alkyl or $C_{1-3}$ alkyl, such as methyl, ethyl, propyl, isopropyl, butyl, sec-butyl, tert-butyl, pentyl, or hexyl), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, n-butoxy, sec-butoxy, or tert-butoxy), deuterated $C_{1-6}$ alkoxy, halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), $C_{2-6}$ alkenyl (e.g., vinyl), or $C_{2-6}$ alkynyl (e.g., ethynyl), and m represents an integer of 0, 1, 2 or 3. Optionally, in some sub-embodiments of the present disclosure, m represents an integer of 0, 1 or 2. Optionally, in some sub-embodiments of the present disclosure, each $R_{a5}$ is identical or different and each independently represents deuterium, halogen (e.g., fluorine, chlorine, bromine, or iodine), hydroxy, mercapto, nitro, amino, cyano, $C_{1-4}$ alkyl (e.g., $C_{1-3}$ alkyl, such as methyl, ethyl, propyl, isopropyl, butyl, sec-butyl, or tert-butyl), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-).

In the embodiments of the present disclosure, the number of substituents is not theoretically limited in any way, or is automatically limited by the size of the building units.

In some embodiments of the present disclosure, R represents O, S, S(O), $S(O)_2$, alkenylene, alkynylene or $N(R_{a6})$, wherein $R_{a6}$ represents H or $C_{1-3}$ alkyl. Optionally, in some sub-embodiments of the present disclosure, R represents O, S, S(O), $S(O)_2$, $C_{2-6}$ alkenylene (e.g., vinylene), $C_{2-6}$ alkynylene (e.g., ethynylene), NH or $N(CH_3)$.

In some embodiments of the present disclosure, R represents a bond.

In some embodiments of the present disclosure, R represents :

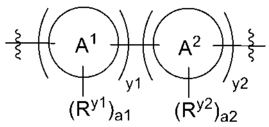

wherein each ring $A^1$ is identical or different and each independently represents nitrogen-containing heterocyclylene, arylene or heteroarylene, y1 represents an integer of 1 or 2, and $(R^{y1})_{a1}$ indicates that each ring $A^1$ is independently optionally substituted with a1 $R^{y1}$ groups, with each $R^{y1}$ being independently deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a1 represents an integer of 0-20; and/or

each ring $A^2$ is identical or different and each independently represents heterocyclylene, cycloalkylene, arylene or heteroarylene, y2 represents an integer of 0 or 1, and $(R^{y2})_{a2}$ indicates that each ring $A^2$ is independently optionally substituted with a2 $R^{y2}$ groups, with each $R^{y2}$ being independently deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a2 represents an integer of 0-20.

In some embodiments of the present disclosure, y1 represents an integer of **1** or 2, and each ring $A^1$ is identical or different and each independently represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene).

In some embodiments of the present disclosure, y1 represents an integer of 1 or 2, and each ring $A^1$ independently represents:

piperidinylene, piperazinylene, morpholinylene, azetidinylene, pyrrolidinylene, imidazolidinylene, pyrazolidylene, oxazolidinylene, thiazolidinylene, thiomorpholinylene, azacycloheptylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, 3-methyl-2-oxa-8-azaspiro[4.5]decanylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene.

In some embodiments of the present disclosure, y1 represents an integer of 1 or 2, and each ring $A^1$ independently represents:

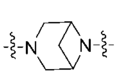 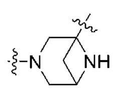 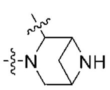 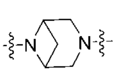 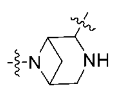 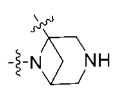 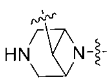 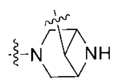 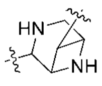 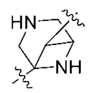

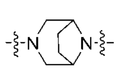 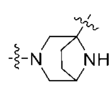 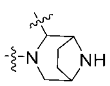 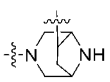 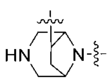 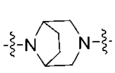 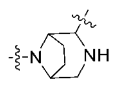 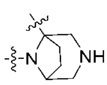 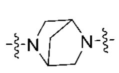 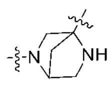

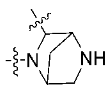 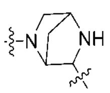 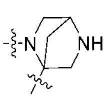 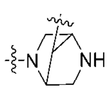 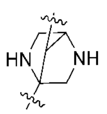 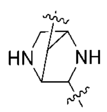 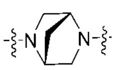 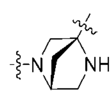 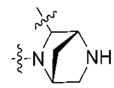 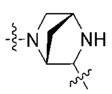

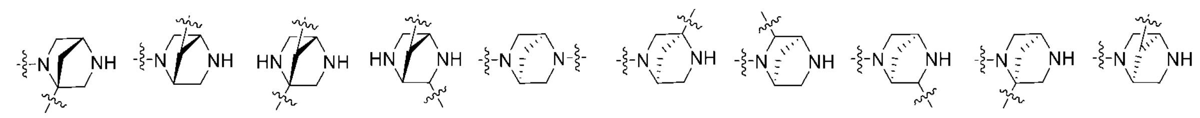

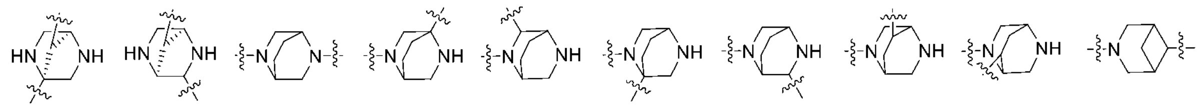

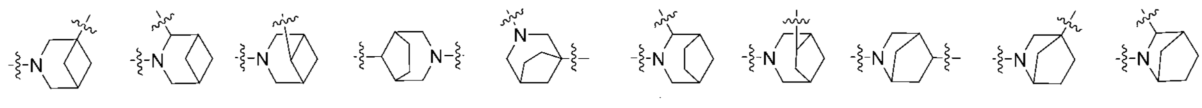

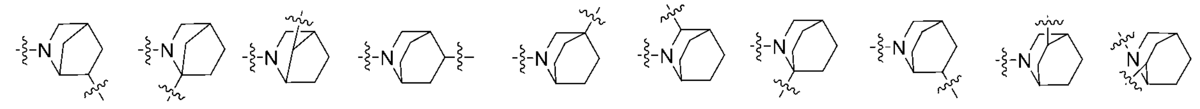

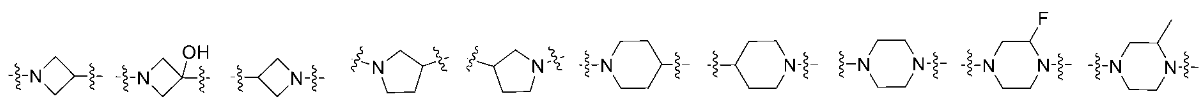

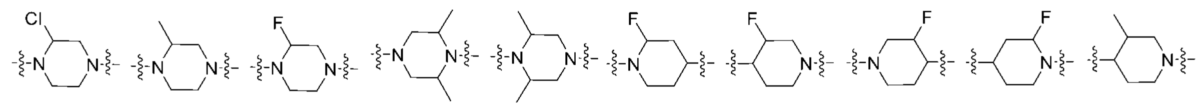

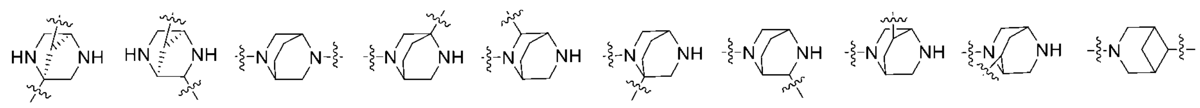

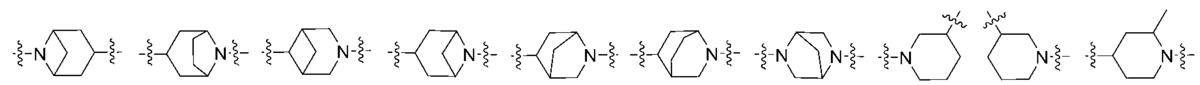

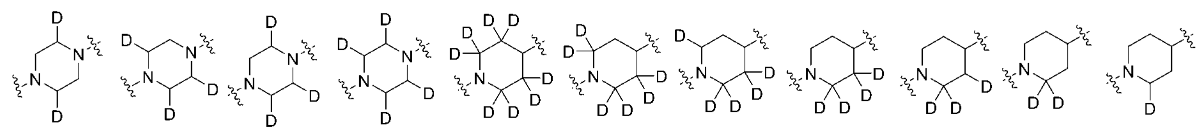

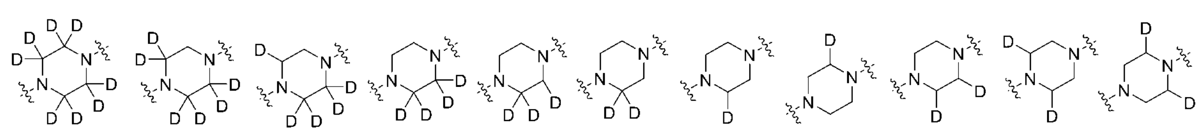

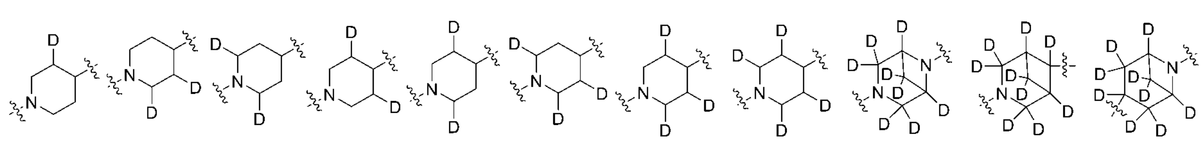

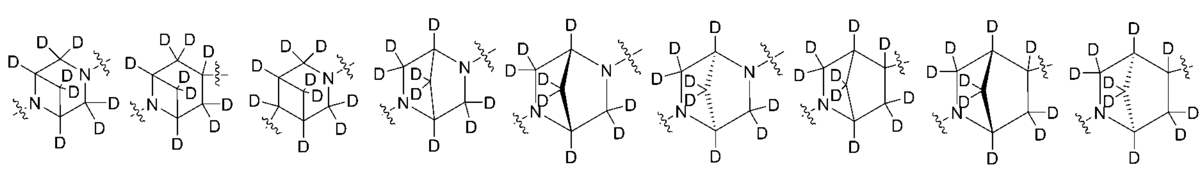

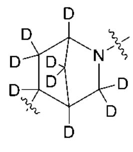 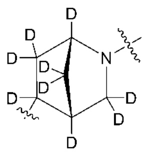 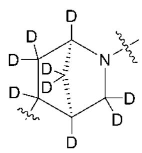 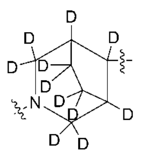 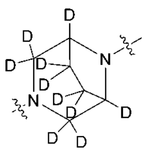 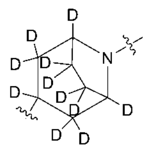 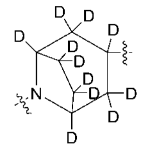 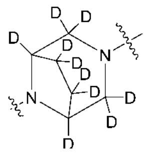

or

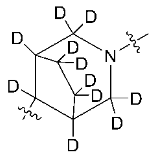

.

In some embodiments of the present disclosure, y1 represents an integer of 1 or 2, and each ring $A^1$ is independently optionally substituted with a1 $R^{y1}$ groups, wherein each $R^{y1}$ is independently deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a1 represents an integer of 0-20. Optionally, in some sub-embodiments of the present disclosure, each $R^{y1}$ independently represents deuterium, halogen (e.g., fluorine, chlorine, bromine, or iodine), hydroxy, mercapto, amino, cyano, $C_{1-4}$ alkyl (e.g., $C_{1-3}$ alkyl, such as methyl, ethyl, propyl, isopropyl, butyl, sec-butyl, or tert-butyl), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-). Optionally, in some sub-embodiments of the present disclosure, a1 represents an integer of 0, 1, 2, 3, 4, 5, 6, 7, 8, 9 or 10.

In some embodiments of the present disclosure, y2 represents an integer of 0 or 1.

In some embodiments of the present disclosure, ring $A^2$ represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene).

In some embodiments of the present disclosure, ring $A^2$ represents:
azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo [2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, *5*- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, $C_{5-15}$ spiro-cycloalkylene, adamantanylene, noradamantanylene, norbomylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene.

In some embodiments of the present disclosure, y2 represents an integer of 0 or 1, and ring $A^2$ represents:

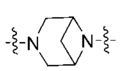 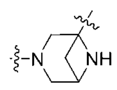 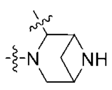 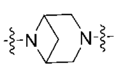 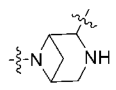 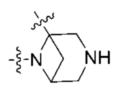 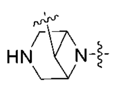 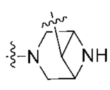 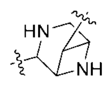 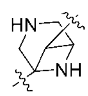

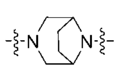 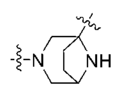 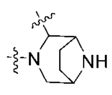 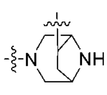 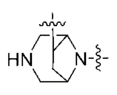 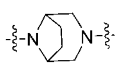 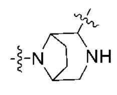 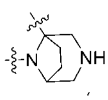 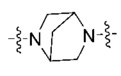 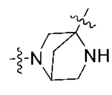

,

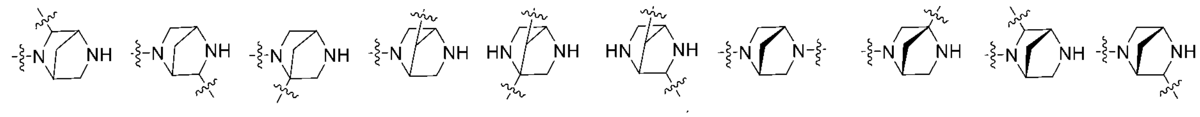

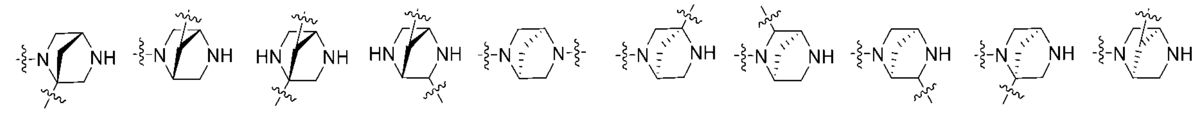

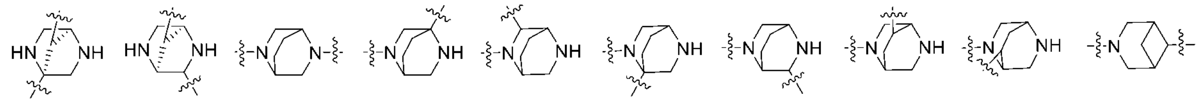

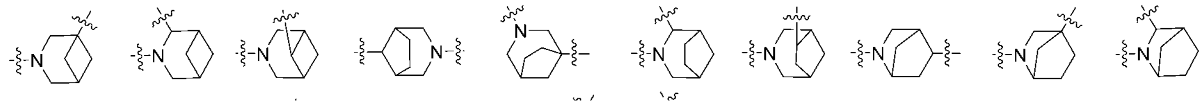

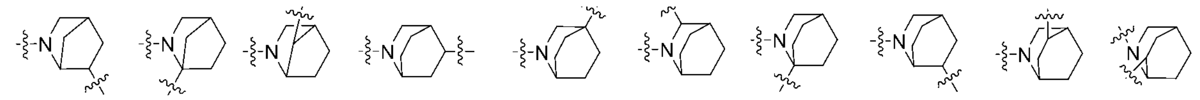

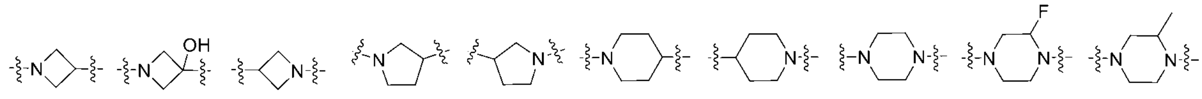

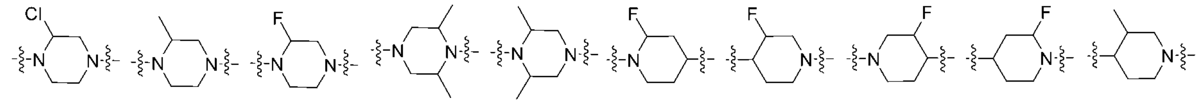

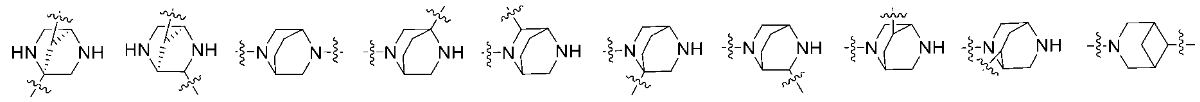

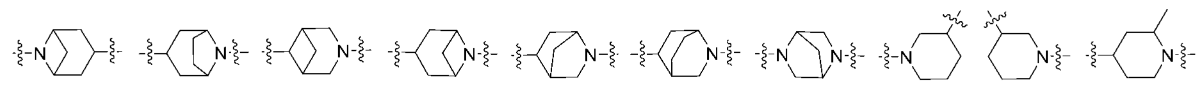

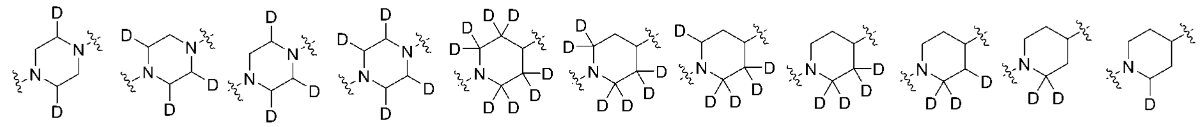

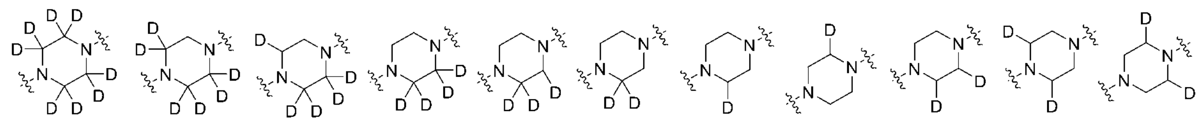

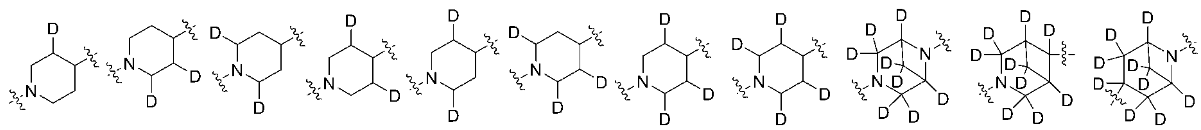

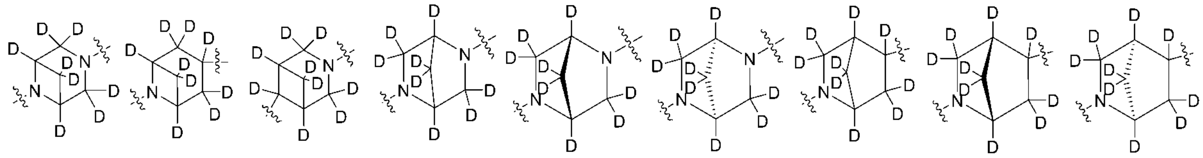

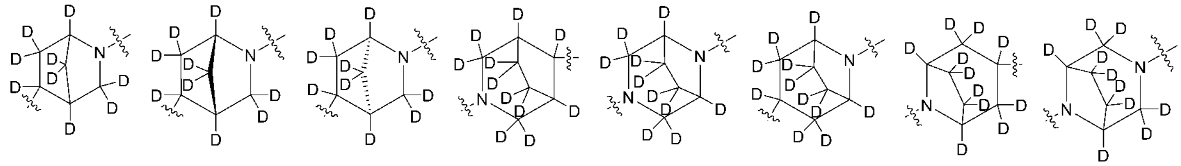

or

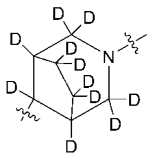

.

In some embodiments of the present disclosure, y2 represents an integer of 0 or 1, and ring $A^2$ is independently optionally substituted with a2 $R^{y2}$ groups, wherein each $R^{y2}$ is independently deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a2 represents an integer of 0-20. Optionally, in some sub-embodiments of the present disclosure, each $R^{y2}$ independently represents deuterium, halogen (e.g., fluorine, chlorine, bromine, or iodine), hydroxy, mercapto, amino, cyano, $C_{1-4}$ alkyl (e.g., $C_{1-3}$ alkyl, such as methyl, ethyl, propyl, isopropyl, butyl, sec-butyl, or tert-butyl), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-). Optionally, in some sub-embodiments of the present disclosure, a2 represents an integer of 0, 1, 2, 3, 4, 5, 6, 7, 8, 9 or 10.

In some embodiments of the present disclosure, R represents :

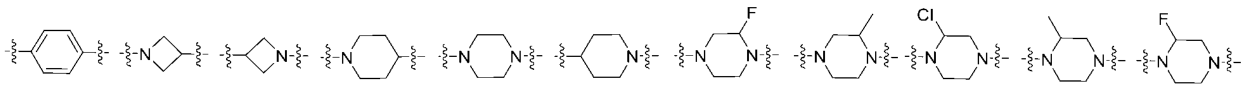

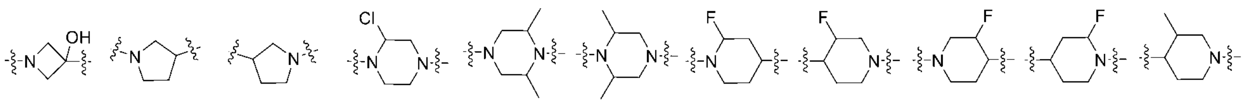

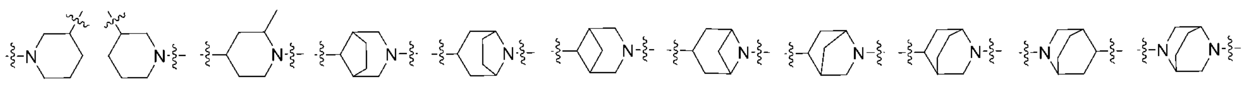

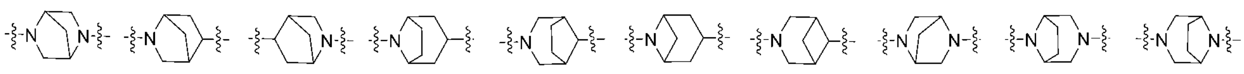

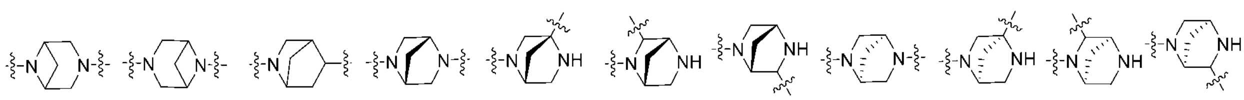

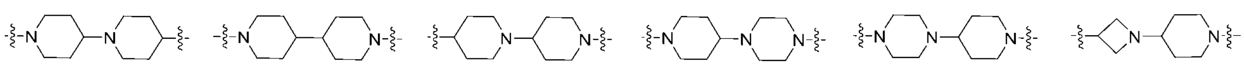

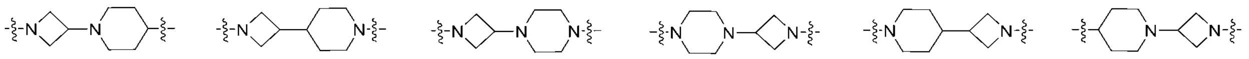
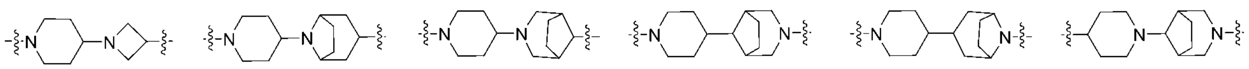
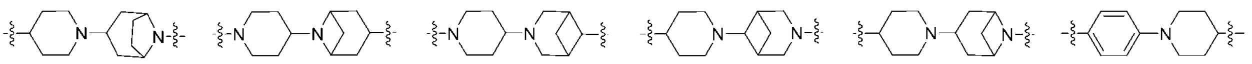
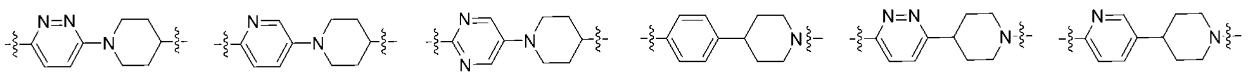
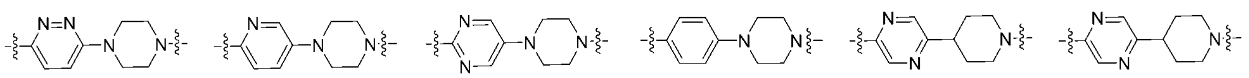
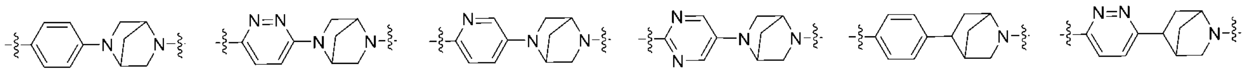
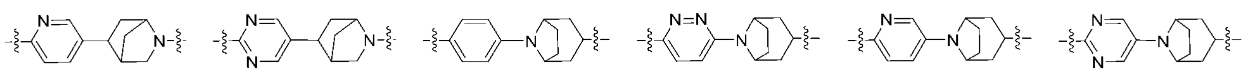
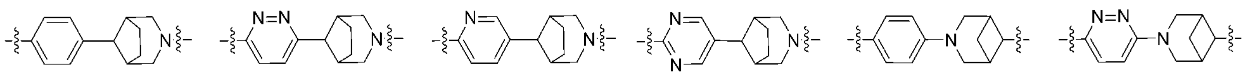
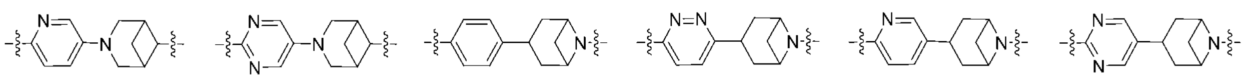
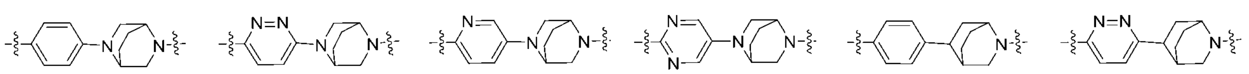
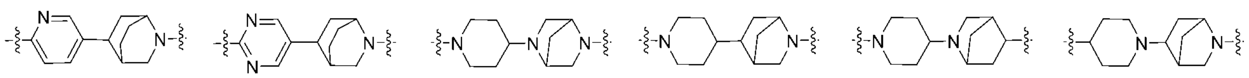
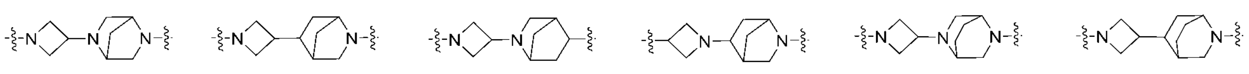
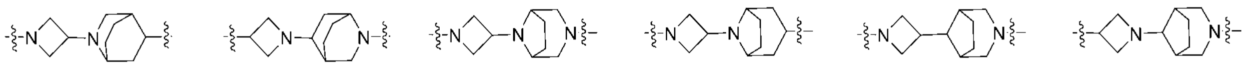
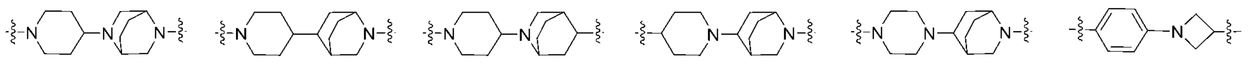
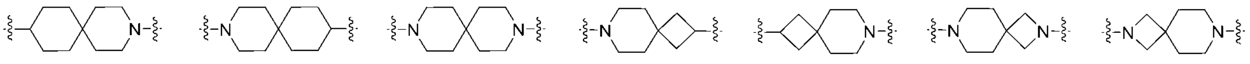
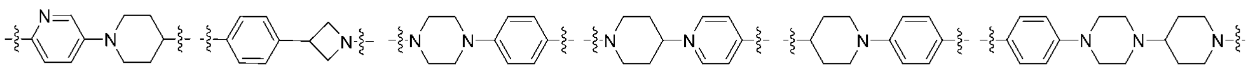
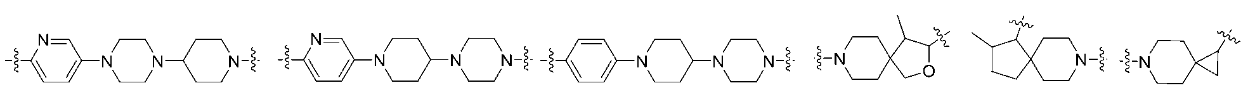

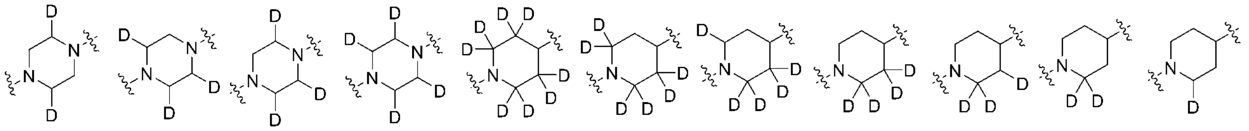
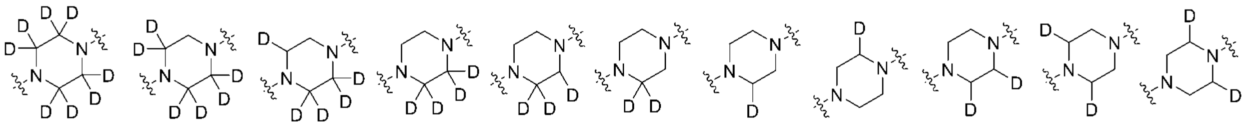
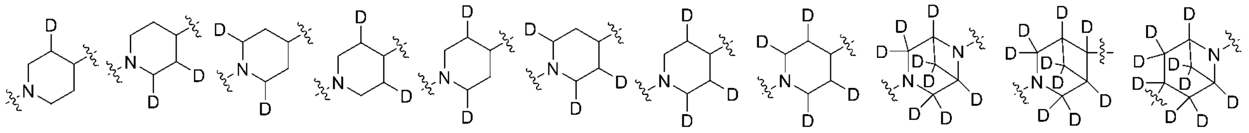
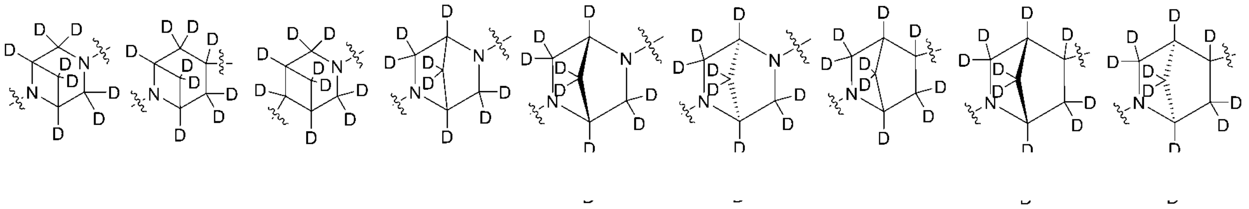
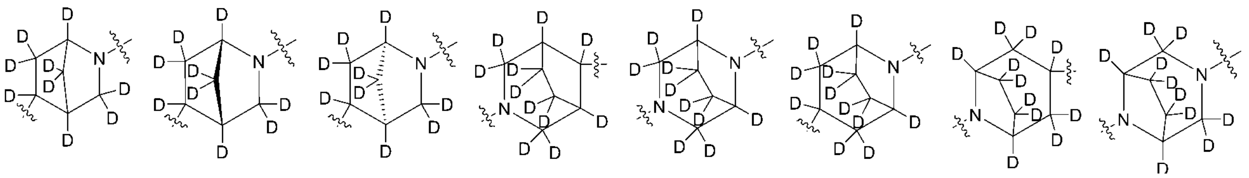

or

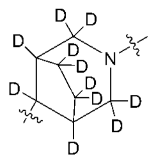

;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl, and $C_{2-6}$ alkenyl.

In some embodiments of the present disclosure, L represents :

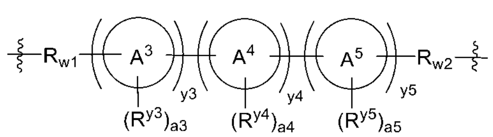

wherein $R_{w1}$ is linked to R, and $R_{w2}$ is linked to X;

$R_{w2}$ represents a bond, C(O), C(O)NH, NHC(O) or N($R_{a6}$), wherein $R_{a6}$ represents H or $C_{1-3}$ alkyl;

each ring $A^3$ is identical or different and each independently represents heterocyclylene, cycloalkylene, arylene, or heteroarylene, y3 represents an integer of 0, 1, 2 or 3, and $(R^{y3})_{a3}$ indicates that each ring $A^3$ is independently optionally substituted with a3 $R^{y3}$ substituents, wherein each $R^{y3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a3 represents an integer of 0-20;

each ring $A^4$ is identical or different and each independently represents heterocyclylene, cycloalkylene, arylene, or heteroarylene, y4 represents an integer of 0, 1, 2 or 3, and $(R^{y4})_{a4}$ indicates that each ring $A^4$ is independently

optionally substituted with a4 $R^{y4}$ substituents, wherein each $R^{y4}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a4 represents an integer of 0-20;

each ring $A^5$ is identical or different and each independently represents heterocyclylene, cycloalkylene, arylene, or heteroarylene, y5 represents an integer of 0, 1, 2 or 3, and $(R^{y5})_{a5}$ indicates that each ring $A^5$ is independently optionally substituted with a5 $R^{y5}$ substituents, wherein each $R^{y5}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a5 represents an integer of 0-20; and/or

$R_{w1}$ represents a bond or an optionally substituted linear or branched $C_{1-20}$ alkylene, wherein any one or more (e.g., 1-15, 1-10, 1-6, 1-4, 2-6, or 1) methylene groups in the main carbon chain skeleton of the linear or branched $C_{1-20}$ alkylene are optionally replaced by one or more groups selected from: $R_a$, $R_b$, and any combination thereof; wherein each $R_a$ is independently selected from the group consisting of: O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, wherein each $R_{a7}$ independently represents H or $C_{1-6}$ alkyl; and when any two or more methylene in the main carbon chain skeleton of the linear or branched $C_{1-20}$ alkylene are replaced by two or more $R_a$ groups, the $R_a$ groups are not directly connected to each other; and wherein each $R_b$ is independently selected from the group consisting of: optionally substituted cycloalkylene, optionally substituted arylene, optionally substituted heterocyclylene, optionally substituted heteroarylene, alkynylene, alkenylene, and any combination thereof.

In some embodiments of the present disclosure, $R_{w2}$ represents a bond, C(O), C(O)NH, NHC(O) or $N(R_{a6})$, wherein $R_{a6}$ represents H or $C_{1-3}$ alkyl.

In some embodiments of the present disclosure, $R_{w2}$ represents a bond.

In some embodiments of the present disclosure, y3 represents an integer of 0, 1, 2 or 3, and each ring $A^3$ independently represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene), or 5- to 30-membered heteroarylene (e.g., 5- to 20-membered heteroarylene, or 5- to 15-membered heteroarylene).

In some embodiments of the present disclosure, y3 represents an integer of 0, 1, 2 or 3, and each ring $A^3$ independently represents:

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, *5*- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, $C_{5-15}$ spiro-cycloalkylene, adamantanylene, noradamantanylene, norbomylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene.

In some embodiments of the present disclosure, y3 represents an integer of 0, 1, 2 or 3, and each ring $A^3$ independently represents:

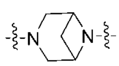 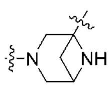 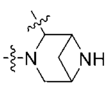 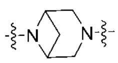 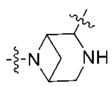 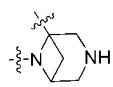 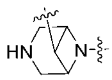 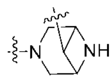 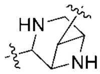 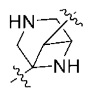

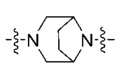 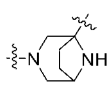 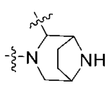 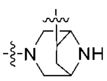 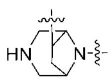 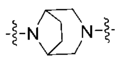 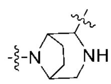 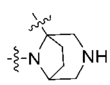 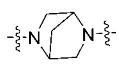 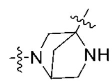

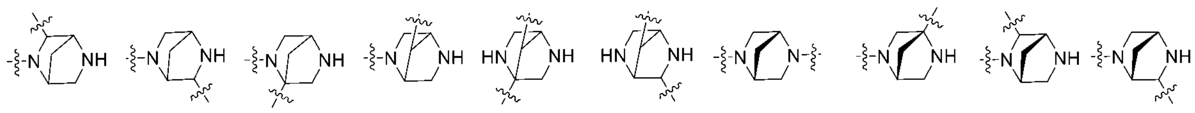

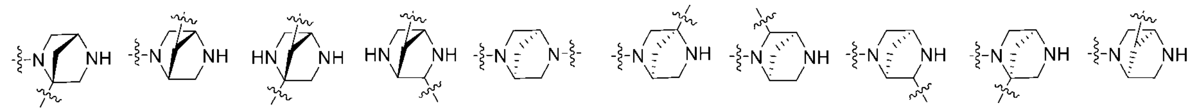

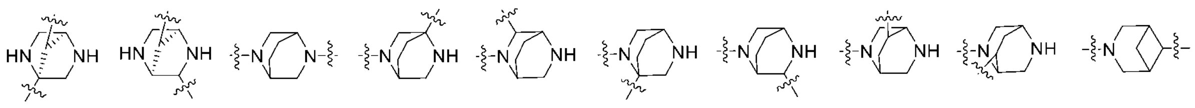

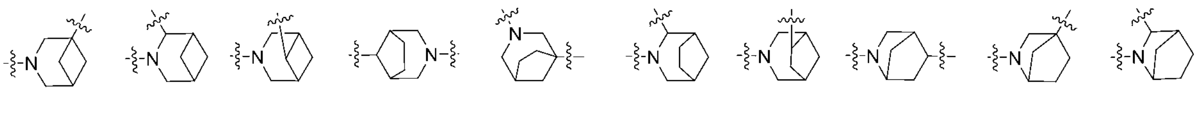

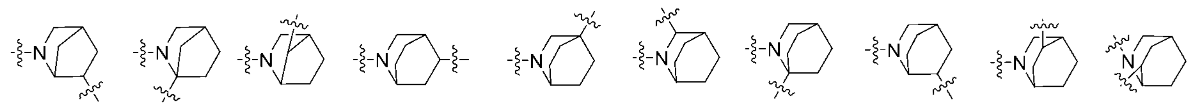

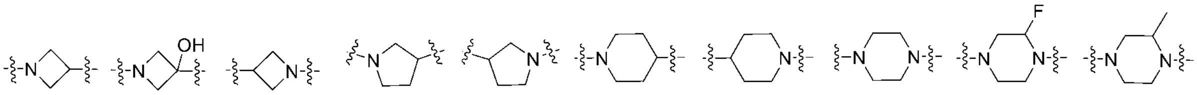

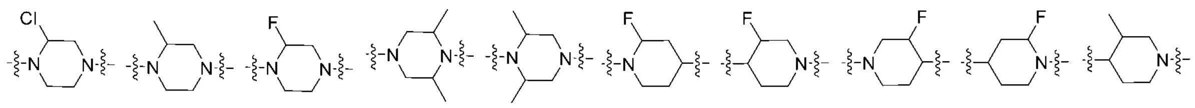

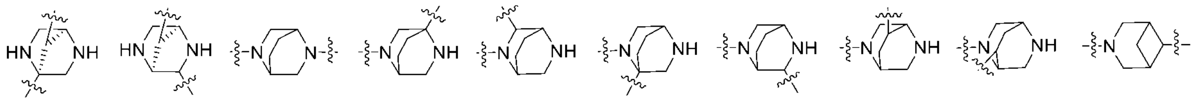

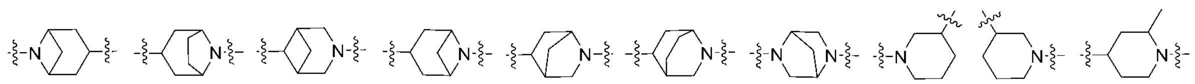

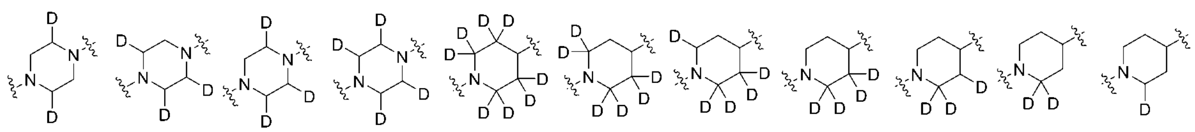

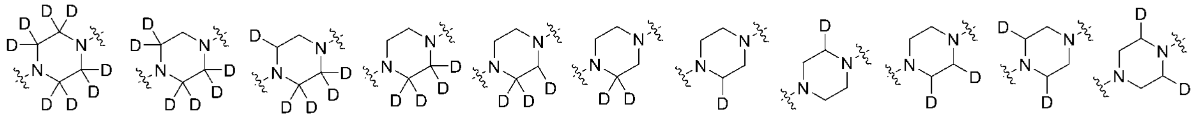

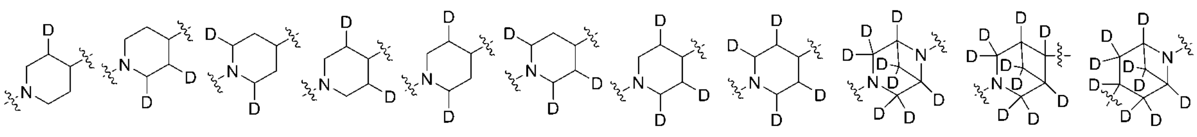

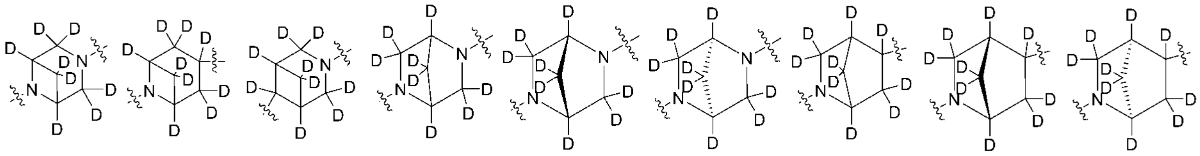

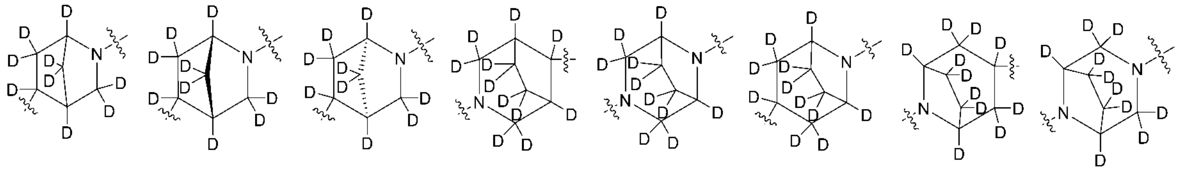

or

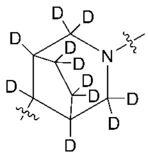

.

In some embodiments of the present disclosure, y3 represents an integer of 0, 1, 2 or 3, and each ring $A^3$ is independently optionally substituted with a3 $R^{y3}$ substituents, wherein each $R^{y3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a3 represents an integer of 0-20. Optionally, in some sub-embodiments of the present disclosure, each $R^{y3}$ independently represents deuterium, halogen (e.g., fluorine, chlorine, bromine, or iodine), hydroxy, mercapto, amino, cyano, $C_{1-4}$ alkyl (e.g., $C_{1-3}$ alkyl, such as methyl, ethyl, propyl, isopropyl, butyl, sec-butyl, or tert-butyl), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O-, or $CH_2ClCH_2$-O-). Optionally, in some sub-embodiments of the present disclosure, a3 represents an integer of 0, 1, 2, 3, 4, 5, 6, 7, 8, 9, 10, 11, 12, 13, 14 or 15.

In some embodiments of the present disclosure, y4 represents an integer of 0, 1, 2 or 3, and each ring $A^4$ independently represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene), or 5- to 30-membered heteroarylene (e.g., 5- to 20-membered heteroarylene, or 5- to 15-membered heteroarylene).

In some embodiments of the present disclosure, y4 represents an integer of 0, 1, 2 or 3, and each ring $A^4$ independently represents:

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo [2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, *5*- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, $C_{5-15}$ spiro-cycloalkylene, adamantanylene, noradamantanylene, norbomylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene.

In some embodiments of the present disclosure, y4 represents an integer of 0, 1, 2 or 3, and each ring $A^4$ independently represents:

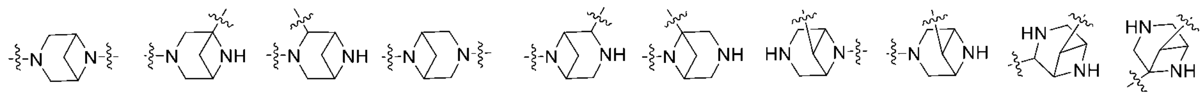

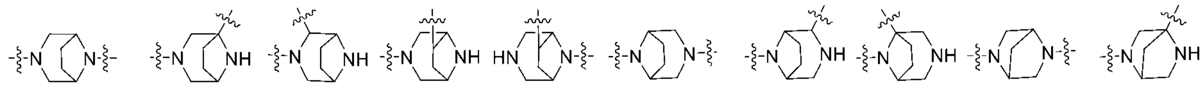

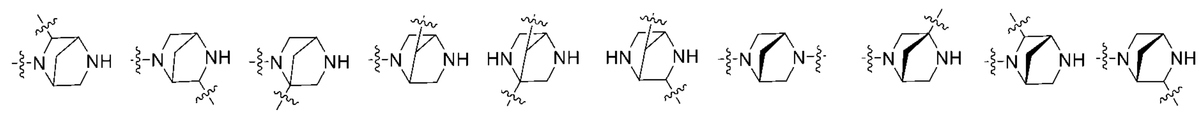

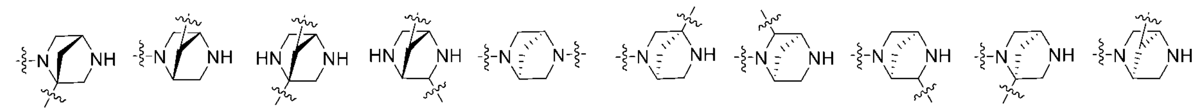

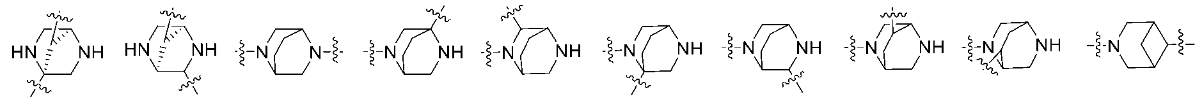

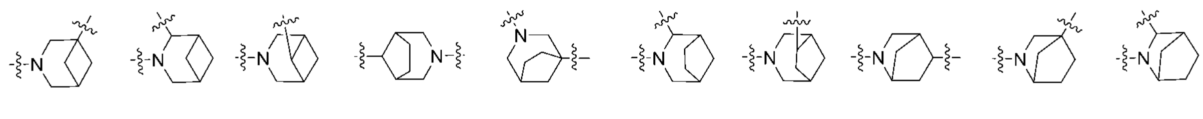

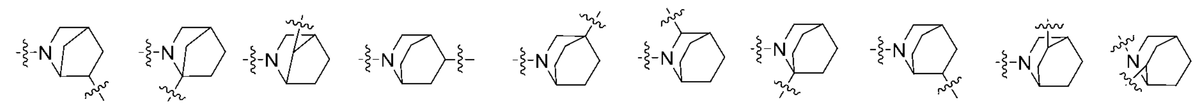

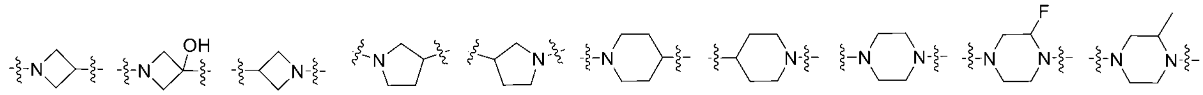

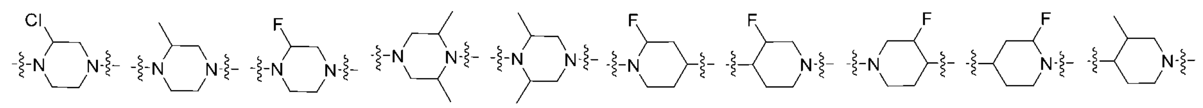

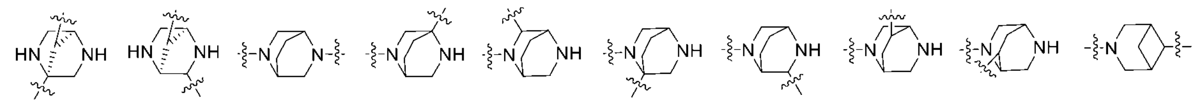

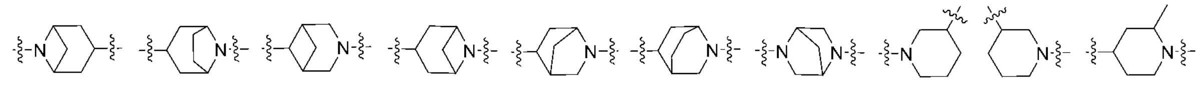

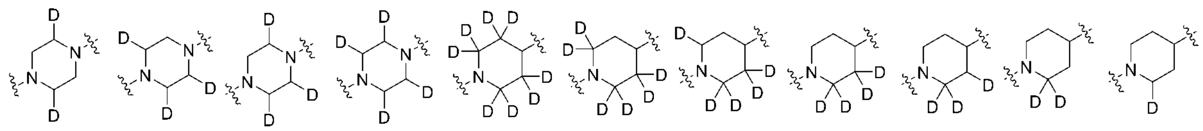

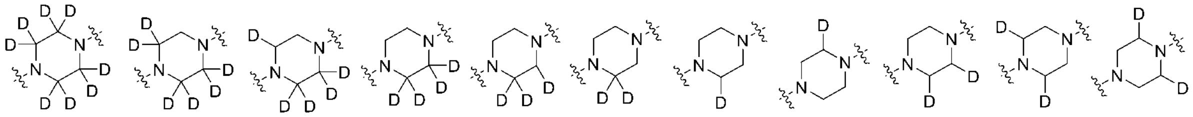

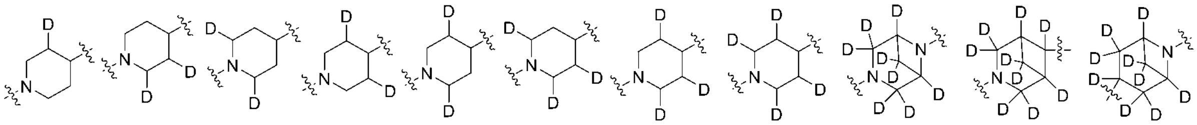

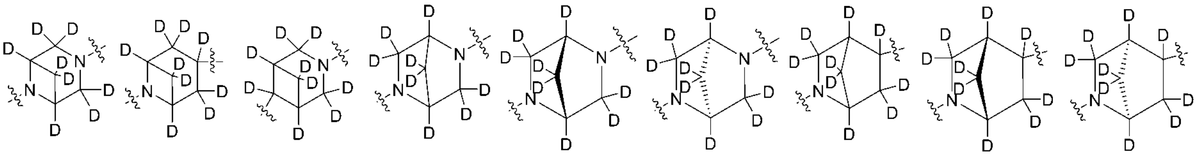

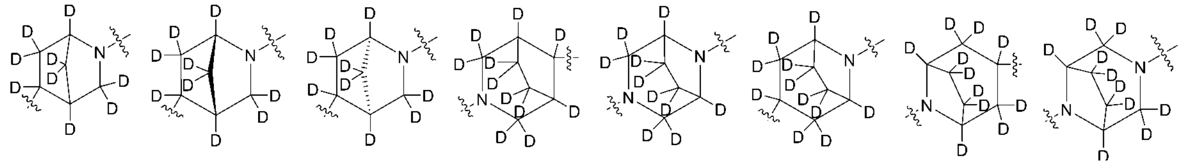

or

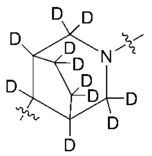

.

In some embodiments of the present disclosure, y4 represents an integer of 0, 1, 2 or 3, and each ring $A^4$ is independently optionally substituted with a4 $R^{y4}$ substituents, wherein each $R^{y4}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a4 represents an integer of 0-20. Optionally, in some sub-embodiments of the present disclosure, each $R^{y4}$ independently represents deuterium, halogen (e.g., fluorine, chlorine, bromine, or iodine), hydroxy, mercapto, amino, cyano, $C_{1-4}$ alkyl (e.g., $C_{1-3}$ alkyl, such as methyl, ethyl, propyl, isopropyl, butyl, sec-butyl, or tert-butyl), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-). Optionally, in some sub-embodiments of the present disclosure, a4 represents an integer of 0, 1, 2, 3, 4, 5, 6, 7, 8, 9, 10, 11, 12, 13, 14 or 15.

In some embodiments of the present disclosure, y5 represents an integer of 0, 1, 2 or 3, and each ring $A^5$ independently represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene).

In some embodiments of the present disclosure, y5 represents an integer of 0, 1, 2 or 3, and each ring $A^5$ independently represents:

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo [2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, *5*- to 20-membered azaspirocycloalkylene, cyclopropylene, cyclobutylene, cyclopentylene, cyclopentenylene, cyclohexylene, cyclohexenylene, cycloheptylene,

cyclooctylene, decalinylene, octahydropentalenylene, octahydro-1H-indenylene, 2,3-dihydro-1H-indenylene, $C_{5-15}$ spiro-cycloalkylene, adamantanylene, noradamantanylene, norbomylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene.

In some embodiments of the present disclosure, y5 represents an integer of 0, 1, 2 or 3, and each ring $A^5$ independently represents:

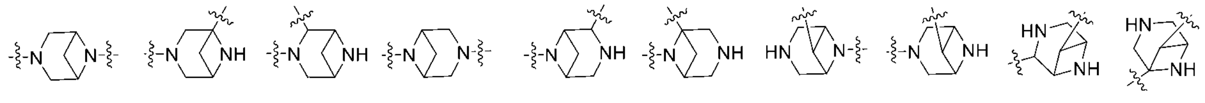

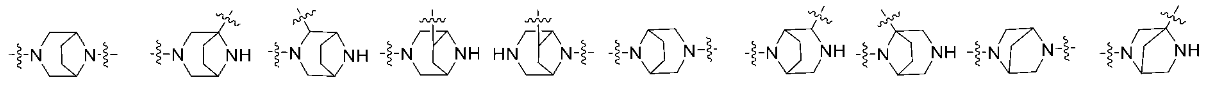

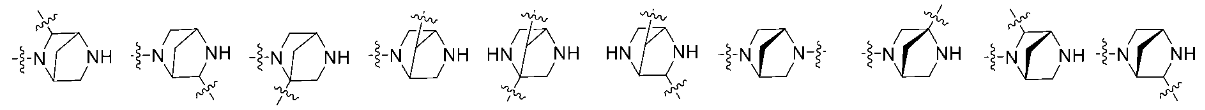

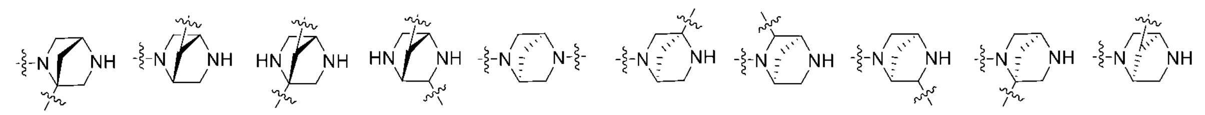

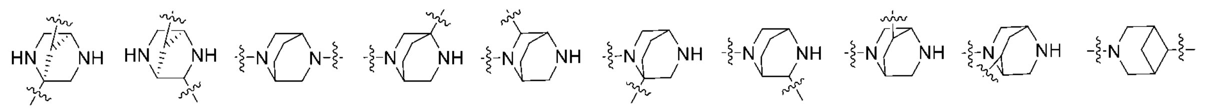

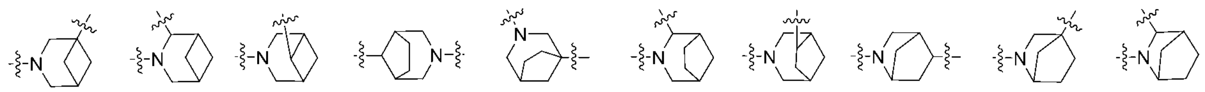

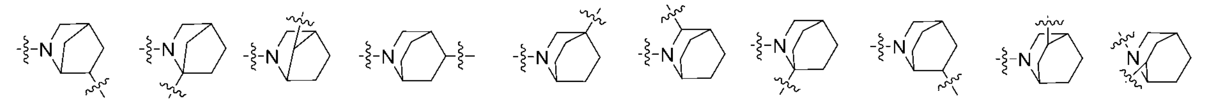

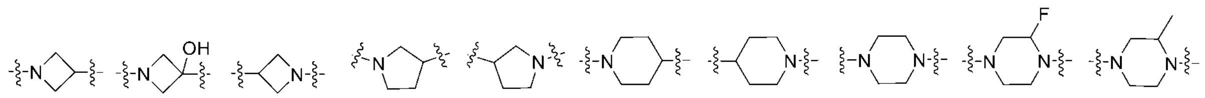

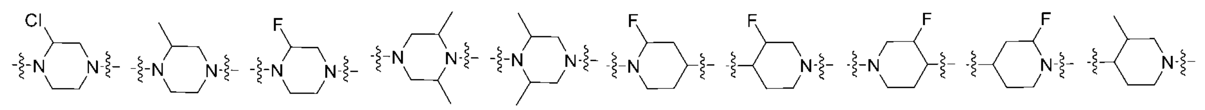

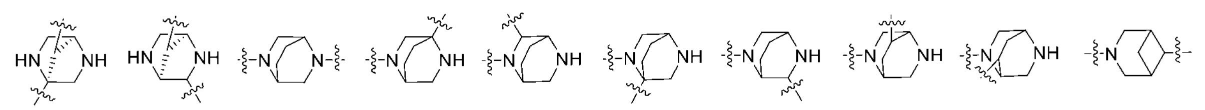

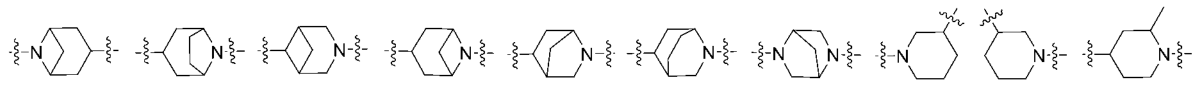

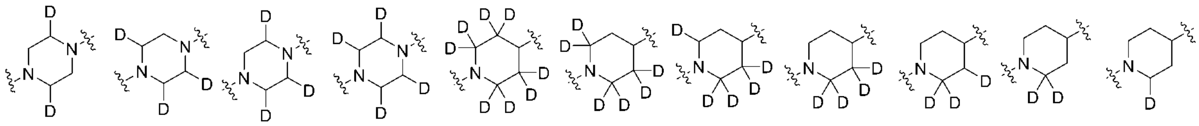

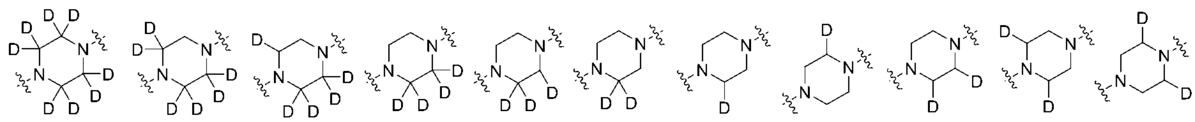

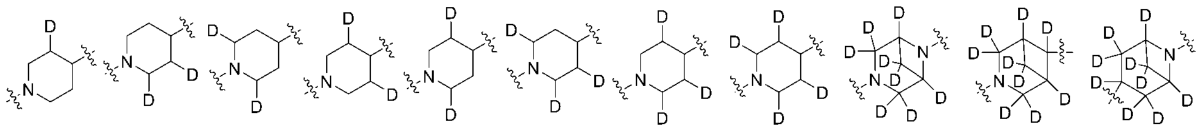

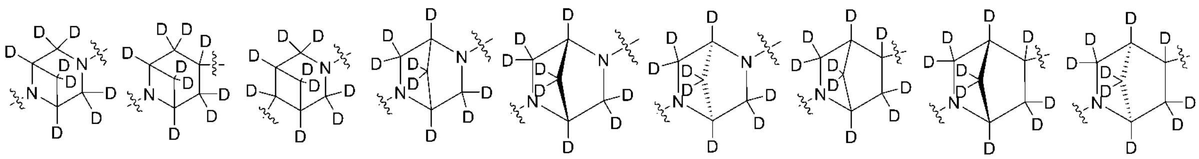

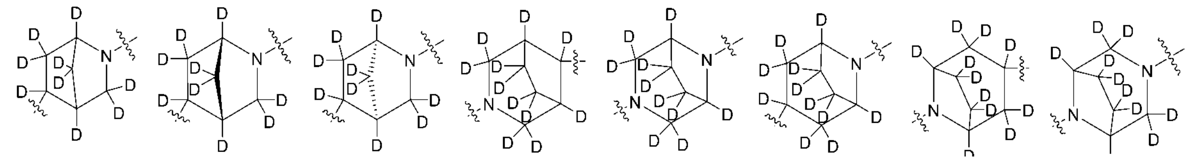

or

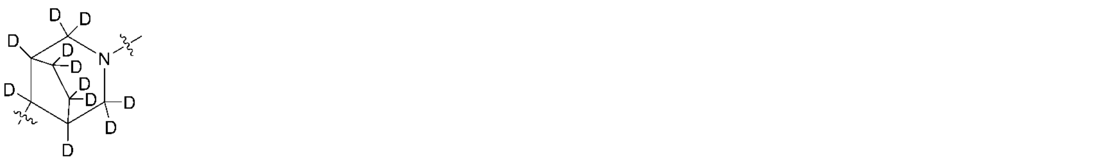

.

In some embodiments of the present disclosure, y5 represents an integer of 0, 1, 2 or 3, and each ring $A^5$ is independently optionally substituted with a5 $R^{y5}$ substituents, wherein each $R^{y5}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a5 represents an integer of 0-20. Optionally, in some sub-embodiments of the present disclosure, each $R^{y5}$ independently represents deuterium, halogen (e.g., fluorine, chlorine, bromine, or iodine), hydroxy, mercapto, amino, cyano, $C_{1-4}$ alkyl (e.g., $C_{1-3}$ alkyl, such as methyl, ethyl, propyl, isopropyl, butyl, sec-butyl, or tert-butyl), halogenated $C_{1-4}$ alkyl (e.g., halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-4}$ alkoxy (e.g., $C_{1-3}$ alkoxy, such as methoxy, ethoxy, propoxy, isopropoxy, n-butoxy, sec-butoxy, or tert-butoxy), or halogenated $C_{1-4}$ alkoxy (e.g., halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-). Optionally, in some sub-embodiments of the present disclosure, a5 represents an integer of 0, 1, 2, 3, 4, 5, 6, 7, 8, 9, 10, 11, 12, 13, 14 or 15.

In some embodiments of the present disclosure, the

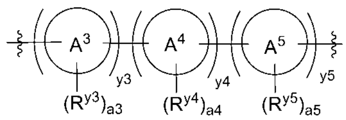

moiety in the general formula of L represents:

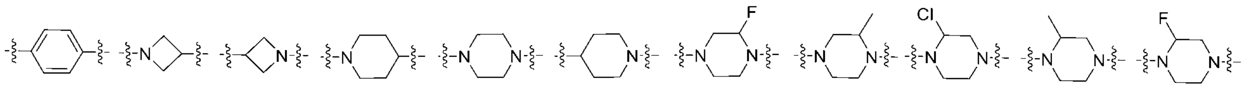

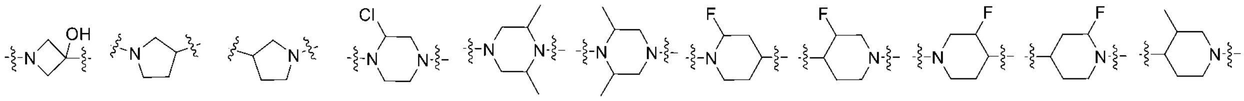

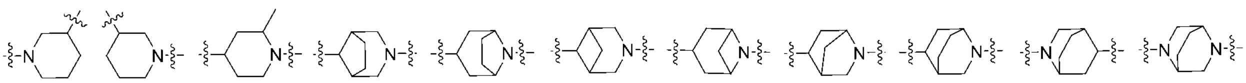

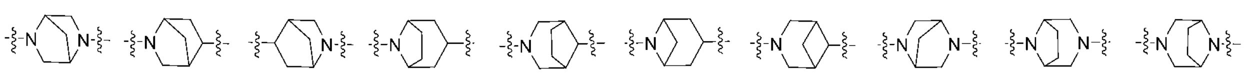

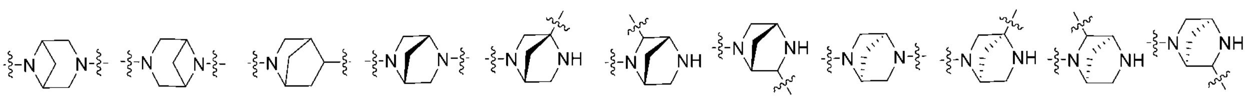

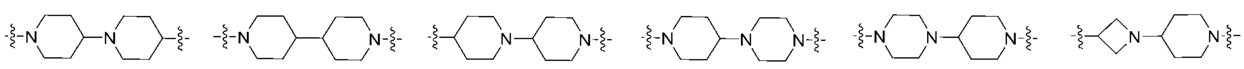

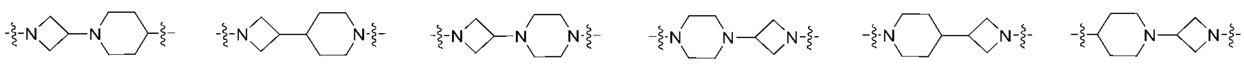

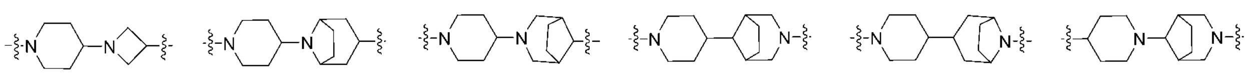

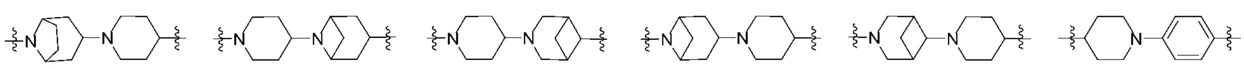

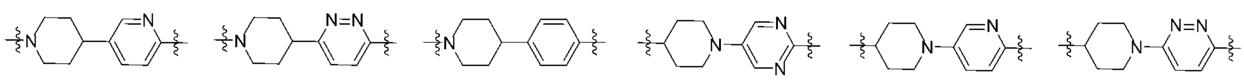

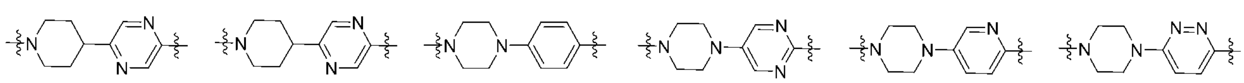

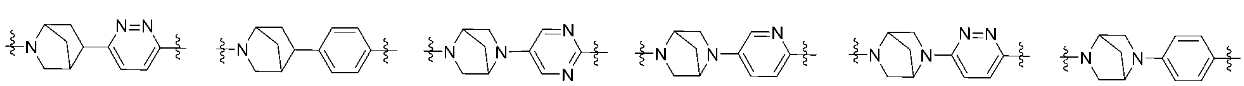

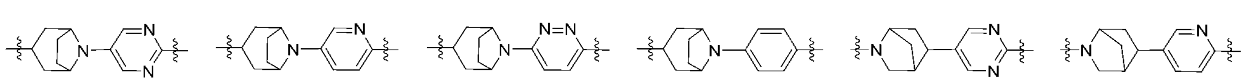

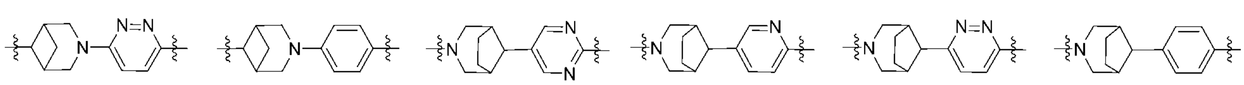

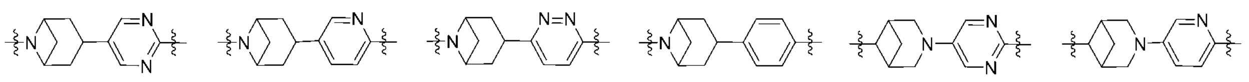

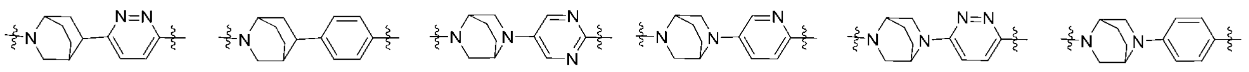

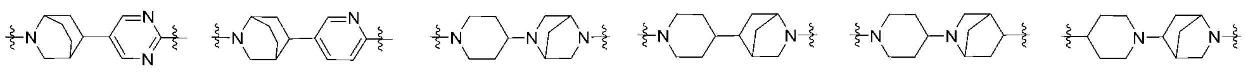

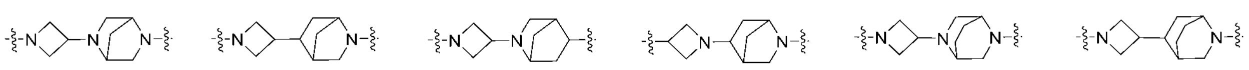

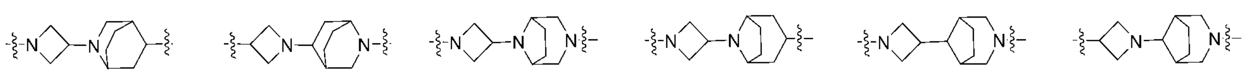

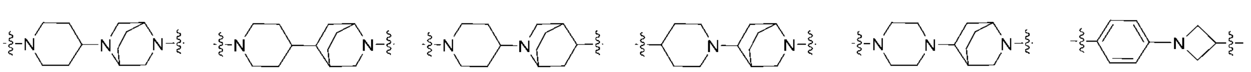

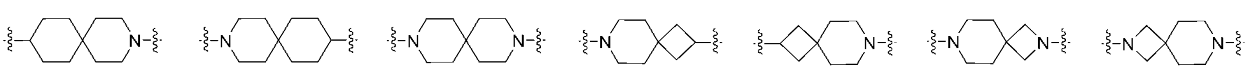

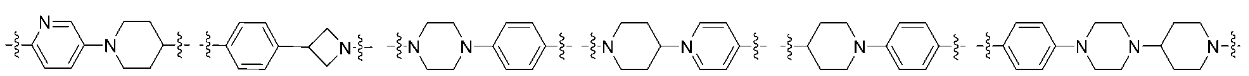

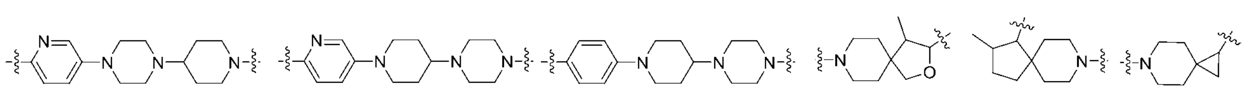

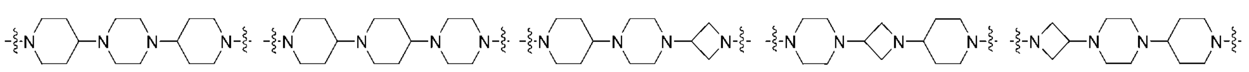

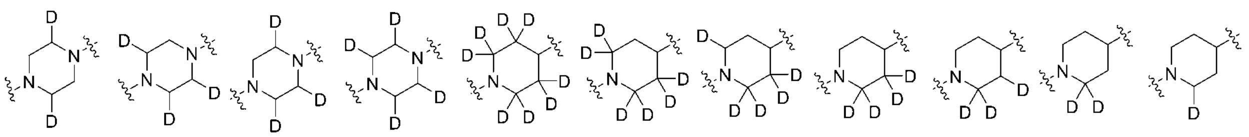

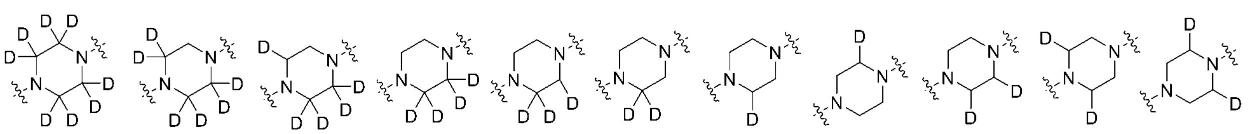

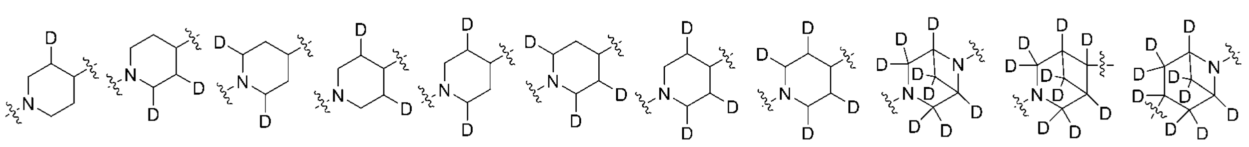

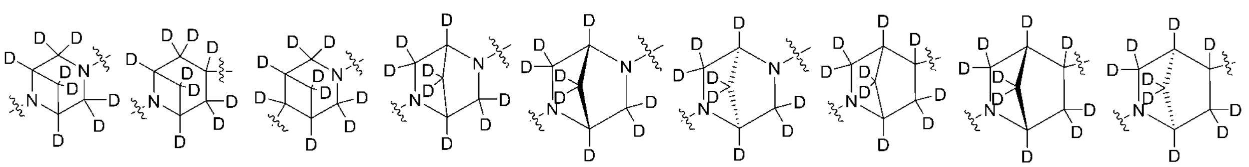

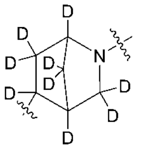 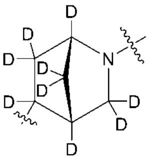 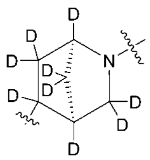 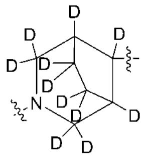 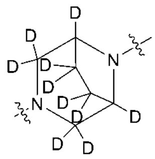 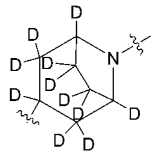 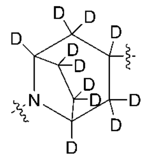 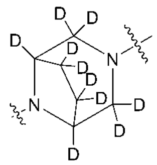

or

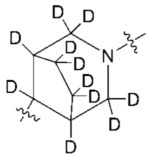 ;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl, and $C_{2-6}$ alkenyl.

In some embodiments of the present disclosure, $R_{w1}$ represents a bond.

In some embodiments of the present disclosure, $R_{w1}$ represents an optionally substituted linear or branched $C_{1-20}$ alkylene, wherein any one or more (e.g., 1-15, 1-10, 1-9, 1-8, 1-7, 1-6, 1-5, 1-4, 1-3 or 1-2, or 1) methylene groups in the main carbon chain skeleton of the linear or branched $C_{1-20}$ alkylene are optionally replaced by one or more (e.g., 1-15, 1-10, 1-9, 1-8, 1-7, 1-6, 1-5, 1-4, 1-3 or 1-2, or 1) groups selected from: $R_a$, $R_b$, and any combination thereof; wherein each $R_a$ is independently selected from the group consisting of: O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, wherein each $R_{a7}$ independently represents H or $C_{1-6}$ alkyl (e.g., $C_{1-4}$ alkyl or $C_{1-3}$ alkyl); and when any two or more methylene in the main carbon chain skeleton of the linear or branched $C_{1-20}$ alkylene are replaced by two or more $R_a$ groups, the $R_a$ groups are not directly connected to each other; and wherein each $R_b$ is independently selected from the group consisting of: optionally substituted cycloalkylene, optionally substituted arylene, optionally substituted heterocyclylene, optionally substituted heteroarylene, alkenylene, alkynylene, and any combination thereof. In some sub-embodiments, each $R_b$ is independently selected from the group consisting of: optionally substituted $C_{3-30}$ cycloalkylene, optionally substituted $C_{5-30}$ arylene, optionally substituted 4- to 30-membered heterocyclylene, optionally substituted 5- to 30-membered heteroarylene, $C_{2-6}$ alkenylene or $C_{2-6}$ alkynylene (preferably, optionally substituted $C_{3-20}$ cycloalkylene, optionally substituted $C_{5-20}$ arylene, optionally substituted 4- to 20-membered heterocyclylene, optionally substituted 5- to 20-membered heteroarylene, $C_{2-4}$ alkenylene or $C_{2-4}$ alkynylene; or more preferably, optionally substituted $C_{3-15}$ cycloalkylene, optionally substituted $C_{5-15}$ arylene, optionally substituted 4- to 15-membered heterocyclylene, optionally substituted 5- to 15-membered heteroarylene, $C_{2-4}$ alkenylene or $C_{2-4}$ alkynylene) , and any combination thereof. In some sub-embodiments, the cycloalkylene, arylene, heterocyclylene, and heteroarylene are each independently optionally substituted with a substituent selected from the group consisting of deuterium, hydroxy, amino, mercapto, nitro, halogen, cyano, oxo, $C_{1-6}$ alkyl (e.g., $C_{1-5}$ alkyl, $C_{1-4}$ alkyl or $C_{1-3}$ alkyl, such as methyl, ethyl, propyl, isopropyl, butyl, sec-butyl, tert-butyl, pentyl, or hexyl), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-6}$ alkoxy (e.g., $C_{14}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), $C_{3-6}$ cycloalkyl, halogenated $C_{3-6}$ cycloalkyl, deuterated $C_{3-6}$ cycloalkyl, 4- to 10-membered heterocyclyl, and any combination thereof. In some sub-embodiments, one or more (e.g., 1-40, 1-35, 1-30, 1-25, 1-20, 1-15, 1-10, 1-9, 1-8, 1-7, 1-6, 1-5, 1-4, 1-3 or 1-2, or 1) hydrogens of said $C_{1-20}$ alkylene are optionally replaced by a substituent selected from: deuterium, hydroxy, amino, mercapto, nitro, halogen, cyano, deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-6}$ alkoxy (e.g., $C_{1-4}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments of the present disclosure, $R_{w1}$ represents:

$-C_{1-15}$ alkylene-;
$-(C(R_{a8})(R_{a9}))_{r1}-(R_a(C(R_{a10})(R_{a11}))_{r2})_{s1}-*$;
$-(C(R_{a8})(R_{a9}))_{r1}-(R_a(C(R_{a10})(R_{a11}))_{r2})_{s1}-(R_a(C(R_{a12})(R_{a13}))_{r3})_{s2}-*$;
$-(C(R_{a8})(R_{a9}))_{r1}-(R_a(C(R_{a10})(R_{a11}))_{r2})_{s1}-(R_a(C(R_{a12})(R_{a13}))_{r3})_{s2}-(R_a(C(R_{a14})(R_{a15}))_{r4})_{s3}-*$;
$-(C(R_{a8})(R_{a9}))_{r1}-((C(R_{a10})(R_{a11}))_{r2}R_a)_{s1}-*$;
$-(C(R_{a8})(R_{a9}))_{r1}-((C(Ra_{10})(R_{a11}))_{r2}R_a)_{s1}-((C(R_{a12})(R_{a13}))_{r3}R_a)_{s2}-*$;
$-(C(R_{a8})(R_{a9}))_{r1}-((C(R_{a10})(R_{a11}))_{r2}R_a)_{s1}-((C(R_{a12})(R_{a13}))_{r3}R_a)_{s2}-((C(R_{a14})(R_{a15}))_{r4}R_a)_{s3}-*$;
$-(C(R_{a8})(R_{a9}))_{r1}-(R_b(C(R_{a10})(R_{a11}))_{r2})_{s1}-*$;
$-(C(R_{a8})(R_{a9}))_{r1}-(R_b(C(R_{a10})(R_{a11}))_{r2})_{s1}-(R_b(C(R_{a12})(R_{a13}))_{r3})_{s2}-*$;
$-(C(R_{a8})(R_{a9}))_{r1}-(R_b(C(R_{a10})(R_{a11}))_{r2})_{s1}-(R_b(C(R_{a12})(R_{a13}))_{r3})_{s2}-(R_b(C(R_{a14})(R_{a15}))_{r4})_{s3}-*$;
$-(C(R_{a8})(R_{a9}))_{r1}-((C(R_{a10})(R_{a11}))_{r2}R_b)_{s1}-*$;
$-(C(R_{a8})(R_{a9}))_{r1}-((C(R_{a10})(R_{a11}))_{r2}R_b)_{s1}-((C(R_{a12})(R_{a13}))_{r3}R_b)_{s2}-*$;
$-(C(R_{a8})(R_{a9}))_{r1}-((C(R_{a10})(R_{a11}))_{r2}R_b)_{s1}-((C(R_{a12})(R_{a13}))_{r3}R_b)_{s2}-((C(R_{a14})(R_{a15}))_{r4}R_b)_{s3}-*$;
$-(C(R_{a8})(R_{a9}))_{r1}-(R_a-R_b-(C(R_{a10})(R_{a11}))_{r2})_{s1}-*$;
$-(C(R_{a8})(R_{a9}))_{r1}-(R_a(C(R_{a10})(R_{a11}))_{r2})_{s1}-(R_b(C(R_{a12})(R_{a13}))_{r3})_{s2}-*$;
$-(C(R_{a8)}(R_{a9}))_{r1}-(R_b-R_a-(C(R_{a10})(R_{a11}))_{r2})_{s1}-*$;
$-(C(R_{a8})(R_{a9}))_{r1}-(R_b(C(R_{a10})(R_{a11}))_{r2})_{s1}-(R_a(C(R_{a12})(R_{a13}))_{r3})_{s2}-*$;
$-(C(R_{a8})(R_{a9}))_{r1}-((C(R_{a10})(R_{a11}))_{r2}-R_a-R_b)_{s1}-*$;
$\sim(C(R_{a8})(R_{a9}))_{r1}-((C(R_{a10})(R_{a11}))_{r2}R_a)_{s1}-((C(R_{a12})(R_{a13})_{r3}R_b)_{s2}-*$;
$-(C(R_{a8})(R_{a9}))_{r1}-((C(R_{a10})(R_{a11}))_{r2}-R_b-R_a)_{s1}-*$; or
$-(C(R_{a8})(R_{a9}))_{r1}-((C(R_{a10})(R_{a11}))_{r2}R_b)_{s1}-((C(R_{a12})(R_{a13}))_{r3}R_a)_{s2}-*$;

wherein each $R_a$ is independently selected from the group consisting of O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, wherein each $R_{a7}$ independently represents H or $C_{1-6}$ alkyl; and each $R_b$ is independently selected from the group consisting of optionally substituted cycloalkylene (e.g., optionally substituted $C_{3-20}$ cycloalkylene), optionally substituted arylene (e.g., optionally substituted $C_{5-20}$ arylene), optionally substituted heterocyclylene (e.g., optionally substituted 4- to 20-membered heterocyclylene), optionally substituted heteroarylene (e.g., 5- to 20-membered heteroarylene), alkynylene (e.g., $C_{2-6}$ alkynylene), alkenylene (e.g., $C_{2-6}$ alkenylene), and any combination thereof; wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;

$R_{a8}$, $R_{a9}$, $R_{a10}$, $R_{a11}$, $R_{a12}$, $R_{a13}$, $R_{a14}$, and $R_{a15}$ each independently represent H, deuterium, hydroxy, amino, mercapto, nitro, halogen, cyano, oxo, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; and

r1, r2, r3, r4, s1, s2, and s3 each independently represent an integer of 1, 2, 3, 4, 5, 6, 7, 8, 9 or 10;

symbol * indicates the point of attachment to R; and

any one or more (e.g., 1-30, 1-25, 1-20, 1-15, 1-10, 1-9, 1-8, 1-7, 1-6, 1-5, 1-4, 1-3 or 1-2, or 1) hydrogens on the main carbon chain skeleton of said $C_{1-15}$ alkylene are optionally replaced by a substituent selected from: deuterium, hydroxy, amino, mercapto, nitro, halogen, cyano, oxo, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof. In the present text, unless otherwise explicitly indicated, the total number of carbon atoms in each general formula of $R_{w1}$ (excluding the carbon atoms of $R_a$ and $R_b$ groups)-namely, the sum of r1+ r2*s1, the sum of r1+ r2*s1+ r3*s2, and the sum of r1+ r2*s1+ r3*s2+ r4*s5-is less than or equal to 20.

In some embodiments of the present disclosure, $R_{w1}$ represents $-C_{1-15}$ alkylene-, wherein any one or more (e.g., 1-30, 1-25, 1-20, 1-15, 1-10, 1-9, 1-8, 1-7, 1-6, 1-5, 1-4, 1-3 or 1-2, or 1) hydrogens on the main carbon chain skeleton of said $C_{1-15}$ alkylene are optionally replaced by a substituent selected from: deuterium, hydroxy, amino, mercapto, nitro, halogen, cyano, oxo, halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-6}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments of the present disclosure, $R_{w1}$ represents the following groups: $-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$ or $-(CH_2)_{15}-$;

wherein any one or more hydrogens of the groups are optionally replaced by a substituent selected from: deuterium, hydroxy, amino, mercapto, nitro, halogen, cyano, oxo, halogenated $C_{1-6}$ alkyl (e.g., halogenated $C_{1-4}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), deuterated $C_{1-6}$ alkyl (e.g., perdeuterated $C_{1-6}$ alkyl, perdeuterated $C_{1-5}$ alkyl or perdeuterated $C_{1-4}$ alkyl, such as $CD_3$, $CD_3CD_2$-, $CD_3CD_2CD_2$- etc.), $C_{1-6}$ alkoxy (e.g., $C_{1-4}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments of the present disclosure, $R_{w1}$ represents:

$-(C(R_{a8})(R_{a9}))_{r1}-(O(C(R_{a10})(R_{a11}))_{r2})_{s1}-*$;
$-(C(R_{a8})(R_{a9}))_{r1}-(O(C(R_{a10})(R_{a11}))_{r2})_{s1}-(O(C(R_{a12})(R_{a13}))_{r3})_{s2}-*$;
$-(C(R_{a8})(R_{a9}))_{r1}-(O(C(R_{a10})(R_{a11}))_{r2})_{s1}-(O(C(R_{a12})(R_{a13}))_{r3})_{s2}-(O(C(R_{a14})(R_{a15}))_{r4})_{s3}-*$;
$-(C(R_{a8})(R_{a9}))_{r1}-((C(R_{a10})(R_{a11}))_{r2}O)_{s1}-*$;
$-(C(R_{a8})(R_{a9}))_{r1}-((C(R_{a10})(R_{a11}))_{r2}O)_{s1}-((C(R_{a12})(R_{a13}))_{r3}O)_{s2}-*$;
$-(C(R_{a8})(R_{a9}))_{r1}-((C(R_{a10})(R_{a11}))_{r2}O)_{s1}-((C(R_{a12})(R_{a13}))_{r3}O)_{s2}-((C(R_{a14})(R_{a15}))_{r4}O)_{s3}-*$;
$-(C(R_{a8})(R_{a9}))_{r1}-(N(R_{a7})(C(R_{a10})(R_{a11}))_{r2})_{s1}-*$;
$-(C(R_{a8})(R_{a9}))_{r1}-(N(R_{a7})(C(R_{a10})(R_{a11}))_{r2})_{s1}-(N(R_{a7})(C(R_{a12})(R_{a13}))_{r3})_{s2}-*$;
$-(C(R_{a8})(R_{a9}))_{r1}-(N(R_{a7})(C(R_{a10})(R_{a11}))_{r2})_{s1}-(N(R_{a7})(C(R_{a12})(R_{a13}))_{r3})_{s2}-(N(R_{a7})(C(R_{a14})(R_{a15}))_{r4})_{s3}-*$;
$-(C(R_{a8})(R_{a9}))_{r1}-((C(R_{a10})(R_{a11}))_{r2}N(R_{a7}))_{s1}-*$;
$-(C(R_{a8})(R_{a9}))_{r1}-((C(R_{a10})(R_{a11}))_{r2}N(R_{a7}))_{s1}-((C(R_{a12})(R_{a13}))_{r3}N(R_{a7}))_{s2}-*$;
$-(C(R_{a8})(R_{a9}))_{r1}-((C(R_{a10})(R_{a11}))_{r2}N(R_{a7}))_{s1}-((C(R_{a12})(R_{a13}))_{r3}N(R_{a7}))_{s2}-((C(R_{a14})(R_{a15}))_{r4}N(R_{a7}))_{s3}-*$;
$-(C(R_{a8})(R_{a9}))_{r1}-(C(O)N(R_{a7})-(C(R_{a10})(R_{a11}))_{r2})_{s1}-*$;
$-(C(R_{a8})(R_{a9}))_{r1}-(C(O)N(R_{a7})-(C(R_{a10})(R_{a11}))_{r2})_{s1}-(C(O)N(R_{a7})-(C(R_{a12})(R_{a13}))_{r3})_{s2}-*$;
$-(C(R_{a8})(R_{a9}))_{r1}-(C(O)N(R_{a7})-(C(R_{a10})(R_{a11}))_{r2})_{s1}-(C(O)N(R_{a7})-(C(R_{a12})(R_{a13}))_{r3})_{s2}-(C(O)N(R_{a7})-(C(R_{a14})(R_{a15}))_{r4})_{s3}-*$;
$-C(R_{a8})(R_{a9}))_{r1}-((C(R_{a10})(R_{a10}))_{r2}-C(O)N(R_{a7}))_{s1}-*$;
$-(C(R_{a8})(R_{a9}))_{r1}-((C(R_{a10})(R_{a11}))_{r2}-C(O)N(R_{a7}))_{s1}-((C(R_{a12})(R_{a13}))_{r3}-C(O)N(R_{a7}))_{s2}-*$;
$-(C(R_{a8})(R_{a9}))_{r1}-((C(R_{a10})(R_{a11}))_{r2}-C(O)N(R_{a7}))_{s1}-((C(R_{a12})(R_{a13}))_{r3}-C(O)N(R_{a7}))_{s2}-((C(R_{a14})(R_{a15}))_{r4}-C(O)N(R_{a7}))_{s3}-*$;
$-(C(R_{a8})(R_{a9}))_{r1}-(C(O)N(R_{a7})-(C(R_{a10})(R_{a11}))_{r2})_{s1}-(O-(C(R_{a12})(R_{a13}))_{r3})_{s2}-*$;
$-(C(R_{a8})(R_{a9}))_{r1}-C(O)N(R_{a7})-(C(R_{a10})(R_{a11}))_{r2}-(O(C(R_{a12})(R_{a13}))_{r3})_{s1}-*$;
$-(C(R_{a8})(R_{a9}))_{r1}-((C(R_{a10})(R_{a11}))_{r2}-C(O)N(R_{a7}))_{s1}-((C(R_{a12})(R_{a13}))_{r3}O)_{s2}-*$;
$-(C(R_{a8})(R_{a9}))_{r1}-(C(R_{a10})(R_{a11}))_{r2}-C(O)N(R_{a7})-((C(R_{a12})(R_{a13}))_{r3}O)_{s1}-*$;
$-(C(R_{a8})(R_{a9}))_{r1}-(N(R_{a7})C(O)-(C(R_{a10})(R_{a11}))_{r2})_{s1}-*$;
$-(C(R_{a8})(R_{a9}))_{r1}-(N(R_{a7})C(O)-(C(R_{a10})(R_{a11}))_{r2})_{s1}-(O-(C(R_{a12})(R_{a13})))_{r3})_{s2}-*$;
$-(C(R_{a8})(R_{a9}))_{r1}-(N(R_{a7})C(O)-(C(R_{a10})(R_{a11}))_{r2})_{s1}-(O-(C(R_{a12})(R_{a13}))_{r3})_{s2}-(O-(C(R_{a14})(R_{a15})_{r4})_{s3}-*$;
$-(C(R_{a8})(R_{a9}))_{r1}-N(R_{a7})C(O)-(C(R_{a10})(R_{a11}))_{r2}-(O(C(R_{a12})(R_{a13})_{r3})_{s1}-*$;
$-(C(R_{a8})(R_{a9}))_{r1}-N(R_{a7})C(O)N(R_{a7})-(C(R_{a10})(R_{a11}))_{r2}-*$;
$-(C(R_{a8})(R_{a9}))_{r1}-((C(R_{a10})(R_{a11}))_{r2}-N(R_{a7})C(O))_{s1}-*$;
$-(C(R_{a8})(R_{a9}))_{r1}-((C(R_{a10})(R_{a11}))_{r2}-N(R_{a7})C(O))_{s1}-((C(R_{a12})(R_{a13})))_{r3}-O)_{s2}-*$;
$-(C(R_{a8})(R_{a9}))_{r1}-((C(R_{a10})(R_{a11}))_{r2}-N(R_{a7})C(O))_{s1}-((C(R_{a12})(R_{a13}))_{r3}O)_{s2}-((C(R_{a14})(R_{a15}))_{r4}O)_{s3}-*$;
$-(C(R_{a8})(R_{a9}))_{r1}-(C(R_{a10})(R_{a11}))_{r2}-N(R_{a7})C(O)-((C(R_{a12})(R_{a13}))_{r3}O)_{s1}-*$;
$-(C(R_{a8})(R_{a9}))_{r1}$-(arylene-$(C(R_{a10})(R_{a11}))_{r2})_{s1}-*$;
$-(C(R_{a8})(R_{a9}))_{r1}$-(arylene-$(C(R_{a10})(R_{a11}))_{r2})_{s1}$-arylene-$(C(R_{a12})(R_{a13}))_{r3}-*$;
$-(C(R_{a8})(R_{a9}))_{r1}-((C(R_{a10})(R_{a11}))_{r2}$-arylene$)_{s1}-*$;
$-(C(R_{a8})(R_{a9}))_{r1}-((C(R_{a10})(R_{a11}))_{r2}$-arylene$)_{s1}-(C(R_{a12})(R_{a13}))_{r3}$-arylene-*;
$-(C(R_{a8})(R_{a9}))_{r1}$-(heterocyclylene-$(C(R_{a10})(R_{a11}))_{r2})_{s1}-*$;
$-(C(R_{a8})(R_{a9}))_{r1}$-(heterocyclylene-$(C(R_{a10})(R_{a11}))_{r2})_{s1}$-(heterocyclylene-$(C(R_{a12})(R_{a13}))_{r3})_{s2}-*$;
$-(C(R_{a8})(R_{a9}))_{r1}-((C(R_{a10})(R_{a11}))_{r2}$-heterocyclylene$)_{s1}-*$;
$-(C(R_{a8})(R_{a9}))_{r1}-((C(R_{a10})(R_{a11}))_{r2}$-heterocyclylene$)_{s1}-((C(R_{a12})(R_{a13}))_{r3}$-heterocyclylene$)_{s2}-*$;
$-(C(R_{a8})(R_{a9}))_{r1}$-(heteroarylene-$(C(R_{a10})(R_{a11}))_{r2})_{s1}-*$;
$-(C(R_{a8})(Ra_{9}))_{r1}$-(heteroarylene-$(C(R_{a10})(R_{a11}))_{r2})_{s1}$-(heteroarylene-$(C(R_{a12})(R_{a13}))_{r3})_{s2}-*$;
$-(C(R_{a8})(R_{a9}))_{r1}-((C(R_{a10})(R_{a11}))_{r2}$-heteroarylene$)_{s1}-*$;
$-(C(R_{a8})(R_{a9}))_{r1}-((C(R_{a10})(R_{a11}))_{r2}$-heteroarylene$)_{s1}-((C(R_{a12})(R_{a13}))_{r3}$-heteroarylene$)_{s2}-*$;
$-(C(R_{a8})(R_{a9}))_{r1}$-(cycloalkylene-$(C(R_{a10})(R_{a11}))_{r2})_{s1}-*$;
$-(C(R_{a8})(R_{a9}))_{r1}$-(cycloalkylene-$(C(R_{a10})(R_{a11}))_{r2})_{s1}$-(cycloalkylene-$(C(R_{a12})(R_{a13}))_{r3})_{s2}-*$;
$-(C(R_{a8})(R_{a9}))_{r1}-((C(R_{a10})(R_{a11}))_{r2}$-cycloalkylene$)_{s1}-*$; or

$-(C(R_{a8})(R_{a9}))_{r1}-((C(R_{a10})(R_{a11}))_{r2}$-cycloalkylene$)_{s1}-((C(R_{a12})(R_{a13}))_{r3}$-cycloalkylene$)_{s2}$-*;
wherein each $R_{a7}$ independently represents H or $C_{1-6}$ alkyl;
the cycloalkylene (e.g., $C_{3-20}$ cycloalkylene), arylene (e.g., $C_{5-20}$ arylene), heterocyclylene (e.g., 4- to 20-membered heterocyclylene), and heteroarylene (e.g., 5- to 20-membered heteroarylene) are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;
$R_{a8}$, $R_{a9}$, $R_{a10}$, $R_{a11}$, $R_{a12}$, $R_{a13}$, $R_{a14}$, and $R_{a15}$ each independently represent H, deuterium, hydroxy, amino, mercapto, nitro, halogen, cyano, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; and
r1, r2, r3, r4, s1, s2, and s3 each independently represent an integer of 1, 2, 3, 4, 5, 6, 7, 8, 9 or 10; and symbol * indicates the point of attachment to R.

In some embodiments, examples of the cycloalkylene in the general formula structure represented by $R_{w1}$ include, but are not limited to, $C_{3-20}$ cycloalkylene, $C_{3-15}$ cycloalkylene, and $C_{3-11}$ cycloalkylene, e.g., cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, spiro-cycloalkylene (e.g., $C_{5-15}$ spiro-cycloalkylene), adamantanylene, noradamantanylene, and norbornylene. The cycloalkylene is optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.
In some embodiments, examples of the arylene in the general formula structure represented by $R_{w1}$ include, but are not limited to, $C_{5-20}$ arylene, $C_{6-20}$ arylene, $C_{5-15}$ arylene, and $C_{6-15}$ arylene, e.g., phenylene or naphthylene. The arylene is optionally substituted with a substituent selected from the group consisting of deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.
In some embodiments, examples of the heterocyclylene in the general formula structure represented by $R_{w1}$ include, but are not limited to, 4- to 20-membered , 4- to 15-membered , 4- to 12-membered, 4- to 11-membered, 4- to 10-membered, 4- to 9-membered, 4- to 8-membered, 4- to 7-membered, 4- to 6-membered, 5- to 15-membered, and 5- to 9-membered heterocyclylene, e.g., azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo [3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and 5- to 20-membered azaspirocycloalkylene. The heterocyclylene is optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.
In some embodiments, examples of the heteroarylene in the general formula structure represented by $R_{w1}$ include, but are not limited to, 5- to 20-membered heteroarylene, 5- to 15-membered, 5- to 10-membered, 5- to 9-membered, 5- to 8-membered, 5- to 7-membered, and 5- to 6-membered heteroarylene, e.g., oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo [1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo [3,2-b]pyridylene, 1H-pyrrolo[2,3-b]pyridylene, pyrrolo[2,1-b]thiazolylene, or imidazo[2,1-b]thiazolylene. The heteroarylene is optionally substituted with a substituent selected from the group consisting of deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.
In some embodiments, examples of $R_{w1}$ include, but are not limited to, the following groups:
$-O-CH_2-$, $-O-(CH_2)_2-$, $-O-(CH_2)_3-$, $-O-(CH_2)_4-$, $-O-(CH_2)_5-$, $-O-(CH_2)_6-$, $-O-(CH_2)_7-$, $-O-(CH_2)_8-$, $-O-(CH_2)_9-$, $-O-(CH_2)_{10}-$,

$-(CH_2)_1-O-$, $-(CH_2)_2-O-$, $-(CH_2)_3-O-$, $-(CH_2)_4-O-$, $-(CH_2)_5-O-$, $-(CH_2)_6-O-$, $-(CH_2)_7-O-$, $-(CH_2)_8-O-$, $-(CH_2)_9-O-$, $-(CH_2)_{10}-O-$, $-CH_2-O-CH_2-$, $-CH_2-O-(CH_2)_2-$, $-(CH_2)_1-O-(CH_2)_3-$, $-(CH_2)_1-O-(CH_2)_4-$, $- (CH_2)_1-O-(CH_2)_5-$, $-(CH_2)_1-O-(CH_2)_6-$, $-(CH_2)_1-O-(CH_2)_7-$, $-(CH_2)_1-O-(CH_2)_8-$, $-(CH_2)_1-O-(CH_2)_9-$, $-(CH_2)_1-O-(CH_2)_{10}-$, $-(CH_2)_2-O-(CH_2)_1-$, $-(CH_2)_2-O-(CH_2)_2-$, $-(CH_2)_2-O-(CH_2)_3-$, $-(CH_2)_2-O-(CH_2)_4-$, $-(CH_2)_2-O-(CH_2)_5-$, $-(CH_2)_2-O-(CH_2)_6-$, $-(CH_2)_2-O-(CH_2)_7-$, $-(CH_2)_2-O-(CH_2)_8-$, $-(CH_2)_2-O-(CH_2)_9-$, $-(CH_2)_2-O-(CH_2)_{10}-$, $-(CH_2)_3-O-(CH_2)_1-$, $-(CH_2)_3-O-(CH_2)_2-$, $-(CH_2)_3-O-(CH_2)_3-$, $-(CH_2)_3-O-(CH_2)_4-$, $-(CH_2)_3-O-(CH_2)_5-$, $-(CH_2)_3-O-(CH_2)_6-$, $-(CH_2)_3-O-(CH_2)_7-$, $-(CH_2)_4-O-(CH_2)_{1-}$., $-(CH_2)_4-O-(CH_2)_2-$, $-(CH_2)_4-O-(CH_2)_3-$, $-(CH_2)_4-O-(CH_2)_4-$, $-(CH_2)_4-O-(CH_2)_5-$, $-(CH_2)_4-O-(CH_2)_6-$, $-(CH_2)_5-O-(CH_2)_1-$, $-(CH_2)_5-O-(CH_2)_2-$, $-(CH_2)_5-O-(CH_2)_3-$, $-(CH_2)_5-O-(CH_2)_4-$, $-(CH_2)_5-O-(CH_2)_5-$, $-(CH_2)_6-O-(CH_2)_1-$, $-(CH_2)_6-O-(CH_2)_2-$, $-(CH_2)_6-O-(CH_2)_3-$, $-(CH_2)_6-O-(CH_2)_4-$, $-(CH_2)_7-O-(CH_2)_1-$, $-(CH_2)_7-O-(CH_2)_2-$, $-(CH_2)_7-O-(CH_2)_3-$, $-(CH_2)_8-O-(CH_2)_1-$, $-(CH_2)_8-O-(CH_2)_2-$, $-CH(CH_3)-O-(CH_2)_1-$, $-CH(CH_3)-O-(CH_2)_2-$, $-CH(CH_3)-O-(CH_2)_3-$, $- CH(CH_3)-O-(CH_2)_4-$, $-CH(CH_3)-O-(CH_2)_5-$, $-CH(CH_3)-O-(CH_2)_6-$, $-CH(CH_3)-O-(CH_2)_7-$, $-CH(CH_3)-O-(CH_2)_8-$, $-CH(CH_3)-O-(CH_2)_9-$, $-CH(CH_3)-O-(CH_2)_{10}-$, $-(O(CH_2)_2)_2-$, $-(O(CH_2)_2)_3-$, $-(O(CH_2)_2)_4-$, $-(O(CH_2)_2)_5-$, $-(O(CH_2)_2)_6-$, $-(O(CH_2)_2)_7-$, $-CH_2-(O(CH_2)_2)_2-$, $-CH_2-(O(CH_2)_2)_3-$, $-CH_2-(O(CH_2)_2)_4-$, $-CH_2-(O(CH_2)_2)_5-$, $-CH_2-(O(CH_2)_2)_6-$, $-CH_2-(O(CH_2)_2)_7-$, $-CH_2-(O(CH_2)_2)_1-OCH_2-$, $-CH_2-(O(CH_2)_2)_2-OCH_2-$, $- CH_2-(O(CH_2)_2)_3-OCH_2-$, $-CH_2-(O(CH_2)_2)_4-OCH_2-$, $-CH_2-(O(CH_2)_2)_5-OCH_2-$, $-(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_3-(O(CH_2)_2)_2-$, $- (CH_2)_3-(O(CH_2)_2)_3-$, $-(CH_2)_3-(O(CH_2)_2)_4-$, $-(CH_2)_3-(O(CH_2)_2)_5-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-CH_2-(O(CH_2)_3)_2-$, $-CH_2-(O(CH_2)_3)_3-$, $-CH_2-(O(CH_2)_3)_4-$, $- (CH_2)_2-(O(CH_2)_3)_2-$, $-(CH_2)_2-(O(CH_2)_3)_3-$, $-(CH_2)_2-(O(CH_2)_3)_4-$, $-(CH_2)_3-(O(CH_2)_3)_2-$, $-(CH_2)_3-(O(CH_2)_3)_3-$, $-(CH_2)_3-(O(CH_2)_3)_4-$, $-CH_2-O-(CH_2)_2-O-(CH_2)_3-$, $-CH_2-(O(CH_2)_2)_2-(O(CH_2)_3)_2-$, $(CH_2)_2-O-(CH_2)_2-O-(CH_2)_3-$, $-(CH_2)_2-(O(CH_2)_2)_2-(O(CH_2)_3)_2-$, $-(CH_2)_3-O-(CH_2)_2-O-(CH_2)_3-$, $-(CH_2)_3-(O(CH_2)_2)_2-(O(CH_2)_3)_2-$, $- CH_2-O-(CH_2)_3-O-(CH_2)_2-$, $-CH_2-(O(CH_2)_3)_2-(O(CH_2)_2)_2-$, $-(CH_2)_2-O-(CH_2)_3-O-(CH_2)_2-$, $-(CH_2)_2-(O(CH_2)_3)_2-(O(CH_2)_2)_2-$, $-(CH_2)_3-O-(CH_2)_3-O-(CH_2)_2-$, $-(CH_2)_3-(O(CH_2)_3)_2-(O(CH_2)_2)_2-$, $-CH_2-O-(CH_2)_2-O-CH_2-$, $-(CH_2)_2-O-(CH_2)_2-O-CH_2-$, $-(CH_2)_2-(O(CH_2)_2)_2-O-(CH_2)_3-$, $-(CH_2)_2-(O(CH_2)_2)_3-O-(CH_2)_3-$, $- (CH_2)_2-(O(CH_2)_2)_4-O-(CH_2)_3-$, $-(CH_2)_5-(O(CH_2)_2)_2-O-(CH_2)_5-$, $-(CH_2)_5-(O(CH_2)_2)_2-O-(CH_2)_6-$, $-(CH_2)_1-N(R_{a7})-(CH_2)_1-$, $-(CH_2)_1-N(R_{a7})-(CH_2)_2-$, $-(CH_2)_1-N(R_{a7})-(CH_2)_3-$, $-(CH_2)_1-N(R_{a7})-(CH_2)_4-$, $-(CH_2)_1-N(R_{a7})-$(CH2)s-, $-(CH_2)_1-N(R_{a7})-(CH_2)_6-$, $-(CH_2)_1-N(R_{a7})-(CH_2)_7-$, $-(CH_2)_1-N(R_{a7})-(CH_2)_8-$, $-(CH_2)_1-N(R_{a7})-(CH_2)_9-$, $-(CH_2)_1-N(R_{a7})-(CH_2)_{10}-$, $-N(R_{a7})-(CH_2)_1-$, $-N(R_{a7})-(CH_2)_2-$, $-N(R_{a7})-(CH_2)_3-$, $-N(R_{a7})-(CH_2)_4-$, $-N(R_{a7})-$(CH2)5-, $-N(R_{a7})-(CH_2)_6-$, $-N(R_{a7})-(CH_2)_7-$, $-N(R_{a7})-(CH_2)_8-$, $-N(R_{a7})-(CH_2)_9-$, $-N(R_{a7})-(CH_2)_{10}-$, $-(CH_2)_2-N(R_{a7})-(CH_2)_1-$, $-(CH_2)_2-N(R_{a7})-(CH_2)_2-$, $-(CH_2)_2-N(R_{a7})-(CH_2)_3-$, $-(CH_2)_2-N(R_{a7})-(CH_2)_4-$, $-(CH_2)_2-N(R_{a7})-$(CH2)s-, $-(CH_2)_2-N(R_{a7})-(CH_2)_6-$, $-(CH_2)_2-N(R_{a7})-(CH_2)_7-$, $-(CH_2)_2-N(R_{a7})-(CH_2)_8-$, $-(CH_2)_2-N(R_{a7})-(CH_2)_9-$, $-(CH_2)_2-N(R_{a7})-(CH_2)_{10}-$, $-(CH_2)_3-N(R_{a7})-(CH_2)_1-$, $-(CH_2)_3-N(R_{a7})-(CH_2)_2-$, $-(CH_2)_3-N(R_{a7})-(CH_2)_3-$, $- (CH_2)_4-N(R_{a7})-(CH_2)_1-$, $-(CH_2)_4-N(R_{a7})-(CH_2)_2-$, $-(CH_2)_4-N(R_{a7})-(CH_2)_3-$, $-(CH_2)_4-N(R_{a7})-(CH_2)_4-$, $-(CH_2)_5-N(R_{a7})-(CH_2)_1-$, $-(CH_2)_5-N(R_{a7})-(CH_2)_2-$, $-(CH_2)_5-N(R_{a7})-(CH_2)_3-$, $-(CH_2)_5-N(R_{a7})-(CH_2)_4-$, $-(CH_2)_5-N(R_{a7})-$(CH2)s-, $-(CH_2)_6-N(R_{a7})-(CH_2)_1-$, $-(CH_2)_6-N(R_{a7})-(CH_2)_2-$, $-(CH_2)_6-N(R_{a7})-(CH_2)_3-$, $-(CH_2)_7-N(R_{a7})-(CH_2)_1-$, $-(CH_2)_7-N(R_{a7})-(CH_2)_2-$, $-(CH_2)_7-N(R_{a7})-(CH_2)_3-$, -(CH2)s-N(Ra7)-(CH2)1-, $-(CH_2)_8-N(R_{a7})-(CH_2)_2-$, $- (CH_2)_8-N(R_{a7})-(CH_2)_3-$, $-CH(CH_3)-N(R_{a7})-(CH_2)_1-$, $-CH(CH_3)-N(R_{a7})-(CH_2)_2-$, $-CH(CH_3)-N(R_{a7})-(CH_2)_3-$, $- CH(CH_3)-N(R_{a7})-(CH_2)_4-$, $-CH(CH_3)-N(R_{a7})-(CH_2)_5-$, $-CH(CH_3)-N(R_{a7})-(CH_2)_6-$, $-CH(CH_3)-N(R_{a7})-$(CH2)7-, $-CH(CH_3)-N(R_{a7})-(CH_2)_8-$, $-CH(CH_3)-N(R_{a7})-(CH_2)_9-$, $-CH(CH_3)-N(R_{a7})-(CH_2)_{10}-$, $-(CH_2)_1-N(R_{a7})-$, $-(CH_2)_2-N(R_{a7})-$, $-(CH_2)_3-N(R_{a7})-$, $-(CH_2)_4-N(R_{a7})-$, $-(CH_2)_5-N(R_{a7})-$, $-(CH_2)_6-N(R_{a7})-$, $-(CH_2)_7-N(R_{a7})-$, $-(CH_2)_8-N(R_{a7})-$, $-(CH_2)_9-N(R_{a7})-$, $-(CH_2)_{10}-N(R_{a7})-$, $-C(O)NHCH_2-$, $-C(O)NH(CH_2)_2-$, $- C(O)NH(CH_2)_3-$, $-C(O)NH(CH_2)_4-$, $-C(O)NH(CH_2)_5-$, $-CH_2C(O)NHCH_2-$, $-(CH_2)_2C(O)NH(CH_2)_2-$, $- (CH_2)_2C(O) NH(CH_2)_3-$, $-(CH_2)_2C(O)NH(CH_2)_4-$, $-(CH_2)_2C(O)NH(CH_2)_5-$, $-(CH_2)_3C(O)NH(CH_2)_3-$, $- (CH_2)_3C(O)NH(CH_2)_4-$, $-(CH_2)_4C(O)NH(CH_2)_4-$, $-(CH_2)_5C(O)NH(CH_2)_5-$, $-(CH_2)_6C(O)NH(CH_2)_7-$, $- (CH_2)_6C(O)NH(CH_2)_6-$, $-(CH_2)_7C(O) NH(CH_2)_7-$, $-(CH_2)_2C(O)NH(CH_2)_2-O-(CH_2)_2-$, $-NHC(O)CH_2-$, $- NHC(O)(CH_2)_2-$, $-NHC(O)(CH_2)_3-$, $-NHC(O)(CH_2)_4-$, $-NHC(O)(CH_2)_5-$, $-CH_2NHC(O)CH_2-$, $- (CH_2)_2NHC(O)(CH_2)_2-$, $-(CH_2)_2NHC(O)(CH_2)_3-$, $-(CH_2)_2NHC(O)(CH_2)_4-$, $-(CH_2)_2NHC(O)(CH_2)_5-$, $- (CH_2)_3NHC(O)(CH_2)_3-$, $-(CH_2)_3NHC(O)(CH_2)_4-$, $-(CH_2)_4NHC(O)(CH_2)_4-$, $-(CH_2)_5NHC(O) (CH_2)_5-$, $- (CH_2)_6NHC(O)(CH_2)_7-$, $-(CH_2)_6NHC(O)(CH_2)_6-$, $-(CH_2)_7NHC(O)(CH_2)_7-$, $-(CH_2)_4NHC(O)(CH_2)_8-$, $- (CH_2)_2NHC(O)(CH_2)_2-0-(CH_2)_2-$, $-(CH_2)_4NHC(O)CH_2-$, -phenylene-$CH_2-$, -phenylene-$(CH_2)_2-$, - phenylene-$(CH_2)_3-$, -phenylene-$(CH_2)_4-$, -phenylene-$(CH_2)_5-$, -phenylene-$(CH_2)_6-$, -phenylene-$(CH_2)_7-$, - phenylene-$(CH_2)_8-$, $-CH_2-$phenylene-$CH_2-$, $-CH_2-$phenylene-$(CH_2)_2-$, $-CH_2-$phenylene-$(CH_2)_3-$, $-CH_2-$phenylene-$(CH_2)_4-$, $-CH_2-$phenylene-$(CH_2)_5-$, $-CH_2-$phenylene-$(CH_2)_6-$, $-CH_2-$phenylene-$(CH_2)_7-$, $-CH_2-$phenylene-$(CH_2)_8-$, $-(CH_2)_2-$phenylene-$(CH_2)_1-$, $-(CH_2)_2-$phenylene-$(CH_2)_2-$, $-(CH_2)_2-$phenylene-$(CH_2)_3-$, $- (CH_2)_2-$phenylene-$(CH_2)_4-$, $-(CH_2)_2-$phenylene-$(CH_2)_5-$, $-(CH_2)_2-$phenylene-$(CH_2)_6-$, $-(CH_2)_2-$phenylene-$(CH_2)_7-$, $-(CH_2)_2-$phenylene-$(CH_2)_8-$, $-(CH_2)_3-$phenylene-$CH_2-$, $-(CH_2)_3-$phenylene-$(CH_2)_2-$, $-(CH_2)_3-$phenylene-$(CH_2)_3-$, $-(CH_2)_3-$phenylene-$(CH_2)_4-$, $-(CH_2)_3-$phenylene-$(CH_2)_5-$, $-(CH_2)_3-$phenylene-$(CH_2)_6-$, $- (CH_2)_3-$phenylene-$(CH_2)_7-$, $-(CH_2)_3-$phenylene-$(CH_2)_8-$, $-(CH_2)_4-$phenylene-$CH_2-$, $-(CH_2)_4-$phenylene-$(CH_2)_2-$, $-(CH_2)_4-$phenylene-$(CH_2)_3-$, $-(CH_2)_4-$phenylene-$(CH_2)_4-$, $-(CH_2)_4-$phenylene-$(CH_2)_5-$, $-(CH_2)_4-$phenylene-$(CH_2)_6-$, $-(CH_2)_4-$phenylene-$(CH_2)_7-$, $-(CH_2)_4-$phenylene-$(CH_2)_8-$, $-(CH_2)_5-$phenylene-$(CH_2)_1-$, $- (CH_2)_5-$phenylene-$(CH_2)_2-$, $-(CH_2)_5-$phenylene-$(CH_2)_3-$, $-(CH_2)_5-$phenylene-$(CH_2)_4-$, $-(CH_2)_5-$phenylene-$(CH_2)_5-$, $-(CH_2)_5-$phenyle-

ne-$(CH_2)_6$-, -$(CH_2)_5$-phenylene-$(CH_2)_7$-, -$(CH_2)_5$-phenylene-$(CH_2)_8$-, -$(CH_2)_6$-phenylene-$(CH_2)_1$-, -$(CH_2)_6$-phenylene-$(CH_2)_2$-, -$(CH_2)_6$-phenylene-$(CH_2)_3$-, -$(CH_2)_6$-phenylene-$(CH_2)_4$-, - $(CH_2)_6$-phenylene-$(CH_2)_5$-, -$(CH_2)_6$-phenylene-$(CH_2)_6$-, -$(CH_2)_6$-phenylene-$(CH_2)_7$-, -$(CH_2)_6$-phenylene-$(CH_2)_8$-, -$(CH_2)_7$-phenylene-$(CH_2)_1$-, -$(CH_2)_7$-phenylene-$(CH_2)_2$-, -$(CH_2)_7$-phenylene-$(CH_2)_3$-, -$(CH_2)_7$-phenylene-$(CH_2)_4$-, -$(CH_2)_7$-phenylene-$(CH_2)_8$-, -$(CH_2)_8$-phenylene-$CH_2$-, -$(CH_2)_8$-phenylene-$(CH_2)_2$-, - $(CH_2)_8$-phenylene-$(CH_2)_3$-, -$(CH_2)_8$-phenylene-$(CH_2)_4$-, -$(CH_2)_8$-phenylene-$(CH_2)_5$-, -$(CH_2)_8$-phenylene-$(CH_2)_6$-, -$(CH_2)_8$-phenylene-$(CH_2)_7$-, -$N(R_{a7})$-$CH_2$-phenylene-$CH_2$-, -$N(R_{a7})$-$CH_2$-phenylene-$(CH_2)_2$-, - $N(R_{a7})$-$CH_2$-phenylene-$(CH_2)_3$-, -$N(R_{a7})$-$CH_2$-phenylene-$(CH_2)_4$-, -$N(R_{a7})$-$CH_2$-phenylene-$(CH_2)_5$-, - $N(R_{a7})$-$CH_2$-phenylene-$(CH_2)_6$-, -$N(R_{a7})$-$CH_2$-phenylene-$(CH_2)_7$-, -$N(R_{a7})$-$CH_2$-phenylene-$(CH_2)_8$-, -$CH_2$-$N(R_{a7})$-$CH_2$-phenylene-$CH_2$-, -$CH_2$-$N(R_{a7})$-$CH_2$-phenylene-$(CH_2)_2$-, -$CH_2$-$N(R_{a7})$-$CH_2$-phenylene-$(CH_2)_3$-, -$CH_2$-$N(R_{a7})$-$CH_2$-phenylene-$(CH_2)_4$-, -$CH_2$-$N(R_{a7})$-$CH_2$-phenylene-$(CH_2)_5$-, -$CH_2$-$N(R_{a7})$-$CH_2$-phenylene-$(CH_2)_6$-, -$CH_2$-$N(R_{a7})$-$CH_2$-phenylene-$(CH_2)_7$-, -$CH_2$-$N(R_{a7})$-$CH_2$-phenylene-$(CH_2)_9$-, -$CH_2$-$N(R_{a7})$-$(CH_2)_2$-phenylene-$CH_2$-, -$CH_2$-$N(R_{a7})$-$(CH_2)_2$-phenylene-$(CH_2)_2$-, -$CH_2$-$N(R_{a7})$-$(CH_2)_2$-phenylene-$(CH_2)_3$-, -$CH_2$-$N(R_{a7})$-$(CH_2)_2$-phenylene-$(CH_2)_4$-, -$CH_2$-$N(R_{a7})$-$(CH_2)_2$-phenylene-$(CH_2)_5$-, -$CH_2$-$N(R_{a7})$-$(CH_2)_2$-phenylene-$(CH_2)_6$-, -$CH_2$-$N(R_{a7})$-$(CH_2)_2$-phenylene-$(CH_2)_7$-, -$CH_2$-$N(R_{a7})$-$(CH_2)_2$-phenylene-$(CH_2)_8$-, - $(CH_2)_2$-$N(R_{a7})$-$CH_2$-phenylene-$CH_2$-, -$(CH_2)_2$-$N(R_{a7})$-$CH_2$-phenylene-$(CH_2)_2$-, -$(CH_2)_2$-$N(R_{a7})$-$CH_2$-phenylene-$(CH_2)_3$-, -$(CH_2)_2$-$N(R_{a7})$-$CH_2$-phenylene-$(CH_2)_4$-, -$(CH_2)_2$-$N(R_{a7})$-$CH_2$-phenylene-$(CH_2)_5$-, - $(CH_2)_2$-$N(R_{a7})$-$CH_2$-phenylene-$(CH_2)_6$-, -$(CH_2)_2$-$N(R_{a7})$-$CH_2$-phenylene-$(CH_2)_7$-, -$(CH_2)_2$-$N(R_{a7})$-$CH_2$-phenylene-$(CH_2)_8$-, -$(CH_2)_3$-$N(R_{a7})$-$CH_2$-phenylene-$CH_2$-, -$(CH_2)_3$-$N(R_{a7})$-$CH_2$-phenylene-$(CH_2)_2$-, - $(CH_2)_3$-$N(R_{a7})$-$CH_2$-phenylene-$(CH_2)_3$-, -$(CH_2)_3$-$N(R_{a7})$-$CH_2$-phenylene-$(CH_2)_8$-, -$(CH_2)_4$-$N(R_{a7})$-$CH_2$-phenylene-$CH_2$-, -$(CH_2)_4$-$N(R_{a7})$-$CH_2$-phenylene-$(CH_2)_2$-, -$(CH_2)_4$-$N(R_{a7})$-$CH_2$-phenylene-$(CH_2)_3$-, - $(CH_2)_4$-$N(R_{a7})$-$CH_2$-phenylene-$(CH_2)_8$-, -$(CH_2)_5$-$N(R_{a7})$-$CH_2$-phenylene-$(CH_2)_3$-, -$(CH_2)_5$-$N(R_{a7})$-$CH_2$-phenylene-$(CH_2)_8$-, -$(CH_2)_6$-$N(R_{a7})$-$CH_2$-phenylene-$(CH_2)_3$-, -$(CH_2)_6$- $N(R_{a7})$-$CH_2$-phenylene-$(CH_2)_8$-, - $(CH_2)_7$-$N(R_{a7})$-$CH_2$-phenylene-$(CH_2)_3$-, -$(CH_2)_8$-$N(R_{a7})$-$CH_2$-phenylene-$CH_2$-, -$(CH_2)_8$-$N(R_{a7})$-$CH_2$-phenylene-$(CH_2)_2$-, -$(CH_2)_8$-$N(R_{a7})$-$CH_2$-phenylene-$(CH_2)_3$-, -$(CH_2)_8$-$N(R_{a7})$-$CH_2$-phenylene-$(CH_2)_4$-, - $(CH_2)_8$-$N(R_{a7})$-$CH_2$-phenylene-$(CH_2)_5$-, -$(CH_2)_8$-$N(R_{a7})$-$CH_2$-phenylene-$(CH_2)_6$-, -piperidinylene-$CH_2$-, - piperidinylene-$(CH_2)_2$-, -piperidinylene-$(CH_2)_3$-, -piperidinylene-$(CH_2)_4$-, -piperidinylene-$(CH_2)_5$-, - piperidinylene-$(CH_2)_6$-, -piperidinylene-$(CH_2)_7$-, -piperidinylene-$(CH_2)_8$-, -$CH_2$-piperidinylene-$CH_2$-, - $CH_2$-piperidinylene-$(CH_2)_2$-, -$CH_2$-piperidinylene-$(CH_2)_3$-, -$CH_2$-piperidinylene-$(CH_2)_4$-, -$CH_2$-piperidinylene-$(CH_2)_5$-,-$CH_2$-piperidinylene-$(CH_2)_6$-,-$CH_2$-piperidinylene-$(CH_2)_7$-,-$CH_2$-piperidinylene-$(CH_2)_8$-, -$(CH_2)_2$-piperidinylene-$(CH_2)_1$-, -$(CH_2)_2$-piperidinylene-$(CH_2)_2$-, -$(CH_2)_2$-piperidinylene-$(CH_2)_3$-, -$(CH_2)_2$-piperidinylene-$(CH_2)_4$-, -$(CH_2)_2$-piperidinylene-$(CH_2)_5$-, -$(CH_2)_2$-piperidinylene-$(CH_2)_6$-, -$(CH_2)_2$-piperidinylene-$(CH_2)_7$-, -$(CH_2)_2$-piperidinylene-$(CH_2)_8$-, -$(CH_2)_3$-piperidinylene-$CH_2$-, -$(CH_2)_3$-piperidinylene-$(CH_2)_2$-, -$(CH_2)_3$-piperidinylene-$(CH_2)_3$-, -$(CH_2)_3$-piperidinylene-$(CH_2)_4$-, -$(CH_2)_3$-piperidinylene-$(CH_2)_5$-, -$(CH_2)_3$-piperidinylene-$(CH_2)_6$-, -$(CH_2)_3$-piperidinylene-$(CH_2)_7$-, -$(CH_2)_3$-piperidinylene-$(CH_2)_8$-, -$(CH_2)_4$-piperidinylene-$CH_2$-, -$(CH_2)_4$-piperidinylene-$(CH_2)_2$-, -$(CH_2)_4$-piperidinylene-$(CH_2)_3$-, -$(CH_2)_4$-piperidinylene-$(CH_2)_4$-, -$(CH_2)_4$-piperidinylene-$(CH_2)_5$-, -$(CH_2)_4$-piperidinylene-$(CH_2)_6$-, -$(CH_2)_4$-piperidinylene-$(CH_2)_7$-, -$(CH_2)_4$-piperidinylene-$(CH_2)_8$-, -$(CH_2)_5$-piperidinylene-$(CH_2)_1$-, -$(CH_2)_5$-piperidinylene-$(CH_2)_2$-, -$(CH_2)_5$-piperidinylene-$(CH_2)_3$-, -$(CH_2)_5$-piperidinylene-$(CH_2)_4$-, -$(CH_2)_5$-piperidinylene-$(CH_2)_5$-, -$(CH_2)_5$-piperidinylene-$(CH_2)_6$-, -$(CH_2)_5$-piperidinylene-$(CH_2)_7$-, -$(CH_2)_5$-piperidinylene-$(CH_2)_8$-, -$(CH_2)_6$-piperidinylene-$(CH_2)_1$-, -$(CH_2)_6$-piperidinylene-$(CH_2)_2$-, -$(CH_2)_6$-piperidinylene-$(CH_2)_3$-, -$(CH_2)_6$-piperidinylene-$(CH_2)_4$-, -$(CH_2)_6$-piperidinylene-$(CH_2)_5$-, -$(CH_2)_6$-piperidinylene-$(CH_2)_6$-, -$(CH_2)_6$-piperidinylene-$(CH_2)_7$-, -$(CH_2)_6$-piperidinylene-$(CH_2)_8$-, -$(CH_2)_7$-piperidinylene-$(CH_2)_1$-, -$(CH_2)_7$-piperidinylene-$(CH_2)_2$-, -$(CH_2)_7$-piperidinylene-$(CH_2)_3$-, -$(CH_2)_7$-piperidinylene-$(CH_2)_4$-, -$(CH_2)_7$-piperidinylene-$(CH_2)_8$-, -$(CH_2)_9$-piperidinylene-$CH_2$-, -$(CH_2)_8$-piperidinylene-$(CH_2)_2$-, -$(CH_2)_8$-piperidinylene-$(CH_2)_3$-, -$(CH_2)_8$-piperidinylene-$(CH_2)_4$-, -$(CH_2)_8$-piperidinylene-$(CH_2)_5$-, -$(CH_2)_8$-piperidinylene-$(CH_2)_6$-, -$(CH_2)_9$-piperidinylene-$(CH_2)_7$-, -$N(R_{a7})$-$CH_2$-piperidinylene-$CH_2$-, -$N(R_{a7})$-$CH_2$-piperidinylene-$(CH_2)_2$-, -$N(R_{a7})$-$CH_2$-piperidinylene-$(CH_2)_3$-, -$N(R_{a7})$-$CH_2$-piperidinylene-$(CH_2)_4$-, -$N(R_{a7})$-$CH_2$-piperidinylene-$(CH_2)_5$-, - $N(R_{a7})$-$CH_2$-piperidinylene-$(CH_2)_6$-, -$N(R_{a7})$-$CH_2$-piperidinylene-$(CH_2)_7$-, -$N(R_{a7})$-$CH_2$-piperidinylene-$(CH_2)_8$-, -$CH_2$-$N(R_{a7})$-$CH_2$-piperidinylene-$CH_2$-, -$CH_2$-$N(R_{a7})$-$CH_2$-piperidinylene-$(CH_2)_2$-, -$CH_2$-$N(R_{a7})$-$CH_2$-piperidinylene-$(CH_2)_3$-, -$CH_2$-$N(R_{a7})$-$CH_2$-piperidinylene-$(CH_2)_4$-, -$CH_2$-$N(R_{a7})$-$CH_2$-piperidinylene-$(CH_2)_5$-, -$CH_2$-$N(R_{a7})$-$CH_2$-piperidinylene-$(CH_2)_6$-, -$CH_2$-$N(R_{a7})$-$CH_2$-piperidinylene-$(CH_2)_7$-, -$CH_2$-$N(R_{a7})$-$CH_2$-piperidinylene-$(CH_2)_8$-, -$CH_2$-$N(R_{a7})$-$(CH_2)_2$-piperidinylene-$CH_2$-, -$CH_2$-$N(R_{a7})$-$(CH_2)_2$-piperidinylene-$(CH_2)_2$-, -$CH_2$-$N(R_{a7})$-$(CH_2)_2$-piperidinylene-$(CH_2)_3$-, -$CH_2$-$N(R_{a7})$-$(CH_2)_2$-piperidinylene-$(CH_2)_4$-, -$CH_2$-$N(R_{a7})$-$(CH_2)_2$-piperidinylene-$(CH_2)_5$-, -$CH_2$-$N(R_{a7})$-$(CH_2)_2$-piperidinylene-$(CH_2)_6$-, -$CH_2$-$N(R_{a7})$-$(CH_2)_2$-piperidinylene-$(CH_2)_7$-, -$CH_2$-$N(R_{a7})$-$(CH_2)_2$-piperidinylene-$(CH_2)_8$-, -$(CH_2)_2$-$N(R_{a7})$-$CH_2$-piperidinylene-$CH_2$-, -$(CH_2)_2$-$N(R_{a7})$-$CH_2$-piperidinylene-$(CH_2)_2$-, -$(CH_2)_2$-$N(R_{a7})$-$CH_2$-piperidinylene-$(CH_2)_3$-, -$(CH_2)_2$-$N(R_{a7})$-$CH_2$-piperidinylene-$(CH_2)_4$-, -$(CH_2)_2$-$N(R_{a7})$-$CH_2$-piperidinylene-$(CH_2)_8$-, - $(CH_2)_2$-$N(R_{a7})$-$CH_2$-piperidinylene-$(CH_2)_6$-, -$(CH_2)_2$-$N(R_{a7})$-$CH_2$-piperidinylene-$(CH_2)_7$-, -$(CH_2)_2$-$N(R_{a7})$-$CH_2$-piperidinylene-$(CH_2)_8$-, -$(CH_2)_3$-$N(R_{a7})$-$CH_2$-piperidinylene-$CH_2$-, -$(CH_2)_3$-$N(Ra_7)$-$CH_2$-piperidinylene-$(CH_2)_2$-, -$(CH_2)_3$-$N(R_{a7})$-$CH_2$-piperidinylene-$(CH_2)_3$-, -$(CH_2)_3$-$N(R_{a7})$-$CH_2$-piperidinylene-$(CH_2)_8$-, -$(CH_2)_4$-$N(R_{a7})$-$CH_2$-piperidinylene-$CH_2$-, -$(CH_2)_4$-$N(R_{a7})$-$CH_2$-piperidinylene-$(CH_2)_2$-, -$(CH_2)_4$-$N(R_{a7})$-$CH_2$-piperidinylene-$(CH_2)_3$-, -$(CH_2)_4$-$N(R_{a7})$-$CH_2$-piperidinylene-$(CH_2)_8$-, -$(CH_2)_5$-$N(R_{a7})$-$CH_2$-piperidinylene-$(CH_2)_3$-, -$(CH_2)_5$-$N(R_{a7})$-$CH_2$-piperidinylene-$(CH_2)_8$-, -$(CH_2)_6$-$N(R_{a7})$-$CH_2$-piperidinylene-$(CH_2)_3$-, -$(CH_2)_6$-$N(R_{a7})$-$CH_2$-piperidinylene-$(CH_2)_8$-,

$-(CH_2)_7-N(R_{a7})-CH_2$-piperidinylene-$(CH_2)_3$-, - $(CH_2)_8-N(R_{a7})-CH_2$-piperidinylene-$CH_2$-, $-(CH_2)_8-N(R_{a7})-CH_2$-piperidinylene-$(CH_2)_2$-, $-(CH_2)_8-N(R_{a7})-CH_2$-piperidinylene-$(CH_2)_3$-, $-(CH_2)_8-N(R_{a7})-CH_2$-piperidinylene-$(CH_2)_4$-, $-(CH_2)_8-N(R_{a7})-CH_2$-piperidinylene-$(CH_2)_5$-, $-(CH_2)_8-N(R_{a7})-CH_2$-piperidinylene-$(CH_2)_6$-, -piperazinylene-$CH_2$-, - piperazinylene-$(CH_2)_2$-, -piperazinylene-$(CH_2)_3$-, -piperazinylene-$(CH_2)_4$-, -piperazinylene-$(CH_2)_5$-, - piperazinylene-$(CH_2)_6$-, -piperazinylene-$(CH_2)_7$-, -piperazinylene-$(CH_2)_8$-, $-CH_2$-piperazinylene-$CH_2$-, - $CH_2$-piperazinylene-$(CH_2)_2$-, $-CH_2$-piperazinylene-$(CH_2)_3$-, $-CH_2$-piperazinylene-$(CH_2)_4$-, $-CH_2$-piperazinylene-$(CH_2)_5$-, $-CH_2$-piperazinylene-$(CH_2)_6$-, $-CH_2$-piperazinylene-$(CH_2)_7$-, $-CH_2$-piperazinylene-$(CH_2)_8$-, $-(CH_2)_2$-piperazinylene-$(CH_2)_1$-, $-(CH_2)_2$-piperazinylene-$(CH_2)_2$-, $-(CH_2)_2$-piperazinylene-$(CH_2)_3$-, $-(CH_2)_2$-piperazinylene-$(CH_2)_4$-, $-(CH_2)_2$-piperazinylene-$(CH_2)_5$-, $-(CH_2)_2$-piperazinylene-$(CH_2)_6$-, $-(CH_2)_2$-piperazinylene-$(CH_2)_7$-, $-(CH_2)_2$-piperazinylene-$(CH_2)_8$-, $-(CH_2)_3$-piperazinylene-$CH_2$-, $-(CH_2)_3$-piperazinylene-$(CH_2)_2$-, $-(CH_2)_3$-piperazinylene-$(CH_2)_3$-, $-(CH_2)_3$-piperazinylene-$(CH_2)_4$-, $-(CH_2)_3$-piperazinylene-$(CH_2)_5$-, $-(CH_2)_3$-piperazinylene-$(CH_2)_6$-, $-(CH_2)_3$-piperazinylene-$(CH_2)_7$-, $-(CH_2)_3$-piperazinylene-$(CH_2)_8$-, $-(CH_2)_4$-piperazinylene-$CH_2$-, $-(CH_2)_4$-piperazinylene-$(CH_2)_2$-, $-(CH_2)_4$-piperazinylene-$(CH_2)_3$-, $-(CH_2)_4$-piperazinylene-$(CH_2)_4$-, $-(CH_2)_4$-piperazinylene-$(CH_2)_5$-, $-(CH_2)_4$-piperazinylene-$(CH_2)_6$-, $-(CH_2)_4$-piperazinylene-$(CH_2)_7$-, $-(CH_2)_4$-piperazinylene-$(CH_2)_9$-, $-(CH_2)_5$-piperazinylene-$(CH_2)_1$-, $-(CH_2)_5$-piperazinylene-$(CH_2)_2$-, $-(CH_2)_5$-piperazinylene-$(CH_2)_3$-, $-(CH_2)_5$-piperazinylene-$(CH_2)_4$-, $-(CH_2)_5$-piperazinylene-$(CH_2)_5$-, $-(CH_2)_5$-piperazinylene-$(CH_2)_6$-, $-(CH_2)_5$-piperazinylene-$(CH_2)_7$-, $-(CH_2)_5$-piperazinylene-$(CH_2)_8$-, $-(CH_2)_6$-piperazinylene-$(CH_2)_1$-, $-(CH_2)_6$-piperazinylene-$(CH_2)_2$-, $-(CH_2)_6$-piperazinylene-$(CH_2)_3$-, $-(CH_2)_6$-piperazinylene-$(CH_2)_4$-, $-(CH_2)_6$-piperazinylene-$(CH_2)_5$-, $-(CH_2)_6$-piperazinylene-$(CH_2)_6$-, $-(CH_2)_6$-piperazinylene-$(CH_2)_7$-, $-(CH_2)_6$-piperazinylene-$(CH_2)_8$-, $-(CH_2)_7$-piperazinylene-$(CH_2)_1$-, $-(CH_2)_7$-piperazinylene-$(CH_2)_2$-, $-(CH_2)_7$-piperazinylene-$(CH_2)_3$-, $-(CH_2)_7$-piperazinylene-$(CH_2)_4$-, $-(CH_2)_8$-piperazinylene-$CH_2$-, $-(CH_2)_8$-piperazinylene-$(CH_2)_2$-, $-(CH_2)_9$-piperazinylene-$(CH_2)_3$-, $-(CH_2)_8$-piperazinylene-$(CH_2)_4$-, $-(CH_2)_9$-piperazinylene-$(CH_2)_5$-, or $-(CH_2)_8$-piperazinylene-$(CH_2)_6$-;

wherein each $R_{a7}$ independently represents H or $C_{1-6}$ alkyl (e.g., methyl, ethyl, propyl, or isopropyl);
each phenylene is independently optionally substituted with a substituent selected from the group consisting of deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; and
the piperazinylene and piperidinylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof. In the present disclosure, unless otherwise specified, any one end of the general formulas provided above may be connected to R of the compound of Formula (I), while the other end is connected to ring $A^3$. For example, in the group $-O-CH_2$-, the O can be connected to R of the compound of Formula (I), and the $CH_2$ can be connected to ring $A^3$ of the compound of Formula (I), and vice versa.

In some embodiments, L represents a bond.

In some embodiments, examples of L include, but are not limited to, the following groups:
C(O), C(O)NH, NHC(O), $-CH_2$-, $-(CH_2)_2$-, $-(CH_2)_3$-, $-(CH_2)_4$-, $-(CH_2)_5$-, $-(CH_2)_6$-, $-(CH_2)_7$-, $-(CH_2)_8$-, - $(CH_2)_9$-, $-(CH_2)_{10}$-, $-(CH_2)_{11}$-, $-(CH_2)_{12}$-, $-(CH_2)_{13}$-, $-(CH_2)_{14}$-, $-(CH_2)_{15}$-; $-O-CH_2$-, $-O-(CH_2)_2$-, $-O-(CH_2)_3$-, - $O-(CH_2)_4$-, $-O-(CH_2)_5$-, $-O-(CH_2)_6$-, $-O-(CH_2)_7$-, $-O-(CH_2)_8$-, $-O-(CH_2)_9$-, $-O-(CH_2)_{10}$-, $-(CH_2)_1-O$-, $-(CH_2)_2-O$-, $-(CH_2)_3-O$-, $-(CH_2)_4-O$-, $-(CH_2)_5-O$-, $-(CH_2)_6-O$-, $-(CH_2)_7-O$-, $-(CH_2)_8-O$-, $-(CH_2)_9-O$-, $-(CH_2)_{10}-O$-,$-CH_2-O-CH_2$-, $-CH_2-O-(CH_2)_2$-, $-(CH_2)_1-O-(CH_2)_3$-, $-(CH_2)_1-O-(CH_2)_4$-, $-(CH_2)_1-O-(CH_2)_5$-, $-(CH_2)_1-O-(CH_2)_6$-, $-(CH_2)_1-O-(CH_2)_7$-, $-(CH_2)_1-O-(CH_2)_8$-, $-(CH_2)_2-O-(CH_2)_1$-, $-(CH_2)_2-O-(CH_2)_2$-, $-(CH_2)_2-O-(CH_2)_3$-, $-(CH_2)_2-O-(CH_2)_4$-, $-(CH_2)_2-O-(CH_2)_5$-, $-(CH_2)_2-O-(CH_2)_6$-, $-(CH_2)_2-O-(CH_2)_7$-, $-(CH_2)_2-O-(CH_2)_8$-, - $(CH_2)_3-O-(CH_2)_1$-, $-(CH_2)_3-O-(CH_2)_2$-, $-(CH_2)_3-O-(CH_2)_3$-, $-(CH_2)_3-O-(CH_2)_4$-, $-(CH_2)_3-O-(CH_2)_5$-, $-(CH_2)_3-O-(CH_2)_6$-, $-(CH_2)_3-O-(CH_2)_7$-, $-(CH_2)_4-O-(CH_2)_1$-, $-(CH_2)_4-O-(CH_2)_2$-, $-(CH_2)_4-O-(CH_2)_3$-, $-(CH_2)_4-O-(CH_2)_4$-, $-(CH_2)_4-O-(CH_2)_5$-, $-(CH_2)_4-O-(CH_2)_6$-, $-(CH_2)_5-O-(CH_2)_1$-, $-(CH_2)_5-O-(CH_2)_2$-, $-(CH_2)_5-O-(CH_2)_3$-, $-(CH_2)_5-O-(CH_2)_4$-, $-(CH_2)_5-O-(CH_2)_5$-, $-(CH_2)_6-O-(CH_2)_1$-, $-(CH_2)_6-O-(CH_2)_2$-, $-(CH_2)_6-O-(CH_2)_3$-, - $(CH_2)_6-O-(CH_2)_4$-, $-(CH_2)_7-O-(CH_2)_1$-, $-(CH_2)_7-O-(CH_2)_2$-, $-(CH_2)_7-O-(CH_2)_3$-, $-(CH_2)_8-O-(CH_2)_1$-, $-(CH_2)_8-O-(CH_2)_2$-, $-CH(CH_3)-O-(CH_2)_1$-, $-CH(CH_3)-O-(CH_2)_2$-, $-CH(CH_3)-O-(CH_2)_3$-, $-CH(CH_3)-O-(CH_2)_4$-, - $CH(CH_3)-O-(CH_2)_5$-, $-CH(CH_3)-O-(CH_2)_6$-, $-CH(CH_3)-O-(CH_2)_7$-, $-CH(CH_3)-O-(CH_2)_9$-, $-(O(CH_2)_2)_2$-, - $(O(CH_2)_2)_3$-, $-(O(CH_2)_2)_4$-, $-(O(CH_2)_2)_5$-, $-(O(CH_2)_2)_6$-, $-CH_2-(O(CH_2)_2)_2$-, $-CH_2-(O(CH_2)_2)_3$-, $-CH_2-(O(CH_2)_2)_4$-, $-CH_2-(O(CH_2)_2)_5$-, $-CH_2-(O(CH_2)_2)_6$-, $-CH_2-(O(CH_2)_2)_1-OCH_2$-, $-CH_2-(O(CH_2)_2)_2-OCH_2$-, - $CH_2-(O(CH_2)_2)_3-OCH_2$-, $-CH_2-(O(CH_2)_2)_4-OCH_2$-, $-CH_2-(O(CH_2)_2)_5-OCH_2$-, $-CH_2-(O(CH_2)_2)_6-OCH_2$-, - $(CH_2)_2-(O(CH_2)_2)_2$-, $-(CH_2)_2-(O(CH_2)_2)_3$-, $-(CH_2)_2-(O(CH_2)_2)_4$-, $-(CH_2)_2-(O(CH_2)_2)_5$-, $-(CH_2)_2-(O(CH_2)_2)_6$-, $-(CH_2)_3-(O(CH_2)_2)_2$-, $-(CH_2)_3-(O(CH_2)_2)_3$-, $-(CH_2)_3-(O(CH_2)_2)_4$-, $-(CH_2)_3-(O(CH_2)_2)_5$-, $-(CH_2)_4-(O(CH_2)_2)_2$-, $-(CH_2)_4-(O(CH_2)_2)_3$-, $-(CH_2)_4-(O(CH_2)_2)_4$-, $-(CH_2)_4-(O(CH_2)_2)_5$-, $-CH_2-(O(CH_2)_3)_2$-, $-CH_2-(O(CH_2)_3)_3$-, - $CH_2-(O(CH_2)_3)_4$-, $-(CH_2)_2-(O(CH_2)_3)_2$-, $-(CH_2)_2-(O(CH_2)_3)_3$-, $-(CH_2)_2-(O(CH_2)_3)_4$-, $-(CH_2)_3-(O(CH_2)_3)_2$-, - $(CH_2)_3-(O(CH_2)_3)_3$-, $-CH_2-O-(CH_2)_2-O-(CH_2)_3$-, $-CH_2-(O(CH_2)_2)_2-(O(CH_2)_3)_2$-,

$(CH_2)_2-O-(CH_2)_2-O-(CH_2)_3-$, $-(CH_2)_2-(O(CH_2)_2)_2-(O(CH_2)_3)_2-$, $-(CH_2)_3-O-(CH_2)_2-O-(CH_2)_3-$, $-(CH_2)_3-(O(CH_2)_2)_2-(O(CH_2)_3)_2-$, - $CH_2-O-(CH_2)_3-O-(CH_2)_2-$, $-CH_2-(O(CH_2)_3)_2-(O(CH_2)_2)_2-$, $(CH_2)_1-N(R_{a7})-(CH_2)_1-$, $-(CH_2)_1-N(R_{a7})-(CH_2)_2-$, $-(CH_2)_1-N(R_{a7})-(CH_2)_3-$, $-(CH_2)_1-N(R_{a7})-(CH_2)_4-$, $-(CH_2)_1-N(R_{a7})-(CH_2)_5-$, $-(CH_2)_1-N(R_{a7})-(CH_2)_6-$, - $N(R_{a7})-(CH_2)_1-$, $-N(R_{a7})-(CH_2)_2-$, $-N(R_{a7})-(CH_2)_3-$, $-N(R_{a7})-(CH_2)_4-$, $-N(R_{a7})-(CH_2)_5-$, $-N(R_{a7})-(CH_2)_6-$, - (CH2)2-$N(R_{a7})-(CH_2)_1-$, $-(CH_2)_2-N(R_{a7})-(CH_2)_2-$, $-(CH_2)_2-N(R_{a7})-(CH_2)_3-$, $-(CH_2)_2-N(R_{a7})-(CH_2)_4-$, $-(CH_2)_2-N(R_{a7})-(CH_2)_5-$, $-(CH_2)_2-N(R_{a7})-(CH_2)_6-$, $-(CH_2)_3-N(R_{a7})-(CH_2)_1-$, $-(CH_2)_3-N(R_{a7})-(CH_2)_2-$, $-(CH_2)_3-N(R_{a7})-$(CH2)3-, $-(CH_2)_4-N(R_{a7})-(CH_2)_1-$, $-(CH_2)_4-N(R_{a7})-(CH_2)_2-$, $-(CH_2)_4-N(R_{a7})-(CH_2)_3-$, $-(CH_2)_4-N(R_{a7})-(CH_2)_4-$, $-(CH_2)_5-N(R_{a7})-(CH_2)_1-$, $-(CH_2)_5-N(R_{a7})-(CH_2)_2-$, $-(CH_2)_5-N(R_{a7})-(CH_2)_3-$, $-(CH_2)_5-N(R_{a7})-(CH_2)_4-$, - $(CH_2)_5-N(R_{a7})-(CH_2)_5-$, $-(CH_2)_6-N(R_{a7})-(CH_2)_1-$, $-(CH_2)_6-N(R_{a7})-(CH_2)_2-$, $-(CH_2)_6-N(R_{a7})-(CH_2)_3-$, $-(CH_2)_7-N(R_{a7})-(CH_2)_1-$, $-(CH_2)_7-N(R_{a7})-(CH_2)_2-$, $-(CH_2)_7-N(R_{a7})-(CH_2)_3-$, $-(CH_2)_8-N(R_{a7})-(CH_2)_1-$, $-(CH_2)_8-N(R_{a7})-$(CH2)2-, $-(CH_2)_8-N(R_{a7})-(CH_2)_3-$, $-CH(CH_3)-N(R_{a7})-(CH_2)_1-$, $-CH(CH_3)-N(R_{a7})-(CH_2)_2-$, $-CH(CH_3)-N(R_{a7})-$(CH2)3-, $-CH(CH_3)-N(R_{a7})-(CH_2)_4-$, $-CH(CH_3)-N(R_{a7})-(CH_2)_5-$, $-CH(CH_3)-N(R_{a7})-(CH_2)_6-$, $-(CH_2)_1-N(R_{a7})-$, $-(CH_2)_2-N(R_{a7})-$, $-(CH_2)_3-N(R_{a7})-$, $-(CH_2)_4-N(R_{a7})-$, $-(CH_2)_5-N(R_{a7})-$, $-(CH_2)_6-N(R_{a7})-$, $-(CH_2)_7-N(R_{a7})-$, - $(CH_2)_8-N(R_{a7})-$, $-(CH_2)_9-N(R_{a7})-$, $(CH_2)_{10}-N(R_{a7})-$, $-C(O)NHCH_2-$, $-C(O)NH(CH_2)_2-$, $-C(O)NH(CH_2)_3-$, - $C(O)NH(CH_2)_4-$, $-C(O)NH(CH_2)_5-$, $-CH_2C(O)NHCH_2-$, $-(CH_2)_2C(O)NH(CH_2)_2-$, $-(CH_2)_2C(O)NH(CH_2)_3-$, $-(CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, - $(CH_2)_4C(O)NH(CH_2)_4-$, $-(CH_2)_5C(O)NH(CH_2)_5-$, $-(CH_2)_2C(O)NH(CH_2)_2-O-(CH_2)_2-$, $-NHC(O)CH_2-$, - $NHC(O)(CH_2)_2-$, $-NHC(O)(CH_2)_3-$, $-NHC(O)(CH_2)_4-$, $-NHC(O)(CH_2)_5-$, $-CH_2NHC(O)CH_2-$, - $(CH_2)_2NHC(O)(CH_2)_2-$, $-(CH_2)_2NHC(O)(CH_2)_3-$, $-(CH_2)_2NHC(O)(CH_2)_4-$, $-(CH_2)_2NHC(O)(CH_2)_5-$, - $(CH_2)_3NHC(O)(CH_2)_3-$, $-(CH_2)_3NHC(O)(CH_2)_4-$, $-(CH_2)_4NHC(O)(CH_2)_4-$, $-(CH_2)_5NHC(O)(CH_2)_5-$, - $(CH_2)_2NHC(O)(CH_2)_2-O-(CH_2)_2-$, $-(CH_2)_4NHC(O)CH_2-$, $-CH_2$-phenylene-$CH_2-$, $-CH_2$-phenylene-$(CH_2)_2-$, $-CH_2$-phenylene-$(CH_2)_3-$, $-CH_2$-phenylene-$(CH_2)_4-$, $-CH_2$-phenylene-$(CH_2)_5-$, $-CH_2$-phenylene-$(CH_2)_6-$, - $(CH_2)_2$-phenylene-$(CH_2)_1-$, $-(CH_2)_2$-phenylene-$(CH_2)_2-$, $-(CH_2)_2$-phenylene-$(CH_2)_3-$, $-(CH_2)_2$-phenylene-$(CH_2)_4-$, $-(CH_2)_2$-phenylene-$(CH_2)_5-$, $-(CH_2)_3$-phenylene-$CH_2-$, $-(CH_2)_3$-phenylene-$(CH_2)_2-$, $-(CH_2)_3$-phenylene-$(CH_2)_3-$, $-(CH_2)_3$-phenylene-$(CH_2)_4-$, $-(CH_2)_3$-phenylene-$(CH_2)_5-$, $-(CH_2)_4$-phenylene-$CH_2-$, - $(CH_2)_4$-phenylene-$(CH_2)_2-$, $-(CH_2)_4$-phenylene-$(CH_2)_3-$, $-(CH_2)_4$-phenylene-$(CH_2)_4-$, $-(CH_2)_4$-phenylene-$(CH_2)_5-$, $-CH_2$-piperidinylene-$CH_2-$, $-CH_2$-piperidinylene-$(CH_2)_2-$, $-CH_2$-piperidinylene-$(CH_2)_3-$, $-CH_2$-piperidinylene-$(CH_2)_4-$, $-CH_2$-piperidinylene-$(CH_2)_5-$, $-CH_2$-piperidinylene-$(CH_2)_6-$, $-(CH_2)_2$-piperidinylene-$(CH_2)_1-$, $-(CH_2)_2$-piperidinylene-$(CH_2)_2-$, $-(CH_2)_2$-piperidinylene-$(CH_2)_3-$, $-(CH_2)_2$-piperidinylene-$(CH_2)_4-$, $-(CH_2)_2$-piperidinylene-$(CH_2)_5-$, $-(CH_2)_3$-piperidinylene-$CH_2-$, $-(CH_2)_3$-piperidinylene-$(CH_2)_2-$, $-(CH_2)_3$-piperidinylene-$(CH_2)_3-$, $-(CH_2)_3$-piperidinylene-$(CH_2)_4-$, $-(CH_2)_3$-piperidinylene-$(CH_2)_5-$, $-CH_2$-piperazinylene-$CH_2-$, $-CH_2$-piperazinylene-$(CH_2)_2-$, $-CH_2$-piperazinylene-$(CH_2)_3-$, $-CH_2$-piperazinylene-$(CH_2)_4-$, $-CH_2$-piperazinylene-$(CH_2)_5-$, $-(CH_2)_2$-piperazinylene-$(CH_2)_1-$, - $(CH_2)_2$-piperazinylene-$(CH_2)_2-$, $-(CH_2)_2$-piperazinylene-$(CH_2)_3-$, $-(CH_2)_2$-piperazinylene-$(CH_2)_4-$, $-(CH_2)_2$-piperazinylene-$(CH_2)_5-$, $-(CH_2)_3$-piperazinylene-$CH_2-$, $-(CH_2)_3$-piperazinylene-$(CH_2)_2-$, $-(CH_2)_3$-piperazinylene-$(CH_2)_3-$, $-(CH_2)_3$-piperazinylene-$(CH_2)_4-$, $-(CH_2)_3$-piperazinylene-$(CH_2)_5-$, $-(CH_2)_4$-piperazinylene-$CH_2-$, $-(CH_2)_4$-piperazinylene-$(CH_2)_2-$, $-(CH_2)_4$-piperazinylene-$(CH_2)_3-$, $-(CH_2)_4$-piperazinylene-$(CH_2)_4-$, $-(CH_2)_4$-piperazinylene-$(CH_2)_5-$, $-(CH_2)_8$-piperazinylene-$CH_2-$, $-(CH_2)_8$-piperazinylene-$(CH_2)_2-$, $-(CH_2)_9$-piperazinylene-$(CH_2)_3-$, $-(CH_2)_8$-piperazinylene-$(CH_2)_4-$, or $-(CH_2)_8$-piperazinylene-$(CH_2)_5-$;

wherein each $R_{a7}$ independently represents H or $C_{1-6}$ alkyl (e.g., methyl, ethyl, propyl, or isopropyl);
each phenylene is independently optionally substituted with a substituent selected from the group consisting of deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;
the piperazinylene and piperidinylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.

In some embodiments, examples of L include, but are not limited to, the following groups:

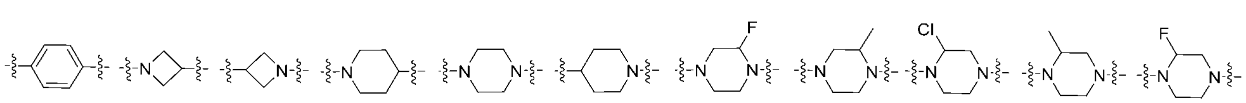

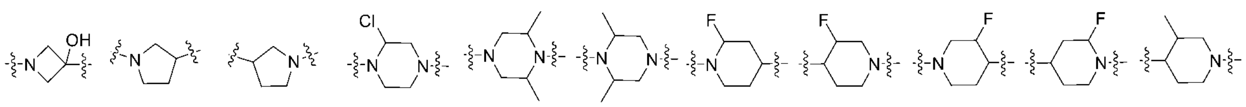

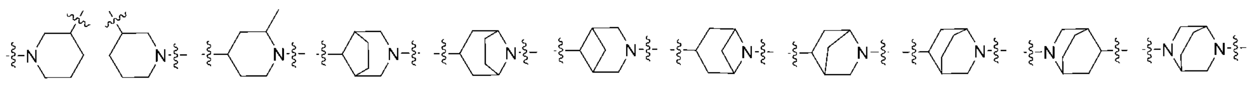

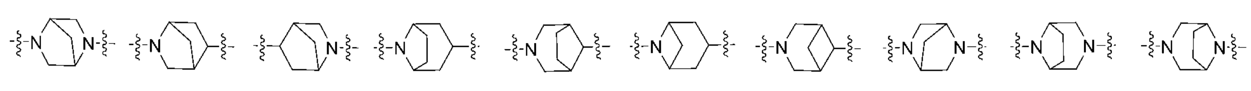

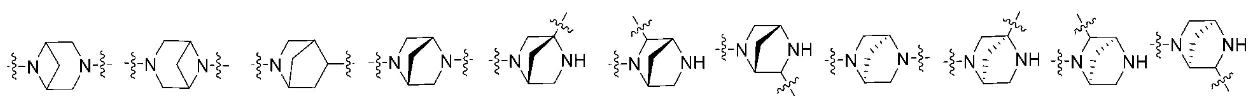

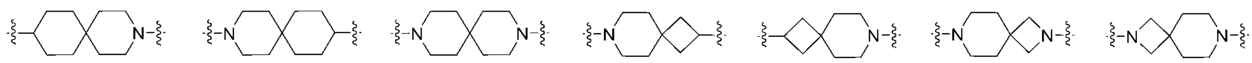

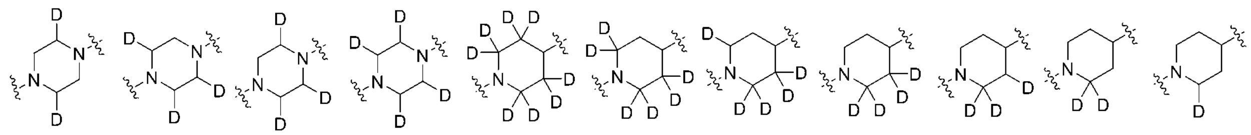

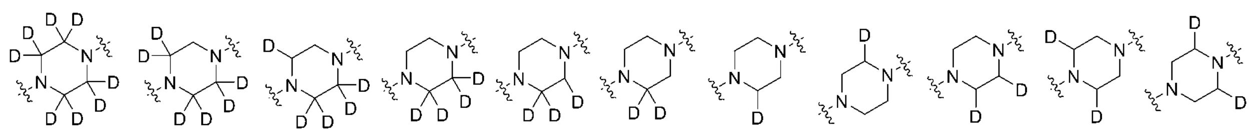

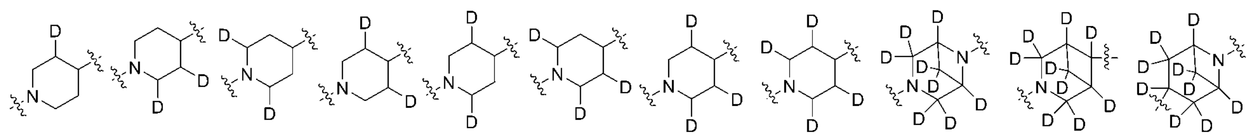

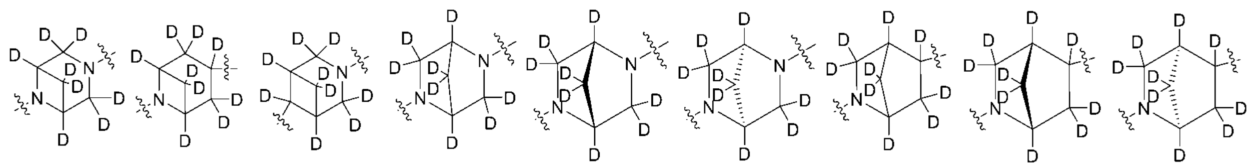

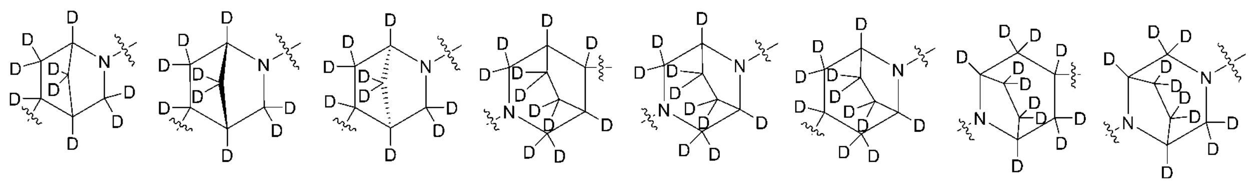

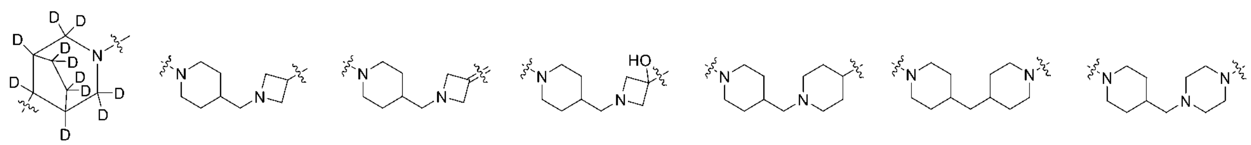

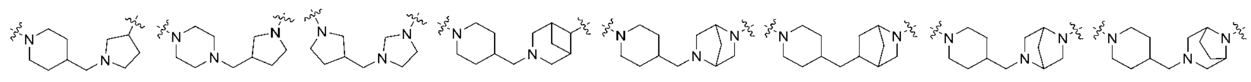

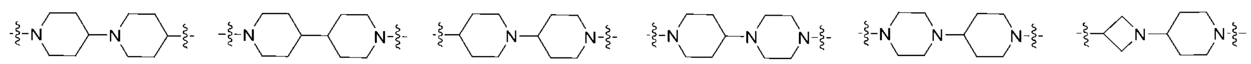

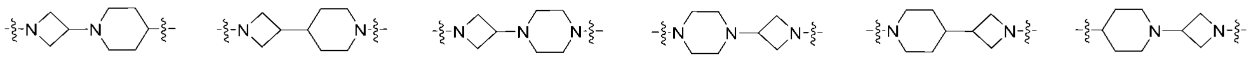

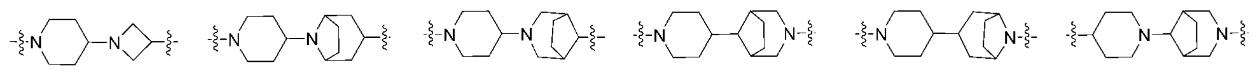

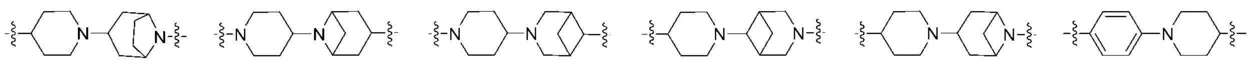

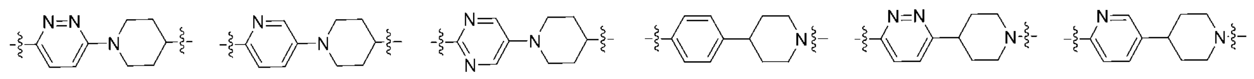

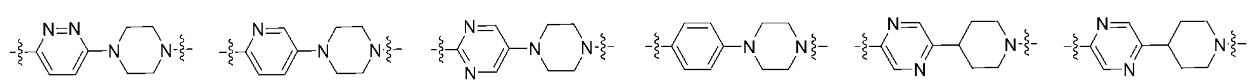

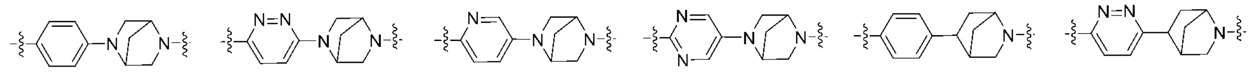

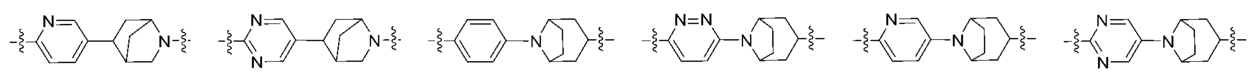

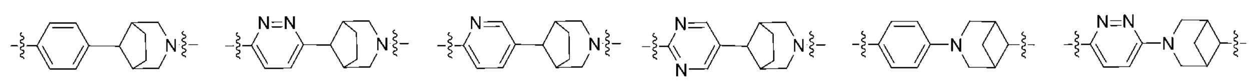

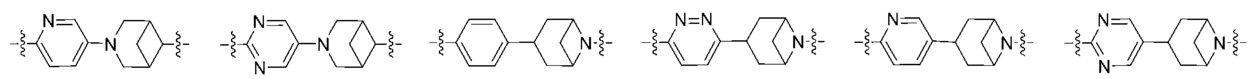

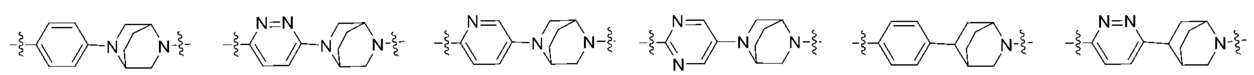

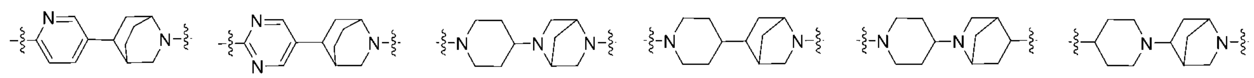

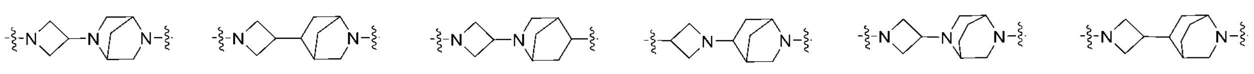

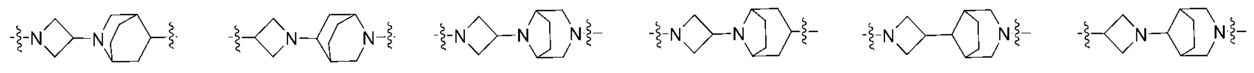

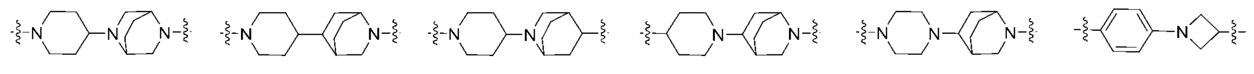

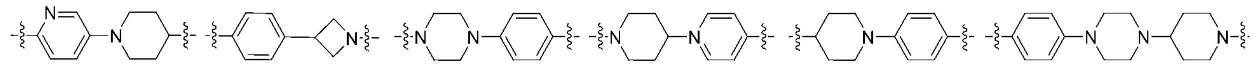

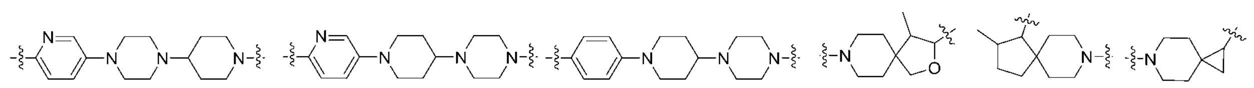

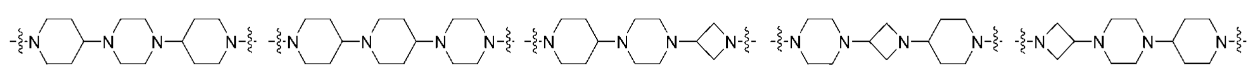

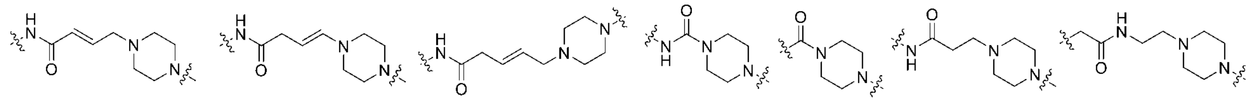
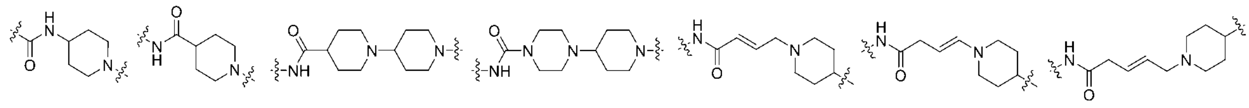
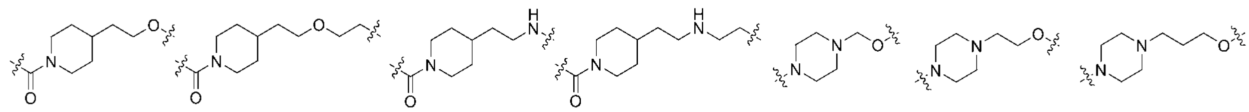
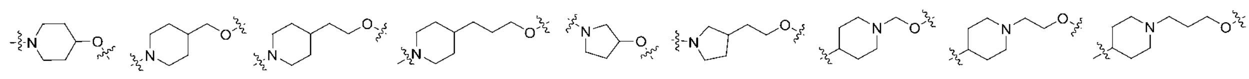
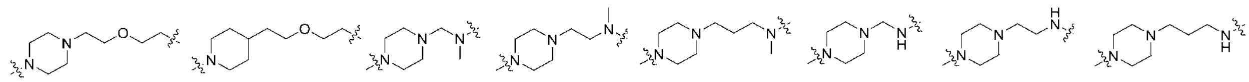
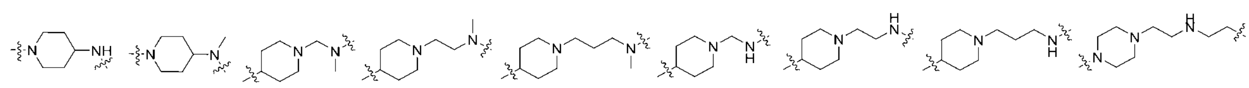
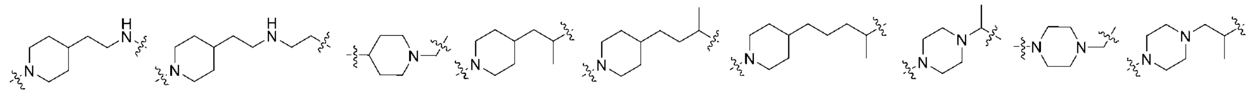
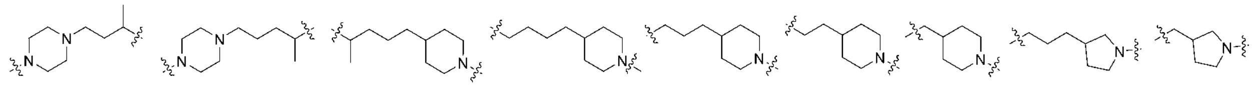
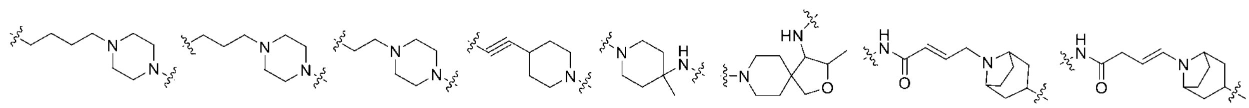
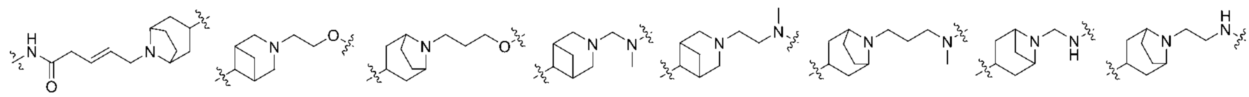
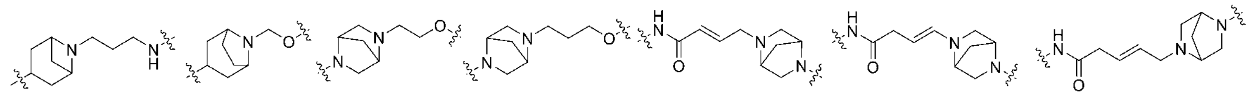
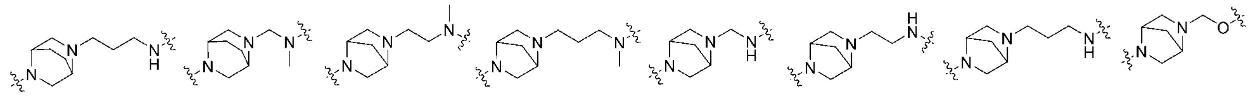
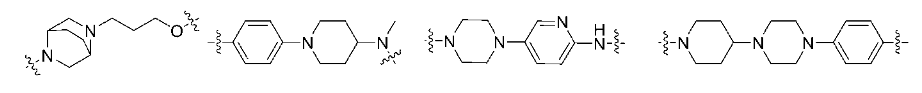 或 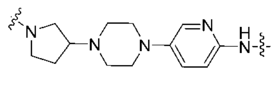 ;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto,

cyano, oxo, $C_{2-6}$ alkynyl, and $C_{2-6}$ alkenyl.

In some embodiments, X represents:

$NR_1R_2$, $C(O)NR_3R_4$, $NHC(O)R_5$, $NHC(O)NR_6R_7$, $OR_8$, $SR_9$, $S(O)_2NR_{10}R_{11}$ or $N(R_{12})S(O)_2R_{13}$, wherein $R_1$, $R_3$, $R_4$, $R_5$, $R_6$, $R_7$, $R_9$, $R_{10}$, $R_{11}$, and $R_{12}$ each independently represent H, optionally substituted linear or branched alkyl (e.g., optionally substituted linear or branched $C_{1-10}$ alkyl, or optionally substituted linear or branched $C_{1-6}$ alkyl), optionally substituted cycloalkyl (e.g., optionally substituted $C_{3-30}$ cycloalkyl, optionally substituted $C_{3-20}$ cycloalkyl, or optionally substituted $C_{3-15}$ cycloalkyl), optionally substituted aryl (e.g., optionally substituted $C_{5-30}$ aryl, optionally substituted $C_{5-20}$ aryl, or optionally substituted $C_{5-15}$ aryl), optionally substituted heterocyclyl (e.g., optionally substituted 4- to 30-membered heterocyclyl, optionally substituted 4- to 20-membered heterocyclyl, or optionally substituted 4- to 15-membered heterocyclyl) or optionally substituted heteroaryl (e.g., optionally substituted 5- to 30-membered heteroaryl, optionally substituted 5- to 20-membered heteroaryl, or optionally substituted 5- to 15-membered heteroaryl), and $R_2$, $R_8$, and $R_{13}$ each independently represent optionally substituted linear or branched alkyl (e.g., optionally substituted linear or branched $C_{1-10}$ alkyl, or optionally substituted linear or branched $C_{1-6}$ alkyl), optionally substituted cycloalkyl (e.g., optionally substituted $C_{3-30}$ cycloalkyl, optionally substituted $C_{3-20}$ cycloalkyl, or optionally substituted $C_{3-15}$ cycloalkyl), optionally substituted aryl (e.g., optionally substituted $C_{5-30}$ aryl, optionally substituted $C_{5-20}$ aryl, or optionally substituted $C_{5-15}$ aryl), optionally substituted heterocyclyl (e.g., optionally substituted 4- to 30-membered heterocyclyl, optionally substituted 4- to 20-membered heterocyclyl, or optionally substituted 4- to 15-membered heterocyclyl), or optionally substituted heteroaryl (e.g., optionally substituted 5- to 30-membered heteroaryl, optionally substituted 5- to 20-membered heteroaryl, or optionally substituted 5- to 15-membered heteroaryl); or wherein $R_3$ and $R_4$, together with the nitrogen atom to which they are attached, form an optionally substituted nitrogen-containing heterocyclyl (e.g., optionally substituted 4- to 30-membered nitrogen-containing heterocyclyl, optionally substituted 4- to 20-membered nitrogen-containing heterocyclyl, or optionally substituted 4- to 15-membered nitrogen-containing heterocyclyl). In some embodiments, each linear or branched alkyl is independently optionally substituted with a substituent selected from the group consisting of deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl, and $C_{2-6}$ alkenyl. In some embodiments, each cycloalkylene, each arylene, each heterocyclylene, each heteroarylene, each cycloalkyl, each aryl, each heterocyclyl, each heteroaryl, and each nitrogen-containing heterocyclyl is independently optionally substituted by a substituent selected from deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, and $C_{2-6}$ alkenyl.

In some embodiments, $R_1$, $R_3$, $R_4$, $R_5$, $R_6$, $R_7$, $R_9$, $R_{10}$, $R_{11}$, and $R_{12}$ each independently represents: H;

linear or branched $C_{1-10}$ alkyl (e.g., linear or branched $C_{1-6}$ alkyl, such as methyl, ethyl, propyl, isopropyl, butyl, isobutyl, sec-butyl, tert-butyl, pentyl, isopentyl, neopentyl, tert-pentyl, and hexyl), which is optionally substituted with one or more (e.g., 1-20, 1-15, 1-10, 1-6, 1-4, 2-6, or 1) substituents selected from the group consisting of: deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl, and $C_{2-6}$ alkenyl;

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornanyl, bicyclo[2.2.1]heptanyl, 2-oxobicyclo[2.2.1]heptanyl, or bicyclo[2.2.1]heptenyl), each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-20, 1-15, 1-10, 1-6, 1-4, 2-6, or 1) substituents selected from the group consisting of: deuterium, halogen, hydroxy, mercapto,

nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenyl or naphthyl, each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_2$, $R_8$, and $R_{13}$ each independently represent:

linear or branched $C_{1-10}$ alkyl, which is optionally substituted with one or more (e.g., 1-20, 1-15, 1-10, 1-6, 1-4, 2-6, or 1) substituents selected from the group consisting of: deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl, and $C_{2-6}$ alkenyl;

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornanyl, bicyclo[2.2.1]heptanyl, 2-oxobicyclo[2.2.1]heptanyl, or bicyclo[2.2.1]heptenyl), each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenyl or naphthyl, each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl,

isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, X represents $C(O)NR_3R_4$, wherein $R_3$ and $R_4$, together with the nitrogen atom to which they are attached, form an optionally substituted 4- to 30-membered nitrogen-containing heterocyclyl (e.g., 4- to 20-membered nitrogen-containing heterocyclyl, or 4- to 15-membered nitrogen-containing heterocyclyl). In some embodiments, examples of the 4- to 30-membered nitrogen-containing heterocyclyl include, but are not limited to, azetidinyl, pyrrolidinyl, imidazolidinyl, pyrazolidyl, oxazolidinyl, thiazolidinyl, piperidinyl, piperazinyl, tetrahydropyridinyl, dihydroxypiperidinyl, difluoro-piperidinyl, morpholinyl, thiomorpholinyl, azacycloheptyl, azacyclooctyl, diazacycloheptanyl, diazacyclooctyl, nitrogen-containing bridged heterocyclyl (e.g., 6- to 20-membered nitrogen-containing bridged heterocyclyl, such as 3-azabicyclo[3.1.0]hexanyl, 3-azabicyclo[3.1.1]heptanyl, 2-azabicyclo[2.2.1]heptanyl, 6-azabicyclo[3.1.1]heptanyl, 2-azabicyclo[2.2.2]octanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 2,5-diazabicyclo[2.2.2]octanyl, and quinuclidinyl), or azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl. In some embodiments, the 4- to 30-membered nitrogen-containing heterocyclyl is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, X represents :

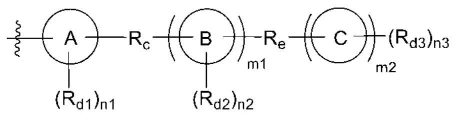

wherein ring A represents heterocyclylene, cycloalkylene, arylene or heteroarylene, and $(R_{d1})_{n1}$ indicates that ring A is optionally substituted with n1 $R_{d1}$ groups, with each $R_{d1}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n1 represents an integer of 0-20;

$R_c$ represents $NR_{c1}$, C(O), $CR_{c2}R_{c3}$ or a bond, wherein $R_{c1}$ represents H, optionally substituted linear or branched alkyl, optionally substituted cycloalkyl, optionally substituted aryl, optionally substituted heterocyclyl or optionally substituted heteroaryl, and $R_{c2}$ and $R_{c3}$ each independently represent H, halogen, optionally substituted linear or branched alkyl, optionally substituted cycloalkyl, optionally substituted aryl, optionally substituted heterocyclyl or optionally substituted heteroaryl;

each ring B is identical or different and each independently represents heterocyclylene, cycloalkylene, arylene, or heteroarylene, ml represents an integer of 0, 1, 2 or 3, and $(R_{d2})_{n2}$ indicates that each ring B is independently optionally substituted with n2 $R_{d2}$ groups, with each $R_{d2}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n2 represents an integer of 0-20;

$R_e$ represents S, O, $CR_{e1}R_{e2}$ or a bond, wherein $R_{e1}$ and $R_{e2}$ each independently represent H, halogen, optionally substituted linear or branched alkyl, optionally substituted cycloalkyl, optionally substituted aryl, optionally substituted heterocyclyl or optionally substituted heteroaryl; and

each ring C is identical or different and each independently represents heterocyclyl, cycloalkyl, aryl, or heteroaryl, m2 represents an integer of 0, 1, 2 or 3, and $(R_{d3})_{n3}$ indicates that each ring C is independently optionally substituted with n3 $R_{d3}$ groups, with each $R_{d3}$ independently representing deuterium, $C_{1-6}$ alkyl, deuterated $C_{1-6}$ alkyl, halogenated

$C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n3 represents an integer of 0-20.

In some embodiments, ring A represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene). In some embodiments, ring A is optionally substituted with n1 $R_{d1}$ substituents, wherein each $R_{d1}$ independently represents deuterium, $C_{1-6}$ alkyl (e.g., $C_{1-4}$ alkyl or $C_{1-3}$ alkyl, such as methyl, ethyl, propyl, isopropyl, butyl, sec-butyl, or tert-butyl), deuterated $C_{1-6}$ alkyl, halogenated $C_{1-6}$ alkyl (e.g., halogenated $C_{1-4}$ alkyl or halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-6}$ alkoxy (e.g., $C_{1-4}$ alkoxy or $C_{1-3}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy or halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), halogen (e.g., fluorine, chlorine, bromine, or iodine), amino, hydroxy, mercapto, cyano, oxo, $C_{2-6}$ alkynyl (e.g., ethynyl) or $C_{2-6}$ alkenyl (e.g., vinyl); and n1 represents an integer of 0, 1, 2, 3, 4, 5, 6, 7, 8, 9, 10, 11, 12, 13, 14, 15, 16, 17, 18, 19 or 20.

In some embodiments, each ring B is identical or different and each independently represents 4- to 30-membered heterocyclylene (e.g., 4- to 20-membered heterocyclylene, or 4- to 15-membered heterocyclylene), $C_{3-30}$ cycloalkylene (e.g., $C_{3-20}$ cycloalkylene, or $C_{3-15}$ cycloalkylene), $C_{5-30}$ arylene (e.g., $C_{5-20}$ arylene, or $C_{5-15}$ arylene) or 5- to 30-membered heteroarylene (e.g., 5- to 20-membered heteroarylene, or 5- to 15-membered heteroarylene). In some embodiments, m1 represents an integer of 0, 1, 2 or 3. In some embodiments, each ring B is optionally substituted with n2 $R_{d2}$ substituents, wherein each $R_{d2}$ independently represents deuterium, $C_{1-6}$ alkyl (e.g., $C_{1-4}$ alkyl or $C_{1-3}$ alkyl, such as methyl, ethyl, propyl, isopropyl, butyl, sec-butyl, or tert-butyl), deuterated $C_{1-6}$ alkyl, halogenated $C_{1-6}$ alkyl (e.g., halogenated $C_{1-4}$ alkyl or halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-6}$ alkoxy (e.g., $C_{1-4}$ alkoxy or $C_{1-3}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy or halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), halogen (e.g., fluorine, chlorine, bromine, or iodine), amino, hydroxy, mercapto, cyano, oxo, $C_{2-6}$ alkynyl (e.g., ethynyl) or $C_{2-6}$ alkenyl (e.g., vinyl); and n2 represents an integer of 0, 1, 2, 3, 4, 5, 6, 7, 8, 9, 10, 11, 12, 13, 14, 15, 16, 17, 18, 19 or 20.

In some embodiments, ring A and ring B each independently represent $C_{3-30}$ cycloalkylene (e.g., $C_{3-20}$ cycloalkylene, or $C_{3-15}$ cycloalkylene). In some embodiments, examples of the $C_{3-30}$ cycloalkylene include, but are not limited to, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_5$-$C_{20}$ spiro-cycloalkylene, such as spiro[3.3]heptylene, spiro[2.5]octylene, spiro[3.5]nonylene, spiro[4.4]nonylene, spiro[4.5]decylene, and spiro[5.5]undecylene), p-menthanylene, m-menthanylene, or bridged cycloalkylene (e.g., $C_6$-$C_{20}$ bridged cycloalkylene, such as adamantanylene, noradamantanylene, bornylene, norbomylene, bicyclo[2.2.1]heptanylene, 2-oxobicyclo[2.2.1]heptanylene, or bicyclo[2.2.1]heptenylene). In some embodiments, the $C_{3-30}$ cycloalkylene is optionally substituted with one or more (e.g., 1, 2, 3, 4, 5, 6, 7, 8, 9, 10, 11, 12, 13, 14, 15, 16, 17, 18, 19 or 20) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, ring A and ring B each independently represent 4- to 30-membered heterocyclylene (e.g., 4- to 20-membered heterocyclylene, or 4- to 15-membered heterocyclylene). In some embodiments, examples of the 4- to 30-membered heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, tetrahydropyridinylene, dihydroxy-piperidinylene, difluoro-piperidinylene, morpholinylene, thiomorpholinylene, azacycloheptanylene, azacyclooctylene, dioxacyclohexylene, azacycloheptanylene, azacyclooctylene, diazacycloheptanylene (e.g., 1,4-diazacycloheptanylene, 4,5-diazacycloheptanylene, 1,3-diazacycloheptanylene), diazacyclooctylene, bridged heterocyclylene (e.g., 6-to 20-membered bridged heterocyclylene, such as 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and quinuclidinylene), azaspirocycloalkylene (e.g., 5- to 20-membered azaspirocycloalkylene, such as 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, and 7-azaspiro[3.5]nonanylene), or octahydropyrrolo[3,4-c]pyrrolylene. In some embodiments, the 4- to 30-membered heterocyclylene is optionally substituted with one or more (e.g., 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$

alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, ring A and ring B each independently represent $C_{5-30}$ arylene (e.g., $C_{5-20}$ arylene, or $C_{5-15}$ arylene). In some embodiments, examples of the $C_{5-30}$ arylene include, but are not limited to, phenylene or naphthylene. In some embodiments, the $C_{5-30}$ arylene is optionally substituted with one or more (e.g., 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, ring A and ring B each independently represent 5- to 30-membered heteroarylene (e.g., 5- to 20-membered heteroarylene, or 5- to 15-membered heteroarylene). In some embodiments, examples of the 5- to 30-membered heteroarylene include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, triazinylene, indolylene, isoindolylene, isoindolinylene, benzofuranylene, chromanylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, 2-oxo-2,3-dihydro-1H-benzo[d]imidazolylene, benzo[b][1,4]oxazinylene, 3,4-dihydro-2H-benzo[b][1,4]oxazinylene, quinolinylene, isoquinolinylene, 1,2,3,4-tetrahydroquinolinylene, naphthyridinylene, cinnolinylene, quinazolinylene, quinoxalinylene, 1,2,3,4-tetrahydroquinoxalinylene, phthalazinylene, 5,6,7,8-tetrahydro-[1,2,4]triazolo[4,3-a]pyrazinylene, 4,5,6,7-tetrahydrothieno[3,2-c]pyridinylene, 5-oxo-6,7-dihydrothieno[3,2-d]pyrimidinylene, thieno[2,3-d]pyrimidinylene, thieno[3,2-d]pyrimidinylene, isoxazolo[4,5-c]pyridinylene, isoxazolo[4,5-c]pyrimidinylene, isoxazolo[4,5-d]pyrimidinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, 5,6,7,8-tetrahydrobenzo[4,5]thieno[2,3-d]pyrimidinylene, or 6,7-dihydro-5H-cyclopenta[4,5]thieno[2,3-d]pyrimidinylene. In some embodiments, 5- to 30-membered heteroarylene is optionally substituted with one or more (e.g., 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, each ring C is identical or different and each independently represents 4- to 30-membered heterocyclyl (e.g., 4- to 20-membered heterocyclyl, or 4- to 15-membered heterocyclyl), $C_{3-30}$ cycloalkyl (e.g., $C_{3-20}$ cycloalkyl, or $C_{3-15}$ cycloalkyl), $C_{5-30}$ aryl (e.g., $C_{5-20}$ aryl, or $C_{5-15}$ aryl) or 5- to 30-membered heteroaryl (e.g., 5- to 20-membered heteroaryl, or 5- to 15-membered heteroaryl). In some embodiments, m2 represents an integer of 0, 1, 2 or 3. In some embodiments, $(R_{g3})_{a3}$ indicates that each ring C is independently optionally substituted with n3 $R_{d3}$ groups, wherein each $R_{d3}$ independently represents deuterium, $C_{1-6}$ alkyl (e.g., $C_{1-4}$ alkyl or $C_{1-3}$ alkyl, such as methyl, ethyl, propyl, isopropyl, butyl, sec-butyl, or tert-butyl), deuterated $C_{1-6}$ alkyl, halogenated $C_{1-6}$ alkyl (e.g., halogenated $C_{1-4}$ alkyl or halogenated $C_{1-3}$ alkyl, such as $F_3C$-, $FCH_2$-, $F_2CH$-, $ClCH_2$-, $Cl_2CH$-, $CF_3CF_2$-, $CF_3CHF$-, $CHF_2CF_2$-, $CHF_2CHF$-, $CF_3CH_2$- or $CH_2ClCH_2$-), $C_{1-6}$ alkoxy (e.g., $C_{1-4}$ alkoxy or $C_{1-3}$ alkoxy, such as methoxy, ethoxy, propoxy, isopropoxy, n-butoxy, sec-butoxy, or tert-butoxy), halogenated $C_{1-6}$ alkoxy (e.g., halogenated $C_{1-4}$ alkoxy or halogenated $C_{1-3}$ alkoxy, such as $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-), halogen (e.g., fluorine, chlorine, bromine, or iodine), amino, hydroxy, mercapto, cyano, oxo, $C_{2-6}$ alkynyl (e.g., ethynyl) or $C_{2-6}$ alkenyl (e.g., vinyl), and n3 represents an integer of 0, 1, 2, 3, 4, 5, 6, 7, 8, 9, 10, 11, 12, 13, 14, 15, 16, 17, 18, 19 or 20.

In some embodiments, each ring C is identical or different and each independently represents $C_{3-30}$ cycloalkyl (e.g., $C_{3-20}$ cycloalkyl, or $C_{3-15}$ cycloalkyl). In some embodiments, examples of the $C_{3-30}$ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornanyl, bicyclo[2.2.1]heptanyl, 2-oxobicyclo[2.2.1]heptanyl, or bicyclo[2.2.1]heptenyl). In some embodiments, the $C_{3-30}$ cycloalkyl is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, each ring C is identical or different and each independently represents 4- to 30-membered heterocyclyl (e.g., 4- to 20-membered heterocyclyl, or 4- to 15-membered heterocyclyl). In some embodiments, examples of the 4- to 30-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl. In some embodiments, 4- to 30-membered heterocyclyl is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, each ring C is identical or different and each independently represents $C_{5-30}$ aryl (e.g., $C_{5-20}$ aryl, or $C_{5-15}$ aryl). In some embodiments, examples of the $C_{5-30}$ aryl include, but are not limited to, phenyl or naphthyl. In some embodiments, the $C_{5-30}$ aryl is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, each ring C is identical or different and each independently represents 5- to 30-membered heteroaryl (e.g., 5- to 20-membered heteroaryl, or 5- to 15-membered heteroaryl). In some embodiments, examples of the 5- to 30-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl. In some embodiments, the 5- to 30-membered heteroaryl is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_c$ represents C(O) or a bond.

In some embodiments, $R_c$ represents $NR_{c1}$, wherein $R_{c1}$ represents H, optionally substituted linear or branched $C_{1-10}$ alkyl (e.g., optionally substituted linear or branched $C_{1-6}$ alkyl), optionally substituted $C_{3-30}$ cycloalkyl (e.g., optionally substituted $C_{3-20}$ cycloalkyl, or optionally substituted $C_{3-15}$ cycloalkyl), optionally substituted $C_{5-30}$ aryl (e.g., optionally substituted $C_{5-20}$ aryl, or optionally substituted $C_{5-15}$ aryl), optionally substituted 4- to 30-membered heterocyclyl (e.g., optionally substituted 4- to 20-membered heterocyclyl, or optionally substituted 4- to 15-membered heterocyclyl) or optionally substituted 5- to 30-membered heteroaryl (e.g., optionally substituted 5- to 20-membered heteroaryl, or optionally substituted 5- to 15-membered heteroaryl).

In some embodiments, $R_c$ represents $NR_{c1}$, wherein $R_{c1}$ represents H or linear or branched $C_{1-10}$ alkyl (e.g., linear or branched $C_{1-6}$ alkyl). In some embodiments, examples of the linear or branched $C_{1-10}$ alkyl include, but are not limited to, methyl, ethyl, propyl, isopropyl, butyl, isobutyl, sec-butyl, tert-butyl, pentyl, isopentyl, neopentyl, tert-pentyl, and hexyl. In some embodiments, the linear or branched $C_{1-10}$ alkyl is optionally substituted with one or more (e.g., 1-20, 1-15, 1-10, 1-6, 1-4, 2-6, or 1) substituents selected from the group consisting of: deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl, and $C_{2-6}$ alkenyl.

In some embodiments, $R_c$ represents $NR_{c1}$, wherein $R_{c1}$ represents optionally substituted $C_{3-30}$ cycloalkyl (e.g., optionally substituted $C_{3-20}$ cycloalkyl, or optionally substituted $C_{3-15}$ cycloalkyl). In some embodiments, examples of the $C_{3-30}$ cycloalkyl include, but are not limited to, cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spirocycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbornanyl, bicyclo[2.2.1]heptanyl, 2-oxobicyclo[2.2.1]heptanyl, or bicyclo[2.2.1]heptenyl). In some embodiments, the $C_{3-30}$ cycloalkyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted

$C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_c$ represents $NR_{c1}$, wherein $R_{c1}$ represents optionally substituted 4- to 30-membered heterocyclyl (e.g., optionally substituted 4- to 20-membered heterocyclyl, or optionally substituted 4- to 15-membered heterocyclyl). In some embodiments, examples of the 4- to 30-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoropiperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl. In some embodiments, the 4- to 30-membered heterocyclyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_c$ represents $NR_{c1}$, wherein $R_{c1}$ represents optionally substituted $C_{5-30}$ aryl (e.g., optionally substituted $C_{5-20}$ aryl, or optionally substituted $C_{5-15}$ aryl). In some embodiments, examples of the $C_{5-30}$ aryl include, but are not limited to, phenyl or naphthyl. In some embodiments, the $C_{5-30}$ aryl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_c$ represents $NR_{c1}$, wherein $R_{c1}$ represents optionally substituted 5- to 30-membered heteroaryl (e.g., optionally substituted 5- to 20-membered heteroaryl, or optionally substituted 5- to 15-membered heteroaryl). In some embodiments, examples of the 5- to 30-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl. In some embodiments, the 5- to 30-membered heteroaryl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_c$ represents $CR_{c2}R_{c3}$, wherein $R_{c2}$ and $R_{c3}$ each independently represent H, halogen, optionally substituted linear or branched $C_{1-10}$ alkyl (e.g., optionally substituted linear or branched $C_{1-6}$ alkyl), optionally substituted $C_{3-30}$ cycloalkyl (e.g., optionally substituted $C_{3-20}$ cycloalkyl, or optionally substituted $C_{3-15}$ cycloalkyl), optionally substituted $C_{5-30}$ aryl (e.g., optionally substituted $C_{5-20}$ aryl, or optionally substituted $C_{5-15}$ aryl), optionally substituted 4- to 30-membered heterocyclyl (e.g., optionally substituted 4- to 20-membered heterocyclyl, or optionally substituted 4- to 15-membered heterocyclyl) or optionally substituted 5- to 30-membered heteroaryl (e.g., optionally substituted 5- to 20-membered heteroaryl, or optionally substituted 5- to 15-membered heteroaryl). In some embodiments, examples of the linear or branched $C_{1-10}$ alkyl include, but are not limited to, methyl, ethyl, propyl, isopropyl, butyl, isobutyl, sec-butyl, tert-butyl, pentyl, isopentyl, neopentyl, tert-pentyl, and hexyl. In some embodiments, the linear or branched $C_{1-10}$ alkyl is optionally substituted with one or more (e.g., 1-20, 1-15, 1-10, 1-6, 1-4, 2-6, or 1) substituents selected from the group consisting of: deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl, and $C_{2-6}$ alkenyl. In some embodiments, examples of the $C_{3-30}$ cycloalkyl include, but are not limited to, cyclopropyl, cyclobutyl, cyclopentyl, cyclopentenyl, cyclohexyl, cyclohexenyl, cycloheptyl,

cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbomanyl, bicyclo[2.2.1]heptanyl, 2-oxobicyclo[2.2.1]heptanyl, or bicyclo[2.2.1]heptenyl). In some embodiments, the $C_{3-30}$ cycloalkyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the 4- to 30-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl. In some embodiments, the 4- to 30-membered heterocyclyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the $C_{5-30}$ aryl include, but are not limited to, phenyl or naphthyl. In some embodiments, the $C_{5-30}$ aryl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the 5-to 30-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo [1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno [2,3-d]pyrimidinyl. In some embodiments, the 5- to 30-membered heteroaryl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, $R_e$ represents S, O or a bond.

In some embodiments, $R_e$ represents $CR_{e1}R_{e2}$, wherein $R_{e1}$ and $R_{e2}$ each independently represent H, halogen, optionally substituted linear or branched $C_{1-10}$ alkyl (e.g., optionally substituted linear or branched $C_{1-6}$ alkyl), optionally substituted $C_{3-30}$ cycloalkyl (e.g., optionally substituted $C_{3-20}$ cycloalkyl, or optionally substituted $C_{3-15}$ cycloalkyl), optionally substituted $C_{5-30}$ aryl (e.g., optionally substituted $C_{5-20}$ aryl, or optionally substituted $C_{5-15}$ aryl), optionally substituted 4- to 30-membered heterocyclyl (e.g., optionally substituted 4- to 20-membered heterocyclyl, or optionally substituted 4- to 15-membered heterocyclyl) or optionally substituted 5- to 30-membered heteroaryl (e.g., optionally substituted 5- to 20-membered heteroaryl, or optionally substituted 5- to 15-membered heteroaryl). In some embodiments, examples of the linear or branched $C_{1-10}$ alkyl include, but are not limited to, methyl, ethyl, propyl, isopropyl, butyl, isobutyl, sec-butyl, tert-butyl, pentyl, isopentyl, neopentyl, tert-pentyl, and hexyl. In some embodiments, the linear or branched $C_{1-10}$ alkyl is optionally substituted with one or more (e.g., 1-20, 1-15, 1-10, 1-6, 1-4, 2-6, or 1) substituents selected from the group consisting of: deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl, and $C_{2-6}$ alkenyl. In some embodiments, examples of the $C_{3-30}$ cycloalkyl include, but are not limited to, cyclopropyl, cyclobutyl, cyclopentyl, cyclopentenyl, cyclohexyl, cyclohexenyl, cycloheptyl,

cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbomanyl, bicyclo[2.2.1]heptanyl, 2-oxobicyclo[2.2.1]heptanyl, or bicyclo[2.2.1]heptenyl). In some embodiments, the $C_{3-30}$ cycloalkyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the 4- to 30-membered heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl. In some embodiments, the 4- to 30-membered heterocyclyl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the $C_{5-30}$ aryl include, but are not limited to, phenyl or naphthyl. In some embodiments, the $C_{5-30}$ aryl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof. In some embodiments, examples of the5-to 30-membered heteroaryl include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo [1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno [2,3-d]pyrimidinyl. In some embodiments, 5- to 30-membered heteroaryl is optionally substituted with one or more (e.g., 1-20, 1-15, 1-10, 1-6, 1-4, 2-6, or 1) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

In some embodiments, X represents :

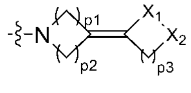

wherein -$X_1$-$X_2$- represents -$(CH_2)_{1-6}$-, -$(CH_2)_{1-5}O$-, -$CH_2O(CH_2)_{1-4}$-, -$(CH_2)_{1-4}OCH_2$-, -$(CH_2)_{1-5}NH$-, -$CH_2NH(CH_2)_{1-4}$-, or -$(CH_2)_{1-4}NHCH_2$-;

p1, p2, and p3 each independently represent an integer of 1, 2, 3 or 4; and

the ring containing -$X_1$-$X_2$- is optionally substituted with one or more (e.g., 1-20, 1-15, 1-10, 1-6, 1-4, 2-6, or 1) substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, $C_{2-6}$ alkenyl, and any combination thereof.

In some embodiments, p1, p2, and p3 each independently represent an integer of 1, 2 or 3.

In some embodiments, X represents :

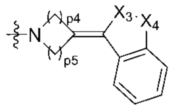

wherein $-X_3-X_4-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;

p4, p5 each independently represent an integer of 1, 2, 3 or 4; and

the benzo-fused ring containing$-X_3-X_4-$ is optionally substituted with one or more (e.g., 1-20, 1-15, 1-10, 1-6, 1-4, 2-6, or 1) substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, $C_{2-6}$ alkenyl or any combination thereof.

In some embodiments, p4 and p5 each independently represent an integer of 1, 2 or 3.

In some embodiments, X represents :

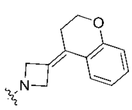 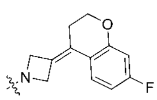 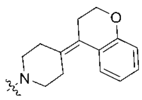 or 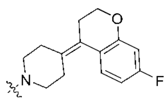.

In some embodiments, examples of the X include, but are not limited to:

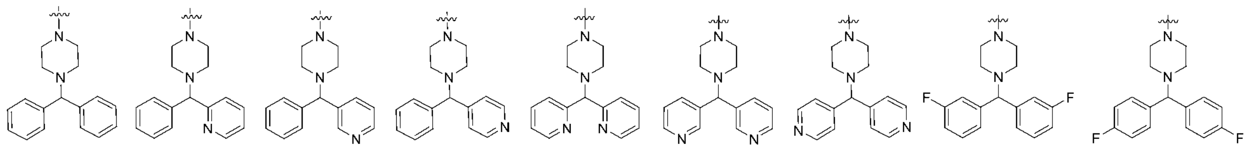

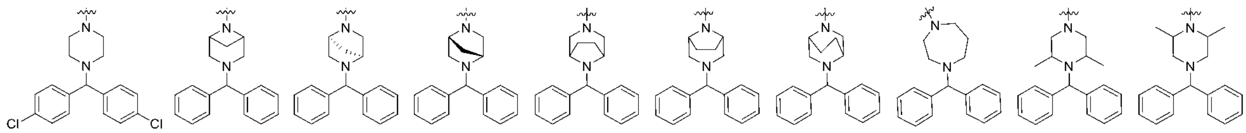

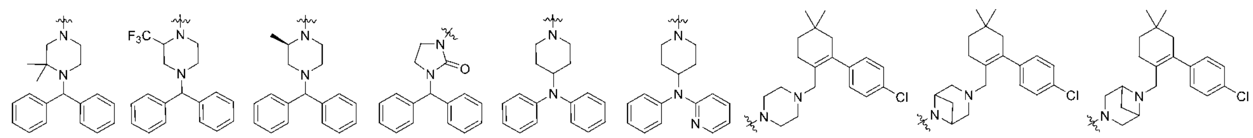

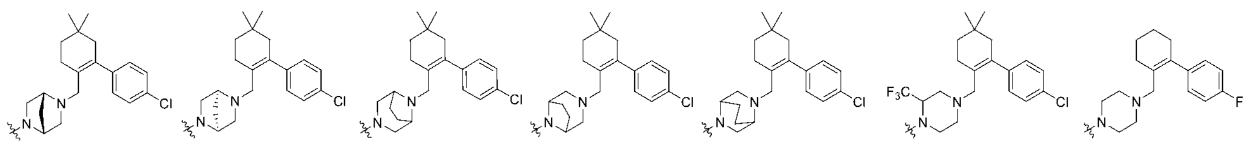

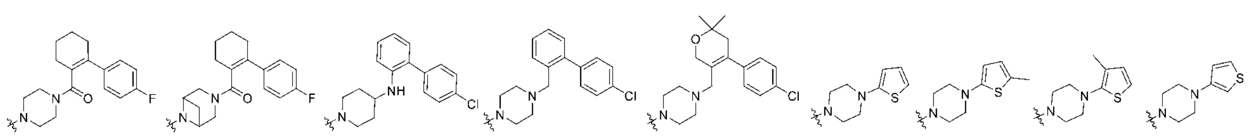

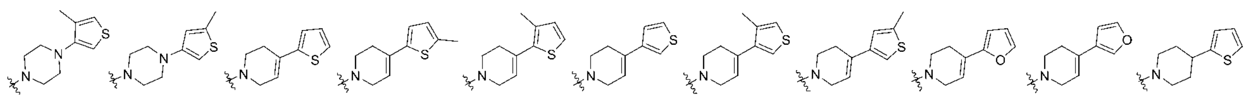

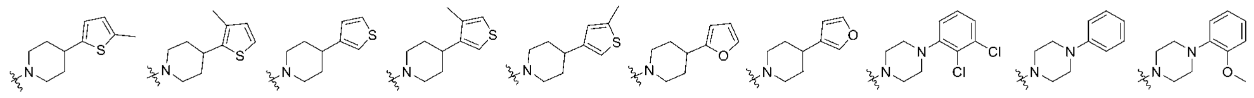

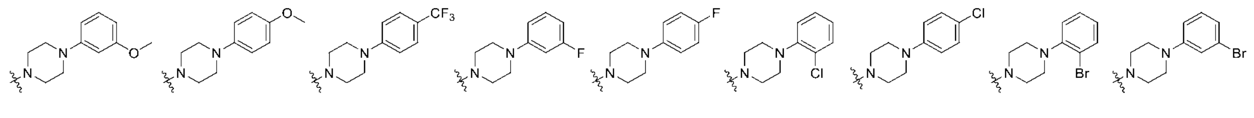

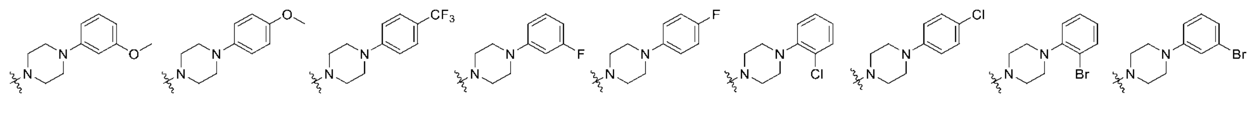

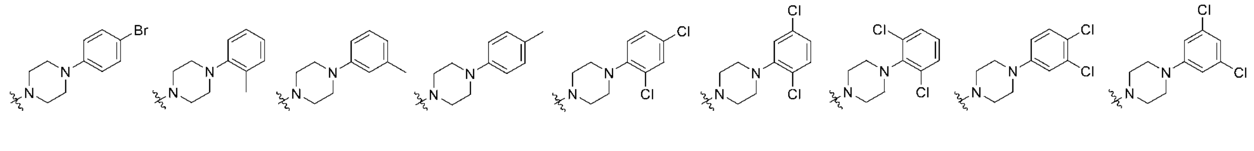

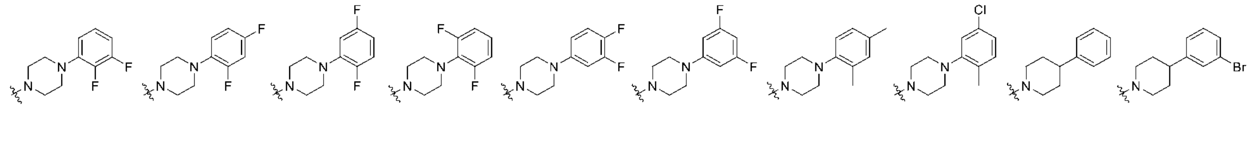

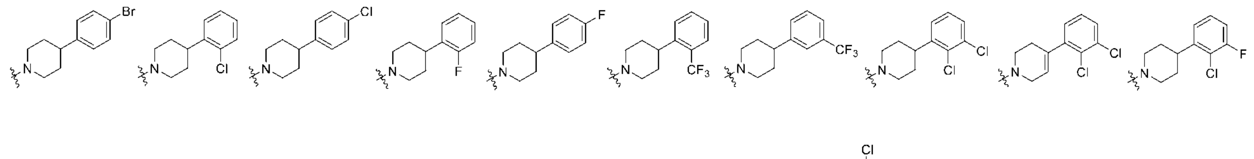

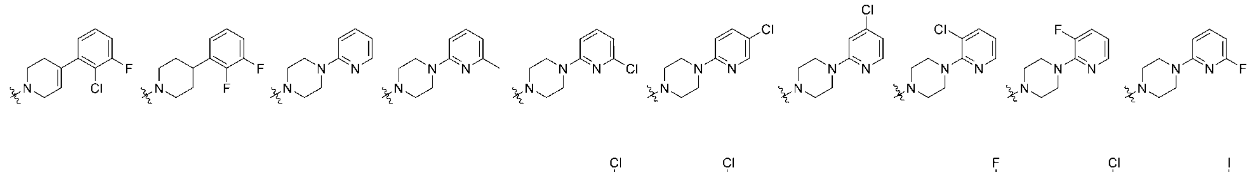

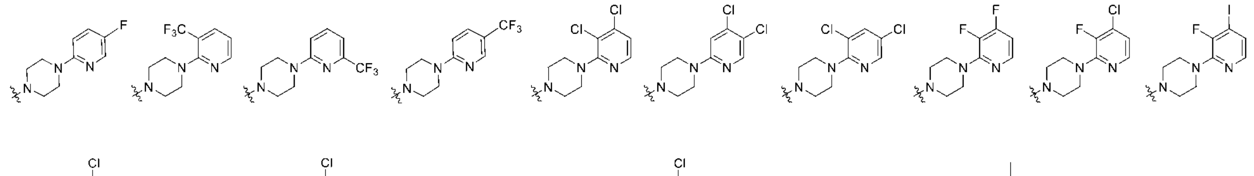

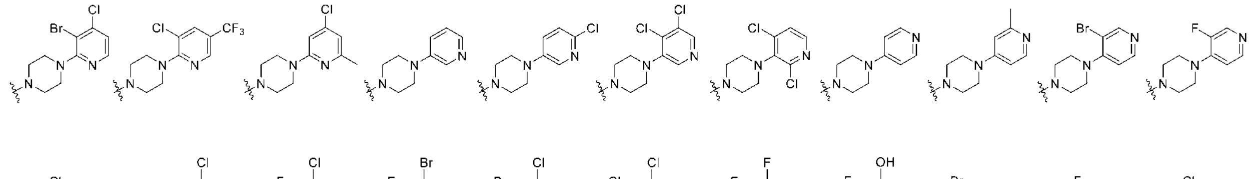

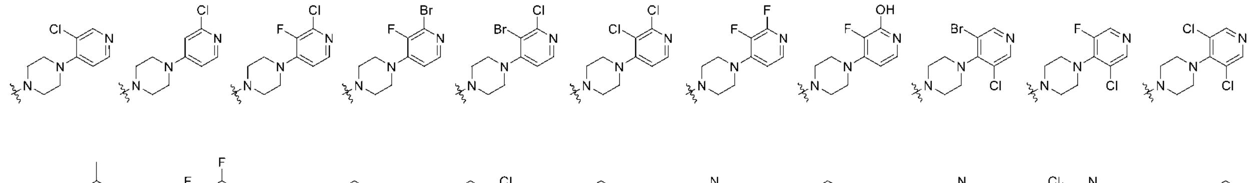

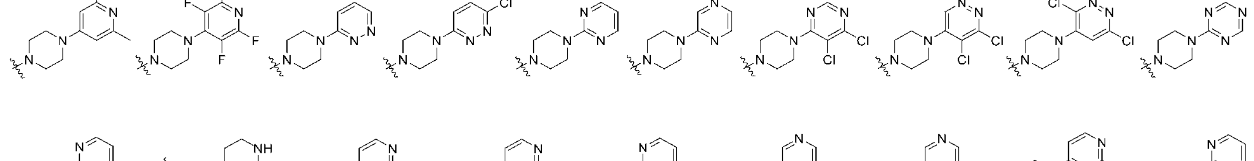

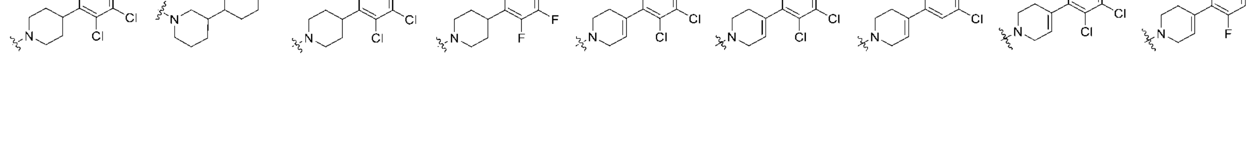

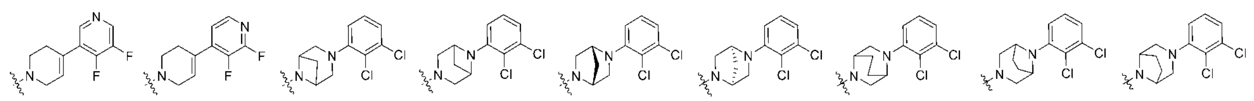

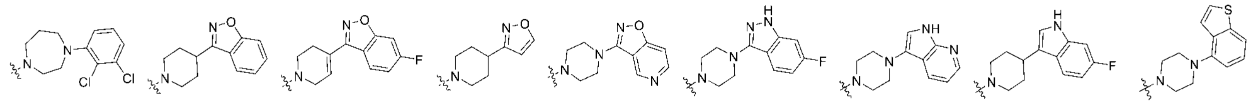

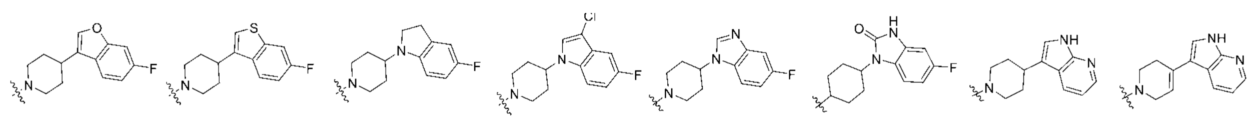

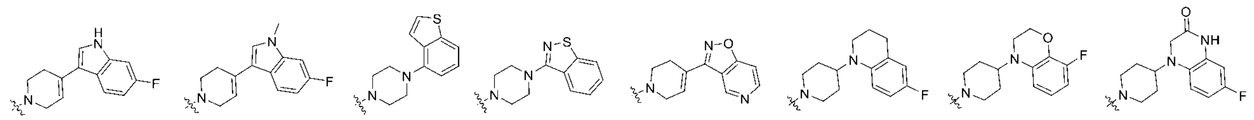

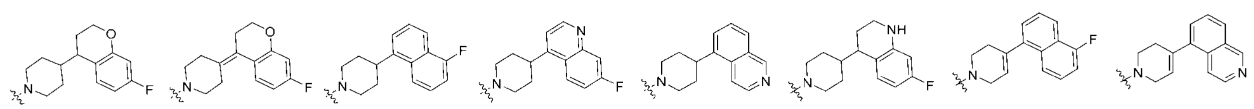

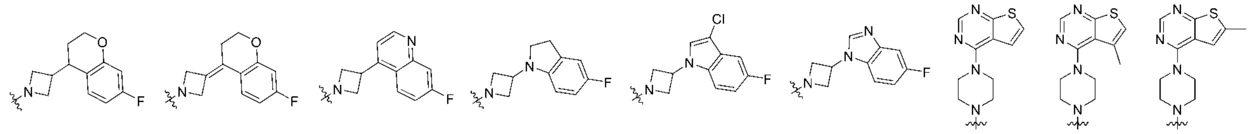

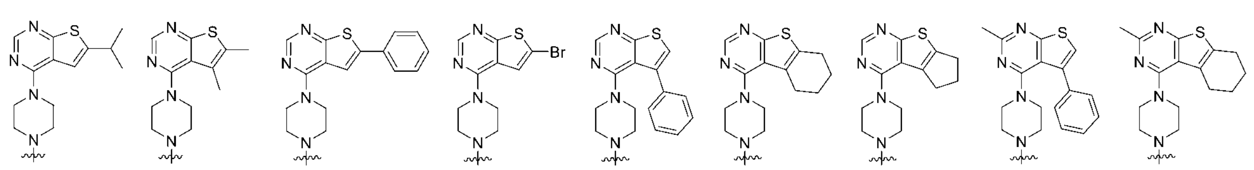

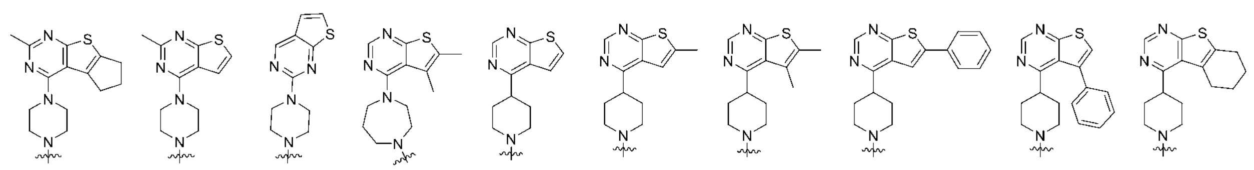

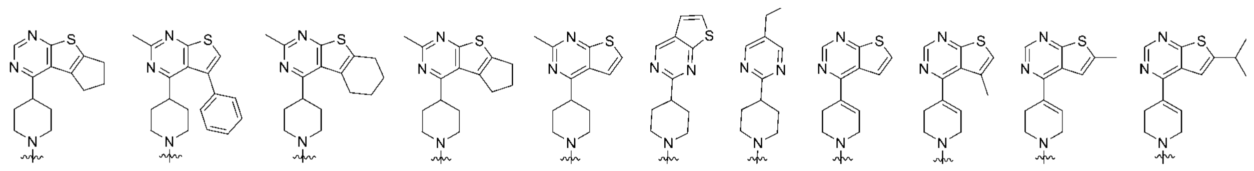

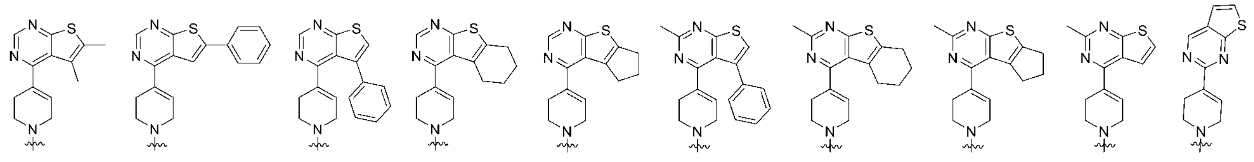

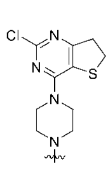 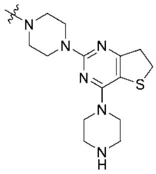 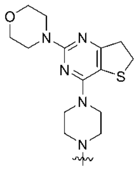 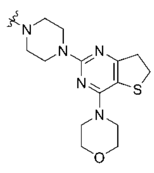 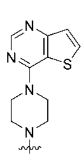 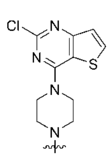 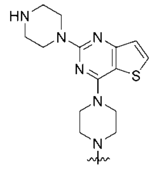 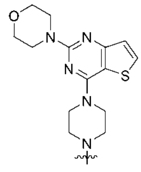 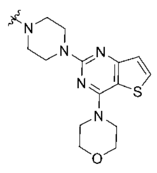

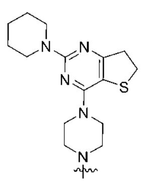 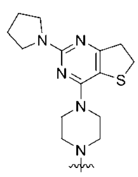 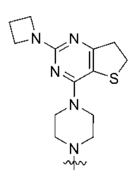 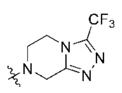 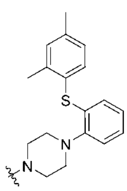 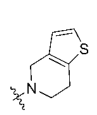 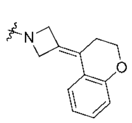 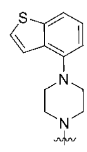 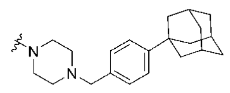

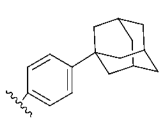 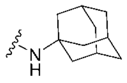 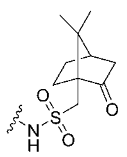 or 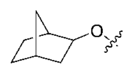 .

In some embodiments of the present disclosure, the compound of Formula (I) is also represented by Formula (I-1), Formula (I-2), Formula (I-3), or Formula (I-4):

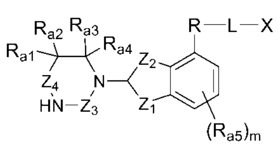
(I-1)

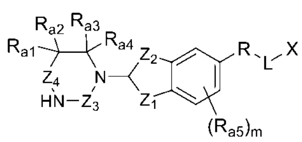
(I-2)

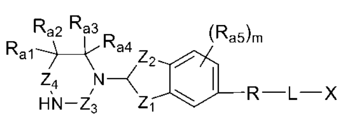
(I-3)

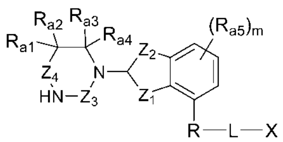
(I-4)

wherein the groups $R_{a1}$, $R_{a2}$, $R_{a3}$, $R_{a4}$, $Z_1$, $Z_2$, $Z_3$, $Z_4$, $(R_{a5})_m$, R, L, and X are as defined in the compound of Formula (I) and its various embodiments in the present disclosure.

In some embodiments of the present disclosure, the compound of Formula (I) is also represented by Formula (I-5):

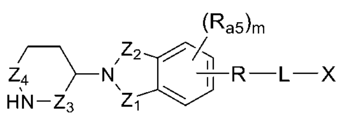
(I-5)

wherein $Z_1$, $Z_2$, $Z_3$, $Z_4$, $(R_{a5})_m$, R, L, and X are as defined in the compound of Formula (I) and its various embodiments in the present disclosure.

In some embodiments of the present disclosure, the compound of Formula (I-5) is also represented by Formula (I-5-1):

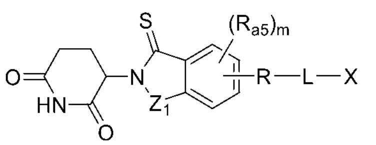
(I-5-1)

wherein $Z_1$, $(R_{a5})_m$, R, L, and X are as defined in the compound of Formula (I) and its various embodiments in the present disclosure.

In some embodiments of the present disclosure, the compound of Formula (I-5) is also represented by Formula (I-5-1A):

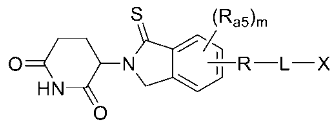

(I-5-1A)

wherein $(R_{a5})_m$, R, L, and X are as defined in the compound of Formula (I) and its various embodiments in the present disclosure.

In some embodiments of the present disclosure, the compound of Formula (I-5) is also represented by Formula (I-5-1B):

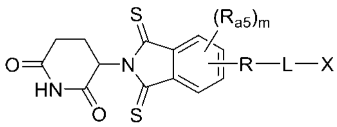

(I-5-1B)

wherein $(R_{a5})_m$, R, L, and X are as defined in the compound of Formula (I) and its various embodiments in the present disclosure.

In some embodiments of the present disclosure, the compound of Formula (I-5) is also represented by Formula (I-5-2):

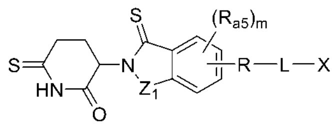

(I-5-2)

wherein $Z_1$, $(R_{a5})_m$, R, L, and X are as defined in the compound of Formula (I) and its various embodiments in the present disclosure.

In some embodiments of the present disclosure, the compound of Formula (I-5) is also represented by Formula (I-5-2A):

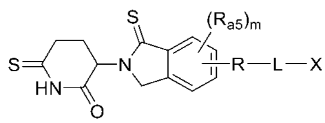

(I-5-2A)

wherein $(R_{a5})_m$, R, L, and X are as defined in the compound of Formula (I) and its various embodiments in the present disclosure.

In some embodiments of the present disclosure, the compound of Formula (I-5) is also represented by Formula (I-5-2B):

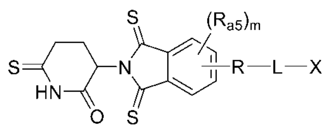

(I-5-2B)

wherein $(R_{a5})_m$, R, L, and X are as defined in the compound of Formula (I) and its various embodiments in the present disclosure.

Preferably the compounds of the present invention and their salts (especially pharmaceutically acceptable salts, such as hydrochloride, etc.), enantiomers, diastereomers, solvates, or polymorphs thereof in Table 1 below are provided:

Table 1. The compounds of the present invention

| Compound No. | Structure of the compounds | The compounds' name | Chinese translation of the compounds' name |
|---|---|---|---|
| GT-05104 | 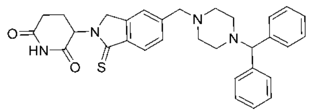 | 3-(5-((4-benzhydrylpiperazin-1-yl)methyl)-1-thioxoisoindolin-2-yl)piperidine-2,6-dione | 3-(5-((4-benzhydrylpiperazin-1-yl)methyl)-1-thioxoisoindolin-2-yl)piperidine-2,6-dione |
| GT-05103 | 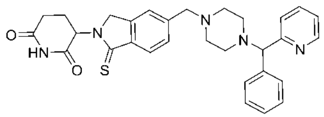 | 3-(5-((4-(phenyl(pyridin-2-yl)methyl)piperazin-1-yl) methyl)-1-thioxoisoindolin-2-yl)piperidine-2,6-dione | 3-(5-((4-(phenyl(pyridin-2-yl)methyl)piperazin-1-yl) methyl)-1-thioxoisoindolin-2-yl)piperidine-2,6-dione |
| GT-07732 | 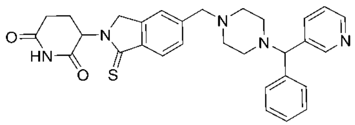 | 3-(5-((4-(phenyl(pyridin-3-yl)methyl)piperazin-1-yl) methyl)-1-thioxoisoindolin-2-yl)piperidine-2,6-dione | 3-(5-((4-(phenyl(pyridin-3-yl)methyl)piperazin-1-yl) methyl)-1-thioxoisoindolin-2-yl)piperidine-2,6-dione |
| GT-07733 | 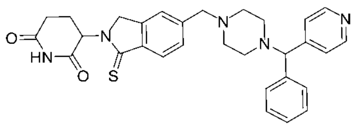 | 3-(5-((4-(phenyl(pyridin-4-yl)methyl)piperazin-1-yl) methyl)-1-thioxoisoindolin-2-yl)piperidine-2,6-dione | 3-(5-((4-(phenyl(pyridin-4-yl)methyl)piperazin-1-yl) methyl)-1-thioxoisoindolin-2-yl)piperidine-2,6-dione |
| GT-07734 | 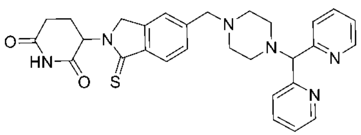 | 3-(5-((4-(di(pyridin-2-yl)methyl)piperazin-1-yl) methyl)-1-thioxoisoindolin-2-yl)piperidine-2,6-dione | 3-(5-((4-(di(pyridin-2-yl)methyl)piperazin-1-yl) methyl)-1-thioxoisoindolin-2-yl)piperidine-2,6-dione |
| GT-07735 | 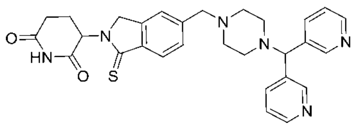 | 3-(5-((4-(di(pyridin-3-yl)methyl)piperazin-1-yl) methyl)-1-thioxoisoindolin-2-yl)piperidine-2,6-dione | 3-(5-((4-(di(pyridin-3-yl)methyl)piperazin-1-yl) methyl)-1-thioxoisoindolin-2-yl)piperidine-2,6-dione |
| | 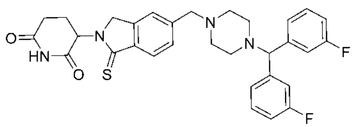 | 3-(5-((4-(bis(3-fluorophenyl)methyl)piperazi n-1-yl) methyl)-1-thioxoisoindolin-2-yl)piperidine-2,6-dione | 3-(5-((4-(bis(3-fluorophenyl)methyl)piperazi n-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-07736 | 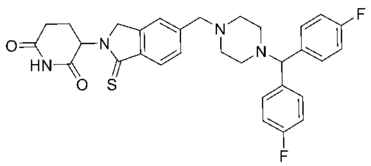 | 3-(5-((4-(bis(4-fluorophenyl)methyl)piperazi n-1-yl) methyl)-1-thioxoisoindolin-2-yl)piperidine-2,6-dione | 3-(5-((4-(bis(4-fluorophenyl)methyl)piperazi n-1-yl) methyl)-1-thioxoisoindolin-2-yl)piperidine-2,6-dione |
| GT-07737 | 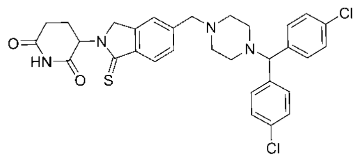 | 3-(5-((4-(bis(4-chlorophenyl)methyl)piperaz in-1-yl) methyl)-1-thioxoisoindolin-2-yl)piperidine-2,6-dione | 3-(5-((4-(bis(4-chlorophenyl)methyl)piperaz in-1-yl) methyl)-1-thioxoisoindolin-2-yl)piperidine-2,6-dione |
| GT-07738 | 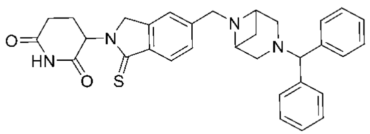 | 3-(5-((3-benzhydryl-3,6-diazabicyclo[3.1.1]heptan-6-yl) methyl)-1-thioxoisoindolin-2-yl)piperidine-2,6-dione | 3-(5-((3-benzhydryl-3,6-diazabicyclo[3.1.1]heptan-6-yl) methyl)-1-thioxoisoindolin-2-yl)piperidine-2,6-dione |
| | 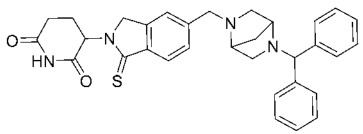 | 3-(5-((5-benzhydryl-2,5-diazabicyclo[2.2.1]heptan-2-yl) methyl)-1-thioxoisoindolin-2-yl)piperidine-2,6-dione | 3-(5-((5-benzhydryl-2,5-diazabicyclo[2.2.1]heptan-2-yl) methyl)-1-thioxoisoindolin-2-yl)piperidine-2,6-dione |
| GT-07740 | 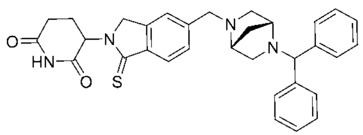 | 3-(5-(((1R,4R)-5-benzhydryl-2,5-diazabicyclo[2.2.1] heptan-2-yl)methyl)-1-thioxoisoindolin-2-yl)piperidine-2,6-dione | 3-(5-(((1R,4R)-5-benzhydryl-2,5-diazabicyclo[2.2.1] heptan-2-yl)methyl)-1-thioxoisoindolin-2-yl)piperidine-2,6-dione |
| GT-07739 | 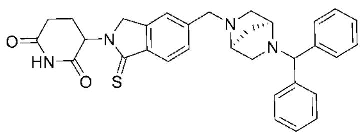 | 3-(5-(((1S,4S)-5-benzhydryl-2,5-diazabicyclo[2.2.1] heptan-2-yl)methyl)-1-thioxoisoindolin-2-yl)piperidine-2,6-dione | 3-(5-(((1S,4S)-5-benzhydryl-2,5-diazabicyclo[2.2.1] heptan-2-yl)methyl)-1-thioxoisoindolin-2-yl)piperidine-2,6-dione |
| GT-07849 | 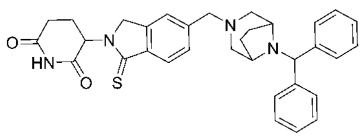 | 3-(5-((8-benzhydryl-3,8-diazabicyclo[3.2.1]octan-3-yl) methyl)-1-thioxoisoindolin-2-yl)piperidine-2,6-dione | 3-(5-((8-benzhydryl-3,8-diazabicyclo[3.2.1]octan-3-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-07850 | 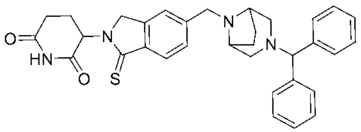 | 3-(5-((3-benzhydryl-3,8-diazabicyclo[3.2.1]octan-8-yl) methyl)-1-thioxoisoindolin-2-yl)piperidine-2,6-dione | 3-(5-((3-benzhydryl-3,8-diazabicyclo[3.2.1]octan-8-yl) methyl)-1-thioxoisoindolin-2-yl)piperidine-2,6-dione |
| GT-07847 | 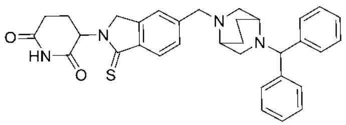 | 3-(5-((5-benzhydryl-2,5-diazabicyclo[2.2.2]octan-2-yl) methyl)-1-thioxoisoindolin-2-yl)piperidine-2,6-dione | 3-(5-((5-benzhydryl-2,5-diazabicyclo[2.2.2]octan-2-yl) methyl)-1-thioxoisoindolin-2-yl)piperidine-2,6-dione |
| GT-07848 | 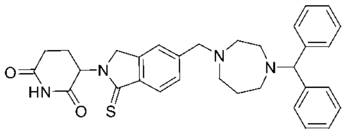 | 3-(5-((4-benzhydryl-1,4-diazepan-1-yl)methyl)-1-thioxoisoindolin-2-yl)piperidine-2,6-dione | 3-(5-((4-benzhydryl-1,4-diazepan-1-yl)methyl)-1-thioxoisoindolin-2-yl)piperidine-2,6-dione |
| | 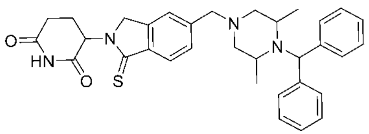 | 3-(5-((4-benzhydryl-3,5-dimethylpiperazin-1-yl) methyl)-1-thioxoisoindolin-2-yl)piperidine-2,6-dione | 3-(5-((4-benzhydryl-3,5-dimethylpiperazin-1-yl) methyl)-1-thioxoisoindolin-2-yl)piperidine-2,6-dione |
| | 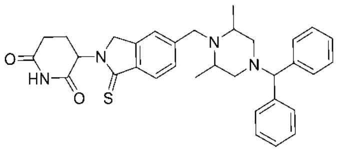 | 3-(5-((4-benzhydryl-2,6-dimethylpiperazin-1-yl) methyl)-1-thioxoisoindolin-2-yl)piperidine-2,6-dione | 3-(5-((4-benzhydryl-2,6-dimethylpiperazin-1-yl) methyl)-1-thioxoisoindolin-2-yl)piperidine-2,6-dione |
| GT-0784 5 | 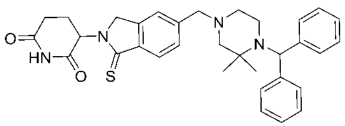 | 3-(5-((4-benzhydryl-3,3-dimethylpiperazin-1-yl) methyl)-1-thioxoisoindolin-2-yl)piperidine-2,6-dione | 3-(5-((4-benzhydryl-3,3-dimethylpiperazin-1-yl) methyl)-1-thioxoisoindolin-2-yl)piperidine-2,6-dione |
| | 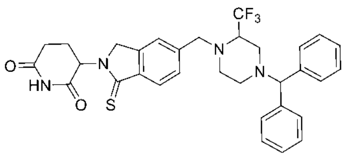 | 3-(5-((4-benzhydryl-2-(trifluoromethyl)piperazin-1-yl) methyl)-1-thioxoisoindolin-2-yl)piperidine-2,6-dione | 3-(5-((4-benzhydryl-2-(trifluoromethyl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 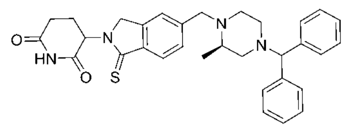 | 3-(5-(((R)-4-benzhydryl-2-methylpiperazin-1-yl) methyl)-1-thioxoisoindolin-2-yl)piperidine-2,6-dione | 3-(5-(((R)-4-benzhydryl-2-methylpiperazin-1-yl) methyl)-1-thioxoisoindolin-2-yl)piperidine-2,6-dione |
| | 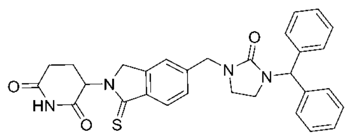 | 3-(5-((3-benzhydryl-2-oxoimidazolidin-1-yl)methyl)-1-thioxoisoindolin-2-yl)piperidine-2,6-dione | 3-(5-((3-benzhydryl-2-oxoimidazolidin-1-yl)methyl)-1-thioxoisoindolin-2-yl)piperidine-2,6-dione |
| GT-05106 | 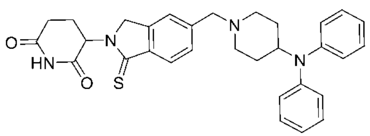 | 3-(5-((4-(diphenylamino)piperidin-1-yl)methyl)-1-thioxoisoindolin-2-yl)piperidine-2,6-dione | 3-(5-((4-(diphenylamino)piperidin-1-yl)methyl)-1-thioxoisoindolin-2-yl)piperidine-2,6-dione |
| GT-07846 | 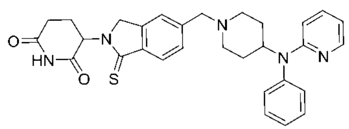 | 3-(5-((4-(phenyl(pyridin-2-yl)amino)piperidin-1-yl) methyl)-1-thioxoisoindolin-2-yl)piperidine-2,6-dione | 3-(5-((4-(phenyl(pyridin-2-yl)amino)piperidin-1-yl) methyl)-1-thioxoisoindolin-2-yl)piperidine-2,6-dione |
| GT-05151 | 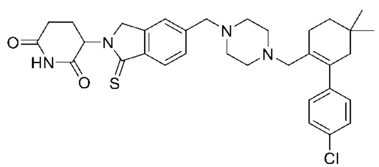 | 3-(5-((4-((4'-chloro-5,5-dimethyl-3,4,5,6-tetrahydro-[1,1'-biphenyl]-2-yl)methyl)piperazin-1-yl) methyl)-1-thioxoisoindolin-2-yl)piperidine-2,6-dione | 3-(5-((4-((4'-chloro-5,5-dimethyl-3,4,5,6-tetrahydro-[1,1'-biphenyl]-2-yl)methyl)piperazin-1-yl) methyl)-1-thioxoisoindolin-2-yl)piperidine-2,6-dione |
| GT-08160 | 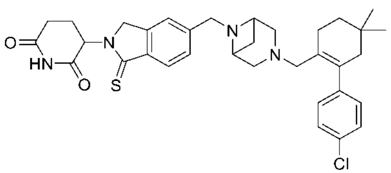 | 3-(5-((3-((4'-chloro-5,5-dimethyl-3,4,5,6-tetrahydro-[1,1-biphenyl]-2-yl)methyl)-3,6-diazabicyclo[3.1.1] heptan-6-yl)methyl)-1-thioxoisoindolin-2-yl)piperidine-2,6-dione | 3-(5-((3-((4'-chloro-5,5-dimethyl-3,4,5,6-tetrahydro-[1,1'-biphenyl]-2-yl)methyl)-3,6-diazabicyclo[3.1.1] heptan-6-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08161 | 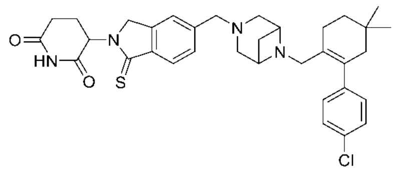 | 3-(5-((6-((4'-chloro-5,5-dimethyl-3,4,5,6-tetrahydro-[1,1-biphenyl]-2-yl)methyl)-3,6-diazabicyclo[3.1.1]heptan-3-yl)methyl)-1-thioxoisoindolin-2-yl)piperidine-2,6-dione | 3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-thioxoisoindolin-2-y)piperidine-2,6-dione |
| | 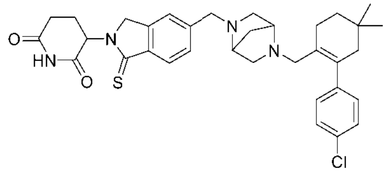 | 3-(5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-thioxoisoindolin-2-yl)piperidine-2,6-dione | 3-(5-((5-((4'-chloro-5,5-dimethyl-3,4,5,6-tetrahydro-[1,1-biphenyl]-2-yl)methyl)-2,5-diazabicyclo[2.2.1]heptan-2-yl)methyl)-1-thioxoisoindolin-2-yl)piperidine-2,6-dione |
| GT-0816 2 | 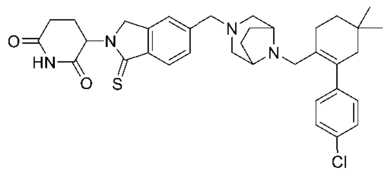 | 3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-thioxoisoindolin-2-yl)piperidine-2,6-dione | 3-(5-((8-((4'-chloro-5,5-dimethyl-3,4,5,6-tetrahydro-[1,1-biphenyl]-2-yl)methyl)-3,8-diazabicyclo[3.2.1]octan-3-yl)methyl)-1-thioxoisoindolin-2-yl)piperidine-2,6-dione |
| GT-08163 | 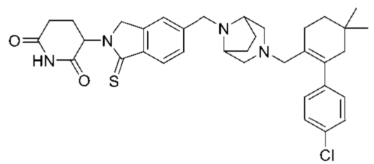 | 3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-thioxoisoindolin-2-yl)piperidine-2,6-dione | 3-(5-((3-((4'-chloro-5,5-dimethyl-3,4,5,6-tetrahydro-[1,1-biphenyl]-2-yl)methyl)-3,8-diazabicyclo[3.2.1]octan-8-yl)methyl)-1-thioxoisoindolin-2-yl)piperidine-2,6-dione |
| | 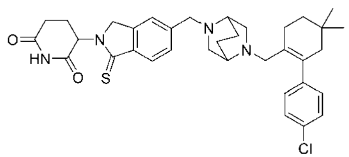 | 3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-thioxoisoindolin-2-yl)piperidine-2,6-dione | 3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 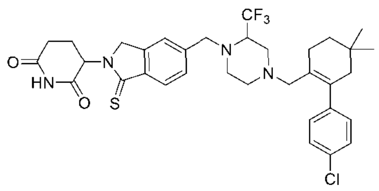 | 3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-1-thioxoisoindolin-2-yl)piperidine-2,6-dione | 3-(5-((4-((4'-chloro-5,5-dimethyl-3,4,5,6-tetrahydro-[1,1-biphenyl]-2-yl)methyl)-2-(trifluoromethyl)piperazin-1-yl)methyl)-1-thioxoisoindolin-2-yl)piperidine-2,6-dione |
| GT-08164 | 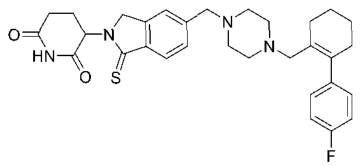 | 3-(5-((4-((4'-fluoro-3,4,5,6-tetrahydro-[1,1'-biphenyl]-2-yl)methyl)piperazin-1-yl)methyl)-1-thioxoisoindolin-2-yl)piperidine-2,6-dione | 3-(5-((4-((4'-fluoro-3,4,5,6-tetrahydro-[1,1'-biphenyl]-2-yl)methyl)piperazin-1-yl)methyl)-1-thioxoisoindolin-2-yl)piperidine-2,6-dione |
| GT-08165 | 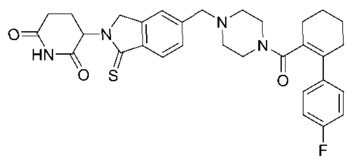 | 3-(5-((4-(4'-fluoro-3,4,5,6-tetrahydro-[1,1'-biphenyl]-2-carbonyl)piperazin-1-yl)methyl)-1-thioxoisoindolin-2-yl) piperidine-2,6-dione | 3-(5-((4-(4'-fluoro-3,4,5,6-tetrahydro-[1,1'-biphenyl]-2-carbonyl)piperazin-1-yl)methyl)-1-thioxoisoindolin-2-yl) piperidine-2,6-dione |
| | 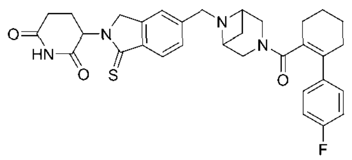 | 3-(5-((3-(4'-fluoro-3,4,5,6-tetrahydro-[1,1'-biphenyl]-2-carbonyl)-3,6-diazabicyclo[3.1.1]heptan-6-yl) methyl)-1-thioxoisoindolin-2-yl)piperidine-2,6-dione | 3-(5-((3-(4'-fluoro-3,4,5,6-tetrahydro-[1,1'-biphenyl]-2-carbonyl)-3,6-diazabicyclo[3.1.1]heptan-6-yl) methyl)-1-thioxoisoindolin-2-yl)piperidine-2,6-dione |
| GT-05109 | 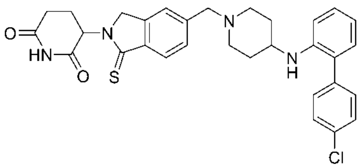 | 3-(5-((4-((4'-chloro-[1,1'-biphenyl]-2-yl)amino)piperidin-1-yl)methyl)-1-thioxoisoindolin-2-yl)piperidine-2,6-dione | 3-(5-((4-((4'-chloro-[1,1'-biphenyl]-2-yl)amino)piperidin-1-yl)methyl)-1-thioxoisoindolin-2-yl)piperidine-2,6-dione |
| GT-08166 | 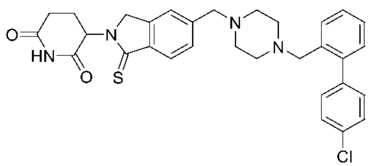 | 3-(5-((4-((4'-chloro-[1,1'-biphenyl]-2-yl)methyl)piperazin-1-yl)methyl)-1-thioxoisoindolin-2-yl)piperidine-2,6-dione | 3-(5-((4-((4'-chloro-[1,1'-biphenyl]-2-yl)methyl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08167 | 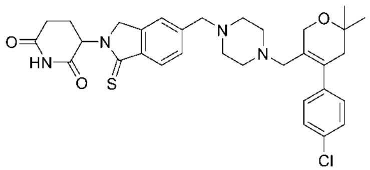 | 3-(5-((4-((4-(4-chlorophenyl)-6,6-dimethyl-5,6-dihydro-2H-pyran-3-yl)methyl)piperazin-1-yl)methyl)-1-thioxoisoindolin-2-yl)piperidine-2,6-dione | 3-(5-((4-((4-(4-chlorophenyl)-6,6-dimethyl-5,6-dihydro-2H-pyran-3-yl)methyl)piperazin-1-yl)methyl)-1-thioxoisoindolin-2-yl)piperidine-2,6-dione |
| GT-08168 | 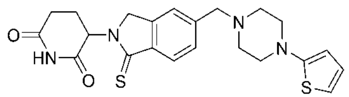 | 3-(5-((4-(thiophen-2-yl)piperazin-1-yl)methyl)-1-thioxoisoindolin-2-yl)piperidine-2,6-dione | 3-(5-((4-(thiophen-2-yl)piperazin-1-yl)methyl)-1-thioxoisoindolin-2-vl)piperidine-2,6-dione |
| GT-05110 | 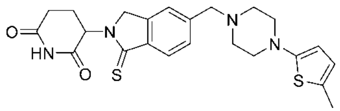 | 3-(5-((4-(5-methylthiophen-2-yl)piperazin-1-yl)methyl)-1-thioxoisoindolin-2-yl)piperidine-2,6-dione | 3-(5-((4-(5-methylthiophen-2-yl)piperazin-1-yl)methyl)-1-thioxoisoindolin-2-yl)piperidine-2,6-dione |
| | 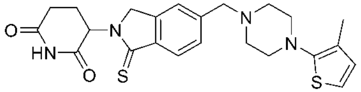 | 3-(5-((4-(3-methylthiophen-2-yl)piperazin-1-yl)methyl)-1-thioxoisoindolin-2-yl)piperidine-2,6-dione | 3-(5-((4-(3-methylthiophen-2-yl)piperazin-1-yl)methyl)-1-thioxoisoindolin-2-yl)piperidine-2,6-dione |
| GT-08169 | 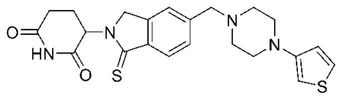 | 3-(5-((4-(thiophen-3-yl)piperazin-1-yl)methyl)-1-thioxoisoindolin-2-yl)piperidine-2,6-dione | 3-(5-((4-(thiophen-3-yl)piperazin-1-yl)methyl)-1-thioxoisoindolin-2-yl)piperidine-2,6-dione |
| GT-05113 | 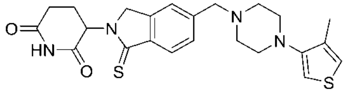 | 3-(5-((4-(4-methylthiophen-3-yl)piperazin-1-yl)methyl)-1-thioxoisoindolin-2-yl)piperidine-2,6-dione | 3-(5-((4-(4-methylthiophen-3-yl)piperazin-1-yl)methyl)-1-thioxoisoindolin-2-yl)piperidine-2,6-dione |
| GT-08170 | 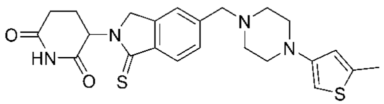 | 3-(5-((4-(5-methylthiophen-3-yl)piperazin-1-yl)methyl)-1-thioxoisoindolin-2-yl)piperidine-2,6-dione | 3-(5-((4-(5-methylthiophen-3-yl)piperazin-1-yl)methyl)-1-thioxoisoindolin-2-yl)piperidine-2,6-dione |
| GT-08171 | 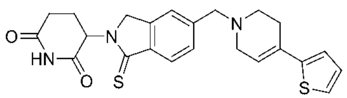 | 3-(5-((4-(thiophen-2-yl)-3,6-dihydropyridin-1 (2H)-yl)methyl)-1-thioxoisoindolin-2-yl)piperidine-2,6-dione | 3-(5-((4-(thiophen-2-yl)-3,6-dihydropyridin-1(2H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-05111 | 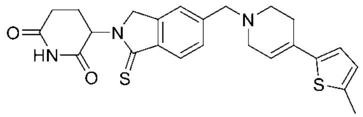 | 3-(5-((4-(5-methylthiophen-2-yl)-3,6-dihydropyridin-1(2H)-yl)methyl)-1-thioxoisoindolin-2-yl)piperidine-2,6-dione | 3-(5-((4-(5-methylthiophen-2-yl)-3,6-dihydropyridin-1(2H)-yl)methyl)-1-thioxoisoindolin-2-yl)piperidine-2,6-dione |
| | 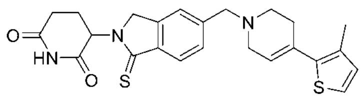 | 3-(5-((4-(3-methylthiophen-2-yl)-3,6-dihydropyridin-1(2H)-yl)methyl)-1-thioxoisoindolin-2-yl)piperidine-2,6-dione | 3-(5-((4-(3-methylthiophen-2-yl)-3,6-dihydropyridin-1(2H)-yl)methyl)-1-thioxoisoindolin-2-yl)piperidine-2,6-dione |
| GT-08172 | 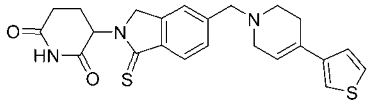 | 3-(5-((4-(thiophen-3-yl)-3,6-dihydropyridin-1 (2H)-yl) methyl)-1-thioxoisoindolin-2-yl)piperidine-2,6-dione | 3- 5-((4-(thiophen-3-yl)-3,6-dihydropyridin-1(2H)-yl) methyl)-1-thioxoisoindolin-2-yl)piperidine-2,6-dione |
| GT-05145 | 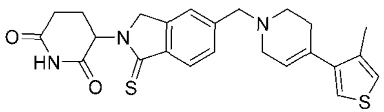 | 3-(5-((4-(4-methylthiophen-3-yl)-3,6-dihydropyridin-1(2H)-yl)methyl)-1-thioxoisoindolin-2-yl)piperidine-2,6-dione | 3-(5-((4-(4-methylthiophen-3-yl)-3,6-dihydropyridin-1(2H)-yl)methyl)-1-thioxoisoindolin-2-yl)piperidine-2,6-dione |
| GT-08327 | 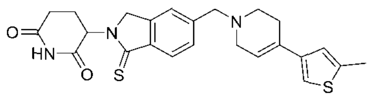 | 3-(5-((4-(5-methylthiophen-3-yl)-3,6-dihydropyridin-1(2H)-yl)methyl)-1-thioxoisoindolin-2-yl)piperidine-2,6-dione | 3-(5-((4-(5-methylthiophen-3-yl)-3,6-dihydropyridin-1(2H)-yl)methyl)-1-thioxoisoindolin-2-yl)piperidine-2,6-dione |
| GT-05147 | 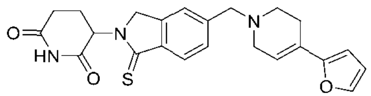 | 3-(5-((4-(furan-2-yl)-3,6-dihydropyridin-1 (2H)-yl) methyl)-1-thioxoisoindolin-2-yl)piperidine-2,6-dione | 3-(5-((4-(furan-2-yl)-3,6-dihydropyridin-1(2H)-yl) methyl)-1-thioxoisoindolin-2-yl)piperidine-2,6-dione |
| GT-08328 | 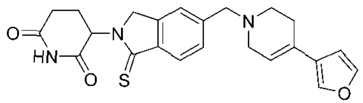 | 3-(5-((4-(furan-3-yl)-3,6-dihydropyridin-1 (2H)-yl) methyl)-1-thioxoisoindolin-2-yl)piperidine-2,6-dione | 3-(5-((4-(furan-3-yl)-3,6-dihydropyridin-1(2H)-yl) methyl)-1-thioxoisoindolin-2-yl)piperidine-2,6-dione |
| GT-08329 | 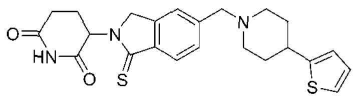 | 3-5-((4-(thiophen-2-yl)piperidin-1-yl)methyl)-1-thioxoisoindolin-2-yl)piperidine-2,6-dione | 3-(5-((4-(thiophen-2-yl)piperidin-1-yl)methyl)-1-thioxoisoindolin-2-yl)piperidine-2,6-dione |
| GT-05112 | 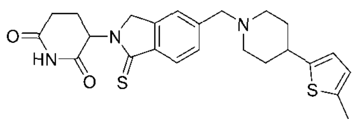 | 3-(5-((4-(5-methylthiophen-2-yl)piperidin-1-yl) methyl)-1-thioxoisoindolin-2-yl)piperidine-2,6-dione | 3-(5-((4-(5-methylthiophen-2-yl)piperidin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08323 | 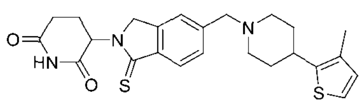 | 3-(5-((4-(3-methylthiophen-2-yl)piperidin-1-yl) methyl)-1-thioxoisoindolin-2-yl)piperidine-2,6-dione | 3-(5-((4-(3-methylthiophen-2-yl)piperidin-1-yl) methyl)-1-thioxoisoindolin-2-yl)piperidine-2,6-dione |
| GT-08324 | 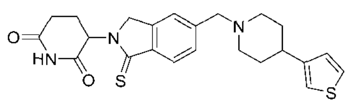 | 3-(5-((4-(thiophen-3-yl)piperidin-1-yl)methyl)-1-thioxoisoindolin-2-yl)piperidine-2,6-dione | 3-(5-((4-(thiophen-3-yl)piperidin-1-yl)methyl)-1-thioxoisoindolin-2-yl)piperidine-2,6-dione |
| GT-08325 | 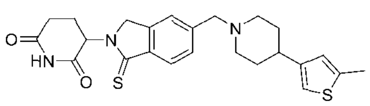 | 3-(5-((4-(5-methylthiophen-3-yl)piperidin-1-yl) methyl)-1-thioxoisoindolin-2-yl)piperidine-2,6-dione | 3-(5-((4-(5-methylthiophen-3-yl)piperidin-1-yl) methyl)-1-thioxoisoindolin-2-yl)piperidine-2,6-dione |
| GT-05146 | 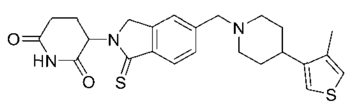 | 3-(5-((4-(4-methylthiophen-3-yl)piperidin-1-yl) methyl)-1-thioxoisoindolin-2-yl)piperidine-2,6-dione | 3-(5-((4-(4-methylthiophen-3-yl)piperidin-1-yl) methyl)-1-thioxoisoindolin-2-yl)piperidine-2,6-dione |
| GT-05148 | 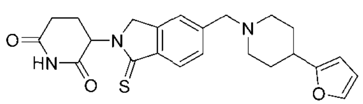 | 3-(5-((4-(furan-2-yl)piperidin-1-yl)methyl)-1-thioxoisoindolin-2-yl)piperidine-2,6-dione | 3-(5-((4-(furan-2-yl)piperidin-1-yl)methyl)-1-thioxoisoindolin-2-yl)piperidine-2,6-dione |
| GT-08326 | 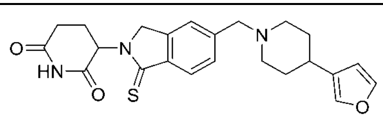 | 3-(5-((4-(furan-3-yl)piperidin-1-yl)methyl)-1-thioxoisoindolin-2-yl)piperidine-2,6-dione | 3-(5-((4-(furan-3-yl)piperidin-1-yl)methyl)-1-thioxoisoindolin-2-yl)piperidine-2,6-dione |
| GT-04474 | 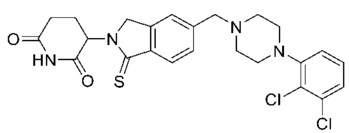 | 3-(5-((4-(2,3-dichlorophenyl)piperazin-1-yl)methyl)-1-thioxoisoindolin-2-yl)piperidine-2,6-dione | 3-(5-((4-(2,3-dichlorophenyl)piperazin-1-yl)methyl)-1-thioxoisoindolin-2-yl)piperidine-2,6-dione |
| GT-08205 | 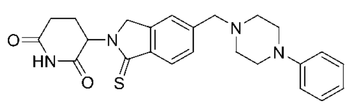 | 3-(5-((4-phenylpiperazin-1-yl)methyl)-1-thioxoisoindolin-2-yl)piperidine-2,6-dione | 3-(5-((4-phenylpiperazin-1-yl)methyl)-1-thioxoisoindolin-2-yl)piperidine-2,6-dione |
| GT-08236 | 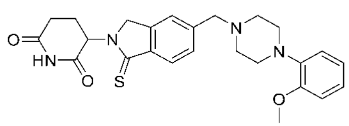 | 3-(5-((4-(2-methoxyphenyl)piperazin-1-yl)methyl)-1-thioxoisoindolin-2-yl)piperidine-2,6-dione | 3-(5-((4-(2-methoxyphenyl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08237 | 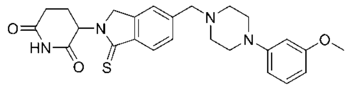 | 3-(5-((4-(3-methoxyphenyl)piperazin-1-yl)methyl)-1-thioxoisoindolin-2-yl)piperidine-2,6-dione | 3-(5-((4-(3-methoxyphenyl)piperazin-1-yl)methyl)-1-thioxoisoindolin-2-yl)piperidine-2,6-dione |
| | 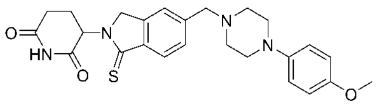 | 3-(5-((4-(4-methoxyphenyl)piperazin-1-yl)methyl)-1-thioxoisoindolin-2-yl)piperidine-2,6-dione | 3-(5-((4-(4-methoxyphenyl)piperazin-1-yl)methyl)-1-thioxoisoindolin-2-yl)piperidine-2,6-dione |
| GT-08239 | 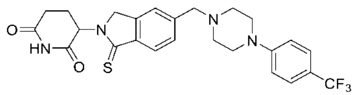 | 3-(1-thioxo-5-((4-(4-(trifluoromethyl)phenyl)piper azin-1-yl)methyl)isoindolin-2-yl)piperidine-2,6-dione | 3-(1-thioxo-5-((4-(4-(trifluoromethyl)phenyl)piper azin-1-yl)methyl)isoindolin-2-yl)piperidine-2,6-dione |
| | 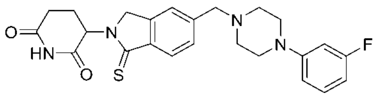 | 3-(5-((4-(3-fluorophenyl)piperazin-1-yl)methyl)-1-thioxoisoindolin-2-yl)piperidine-2,6-dione | 3-(5-((4-(3-fluorophenyl)piperazin-1-yl)methyl)-1-thioxoisoindolin-2-yl)piperidine-2,6-dione |
| GT-08240 | 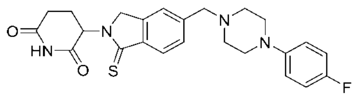 | 3-(5-((4-(4-fluorophenyl)piperazin-1-yl)methyl)-1-thioxoisoindolin-2-yl)piperidine-2,6-dione | 3-(5-((4-(4-fluorophenyl)piperazin-1-yl)methyl)-1-thioxoisoindolin-2-yl)piperidine-2,6-dione |
| GT-08241 | 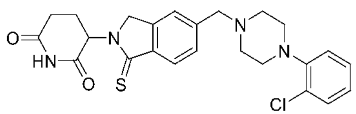 | 3-(5-((4-(2-chlorophenyl)piperazin-1-yl)methyl)-1-thioxoisoindolin-2-yl)piperidine-2,6-dione | 3-(5-((4-(2-chlorophenyl)piperazin-1-yl)methyl)-1-thioxoisoindolin-2-yl)piperidine-2,6-dione |
| | 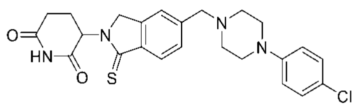 | 3-(5-((4-(4-chlorophenyl)piperazin-1-yl)methyl)-1-thioxoisoindolin-2-yl)piperidine-2,6-dione | 3-(5-((4-(4-chlorophenyl)piperazin-1-yl)methyl)-1-thioxoisoindolin-2-yl)piperidine-2,6-dione |
| GT-08242 | 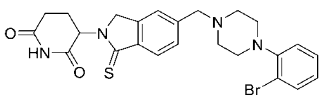 | 3-(5-((4-(2-bromophenyl)piperazin-1-yl)methyl)-1-thioxoisoindolin-2-yl)piperidine-2,6-dione | 3-(5-((4-(2-bromophenyl)piperazin-1-yl)methyl)-1-thioxoisoindolin-2-yl)piperidine-2,6-dione |
| GT-08238 | 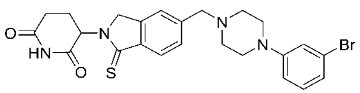 | 3-(5-((4-(3-bromophenyl)piperazin-1-yl)methyl)-1-thioxoisoindolin-2-yl)piperidine-2,6-dione | 3-(5-((4-(3-bromophenyl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08600 | 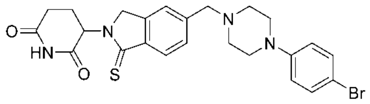 | 3-(5-((4-(4-bromophenyl)piperazin-1-yl)methyl)-1-thioxoisoindolin-2-yl)piperidine-2,6-dione | 3-(5-((4-(4-bromophenyl)piperazin-1-yl)methyl)-1-thioxoisoindolin-2-yl)piperidine-2,6-dione |
| GT-08601 | 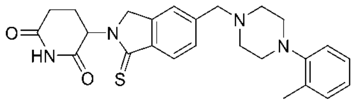 | 3- 1-thioxo-5-((4-(o-tolyl)piperazin-1-yl)methyl)isoindolin-2-yl)piperidine-2,6-dione | 3-(1-thioxo-5-((4-(o-tolyl)piperazin-1-yl)methyl)isoindolin-2-yl)piperidine-2,6-dione |
| GT-0860 2 | 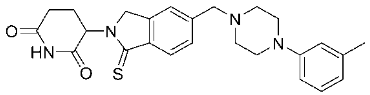 | 3-(1-thioxo-5-((4-(m-tolyl)piperazin-1-yl)methyl)isoindolin-2-yl)piperidine-2,6-dione | 3-(1-thioxo-5-((4-(m-tolyl)piperazin-1-yl)methyl)isoindolin-2-yl)piperidine-2,6-dione |
| GT-0860 3 | 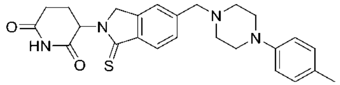 | 3-(1-thioxo-5-((4-(p-tolyl)piperazin-1-yl)methyl)isoindolin-2-yl)piperidine-2,6-dione | 3-(1-thioxo-5-((4-(p-tolyl)piperazin-1-yl)methyl)isoindolin-2-yl)piperidine-2,6-dione |
| GT-0860 4 | 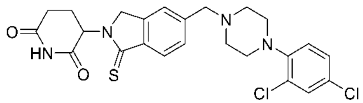 | 3-(5-((4-(2,4-dichlorophenyl)piperazin-1-yl)methyl)-1-thioxoisoindolin-2-yl)piperidine-2,6-dione | 3-(5-((4-(2,4-dichlorophenyl)piperazin-1-yl)methyl)-1-thioxoisoindolin-2-yl)piperidine-2,6-dione |
| GT-08611 | 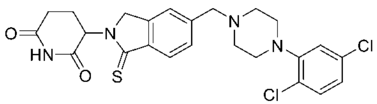 | 3-(5-((4-(2,5-dichlorophenyl)piperazin-1-yl)methyl)-1-thioxoisoindolin-2-yl)piperidine-2,6-dione | 3-(5-((4-(2,5-dichlorophenyl)piperazin-1-yl)methyl)-1-thioxoisoindolin-2-vl)piperidine-2,6-dione |
|  | 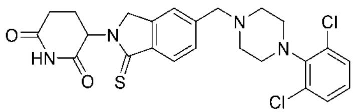 | 3-(5-((4-(2,6-dichlorophenyl)piperazin-1-yl)methyl)-1-thioxoisoindolin-2-yl)piperidine-2,6-dione | 3-(5-((4-(2,6-dichlorophenyl)piperazin-1-yl)methyl)-1-thioxoisoindolin-2-yl)piperidine-2,6-dione |
| GT-08612 | 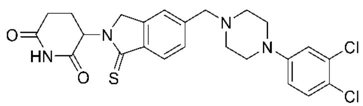 | 3-(5-((4-(3,4-dichlorophenyl)piperazin-1-yl)methyl)-1-thioxoisoindolin-2-yl)piperidine-2,6-dione | 3-(5-((4-(3,4-dichlorophenyl)piperazin-1-yl)methyl)-1-thioxoisoindolin-2-yl)piperidine-2,6-dione |
| GT-08624 | 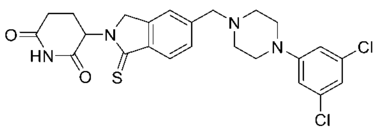 | 3-(5-((4-(3,5-dichlorophenyl)piperazin-1-yl)methyl)-1-thioxoisoindolin-2-yl)piperidine-2,6-dione | 3-(5-((4-(3,5-dichlorophenyl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08210 | 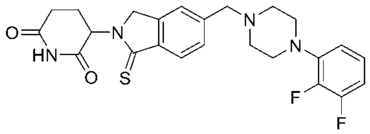 | 3-(5-((4-(2,3-difluorophenyl)piperazin-1-yl)methyl)-1-thioxoisoindolin-2-yl)piperidine-2,6-dione | 3-(5-((4-(2,3-difluorophenyl)piperazin-1-yl)methyl)-1-thioxoisoindolin-2-yl)piperidine-2,6-dione |
| GT-08613 | 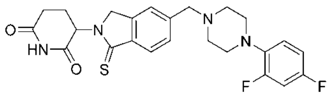 | 3-(5-((4-(2,4-difluorophenyl)piperazin-1-yl)methyl)-1-thioxoisoindolin-2-yl)piperidine-2,6-dione | 3-(5-((4-(2,4-difluorophenyl)piperazin-1-yl)methyl)-1-thioxoisoindolin-2-yl)piperidine-2,6-dione |
| GT-08625 | 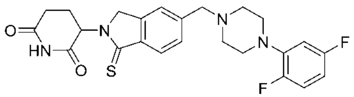 | 3-(5-((4-(2,5-difluorophenyl)piperazin-1-yl)methyl)-1-thioxoisoindolin-2-yl)piperidine-2,6-dione | 3-(5-((4-(2,5-difluorophenyl)piperazin-1-yl)methyl)-1-thioxoisoindolin-2-yl)piperidine-2,6-dione |
| GT-08626 | 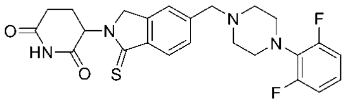 | 3-(5-((4-(2,6-difluorophenyl)piperazin-1-yl)methyl)-1-thioxoisoindolin-2-yl)piperidine-2,6-dione | 3-(5-((4-(2,6-difluorophenyl)piperazin-1-yl)methyl)-1-thioxoisoindolin-2-yl)piperidine-2,6-dione |
| GT-08627 | 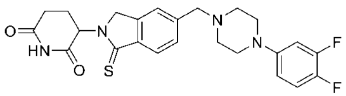 | 3-(5-((4-(3,4-difluorophenyl)piperazin-1-yl)methyl)-1-thioxoisoindolin-2-yl)piperidine-2,6-dione | 3-(5-((4-(3,4-difluorophenyl)piperazin-1-yl)methyl)-1-thioxoisoindolin-2-yl)piperidine-2,6-dione |
| GT-08628 | 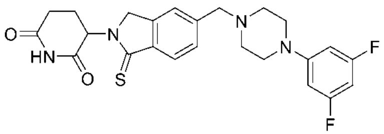 | 3-(5-((4-(3,5-difluorophenyl)piperazin-1-yl)methyl)-1-thioxoisoindolin-2-yl)piperidine-2,6-dione | 3-(5-((4-(3,5-difluorophenyl)piperazin-1-yl)methyl)-1-thioxoisoindolin-2-yl)piperidine-2,6-dione |
| GT-08614 | 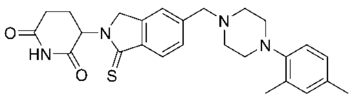 | 3-(5-((4-(2,4-dimethylphenyl)piperazin-1-yl)methyl)-1-thioxoisoindolin-2-yl)piperidine-2,6-dione | 3-(5-((4-(2,4-dimethylphenyl)piperazin-1-yl)methyl)-1-thioxoisoindolin-2-yl)piperidine-2,6-dione |
| GT-08615 | 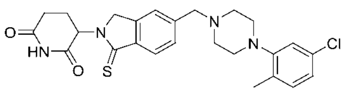 | 3-(5-((4-(5-chloro-2-methylphenyl)piperazin-1-yl) methyl)-1-thioxoisoindolin-2-yl)piperidine-2,6-dione | 3-(5-((4-(5-chloro-2-methylphenyl)piperazin-1-yl) methyl)-1-thioxoisoindolin-2-yl)piperidine-2,6-dione |
| GT-08616 | 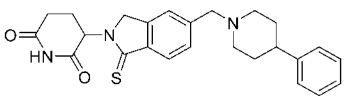 | 3-(5-((4-phenylpiperidin-1-yl)methyl)-1-thioxoisoindolin-2-yl)piperidine-2,6-dione | 3-(5-((4-phenylpiperidin-1-yl)methyl)-1-thioxoisoindolin-2-vl)piperidine-2,6-dione |

(continued)

| Compound No. | Structure of the compounds | The compounds' name | Chinese translation of the compounds' name |
|---|---|---|---|
| GT-08617 | 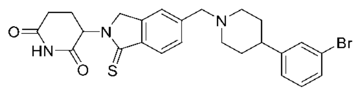 | 3-(5-((4-(3-bromophenyl)piperidin-1-yl)methyl)-1-thioxoisoindolin-2-yl)piperidine-2,6-dione | 3-(5-((4-(3-bromophenyl)piperidin-1-yl)methyl)-1-thioxoisoindolin-2-yl)piperidine-2,6-dione |
| GT-08618 | 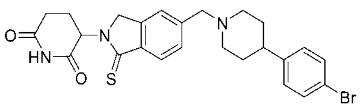 | 3-(5-((4-(4-bromophenyl)piperidin-1-yl)methyl)-1-thioxoisoindolin-2-yl)piperidine-2,6-dione | 3-(5-((4-(4-bromophenyl)piperidin-1-yl)methyl)-1-thioxoisoindolin-2-yl)piperidine-2,6-dione |
| | 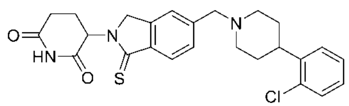 | 3-(5-((4-(2-chlorophenyl)piperidin-1-yl)methyl)-1-thioxoisoindolin-2-yl)piperidine-2,6-dione | 3-(5-((4-(2-chlorophenyl)piperidin-1-yl)methyl)-1-thioxoisoindolin-2-yl)piperidine-2,6-dione |
| GT-08619 | 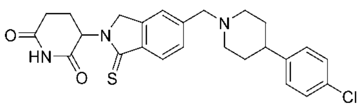 | 3-(5-((4-(4-chlorophenyl)piperidin-1-yl)methyl)-1-thioxoisoindolin-2-yl)piperidine-2,6-dione | 3-(5-((4-(4-chlorophenyl)piperidin-1-yl)methyl)-1-thioxoisoindolin-2-yl)piperidine-2,6-dione |
| GT-08620 | 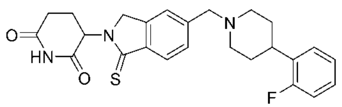 | 3-(5-((4-(2-fluorophenyl)piperidin-1-yl)methyl)-1-thioxoisoindolin-2-yl)piperidine-2,6-dione | 3-(5-((4-(2-fluorophenyl)piperidin-1-yl)methyl)-1-thioxoisoindolin-2-yl)piperidine-2,6-dione |
| GT-08621 | 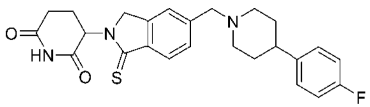 | 3-(5-((4-(4-fluorophenyl)piperidin-1-yl)methyl)-1-thioxoisoindolin-2-yl)piperidine-2,6-dione | 3-(5-((4-(4-fluorophenyl)piperidin-1-yl)methyl)-1-thioxoisoindolin-2-yl)piperidine-2,6-dione |
| GT-08622 | 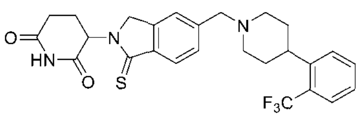 | 3-1-thioxo-5-((4-(2-(trifluoromethyl)phenyl)piper idin-1-yl)methyl)isoindolin-2-yl)piperidine-2,6-dione | 3-(1-thioxo-5-((4-(2-(trifluoromethyl)phenyl)piper idin-1-yl)methyl)isoindolin-2-yl)piperidine-2,6-dione |
| GT-08623 | 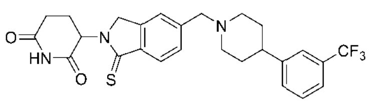 | 3-(1-thioxo-5-((4-(3-(trifluoromethyl)phenyl)piper idin-1-yl)methyl)isoindolin-2-yl)piperidine-2,6-dione | 3-(1-thioxo-5-((4-(3-(trifluoromethyl)phenyl)piper idin-1-yl)methyl)isoindolin-2-yl)piperidine-2,6-dione |
| GT-05150 | 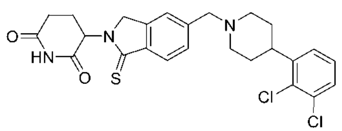 | 3-(5-((4-(2,3-dichlorophenyl)piperidin-1-yl)methyl)-1-thioxoisoindolin-2-yl)piperidine-2,6-dione | 3-(5-((4-(2,3-dichlorophenyl)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08330 | 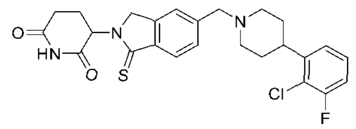 | 3-(5-((4-(2-chloro-3-fluorophenyl)piperidin-1-yl) methyl)-1-thioxoisoindolin-2-yl)piperidine-2,6-dione | 3-(5-((4-(2-chloro-3-fluorophenyl)piperidin-1-yl) methyl)-1-thioxoisoindolin-2-yl)piperidine-2,6-dione |
| GT-08331 | 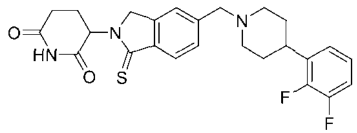 | 3-(5-((4-(2,3-difluorophenyl)piperidin-1-yl)methyl)-1-thioxoisoindolin-2-yl)piperidine-2,6-dione | 3-(5-((4-(2,3-difluorophenyl)piperidin-1-yl)methyl)-1-thioxoisoindolin-2-yl)piperidine-2,6-dione |
| GT-05149 | 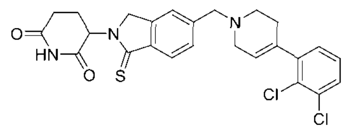 | 3-(5-((4-(2,3-dichlorophenyl)-3,6-dihydropyridin-1 (2H)-yl)methyl)-1-thioxoisoindolin-2-yl)piperidine-2,6-dione | 3-(5-((4-(2,3-dichlorophenyl)-3,6-dihydropyridin-1 (2H)-yl)methyl)-1-thioxoisoindolin-2-yl)piperidine-2,6-dione |
| GT-08206 | 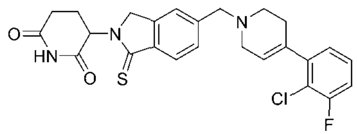 | 3-(5-((4-(2-chloro-3-fluorophenyl)-3,6-dihydropyridin-1 (2H)-yl)methyl)-1-thioxoisoindolin-2-yl)piperidine-2,6-dione | 3-(5-((4-(2-chloro-3-fluorophenyl)-3,6-dihydropyridin-1 (2H)-yl)methyl)-1-thioxoisoindolin-2-yl)piperidine-2,6-dione |
| GT-09216 | 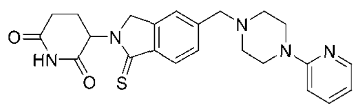 | 3-(5-((4-(pyridin-2-yl)piperazin-1-yl)methyl)-1-thioxoisoindolin-2-yl)piperidine-2,6-dione | 3-(5-((4-(pyridin-2-yl)piperazin-1-yl)methyl)-1-thioxoisoindolin-2-yl)piperidine-2,6-dione |
| GT-09214 | 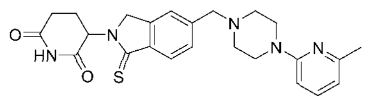 | 3-(5-((4-(6-methylpyridin-2-yl)piperazin-1-yl)methyl)-1-thioxoisoindolin-2-yl)piperidine-2,6-dione | 3-(5-((4-(6-methylpyridin-2-yl)piperazin-1-yl)methyl)-1-thioxoisoindolin-2-yl)piperidine-2,6-dione |
| GT-09215 | 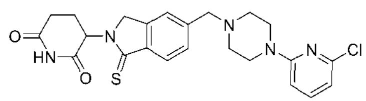 | 3-(5-((4-(6-chloropyridin-2-yl)piperazin-1-yl)methyl)-1-thioxoisoindolin-2-yl)piperidine-2,6-dione | 3-(5-((4-(6-chloropyridin-2-yl)piperazin-1-yl)methyl)-1-thioxoisoindolin-2-yl)piperidine-2,6-dione |
| GT-09217 | 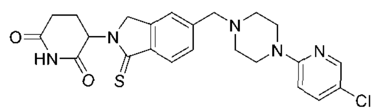 | 3-(5-((4-(5-chloropyridin-2-yl)piperazin-1-yl)methyl)-1-thioxoisoindolin-2-yl)piperidine-2,6-dione | 3-(5-((4-(5-chloropyridin-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-09218 | 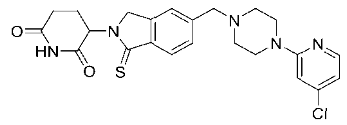 | 3-(5-((4-(4-chloropyridin-2-yl)piperazin-1-yl)methyl)-1-thioxoisoindolin-2-yl)piperidine-2,6-dione | 3-(5-((4-(4-chloropyridin-2-yl)piperazin-1-yl)methyl)-1-thioxoisoindolin-2-yl)piperidine-2,6-dione |
| GT-09218 | 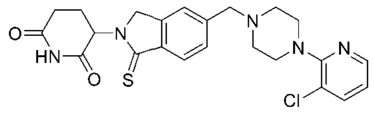 | 3- 5-((4-(3-chloropyridin-2-yl)piperazin-1-yl)methyl)-1-thioxoisoindolin-2-yl)piperidine-2,6-dione | 3-(5-((4-(3-chloropyridin-2-yl)piperazin-1-yl)methyl)-1-thioxoisoindolin-2-yl)piperidine-2,6-dione |
| GT-09220 | 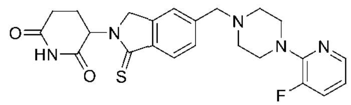 | 3-(5-((4-(3-fluoropyridin-2-yl)piperazin-1-yl)methyl)-1-thioxoisoindolin-2-yl)piperidine-2,6-dione | 3-(5-((4-(3-fluoropyridin-2-yl)piperazin-1-yl)methyl)-1-thioxoisoindolin-2-yl)piperidine-2,6-dione |
| GT-09221 | 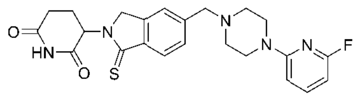 | 3-(5-((4-(6-fluoropyridin-2-yl)piperazin-1-yl)methyl)-1-thioxoisoindolin-2-yl)piperidine-2,6-dione | 3-(5-((4-(6-fluoropyridin-2-yl)piperazin-1-yl)methyl)-1-thioxoisoindolin-2-yl)piperidine-2,6-dione |
| GT-09222 | 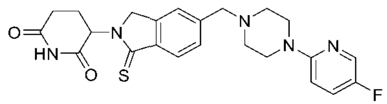 | 3-(5-((4-(5-fluoropyridin-2-yl)piperazin-1-yl)methyl)-1-thioxoisoindolin-2-yl)piperidine-2,6-dione | 3-(5-((4-(5-fluoropyridin-2-yl)piperazin-1-yl)methyl)-1-thioxoisoindolin-2-yl)piperidine-2,6-dione |
| GT-0922 3 | 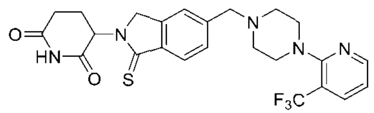 | 3-(1-thioxo-5-((4-(3-(trifluoromethyl)pyridin-2-yl)piperazin-1-yl)methyl)isoindolin-2-yl)piperidine-2,6-dione | 3-(1-thioxo-5-((4-(3-(trifluoromethyl)pyridin-2-yl)piperazin-1-yl)methyl)isoindolin-2-yl)piperidine-2,6-dione |
| GT-0922 4 | 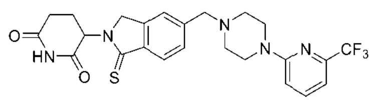 | 3-(1-thioxo-5-((4-(6-(trifluoromethyl)pyridin-2-yl)piperazin-1-yl)methyl)isoindolin-2-yl)piperidine-2,6-dione | 3-(1-thioxo-5-((4-(6-(trifluoromethyl)pyridin-2-yl)piperazin-1-yl)methyl)isoindolin-2-yl)piperidine-2,6-dione |
| GT-0922 5 | 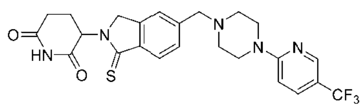 | 3-(1-thioxo-5-((4-(5-(trifluoromethyl)pyridin-2-yl)piperazin-1-yl)methyl)isoindolin-2-yl)piperidine-2,6-dione | 3-(1-thioxo-5-((4-(5-(trifluoromethyl)pyridin-2-yl)piperazin-1-yl)methyl)isoindolin-2-yl)piperidine-2,6-dione |

(continued)

| Compound No. | Structure of the compounds | The compounds' name | Chinese translation of the compounds' name |
|---|---|---|---|
| | 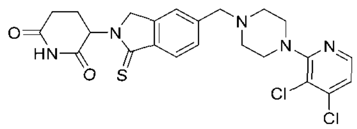 | 3-(5-((4-(3,4-dichloropyridin-2-yl)piperazin-1-yl) methyl)-1-thioxoisoindolin-2-yl)piperidine-2,6-dione | 3-(5-((4-(3,4-dichloropyridin-2-yl)piperazin-1-yl) methyl)-1-thioxoisoindolin-2-yl)piperidine-2,6-dione |
| GT-0922 6 | 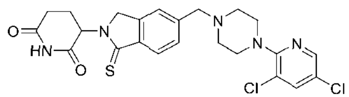 | 3-(5-((4-(3,5-dichloropyridin-2-yl)piperazin-1-yl) methyl)-1-thioxoisoindolin-2-yl)piperidine-2,6-dione | 3-(5-((4-(3,5-dichloropyridin-2-yl)piperazin-1-yl) methyl)-1-thioxoisoindolin-2-yl)piperidine-2,6-dione |
| | 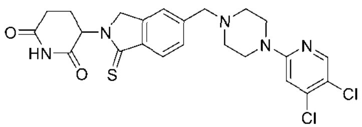 | 3-(5-((4-(4,5-dichloropyridin-2-yl)piperazin-1-yl) methyl)-1-thioxoisoindolin-2-yl)piperidine-2,6-dione | 3-(5-((4-(4,5-dichloropyridin-2-yl)piperazin-1-yl) methyl)-1-thioxoisoindolin-2-yl)piperidine-2,6-dione |
| GT-09235 | 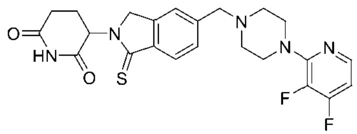 | 3-(5-((4-(3,4-difluoropyridin-2-yl)piperazin-1-yl) methyl)-1-thioxoisoindolin-2-yl)piperidine-2,6-dione | 3-(5-((4-(3,4-difluoropyridin-2-yl)piperazin-1-yl) methyl)-1-thioxoisoindolin-2-yl)piperidine-2,6-dione |
| GT-0924 | 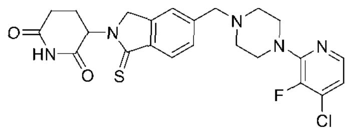 | 3-(5-((4-(4-chloro-3-fluoropyridin-2-yl)piperazin-1-yl) methyl)-1-thioxoisoindolin-2-yl)piperidine-2,6-dione | 3-(5-((4-(4-chloro-3-fluoropyridin-2-yl)piperazin-1-yl) methyl)-1-thioxoisoindolin-2-yl)piperidine-2,6-dione |
| GT-09227 | 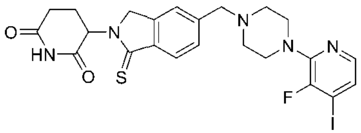 | 3-(5-((4-(3-fluoro-4-iodopyridin-2-yl)piperazin-1-yl) methyl)-1-thioxoisoindolin-2-yl)piperidine-2,6-dione | 3-(5-((4-(3-fluoro-4-iodopyridin-2-yl)piperazin-1-yl) methyl)-1-thioxoisoindolin-2-yl)piperidine-2,6-dione |
| GT-09245 | 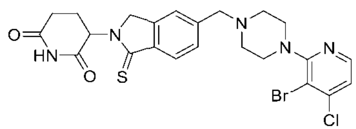 | 3-(5-((4-(3-bromo-4-chloropyridin-2-yl)piperazin-1-yl) methyl)-1-thioxoisoindolin-2-yl)piperidine-2,6-dione | 3-(5-((4-(3-bromo-4-chloropyridin-2-yl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0922 8 | 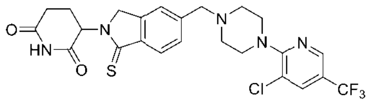 | 3-(5-((4-(3-chloro-5-(trifluoromethyl)pyridin-2-yl)piperazin-1-yl)methyl)-1-thioxoisoindolin-2-yl)piperidine-2,6-dione | 3-(5-((4-(3-chloro-5-(trifluoromethyl)pyridin-2-yl)piperazin-1-yl)methyl)-1-thioxoisoindolin-2-yl)piperidine-2,6-dione |
| | 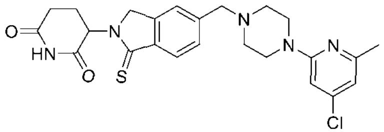 | 3-(5-((4-(4-chloro-6-methylpyridin-2-yl)piperazin-1-yl) methyl)-1-thioxoisoindolin-2-yl)piperidine-2,6-dione | 3-(5-((4-(4-chloro-6-methylpyridin-2-yl)piperazin-1-yl) methyl)-1-thioxoisoindolin-2-yl)piperidine-2,6-dione |
| GT-0922 9 | 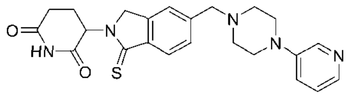 | 3-(5-((4-(pyridin-3-yl)piperazin-1-yl)methyl)-1-thioxoisoindolin-2-yl)piperidine-2,6-dione | 3-(5-((4-(pyridin-3-yl)piperazin-1-yl)methyl)-1-thioxoisoindolin-2-yl)piperidine-2,6-dione |
| GT-09230 | 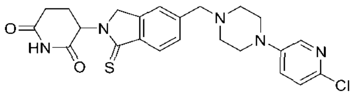 | 3-(5-((4-(6-chloropyridin-3-yl)piperazin-1-yl)methyl)-1-thioxoisoindolin-2-yl)piperidine-2,6-dione | 3-(5-((4-(6-chloropyridin-3-yl)piperazin-1-yl)methyl)-1-thioxoisoindolin-2-vl)piperidine-2,6-dione |
| GT-09231 | 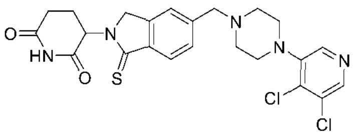 | 3-(5-((4-(4,5-dichloropyridin-3-yl)piperazin-1-yl) methyl)-1-thioxoisoindolin-2-yl)piperidine-2,6-dione | 3-(5-((4-(4,5-dichloropyridin-3-yl)piperazin-1-yl) methyl)-1-thioxoisoindolin-2-yl)piperidine-2,6-dione |
| GT-09232 | 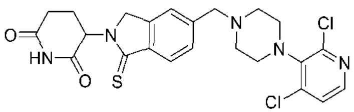 | 3-(5-((4-(2,4-dichloropyridin-3-yl)piperazin-1-yl) methyl)-1-thioxoisoindolin-2-yl)piperidine-2,6-dione | 3-(5-((4-(2,4-dichloropyridin-3-yl)piperazin-1-yl) methyl)-1-thioxoisoindolin-2-yl)piperidine-2,6-dione |
| GT-0923 3 | 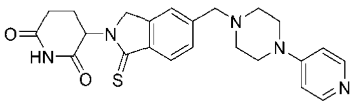 | 3-(5-((4-(pyridin-4-yl)piperazin-1-yl)methyl)-1-thioxoisoindolin-2-yl)piperidine-2,6-dione | 3-(5-((4-(pyridin-4-yl)piperazin-1-yl)methyl)-1-thioxoisoindolin-2-yl)piperidine-2,6-dione |
| GT-0923 4 | 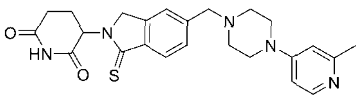 | 3-(5-((4-(2-methylpyridin-4-yl)piperazin-1-yl)methyl)-1-thioxoisoindolin-2-yl)piperidine-2,6-dione | 3-(5-((4-(2-methylpyridin-4-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0923 6 | 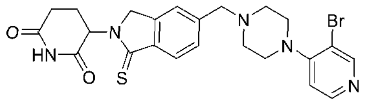 | 3-(5-((4-(3-bromopyridin-4-yl)piperazin-1-yl)methyl)-1-thioxoisoindolin-2-yl)piperidine-2,6-dione | 3-(5-((4-(3-bromopyridin-4-yl)piperazin-1-yl)methyl)-1-thioxoisoindolin-2-yl)piperidine-2,6-dione |
| GT-0923 7 | 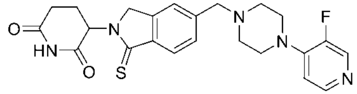 | 3-(5-((4-(3-fluoropyridin-4-yl)piperazin-1-yl)methyl)-1-thioxoisoindolin-2-yl)piperidine-2,6-dione | 3-(5-((4-(3-fluoropyridin-4-yl)piperazin-1-yl)methyl)-1-thioxoisoindolin-2-yl)piperidine-2,6-dione |
| GT-0923 8 | 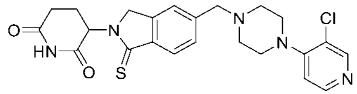 | 3-(5-((4-(3-chloropyridin-4-yl)piperazin-1-yl)methyl)-1-thioxoisoindolin-2-yl)piperidine-2,6-dione | 3-(5-((4-(3-chloropyridin-4-yl)piperazin-1-yl)methyl)-1-thioxoisoindolin-2-yl)piperidine-2,6-dione |
| | 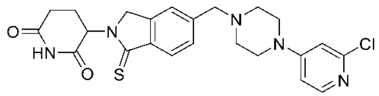 | 3-(5-((4-(2-chloropyridin-4-yl)piperazin-1-yl)methyl)-1-thioxoisoindolin-2-yl)piperidine-2,6-dione | 3-(5-((4-(2-chloropyridin-4-yl)piperazin-1-yl)methyl)-1-thioxoisoindolin-2-yl)piperidine-2,6-dione |
| GT-0923 9 | 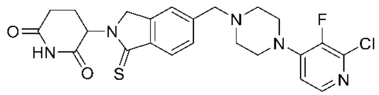 | 3-(5-((4-(2-chloro-3-fluoropyridin-4-yl)piperazin-1-yl) methyl)-1-thioxoisoindolin-2-yl)piperidine-2,6-dione | 3-(5-((4-(2-chloro-3-fluoropyridin-4-yl)piperazin-1-yl) methyl)-1-thioxoisoindolin-2-yl)piperidine-2,6-dione |
| GT-0924 1 | 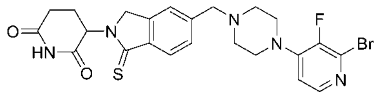 | 3-(5-((4-(2-bromo-3-fluoropyridin-4-yl)piperazin-1-yl) methyl)-1-thioxoisoindolin-2-yl)piperidine-2,6-dione | 3-(5-((4-(2-bromo-3-fluoropyridin-4-yl)piperazin-1-yl) methyl)-1-thioxoisoindolin-2-yl)piperidine-2,6-dione |
| GT-0924 0 | 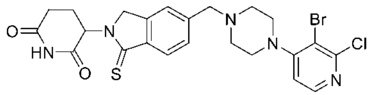 | 3-(5-((4-(3-bromo-2-chloropyridin-4-yl)piperazin-1-yl) methyl)-1-thioxoisoindolin-2-yl)piperidine-2,6-dione | 3-(5-((4-(3-bromo-2-chloropyridin-4-yl)piperazin-1-yl) methyl)-1-thioxoisoindolin-2-yl)piperidine-2,6-dione |
| GT-0935 4 | 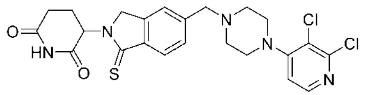 | 3-(5-((4-(2,3-dichloropyridin-4-yl)piperazin-1-yl) methyl)-1-thioxoisoindolin-2-yl)piperidine-2,6-dione | 3-(5-((4-(2,3-dichloropyridin-4-yl)piperazin-1-yl) methyl)-1-thioxoisoindolin-2-vl)piperidine-2,6-dione |
| GT-0924 2 | 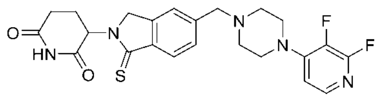 | 3-(5-((4-(2,3-difluoropyridin-4-yl)piperazin-1-yl) methyl)-1-thioxoisoindolin-2-yl)piperidine-2,6-dione | 3-(5-((4-(2,3-difluoropyridin-4-yl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 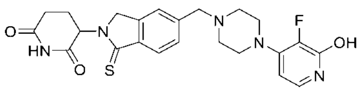 | 3-(5-((4-(3-fluoro-2-hydroxypyridin-4-yl)piperazin-1-yl) methyl)-1-thioxoisoindolin-2-yl)piperidine-2,6-dione | 3-(5-((4-(3-fluoro-2-hydroxypyridin-4-yl)piperazin-1-yl) methyl)-1-thioxoisoindolin-2-yl)piperidine-2,6-dione |
| GT-0926 4 | 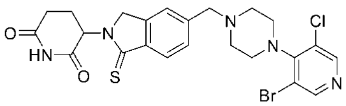 | 3-(5-((4-(3-bromo-5-chloropyridin-4-yl)piperazin-1-yl) methyl)-1-thioxoisoindolin-2-yl)piperidine-2,6-dione | 3-(5-((4-(3-bromo-5-chloropyridin-4-yl)piperazin-1-yl) methyl)-1-thioxoisoindolin-2-yl)piperidine-2,6-dione |
| GT-0924 3 | 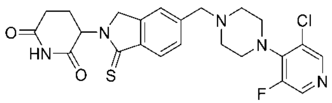 | 3-(5-((4-(3-chloro-5-fluoropyridin-4-yl)piperazin-1-yl) methyl)-1-thioxoisoindolin-2-yl)piperidine-2,6-dione | 3-(5-((4-(3-chloro-5-fluoropyridin-4-yl)piperazin-1-yl) methyl)-1-thioxoisoindolin-2-yl)piperidine-2,6-dione |
| GT-0924 6 | 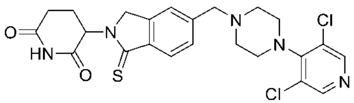 | 3-(5-((4-(3,5-dichloropyridin-4-yl)piperazin-1-yl) methyl)-1-thioxoisoindolin-2-yl)piperidine-2,6-dione | 3-(5-((4-(3,5-dichloropyridin-4-yl)piperazin-1-yl) methyl)-1-thioxoisoindolin-2-yl)piperidine-2,6-dione |
| GT-0924 7 | 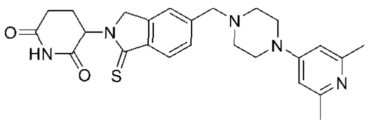 | 3-(5-((4-(2,6-dimethylpyridin-4-yl)piperazin-1-yl) methyl)-1-thioxoisoindolin-2-yl)piperidine-2,6-dione | 3-(5-((4-(2,6-dimethylpyridin-4-yl)piperazin-1-yl) methyl)-1-thioxoisoindolin-2-yl)piperidine-2,6-dione |
| GT-0925 0 | 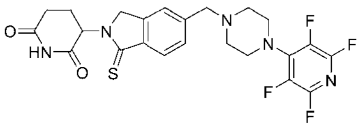 | 3-(5-((4-(perfluoropyridin-4-yl)piperazin-1-yl)methyl)-1-thioxoisoindolin-2-yl)piperidine-2,6-dione | 3-(5-((4-(perfluoropyridin-4-yl)piperazin-1-yl)methyl)-1-thioxoisoindolin-2-yl)piperidine-2,6-dione |
| GT-0926 5 | 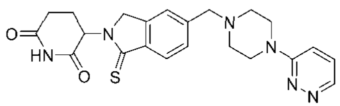 | 3-(5-((4-(pyridazin-3-yl)piperazin-1-yl)methyl)-1-thioxoisoindolin-2-yl)piperidine-2,6-dione | 3-(5-((4-(pyridazin-3-yl)piperazin-1-yl)methyl)-1-thioxoisoindolin-2-yl)piperidine-2,6-dione |
| GT-0925 1 | 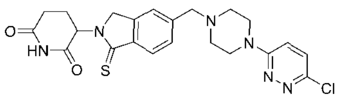 | 3-(5-((4-(6-chloropyridazin-3-yl)piperazin-1-yl) methyl)-1-thioxoisoindolin-2-yl)piperidine-2,6-dione | 3-(5-((4-(6-chloropyridazin-3-yl)piperazin-1-yl) methyl)-1-thioxoisoindolin-2-yl)piperidine-2,6-dione |
| GT-0925 2 | 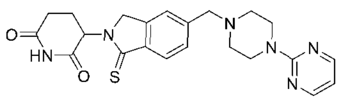 | 3-(5-((4-(pyrimidin-2-yl)piperazin-1-yl)methyl)-1-thioxoisoindolin-2-yl)piperidine-2,6-dione | 3-(5-((4-(pyrimidin-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0925 3 | 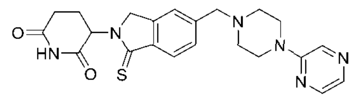 | 3-(5-((4-(pyrazin-2-yl)piperazin-1-yl)methyl)-1-thioxoisoindolin-2-yl)piperidine-2,6-dione | 3-(5-((4-(pyrazin-2-yl)piperazin-1-yl)methyl)-1-thioxoisoindolin-2-yl)piperidine-2,6-dione |
| GT-0925 4 | 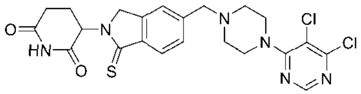 | 3-(5-((4-(5,6-dichloropyrimidin-4-yl)piperazin-1-yl)methyl)-1-thioxoisoindolin-2-yl)piperidine-2,6-dione | 3-(5-((4-(5,6-dichloropyrimidin-4-yl)piperazin-1-yl)methyl)-1-thioxoisoindolin-2-yl)piperidine-2,6-dione |
| GT-0924 8 | 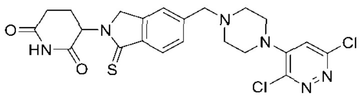 | 3-(5-((4-(3,6-dichloropyridazin-4-yl)piperazin-1-yl)methyl)-1-thioxoisoindolin-2-yl)piperidine-2,6-dione | 3-(5-((4-(3,6-dichloropyridazin-4-yl)piperazin-1-yl)methyl)-1-thioxoisoindolin-2-yl)piperidine-2,6-dione |
| GT-0925 5 | 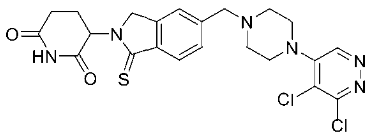 | 3-(5-((4-(5,6-dichloropyridazin-4-yl)piperazin-1-yl)methyl)-1-thioxoisoindolin-2-yl)piperidine-2,6-dione | 3-(5-((4-(5,6-dichloropyridazin-4-yl)piperazin-1-yl)methyl)-1-thioxoisoindolin-2-yl)piperidine-2,6-dione |
| GT-0924 9 | 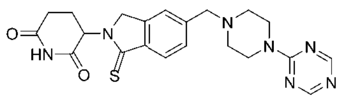 | 3-(5-((4-(1,3,5-triazin-2-yl)piperazin-1-yl)methyl)-1-thioxoisoindolin-2-yl)piperidine-2,6-dione | 3-(5-((4-(1,3,5-triazin-2-yl)piperazin-1-yl)methyl)-1-thioxoisoindolin-2-yl)piperidine-2,6-dione |
| GT-0848 8 | 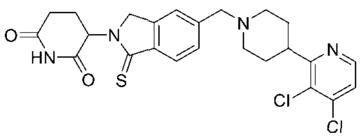 | 3-(5-((4-(3,4-dichloropyridin-2-yl)piperidin-1-yl)methyl)-1-thioxoisoindolin-2-yl)piperidine-2,6-dione | 3-(5-((4-(3,4-dichloropyridin-2-yl)piperidin-1-yl)methyl)-1-thioxoisoindolin-2-yl)piperidine-2,6-dione |
| GT-0847 5 | 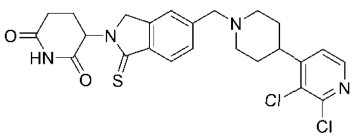 | 3-(5-((4-(2,3-dichloropyridin-4-yl)piperidin-1-yl)methyl)-1-thioxoisoindolin-2-yl)piperidine-2,6-dione | 3-(5-((4-(2,3-dichloropyridin-4-yl)piperidin-1-yl)methyl)-1-thioxoisoindolin-2-yl)piperidine-2,6-dione |
| GT-0847 3 | 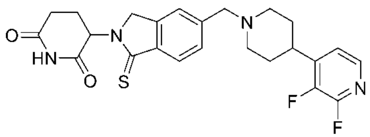 | 3-(5-((4-(2,3-difluoropyridin-4-yl)piperidin-1-yl)methyl)-1-thioxoisoindolin-2-yl)piperidine-2,6-dione | 3-(5-((4-(2,3-difluoropyridin-4-yl)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 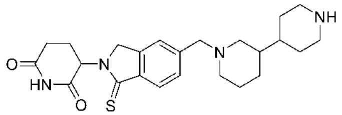 | 3-(5-([3,4'-bipiperidin]-1-ylmethyl)-1-thioxoisoindolin-2-yl)piperidine-2,6-dione | 3-(5-([3,4'-bipiperidin]-1-ylmethyl)-1-thioxoisoindolin-2-yl)piperidine-2,6-dione |
| | 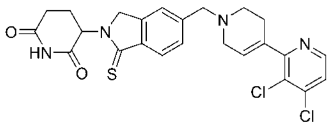 | 3-(5-((3,4-dichloro-3',6'-dihydro-[2,4'-bipyridin]-1'(2'H)-yl)methyl)-1-thioxoisoindolin-2-yl)piperidine-2,6-dione | 3-(5-((3,4-dichloro-3',6'-dihydro-[2,4'-bipyridin]-1'(2'H)-yl)methyl)-1-thioxoisoindolin-2-yl)piperidine-2,6-dione |
| GT-0848 9 | 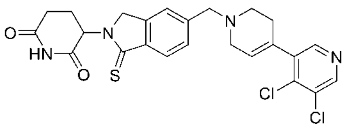 | 3-(5-((4,5-dichloro-3',6'-dihydro-[3,4'-bipyridin]-1'(2'H)-yl)methyl)-1-thioxoisoindolin-2-yl)piperidine-2,6-dione | 3-(5-((4,5-dichloro-3',6'-dihydro-[3,4'-bipyridin]-1'(2'H)-yl)methyl)-1-thioxoisoindolin-2-yl)piperidine-2,6-dione |
| GT-0849 0 | 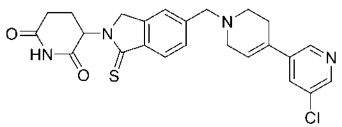 | 3-(5-((5-chloro-3',6'-dihydro-[3,4'-bipyridin]-1'(2'H)-yl) methyl)-1-thioxoisoindolin-2-yl)piperidine-2,6-dione | 3-(5-((5-chloro-3',6'-dihydro-[3,4'-bipyridin]-1'(2'H)-yl) methyl)-1-thioxoisoindolin-2-yl)piperidine-2,6-dione |
| GT-0848 2 | 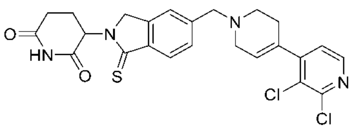 | 3-(5-((2',3'-dichloro-3,6-dihydro-[4,4'-bipyridin]-1(2H)-yl)methyl)-1-thioxoisoindolin-2-yl)piperidine-2,6-dione | 3-(5-((2',3'-dichloro-3,6-dihydro-[4,4'-bipyridin]-1(2H)-yl)methyl)-1-thioxoisoindolin-2-yl)piperidine-2,6-dione |
| GT-0849 1 | 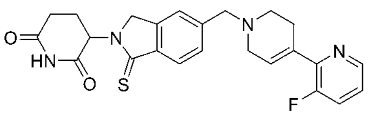 | 3-(5-((3-fluoro-3',6'-dihydro-[2,4'-bipyridin]-1'(2'H)-yl) methyl)-1-thioxoisoindolin-2-yl)piperidine-2,6-dione | 3- 5-((3-fluoro-3',6'-dihydro-[2,4'-bipyridin]-1'(2'H)-yl) methyl)-1-thioxoisoindolin-2-yl)piperidine-2,6-dione |
| GT-0847 4 | 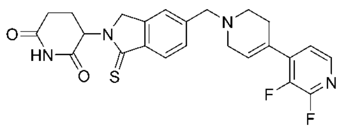 | 3-(5-((2',3'-difluoro-3,6-dihydro-[4,4'-bipyridin]-1(2H)-yl)methyl)-1-thioxoisoindolin-2-yl)piperidine-2,6-dione | 3-(5-((2',3'-difluoro-3,6-dihydro-[4,4'-bipyridin]-1(2H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0518 2 | 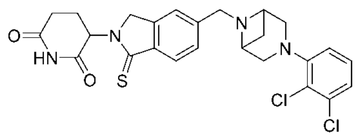 | 3-(5-((3-(2,3-dichlorophenyl)-3,6-diazabicyclo[3.1.1] heptan-6-yl)methyl)-1-thioxoisoindolin-2-yl)piperidine-2,6-dione | 3-(5-((3-(2,3-dichlorophenyl)-3,6-diazabicyclo[3.1.1] heptan-6-yl)methyl)-1-thioxoisoindolin-2-yl)piperidine-2,6-dione |
| GT-0847 6 | 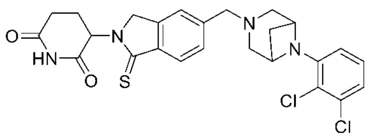 | 3-(5-((6-(2,3-dichlorophenyl)-3,6-diazabicyclo[3.1.1] heptan-3-yl)methyl)-1-thioxoisoindolin-2-yl)piperidine-2,6-dione | 3-(5-((6-(2,3-dichlorophenyl)-3,6-diazabicyclo[3.1.1] heptan-3-yl)methyl)-1-thioxoisoindolin-2-yl)piperidine-2,6-dione |
| GT-0847 7 | 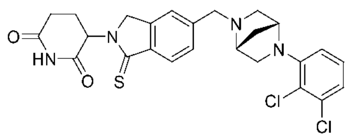 | 3-(5-(((1R,4R)-5-(2,3-dichlorophenyl)-2,5-diazabicyclo [2.2.1]heptan-2-yl)methyl)-1-thioxoisoindolin-2-yl)piperidine-2,6-dione | 3-(5-(((1R,4R)-5-(2,3-dichlorophenyl)-2,5-diazabicyclo [2.2.1]heptan-2-yl)methyl)-1-thioxoisoindolin-2-yl)piperidine-2,6-dione |
| GT-0518 3 | 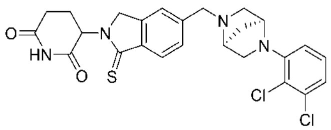 | 3-(5-(((1S,4S)-5-(2,3-dichlorophenyl)-2,5-diazabicyclo [2.2.1]heptan-2-yl)methyl)-1-thioxoisoindolin-2-yl)piperidine-2,6-dione | 3-(5-(((1S,4S)-5-(2,3-dichlorophenyl)-2,5-diazabicyclo [2.2.1]heptan-2-yl)methyl)-1-thioxoisoindolin-2-yl)piperidine-2,6-dione |
| GT-0847 8 | 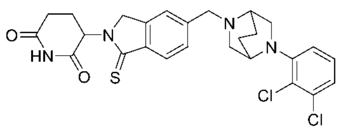 | 3-(5-((5-(2,3-dichlorophenyl)-2,5-diazabicyclo[2.2.2]octan-2-yl)methyl)-1-thioxoisoindolin-2-yl)piperidine-2,6-dione | 3-(5-((5-(2,3-dichlorophenyl)-2,5-diazabicyclo[2.2.2]octan-2-yl)methyl)-1-thioxoisoindolin-2-yl)piperidine-2,6-dione |
| GT-0847 9 | 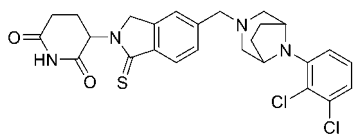 | 3-(5-((8-(2,3-dichlorophenyl)-3,8-diazabicyclo[3.2.1]octan-3-yl)methyl)-1-thioxoisoindolin-2-yl)piperidine-2,6-dione | 3-(5-((8-(2,3-dichlorophenyl)-3,8-diazabicyclo[3.2.1]octan-3-yl)methyl)-1-thioxoisoindolin-2-yl)piperidine-2,6-dione |
| GT-08480 | 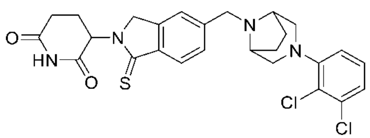 | 3-(5-((3-(2,3-dichlorophenyl)-3,8-diazabicyclo[3.2.1]octan-8-yl)methyl)-1-thioxoisoindolin-2-yl)piperidine-2,6-dione | 3-(5-((3-(2,3-dichlorophenyl)-3,8-diazabicyclo[3.2.1]octan-8-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08481 | 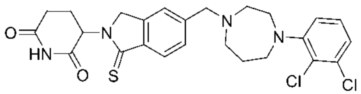 | 3-(5-((4-(2,3-dichlorophenyl)-1,4-diazepan-1-yl) methyl)-1-thioxoisoindolin-2-yl)piperidine-2,6-dione | 3-(5-((4-(2,3-dichlorophenyl)-1,4-diazepan-1-yl) methyl)-1-thioxoisoindolin-2-vl)piperidine-2,6-dione |
| GT-05089 | 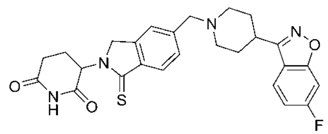 | 3-(5-((4-(6-fluorobenzo[d]isoxazol-3-yl)piperidin-1-yl) methyl)-1-thioxoisoindolin-2-yl)piperidine-2,6-dione | 3-(5-((4-(6-fluorobenzo[d]isoxazol-3-yl)piperidin-1-yl) methyl)-1-thioxoisoindolin-2-yl)piperidine-2,6-dione |
| GT-09266 | 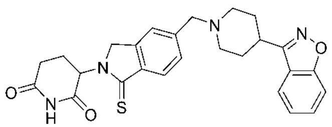 | 3-(5-((4-(benzo[d]isoxazol-3-yl)piperidin-1-yl) methyl)-1-thioxoisoindolin-2-yl)piperidine-2,6-dione | 3-(5-((4-(benzo[d]isoxazol-3-yl)piperidin-1-yl) methyl)-1-thioxoisoindolin-2-yl)piperidine-2,6-dione |
| GT-09256 | 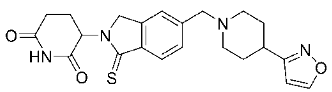 | 3-(5-((4-(isoxazol-3-yl)piperidin-1-yl)methyl)-1-thioxoisoindolin-2-yl)piperidine-2,6-dione | 3-(5-((4-(isoxazol-3-yl)piperidin-1-yl)methyl)-1-thioxoisoindolin-2-yl)piperidine-2,6-dione |
| GT-09257 | 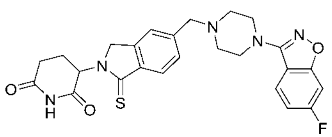 | 3-(5-((4-(6-fluorobenzo[d]isoxazol-3-yl)piperazin-1-yl) methyl)-1-thioxoisoindolin-2-yl)piperidine-2,6-dione | 3-(5-((4-(6-fluorobenzo[d]isoxazol-3-yl)piperazin-1-yl) methyl)-1-thioxoisoindolin-2-yl)piperidine-2,6-dione |
| GT-09258 | 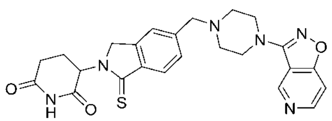 | 3-(5-((4-(isoxazolo[4,5-c]pyridin-3-yl)piperazin-1-yl) methyl)-1-thioxoisoindolin-2-yl)piperidine-2,6-dione | 3-(5-((4-(isoxazolo[4,5-c]pyridin-3-yl)piperazin-1-yl) methyl)-1-thioxoisoindolin-2-yl)piperidine-2,6-dione |
| GT-09259 | 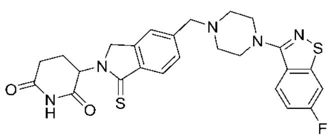 | 3-(5-((4-(6-fluorobenzo[d]isothiazol-3-yl)piperazin-1-yl) methyl)-1-thioxoisoindolin-2-yl)piperidine-2,6-dione | 3-(5-((4-(6-fluorobenzo[d]isothiazol-3-yl)piperazin-1-yl) methyl)-1-thioxoisoindolin-2-yl)piperidine-2,6-dione |
| GT-09260 | 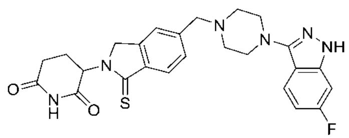 | 3-(5-((4-(6-fluoro-1H-indazol-3-yl)piperazin-1-yl) methyl)-1-thioxoisoindolin-2-yl)piperidine-2,6-dione | 3-(5-((4-(6-fluoro-1H-indazol-3-yl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-09261 | 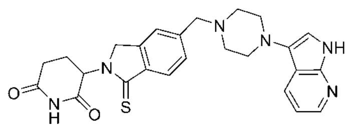 | 3-(5-((4-(1H-pyrrolo[2,3-b]pyridin-3-yl)piperazin-1-yl) methyl)-1-thioxoisoindolin-2-yl)piperidine-2,6-dione | 3-(5-((4-(1H-pyrrolo[2,3-b]pyridin-3-yl)piperazin-1-yl) methyl)-1-thioxoisoindolin-2-yl)piperidine-2,6-dione |
| GT-05223 | 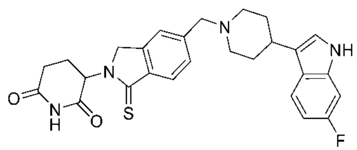 | 3-(5-((4-(6-fluoro-1H-indol-3-yl)piperidin-1-yl) methyl)-1-thioxoisoindolin-2-yl)piperidine-2,6-dione | 3-(5-((4-(6-fluoro-1H-indol-3-yl)piperidin-1-yl) methyl)-1-thioxoisoindolin-2-yl)piperidine-2,6-dione |
| GT-0926 7 | 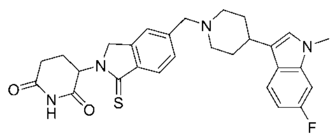 | 3-(5-((4-(6-fluoro-1-methyl-1H-indol-3-yl)piperidin-1-yl) methyl)-1-thioxoisoindolin-2-yl)piperidine-2,6-dione | 3-(5-((4-(6-fluoro-1-methyl-1H-indol-3-yl)piperidin-1-yl) methyl)-1-thioxoisoindolin-2-yl)piperidine-2,6-dione |
| GT-0926 9 | 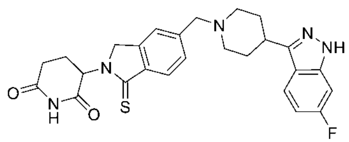 | 3-(5-((4-(6-fluoro-1H-indazol-3-yl)piperidin-1-yl) methyl)-1-thioxoisoindolin-2-yl)piperidine-2,6-dione | 3-(5-((4-(6-fluoro-1H-indazol-3-yl)piperidin-1-yl) methyl)-1-thioxoisoindolin-2-yl)piperidine-2,6-dione |
| GT-0524 4 | 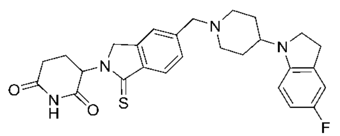 | 3-(5-((4-(5-fluoroindolin-1-yl)piperidin-1-yl)methyl)-1-thioxoisoindolin-2-yl)piperidine-2,6-dione | 3-(5-((4-(5-fluoroindolin-1-yl)piperidin-1-yl)methyl)-1-thioxoisoindolin-2-yl)piperidine-2,6-dione |
| GT-0926 2 | 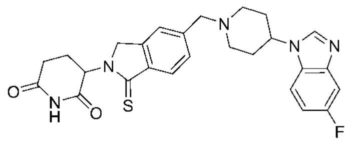 | 3-(5-((4-(5-fluoro-1H-benzo[d]imidazol-1-yl)piperidin-1-yl)methyl)-1-thioxoisoindolin-2-yl)piperidine-2,6-dione | 3-(5-((4-(5-fluoro-1H-benzo[d]imidazol-1-yl)piperidin-1-yl)methyl)-1-thioxoisoindolin-2-yl)piperidine-2,6-dione |
| GT-0926 3 | 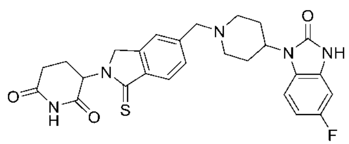 | 3-(5-((4-(5-fluoro-2-oxo-2,3-dihydro-1H-benzo[d]imidazol-1-yl)piperidin-1-yl)methyl)-1-thioxoisoindolin-2-yl) piperidine-2,6-dione | 3-(5-((4-(5-fluoro-2-oxo-2,3-dihydro-1H-benzo[d]imidazol-1-yl)piperidin-1-yl)methyl)-1-thioxoisoindolin-2-yl) piperidine-2,6-dione |

(continued)

| Compound No. | Structure of the compounds | The compounds' name | Chinese translation of the compounds' name |
|---|---|---|---|
| GT-0926 8 | 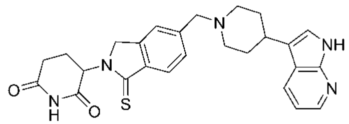 | 3-(5-((4-(1H-pyrrolo[2,3-b]pyridin-3-yl)piperidin-1-yl) methyl)-1-thioxoisoindolin-2-yl)piperidine-2,6-dione | 3-(5-((4-(1H-pyrrolo[2,3-b]pyridin-3-yl)piperidin-1-yl) methyl)-1-thioxoisoindolin-2-yl)piperidine-2,6-dione |
| GT-0524 8 | 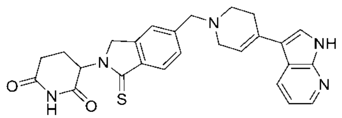 | 3-(5-((4-(1H-pyrrolo[2,3-b]pyridin-3-yl)-3,6-dihydropyridin-1 (2H)-yl)methyl)-1-thioxoisoindolin-2-yl)piperidine-2,6-dione | 3-(5-((4-(1H-pyrrolo[2,3-b]pyridin-3-yl)-3,6-dihydropyridin-1(2H)-yl)methyl)-1-thioxoisoindolin-2-yl)piperi-dine-2,6-dione |
| | 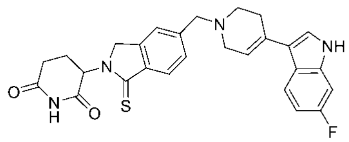 | 3-(5-((4-(6-fluoro-1H-indol-3-yl)-3,6-dihydropyridin-1(2H)-yl)methyl)-1-thioxoisoindolin-2-yl)piperidine-2,6-dione | 3-(5-((4-(6-fluoro-1H-indol-3-yl)-3,6-dihydropyridin-1(2H)-yl)methyl)-1-thioxoisoindolin-2-yl)piperidine-2,6-dione |
| GT-0524 7 | 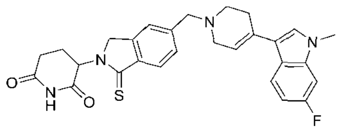 | 3-(5-((4-(6-fluoro-1-methyl-1H-indol-3-yl)-3,6-dihydropyridin-1(2H)-yl)methyl)-1-thioxoisoindolin-2-yl)piperidine-2,6-dione | 3-(5-((4-(6-fluoro-1-methyl-1H-indol-3-yl)-3,6-dihydropyridin-1(2H)-yl)methyl)-1-thioxoisoindolin-2-yl)piperidine-2,6-dione |
| GT-0927 0 | 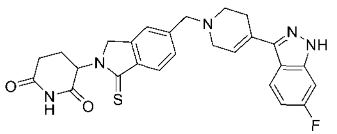 | 3-(5-((4-(6-fluoro-1H-indazol-3-yl)-3,6-dihydropyridin-1(2H)-yl)methyl)-1-thioxoisoindolin-2-yl)piperidine-2,6-dione | 3-(5-((4-(6-fluoro-1H-indazol-3-yl)-3,6-dihydropyridin-1(2H)-yl)methyl)-1-thioxoisoindolin-2-yl)piperidine-2,6-dione |
| GT-0927 1 | 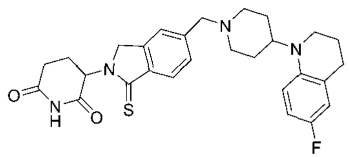 | 3-(5-((4-(6-fluoro-3,4-dihydroquinolin-1(2H)-yl)piperidin-1-yl)methyl)-1-thioxoisoindolin-2-yl)piperidine-2,6-dione | 3-(5-((4-(6-fluoro-3,4-dihydroquinolin-1(2H)-yl)piperidin-1-yl)methyl)-1-thioxoisoindolin-2-yl)piperidine-2,6-dione |
| GT-09272 | 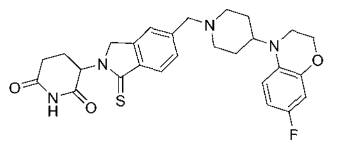 | 3-(5-((4-(7-fluoro-2,3-dihydro-4H-benzo[b][1,4]oxazin-4-yl)piperidin-1-yl)methyl)-1-thioxoisoindolin-2-yl) piperidine-2,6-dione | 3-(5-((4-(7-fluoro-2,3-dihydro-4H-benzo[b][1,4]oxazin-4-yl)piperidin-1-yl)methyl)-1-thioxoisoindolin-2-vl) piperidine-2,6-dione |

(continued)

| Compound No. | Structure of the compounds | The compounds' name | Chinese translation of the compounds' name |
|---|---|---|---|
| GT-09273 | 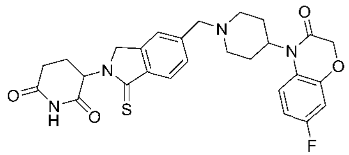 | 3-(5-((4-(7-fluoro-3-oxo-2,3-dihydro-4H-benzo[b][1,4] oxazin-4-yl)piperidin-1-yl)methyl)-1-thioxoisoindolin-2-yl)piperidine-2,6-dione | 3-(5-((4-(7-fluoro-3-oxo-2,3-dihydro-4H-benzo[b][1,4] oxazin-4-yl)piperidin-1-yl)methyl)-1-thioxoisoindolin-2-vl)piperidine-2,6-dione |
| GT-09274 | 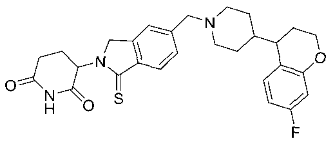 | 3-(5-((4-(7-fluorochroman-4-yl)piperidin-1-yl)methyl)-1-thioxoisoindolin-2-yl)piperidine-2,6-dione | 3-(5-((4-(7-fluorochroman-4-yl)piperidin-1-yl)methyl)-1-thioxoisoindolin-2-yl)piperidine-2,6-dione |
| | 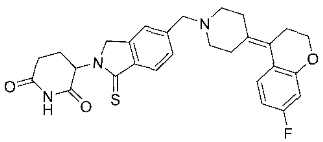 | 3-(5-((4-(7-fluorochroman-4-ylidene)piperidin-1-yl) methyl)-1-thioxoisoindolin-2-yl)piperidine-2,6-dione | 3-(5-((4-(7-fluorochroman-4-ylidene)piperidin-1-yl) methyl)-1-thioxoisoindolin-2-yl)piperidine-2,6-dione |
| GT-09275 | 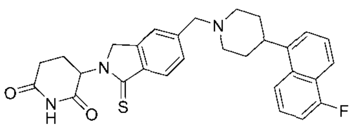 | 3-(5-((4-(5-fluoronaphthalen-1-yl)piperidin-1-yl) methyl)-1-thioxoisoindolin-2-yl)piperidine-2,6-dione | 3-(5-((4-(5-fluoronaphthalen-1-yl)piperidin-1-yl) methyl)-1-thioxoisoindolin-2-yl)piperidine-2,6-dione |
| GT-09278 | 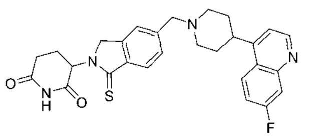 | 3-(5-((4-(7-fluoroquinolin-4-yl)piperidin-1-yl)methyl)-1-thioxoisoindolin-2-yl)piperidine-2,6-dione | 3-(5-((4-(7-fluoroquinolin-4-yl)piperidin-1-yl)methyl)-1-thioxoisoindolin-2-yl)piperidine-2,6-dione |
| GT-09277 | 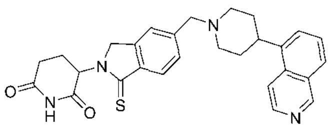 | 3-(5-((4-(isoquinolin-5-yl)piperidin-1-yl)methyl)-1-thioxoisoindolin-2-yl)piperidine-2,6-dione | 3-(5-((4-(isoquinolin-5-yl)piperidin-1-yl)methyl)-1-thioxoisoindolin-2-yl)piperidine-2,6-dione |
| GT-09276 | 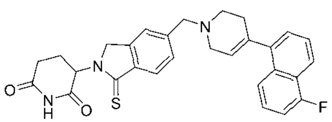 | 3-(5-((4-(5-fluoronaphthalen-1-yl)-3,6-dihydropyridin-1(2H)-yl)methyl)-1-thioxoisoindolin-2-yl)piperidine-2,6-dione | 3-(5-((4-(5-fluoronaphthalen-1-yl)-3,6-dihydropyridin-1(2H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-05245 | 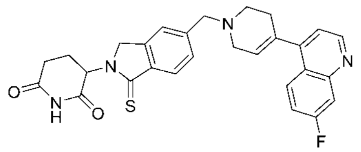 | 3-(5-((4-(7-fluoroquinolin-4-yl)-3,6-dihydropyridin-1(2H)-yl)methyl)-1-thioxoisoindolin-2-yl)piperidine-2,6-dione | 3-(5-((4-(7-fluoroquinolin-4-yl)-3,6-dihydropyridin-1(2H)-yl)methyl)-1-thioxoisoindolin-2-yl)piperidine-2,6-dione |
| GT-05246 | 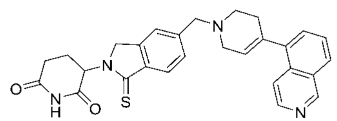 | 3-(5-((4-(isoquinolin-5-yl)-3,6-dihydropyridin-1(2H)-yl)methyl)-1-thioxoisoindolin-2-yl)piperidine-2,6-dione | 3-(5-((4-(isoquinolin-5-yl)-3,6-dihydropyridin-1(2H)-yl)methyl)-1-thioxoisoindolin-2-yl)piperidine-2,6-dione |
| | 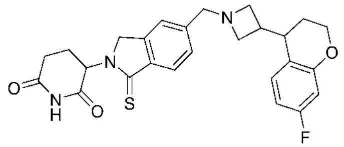 | 3-(5-((3-(7-fluorochroman-4-yl)azetidin-1-yl)methyl)-1-thioxoisoindolin-2-yl)piperidine-2,6-dione | 3-(5-((3-(7-fluorochroman-4-yl)azetidin-1-yl)methyl)-1-thioxoisoindolin-2-yl)piperidine-2,6-dione |
| | 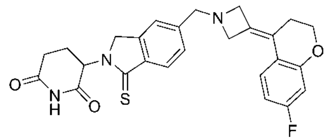 | 3-(5-((3-(7-fluorochroman-4-ylidene)azetidin-1-yl)methyl)-1-thioxoisoindolin-2-yl)piperidine-2,6-dione | 3-(5-((3-(7-fluorochroman-4-ylidene)azetidin-1-yl)methyl)-1-thioxoisoindolin-2-yl)piperidine-2,6-dione |
| GT-09279 | 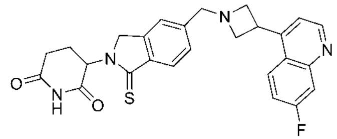 | 3-(5-((3-(7-fluoroquinolin-4-yl)azetidin-1-yl)methyl)-1-thioxoisoindolin-2-yl)piperidine-2,6-dione | 3-(5-((3-(7-fluoroquinolin-4-yl)azetidin-1-yl)methyl)-1-thioxoisoindolin-2-yl)piperidine-2,6-dione |
| | 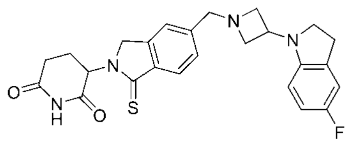 | 3-(5-((3-(5-fluoroindolin-1-yl)azetidin-1-yl)methyl)-1-thioxoisoindolin-2-yl)piperidine-2,6-dione | 3-(5-((3-(5-fluoroindolin-1-yl)azetidin-1-yl)methyl)-1-thioxoisoindolin-2-yl)piperidine-2,6-dione |
| | 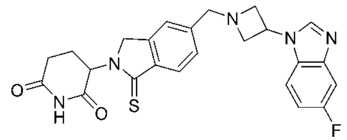 | 3-(5-((3-(5-fluoro-1H-benzo[d]imidazol-1-yl)azetidin-1-yl)methyl)-1-thioxoisoindolin-2-yl)piperidine-2,6-dione | 3-(5-((3-(5-fluoro-1H-benzo[d]imidazol-1-yl)azetidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-09280 | 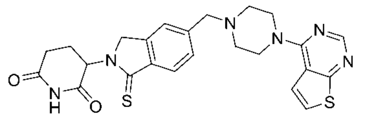 | 3-(5-((4-(thieno[2,3-d]pyrimidin-4-yl)piperazin-1-yl) methyl)-1-thioxoisoindolin-2-yl)piperidine-2,6-dione | 3-(5-((4-(thieno[2,3-d]pyrimidin-4-yl)piperazin-1-yl) methyl)-1-thioxoisoindolin-2-yl)piperidine-2,6-dione |
| GT-09281 | 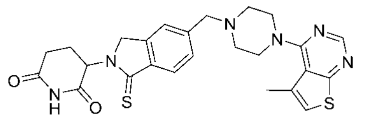 | 3-(5-((4-(5-methylthieno[2,3-d]pyrimidin-4-yl)piperazin-1-yl)methyl)-1-thioxoisoindolin-2-yl)piperidine-2,6-dione | 3-(5-((4-(5-methylthieno[2,3-d]pyrimidin-4-yl)piperazin-1-yl)methyl)-1-thioxoisoindolin-2-yl)piperidine-2,6-dione |
| GT-09282 | 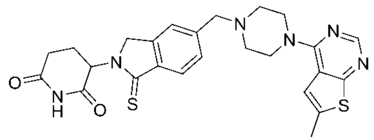 | 3-(5-((4-(6-methylthieno[2,3-d]pyrimidin-4-yl)piperazin-1-yl)methyl)-1-thioxoisoindolin-2-yl)piperidine-2,6-dione | 3-(5-((4-(6-methylthieno[2,3-d]pyrimidin-4-yl)piperazin-1-yl)methyl)-1-thioxoisoindolin-2-yl)piperidine-2,6-dione |
| GT-09284 | 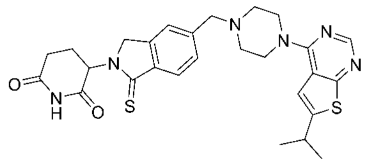 | 3-(5-((4-(6-isopropylthieno[2,3-d]pyrimidin-4-yl)piperazin-1-yl)methyl)-1-thioxoisoindolin-2-yl)piperidine-2,6-dione | 3-(5-((4-(6-isopropylthieno[2,3-d]pyrimidin-4-yl)piperazin-1-yl)methyl)-1-thioxoisoindolin-2-yl)piperidine-2,6-dione |
| GT-09285 | 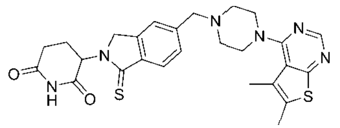 | 3-(5-((4-(5,6-dimethylthieno[2,3-d]pyrimidin-4-yl)piperazin-1-yl)methyl)-1-thioxoisoindolin-2-yl)piperidine-2,6-dione | 3-(5-((4-(5,6-dimethylthieno[2,3-d]pyrimidin-4-yl)piperazin-1-yl)methyl)-1-thioxoisoindolin-2-yl)piperidine-2,6-dione |
| GT-09296 | 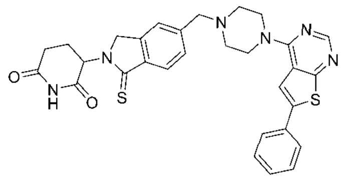 | 3-(5-((4-(6-phenylthieno[2,3-d]pyrimidin-4-yl)piperazin-1-yl)methyl)-1-thioxoisoindolin-2-yl)piperidine-2,6-dione | 3-(5-((4-(6-phenylthieno[2,3-d]pyrimidin-4-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-09295 | 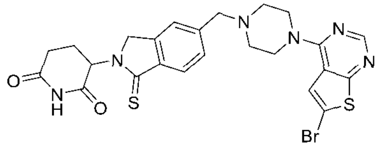 | 3-(5-((4-(6-bromothieno[2,3-d]pyrimidin-4-yl)piperazin-1-yl)methyl)-1-thioxoisoindolin-2-yl)piperidine-2,6-dione | 3-(5-((4-(6-bromothieno[2,3-d]pyrimidin-4-yl)piperazin-1-yl)methyl)-1-thioxoisoindolin-2-yl)piperidine-2,6-dione |
| GT-09297 | 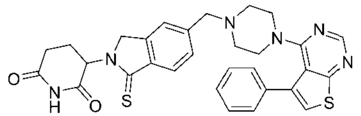 | 3-(5-((4-(5-phenylthieno[2,3-d]pyrimidin-4-yl)piperazin-1-yl)methyl)-1-thioxoisoindolin-2-yl)piperidine-2,6-dione | 3-(5-((4-(5-phenylthieno[2,3-d]pyrimidin-4-yl)piperazin-1-yl)methyl)-1-thioxoisoindolin-2-yl)piperidine-2,6-dione |
| GT-09300 | 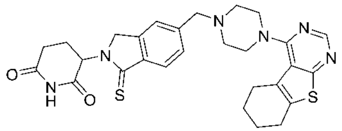 | 3-(5-((4-(5,6,7,8-tetrahydrobenzo[4,5]thieno[2 ,3-d]pyrimidin-4-yl)piperazin-1-yl)methyl)-1-thioxoisoindolin-2-yl)piperidine-2,6-dione | 3-(5-((4-(5,6,7,8-tetrahydrobenzo[4,5]thieno[2 ,3-d]pyrimidin-4-yl)piperazin-1-yl)methyl)-1-thioxoisoindolin-2-yl)piperidine-2,6-dione |
| GT-09301 | 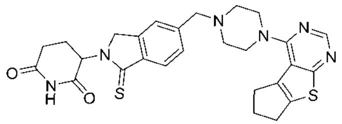 | 3-(5-((4-(6,7-dihydro-5H-cyclopenta[4,5]thieno[2,3-d]pyrimidin-4-yl)piperazin-1-yl)methyl)-1-thioxoisoindolin-2-yl)piperidine-2,6-dione | 3-(5-((4-(6,7-dihydro-5H-cyclopenta[4,5]thieno[2,3-d]pyrimidin-4-yl)piperazin-1-yl)methyl)-1-thioxoisoindolin-2-yl)piperidine-2,6-dione |
| GT-09308 | 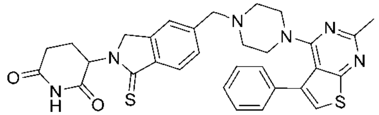 | 3-(5-((4-(2-methyl-5-phenylthieno[2,3-d]pyrimidin-4-yl)piperazin-1-yl)methyl)-1-thioxoisoindolin-2-yl)piperidine-2,6-dione | 3-(5-((4-(2-methyl-5-phenylthieno[2,3-d]pyrimidin-4-yl)piperazin-1-yl)methyl)-1-thioxoisoindolin-2-yl)piperidine-2,6-dione |
| GT-09299 | 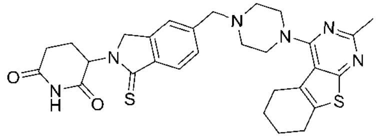 | 3-(5-((4-(2-methyl-5,6,7,8-tetrahydrobenzo[4,5]thieno[2 ,3-d]pyrimidin-4-yl)piperazin-1-yl)methyl)-1-thioxoisoindolin-2-yl)piperidine-2,6-dione | 3-(5-((4-(2-methyl-5,6,7,8-tetrahydrobenzo[4,5]thieno[2 ,3-d]pyrimidin-4-yl)piperazin-1-yl)methyl)-1-thioxoisoindolin-2-yl)piperidine-2,6-dione |
| GT-09302 | 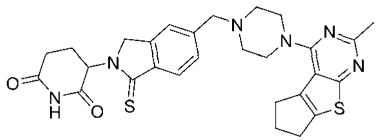 | 3-(5-((4-(2-methyl-6,7-dihydro-5H-cyclopenta[4,5]thieno[2,3-d]pyrimidin-4-yl)piperazin-1-yl)methyl)-1-thioxoisoindolin-2-yl)piperidine-2,6-dione | 3-(5-((4-(2-methyl-6,7-dihydro-5H-cyclopenta[4,5]thieno[2,3-d]pyrimidin-4-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-09298 | 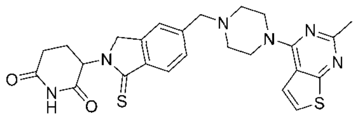 | 3-(5-((4-(2-methylthieno[2,3-d]pyrimidin-4-yl)piperazin-1-yl)methyl)-1-thioxoisoindolin-2-yl)piperidine-2,6-dione | 3-(5-((4-(2-methylthieno[2,3-d]pyrimidin-4-yl)piperazin-1-yl)methyl)-1-thioxoisoindolin-2-yl)piperidine-2,6-dione |
| GT-09303 | 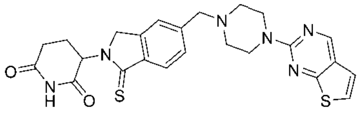 | 3-(5-((4-(thieno[2,3-d]pyrimidin-2-yl)piperazin-1-yl) methyl)-1-thioxoisoindolin-2-yl)piperidine-2,6-dione | 3-(5-((4-(thieno[2,3-d]pyrimidin-2-yl)piperazin-1-yl) methyl)-1-thioxoisoindolin-2-yl)piperidine-2,6-dione |
| GT-09287 | 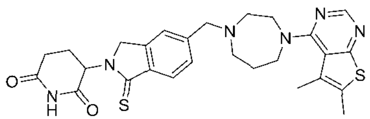 | 3-(5-((4-(5,6-dimethylthieno[2,3-d]pyrimidin-4-yl)-1,4-diazepan-1-yl)methyl)-1-thioxoisoindolin-2-yl)piperidine-2,6-dione | 3-(5-((4-(5,6-dimethylthieno[2,3-d]pyrimidin-4-yl)-1,4-diazepan-1-yl)methyl)-1-thioxoisoindolin-2-yl)piperidine-2,6-dione |
| GT-0930 4 | 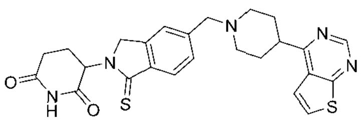 | 3-(5-((4-(thieno[2,3-d]pyrimidin-4-yl)piperidin-1-yl) methyl)-1-thioxoisoindolin-2-yl)piperidine-2,6-dione | 3-(5-((4-(thieno[2,3-d]pyrimidin-4-yl)piperidin-1-yl) methyl)-1-thioxoisoindolin-2-yl)piperidine-2,6-dione |
| GT-0930 5 | 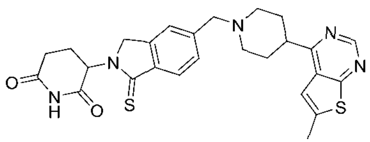 | 3-(5-((4-(6-methylthieno[2,3-d]pyrimidin-4-yl)piperidin-1-yl)methyl)-1-thioxoisoindolin-2-yl)piperidine-2,6-dione | 3-(5-((4-(6-methylthieno[2,3-d]pyrimidin-4-yl)piperidin-1-yl)methyl)-1-thioxoisoindolin-2-yl)piperidine-2,6-dione |
| | 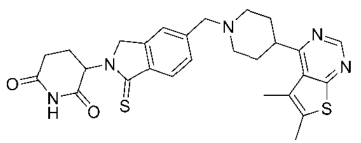 | 3-(5-((4-(5,6-dimethylthieno[2,3-d]pyrimidin-4-yl)piperidin-1-yl)methyl)-1-thioxoisoindolin-2-yl)piperidine-2,6-dione | 3-(5-((4-(5,6-dimethylthieno[2,3-d]pyrimidin-4-yl)piperidin-1-yl)methyl)-1-thioxoisoindolin-2-yl)piperidine-2,6-dione |
| GT-0930 6 | 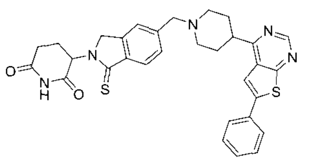 | 3-(5-((4-(6-phenylthieno[2,3-d]pyrimidin-4-yl)piperidin-1-yl)methyl)-1-thioxoisoindolin-2-yl)piperidine-2,6-dione | 3-(5-((4-(6-phenylthieno[2,3-d]pyrimidin-4-yl)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0930 7 | 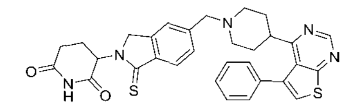 | 3-(5-((4-(5-phenylthieno[2,3-d]pyrimidin-4-yl)piperidin-1-yl)methyl)-1-thioxoisoindolin-2-yl)piperidine-2,6-dione | 3-(5-((4-(5-phenylthieno[2,3-d]pyrimidin-4-yl)piperidin-1-yl)methyl)-1-thioxoisoindolin-2-yl)piperidine-2,6-dione |
| | 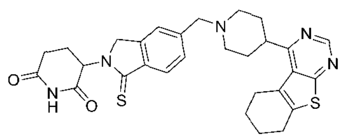 | 3-(5-((4-(5,6,7,8-tetrahydrobenzo[4,5]thieno[2 ,3-d]pyrimidin-4-yl)piperidin-1-yl)methyl)-1-thioxoisoindolin-2-yl)piperidine-2,6-dione | 3-(5-((4-(5,6,7,8-tetrahydrobenzo[4,5]thieno[2 ,3-d]pyrimidin-4-yl)piperidin-1-yl)methyl)-1-thioxoisoindolin-2-yl)piperidine-2,6-dione |
| GT-0931 1 | 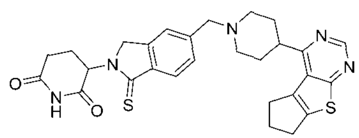 | 3-(5-((4-(6,7-dihydro-5H-cyclopenta[4,5]thieno[2,3-d] pyrimidin-4-yl)piperidin-1-yl)methyl)-1-thioxoisoindolin-2-yl)piperidine-2,6-dione | 3-(5-((4-(6,7-dihydro-5H-cyclopenta[4,5]thieno[2,3-d] pyrimidin-4-yl)piperidin-1-yl)methyl)-1-thioxoisoindolin-2-yl)piperidine-2,6-dione |
| GT-0930 8 | 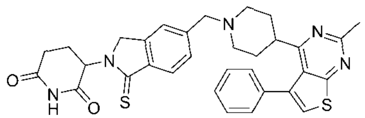 | 3-(5-((4-(2-methyl-5-phenylthieno[2,3-d]pyrimidin-4-yl) piperidin-1-yl)methyl)-1-thioxoisoindolin-2-yl)piperidine-2,6-dione | 3-(5-((4-(2-methyl-5-phenylthieno[2,3-d]pyrimidin-4-yl) piperidin-1-yl)methyl)-1-thioxoisoindolin-2-yl)piperidine-2,6-dione |
| GT-0931 0 | 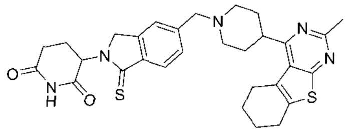 | 3-(5-((4-(2-methyl-5,6,7,8-tetrahydrobenzo[4,5]thieno [2 ,3-d]pyrimidin-4-yl)piperidin-1-yl)methyl)-1-thioxoisoindolin-2-yl)piperidine-2,6-dione | 3-(5-((4-(2-methyl-5,6,7,8-tetrahydrobenzo[4,5]thieno [2 ,3-d]pyrimidin-4-yl)piperidin-1-yl)methyl)-1-thioxoisoindolin-2-yl)piperidine-2,6-dione |
| GT-0931 2 | 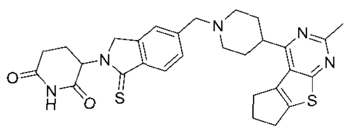 | 3-(5-((4-(2-methyl-6,7-dihydro-5H-cyclopenta[4,5]thieno[2,3-d]pyrimidin-4-yl)piperidin-1-yl)methyl)-1-thioxoisoindolin-2-yl)piperidine-2,6-dione | 3-(5-((4-(2-methyl-6,7-dihydro-5H-cyclopenta[4,5]thieno[2,3-d]pyrimidin-4-yl)piperidin-1-yl)methyl)-1-thioxoisoindolin-2-yl)piperidine-2,6-dione |
| GT-0930 9 | 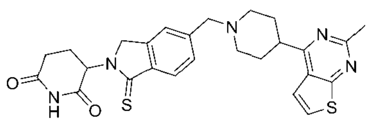 | 3-(5-((4-(2-methylthieno[2,3-d]pyrimidin-4-yl)piperidin-1-yl)methyl)-1-thioxoisoindolin-2-yl)piperidine-2,6-dione | 3-(5-((4-(2-methylthieno[2,3-d]pyrimidin-4-yl)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0931 3 | 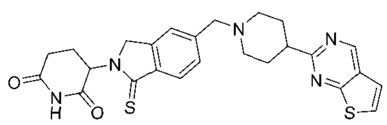 | 3-(5-((4-(thieno[2,3-d]pyrimidin-2-yl)piperidin-1-yl) methyl)-1-thioxoisoindolin-2-yl)piperidine-2,6-dione | 3-(5-((4-(thieno[2,3-d]pyrimidin-2-yl)piperidin-1-yl) methyl)-1-thioxoisoindolin-2-yl)piperidine-2,6-dione |
| | 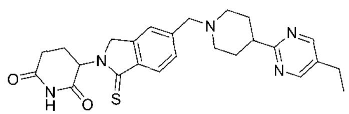 | 3-(5-((4-(5-ethylpyrimidin-2-yl)piperidin-1-yl)methyl)-1-thioxoisoindolin-2-yl)piperidine-2,6-dione | 3-(5-((4-(5-ethylpyrimidin-2-yl)piperidin-1-yl)methyl)-1-thioxoisoindolin-2-yl)piperidine-2,6-dione |
| GT-0931 4 | 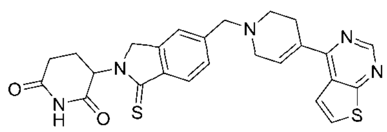 | 3-(5-((4-(thieno[2,3-d]pyrimidin-4-yl)-3,6-dihydropyridin-1(2H)-yl)methyl)-1-thioxoisoindolin-2-yl)piperidine-2,6-dione | 3-(5-((4-(thieno[2,3-d]pyrimidin-4-yl)-3,6-dihydropyridin-1(2H)-yl)methyl)-1-thioxoisoindolin-2-yl)piperidine-2,6-dione |
| GT-0931 5 | 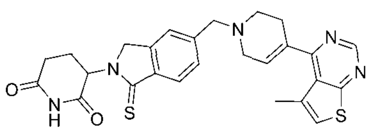 | 3-(5-((4-(5-methylthieno[2,3-d]pyrimidin-4-yl)-3,6-dihydropyridin-1(2H)-yl)methyl)-1-thioxoisoindolin-2-yl)piperidine-2,6-dione | 3-(5-((4-(5-methylthieno[2,3-d]pyrimidin-4-yl)-3,6-dihydropyridin-1(2H)-yl)methyl)-1-thioxoisoindolin-2-yl)piperidine-2,6-dione |
| GT-0931 6 | 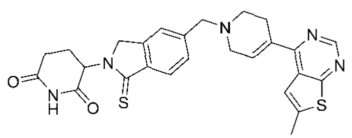 | 3-(5-((4-(6-methylthieno[2,3-d]pyrimidin-4-yl)-3,6-dihydropyridin-1(2H)-yl)methyl)-1-thioxoisoindolin-2-yl)piperidine-2,6-dione | 3-(5-((4-(6-methylthieno[2,3-d]pyrimidin-4-yl)-3,6-dihydropyridin-1(2H)-yl)methyl)-1-thioxoisoindolin-2-yl)piperidine-2,6-dione |
| GT-0931 7 | 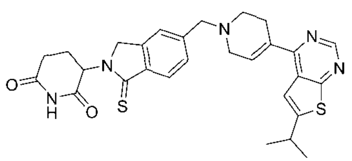 | 3-(5-((4-(6-isopropylthieno[2,3-d]pyrimidin-4-yl)-3,6-dihydropyridin-1(2H)-yl)methyl)-1-thioxoisoindolin-2-yl) piperidine-2,6-dione | 3-(5-((4-(6-isopropylthieno[2,3-d]pyrimidin-4-yl)-3,6-dihydropyridin-1(2H)-yl)methyl)-1-thioxoisoindolin-2-yl) piperidine-2,6-dione |
| GT-0931 8 | 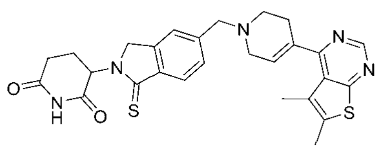 | 3-(5-((4-(5,6-dimethylthieno[2,3-d]pyrimidin-4-yl)-3,6-dihydropyridin-1(2H)-yl)methyl)-1-thioxoisoindolin-2-yl) piperidine-2,6-dione | 3-(5-((4-(5,6-dimethylthieno[2,3-d]pyrimidin-4-yl)-3,6-dihydropyridin-1(2H)-yl)methyl)-1-thioxoisoindolin-2-yl) piperidine-2,6-dione |

(continued)

| Compound No. | Structure of the compounds | The compounds' name | Chinese translation of the compounds' name |
|---|---|---|---|
| GT-0931 9 | 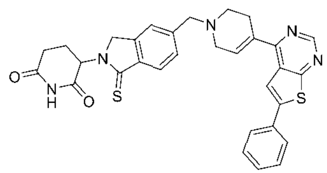 | 3-(5-((4-(6-phenylthieno[2,3-d]pyrimidin-4-yl)-3,6-dihydropyridin-1(2H)-yl)methyl)-1-thioxoisoindolin-2-yl)piperidine-2,6-dione | 3-(5-((4-(6-phenylthieno[2,3-d]pyrimidin-4-yl)-3,6-dihydropyridin-1(2H)-yl)methyl)-1-thioxoisoindolin-2-yl)piperidine-2,6-dione |
| GT-0932 0 | 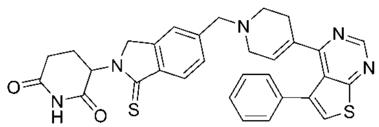 | 3-(5-((4-(5-phenylthieno[2,3-d]pyrimidin-4-yl)-3,6-dihydropyridin-1(2H)-yl)methyl)-1-thioxoisoindolin-2-yl)piperidine-2,6-dione | 3-(5-((4-(5-phenylthieno[2,3-d]pyrimidin-4-yl)-3,6-dihydropyridin-1(2H)-yl)methyl)-1-thioxoisoindolin-2-yl)piperidine-2,6-dione |
| GT-0932 4 | 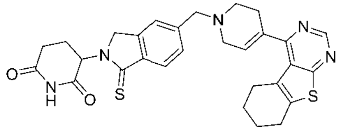 | 3-(5-((4-(5,6,7,8-tetrahydrobenzo[4,5]thieno[2 ,3-d]pyrimidin-4-yl)-3,6-dihydropyridin-1 (2H)-yl)methyl)-1-thioxoisoindolin-2-yl)piperidine-2,6-dione | 3-(5-((4-(5,6,7,8-tetrahydrobenzo[4,5]thieno[2 ,3-d]pyrimidin-4-yl)-3,6-dihydropyridin-1(2H)-yl)methyl)-1-thioxoisoindolin-2-yl)piperidine-2,6-dione |
| GT-0932 5 | 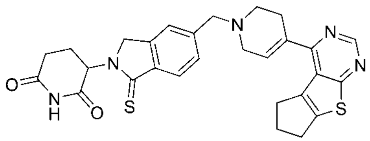 | 3-(5-((4-(6,7-dihydro-5H-cyclopenta[4,5]thieno[2,3-d] pyrimidin-4-yl)-3,6-dihydropyridin-1 (2H)-yl)methyl)-1-thioxoisoindolin-2-yl)piperidine-2,6-dione | 3-(5-((4-(6,7-dihydro-5H-cyclopenta[4,5]thieno[2,3-d] pyrimidin-4-yl)-3,6-dihydropyridin-1(2H)-yl)methyl)-1-thioxoisoindolin-2-yl)piperidine-2,6-dione |
| GT-0932 1 | 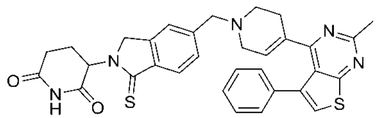 | 3-(5-((4-(2-methyl-5-phenylthieno[2,3-d]pyrimidin-4-yl)-3,6-dihydropyridin-1 (2H)-yl)methyl)-1-thioxoisoindolin-2-yl)piperidine-2,6-dione | 3-(5-((4-(2-methyl-5-phenylthieno[2,3-d]pyrimidin-4-yl)-3,6-dihydropyridin-1(2H)-yl)methyl)-1-thioxoisoindolin-2-yl)piperidine-2,6-dione |
| GT-0932 3 | 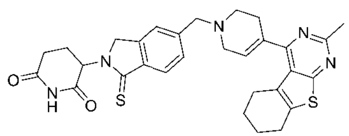 | 3-(5-((4-(2-methyl-5,6,7,8-tetrahydrobenzo[4,5]thieno [2 ,3-d]pyrimidin-4-yl)-3,6-dihydropyridin-1 (2H)-yl) methyl)-1-thioxoisoindolin-2-yl)piperidine-2,6-dione | 3-(5-((4-(2-methyl-5,6,7,8-tetrahydrobenzo[4,5]thieno [2 ,3-d]pyrimidin-4-yl)-3,6-dihydropyridin-1(2H)-yl) methyl)-1-thioxoisoindolin-2-yl)piperidine-2,6-dione |
| GT-0932 6 | 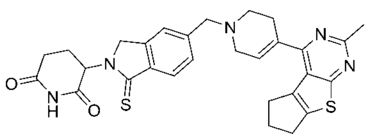 | 3-(5-((4-(2-methyl-6,7-dihydro-5H-cyclopenta[4,5]thieno[2,3-d]pyrimidin-4-yl)-3,6-dihydropyridin-1 (2H)-yl) methyl)-1-thioxoisoindolin-2-yl)piperidine-2,6-dione | 3-(5-((4-(2-methyl-6,7-dihydro-5H-cyclopenta[4,5]thieno[2,3-d]pyrimidin-4-yl)-3,6-dihydropyridin-1(2H)-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-09322 | 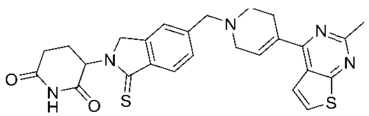 | 3-(5-((4-(2-methylthieno[2,3-d]pyrimidin-4-yl)-3,6-dihydropyridin-1(2H)-yl)methyl)-1-thioxoisoindolin-2-yl)piperidine-2,6-dione | 3-(5-((4-(2-methylthieno[2,3-d]pyrimidin-4-yl)-3,6-dihydropyridin-1(2H)-yl)methyl)-1-thioxoisoindolin-2-yl)piperidine-2,6-dione |
| GT-09327 | 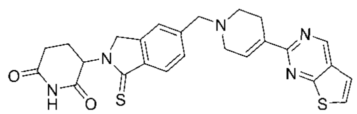 | 3-(5-((4-(thieno[2,3-d]pyrimidin-2-yl)-3,6-dihydropyridin-1 (2H)-yl)methyl)-1-thioxoisoindolin-2-yl)piperidine-2,6-dione | 3-(5-((4-(thieno[2,3-d]pyrimidin-2-yl)-3,6-dihydropyridin-1(2H)-yl)methyl)-1-thioxoisoindolin-2-yl)piperidine-2,6-dione |
| GT-09328 | 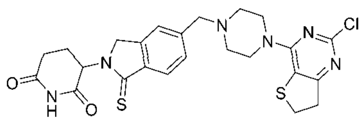 | 3-(5-((4-(2-chloro-6,7-dihydrothieno[3,2-d]pyrimidin-4-yl)piperazin-1-yl)methyl)-1-thioxoisoindolin-2-yl)piperidine-2,6-dione | 3-(5-((4-(2-chloro-6,7-dihydrothieno[3,2-d]pyrimidin-4-yl)piperazin-1-yl)methyl)-1-thioxoisoindolin-2-yl)piperidine-2,6-dione |
| GT-09329 | 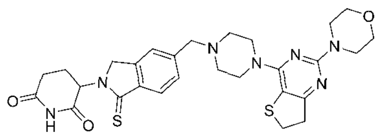 | 3-(5-((4-(2-morpholino-6,7-dihydrothieno[3,2-d]pyrimidin-4-yl)piperazin-1-yl)methyl)-1-thioxoisoindolin-2-yl) piperidine-2,6-dione | 3-(5-((4-(2-morpholino-6,7-dihydrothieno[3,2-d]pyrimidin-4-yl)piperazin-1-yl)methyl)-1-thioxoisoindolin-2-yl) piperidine-2,6-dione |
| | 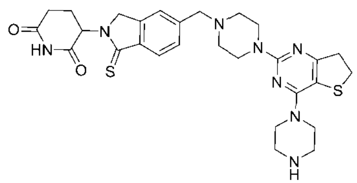 | 3-(5-((4-(4-(piperazin-1-yl)-6,7-dihydrothieno[3,2-d]pyrimidin-2-yl)piperazin-1-yl)methyl)-1-thioxoisoindolin-2-yl)piperidine-2,6-dione | 3-(5-((4-(4-(piperazin-1-yl)-6,7-dihydrothieno[3,2-d]pyrimidin-2-yl)piperazin-1-yl)methyl)-1-thioxoisoindolin-2-yl)piperidine-2,6-dione |
| GT-09330 | 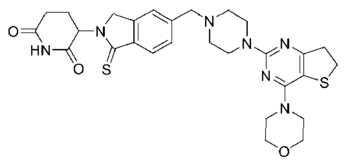 | 3-(5-((4-(4-morpholino-6,7-dihydrothieno[3,2-d]pyrimidin-2-yl)piperazin-1-yl)methyl)-1-thioxoisoindolin-2-yl) piperidine-2,6-dione | 3-(5-((4-(4-morpholino-6,7-dihydrothieno[3,2-d]pyrimidin-2-yl)piperazin-1-yl)methyl)-1-thioxoisoindolin-2-yl) piperidine-2,6-dione |
| | 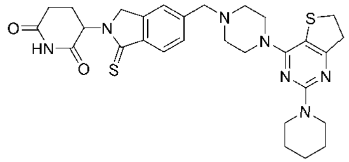 | 3-(5-((4-(2-(piperidin-1-yl)-6,7-dihydrothieno[3,2-d]pyrimidin-4-yl)piperazin-1-yl)methyl)-1-thioxoisoindolin-2-yl)piperidine-2,6-dione | 3-(5-((4-(2-(piperidin-1-yl)-6,7-dihydrothieno[3,2-d]pyrimidin-4-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 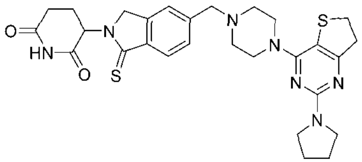 | 3-(5-((4-(2-(pyrrolidin-1-yl)-6,7-dihydrothieno[3,2-d]pyrimidin-4-yl)piperazin-1-yl)methyl)-1-thioxoisoindolin-2-yl)piperidine-2,6-dione | 3- 5-((4-(2-(pyrrolidin-1-yl)-6,7-dihydrothieno[3,2-d]pyrimidin-4-yl)piperazin-1-yl)methyl)-1-thioxoisoindolin-2-yl)piperidine-2,6-dione |
| | 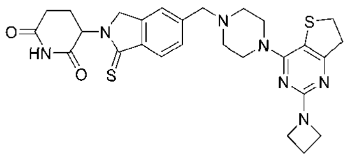 | 3-(5-((4-(2-(azetidin-1-yl)-6,7-dihydrothieno[3,2-d]pyrimidin-4-yl)piperazin-1-yl)methyl)-1-thioxoisoindolin-2-yl)piperidine-2,6-dione | 3-(5-((4-(2-(azetidin-1-yl)-6,7-dihydrothieno[3,2-d]pyrimidin-4-yl)piperazin-1-yl)methyl)-1-thioxoisoindolin-2-yl)piperidine-2,6-dione |
| | 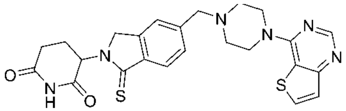 | 3-(5-((4-(thieno[3,2-d]pyrimidin-4-yl)piperazin-1-yl)methyl)-1-thioxoisoindolin-2-yl)piperidine-2,6-dione | 3-(5-((4-(thieno[3,2-d]pyrimidin-4-yl)piperazin-1-yl)methyl)-1-thioxoisoindolin-2-yl)piperidine-2,6-dione |
| GT-09331 | 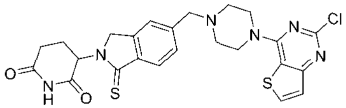 | 3-(5-((4-(2-chlorothieno[3,2-d]pyrimidin-4-yl)piperazin-1-yl)methyl)-1-thioxoisoindolin-2-yl)piperidine-2,6-dione | 3-(5-((4-(2-chlorothieno[3,2-d]pyrimidin-4-yl)piperazin-1-yl)methyl)-1-thioxoisoindolin-2-yl)piperidine-2,6-dione |
| GT-09333 | 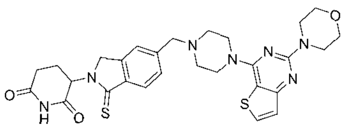 | 3-(5-((4-(2-morpholinothieno[3,2-d]pyrimidin-4-yl)piperazin-1-yl)methyl)-1-thioxoisoindolin-2-yl)piperidine-2,6-dione | 3-(5-((4-(2-morpholinothieno[3,2-d]pyrimidin-4-yl)piperazin-1-yl)methyl)-1-thioxoisoindolin-2-yl)piperidine-2,6-dione |
| GT-09365 | 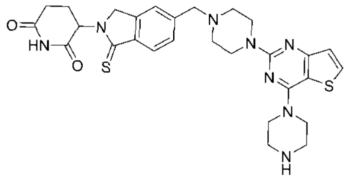 | 3-(5-((4-(4-(piperazin-1-yl)thieno[3,2-d]pyrimidin-2-yl)piperazin-1-yl)methyl)-1-thioxoisoindolin-2-yl)piperidine-2,6-dione | 3- 5-((4-(4-(piperazin-1-yl)thieno[3,2-d]pyrimidin-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 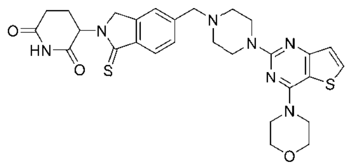 | 3-(5-((4-(4-morpholinothieno[3,2-d]pyrimidin-2-yl)piperazin-1-yl)methyl)-1-thioxoisoindolin-2-yl)piperidine-2,6-dione | 3-(5-((4-(4-morpholinothieno[3,2-d]pyrimidin-2-yl)piperazin-1-yl)methyl)-1-thioxoisoindolin-2-yl)piperidine-2,6-dione |
| GT-05088 | 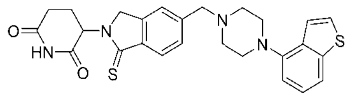 | 3-(5-((4-(benzo[b]thiophen-4-yl)piperazin-1-yl) methyl)-1-thioxoisoindolin-2-yl)piperidine-2,6-dione | 3-(5-((4-(benzo[b]thiophen-4-yl)piperazin-1-yl) methyl)-1-thioxoisoindolin-2-yl)piperidine-2,6-dione |
| GT-05090 | 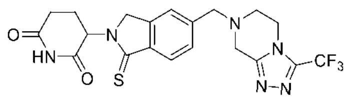 | 3-(1-thioxo-5-((3-(trifluoromethyl)-5,6-dihydro-[1,2,4] triazolo[4,3-a]pyrazin-7(8H)-yl)methyl)isoindolin-2-yl) piperidine-2,6-dione | 3-(1-thioxo-5-((3-(trifluoromethyl)-5,6-dihydro-[1,2,4] triazolo[4,3-a]pyrazin-7(8H)-yl)methyl)isoindolin-2-yl) piperidine-2,6-dione |
| GT-05092 | 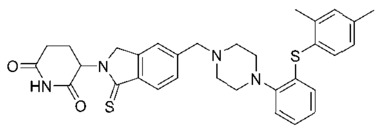 | 3-(5-((4-(2-((2,4-dimethylphenyl)thio)phenyl) piperazin-1-yl)methyl)-1-thioxoisoindolin-2-yl)piperidine-2,6-dione | 3-(5-((4-(2-((2,4-dimethylphenyl)thio)phenyl) piperazin-1-yl)methyl)-1-thioxoisoindolin-2-yl)piperidine-2,6-dione |
| GT-05093 | 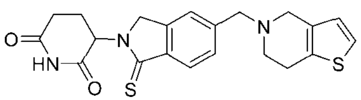 | 3-(5-((6,7-dihydrothieno[3,2-c]pyridin-5(4H)-yl) methyl)-1-thioxoisoindolin-2-yl)piperidine-2,6-dione | 3-(5-((6,7-dihydrothieno[3,2-c]pyridin-5(4H)-yl) methyl)-1-thioxoisoindolin-2-yl)piperidine-2,6-dione |
| GT-05091 | 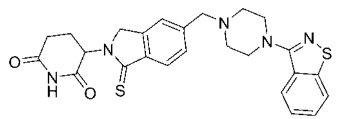 | 3-(5-((4-(benzo[d]isothiazol-3-yl)piperazin-1-yl) methyl)-1-thioxoisoindolin-2-yl)piperidine-2,6-dione | 3-(5-((4-(benzo[d]isothiazol-3-yl)piperazin-1-yl) methyl)-1-thioxoisoindolin-2-yl)piperidine-2,6-dione |
| | 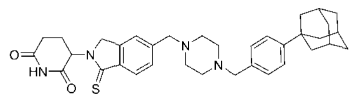 | 3-(5-((4-(4-(adamantan-1-yl)benzyl)piperazin-1-yl) methyl)-1-thioxoisoindolin-2-yl)piperidine-2,6-dione | 3-(5-((4-(4-(adamantan-1-yl)benzyl)piperazin-1-yl) methyl)-1-thioxoisoindolin-2-yl)piperidine-2,6-dione |
| | 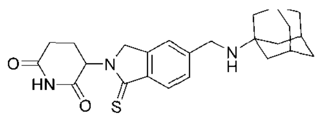 | 3-(5-(((adamantan-1-yl)amino)methyl)-1-thioxoisoindolin-2-yl)piperidine-2,6-dione | 3-(5-(((adamantan-1-yl)amino)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 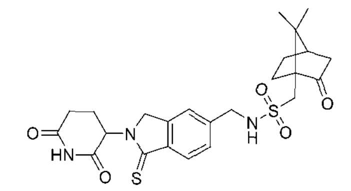 | 1-((1R,4R)-7,7-dimethyl-2-oxobicyclo[2.2.1]heptan-1-yl)-N-((2-(2,6-dioxopiperidin-3-yl)-1-thioxoisoindolin-5-yl)methyl)methanesulfonami de | 1-((1R,4R)-7,7-dimethyl-2-oxobicyclo[2.2.1]heptan-1-yl)-N-((2-(2,6-dioxopiperidin-3-yl)-1-thioxoisoindolin-5-yl)methyl)methanesulfonami de |
| | 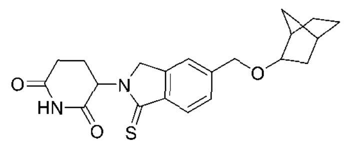 | 3-(5-((((1R,4S)-bicyclo[2.2.1]heptan-2-yl)oxy) methyl)-1-thioxoisoindolin-2-yl)piperidine-2,6-dione | 3-(5-((((1R,4S)-bicyclo[2.2.1]heptan-2-yl)oxy) methyl)-1-thioxoisoindolin-2-yl)piperidine-2,6-dione |
| | 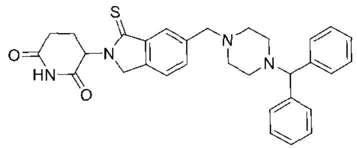 | 3-(6-((4-benzhydrylpiperazin-1-yl)methyl)-1-thioxoisoindolin-2-yl)piperidine-2,6-dione | 3-(6-((4-benzhydrylpiperazin-1-yl)methyl)-1-thioxoisoindolin-2-yl)piperidine-2,6-dione |
| GT-05041 | 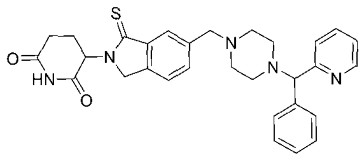 | 3-(6-((4-(phenyl(pyridin-2-yl)methyl)piperazin-1-yl) methyl)-1-thioxoisoindolin-2-yl)piperidine-2,6-dione | 3-(6-((4-(phenyl(pyridin-2-yl)methyl)piperazin-1-yl) methyl)-1-thioxoisoindolin-2-yl)piperidine-2,6-dione |
| | 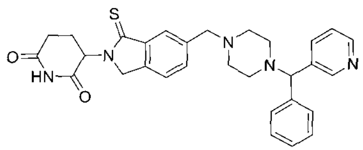 | 3-(6-((4-(phenyl(pyridin-3-yl)methyl)piperazin-1-yl) methyl)-1-thioxoisoindolin-2-yl)piperidine-2,6-dione | 3-(6-((4-(phenyl(pyridin-3-yl)methyl)piperazin-1-yl) methyl)-1-thioxoisoindolin-2-yl)piperidine-2,6-dione |
| | 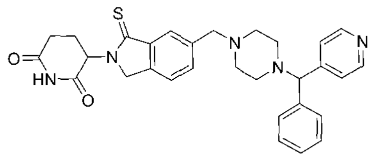 | 3-(6-((4-(phenyl(pyridin-4-yl)methyl)piperazin-1-yl) methyl)-1-thioxoisoindolin-2-yl)piperidine-2,6-dione | 3-(6-((4-(phenyl(pyridin-4-yl)methyl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 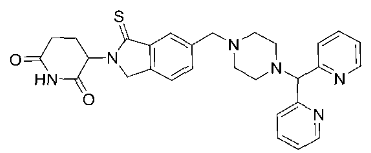 | 3-(6-((4-(di(pyridin-2-yl)methyl)piperazin-1-yl) methyl)-1-thioxoisoindolin-2-yl)piperidine-2,6-dione | 3-(6-((4-(di(pyridin-2-yl)methyl)piperazin-1-yl) methyl)-1-thioxoisoindolin-2-yl)piperidine-2,6-dione |
| | 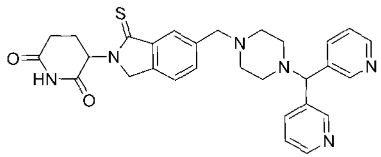 | 3-(6-((4-(di(pyridin-3-yl)methyl)piperazin-1-yl) methyl)-1-thioxoisoindolin-2-yl)piperidine-2,6-dione | 3-(6-((4-(di(pyridin-3-yl)methyl)piperazin-1-yl) methyl)-1-thioxoisoindolin-2-yl)piperidine-2,6-dione |
| | 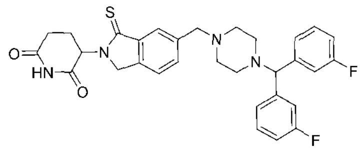 | 3-(6-((4-(bis(3-fluorophenyl)methyl)piperazi n-1-yl) methyl)-1-thioxoisoindolin-2-yl)piperidine-2,6-dione | 3-(6-((4-(bis(3-fluorophenyl)methyl)piperazi n-1-yl) methyl)-1-thioxoisoindolin-2-yl)piperidine-2,6-dione |
| | 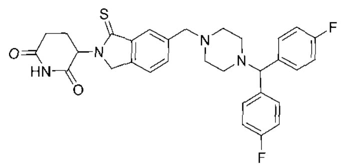 | 3-(6-((4-(bis(4-fluorophenyl)methyl)piperazi n-1-yl) methyl)-1-thioxoisoindolin-2-yl)piperidine-2,6-dione | 3-(6-((4-(bis(4-fluorophenyl)methyl)piperazi n-1-yl) methyl)-1-thioxoisoindolin-2-yl)piperidine-2,6-dione |
| | 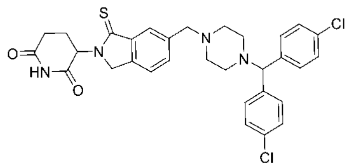 | 3-(6-((4-(bis(4-chlorophenyl)methyl)piperaz in-1-yl) methyl)-1-thioxoisoindolin-2-yl)piperidine-2,6-dione | 3-(6-((4-(bis(4-chlorophenyl)methyl)piperaz in-1-yl) methyl)-1-thioxoisoindolin-2-yl)piperidine-2,6-dione |
| | 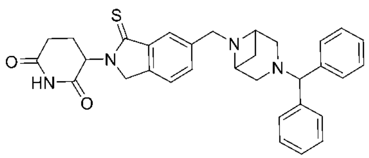 | 3-(6-((3-benzhydryl-3,6-diazabicyclo[3.1.1]heptan-6-yl) methyl)-1-thioxoisoindolin-2-yl)piperidine-2,6-dione | 3-(6-((3-benzhydryl-3,6-diazabicyclo[3.1.1]heptan-6-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 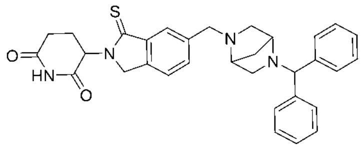 | 3-(6-((5-benzhydryl-2,5-diazabicyclo[2.2.1]heptan-2-yl) methyl)-1-thioxoisoindolin-2-yl)piperidine-2,6-dione | 3-(6-((5-benzhydryl-2,5-diazabicyclo[2.2.1]heptan-2-yl) methyl)-1-thioxoisoindolin-2-yl)piperidine-2,6-dione |
| | 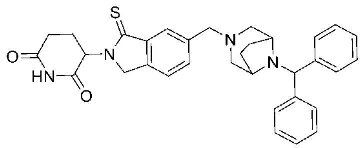 | 3-(6-((8-benzhydryl-3,8-diazabicyclo[3.2.1]octan-3-yl) methyl)-1-thioxoisoindolin-2-yl)piperidine-2,6-dione | 3-(6-((8-benzhydryl-3,8-diazabicyclo[3.2.1]octan-3-yl) methyl)-1-thioxoisoindolin-2-yl)piperidine-2,6-dione |
| | 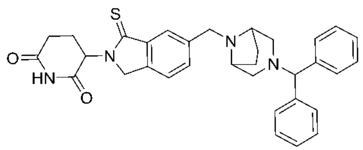 | 3-(6-((3-benzhydryl-3,8-diazabicyclo[3.2.1]octan-8-yl) methyl)-1-thioxoisoindolin-2-yl)piperidine-2,6-dione | 3-(6-((3-benzhydryl-3,8-diazabicyclo[3.2.1]octan-8-yl) methyl)-1-thioxoisoindolin-2-yl)piperidine-2,6-dione |
| | 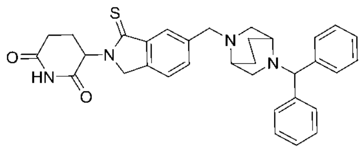 | 3-(6-((5-benzhydryl-2,5-diazabicyclo[2.2.2]octan-2-yl) methyl)-1-thioxoisoindolin-2-yl)piperidine-2,6-dione | 3-(6-((5-benzhydryl-2,5-diazabicyclo[2.2.2]octan-2-yl) methyl)-1-thioxoisoindolin-2-yl)piperidine-2,6-dione |
| | 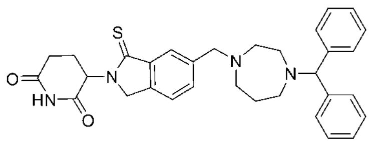 | 3-(6-((4-benzhydryl-1,4-diazepan-1-yl)methyl)-1-thioxoisoindolin-2-yl)piperidine-2,6-dione | 3-(6-((4-benzhydryl-1,4-diazepan-1-yl)methyl)-1-thioxoisoindolin-2-yl)piperidine-2,6-dione |
| | 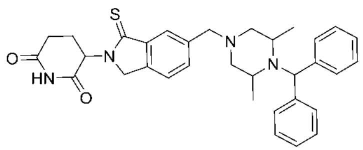 | 3-(6-((4-benzhydryl-3,5-dimethylpiperazin-1-yl) methyl)-1-thioxoisoindolin-2-yl)piperidine-2,6-dione | 3-(6-((4-benzhydryl-3,5-dimethylpiperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 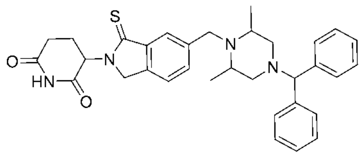 | 3-(6-((4-benzhydryl-2,6-dimethylpiperazin-1-yl) methyl)-1-thioxoisoindolin-2-yl)piperidine-2,6-dione | 3-(6-((4-benzhydryl-2,6-dimethylpiperazin-1-yl) methyl)-1-thioxoisoindolin-2-yl)piperidine-2,6-dione |
| | 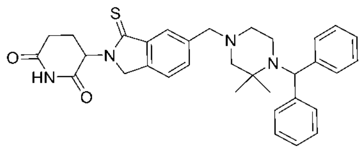 | 3-(6-((4-benzhydryl-3,3-dimethylpiperazin-1-yl) methyl)-1-thioxoisoindolin-2-yl)piperidine-2,6-dione | 3-(6-((4-benzhydryl-3,3-dimethylpiperazin-1-yl) methyl)-1-thioxoisoindolin-2-yl)piperidine-2,6-dione |
| | 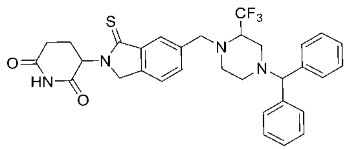 | 3-(6-((4-benzhydryl-2-(trifluoromethyl)piperazin-1-yl) methyl)-1-thioxoisoindolin-2-yl)piperidine-2,6-dione | 3-(6-((4-benzhydryl-2-(trifluoromethyl)piperazin-1-yl) methyl)-1-thioxoisoindolin-2-yl)piperidine-2,6-dione |
| | 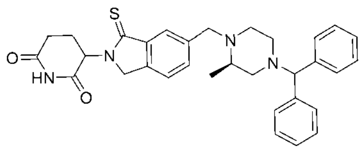 | 3-(6-(((R)-4-benzhydryl-2-methylpiperazin-1-yl) methyl)-1-thioxoisoindolin-2-yl)piperidine-2,6-dione | 3-(6-(((R)-4-benzhydryl-2-methylpiperazin-1-yl) methyl)-1-thioxoisoindolin-2-yl)piperidine-2,6-dione |
| | 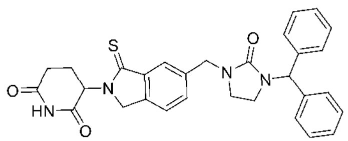 | 3-(6-((3-benzhydryl-2-oxoimidazolidin-1-yl)methyl)-1-thioxoisoindolin-2-yl)piperidine-2,6-dione | 3-(6-((3-benzhydryl-2-oxoimidazolidin-1-yl)methyl)-1-thioxoisoindolin-2-yl)piperidine-2,6-dione |
| | 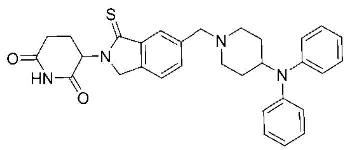 | 3-(6-((4-(diphenylamino)piperidin-1-yl)methyl)-1-thioxoisoindolin-2-yl)piperidine-2,6-dione | 3-(6-((4-(diphenylamino)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 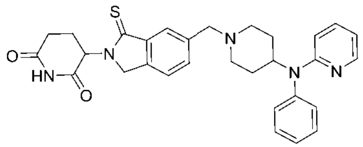 | 3-(6-((4-(phenyl(pyridin-2-yl)amino)piperidin-1-yl) methyl)-1-thioxoisoindolin-2-yl)piperidine-2,6-dione | 3-(6-((4-(phenyl(pyridin-2-yl)amino)piperidin-1-yl) methyl)-1-thioxoisoindolin-2-yl)piperidine-2,6-dione |
| | 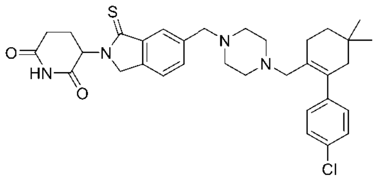 | 3-(6-((4-((4'-chloro-5,5-dimethyl-3,4,5,6-tetrahydro-[1,1'-biphenyl]-2-yl)methyl)piperazin-1-yl) methyl)-1-thioxoisoindolin-2-yl)piperidine-2,6-dione | 3-(6-((4-((4'-chloro-5,5-dimethyl-3,4,5,6-tetrahydro-[1,1'-biphenyl]-2-yl)methyl)piperazin-1-yl) methyl)-1-thioxoisoindolin-2-yl)piperidine-2,6-dione |
| | 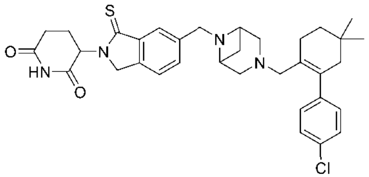 | 3-(6-((3-((4'-chloro-5,5-dimethyl-3,4,5,6-tetrahydro-[1,1'-biphenyl]-2-yl)methyl)-3,6-diazabicyclo[3.1.1] heptan-6-yl)methyl)-1-thioxoisoindolin-2-yl)piperidine-2,6-dione | 3-(6-((3-((4'-chloro-5,5-dimethyl-3,4,5,6-tetrahydro-[1,1-biphenyl]-2-yl)methyl)-3,6-diazabicyclo[3.1.1] heptan-6-yl)methyl)-1-thioxoisoindolin-2-yl)piperidine-2,6-dione |
| | 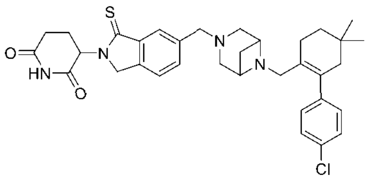 | 3-(6-((6-((4'-chloro-5,5-dimethyl-3,4,5,6-tetrahydro-[1,1'-biphenyl]-2-yl)methyl)-3,6-diazabicyclo[3.1.1] heptan-3-yl)methyl)-1-thioxoisoindolin-2-yl)piperidine-2,6-dione | 3-(6-((6-((4'-chloro-5,5-dimethyl-3,4,5,6-tetrahydro-[1,1'-biphenyl]-2-yl)methyl)-3,6-diazabicyclo[3.1.1] heptan-3-yl)methyl)-1-thioxoisoindolin-2-yl)piperidine-2,6-dione |
| | 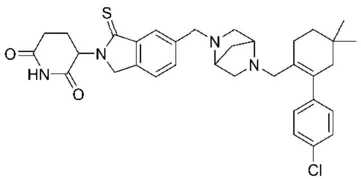 | 3-(6-((5-((4'-chloro-5,5-dimethyl-3,4,5,6-tetrahydro-[1,1'-biphenyl]-2-yl)methyl)-2,5-diazabicyclo[2.2.1] heptan-2-yl)methyl)-1-thioxoisoindolin-2-yl)piperidine-2,6-dione | 3-(6-((5-((4'-chloro-5,5-dimethyl-3,4,5,6-tetrahydro-[1,1'-biphenyl]-2-yl)methyl)-2,5-diazabicyclo[2.2.1] heptan-2-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 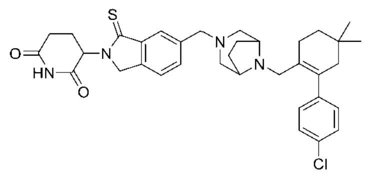 | 3-(6-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-thioxoisoindolin-2-yl)piperidine-2,6-dione | 3-(6-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-thioxoisoindolin-2-yl)piperidine-2,6-dione |
| | 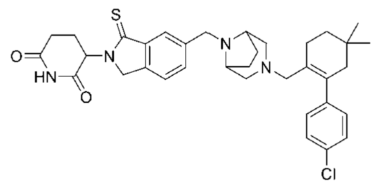 | 3-(6-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-thioxoisoindolin-2-yl)piperidine-2,6-dione | 3-(6-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-thioxoisoindolin-2-yl)piperidine-2,6-dione |
| | 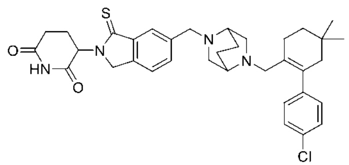 | 3-(6-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-thioxoisoindolin-2-yl)piperidine-2,6-dione | 3-(6-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-thioxoisoindolin-2-yl)piperidine-2,6-dione |
| | 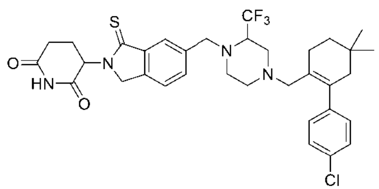 | 3-(6-((4-((4'-chloro-5,5-dimethyl-3,4,5,6-tetrahydro-[1,1'-biphenyl]-2-yl)methyl)-2-(trifluoromethyl)piperazin-1-yl)methyl)-1-thioxoisoindolin-2-yl)piperidine-2,6-dione | 3-(6-((4-((4'-chloro-5,5-dimethyl-3,4,5,6-tetrahydro-[1,1'-biphenyl]-2-yl)methyl)-2-(trifluoromethyl)piperazin-1-yl)methyl)-1-thioxoisoindolin-2-yl)piperidine-2,6-dione |
| | 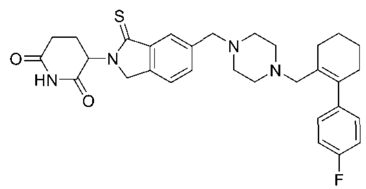 | 3-(6-((4-((4'-fluoro-3,4,5,6-tetrahydro-[1,1'-biphenyl]-2-yl)methyl)piperazin-1-yl)methyl)-1-thioxoisoindolin-2-yl)piperidine-2,6-dione | 3-·(6-((4-((4'-fluoro-3,4,5,6-tetrahydro-[1,1'-biphenyl]-2-yl)methyl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 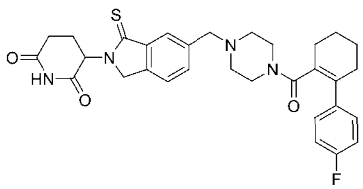 | 3-(6-((4-(4'-fluoro-3,4,5,6-tetrahydro-[1,1'-biphenyl]-2-carbonyl)piperazin-1-yl)methyl)-1-thioxoisoindolin-2-yl) piperidine-2,6-dione | 3-(6-((4-(4'-fluoro-3,4,5,6-tetrahydro-[1,1'-biphenyl]-2-carbonyl)piperazin-1-yl)methyl)-1-thioxoisoindolin-2-yl) piperidine-2,6-dione |
| | 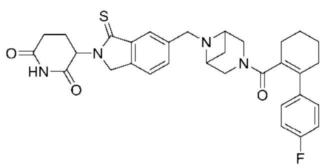 | 3-(6-((3-(4'-fluoro-3,4,5,6-tetrahydro-[1,1'-biphenyl]-2-carbonyl)-3,6-diazabicyclo[3.1.1]heptan-6-yl) methyl)-1-thioxoisoindolin-2-yl)piperidine-2,6-dione | 3-(6-((3-(4'-fluoro-3,4,5,6-tetrahydro-[1,1'-biphenyl]-2-carbonyl)-3,6-diazabicyclo[3.1.1]heptan-6-yl) methyl)-1-thioxoisoindolin-2-yl)piperidine-2,6-dione |
| | 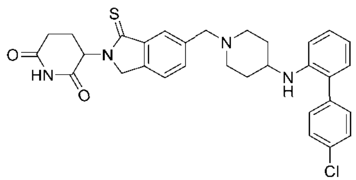 | 3-(6-((4-((4'-chloro-[1,1'-biphenyl]-2-yl)amino)piperidin-1-yl)methyl)-1-thioxoisoindolin-2-yl)piperidine-2,6-dione | 3-(6-((4-((4'-chloro-[1,1'-biphenyl]-2-yl)amino)piperidin-1-yl)methyl)-1-thioxoisoindolin-2-yl)piperidine-2,6-dione |
| | 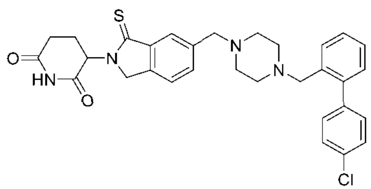 | 3-(6-((4-((4'-chloro-[1,1'-biphenyl]-2-yl)methyl)piperazin-1-yl)methyl)-1-thioxoisoindolin-2-yl)piperidine-2,6-dione | 3-(6-((4-((4'-chloro-[1,1'-biphenyl]-2-yl)methyl)piperazin-1-yl)methyl)-1-thioxoisoindolin-2-yl)piperidine-2,6-dione |
| | 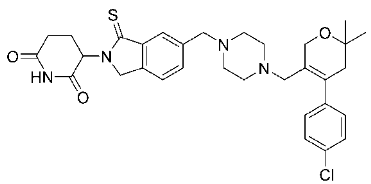 | 3-(6-((4-((4-(4-chlorophenyl)-6,6-dimethyl-5,6-dihydro-2H-pyran-3-yl)methyl)piperazin-1-yl)methyl)-1-thioxoisoindolin-2-yl)piperidine-2,6-dione | 3-(6-((4-((4-(4-chlorophenyl)-6,6-dimethyl-5,6-dihydro-2H-pyran-3-yl)methyl)piperazin-1-yl)methyl)-1-thioxoisoindolin-2-yl)piperidine-2,6-dione |
| | 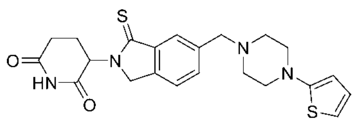 | 3-(6-((4-(thiophen-2-yl)piperazin-1-yl)methyl)-1-thioxoisoindolin-2-yl)piperidine-2,6-dione | 3-(6-((4-(thiophen-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 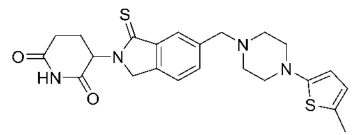 | 3-(6-((4-(5-methylthiophen-2-yl)piperazin-1-yl) methyl)-1-thioxoisoindolin-2-yl)piperidine-2,6-dione | 3-(6-((4-(5-methylthiophen-2-yl)piperazin-1-yl) methyl)-1-thioxoisoindolin-2-yl)piperidine-2,6-dione |
| | 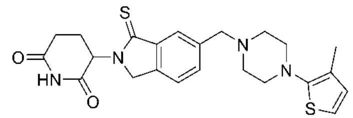 | 3-(6-((4-(3-methylthiophen-2-yl)piperazin-1-yl) methyl)-1-thioxoisoindolin-2-yl)piperidine-2,6-dione | 3-(6-((4-(3-methylthiophen-2-yl)piperazin-1-yl) methyl)-1-thioxoisoindolin-2-yl)piperidine-2,6-dione |
| | 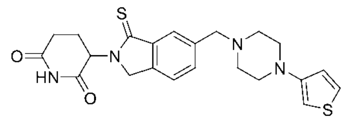 | 3-(6-((4-(thiophen-3-yl)piperazin-1-yl)methyl)-1-thioxoisoindolin-2-yl)piperidine-2,6-dione | 3-(6-((4-(thiophen-3-yl)piperazin-1-yl)methyl)-1-thioxoisoindolin-2-yl)piperidine-2,6-dione |
| | 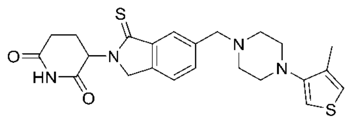 | 3-(6-((4-(4-methylthiophen-3-yl)piperazin-1-yl) methyl)-1-thioxoisoindolin-2-yl)piperidine-2,6-dione | 3-(6-((4-(4-methylthiophen-3-yl)piperazin-1-yl) methyl)-1-thioxoisoindolin-2-yl)piperidine-2,6-dione |
| | 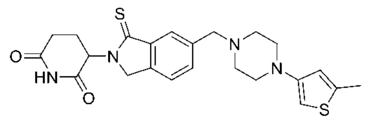 | 3-(6-((4-(5-methylthiophen-3-yl)piperazin-1-yl) methyl)-1-thioxoisoindolin-2-yl)piperidine-2,6-dione | 3-(6-((4-(5-methylthiophen-3-yl)piperazin-1-yl) methyl)-1-thioxoisoindolin-2-yl)piperidine-2,6-dione |
| | 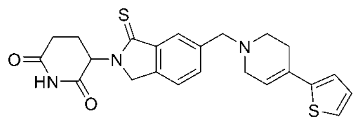 | 3-(6-((4-(thiophen-2-yl)-3,6-dihydropyridin-1 (2H)-yl) methyl)-1-thioxoisoindolin-2-yl)piperidine-2,6-dione | 3-(6-((4-(thiophen-2-yl)-3,6-dihydropyridin-1(2H)-yl) methyl)-1-thioxoisoindolin-2-yl)piperidine-2,6-dione |
| | 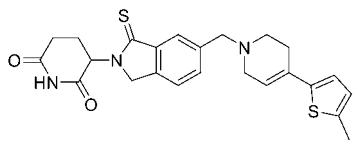 | 3-(6-((4-(5-methylthiophen-2-yl)-3,6-dihydropyridin-1(2H)-yl)methyl)-1-thioxoisoindolin-2-yl)piperidine-2,6-dione | 3-(6-((4-(5-methylthiophen-2-yl)-3,6-dihydropyridin-1(2H)-yl)methyl)-1-thioxoisoindolin-2-yl)piperi-dine-2,6-dione |

(continued)

| Compound No. | Structure of the compounds | The compounds' name | Chinese translation of the compounds' name |
|---|---|---|---|
| GT-05032 | 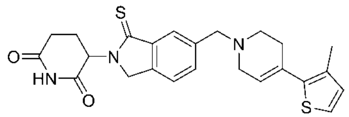 | 3-(6-((4-(3-methylthiophen-2-yl)-3,6-dihydropyridin-1(2H)-yl)methyl)-1-thioxoisoindolin-2-yl)piperidine-2,6-dione | 3-(6-((4-(3-methylthiophen-2-yl)-3,6-dihydropyridin-1(2H)-yl)methyl)-1-thioxoisoindolin-2-yl)piperidine-2,6-dione |
| | 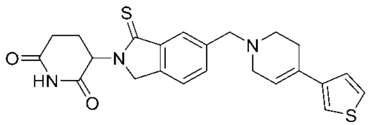 | 3-(6-((4-(thiophen-3-yl)-3,6-dihydropyridin-1 (2H)-yl)methyl)-1-thioxoisoindolin-2-yl)piperidine-2,6-dione | 3-(6-((4-(thiophen-3-yl)-3,6-dihydropyridin-1(2H)-yl)methyl)-1-thioxoisoindolin-2-yl)piperidine-2,6-dione |
| | 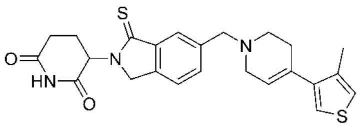 | 3-(6-((4-(4-methylthiophen-3-yl)-3,6-dihydropyridin-1(2H)-yl)methyl)-1-thioxoisoindolin-2-yl)piperidine-2,6-dione | 3-(6-((4-(4-methylthiophen-3-yl)-3,6-dihydropyridin-1(2H)-yl)methyl)-1-thioxoisoindolin-2-yl)piperidine-2,6-dione |
| | 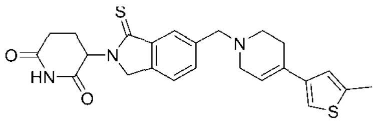 | 3-(6-((4-(5-methylthiophen-3-yl)-3,6-dihydropyridin-1(2H)-yl)methyl)-1-thioxoisoindolin-2-yl)piperidine-2,6-dione | 3-(6-((4-(5-methylthiophen-3-yl)-3,6-dihydropyridin-1(2H)-yl)methyl)-1-thioxoisoindolin-2-yl)piperidine-2,6-dione |
| | 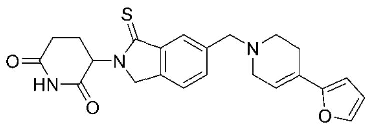 | 3-(6-((4-(furan-2-yl)-3,6-dihydropyridin-1 (2H)-yl)methyl)-1-thioxoisoindolin-2-yl)piperidine-2,6-dione | 3-(6-((4-(furan-2-yl)-3,6-dihydropyridin-1(2H)-yl)methyl)-1-thioxoisoindolin-2-yl)piperidine-2,6-dione |
| | 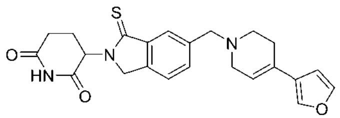 | 3-(6-((4-(furan-3-yl)-3,6-dihydropyridin-1 (2H)-yl)methyl)-1-thioxoisoindolin-2-yl)piperidine-2,6-dione | 3-(6-((4-(furan-3-yl)-3,6-dihydropyridin-1(2H)-yl)methyl)-1-thioxoisoindolin-2-yl)piperidine-2,6-dione |
| | 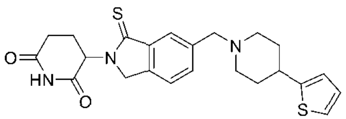 | 3-(6-((4-(thiophen-2-yl)piperidin-1-yl)methyl)-1-thioxoisoindolin-2-yl)piperidine-2,6-dione | 3-(6-((4-(thiophen-2-yl)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 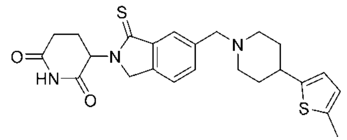 | 3-(6-((4-(5-methylthiophen-2-yl)piperidin-1-yl)methyl)-1-thioxoisoindolin-2-yl)piperidine-2,6-dione | 3-(6-((4-(5-methylthiophen-2-yl)piperidin-1-yl)methyl)-1-thioxoisoindolin-2-yl)piperidine-2,6-dione |
| | 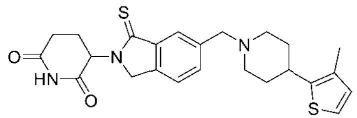 | 3-(6-((4-(3-methylthiophen-2-yl)piperidin-1-yl)methyl)-1-thioxoisoindolin-2-yl)piperidine-2,6-dione | 3-(6-((4-(3-methylthiophen-2-yl)piperidin-1-yl)methyl)-1-thioxoisoindolin-2-yl)piperidine-2,6-dione |
| | 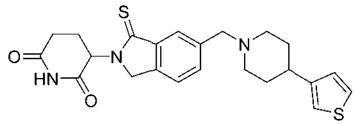 | 3-(6-((4-(thiophen-3-yl)piperidin-1-yl)methyl)-1-thioxoisoindolin-2-yl)piperidine-2,6-dione | 3-(6-((4-(thiophen-3-yl)piperidin-1-yl)methyl)-1-thioxoisoindolin-2-yl)piperidine-2,6-dione |
| | 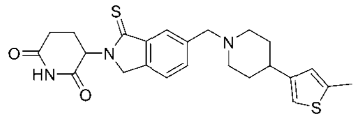 | 3-(6-((4-(5-methylthiophen-3-yl)piperidin-1-yl)methyl)-1-thioxoisoindolin-2-yl)piperidine-2,6-dione | 3-(6-((4-(5-methylthiophen-3-yl)piperidin-1-yl)methyl)-1-thioxoisoindolin-2-yl)piperidine-2,6-dione |
| | 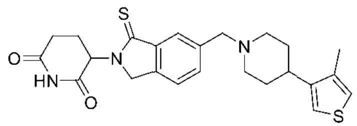 | 3-(6-((4-(4-methylthiophen-3-yl)piperidin-1-yl)methyl)-1-thioxoisoindolin-2-yl)piperidine-2,6-dione | 3-(6-((4-(4-methylthiophen-3-yl)piperidin-1-yl)methyl)-1-thioxoisoindolin-2-yl)piperidine-2,6-dione |
| | 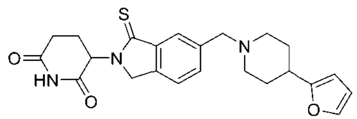 | 3-(6-((4-(furan-2-yl)piperidin-1-yl)methyl)-1-thioxoisoindolin-2-yl)piperidine-2,6-dione | 3-(6-((4-(furan-2-yl)piperidin-1-yl)methyl)-1-thioxoisoindolin-2-yl)piperidine-2,6-dione |
| | 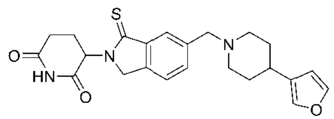 | 3-(6-((4-(furan-3-yl)piperidin-1-yl)methyl)-1-thioxoisoindolin-2-yl)piperidine-2,6-dione | 3-(6-((4-(furan-3-yl)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-05033 | 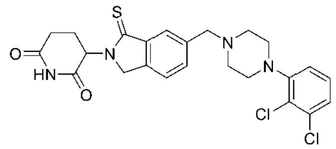 | 3-(6-((4-(2,3-dichlorophenyl)piperazin-1-yl)methyl)-1-thioxoisoindolin-2-yl)piperidine-2,6-dione | 3-(6-((4-(2,3-dichlorophenyl)piperazin-1-yl)methyl)-1-thioxoisoindolin-2-yl)piperidine-2,6-dione |
| | 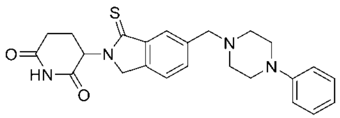 | 3-(6-((4-phenylpiperazin-1-yl)methyl)-1-thioxoisoindolin-2-yl)piperidine-2,6-dione | 3-(6-((4-phenylpiperazin-1-yl)methyl)-1-thioxoisoindolin-2-yl)piperidine-2,6-dione |
| | 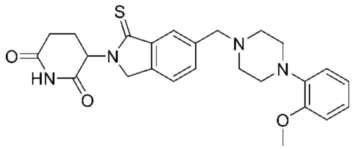 | 3-(6-((4-(2-methoxyphenyl)piperazin-1-yl)methyl)-1-thioxoisoindolin-2-yl)piperidine-2,6-dione | 3-(6-((4-(2-methoxyphenyl)piperazin-1-yl)methyl)-1-thioxoisoindolin-2-yl)piperidine-2,6-dione |
| | 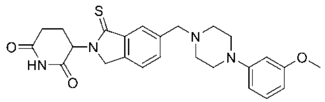 | 3-(6-((4-(3-methoxyphenyl)piperazin-1-yl)methyl)-1-thioxoisoindolin-2-yl)piperidine-2,6-dione | 3-(6-((4-(3-methoxyphenyl)piperazin-1-yl)methyl)-1-thioxoisoindolin-2-yl)piperidine-2,6-dione |
| | 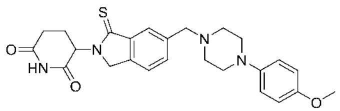 | 3-(6-((4-(4-methoxyphenyl)piperazin-1-yl)methyl)-1-thioxoisoindolin-2-yl)piperidine-2,6-dione | 3-(6-((4-(4-methoxyphenyl)piperazin-1-yl)methyl)-1-thioxoisoindolin-2-yl)piperidine-2,6-dione |
| | 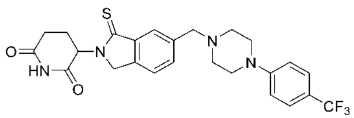 | 3-(1-thioxo-6-((4-(4-(trifluoromethyl)phenyl)piper azin-1-yl)methyl)isoindolin-2-yl)piperidine-2,6-dione | 3-(1-thioxo-6-((4-(4-(trifluoromethyl)phenyl)piper azin-1-yl)methyl)isoindolin-2-yl)piperidine-2,6-dione |
| | 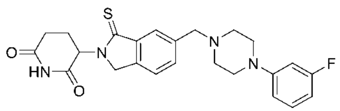 | 3-(6-((4-(3-fluorophenyl)piperazin-1-yl)methyl)-1-thioxoisoindolin-2-yl)piperidine-2,6-dione | 3-(6-((4-(3-fluorophenyl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 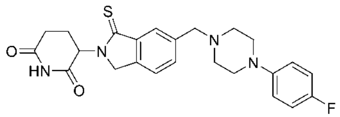 | 3-(6-((4-(4-fluorophenyl)piperazin-1-yl)methyl)-1-thioxoisoindolin-2-yl)piperidine-2,6-dione | 3-(6-((4-(4-fluorophenyl)piperazin-1-yl)methyl)-1-thioxoisoindolin-2-yl)piperidine-2,6-dione |
| | 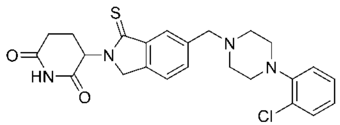 | 3-(6-((4-(2-chlorophenyl)piperazin-1-yl)methyl)-1-thioxoisoindolin-2-yl)piperidine-2,6-dione | 3-(6-((4-(2-chlorophenyl)piperazin-1-yl)methyl)-1-thioxoisoindolin-2-yl)piperidine-2,6-dione |
| | 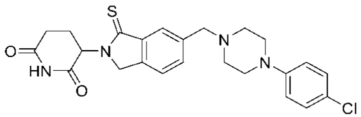 | 3-(6-((4-(4-chlorophenyl)piperazin-1-yl)methyl)-1-thioxoisoindolin-2-yl)piperidine-2,6-dione | 3-(6-((4-(4-chlorophenyl)piperazin-1-yl)methyl)-1-thioxoisoindolin-2-yl)piperidine-2,6-dione |
| | 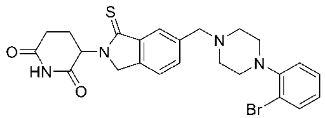 | 3-(6-((4-(2-bromophenyl)piperazin-1-yl)methyl)-1-thioxoisoindolin-2-yl)piperidine-2,6-dione | 3-(6-((4-(2-bromophenyl)piperazin-1-yl)methyl)-1-thioxoisoindolin-2-yl)piperidine-2,6-dione |
| | 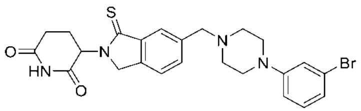 | 3-(6-((4-(3-bromophenyl)piperazin-1-yl)methyl)-1-thioxoisoindolin-2-yl)piperidine-2,6-dione | 3-(6-((4-(3-bromophenyl)piperazin-1-yl)methyl)-1-thioxoisoindolin-2-yl)piperidine-2,6-dione |
| | 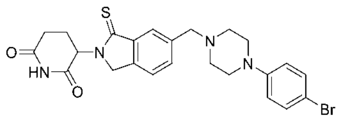 | 3-(6-((4-(4-bromophenyl)piperazin-1-yl)methyl)-1-thioxoisoindolin-2-yl)piperidine-2,6-dione | 3-(6-((4-(4-bromophenyl)piperazin-1-yl)methyl)-1-thioxoisoindolin-2-yl)piperidine-2,6-dione |
| | 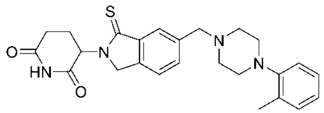 | 3-(1-thioxo-6-((4-(o-tolyl)piperazin-1-yl)methyl)isoindolin-2-yl)piperidine-2,6-dione | 3-(1-thioxo-6-((4-(o-tolyl)piperazin-1-yl)methyl)isoindolin-2-yl)piperidine-2,6-dione |
| | 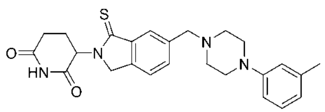 | 3-(1-thioxo-6-((4-(m-tolyl)piperazin-1-yl)methyl)isoindolin-2-yl)piperidine-2,6-dione | 3-(1-thioxo-6-((4-(m-tolyl)piperazin-1-yl)methyl)isoindolin-2-yl)piperidine-2,6-dione |

(continued)

| Compound No. | Structure of the compounds | The compounds' name | Chinese translation of the compounds' name |
|---|---|---|---|
| | 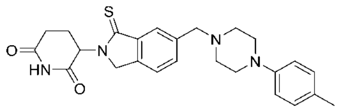 | 3-(1-thioxo-6-((4-(p-tolyl)piperazin-1-yl)methyl)isoindolin-2-yl)piperidine-2,6-dione | 3-(1-thioxo-6-((4-(p-tolyl)piperazin-1-yl)methyl)isoindolin-2-yl)piperidine-2,6-dione |
| | 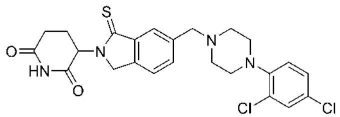 | 3-(6-((4-(2,4-dichlorophenyl)piperazin-1-yl)methyl)-1-thioxoisoindolin-2-yl)piperidine-2,6-dione | 3-(6-((4-(2,4-dichlorophenyl)piperazin-1-yl)methyl)-1-thioxoisoindolin-2-yl)piperidine-2,6-dione |
| | 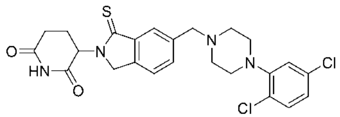 | 3-(6-((4-(2,5-dichlorophenyl)piperazin-1-yl)methyl)-1-thioxoisoindolin-2-yl)piperidine-2,6-dione | 3-(6-((4-(2,5-dichlorophenyl)piperazin-1-yl)methyl)-1-thioxoisoindolin-2-yl)piperidine-2,6-dione |
| | 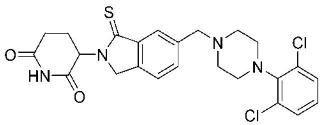 | 3-(6-((4-(2,6-dichlorophenyl)piperazin-1-yl)methyl)-1-thioxoisoindolin-2-yl)piperidine-2,6-dione | 3-(6-((4-(2,6-dichlorophenyl)piperazin-1-yl)methyl)-1-thioxoisoindolin-2-yl)piperidine-2,6-dione |
| | 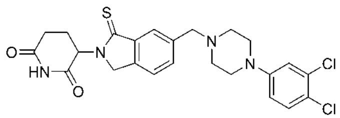 | 3-(6-((4-(3,4-dichlorophenyl)piperazin-1-yl)methyl)-1-thioxoisoindolin-2-yl)piperidine-2,6-dione | 3-(6-((4-(3,4-dichlorophenyl)piperazin-1-yl)methyl)-1-thioxoisoindolin-2-yl)piperidine-2,6-dione |
| | 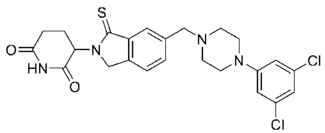 | 3-(6-((4-(3,5-dichlorophenyl)piperazin-1-yl)methyl)-1-thioxoisoindolin-2-yl)piperidine-2,6-dione | 3-(6-((4-(3,5-dichlorophenyl)piperazin-1-yl)methyl)-1-thioxoisoindolin-2-yl)piperidine-2,6-dione |
| | 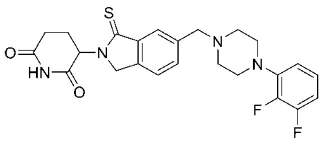 | 3-(6-((4-(2,3-difluorophenyl)piperazin-1-yl)methyl)-1-thioxoisoindolin-2-yl)piperidine-2,6-dione | 3-(6-((4-(2,3-difluorophenyl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 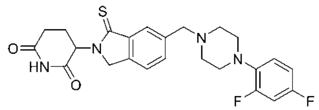 | 3-(6-((4-(2,4-difluorophenyl)piperazin-1-yl)methyl)-1-thioxoisoindolin-2-yl)piperidine-2,6-dione | 3-(6-((4-(2,4-difluorophenyl)piperazin-1-yl)methyl)-1-thioxoisoindolin-2-yl)piperidine-2,6-dione |
| | 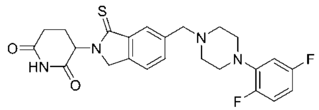 | 3-(6-((4-(2,5-difluorophenyl)piperazin-1-yl)methyl)-1-thioxoisoindolin-2-yl)piperidine-2,6-dione | 3-(6-((4-(2,5-difluorophenyl)piperazin-1-yl)methyl)-1-thioxoisoindolin-2-yl)piperidine-2,6-dione |
| | 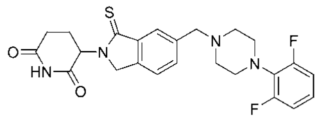 | 3-(6-((4-(2,6-difluorophenyl)piperazin-1-yl)methyl)-1-thioxoisoindolin-2-yl)piperidine-2,6-dione | 3-(6-((4-(2,6-difluorophenyl)piperazin-1-yl)methyl)-1-thioxoisoindolin-2-yl)piperidine-2,6-dione |
| | 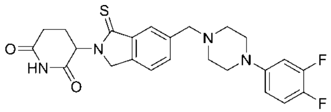 | 3-(6-((4-(3,4-difluorophenyl)piperazin-1-yl)methyl)-1-thioxoisoindolin-2-yl)piperidine-2,6-dione | 3-(6-((4-(3,4-difluorophenyl)piperazin-1-yl)methyl)-1-thioxoisoindolin-2-yl)piperidine-2,6-dione |
| | 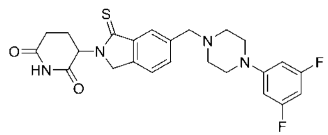 | 3-(6-((4-(3,5-difluorophenyl)piperazin-1-yl)methyl)-1-thioxoisoindolin-2-yl)piperidine-2,6-dione | 3-(6-((4-(3,5-difluorophenyl)piperazin-1-yl)methyl)-1-thioxoisoindolin-2-yl)piperidine-2,6-dione |
| | 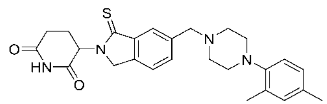 | 3-(6-((4-(2,4-dimethylphenyl)piperazin-1-yl)methyl)-1-thioxoisoindolin-2-yl)piperidine-2,6-dione | 3-(6-((4-(2,4-dimethylphenyl)piperazin-1-yl)methyl)-1-thioxoisoindolin-2-yl)piperidine-2,6-dione |
| | 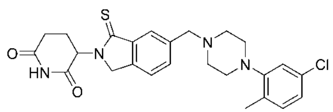 | 3-(6-((4-(5-chloro-2-methylphenyl)piperazin-1-yl) methyl)-1-thioxoisoindolin-2-yl)piperidine-2,6-dione | 3-(6-((4-(5-chloro-2-methylphenyl)piperazin-1-yl) methyl)-1-thioxoisoindolin-2-yl)piperidine-2,6-dione |
| | 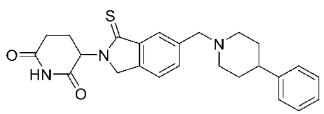 | 3-(6-((4-phenylpiperidin-1-yl)methyl)-1-thioxoisoindolin-2-yl)piperidine-2,6-dione | 3-(6-((4-phenylpiperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 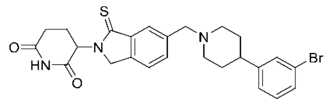 | 3-(6-((4-(3-bromophenyl)piperidin-1-yl)methyl)-1-thioxoisoindolin-2-yl)piperidine-2,6-dione | 3-(6-((4-(3-bromophenyl)piperidin-1-yl)methyl)-1-thioxoisoindolin-2-yl)piperidine-2,6-dione |
| | 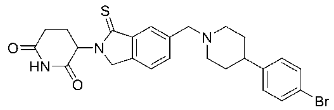 | 3-(6-((4-(4-bromophenyl)piperidin-1-yl)methyl)-1-thioxoisoindolin-2-yl)piperidine-2,6-dione | 3-(6-((4-(4-bromophenyl)piperidin-1-yl)methyl)-1-thioxoisoindolin-2-yl)piperidine-2,6-dione |
| | 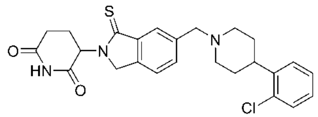 | 3-(6-((4-(2-chlorophenyl)piperidin-1-yl)methyl)-1-thioxoisoindolin-2-yl)piperidine-2,6-dione | 3-(6-((4-(2-chlorophenyl)piperidin-1-yl)methyl)-1-thioxoisoindolin-2-yl)piperidine-2,6-dione |
| | 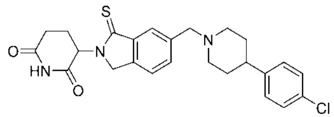 | 3-(6-((4-(4-chlorophenyl)piperidin-1-yl)methyl)-1-thioxoisoindolin-2-yl)piperidine-2,6-dione | 3-(6-((4-(4-chlorophenyl)piperidin-1-yl)methyl)-1-thioxoisoindolin-2-yl)piperidine-2,6-dione |
| | 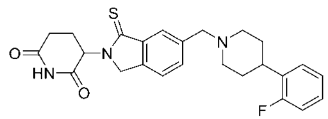 | 3-(6-((4-(2-fluorophenyl)piperidin-1-yl)methyl)-1-thioxoisoindolin-2-yl)piperidine-2,6-dione | 3-(6-((4-(2-fluorophenyl)piperidin-1-yl)methyl)-1-thioxoisoindolin-2-yl)piperidine-2,6-dione |
| | 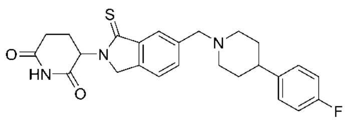 | 3-(6-((4-(4-fluorophenyl)piperidin-1-yl)methyl)-1-thioxoisoindolin-2-yl)piperidine-2,6-dione | 3-(6-((4-(4-fluorophenyl)piperidin-1-yl)methyl)-1-thioxoisoindolin-2-yl)piperidine-2,6-dione |
| | 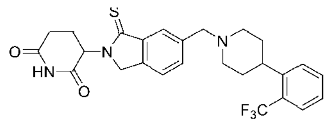 | 3-(1-thioxo-6-((4-(2-(trifluoromethyl)phenyl)piper idin-1-yl)methyl)isoindolin-2-yl)piperidine-2,6-dione | 3-(1-thioxo-6-((4-(2-(trifluoromethyl)phenyl)piper idin-1-yl)methyl)isoindolin-2-yl)piperidine-2,6-dione |
| | 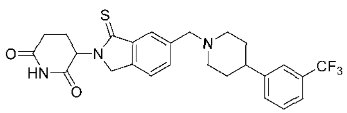 | 3-(1-thioxo-6-((4-(3-(trifluoromethyl)phenyl)piper idin-1-yl)methyl)isoindolin-2-yl)piperidine-2,6-dione | 3-(1-thioxo-6-((4-(3-(trifluoromethyl)phenyl)piper idin-1-yl)methyl)isoindolin-2-yl)piperidine-2,6-dione |

(continued)

| Compound No. | Structure of the compounds | The compounds' name | Chinese translation of the compounds' name |
|---|---|---|---|
| | 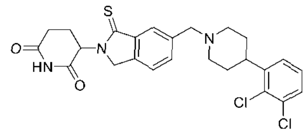 | 3-(6-((4-(2,3-dichlorophenyl)piperidin-1-yl)methyl)-1-thioxoisoindolin-2-yl)piperidine-2,6-dione | 3-(6-((4-(2,3-dichlorophenyl)piperidin-1-yl)methyl)-1-thioxoisoindolin-2-yl)piperidine-2,6-dione |
| | 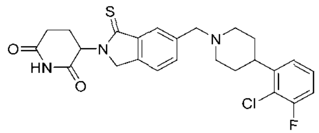 | 3-(6-((4-(2-chloro-3-fluorophenyl)piperidin-1-yl) methyl)-1-thioxoisoindolin-2-yl)piperidine-2,6-dione | 3-(6-((4-(2-chloro-3-fluorophenyl)piperidin-1-yl) methyl)-1-thioxoisoindolin-2-yl)piperidine-2,6-dione |
| | 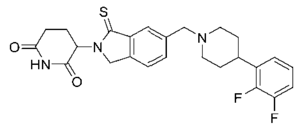 | 3-(6-((4-(2,3-difluorophenyl)piperidin-1-yl)methyl)-1-thioxoisoindolin-2-yl)piperidine-2,6-dione | 3-(6-((4-(2,3-difluorophenyl)piperidin-1-yl)methyl)-1-thioxoisoindolin-2-yl)piperidine-2,6-dione |
| | 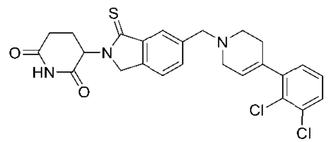 | 3-(6-((4-(2,3-dichlorophenyl)-3,6-dihydropyridin-1 (2H)-yl)methyl)-1-thioxoisoindolin-2-yl)piperidine-2,6-dione | 3-(6-((4-(2,3-dichlorophenyl)-3,6-dihydropyridin-1(2H)-yl)methyl)-1-thioxoisoindolin-2-yl)piperi-dine-2,6-dione |
| | 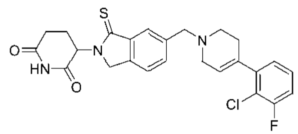 | 3-(6-((4-(2-chloro-3-fluorophenyl)-3,6-dihydropyridin-1(2H)-yl)methyl)-1-thioxoisoindolin-2-yl)piperidine-2,6-dione | 3-(6-((4-(2-chloro-3-fluorophenyl)-3,6-dihydropyridin-1(2H)-yl)methyl)-1-thioxoisoindolin-2-yl)piperi-dine-2,6-dione |
| | 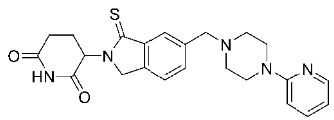 | 3-(6-((4-(pyridin-2-yl)piperazin-1-yl)methyl)-1-thioxoisoindolin-2-yl)piperidine-2,6-dione | 3-(6-((4-(pyridin-2-yl)piperazin-1-yl)methyl)-1-thioxoisoindolin-2-yl)piperidine-2,6-dione |
| | 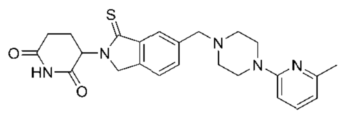 | 3-(6-((4-(6-methylpyridin-2-yl)piperazin-1-yl)methyl)-1-thioxoisoindolin-2-yl)piperidine-2,6-dione | 3-(6-((4-(6-methylpyridin-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 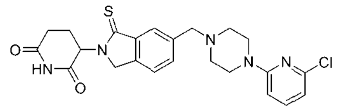 | 3-(6-((4-(6-chloropyridin-2-yl)piperazin-1-yl)methyl)-1-thioxoisoindolin-2-yl)piperidine-2,6-dione | 3-(6-((4-(6-chloropyridin-2-yl)piperazin-1-yl)methyl)-1-thioxoisoindolin-2-yl)piperidine-2,6-dione |
| | 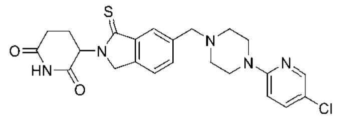 | 3-(6-((4-(5-chloropyridin-2-yl)piperazin-1-yl)methyl)-1-thioxoisoindolin-2-yl)piperidine-2,6-dione | 3-(6-((4-(5-chloropyridin-2-yl)piperazin-1-yl)methyl)-1-thioxoisoindolin-2-yl)piperidine-2,6-dione |
| | 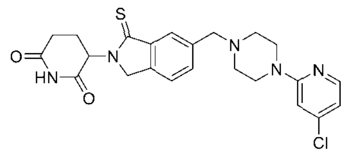 | 3-(6-((4-(4-chloropyridin-2-yl)piperazin-1-yl)methyl)-1-thioxoisoindolin-2-yl)piperidine-2,6-dione | 3-(6-((4-(4-chloropyridin-2-yl)piperazin-1-yl)methyl)-1-thioxoisoindolin-2-yl)piperidine-2,6-dione |
| | 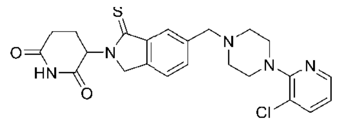 | 1-(6-((4-(3-chloropyridin-2-yl)piperazin-1-yl)methyl)-1-oxoisoindolin-2-yl)dihydropyrimidine-2,4(1H,3H)-dione | 1-(6-((4-(3-chloropyridin-2-yl)piperazin-1-yl)methyl)-1-oxoisoindolin-2-yl)dihydropyrimidine-2,4(1H,3H)-dione |
| | 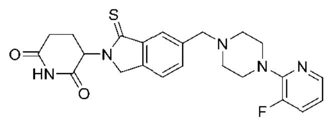 | 1-(6-((4-(3-fluoropyridin-2-yl)piperazin-1-yl)methyl)-1-oxoisoindolin-2-yl)dihydropyrimidine-2,4(1H,3H)-dione | 1-(6-((4-(3-fluoropyridin-2-yl)piperazin-1-yl)methyl)-1-oxoisoindolin-2-yl)dihydropyrimidine-2,4(1H,3H)-dione |
| | 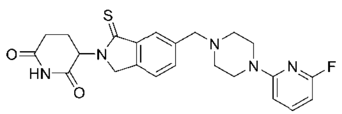 | 3-(6-((4-(6-fluoropyridin-2-yl)piperazin-1-yl)methyl)-1-thioxoisoindolin-2-yl)piperidine-2,6-dione | 3-(6-((4-(6-fluoropyridin-2-yl)piperazin-1-yl)methyl)-1-thioxoisoindolin-2-yl)piperidine-2,6-dione |
| | 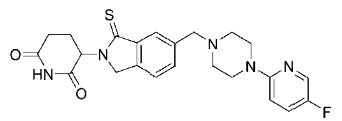 | 3-(6-((4-(5-fluoropyridin-2-yl)piperazin-1-yl)methyl)-1-thioxoisoindolin-2-yl)piperidine-2,6-dione | 3-(6-((4-(5-fluoropyridin-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 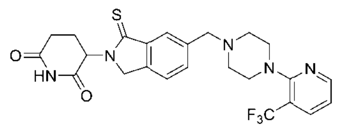 | 3-(1-thioxo-6-((4-(3-(trifluoromethyl)pyridin-2-yl)piperazin-1-yl)methyl)isoindolin-2-yl)piperidine-2,6-dione | 3-(1-thioxo-6-((4-(3-(trifluoromethyl)pyridin-2-yl)piperazin-1-yl)methyl)isoindolin-2-yl)piperidine-2,6-dione |
| | 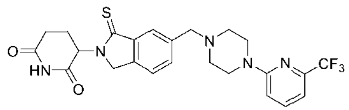 | 3-(1-thioxo-6-((4-(6-(trifluoromethyl)pyridin-2-yl)piperazin-1-yl)methyl)isoindolin-2-yl)piperidine-2,6-dione | 3-(1-thioxo-6-((4-(6-(trifluoromethyl)pyridin-2-yl)piperazin-1-yl)methyl)isoindolin-2-yl)piperidine-2,6-dione |
| | 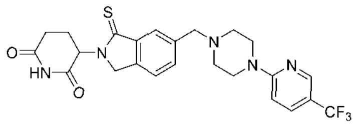 | 3-(1-thioxo-6-((4-(5-(trifluoromethyl)pyridin-2-yl)piperazin-1-yl)methyl)isoindolin-2-yl)piperidine-2,6-dione | 3-(1-thioxo-6-((4-(5-(trifluoromethyl)pyridin-2-yl)piperazin-1-yl)methyl)isoindolin-2-yl)piperidine-2,6-dione |
| | 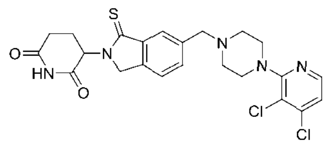 | 3-(6-((4-(3,4-dichloropyridin-2-yl)piperazin-1-yl) methyl)-1-thioxoisoindolin-2-yl)piperidine-2,6-dione | 3-(6-((4-(3,4-dichloropyridin-2-yl)piperazin-1-yl) methyl)-1-thioxoisoindolin-2-yl)piperidine-2,6-dione |
| | 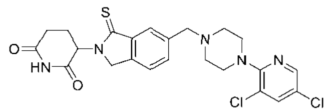 | 3-(6-((4-(3,5-dichloropyridin-2-yl)piperazin-1-yl) methyl)-1-thioxoisoindolin-2-yl)piperidine-2,6-dione | 3-(6-((4-(3,5-dichloropyridin-2-yl)piperazin-1-yl) methyl)-1-thioxoisoindolin-2-yl)piperidine-2,6-dione |
| | 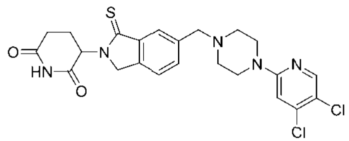 | 3-(6-((4-(4,5-dichloropyridin-2-yl)piperazin-1-yl) methyl)-1-thioxoisoindolin-2-yl)piperidine-2,6-dione | 3-(6-((4-(4,5-dichloropyridin-2-yl)piperazin-1-yl) methyl)-1-thioxoisoindolin-2-yl)piperidine-2,6-dione |
| | 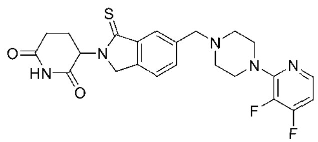 | 3-(6-((4-(3,4-difluoropyridin-2-yl)piperazin-1-yl) methyl)-1-thioxoisoindolin-2-yl)piperidine-2,6-dione | 3-(6-((4-(3,4-difluoropyridin-2-yl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 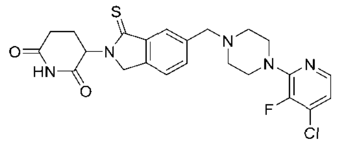 | 3-(6-((4-(4-chloro-3-fluoropyridin-2-yl)piperazin-1-yl) methyl)-1-thioxoisoindolin-2-yl)piperidine-2,6-dione | 3-(6-((4-(4-chloro-3-fluoropyridin-2-yl)piperazin-1-yl) methyl)-1-thioxoisoindolin-2-yl)piperidine-2,6-dione |
| | 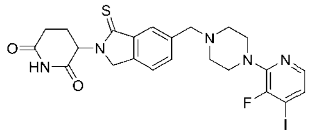 | 3-(6-((4-(3-fluoro-4-iodopyridin-2-yl)piperazin-1-yl) methyl)-1-thioxoisoindolin-2-yl)piperidine-2,6-dione | 3-(6-((4-(3-fluoro-4-iodopyridin-2-yl)piperazin-1-yl) methyl)-1-thioxoisoindolin-2-yl)piperidine-2,6-dione |
| | 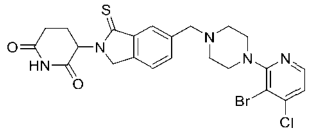 | 3-(6-((4-(3-bromo-4-chloropyridin-2-yl)piperazin-1-yl) methyl)-1-thioxoisoindolin-2-yl)piperidine-2,6-dione | 3-(6-((4-(3-bromo-4-chloropyridin-2-yl)piperazin-1-yl) methyl)-1-thioxoisoindolin-2-yl)piperidine-2,6-dione |
| | 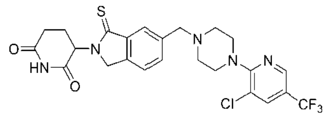 | 3-(6-((4-(3-chloro-5-(trifluoromethyl)pyridin-2-yl)piperazin-1-yl)methyl)-1-thioxoisoindolin-2-yl)piperidine-2,6-dione | 3-(6-((4-(3-chloro-5-(trifluoromethyl)pyridin-2-yl)piperazin-1-yl)methyl)-1-thioxoisoindolin-2-yl)piperidine-2,6-dione |
| | 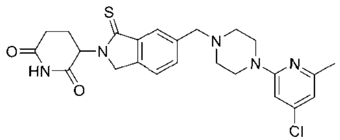 | 3-(6-((4-(4-chloro-6-methylpyridin-2-yl)piperazin-1-yl) methyl)-1-thioxoisoindolin-2-yl)piperidine-2,6-dione | 3-(6-((4-(4-chloro-6-methylpyridin-2-yl)piperazin-1-yl) methyl)-1-thioxoisoindolin-2-yl)piperidine-2,6-dione |
| | 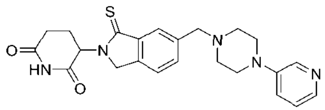 | 3-(6-((4-(pyridin-3-yl)piperazin-1-yl)methyl)-1-thioxoisoindolin-2-yl)piperidine-2,6-dione | 3-(6-((4-(pyridin-3-yl)piperazin-1-yl)methyl)-1-thioxoisoindolin-2-yl)piperidine-2,6-dione |
| | 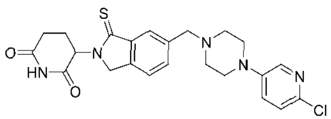 | 3-(6-((4-(6-chloropyridin-3-yl)piperazin-1-yl)methyl)-1-thioxoisoindolin-2-yl)piperidine-2,6-dione | 3-(6-((4-(6-chloropyridin-3-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 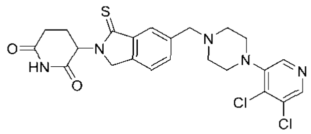 | 3-(6-((4-(4,5-dichloropyridin-3-yl)piperazin-1-yl)methyl)-1-thioxoisoindolin-2-yl)piperidine-2,6-dione | 3-(6-((4-(4,5-dichloropyridin-3-yl)piperazin-1-yl)methyl)-1-thioxoisoindolin-2-yl)piperidine-2,6-dione |
| | 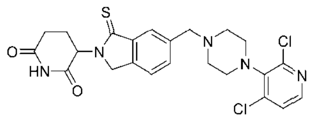 | 3-(6-((4-(2,4-dichloropyridin-3-yl)piperazin-1-yl)methyl)-1-thioxoisoindolin-2-yl)piperidine-2,6-dione | 3-(6-((4-(2,4-dichloropyridin-3-yl)piperazin-1-yl)methyl)-1-thioxoisoindolin-2-yl)piperidine-2,6-dione |
| | 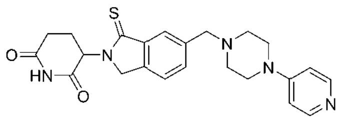 | 3-(6-((4-(pyridin-4-yl)piperazin-1-yl)methyl)-1-thioxoisoindolin-2-yl)piperidine-2,6-dione | 3-(6-((4-(pyridin-4-yl)piperazin-1-yl)methyl)-1-thioxoisoindolin-2-yl)piperidine-2,6-dione |
| | 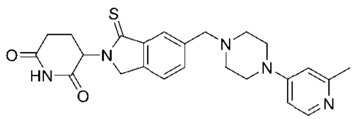 | 3-(6-((4-(2-methylpyridin-4-yl)piperazin-1-yl)methyl)-1-thioxoisoindolin-2-yl)piperidine-2,6-dione | 3-(6-((4-(2-methylpyridin-4-yl)piperazin-1-yl)methyl)-1-thioxoisoindolin-2-yl)piperidine-2,6-dione |
| | 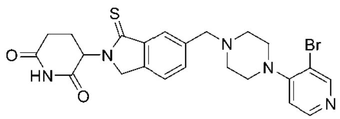 | 3-(6-((4-(3-bromopyridin-4-yl)piperazin-1-yl)methyl)-1-thioxoisoindolin-2-yl)piperidine-2,6-dione | 3-(6-((4-(3-bromopyridin-4-yl)piperazin-1-yl)methyl)-1-thioxoisoindolin-2-yl)piperidine-2,6-dione |
| | 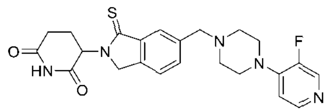 | 3-(6-((4-(3-fluoropyridin-4-yl)piperazin-1-yl)methyl)-1-thioxoisoindolin-2-yl)piperidine-2,6-dione | 3-(6-((4-(3-fluoropyridin-4-yl)piperazin-1-yl)methyl)-1-thioxoisoindolin-2-yl)piperidine-2,6-dione |
| | 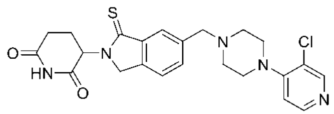 | 3-(6-((4-(3-chloropyridin-4-yl)piperazin-1-yl)methyl)-1-thioxoisoindolin-2-yl)piperidine-2,6-dione | 3-(6-((4-(3-chloropyridin-4-yl)piperazin-1-yl)methyl)-1-thioxoisoindolin-2-yl)piperidine-2,6-dione |
| | 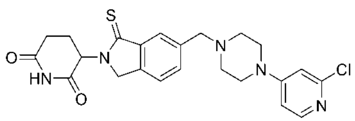 | 3-(6-((4-(2-chloropyridin-4-yl)piperazin-1-yl)methyl)-1-thioxoisoindolin-2-yl)piperidine-2,6-dione | 3-(6-((4-(2-chloropyridin-4-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 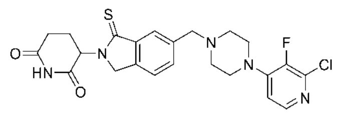 | 3-(6-((4-(2-chloro-3-fluoropyridin-4-yl)piperazin-1-yl) methyl)-1-thioxoisoindolin-2-yl)piperidine-2,6-dione | 3-(6-((4-(2-chloro-3-fluoropyridin-4-yl)piperazin-1-yl) methyl)-1-thioxoisoindolin-2-yl)piperidine-2,6-dione |
| | 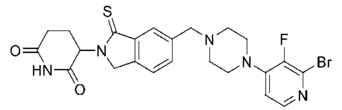 | 3-(6-((4-(2-bromo-3-fluoropyridin-4-yl)piperazin-1-yl) methyl)-1-thioxoisoindolin-2-yl)piperidine-2,6-dione | 3-(6-((4-(2-bromo-3-fluoropyridin-4-yl)piperazin-1-yl) methyl)-1-thioxoisoindolin-2-yl)piperidine-2,6-dione |
| | 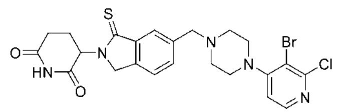 | 3-(6-((4-(3-bromo-2-chloropyridin-4-yl)piperazin-1-yl) methyl)-1-thioxoisoindolin-2-yl)piperidine-2,6-dione | 3-(6-((4-(3-bromo-2-chloropyridin-4-yl)piperazin-1-yl) methyl)-1-thioxoisoindolin-2-yl)piperidine-2,6-dione |
| | 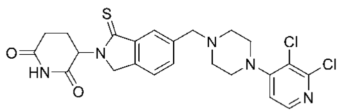 | 3-(6-((4-(2,3-dichloropyridin-4-yl)piperazin-1-yl) methyl)-1-thioxoisoindolin-2-yl)piperidine-2,6-dione | 3-(6-((4-(2,3-dichloropyridin-4-yl)piperazin-1-yl) methyl)-1-thioxoisoindolin-2-yl)piperidine-2,6-dione |
| | 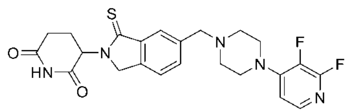 | 3-(6-((4-(2,3-difluoropyridin-4-yl)piperazin-1-yl) methyl)-1-thioxoisoindolin-2-yl)piperidine-2,6-dione | 3-(6-((4-(2,3-difluoropyridin-4-yl)piperazin-1-yl) methyl)-1-thioxoisoindolin-2-vl)piperidine-2,6-dione |
| | 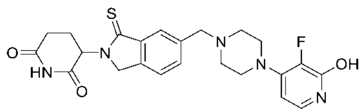 | 3-(6-((4-(3-fluoro-2-hydroxypyridin-4-yl)piperazin-1-yl) methyl)-1-thioxoisoindolin-2-yl)piperidine-2,6-dione | 3-(6-((4-(3-fluoro-2-hydroxypyridin-4-yl)piperazin-1-yl) methyl)-1-thioxoisoindolin-2-yl)piperidine-2,6-dione |
| | 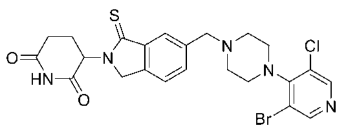 | 3-(6-((4-(3-bromo-5-chloropyridin-4-yl)piperazin-1-yl) methyl)-1-thioxoisoindolin-2-yl)piperidine-2,6-dione | 3-(6-((4-(3-bromo-5-chloropyridin-4-yl)piperazin-1-yl) methyl)-1-thioxoisoindolin-2-yl)piperidine-2,6-dione |
| | 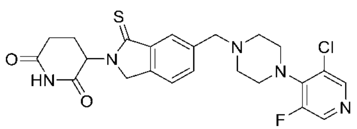 | 3-(6-((4-(3-chloro-5-fluoropyridin-4-yl)piperazin-1-yl) methyl)-1-thioxoisoindolin-2-yl)piperidine-2,6-dione | 3-(6-((4-(3-chloro-5-fluoropyridin-4-yl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 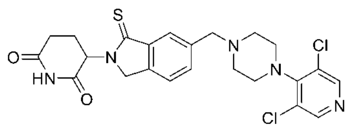 | 3-(6-((4-(3,5-dichloropyridin-4-yl)piperazin-1-yl) methyl)-1-thioxoisoindolin-2-yl)piperidine-2,6-dione | 3-(6-((4-(3,5-dichloropyridin-4-yl)piperazin-1-yl) methyl)-1-thioxoisoindolin-2-yl)piperidine-2,6-dione |
| | 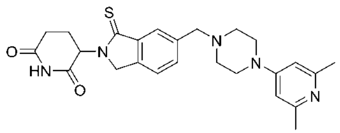 | 3-(6-((4-(2,6-dimethylpyridin-4-yl)piperazin-1-yl) methyl)-1-thioxoisoindolin-2-yl)piperidine-2,6-dione | 3-(6-((4-(2,6-dimethylpyridin-4-yl)piperazin-1-yl) methyl)-1-thioxoisoindolin-2-yl)piperidine-2,6-dione |
| | 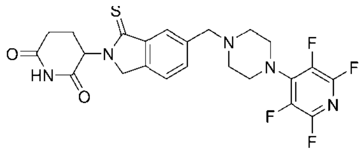 | 3-(6-((4-(perfluoropyridin-4-yl)piperazin-1-yl)methyl)-1-thioxoisoindolin-2-yl)piperidine-2,6-dione | 3-(6-((4-(perfluoropyridin-4-yl)piperazin-1-yl)methyl)-1-thioxoisoindolin-2-yl)piperidine-2,6-dione |
| | 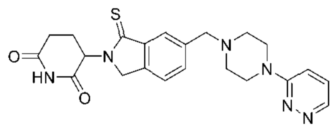 | 3-(6-((4-(pyridazin-3-yl)piperazin-1-yl)methyl)-1-thioxoisoindolin-2-yl)piperidine-2,6-dione | 3-(6-((4-(pyridazin-3-yl)piperazin-1-yl)methyl)-1-thioxoisoindolin-2-yl)piperidine-2,6-dione |
| | 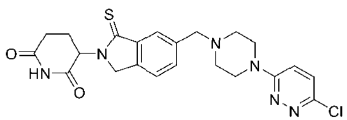 | 3-(6-((4-(6-chloropyridazin-3-yl)piperazin-1-yl) methyl)-1-thioxoisoindolin-2-yl)piperidine-2,6-dione | 3-(6-((4-(6-chloropyridazin-3-yl)piperazin-1-yl) methyl)-1-thioxoisoindolin-2-yl)piperidine-2,6-dione |
| | 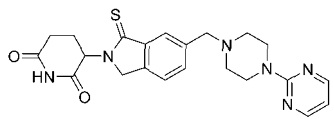 | 3-(6-((4-(pyrimidin-2-yl)piperazin-1-yl)methyl)-1-thioxoisoindolin-2-yl)piperidine-2,6-dione | 3-(6-((4-(pyrimidin-2-yl)piperazin-1-yl)methyl)-1-thioxoisoindolin-2-yl)piperidine-2,6-dione |
| | 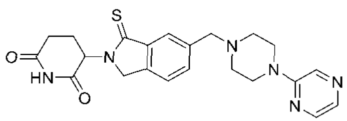 | 3-(6-((4-(pyrazin-2-yl)piperazin-1-yl)methyl)-1-thioxoisoindolin-2-yl)piperidine-2,6-dione | 3-(6-((4-(pyrazin-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 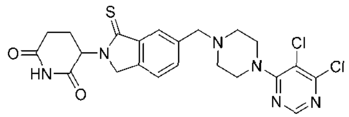 | 3-(6-((4-(5,6-dichloropyrimidin-4-yl)piperazin-1-yl) methyl)-1-thioxoisoindolin-2-yl)piperidine-2,6-dione | 3-(6-((4-(5,6-dichloropyrimidin-4-yl)piperazin-1-yl) methyl)-1-thioxoisoindolin-2-yl)piperidine-2,6-dione |
| | 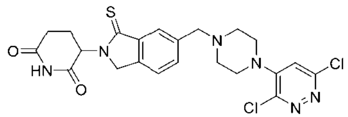 | 3-(6-((4-(3,6-dichloropyridazin-4-yl)piperazin-1-yl) methyl)-1-thioxoisoindolin-2-yl)piperidine-2,6-dione | 3-(6-((4-(3,6-dichloropyridazin-4-yl)piperazin-1-yl) methyl)-1-thioxoisoindolin-2-yl)piperidine-2,6-dione |
| | 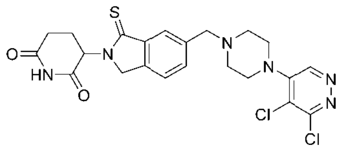 | 3-(6-((4-(5,6-dichloropyridazin-4-yl)piperazin-1-yl) methyl)-1-thioxoisoindolin-2-yl)piperidine-2,6-dione | 3-(6-((4-(5,6-dichloropyridazin-4-yl)piperazin-1-yl) methyl)-1-thioxoisoindolin-2-yl)piperidine-2,6-dione |
| | 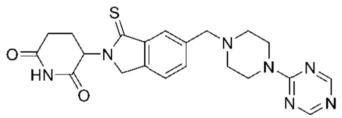 | 3-(6-((4-(1,3,5-triazin-2-yl)piperazin-1-yl)methyl)-1-thioxoisoindolin-2-yl)piperidine-2,6-dione | 3-(6-((4-(1,3,5-triazin-2-yl)piperazin-1-yl)methyl)-1-thioxoisoindolin-2-yl)piperidine-2,6-dione |
| | 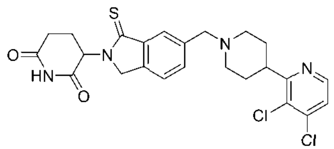 | 3-(6-((4-(3,4-dichloropyridin-2-yl)piperidin-1-yl) methyl)-1-thioxoisoindolin-2-yl)piperidine-2,6-dione | 3-(6-((4-(3,4-dichloropyridin-2-yl)piperidin-1-yl) methyl)-1-thioxoisoindolin-2-yl)piperidine-2,6-dione |
| | 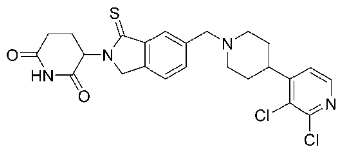 | 3-(6-((4-(2,3-dichloropyridin-4-yl)piperidin-1-yl) methyl)-1-thioxoisoindolin-2-yl)piperidine-2,6-dione | 3-(6-((4-(2,3-dichloropyridin-4-yl)piperidin-1-yl) methyl)-1-thioxoisoindolin-2-yl)piperidine-2,6-dione |
| | 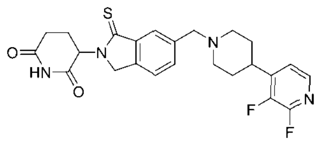 | 3-(6-((4-(2,3-difluoropyridin-4-yl)piperidin-1-yl) methyl)-1-thioxoisoindolin-2-yl)piperidine-2,6-dione | 3-(6-((4-(2,3-difluoropyridin-4-yl)piperidin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 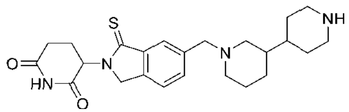 | 3-(6-([3,4'-bipiperidin]-1-ylmethyl)-1-thioxoisoindolin-2-yl)piperidine-2,6-dione | 3-(6-([3,4'-bipiperidin]-1-ylmethyl)-1-thioxoisoindolin-2-yl)piperidine-2,6-dione |
| | 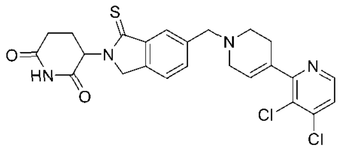 | 3-(6-((3,4-dichloro-3',6'-dihydro-[2,4'-bipyridin]-1'(2'H)-yl)methyl)-1-thioxoisoindolin-2-yl)piperidine-2,6-dione | 3-(6-((3,4-dichloro-3',6'-dihydro-[2,4'-bipyridin]-1'(2'H)-yl)methyl)-1-thioxoisoindolin-2-yl)piperidine-2,6-dione |
| | 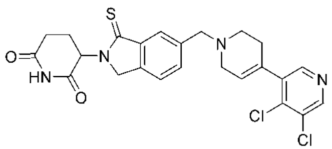 | 3-(6-((4,5-dichloro-3',6'-dihydro-[3,4'-bipyridin]-1'(2'H)-yl)methyl)-1-thioxoisoindolin-2-yl)piperidine-2,6-dione | 3-(6-((4,5-dichloro-3',6'-dihydro-[3,4'-bipyridin]-1'(2'H)-yl)methyl)-1-thioxoisoindolin-2-yl)piperidine-2,6-dione |
| | 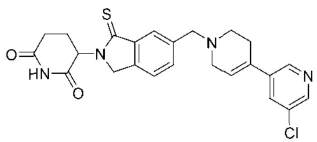 | 3-(6-((5-chloro-3',6'-dihydro-[3,4'-bipyridin]-1'(2'H)-yl)methyl)-1-thioxoisoindolin-2-yl)piperidine-2,6-dione | 3-(6-((5-chloro-3',6'-dihydro-[3,4'-bipyridin]-1'(2'H)-yl)methyl)-1-thioxoisoindolin-2-yl)piperidine-2,6-dione |
| | 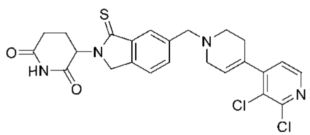 | 3-(6-((2',3'-dichloro-3,6-dihydro-[4,4'-bipyridin]-1(2H)-yl)methyl)-1-thioxoisoindolin-2-yl)piperidine-2,6-dione | 3-(6-((2',3'-dichloro-3,6-dihydro-[4,4'-bipyridin]-1(2H)-yl)methyl)-1-thioxoisoindolin-2-yl)piperidine-2,6-dione |
| | 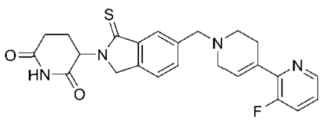 | 3-(6-((3-fluoro-3',6'-dihydro-[2,4'-bipyridin]-1'(2'H)-yl)methyl)-1-thioxoisoindolin-2-yl)piperidine-2,6-dione | 3-(6-((3-fluoro-3',6'-dihydro-[2,4'-bipyridin]-1'(2'H)-yl)methyl)-1-thioxoisoindolin-2-yl)piperidine-2,6-dione |
| | 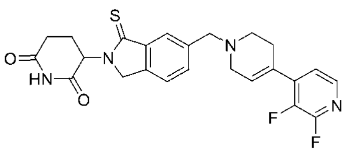 | 3-(6-((2',3'-difluoro-3,6-dihydro-[4,4'-bipyridin]-1(2H)-yl)methyl)-1-thioxoisoindolin-2-yl)piperidine-2,6-dione | 3-(6-((2',3'-difluoro-3,6-dihydro-[4,4'-bipyridin]-1(2H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 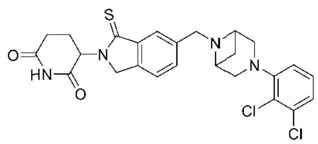 | 3-(6-((3-(2,3-dichlorophenyl)-3,6-diazabicyclo[3.1.1]heptan-6-yl)methyl)-1-thioxoisoindolin-2-yl)piperidine-2,6-dione | 3-(6-((3-(2,3-dichlorophenyl)-3,6-diazabicyclo[3.1.1]heptan-6-yl)methyl)-1-thioxoisoindolin-2-yl)piperidine-2,6-dione |
| | 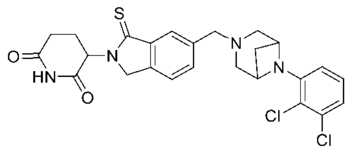 | 3-(6-((6-(2,3-dichlorophenyl)-3,6-diazabicyclo[3.1.1]heptan-3-yl)methyl)-1-thioxoisoindolin-2-yl)piperidine-2,6-dione | 3-(6-((6-(2,3-dichlorophenyl)-3,6-diazabicyclo[3.1.1]heptan-3-yl)methyl)-1-thioxoisoindolin-2-yl)piperidine-2,6-dione |
| | 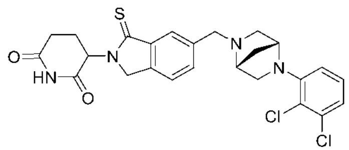 | 3-(6-(((1R,4R)-5-(2,3-dichlorophenyl)-2,5-diazabicyclo[2.2.1]heptan-2-yl)methyl)-1-thioxoisoindolin-2-yl)piperidine-2,6-dione | 3-(6-(((1R,4R)-5-(2,3-dichlorophenyl)-2,5-diazabicyclo[2.2.1]heptan-2-yl)methyl)-1-thioxoisoindolin-2-yl)piperidine-2,6-dione |
| | 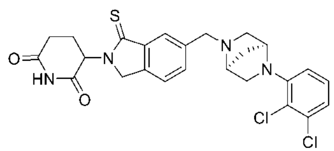 | 3-(6-(((1S,4S)-5-(2,3-dichlorophenyl)-2,5-diazabicyclo[2.2.1]heptan-2-yl)methyl)-1-thioxoisoindolin-2-yl)piperidine-2,6-dione | 3-(6-(((1S,4S)-5-(2,3-dichlorophenyl)-2,5-diazabicyclo[2.2.1]heptan-2-yl)methyl)-1-thioxoisoindolin-2-yl)piperidine-2,6-dione |
| | 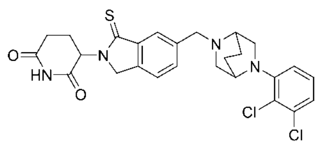 | 3-(6-((5-(2,3-dichlorophenyl)-2,5-diazabicyclo[2.2.2]octan-2-yl)methyl)-1-thioxoisoindolin-2-yl)piperidine-2,6-dione | 3-(6-((5-(2,3-dichlorophenyl)-2,5-diazabicyclo[2.2.2]octan-2-yl)methyl)-1-thioxoisoindolin-2-yl)piperidine-2,6-dione |
| | 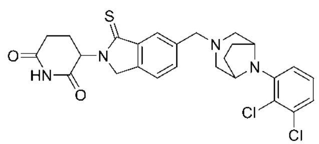 | 3-(6-((8-(2,3-dichlorophenyl)-3,8-diazabicyclo[3.2.1]octan-3-yl)methyl)-1-thioxoisoindolin-2-yl)piperidine-2,6-dione | 3-(6-((8-(2,3-dichlorophenyl)-3,8-diazabicyclo[3.2.1]octan-3-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 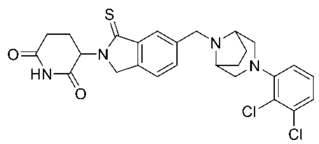 | 3-(6-((3-(2,3-dichlorophenyl)-3,8-diazabicyclo[3.2.1]octan-8-yl)methyl)-1-thioxoisoindolin-2-yl)piperidine-2,6-dione | 3-(6-((3-(2,3-dichlorophenyl)-3,8-diazabicyclo[3.2.1]octan-8-yl)methyl)-1-thioxoisoindolin-2-yl)piperidine-2,6-dione |
| | 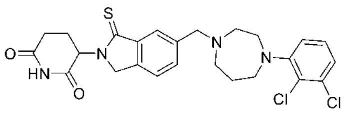 | 3-(6-((4-(2,3-dichlorophenyl)-1,4-diazepan-1-yl)methyl)-1-thioxoisoindolin-2-yl)piperidine-2,6-dione | 3-(6-((4-(2,3-dichlorophenyl)-1,4-diazepan-1-yl)methyl)-1-thioxoisoindolin-2-yl)piperidine-2,6-dione |
| GT-05031 | 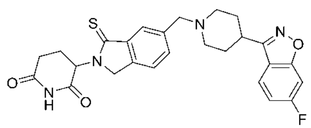 | 3-(6-((4-(6-fluorobenzo[d]isoxazol-3-yl)piperidin-1-yl)methyl)-1-thioxoisoindolin-2-yl)piperidine-2,6-dione | 3-(6-((4-(6-fluorobenzo[d]isoxazol-3-yl)piperidin-1-yl)methyl)-1-thioxoisoindolin-2-yl)piperidine-2,6-dione |
| | 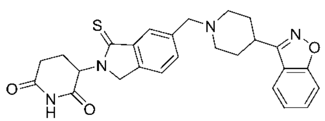 | 3-(6-((4-(benzo[d]isoxazol-3-yl)piperidin-1-yl)methyl)-1-thioxoisoindolin-2-yl)piperidine-2,6-dione | 3-(6-((4-(benzo[d]isoxazol-3-yl)piperidin-1-yl)methyl)-1-thioxoisoindolin-2-yl)piperidine-2,6-dione |
| | 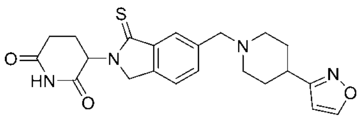 | 3-(6-((4-(isoxazol-3-yl)piperidin-1-yl)methyl)-1-thioxoisoindolin-2-yl)piperidine-2,6-dione | 3-(6-((4-(isoxazol-3-yl)piperidin-1-yl)methyl)-1-thioxoisoindolin-2-yl)piperidine-2,6-dione |
| | 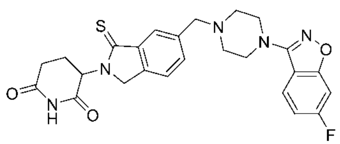 | 3-(6-((4-(6-fluorobenzo[d]isoxazol-3-yl)piperazin-1-yl)methyl)-1-thioxoisoindolin-2-yl)piperidine-2,6-dione | 3-(6-((4-(6-fluorobenzo[d]isoxazol-3-yl)piperazin-1-yl)methyl)-1-thioxoisoindolin-2-yl)piperidine-2,6-dione |
| | 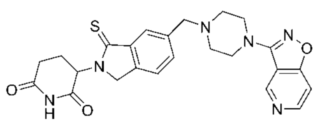 | 3-(6-((4-(isoxazolo[4,5-c]pyridin-3-yl)piperazin-1-yl)methyl)-1-thioxoisoindolin-2-yl)piperidine-2,6-dione | 3-(6-((4-(isoxazolo[4,5-c]pyridin-3-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 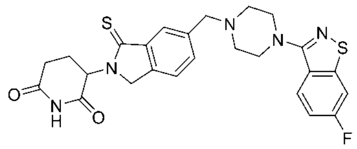 | 3-(6-((4-(6-fluorobenzo[d]isothiazol-3-yl)piperazin-1-yl) methyl)-1-thioxoisoindolin-2-yl)piperidine-2,6-dione | 3-(6-((4-(6-fluorobenzo[d]isothiazol-3-yl)piperazin-1-yl) methyl)-1-thioxoisoindolin-2-yl)piperidine-2,6-dione |
| | 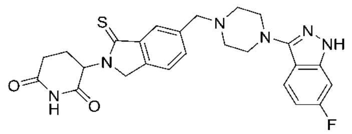 | 3-(6-((4-(6-fluoro-1H-indazol-3-yl)piperazin-1-yl) methyl)-1-thioxoisoindolin-2-yl)piperidine-2,6-dione | 3-(6-((4-(6-fluoro-1H-indazol-3-yl)piperazin-1-yl) methyl)-1-thioxoisoindolin-2-yl)piperidine-2,6-dione |
| | 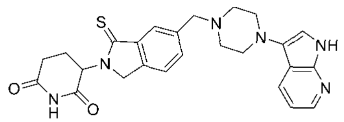 | 3-(6-((4-(1H-pyrrolo[2,3-b]pyridin-3-yl)piperazin-1-yl) methyl)-1-thioxoisoindolin-2-yl)piperidine-2,6-dione | 3-(6-((4-(1H-pyrrolo[2,3-b]pyridin-3-yl)piperazin-1-yl) methyl)-1-thioxoisoindolin-2-yl)piperidine-2,6-dione |
| | 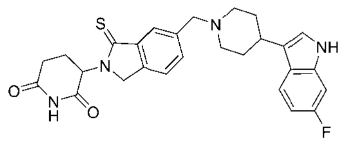 | 3-(6-((4-(6-fluoro-1H-indol-3-yl)piperidin-1-yl) methyl)-1-thioxoisoindolin-2-yl)piperidine-2,6-dione | 3-(6-((4-(6-fluoro-1H-indol-3-yl)piperidin-1-yl) methyl)-1-thioxoisoindolin-2-yl)piperidine-2,6-dione |
| | 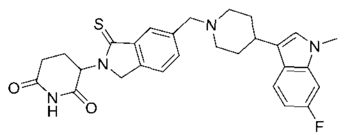 | 3-(6-((4-(6-fluoro-1-methyl-1H-indol-3-yl)piperidin-1-yl) methyl)-1-thioxoisoindolin-2-yl)piperidine-2,6-dione | 3-(6-((4-(6-fluoro-1-methyl-1H-indol-3-yl)piperidin-1-yl) methyl)-1-thioxoisoindolin-2-yl)piperidine-2,6-dione |
| | 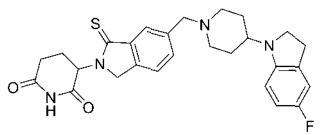 | 3-(6-((4-(5-fluoroindolin-1-yl)piperidin-1-yl)methyl)-1-thioxoisoindolin-2-yl)piperidine-2,6-dione | 3-(6-((4-(5-fluoroindolin-1-yl)piperidin-1-yl)methyl)-1-thioxoisoindolin-2-yl)piperidine-2,6-dione |
| | 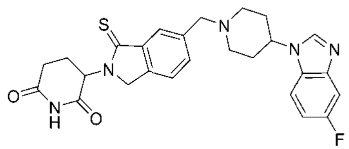 | 3-(6-((4-(5-fluoro-1H-benzo[d]imidazol-1-yl)piperidin-1-yl)methyl)-1-thioxoisoindolin-2-yl)piperidine-2,6-dione | 3-(6-((4-(5-fluoro-1H-benzo[d]imidazol-1-yl)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 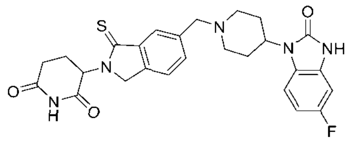 | 3-(6-((4-(5-fluoro-2-oxo-2,3-dihydro-1H-benzo[d]imidazol-1-yl)piperidin-1-yl)methyl)-1-thioxoisoindolin-2-yl) piperidine-2,6-dione | 3-(6-((4-(5-fluoro-2-oxo-2,3-dihydro-1H-benzo[d]imidazol-1-yl)piperidin-1-yl)methyl)-1-thioxoisoindolin-2-yl) piperidine-2,6-dione |
| | 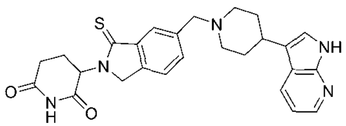 | 3-(6-((4-(1H-pyrrolo[2,3-b]pyridin-3-yl)piperidin-1-yl) methyl)-1-thioxoisoindolin-2-yl)piperidine-2,6-dione | 3-(6-((4-(1H-pyrrolo[2,3-b]pyridin-3-yl)piperidin-1-yl) methyl)-1-thioxoisoindolin-2-yl)piperidine-2,6-dione |
| | 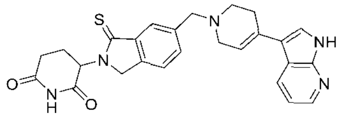 | 3-(6-((4-(1H-pyrrolo[2,3-b]pyridin-3-yl)-3,6-dihydropyridin-1 (2H)-yl)methyl)-1-thioxoisoindolin-2-yl)piperidine-2,6-dione | 3-(6-((4-(1H-pyrrolo[2,3-b]pyridin-3-yl)-3,6-dihydropyridin-1 (2H)-yl)methyl)-1-thioxoisoindolin-2-yl)piperidine-2,6-dione |
| | 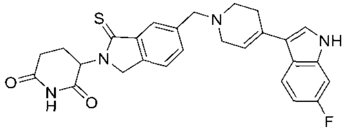 | 3-(6-((4-(6-fluoro-1H-indol-3-yl)-3,6-dihydropyridin-1(2H)-yl)methyl)-1-thioxoisoindolin-2-yl)piperidine-2,6-dione | 3-(6-((4-(6-fluoro-1H-indol-3-yl)-3,6-dihydropyridin-1(2H)-yl)methyl)-1-thioxoisoindolin-2-yl)piperidine-2,6-dione |
| | 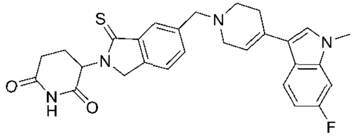 | 3-(6-((4-(6-fluoro-1-methyl-1H-indol-3-yl)-3,6-dihydropyridin-1(2H)-yl)methyl)-1-thioxoisoindolin-2-yl)piperidine-2,6-dione | 3-(6-((4-(6-fluoro-1-methyl-1H-indol-3-yl)-3,6-dihydropyridin-1(2H)-yl)methyl)-1-thioxoisoindolin-2-yl)piperidine-2,6-dione |
| | 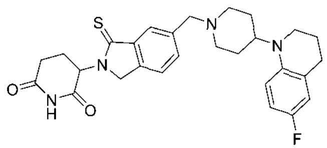 | 3-(6-((4-(6-fluoro-3,4-dihydroquinolin-1(2H)-yl)piperidin-1-yl)methyl)-1-thioxoisoindolin-2-yl)piperidine-2,6-dione | 3-(6-((4-(6-fluoro-3,4-dihydroquinolin-1 (2H)-yl)piperidin-1-yl)methyl)-1-thioxoisoindolin-2-yl)piperidine-2,6-dione |
| | 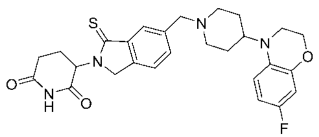 | 3-(6-((4-(7-fluoro-2,3-dihydro-4H-benzo[b][1,4]oxazin-4-yl)piperidin-1-yl)methyl)-1-thioxoisoindolin-2-yl) piperidine-2,6-dione | 3-(6-((4-(7-fluoro-2,3-dihydro-4H-benzo[b][1,4]oxazin-4-yl)piperidin-1-yl)methyl)-1-thioxoisoindolin-2-yl) piperidine-2,6-dione |

(continued)

| Compound No. | Structure of the compounds | The compounds' name | Chinese translation of the compounds' name |
|---|---|---|---|
| | 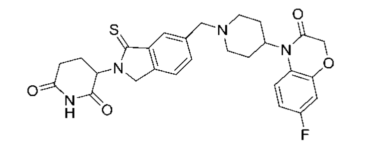 | 3-(6-((4-(7-fluoro-3-oxo-2,3-dihydro-4H-benzo[b][1,4]oxazin-4-yl)piperidin-1-yl)methyl)-1-thioxoisoindolin-2-yl)piperidine-2,6-dione | 3-(6-((4-(7-fluoro-3-oxo-2,3-dihydro-4H-benzo[b][1,4]oxazin-4-yl)piperidin-1-yl)methyl)-1-thioxoisoindolin-2-yl)piperidine-2,6-dione |
| | 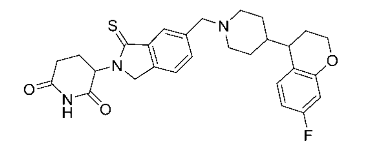 | 3-(6-((4-(7-fluorochroman-4-yl)piperidin-1-yl)methyl)-1-thioxoisoindolin-2-yl)piperidine-2,6-dione | 3-(6-((4-(7-fluorochroman-4-yl)piperidin-1-yl)methyl)-1-thioxoisoindolin-2-yl)piperidine-2,6-dione |
| | 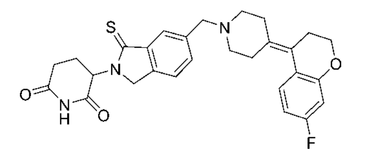 | 3-(6-((4-(7-fluorochroman-4-ylidene)piperidin-1-yl)methyl)-1-thioxoisoindolin-2-yl)piperidine-2,6-dione | 3-(6-((4-(7-fluorochroman-4-ylidene)piperidin-1-yl)methyl)-1-thioxoisoindolin-2-yl)piperidine-2,6-dione |
| | 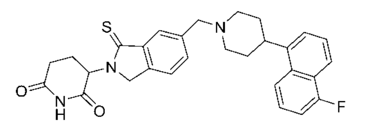 | 3-(6-((4-(5-fluoronaphthalen-1-yl)piperidin-1-yl)methyl)-1-thioxoisoindolin-2-yl)piperidine-2,6-dione | 3-(6-((4-(5-fluoronaphthalen-1-yl)piperidin-1-yl)methyl)-1-thioxoisoindolin-2-yl)piperidine-2,6-dione |
| | 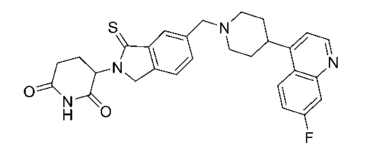 | 3-(6-((4-(7-fluoroquinolin-4-yl)piperidin-1-yl)methyl)-1-thioxoisoindolin-2-yl)piperidine-2,6-dione | 3-(6-((4-(7-fluoroquinolin-4-yl)piperidin-1-yl)methyl)-1-thioxoisoindolin-2-yl)piperidine-2,6-dione |
| | 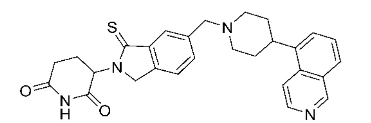 | 3-(6-((4-(isoquinolin-5-yl)piperidin-1-yl)methyl)-1-thioxoisoindolin-2-yl)piperidine-2,6-dione | 3-(6-((4-(isoquinolin-5-yl)piperidin-1-yl)methyl)-1-thioxoisoindolin-2-yl)piperidine-2,6-dione |
| | 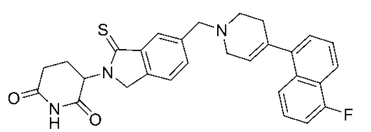 | 3-(6-((4-(5-fluoronaphthalen-1-yl)-3,6-dihydropyridin-1(2H)-yl)methyl)-1-thioxoisoindolin-2-yl)piperidine-2,6-dione | 3-(6-((4-(5-fluoronaphthalen-1-yl)-3,6-dihydropyridin-1(2H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 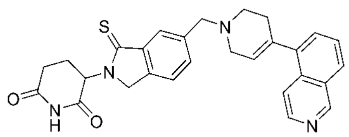 | 3-(6-((4-(isoquinolin-5-yl)-3,6-dihydropyridin-1(2H)-yl) methyl)-1-thioxoisoindolin-2-yl)piperidine-2,6-dione | 3-(6-((4-(isoquinolin-5-yl)-3,6-dihydropyridin-1(2H)-yl) methyl)-1-thioxoisoindolin-2-yl)piperidine-2,6-dione |
| | 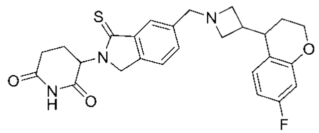 | 3-(6-((3-(7-fluorochroman-4-yl)azetidin-1-yl)methyl)-1-thioxoisoindolin-2-yl)piperidine-2,6-dione | 3-(6-((3-(7-fluorochroman-4-yl)azetidin-1-yl)methyl)-1-thioxoisoindolin-2-yl)piperidine-2,6-dione |
| | 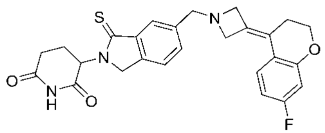 | 3-(6-((3-(7-fluorochroman-4-ylidene)azetidin-1-yl) methyl)-1-thioxoisoindolin-2-yl)piperidine-2,6-dione | 3-(6-((3-(7-fluorochroman-4-ylidene)azetidin-1-yl) methyl)-1-thioxoisoindolin-2-yl)piperidine-2,6-dione |
| | 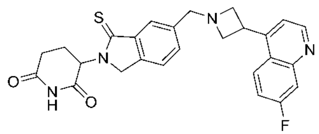 | 3-(6-((3-(7-fluoroquinolin-4-yl)azetidin-1-yl)methyl)-1-thioxoisoindolin-2-yl)piperidine-2,6-dione | 3-(6-((3-(7-fluoroquinolin-4-yl)azetidin-1-yl)methyl)-1-thioxoisoindolin-2-yl)piperidine-2,6-dione |
| | 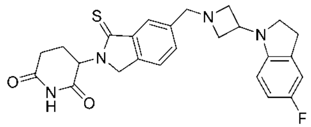 | 3-(6-((3-(5-fluoroindolin-1-yl)azetidin-1-yl)methyl)-1-thioxoisoindolin-2-yl)piperidine-2,6-dione | 3-(6-((3-(5-fluoroindolin-1-yl)azetidin-1-yl)methyl)-1-thioxoisoindolin-2-yl)piperidine-2,6-dione |
| | 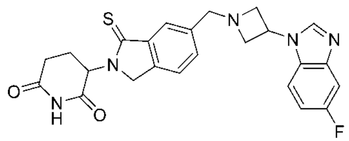 | 3-(6-((3-(5-fluoro-1H-benzo[d]imidazol-1-yl)azetidin-1-yl)methyl)-1-thioxoisoindolin-2-yl)piperidine-2,6-dione | 3-(6-((3-(5-fluoro-1H-benzo[d]imidazol-1-yl)azetidin-1-yl)methyl)-1-thioxoisoindolin-2-yl)piperidine-2,6-dione |
| | 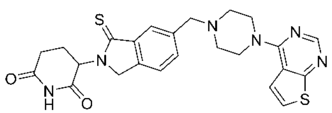 | 3-(6-((4-(thieno[2,3-d]pyrimidin-4-yl)piperazin-1-yl) methyl)-1-thioxoisoindolin-2-yl)piperidine-2,6-dione | 3-(6-((4-(thieno[2,3-d]pyrimidin-4-yl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 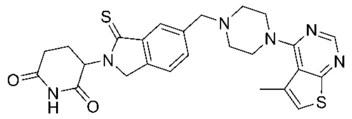 | 3-(5-((4-(5-methylthieno[2,3-d]pyrimidin-4-yl)piperazin-1-yl)methyl)-1-thioxoisoindolin-2-yl)piperidine-2,6-dione | 3-(5-((4-(5-methylthieno[2,3-d]pyrimidin-4-yl)piperazin-1-yl)methyl)-1-thioxoisoindolin-2-yl)piperidine-2,6-dione |
| | 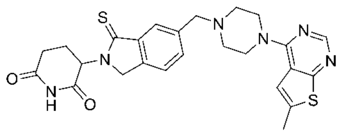 | 3-(6-((4-(6-methylthieno[2,3-d]pyrimidin-4-yl)piperazin-1-yl)methyl)-1-thioxoisoindolin-2-yl)piperidine-2,6-dione | 3-(6-((4-(6-methylthieno[2,3-d]pyrimidin-4-yl)piperazin-1-yl)methyl)-1-thioxoisoindolin-2-yl)piperidine-2,6-dione |
| | 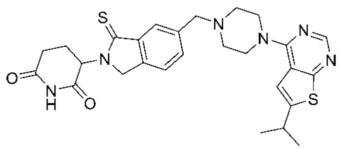 | 3-(6-((4-(6-isopropylthieno[2,3-d]pyrimidin-4-yl)piperazin-1-yl)methyl)-1-thioxoisoindolin-2-yl)piperidine-2,6-dione | 3-(6-((4-(6-isopropylthieno[2,3-d]pyrimidin-4-yl)piperazin-1-yl)methyl)-1-thioxoisoindolin-2-yl)piperidine-2,6-dione |
| | 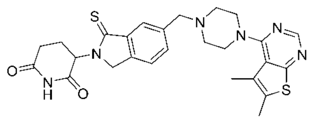 | 3-(6-((4-(5,6-dimethylthieno[2,3-d]pyrimidin-4-yl)piperazin-1-yl)methyl)-1-thioxoisoindolin-2-yl)piperidine-2,6-dione | 3-(6-((4-(5,6-dimethylthieno[2,3-d]pyrimidin-4-yl)piperazin-1-yl)methyl)-1-thioxoisoindolin-2-yl)piperidine-2,6-dione |
| | 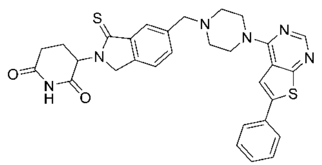 | 3-(6-((4-(6-phenylthieno[2,3-d]pyrimidin-4-yl)piperazin-1-yl)methyl)-1-thioxoisoindolin-2-yl)piperidine-2,6-dione | 3-(6-((4-(6-phenylthieno[2,3-d]pyrimidin-4-yl)piperazin-1-yl)methyl)-1-thioxoisoindolin-2-yl)piperidine-2,6-dione |
| | 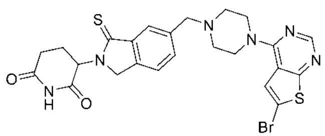 | 3-(6-((4-(6-bromothieno[2,3-d]pyrimidin-4-yl)piperazin-1-yl)methyl)-1-thioxoisoindolin-2-yl)piperidine-2,6-dione | 3-(6-((4-(6-bromothieno[2,3-d]pyrimidin-4-yl)piperazin-1-yl)methyl)-1-thioxoisoindolin-2-yl)piperidine-2,6-dione |
| | 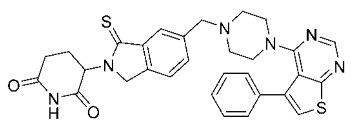 | 3-(6-((4-(5-phenylthieno[2,3-d]pyrimidin-4-yl)piperazin-1-yl)methyl)-1-thioxoisoindolin-2-yl)piperidine-2,6-dione | 3-(6-((4-(5-phenylthieno[2,3-d]pyrimidin-4-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 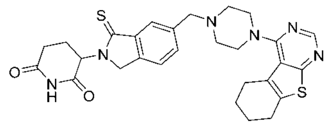 | 3-(6-((4-(5,6,7,8-tetrahydrobenzo[4,5]thieno[2 ,3-d]pyrimidin-4-yl)piperazin-1-yl)methyl)-1-thioxoisoindolin-2-yl)piperidine-2,6-dione | 3-(6-((4-(5,6,7,8-tetrahydrobenzo[4,5]thieno[2 ,3-d]pyrimidin-4-yl)piperazin-1-yl)methyl)-1-thioxoisoindolin-2-yl)piperidine-2,6-dione |
| | 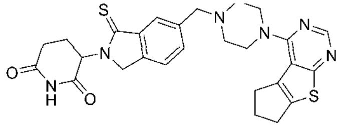 | 3-(6-((4-(6,7-dihydro-5H-cyclopenta[4,5]thieno[2,3-d] pyrimidin-4-yl)piperazin-1-yl)methyl)-1-thioxoisoindolin-2-yl)piperidine-2,6-dione | 3-(6-((4-(6,7-dihydro-5H-cyclopenta[4,5]thieno[2,3-d] pyrimidin-4-yl)piperazin-1-yl)methyl)-1-thioxoisoindolin-2-yl)piperidine-2,6-dione |
| | 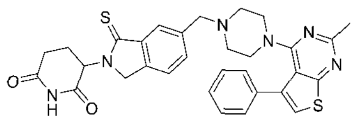 | 3-(6-((4-(2-methyl-5-phenylthieno[2,3-d]pyrimidin-4-yl) piperazin-1-yl)methyl)-1-thioxoisoindolin-2-yl)piperidine-2,6-dione | 3-(6-((4-(2-methyl-5-phenylthieno[2,3-d]pyrimidin-4-yl) piperazin-1-yl)methyl)-1-thioxoisoindolin-2-yl)piperi-dine-2,6-dione |
| | 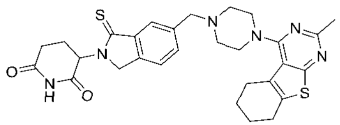 | 3-(6-((4-(2-methyl-5,6,7,8-tetrahydrobenzo[4,5]thieno [2 ,3-d]pyrimidin-4-yl)piperazin-1-yl)methyl)-1-thioxoisoindolin-2-yl)piperidine-2,6-dione | 3-(6-((4-(2-methyl-5,6,7,8-tetrahydrobenzo[4,5]thieno [2 ,3-d]pyrimidin-4-yl)piperazin-1-yl)methyl)-1-thioxoisoindolin-2-yl)piperidine-2,6-dione |
| | 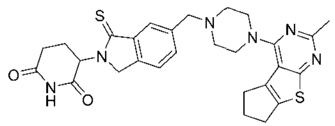 | 3-(6-((4-(2-methyl-6,7-dihydro-5H-cyclopenta[4,5]thieno[2,3-d]pyrimidin-4-yl)piperazin-1-yl)methyl)-1-thioxoisoindolin-2-yl)piperidine-2,6-dione | 3-(6-((4-(2-methyl-6,7-dihydro-5H-cyclopenta[4,5]thieno[2,3-d]pyrimidin-4-yl)piperazin-1-yl)methyl)-1-thioxoisoindolin-2-yl)piperidine-2,6-dione |
| | 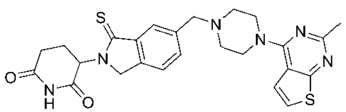 | 3-(6-((4-(2-methylthieno[2,3-d]pyrimidin-4-yl)piperazin-1-yl)methyl)-1-thioxoisoindolin-2-yl)piperidine-2,6-dione | 3-(6-((4-(2-methylthieno[2,3-d]pyrimidin-4-yl)piperazin-1-yl)methyl)-1-thioxoisoindolin-2-yl)piperidine-2,6-dione |
| | 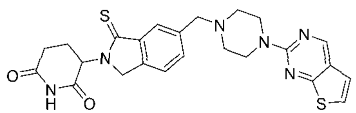 | 3-(6-((4-(thieno[2,3-d]pyrimidin-2-yl)piperazin-1-yl) methyl)-1-thioxoisoindolin-2-yl)piperidine-2,6-dione | 3-(6-((4-(thieno[2,3-d]pyrimidin-2-yl)piperazin-1-yl) methyl)-1-thioxoisoindolin-2-yl)piperidine-2,6-dione |
| | 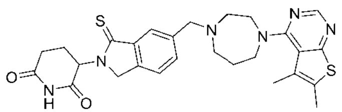 | 3-(6-((4-(5,6-dimethylthieno[2,3-d]pyrimidin-4-yl)-1,4-diazepan-1-yl)methyl)-1-thioxoisoindolin-2-yl)piperidine-2,6-dione | 3-(6-((4-(5,6-dimethylthieno[2,3-d]pyrimidin-4-yl)-1,4-diazepan-1-yl)methyl)-1-thioxoisoindolin-2-yl)piperi-dine-2,6-dione |

(continued)

| Compound No. | Structure of the compounds | The compounds' name | Chinese translation of the compounds' name |
|---|---|---|---|
| | 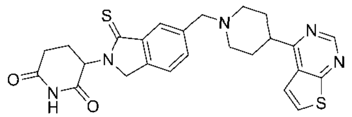 | 3-(6-((4-(thieno[2,3-d]pyrimidin-4-yl)piperidin-1-yl) methyl)-1-thioxoisoindolin-2-yl)piperidine-2,6-dione | 3-(6-((4-(thieno[2,3-d]pyrimidin-4-yl)piperidin-1-yl) methyl)-1-thioxoisoindolin-2-yl)piperidine-2,6-dione |
| | 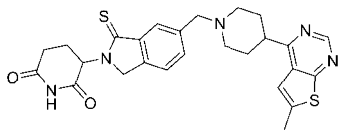 | 3-(6-((4-(6-methylthieno[2,3-d]pyrimidin-4-yl)piperidin-1-yl)methyl)-1-thioxoisoindolin-2-yl)piperidine-2,6-dione | 3-(6-((4-(6-methylthieno[2,3-d]pyrimidin-4-yl)piperidin-1-yl)methyl)-1-thioxoisoindolin-2-yl)piperidine-2,6-dione |
| | 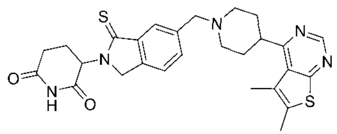 | 3-(6-((4-(5,6-dimethylthieno[2,3-d]pyrimidin-4-yl)piperidin-1-yl)methyl)-1-thioxoisoindolin-2-yl)piperidine-2,6-dione | 3-(6-((4-(5,6-dimethylthieno[2,3-d]pyrimidin-4-yl)piperidin-1-yl)methyl)-1-thioxoisoindolin-2-yl)piperidine-2,6-dione |
| | 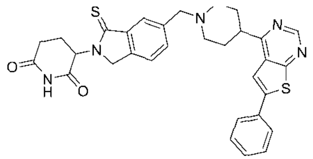 | 3-(6-((4-(6-phenylthieno[2,3-d]pyrimidin-4-yl)piperidin-1-yl)methyl)-1-thioxoisoindolin-2-yl)piperidine-2,6-dione | 3-(6-((4-(6-phenylthieno[2,3-d]pyrimidin-4-yl)piperidin-1-yl)methyl)-1-thioxoisoindolin-2-yl)piperidine-2,6-dione |
| | 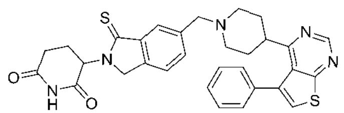 | 3-(6-((4-(5-phenylthieno[2,3-d]pyrimidin-4-yl)piperidin-1-yl)methyl)-1-thioxoisoindolin-2-yl)piperidine-2,6-dione | 3-(6-((4-(5-phenylthieno[2,3-d]pyrimidin-4-yl)piperidin-1-yl)methyl)-1-thioxoisoindolin-2-yl)piperidine-2,6-dione |
| | 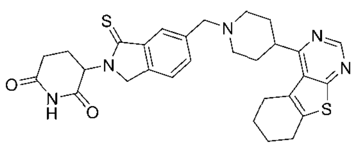 | 3-(6-((4-(5,6,7,8-tetrahydrobenzo[4,5]thieno[2 ,3-d]pyrimidin-4-yl)piperidin-1-yl)methyl)-1-thioxoisoindolin-2-yl)piperidine-2,6-dione | 3-(6-((4-(5,6,7,8-tetrahydrobenzo[4,5]thieno[2 ,3-d]pyrimidin-4-yl)piperidin-1-yl)methyl)-1-thioxoisoindolin-2-yl)piperidine-2,6-dione |
| | 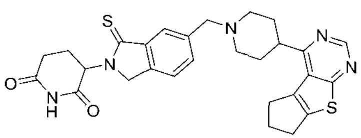 | 3-(6-((4-(6,7-dihydro-5H-cyclopenta[4,5]thieno[2,3-d] pyrimidin-4-yl)piperidin-1-yl)methyl)-1-thioxoisoindolin-2-yl)piperidine-2,6-dione | 3-(6-((4-(6,7-dihydro-5H-cyclopenta[4,5]thieno[2,3-d] pyrimidin-4-yl)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 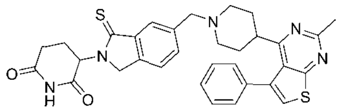 | 3-(6-((4-(2-methyl-5-phenylthieno[2,3-d]pyrimidin-4-yl)piperidin-1-yl)methyl)-1-thioxoisoindolin-2-yl)piperidine-2,6-dione | 3-(6-((4-(2-methyl-5-phenylthieno[2,3-d]pyrimidin-4-yl)piperidin-1-yl)methyl)-1-thioxoisoindolin-2-yl)piperidine-2,6-dione |
| | 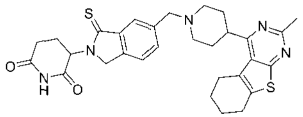 | 3-(6-((4-(2-methyl-5,6,7,8-tetrahydrobenzo[4,5]thieno[2 ,3-d]pyrimidin-4-yl)piperidin-1-yl)methyl)-1-thioxoisoindolin-2-yl)piperidine-2,6-dione | 3-(6-((4-(2-methyl-5,6,7,8-tetrahydrobenzo[4,5]thieno[2 ,3-d]pyrimidin-4-yl)piperidin-1-yl)methyl)-1-thioxoisoindolin-2-yl)piperidine-2,6-dione |
| | 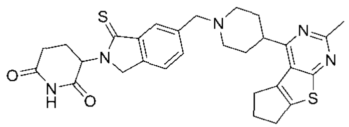 | 3-(6-((4-(2-methyl-6,7-dihydro-5H-cyclopenta[4,5]thieno[2,3-d]pyrimidin-4-yl)piperidin-1-yl)methyl)-1-thioxoisoindolin-2-yl)piperidine-2,6-dione | 3-(6-((4-(2-methyl-6,7-dihydro-5H-cyclopenta[4,5]thieno[2,3-d]pyrimidin-4-yl)piperidin-1-yl)methyl)-1-thioxoisoindolin-2-yl)piperidine-2,6-dione |
| | 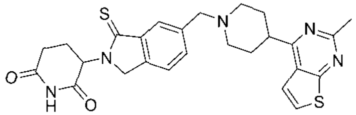 | 3-(6-((4-(2-methylthieno[2,3-d]pyrimidin-4-yl)piperidin-1-yl)methyl)-1-thioxoisoindolin-2-yl)piperidine-2,6-dione | 3-(6-((4-(2-methylthieno[2,3-d]pyrimidin-4-yl)piperidin-1-yl)methyl)-1-thioxoisoindolin-2-yl)piperidine-2,6-dione |
| | 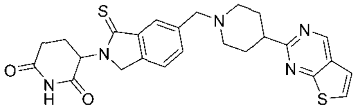 | 3-(6-((4-(thieno[2,3-d]pyrimidin-2-yl)piperidin-1-yl)methyl)-1-thioxoisoindolin-2-yl)piperidine-2,6-dione | 3-(6-((4-(thieno[2,3-d]pyrimidin-2-yl)piperidin-1-yl)methyl)-1-thioxoisoindolin-2-yl)piperidine-2,6-dione |
| | 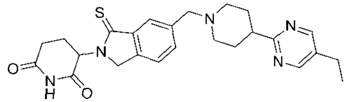 | 3-(6-((4-(5-ethylpyrimidin-2-yl)piperidin-1-yl)methyl)-1-thioxoisoindolin-2-yl)piperidine-2,6-dione | 3-(6-((4-(5-ethylpyrimidin-2-yl)piperidin-1-yl)methyl)-1-thioxoisoindolin-2-yl)piperidine-2,6-dione |
| | 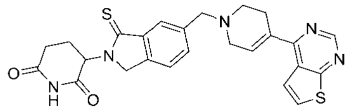 | 3-(6-((4-(thieno[2,3-d]pyrimidin-4-yl)-3,6-dihydropyridin-1 (2H)-yl)methyl)-1-thioxoisoindolin-2-yl)piperidine-2,6-dione | 3-(6-((4-(thieno[2,3-d]pyrimidin-4-yl)-3,6-dihydropyridin-1(2H)-yl)methyl)-1-thioxoisoindolin-2-yl)piperidine-2,6-dione |
| | 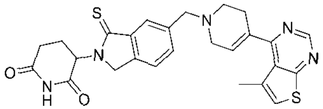 | 3-(6-((4-(5-methylthieno[2,3-d]pyrimidin-4-yl)-3,6-dihydropyridin-1(2H)-yl)methyl)-1-thioxoisoindolin-2-yl)piperidine-2,6-dione | 3-(6-((4-(5-methylthieno[2,3-d]pyrimidin-4-yl)-3,6-dihydropyridin-1(2H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 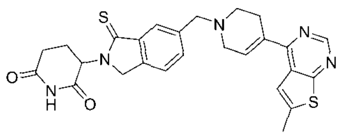 | 3-(6-((4-(6-methylthieno[2,3-d]pyrimidin-4-yl)-3,6-dihydropyridin-1(2H)-yl)methyl)-1-thioxoisoindolin-2-yl)piperidine-2,6-dione | 3-(6-((4-(6-methylthieno[2,3-d]pyrimidin-4-yl)-3,6-dihydropyridin-1(2H)-yl)methyl)-1-thioxoisoindolin-2-yl)piperidine-2,6-dione |
| | 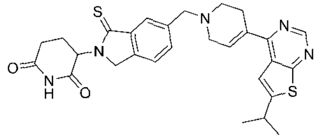 | 3-(6-((4-(6-isopropylthieno[2,3-d]pyrimidin-4-yl)-3,6-dihydropyridin-1(2H)-yl)methyl)-1-thioxoisoindolin-2-yl) piperidine-2,6-dione | 3-(6-((4-(6-isopropylthieno[2,3-d]pyrimidin-4-yl)-3,6-dihydropyridin-1(2H)-yl)methyl)-1-thioxoisoindolin-2-yl) piperidine-2,6-dione |
| | 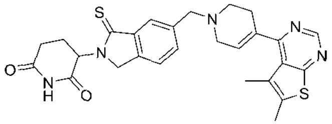 | 3-(6-((4-(5,6-dimethylthieno[2,3-d]pyrimidin-4-yl)-3,6-dihydropyridin-1(2H)-yl)methyl)-1-thioxoisoindolin-2-yl) piperidine-2,6-dione | 3-(6-((4-(5,6-dimethylthieno[2,3-d]pyrimidin-4-yl)-3,6-dihydropyridin-1(2H)-yl)methyl)-1-thioxoisoindolin-2-yl) piperidine-2,6-dione |
| | 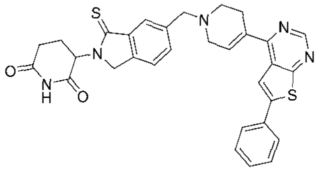 | 3-(6-((4-(6-phenylthieno[2,3-d]pyrimidin-4-yl)-3,6-dihydropyridin-1(2H)-yl)methyl)-1-thioxoisoindolin-2-yl)piperidine-2,6-dione | 3-(6-((4-(6-phenylthieno[2,3-d]pyrimidin-4-yl)-3,6-dihydropyridin-1(2H)-yl)methyl)-1-thioxoisoindolin-2-yl)piperidine-2,6-dione |
| | 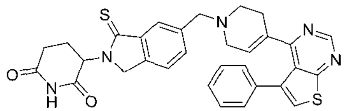 | 3-(6-((4-(5-phenylthieno[2,3-d]pyrimidin-4-yl)-3,6-dihydropyridin-1(2H)-yl)methyl)-1-thioxoisoindolin-2-yl)piperidine-2,6-dione | 3-(6-((4-(5-phenylthieno[2,3-d]pyrimidin-4-yl)-3,6-dihydropyridin-1(2H)-yl)methyl)-1-thioxoisoindolin-2-yl)piperidine-2,6-dione |
| | 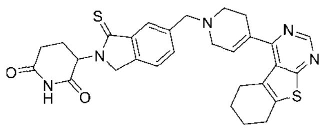 | 3-(6-((4-(5,6,7,8-tetrahydrobenzo[4,5]thieno[2 ,3-d]pyrimidin-4-yl)-3,6-dihydropyridin-1(2H)-yl)methyl)-1-thioxoisoindolin-2-yl)piperidine-2,6-dione | 3-(6-((4-(5,6,7,8-tetrahydrobenzo[4,5]thieno[2 ,3-d]pyrimidin-4-yl)-3,6-dihydropyridin-1(2H)-yl)methyl)-1-thioxoisoindolin-2-yl)piperidine-2,6-dione |
| | 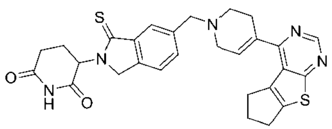 | 3-(6-((4-(6,7-dihydro-5H-cyclopenta[4,5]thieno[2,3-d] pyrimidin-4-yl)-3,6-dihydropyridin-1(2H)-yl)methyl)-1-thioxoisoindolin-2-yl)piperidine-2,6-dione | 3-(6-((4-(6,7-dihydro-5H-cyclopenta[4,5]thieno[2,3-d] pyrimidin-4-yl)-3,6-dihydropyridin-1(2H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 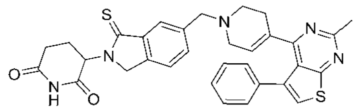 | 3-(6-((4-(2-methyl-5-phenylthieno[2,3-d]pyrimidin-4-yl)-3,6-dihydropyridin-1 (2H)-yl)methyl)-1-thioxoisoindolin-2-yl)piperidine-2,6-dione | 3-(6-((4-(2-methyl-5-phenylthieno[2,3-d]pyrimidin-4-yl)-3,6-dihydropyridin-1(2H)-yl)methyl)-1-thioxoisoindolin-2-yl)piperidine-2,6-dione |
| | 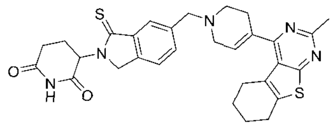 | 3-(6-((4-(2-methyl-5,6,7,8-tetrahydrobenzo[4,5]thieno [2 ,3-d]pyrimidin-4-yl)-3,6-dihydropyridin-1 (2H)-yl) methyl)-1-thioxoisoindolin-2-yl)piperidine-2,6-dione | 3-(6-((4-(2-methyl-5,6,7,8-tetrahydrobenzo[4,5]thieno [2 ,3-d]pyrimidin-4-yl)-3,6-dihydropyridin-1(2H)-yl) methyl)-1-thioxoisoindolin-2-yl)piperidine-2,6-dione |
| | 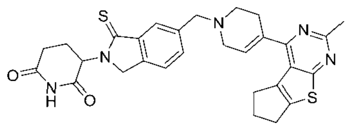 | 3-(6-((4-(2-methyl-6,7-dihydro-5H-cyclopenta[4,5]thieno[2,3-d]pyrimidin-4-yl)-3,6-dihydropyridin-1 (2H)-yl) methyl)-1-thioxoisoindolin-2-yl)piperidine-2,6-dione | 3-(6-((4-(2-methyl-6,7-dihydro-5H-cyclopenta[4,5]thieno[2,3-d]pyrimidin-4-yl)-3,6-dihydropyridin-1(2H)-yl) methyl)-1-thioxoisoindolin-2-yl)piperidine-2,6-dione |
| | 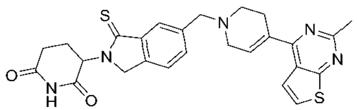 | 3-(6-((4-(2-methylthieno[2,3-d]pyrimidin-4-yl)-3,6-dihydropyridin-1(2H)-yl)methyl)-1-thioxoisoindolin-2-yl)piperidine-2,6-dione | 3-(6-((4-(2-methylthieno[2,3-d]pyrimidin-4-yl)-3,6-dihydropyridin-1(2H)-yl)methyl)-1-thioxoisoindolin-2-yl)piperidine-2,6-dione |
| | 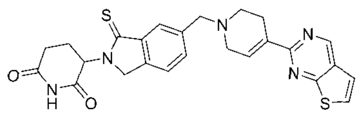 | 3-(6-((4-(thieno[2,3-d]pyrimidin-2-yl)-3,6-dihydropyridin-1 (2H)-yl)methyl)-1-thioxoisoindolin-2-yl)piperidine-2,6-dione | 3-(6-((4-(thieno[2,3-d]pyrimidin-2-yl)-3,6-dihydropyridin-1(2H)-yl)methyl)-1-thioxoisoindolin-2-yl)piperidine-2,6-dione |
| | 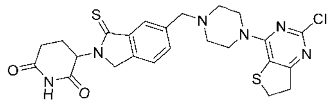 | 3-(6-((4-(2-chloro-6,7-dihydrothieno[3,2-d]pyrimidin-4-yl)piperazin-1-yl)methyl)-1-thioxoisoindolin-2-yl)piperidine-2,6-dione | 3-(6-((4-(2-chloro-6,7-dihydrothieno[3,2-d]pyrimidin-4-yl)piperazin-1-yl)methyl)-1-thioxoisoindolin-2-yl)piperidine-2,6-dione |
| | 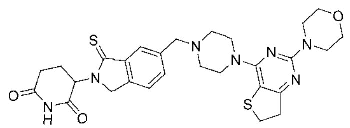 | 3-(6-((4-(2-morpholino-6,7-dihydrothieno[3,2-d]pyrimidin-4-yl)piperazin-1-yl)methyl)-1-thioxoisoindolin-2-yl) piperidine-2,6-dione | 3-(6-((4-(2-morpholino-6,7-dihydrothieno[3,2-d]pyrimidin-4-yl)piperazin-1-yl)methyl)-1-thioxoisoindolin-2-yl) piperidine-2,6-dione |

(continued)

| Compound No. | Structure of the compounds | The compounds' name | Chinese translation of the compounds' name |
| --- | --- | --- | --- |
| | 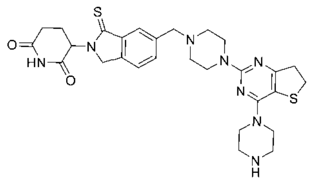 | 3-(6-((4-(4-(piperazin-1-yl)-6,7-dihydrothieno[3,2-d]pyrimidin-2-yl)piperazin-1-yl)methyl)-1-thioxoisoindolin-2-yl)piperidine-2,6-dione | 3-(6-((4-(4-(piperazin-1-yl)-6,7-dihydrothieno[3,2-d]pyrimidin-2-yl)piperazin-1-yl)methyl)-1-thioxoisoindolin-2-yl)piperidine-2,6-dione |
| | 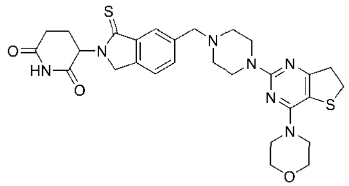 | 3-(6-((4-(4-morpholino-6,7-dihydrothieno[3,2-d]pyrimidin-2-yl)piperazin-1-yl)methyl)-1-thioxoisoindolin-2-yl) piperidine-2,6-dione | 3-(6-((4-(4-morpholino-6,7-dihydrothieno[3,2-d]pyrimidin-2-yl)piperazin-1-yl)methyl)-1-thioxoisoindolin-2-yl) piperidine-2,6-dione |
| | 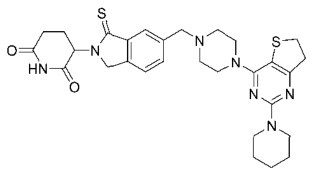 | 3-(6-((4-(2-(piperidin-1-yl)-6,7-dihydrothieno[3,2-d]pyrimidin-4-yl)piperazin-1-yl)methyl)-1-thioxoisoindolin-2-yl)piperidine-2,6-dione | 3-(6-((4-(2-(piperidin-1-yl)-6,7-dihydrothieno[3,2-d]pyrimidin-4-yl)piperazin-1-yl)methyl)-1-thioxoisoindolin-2-yl)piperidine-2,6-dione |
| | 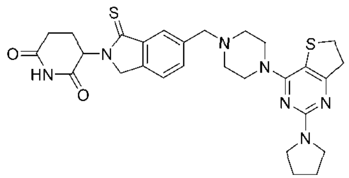 | 3-(6-((4-(2-(pyrrolidin-1-yl)-6,7-dihydrothieno[3,2-d]pyrimidin-4-yl)piperazin-1-yl)methyl)-1-thioxoisoindolin-2-yl)piperidine-2,6-dione | 3-(6-((4-(2-(pyrrolidin-1-yl)-6,7-dihydrothieno[3,2-d]pyrimidin-4-yl)piperazin-1-yl)methyl)-1-thioxoisoindolin-2-yl)piperidine-2,6-dione |
| | 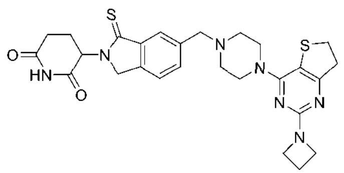 | 3-(6-((4-(2-(azetidin-1-yl)-6,7-dihydrothieno[3,2-d]pyrimidin-4-yl)piperazin-1-yl)methyl)-1-thioxoisoindolin-2-yl)piperidine-2,6-dione | 3-(6-((4-(2-(azetidin-1-yl)-6,7-dihydrothieno[3,2-d]pyrimidin-4-yl)piperazin-1-yl)methyl)-1-thioxoisoindolin-2-yl)piperidine-2,6-dione |
| | 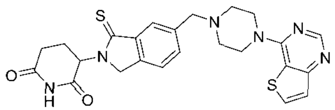 | 3-(6-((4-(thieno[3,2-d]pyrimidin-4-yl)piperazin-1-yl) methyl)-1-thioxoisoindolin-2-yl)piperidine-2,6-dione | 3-(6-((4-(thieno[3,2-d]pyrimidin-4-yl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 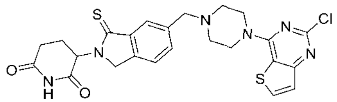 | 3-(6-((4-(2-chlorothieno[3,2-d]pyrimidin-4-yl)piperazin-1-yl)methyl)-1-thioxoisoindolin-2-yl)piperidine-2,6-dione | 3-(6-((4-(2-chlorothieno[3,2-d]pyrimidin-4-yl)piperazin-1-yl)methyl)-1-thioxoisoindolin-2-yl)piperidine-2,6-dione |
| | 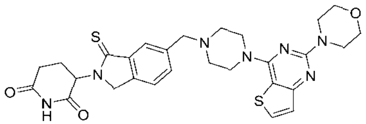 | 3-(6-((4-(2-morpholinothieno[3,2-d]pyrimidin-4-yl)piperazin-1-yl)methyl)-1-thioxoisoindolin-2-yl)piperidine-2,6-dione | 3-(6-((4-(2-morpholinothieno[3,2-d]pyrimidin-4-yl)piperazin-1-yl)methyl)-1-thioxoisoindolin-2-yl)piperidine-2,6-dione |
| | 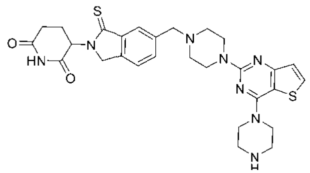 | 3-(6-((4-(4-(piperazin-1-yll)thieno[3,2-d]pyrimidin-2-yl) piperazin-1-yl)methyl)-1-thioxoisoindolin-2-yl)piperidine-2,6-dione | 3-(6-((4-(4-(piperazin-1-yl)thieno[3,2-d]pyrimidin-2-yl) piperazin-1-yl)methyl)-1-thioxoisoindolin-2-yl)piperidine-2,6-dione |
| | 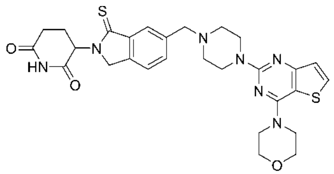 | 3-(6-((4-(4-morpholinothieno[3,2-d]pyrimidin-2-yl)piperazin-1-yl)methyl)-1-thioxoisoindolin-2-yl)piperidine-2,6-dione | 3-(6-((4-(4-morpholinothieno[3,2-d]pyrimidin-2-yl)piperazin-1-yl)methyl)-1-thioxoisoindolin-2-yl)piperidine-2,6-dione |
| GT-05035 | 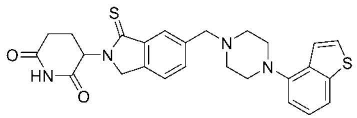 | 3-(6-((4-(benzo[b]thiophen-4-yl)piperazin-1-yl) methyl)-1-thioxoisoindolin-2-yl)piperidine-2,6-dione | 3-(6-((4-(benzo[b]thiophen-4-yl)piperazin-1-yl) methyl)-1-thioxoisoindolin-2-yl)piperidine-2,6-dione |
| | 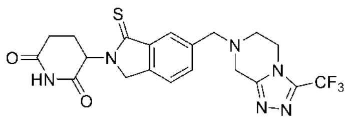 | 3-(1-thioxo-6-((3-(trifluoromethyl)-5,6-dihydro-[1,2,4] triazolo[4,3-a]pyrazin-7(8H)-yl)methyl)isoindolin-2-yl) piperidine-2,6-dione | 3-(1-thioxo-6-((3-(trifluoromethyl)-5,6-dihydro-[1,2,4] triazolo[4,3-a]pyrazin-7(8H)-yl)methyl)isoindolin-2-yl) piperidine-2,6-dione |
| GT-05038 | 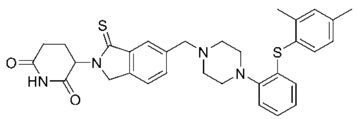 | 3-(6-((4-(2-((2,4-dimethylphenyl)thio)phenyl) piperazin-1-yl)methyl)-1-thioxoisoindolin-2-yl)piperidine-2,6-dione | 3-(6-((4-(2-((2,4-dimethylphenyl)thio)phenyl) piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-05039 | 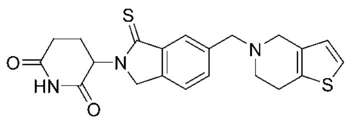 | 3-(6-((6,7-dihydrothieno[3,2-c]pyridin-5(4H)-yl) methyl)-1-thioxoisoindolin-2-yl)piperidine-2,6-dione | 3-(6-((6,7-dihydrothieno[3,2-c]pyridin-5(4H)-yl) methyl)-1-thioxoisoindolin-2-yl)piperidine-2,6-dione |
| GT-05040 | 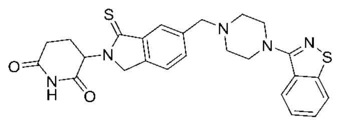 | 3-(6-((4-(benzo[d]isothiazol-3-yl)piperazin-1-yl) methyl)-1-thioxoisoindolin-2-yl)piperidine-2,6-dione | 3-(6-((4-(benzo[d]isothiazol-3-yl)piperazin-1-yl) methyl)-1-thioxoisoindolin-2-yl)piperidine-2,6-dione |
| | 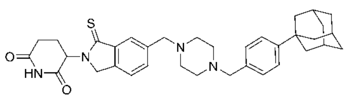 | 3-(6-((4-(4-(adamantan-1-yl)benzyl)piperazin-1-yl) methyl)-1-thioxoisoindolin-2-yl)piperidine-2,6-dione | 3-(6-((4-(4-(adamantan-1-yl)benzyl)piperazin-1-yl) methyl)-1-thioxoisoindolin-2-yl)piperidine-2,6-dione |
| | 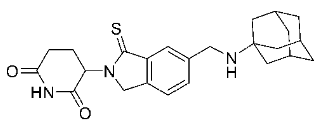 | 3-(6-((adamantan-1-yl)amino)methyl)-1-thioxoisoindolin-2-yl)piperidine-2,6-dione | 3-(6-((adamantan-1-yl)amino)methyl)-1-thioxoisoindolin-2-yl)piperidine-2,6-dione |
| | 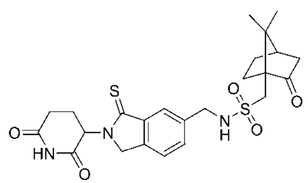 | 1-((1R,4R)-7,7-dimethyl-2-oxobicyclo[2.2.1]heptan-1-yl)-N-((2-(2,6-dioxopiperidin-3-yl)-3-thioxoisoindolin-5-yl)methyl)methanesulfonamide | 1-((1R,4R)-7,7-dimethyl-2-oxobicyclo[2.2.1]heptan-1-yl)-N-((2-(2,6-dioxopiperidin-3-yl)-3-thioxoisoindolin-5-yl)methyl)methanesulfonamide |
| | 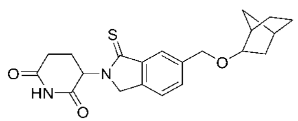 | 3-(6-((((1R,4S)-bicyclo[2.2.1]heptan-2-yl)oxy) methyl)-1-thioxoisoindolin-2-yl)piperidine-2,6-dione | 3-(6-((((1R,4S)-bicyclo[2.2.1]heptan-2-yl)oxy) methyl)-1-thioxoisoindolin-2-yl)piperidine-2,6-dione |
| GT-08191 | 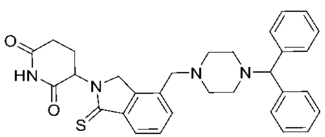 | 3-(4-((4-benzhydrylpiperazin-1-yl)methyl)-1-thioxoisoindolin-2-yl)piperidine-2,6-dione | 3-(4-((4-benzhydrylpiperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0819 2 | 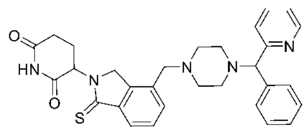 | 3-(4-((4-(phenyl(pyridin-2-yl)methyl)piperazin-1-yl) methyl)-1-thioxoisoindolin-2-yl)piperidine-2,6-dione | 3-(4-((4-(phenyl(pyridin-2-yl)methyl)piperazin-1-yl) methyl)-1-thioxoisoindolin-2-yl)piperidine-2,6-dione |
| GT-0819 3 | 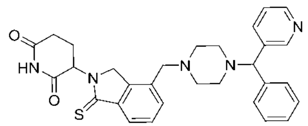 | 3-(4-((4-(phenyl(pyridin-3-yl)methyl)piperazin-1-yl) methyl)-1-thioxoisoindolin-2-yl)piperidine-2,6-dione | 3-(4-((4-(phenyl(pyridin-3-yl)methyl)piperazin-1-yl) methyl)-1-thioxoisoindolin-2-yl)piperidine-2,6-dione |
| GT-0820 4 | 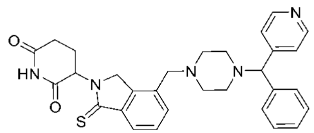 | 3-(4-((4-(phenyl(pyridin-4-yl)methyl)piperazin-1-yl) methyl)-1-thioxoisoindolin-2-yl)piperidine-2,6-dione | 3-(4-((4-(phenyl(pyridin-4-yl)methyl)piperazin-1-yl) methyl)-1-thioxoisoindolin-2-yl)piperidine-2,6-dione |
| GT-0820 1 | 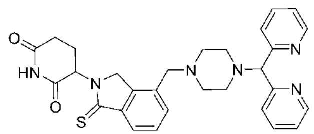 | 3-(4-((4-(di(pyridin-2-yl)methyl)piperazin-1-yl) methyl)-1-thioxoisoindolin-2-yl)piperidine-2,6-dione | 3-(4-((4-(di(pyridin-2-yl)methyl)piperazin-1-yl) methyl)-1-thioxoisoindolin-2-yl)piperidine-2,6-dione |
| | 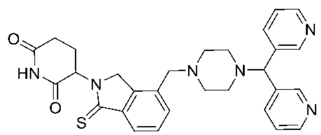 | 3-(4-((4-(di(pyridin-3-yl)methyl)piperazin-1-yl) methyl)-1-thioxoisoindolin-2-yl)piperidine-2,6-dione | 3-(4-((4-(di(pyridin-3-yl)methyl)piperazin-1-yl) methyl)-1-thioxoisoindolin-2-yl)piperidine-2,6-dione |
| | 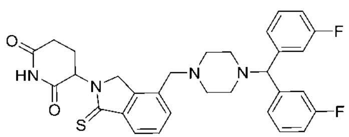 | 3-(4-((4-(bis(3-fluorophenyl)methyl)piperazi n-1-yl) methyl)-1-thioxoisoindolin-2-yl)piperidine-2,6-dione | 3-(4-((4-(bis(3-fluorophenyl)methyl)piperazi n-1-yl) methyl)-1-thioxoisoindolin-2-yl)piperidine-2,6-dione |
| GT-08202 | 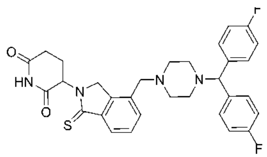 | 3-(4-((4-(bis(4-fluorophenyl)methyl)piperazi n-1-yl) methyl)-1-thioxoisoindolin-2-yl)piperidine-2,6-dione | 3-(4-((4-(bis(4-fluorophenyl)methyl)piperazi n-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08203 | 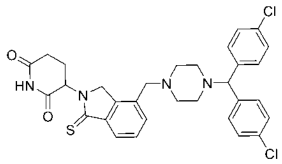 | 3-(4-((4-(bis(4-chlorophenyl)methyl)piperaz in-1-yl) methyl)-1-thioxoisoindolin-2-yl)piperidine-2,6-dione | 3-(4-((4-(bis(4-chlorophenyl)methyl)piperaz in-1-yl) methyl)-1-thioxoisoindolin-2-yl)piperidine-2,6-dione |
| | 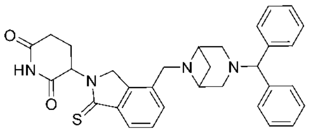 | 3-(4-((3-benzhydryl-3,6-diazabicyclo[3.1.1]heptan-6-yl) methyl)-1-thioxoisoindolin-2-yl)piperidine-2,6-dione | 3-(4-((3-benzhydryl-3,6-diazabicyclo[3.1.1]heptan-6-yl) methyl)-1-thioxoisoindolin-2-yl)piperidine-2,6-dione |
| | 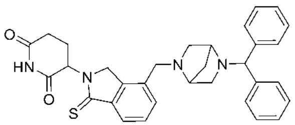 | 3-(4-((5-benzhydryl-2,5-diazabicyclo[2.2.1]heptan-2-yl) methyl)-1-thioxoisoindolin-2-yl)piperidine-2,6-dione | 3-(4-((5-benzhydryl-2,5-diazabicyclo[2.2.1]heptan-2-yl) methyl)-1-thioxoisoindolin-2-yl)piperidine-2,6-dione |
| GT-08208 | 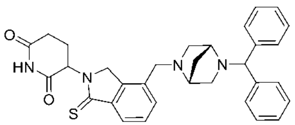 | 3-(4-(((1R,4R)-5-benzhydryl-2,5-diazabicyclo[2.2.1] heptan-2-yl)methyl)-1-thioxoisoindolin-2-yl)piperidine-2,6-dione | 3-(4-(((1R,4R)-5-benzhydryl-2,5-diazabicyclo[2.2.1] heptan-2-yl)methyl)-1-thioxoisoindolin-2-yl)piperidine-2,6-dione |
| GT-08207 | 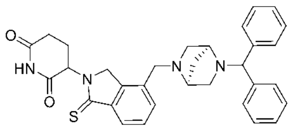 | 3-(4-(((1 S,4S)-5-benzhydryl-2,5-diazabicyclo[2.2.1] heptan-2-yl)methyl)-1-thioxoisoindolin-2-yl)piperidine-2,6-dione | 3-(4-(((1S,4S)-5-benzhydryl-2,5-diazabicyclo[2.2.1] heptan-2-yl)methyl)-1-thioxoisoindolin-2-yl)piperidine-2,6-dione |
| GT-08234 | 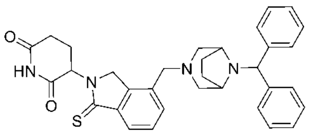 | 3-(4-((8-benzhydryl-3,8-diazabicyclo[3.2.1]octan-3-yl) methyl)-1-thioxoisoindolin-2-yl)piperidine-2,6-dione | 3-(4-((8-benzhydryl-3,8-diazabicyclo[3.2.1]octan-3-yl) methyl)-1-thioxoisoindolin-2-yl)piperidine-2,6-dione |
| GT-08235 | 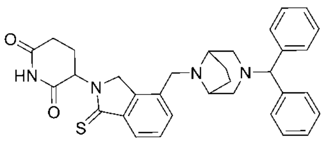 | 3-(4-((3-benzhydryl-3,8-diazabicyclo[3.2.1]octan-8-yl) methyl)-1-thioxoisoindolin-2-yl)piperidine-2,6-dione | 3-(4-((3-benzhydryl-3,8-diazabicyclo[3.2.1]octan-8-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08233 | 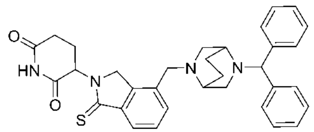 | 3-(4-((5-benzhydryl-2,5-diazabicyclo[2.2.2]octan-2-yl) methyl)-1-thioxoisoindolin-2-yl)piperidine-2,6-dione | 3-(4-((5-benzhydryl-2,5-diazabicyclo[2.2.2]octan-2-yl) methyl)-1-thioxoisoindolin-2-yl)piperidine-2,6-dione |
| | 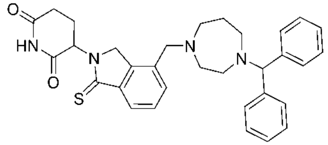 | 3-(4-((4-benzhydryl-1,4-diazepan-1-yl)methyl)-1-thioxoisoindolin-2-yl)piperidine-2,6-dione | 3-(4-((4-benzhydryl-1,4-diazepan-1-yl)methyl)-1-thioxoisoindolin-2-yl)piperidine-2,6-dione |
| | 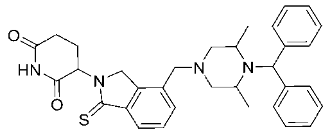 | 3-(4-((4-benzhydryl-3,5-dimethylpiperazin-1-yl) methyl)-1-thioxoisoindolin-2-yl)piperidine-2,6-dione | 3-(4-((4-benzhydryl-3,5-dimethylpiperazin-1-yl) methyl)-1-thioxoisoindolin-2-yl)piperidine-2,6-dione |
| | 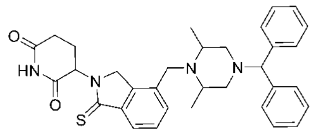 | 3-(4-((4-benzhydryl-2,6-dimethylpiperazin-1-yl) methyl)-1-thioxoisoindolin-2-yl)piperidine-2,6-dione | 3-(4-((4-benzhydryl-2,6-dimethylpiperazin-1-yl) methyl)-1-thioxoisoindolin-2-yl)piperidine-2,6-dione |
| GT-08231 | 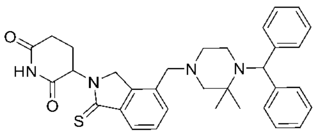 | 3-(4-((4-benzhydryl-3,3-dimethylpiperazin-1-yl) methyl)-1-thioxoisoindolin-2-yl)piperidine-2,6-dione | 3-(4-((4-benzhydryl-3,3-dimethylpiperazin-1-yl) methyl)-1-thioxoisoindolin-2-yl)piperidine-2,6-dione |
| | 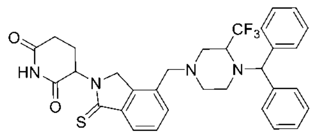 | 3-(4-((4-benzhydryl-2-(trifluoromethyl)piperazin-1-yl) methyl)-1-thioxoisoindolin-2-yl)piperidine-2,6-dione | 3-(4-((4-benzhydryl-2-(trifluoromethyl)piperazin-1-yl) methyl)-1-thioxoisoindolin-2-yl)piperidine-2,6-dione |
| GT-08232 | 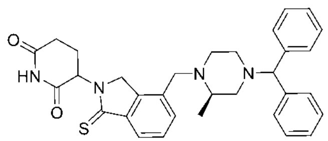 | 3-(4-(((R)-4-benzhydryl-2-methylpiperazin-1-yl) methyl)-1-thioxoisoindolin-2-yl)piperidine-2,6-dione | 3-(4-(((R)-4-benzhydryl-2-methylpiperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 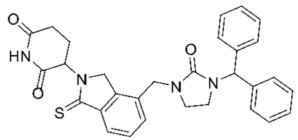 | 3-(4-((3-benzhydryl-2-oxoimidazolidin-1-yl)methyl)-1-thioxoisoindolin-2-yl)piperidine-2,6-dione | 3-(4-((3-benzhydryl-2-oxoimidazolidin-1-yl)methyl)-1-thioxoisoindolin-2-yl)piperidine-2,6-dione |
| GT-08209 | 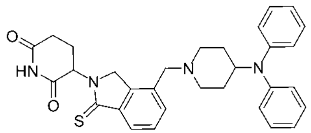 | 3-(4-((4-(diphenylamino)piperidin-1-yl)methyl)-1-thioxoisoindolin-2-yl)piperidine-2,6-dione | 3-(4-((4-(diphenylamino)piperidin-1-yl)methyl)-1-thioxoisoindolin-2-yl)piperidine-2,6-dione |
| GT-08243 | 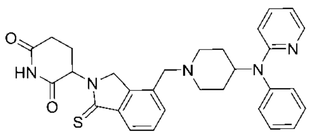 | 3-(4-((4-(phenyl(pyridin-2-yl)amino)piperidin-1-yl)methyl)-1-thioxoisoindolin-2-yl)piperidine-2,6-dione | 3-(4-((4-(phenyl(pyridin-2-yl)amino)piperidin-1-yl)methyl)-1-thioxoisoindolin-2-yl)piperidine-2,6-dione |
| GT-08303 | 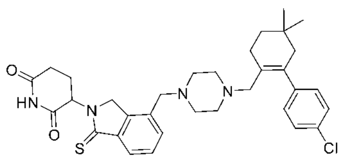 | 3-(4-((4-((4'-chloro-5,5-dimethyl-3,4,5,6-tetrahydro-[1,1'-biphenyl]-2-yl)methyl)piperazin-1-yl)methyl)-1-thioxoisoindolin-2-yl)piperidine-2,6-dione | 3-(4-((4-((4'-chloro-5,5-dimethyl-3,4,5,6-tetrahydro-[1,1'-biphenyl]-2-yl)methyl)piperazin-1-yl)methyl)-1-thioxoisoindolin-2-yl)piperidine-2,6-dione |
| | 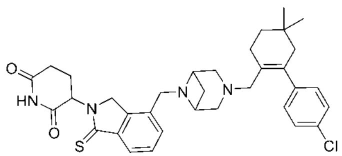 | 3-(4-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-thioxoisoindolin-2-yl)piperidine-2,6-dione | 3-(4-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-thioxoisoindolin-2-yl)piperidine-2,6-dione |
| GT-08271 | 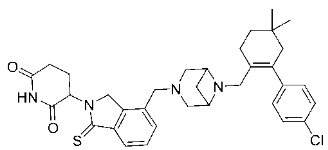 | 3-(4-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-thioxoisoindolin-2-yl)piperidine-2,6-dione | 3-(4-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 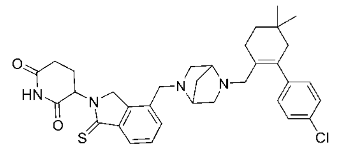 | 3-(4-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-thioxoisoindolin-2-yl)piperidine-2,6-dione | 3-(4-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-thioxoisoindolin-2-yl)piperidine-2,6-dione |
| GT-08291 | 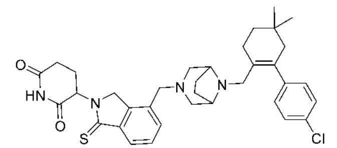 | 3-(4-((8-((4'-chloro-5,5-dimethyl-3,4,5,6-tetrahydro-[1,1-biphenyl]-2-yl)methyl)-3,8-diazabicyclo[3.2.1]octan-3-yl)methyl)-1-thioxoisoindolin-2-yl)piperidine-2,6-dione | 3-(4-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-thioxoisoindolin-2-yl)piperidine-2,6-dione |
| GT-0829 2 | 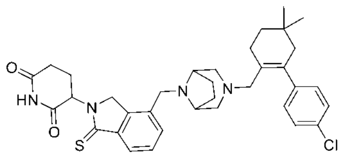 | 3-(4-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-thioxoisoindolin-2-yl)piperidine-2,6-dione | 3-(4-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-thioxoisoindolin-2-yl)piperidine-2,6-dione |
| GT-08302 | 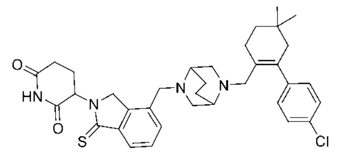 | 3-(4-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-thioxoisoindolin-2-yl)piperidine-2,6-dione | 3-(4-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-thioxoisoindolin-2-yl)piperidine-2,6-dione |
| | 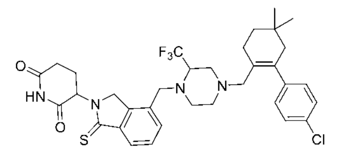 | 3-(4-((4-((4'-chloro-5,5-dimethyl-3,4,5,6-tetrahydro-[1,1'-biphenyl]-2-yl)methyl)-2-(trifluoromethyl)piperazin-1-yl)methyl)-1-thioxoisoindolin-2-yl)piperidine-2,6-dione | 3-(4-((4-((4'-chloro-5,5-dimethyl-3,4,5,6-tetrahydro-[1,1'-biphenyl]-2-yl)methyl)-2-(trifluoromethyl)piperazin-1-yl)methyl)-1-thioxoisoindolin-2-yl)piperidine-2,6-dione |
| GT-08293 | 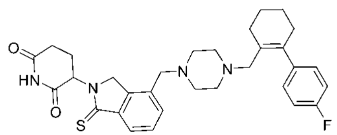 | 3-(4-((4-((4'-fluoro-3,4,5,6-tetrahydro-[1,1'-biphenyl]-2-yl)methyl)piperazin-1-yl)methyl)-1-thioxoisoindolin-2-yl)piperidine-2,6-dione | 3-(4-((4-((4'-fluoro-3,4,5,6-tetrahydro-[1,1'-biphenyl]-2-yl)methyl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08294 | 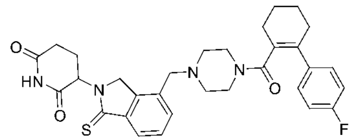 | 3-(4-((4-(4'-fluoro-3,4,5,6-tetrahydro-[1,1'-biphenyl]-2-carbonyl)piperazin-1-yl)methyl)-1-thioxoisoindolin-2-yl) piperidine-2,6-dione | 3-(4-((4-(4'-fluoro-3,4,5,6-tetrahydro-[1,1'-biphenyl]-2-carbonyl)piperazin-1-yl)methyl)-1-thioxoisoindolin-2-yl) piperidine-2,6-dione |
| | 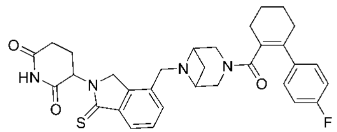 | 3-(4-((3-(4'-fluoro-3,4,5,6-tetrahydro-[1,1'-biphenyl]-2-carbonyl)-3,6-diazabicyclo[3.1.1]heptan-6-yl) methyl)-1-thioxoisoindolin-2-yl)piperidine-2,6-dione | 3-(4-((3-(4'-fluoro-3,4,5,6-tetrahydro-[1,1'-biphenyl]-2-carbonyl)-3,6-diazabicyclo[3.1.1]heptan-6-yl) methyl)-1-thioxoisoindolin-2-yl)piperidine-2,6-dione |
| GT-09034 | 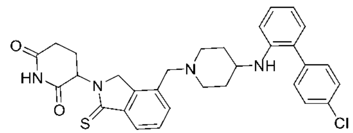 | 3-(4-((4-((4'-chloro-[1,1'-biphenyl]-2-yl)amino)piperidin-1-yl)methyl)-1-thioxoisoindolin-2-yl)piperidine-2,6-dione | 3-(4-((4-((4'-chloro-[1,1'-biphenyl]-2-yl)amino)piperidin-1-yl)methyl)-1-thioxoisoindolin-2-yl)piperidine-2,6-dione |
| GT-08295 | 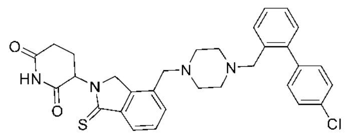 | 3-(4-((4-((4'-chloro-[1,1'-biphenyl]-2-yl)methyl)piperazin-1-yl)methyl)-1-thioxoisoindolin-2-yl)piperidine-2,6-dione | 3-(4-((4-((4'-chloro-[1,1'-biphenyl]-2-yl)methyl)piperazin-1-yl)methyl)-1-thioxoisoindolin-2-yl)piperidine-2,6-dione |
| GT-08296 | 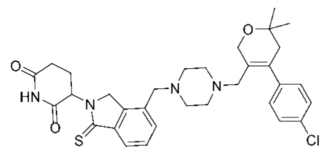 | 3-(4-((4-((4-(4-chlorophenyl)-6,6-dimethyl-5,6-dihydro-2H-pyran-3-yl)methyl)piperazin-1-yl)methyl)-1-thioxoisoindolin-2-yl)piperidine-2,6-dione | 3-(4-((4-((4-(4-chlorophenyl)-6,6-dimethyl-5,6-dihydro-2H-pyran-3-yl)methyl)piperazin-1-yl)methyl)-1-thioxoisoindolin-2-yl)piperidine-2,6-dione |
| GT-08297 | 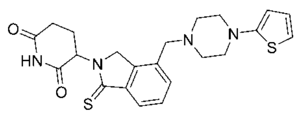 | 3-(4-((4-(thiophen-2-yl)piperazin-1-yl)methyl)-1-thioxoisoindolin-2-yl)piperidine-2,6-dione | 3-(4-((4-(thiophen-2-yl)piperazin-1-yl)methyl)-1-thioxoisoindolin-2-yl)piperidine-2,6-dione |
| GT-0830 5 | 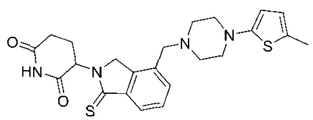 | 3-(4-((4-(5-methylthiophen-2-yl)piperazin-1-yl) methyl)-1-thioxoisoindolin-2-yl)piperidine-2,6-dione | 3-(4-((4-(5-methylthiophen-2-yl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 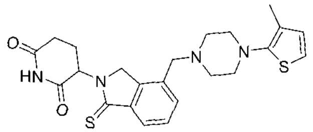 | 3-(4-((4-(3-methylthiophen-2-yl)piperazin-1-yl) methyl)-1-thioxoisoindolin-2-yl)piperidine-2,6-dione | 3-(4-((4-(3-methylthiophen-2-yl)piperazin-1-yl) methyl)-1-thioxoisoindolin-2-yl)piperidine-2,6-dione |
| GT-0831 3 | 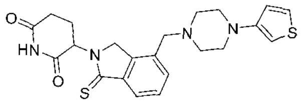 | 3-(4-((4-(thiophen-3-yl)piperazin-1-yl)methyl)-1-thioxoisoindolin-2-yl)piperidine-2,6-dione | 3-(4-((4-(thiophen-3-yl)piperazin-1-yl)methyl)-1-thioxoisoindolin-2-yl)piperidine-2,6-dione |
| GT-08306 | 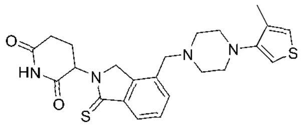 | 3-(4-((4-(4-methylthiophen-3-yl)piperazin-1-yl) methyl)-1-thioxoisoindolin-2-yl)piperidine-2,6-dione | 3-(4-((4-(4-methylthiophen-3-yl)piperazin-1-yl) methyl)-1-thioxoisoindolin-2-yl)piperidine-2,6-dione |
| GT-08298 | 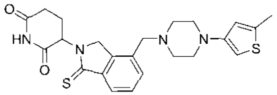 | 3-(4-((4-(5-methylthiophen-3-yl)piperazin-1-yl) methyl)-1-thioxoisoindolin-2-yl)piperidine-2,6-dione | 3-(4-((4-(5-methylthiophen-3-yl)piperazin-1-yl) methyl)-1-thioxoisoindolin-2-yl)piperidine-2,6-dione |
| GT-08299 | 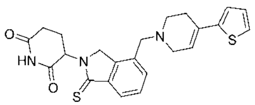 | 3-(4-((4-(thiophen-2-yl)-3,6-dihydropyridin-1 (2H)-yl) methyl)-1-thioxoisoindolin-2-yl)piperidine-2,6-dione | 3-(4-((4-(thiophen-2-yl)-3,6-dihydropyridin-1(2H)-yl) methyl)-1-thioxoisoindolin-2-yl)piperidine-2,6-dione |
| GT-08307 | 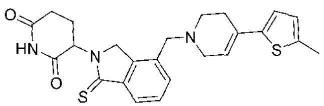 | 3-(4-((4-(5-methylthiophen-2-yl)-3,6-dihydropyridin-1(2H)-yl)methyl)-1-thioxoisoindolin-2-yl)piperidine-2,6-dione | 3-(4-((4-(5-methylthiophen-2-yl)-3,6-dihydropyridin-1(2H)-yl)methyl)-1-thioxoisoindolin-2-yl)piperi-dine-2,6-dione |
| | 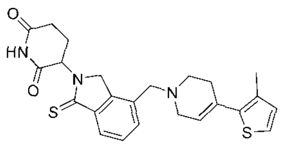 | 3-(4-((4-(3-methylthiophen-2-yl)-3,6-dihydropyridin-1(2H)-yl)methyl)-1-thioxoisoindolin-2-yl)piperidine-2,6-dione | 3-(4-((4-(3-methylthiophen-2-yl)-3,6-dihydropyridin-1(2H)-yl)methyl)-1-thioxoisoindolin-2-yl)piperidine-2,6-dione |
| GT-08300 | 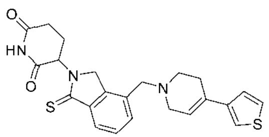 | 3-(4-((4-(thiophen-3-yl)-3,6-dihydropyridin-1(2H)-yl) methyl)-1-thioxoisoindolin-2-yl)piperidine-2,6-dione | 3-(4-((4-(thiophen-3-yl)-3,6-dihydropyridin-1(2H)-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08308 | 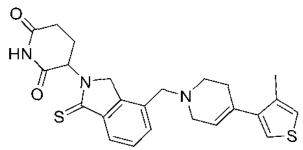 | 3-(4-((4-(4-methylthiophen-3-yl)-3,6-dihydropyridin-1(2H)-yl)methyl)-1-thioxoisoindolin-2-yl)piperidine-2,6-dione | 3-(4-((4-(4-methylthiophen-3-yl)-3,6-dihydropyridin-1(2H)-yl)methyl)-1-thioxoisoindolin-2-yl)piperidine-2,6-dione |
| GT-08318 | 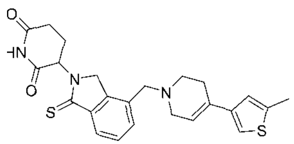 | 3-(4-((4-(5-methylthiophen-3-yl)-3,6-dihydropyridin-1(2H)-yl)methyl)-1-thioxoisoindolin-2-yl)piperidine-2,6-dione | 3-(4-((4-(5-methylthiophen-3-yl)-3,6-dihydropyridin-1(2H)-yl)methyl)-1-thioxoisoindolin-2-yl)piperidine-2,6-dione |
| GT-08311 | 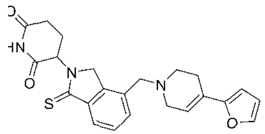 | 3-(4-((4-(furan-2-yl)-3,6-dihydropyridin-1 (2H)-yl) methyl)-1-thioxoisoindolin-2-yl)piperidine-2,6-dione | 3-(4-((4-(furan-2-yl)-3,6-dihydropyridin-1(2H)-yl) methyl)-1-thioxoisoindolin-2-yl)piperidine-2,6-dione |
| GT-08319 | 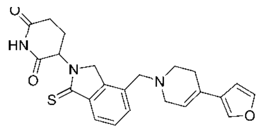 | 3-(4-((4-(furan-3-yl)-3,6-dihydropyridin-1 (2H)-yl) methyl)-1-thioxoisoindolin-2-yl)piperidine-2,6-dione | 3-(4-((4-(furan-3-yl)-3,6-dihydropyridin-1(2H)-yl) methyl)-1-thioxoisoindolin-2-yl)piperidine-2,6-dione |
| GT-08320 | 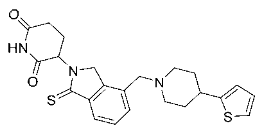 | 3-(4-((4-(thiophen-2-yl)piperidin-1-yl)methyl)-1-thioxoisoindolin-2-yl)piperidine-2,6-dione | 3-(4-((4-(thiophen-2-yl)piperidin-1-yl)methyl)-1-thioxoisoindolin-2-yl)piperidine-2,6-dione |
| GT-08312 | 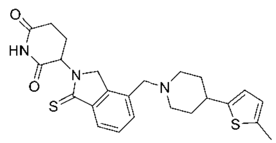 | 3-(4-((4-(5-methylthiophen-2-yl)piperidin-1-yl) methyl)-1-thioxoisoindolin-2-yl)piperidine-2,6-dione | 3-(4-((4-(5-methylthiophen-2-yl)piperidin-1-yl) methyl)-1-thioxoisoindolin-2-yl)piperidine-2,6-dione |
| GT-08314 | 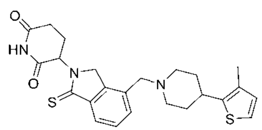 | 3-(4-((4-(3-methylthiophen-2-yl)piperidin-1-yl) methyl)-1-thioxoisoindolin-2-yl)piperidine-2,6-dione | 3-(4-((4-(3-methylthiophen-2-yl)piperidin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08315 | 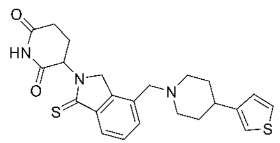 | 3-(4-((4-(thiophen-3-yl)piperidin-1-yl)methyl)-1-thioxoisoindolin-2-yl)piperidine-2,6-dione | 3-(4-((4-(thiophen-3-yl)piperidin-1-yl)methyl)-1-thioxoisoindolin-2-yl)piperidine-2,6-dione |
| GT-08316 | 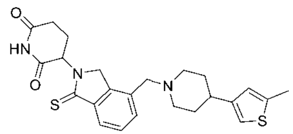 | 3-(4-((4-(5-methylthiophen-3-yl)piperidin-1-yl) methyl)-1-thioxoisoindolin-2-yl)piperidine-2,6-dione | 3-(4-((4-(5-methylthiophen-3-yl)piperidin-1-yl) methyl)-1-thioxoisoindolin-2-yl)piperidine-2,6-dione |
| GT-08309 | 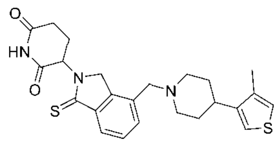 | 3-(4-((4-(4-methylthiophen-3-yl)piperidin-1-yl) methyl)-1-thioxoisoindolin-2-yl)piperidine-2,6-dione | 3-(4-((4-(4-methylthiophen-3-yl)piperidin-1-yl) methyl)-1-thioxoisoindolin-2-yl)piperidine-2,6-dione |
| GT-08310 | 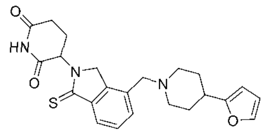 | 3-(4-((4-(furan-2-yl)piperidin-1-yl)methyl)-1-thioxoisoindolin-2-yl)piperidine-2,6-dione | 3-(4-((4-(furan-2-yl)piperidin-1-yl)methyl)-1-thioxoisoindolin-2-yl)piperidine-2,6-dione |
| GT-08317 | 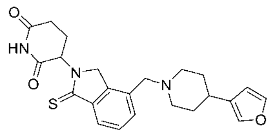 | 3-(4-((4-(furan-3-yl)piperidin-1-yl)methyl)-1-thioxoisoindolin-2-yl)piperidine-2,6-dione | 3-(4-((4-(furan-3-yl)piperidin-1-yl)methyl)-1-thioxoisoindolin-2-yl)piperidine-2,6-dione |
| GT-08258 | 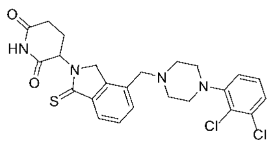 | 3-(4-((4-(2,3-dichlorophenyl)piperazin-1-yl)methyl)-1-thioxoisoindolin-2-yl)piperidine-2,6-dione | 3-(4-((4-(2,3-dichlorophenyl)piperazin-1-yl)methyl)-1-thioxoisoindolin-2-yl)piperidine-2,6-dione |
| GT-08259 | 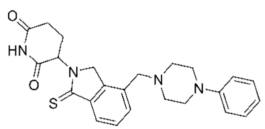 | 3-(4-((4-phenylpiperazin-1-yl)methyl)-1-thioxoisoindolin-2-yl)piperidine-2,6-dione | 3-(4-((4-phenylpiperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08260 | 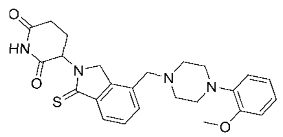 | 3-(4-((4-(2-methoxyphenyl)piperazin-1-yl)methyl)-1-thioxoisoindolin-2-yl)piperidine-2,6-dione | 3-(4-((4-(2-methoxyphenyl)piperazin-1-yl)methyl)-1-thioxoisoindolin-2-yl)piperidine-2,6-dione |
| GT-08304 | 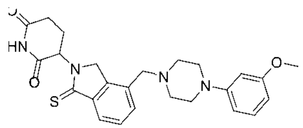 | 3-(4-((4-(3-methoxyphenyl)piperazin-1-yl)methyl)-1-thioxoisoindolin-2-yl)piperidine-2,6-dione | 3-(4-((4-(3-methoxyphenyl)piperazin-1-yl)methyl)-1-thioxoisoindolin-2-yl)piperidine-2,6-dione |
| GT-08495 | 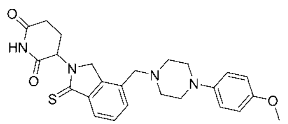 | 3-(4-((4-(4-methoxyphenyl)piperazin-1-yl)methyl)-1-thioxoisoindolin-2-yl)piperidine-2,6-dione | 3-(4-((4-(4-methoxyphenyl)piperazin-1-yl)methyl)-1-thioxoisoindolin-2-yl)piperidine-2,6-dione |
| GT-08262 | 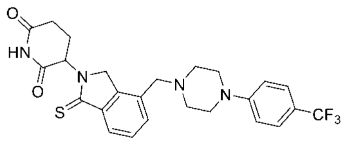 | 3-(1-thioxo-4-((4-(4-(trifluoromethyl)phenyl)piper azin-1-yl)methyl)isoindolin-2-yl)piperidine-2,6-dione | 3-(1-thioxo-4-((4-(4-(trifluoromethyl)phenyl)piper azin-1-yl)methyl)isoindolin-2-yl)piperidine-2,6-dione |
| GT-08263 | 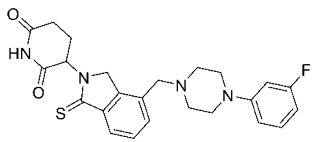 | 3-(4-((4-(3-fluorophenyl)piperazin-1-yl)methyl)-1-thioxoisoindolin-2-yl)piperidine-2,6-dione | 3-(4-((4-(3-fluorophenyl)piperazin-1-yl)methyl)-1-thioxoisoindolin-2-yl)piperidine-2,6-dione |
| GT-08264 | 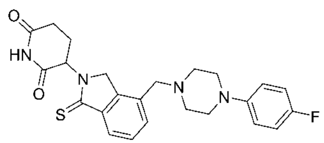 | 3-(4-((4-(4-fluorophenyl)piperazin-1-yl)methyl)-1-thioxoisoindolin-2-yl)piperidine-2,6-dione | 3-(4-((4-(4-fluorophenyl)piperazin-1-yl)methyl)-1-thioxoisoindolin-2-yl)piperidine-2,6-dione |
| GT-08265 | 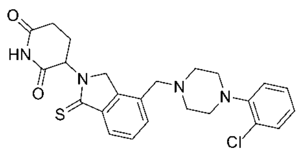 | 3-(4-((4-(2-chlorophenyl)piperazin-1-yl)methyl)-1-thioxoisoindolin-2-yl)piperidine-2,6-dione | 3-(4-((4-(2-chlorophenyl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08266 | 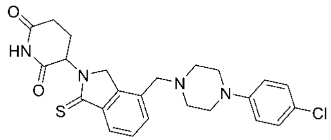 | 3-(4-((4-(4-chlorophenyl)piperazin-1-yl)methyl)-1-thioxoisoindolin-2-yl)piperidine-2,6-dione | 3-(4-((4-(4-chlorophenyl)piperazin-1-yl)methyl)-1-thioxoisoindolin-2-yl)piperidine-2,6-dione |
| GT-08267 | 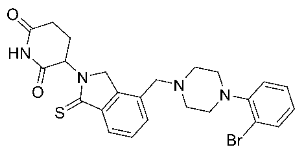 | 3-(4-((4-(2-bromophenyl)piperazin-1-yl)methyl)-1-thioxoisoindolin-2-yl)piperidine-2,6-dione | 3-(4-((4-(2-bromophenyl)piperazin-1-yl)methyl)-1-thioxoisoindolin-2-yl)piperidine-2,6-dione |
| GT-08261 | 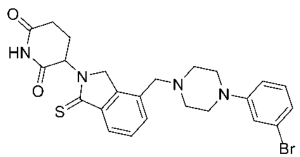 | 3-(4-((4-(3-bromophenyl)piperazin-1-yl)methyl)-1-thioxoisoindolin-2-yl)piperidine-2,6-dione | 3-(4-((4-(3-bromophenyl)piperazin-1-yl)methyl)-1-thioxoisoindolin-2-yl)piperidine-2,6-dione |
| GT-08496 | 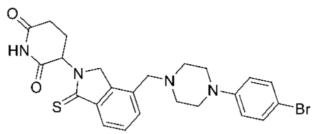 | 3-(4-((4-(4-bromophenyl)piperazin-1-yl)methyl)-1-thioxoisoindolin-2-yl)piperidine-2,6-dione | 3-(4-((4-(4-bromophenyl)piperazin-1-yl)methyl)-1-thioxoisoindolin-2-yl)piperidine-2,6-dione |
| GT-0849 7 | 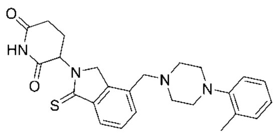 | 3-(1-thioxo-4-((4-(o-tolyl)piperazin-1-yl)methyl)isoindolin-2-yl)piperidine-2,6-dione | 3-(1-thioxo-4-((4-(o-tolyl)piperazin-1-yl)methyl)isoindolin-2-yl)piperidine-2,6-dione |
| GT-0849 8 | 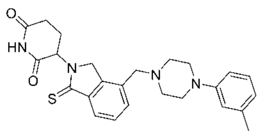 | 3-(1-thioxo-4-((4-(m-tolyl)piperazin-1-yl)methyl)isoindolin-2-yl)piperidine-2,6-dione | 3-(1-thioxo-4-((4-(m-tolyl)piperazin-1-yl)methyl)isoindolin-2-yl)piperidine-2,6-dione |
| GT-0849 9 | 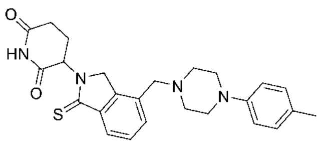 | 3-(1-thioxo-4-((4-(p-tolyl)piperazin-1-yl)methyl)isoindolin-2-yl)piperidine-2,6-dione | 3-(1-thioxo-4-((4-(p-tolyl)piperazin-1-yl)methyl)isoindolin-2-yl)piperidine-2,6-dione |

(continued)

| Compound No. | Structure of the compounds | The compounds' name | Chinese translation of the compounds' name |
|---|---|---|---|
| GT-0850 0 | 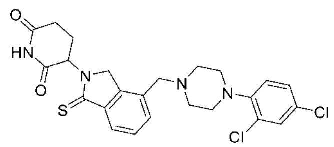 | 3-(4-((4-(2,4-dichlorophenyl)piperazin-1-yl)methyl)-1-thioxoisoindolin-2-yl)piperidine-2,6-dione | 3-(4-((4-(2,4-dichlorophenyl)piperazin-1-yl)methyl)-1-thioxoisoindolin-2-yl)piperidine-2,6-dione |
| GT-08501 | 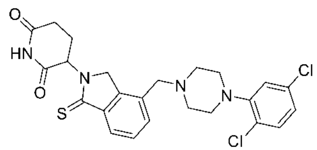 | 3-(4-((4-(2,5-dichlorophenyl)piperazin-1-yl)methyl)-1-thioxoisoindolin-2-yl)piperidine-2,6-dione | 3-(4-((4-(2,5-dichlorophenyl)piperazin-1-yl)methyl)-1-thioxoisoindolin-2-yl)piperidine-2,6-dione |
| | 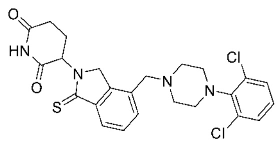 | 3-(4-((4-(2,6-dichlorophenyl)piperazin-1-yl)methyl)-1-thioxoisoindolin-2-yl)piperidine-2,6-dione | 3-(4-((4-(2,6-dichlorophenyl)piperazin-1-yl)methyl)-1-thioxoisoindolin-2-yl)piperidine-2,6-dione |
| GT-08502 | 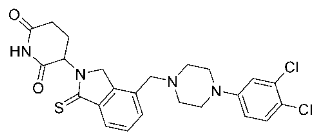 | 3-(4-((4-(3,4-dichlorophenyl)piperazin-1-yl)methyl)-1-thioxoisoindolin-2-yl)piperidine-2,6-dione | 3-(4-((4-(3,4-dichlorophenyl)piperazin-1-yl)methyl)-1-thioxoisoindolin-2-yl)piperidine-2,6-dione |
| GT-08629 | 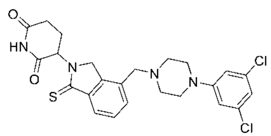 | 3-(4-((4-(3,5-dichlorophenyl)piperazin-1-yl)methyl)-1-thioxoisoindolin-2-yl)piperidine-2,6-dione | 3-(4-((4-(3,5-dichlorophenyl)piperazin-1-yl)methyl)-1-thioxoisoindolin-2-yl)piperidine-2,6-dione |
| GT-08268 | 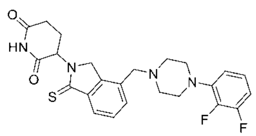 | 3-(4-((4-(2,3-difluorophenyl)piperazin-1-yl)methyl)-1-thioxoisoindolin-2-yl)piperidine-2,6-dione | 3-(4-((4-(2,3-difluorophenyl)piperazin-1-yl)methyl)-1-thioxoisoindolin-2-yl)piperidine-2,6-dione |
| GT-08503 | 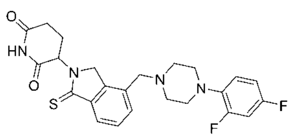 | 3-(4-((4-(2,4-difluorophenyl)piperazin-1-yl)methyl)-1-thioxoisoindolin-2-yl)piperidine-2,6-dione | 3-(4-((4-(2,4-difluorophenyl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08593 | 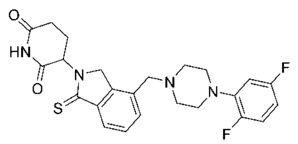 | 3-(4-((4-(2,5-difluorophenyl)piperazin-1-yl)methyl)-1-thioxoisoindolin-2-yl)piperidine-2,6-dione | 3-(4-((4-(2,5-difluorophenyl)piperazin-1-yl)methyl)-1-thioxoisoindolin-2-yl)piperidine-2,6-dione |
| GT-08633 | 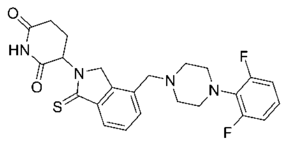 | 3-(4-((4-(2,6-difluorophenyl)piperazin-1-yl)methyl)-1-thioxoisoindolin-2-yl)piperidine-2,6-dione | 3-(4-((4-(2,6-difluorophenyl)piperazin-1-yl)methyl)-1-thioxoisoindolin-2-yl)piperidine-2,6-dione |
| GT-08631 | 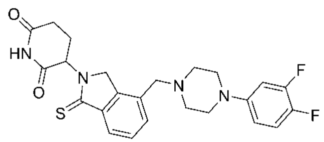 | 3-(4-((4-(3,4-difluorophenyl)piperazin-1-yl)methyl)-1-thioxoisoindolin-2-yl)piperidine-2,6-dione | 3-(4-((4-(3,4-difluorophenyl)piperazin-1-yl)methyl)-1-thioxoisoindolin-2-yl)piperidine-2,6-dione |
| GT-08632 | 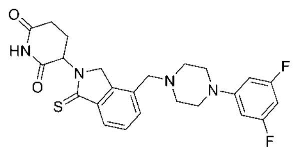 | 3-(4-((4-(3,5-difluorophenyl)piperazin-1-yl)methyl)-1-thioxoisoindolin-2-yl)piperidine-2,6-dione | 3-(4-((4-(3,5-difluorophenyl)piperazin-1-yl)methyl)-1-thioxoisoindolin-2-yl)piperidine-2,6-dione |
| GT-08504 | 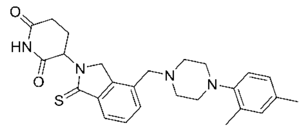 | 3-(4-((4-(2,4-dimethylphenyl)piperazin-1-yl)methyl)-1-thioxoisoindolin-2-yl)piperidine-2,6-dione | 3-(4-((4-(2,4-dimethylphenyl)piperazin-1-yl)methyl)-1-thioxoisoindolin-2-yl)piperidine-2,6-dione |
| GT-08505 | 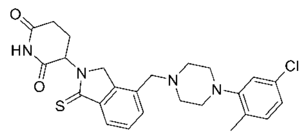 | 3-(4-((4-(5-chloro-2-methylphenyl)piperazin-1-yl) methyl)-1-thioxoisoindolin-2-yl)piperidine-2,6-dione | 3-(4-((4-(5-chloro-2-methylphenyl)piperazin-1-yl) methyl)-1-thioxoisoindolin-2-yl)piperidine-2,6-dione |
| GT-08506 | 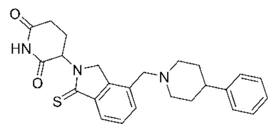 | 3-(4-((4-phenylpiperidin-1-yl)methyl)-1-thioxoisoindolin-2-yl)piperidine-2,6-dione | 3-(4-((4-phenylpiperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08507 | 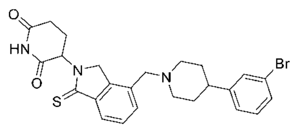 | 3-(4-((4-(3-bromophenyl)piperidin-1-yl)methyl)-1-thioxoisoindolin-2-yl)piperidine-2,6-dione | 3-(4-((4-(3-bromophenyl)piperidin-1-yl)methyl)-1-thioxoisoindolin-2-yl)piperidine-2,6-dione |
| GT-08592 | 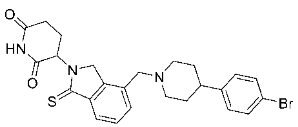 | 3-(4-((4-(4-bromophenyl)piperidin-1-yl)methyl)-1-thioxoisoindolin-2-yl)piperidine-2,6-dione | 3-(4-((4-(4-bromophenyl)piperidin-1-yl)methyl)-1-thioxoisoindolin-2-yl)piperidine-2,6-dione |
| | 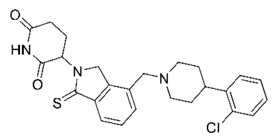 | 3-(4-((4-(2-chlorophenyl)piperidin-1-yl)methyl)-1-thioxoisoindolin-2-yl)piperidine-2,6-dione | 3-(4-((4-(2-chlorophenyl)piperidin-1-yl)methyl)-1-thioxoisoindolin-2-yl)piperidine-2,6-dione |
| GT-08594 | 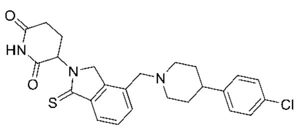 | 3-(4-((4-(4-chlorophenyl)piperidin-1-yl)methyl)-1-thioxoisoindolin-2-yl)piperidine-2,6-dione | 3-(4-((4-(4-chlorophenyl)piperidin-1-yl)methyl)-1-thioxoisoindolin-2-yl)piperidine-2,6-dione |
| GT-08595 | 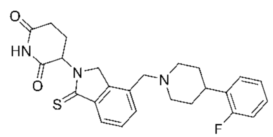 | 3-(4-((4-(2-fluorophenyl)piperidin-1-yl)methyl)-1-thioxoisoindolin-2-yl)piperidine-2,6-dione | 3-(4-((4-(2-fluorophenyl)piperidin-1-yl)methyl)-1-thioxoisoindolin-2-yl)piperidine-2,6-dione |
| GT-08596 | 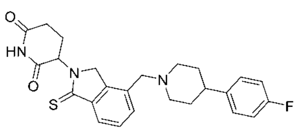 | 3-(4-((4-(4-fluorophenyl)piperidin-1-yl)methyl)-1-thioxoisoindolin-2-yl)piperidine-2,6-dione | 3-(4-((4-(4-fluorophenyl)piperidin-1-yl)methyl)-1-thioxoisoindolin-2-yl)piperidine-2,6-dione |
| GT-08598 | 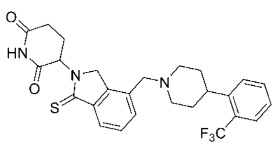 | 3-(1-thioxo-4-((4-(2-(trifluoromethyl)phenyl)piper idin-1-yl)methyl)isoindolin-2-yl)piperidine-2,6-dione | 3-(1-thioxo-4-((4-(2-(trifluoromethyl)phenyl)piper idin-1-yl)methyl)isoindolin-2-yl)piperidine-2,6-dione |

(continued)

| Compound No. | Structure of the compounds | The compounds' name | Chinese translation of the compounds' name |
|---|---|---|---|
| GT-08599 | 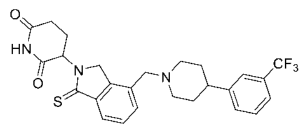 | 3-(1-thioxo-4-((4-(3-(trifluoromethyl)phenyl)piper idin-1-yl)methyl)isoindolin-2-yl)piperidine-2,6-dione | 3-(1-thioxo-4-((4-(3-(trifluoromethyl)phenyl)piper idin-1-yl)methyl)isoindolin-2-yl)piperidine-2,6-dione |
| GT-08269 | 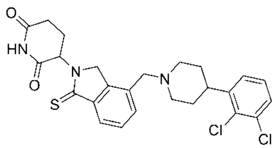 | 3-(4-((4-(2,3-dichlorophenyl)piperidin-1-yl)methyl)-1-thioxoisoindolin-2-yl)piperidine-2,6-dione | 3-(4-((4-(2,3-dichlorophenyl)piperidin-1-yl)methyl)-1-thioxoisoindolin-2-yl)piperidine-2,6-dione |
| GT-08321 | 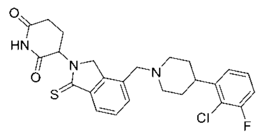 | 3-(4-((4-(2-chloro-3-fluorophenyl)piperidin-1-yl) methyl)-1-thioxoisoindolin-2-yl)piperidine-2,6-dione | 3-(4-((4-(2-chloro-3-fluorophenyl)piperidin-1-yl) methyl)-1-thioxoisoindolin-2-yl)piperidine-2,6-dione |
| GT-08322 | 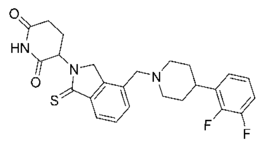 | 3-(4-((4-(2,3-difluorophenyl)piperidin-1-yl)methyl)-1-thioxoisoindolin-2-yl)piperidine-2,6-dione | 3-(4-((4-(2,3-difluorophenyl)piperidin-1-yl)methyl)-1-thioxoisoindolin-2-yl)piperidine-2,6-dione |
| GT-08270 | 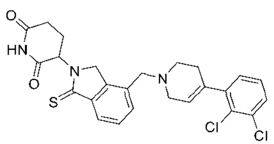 | 3-(4-((4-(2,3-dichlorophenyl)-3,6-dihydropyridin-1(2H)-yl)methyl)-1-thioxoisoindolin-2-yl)piperidine-2,6-dione | 3-(4-((4-(2,3-dichlorophenyl)-3,6-dihydropyridin-1 (2H)-yl)methyl)-1-thioxoisoindolin-2-yl)piperidine-2,6-dione |
| GT-08301 | 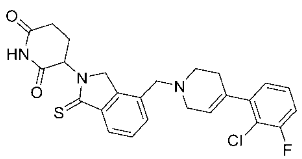 | 3-(4-((4-(2-chloro-3-fluorophenyl)-3,6-dihydropyridin-1 (2H)-yl)methyl)-1-thioxoisoindolin-2-yl)piperidine-2,6-dione | 3-(4-((4-(2-chloro-3-fluorophenyl)-3,6-dihydropyridin-1 (2H)-yl)methyl)-1-thioxoisoindolin-2-yl)piperidine-2,6-dione |
| GT-08691 | 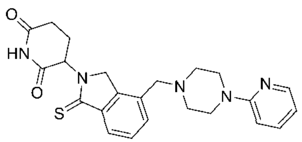 | 3-(4-((4-(pyridin-2-yl)piperazin-1-yl)methyl)-1-thioxoisoindolin-2-yl)piperidine-2,6-dione | 3-(4-((4-(pyridin-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0869 2 | 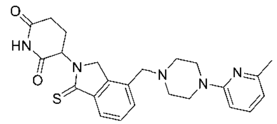 | 3-(4-((4-(6-methylpyridin-2-yl)piperazin-1-yl)methyl)-1-thioxoisoindolin-2-yl)piperidine-2,6-dione | 3-(4-((4-(6-methylpyridin-2-yl)piperazin-1-yl)methyl)-1-thioxoisoindolin-2-yl)piperidine-2,6-dione |
| GT-0869 3 | 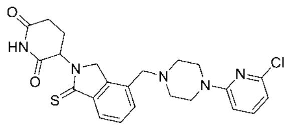 | 3-(4-((4-(6-chloropyridin-2-yl)piperazin-1-yl)methyl)-1-thioxoisoindolin-2-yl)piperidine-2,6-dione | 3-(4-((4-(6-chloropyridin-2-yl)piperazin-1-yl)methyl)-1-thioxoisoindolin-2-yl)piperidine-2,6-dione |
| GT-0869 4 | 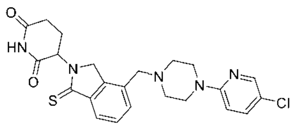 | 3-(4-((4-(5-chloropyridin-2-yl)piperazin-1-yl)methyl)-1-thioxoisoindolin-2-yl)piperidine-2,6-dione | 3-(4-((4-(5-chloropyridin-2-yl)piperazin-1-yl)methyl)-1-thioxoisoindolin-2-yl)piperidine-2,6-dione |
| GT-0869 5 | 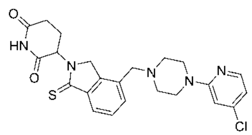 | 3-(4-((4-(4-chloropyridin-2-yl)piperazin-1-yl)methyl)-1-thioxoisoindolin-2-yl)piperidine-2,6-dione | 3-(4-((4-(4-chloropyridin-2-yl)piperazin-1-yl)methyl)-1-thioxoisoindolin-2-yl)piperidine-2,6-dione |
| GT-0869 6 | 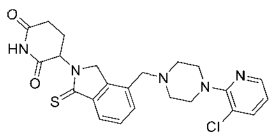 | 3-(4-((4-(3-chloropyridin-2-yl)piperazin-1-yl)methyl)-1-thioxoisoindolin-2-yl)piperidine-2,6-dione | 3-(4-((4-(3-chloropyridin-2-yl)piperazin-1-yl)methyl)-1-thioxoisoindolin-2-yl)piperidine-2,6-dione |
| GT-0869 7 | 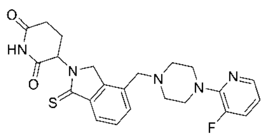 | 3-(4-((4-(3-fluoropyridin-2-yl)piperazin-1-yl)methyl)-1-thioxoisoindolin-2-yl)piperidine-2,6-dione | 3-(4-((4-(3-fluoropyridin-2-yl)piperazin-1-yl)methyl)-1-thioxoisoindolin-2-yl)piperidine-2,6-dione |
| GT-0869 8 | 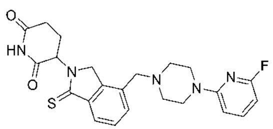 | 3-(4-((4-(6-fluoropyridin-2-yl)piperazin-1-yl)methyl)-1-thioxoisoindolin-2-yl)piperidine-2,6-dione | 3-(4-((4-(6-fluoropyridin-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08699 | 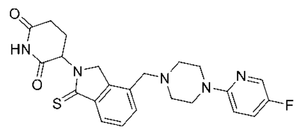 | 3-(4-((4-(5-fluoropyridin-2-yl)piperazin-1-yl)methyl)-1-thioxoisoindolin-2-yl)piperidine-2,6-dione | 3-(4-((4-(5-fluoropyridin-2-yl)piperazin-1-yl)methyl)-1-thioxoisoindolin-2-yl)piperidine-2,6-dione |
| GT-08700 | 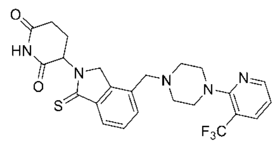 | 3-(1-thioxo-4-((4-(3-(trifluoromethyl)pyridin-2-yl)piperazin-1-yl)methyl)isoindolin-2-yl)piperidine-2,6-dione | 3-(1-thioxo-4-((4-(3-(trifluoromethyl)pyridin-2-yl)piperazin-1-yl)methyl)isoindolin-2-yl)piperidine-2,6-dione |
| GT-08701 | 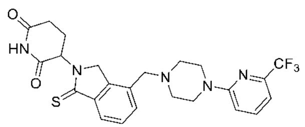 | 3-(1-thioxo-4-((4-(6-(trifluoromethyl)pyridin-2-yl)piperazin-1-yl)methyl)isoindolin-2-yl)piperidine-2,6-dione | 3-(1-thioxo-4-((4-(6-(trifluoromethyl)pyridin-2-yl)piperazin-1-yl)methyl)isoindolin-2-yl)piperidine-2,6-dione |
| GT-08702 | 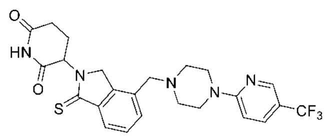 | 3-(1-thioxo-4-((4-(5-(trifluoromethyl)pyridin-2-yl)piperazin-1-yl)methyl)isoindolin-2-yl)piperidine-2,6-dione | 3-(1-thioxo-4-((4-(5-(trifluoromethyl)pyridin-2-yl)piperazin-1-yl)methyl)isoindolin-2-yl)piperidine-2,6-dione |
| | 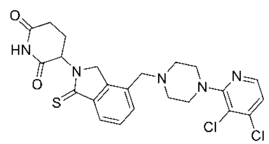 | 3-(4-((4-(3,4-dichloropyridin-2-yl)piperazin-1-yl)methyl)-1-thioxoisoindolin-2-yl)piperidine-2,6-dione | 3-(4-((4-(3,4-dichloropyridin-2-yl)piperazin-1-yl)methyl)-1-thioxoisoindolin-2-yl)piperidine-2,6-dione |
| GT-08703 | 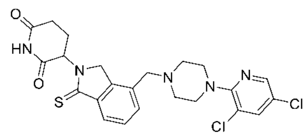 | 3-(4-((4-(3,5-dichloropyridin-2-yl)piperazin-1-yl)methyl)-1-thioxoisoindolin-2-yl)piperidine-2,6-dione | 3-(4-((4-(3,5-dichloropyridin-2-yl)piperazin-1-yl)methyl)-1-thioxoisoindolin-2-yl)piperidine-2,6-dione |
| | 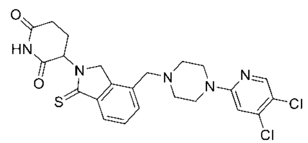 | 3-(4-((4-(4,5-dichloropyridin-2-yl)piperazin-1-yl)methyl)-1-thioxoisoindolin-2-yl)piperidine-2,6-dione | 3-(4-((4-(4,5-dichloropyridin-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08728 | 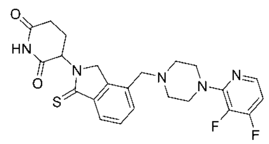 | 3-(4-((4-(3,4-difluoropyridin-2-yl)piperazin-1-yl) methyl)-1-thioxoisoindolin-2-yl)piperidine-2,6-dione | 3-(4-((4-(3,4-difluoropyridin-2-yl)piperazin-1-yl) methyl)-1-thioxoisoindolin-2-yl)piperidine-2,6-dione |
| GT-08803 | 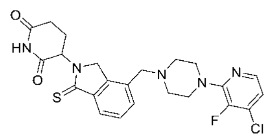 | 3-(4-((4-(4-chloro-3-fluoropyridin-2-yl)piperazin-1-yl) methyl)-1-thioxoisoindolin-2-yl)piperidine-2,6-dione | 3-(4-((4-(4-chloro-3-fluoropyridin-2-yl)piperazin-1-yl) methyl)-1-thioxoisoindolin-2-yl)piperidine-2,6-dione |
| GT-08704 | 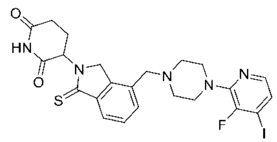 | 3-(4-((4-(3-fluoro-4-iodopyridin-2-yl)piperazin-1-yl) methyl)-1-thioxoisoindolin-2-yl)piperidine-2,6-dione | 3-(4-((4-(3-fluoro-4-iodopyridin-2-yl)piperazin-1-yl) methyl)-1-thioxoisoindolin-2-yl)piperidine-2,6-dione |
| GT-08804 | 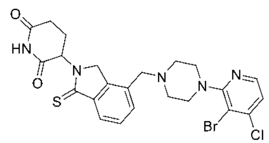 | 3-(4-((4-(3-bromo-4-chloropyridin-2-yl)piperazin-1-yl) methyl)-1-thioxoisoindolin-2-yl)piperidine-2,6-dione | 3-(4-((4-(3-bromo-4-chloropyridin-2-yl)piperazin-1-yl) methyl)-1-thioxoisoindolin-2-yl)piperidine-2,6-dione |
| GT-0870 5 | 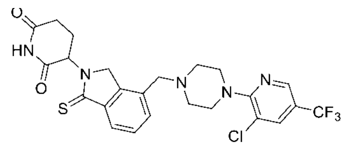 | 3-(4-((4-(3-chloro-5-(trifluoromethyl)pyridin-2-yl)piperazin-1-yl)methyl)-1-thioxoisoindolin-2-yl)piperidine-2,6-dione | 3-(4-((4-(3-chloro-5-(trifluoromethyl)pyridin-2-yl)piperazin-1-yl)methyl)-1-thioxoisoindolin-2-yl)piperidine-2,6-dione |
| | 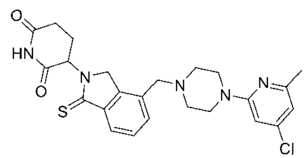 | 3-(4-((4-(4-chloro-6-methylpyridin-2-yl)piperazin-1-yl) methyl)-1-thioxoisoindolin-2-yl)piperidine-2,6-dione | 3-(4-((4-(4-chloro-6-methylpyridin-2-yl)piperazin-1-yl) methyl)-1-thioxoisoindolin-2-yl)piperidine-2,6-dione |
| GT-0870 6 | 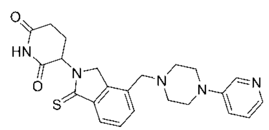 | 3-(4-((4-(pyridin-3-yl)piperazin-1-yl)methyl)-1-thioxoisoindolin-2-yl)piperidine-2,6-dione | 3-(4-((4-(pyridin-3-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0872 3 | 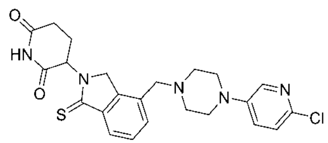 | 3-(4-((4-(6-chloropyridin-3-yl)piperazin-1-yl)methyl)-1-thioxoisoindolin-2-yl)piperidine-2,6-dione | 3-(4-((4-(6-chloropyridin-3-yl)piperazin-1-yl)methyl)-1-thioxoisoindolin-2-yl)piperidine-2,6-dione |
| GT-0872 4 | 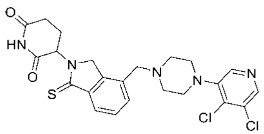 | 3-(4-((4-(4,5-dichloropyridin-3-yl)piperazin-1-yl) methyl)-1-thioxoisoindolin-2-yl)piperidine-2,6-dione | 3-(4-((4-(4,5-dichloropyridin-3-yl)piperazin-1-yl) methyl)-1-thioxoisoindolin-2-yl)piperidine-2,6-dione |
| GT-0872 5 | 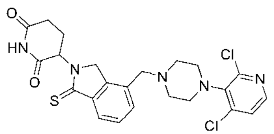 | 3-(4-((4-(2,4-dichloropyridin-3-yl)piperazin-1-yl) methyl)-1-thioxoisoindolin-2-yl)piperidine-2,6-dione | 3-(4-((4-(2,4-dichloropyridin-3-yl)piperazin-1-yl) methyl)-1-thioxoisoindolin-2-yl)piperidine-2,6-dione |
| GT-0872 6 | 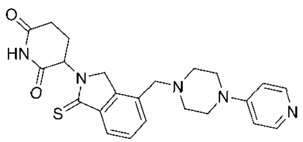 | 3-(4-((4-(pyridin-4-yl)piperazin-1-yl)methyl)-1-thioxoisoindolin-2-yl)piperidine-2,6-dione | 3-(4-((4-(pyridin-4-yl)piperazin-1-yl)methyl)-1-thioxoisoindolin-2-yl)piperidine-2,6-dione |
| GT-0872 7 | 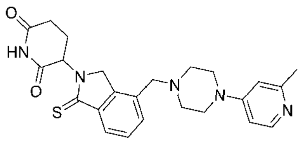 | 3-(4-((4-(2-methylpyridin-4-yl)piperazin-1-yl)methyl)-1-thioxoisoindolin-2-yl)piperidine-2,6-dione | 3-(4-((4-(2-methylpyridin-4-yl)piperazin-1-yl)methyl)-1-thioxoisoindolin-2-yl)piperidine-2,6-dione |
| GT-0872 9 | 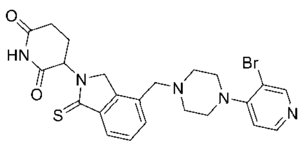 | 3-(4-((4-(3-bromopyridin-4-yl)piperazin-1-yl)methyl)-1-thioxoisoindolin-2-yl)piperidine-2,6-dione | 3-(4-((4-(3-bromopyridin-4-yl)piperazin-1-yl)methyl)-1-thioxoisoindolin-2-yl)piperidine-2,6-dione |
| GT-0873 0 | 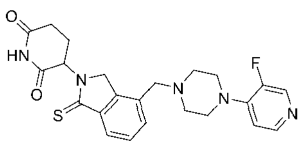 | 3-(4-((4-(3-fluoropyridin-4-yl)piperazin-1-yl)methyl)-1-thioxoisoindolin-2-yl)piperidine-2,6-dione | 3-(4-((4-(3-fluoropyridin-4-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0873 1 | 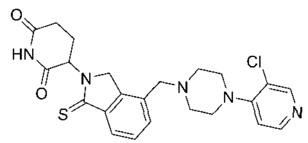 | 3-(4-((4-(3-chloropyridin-4-yl)piperazin-1-yl)methyl)-1-thioxoisoindolin-2-yl)piperidine-2,6-dione | 3-(4-((4-(3-chloropyridin-4-yl)piperazin-1-yl)methyl)-1-thioxoisoindolin-2-yl)piperidine-2,6-dione |
| | 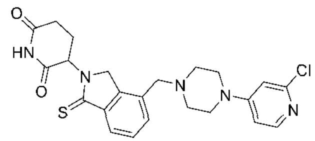 | 3-(4-((4-(2-chloropyridin-4-yl)piperazin-1-yl)methyl)-1-thioxoisoindolin-2-yl)piperidine-2,6-dione | 3-(4-((4-(2-chloropyridin-4-yl)piperazin-1-yl)methyl)-1-thioxoisoindolin-2-yl)piperidine-2,6-dione |
| GT-0873 2 | 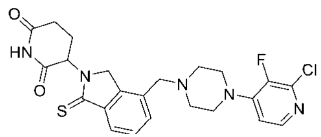 | 3-(4-((4-(2-chloro-3-fluoropyridin-4-yl)piperazin-1-yl) methyl)-1-thioxoisoindolin-2-yl)piperidine-2,6-dione | 3-(4-((4-(2-chloro-3-fluoropyridin-4-yl)piperazin-1-yl) methyl)-1-thioxoisoindolin-2-yl)piperidine-2,6-dione |
| GT-08733 | 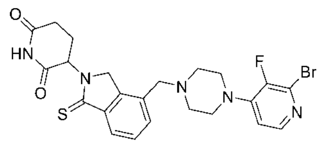 | 3-(4-((4-(2-bromo-3-fluoropyridin-4-yl)piperazin-1-yl) methyl)-1-thioxoisoindolin-2-yl)piperidine-2,6-dione | 3-(4-((4-(2-bromo-3-fluoropyridin-4-yl)piperazin-1-yl) methyl)-1-thioxoisoindolin-2-yl)piperidine-2,6-dione |
| GT-08734 | 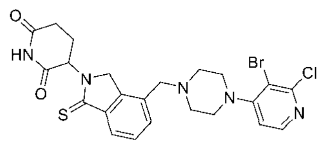 | 3-(4-((4-(3-bromo-2-chloropyridin-4-yl)piperazin-1-yl) methyl)-1-thioxoisoindolin-2-yl)piperidine-2,6-dione | 3-(4-((4-(3-bromo-2-chloropyridin-4-yl)piperazin-1-yl) methyl)-1-thioxoisoindolin-2-yl)piperidine-2,6-dione |
| GT-08735 | 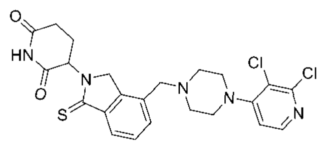 | 3-(4-((4-(2,3-dichloropyridin-4-yl)piperazin-1-yl) methyl)-1-thioxoisoindolin-2-yl)piperidine-2,6-dione | 3-(4-((4-(2,3-dichloropyridin-4-yl)piperazin-1-yl) methyl)-1-thioxoisoindolin-2-yl)piperidine-2,6-dione |
| GT-08736 | 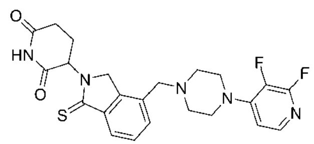 | 3-(4-((4-(2,3-difluoropyridin-4-yl)piperazin-1-yl) methyl)-1-thioxoisoindolin-2-yl)piperidine-2,6-dione | 3-(4-((4-(2,3-difluoropyridin-4-yl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 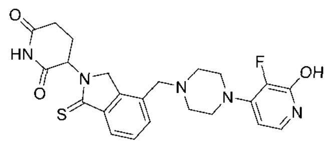 | 3-(4-((4-(3-fluoro-2-hydroxypyridin-4-yl)piperazin-1-yl) methyl)-1-thioxoisoindolin-2-yl)piperidine-2,6-dione | 3-(4-((4-(3-fluoro-2-hydroxypyridin-4-yl)piperazin-1-yl) methyl)-1-thioxoisoindolin-2-yl)piperidine-2,6-dione |
| GT-08801 | 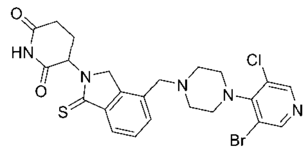 | 3-(4-((4-(3-bromo-5-chloropyridin-4-yl)piperazin-1-yl) methyl)-1-thioxoisoindolin-2-yl)piperidine-2,6-dione | 3-(4-((4-(3-bromo-5-chloropyridin-4-yl)piperazin-1-yl) methyl)-1-thioxoisoindolin-2-yl)piperidine-2,6-dione |
| GT-08802 | 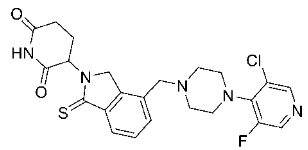 | 3-(4-((4-(3-chloro-5-fluoropyridin-4-yl)piperazin-1-yl) methyl)-1-thioxoisoindolin-2-yl)piperidine-2,6-dione | 3-(4-((4-(3-chloro-5-fluoropyridin-4-yl)piperazin-1-yl) methyl)-1-thioxoisoindolin-2-yl)piperidine-2,6-dione |
| GT-08805 | 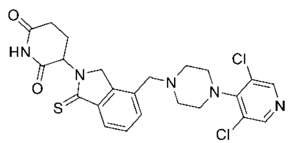 | 3-(4-((4-(3,5-dichloropyridin-4-yl)piperazin-1-yl) methyl)-1-thioxoisoindolin-2-yl)piperidine-2,6-dione | 3-(4-((4-(3,5-dichloropyridin-4-yl)piperazin-1-yl) methyl)-1-thioxoisoindolin-2-yl)piperidine-2,6-dione |
| GT-08806 | 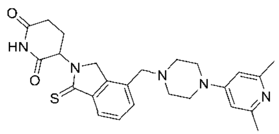 | 3-(4-((4-(2,6-dimethylpyridin-4-yl)piperazin-1-yl) methyl)-1-thioxoisoindolin-2-yl)piperidine-2,6-dione | 3-(4-((4-(2,6-dimethylpyridin-4-yl)piperazin-1-yl) methyl)-1-thioxoisoindolin-2-yl)piperidine-2,6-dione |
| GT-08809 | 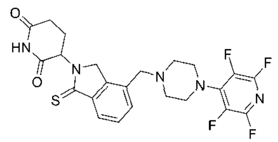 | 3-(4-((4-(perfluoropyridin-4-yl)piperazin-1-yl)methyl)-1-thioxoisoindolin-2-yl)piperidine-2,6-dione | 3-(4-((4-(perfluoropyridin-4-yl)piperazin-1-yl)methyl)-1-thioxoisoindolin-2-yl)piperidine-2,6-dione |
| GT-08810 | 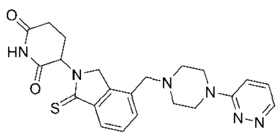 | 3-(4-((4-(pyridazin-3-yl)piperazin-1-yl)methyl)-1-thioxoisoindolin-2-yl)piperidine-2,6-dione | 3-(4-((4-(pyridazin-3-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08811 | 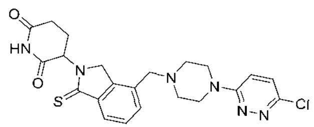 | 3-(4-((4-(6-chloropyridazin-3-yl)piperazin-1-yl) methyl)-1-thioxoisoindolin-2-yl)piperidine-2,6-dione | 3-(4-((4-(6-chloropyridazin-3-yl)piperazin-1-yl) methyl)-1-thioxoisoindolin-2-yl)piperidine-2,6-dione |
| GT-08812 | 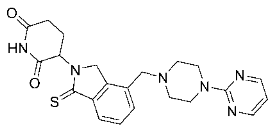 | 3-(4-((4-(pyrimidin-2-yl)piperazin-1-yl)methyl)-1-thioxoisoindolin-2-yl)piperidine-2,6-dione | 3-(4-((4-(pyrimidin-2-yl)piperazin-1-yl)methyl)-1-thioxoisoindolin-2-yl)piperidine-2,6-dione |
| GT-08813 | 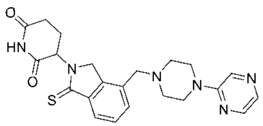 | 3-(4-((4-(pyrazin-2-yl)piperazin-1-yl)methyl)-1-thioxoisoindolin-2-yl)piperidine-2,6-dione | 3-(4-((4-(pyrazin-2-yl)piperazin-1-yl)methyl)-1-thioxoisoindolin-2-yl)piperidine-2,6-dione |
| GT-08814 | 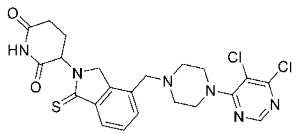 | 3-(4-((4-(5,6-dichloropyrimidin-4-yl)piperazin-1-yl) methyl)-1-thioxoisoindolin-2-yl)piperidine-2,6-dione | 3-(4-((4-(5,6-dichloropyrimidin-4-yl)piperazin-1-yl) methyl)-1-thioxoisoindolin-2-yl)piperidine-2,6-dione |
| GT-08807 | 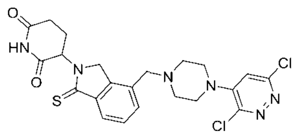 | 3-(4-((4-(3,6-dichloropyridazin-4-yl)piperazin-1-yl) methyl)-1-thioxoisoindolin-2-yl)piperidine-2,6-dione | 3-(4-((4-(3,6-dichloropyridazin-4-yl)piperazin-1-yl) methyl)-1-thioxoisoindolin-2-yl)piperidine-2,6-dione |
| GT-08815 | 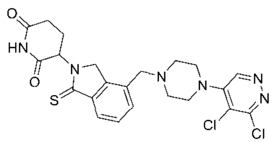 | 3-(4-((4-(5,6-dichloropyridazin-4-yl)piperazin-1-yl) methyl)-1-thioxoisoindolin-2-yl)piperidine-2,6-dione | 3-(4-((4-(5,6-dichloropyridazin-4-yl)piperazin-1-yl) methyl)-1-thioxoisoindolin-2-yl)piperidine-2,6-dione |
| GT-08808 | 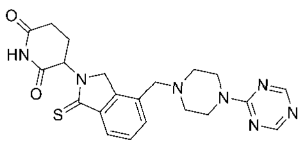 | 3-(4-((4-(1,3,5-triazin-2-yl)piperazin-1-yl)methyl)-1-thioxoisoindolin-2-yl)piperidine-2,6-dione | 3-(4-((4-(1,3,5-triazin-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08457 | 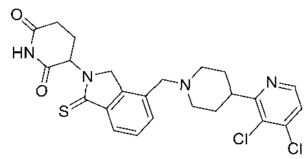 | 3-(4-((4-(3,4-dichloropyridin-2-yl)piperidin-1-yl) methyl)-1-thioxoisoindolin-2-yl)piperidine-2,6-dione | 3-(4-((4-(3,4-dichloropyridin-2-yl)piperidin-1-yl) methyl)-1-thioxoisoindolin-2-yl)piperidine-2,6-dione |
| GT-08458 | 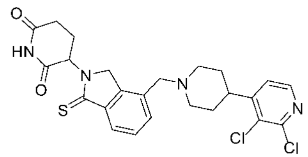 | 3-(4-((4-(2,3-dichloropyridin-4-yl)piperidin-1-yl) methyl)-1-thioxoisoindolin-2-yl)piperidine-2,6-dione | 3-(4-((4-(2,3-dichloropyridin-4-yl)piperidin-1-yl) methyl)-1-thioxoisoindolin-2-yl)piperidine-2,6-dione |
| GT-08459 | 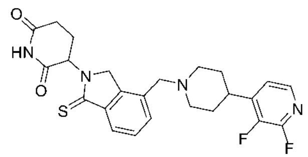 | 3-(4-((4-(2,3-difluoropyridin-4-yl)piperidin-1-yl) methyl)-1-thioxoisoindolin-2-yl)piperidine-2,6-dione | 3-(4-((4-(2,3-difluoropyridin-4-yl)piperidin-1-yl) methyl)-1-thioxoisoindolin-2-yl)piperidine-2,6-dione |
| | 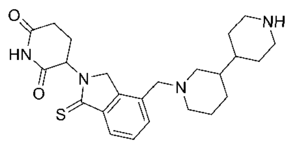 | 3-(4-([3,4'-bipiperidin]-1-ylmethyl)-1-thioxoisoindolin-2-yl)piperidine-2,6-dione | 3-(4-([3,4'-bipiperidin]-1-ylmethyl)-1-thioxoisoindolin-2-yl)piperidine-2,6-dione |
| | 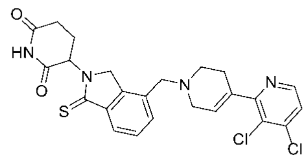 | 3-(4-((3,4-dichloro-3',6'-dihydro-[2,4'-bipyridin]-1'(2'H)-yl)methyl)-1-thioxoisoindolin-2-yl)piperidine-2,6-dione | 3-(4-((3,4-dichloro-3',6'-dihydro-[2,4'-bipyridin]-1'(2'H)-yl)methyl)-1-thioxoisoindolin-2-yl)piperidine-2,6-dione |
| GT-08460 | 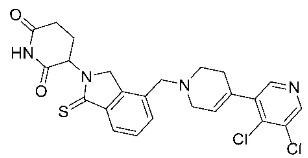 | 3-(4-((4,5-dichloro-3',6'-dihydro-[3,4'-bipyridin]-1'(2'H)-yl)methyl)-1-thioxoisoindolin-2-yl)piperidine-2,6-dione | 3-(4-((4,5-dichloro-3',6'-dihydro-[3,4'-bipyridin]-1'(2'H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08461 | 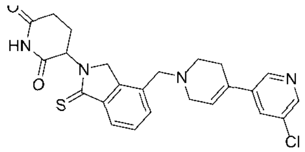 | 3-(4-((5-chloro-3',6'-dihydro-[3,4'-bipyridin]-1'(2'H)-yl) methyl)-1-thioxoisoindolin-2-yl)piperidine-2,6-dione | 3-(4-((5-chloro-3',6'-dihydro-[3,4'-bipyridin]-1'(2'H)-yl) methyl)-1-thioxoisoindolin-2-yl)piperidine-2,6-dione |
| GT-08462 | 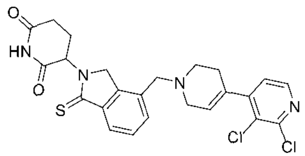 | 3-(4-((2',3'-dichloro-3,6-dihydro-[4,4'-bipyridin]-1(2H)-yl)methyl)-1-thioxoisoindolin-2-yl)piperidine-2,6-dione | 3-(4-((2',3'-dichloro-3,6-dihydro-[4,4'-bipyridin]-1(2H)-yl)methyl)-1-thioxoisoindolin-2-yl)piperidine-2,6-dione |
| GT-08463 | 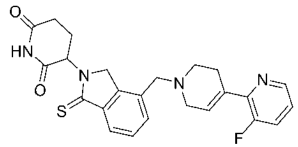 | 3-(4-((3-fluoro-3',6'-dihydro-[2,4'-bipyridin]-1'(2'H)-yl) methyl)-1-thioxoisoindolin-2-yl)piperidine-2,6-dione | 3-(4-((3-fluoro-3',6'-dihydro-[2,4'-bipyridin]-1'(2'H)-yl) methyl)-1-thioxoisoindolin-2-yl)piperidine-2,6-dione |
| GT-08464 | 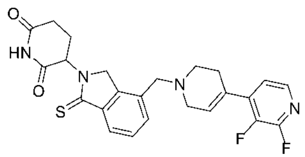 | 3-(4-((2',3'-difluoro-3,6-dihydro-[4,4'-bipyridin]-1(2H)-yl)methyl)-1-thioxoisoindolin-2-yl)piperidine-2,6-dione | 3-(4-((2',3'-difluoro-3,6-dihydro-[4,4'-bipyridin]-1(2H)-yl)methyl)-1-thioxoisoindolin-2-yl)piperidine-2,6-dione |
| GT-08465 | 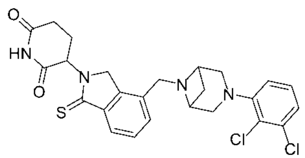 | 3-(4-((3-(2,3-dichlorophenyl)-3,6-diazabicyclo[3.1.1] heptan-6-yl)methyl)-1-thioxoisoindolin-2-yl)piperidine-2,6-dione | 3-(4-((3-(2,3-dichlorophenyl)-3,6-diazabicyclo[3.1.1] heptan-6-yl)methyl)-1-thioxoisoindolin-2-yl)piperidine-2,6-dione |
| GT-08466 | 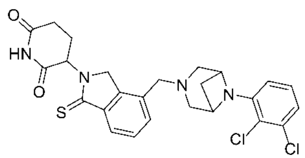 | 3-(4-((6-(2,3-dichlorophenyl)-3,6-diazabicyclo[3.1.1] heptan-3-yl)methyl)-1-thioxoisoindolin-2-yl)piperidine-2,6-dione | 3-(4-((6-(2,3-dichlorophenyl)-3,6-diazabicyclo[3.1.1] heptan-3-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08467 | 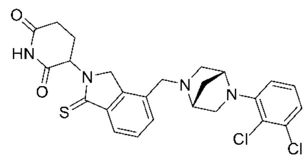 | 3-(4-(((1R,4R)-5-(2,3-dichlorophenyl)-2,5-diazabicyclo [2.2.1]heptan-2-yl)methyl)-1-thioxoisoindolin-2-yl)piperidine-2,6-dione | 3-(4-(((1R,4R)-5-(2,3-dichlorophenyl)-2,5-diazabicyclo [2.2.1]heptan-2-yl)methyl)-1-thioxoisoindolin-2-yl)piperidine-2,6-dione |
| GT-08468 | 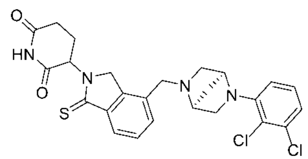 | 3- 4-(((1S,4S)-5-(2,3-dichlorophenyl)-2,5-diazabicyclo [2.2.1]heptan-2-yl)methyl)-1-thioxoisoindolin-2-yl)piperidine-2,6-dione | 3-(4-(((1 S,4S)-5-(2,3-dichlorophenyl)-2,5-diazabicyclo [2.2.1]heptan-2-yl)methyl)-1-thioxoisoindolin-2-yl)piperidine-2,6-dione |
| GT-08469 | 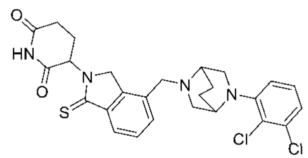 | 3-(4-((5-(2,3-dichlorophenyl)-2,5-diazabicyclo[2.2.2]octan-2-yl)methyl)-1-thioxoisoindolin-2-yl)piperidine-2,6-dione | 3-(4-((5-(2,3-dichlorophenyl)-2,5-diazabicyclo[2.2.2]octan-2-yl)methyl)-1-thioxoisoindolin-2-yl)piperidine-2,6-dione |
| GT-08470 | 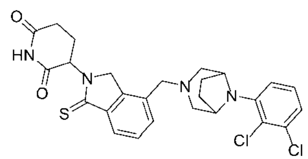 | 3-(4-((8-(2,3-dichlorophenyl)-3,8-diazabicyclo[3.2.1]octan-3-yl)methyl)-1-thioxoisoindolin-2-yl)piperidine-2,6-dione | 3-(4-((8-(2,3-dichlorophenyl)-3,8-diazabicyclo[3.2.1]octan-3-yl)methyl)-1-thioxoisoindolin-2-yl)piperidine-2,6-dione |
| GT-08471 | 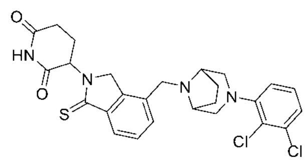 | 3-(4-((3-(2,3-dichlorophenyl)-3,8-diazabicyclo[3.2.1]octan-8-yl)methyl)-1-thioxoisoindolin-2-yl)piperidine-2,6-dione | 3-(4-((3-(2,3-dichlorophenyl)-3,8-diazabicyclo[3.2.1]octan-8-yl)methyl)-1-thioxoisoindolin-2-yl)piperidine-2,6-dione |
| GT-08472 | 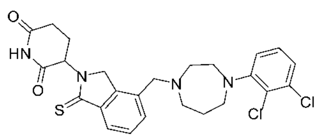 | 3-(4-((4-(2,3-dichlorophenyl)-1,4-diazepan-1-yl) methyl)-1-thioxoisoindolin-2-yl)piperidine-2,6-dione | 3-(4-((4-(2,3-dichlorophenyl)-1,4-diazepan-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-04997 | 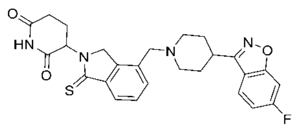 | 3-(4-((4-(6-fluorobenzo[d]isoxazol-3-yl)piperidin-1-yl) methyl)-1-thioxoisoindolin-2-yl)piperidine-2,6-dione | 3-(4-((4-(6-fluorobenzo[d]isoxazol-3-yl)piperidin-1-yl) methyl)-1-thioxoisoindolin-2-yl)piperidine-2,6-dione |
| GT-08816 | 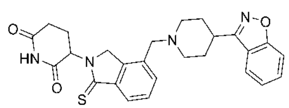 | 3-(4-((4-(benzo[d]isoxazol-3-yl)piperidin-1-yl) methyl)-1-thioxoisoindolin-2-yl)piperidine-2,6-dione | 3-(4-((4-(benzo[d]isoxazol-3-yl)piperidin-1-yl) methyl)-1-thioxoisoindolin-2-yl)piperidine-2,6-dione |
| GT-08817 | 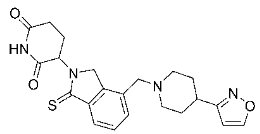 | 3-(4-((4-(isoxazol-3-yl)piperidin-1-yl)methyl)-1-thioxoisoindolin-2-yl)piperidine-2,6-dione | 3-(4-((4-(isoxazol-3-yl)piperidin-1-yl)methyl)-1-thioxoisoindolin-2-yl)piperidine-2,6-dione |
| GT-08818 | 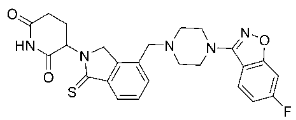 | 3-(4-((4-(6-fluorobenzo[d]isoxazol-3-yl)piperazin-1-yl) methyl)-1-thioxoisoindolin-2-yl)piperidine-2,6-dione | 3-(4-((4-(6-fluorobenzo[d]isoxazol-3-yl)piperazin-1-yl) methyl)-1-thioxoisoindolin-2-yl)piperidine-2,6-dione |
| GT-08838 | 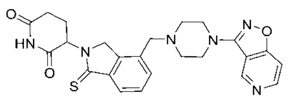 | 3-(4-((4-(isoxazolo[4,5-c]pyridin-3-yl)piperazin-1-yl) methyl)-1-thioxoisoindolin-2-yl)piperidine-2,6-dione | 3-(4-((4-(isoxazolo[4,5-c]pyridin-3-yl)piperazin-1-yl) methyl)-1-thioxoisoindolin-2-yl)piperidine-2,6-dione |
| GT-08839 | 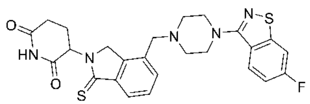 | 3-(4-((4-(6-fluorobenzo[d]isothiazol-3-yl)piperazin-1-yl) methyl)-1-thioxoisoindolin-2-yl)piperidine-2,6-dione | 3-(4-((4-(6-fluorobenzo[d]isothiazol-3-yl)piperazin-1-yl) methyl)-1-thioxoisoindolin-2-yl)piperidine-2,6-dione |
| GT-08840 | 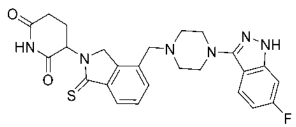 | 3-(4-((4-(6-fluoro-1H-indazol-3-yl)piperazin-1-yl) methyl)-1-thioxoisoindolin-2-yl)piperidine-2,6-dione | 3-(4-((4-(6-fluoro-1H-indazol-3-yl)piperazin-1-yl) methyl)-1-thioxoisoindolin-2-yl)piperidine-2,6-dione |
| GT-0884 1 | 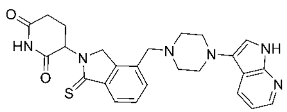 | 3-(4-((4-(1H-pyrrolo[2,3-b]pyridin-3-yl)piperazin-1-yl) methyl)-1-thioxoisoindolin-2-yl)piperidine-2,6-dione | 3-(4-((4-(1H-pyrrolo[2,3-b]pyridin-3-yl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0849 2 | 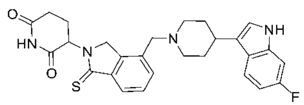 | 3-(4-((4-(6-fluoro-1H-indol-3-yl)piperidin-1-yl) methyl)-1-thioxoisoindolin-2-yl)piperidine-2,6-dione | 3-(4-((4-(6-fluoro-1H-indol-3-yl)piperidin-1-yl) methyl)-1-thioxoisoindolin-2-yl)piperidine-2,6-dione |
| GT-08844 | 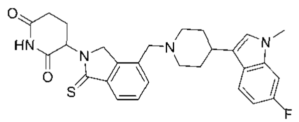 | 3-(4-((4-(6-fluoro-1-methyl-1H-indol-3-yl)piperidin-1-yl) methyl)-1-thioxoisoindolin-2-yl)piperidine-2,6-dione | 3-(4-((4-(6-fluoro-1-methyl-1H-indol-3-yl)piperidin-1-yl) methyl)-1-thioxoisoindolin-2-yl)piperidine-2,6-dione |
| GT-08849 | 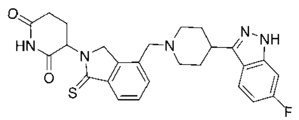 | 3-(4-((4-(6-fluoro-1H-indazol-3-yl)piperidin-1-yl) methyl)-1-thioxoisoindolin-2-yl)piperidine-2,6-dione | 3-(4-((4-(6-fluoro-1H-indazol-3-yl)piperidin-1-yl) methyl)-1-thioxoisoindolin-2-yl)piperidine-2,6-dione |
| GT-08845 | 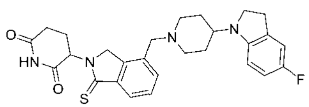 | 3-(4-((4-(5-fluoroindolin-1-yl)piperidin-1-yl)methyl)-1-thioxoisoindolin-2-yl)piperidine-2,6-dione | 3-(4-((4-(5-fluoroindolin-1-yl)piperidin-1-yl)methyl)-1-thioxoisoindolin-2-yl)piperidine-2,6-dione |
| GT-08842 | 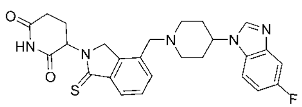 | 3-(4-((4-(5-fluoro-1H-benzo[d]imidazol-1-yl)piperidin-1-yl)methyl)-1-thioxoisoindolin-2-yl)piperidine-2,6-dione | 3-(4-((4-(5-fluoro-1H-benzo[d]imidazol-1-yl)piperidin-1-yl)methyl)-1-thioxoisoindolin-2-yl)piperidine-2,6-dione |
| GT-08843 | 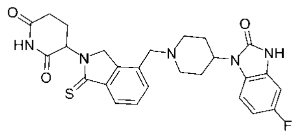 | 3-(4-((4-(5-fluoro-2-oxo-2,3-dihydro-1H-benzo[d]imidazol-1-yl)piperidin-1-yl)methyl)-1-thioxoisoindolin-2-yl) piperidine-2,6-dione | 3-(4-((4-(5-fluoro-2-oxo-2,3-dihydro-1H-benzo[d]imidazol-1-yl)piperidin-1-yl)methyl)-1-thioxoisoindolin-2-yl) piperidine-2,6-dione |
| GT-08848 | 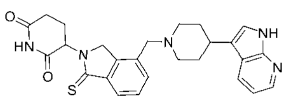 | 3-(4-((4-(1H-pyrrolo[2,3-b]pyridin-3-yl)piperidin-1-yl) methyl)-1-thioxoisoindolin-2-yl)piperidine-2,6-dione | 3-(4-((4-(1H-pyrrolo[2,3-b]pyridin-3-yl)piperidin-1-yl) methyl)-1-thioxoisoindolin-2-yl)piperidine-2,6-dione |
| GT-08494 | 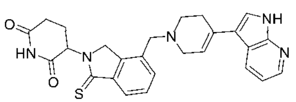 | 3-(4-((4-(1H-pyrrolo[2,3-b]pyridin-3-yl)-3,6-dihydropyridin-1 (2H)-yl)methyl)-1-thioxoisoindolin-2-yl)piperidine-2,6-dione | 3-(4-((4-(1H-pyrrolo[2,3-b]pyridin-3-yl)-3,6-dihydropyridin-1(2H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 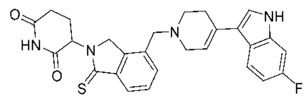 | 3-(4-((4-(6-fluoro-1H-indol-3-yl)-3,6-dihydropyridin-1(2H)-yl)methyl)-1-thioxoisoindolin-2-yl)piperidine-2,6-dione | 3-(4-((4-(6-fluoro-1H-indol-3-yl)-3,6-dihydropyridin-1(2H)-yl)methyl)-1-thioxoisoindolin-2-yl)piperidine-2,6-dione |
| GT-08493 | 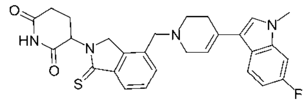 | 3-(4-((4-(6-fluoro-1-methyl-1H-indol-3-yl)-3,6-dihydropyridin-1 (2H)-yl)methyl)-1-thioxoisoindolin-2-yl)piperidine-2,6-dione | 3-(4-((4-(6-fluoro-1-methyl-1H-indol-3-yl)-3,6-dihydropyridin-1(2H)-yl)methyl)-1-thioxoisoindolin-2-yl)piperidine-2,6-dione |
| GT-08850 | 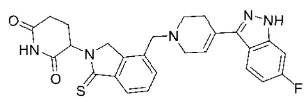 | 3-(4-((4-(6-fluoro-1H-indazol-3-yl)-3,6-dihydropyridin-1 (2H)-yl)methyl)-1-thioxoisoindolin-2-yl)piperidine-2,6-dione | 3-(4-((4-(6-fluoro-1H-indazol-3-yl)-3,6-dihydropyridin-1 (2H)-yl)methyl)-1-thioxoisoindolin-2-yl)piperidine-2,6-dione |
| GT-08851 | 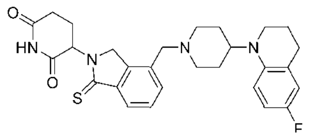 | 3-(4-((4-(6-fluoro-3,4-dihydroquinolin-1(2H)-yl)piperidin-1-yl)methyl)-1-thioxoisoindolin-2-yl)piperidine-2,6-dione | 3-(4-((4-(6-fluoro-3,4-dihydroquinolin-1 (2H)-yl)piperidin-1-yl)methyl)-1-thioxoisoindolin-2-yl)piperidine-2,6-dione |
| GT-0885 2 | 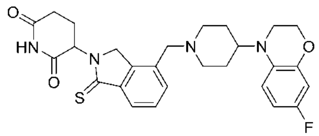 | 3-(4-((4-(7-fluoro-2,3-dihydro-4H-benzo[b][1,4]oxazin-4-yl)piperidin-1-yl)methyl)-1-thioxoisoindolin-2-yl) piperidine-2,6-dione | 3-(4-((4-(7-fluoro-2,3-dihydro-4H-benzo[b][1,4]oxazin-4-yl)piperidin-1-yl)methyl)-1-thioxoisoindolin-2-yl) piperidine-2,6-dione |
| GT-0885 3 | 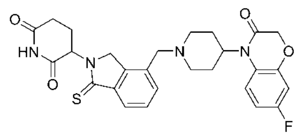 | 3-(4-((4-(7-fluoro-3-oxo-2,3-dihydro-4H-benzo[b][1,4] oxazin-4-yl)piperidin-1-yl)methyl)-1-thioxoisoindolin-2-yl)piperidine-2,6-dione | 3-(4-((4-(7-fluoro-3-oxo-2,3-dihydro-4H-benzo[b][1,4] oxazin-4-yl)piperidin-1-yl)methyl)-1-thioxoisoindolin-2-yl)piperidine-2,6-dione |
| GT-0885 4 | 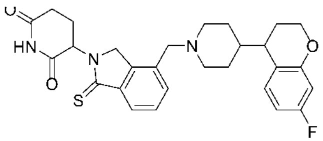 | 3-(4-((4-(7-fluorochroman-4-yl)piperidin-1-yl)methyl)-1-thioxoisoindolin-2-yl)piperidine-2,6-dione | 3-(4-((4-(7-fluorochroman-4-yl)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 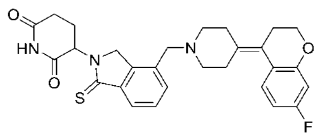 | 3-(4-((4-(7-fluorochroman-4-ylidene)piperidin-1-yl) methyl)-1-thioxoisoindolin-2-yl)piperidine-2,6-dione | 3-(4-((4-(7-fluorochroman-4-ylidene)piperidin-1-yl) methyl)-1-thioxoisoindolin-2-yl)piperidine-2,6-dione |
| GT-0885 5 | 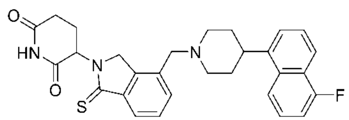 | 3-(4-((4-(5-fluoronaphthalen-1-yl)piperidin-1-yl) methyl)-1-thioxoisoindolin-2-yl)piperidine-2,6-dione | 3-(4-((4-(5-fluoronaphthalen-1-yl)piperidin-1-yl) methyl)-1-thioxoisoindolin-2-yl)piperidine-2,6-dione |
| | 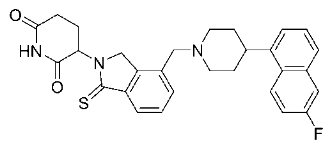 | 3-(4-((4-(5-fluoronaphthalen-1-yl)piperidin-1-yl) methyl)-1-thioxoisoindolin-2-yl)piperidine-2,6-dione | 3-(4-((4-(5-fluoronaphthalen-1-yl)piperidin-1-yl) methyl)-1-thioxoisoindolin-2-yl)piperidine-2,6-dione |
| GT-08858 | 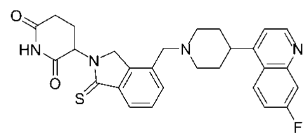 | 3-(4-((4-(7-fluoroquinolin-4-yl)piperidin-1-yl)methyl)-1-thioxoisoindolin-2-yl)piperidine-2,6-dione | 3-(4-((4-(7-fluoroquinolin-4-yl)piperidin-1-yl)methyl)-1-thioxoisoindolin-2-yl)piperidine-2,6-dione |
| GT-08857 | 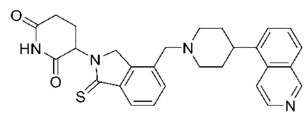 | 3-(4-((4-(isoquinolin-5-yl)piperidin-1-yl)methyl)-1-thioxoisoindolin-2-yl)piperidine-2,6-dione | 3-(4-((4-(isoquinolin-5-yl)piperidin-1-yl)methyl)-1-thioxoisoindolin-2-yl)piperidine-2,6-dione |
| GT-08856 | 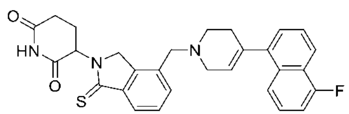 | 3-(4-((4-(5-fluoronaphthalen-1-yl)-3,6-dihydropyridin-1(2H)-yl)methyl)-1-thioxoisoindolin-2-yl)piperidine-2,6-dione | 3-(4-((4-(5-fluoronaphthalen-1-yl)-3,6-dihydropyridin-1(2H)-yl)methyl)-1-thioxoisoindolin-2-yl)piperidine-2,6-dione |
| GT-08633 | 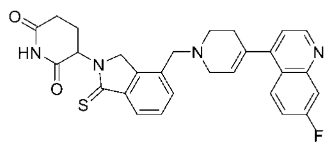 | 3-(4-((4-(7-fluoroquinolin-4-yl)-3,6-dihydropyridin-1(2H)-yl)methyl)-1-thioxoisoindolin-2-yl)piperidine-2,6-dione | 3-(4-((4-(7-fluoroquinolin-4-yl)-3,6-dihydropyridin-1(2H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08634 | 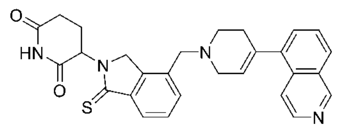 | 3-(4-((4-(isoquinolin-5-yl)-3,6-dihydropyridin-1 (2H)-yl) methyl)-1-thioxoisoindolin-2-yl)piperidine-2,6-dione | 3-(4-((4-(isoquinolin-5-yl)-3,6-dihydropyridin-1(2H)-yl) methyl)-1-thioxoisoindolin-2-yl)piperidine-2,6-dione |
| GT-08859 | 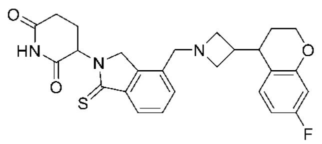 | 3-(4-((3-(7-fluorochroman-4-yl)azetidin-1-yl)methyl)-1-thioxoisoindolin-2-yl)piperidine-2,6-dione | 3-(4-((3-(7-fluorochroman-4-yl)azetidin-1-yl)methyl)-1-thioxoisoindolin-2-yl)piperidine-2,6-dione |
| | 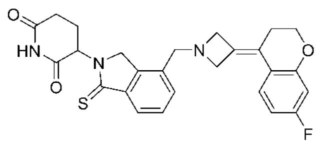 | 3-(4-((3-(7-fluorochroman-4-ylidene)azetidin-1-yl) methyl)-1-thioxoisoindolin-2-yl)piperidine-2,6-dione | 3-(4-((3-(7-fluorochroman-4-ylidene)azetidin-1-yl) methyl)-1-thioxoisoindolin-2-yl)piperidine-2,6-dione |
| GT-08860 | 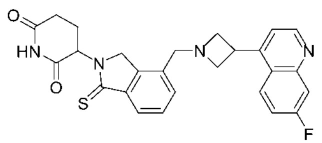 | 3-(4-((3-(7-fluoroquinolin-4-yl)azetidin-1-yl)methyl)-1-thioxoisoindolin-2-yl)piperidine-2,6-dione | 3-(4-((3-(7-fluoroquinolin-4-yl)azetidin-1-yl)methyl)-1-thioxoisoindolin-2-yl)piperidine-2,6-dione |
| GT-50863 | 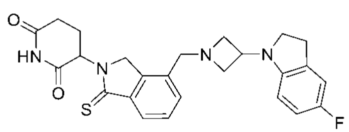 | 3-(4-((3-(5-fluoroindolin-1-yl)azetidin-1-yl)methyl)-1-thioxoisoindolin-2-yl)piperidine-2,6-dione | 3-(4-((3-(5-fluoroindolin-1-yl)azetidin-1-yl)methyl)-1-thioxoisoindolin-2-yl)piperidine-2,6-dione |
| GT-08861 | 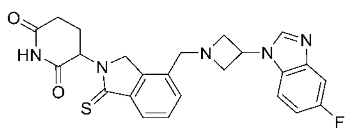 | 3-(4-((3-(5-fluoro-1H-benzo[d]imidazol-1-yl)azetidin-1-yl)methyl)-1-thioxoisoindolin-2-yl)piperidine-2,6-dione | 3-(4-((3-(5-fluoro-1H-benzo[d]imidazol-1-yl)azetidin-1-yl)methyl)-1-thioxoisoindolin-2-yl)piperidine-2,6-dione |
| GT-08912 | 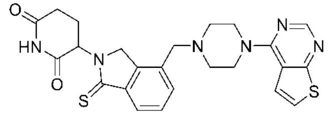 | 3-(4-((4-(thieno[2,3-d]pyrimidin-4-yl)piperazin-1-yl) methyl)-1-thioxoisoindolin-2-yl)piperidine-2,6-dione | 3-(4-((4-(thieno[2,3-d]pyrimidin-4-yl)piperazin-1-yl) methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-08913 | 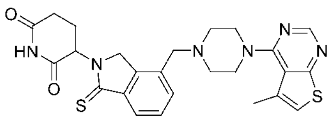 | 3-(4-((4-(5-methylthieno[2,3-d]pyrimidin-4-yl)piperazin-1-yl)methyl)-1-thioxoisoindolin-2-yl)piperidine-2,6-dione | 3-(4-((4-(5-methylthieno[2,3-d]pyrimidin-4-yl)piperazin-1-yl)methyl)-1-thioxoisoindolin-2-yl)piperidine-2,6-dione |
| GT-0891 4 | 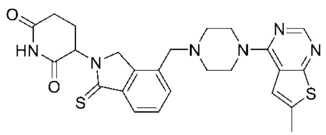 | 3-(4-((4-(6-methylthieno[2,3-d]pyrimidin-4-yl)piperazin-1-yl)methyl)-1-thioxoisoindolin-2-yl)piperidine-2,6-dione | 3-(4-((4-(6-methylthieno[2,3-d]pyrimidin-4-yl)piperazin-1-yl)methyl)-1-thioxoisoindolin-2-yl)piperidine-2,6-dione |
| GT-0891 5 | 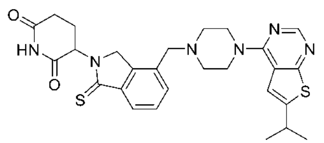 | 3-(4-((4-(6-isopropylthieno[2,3-d]pyrimidin-4-yl)piperazin-1-yl)methyl)-1-thioxoisoindolin-2-yl)piperidine-2,6-dione | 3-(4-((4-(6-isopropylthieno[2,3-d]pyrimidin-4-yl)piperazin-1-yl)methyl)-1-thioxoisoindolin-2-yl)piperidine-2,6-dione |
| GT-08916 | 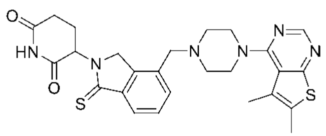 | 3-(4-((4-(5,6-dimethylthieno[2,3-d]pyrimidin-4-yl)piperazin-1-yl)methyl)-1-thioxoisoindolin-2-yl)piperidine-2,6-dione | 3-(4-((4-(5,6-dimethylthieno[2,3-d]pyrimidin-4-yl)piperazin-1-yl)methyl)-1-thioxoisoindolin-2-yl)piperidine-2,6-dione |
| GT-08919 | 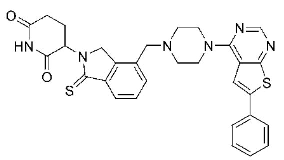 | 3-(4-((4-(6-phenylthieno[2,3-d]pyrimidin-4-yl)piperazin-1-yl)methyl)-1-thioxoisoindolin-2-yl)piperidine-2,6-dione | 3-(4-((4-(6-phenylthieno[2,3-d]pyrimidin-4-yl)piperazin-1-yl)methyl)-1-thioxoisoindolin-2-yl)piperidine-2,6-dione |
| GT-0891 8 | 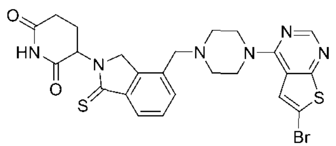 | 3-(4-((4-(6-bromothieno[2,3-d]pyrimidin-4-yl)piperazin-1-yl)methyl)-1-thioxoisoindolin-2-yl)piperidine-2,6-dione | 3-(4-((4-(6-bromothieno[2,3-d]pyrimidin-4-yl)piperazin-1-yl)methyl)-1-thioxoisoindolin-2-yl)piperidine-2,6-dione |
| GT-0892 0 | 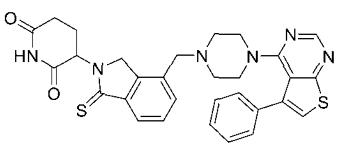 | 3-(4-((4-(5-phenylthieno[2,3-d]pyrimidin-4-yl)piperazin-1-yl)methyl)-1-thioxoisoindolin-2-yl)piperidine-2,6-dione | 3-(4-((4-(5-phenylthieno[2,3-d]pyrimidin-4-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0892 4 | 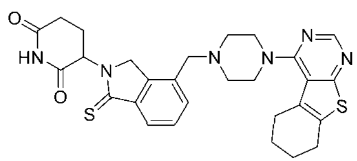 | 3-(4-((4-(5,6,7,8-tetrahydrobenzo[4,5]thieno[2 ,3-d]pyrimidin-4-yl)piperazin-1-yl)methyl)-1-thioxoisoindolin-2-yl)piperidine-2,6-dione | 3-(4-((4-(5,6,7,8-tetrahydrobenzo[4,5]thieno[2 ,3-d]pyrimidin-4-yl)piperazin-1-yl)methyl)-1-thioxoisoindolin-2-yl)piperidine-2,6-dione |
| GT-0892 5 | 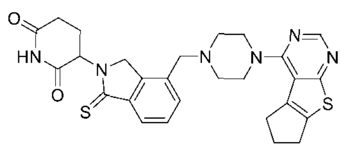 | 3-(4-((4-(6,7-dihydro-5H-cyclopenta[4,5]thieno[2,3-d]pyrimidin-4-yl)piperazin-1-yl)methyl)-1-thioxoisoindolin-2-yl)piperidine-2,6-dione | 3-(4-((4-(6,7-dihydro-5H-cyclopenta[4,5]thieno[2,3-d]pyrimidin-4-yl)piperazin-1-yl)methyl)-1-thioxoisoindolin-2-yl)piperidine-2,6-dione |
| GT-0892 1 | 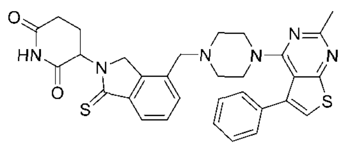 | 3-(4-((4-(2-methyl-5-phenylthieno[2,3-d]pyrimidin-4-yl)piperazin-1-yl)methyl)-1-thioxoisoindolin-2-yl)piperidine-2,6-dione | 3-(4-((4-(2-methyl-5-phenylthieno[2,3-d]pyrimidin-4-yl)piperazin-1-yl)methyl)-1-thioxoisoindolin-2-yl)piperidine-2,6-dione |
| GT-0892 3 | 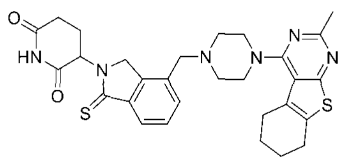 | 3-(4-((4-(2-methyl-5,6,7,8-tetrahydrobenzo[4,5]thieno[2 ,3-d]pyrimidin-4-yl)piperazin-1-yl)methyl)-1-thioxoisoindolin-2-yl)piperidine-2,6-dione | 3-(4-((4-(2-methyl-5,6,7,8-tetrahydrobenzo[4,5]thieno[2 ,3-d]pyrimidin-4-yl)piperazin-1-yl)methyl)-1-thioxoisoindolin-2-yl)piperidine-2,6-dione |
| GT-0892 6 | 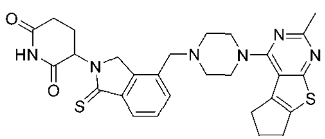 | 3-(4-((4-(2-methyl-6,7-dihydro-5H-cyclopenta[4,5]thieno[2,3-d]pyrimidin-4-yl)piperazin-1-yl)methyl)-1-thioxoisoindolin-2-yl)piperidine-2,6-dione | ·3-(4-((4-(2-methyl-6,7-dihydro-5H-cyclopenta[4,5]thieno[2,3-d]pyrimidin-4-yl)piperazin-1-yl)methyl)-1-thioxoisoindolin-2-yl)piperidine-2,6-dione |
| GT-0892 2 | 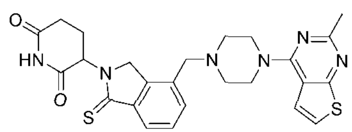 | 3-(4-((4-(2-methylthieno[2,3-d]pyrimidin-4-yl)piperazin-1-yl)methyl)-1-thioxoisoindolin-2-yl)piperidine-2,6-dione | 3-(4-((4-(2-methylthieno[2,3-d]pyrimidin-4-yl)piperazin-1-yl)methyl)-1-thioxoisoindolin-2-yl)piperidine-2,6-dione |
| GT-0892 7 | 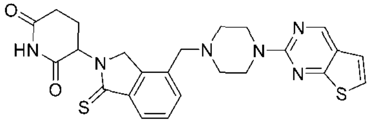 | 3-(4-((4-(thieno[2,3-d]pyrimidin-2-yl)piperazin-1-yl)methyl)-1-thioxoisoindolin-2-yl)piperidine-2,6-dione | 3-(4-((4-(thieno[2,3-d]pyrimidin-2-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0891 7 | 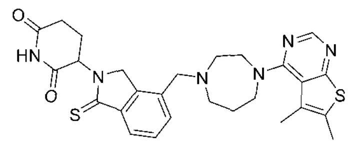 | 3-(4-((4-(5,6-dimethylthieno[2,3-d]pyrimidin-4-yl)-1,4-diazepan-1-yl)methyl)-1-thioxoisoindolin-2-yl)piperidine-2,6-dione | 3-(4-((4-(5,6-dimethylthieno[2,3-d]pyrimidin-4-yl)-1,4-diazepan-1-yl)methyl)-1-thioxoisoindolin-2-yl)piperidine-2,6-dione |
| GT-0892 8 | 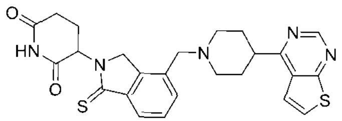 | 3-(4-((4-(thieno[2,3-d]pyrimidin-4-yl)piperidin-1-yl) methyl)-1-thioxoisoindolin-2-yl)piperidine-2,6-dione | 3-(4-((4-(thieno[2,3-d]pyrimidin-4-yl)piperidin-1-yl) methyl)-1-thioxoisoindolin-2-yl)piperidine-2,6-dione |
| | 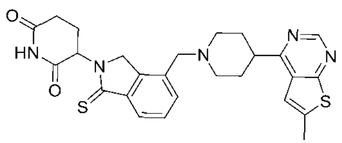 | 3-(4-((4-(6-methylthieno[2,3-d]pyrimidin-4-yl)piperidin-1-yl)methyl)-1-thioxoisoindolin-2-yl)piperidine-2,6-dione | 3-(4-((4-(6-methylthieno[2,3-d]pyrimidin-4-yl)piperidin-1-yl)methyl)-1-thioxoisoindolin-2-yl)piperidine-2,6-dione |
| | 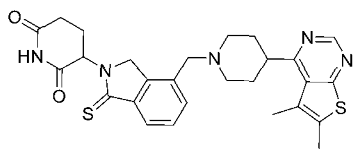 | 3-(4-((4-(5,6-dimethylthieno[2,3-d]pyrimidin-4-yl)piperidin-1-yl)methyl)-1-thioxoisoindolin-2-yl)piperidine-2,6-dione | 3-(4-((4-(5,6-dimethylthieno[2,3-d]pyrimidin-4-yl)piperidin-1-yl)methyl)-1-thioxoisoindolin-2-yl)piperidine-2,6-dione |
| GT-0892 9 | 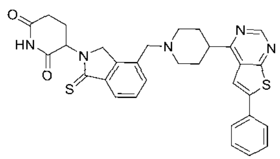 | 3-(4-((4-(6-phenylthieno[2,3-d]pyrimidin-4-yl)piperidin-1-yl)methyl)-1-thioxoisoindolin-2-yl)piperidine-2,6-dione | 3-(4-((4-(6-phenylthieno[2,3-d]pyrimidin-4-yl)piperidin-1-yl)methyl)-1-thioxoisoindolin-2-yl)piperidine-2,6-dione |
| GT-0893 0 | 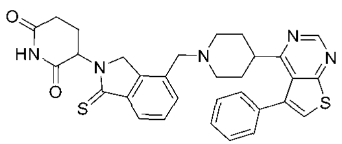 | 3-(4-((4-(5-phenylthieno[2,3-d]pyrimidin-4-yl)piperidin-1-yl)methyl)-1-thioxoisoindolin-2-yl)piperidine-2,6-dione | 3-(4-((4-(5-phenylthieno[2,3-d]pyrimidin-4-yl)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 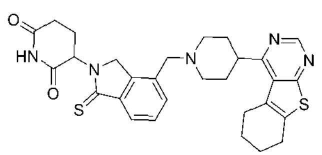 | 3-(4-((4-(5,6,7,8-tetrahydrobenzo[4,5]thieno[2 ,3-d]pyrimidin-4-yl)piperidin-1-yl)methyl)-1-thioxoisoindolin-2-yl)piperidine-2,6-dione | 3-(4-((4-(5,6,7,8-tetrahydrobenzo[4,5]thieno[2 ,3-d]pyrimidin-4-yl)piperidin-1-yl)methyl)-1-thioxoisoindolin-2-yl)piperidine-2,6-dione |
| GT-0893 4 | 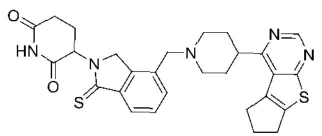 | 3-(4-((4-(6,7-dihydro-5H-cyclopenta[4,5]thieno[2,3-d]pyrimidin-4-yl)piperidin-1-yl)methyl)-1-thioxoisoindolin-2-yl)piperidine-2,6-dione | 3-(4-((4-(6,7-dihydro-5H-cyclopenta[4,5]thieno[2,3-d]pyrimidin-4-yl)piperidin-1-yl)methyl)-1-thioxoisoindolin-2-yl)piperidine-2,6-dione |
| GT-0893 1 | 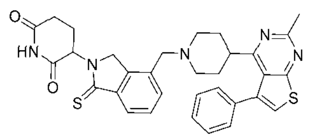 | 3-(4-((4-(2-methyl-5-phenylthieno[2,3-d]pyrimidin-4-yl)piperidin-1-yl)methyl)-1-thioxoisoindolin-2-yl)piperidine-2,6-dione | 3-(4-((4-(2-methyl-5-phenylthieno[2,3-d]pyrimidin-4-yl)piperidin-1-yl)methyl)-1-thioxoisoindolin-2-yl)piperidine-2,6-dione |
| GT-0893 3 | 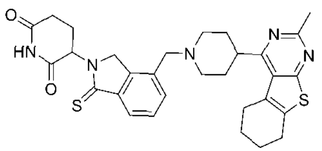 | 3-(4-((4-(2-methyl-5,6,7,8-tetrahydrobenzo[4,5]thieno[2 ,3-d]pyrimidin-4-yl)piperidin-1-yl)methyl)-1-thioxoisoindolin-2-yl)piperidine-2,6-dione | 3-(4-((4-(2-methyl-5,6,7,8-tetrahydrobenzo[4,5]thieno[2 ,3-d]pyrimidin-4-yl)piperidin-1-yl)methyl)-1-thioxoisoindolin-2-yl)piperidine-2,6-dione |
| GT-0893 5 | 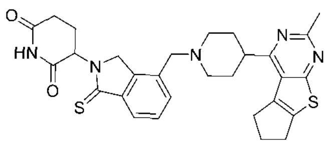 | 3-(4-((4-(2-methyl-6,7-dihydro-5H-cyclopenta[4,5]thieno[2,3-d]pyrimidin-4-yl)piperidin-1-yl)methyl)-1-thioxoisoindolin-2-yl)piperidine-2,6-dione | 3-(4-((4-(2-methyl-6,7-dihydro-5H-cyclopenta[4,5]thieno[2,3-d]pyrimidin-4-yl)piperidin-1-yl)methyl)-1-thioxoisoindolin-2-yl)piperidine-2,6-dione |
| GT-0893 2 | 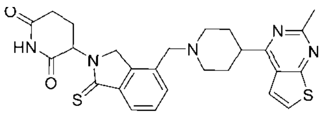 | 3-(4-((4-(2-methylthieno[2,3-d]pyrimidin-4-yl)piperidin-1-yl)methyl)-1-thioxoisoindolin-2-yl)piperidine-2,6-dione | 3-(4-((4-(2-methylthieno[2,3-d]pyrimidin-4-yl)piperidin-1-yl)methyl)-1-thioxoisoindolin-2-yl)piperidine-2,6-dione |
| GT-0893 6 | 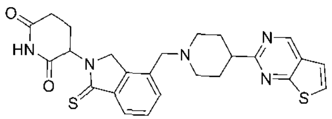 | 3-(4-((4-(thieno[2,3-d]pyrimidin-2-yl)piperidin-1-yl)methyl)-1-thioxoisoindolin-2-yl)piperidine-2,6-dione | 3-(4-((4-(thieno[2,3-d]pyrimidin-2-yl)piperidin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 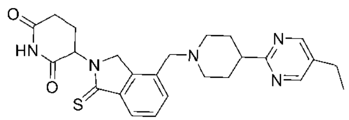 | 3-(4-((4-(5-ethylpyrimidin-2-yl)piperidin-1-yl)methyl)-1-thioxoisoindolin-2-yl)piperidine-2,6-dione | 3-(4-((4-(5-ethylpyrimidin-2-yl)piperidin-1-yl)methyl)-1-thioxoisoindolin-2-yl)piperidine-2,6-dione |
| GT-0893 7 | 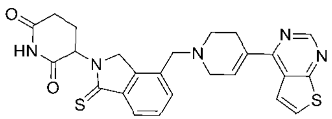 | 3-(4-((4-(thieno[2,3-d]pyrimidin-4-yl)-3,6-dihydropyridin-1(2H)-yl)methyl)-1-thioxoisoindolin-2-yl)piperidine-2,6-dione | 3-(4-((4-(thieno[2,3-d]pyrimidin-4-yl)-3,6-dihydropyridin-1(2H)-yl)methyl)-1-thioxoisoindolin-2-yl)piperidine-2,6-dione |
| GT-0893 8 | 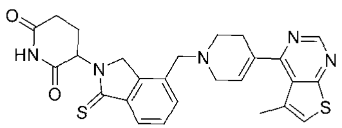 | 3-(4-((4-(5-methylthieno[2,3-d]pyrimidin-4-yl)-3,6-dihydropyridin-1(2H)-yl)methyl)-1-thioxoisoindolin-2-yl)piperidine-2,6-dione | 3-(4-((4-(5-methylthieno[2,3-d]pyrimidin-4-yl)-3,6-dihydropyridin-1(2H)-yl)methyl)-1-thioxoisoindolin-2-yl)piperidine-2,6-dione |
| GT-0894 7 | 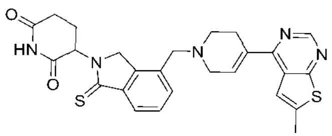 | 3-(4-((4-(6-methylthieno[2,3-d]pyrimidin-4-yl)-3,6-dihydropyridin-1(2H)-yl)methyl)-1-thioxoisoindolin-2-yl)piperidine-2,6-dione | 3-(4-((4-(6-methylthieno[2,3-d]pyrimidin-4-yl)-3,6-dihydropyridin-1(2H)-yl)methyl)-1-thioxoisoindolin-2-yl)piperidine-2,6-dione |
| GT-0895 4 | 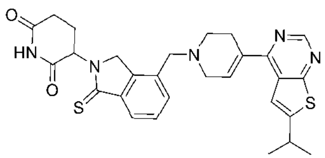 | 3-(4-((4-(6-isopropylthieno[2,3-d]pyrimidin-4-yl)-3,6-dihydropyridin-1(2H)-yl)methyl)-1-thioxoisoindolin-2-yl) piperidine-2,6-dione | 3-(4-((4-(6-isopropylthieno[2,3-d]pyrimidin-4-yl)-3,6-dihydropyridin-1(2H)-yl)methyl)-1-thioxoisoindolin-2-yl) piperidine-2,6-dione |
| GT-0895 5 | 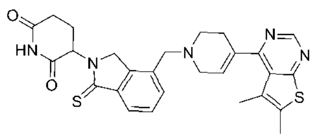 | 3-(4-((4-(5,6-dimethylthieno[2,3-d]pyrimidin-4-yl)-3,6-dihydropyridin-1(2H)-yl)methyl)-1-thioxoisoindolin-2-yl) piperidine-2,6-dione | 3-(4-((4-(5,6-dimethylthieno[2,3-d]pyrimidin-4-yl)-3,6-dihydropyridin-1(2H)-yl)methyl)-1-thioxoisoindolin-2-yl) piperidine-2,6-dione |
| GT-0895 6 | 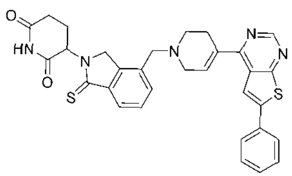 | 3-(4-((4-(6-phenylthieno[2,3-d]pyrimidin-4-yl)-3,6-dihydropyridin-1(2H)-yl)methyl)-1-thioxoisoindolin-2-yl)piperidine-2,6-dione | 3-(4-((4-(6-phenylthieno[2,3-d]pyrimidin-4-yl)-3,6-dihydropyridin-1(2H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0895 7 | 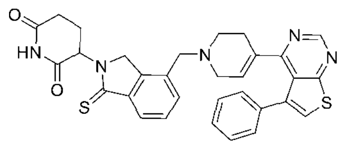 | 3-(4-((4-(5-phenylthieno[2,3-d]pyrimidin-4-yl)-3,6-dihydropyridin-1 (2H)-yl)methyl)-1-thioxoisoindolin-2-yl)piperidine-2,6-dione | 3-(4-((4-(5-phenylthieno[2,3-d]pyrimidin-4-yl)-3,6-dihydropyridin-1(2H)-yl)methyl)-1-thioxoisoindolin-2-yl)piperidine-2,6-dione |
| GT-0896 2 | 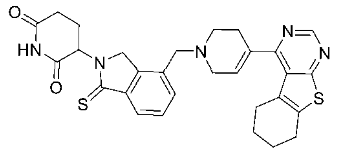 | 3-(4-((4-(5,6,7,8-tetrahydrobenzo[4,5]thieno[2 ,3-d]pyrimidin-4-yl)-3,6-dihydropyridin-1 (2H)-yl)methyl)-1-thioxoisoindolin-2-yl)piperidine-2,6-dione | 3-(4-((4-(5,6,7,8-tetrahydrobenzo[4,5]thieno[2 ,3-d]pyrimidin-4-yl)-3,6-dihydropyridin-1(2H)-yl)methyl)-1-thioxoisoindolin-2-yl)piperidine-2,6-dione |
| GT-0896 3 | 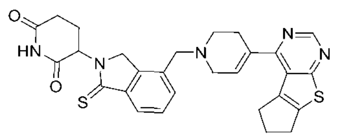 | 3-(4-((4-(6,7-dihydro-5H-cyclopenta[4,5]thieno[2,3-d] pyrimidin-4-yl)-3,6-dihydropyridin-1 (2H)-yl)methyl)-1-thioxoisoindolin-2-yl)piperidine-2,6-dione | 3-(4-((4-(6,7-dihydro-5H-cyclopenta[4,5]thieno[2,3-d] pyrimidin-4-yl)-3,6-dihydropyridin-1(2H)-yl)methyl)-1-thioxoisoindolin-2-yl)piperidine-2,6-dione |
| GT-0895 8 | 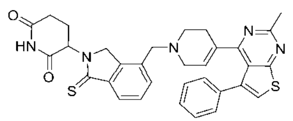 | 3-(4-((4-(2-methyl-5-phenylthieno[2,3-d]pyrimidin-4-yl)-3,6-dihydropyridin-1 (2H)-yl)methyl)-1-thioxoisoindolin-2-yl)piperidine-2,6-dione | 3-(4-((4-(2-methyl-5-phenylthieno[2,3-d]pyrimidin-4-yl)-3,6-dihydropyridin-1(2H)-yl)methyl)-1-thioxoisoindolin-2-vl)piperidine-2,6-dione |
| GT-0896 1 | 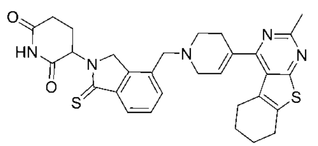 | 3-(4-((4-(2-methyl-5,6,7,8-tetrahydrobenzo[4,5]thieno [2 ,3-d]pyrimidin-4-yl)-3,6-dihydropyridin-1 (2H)-yl) methyl)-1-thioxoisoindolin-2-yl)piperidine-2,6-dione | 3-(4-((4-(2-methyl-5,6,7,8-tetrahydrobenzo[4,5]thieno [2 ,3-d]pyrimidin-4-yl)-3,6-dihydropyridin-1(2H)-yl) methyl)-1-thioxoisoindolin-2-vl)piperidine-2,6-dione |
| GT-0896 4 | 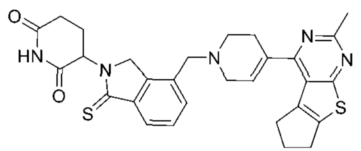 | 3-(4-((4-(2-methyl-6,7-dihydro-5H-cyclopenta[4,5]thieno[2,3-d]pyrimidin-4-yl)-3,6-dihydropyridin-1 (2H)-yl) methyl)-1-thioxoisoindolin-2-yl)piperidine-2,6-dione | 3-(4-((4-(2-methyl-6,7-dihydro-5H-cyclopenta[4,5]thieno[2,3-d]pyrimidin-4-yl)-3,6-dihydropyridin-1(2H)-yl) methyl)-1-thioxoisoindolin-2-yl)piperidine-2,6-dione |
| GT-0895 9 | 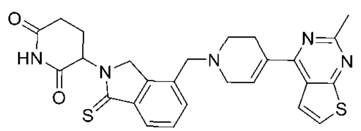 | 3-(4-((4-(2-methylthieno[2,3-d]pyrimidin-4-yl)-3,6-dihydropyridin-1(2H)-yl)methyl)-1-thioxoisoindolin-2-yl)piperidine-2,6-dione | 3-(4-((4-(2-methylthieno[2,3-d]pyrimidin-4-yl)-3,6-dihydropyridin-1(2H)-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0896 5 | 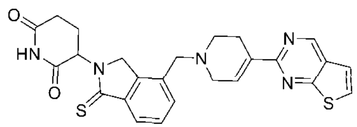 | 3-(4-((4-(thieno[2,3-d]pyrimidin-2-yl)-3,6-dihydropyridin-1 (2H)-yl)methyl)-1-thioxoisoindolin-2-yl)piperidine-2,6-dione | 3-(4-((4-(thieno[2,3-d]pyrimidin-2-yl)-3,6-dihydropyridin-1(2H)-yl)methyl)-1-thioxoisoindolin-2-yl)piperidine-2,6-dione |
| GT-0896 6 | 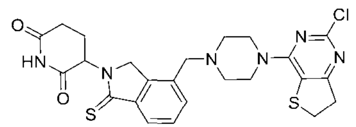 | 3-(4-((4-(2-chloro-6,7-dihydrothieno[3,2-d]pyrimidin-4-yl)piperazin-1-yl)methyl)-1-thioxoisoindolin-2-yl)piperidine-2,6-dione | 3-(4-((4-(2-chloro-6,7-dihydrothieno[3,2-d]pyrimidin-4-yl)piperazin-1-yl)methyl)-1-thioxoisoindolin-2-yl)piperidine-2,6-dione |
| GT-0896 8 | 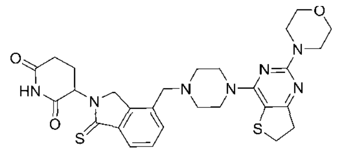 | 3-(4-((4-(2-morpholino-6,7-dihydrothieno[3,2-d]pyrimidin-4-yl)piperazin-1-yl)methyl)-1-thioxoisoindolin-2-yl) piperidine-2,6-dione | 3-(4-((4-(2-morpholino-6,7-dihydrothieno[3,2-d]pyrimidin-4-yl)piperazin-1-yl)methyl)-1-thioxoisoindolin-2-yl) piperidine-2,6-dione |
| GT-0903 5 | 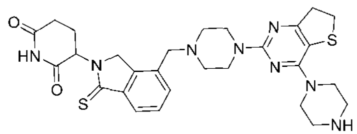 | 3-(4-((4-(4-(piperazin-1-yl)-6,7-dihydrothieno[3,2-d]pyrimidin-2-yl)piperazin-1-yl)methyl)-1-thioxoisoindolin-2-yl)piperidine-2,6-dione | 3-(4-((4-(4-(piperazin-1-yl)-6,7-dihydrothieno[3,2-d]pyrimidin-2-yl)piperazin-1-yl)methyl)-1-thioxoisoindolin-2-yl)piperidine-2,6-dione |
| GT-0896 9 | 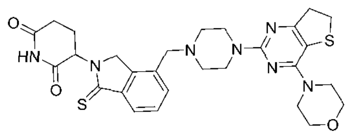 | 3-(4-((4-(4-morpholino-6,7-dihydrothieno[3,2-d]pyrimidin-2-yl)piperazin-1-yl)methyl)-1-thioxoisoindolin-2-yl) piperidine-2,6-dione | 3-(4-((4-(4-morpholino-6,7-dihydrothieno[3,2-d]pyrimidin-2-yl)piperazin-1-yl)methyl)-1-thioxoisoindolin-2-yl) piperidine-2,6-dione |
| | 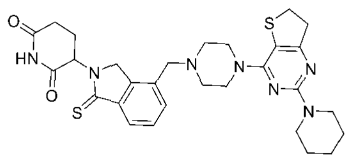 | 3-(4-((4-(2-(piperidin-1-yl)-6,7-dihydrothieno[3,2-d]pyrimidin-4-yl)piperazin-1-yl)methyl)-1-thioxoisoindolin-2-yl)piperidine-2,6-dione | 3-(4-((4-(2-(piperidin-1-yl)-6,7-dihydrothieno[3,2-d]pyrimidin-4-yl)piperazin-1-yl)methyl)-1-thioxoisoindolin-2-yl)piperidine-2,6-dione |
| | 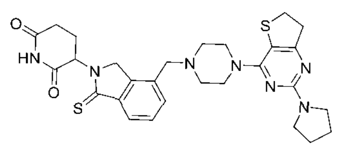 | 3-(4-((4-(2-(pyrrolidin-1-yl)-6,7-dihydrothieno[3,2-d]pyrimidin-4-yl)piperazin-1-yl)methyl)-1-thioxoisoindolin-2-yl)piperidine-2,6-dione | 3-(4-((4-(2-(pyrrolidin-1-yl)-6,7-dihydrothieno[3,2-d]pyrimidin-4-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| | 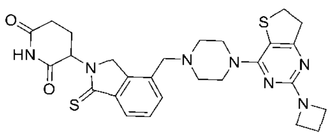 | 3-(4-((4-(2-(azetidin-1-yl)-6,7-dihydrothieno[3,2-d]pyrimidin-4-yl)piperazin-1-yl)methyl)-1-thioxoisoindolin-2-yl)piperidine-2,6-dione | 3-(4-((4-(2-(azetidin-1-yl)-6,7-dihydrothieno[3,2-d]pyrimidin-4-yl)piperazin-1-yl)methyl)-1-thioxoisoindolin-2-yl)piperidine-2,6-dione |
| GT-0897 0 | 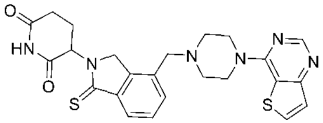 | 3-(4-((4-(thieno[3,2-d]pyrimidin-4-yl)piperazin-1-yl)methyl)-1-thioxoisoindolin-2-yl)piperidine-2,6-dione | 3-(4-((4-(thieno[3,2-d]pyrimidin-4-yl)piperazin-1-yl)methyl)-1-thioxoisoindolin-2-yl)piperidine-2,6-dione |
| GT-0897 1 | 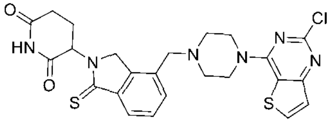 | 3-(4-((4-(2-chlorothieno[3,2-d]pyrimidin-4-yl)piperazin-1-yl)methyl)-1-thioxoisoindolin-2-yl)piperidine-2,6-dione | 3-(4-((4-(2-chlorothieno[3,2-d]pyrimidin-4-yl)piperazin-1-yl)methyl)-1-thioxoisoindolin-2-yl)piperidine-2,6-dione |
| GT-0903 2 | 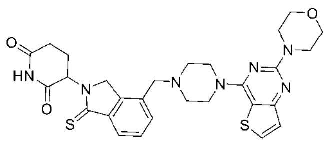 | 3-(4-((4-(2-morpholinothieno[3,2-d]pyrimidin-4-yl)piperazin-1-yl)methyl)-1-thioxoisoindolin-2-yl)piperidine-2,6-dione | 3-(4-((4-(2-morpholinothieno[3,2-d]pyrimidin-4-yl)piperazin-1-yl)methyl)-1-thioxoisoindolin-2-yl)piperidine-2,6-dione |
| GT-0903 6 | 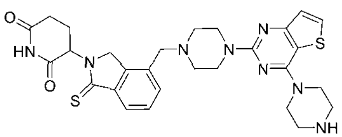 | 3-(4-((4-(4-(piperazin-1-yl)thieno[3,2-d]pyrimidin-2-yl)piperazin-1-yl)methyl)-1-thioxoisoindolin-2-yl)piperidine-2,6-dione | 3-(4-((4-(4-(piperazin-1-yl)thieno[3,2-d]pyrimidin-2-yl)piperazin-1-yl)methyl)-1-thioxoisoindolin-2-yl)piperidine-2,6-dione |
| GT-0903 3 | 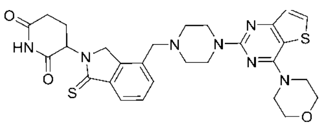 | 3-(4-((4-(4-morpholinothieno[3,2-d]pyrimidin-2-yl)piperazin-1-yl)methyl)-1-thioxoisoindolin-2-yl)piperidine-2,6-dione | 3-(4-((4-(4-morpholinothieno[3,2-d]pyrimidin-2-yl)piperazin-1-yl)methyl)-1-thioxoisoindolin-2-yl)piperidine-2,6-dione |
| GT-0836 4 | 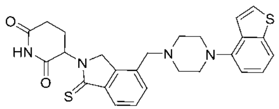 | 3-(4-((4-(benzo[b]thiophen-4-yl)piperazin-1-yl)methyl)-1-thioxoisoindolin-2-yl)piperidine-2,6-dione | 3-(4-((4-(benzo[b]thiophen-4-yl)piperazin-1-yl)methyl)-1-thioxoisoindolin-2-yl)piperidine-2,6-dione |

(continued)

| Compound No. | Structure of the compounds | The compounds' name | Chinese translation of the compounds' name |
|---|---|---|---|
| GT-0836 5 | 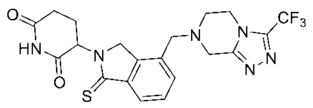 | 3-(1-thioxo-4-((3-(trifluoromethyl)-5,6-dihydro-[1,2,4] triazolo[4,3-a]pyrazin-7(8H)-yl)methyl)isoindolin-2-yl) piperidine-2,6-dione | 3-(1-thioxo-4-((3-(trifluoromethyl)-5,6-dihydro-[1,2,4] triazolo[4,3-a]pyrazin-7(8H)-yl)methyl)isoindolin-2-yl) piperidine-2,6-dione |
| GT-0833 2 | 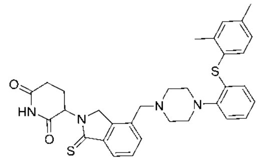 | 3-(4-((4-(2-((2,4-dimethylphenyl)thio)phenyl) piperazin-1-yl)methyl)-1-thioxoisoindolin-2-yl)piperidine-2,6-dione | 3-(4-((4-(2-((2,4-dimethylphenyl)thio)phenyl) piperazin-1-yl)methyl)-1-thioxoisoindolin-2-yl)piperidine-2,6-dione |
| GT-0833 3 | 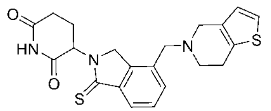 | 3-(4-((6,7-dihydrothieno[3,2-c]pyridin-5(4H)-yl) methyl)-1-thioxoisoindolin-2-yl)piperidine-2,6-dione | 3-(4-((6,7-dihydrothieno[3,2-c]pyridin-5(4H)-yl) methyl)-1-thioxoisoindolin-2-yl)piperidine-2,6-dione |
| GT-0836 6 | 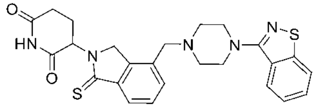 | 3-(4-((4-(benzo[d]isothiazol-3-yl)piperazin-1-yl) methyl)-1-thioxoisoindolin-2-yl)piperidine-2,6-dione | 3-(4-((4-(benzo[d]isothiazol-3-yl)piperazin-1-yl) methyl)-1-thioxoisoindolin-2-yl)piperidine-2,6-dione |
| | 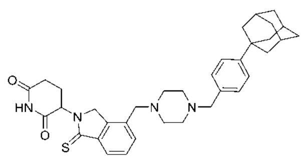 | 3-(4-((4-(4-(adamantan-1-yl)benzyl)piperazin-1-yl) methyl)-1-thioxoisoindolin-2-yl)piperidine-2,6-dione | 3-(4-((4-(4-(adamantan-1-yl)benzyl)piperazin-1-yl) methyl)-1-thioxoisoindolin-2-yl)piperidine-2,6-dione |
| | 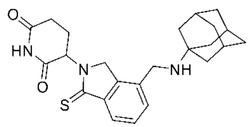 | 3-(4-(((adamantan-1-yl)amino)methyl)-1-thioxoisoindolin-2-yl)piperidine-2,6-dione | 3-(4-(((adamantan-1-yl)amino)methyl)-1-thioxoisoindolin-2-yl)piperidine-2,6-dione |
| | 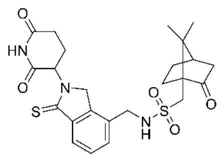 | 1-((1R,4R)-7,7-dimethyl-2-oxobicyclo[2.2.1]heptan-1-yl)-N-((2-(2,6-dioxopiperidin-3-yl)-1-thioxoisoindolin-4-yl)methyl)methanesulfonamide | 1-((1R,4R)-7,7-dimethyl-2-oxobicyclo[2.2.1]heptan-1-yl)-N-((2-(2,6-dioxopiperidin-3-yl)-1-thioxoisoindolin-4-yl)methyl)methanesulfonamide |

(continued)

| Compound No. | Structure of the compounds | The compounds' name | Chinese translation of the compounds' name |
|---|---|---|---|
| | 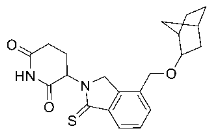 | 3-(4-((((1R,4S)-bicyclo[2.2.1]heptan-2-yl)oxy) methyl)-1-thioxoisoindolin-2-yl)piperidine-2,6-dione | 3-(4-((((1R,4S)-bicyclo[2.2.1]heptan-2-yl)oxy) methyl)-1-thioxoisoindolin-2-yl)piperidine-2,6-dione |
| GT-0896 0 | 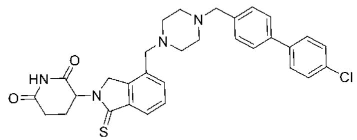 | 3-(4-((4-((4'-chloro-[1,1'-biphenyl]-4-yl)methyl)piperazin-1-yl)methyl)-1-thioxoisoindolin-2-yl)piperidine-2,6-dione | 3-(4-((4-((4'-chloro-[1,1'-biphenyl]-4-yl)methyl)piperazin-1-yl)methyl)-1-thioxoisoindolin-2-yl)piperidine-2,6-dione |
| GT-05712 | 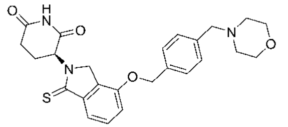 | (S)-3-(4-((4-(morpholinomethyl)benzyl)o xy)-1-thioxoisoindolin-2-yl)piperidine-2,6-dione | (S)-3-(4-((4-(morpholinomethyl)benzyl)o xy)-1-thioxoisoindolin-2-yl)piperidine-2,6-dione |
| GT-05957 | 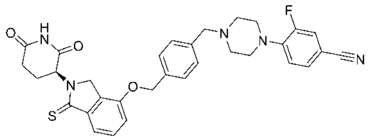 | (S)-4-(4-(4-(((2-(2,6-dioxopiperidin-3-yl)-1-thioxoisoindolin-4-yl)oxy)methyl)benzyl)pipera zin-1-yl)-3-fluorobenzonitrile | (S)-4-(4-(4-(((2-(2,6-dioxopiperidin-3-yl)-1-thioxoisoindolin-4-yl)oxy)methyl)benzyl)pipera zin-1-yl)-3-fluorobenzonitrile |

**II. Other Forms of Compounds** (including salts, enantiomers, stereoisomers, solvates, isotopically enriched analogs, prodrugs, or polymorphs of compounds)

The compounds of the present disclosure have the structures of any one of Formula (I), Formula (I-1), Formula (I-2), Formula (I-3), Formula (I-4), Formula (I-5), Formula (I-5-1), Formula (I-5-1A), Formula (I-5-1B), Formula (I-5-2), Formula (I-5-2A) or Formula (I-5-2B). Unless otherwise specified, all references to the compounds of the present disclosure also include compounds of any one of Formula (I), Formula (I-1), Formula (I-2), Formula (I-3), Formula (I-4), Formula (I-5), Formula (I-5-1), Formula (I-5-1A), Formula (I-5-1B), Formula (I-5-2), Formula (I-5-2A) or Formula (I-5-2B) and specific compounds within the scope of these general formulae.

It should be recognized that compounds of the present disclosure (including compounds of Formula (I), Formula (I-1), Formula (I-2), Formula (I-3), Formula (I-4), Formula (I-5), Formula (I-5-1), Formula (I-5-1A), Formula (I-5-1B), Formula (I-5-2), Formula (I-5-2A) or Formula (I-5-2B)) may have a stereo-configuration and thus can exist in more than one stereoisomeric form. The present disclosure also relates to optically enriched compounds having a stereo-configuration, e.g., greater than about 90% enantiomeric/diastereomeric excess ("ee"), such as about 95% ee or 97% ee, or greater than 99% ee, and mixtures thereof, including racemic mixtures. As used herein, "optically enriched" means that a mixture of enantiomers consists of a significantly greater proportion of one enantiomer, and can be described by enantiomeric excess (ee%). Purification of isomers and separation of mixtures of isomers can be accomplished by standard techniques known in the art (e.g., column chromatography, preparative TLC, preparative HPLC, asymmetric synthesis (e.g., by using chiral intermediates) and/or or chiral resolution, etc.).

In some embodiments, polymorph forms or salts of the compounds of the present disclosure are also provided. Salts of the compounds of the present disclosure can be pharmaceutically acceptable salts including, but not limited to,hydrohalide (including hydrochloride, hydrobromide), sulfate, maleate, sulfonate, citrate, lactate, lactobionate, L-tartrate, fumarate, L-malate, L-lactate, α-ketoglutarate, hippurate, D-glucuronate, D-gluconate, α-D-glucoheptonate, glycolate, mucate, L-ascorbate, orotate, picrate, glycinate, alaninate, arginate, cinnamate, laurate, pamoate, sebacate, besylate, methanesulfonate, ethanesulfonate, edisylate,formate, acetate, 2,2-dichloroacetate, trimethylacetate, propionate, valerate, palmitate, triphenylacetate, 2-ethyl-butane-1,4-dioate, iodate, nicotinate, L-pyroglutamate, L-prolinate, ferulate, 2-hydroxyethanesulfonate, nitrate, gentisate, cholate, salicylate, terephthalate, glutarate, adipate, stearate, oleate, undecylenate, camphorate, camsylate, dodecylsulfate, phosphate, thiocyanate, dihydrogen phosphate, pyrophosphate, metaphosphate, oxalate, carbonate, malonate, benzoate, mandelate, succinate, pyruvate, 4-chlorobenzenesulfonate, 1,5-naphthalenedisulfonate, 3-hydroxy-2-naphthoate, 1-hydroxy-2-naphthoate, 2-naphthalenesulfonate, trifluoroacetate, glycolate, or 4-methylbenzenesulfonate, etc., of the compounds of the present disclosure. The compounds of the present disclosure can exist as non-solvated or solvated forms in pharmaceutically acceptable solvents such as water, ethanol, and the like. In some embodiments, the compounds of the present disclosure can be prepared as prodrugs or precursor drugs. Prodrugs can be converted into parent drugs in the body to play their role. In some embodiments, isotopically-labeled compounds of the present disclosure are also provided, examples of which include deuterium (D or $^2H$).

**III. Compositions/Formulations**

In some embodiments, the present disclosure provides a pharmaceutical composition comprising as active ingredient the compound of the present disclosure or a pharmaceutically acceptable salt, solvate, isotopically enriched analog, polymorph, prodrug, stereoisomer (including enantiomer), or mixture of stereoisomers thereof, and at least one pharmaceutically acceptable carrier.

In some embodiments, pharmaceutically acceptable carriers include, but are not limited to, fillers, stabilizers, dispersants, suspending agents, diluents, excipients, thickeners, colorants, solvents, or encapsulating materials. Each carrier must be "acceptable" in the sense of being compatible with the other ingredients of the formulation (including the compounds useful in the present disclosure) and not injurious to the patient. Some examples of materials that can be used as pharmaceutically acceptable carriers include: sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; polyethylene oxide, polyvinylpyrrolidone, polyacrylamide, poloxamer; and other common nontoxic compatible substances used in pharmaceutical formulations.

The pharmaceutical composition of the present disclosure can further comprise at least one second therapeutic agent, e.g., an anticancer agent. The second therapeutic agent may be used in combination with the compounds of Formula (I) described in the present disclosure to treat the diseases or disorders as disclosed herein. The second

therapeutic agent includes, but is not limited to, chemotherapeutic agents, immunotherapeutic agents, gene therapy agents, and the like.

The pharmaceutical composition of the present disclosure comprising, as an active ingredient, the compounds of Formula (I) of the present disclosure or a pharmaceutically acceptable salt thereof can be formulated into any suitable formulations such as sprays, patches, tablets (such as conventional tablets, dispersible tablets, orally disintegrating tablets), capsules (such as soft capsules, hard capsules, entericcoated capsules), dragees, troches, powders, granules, powder injections, suppositories, or liquid formulations (such as suspensions (e.g., aqueous or oily suspensions), solutions, emulsions, or syrups), or conventional injection dosage forms such as injectable solutions (e.g., sterile injectable solutions formulated according to methods known in the art using water, Ringer's solution, or isotonic sodium chloride solution or the like as a vehicle or solvent) or lyophilized injectable formulation and the like, depending upon a suitable route of administration (including, but not limited to, nasal administration, inhalation administration, topical administration, oral administration, oral mucosal administration, rectal administration, intrapleural administration, intraperitoneal administration, vaginal administration, intramuscular administration, subcutaneous administration, transdermal administration, epidural administration, intrathecal administration, and intravenous administration). Those skilled in the art can also formulate the compounds of Formula (I) of the present disclosure into conventional, dispersible, chewable, orally disintegrating or rapidly dissolving formulations, or sustained-release capsules or controlled-release capsules as needed.

The compounds of Formula (I) of the present disclosure, as an active ingredient, is contained in the pharmaceutically acceptable carrier or diluent in an amount sufficient to deliver to a subject a therapeutically effective amount for the indication to be treated, without causing serious toxic effects in the subject treated. A dosage of the active compound for all diseases or disorders mentioned herein ranges, for example, from about 5ng/kg to 500mg/kg, from about 10ng/kg to 300mg/kg per day, such as from 0.1 to 100 mg/kg, or from 0.5 to about 25mg per kilogram body weight of the subject per day.

The compounds of Formula (I) of the present disclosure or pharmaceutically acceptable salts thereof may be conveniently administered in any suitable unit dosage form. Suitable unit dosage form specifications include, but are not limited to, less than 1mg, 1mg to 3000mg, 5mg to 1000mg, for example, 5 to 500mg, 25 to 250mg of active ingredient per unit dosage form.

## IV. Kits/Packaged Products

The compounds of Formula (I) of the present disclosure or a pharmaceutically acceptable salt, solvate, isotopically enriched analog, polymorph, prodrug, stereoisomer (including enantiomer), or mixture of stereoisomers thereof is used as a medicament. The medicament of the present disclosure or the pharmaceutical composition of the present disclosure may be presented in a kit/packaged product. The kit/packaged product may include a package or container including, but not limited to, ampoules, blister packs, pharmaceutical plastic bottles, vials, pharmaceutical glass bottles, containers, syringes, laminated flexible packaging, co-extruded film infusion containers, test tubes and dispensing devices, and the like. The kit/packaged product may contain instructions for use of the product.

## V. Treatment Methods and Uses

The compounds of Formula (I) or a pharmaceutically acceptable salt, solvate, isotopically enriched analog, polymorph, prodrug, stereoisomer (including enantiomer), or mixture of stereoisomers thereof can be used as a medicament. Especially the compound of Formula (I) of the present disclosure or a pharmaceutically acceptable salt, solvate, isotopically enriched analog, polymorph, prodrug, stereoisomer (including enantiomer), or mixture of stereoisomers thereof can be used for the manufacture of a medicament for the prevention and/or treatment of a disease or disorder. The disease or disorder includes a disease or disorder associated with a cereblon protein. In some embodiments, the disease or disorder comprises: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes. In some embodiments, the disease or disorder includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, includingacute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemiaassociated anemia, monocytic leukemia, myelomonocytic leukemia, T-lymphocytic leukemia, acute T-lymphocytic leukemia; lymphoma, including diffuse large B-cell lymphoma, non-Hodgkin' s lymphoma, Hodgkin' s lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell

lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht's syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

The present disclosure provides a method for preventing and/or treating a disease or disorder in a subject, comprising administering to the subject a therapeutically effective amount of the compounds of Formula (I) of the present disclosure or a pharmaceutically acceptable salt thereof, or the pharmaceutical composition of the present disclosure comprising as an active ingredient the compounds of Formula (I) of the present disclosure or a pharmaceutically acceptable salt thereof. In some embodiments, the disease or disorder includes a disease or disorder associated with a cereblon protein. In some embodiments, the disease or disorder includes: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes. In some embodiments, the disease or disorder includes, but is not limited to, myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, includingacute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemiaassociated anemia, monocytic leukemia, myelomonocytic leukemia, T-lymphocytic leukemia, acute T-lymphocytic leukemia; lymphoma, including diffuse large B-cell lymphoma, non-Hodgkin' s lymphoma, Hodgkin' s lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral

ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

In the method for preventing and/or treating a disease or disorder, the compound of Formula (I) of the present disclosure or the pharmaceutical composition comprising as an active ingredient the compound of Formula (I) of the present disclosure is administered to the subject through at least one mode of administration selected from the group consisting of nasal administration, inhalation administration, topical administration, oral administration, oral mucosal administration, rectal administration, pleural cavity administration, peritoneal administration, intramuscular administration, subcutaneous, transdermal, epidural, intrathecal, and intravenous administration.

The term "treatment" or "treating" refers to administering to a subject the compound of Formula (I) of the present disclosure, or a pharmaceutically acceptable salt thereof, or the pharmaceutical composition comprising, as an active ingredient, the compound of Formula (I) of the present disclosure or a pharmaceutically acceptable salt thereof, to mitigate (alleviate) undesirable diseases or conditions, such as the development of a cancer or tumor. The beneficial or desired clinical results of the present disclosure include, but are not limited to: alleviating symptoms, reducing the severity of the disease, stabilizing the state of the disease, slowing down or delaying the progression of the disease, improving or alleviating the condition, and alleviating the disease.

A "therapeutic effective amount" of the compound of the present disclosure depends on a variety of factors, including the activity of the specific compound used, the metabolic stability of the compound and the duration of its action, the age, sex and weight of the patient, the patient's current medical condition, the route and duration of administration, the excretion rate, the combined administration of additional drugs, and the progression of the diseases or conditions of the patient being treated. Those skilled in the art will be able to determine appropriate dosages based on these and other factors.

It is to be understood that the choice of using one or more active compounds and/or compositions and their dosage depends on the basic situations of the individual (which should generally render the individual situation to achieve the best effect). Dosing and dosing regimens should be within the ability of those skilled in the art, and the appropriate dosage depends on many factors including the knowledge and ability of the physicians, veterinarians or researchers (see e.g., Jun Li (chief editor), "Clinical Pharmacology", 4th edition, People's Public Health Press, 2008).

As used herein, the term "patient" or "subject" to be treated refers to animal, for example mammal, including but not limited to primate (such as human being), cow, sheep, goat, horse, dog, cat, rabbit, guinea pig, rat, mice, etc.

## VI. Preparation Method

The compound of Formula (II) mentioned below is a sub-embodiment of the compound of Formula (I) of the present disclosure. The present disclosure provides a method for preparing a compound of Formula (II), said method being selected from any one of methods (i), (ii), (iii), (iv), and (v):

the method (i) comprises reacting a compound of Formula (M1) with a compound of Formula (M2) to prepare the compound of Formula (II):

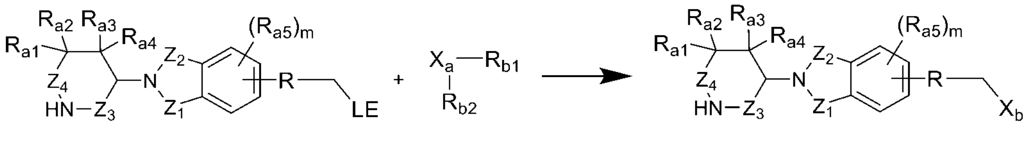

(M1) (M2) (II)

wherein the group LE represents Cl, Br, I, mesyloxy (i.e., OMs), tosyloxy (i.e., OTs), or orthonitrobenzenesulfonyl (i.e., ONs); the group $X_a$ represents NH; the group $R_{b1}$ represents $R_1$; the group $R_{b2}$ represents $R_2$ (i.e., $R_{b1}$-$X_a$-$R_{b2}$ of Formula (M2) is $R_1$-NH-$R_2$); and $X_b$ of the compound of Formula (II) correspondingly represents $NR_1R_2$, wherein the groups $R_1$, $R_2$, $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure;

the method (ii) comprises reacting a compound of Formula (M1) with a compound of Formula (M2) to prepare the

compound of Formula (II):

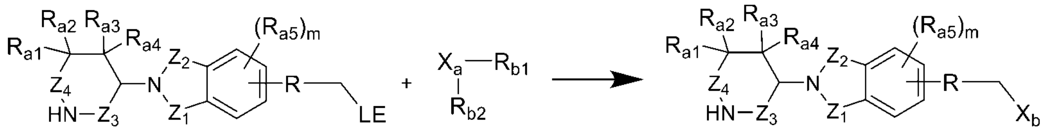

(M1) (M2) (II)

wherein the group LE represents Cl, Br, I, OMs, OTs, or ONs; the group $X_a$ represents NH; the group $R_{b1}$ and the group $R_{b2}$ together with the nitrogen atom of $X_a$ to which they are attached form a nitrogen-containing heterocycle $A_a$ which is substituted by n1 substituent(s) $R_{a1}$, and a substituent represented by the formula of

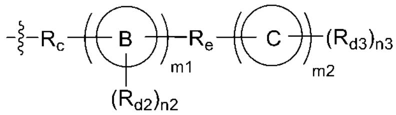

,

i.e., the compound of Formula (M2) has a structure represented by the following formula:

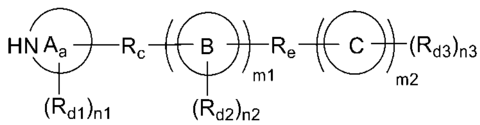

,

and $X_b$ of the compound of Formula (II) correspondingly represents a structure represented by the following general formula:

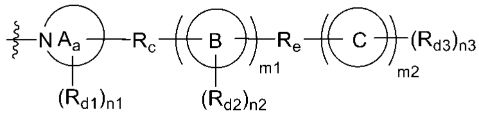

,

wherein the nitrogen-containing heterocycle $A_a$ represents 4- to 30-membered nitrogen-containing heterocyclyl (e.g., 4- to 20-membered nitrogen-containing heterocyclyl, or 4- to 15-membered nitrogen-containing heterocyclyl); and $(R_{d1})_{n1}$, n1, $R_c$, ring B, m1, $(R_{d2})_{n2}$, n2, $R_e$, ring C, m2, $(R_{d3})_{n3}$, n3, $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure;
the method (iii) comprises reacting a compound of Formula (M1) with a compound of Formula (M2) to prepare the compound of Formula (II):

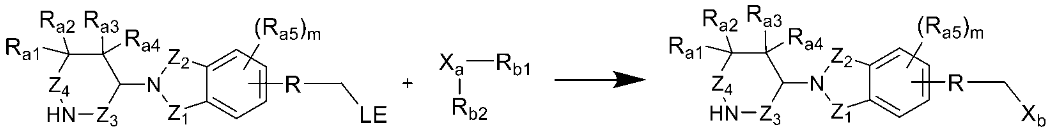

(M1) (M2) (II)

wherein (a) the group LE represents C(O)Cl or COOH; $R_{b1}$-$X_a$-$R_{b2}$ of Formula (M2) represents $R_3$-NH-$R_4$; and $X_b$ of the compound of Formula (II) correspondingly represents $C(O)NR_3R_4$; or
(b) the group LE represents $S(O)_2Cl$; $R_{b1}$-$X_a$-$R_{b2}$ of Formula (M2) represents $R_{10}$-NH-$R_{11}$; and $X_b$ of the compound of Formula (II) correspondingly represents $S(O)_2NR_{10}R_{11}$, and
wherein $R_3$, $R_4$, $R_{10}$, $R_{11}$, $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure;

the method (iv) comprises reacting a compound of Formula (M1) with a compound of Formula (M2) to prepare the compound of Formula (II):

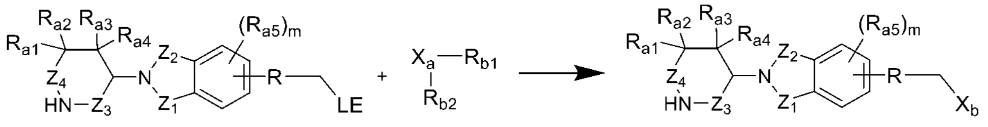

(M1) (M2) (II)

wherein the group LE represents $NH_2$; the group $R_{b1}$-$X_a$ represents $R_5$-C(O) or $R_{13}$-$S(O)_2$; the group $R_{b2}$ represents OH or Cl, (i.e., $R_{b1}$-$X_a$-$R_{b2}$ of Formula (M2) represents: $R_5$-C(O)-OH, $R_5$-C(O)-Cl, $R_{13}$-$S(O)_2$-OH, or $R_{13}$-$S(O)_2$-Cl) ; and $X_b$ of the compound of Formula (II) correspondingly represents $NHC(O)R_5$ or $NHS(O)_2R_{13}$, wherein $R_5$, $R_{13}$, $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure;

the method (v) comprises reacting a compound of Formula (M1) with a compound of Formula (M2) to prepare the compound of Formula (II):

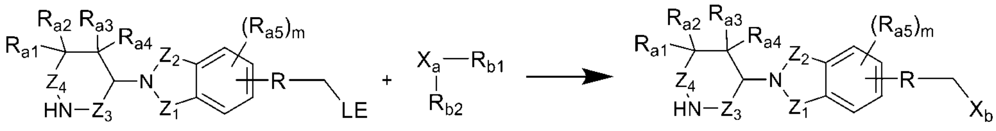

(M1) (M2) (II)

wherein the group LE represents Cl, Br or I; the group $R_{b1}$-$X_a$ represents $R_8$-O or $R_9$-S; the group $R_{b2}$ represents H, (i.e., $R_{b1}$-$X_a$-$R_{b2}$ of Formula (M2) represents: $R_8$-OH or $R_9$-SH); and $X_b$ of the compound of Formula (II) correspondingly represents $OR_8$ or $SR_9$, wherein $R_5$, $R_9$, $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure.

In the method (i) of the present disclosure, the compound of Formula (M2) is represented by the structure of Formula (M2-1) below, and the compound of Formula (II) is represented by the structure of Formula (II-1) below:

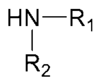

(M2-1)

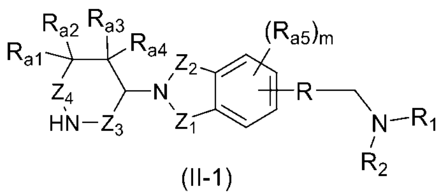

(II-1)

wherein the groups $R_{a1}$, $R_{a2}$, $R_{a3}$, $R_{a4}$, $(R_{a5})_m$, $Z_1$, $Z_2$, $Z_3$, $Z_4$, R, $R_1$, and $R_2$ are as defined in the compound of Formula (I) and its various sub-embodiments in the present disclosure.

In the method (ii) of the present disclosure, the compound of Formula (M2) is represented by the structure of Formula (M2-2) below, and the compound of Formula (II) is represented by the structure of Formula (II-2) below:

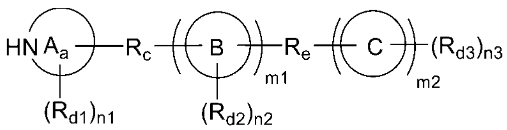

(M2-2)

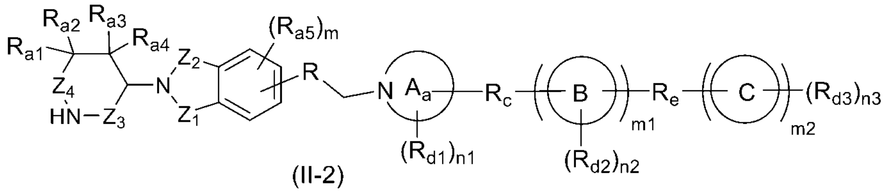

(II-2)

wherein the nitrogen-containing heterocycle $A_a$ represents 4- to 30-membered nitrogen-containing heterocyclyl (e.g., 4- to 20-membered nitrogen-containing heterocyclyl, or 4- to 15-membered nitrogen-containing heterocyclyl); and the groups

$R_{a1}$, $R_{a2}$, $R_{a3}$, $R_{a4}$, $(R_{a5})_m$, $Z_1$, $Z_2$, $Z_3$, $Z_4$, R, $(R_{d1})_{n1}$, n1, $R_c$, ring B, m1, $(R_{d2})_{n2}$, n2, $R_e$, ring C, m2, $(R_{d3})_{n3}$, and n3 are as defined in the compound of Formula (I) and its various sub-embodiments in the present disclosure.

In the method (iii) of the present disclosure, the compound of Formula (M2) is represented by the formula of $R_3$-NH-$R_4$, and the compound of Formula (II) is represented by the structure of Formula (II-3) below:

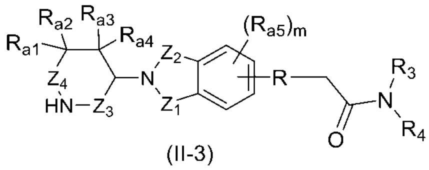

(II-3)

wherein $R_3$, $R_4$, $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure.

In the method (iii) of the present disclosure, the compound of Formula (M2) is represented by the formula of $R_{10}$-NH-$R_{11}$, and the compound of Formula (II) is represented by the structure of Formula (II-4) below:

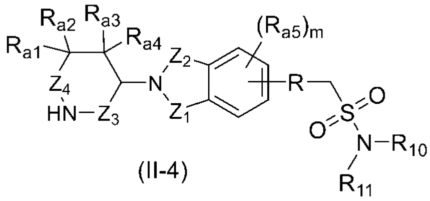

(II-4)

wherein $R_{10}$, $R_{11}$, $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure.

In the method (iv) of the present disclosure, the compound of Formula (M2) can be represented by the formula of $R_5$-C(O)-OH or $R_5$-C(O)-Cl, and the compound of Formula (II) is represented by the structure of Formula (II-5) below:

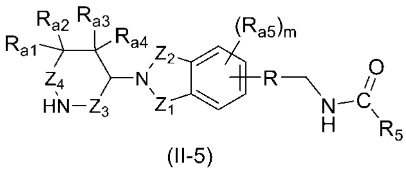

(II-5)

wherein $R_5$, $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure.

In the method (iv) of the present disclosure, the compound of Formula (M2) can be represented by the formula of $R_{13}$-S(O)$_2$-OH or $R_{13}$-S(O)$_2$-Cl, and the compound of Formula (II) is represented by the structure of Formula (II-6) below:

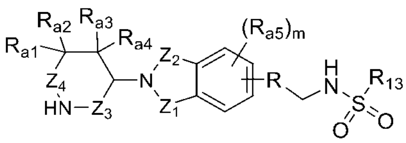

(II-6)

wherein $R_{13}$, $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure.

In the method (v) of the present disclosure, the compound of Formula (M2) can be represented by the formula of $R_8$-OH, and the compound of Formula (II) is represented by the structure of Formula (II-7) below:

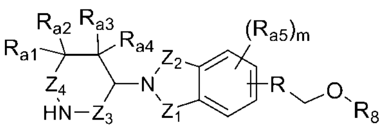

(II-7)

wherein $R_5$, $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure.

In the method (v) of the present disclosure, the compound of Formula (M2) can be represented by the formula of $R_9$-SH, and the compound of Formula (II) is represented by the structure of Formula (II-8) below:

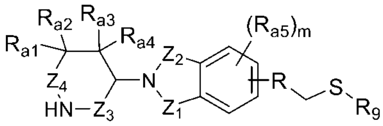

(II-8)

wherein $R_9$, $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure.

In the methods (i) and (ii) of the present disclosure, the compound of Formula (M1) undergoes an aminoalkylation reaction with the compound of Formula (M2). The aminoalkylation reaction may be conducted, for example, in the presence of an organic base and sodium iodide at a temperature ranging from room temperature to 80°C (e.g., 40°C to 60°C, 40°C to 50°C, or 50°C to 60°C). Examples of the organic base include, but are not limited to, DIEA or triethylamine.

The molar ratio of the compound of Formula (M1) to the compound of Formula (M2) may be, for example, 1:1.1-2, 1:1.1-1.5, 1:1.1-1.2, or 1:1.2-1.3.

In some embodiments, when the group LE represents Cl, Br, or I, the compound of Formula (M1) may be prepared by a halogenation reaction of a compound of Formula (M3) with a hydrohalic acid:

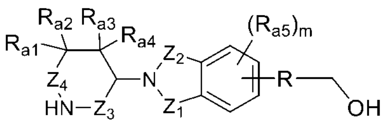

(M3)

wherein the groups $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments in the present disclosure. In some embodiments, R may represent a bond.

In some embodiments, the halogenation reaction can be conducted at room temperature in the presence of, for example, a hydrohalic acid and acetic acid.

In some embodiments, when the group LE represents mesyloxy, the compound of Formula (M1) may be prepared by reacting a compound of Formula (M3) with methanesulfonic anhydride or methanesulfonyl chloride:

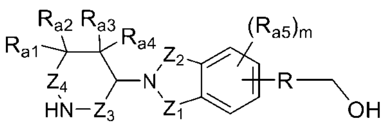

(M3)

wherein the groups $R_{a1}$, $R_{a2}$, $R_{a3}$, $R_{a4}$, $(R_{a5})_m$, R, $Z_1$, $Z_2$, $Z_3$, $Z_4$, and R are as defined in the compound of Formula (I) and its various sub-embodiments in the present disclosure.

In some embodiments, R in the compound of Formula (M1), the compound of Formula (II), and the compound of Formula (M3) represents a bond.

In some embodiments, the compound of Formula (M3) (where R represents a bond) may be prepared by a coupling reaction of a compound of Formula (M4) with (tributylstannyl)methanol catalyzed by a palladium catalyst:

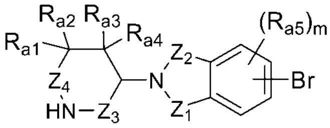

(M4)

wherein the groups $R_{a1}$, $R_{a2}$, $R_{a3}$, $R_{a4}$, $(R_{a5})_m$, $Z_1$, $Z_2$, $Z_3$, and $Z_4$ are as defined in the compound of Formula (I) and its

various sub-embodiments in the present disclosure.

In some embodiments, the coupling reaction can be conducted, for example, in the presence of tetrakis(triphenylphosphine)palladium and 1,4-dioxane (or DMF) at a temperature ranging from 40°C to 120°C.

In some embodiments, the thiocarbonyl compound of Formula (M4) may be prepared by thionation of a carbonyl compound of Formula (M5) with a thionation reagent:

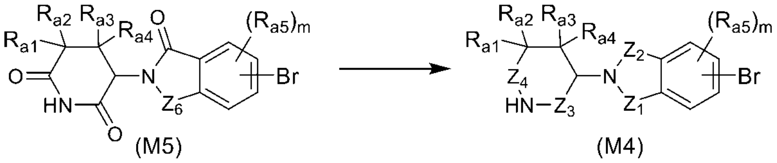

(M5) (M4)

wherein, in the carbonyl compound of Formula (M5), $Z_6$ represents C(O), $CH_2$ or $CD_2$; and in the thiocarbonyl compound of Formula (M4), $Z_1$ represents C(O), C(S), $CH_2$ or $CD_2$; and $Z_2$, $Z_3$, and $Z_4$ each independently represent C(O) or C(S), wherein at least one of $Z_1$, $Z_2$, $Z_3$, and $Z_4$ represents C(S); and the groups $R_{a1}$, $R_{a2}$, $R_{a3}$, $R_{a4}$, and $(R_{a5})_m$ are as defined in the compound of Formula (I) and its various sub-embodiments in the present disclosure. When $Z_6$ in the starting carbonyl compound of Formula (M5) represents $CH_2$ or $CD_2$, the corresponding $Z_1$ in the product thiocarbonyl compound of Formula (M4) represents $CH_2$ or $CD_2$. When $Z_6$ in the starting carbonyl compound of Formula (M5) represents C(O), the corresponding $Z_1$ in the product thiocarbonyl compound of Formula (M4) represents C(O) or C(S).

In some embodiments, examples of the thionation reagent include, but are not limited to, carbon disulfide, hexamethyldisilathiane, sulfur, thiourea, hydrogen sulfide, phosphorus pentasulfide, Lawesson's Reagent (CAS No.:19172-47-5), Belleau's reagent (CAS No.: 88816-02-8), and Davy's reagent (CAS No.: 82737-61-9) etc..

In some embodiments, the carbonyl compound of Formula (M5) is reacted with a thionation reagent (e.g., Lawesson's reagent) in a solvent (e.g., 1,4-dioxane) at a temperature of 40°C to 150°C to prepare the thiocarbonyl compound of Formula (M4).

In some embodiments, at least one of Zi, $Z_2$, $Z_3$, and $Z_4$ in the thiocarbonyl compound of Formula (M4) represents C(S). In some embodiments, at least two of $Z_1$, $Z_2$, $Z_3$, and $Z_4$ in the thiocarbonyl compound of Formula (M4) represent C(S). In some embodiments, at least three of $Z_1$, $Z_2$, $Z_3$, and $Z_4$ represent C(S). In some embodiments, each of Zi, $Z_2$, $Z_3$, and $Z_4$ represents C(S).

In another aspect, the present disclosure provides a method for preparing a compound of Formula (I), comprising preparing the compound of Formula (I) by thionation of a compound of Formula (A) with a thionation reagent:

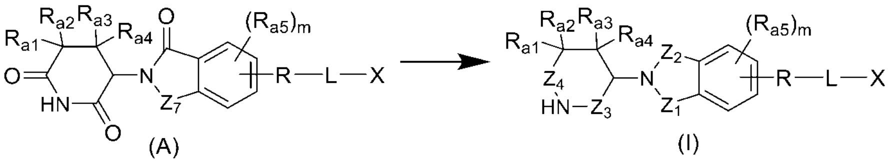

(A) (I)

wherein $Z_7$ of the carbonyl compound of Formula (A) represents C(O), $CH_2$ or $CD_2$;

$Z_1$ of the compound of Formula (I) represents C(O), C(S), $CH_2$ or $CD_2$; $Z_2$, $Z_3$, and $Z_4$ each independently represent C(O) or C(S), wherein at least one of $Z_1$, $Z_2$, $Z_3$, and $Z_4$ represents C(S); and

wherein the groups $R_{a1}$, $R_{a2}$, $R_{a3}$, $R_{a4}$, $(R_{a5})_m$, R, L, and X are as defined in the compound of Formula (I) and its various sub-embodiments in the present disclosure.

When $Z_7$ in the starting compound of Formula (A) represents $CH_2$ or $CD_2$, the corresponding $Z_1$ in the product compound of Formula (I) represents $CH_2$ or $CD_2$. When $Z_7$ in the starting compound of Formula (A) represents C(O), the corresponding $Z_1$ in the product compound of Formula (I) represents C(O) or C(S).

In some embodiments of of the method for preparing the compound of Formula (I), examples of the thionation reagent include, but are not limited to, carbon disulfide, hexamethyldisilathiane, sulfur, thiourea, hydrogen sulfide, phosphorus pentasulfide, Lawesson's Reagent (CAS No.: 19172-47-5), Belleau's reagent (CAS No.: 88816-02-8), and Davy's reagent (CAS No.: 82737-61-9) etc..

In some embodiments of the method for preparing the compound of Formula (I), the compound of Formula (A) is reacted with a thionation reagent (e.g., Lawesson's reagent) in a solvent (e.g., 1,4-dioxane) at a temperature of 40°C to 150°C to prepare the compound of Formula (I).

In some embodiments of the method for preparing the compound of Formula (I), at least one of $Z_1$, $Z_2$, $Z_3$, and $Z_4$ in the compound of Formula (I) represents C(S). In some embodiments, at least two of $Z_1$, $Z_2$, $Z_3$, and $Z_4$ in the compound of Formula (I) represent C(S). In some embodiments, at least three of $Z_1$, $Z_2$, $Z_3$, and $Z_4$ represent C(S). In some

embodiments, each of $Z_1$, $Z_2$, $Z_3$, and $Z_4$ represents C(S).

## VII. Definitions

Unless otherwise specified, the following words, phrases and symbols used herein generally have the meanings as described below.

In general, the nomenclature used herein (including the IUPAC nomenclature) and the laboratory procedures described below (including those used in cell culture, organic chemistry, analytical chemistry, and pharmacology, etc.) are those well-known and commonly used in the art. Unless otherwise defined, all scientific and technical terms used herein in connection with the present disclosure described herein have the same meaning as commonly understood by one skill in the art. In addition, the use of the word "a" or "an" when used in conjunction with the term "comprising" or a noun in the claims and/or the specification may mean "one", but it is also consistent with the meaning of "one or more", "at least one", and "one or more than one". Similarly, the terms "another" or "other" can mean at least a second or more.

It should be understood that whenever the term "comprise" or "include" is used herein to describe various aspects, other similar aspects described by "consisting of" and/or "consisting essentially of" are also provided.

As used herein, the term "about" used alone or in combination refers to approximately, roughly, nearly, or around. When the term "about" is used in conjunction with a numerical range, it modifies that range by extending the boundaries above and below the stated numerical value. In general, the term "about" can modify a numerical value above and below the stated value by an upward or downward (increasing or decreasing) variation, e.g., 10%, 5%, 2%, or 1%.

As used herein, the wording "...represents a bond" used alone or in combination means that the referenced group is a bond linker (that is, the referenced group is absent). For example, the wording "R represents a bond" means that R is a bond linker. In other words, when R represents a bond, the L in the structure of Formula (I) is directly connected to the benzene ring of the benzo-fused ring in the structure of Formula (I). For example, the wording "$R_{w2}$ represents a bond" means that $R_{w2}$ is a bond linker. In other words, when $R_{w2}$ represents a bond, ring $A^5$ in the group L is directly linked to X.

In this document, the term "optionally substituted" used alone or in combination means that the referenced group may be unsubstituted or substituted with one or more substituents as defined here. Herein, the wording "optionally substituted with..." and "unsubstituted or substituted" can be used interchangeably. The term "substituted" generally represents the replacement of one or more hydrogen atoms in the referenced structure by identical or different specific substituents. The number of substituents is not theoretically limited in any way, or is automatically limited by the size of the building units (i.e., the total number of replaceable hydrogen atoms in the building units), or as clearly defined herein.

Herein, a bond interrupted by a wavy line shows the point of attachment of the depicted group to the rest of the molecule. For example, the monovalent group X depicted below

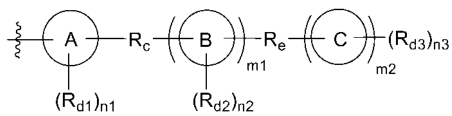

shows the point of attachment of ring A in said group X to group L in the structure of Formula (I). Herein, unless otherwise expressly specified, the two bonds interrupted by wavy lines in a depicted divalent group show the two points of attachment of the depicted group to the rest of the molecule. For example, in the structure of group L depicted below,

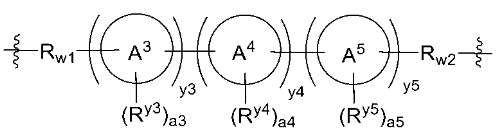

,

as defined herein, $R_{w1}$ is connected to R, and $R_{w2}$ is connected to X. For instance, the group represented by $R_{w1}$ is $-O-CH_2-$, either end of which may be connected to R of the compound of Formula (I), while the other end is connected to ring $A^3$. In other words, in the group $-O-CH_2-$ represented by $R_{w1}$, the O atom may be connected to R of the compound of Formula (I), and the $CH_2$ may be connected to ring $A^3$ of the compound of Formula (I), or vice versa. As another example, the divalent group R depicted below

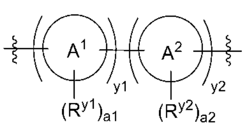

may have either end connected to the isoindolinyl ring of the compound of Formula (I), while the other end is connected to group L. In other words, ring $A^1$ of the divalent group R may be connected to the isoindolinyl ring of the compound of Formula (I), and the other end may be connected to group L, or vice versa.

As used herein, the term "replaced" in the expression "any one or more methylene groups in the main carbon chain skeleton of a linear or branched $C_{1-20}$ alkylene are optionally be replaced by one or more groups selected from: $R_a$, $R_b$, and any combination thereof" used alone or in combination, has a definition known in the art, namely it may refer to one or more groups $R_a$, $R_b$, or any combination of groups $R_a$ and $R_b$ optionally replacing any one or more methylene groups in the main carbon chain skeleton of a $C_{1-20}$ alkylene. Herein, examples of the above-mentioned expression may include, but are not limited to, that any one or more (e.g., 1-20 or 1-15, 1-10, 1-9, 1-8, 1-7, 1-6, 1-5, 1-4, 1-3, or 1-2, or 1) methylene groups in the main carbon chain skeleton of a $C_{1-20}$ alkylene optionally are replaced by one or more (e.g., 1-20 or 1-15, 1-10, 1-9, 1-8, 1-7, 1-6, 1-5, 1-4, 1-3, or 1-2, or 1) groups $R_a$ as defined herein, and/or one or more (e.g., 1-20 or 1-15, 1-10, 1-9, 1-8, 1-7, 1-6, 1-5, 1-4, 1-3, or 1-2, or 1) groups $R_b$ as defined herein, and/or one or more (e.g., 1-20 or 1-15, 1-10, 1-9, 1-8, 1-7, 1-6, 1-5, 1-4, 1-3, or 1-2, or 1) of any combination of groups $R_a$ and $R_b$, and the resulting main chain skeleton group conforms to the covalent bond theory. When two or more methylene groups in the main carbon chain skeleton of the linear or branched $C_{1-20}$ alkylene are replaced by two or more groups $R_a$, the respective groups $R_a$ are not directly connected to each other. When any one or more methylene groups in the main carbon chain skeleton of the linear or branched $C_{1-20}$ alkylene are replaced by one or more groups $R_a$, and/or one or more groups $R_b$, and/or one or more of any combination of groups $R_a$ and $R_b$, the resulting main chain skeleton group may contain one or more (e.g., 1-20 or 1-15, 1-10, 1-9, 1-8, 1-7, 1-6, 1-5, 1-4, 1-3, or 1-2, or 1) of the following fragments, such as but not limited to: "$-(CH_2)_{0-5}-R_a-(CH_2)_{0-5}-$" fragment, "$-R_a-(CH_2)_{1-5}-R_a-$" fragment, "$-(CH_2)_{0-5}-(R_b)_{0-5}-(CH_2)_{0-5}-$" fragment, "$-(R_b)_{0-5}-(CH_2)_{1-5}-(R_b)_{0-5}-$" fragment, "$-(CH_2)_{0-5}-R_a-(R_b)_{0-5}-(CH_2)_{0-5}-$" fragment, "$-R_a-(CH_2)_{0-5}-(R_b)_{0-5}-(CH_2)_{0-5}-$" fragment, "$-(CH_2)_{0-5}-R_a-(CH_2)_{0-5}-(R_b)_{0-5}-$" fragment etc., wherein each $R_a$ is the same or different, each $R_b$ is the same or different, and as defined herein.

As used herein, the expression "one or more hydrogens of the $C_{x-y}$ alkylene are replaced by...", used alone or in combination, means that any one or more hydrogens of the linear or branched $C_{x-y}$ alkylene are replaced by a substituent(s) as defined herein. In other words, the $C_{x-y}$ alkylene is optionally substituted with one or more substituents as defined herein. Herein, the term "one or more" may refer to part or all hydrogen atoms of each referenced alkylene group, including but not limited to 1-40 hydrogens, e.g., 1-30, such as 1-25, 1-20, 1-15, 1-10, 1-5, 1-4, 1-3, 1-2 or 1 hydrogen atoms. The number of hydrogens to be replaced is in principle not limited in any way, or is automatically limited by the size of the building unit.

As used herein, the term "deuterated" used alone or in combination, indicates that one or more hydrogen atoms in the referenced group are replaced by deuterium atoms.

As used herein, the term "oxo" or "oxo group" used alone or in combination, refers to =O.

As used herein, the term "C(O)" or "C(=O)" used alone or in combination, refers to a carbonyl group.

As used herein, the term "C(S)" or "C(=S)" used alone or in combination, refers to a thiocarbonyl group.

As used herein, the term "halogen atom" or "halogen", used alone or in combination, refers to fluorine, chlorine, bromine, or iodine.

As used herein, the term "alkyl", used alone or in combination, refers to a linear or branched alkyl group. The term "$C_x$-$C_y$ alkyl" or "$C_{x-y}$ alkyl" (x and y each being an integer) refers to a linear or branched alkyl group containing from x to y carbon atoms. The term "$C_{1-10}$ alkyl" used alone or in combination in the present disclosure refers to a linear or branched alkyl group containing from 1 to 10 carbon atoms. Examples of the $C_{1-10}$ alkyl of the present disclosure may include a $C_{1-9}$ alkyl, $C_{1-8}$ alkyl, $C_{2-8}$ alkyl, $C_{1-7}$ alkyl, $C_{1-6}$ alkyl, $C_{1-5}$ alkyl, $C_{1-4}$ alkyl, $C_1$-$C_3$ alkyl, and $C_1$-$C_2$ alkyl. Representative examples include methyl, ethyl, n-propyl, isopropyl, n-butyl, isobutyl, sec-butyl, tert-butyl, pentyl, isopentyl, neopentyl, tert-pentyl, and hexyl. The term "$C_{1-3}$ alkyl" or "$C_1$-$C_3$ alkyl" in the present disclosure refers to an alkyl group containing from 1 to 3 carbon atoms, and representative examples thereof include methyl, ethyl, n-propyl, and isopropyl. In the present disclosure, the "alkyl" is optionally substituted with one or more substituents optionally selected from the group consisting of halogen, hydroxyl, cyano, $C_{1-3}$ alkyl, $C_{1-3}$ alkoxy, halogenated $C_{1-6}$ alkoxy, halogenated $C_{1-6}$ alkyl (e.g., trifluoromethyl), $C_{3-6}$ cycloalkyl, 4- to 7-membered heterocyclyl, or a combination thereof.

As used herein, the term "halogenated alkyl", used alone or in combination, refers to a linear or branched alkyl group substituted with one or more halogens, wherein one or more hydrogen atom(s) of the alkyl group are replaced with one or more halogens. The term "halogenated $C_x$-$C_y$ alkyl" or "halogenated $C_{x-y}$ alkyl" (x and y are each an integer) refers to a linear or branched alkyl containing from x to y carbon atoms substituted with one or more halogens. The term "halogenated $C_{1-10}$ alkyl" used alone or in combination in the present disclosure refers to a linear or branched alkyl group containing from 1 to 10 carbon atoms substituted with one or more halogens. Examples of the halogenated $C_{1-10}$ alkyl group of the present disclosure include halogenated $C_{1-9}$ alkyl group, e.g., halogenated $C_{1-8}$ alkyl group, halogenated $C_{2-8}$ alkyl group, halogenated $C_{1-7}$ alkyl group, halogenated $C_{1-6}$ alkyl, halogenated $C_{1-5}$ alkyl, or halogenated $C_{1-4}$ alkyl. Representative examples include halomethyl, haloethyl, halo-n-propyl, haloisopropyl, halo-n-butyl, haloisobutyl, halo-sec-butyl, halo-tert-butyl, halopentyl, haloisoamyl, haloneopentyl, halo-tert-pentyl, halohexyl, haloheptyl, halooctyl, halononyl, and halodecyl. The term "halogenated $C_{1-3}$ alkyl" or "halogenated $C_1$-$C_3$ alkyl" of the present disclosure

refers to an alkyl group containing from 1 to 3 carbon atoms substituted with one or more halogens, and its representative examples include halomethyl (e.g., trifluoromethyl), haloethyl, halo-n-propyl and haloisopropyl.

As used herein, the term "deuterated alkyl", used alone or in combination, refers to a linear or branched alkyl group substituted with one or more deuterium atoms, wherein one or more hydrogen atoms of the alkyl group are replaced with one or more deuterium atoms. The term "deuterated $C_{x-}C_y$ alkyl" or "deuterated $C_{x-y}$ alkyl" (x and y are each an integer) refers to a linear or branched alkyl containing from x to y carbon atoms substituted with one or more deuterium atoms. The term "deuterated $C_{1-10}$ alkyl" used alone or in combination in the present disclosure refers to a linear or branched alkyl group containing from 1 to 10 carbon atoms substituted with one or more deuterium atoms. Examples of the deuterated $C_{1-10}$ alkyl group of the present disclosure include deuterated $C_{1-9}$ alkyl group, e.g., deuterated $C_{1-4}$ alkyl group, deuterated $C_{2-8}$ alkyl group, deuterated $C_{1-7}$ alkyl group, deuterated $C_{1-8}$ alkyl, deuterated $C_{1-5}$ alkyl, or deuterated $C_{1-4}$ alkyl. Representative examples include perdeuterated methyl ($CD_3$), perdeuterated ethyl ($CD_3CD_2$), perdeuterated n-propyl, perdeuterated isopropyl, perdeuterated n-butyl, perdeuterated isobutyl, perdeuterated sec-butyl, perdeuterated tert-butyl, perdeuterated pentyl, perdeuterated isopentyl, perdeuterated neopentyl, perdeuterated tert-pentyl, and perdeuterated hexyl. The term "deuterated $C_{1-3}$ alkyl" or "deuterated $C_{1-}C_3$ alkyl" of the present disclosure refers to an alkyl group containing from 1 to 3 carbon atoms substituted with one or more deuterium atoms, and its representative examples include perdeuterated methyl ($CD_3$) and perdeuterated ethyl ($CD_3CD_2$).

As used herein, the term "alkylene" (which is used interchangeably with "alkylene chain"), used alone or in combination, refers to a linear or branched divalent saturated hydrocarbon group composed of carbon and hydrogen atoms, which is the divalent form of a linear or branched alkyl group. The term "$C_x$-$C_y$ alkylene" or "$C_{x-y}$ alkylene" (x and y each being an integer) refers to a linear or branched alkylene group containing from x to y carbon atoms. The term "$C_1$-$C_{20}$ alkylene" as used herein, whether alone or in combination, refers to a linear or branched alkylene containing 1 to 20 carbon atoms, examples of which include, but are not limited to, $C_1$-$C_{20}$ alkylene, $C_1$-$C_{15}$ alkylene, $C_1$-$C_{14}$ alkylene, $C_1$-$C_{13}$ alkylene, $C_1$-$C_{12}$ alkylene, $C_1$-$C_{11}$ alkylene, $C_1$-$C_{10}$ alkylene, $C_1$-$C_9$ alkylene, $C_1$-$C_8$ alkylene, $C_1$-$C_7$ alkylene, $C_1$-$C_6$ alkylene, $C_1$-$C_5$ alkylene, $C_1$-$C_4$ alkylene, $C_1$-$C_3$ alkylene, and $C_1$-$C_2$ alkylene. Representative examples include, but are not limited to, methylene (i.e., $-CH_2-$), ethylene (e.g., $-CH_2CH_2-$), propylene, isopropylene, butylene, isobutylene, sec-butylene, tert-butylene, n-pentylene, isopentylene, neopentylidene, tert-pentylene, hexylene, heptylene, octylene, nonylene, decylene, undecylene, dodecylene, tridecylene, tetradecylene, and pentadecylene. In the present disclosure, the "alkylene" is optionally substituted with one or more substituents optionally selected from $C_1$-$C_3$ alkyl, $C_{3-6}$ cycloalkyl, hydroxyl, amino, mercapto, halogen, $C_1$-$C_3$ alkoxy, $C_1$-$C_3$ alkylamino, halogenated $C_1$-$C_3$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "alkoxy", used alone or in combination, refers to a linear or branched alkoxy group having structural formula of alkyl-O-. Optionally, the alkyl portion of the alkoxy group may contain 1-10 (e.g., 1-6, 1-4, or 1-3) carbon atoms. Representative examples of "alkoxy" include, but are not limited to, methoxy, ethoxy, propoxy, isopropoxy, n-butoxy, isobutoxy, tert-butoxy, pentyloxy, 2-pentyloxy, isopentyloxy, neopentyloxy, hexyloxy, 2-hexyloxy, 3-hexyloxy, 3-methylpentyloxy, etc. The term "$C_1$-$C_3$ alkoxy" or "$C_{1-3}$ alkoxy" refers to a linear or branched alkoxy group containing from 1 to 3 carbon atoms. Representative examples of $C_{1-3}$ alkoxy include, but are not limited to, methoxy, ethoxy, n-propoxy, and isopropoxy.

As used herein, the term "halogenated alkoxy", used alone or in combination, refers to an alkoxy group substituted with one or more halogen atoms. Optionally, the alkyl portion of the alkoxy group may contain 1-10 (e.g., 1-6, 1-4, or 1-3) carbon atoms. Examples of "halogenated alkoxy" include halogenated $C_{1-6}$ alkoxy or halogenated $C_{1-4}$ alkoxy. Representative examples include, but are not limited to, $F_3C$-O-, $FCH_2$-O-, $F_2CH$-O-, $ClCH_2$-O-, $Cl_2CH$-O-, $CF_3CF_2$-O-, $CF_3CHF$-O-, $CHF_2CF_2$-O-, $CHF_2CHF$-O-, $CF_3CH_2$-O- or $CH_2ClCH_2$-O-.

As used herein, the term "heteroaryl", used alone or in combination, refers to a 5- to 30-membered (e.g., 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, 6- to 9-membered, or 6- to 20-membered) monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroaryl groups include bicyclic, tricyclic or tetracyclic heteroaryl groups, which contain one aromatic ring having one or more heteroatoms independently selected from O, S and N, and the remaining rings which may be a saturated, partially unsaturated or aromatic ring and can be carbocyclic ring or contain one or more heteroatoms independently selected from O, S and N. Examples of monocyclic heteroaryl groups include, but are not limited to, furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, tetrazolyl, and triazinyl. Examples of bicyclic heteroaryl groups include, but are not limited to, indolyl, isoindolyl, isoindolinyl, benzofuranyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, quinolinyl, isoquinolinyl, naphthyridinyl, cinnolinyl, quinazolinyl, quinoxalinyl, phthalazinyl, oxazolopyridyl, furopyridyl, pteridyl, purinyl, pyridopyridyl, pyrazolo[1,5-a]pyridyl, pyrazolo[1,5-a]pyrimidinyl, imidazo[1,2-a]pyridyl, 1H-pyrrolo[3,2-b]pyridyl, 1H-pyrrolo[2,3-b]pyridyl, pyrrolo[2,1-b]thiazolyl, imidazo[2,1-b]thiazolyl, chromanyl, and 6,7-dihydrothieno[3,2-d]pyrimidinyl. Examples of tricyclic or polycyclic heteroaryl groups include, but are not limited to, acridinyl, benzindolyl, carbazolyl, dibenzofuranyl, xanthyl, 5,6,7,8-tetrahydrobenzo[4,5]thieno[2,3-d]pyrimidinyl, and 6,7-dihydro-5H-cyclopenta[4,5]thieno[2,3-d]pyrimidinyl. The heteroaryl group may be unsubstituted or substituted. A substituted heteroaryl refers to heteroaryl substituted one or more times (e.g., 1-4, 1-3, or 1-2 times) by a substituent(s) optionally selected from the group consisting of $C_1$-$C_3$ alkyl, $C_{3-6}$ cycloalkyl, hydroxy, amino, mercapto, halogen, $C_1$-$C_3$ alkoxy, $C_{1-3}$ alkylamino, halogenated $C_1$-$C_3$ alkyl, amino-substituted $C_{1-3}$ alkylene, $C_{1-3}$ alkyl-NHC(O)-, $C_{1-3}$ alkyl-C(O)NH-, cyano, and any combination thereof.

As used herein, the term "heteroarylene", used alone or in combination, refers to a 5- to 30-membered (e.g., 5- to 20-membered, 5- to 15-membered, 5- to 12-membered, 5- to 11-membered, 5- to 10-membered, 5- to 9-membered, 5- to 8-membered, 5- to 7-membered, 5- to 6-membered, 6- to 15-membered, 6- to 9-membered, or 6- to 20-membered) bivalent monocyclic, bicyclic, or polycyclic cyclic hydrocarbon radical containing at least one aromatic ring having one or more (e.g., from 1 to 6, or from 1 to 5, or from 1 to 4, or from 1 to 3) heteroatoms independently selected from the group consisting of oxygen, nitrogen, and sulfur. Bicyclic or polycyclic heteroarylene groups include bicyclic, tricyclic or tetracyclic heteroarylene groups, which contain one aromatic ring having one or more heteroatoms independently selected from O, S and N, and the remaining ring(s) which may be a saturated, partially unsaturated or aromatic ring and can be carbocyclic ring or contain one or more heteroatoms independently selected from O, S and N. Examples of monocyclic heteroarylene groups include, but are not limited to, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, tetrazolylene, and triazinylene. Examples of bicyclic heteroarylene groups include, but are not limited to, indolylene, isoindolylene, isoindolinylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, oxazolopyridylene, furopyridylene, pteridylene, purinylene, pyridopyridylene, pyrazolo[1,5-a]pyridylene, pyrazolo [1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b] thiazolylene, imidazo[2,1-b]thiazolylene, chromanylene, and 6,7-dihydrothieno[3,2-d]pyrimidinylene. Examples of tricyclic or polycyclic heteroarylene groups include, but are not limited to, acridinylene, benzindolylene, carbazolylene, dibenzofuranylene, xanthylene, 5,6,7,8-tetrahydrobenzo[4,5]thieno[2,3-d]pyrimidinylene, and 6,7-dihydro-5H-cyclopenta[4,5]thieno[2,3-d]pyrimidinylene. The heteroarylene group may be unsubstituted or substituted. A substituted heteroarylene refers to heteroarylene substituted one or more times (e.g., 1-4, 1-3, or 1-2 times) by a substituent(s) optionally selected from the group consisting of $C_1$-$C_3$ alkyl, $C_{3-6}$cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_{1-3}$alkylene, $C_{1-3}$ alkyl-NHC(O)-, $C_{1-3}$ alkyl-C(O)NH-, cyano, and any combination thereof.

As used herein, the term "aryl", used alone or in combination, refers to a monovalent aromatic hydrocarbon group containing from 5 to 30 (e.g., from 5 to 20, from 5 to 15, from 5 to 12, from 5 to 10, from 5 to 9, from 5 to 8, from 5 to 7, from 5 to 6, from 6 to 15, from 6 to 9, or from 6 to 20) carbon atoms and optionally one or more fused rings, such as phenyl group, naphthyl group, or fluorenyl group. As used herein, the "aryl" is optionally substituted. A substituted aryl group refers to an aryl group substituted one or more times (e.g., 1-4, 1-3, or 1-2 times) with a substituent(s). For example, aryl is mono-, di-, tri-, or poly-substituted with a substituent(s) optionally selected from e.g., $C_1$-$C_3$ alkyl, $C_{3-6}$ cycloalkyl, hydroxyl, amino, mercapto, halogen, $C_1$-$C_3$ alkoxy, $C_1$-$C_3$ alkylamino, halogenated $C_1$-$C_3$ alkyl, amino-substituted $C_{1-3}$ alkylene, $C_{1-3}$ alkyl-NHC(O)-, $C_{1-3}$ alkyl-C(O)NH-, cyano or any combination thereof.

As used herein, the term "arylene", used alone or in combination, refers to a divalent aromatic hydrocarbon group containing from 5 to 30 (e.g., from 5 to 20, from 5 to 15, from 5 to 12, from 5 to 10, from 5 to 9, from 5 to 8, from 5 to 7, from 5 to 6, from 6 to 15, from 6 to 9, or from 6 to 20) carbon atoms and optionally one or more fused rings, such as phenylene (e.g.,

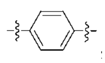

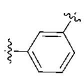,

and

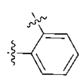

), naphthylene, or fluorenylene. As used herein, the "arylene" is optionally substituted. A substituted arylene refers to an arylene group substituted one or more times (e.g., 1-4, 1-3, or 1-2 times) with a substituent(s). For example, arylene is mono-, di-, tri-, or poly-substituted with a substituent(s) optionally selected from e.g., $C_1$-$C_3$ alkyl, $C_{3-6}$ cycloalkyl, hydroxyl, amino, mercapto, halogen, $C_1$-$C_3$ alkoxy, $C_1$-$C_3$ alkylamino, halogenated $C_1$-$C_3$ alkyl, amino-substituted $C_{1-3}$ alkylene, $C_{1-3}$ alkyl-NHC(O)-, $C_{1-3}$ alkyl-C(O)NH-, cyano, or any combination thereof.

As used herein, the term "cycloalkyl", used alone or in combination, refers to a saturated or partially unsaturated (i.e., containing one or more double bonds, but not having a fully conjugated $\pi$-electron system) monocyclic or bicyclic or tricyclic or polycyclic cyclic hydrocarbon radical, which in some embodiments contains from 3 to 30 carbon atoms (i.e., $C_{3-30}$ cycloalkyl), or from 3 to 25 carbon atoms (i.e., $C_{3-25}$ cycloalkyl), or from 3 to 20 carbon atoms (i.e., $C_{3-20}$ cycloalkyl), or from 3 to 15 carbon atoms (i.e., $C_{3-15}$ cycloalkyl), or from 3 to 12 carbon atoms (i.e., $C_{3-12}$ cycloalkyl), or from 3 to 11 carbon atoms (i.e., $C_{3-11}$ cycloalkyl), or from 3 to 10 carbon atoms (i.e., $C_{3-10}$ cycloalkyl), or from 3 to 8 carbon atoms ( i.e., $C_{3-8}$ cycloalkyl), or from 3 to 7 carbon atoms (i.e., $C_{3-7}$ cycloalkyl), or from 3 to 6 carbon atoms (i.e., $C_{3-6}$ cycloalkyl), or from 4 to 20 carbon atoms ( i.e., $C_{4-20}$ cycloalkyl), or from 4 to 15 carbon atoms ( i.e., $C_{4-15}$ cycloalkyl), or from 4 to 12 carbon atoms (i.e., $C_{4-12}$ cycloalkyl), or from 4 to 10 carbon atoms (i.e., $C_{4-10}$ cycloalkyl). The term "cycloalkyl" includes monocyclic, bicyclic, tricyclic, and polycyclic cyclic hydrocarbon radical having from 3 to 30 carbon atoms. Examples of the term "cycloalkyl" include, but are not limited to, monocyclic cycloalkyl, bridged cycloalkyl (e.g., $C_{5-30}$ bridged cycloalkyl or $C_{5-20}$ bridged cycloalkyl or $C_{5-15}$ bridged cycloalkyl or $C_{7-15}$ bridged cycloalkyl), fused cycloalkyl (e.g., $C_{5-30}$ fused cycloalkyl, $C_{5-20}$ fused cycloalkyl, $C_{5-15}$ fused cycloalkyl, $C_{6-30}$ fused cycloalkyl, $C_{6-20}$ fused cycloalkyl, $C_{6-15}$ fused cycloalkyl, $C_{7-30}$ fused cycloalkyl, $C_{7-20}$ fused cycloalkyl, $C_{7-15}$ fused cycloalkyl, and $C_{8-15}$ fused cycloalkyl), and spiro-cycloalkyl (e.g., $C_{5-30}$ spiro-cycloalkyl, $C_{5-20}$ spiro-cycloalkyl, $C_{5-15}$ spiro-cycloalkyl, $C_{6-30}$ spiro-cycloalkyl, $C_{6-20}$ spiro-cycloalkyl, $C_{6-15}$ spiro-cycloalkyl, $C_{7-30}$ spiro-cycloalkyl, $C_{7-20}$ spiro-cycloalkyl, $C_{7-15}$ spiro-cycloalkyl, and $C_{8-15}$ spiro-cycloalkyl). Representative examples of monocyclic cycloalkyl groups include, but are not limited to, cyclopropyl, cyclobutyl, cyclopentyl, cyclopentenyl, cyclohexyl, cyclohexenyl, cycloheptyl, and cyclooctyl. Examples of bridged cycloalkyl, fused cycloalkyl and spiro-cycloalkyl groups include, but are not limited to, decalinyl, octahydropentalenyl, octahydro-1H-indenyl, $C_{5-20}$ spiro-cycloalkyl, $C_{5-15}$ spiro-cycloalkyl, adamantanyl, noradamantanyl, bornyl, and norbornyl (also named as bicyclo [2.2.1]heptyl by the IUPAC system). As used herein, the "cycloalkyl" is optionally mono- or poly-substituted, such as, but not limited to, 2,2-, 2,3-, 2,4-, 2,5-, or 2,6-disubstituted cyclohexyl. The substituents of the substituted "cycloalkyl" can be optionally one or more (e.g., 1-5, 1-4, 1-3, 1-2, or 1) substituents selected from $C_1$-$C_3$ alkyl, $C_{3-6}$ cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_{1-3}$ alkylene, $C_{1-3}$ alkyl-NHC(O)-, $C_{1-3}$ alkyl-C(O)NH-, cyano, and any combination thereof. Examples of "$C_{3-6}$ cycloalkyl" include, but are not limited to, cyclopropyl, cyclobutyl, cyclopentyl,, cyclopentenyl, and cyclohexyl.

As used herein, the term "$C_{x-y}$ spiro-cycloalkyl" (x and y each being an integer), used alone or in combination, refers to a spiro-cycloalkyl group containing from x to y carbon atoms. As used herein, the term "$C_{5-30}$ spiro-cycloalkyl", used alone or in combination, refers to a spiro-cycloalkyl group containing from 5 to 30 carbon atoms (e.g., but not limited to, 5-20, 5-15, 7-20, 7-15, 5-11, 5-10, and 7-9 carbon atoms). The term "$C_{5-30}$ spirocycloalkyl" includes "$C_{5-20}$ spirocycloalkyl", "$C_{5-15}$ spirocycloalkyl", "$C_{7-15}$ spirocycloalkyl" and "$C_{7-20}$ spirocycloalkyl", and representative examples of which include, but are not limited to, spiro[3.3]heptanyl, spiro[2.5]octanyl, spiro[3.5]nonanyl, spiro[4.4]nonanyl, spiro[4.5]decanyl, and spiro[5.5]undecanyl. The "$C_{5-30}$ spiro-cycloalkyl" is optionally further substituted with one or more substituents selected from the group consisting of $C_{1-3}$ alkyl, halogenated $C_{1-3}$ alkyl, $C_{1-3}$ alkoxy, deuterated $C_{1-3}$ alkoxy, $C_{1-3}$ alkylamino, amino, oxo, halogen, hydroxy, cyano, and any combination thereof.

As used herein, the term "$C_{x-y}$ bridged cycloalkyl" (where x and y are integers) used alone or in combination, refers to a bridged cycloalkyl group containing x to y carbon atoms. In the present invention, the term "$C_{5-30}$ bridged cycloalkyl" used alone or in combination, refers to a bridged cycloalkyl group containing from 5 to 30 carbon atoms (e.g., but not limited to, 5-20, 6-20, 7-20, 5-15, 7-15, 5-11, 5-10, and 7-9 carbon atoms). The term "$C_{5-30}$ bridged cycloalkyl" includes "$C_{5-20}$ bridged cycloalkyl", "$C_{6-20}$ bridged cycloalkyl", "$C_{7-20}$ bridged cycloalkyl", "$C_{5-15}$ bridged cycloalkyl", and "$C_{7-15}$ bridged cycloalkyl", and representative examples of which include, but are not limited to, adamantanyl, noradamantanyl, bornyl, norbomanyl (systematically named bicyclo[2.2.1]heptanyl), 2-oxobicyclo[2.2.1]heptanyl, bicyclo[2.2.1]heptenyl, and cubanyl. Said " $C_{5-30}$ bridged cycloalkyl" may be optionally substituted with 1 to 10 substituents selected from: $C_{1-3}$ alkyl, deuterated $C_{1-3}$ alkyl, $C_{1-3}$ alkoxy, halogen, halogenated $C_{1-3}$ alkyl, $C_{1-3}$ alkylamino, amino, hydroxy, cyano, oxo, and any combination thereof.

As used herein, the term "cycloalkylene", used alone or in combination, refers to a saturated or partially unsaturated (i.e., containing one or more double bonds, but not having a fully conjugated $\pi$-electron system) monocyclic, bicyclic, tricyclic or polycyclic divalent cyclic hydrocarbon radical, which in some embodiments contains from 3 to 30 carbon atoms (i.e., $C_{3-30}$ cycloalkylene), or from 3 to 25 carbon atoms (i.e., $C_{3-25}$ cycloalkylene), from 3 to 20 carbon atoms (i.e., $C_{3-20}$ cycloalkylene), or from 3 to 15 carbon atoms (i.e., $C_{3-15}$ cycloalkylene), or from 3 to 12 carbon atoms (i.e., $C_{3-12}$ cycloalkylene), or from 3 to 11 carbon atoms (i.e., $C_{3-11}$ cycloalkylene), or from 3 to 10 carbon atoms (i.e., $C_{3-10}$ cycloalkylene), or from 3 to 8 carbon atoms ( i.e., $C_{3-8}$ cycloalkylene), or from 3 to 7 carbon atoms (i.e., $C_{3-7}$ cycloalkylene), or from 3 to 6 carbon atoms (i.e., $C_{3-6}$ cycloalkylene), or from 4 to 20 carbon atoms (i.e., $C_{4-20}$ cycloalkylene), or from 4 to 15

carbon atoms (i.e., $C_{4-15}$ cycloalkylene), or from 4 to 12 carbon atoms (i.e., $C_{4-12}$ cycloalkylene), or from 4 to 10 carbon atoms (i.e., $C_{4-10}$ cycloalkylene). The term "cycloalkylene" includes monocyclic, bicyclic, tricyclic and polycyclic divalent cyclic hydrocarbon radical having from 3 to 30 carbon atoms. Examples of the term "cycloalkylene" include, but are not limited to, monocyclic cycloalkylene, bridged cycloalkylene (e.g., $C_{5-30}$ bridged cycloalkylene, $C_{5-20}$ bridged cycloalkylene, $C_{5-15}$ bridged cycloalkylene or $C_{7-15}$ bridged cycloalkylene), fused cycloalkylene (e.g., $C_{5-30}$ fused cycloalkylene, $C_{5-20}$ fused cycloalkylene, $C_{5-15}$ fused cycloalkylene, $C_{6-30}$ fused cycloalkylene, $C_{6-20}$ fused cycloalkylene, $C_{6-15}$ fused cycloalkylene, $C_{7-30}$ fused cycloalkylene, $C_{7-20}$ fused cycloalkylene, $C_{7-15}$ fused cycloalkylene, and $C_{8-15}$ fused cycloalkylene), and spiro-cycloalkylene (e.g., $C_{5-30}$ spiro-cycloalkylene, $C_{5-20}$ spiro-cycloalkylene, $C_{5-15}$ spiro-cycloalkylene, $C_{6-30}$ spiro-cycloalkylene, $C_{6-20}$ spiro-cycloalkylene, $C_{6-15}$ spiro-cycloalkylene, $C_{7-30}$ spiro-cycloalkylene, $C_{7-20}$ spiro-cycloalkylene, $C_{7-15}$ spiro-cycloalkylene, and $C_{8-15}$ spiro-cycloalkylene). Representative examples of monocyclic cycloalkylene groups include, but are not limited to, cyclopropylene, cyclobutylene, cyclopentylene, cyclopentenylene, cyclohexylene, cyclohexenylene, cycloheptylene, and cyclooctylene. Examples of bridged cycloalkylene, fused cycloalkylene and spiro-cycloalkylene groups include, but are not limited to, decalinylene, octahydropentalenylene, octahydro-1H-indenylene, 2,3-dihydro-1H-indenylene, $C_{5-20}$ spiro-cycloalkylene (e.g., $C_{5-15}$ spiro-cycloalkylene), adamantanylene, noradamantanylene, norbomylene (also named as bicyclo[2.2.1]heptylene by the IUPAC system). As used herein, the "cycloalkylene" is optionally mono- or poly-substituted, such as, but not limited to, 2,2-, 2,3-, 2,4-, 2,5-, or 2,6-disubstituted cyclohexylene. The substituents of the substituted "cycloalkylene" can be optionally one or more (e.g., 1-5, 1-4, 1-3, 1-2, or 1) substituents selected from the group consisting of $C_1$-$C_3$ alkyl, $C_{3-6}$cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_{1-3}$alkylene, $C_{1-3}$ alkyl-NHC(O)-, $C_{1-3}$ alkyl-C(O)NH-, cyano, and any combination thereof.

As used herein, the term "$C_{x-y}$ spiro-cycloalkylene" (x and y each being an integer), used alone or in combination, refers to a spiro-cycloalkylene group containing from x to y carbon atoms. As used herein, the term "$C_{5-30}$ spiro-cycloalkylene", used alone or in combination, refers to a spiro-cycloalkylene group containing from 5 to 30 carbon atoms (e.g., 5-20, 5-15, 7-20, 7-15, 5-11, 5-10, or 7-9 carbon atoms). The term "$C_{5-30}$ spiro-cycloalkylene" includes "$C_{5-20}$ spiro-cycloalkylene", "$C_{5-15}$ spiro-cycloalkylene", "$C_{7-15}$ spiro-cycloalkylene", and "$C_{7-20}$ spiro-cycloalkylene", and representative examples of which include, but are not limited to, spiro[3.3]heptylene, spiro[2.5]octylene, spiro[3.5]nonylene, spiro [4.4]nonylene, spiro[4.5]decylene, and spiro[5.5]undecylene. The "$C_{5-30}$ spiro-cycloalkylene" is optionally further substituted with one or more substituents selected from the group consisting of $C_1$-$C_3$ alkyl, $C_{3-6}$ cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_{1-3}$ alkylene, $C_{1-3}$ alkyl-NHC(O)-, $C_{1-3}$ alkyl-C(O)NH-, cyano, and any combination thereof.

As used herein, the term "$C_{x-y}$ bridged cycloalkylene" (where x and y are integers) used alone or in combination, refers to a bridged cycloalkylene group containing x to y carbon atoms. In the present invention, the term "$C_{5-30}$ bridged cycloalkylene" used alone or in combination, refers to a bridged cycloalkylene group containing 5 to 30 (e.g., but not limited to, 5-20, 6-20, 7-20, 5-15, 7-15, 5-11, 5-10, and 7-9) carbon atoms. The term "$C_{5-30}$ bridged cycloalkylene" includes "$C_{5-20}$ bridged cycloalkylene", "$C_{6-20}$ bridged cycloalkylene", "$C_{7-20}$ bridged cycloalkylene", "$C_{5-15}$ bridged cycloalkylene", and "$C_{7-15}$ bridged cycloalkylene", and representative examples of which include, but are not limited to, adamantanylene, noradamantanylene, bornylene, bicyclo[2.2.1]heptanylene, 2-oxobicyclo[2.2.1]heptanylene, bicyclo[2.2.1]heptenylene, and cubanylene. Said "$C_{5-15}$ bridged cycloalkylene" may be optionally substituted with one or more substituents selected from: $C_1$-$C_3$ alkyl, $C_{3-6}$ cycloalkyl, hydroxy, amino, mercapto, halogen, $C_1$-$C_3$ alkoxy, $C_1$-$C_3$ alkylamino, halogenated $C_1$-$C_3$ alkyl, amino-substituted $C_{1-3}$ alkylene, $C_{1-3}$ alkyl-NHC(O)-, $C_{1-3}$ alkyl-C(O)NH-, cyano, and any combination thereof.

As used herein, the term "heterocyclyl" or "heterocyclic group", used alone or in combination, refers to a 4- to 30-membered (e.g., 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds, but not having a fully conjugated $\pi$-electron system) monocyclic, bicyclic, tricyclic or polycyclic cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclyl group include, but not limited to, monocyclic heterocyclyl (e.g., 4- to 30-membered monocyclic heterocyclyl, and 4- to 20-membered monocyclic heterocyclyl), bridged heterocyclyl (e.g., 5- to 30-membered bridged heterocyclyl, 5- to 20-membered bridged heterocyclyl, 7- to 20-membered bridged heterocyclyl, and 7- to 15-membered bridged heterocyclyl), fused heterocyclyl (e.g., 5- to 30-membered fused heterocyclyl, and 5- to 20-membered fused heterocyclyl), and spiro-heterocyclyl groups (e.g., 5- to 30-membered spiro-heterocyclyl, and 5- to 20-membered spiro-heterocyclyl). Representative examples of the monocyclic heterocyclyl include, but are not limited to, azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, morpholinyl, thiomorpholinyl, dioxacyclohexyl, azacycloheptyl, azacyclooctyl, diazacycloheptanyl (e.g., 1,4-diazacycloheptan-1-yl) and diazacyclooctyl. Examples of bridged heterocyclyl, fused heterocyclyl and spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, octahydro-1H-indolyl, and azaspirocycloalkyl (e.g., 5- to 20-membered azaspirocycloalkyl, such as 3-azaspiro[5.5]undecan-3-yl). The heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, and any combination thereof.

As used herein, the term "nitrogen-containing heterocyclyl", used alone or in combination, refers to 4- to 30-membered (e.g., 4- to 30-membered, 4- to 25-membered, 4- to 20-membered, 4- to 15-membered, 4- to 14-membered, 4- to 13-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds, but not having a fully conjugated $\pi$-electron system) monocyclic, bicyclic, tricyclic or polycyclic cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing heterocyclyl group include, but not limited to, nitrogen-containing monocyclic heterocyclyl (e.g., 4- to 30-membered nitrogen-containing monocyclic heterocyclyl, and 4- to 20-membered nitrogen-containing monocyclic heterocyclyl), nitrogen-containing bridged heterocyclyl (e.g., 5- to 30-membered nitrogen-containing bridged heterocyclyl, 5- to 20-membered nitrogen-containing bridged heterocyclyl, 5- to 15-membered nitrogen-containing bridged heterocyclyl, 7- to 20-membered nitrogen-containing bridged heterocyclyl, and 7- to 15-membered nitrogen-containing bridged heterocyclyl), nitrogen-containing fused heterocyclyl (e.g., 5- to 30-membered nitrogen-containing fused heterocyclyl, and 5- to 20-membered nitrogen-containing fused heterocyclyl), and nitrogen-containing spiro-heterocyclyl groups (e.g., 5- to 30-membered nitrogen-containing spiro-heterocyclyl, and 5- to 20-membered nitrogen-containing spiro-heterocyclyl). Representative examples of the nitrogen-containing monocyclic heterocyclyl include, but are not limited to, azetidinyl, pyrrolidinyl, imidazolidinyl, pyrazolidyl, oxazolidinyl, thiazolidinyl, piperidinyl, piperazinyl, morpholinyl, thiomorpholinyl, azacycloheptyl, azacyclooctyl, diazacycloheptanyl (e.g., 1,4-diazacycloheptan-1-yl), and diazacyclooctyl. Examples of nitrogen-containing bridged heterocyclyl, nitrogen-containing fused heterocyclyl and nitrogen-containing spiro-heterocyclyl groups include, but are not limited to, 6-azabicyclo[3.1.1]heptan-3-yl, 2,5-diazabicyclo[2.2.1]heptan-2-yl, 3,6-diazabicyclo[3.1.1]heptan-3-yl, 3-azabicyclo[3.2.1]octan-8-yl, 3,8-diazabicyclo[3.2.1]octan-8-yl, 3,8-diazabicyclo[3.2.1]octan-3-yl, 2,5-diazabicyclo[2.2.2]octan-2-yl, octahydro-1H-indolyl, and azaspirocycloalkyl (e.g., 5- to 20-membered azaspirocycloalkyl, such as 3-azaspiro[5.5]undecan-3-yl). The nitrogen-containing heterocyclyl may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, and any combination thereof.

As used herein, the term "heterocyclylene", used alone or in combination, refers to a 4- to 30-membered saturated or partially unsaturated (i.e., containing one or more double bonds, but not having a fully conjugated $\pi$-electron system) monocyclic, bicyclic, tricyclic or polycyclic bivalent cyclic hydrocarbon group containing one or more (e.g., from 1 to 5, or from 1 to 4, from 1 to 3, from 1 to 2, or 1) heteroatoms independently selected from sulfur, oxygen, and nitrogen. Examples of the heterocyclylene groups include, but are not limited to, monocyclic heterocyclylene (e.g., 4- to 30-membered monocyclic heterocyclylene, and 4- to 20-membered monocyclic heterocyclylene), bridged heterocyclylene (e.g., 5- to 30-membered bridged heterocyclylene, 5- to 20-membered bridged heterocyclylene, 7- to 20-membered bridged heterocyclylene, and 7- to 15-membered bridged heterocyclylene), fused heterocyclylene (e.g., 5-to 30-membered fused heterocyclylene, and 5- to 20-membered fused heterocyclylene) and spiro-heterocyclylene groups (e.g., 5- to 30-membered spiro-heterocyclylene, and 5- to 20-membered spiro-heterocyclylene). Representative examples of the monocyclic heterocyclylene include, but are not limited to, azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, diazacycloheptanylene (e.g., 1,4-diazacycloheptanylene, 4,5-diazacycloheptanylene, 1,3-diazacycloheptanylene), and diazacyclooctylene. Examples of the bridged heterocyclylene, fused heterocyclylene and spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, octahydro-1H-indolylene, and azaspirocycloalkylene (e.g., 5- to 20-membered azaspirocycloalkylene, such as 3-azaspiro[5.5]undecanylene). The heterocyclylene may be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated

$C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, and any combination thereof.

As used herein, the term " nitrogen-containing heterocyclylene", used alone or in combination, refers to a 4- to 30-membered (e.g., 4- to 30-membered, 4- to 25-membered, 4- to 20-membered, 4- to 15-membered, 4- to 14-membered, 4- to 12-membered, 4- to 11-membered, 4- to 10-membered, 4- to 9-membered, 4- to 8-membered, 4- to 7-membered, 4- to 6-membered, 4- to 5-membered, 5- to 9-membered, 5- to 30-membered, 5- to 20-membered, or 5- to 15-membered) saturated or partially unsaturated (i.e., containing one or more double bonds, but not having a fully conjugated $\pi$-electron system) monocyclic, bicyclic, tricyclic or polycyclic bivalent cyclic hydrocarbon group containing one nitrogen atom and optionally one or more (e.g., from 1 to 5, or from 1 to 4, from 1 to 3, from 1 to 2, or 1) heteroatoms independently selected from sulfur, oxygen, and nitrogen. Examples of the nitrogen-containing heterocyclylene group include, but not limited to, nitrogen-containing monocyclic heterocyclylene (e.g., 4-to 30-membered nitrogen-containing monocyclic heterocyclylene, and 4- to 20-membered nitrogen-containing monocyclic heterocyclylene), nitrogen-containing bridged heterocyclylene (e.g., 5- to 30-membered nitrogen-containing bridged heterocyclylene, 5- to 20-membered nitrogen-containing bridged heterocyclylene, 7- to 20-membered nitrogen-containing bridged heterocyclylene, and 7- to 15-membered nitrogen-containing bridged heterocyclylene), nitrogen-containing fused heterocyclylene (e.g., 5- to 30-membered nitrogen-containing fused heterocyclylene, and 5- to 20-membered nitrogen-containing fused heterocyclylene), and nitrogen-containing spiro-heterocyclylene (e.g., 5- to 30-membered nitrogen-containing spiro-heterocyclylene, and 5- to 20-membered nitrogen-containing spiro-heterocyclylene). Representative examples of the nitrogen-containing monocyclic heterocyclylene include, but are not limited to, azetidinylene, pyrrolidinylene, imidazolidinylene, pyrazolidylene, oxazolidinylene, thiazolidinylene, piperidinylene, piperazinylene, morpholinylene, thiomorpholinylene, azacycloheptanylene, azacyclooctylene, diazacycloheptanylene (e.g., 1,4-diazacycloheptanylene, 4,5-diazacycloheptanylene, 1,3-diazacycloheptanylene), and diazacyclooctylene. Examples of nitrogen-containing bridged heterocyclylene, nitrogen-containing fused heterocyclylene, and nitrogen-containing spiro-heterocyclylene groups include, but are not limited to, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, octahydro-1H-indolylene, and azaspirocycloalkylene (e.g., 5- to 20-membered azaspirocycloalkylene, such as 3-azaspiro[5.5]undecanylene). The nitrogen-containing heterocyclylenemay be unsubstituted or substituted as explicitly defined (e.g., mono-, di-, tri-, or poly-substituted) by a substituent(s) optionally selected from the group consisting of deuterium, hydroxy, amino, mercapto, nitro, halogen, cyano, optionally deuterated $C_{1-6}$ alkyl, halogenated $C_{1-6}$ alkyl, optionally deuterated $C_{3-6}$ cycloalkyl, optionally deuterated $C_{1-6}$ alkoxy, optionally deuterated $C_{1-6}$ alkyl-NH-, $NH_2$-$C_{1-6}$ alkylene, optionally deuterated $C_{1-6}$ alkyl-NHC(O)-, optionally deuterated $C_{1-6}$ alkyl-C(O)NH-, and any combination thereof.

As used herein, the term "alkynylene", used alone or in combination, refers to a linear or branched bivalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynylene include $C_{2-8}$ alkynylene, $C_{2-6}$ alkynylene, or $C_{2-4}$ alkynylene, with representative but non-limiting examples including ethynylene, 1-propynylene, 1-butynylene, and 1,3-diynylene.

As used herein, the suffix "ylylidene" for a group name indicates that the mentioned group is connected to the rest of the molecule through one single bond and one double bond, constituting a trivalent group.

As used herein, the term "alkynyl", used alone or in combination, refers to a linear or branched monovalent hydrocarbon group containing from 2 to 8 (e.g., from 2 to 6, from 2 to 5, from 2 to 4, or preferably 2) carbon atoms and having one or more (e.g., from 1 to 3, from 1 to 2, or 1) carbon-carbon triple bonds. Examples of alkynyl include $C_{2-8}$ alkynyl, $C_{2-6}$ alkynyl, or $C_{2-4}$ alkynyl, with representative but non-limiting examples including ethynyl, 1-propynyl, 1-butynyl, and 1,3-diynyl.

As used herein, the term "alkenylene", used alone or in combination, refers to a linear or branched divalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of alkenylene groups include $C_{2-8}$ alkenylene, $C_{2-6}$ alkenylene, or $C_{2-4}$ alkenylene, with representative but non-limiting examples including vinylene (e.g., -CH=CH-), 1-propenylene, allylidene, 1-butenylene, 2-butenylene, 3-butenylene, isobutenylene, pentenylene, n-pent-2,4-dienylene, 1-methyl-but-1-enylene, 2-methyl-but-1-enylene, 3-methyl-but-1-enylene, 1-methyl-but-2-enylene, 2-methyl-but-2-enylene, 3-methyl-but-2-enylene, 1-methyl-but-3-enylene, 2-methyl-but-3-enylene, 3-methyl-but-3-enylene, and hexenylene.

As used herein, the term "alkenyl", used alone or in combination, refers to a linear or branched monovalent hydrocarbon group containing from 2 to 8 (e.g., from 2 to 6, from 2 to 5, from 2 to 4, from 2 to 3, or 2) carbon atoms and having one or more (e.g., from 1 to 3, from 1 to 2, or 1) carbon-carbon double bonds. Examples of "alkenyl" include $C_{2-8}$ alkenyl, $C_{2-6}$ alkenyl or $C_{2-4}$ alkenyl, with representative but non-limiting examples including, vinyl (e.g., $CH_2$=CH-), 1-propenyl, allyl, 1-butenyl, 2-butenyl, 3-butenyl, isobutenyl, pentenyl, n-pent-2,4-dienyl, 1-methyl-but-1-enyl, 2-methyl-but-1-enyl, 3-methyl-but-1-enyl, 1-methyl-but-2-enyl, 2-methyl-but-2-enyl, 3-methyl-but-2-enyl, 1-methyl-but-3-enyl, 2-methyl-but-3-enyl, 3-methyl-but-3-enyl, and hexenyl.

As used herein, "bornylane" or "bornane" (also known as 1,7,7-trimethylbicyclo[2.2.1]heptane; camphane) has a definition known to those skilled in the art. As used herein, "camphanyl" or "bornyl" refers to a monovalent group of bornane, i.e., the group remaining after any one of the hydrogens in bornane is removed. Representative examples of "bornyl" include, but are not limited to, 1,7,7-trimethylbicyclo[2.2.1]heptan-2-yl, 1,7,7-trimethylbicyclo[2.2.1]heptan-3-yl, 1,7,7-trimethylbicyclo[2.2.1]heptan-4-yl, 1,7,7-trimethylbicyclo[2.2.1]heptan-5-yl, 1,7,7-trimethylbicyclo[2.2.1]heptan-6-yl,

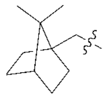 or 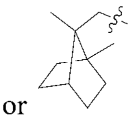 .

As used herein, "bicyclo[2.2.1]heptane" also known as "norbornane", has a definition known to those skilled in the art. As used herein, "bicyclo[2.2.1]heptyl" or "norbornyl" refers to a monovalent group of bicyclo[2.2.1]heptane, i.e., the group remaining after any hydrogen in bicyclo[2.2.1]heptane is removed. Representative examples of "bicyclo[2.2.1] heptyl" include, but are not limited to, bicyclo[2.2.1]heptan-2-yl, bicyclo[2.2.1]heptan-3-yl, bicyclo[2.2.1]heptan-4-yl, bicyclo[2.2.1]heptan-5-yl, or bicyclo[2.2.1]heptan-6-yl.

As used herein, the term "bicyclo[2.2.1]heptene" has a definition known to those skilled in the art. As used herein, "bicyclo[2.2.1]heptenyl" refers to a monovalent group of bicyclo[2.2.1]heptene, i.e., the group remaining after any hydrogen in bicyclo[2.2.1]heptene is removed. Representative examples of "bicyclo[2.2.1]heptenyl" include, but are not limited to, bicyclo[2.2.1]hept-5-en-2-yl, bicyclo[2.2.1]hept-5-en- 3-yl, or bicyclo[2.2.1]hept-5-en-7-yl.

As used herein, "adamantane" (also known as tricyclo[3.3.1.1$^{3,7}$]decane) has a definition known to those skilled in the art, and its structural formula is e.g., as follows:

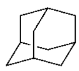.

As used herein, "adamantanyl" refers to a monovalent group of adamantane, that is, the group remaining after any hydrogen in adamantane is removed. Representative examples of "adamantanyl" include, but are not limited to, 1-adamantanyl, 2-adamantanyl, 3-adamantanyl, 4-adamantanyl, 5-adamantanyl, 6-adamantanyl, 7-adamantanyl, 8-adamantanyl, 9-adamantanyl, or 10-adamantanyl.

As used herein, "noradamantane" has a definition known to those skilled in the art, and its structural formula is e.g., as follows:

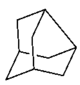 or 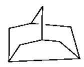.

As used herein, "noradamantanyl" refers to a monovalent group of noradamantane, that is, the group remaining after any hydrogen in noradamantane is removed. Representative examples of "noradamantanyl" include, but are not limited to, 1-noradamantanyl, 2-noradamantanyl, 3-noradamantanyl, 4-noradamantanyl, 5-noradamantanyl, 6-noradamantanyl, 7-noradamantanyl, 8-noradamantanyl or 9-noradamantanyl.

As used herein, "adamantanamine" has the definitions known to those skilled in the art, namely referring to an adamantane having an amino substituent, wherein the amino substituent can replace a hydrogen on a carbon at any position in the adamantane. An example of "adamantanamine" can be adamantan-1-amine (corresponding English chemical name is adamantan-1-amine or Tricyclo[3.3.1.1$^{3,7}$]decan-1-amine; CAS No.: 768-94-5), with the following structural Formula:

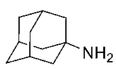.

Salts or pharmaceutically acceptable salts, enantiomers, stereoisomers, solvates, polymorphs of the compounds of Formula (I) of the present disclosure are also encompassed within the scope of the present invention.

In all embodiments of the present disclosure, the salts or pharmaceutically acceptable salts of the compounds of Formula (I) refer to non-toxic inorganic or organic acid and/or base addition salts. Examples include: sulfates, hydrohalides (including hydrochlorides and hydrobromates), maleate, sulfonate, citrate, lactate, lactobionate, L-tartrate, fumarate, L-malate, L-lactate, α-ketoglutarate, hippurate, D-glucuronate, D-gluconate, α-D-glucoheptonate, glycolate, mucate, L-ascorbate, orotate, picrate, glycinate, alaninate, arginate, cinnamate, laurate, pamoate, sebacate, besylate, methanesulfonate, ethanesulfonate, edisylate,formate, acetate, 2,2-dichloroacetate, trimethylacetate, propionate, valerate, palmitate, triphenylacetate, 2-ethyl-butane-1,4-dioate, iodate, nicotinate, L-pyroglutamate, L-prolinate, ferulate, 2-hydroxyethanesulfonate, nitrate, gentisate, cholate, salicylate, terephthalate, glutarate, adipate, stearate, oleate, undecylenate, camphorate, camsylate, dodecylsulfate, phosphate, thiocyanate, dihydrogen phosphate, pyrophosphate, metaphosphate, oxalate, carbonate, malonate, benzoate, mandelate, succinate, pyruvate, 4-chlorobenzenesulfonate, 1,5-naphthalenedisulfonate, 3-hydroxy-2-naphthoate, 1-hydroxy-2-naphthoate, 2-naphthalenesulfonate, trifluoroacetate, glycolate, or 4-methylbenzenesulfonate, etc.

"Pharmaceutically acceptable carrier" refers to a pharmaceutically acceptable material, such as a filler, stabilizer, dispersant, suspending agent, diluent, excipient, thickener, solvent, or encapsulating material, with which the useful compounds according to the present disclosure are carried or transported into or administered to a patient so that they can perform their intended function. Generally, such constructs are carried or transported from one organ or part of the body to another organ or part of the body. The carrier is compatible with the other ingredients of the formulation, including the compounds useful in the present disclosure, and is not harmful to the patient, and the carrier must be "acceptable". Some examples of materials that can be used as pharmaceutically acceptable carriers include sugars such as lactose, glucose, and sucrose; starches such as corn starch and potato starch; cellulose and its derivatives such as sodium carboxymethyl cellulose, ethyl cellulose and cellulose acetate; powdered tragacanth; malt; gelatin; talc; excipients such as cocoa butter and suppository wax; oils such as peanut oil, cottonseed oil, safflower oil, sesame oil, olive oil, corn oil and soybean oil; glycols such as propylene glycol; polyols such as glycerol, sorbitol, mannitol, and polyethylene glycol; esters such as ethyl oleate and ethyl laurate; agar; buffers such as magnesium hydroxide and aluminum hydroxide; surfactant phosphate buffer solution; and other common non-toxic compatible substances used in pharmaceutical formulations.

As used herein, the term "room temperature" refers to the ambient temperature, such as 20-30°C.

As used herein, "stereoisomer" refers to a compound with the same chemical structural formula, but a different arrangement of atoms or groups in space. Stereoisomers include enantiomers, diastereomers, conformational isomers (rotational isomers), geometric isomers (cis/trans isomers), atropisomers, and so on.

As used herein, the term "solvate" refers to an association or complex formed by the interaction between one or more solvent molecules and the compounds of the present invention. Examples of solvents include water, isopropanol, ethanol, methanol, DMSO, ethyl acetate, acetic acid and ethanolamine. The term "hydrate" means that a complex formed with water.

As used herein, the term "chiral" refers to a molecule that is nonsuperimposable on its mirror image; whereas "achiral" refers to a molecule that can be superimposed on its mirror image.

As used herein, the term "enantiomers" refers to two isomers of a compound that are nonsuperimposable mirror images.

As used herein, the term "diastereomers" refers to stereoisomers that have two or more chiral centers but are not mirror images of each other. The diastereomers have different physical properties, such as melting point, boiling point, spectral properties and reactivity. The diastereomeric mixture can be separated by high-resolution analytical operations such as electrophoresis and chromatography, such as HPLC.

As used herein, "p-menthane" has the definitions known to those skilled in the art, and its structural formula is e.g., as follows:

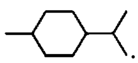.

As used herein, "*p*-menthanyl" refers to a monovalent group of *p*-menthane, that is, the group remaining after any hydrogen in *p*-menthane is removed. Representative examples of "p-menthanyl" include, but are not limited to,

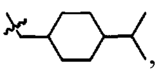, 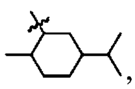, 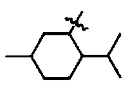, 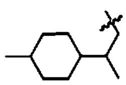,

or

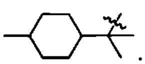.

As used herein, "*m*-menthane" has the definitions known to those skilled in the art, and its structural formula is e.g., as follows:

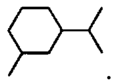.

As used herein, "*m*-menthanyl" refers to a monovalent group of *m*-menthane, that is, the group remaining after any hydrogen in *m*-menthane is removed. Representative examples of "*m*-menthanyl" include, but are not limited to,

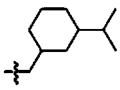, 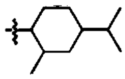, 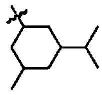, 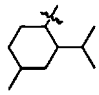,

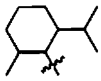, 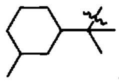, 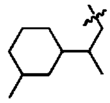, or 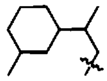.

As used herein, "Quinuclidine" (also known as 1-azabicyclo[2.2.2]octane) has the definitions known to those skilled in the art, and its structural formula is e.g., as follows:

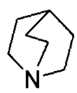.

As used herein, "quinuclidinyl" refers to a monovalent group of Quinuclidine, that is, the group remaining after any hydrogen in Quinuclidine is removed. Representative examples of "quinuclidinyl" include, but are not limited to,

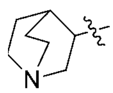, 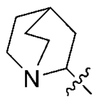, 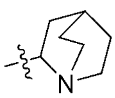, 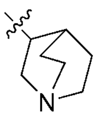, 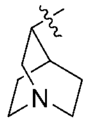, 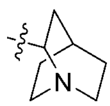, or 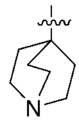.

**Description of the Drawings**

Fig. 1 shows the Western blot assay results of the degradation of target substrate proteins by the compounds of the present disclosure.

**Examples**

In the following description, numerous specific details are set forth in order to provide a thorough understanding of the present disclosure. The present disclosure may be practiced without some or all of these specific details. In other cases, well-known process operations have not been described in detail in order not to unnecessarily obscure the present disclosure. Although the present disclosure will be described in conjunction with specific embodiments, it should be understood that this is not intended to limit the present disclosure to these embodiments.

The following abbreviations are used throughout the specification and examples:

| | |
|---|---|
| AcOH | acetic acid |
| Boc | t-Butyloxy carbonyl |
| DCM | dichloromethane |
| DIEA or DIPEA | N, N-diisopropylethylamine |
| DMF | N,N-dimethylformamide |
| DMSO | dimethyl sulfoxide |
| EA | ethyl acetate |
| ESI | electrospray ionization |
| equiv | equivalent |
| EtOH | ethanol |
| EtOAc | ethyl acetate |
| HPLC | high performance liquid chromatography |

| | |
|---|---|
| HRMS | high resolution mass spectrometry |
| LC-MS | liquid chromatography-mass spectrometry |
| LRMS | low resolution mass spectrometry |
| LC | liquid chromatography |
| Me | methyl |
| MeCN | acetonitrile |
| MeOH | methanol |
| MS | mass spectrometry |
| MsCl | methanesulfonyl chloride |
| MsO- | methanesulfonyloxy |
| $Ms_2O$ | methanesulfonic anhydride |
| $^1H$ NMR | Proton nuclear magnetic resonance |
| MeO- | methoxy |
| ONs | o-nitrobenzenesulfonyl |
| rt | room temperature |
| tBu | tert-butyl |
| TEA | triethylamine |
| TFA | trifluoroacetic acid |
| $T_fO$- | trifluoromethanesulfonate |
| TLC | thin layer chromatography |
| TMS | tetramethylsilane |
| TsO- | tosyloxy |
| Xantphos | 4,5-bis(diphenylphosphino)-9,9-dimethylxanthene |
| X-Phos | 2-dicyclohexylphosphino-2',4',6'-triisopropylbiphenyl |

In the present disclosure, the $^1H$ NMR spectrum was recorded on a Bruker-500MHz nuclear magnetic resonance instrument, by using, as a solvent, $CD_3OD$ ($\delta$ = 3.31 ppm) containing 0.1% TMS (as an internal standard); or using, as a solvent, $CDCl_3$ ($\delta$ = 7.26 ppm) containing 0.1% TMS (as an internal standard); or using, as a solvent, DMSO-$d_6$ ($\delta$ = 2.50 ppm) containing 0.03% TMS (as an internal standard). LC-MS spectra were recorded on a Sciex API 2000 mass spectrometer equipped with an Agilent 1100 binary pump, DAD and ELSD detectors, or on an Agilent 1260-6125B single quadrupole LC-MS system equipped with an Agilent 1260 quaternary pump, DAD and ELSD detectors. HPLC preparative purification was performed using a SHIMADZU LC-20AP system. HPLC purity was determined using either a SHIMADZU LC-30AP or Waters 1525 system. All reactions were carried out under ambient atmosphere unless otherwise specified. Reaction progress was monitored by TLC or LC-MS.

Solvents and reagents are processed as follows:

the solvents used in the reactions such as DCM, DMF, anhydrous EtOH, and anhydrous MeOH were commercially available;
preparative HPLC was performed using HPLC-grade $CH_3CN$ and deionized water;
all other reactants, reagents and chemicals were commercially available, unless otherwise specified, or were synthesized using methods known in the art.

Unless otherwise specified, the materials and reagents used in the following examples are commercially available and used directly, or can be synthesized by using methods known in the art.

**General synthesis methods**

Compounds and/or pharmaceutically acceptable salts thereof of the present disclosure can be synthesized using commercially available raw materials by synthetic techniques known in the art. The synthetic schemes described below illustrate the preparation of most compounds. The starting materials or reagents used in each scheme can be purchased from commercial sources or prepared by methods known to those skilled in the art. One skilled in the art can prepare the salts, racemates, enantiomers, phosphates, sulfates, hydrochlorides and prodrug forms of the compounds of Formula (I) of the present disclosure according to routine techniques in the art.

Scheme 1:

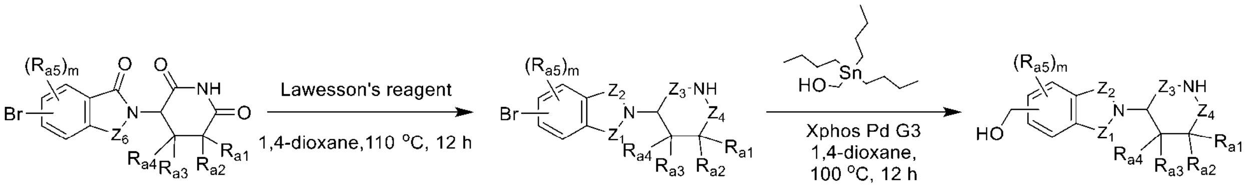


Scheme 1

In Scheme 1, $Z_6$ represents C(O), $CH_2$ or $CD_2$; $Z_1$ represents C(O), C(S), $CH_2$ or $CD_2$; $Z_2$, $Z_3$, and $Z_4$ each independently represent C(O) or C(S); wherein at least one of $Z_1$, $Z_2$, $Z_3$, and $Z_4$ represents C(S).

In some embodiments, at least two of $Z_1$, $Z_2$, $Z_3$, and $Z_4$ in the product of step 1 represent C(S). In some embodiments, at least three of $Z_1$, $Z_2$, $Z_3$, and $Z_4$ represent C(S). In some embodiments, all of $Z_1$, $Z_2$, $Z_3$, and $Z_4$ represent C(S).

In Scheme 1, the thionation reagent Lawesson's reagent may also be replaced with other suitable thionation reagents, such as, but not limited to, carbon disulfide, hexamethyldisilathiane, sulfur, thiourea, hydrogen sulfide, phosphorus pentasulfide, Lawesson's Reagent, Belleau's reagent, and Davy's reagent.

In Scheme 1, the substituent Br on the reaction substrate in step 1 may be located at the 4-, 5-, 6-, or 7-position of the benzene ring of the isoindolinyl group. Accordingly, the substituent Br in the resulting intermediate product and the substituent-$CH_2$-OH in the product of step 2 are also located at the 4-, 5-, 6-, or 7-position of the benzene ring of the isoindolinyl group. $R_{a1}$, $R_{a2}$, $R_{a3}$, $R_{a4}$, and $(R_{a5})_m$ are as defined in the compound of Formula (I) and its various sub-embodiments of the present disclosure.

Scheme 2: (Sulfonate ester formation)

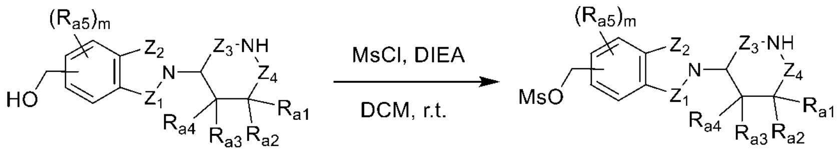


Scheme 2

In Scheme 2, $Z_1$ represents C(O), C(S), $CH_2$ or $CD_2$; $Z_2$, $Z_3$, and $Z_4$ each independently represent C(O) or C(S), wherein at least one of $Z_1$, $Z_2$, $Z_3$, and $Z_4$ represents C(S).

In Scheme 2, the substituent -$CH_2$-OH on the reaction substrate may be located at the 4-, 5-, 6-, or 7-position of the benzene ring of the isoindolinyl group. Accordingly, the substituent -$CH_2$-OMs in the resulting product is also located at the 4-, 5-, 6-, or 7-position of the benzene ring of the isoindolinyl group. $R_{a1}$, $R_{a2}$, $R_{a3}$, $R_{a4}$, and $(R_{a5})_m$ are as defined in the compound of Formula (I) and its various sub-embodiments of the present disclosure.

Scheme 3:

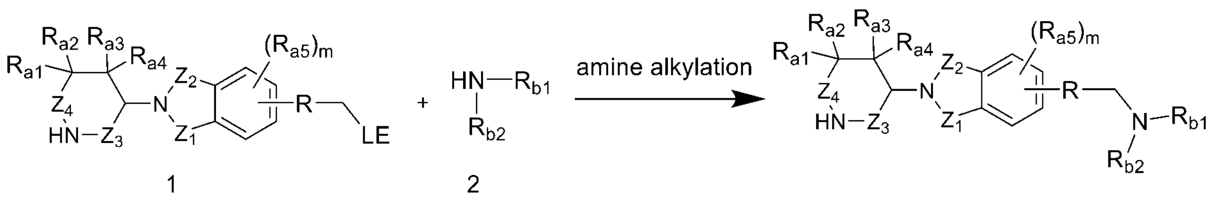


1 2

Scheme 3

In Scheme 3, the groups $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments of the present disclosure. The group LE represents Cl, Br, I, OMs, OTs, or ONs. Amine substrate 2 is a compound corresponding to the X moiety containing an amino group or a nitrogen-containing heterocycle in the compound of Formula (I). In some embodiments, group $R_{b1}$ of amine substrate 2 is as defined for group $R_1$ of the compound of Formula (I) and its various sub-embodiments, and group $R_{b2}$ is as defined for group $R_2$ of the compound of Formula (I) and its various sub-embodiments. In some embodiments, group $R_{b1}$ and group $R_{b2}$ of amine substrate 2, together with the nitrogen atom to which they are attached, form a nitrogen-containing heterocycle $A_a$ substituted by n1 substituents $R_{d1}$ and a substituent represented by the formula of

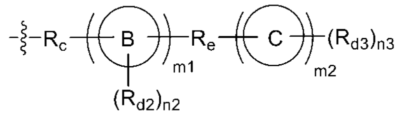

,

i.e., the amine substrate 2 has a structure of the formula:

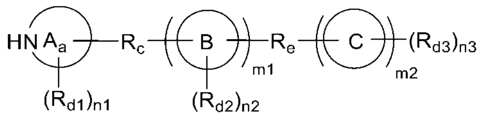

,

and the product has a structure of the formula:

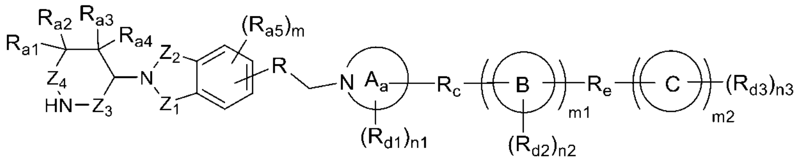

,

wherein the nitrogen-containing heterocycle $A_a$ represents 4- to 30-membered nitrogen-containing heterocyclyl (e.g., 4- to 20-membered nitrogen-containing heterocyclyl, or 4- to 15-membered nitrogen-containing heterocyclyl). The groups $R_{a1}$, $R_{a2}$, $R_{a3}$, $R_{a4}$, $(R_{a5})_m$, $Z_1$, $Z_2$, $Z_3$, $Z_4$, R, $(R_{d1})_{n1}$, n1, $R_c$, ring B, m1, $(R_{d2})_{n2}$, n2, $R_e$, ring C, m2, $(R_{d3})_{n3}$, and n3 are as defined in the compound of Formula (I) and its various sub-embodiments of the present disclosure.

The amine alkylation reaction in Scheme 3 may be conducted, for example, in the presence of DIEA and sodium iodide, or triethylamine and sodium iodide, at a temperature ranging from room temperature to 80°C (e.g., 40°C-60°C, 40°C-50°C, or 50°C-60°C). The molar ratio of substrate 1 to substrate 2 may be, for example, 1:1.1-2, 1:1.1-1.5, 1:1.1-1.2, or 1:1.2-1.3.

For example, the specific procedure for Scheme 3 may be as follows:

To a solution of methanesulfonate substrate 1 (1.3 eq.) and amine substrate 2 (1.0 eq.) in anhydrous DMF (3 mL) are added triethylamine (3.0 eq.) and sodium iodide (1.0 eq.). The reaction mixture is stirred at room temperature for 3 hours. After monitoring the reaction via LCMS and confirming its completion, the reaction mixture is filtered, and the filtrate is separated and purified by high-performance liquid chromatography to give the target compound.

Scheme 4:

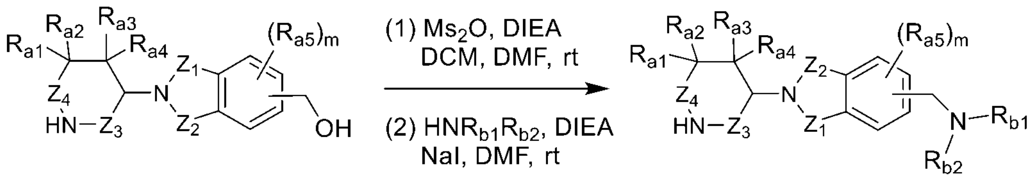


Scheme 4

In Scheme 4, the groups $R_{a1}$, $R_{a2}$, $R_{a3}$, $R_{a4}$, $(R_{a5})_m$, $Z_1$, $Z_2$, $Z_3$, $Z_4$, and R are as defined in the compound of Formula (I) and its various sub-embodiments of the present disclosure. The amine substrate $HNR_{b1}R_{b2}$ in step (2) is a compound corresponding to the X moiety containing an amino group or a nitrogen-containing heterocycle in the compound of Formula (I), and the groups $R_{b1}$ and $R_{b2}$ are as defined for groups $R_1$ and $R_2$ of the compound of Formula (I) and its various sub-embodiments, or as defined in Scheme 3.

For example, the specific procedure for Scheme 4 may be as follows:

To a solution of hydroxyl substrate (1.0 eq.) in anhydrous DMF (0.5 mL) and dichloromethane (5 mL) are added diisopropylethylamine (3.0 eq.) and methanesulfonic anhydride (0.9 eq.). The reaction mixture is stirred at room temperature for 5 minutes. After monitoring the reaction via TLC and confirming its completion, a solution of the corresponding amine substrate (1.0 eq.) in DMF (0.5 mL) is added to the reaction mixture, and the mixture is then stirred at room temperature for 3 hours. After monitoring the reaction via LCMS and confirming its completion, the reaction mixture is filtered and concentrated, and the residue is separated and purified by high-performance liquid chromatography to give the target compound.

Scheme 5:

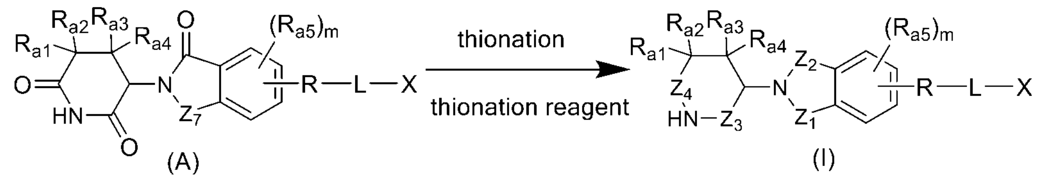


Scheme 5

In Scheme 5, $Z_7$ in the compound of Formula (A) represents C(O), $CH_2$ or $CD_2$; $Z_1$ in the compound of Formula (I) represents C(O), C(S), $CH_2$ or $CD_2$; $Z_2$, $Z_3$, and $Z_4$ each independently represent C(O) or C(S), wherein at least one of $Z_1$, $Z_2$, $Z_3$, and $Z_4$ represents C(S); and the groups $R_{a1}$, $R_{a2}$, $R_{a3}$, $R_{a4}$, $(R_{a5})_m$, R, L, and X are as defined in the compound of Formula (I) and its various sub-embodiments of the present disclosure. The amine substrate $HNR_{b1}R_{b2}$ in step (2) is a compound corresponding to the X moiety containing an amino group or a nitrogen-containing heterocycle in the compound of Formula (I); and the groups $R_{b1}$ and $R_{b2}$ are as defined for groups $R_1$ and $R_2$ of the compound of Formula (I) and its various sub-embodiments, or as defined in Scheme 3.

The thionation reagent in Scheme 5 may be Lawesson's reagent, or may be other thionation reagents, such as but not limited to carbon disulfide, hexamethyldisilathiane, sulfur, thiourea, hydrogen sulfide, phosphorus pentasulfide, Belleau's reagent, and Davy's reagent.

The specific procedure for Scheme 5 may be as follows:

To a solution of the compound of Formula (A) (1.0 eq.) in 1,4-dioxane (10 mL) is added Lawesson's reagent (1.0 eq.). The reaction mixture is heated to 120°C and stirred under a nitrogen atmosphere for 16 hours. After completion of the reaction is confirmed by TLC, the reaction mixture is filtered and concentrated. The residue is separated and purified by high-performance liquid chromatography to afford the target compound.

Depending upon the target compounds, the above schemes and their reaction substrates, reaction conditions (including reaction dosage, temperature, duration, etc.), work up, etc. can be appropriately modified and adjusted by techniques and methods well known to those skilled in the art to obtain the desired target compounds. The obtained target compounds can be further modified by the substituents and the like to obtain subsequent target compounds through methods well known to those skilled in the art.

## Examples

### Intermediate Example 1: Preparation of 3-(5-(hydroxymethyl)-1-thioxoisoindolin-2-yl)piperidine-2,6-dione (GT-06977)

The target compound (GT-06977) was prepared by referring to the method of Scheme 1.

Step 1: To a solution of 3-(5-bromo-1-oxoisoindolin-2-yl)piperidine-2,6-dione (20 g, 61.891 mmol) in 1,4-dioxane (200 mL) was added Lawesson's reagent (11.26 g, 27.851 mmol). The reaction mixture was stirred at 110°C for 12 hours. Reaction completion was monitored by TLC. The reaction mixture was concentrated, and the residue was purified by column chromatography to afford 3-(5-bromo-1-thioisoindolin-2-yl)piperidine-2,6-dione as an off-white solid (10 g, 29.480 mmol, yield 47.64%).

Step 2: To a solution of 3-(5-bromo-1-thioisoindolin-2-yl)piperidine-2,6-dione (6 g, 17.688 mmol) and (tributyl-λ4-stannanyl)methanol (8.52 g, 26.532 mmol) in 1,4-dioxane (60 mL) was added XPhos Pd G3 (0.1 g, 0.147 mmol). The reaction mixture was stirred at 100°C under an argon atmosphere for 3 hours. The reaction mixture was concentrated, and the residue was purified by column chromatography to afford the target compound (GT-06977) as a gray solid (1 g, yield 19.46%). $^1$H NMR (400 MHz, DMSO) δ 11.17 (s, 1H), 7.91 (d, J = 7.9 Hz, 1H), 7.66 (s, 1H), 7.55 (d, J = 8.0 Hz, 1H), 6.09 - 5.91 (m, 1H), 5.49 (t, J = 5.7 Hz, 1H), 4.91 - 4.67 (m, 4H), 3.13 - 2.93 (m, 1H), 2.79 - 2.58 (m, 2H), 2.16 (dd, J = 8.9, 3.6 Hz, 1H). LCMS (ESI) calcd. for $C_{14}H_{15}N_2O_3S^+$ [M+H]+: 291.08, found, 291.0.

### Intermediate Example 2: Preparation of 3-(4-(hydroxymethyl)-1-thioxoisoindolin-2-yl)piperidine-2,6-dione (GT-06981)

With reference to the method of Scheme 1 or Intermediate Example 1, the target compound (GT-06981) was obtained as a gray solid (0.8 g, yield 33.32%). $^1$HNMR (400 MHz, DMSO) δ 11.12 (s, 1H), 7.80 (d, J = 7.4 Hz, 1H), 7.61 (d, J = 7.4 Hz, 1H), 7.53 (t, J = 7.6 Hz, 1H), 5.98 (d, J = 8.3 Hz, 1H), 5.37 (d, J = 5.4 Hz, 1H), 4.79 (dd, J = 49.7, 20.2 Hz, 2H), 4.65 (d, J = 4.1 Hz, 2H), 2.96 (s, 1H), 2.64 (d, J = 18.4 Hz, 2H), 2.09 (dd, J = 8.9, 3.5 Hz, 1H). $^{13}$C NMR (101 MHz, DMSO-d6) δ 194.06, 173.08, 170.28, 139.30, 138.79, 137.62, 130.10, 128.82, 124.03, 61.22, 56.07, 31.53, 22.58. LCMS (ESI) calcd.

for $C_{14}H_{15}N_2O_3S^+$ [M+H]+: 291.08, found, 291.0.

**Intermediate Example 3: Preparation of 3-(6-(hydroxymethyl)-1-thioxoisoindolin-2-yl)piperidine-2,6-dione (GT-05566)**

With reference to the method of Scheme 1 or Intermediate Example 1, the target compound (GT-05566) was obtained (gray solid, 1 g, yield 38.91%). $^1$HNMR (400 MHz, DMSO) δ 11.06 (d, J = 31.4 Hz, 1H), 7.86 (d, J = 19.3 Hz, 1H), 7.60 (d, J = 7.8 Hz, 2H), 5.95 (d, J = 8.9 Hz, 1H), 5.41 (t, J = 5.7 Hz, 1H), 4.82 - 4.60 (m, 4H), 3.00 - 2.90 (m, 1H), 2.64 (d, J = 17.9 Hz, 1H), 2.54 (s, 1H), 2.16 - 2.05 (m, 1H). LCMS (ESI) calcd. for $C_{14}H_{15}N_2O_3S^+$ [M+H]+: 291.08, found, 291.0.

**Intermediate Example 4: Preparation of 3-(5-bromo-1-thioxoisoindolin-2-yl)piperidine-2,6-dione (GT-09531)**

The target compound (GT-09531) was prepared by referring to the method of step 1 of Scheme 1 or Intermediate Example 1.

To a solution of 3-(5-bromo-1-oxoisoindolin-2-yl)piperidine-2,6-dione (1.0 eq.) in 1,4-dioxane (20 mL) was added Lawesson's reagent (0.4 eq.). The reaction mixture was stirred at 110°C for 12 hours. Reaction completion was monitored by TLC. The reaction mixture was concentrated, and the residue was purified by column chromatography to afford the target compound (GT-09531) as an off-white solid (yield 48%). $^1$H NMR (400 MHz, DMSO) δ 11.11 (s, 1H), 7.94 (s, 1H), 7.79 (dd, J = 23.5, 8.2 Hz, 2H), 5.91 (dd, J = 12.8, 4.5 Hz, 1H), 4.77 (dd, J = 52.4, 20.3 Hz, 2H), 3.03 - 2.86 (m, 1H), 2.59 (dd, J = 39.1, 11.0 Hz, 2H), 2.15 - 2.03 (m, 1H). LCMS (ESI): [M+H]$^+$; calcd. 338.97, found, 339.0.

**Intermediate Example 5: Preparation of 3-(1-thioxoisoindolin-2-yl)piperidine-2,6-dione (GT-05563)**

To a solution of 3-(5-bromo-1-thioisoindolin-2-yl)piperidine-2,6-dione (1.0 eq.) in 1,4-dioxane (10 mL) was added XPhos Pd G3 (0.02 eq.). The reaction mixture was stirred at 100°C under an argon atmosphere for 3 hours. The reaction mixture was concentrated, and the residue was purified by column chromatography to afford the target compound (GT-05563) as a gray solid (yield 9.5%). $^1$H NMR (400 MHz, DMSO-d6) δ 11.13 (s, 1H), 7.92 (d, *J = 7.6* Hz, 1H), 7.68 (d, *J* = 3.3 Hz, 2H), 7.61 - 7.53 (m, 1H), 6.14 - 5.80 (m, 1H), 4.77 (dd, *J* = 56.0, 20.0 Hz, 2H), 3.04 - 2.90 (m, 1H), 2.78 - 2.54 (m, 2H), 2.17 - 2.05 (m, 1H). LCMS (ESI) calcd. for $C_{13}H_{13}N_2O_2S^+$ [M+H]$^+$: 261.07, found, 261.1.

**Intermediate Example 6: Preparation of (2-(2,6-dioxopiperidin-3-yl)-1-thioxoisoindolin-5-yl)methyl methanesulfonate (GT-09596)**

With reference to the method of Scheme II-2, the target compound GT-09596 was obtained (343 mg, crude, yield 112%). LCMS (ESI) calcd. for $C_{15}H_{17}N_2O_5S_2^+$ [M+H]$^+$: 369.06, found, 369.1.

**Intermediate Example 7: Preparation of ((2-(2,6-dioxopiperidin-3-yl)-1-thioxoisoindolin-4-yl)methyl methanesulfonate (GT-09597)**

With reference to the method of Scheme II-2, the target compound GT-09597 was obtained (500 mg, crude, yield 120%). LCMS (ESI) calcd. for $C_{15}H_{17}N_2O_5S_2^+$ [M+H]$^+$: 369.06, found, 369.1.

**Intermediate Example 8: Preparation of (2-(2,6-dioxopiperidin-3-yl)-3-thioxoisoindolin-5-yl)methyl methanesulfonate (GT-09598)**

With reference to the method of Scheme II-2, the target compound GT-09598 was obtained (190 mg, crude, yield 115%). LCMS (ESI) calcd. for $C_{15}H_{17}N_2O_5S_2^+$ [M+H]$^+$: 369.06, found, 369.1.

**Example 1: Preparation of 3-(5-((4-benzhydrylpiperazin-1-yl)methyl)-1-thioxoisoindolin-2-yl)piperidine-2,6-dione (GT-05104)**

With reference to the method of Scheme 4, the target compound GT-05104 was obtained (23 mg, white solid, yield 24%). $^1$H NMR (400 MHz, DMSO-d6) δ 11.13 (s, 1H), 10.57 (s, 1H), 7.10 (d, *J* = 7.9 Hz, 1H), 7.85 (s, 1H), 7.72 (d, *J* = 8.0 Hz, 1H), 7.51 - 7.40 (m, 3H), 7.38 - 7.25 (m, 5H), 7.24 - 7.18 (m, 2H), 5.95 (d, *J* = 9.0 Hz, 1H), 4.85 (d, *J* = 20.4 Hz, 1H), 4.71 (d, *J* = 20.3 Hz, 1H), 4.54 - 4.35 (m, 2H), 3.34 - 3.27 (m, 3H), 3.20 - 3.08 (m, 3H), 2.96 - 2.93 (m, 2H), 2.84 - 276 (m, 2H), 2.71 - 2.60 (m, 1H), 2.33 - 2.25 (m, 2H), 2.15 - 2.02 (m, 1H). LCMS (ESI) calcd. for $C_{31}H_{33}N_4O_2S^+$ [M+H]$^+$: 525.23, found, 525.3.

**Example 2: Preparation of 3-(5-((4-(phenyl(pyridin-2-yl)methyl)piperazin-1-yl)methyl)-1-thioxoisoindolin-2-yl) piperidine-2,6-dione (GT-05103)**

With reference to the method of Scheme 4, the target compound GT-05103 was obtained (24 mg, white solid, yield 25%). $^1H$ NMR (400 MHz, DMSO-d6) δ 11.10 (s, 1H), 10.99 (s, 1H), 8.64 (d, *J* = 4.2 Hz, 1H), 8.08 - 7.95 (m, 1H), 7.88 (t, *J* = 6.5 Hz, 1H), 7.82 (s, 1H), 7.76 (d, *J* = 7.1 Hz, 1H), 7.70 (d, *J* = 7.6 Hz, 1H), 7.53 (d, *J* = 7.2 Hz, 2H), 7.48 (s, 1H), 7.38 (t, *J* = 7.3 Hz, 3H), 7.35 - 7.27 (m, 1H), 5.33 - 5.26 (m, 1H), 4.51 - 4.39 (m, 2H), 3.40 - 3.14 (m, 6H), 3.02 - 2.75 (m, 5H), 2.71 - 2.58 (m, 2H), 2.17 - 2.09 (m, 1H), 1.38 - 1.43 (m, 1H). LCMS (ESI) calcd. for $C_{30}H_{32}N_5O_2S^+$ $[M+H]^+$: 526.23, found, 526.2.

**Example 3: Preparation of 3-(5-((4-(phenyl(pyridin-2-yl)methyl)piperazin-1-yl)methyl)-1-thioxoisoindolin-2-yl) piperidine-2,6-dione (GT-05106)**

With reference to the method of Scheme 4, the target compound GT-05106 was obtained (13 mg, white solid, yield 13%). $^1H$ NMR (400 MHz, DMSO-d6) δ 11.14 (s, 1H), 9.81 (s, 1H), 7.97 (d, *J* = 8.0 Hz, 1H), 7.80 (s, 1H), 7.68 (d, *J* = 7.9 Hz, 1H), 7.29 (t, *J* = 7.9 Hz, 4H), 7.01 (t, *J* = 7.3 Hz, 2H), 6.81 (d, *J* = 7.7 Hz, 4H), 5.95 (d, *J* = 9.0 Hz, 1H), 4.86 (d, *J* = 20.2 Hz, 1H), 4.73 (d, *J* = 20.0 Hz, 1H), 4.39 (d, *J* = 4.2 Hz, 2H), 4.26 - 4.19 (m, 1H), 3.43 - 3.40 (m, 3H), 3.20 - 3.17 (m, 3H), 2.99 - 2.93 (m, 1H), 2.67 - 2.63 (m, 1H), 2.10 - 2.07 (m, 3H), 1.62 - 1.59 (m, 1H). LCMS (ESI) calcd. for $C_{31}H_{33}N_4O_2S^+$ $[M+H]^+$: 525.23, found, 525.2.

**Example 4: Preparation of 3-(5-((4-((4'-chloro-5,5-dimethyl-3,4,5,6-tetrahydro-[1,1'-biphenyl]-2-yl)methyl)piperazin-1-yl)methyl)-1-thioxoisoindolin-2-yl)piperidine-2,6-dione (GT-05151)**

With reference to the method of Scheme 4, the target compound GT-05151 was obtained (18 mg, white solid, yield 17%). $^1H$ NMR (400 MHz, DMSO-d6) δ 11.13 (s, 1H), 7.99 - 7.87 (m, 1H), 7.83 - 7.55 (m, 2H), 7.42 (d, *J* = 8.0 Hz, 2H), 7.11 (d, *J* = 8.0 Hz, 2H), 5.97 - 5.93 (m, 1H), 4.83 (d, *J* = 21.2 Hz, 1H), 4.70 (d, *J* = 19.4 Hz, 1H), 3.27 - 3.19 (m, 4H), 3.10 - 3.06 (m, 2H), 2.98 - 2.89 (m, 2H), 2.70 - 2.62 (m, 2H), 2.35 - 2.18 (m, 4H), 2.15 - 2.06 (m, 2H), 2.01 (brs, 3H), 1.46 - 1.42 (m, 3H), 0.95 (s, 6H). LCMS (ESI) calcd. for $C_{33}H_{40}ClN_4O_2S^+$ $[M+H]^+$: 591.26, found, 591.3.

**Example 5: Preparation of 3-(5-((4-((4'-chloro-[1,1'-biphenyl]-2-yl)amino)piperidin-1-yl)methyl)-1-thioxoisoindolin-2-yl)piperidine-2,6-dione (GT-05109)**

With reference to the method of Scheme 4, the target compound GT-05109 was obtained (18 mg, white solid, yield 19%). $^1H$ NMR (400 MHz, DMSO-d6) δ 11.16 (s, 1H), 10.18 (s, 1H), 8.02 - 7.95 (m, 1H), 7.76 - 7.72 (m, 1H), 7.50 - 7.46 (m, 3H), 7.40 - 7.38 (m, 2H), 7.22 - 7.14 (m, 1H), 6.99 (dd, *J* = 7.5, 1.5 Hz, 1H), 6.85 - 6.76 (m, 1H), 6.71 - 6.69 (m, 1H), 5.98 - 5.95 (m, 1H), 4.88 (d, *J = 20.2* Hz, 1H), 4.74 (d, *J* = 20.6 Hz, 1H), 4.42 (d, *J* = 4.5 Hz, 2H), 3.31 - 3.28 (m, 2H), 3.00 - 2.94 (m, 4H), 2.67 - 2.61 (m, 2H), 2.11 - 2.05 (m, 3H), 2.01 - 1.93 (m, 1H), 1.67 - 1.50 (m, 2H). LCMS (ESI) calcd. for $C_{31}H_{32}ClN_4O_2S^+$ $[M+H]^+$: 559.19, found, 559.2.

**Example 6: Preparation of 3-(5-((4-(2,3-dichlorophenyl)piperazin-1-yl)methyl)-1-thioxoisoindolin-2-yl)piperidine-2,6-dione (GT-04474)**

With reference to the method of Scheme 4, the target compound GT-04474 was obtained (7 mg, white solid, yield 35%). $^1H$ NMR (400 MHz, DMSO) δ 11.14 (s, 1H), 10.37 (s, 1H), 8.01 (d, *J* = 9.6 Hz, 1H), 7.88 (s, 1H), 7.77 (t, *J* = 5.8 Hz, 1H), 7.41 - 7.32 (m, 3H), 7.23 - 7.15 (m, 1H), 6.01 - 5.93 (m, 1H), 4.89 (d, *J* = 20.4 Hz, 1H), 4.76 (d, *J* = 19.9 Hz, 1H), 4.59 (brs, 2H), 3.54 - 3.41 (m, 4H), 3.15 - 3.04 (m, 3H), 3.00 - 2.94 (m, 1H), 2.67 - 2.60 (m, 2H), 2.59 - 2.56(m, 2H), 2.14 - 2.06 (m, 1H). LCMS (ESI) calcd. for $C_{24}H_{25}Cl_2N_4O_2S^+$ $[M+H]^+$: 503.11, found, 503.1.

**Example 7: Preparation of 3-(5-((4-(2,3-dichlorophenyl)-3,6-dihydropyridin-1(2H)-yl)methyl)-1-thioxoisoindolin-2-yl)piperidine-2,6-dione (GT-05149)**

With reference to the method of Scheme 4, the target compound GT-05149 was obtained (9 mg, white solid, yield 10%). $^1H$ NMR (400 MHz, DMSO-d6) δ 11.15 (s, 1H), 10.67 (s, 1H), 8.00 (d, *J* = 7.8 Hz, 1H), 7.91 (s, 1H), 7.79 (d, *J* = 7.9 Hz, 1H), 7.63 (d, *J = 7.9* Hz, 1H), 7.40 (t, *J* = 7.8 Hz, 1H), 7.25 (dd, *J* = 7.7, 1.5 Hz, 1H), 5.97 (d, *J* = 10.3 Hz, 1H), 5.75 (s, 1H), 4.89 (d, *J* = 20.4 Hz, 1H), 4.76 (d, *J* = 20.3 Hz, 1H), 4.59 (s, 2H), 3.90 - 3.75 (m, 2H), 3.66 - 3.61 (m, 1H), 3.06 - 2.90 (m, 1H), 2.89 - 2.74 (m, 1H), 2.73 - 2.62 (m, 2H), 2.56 -2.51(m, 2H), 2.14 - 2.09 (m, 1H). LCMS (ESI) calcd. for $C_{25}H_{24}Cl_2N_3O_2S^+$ $[M+H]^+$: 500.10, found, 500.1.

**Example 8: Preparation of 3-(5-((4-(2,3-dichlorophenyl)piperidin-1-yl)methyl)-1-thioxoisoindolin-2-yl)piperidine-2,6-dione (GT-05150)**

With reference to the method of Scheme 4, the target compound GT-05150 was obtained (4 mg, white solid, yield 4%). LCMS (ESI) calcd. for $C_{25}H_{26}Cl_2N_3O_2S^+$ [M+H]$^+$: 502.11, found, 502.1.

**Example 9: Preparation of 3-(5-((4-(5-methylthiophen-2-yl)piperazin-1-yl)methyl)-1-thioxoisoindolin-2-yl)piperidine-2,6-dione (GT-05110)**

With reference to the method of Scheme 4, the target compound GT-05110 was obtained (16 mg, white solid, yield 19%). $^1$H NMR (400 MHz, DMSO-d6) δ 11.14 (s, 1H), 9.44 (brs, 1H), 7.98 - 7.94 (m, 1H), 7.92 - 7.86 (m, 1H), 7.86 - 7.82 (m, 1H), 7.82 - 7.70 (m, 1H), 5.98 - 5.93 (m, 1H), 4.86 (d, *J* = 20.4 Hz, 1H), 4.72 (d, *J* = 20.2 Hz, 1H), 4.63 (s, 1H), 3.37 - 3.32(m, 4H), 3.28 - 3.12 (m, 3H), 3.03 - 2.91 (m, 2H), 2.68 - 2.63 (m, 2H), 2.57 - 2.54 (m, 1H), 2.47 (s, 3H), 2.14 - 2.04 (m, 1H). LCMS (ESI) calcd. for $C_{23}H_{27}N_4O_2S_2^+$ [M+H]$^+$: 455.16, found, 455.2.

**Example 10: Preparation of 3-(5-((4-(4-methylthiophen-3-yl)piperazin-1-yl)methyl)-1-thioxoisoindolin-2-yl)piperidine-2,6-dione (GT-05113)**

With reference to the method of Scheme 4, the target compound GT-05113 was obtained (27 mg, white solid, yield 32%). $^1$H NMR (400 MHz, DMSO-d6) δ 11.11 (s, 1H), 7.96 - 7.88 (m, 2H), 7.84 (d, *J* = 7.9 Hz, 1H), 7.13 (d, *J* = 2.3 Hz, 1H), 6.82 (d, *J* = 3.2 Hz, 1H), 4.63 - 4.55 (m, 2H), 3.42 - 3.39 (m, 2H), 3.32 - 3.21 (m, 5H), 3.05 - 2.99 (m, 3H), 2.93 - 2.76 (m, 2H), 2.64 - 2.60 (m, 2H), 2.21 - 2.15 (m, 1H), 2.10 (s, 3H). LCMS (ESI) calcd. for $C_{23}H_{27}N_4O_2S_2^+$ [M+H]$^+$: 455.16, found, 455.2.

**Example 11: Preparation of 3-(5-((4-(5-methylthiophen-2-yl)piperidin-1-yl)methyl)-1-thioxoisoindolin-2-yl)piperidine-2,6-dione (GT-05112)**

With reference to the method of Scheme 4, the target compound GT-05112 was obtained (23 mg, white solid, yield 27%). $^1$H NMR (400 MHz, DMSO-d6) δ 11.14 (s, 1H), 10.25 (s, 1H), 7.99 (d, *J* = 7.8 Hz, 1H), 7.88 (s, 1H), 7.75 (d, *J* = 7.9 Hz, 1H), 6.69 - 6.59 (m, 2H), 5.96 (d, *J* = 11.7 Hz, 1H), 4.88 (d, *J* = 20.4 Hz, 1H), 4.75 (d, *J* = 20.3 Hz, 1H), 4.46 (d, *J* = 4.5 Hz, 2H), 3.43 (d, *J* = 11.8 Hz, 2H), 3.15 - 2.91 (m, 5H), 2.67 - 2.63 (m, 2H), 2.38 (s, 3H), 2.10 - 2.08 (m, 3H), 1.97 - 1.81 (m, 2H). LCMS (ESI) calcd. for $C_{24}H_{28}N_3O_2S_2^+$ [M+H]$^+$: 454.16, found, 454.1.

**Example 12: Preparation of 3-(5-((4-(4-methylthiophen-3-yl)piperidin-1-yl)methyl)-1-thioxoisoindolin-2-yl)piperidine-2,6-dione (GT-05146)**

With reference to the method of Scheme 4, the target compound GT-05146 was obtained (4 mg, white solid, yield 5%). LCMS (ESI) calcd. for $C_{24}H_{28}N_3O_2S_2^+$ [M+H]$^+$: 454.16, found, 454.1.

**Example 13: Preparation of 3-(5-((4-(furan-2-yl)piperidin-1-yl)methyl)-1-thioxoisoindolin-2-yl)piperidine-2,6-dione (GT-05148)**

With reference to the method of Scheme 4, the target compound GT-05148 was obtained (5 mg, white solid, yield 6%). LCMS (ESI) calcd. for $C_{23}H_{26}N_3O_3S^+$ [M+H]$^+$: 424.17, found, 424.2.

**Example 14: Preparation of 3-(5-((4-(5-methylthiophen-2-yl)-3,6-dihydropyridin-1(2H)-yl)methyl)-1-thioxoisoindolin-2-yl)piperidine-2,6-dione (GT-05111)**

With reference to the method of Scheme 4, the target compound GT-05111 was obtained (24 mg, white solid, yield 29%). $^1$H NMR (400 MHz, DMSO-d6) δ 11.16 (s, 1H), 10.55 (s, 1H), 7.99 (d, *J* = 7.8 Hz, 1H), 7.89 (s, 1H), 7.76 (d, *J* = 8.0 Hz, 1H), 6.95 (d, *J* = 3.4 Hz, 1H), 6.74 (d, *J* = 2.4 Hz, 1H), 5.97 (d, *J* = 15.9 Hz, 1H), 5.90 (s, 1H), 4.88 (d, *J* = 20.4 Hz, 1H), 4.75 (d, *J* = 20.3 Hz, 1H), 4.57 - 4.51 (m, 2H), 3.80 - 3.75 (m, 2H), 3.61 - 3.58(m, 1H), 3.29 - 3.20 (m, 1H), 2.97 - 2.93(m, 1H), 2.77 (s, 2H), 2.71 - 2.58 (m, 2H), 2.41 (s, 3H), 2.15 - 2.03 (m, 1H). LCMS (ESI) calcd. for $C_{24}H_{26}N_3O_2S_2^+$ [M+H]$^+$: 452.15, found, 452.2.

**Example 15: Preparation of 3-(5-((4-(4-methylthiophen-3-yl)-3,6-dihydropyridin-1(2H)-yl)methyl)-1-thioxoisoindolin-2-yl)piperidine-2,6-dione (GT-05145)**

With reference to the method of Scheme 4, the target compound GT-05145 was obtained (3 mg, white solid, yield 4%). $^1$H NMR (400 MHz, DMSO-d6) δ 11.16 (s, 1H), 10.52 (s, 1H), 8.00 (d, *J* = 7.9 Hz, 1H), 7.90 (s, 1H), 7.78 (d, *J* = 7.7 Hz, 1H), 7.44 (d, *J* = 3.1 Hz, 1H), 7.20 (s, 1H), 6.04 - 5.91 (m, 1H), 5.79 (s, 1H), 4.89 (d, *J* = 20.5 Hz, 1H), 4.75 (d, *J* = 20.7 Hz, 1H), 4.63 - 4.49 (m, 2H), 3.86 - 3.70 (m, 2H), 3.66 - 3.59 (m, 1H), 3.06 - 2.91 (m, 2H), 2.85 - 2.76(m, 1H), 2.72 - 2.51 (m, 4H), 2.23 (s, 3H), 2.13 - 2.07 (m, 1H). LCMS (ESI) calcd. for $C_{24}H_{26}N_3O_2S_2^+$ [M+H]$^+$: 452.15, found, 452.1.

**Example 16: Preparation of 3-(5-((4-(furan-2-yl)-3,6-dihydropyridin-1(2H)-yl)methyl)-1-thioxoisoindolin-2-yl)piperidine-2,6-dione (GT-05147)**

With reference to the method of Scheme 4, the target compound GT-05147 was obtained (14 mg, white solid, yield 19%). $^1$H NMR (400 MHz, DMSO-d6) δ 11.15 (s, 1H), 10.65 (s, 1H), 7.99 (d, *J* = 8.0 Hz, 1H), 7.90 (s, 1H), 7.78 (d, *J* = 7.9 Hz, 1H), 7.68 (s, 1H), 6.59 - 6.48 (m, 2H), 6.09 (s, 1H), 5.99 - 5.91 (m, 1H), 4.88 (d, *J* = 20.1 Hz, 1H), 4.75 (d, *J* = 20.5 Hz, 1H), 4.66 - 4.48 (m, 2H), 3.86 - 3.71 (m, 2H), 3.67 - 3.63 (m, 1H), 3.05 - 2.89 (m, 2H), 2.78 - 2.65 (m, 3H), 2.63 - 2.57 (m, 1H), 2.13 - 2.09 (m, 1H). LCMS (ESI) calcd. for $C_{23}H_{24}N_3O_3S^+$ [M+H]$^+$: 422.15, found, 422.2.

**Example 17: Preparation of 3-(5-((3-(2,3-dichlorophenyl)-3,6-diazabicyclo[3.1.1]heptan-6-yl)methyl)-1-thioxoisoindolin-2-yl)piperidine-2,6-dione (GT-05182)**

With reference to the method of Scheme 4, the target compound GT-05182 was obtained (16 mg, white solid, yield 17%). $^1$H NMR (400 MHz, DMSO-d6) δ 11.14 (s, 1H), 8.00 - 7.93 (m, 1H), 7.90 - 7.87 (m, 1H), 7.83 (d, *J* = 8.4 Hz, 1H), 7.79 - 7.71 (m, 1H), 7.45 - 7.33 (m, 2H), 5.98 - 5.94 (m, 1H), 4.92 - 4.66 (m, 3H), 4.60 - 4.42 (m, 1H), 4.36 (d, *J* = 5.8 Hz, 1H), 3.91 (d, *J* = 12.7 Hz, 1H), 3.78 - 3.75 (m, 2H), 3.25 - 3.15 (m, 1H), 3.07 - 2.95 (m, 2H), 2.72 - 2.54 (m, 2H), 2.41 - 2.39 (m, 1H), 2.35 - 2.28 (m, 1H), 2.17 - 2.02 (m, 1H). LCMS (ESI) calcd. for $C_{25}H_{25}Cl_2N_4O_2S^+$ [M+H]$^+$: 515.11, found, 515.1.

**Example 18: Preparation of 3-(5-(((1S,4S)-5-(2,3-dichlorophenyl)-2,5-diazabicyclo[2.2.1]heptan-2-yl)methyl)-1-thioxoisoindolin-2-yl)piperidine-2,6-dione (GT-05183)**

With reference to the method of Scheme 4, the target compound GT-05183 was obtained (18 mg, white solid, yield 19%). $^1$H NMR (400 MHz, DMSO-d6) δ 11.14 (s, 1H), 9.93 (s, 1H), 7.98 - 7.92 (m, 2H), 7.83 - 7.78 (m, 1H), 7.19 - 7.17 (m, 1H), 7.12 - 7.10 (m, 1H), 6.97 (d, *J* = 8.6 Hz, 1H), 6.02 - 5.89 (m, 1H), 4.87 - 4.74 (m, 1H), 4.71 (brs, 1H), 4.54 (brs, 1H), 4.48 - 4.43 (m, 1H), 4.40 (brs, 1H), 3.97 - 3.90 (m, 1H), 3.71 - 3.55 (m, 2H), 3.26 - 3.19 (m, 3H), 3.04 - 2.91 (m, 1H), 2.68 - 2.66 (m, 1H), 2.63 - 2.56 (m, 2H), 2.19 - 2.16 (m, 1H), 2.14 - 2.03 (m, 1H). LCMS (ESI) calcd. for $C_{25}H_{25}Cl_2N_4O_2S^+$ [M+H]$^+$: 515.11, found, 515.1.

**Example 19: Preparation of 3-(5-((4-(6-fluorobenzo[d]isoxazol-3-yl)piperidin-1-yl)methyl)-1-thioxoisoindolin-2-yl)piperidine-2,6-dione (GT-05089)**

With reference to the method of Scheme 4, the target compound GT-05089 was obtained (35 mg, white solid, yield 38%). $^1$H NMR (400 MHz, DMSO-d6) δ 11.15 (s, 1H), 8.15 (brs, 1H), 8.04 - 7.91 (m, 2H), 7.86 - 7.82 (m, 1H), 7.72 (dd, *J* = 9.0, 2.0 Hz, 1H), 7.34 (td, *J* = 9.1, 2.0 Hz, 1H), 5.96 (d, *J* = 13.1 Hz, 1H), 4.90 (d, *J* = 20.5 Hz, 1H), 4.78 (d, *J* = 20.5 Hz, 1H), 4.63 (s, 1H), 4.52 (d, *J* = 4.5 Hz, 2H), 3.55 (d, *J* = 11.1 Hz, 2H), 3.48 - 3.42 (m, 1H), 3.17 - 3.13 (m, 2H), 3.06 - 2.86 (m, 1H), 2.67 - 2.63 (m, 2H), 2.67 - 2.58 (m, 1H), 2.33 - 2.30 (m, 1H), 2.15 - 2.03 (m, 1H). LCMS (ESI) calcd. for $C_{26}H_{26}FN_4O_3S^+$ [M+H]$^+$: 493.17, found, 493.2.

**Example 20: Preparation of 3-(5-((4-(6-fluoro-1H-indol-3-yl)piperidin-1-yl)methyl)-1-thioxoisoindolin-2-yl)piperidine-2,6-dione (GT-05243)**

With reference to the method of Scheme 4, the target compound GT-05243 was obtained (10 mg, white solid, yield 14%). $^1$H NMR (400 MHz, DMSO-d6) δ 11.15 (s, 1H), 10.95 (s, 1H), 10.22 (s, 1H), 8.01 (d, *J* = 8.0 Hz, 1H), 7.91 (s, 1H), 7.78 (d, *J* = 8.2 Hz, 1H), 7.68 - 7.59 (m, 1H), 7.14 - 7.09 (m, 2H), 6.84 (t, *J* = 8.1 Hz, 1H), 5.98 - 5.95 (m, 1H), 4.90 (d, *J* = 20.3 Hz, 1H), 4.76 (d, *J* = 20.0 Hz, 1H), 4.50 (d, *J* = 4.3 Hz, 2H), 3.48 (d, *J* = 9.8 Hz, 2H), 3.16 - 3.10 (m, 3H), 3.04 - 2.95 (m, 3H), 2.65 (d, *J* = 15.4 Hz, 2H), 2.16 - 2.01 (m, 3H). LCMS (ESI) calcd. for $C_{27}H_{28}FN_4O_2S^+$ [M+H]$^+$: 491.19, found, 491.2.

**Example 21: Preparation of 3-(5-((4-(5-fluoroindolin-1-yl)piperidin-1-yl)methyl)-1-thioxoisoindolin-2-yl)piperidine-2,6-dione (GT-05244)**

With reference to the method of Scheme 4, the target compound GT-05244 was obtained (8 mg, white solid, yield 9%). $^1H$ NMR (400 MHz, DMSO-d6) δ 11.14 (s, 1H), 10.51 (s, 1H), 7.98 (d, *J* = 8.0 Hz, 1H), 7.90 (s, 1H), 7.76 (d, *J* = 8.2 Hz, 1H), 6.90 (d, *J* = 6.5 Hz, 1H), 6.79 (t, *J* = 9.1 Hz, 1H), 6.47 (dd, *J* = 8.6, 4.3 Hz, 1H), 5.99 - 5.92 (m, 1H), 4.88 (d, *J* = 20.4 Hz, 1H), 4.75 (d, *J* = 20.4 Hz, 1H), 4.44 (d, *J* = 4.7 Hz, 2H), 3.68 - 3.60 (m, 1H), 3.28 (t, *J* = 8.3 Hz, 3H), 3.05 - 2.94 (m, 3H), 2.87 (t, *J* = 8.3 Hz, 2H), 2.67 - 2.63 (m, 2H), 2.14 - 2.08 (m, 2H), 2.02 - 1.94 (m, 2H), 1.84 - 1.81 (m, 2H). LCMS (ESI) calcd. for $C_{27}H_{30}FN_4O_2S^+$ [M+H]$^+$: 493.21, found, 493.2.

**Example 22: Preparation of 3-(5-((4-(7-fluoroquinolin-4-yl)-3,6-dihydropyridin-1(2H)-yl)methyl)-1-thioxoisoindolin-2-yl)piperidine-2,6-dione (GT-05245)**

With reference to the method of Scheme 4, the target compound GT-05245 was obtained (7 mg, white solid, yield 8%). $^1H$ NMR (400 MHz, DMSO-d6) δ 11.34 (s, 1H), 11.15 (s, 1H), 8.98 (d, *J* = 4.6 Hz, 1H), 8.41 - 8.31 (m, 1H), 8.02 (d, *J* = 3.2 Hz, 1H), 8.01 - 7.93 (m, 1H), 7.91 - 7.81 (m, 2H), 7.66 - 7.55 (m, 1H), 7.46 (d, *J* = 4.4 Hz, 1H), 5.99 (d, *J* = 6.7 Hz, 1H), 5.88 (s, 1H), 4.91 (d, *J* = 20.5 Hz, 1H), 4.77 (d, *J* = 20.6 Hz, 1H), 4.67 - 4.63 (m, 2H), 3.87 (brs, 2H), 3.42 - 3.41 (m, 2H), 3.28 - 3.13 (m, 1H), 3.08 - 2.91 (m, 2H), 2.77 - 2.61 (m, 2H), 2.19 - 2.05 (m, 1H). LCMS (ESI) calcd. for $C_{28}H_{26}FN_4O_2S^+$ [M+H]$^+$: 501.18, found, 501.1.

Example 23: Preparation of 3-(5-((4-(isoquinolin-5-yl)-3,6-dihydropyridin-1(2H)-yl)methyl)-1-thioxoisoindolin-2-yl)piperidine-2,6-dione (GT-05246)

With reference to the method of Scheme 4, the target compound GT-05246 was obtained (8 mg, white solid, yield 9%). $^1H$ NMR (400 MHz, DMSO-d6) δ 11.12 (s, 1H), 10.35 (s, 1H), 9.69 (s, 2H), 8.92 (d, *J* = 7.1 Hz, 1H), 8.85 (d, *J* = 6.9 Hz, 1H), 8.50 (dd, *J* = 5.8, 3.6 Hz, 1H), 8.09 (dd, *J* = 6.5, 2.9 Hz, 2H), 7.95 (d, *J* = 8.0 Hz, 1H), 7.91 (s, 1H), 7.81 (d, *J* = 8.0 Hz, 1H), 6.15 (s, 2H), 5.93 (d, *J* = 8.4 Hz, 1H), 5.86 (s, 1H), 4.84 (d, *J* = 20.5 Hz, 1H), 4.69 (d, *J* = 20.4 Hz, 1H), 3.78 (s, 2H), 3.42 - 3.39 (m, 2H), 3.04 - 2.88 (m, 1H), 2.77 - 2.60 (m, 3H), 2.14 - 2.02 (m, 1H). LCMS (ESI) calcd. for $C_{28}H_{27}N_4O_2S^+$ [M+H]$^+$: 483.18, found, 483.2.

**Example 24: Preparation of 3-(5-((4-(6-fluoro-1-methyl-1H-indol-3-yl)-3,6-dihydropyridin-1(2H)-yl)methyl)-1-thioxoisoindolin-2-yl)piperidine-2,6-dione (GT-05247)**

With reference to the method of Scheme 4, the target compound GT-05247 was obtained (9 mg, white solid, yield 10%). LCMS (ESI) calcd. for $C_{28}H_{28}FN_4O_2S^+$ [M+H]$^+$: 503.19, found, 503.2.

**Example 25: Preparation of 3-(5-((4-(1H-pyrrolo[2,3-b]pyridin-3-yl)-3,6-dihydropyridin-1(2H)-yl)methyl)-1-thioxoisoindolin-2-yl)piperidine-2,6-dione (GT-05248)**

With reference to the method of Scheme 4, the target compound GT-05248 was obtained (7 mg, white solid, yield 8%). $^1H$ NMR (400 MHz, DMSO-d6) δ 11.89 (s, 1H), 11.15 (s, 1H), 10.47 (s, 1H), 8.24 (t, *J* = 6.6 Hz, 2H), 8.01 (d, *J* = 7.7 Hz, 1H), 7.91 (s, 1H), 7.79 (d, *J* = 7.9 Hz, 1H), 7.68 (s, 1H), 7.13 (dd, *J* = 8.0, 4.8 Hz, 1H), 6.18 (s, 1H), 6.02 - 5.95 (m, 1H), 4.90 (d, *J* = 20.7 Hz, 1H), 4.76 (d, *J* = 19.7 Hz, 1H), 4.67 - 4.54 (m, 2H), 3.86 - 3.81 (m, 2H), 3.69 - 3.66 (m, 1H), 2.99 -2.97 (m, 2H), 2.88 - 2.84 (m, 2H), 2.69 -2.67 (m, 1H), 2.13 - 2.06 (m, 2H). LCMS (ESI) calcd. for $C_{26}H_{26}N_5O_2S^+$ [M+H]$^+$: 472.18, found, 472.2.

**Example 26: Preparation of 3-(5-((4-(benzo[d]isothiazol-3-yl)piperazin-1-yl)methyl)-1-thioxoisoindolin-2-yl)piperidine-2,6-dione (GT-05091)**

With reference to the method of Scheme 4, the target compound GT-05091 was obtained (38 mg, white solid, yield 43%). $^1H$ NMR (400 MHz, DMSO-d6) δ 11.15 (s, 1H), 11.04 (s, 1H), 8.11 (t, *J* = 8.0 Hz, 2H), 8.00 (d, *J* = 7.9 Hz, 1H), 7.94 (s, 1H), 7.80 (d, *J* = 7.8 Hz, 1H), 7.59 (t, *J* = 7.7 Hz, 1H), 7.47 (dd, *J* = 14.1, 7.0 Hz, 1H), 5.97 (d, *J* = 7.5 Hz, 1H), 4.89 (d, *J* = 20.4 Hz, 1H), 4.76 (d, *J* = 20.9 Hz, 1H), 4.58 (brs, 2H), 4.08 (d, *J* = 12.9 Hz, 2H), 3.48 - 3.42 (m, 4H), 3.06 - 2.88 (m, 2H), 2.67 - 2.61 (m, 2H), 2.57 - 2.53 (m, 1H), 2.15 - 2.01 (m, 1H). LCMS (ESI) calcd. for $C_{25}H_{26}N_5O_2S_2^+$ [M+H]$^+$: 492.15, found, 492.1.

**Example 27: Preparation of 3-(5-((4-(benzo[b]thiophen-4-yl)piperazin-1-yl)methyl)-1-thioxoisoindolin-2-yl)piperidine-2,6-dione (GT-05088)**

With reference to the method of Scheme 4, the target compound GT-05088 was obtained (39 mg, white solid, yield 43%). $^1$H NMR (400 MHz, DMSO-d6) δ 11.16 (s, 1H), 10.94 (s, 1H), 8.01 (d, *J* = 7.9 Hz, 1H), 7.96 (s, 1H), 7.82 (d, *J* = 8.1 Hz, 1H), 7.76 (d, *J* = 5.5 Hz, 1H), 7.70 (d, *J* = 8.1 Hz, 1H), 7.48 (d, *J* = 5.5 Hz, 1H), 7.31 (t, *J* = 7.8 Hz, 1H), 6.96 (d, *J* = 7.6 Hz, 1H), 5.97 (d, *J* = 8.2 Hz, 1H), 4.90 (d, *J* = 20.4 Hz, 1H), 4.76 (d, *J* = 20.3 Hz, 1H), 4.60 (d, *J* = 4.5 Hz, 2H), 3.59 - 3.51 (m, 4H), 3.41 - 3.38 (m, 2H), 3.22 - 3.16 (m, 2H), 3.05 - 2.90 (m, 1H), 2.67 - 2.59 (m, 1H), 2.59 - 2.51 (m, 1H), 2.17 - 2.04 (m, 1H). LCMS (ESI) calcd. for $C_{26}H_{27}N_4O_2S_2^+$ $[M+H]^+$: 491.16, found, 491.2.

**Example 28: Preparation of 3-(5-((4-(2-((2,4-dimethylphenyl)thio)phenyl)piperazin-1-yl)methyl)-1-thioxoisoindolin-2-yl)piperidine-2,6-dione (GT-05092)**

With reference to the method of Scheme 4, the target compound GT-05092 was obtained (38 mg, white solid, yield 36%). $^1$H NMR (400 MHz, DMSO-d6) δ 11.14 (s, 1H), 10.84 (s, 1H), 8.00 (d, *J* = 7.9 Hz, 1H), 7.94 (s, 1H), 7.81 (d, *J* = 8.0 Hz, 1H), 7.32 (d, *J* = 7.8 Hz, 1H), 7.23 (s, 1H), 7.20 - 7.13 (m, 2H), 7.10 (d, *J* = 8.0 Hz, 1H), 7.02 - 6.89 (m, 1H), 6.41 (d, *J* = 7.6 Hz, 1H), 5.97 (d, *J* = 8.1 Hz, 1H), 4.90 (d, *J* = 20.4 Hz, 1H), 4.76 (d, *J* = 20.4 Hz, 1H), 4.58 (d, *J* = 4.1 Hz, 2H), 3.49 - 3.46 (m, 2H), 3.41 - 3.39 (m, 2H), 3.28 - 3.10 (m, 5H), 3.03 - 2.89 (m, 1H), 2.68 - 2.63 (m, 1H), 2.59 - 2.56 (m, 1H), 2.32 (s, 3H), 2.23 (s, 3H), 2.14 - 2.07 (m, 1H). LCMS (ESI) calcd. for $C_{32}H_{35}N_4O_2S_2^+$ $[M+H]^+$: 571.22, found, 571.2.

**Example 29: Preparation of 3-(1-thioxo-5-((3-(trifluoromethyl)-5,6-dihydro-[1,2,4]triazolo[4,3-a]pyrazin-7(8H)-yl)methyl)isoindolin-2-yl)piperidine-2,6-dione (GT-05090)**

With reference to the method of Scheme 4, the target compound GT-05090 was obtained (30 mg, white solid, yield 35%). $^1$H NMR (400 MHz, DMSO-d6) δ 11.12 (s, 1H), 7.90 (d, *J* = 7.9 Hz, 1H), 7.70 (s, 1H), 7.58 (d, *J* = 8.0 Hz, 1H), 5.96 (d, *J* = 8.9 Hz, 1H), 4.83 (d, *J* = 20.1 Hz, 1H), 4.69 (d, *J* = 20.2 Hz, 1H), 4.19 (t, *J* = 5.4 Hz, 2H), 3.98 (d, *J* = 5.4 Hz, 4H), 3.02 - 2.97 (m, 2H), 2.96 - 2.92 (m, 1H), 2.67 - 2.62 (m, 1H), 2.51 - 2.49 (m, 1H), 2.15 - 2.01 (m, 1H). LCMS (ESI) calcd. for $C_{20}H_{20}F_3N_6O_2S^+$ $[M+H]^+$: 465.13, found, 465.1.

**Example 30: Preparation of 3-(5-((6,7-dihydrothieno[3,2-c]pyridin-5(4H)-yl)methyl)-1-thioxoisoindolin-2-yl)piperidine-2,6-dione (GT-05093)**

With reference to the method of Scheme 4, the target compound GT-05093 was obtained (27 mg, white solid, yield 35%). $^1$H NMR (400 MHz, DMSO-d6) δ 11.16 (s, 1H), 11.03 (s, 1H), 7.99 (d, *J* = 7.9 Hz, 1H), 7.93 (s, 1H), 7.81 (s, 1H), 7.46 (d, *J* = 5.0 Hz, 1H), 6.89 (d, *J* = 5.0 Hz, 1H), 5.97 (d, *J* = 7.6 Hz, 1H), 4.89 (d, *J* = 20.2 Hz, 1H), 4.75 (d, *J* = 20.8 Hz, 1H), 4.72 - 4.51 (m, 2H), 4.22 (brs, 2H), 3.82 - 3.76 (m, 1H), 3.47 - 3.40 (m, 2H), 3.15 (t, *J* = 16.4 Hz, 2H), 3.06 - 2.90 (m, 1H), 2.65 (d, *J* = 17.0 Hz, 1H), 2.57 (d, *J* = 12.3 Hz, 1H), 2.21 - 1.99 (m, 1H). LCMS (ESI) calcd. for $C_{21}H_{22}N_3O_2S_2^+$ $[M+H]^+$: 412.11, found, 412.1.

**Example 31: Preparation of 3-(6-((4-(phenyl(pyridin-2-yl)methyl)piperazin-1-yl)methyl)-1-thioxoisoindolin-2-yl)piperidine-2,6-dione (GT-05041)**

With reference to the method of Scheme 4, the target compound GT-05041 was obtained (8 mg, white solid, yield 8%). $^1$H NMR (400 MHz, DMSO-d6) δ 11.13 (s, 1H), 8.66 (d, *J* = 4.1 Hz, 1H), 8.04 - 8.00 (m, 1H), 7.91 (d, *J* = 7.7 Hz, 1H), 7.78 - 7.70 (m, 2H), 7.68 (t, *J* = 7.6 Hz, 1H), 7.55 (d, *J* = 7.3 Hz, 2H), 7.49 (d, *J* = 11.5 Hz, 1H), 7.39 (t, *J* = 7.4 Hz, 2H), 7.33 (d, *J* = 7.1 Hz, 1H), 5.43 - 5.34 (m, 1H), 4.88 (d, *J* = 20.6 Hz, 1H), 4.73 (d, *J* = 19.9 Hz, 1H), 4.53 - 4.50 (m, 2H), 3.39 - 3.27 (m, 4H), 3.25 - 3.21 (m, 3H), 3.01 - 2.94 (m, 4H), 2.14 - 1.99 (m, 1H), 1.36 - 1.33 (m, 1H). LCMS (ESI) calcd. for $C_{30}H_{32}N_5O_2S^+$ $[M+H]^+$: 526.23, found, 526.3.

**Example 32: Preparation of 3-(6-((4-(2,3-dichlorophenyl)piperazin-1-yl)methyl)-1-thioxoisoindolin-2-yl)piperidine-2,6-dione (GT-05033)**

With reference to the method of Scheme 4, the target compound GT-05033 was obtained (17 mg, white solid, yield 19%). $^1$H NMR (400 MHz, DMSO-d6) δ 11.13 (s, 1H), 10.42 (brs, 1H), 8.17 (s, 1H), 7.90 (d, *J* = 6.9 Hz, 1H), 7.79 (d, *J* = 7.9 Hz, 1H), 7.39 - 7.33 (m, 2H), 7.20 (d, *J* = 7.0 Hz, 1H), 6.02 - 5.89 (m, 1H), 4.89 (d, *J* = 20.2 Hz, 1H), 4.75 (d, *J* = 20.8 Hz, 1H), 4.61 (brs, 2H), 3.45 - 3.40 (m, 4H), 3.11 - 3.02 (m, 3H), 2.98 - 2.94 (m, 2H), 2.68 - 2.61 (m, 2H), 2.15 - 2.02 (m, 1H). LCMS (ESI) calcd. for $C_{24}H_{25}Cl_2N_4O_2S^+$ $[M+H]^+$: 503.11, found, 503.1.

**Example 33: Preparation of 3-(6-((4-(3-methylthiophen-2-yl)-3,6-dihydropyridin-1(2H)-yl)methyl)-1-thioxoisoindolin-2-yl)piperidine-2,6-dione (GT-05032)**

With reference to the method of Scheme 4, the target compound GT-05032 was obtained (17mg, white solid, yield 20%). $^1$H NMR (400 MHz, DMSO-d6) δ 11.14 (s, 1H), 10.37 (s, 1H), 8.17 (s, 1H), 7.88 (d, *J* = 7.8 Hz, 1H), 7.79 (d, *J* = 7.7 Hz, 1H), 7.40 (d, *J* = 5.1 Hz, 1H), 6.92 (d, *J* = 5.0 Hz, 1H), 6.02 - 5.90 (m, 1H), 5.85 (s, 1H), 4.83 (dd, *J* = 57.9, 20.3 Hz, 2H), 4.66 - 4.52 (m, 2H), 3.83 - 3.74 (m, 2H), 3.67 - 3.53 (m, 1H), 3.04 - 2.89 (m, 2H), 2.86 - 2.73 (m, 2H), 2.73 - 2.61 (m, 3H), 2.24 (s, 3H), 2.16 - 2.05 (m, 1H). LCMS (ESI) calcd. for $C_{24}H_{26}N_3O_2S_2^+$ [M+H]$^+$: 452.15, found, 452.1.

**Example 34: Preparation of 3-(6-((4-(6-fluorobenzo[d]isoxazol-3-yl)piperidin-1-yl)methyl)-1-thioxoisoindolin-2-yl)piperidine-2,6-dione (GT-05031)**

With reference to the method of Scheme 4, the target compound GT-05031 was obtained (9 mg, white solid, yield 10%). $^1$H NMR (400 MHz, DMSO-d6) δ 11.13 (s, 1H), 10.14 (s, 1H), 8.16 (s, 1H), 8.13 - 8.04 (m, 1H), 7.89 (d, *J* = 8.0 Hz, 1H), 7.80 (d, *J* = 7.8 Hz, 1H), 7.77 - 7.67 (m, 1H), 7.34 (t, *J* = 9.4 Hz, 1H), 5.98 - 5.94 (m, 1H), 4.90 (d, *J* = 20.3 Hz, 1H), 4.76 (d, *J* = 19.9 Hz, 1H), 4.57 - 4.51 (m, 2H), 3.61 - 3.50 (m, 2H), 3.19 - 3.10 (m, 2H), 2.97 - 2.92 (m, 1H), 2.68 - 2.61 (m, 2H), 2.37 - 2.24 (m, 3H), 2.21 - 2.03 (m, 3H). LCMS (ESI) calcd. for $C_{26}H_{26}FN_4O_3S^+$ [M+H]$^+$: 493.17, found, 493.2.

**Example 35: Preparation of 3-(6-((4-(benzo[d]isothiazol-3-yl)piperazin-1-yl)methyl)-1-thioxoisoindolin-2-yl)piperidine-2,6-dione (GT-05040)**

With reference to the method of Scheme 4, the target compound GT-05040 was obtained (6 mg, white solid, yield 7%). $^1$H NMR (400 MHz, DMSO-d6) δ 11.14 (s, 1H), 8.17 - 8.10 (m, 3H), 7.86 - 7.82 (m, 2H), 7.60 (t, *J* = 7.7 Hz, 1H), 7.47 (t, *J* = 7.3 Hz, 1H), 6.01 - 5.95 (m, 1H), 4.90 (d, *J* = 20.1 Hz, 1H), 4.76 (d, *J* = 20.2 Hz, 1H), 4.63 (brs, 2H), 4.12 (d, *J* = 12.0 Hz, 2H), 3.52 - 3.46 (m, 3H), 3.10 - 2.84 (m, 2H), 2.74 - 2.57 (m, 2H), 2.15 - 2.09 (m, 2H), 1.41 - 1.26 (m, 1H). LCMS (ESI) calcd. for $C_{25}H_{26}N_5O_2S_2^+$ [M+H]$^+$: 492.15, found, 492.2.

**Example 36: Preparation of 3-(6-((4-(benzo[b]thiophen-4-yl)piperazin-1-yl)methyl)-1-thioxoisoindolin-2-yl)piperidine-2,6-dione (GT-05035)**

With reference to the method of Scheme 4, the target compound GT-05035 was obtained (20 mg, white solid, yield 24%). $^1$H NMR (400 MHz, DMSO-d6) δ 11.16 (s, 1H), 8.18 (s, 1H), 7.92 (d, *J* = 7.0 Hz, 1H), 7.81 (d, *J* = 7.9 Hz, 1H), 7.77 (d, *J* = 5.5 Hz, 1H), 7.71 (d, *J* = 8.0 Hz, 1H), 7.50 (d, *J* = 5.6 Hz, 1H), 7.31 (t, *J* = 7.9 Hz, 1H), 6.96 (d, *J* = 7.6 Hz, 1H), 6.02 - 5.93 (m, 1H), 4.90 (d, *J* = 20.9 Hz, 1H), 4.76 (d, *J* = 20.4 Hz, 1H), 4.64 (d, *J* = 4.7 Hz, 2H), 3.57 (d, *J* = 12.7 Hz, 2H), 3.46 (t, *J* = 9.8 Hz, 4H), 3.13 (d, *J* = 11.8 Hz, 2H), 3.04 - 2.91 (m, 1H), 2.67 - 2.57 (m, 2H), 2.19 - 2.03 (m, 1H). LCMS (ESI) calcd. for $C_{26}H_{27}N_4O_2S_2^+$ [M+H]$^+$: 491.16, found, 491.2.

**Example 37: Preparation of 3-(6-((4-(2-((2,4-dimethylphenyl)thio)phenyl)piperazin-1-yl)methyl)-1-thioxoisoindolin-2-yl)piperidine-2,6-dione (GT-05038)**

With reference to the method of Scheme 4, the target compound GT-05038 was obtained (17 mg, white solid, yield 16%). $^1$H NMR (400 MHz, DMSO-d6) δ 11.16 (s, 1H), 10.31 (s, 1H), 7.90 (d, *J* = 7.0 Hz, 1H), 7.80 (d, *J* = 8.0 Hz, 1H), 7.33 (d, *J* = 7.8 Hz, 1H), 7.24 (s, 1H), 7.13 (dd, *J* = 17.0, 6.2 Hz, 4H), 7.02 - 6.93 (m, 1H), 6.40 (d, *J* = 8.1 Hz, 1H), 4.90 (d, *J* = 20.8 Hz, 1H), 4.76 (d, *J* = 20.6 Hz, 1H), 4.65 - 4.60 (m, 2H), 3.54 - 3.39 (m, 4H), 3.15 - 3.09 (m, 3H), 3.01 - 2.90 (m, 1H), 2.64 - 2.61 (m, 1H), 2.32 (s, 3H), 2.23 (s, 3H), 2.15 - 2.03 (m, 1H). LCMS (ESI) calcd. for $C_{32}H_{35}N_4O_2S_2^+$ [M+H]$^+$: 571.22, found, 571.2.

**Example 38: Preparation of 3-(6-((6,7-dihydrothieno[3,2-c]pyridin-5(4H)-yl)methyl)-1-thioxoisoindolin-2-yl)piperidine-2,6-dione (GT-05039)**

With reference to the method of Scheme 4, the target compound GT-05039 was obtained (6 mg, white solid, yield 8%). $^1$H NMR (400 MHz, DMSO-d6) δ 11.14 (s, 1H), 7.99 (d, *J* = 7.9 Hz, 1H), 7.93 (s, 1H), 7.81 (s, 1H), 7.46 (d, *J* = 5.0 Hz, 1H), 6.89 (d, *J* = 5.0 Hz, 1H), 4.87 (d, *J* = 20.8 Hz, 1H), 4.75 (d, *J* = 20.8 Hz, 1H), 4.70 - 4,62 (m, 1H), 4.27 - 4.22 (m, 1H), 3.81 - 3.77 (m, 1H), 3.46 - 3.42(m, 2H), 3.15 - 3.11 (m, 3H), 3.05 - 2.78 (m, 2H), 2.66 - 2.62 (m, 2H), 2.13 - 2.07 (m, 1H). LCMS (ESI) calcd. for $C_{21}H_{22}N_3O_2S_2^+$ [M+H]$^+$: 412.11, found, 412.1.

**Example 39: Preparation of 3-(4-((4-(6-fluorobenzo[d]isoxazol-3-yl)piperidin-1-yl)methyl)-1-thioxoisoindolin-2-yl)piperidine-2,6-dione (GT-04997)**

With reference to the method of Scheme 4, the target compound GT-04997 was obtained (14 mg, white solid, yield 15%). $^1$H NMR (400 MHz, DMSO-d6) δ 11.21 (s, 1H), 10.48 (s, 1H), 8.15 (dd, *J* = 8.7, 5.4 Hz, 1H), 8.02 (d, *J* = 7.7 Hz, 1H), 7.95 (d, *J* = 7.7 Hz, 1H), 7.73 - 7.69 (m, 2H), 7.34 (t, *J* = 8.2 Hz, 1H), 6.11 - 5.93 (m, 1H), 5.23 (d, *J* = 20.1 Hz, 1H), 4.93 (d, *J* = 20.4 Hz, 1H), 4.56 - 4.43 (m, 2H), 3.70 - 3.52 (m, 2H), 3.49 - 3.43 (m, 2H), 3.02 - 2.99 (m, 1H), 2.77 - 2.63 (m, 2H), 2.47 - 2.43 (m, 1H), 2.31 - 2.19 (m, 4H), 2.19 - 2.10 (m, 1H). LCMS (ESI) calcd. for $C_{26}H_{26}FN_4O_3S^+$ [M+H]$^+$: 493.17, found, 493.2.

**Example 40: Preparation of 3-(4-((4-(thieno[2,3-d]pyrimidin-4-yl)piperazin-1-yl)methyl)-1-thioxoisoindolin-2-yl) piperidine-2,6-dione (GT-08912)**

With reference to the method of Scheme 4, the target compound (GT-08912) was obtained (white solid, 10 mg, yield 24 %). $^1$H NMR (400 MHz, DMSO) δ 11.88 (s, 1H), 11.20 (s, 1H), 8.55 (s, 1H), 8.02 (dd, *J* = 10.7, 7.7 Hz, 2H), 7.77 (d, *J* = 6.1 Hz, 1H), 7.72 - 7.68 (m, 2H), 6.01 (d, *J* = 7.9 Hz, 1H), 5.41 (d, *J* = 20.4 Hz, 1H), 4.92 (d, *J* = 20.3 Hz, 1H), 4.70 (d, *J* = 13.3 Hz, 2H), 4.53 (d, *J* = 13.1 Hz, 1H), 4.43 (d, *J* = 13.0 Hz, 1H), 3.55 (d, *J* = 10.9 Hz, 1H), 3.41 (d, 1H), 3.33 (s, 2H), 3.04 - 2.94 (m, 1H), 2.69 (d, *J* = 16.3 Hz, 1H), 2.47 - 2.39 (m, 1H), 2.15 -2.09 (m, 1H). LCMS (ESI) calcd. for $C_{24}H_{25}N_6O_2S_2{}^+$ [M+H]$^+$: 493.15, found, 493.1.

**Example 41: Preparation of 3-(4-((4-(5-methylthieno[2,3-d]pyrimidin-4-yl)piperazin-1-yl)methyl)-1-thioxoisoindolin-2-yl)piperidine-2,6-dione (GT-08913)**

With reference to the method of Scheme 4, the target compound (GT-08913) was obtained (white solid, 6 mg, yield 14 %). $^1$H NMR (400 MHz, DMSO) δ 11.51 (s, 1H), 11.21 (s, 1H), 8.62 (s, 1H), 8.06 - 7.97 (m, 2H), 7.69 (t, *J* = 7.7 Hz, 1H), 7.48 (s, 1H), 6.02 (d, *J* = 8.8 Hz, 1H), 5.40 (d, *J* = 20.3 Hz, 1H), 4.91 (d, 1H), 4.59 - 4.39 (m, 2H), 3.93 (d, *J* = 13.5 Hz, 2H), 3.66 - 3.58 (m, 2H), 3.40 (s, 4H), 3.02 - 2.94 (m, 1H), 2.69 (d, *J* = 17.2 Hz, 1H), 2.56 (s, 3H), 2.47 - 2.41 (m, 1H), 2.15 - 2.08 (m, 1H). LCMS (ESI) calcd. for $C_{25}H_{27}N_6O_2S_2{}^+$ [M+H]$^+$: 507.16, found, 507.2 .

**Example 42: Preparation of 3-(4-((4-(6-methylthieno[2,3-d]pyrimidin-4-yl)piperazin-1-yl)methyl)-1-thioxoisoindolin-2-yl)piperidine-2,6-dione (GT-08914)**

With reference to the method of Scheme 4, the target compound (GT-08914) was obtained (white solid, 11 mg, yield 26 %). $^1$HNMR (400 MHz, DMSO) δ 11.60 (s, 1H), 11.20 (s, 1H), 8.47 (s, 1H), 8.01 (d, *J* = 7.7 Hz, 2H), 7.69 (t, *J* = 7.7 Hz, 1H), 7.40 (s, 1H), 6.01 (d, *J* = 9.1 Hz, 1H), 5.36 (d, *J* = 20.3 Hz, 1H), 4.91 (d, *J* = 20.3 Hz, 1H), 4.63 (d, *J* = 15.7 Hz, 2H), 4.48 (dd, *J* = 33.1, 13.4 Hz, 2H), 3.70 (dd, *J* = 28.4, 14.0 Hz, 2H), 3.43 - 3.21 (m, 4H), 3.05 - 2.93 (m, 1H), 2.69 (d, *J* = 17.1 Hz, 1H), 2.57 (s, 3H), 2.48 - 2.40 (m, 1H), 2.16 - 2.08 (m, 1H). LCMS (ESI) calcd. for $C_{25}H_{27}N_6O_2S_2{}^+$ [M+H]$^+$: 507.16, found, 507.2.

**Example 43: Preparation of 3-(4-((4-(6-isopropylthieno[2,3-d]pyrimidin-4-yl)piperazin-1-yl)methyl)-1-thioxoisoindolin-2-yl)piperidine-2,6-dione (GT-08915)**

With reference to the method of Scheme 4, the target compound (GT-08915) was obtained (white solid, 14 mg, yield 31 %). $^1$H NMR (400 MHz, DMSO) δ 11.67 (s, 1H), 11.20 (s, 1H), 8.49 (s, 1H), 8.02 (dd, *J* = 11.1, 7.7 Hz, 2H), 7.72 - 7.67 (m, 1H), 7.36 (s, 1H), 6.01 (d, *J* = 9.5 Hz, 1H), 5.39 (d, *J* = 20.3 Hz, 1H), 4.92 (d, *J* = 20.4 Hz, 1H), 4.64 (d, *J*= 13.6 Hz, 2H), 4.49 (dd, *J* = 33.6, 12.9 Hz, 2H), 3.69 - 3.59 (m, 2H), 3.54 - 3.40 (m, 2H), 3.34 - 3.25 (m, 3H), 3.05 - 2.94 (m, 1H), 2.67 (d, 1H), 2.48 - 2.37 (m, 1H), 2.16 - 2.09 (m, 1H), 1.34 (d, *J* = 6.8 Hz, 6H). LCMS (ESI) calcd. for $C_{27}H_{31}N_6O_2S_2{}^+$ [M+H]$^+$: 535.19, found, 535.2.

**Example 44: Preparation of 3-(4-((4-(5,6-dimethylthieno[2,3-d]pyrimidin-4-yl)piperazin-1-yl)methyl)-1-thioxoisoindolin-2-yl)piperidine-2,6-dione (GT-08916)**

With reference to the method of Scheme 4, the target compound (GT-08916) was obtained (white solid, 15 mg, yield 34 %). $^1$HNMR (400 MHz, DMSO) δ 11.64 (s, 1H), 11.21 (s, 1H), 8.56 (s, 1H), 8.02 (dd, *J* = 16.4, 7.6 Hz, 2H), 7.68 (t, *J* = 7.7 Hz, 1H), 6.02 (d, *J* = 9.4 Hz, 1H), 5.44 (d, *J* = 20.4 Hz, 1H), 4.97 - 4.87 (m, 1H), 4.51 (dd, *J* = 36.0, 12.8 Hz, 2H), 3.88 (d, *J* = 12.0 Hz, 2H), 3.62 - 3.52 (m, 4H), 3.38 (s, 3H), 3.03 - 2.95 (m, 1H), 2.67 (d, 1H), 2.46 (s, 3H), 2.43 (s, 3H), 2.15 - 2.09 (m, 1H). LCMS (ESI) calcd. for $C_{26}H_{29}N_6O_2S_2{}^+$ [M+H]$^+$: 521.18, found, 521.2.

**Example 45: Preparation of 3-(4-((4-(5,6-dimethylthieno[2,3-d]pyrimidin-4-yl)-1,4-diazepan-1-yl)methyl)-1-thioxoisoindolin-2-yl)piperidine-2,6-dione (GT-08917)**

With reference to the method of Scheme 4, the target compound (GT-08917) was obtained (white solid, 6 mg, yield 14 %). $^1$H NMR (400 MHz, DMSO) δ 11.25 (s, 1H), 11.20 (s, 1H), 8.45 (s, 1H), 8.01 (dd, *J* = 15.4, 7.6 Hz, 2H), 7.67 (t, *J* = 7.6 Hz, 1H), 6.01 (d, *J* = 8.5 Hz, 1H), 5.28 (d, *J* = 20.5 Hz, 1H), 4.87 (d, *J* = 20.2 Hz, 1H), 4.53 - 4.38 (m, 2H), 4.10 (s, 2H), 3.82 (s, 4H), 3.48 - 3.30 (m, 3H), 3.20 (s, 1H), 3.04 - 2.93 (m, 1H), 2.69 (d, *J* = 15.0 Hz, 1H), 2.42 (s, 3H), 2.35 (s, 3H), 2.17 - 2.08 (m, 2H). LCMS (ESI) calcd. for $C_{27}H_{31}N_6O_2S_2{}^+$ [M+H]$^+$: 535.19, found, 535.2.

**Example 46: Preparation of 3-(4-((4-(6-bromothieno[2,3-d]pyrimidin-4-yl)piperazin-1-yl)methyl)-1-thioxoisoindolin-2-yl)piperidine-2,6-dione (GT-08918)**

With reference to the method of Scheme 4, the target compound (GT-08918) was obtained (white solid, 16 mg, yield 32.49 %). $^1$H NMR (400 MHz, DMSO) δ 11.74 (s, 1H), 11.21 (s, 1H), 8.50 (s, 1H), 8.01 (dd, *J* = 7.6, 1.8 Hz, 2H), 7.94 - 7.93 (m, 1H), 7.71 - 7.68 (m, 1H), 6.01 (d, *J* = 7.9 Hz, 1H), 5.39 (d, *J* = 20.4 Hz, 1H), 4.92 (d, *J* = 20.3 Hz, 1H), 4.66 - 4.51 (m, 5H), 3.55 - 3.50 (m, 2H), 3.39 - 3.26 (m, 4H), 2.69 (d, *J* = 16.4 Hz, 2H), 2.16 - 2.10 (m, 1H). LCMS (ESI) calcd. for $C_{24}H_{24}BrN_6O_2S_2{}^+$ [M+H]$^+$: 571.06 , found, 571.0.

**Example 47: Preparation of 3-(4-((4-(6-phenylthieno[2,3-d]pyrimidin-4-yl)piperazin-1-yl)methyl)-1-thioxoisoindolin-2-yl)piperidine-2,6-dione (GT-08919)**

With reference to the method of Scheme 4, the target compound (GT-08919) was obtained (white solid, 17 mg, yield 35 %). $^1$H NMR (400 MHz, DMSO) δ 11.71 (s, 1H), 11.18 (d, *J* = 17.1 Hz, 1H), 8.52 (s, 1H), 8.06 - 7.99 (m, 3H), 7.87 (d, 2H), 7.70 (t, *J* = 7.7 Hz, 1H), 7.50 (t, *J* = 7.5 Hz, 2H), 7.42 (t, *J* = 7.3 Hz, 1H), 6.02 (d, *J* = 8.2 Hz, 1H), 5.38 (d, *J* = 20.3 Hz, 1H), 4.93 (d, *J* = 20.3 Hz, 1H), 4.76 (d, *J* = 13.3 Hz, 2H), 4.51 (dd, *J* = 31.5, 13.0 Hz, 2H), 3.82 - 3.70 (m, 2H), 3.42 - 3.26 (m, 4H), 3.04 - 2.94 (m, 1H), 2.68 (d, *J* = 16.8 Hz, 1H), 2.48 - 2.39 (m, 1H), 2.15 - 2.09 (m, 1H). LCMS (ESI) calcd. for $C_{30}H_{29}N_6O_2S_2{}^+$ [M+H]$^+$: 569.18, found, 569.2.

**Example 48: Preparation of 3-(4-((4-(5-phenylthieno[2,3-d]pyrimidin-4-yl)piperazin-1-yl)methyl)-1-thioxoisoindolin-2-yl)piperidine-2,6-dione (GT-08920)**

With reference to the method of Scheme 4, the target compound (GT-08920) was obtained (white solid, 18 mg, yield 37 %). $^1$H NMR (400 MHz, DMSO) δ 11.48 (s, 1H), 11.21 (s, 1H), 8.68 (s, 1H), 8.00 (d, *J* = 7.6 Hz, 1H), 7.84 (d, *J* = 7.5 Hz, 1H), 7.77 (s, 1H), 7.66 (t, *J* = 7.6 Hz, 1H), 7.45 (d, *J* = 7.1 Hz, 2H), 7.39 (t, *J* = 7.4 Hz, 2H), 7.32 (t, *J* = 7.2 Hz, 1H), 6.00 (d, *J* = 8.5 Hz, 1H), 5.18 (d, *J* = 20.3 Hz, 1H), 4.79 (d, *J* = 20.3 Hz, 1H), 4.28 (dd, *J* = 34.9, 13.1 Hz, 2H), 3.76 (t, *J* = 14.0 Hz, 2H), 3.29 - 3.06 (m, 4H), 3.05 - 2.94 (m, 1H), 2.69 (d, *J* = 17.2 Hz, 1H), 2.66 - 2.52 (m, 2H), 2.49 - 2.35 (m, 1H), 2.17 - 2.05 (m, 1H). LCMS (ESI) calcd. for $C_{30}H_{29}N_6O_2S_2{}^+$ [M+H]$^+$: 569.18, found, 569.2.

**Example 49: Preparation of 3-(4-((4-(2-methyl-5-phenylthieno[2,3-d]pyrimidin-4-yl)piperazin-1-yl)methyl)-1-thioxoisoindolin-2-yl)piperidine-2,6-dione (GT-08921)**

With reference to the method of Scheme 4, the target compound (GT-08921) was obtained (white solid, 12 mg, yield 24 %). $^1$H NMR (400 MHz, DMSO) δ 11.43 (s, 1H), 11.21 (s, 1H), 8.00 (d, *J* = 7.7 Hz, 1H), 7.83 (d, *J* = 7.5 Hz, 1H), 7.72 - 7.61 (m, 2H), 7.43 (d, *J* = 7.0 Hz, 2H), 7.37 (t, *J* = 7.5 Hz, 2H), 7.30 (t, *J* = 7.2 Hz, 1H), 6.00 (d, *J* = 8.2 Hz, 1H), 5.17 (d, *J* = 20.3 Hz, 1H), 4.78 (d, *J* = 20.4 Hz, 1H), 4.28 (dd, *J* = 36.0, 13.1 Hz, 2H), 3.83 - 3.69 (m, 3H), 3.28 - 3.07 (m, 4H), 3.03 - 2.94 (m, 1H), 2.69 (d, *J* = 16.5 Hz, 1H), 2.62 (s, 3H), 2.57 (d, *J* = 22.2 Hz, 1H), 2.44 (d, *J* = 14.2 Hz, 1H), 2.16 - 2.05 (m, 1H). LCMS (ESI) calcd. for $C_{31}H_{31}N_6O_2S_2{}^+$ [M+H]$^+$: 583.19, found, 583.2.

**Example 50: Preparation of 3-(4-((4-(2-methylthieno[2,3-d]pyrimidin-4-yl)piperazin-1-yl)methyl)-1-thioxoisoindolin-2-yl)piperidine-2,6-dione (GT-08922)**

With reference to the method of Scheme 4, the target compound (GT-08922) was obtained (white solid, 8 mg, yield 19 %). $^1$H NMR (400 MHz, DMSO) δ 11.86 (s, 1H), 11.20 (s, 1H), 8.01 (dd, *J* = 7.6, 3.2 Hz, 2H), 7.73 - 7.66 (m, 3H), 6.01 (d, *J* = 8.5 Hz, 1H), 5.42 (d, *J* = 20.3 Hz, 1H), 4.92 (d, *J* = 20.4 Hz, 1H), 4.74 (d, *J* = 11.8 Hz, 2H), 4.54 (d, *J* = 13.2 Hz, 1H), 4.39 (d, *J* = 39.5, 15.1 Hz, 1H), 3.78 (s, 2H), 3.38 (d, *J* = 36.3 Hz, 4H), 3.05 - 2.94 (m, 1H), 2.69 (d, *J* = 17.9 Hz, 1H), 2.56 (s, 3H), 2.47 - 2.36 (m, 1H), 2.16 - 2.07 (m, 1H). LCMS (ESI) calcd. for $C_{25}H_{27}N_6O_2S_2{}^+$ [M+H]$^+$: 507.16, found, 507.2.

**Example 51: Preparation of 3-(4-((4-(2-methyl-5,6,7,8-tetrahydrobenzo[4,5]thieno[2,3-d]pyrimidin-4-yl)piperazin-1-yl)methyl)-1-thioxoisoindolin-2-yl)piperidine-2,6-dione (GT-08923)**

With reference to the method of Scheme 4, the target compound (GT-08923) was obtained (white solid, 12 mg, yield 25 %). $^1$H NMR (400 MHz, DMSO) δ 11.76 (s, 1H), 11.21 (s, 1H), 8.07 (d, *J* = 7.5 Hz, 1H), 8.00 (d, *J* = 7.5 Hz, 1H), 7.68 (t, *J* = 7.7 Hz, 1H), 6.03 (d, *J* = 8.5 Hz, 1H), 5.47 (d, *J* = 20.4 Hz, 1H), 4.93 (d, *J* = 20.4 Hz, 1H), 4.51 (dd, *J* = 38.5, 13.1 Hz, 2H), 3.87 (s, 2H), 3.65 - 3.61 (m, 1H), 3.54 (d, *J* = 8.2 Hz, 2H), 3.37 (s, 3H), 3.05 - 2.96 (m, 1H), 2.90 - 2.83 (m, 4H), 2.70 (d, *J* = 17.2 Hz, 1H), 2.55 (s, 3H), 2.46 - 2.40 (m, 1H), 2.15 - 2.09 (m, 1H), 1.91 - 1.85 (m, 2H), 1.79 - 1.72 (m, 2H). LCMS (ESI) calcd. for $C_{29}H_{33}N_6O_2S_2$ $^+$ [M+H]$^+$: 561.21, found, 561.3.

**Example 52: Preparation of 3-(4-((4-(5,6,7,8-tetrahydrobenzo[4,5]thieno[2,3-d]pyrimidin-4-yl)piperazin-1-yl)methyl)-1-thioxoisoindolin-2-yl)piperidine-2,6-dione (GT-08924)**

With reference to the method of Scheme 4, the target compound (GT-08924) was obtained (white solid, 14 mg, yield 29.64 %). $^1$H NMR (400 MHz, DMSO) δ 11.62 (s, 1H), 11.21 (s, 1H), 8.56 (s, 1H), 8.05 (d, *J* = 7.6 Hz, 1H), 8.00 (d, *J* = 7.5 Hz, 1H), 7.69 (t, *J* = 7.7 Hz, 1H), 6.02 (d, *J* = 8.6 Hz, 1H), 5.44 (d, *J* = 20.3 Hz, 1H), 4.93 (d, *J* = 20.3 Hz, 1H), 4.51 (dd, *J* = 34.5, 12.8 Hz, 2H), 3.88 (d, *J* = 13.4 Hz, 2H), 3.59 - 3.51 (m, 3H), 3.39 (s, 3H), 3.04 - 2.96 (m, 1H), 2.96 - 2.91 (m, 2H), 2.89 (t, *J* = 5.8 Hz, 2H), 2.69 (d, *J* = 17.6 Hz, 1H), 2.47 - 2.38 (m, 1H), 2.15 - 2.08 (m, 1H), 1.93 - 1.85 (m, 2H), 1.81 - 1.72 (m, 2H). LCMS (ESI) calcd. for $C_{23}H_{31}N_6O_2S_2$ $^+$ [M+H]$^+$: 547.19, found, 547.2.

**Example 53: Preparation of 3-(4-((4-(6,7-dihydro-5H-cyclopenta[4,5]thieno[2,3-d]pyrimidin-4-yl)piperazin-1-yl)methyl)-1-thioxoisoindolin-2-yl)piperidine-2,6-dione (GT-08925)**

With reference to the method of Scheme 4, the target compound (GT-08925) was obtained (white solid, 11 mg, yield 24 %). $^1$H NMR (400 MHz, DMSO) δ 11.69 (s, 1H), 11.21 (s, 1H), 8.51 (s, 1H), 8.02 (dd, *J* = 16.5, 7.6 Hz, 2H), 7.68 (t, *J* = 7.7 Hz, 1H), 6.02 (d, *J* = 8.5 Hz, 1H), 5.42 (d, *J* = 20.4 Hz, 1H), 4.92 (d, *J* = 20.3 Hz, 1H), 4.49 (dd, *J* = 37.0, 12.8 Hz, 2H), 4.16 (d, *J* = 13.5 Hz, 2H), 3.54 (d, *J* = 11.5 Hz, 3H), 3.42 - 3.26 (m, 4H), 3.06 (t, *J* = 7.0 Hz, 2H), 3.03 - 2.97 (m, 3H), 2.69 (d, *J* = 16.1 Hz, 1H), 2.43 - 2.35 (m, 3H), 2.15 - 2.09 (m, 1H). LCMS (ESI) calcd. for $C_{27}H_{29}N_6O_2S_2$ $^+$ [M+H]$^+$: 533.18, found, 533.2.

**Example 54: Preparation of 3-(4-((4-(2-methyl-6,7-dihydro-5H-cyclopenta[4,5]thieno[2,3-d]pyrimidin-4-yl)piperazin-1-yl)methyl)-1-thioxoisoindolin-2-yl)piperidine-2,6-dione (GT-08926)**

With reference to the method of Scheme 4, the target compound (GT-08926) was obtained (white solid, 12 mg, yield 26 %). $^1$H NMR (400 MHz, DMSO) δ 11.82 (s, 1H), 11.21 (s, 1H), 8.05 (d, *J* = 7.6 Hz, 1H), 8.00 (d, *J* = 7.7 Hz, 1H), 7.68 (t, *J* = 7.7 Hz, 1H), 6.02 (d, *J* = 8.9 Hz, 1H), 5.45 (d, *J* = 20.4 Hz, 1H), 4.89 (d, *J* = 23.1 Hz, 1H), 4.49 (dd, *J* = 40.4, 12.9 Hz, 2H), 4.19 (d, *J* = 13.3 Hz, 2H), 3.71 - 3.63 (m, 2H), 3.56 (d, *J* = 12.5 Hz, 1H), 3.43 - 3.30 (m, 3H), 3.06 - 2.95 (m, 5H), 2.69 (d, *J* = 16.0 Hz, 1H), 2.55 (s, 3H), 2.43 - 2.32 (m, 3H), 2.14 - 2.07 (m, 1H). LCMS (ESI) calcd. for $C_{28}H_{31}N_6O_2S_2$ $^+$ [M+H]$^+$: 547.19, found, 547.2.

**Example 55: Preparation of 3-(4-((4-(thieno[2,3-d]pyrimidin-2-yl)piperazin-1-yl)methyl)-1-thioxoisoindolin-2-yl)piperidine-2,6-dione (GT-08927)**

With reference to the method of Scheme 4, the target compound (GT-08927) was obtained (white solid, 13 mg, yield 31 %). $^1$H NMR (400 MHz, DMSO) δ 11.66 (s, 1H), 11.20 (s, 1H), 8.96 (s, 1H), 8.01 (t, *J* = 8.3 Hz, 2H), 7.69 (t, 1H), 7.45 (d, *J* = 5.9 Hz, 1H), 7.33 (d, *J* = 5.9 Hz, 1H), 6.01 (d, *J* = 8.7 Hz, 1H), 5.38 (d, *J* = 20.3 Hz, 1H), 4.90 (d, *J* = 20.3 Hz, 1H), 4.78 (d, *J* = 13.8 Hz, 2H), 4.45 (dd, *J* = 34.6, 13.0 Hz, 2H), 3.62 - 3.48 (m, 3H), 3.41 (d, *J* = 11.1 Hz, 1H), 3.22 (s, 2H), 3.04 - 2.93 (m, 1H), 2.69 (d, *J* = 16.5 Hz, 1H), 2.49 - 2.34 (m, 1H), 2.16 - 2.08 (m, 1H). LCMS (ESI) calcd. for $C_{24}H_{25}N_6O_2S_2$ $^+$ [M+H]$^+$: 493.15, found, 493.2.

**Example 56: Preparation of 3-(4-((4-(thieno[2,3-d]pyrimidin-4-yl)piperidin-1-yl)methyl)-1-thioxoisoindolin-2-yl)piperidine-2,6-dione (GT-08928)**

With reference to the method of Scheme 4, the target compound (GT-08928) was obtained (white solid, 3 mg, yield 8 %). $^1$H NMR (400 MHz, DMSO) δ 11.21 (s, 1H), 11.06 (s, 1H), 9.03 (s, 1H), 8.03 - 8.00 (m, 2H), 7.86 - 7.81 (m, 1H), 7.70 (t, *J* = 7.7 Hz, 1H), 7.63 - 7.51 (m, 1H), 6.04 - 5.96 (m, 1H), 5.32 (d, *J* = 20.3 Hz, 1H), 4.94 (d, *J* = 20.3 Hz, 1H), 4.65 (s, 1H), 4.59 - 4.38 (m, 2H), 3.64 - 3.58 (m, 3H), 3.31 - 3.22 (m, 2H), 3.04 - 2.96 (m, 1H), 2.70 (d, *J* = 20.7 Hz, 1H), 2.43 - 2.33 (m, 2H), 2.17 - 2.11 (m, 1H), 2.06 (d, *J* = 13.5 Hz, 2H). LCMS (ESI) calcd. for $C_{25}H_{26}N_5O_2S_2$ $^+$ [M+H]$^+$: 492.15, found, 492.2.

**Example 57: Preparation of 3-(4-((4-(6-phenylthieno[2,3-d]pyrimidin-4-yl)piperidin-1-yl)methyl)-1-thioxoisoindolin-2-yl)piperidine-2,6-dione (GT-08929)**

With reference to the method of Scheme 4, the target compound (GT-08929) was obtained (white solid, 10 mg, yield 21 %). $^1$H NMR (400 MHz, DMSO) δ 11.21 (s, 1H), 11.04 (s, 1H), 9.01 (s, 1H), 8.30 (s, 1H), 8.03 (d, *J* = 7.8 Hz, 2H), 7.91 (d, 2H), 7.71 (t, *J* = 7.7 Hz, 1H), 7.55 (t, *J* = 7.4 Hz, 2H), 7.48 (t, *J* = 7.3 Hz, 1H), 6.02 (d, *J* = 11.6 Hz, 1H), 5.30 (d, *J* = 20.3 Hz, 1H), 4.95 (d, *J* = 20.3 Hz, 1H), 4.57 - 4.43 (m, 2H), 3.69 - 3.57 (m, 3H), 3.27 - 3.21 (m, 2H), 3.05 - 2.97 (m, 1H), 2.71 (d, *J* = 17.9 Hz, 1H), 2.46 - 2.33 (m, 3H), 2.17 - 2.08 (m, 3H). LCMS (ESI) calcd. for $C_{31}H_{30}N_5O_2S_2{}^+$ [M+H]$^+$: 568.18, found, 568.2.

**Example 58: Preparation of 3-(4-((4-(5-phenylthieno[2,3-d]pyrimidin-4-yl)piperidin-1-yl)methyl)-1-thioxoisoindolin-2-yl)piperidine-2,6-dione (GT-08930)**

With reference to the method of Scheme 4, the target compound (GT-08930) was obtained (white solid, 13 mg, yield 27 %). $^1$H NMR (400 MHz, DMSO) δ 11.23 (s, 1H), 10.84 (s, 1H), 9.06 (s, 1H), 8.01 (d, *J* = 7.6 Hz, 1H), 7.90 - 7.82 (m, 2H), 7.68 (t, *J* = 7.7 Hz, 1H), 7.53 - 7.46 (m, 5H), 6.02 (d, *J* = 9.1 Hz, 1H), 5.17 (d, *J* = 20.4 Hz, 1H), 4.83 (d, *J* = 20.3 Hz, 1H), 4.37 - 4.19 (m, 2H), 3.38 - 3.33 (m, 2H), 3.06 - 2.96 (m, 1H), 2.86 - 2.77 (m, 1H), 2.71 (d, *J* = 16.3 Hz, 1H), 2.49 - 2.41 (m, 1H), 2.32 - 2.08 (m, 5H), 1.78 (d, *J* = 12.4 Hz, 2H). LCMS (ESI) calcd. for $C_{31}H_{30}N_5O_2S_2{}^+$ [M+H]$^+$: 568.18, found, 568.2.

**Example 59: Preparation of 3-(4-((4-(2-methyl-5-phenylthieno[2,3-d]pyrimidin-4-yl)piperidin-1-yl)methyl)-1-thioxoisoindolin-2-yl)piperidine-2,6-dione (GT-08931)**

With reference to the method of Scheme 4, the target compound (GT-08931) was obtained (white solid, 11 mg, yield 23 %). $^1$HNMR (400 MHz, DMSO) δ 11.23 (s, 1H), 10.71 (s, 1H), 8.01 (d, *J* = 7.6 Hz, 1H), 7.88 (d, *J* = 7.6 Hz, 1H), 7.71 - 7.67 (m, 2H), 7.51 - 7.47 (m, 5H), 6.02 (d, *J* = 9.8 Hz, 1H), 5.13 (d, *J* = 20.4 Hz, 1H), 4.83 (d, *J* = 20.3 Hz, 1H), 4.36 - 4.25 (m, 2H), 3.39 (dd, *J* = 23.3, 10.8 Hz, 3H), 3.07 - 2.98 (m, 1H), 2.82 - 2.72 (m, 2H), 2.69 (s, 3H), 2.33 - 2.24 (m, 2H), 2.21 - 2.11 (m, 3H), 1.76 (d, *J* = 13.1 Hz, 2H). LCMS (ESI) calcd. for $C_{32}H_{32}N_5O_2S_2{}^+$ [M+H]$^+$: 582.20, found, 582.2.

**Example 60: Preparation of 3-(4-((4-(2-methylthieno[2,3-d]pyrimidin-4-yl)piperidin-1-yl)methyl)-1-thioxoisoindolin-2-yl)piperidine-2,6-dione (GT-08932)**

With reference to the method of Scheme 4, the target compound (GT-08932) was obtained (white solid, 8 mg, yield 19 %). $^1$H NMR (400 MHz, DMSO) δ 11.21 (s, 1H), 10.89 (s, 1H), 8.01 (t, *J* = 6.5 Hz, 2H), 7.88 (d, *J* = 6.0 Hz, 1H), 7.80 - 7.78 (m, 1H), 7.73 - 7.67 (m, 1H), 6.00 (t, *J* = 12.8 Hz, 1H), 5.29 (d, *J* = 20.3 Hz, 1H), 4.93 (d, *J* = 20.3 Hz, 1H), 4.79 (d, *J* = 31.8 Hz, 1H), 4.65 (s, 1H), 4.53 - 4.41 (m, 2H), 3.60 - 3.52 (m, 3H), 3.31 - 3.21 (m, 2H), 3.02 - 2.95 (m, 1H), 2.69 (s, 3H), 2.41 - 2.31 (m, 2H), 2.16 - 2.10 (m, 1H), 2.04 (d, *J* = 13.9 Hz, 2H). LCMS (ESI) calcd. for $C_{26}H_{28}N_5O_2S_2{}^+$ [M+H]$^+$: 506.17, found, 506.2.

**Example 61: Preparation of 3-(4-((4-(2-methyl-5,6,7,8-tetrahydrobenzo[4,5]thieno[2,3-d]pyrimidin-4-yl)piperidin-1-yl)methyl)-1-thioxoisoindolin-2-yl)piperidine-2,6-dione (GT-08933)**

With reference to the method of Scheme 4, the target compound (GT-08933) was obtained (white solid, 10 mg, yield 21 %). $^1$H NMR (400 MHz, DMSO) δ 11.21 (s, 1H), 10.51 (s, 1H), 7.99 (d, 2H), 7.68 (t, 1H), 6.02 (d, *J* = 8.9 Hz, 1H), 5.23 (d, *J* = 20.3 Hz, 1H), 4.93 (d, *J* = 20.3 Hz, 1H), 4.52 - 4.34 (m, 2H), 3.68 - 3.57 (m, 2H), 3.37 - 3.28 (m, 3H), 3.04 - 2.96 (m, 3H), 2.86 (s, 2H), 2.68 (d, 1H), 2.63 (s, 3H), 2.48 - 2.42 (m, 1H), 2.35 - 2.26 (m, 2H), 2.18 - 2.12 (m, 1H), 1.99 (d, *J* = 13.0 Hz, 2H), 1.87 (s, 4H). LCMS (ESI) calcd. for $C_{30}H_{34}N_5O_2S_2{}^+$ [M+H]$^+$: 560.21, found, 560.3.

**Example 62: Preparation of 3-(4-((4-(6,7-dihydro-5H-cyclopenta[4,5]thieno[2,3-d]pyrimidin-4-yl)piperidin-1-yl)methyl)-1-thioxoisoindolin-2-yl)piperidine-2,6-dione (GT-08934)**

With reference to the method of Scheme 4, the target compound (GT-08934) was obtained (white solid, 10 mg, yield 22 %). $^1$H NMR (400 MHz, DMSO) δ 11.21 (s, 1H), 10.94 (s, 1H), 8.91 (s, 1H), 8.09 - 7.96 (m, 2H), 7.74 - 7.63 (m, 1H), 6.02 (d, *J* = 7.2 Hz, 1H), 5.33 (d, *J* = 20.3 Hz, 1H), 4.93 (d, *J* = 20.2 Hz, 1H), 4.54 - 4.33 (m, 2H), 3.56 (s, 2H), 3.45 - 3.25 (m, 4H), 3.15 (t, *J* = 7.2 Hz, 2H), 3.07 (t, *J* = 7.2 Hz, 2H), 3.02 - 2.95 (m, 1H), 2.71 (d, *J* = 18.1 Hz, 1H), 2.48 - 2.26 (m, 4H), 2.17 - 2.10 (m, 1H), 2.00 (d, *J* = 13.1 Hz, 2H). LCMS (ESI) calcd. for $C_{28}H_{30}N_5O_2S_2{}^+$ [M+H]$^+$: 532.18 , found, 532.2.

**Example 63: Preparation of 3-(4-((4-(2-methyl-6,7-dihydro-5H-cyclopenta[4,5]thieno[2,3-d]pyrimidin-4-yl)piperidin-1-yl)methyl)-1-thioxoisoindolin-2-yl)piperidine-2,6-dione (GT-08935)**

With reference to the method of Scheme 4, the target compound (GT-08935) was obtained (white solid, 5 mg,

yield 11 %). $^1$H NMR (400 MHz, DMSO) δ 11.24 - 11.14 (m, 1H), 10.52 (s, 1H), 8.04 - 7.97 (m, 2H), 7.70 (t, *J* = 7.7 Hz, 1H), 6.03 (d, *J* = 9.6 Hz, 1H), 5.23 (d, *J* = 20.2 Hz, 1H), 4.93 (d, *J* = 20.3 Hz, 1H), 4.51 - 4.36 (m, 2H), 3.56 - 3.52 (m, 2H), 3.37 - 3.28 (m, 4H), 3.12 (t, *J* = 6.9 Hz, 2H), 3.06 - 3.00 (m, 3H), 2.74 - 2.66 (m, 2H), 2.65 (s, 3H), 2.48 - 2.46 (m, 1H), 2.33 - 2.25 (m, 2H), 2.19 - 2.13 (m, 1H), 1.99 (d, *J* = 13.4 Hz, 2H). LCMS (ESI) calcd. for $C_{29}H_{32}N_5O_2S_2{}^+$ $[M+H]^+$: 546.20, found, 546.2.

**Example 64: Preparation of 3-(4-((4-(thieno[2,3-d]pyrimidin-2-yl)piperidin-1-yl)methyl)-1-thioxoisoindolin-2-yl)piperidine-2,6-dione (GT-08936)**

With reference to the method of Scheme 4, the target compound (GT-08936) was obtained (white solid, 4 mg, yield 10 %). $^1$H NMR (400 MHz, DMSO) δ 11.20 (s, 1H), 10.92 (s, 1H), 9.30 (s, 1H), 8.08 - 7.97 (m, 2H), 7.92 (d, *J* = 5.9 Hz, 1H), 7.69 (t, *J* = 13.8, 6.1 Hz, 1H), 7.56 (d, *J* = 5.9 Hz, 1H), 6.01 (s, 1H), 5.33 (d, *J* = 20.3 Hz, 1H), 4.92 (d, 1H), 4.52 - 4.36 (m, 2H), 3.50 - 3.44 (m, 2H), 3.30 - 3.21 (m, 3H), 3.02 - 2.96 (m, 1H), 2.74 - 2.59 (m, 2H), 2.47 - 2.39 (m, 1H), 2.26 (s, 3H), 2.15 - 2.08 (m, 1H). LCMS (ESI) calcd. for $C_{25}H_{26}N_5O_2S_2{}^+$ $[M+H]^+$: 492.15, found, 492.2.

**Example 65: Preparation of 3-(4-((4-(thieno[2,3-d]pyrimidin-4-yl)-3,6-dihydropyridin-1(2H)-yl)methyl)-1-thioxoisoindolin-2-yl)piperidine-2,6-dione (GT-08937)**

With reference to the method of Scheme 4, the target compound (GT-08937) was obtained (yellow solid, 9 mg, yield 22 %). $^1$H NMR (400 MHz, DMSO) δ 11.43 (d, 1H), 11.17 (s, 1H), 9.08 (s, 1H), 8.09 - 8.00 (m, 3H), 7.78 (t, *J* = 5.6 Hz, 1H), 7.71 (t, *J* = 7.7 Hz, 1H), 6.67 (s, 1H), 6.03 (d, *J* = 9.5 Hz, 1H), 5.37 (dd, *J* = 39.3, 20.5 Hz, 1H), 5.05 - 4.88 (m, 1H), 4.68 - 4.48 (m, 2H), 4.09 - 3.93 (m, 2H), 3.72 (s, 2H), 3.45 (s, 1H), 3.16 - 2.97 (m, 3H), 2.70 (d, *J* = 17.8 Hz, 1H), 2.49 - 2.38 (m, 1H), 2.14 (d, *J* = 5.5 Hz, 1H). LCMS (ESI) $C_{25}H_{24}N_5O_2S_2{}^+$ $[M+H]^+$: 490.14, found, 490.2.

**Example 66: Preparation of 3-(4-((4-(5-methylthieno[2,3-d]pyrimidin-4-yl)-3,6-dihydropyridin-1(2H)-yl)methyl)-1-thioxoisoindolin-2-yl)piperidine-2,6-dione (GT-08938)**

With reference to the method of Scheme 4, the target compound (GT-08938) was obtained (white solid, 17 mg, yield 37 %). $^1$H NMR (400 MHz, DMSO) δ 11.60 (s, 1H), 11.22 (s, 1H), 9.03 (s, 1H), 8.13 - 8.06 (m, 1H), 8.02 (d, *J* = 7.6 Hz, 1H), 7.71 (t, *J* = 7.7 Hz, 1H), 7.65 (s, 1H), 6.02 (s, 1H), 5.93 (s, 1H), 5.41 (dd, *J* = 35.5, 20.5 Hz, 1H), 5.06 - 4.90 (m, 1H), 4.66 - 4.50 (m, 2H), 3.99 (s, 1H), 3.91 - 3.75 (m, 2H), 3.46 (s, 1H), 3.20 - 3.07 (m, 1H), 3.05 - 2.89 (m, 2H), 2.70 (d, *J* = 18.0 Hz, 1H), 2.44 (d, *J* = 7.2 Hz, 3H), 2.18 - 2.10 (m, 1H). LCMS (ESI) calcd. for $C_{26}H_{26}N_5O_2S_2{}^+$ $[M+H]^+$: 504.15, found, 504.1.

**Example 67: Preparation of 3-(4-((4-(6-methylthieno[2,3-d]pyrimidin-4-yl)-3,6-dihydropyridin-1(2H)-yl)methyl)-1-thioxoisoindolin-2-yl)piperidine-2,6-dione** (GT-08947)

With reference to the method of Scheme 4, the target compound (GT-08947) was obtained (yellow solid, 10 mg, yield 24 %). $^1$H NMR (400 MHz, DMSO) δ 11.51 (s, 1H), 11.22 (s, 1H), 9.01 (d, *J* = 23.8 Hz, 1H), 8.08 (t, *J* = 6.9 Hz, 1H), 8.01 (d, *J* = 8.2 Hz, 1H), 7.71 (t, *J* = 7.7 Hz, 1H), 7.51 - 7.46 (m, 1H), 6.64 (s, 1H), 6.03 (d, *J* = 9.0 Hz, 1H), 5.40 (dd, *J* = 45.9, 20.4 Hz, 1H), 5.02 - 4.86 (m, 1H), 4.69 - 4.41 (m, 2H), 4.12 - 3.95 (m, 2H), 3.45 - 3.41 (m, 1H), 3.18 - 2.97 (m, 3H), 2.72 - 2.62 (m, 5H), 2.45 (s, 1H), 2.21 - 2.06 (m, 1H). LCMS (ESI) $C_{26}H_{26}N_5O_2S_2{}^+$ $[M+H]^+$: 504.15, found, 504.2.

**Example 68: Preparation of 3-(4-((4-(6-isopropylthieno[2,3-d]pyrimidin-4-yl)-3,6-dihydropyridin-1(2H)-yl)methyl)-1-thioxoisoindolin-2-yl)piperidine-2,6-dione (GT-08954)**

With reference to the method of Scheme 4, the target compound (GT-08954) was obtained (white solid, 15 mg, yield 33 %). $^1$H NMR (400 MHz, DMSO) δ 11.38 (d, *J* = 32.0 Hz, 1H), 11.22 (s, 1H), 8.99 (s, 1H), 8.08 - 7.99 (m, 2H), 7.71 (t, *J* = 7.7 Hz, 1H), 7.47 (s, 1H), 6.65 (s, 1H), 6.03 (d, *J* = 8.7 Hz, 1H), 5.39 (dd, *J* = 46.7, 20.4 Hz, 1H), 5.06 - 4.91 (m, 1H), 4.69 - 4.48 (m, 2H), 4.11 - 3.88 (m, 2H), 3.79 - 3.68 (m, 1H), 3.48 - 3.39 (m, 2H), 3.35 - 3.29 (m, 1H), 3.09 - 2.96 (m, 3H), 2.70 (d, *J* = 17.2 Hz, 1H), 2.13 (d, *J* = 3.3 Hz, 1H), 1.36 (d, *J* = 6.8 Hz, 6H). LCMS (ESI) calcd. for $C_{23}H_{30}N_5O_2S_2{}^+$ $[M+H]^+$: 532.18, found, 532.2.

**Example 69: Preparation of 3-(4-((4-(5,6-dimethylthieno[2,3-d]pyrimidin-4-yl)-3,6-dihydropyridin-1(2H)-yl)methyl)-1-thioxoisoindolin-2-yl)piperidine-2,6-dione (GT-08955)**

With reference to the method of Scheme 4, the target compound (GT-08955) was obtained (white solid, 8 mg, yield 19 %). $^1$H NMR (400 MHz, DMSO) δ 11.49 (s, 1H), 11.22 (s, 1H), 8.94 (s, 1H), 8.07 (d, *J* = 7.5 Hz, 1H), 8.02 (d, *J* = 7.6 Hz, 1H), 7.76 - 7.67 (m, 2H), 6.02 (s, 1H), 5.82 (d, *J* = 32.7 Hz, 2H), 5.43 - 5.28 (m, 1H), 5.01 - 4.91 (m, 1H), 4.06 - 3.86 (m, 3H), 3.50 - 3.44 (m, 2H), 3.16 - 2.94 (m, 3H), 2.76 (t, *J* = 7.1 Hz, 1H), 2.54 (s, 2H), 2.30 (d, *J* = 5.4 Hz, 3H), 2.25 (s, 1H), 2.17 -

2.11 (m, 1H). LCMS (ESI) calcd. for $C_{27}H_{28}N_5O_2S_2$ + [M+H]+: 518.17, found, 518.2.

**Example 70: Preparation of 3-(4-((4-(6-phenylthieno[2,3-d]pyrimidin-4-yl)-3,6-dihydropyridin-1(2H)-yl) methyl)-1-thioxoisoindolin-2-yl)piperidine-2,6-dione (GT-08956)**

With reference to the method of Scheme 4, the target compound (GT-08956) was obtained (yellow solid, 15 mg, yield 31 %). $^1$H NMR (400 MHz, DMSO) δ 11.21 (s, 2H), 9.06 (s, 1H), 8.14 (d, *J*= 2.7 Hz, 1H), 8.11 - 7.99 (m, 2H), 7.90 (d, *J* = 7.6 Hz, 2H), 7.73 (t, *J* = 7.6 Hz, 1H), 7.56 - 7.46 (m, 3H), 6.81 (s, 1H), 6.04 (s, 1H), 5.35 (dd, *J* = 54.6, 20.3 Hz, 1H), 4.99 (t, 1H), 4.73 - 4.47 (m, 2H), 4.16 - 3.95 (m, 2H), 3.79 (s, 1H), 3.12 - 2.92 (m, 3H), 2.67 (t, *J* = 20.4 Hz, 2H), 2.49 -2.39 (m, 1H), 2.20 - 2.08 (m, 1H). LCMS (ESI) calcd. for $C_{31}H_{28}N_5O_2S_2$ + [M+H]+: 566.17, found, 566.2.

**Example 71: Preparation of 3-(4-((4-(5-phenylthieno[2,3-d]pyrimidin-4-yl)-3,6-dihydropyridin-1(2H)-yl) methyl)-1-thioxoisoindolin-2-yl)piperidine-2,6-dione (GT-08957)**

With reference to the method of Scheme 4, the target compound (GT-08957) was obtained (white solid, 13 mg, yield 27 %). $^1$H NMR (400 MHz, DMSO) δ 11.20 (d, 2H), 9.13 (s, 1H), 8.05 (d, *J*= 7.7 Hz, 1H), 7.98 (s, 1H), 7.97 - 7.89 (m, 1H), 7.78 - 7.66 (m, 1H), 7.40 - 7.32 (m, 4H), 7.16 (t, *J* = 7.1 Hz, 1H), 6.03 (s, 1H), 5.25 (s, 1H), 5.11 (d, *J* = 20.1 Hz, 1H), 4.83 (d, *J* = 20.6 Hz, 1H), 4.49 - 4.22 (m, 2H), 3.54 (s, 1H), 3.31 (d, *J* = 15.7 Hz, 1H), 3.11 - 2.95 (m, 2H), 2.72 (d, *J* = 16.6 Hz, 4H), 2.21 - 2.12 (m, 1H). LCMS (ESI) calcd. for $C_{31}H_{28}N_5O_2S_2$ + [M+H]+: 566.17, found, 566.2.

**Example 72: Preparation of 3-(4-((4-(2-methyl-5-phenylthieno[2,3-d]pyrimidin-4-yl)-3,6-dihydropyridin-1(2H)-yl) methyl)-1-thioxoisoindolin-2-yl)piperidine-2,6-dione (GT-08958)**

With reference to the method of Scheme 4, the target compound (GT-08958) was obtained (white solid, 16 mg, yield 33 %). $^1$H NMR (400 MHz, DMSO) δ 11.20 (d, *J* = 17.3 Hz, 2H), 8.05 (d, *J*= 7.6 Hz, 1H), 8.01 - 7.90 (m, 1H), 7.84 (s, 1H), 7.75 - 7.67 (m, 1H), 7.39 - 7.26 (m, 4H), 7.12 (t, *J*= 7.1 Hz, 1H), 6.04 (s, 1H), 5.25 (s, 1H), 5.12 (d, *J* = 20.3 Hz, 1H), 4.90 - 4.77 (m, 1H), 4.48 - 4.26 (m, 2H), 3.59 - 3.43 (m, 2H), 3.30 (s, 1H), 3.09 - 2.94 (m, 2H), 2.74 (s, 3H), 2.73 - 2.62 (m, 3H), 2.20 - 2.10 (m, 1H). LCMS (ESI) calcd. for $C_{32}H_{30}N_5O_2S_2$ + [M+H]+: 580.18, found, 580.2.

**Example 73: Preparation of 3-(4-((4-(2-methylthieno[2,3-d]pyrimidin-4-yl)-3,6-dihydropyridin-1(2H)-yl) methyl)-1-thioxoisoindolin-2-yl)piperidine-2,6-dione (GT-08959)**

With reference to the method of Scheme 4, the target compound(GT-08959) was obtained (white solid, 8 mg, yield 19 %). $^1$H NMR (400 MHz, DMSO) δ 11.52 (s, 1H), 11.22 (s, 1H), 8.08 (t, *J*= 7.0 Hz, 1H), 8.02 (d, *J*= 7.5 Hz, 1H), 7.91 (d, *J* = 6.0 Hz, 1H), 7.73 - 7.67 (m, 2H), 6.64 (s, 1H), 6.03 (d, *J* = 9.8 Hz, 1H), 5.40 (dd, *J* = 44.9, 20.3 Hz, 1H), 5.05 - 4.88 (m, 1H), 4.69 - 4.50 (m, 2H), 4.02 (s, 2H), 3.75 - 3.66 (m, 1H), 3.43 (t, *J* = 6.9, 4.9 Hz, 2H), 3.11 - 2.95 (m, 3H), 2.74 (d, *J*= 5.6 Hz, 1H), 2.72 (s, 3H), 2.18 - 2.11 (m, 1H). LCMS (ESI) calcd. for $C_{26}H_{26}N_5O_2S_2$ + [M+H]+: 504.15, found, 504.2.

**Example 74: Preparation of 3-(4-((4-((4'-chloro-[1,1'-biphenyl]-4-yl)methyl)piperazin-1-yl)methyl)-1-thioxoisoindolin-2-yl)piperidine-2,6-dione (GT-08960)**

With reference to the method of Scheme 4, the target compound (GT-08960) was obtained (white solid, 9 mg, yield 18 %). $^1$H NMR (400 MHz, DMSO) δ 11.18 (s, 1H), 7.93 (d, *J* = 7.4 Hz, 1H), 7.83 (s, 1H), 7.78 - 7.67 (m, 6H), 7.61 (t, *J* = 7.8 Hz, 1H), 7.54 (d, 2H), 5.99 (d, *J* = 9.5 Hz, 1H), 5.15 (s, 1H), 4.83 (d, *J* = 19.0 Hz, 1H), 4.72 - 3.68 (m, 6H), 3.43 - 3.32 (m, 4H), 3.24 (s, 2H), 3.16 - 2.81 (m, 3H), 2.67 (d, 1H), 2.62 - 2.52 (m, 1H), 2.17 - 2.05 (m, 1H). LCMS (ESI) calcd. for $C_{31}H_{32}ClN_4O_2S$ + [M+H]+: 559.19, found, 559.3.

**Example 75: Preparation of 3-(4-((4-(2-methyl-5,6,7,8-tetrahydrobenzo[4,5]thieno[2,3-d]pyrimidin-4-yl)-3,6-dihydropyridin-1(2H)-yl)methyl)-1-thioxoisoindolin-2-yl)piperidine-2,6-dione (GT-08961)**

With reference to the method of Scheme 4, the target compound (GT-08961) was obtained (white solid, 12 mg, yield 26 %). $^1$H NMR (400 MHz, DMSO) δ 11.33 (d, *J* = 24.0 Hz, 1H), 11.22 (s, 1H), 8.08 - 7.99 (m, 2H), 7.71 (t, *J*= 7.7 Hz, 1H), 6.05 (s, 1H), 5.85 (s, 1H), 5.36 (q, *J*= 30.7, 20.6 Hz, 1H), 4.96 (q, *J*= 20.1, 11.0 Hz, 1H), 4.61 (s, 2H), 3.93 (d, *J* = 33.5 Hz, 2H), 3.49 - 3.37 (m, 2H), 3.05 - 2.96 (m, 2H), 2.87 (s, 4H), 2.74 (d, *J* = 10.4 Hz, 1H), 2.66 (s, 3H), 2.18 - 2.10 (m, 1H), 1.80 (d, *J* = 31.4 Hz, 5H). LCMS (ESI) calcd. for $C_{30}H_{32}N_5O_2S_2$ + [M+H]+: 558.20, found, 558.2 .

**Example 76: Preparation of 3-(4-((4-(5,6,7,8-tetrahydrobenzo[4,5]thieno[2,3-d]pyrimidin-4-yl)-3,6-dihydropyridin-1(2H)-yl)methyl)-1-thioxoisoindolin-2-yl)piperidine-2,6-dione (GT-08962)**

With reference to the method of Scheme 4, the target compound (GT-08962) was obtained (white solid, 12 mg, yield 26 %). $^1$H NMR (400 MHz, DMSO) δ 11.44 (d, *J* = 24.2 Hz, 1H), 11.22 (s, 1H), 8.94 (s, 1H), 8.10 - 8.00 (m, 2H), 7.71 (t, *J*= 7.7 Hz, 1H), 6.02 (s, 1H), 5.88 (s, 1H), 5.38 (dd, *J*= 31.5, 20.3 Hz, 1H), 4.97 (dd, *J* = 20.3, 11.4 Hz, 1H), 4.68 - 4.48 (m, 2H), 3.99 (s, 1H), 3.69 (d, *J* = 23.3 Hz, 1H), 3.46 - 3.40 (m, 1H), 3.09 - 2.95 (m, 2H), 2.91 (s, 3H), 2.77 - 2.55 (m, 3H), 2.18 - 2.11 (m, 1H), 1.81 (d, *J* = 33.5 Hz, 5H). LCMS (ESI) calcd. for $C_{29}H_{30}N_5O_2S_2{}^+$ [M+H]$^+$: 544.18, found, 544.2.

**Example 77: Preparation of 3-(4-((4-(6,7-dihydro-5H-cyclopenta[4,5]thieno[2,3-d]pyrimidin-4-yl)-3,6-dihydropyridin-1(2H)-yl)methyl)-1-thioxoisoindolin-2-yl)piperidine-2,6-dione (GT-08963)**

With reference to the method of Scheme 4, the target compound (GT-08963) was obtained (white solid, 7 mg, yield 16 %). $^1$H NMR (400 MHz, DMSO) δ 11.47 (d, *J* = 24.5 Hz, 1H), 11.22 (s, 1H), 8.94 (s, 1H), 8.03 (t, *J*= 8.4 Hz, 2H), 7.71 (t, *J*= 7.6 Hz, 1H), 6.11 (s, 1H), 6.01 (s, 1H), 5.40 (q, *J*= 27.0, 20.5 Hz, 1H), 5.01 - 4.91 (m, 1H), 4.79 (dd, *J* = 51.4, 20.3 Hz, 1H), 4.63 - 4.49 (m, 2H), 4.11 - 3.86 (m, 2H), 3.07 - 2.99 (m, 5H), 2.67 (d, 2H), 2.43 - 2.31 (m, 3H), 2.17 - 2.06 (m, 2H). LCMS (ESI) calcd. for $C_{28}H_{28}N_5O_2S_2{}^+$ [M+H]$^+$: 530.17, found, 530.2.

**Example 78: Preparation of 3-(4-((4-(2-methyl-6,7-dihydro-5H-cyclopenta[4,5]thieno[2,3-d]pyrimidin-4-yl)-3,6-dihydropyridin-1(2H)-yl)methyl)-1-thioxoisoindolin-2-yl)piperidine-2,6-dione (GT-08964)**

With reference to the method of Scheme 4, the target compound (GT-08964) was obtained (white solid, 9 mg, yield 20 %). $^1$H NMR (400 MHz, DMSO) δ 11.63 (s, 1H), 11.22 (s, 1H), 8.06 (d, *J*= 7.5 Hz, 1H), 8.02 (d, *J* = 7.7 Hz, 1H), 7.71 (t, *J* = 7.7 Hz, 1H), 6.06 (s, 1H), 6.01 (s, 1H), 5.44 (q, *J* = 32.4, 20.4 Hz, 1H), 4.97 (q, *J* = 20.3, 12.5 Hz, 1H), 4.68 - 4.50 (m, 2H), 4.00 (s, 2H), 3.85 (d, *J* = 17.0 Hz, 1H), 3.43 (s, 1H), 3.06 - 2.90 (m, 6H), 2.87 - 2.80 (m, 1H), 2.71 (s, 1H), 2.68 (s, 3H), 2.49 - 2.25 (m, 3H), 2.16 - 2.10 (m, 1H). LCMS (ESI) calcd. for $C_{29}H_{30}N_5O_2S_2{}^+$ [M+H]$^+$: 544.18, found, 544.2.

**Example 79: Preparation of 3-(4-((4-(thieno[2,3-d]pyrimidin-2-yl)-3,6-dihydropyridin-1(2H)-yl)methyl)-1-thioxoisoindolin-2-yl)piperidine-2,6-dione (GT-08965)**

With reference to the method of Scheme 4, the target compound (GT-08965) was obtained (white solid, 7 mg, yield 17 %). $^1$H NMR (400 MHz, DMSO) δ 11.32 - 11.09 (m, 2H), 9.32 (s, 1H), 8.09 - 8.02 (m, 1H), 7.97 (d, *J*= 5.9 Hz, 1H), 7.71 (t, *J*= 7.8 Hz, 1H), 7.62 - 7.56 (m, 1H), 7.21 (s, 1H), 6.09 - 5.91 (m, 1H), 5.33 (q, *J* = 31.0, 20.4 Hz, 1H), 4.91 (q, *J*= 38.1, 20.2 Hz, 1H), 4.66 - 4.49 (m, 2H), 4.06 - 3.96 (m, 1H), 3.74 - 3.66 (m, 1H), 3.41 (s, 2H), 3.15 - 2.93 (m, 3H), 2.70 (d, *J*= 18.4 Hz, 1H), 2.49 - 2.38 (m, 1H), 2.16 - 2.05 (m, 1H). LCMS (ESI) calcd. for $C_{25}H_{24}N_5O_2S_2{}^+$ [M+H]$^+$: 490.14, found, 490.2.

**Example 180: Preparation of 3-(4-((4-(2-chloro-6,7-dihydrothieno[3,2-d]pyrimidin-4-yl)piperazin-1-yl)methyl)-1-thioxoisoindolin-2-yl)piperidine-2,6-dione (GT-08966)**

With reference to the method of Scheme 4, the target compound (GT-08966) was obtained (white solid, 14 mg, yield 32 %). $^1$H NMR (400 MHz, DMSO) δ 11.80 (s, 1H), 11.17 (d, *J* = 31.8 Hz, 1H), 8.00 *(d, J= 7.7, 1.7* Hz, 2H), 7.68 (t, 1H), 6.01 *(d, J=* 8.5 Hz, 1H), 5.40 (d, *J* = 20.4 Hz, 1H), 4.88 (d, 1H), 4.52 - 4.38 (m, 3H), 3.77 (s, 1H), 3.61 - 3.46 (m, 3H), 3.38 (t, *J* = 8.1 Hz, 3H), 3.22 (t, *J* = 8.1 Hz, 4H), 3.05 - 2.92 (m, 1H), 2.65 (d, *J* = 20.5 Hz, 1H), 2.49 - 2.35 (m, 1H), 2.16 - 2.06 (m, 1H). LCMS (ESI) calcd. for $C_{24}H_{26}ClN_6O_2S_2{}^+$ [M+H]$^+$: 529.12, found, 529.2.

**Example 81: Preparation of 3-(4-((4-(2-morpholino-6,7-dihydrothieno[3,2-d]pyrimidin-4-yl)piperazin-1-yl)methyl)-1-thioxoisoindolin-2-yl)piperidine-2,6-dione (GT-08968)**

With reference to the method of Scheme 4, the target compound (GT-08968) was obtained (white solid, 11 mg, yield 23 %). $^1$H NMR (400 MHz, DMSO) δ 11.88 (s, 1H), 11.21 (s, 1H), 8.07 - 7.94 (m, 2H), 7.67 (t, *J* = 7.7 Hz, 1H), 6.01 (d, *J* = 8.5 Hz, 1H), 5.42 (d, *J* = 20.3 Hz, 1H), 4.90 (d, *J* = 20.4 Hz, 1H), 4.54 - 4.35 (m, 4H), 3.70 - 3.68 (m, 5H), 3.67 - 3.64 (m, 6H), 3.37 - 3.31 (m, 3H), 3.28 - 3.17 (m, 4H), 3.05 - 2.95 (m, 1H), 2.68 (d, 1H), 2.48 - 2.35 (m, 1H), 2.16 - 2.07 (m, 1H). LCMS (ESI) calcd. for $C_{28}H_{34}N_7O_3S_2{}^+$ [M+H]$^+$: 580.22, found, 580.3.

**Example 82: Preparation of 3-(4-((4-(4-morpholino-6,7-dihydrothieno[3,2-d]pyrimidin-2-yl)piperazin-1-yl)methyl)-1-thioxoisoindolin-2-yl)piperidine-2,6-dione (GT-08969)**

With reference to the method of Scheme 4, the target compound (GT-08969) was obtained (white solid, 10 mg,

yield 21 %). $^1$H NMR (400 MHz, DMSO) δ 11.72 (s, 1H), 11.16 (s, 1H), 8.13 - 7.94 (m, 2H), 7.67 (t, 1H), 6.01 (d, *J* = 8.1 Hz, 1H), 5.42 (d, *J* = 20.4 Hz, 1H), 4.90 (d, *J* = 20.2 Hz, 1H), 4.61 (d, *J*= 12.2 Hz, 2H), 4.51 - 4.38 (m, 2H), 3.67 - 3.63 (m, 7H), 3.55 - 3.44 (m, 4H), 3.30 (t, *J* = 8.3 Hz, 3H), 3.22 - 3.11 (m, 4H), 3.03 - 2.94 (m, 1H), 2.69 (d, *J* = 16.4 Hz, 1H), 2.47 - 2.36 (m, 1H), 2.17 - 2.07 (m, 1H). LCMS (ESI) calcd. for $C_{28}H_{34}N_7O_3S_2{}^+$ [M+H]$^+$: 580.22, found, 580.3.

**Example 83: Preparation of 3-(4-((4-(thieno[3,2-d]pyrimidin-4-yl)piperazin-1-yl)methyl)-1-thioxoisoindolin-2-yl) piperidine-2,6-dione (GT-08970)**

With reference to the method of Scheme 4, the target compound (GT-08970) was obtained (white solid, 4 mg, yield 10 %). $^1$H NMR (400 MHz, DMSO) δ 12.27 (s, 1H), 11.20 (s, 1H), 8.90 (s, 1H), 8.61 (d, *J* = 5.6 Hz, 1H), 8.01 (t, 2H), 7.72 - 7.64 (m, 2H), 6.01 (d, *J* = 8.3 Hz, 1H), 5.45 (d, *J* = 20.4 Hz, 1H), 4.92 (d, *J* = 20.3 Hz, 3H), 4.56 (d, *J*= 13.2 Hz, 1H), 4.43 (d, *J*= 13.3 Hz, 1H), 4.11 - 3.96 (m, 3H), 3.41 - 3.31 (m, 3H), 3.02 - 2.95 (m, 1H), 2.69 (d, *J* = 15.7 Hz, 1H), 2.45 (d, *J* = 13.5 Hz, 1H), 2.16 - 2.09 (m, 1H). LCMS (ESI) calcd. for $C_{24}H_{25}N_6O_2S_2{}^+$ [M+H]$^+$: 493.15, found, 493.1.

**Example 84: Preparation of 3-(4-((4-(2-chlorothieno[3,2-d]pyrimidin-4-yl)piperazin-1-yl)methyl)-1-thioxoisoindolin-2-yl)piperidine-2,6-dione (GT-08971)**

With reference to the method of Scheme 4, the target compound (GT-08971) was obtained (white solid, 11 mg, yield 25 %). $^1$H NMR (400 MHz, DMSO) δ 11.75 (s, 1H), 11.20 (s, 1H), 8.39 (d, *J*= 5.5 Hz, 1H), 7.99 (t, *J* = 6.9 Hz, 2H), 7.69 (t, *J* = 7.7 Hz, 1H), 7.47 (d, *J*= 5.5 Hz, 1H), 6.05 (d, *J* = 23.3 Hz, 1H), 5.34 (d, *J* = 20.4 Hz, 1H), 4.88 (d, *J*= 19.8 Hz, 1H), 4.68 (s, 2H), 4.45 (d, *J* = 26.0 Hz, 2H), 3.81 (d, *J* = 11.5 Hz, 2H), 3.55 (s, 2H), 3.33 (s, 2H), 3.05 - 2.94 (m, 1H), 2.69 (d, *J* = 16.5 Hz, 1H), 2.44 (d, *J*= 9.9 Hz, 1H), 2.16 - 2.08 (m, 1H). LCMS (ESI) calcd. for $C_{24}H_{24}ClN_6O_2S_2{}^+$ [M+H]$^+$: 527.11, found, 527.2.

**Example 85: Preparation of 3-(4-((4-(2-morpholinothieno[3,2-d]pyrimidin-4-yl)piperazin-1-yl)methyl)-1-thioxoisoindolin-2-yl)piperidine-2,6-dione (GT-09032)**

With reference to the method of Scheme 4, the target compound (GT-09032) was obtained (white solid, 20 mg, yield 41 %). $^1$H NMR (400 MHz, DMSO) δ 12.06 (s, 1H), 11.20 (s, 1H), 8.35 (d, *J*= 5.3 Hz, 1H), 8.02 (d, *J*= 13.5 Hz, 2H), 7.72 - 7.65 (m, 1H), 7.58 (s, 1H), 6.01 (d, *J* = 9.3 Hz, 1H), 5.41 (d, *J* = 20.3 Hz, 1H), 4.92 (d, 1H), 4.82 - 4.59 (m, 2H), 4.57 - 4.35 (m, 2H), 3.84 (s, 6H), 3.72 (d, *J* = 4.7 Hz, 4H), 3.51 (s, 2H), 3.03 - 2.95 (m, 1H), 2.68 (d, 1H), 2.49 - 2.35 (m, 1H), 2.16 - 2.06 (m, 1H), 1.38 - 1.25 (m, 2H). LCMS (ESI) calcd. for $C_{28}H_{32}N_7O_3S_2{}^+$ [M+H]$^+$: 578.20, found, 578.3.

**Example 86: Preparation of 3-(4-((4-(4-morpholinothieno[3,2-d]pyrimidin-2-yl)piperazin-1-yl)methyl)-1-thioxoisoindolin-2-yl)piperidine-2,6-dione (GT-09033)**

With reference to the method of Scheme 4, the target compound (GT-09033) was obtained (white solid, 9 mg, yield 19 %). $^1$H NMR (400 MHz, DMSO) δ 11.83 (s, 1H), 11.20 (s, 1H), 8.33 (d, *J*= 5.3 Hz, 1H), 8.02 (d, *J* = 13.2 Hz, 2H), 7.67 (t, *J*= 15.0, 7.3 Hz, 1H), 7.52 (s, 1H), 6.02 (d, *J* = 9.3 Hz, 1H), 5.41 (d, *J* = 20.2 Hz, 1H), 4.91 (d, *J* = 20.4 Hz, 1H), 4.76 (s, 2H), 4.54 - 4.35 (m, 2H), 3.98 (s, 4H), 3.80 - 3.76 (m, 4H), 3.60 (s, 2H), 3.30 (s, 3H), 3.00 (dd, *J* = 15.4, 10.5 Hz, 1H), 2.69 (d, *J*= 15.5 Hz, 1H), 2.48 - 2.35 (m, 1H), 2.17 - 2.05 (m, 1H), 1.43 - 1.16 (m, 2H). LCMS (ESI) calcd. for $C_{28}H_{32}N_7O_3S_2{}^+$ [M+H]$^+$: 578.20, found, 578.3.

**Example 87: Preparation of 3-(4-((4-((4'-chloro-[1,1'-biphenyl]-2-yl)amino)piperidin-1-yl)methyl)-1-thioxoisoindolin-2-yl)piperidine-2,6-dione (GT-09034)**

With reference to the method of Scheme 4, the target compound (GT-09034) was obtained (white solid, 4 mg, yield 9 %). $^1$H NMR (400 MHz, DMSO) δ 11.18 (d, *J* = 23.6 Hz, 1H), 10.79 (s, 1H), 7.98 (t, *J= 7.5 Hz,* 1H), 7.67 (t, *J* = 7.6 Hz, 1H), 7.49 *(d, J*= 8.2 Hz, 3H), 7.41 (t, *J* = 8.9 Hz, 2H), 7.19 (t, *J* = 7.7 Hz, 1H), 7.02 - 6.97 (m, 1H), 6.81 (d, *J* = 8.1 Hz, 1H), 6.73 (t, *J*= 7.4 Hz, 1H), 5.99 (s, 1H), 4.99 - 4.88 (m, 1H), 4.40 (dd, *J* = 29.0, 15.6 Hz, 2H), 3.21 - 2.95 (m, 5H), 2.70 (d, *J* = 17.1 Hz, 2H), 2.47 - 2.36 (m, 1H), 2.19 - 1.99 (m, 4H), 1.86 - 1.59 (m, 2H), 1.29 - 1.15 (m, 1H). LCMS (ESI) calcd. for $C_{31}H_{32}ClN_4O_2S{}^+$ [M+H]$^+$: 559.19, found, 559.2.

**Example 88: Preparation of 3-(4-((4-(4-(piperazin-1-yl)-6,7-dihydrothieno[3,2-d]pyrimidin-2-yl)piperazin-1-yl) methyl)-1-thioxoisoindolin-2-yl)piperidine-2,6-dione (GT-09035)**

With reference to the method of Scheme 4, the target compound (GT-09035) was obtained (white solid, 4 mg, yield 71 %). $^1$H NMR (400 MHz, DMSO) δ 11.63 (s, 1H), 11.21 (s, 1H), 9.42 (s, 2H), 8.00 (t, *J*= 7.1 Hz, 2H), 7.69 (t, 1H), 6.01 (d, *J*= 8.4 Hz, 1H), 5.41 (d, *J= 20.3* Hz, 1H), 4.91 (d, *J* = 20.1 Hz, 1H), 4.59 (d, *J*= 13.1 Hz, 2H), 4.52 - 4.35 (m, 2H), 3.83 (s,

4H), 3.50 - 3.43 (m, 4H), 3.28 (t, *J*= 8.1 Hz, 2H), 3.18 - 3.09 (m, 8H), 3.05 - 2.95 (m, 1H), 2.69 (d, *J* = 16.0 Hz, 1H), 2.48 - 2.39 (m, 1H), 2.15 - 2.05 (m, 1H). LCMS (ESI) calcd. for $C_{28}H_{35}N_8O_2S_2{}^+$ [M+H]$^+$: 579.23, found, 579.3.

**Example 89: Preparation of 3-(4-((4-(4-(piperazin-1-yl)thieno[3,2-d]pyrimidin-2-yl)piperazin-1-yl)methyl)-1-thioxoisoindolin-2-yl)piperidine-2,6-dione (GT-09036)**

With reference to the method of Scheme 4, the target compound (GT-09036) was obtained (white solid, 14 mg, yield 87 %). $^1$H NMR (400 MHz, DMSO) δ 11.88 (s, 1H), 11.21 (s, 1H), 9.68 (s, 2H), 8.34 (d, *J*= 5.4 Hz, 1H), 8.01 (t, *J* = 6.9 Hz, 2H), 7.68 (t, *J*= 7.7 Hz, 1H), 7.52 (d, *J*= 5.5 Hz, 1H), 6.02 (d, *J* = 7.9 Hz, 1H), 5.44 (d, *J* = 20.4 Hz, 1H), 4.91 (d, *J* = 20.6 Hz, 1H), 4.81 (s, 2H), 4.57 - 4.41 (m, 2H), 4.20 (s, 4H), 3.69 (s, 2H), 3.39 (s, 2H), 3.28 (s, 6H), 3.03 - 2.94 (m, 1H), 2.69 (d, *J*= 15.9 Hz, 1H), 2.45 (d, *J*= 13.0 Hz, 1H), 2.15 - 2.07 (m, 1H). LCMS (ESI) calcd. for $C_{28}H_{33}N_8O_2S_2{}^+$ [M+H]$^+$: 577.22, found, 577.3.

**Example 90: Preparation of 3-(5-((4-(6-methylpyridin-2-yl)piperazin-1-yl)methyl)-1-thioxoisoindolin-2-yl)piperidine-2,6-dione (GT-09214)**

With reference to the method of Scheme 4, the target compound (GT-09214) was obtained (red solid, 11 mg, yield 21 %). $^1$H NMR (400 MHz, DMSO) δ 11.14 (s, 1H), 8.01 - 7.94 (m, 1H), 7.92 - 7.89 (m, 1H), 7.82 (d, *J* = 8.0 Hz, 1H), 7.02 (s, 1H), 6.80 (d, *J*= 6.8 Hz, 1H), 4.81 (dd, *J* = 58.2, 20.3 Hz, 1H), 4.60 - 4.50 (m, 2H), 4.48 (s, 2H), 3.94-3.88 (m, 3H), 3.57 (s, 2H), 3.42 (s, 2H), 3.24-3.18 (m, 3H), 2.96 (d, *J* = 12.3 Hz, 1H), 2.85 (s, 1H), 2.66 (d, *J* = 13.6 Hz, 1H), 2.57 (d, *J*= 17.3 Hz, 1H), 2.19 - 2.03 (m, 1H). LCMS (ESI) calcd. for $C_{24}H_{28}N_5O_2S^+$ [M+H]$^+$: 450.20, found, 450.1.

**Example 91: Preparation of 3-(5-((4-(6-chloropyridin-2-yl)piperazin-1-yl)methyl)-1-thioxoisoindolin-2-yl)piperidine-2,6-dione (GT-09215)**

With reference to the method of Scheme 4, the target compound (GT-09215) was obtained (red solid, 10 mg, yield 18 %). $^1$H NMR (400 MHz, DMSO) δ 11.14 (s, 1H), 7.98 (d, *J* = 8.0 Hz, 1H), 7.91 (s, 1H), 7.78 (s, 1H), 7.64 (t, *J* = 7.9 Hz, 1H), 6.89 (d, *J*= 8.5 Hz, 1H), 6.78 (d, *J*= 7.5 Hz, 1H), 4.88 (d, *J*= 20.4 Hz, 1H), 4.74 (d, *J* = 20.3 Hz, 1H), 4.50 (s, 2H), 4.33 (d, *J* = 13.5 Hz, 2H), 3.40 (s, 2H), 3.31 - 3.27 (m, 2H), 3.09 (s, 2H), 3.00 - 2.92 (m, 1H), 2.73 (s, 1H), 2.70 - 2.56 (m, 2H), 2.13 - 2.06 (m, 1H). LCMS (ESI) calcd. for $C_{23}H_{25}ClN_5O_2S^+$ [M+H]$^+$: 470.14, found, 470.1.

**Example 92: Preparation of 3-(5-((4-(pyridin-2-yl)piperazin-1-yl)methyl)-1-thioxoisoindolin-2-yl)piperidine-2,6-dione (GT-09216)**

With reference to the method of Scheme 4, the target compound (GT-09216) was obtained (red solid, 13 mg, yield 25 %). $^1$H NMR (400 MHz, DMSO) δ 11.14 (s, 1H), 8.12 (d, *J* = 5.5 Hz, 1H), 7.97 (d, *J= 8.7 Hz,* 1H), 7.91 (d, *J* = 3.3 Hz, 1H), 7.87 - 7.79 (m, 1H), 7.23 (d, *J* = 7.6 Hz, 1H), 6.94 (t, *J* = 6.2 Hz, 1H), 4.91 - 4.70 (m, 1H), 4.53 (d, *J* = 9.7 Hz, 2H), 4.48 (s, 2H), 3.95 (s, 1H), 3.89 - 3.72 (m, 1H), 3.60 (s, 2H), 3.43 (s, 2H), 3.20 (d, *J* = 21.5 Hz, 3H), 2.97 (s, 1H), 2.65 (d, *J* = 17.2 Hz, 1H), 2.17 - 2.02 (m, 1H). LCMS (ESI) calcd. for $C_{23}H_{26}N_5O_2S^+$ [M+H]$^+$: 436.18, found, 436.1.

**Example 93: Preparation of 3-(5-((4-(5-chloropyridin-2-yl)piperazin-1-yl)methyl)-1-thioxoisoindolin-2-yl)piperidine-2,6-dione (GT-09217)**

With reference to the method of Scheme 4, the target compound (GT-09217) was obtained (white solid, 22 mg, yield 39 %). $^1$H NMR (400 MHz, DMSO) δ 11.14 (s, 1H), 8.17 (d, *J* = 2.4 Hz, 1H), 7.96 (d, *J* = 9.8 Hz, 1H), 7.90 (d, *J* = 4.3 Hz, 1H), 7.83 (t, *J*= 9.1 Hz, 1H), 7.70 (dd, *J* = 9.1, 2.6 Hz, 1H), 6.99 (dd, *J* = 9.1, 4.0 Hz, 1H), 4.81 (d, *J* = 37.2 Hz, 1H), 4.50 (s, 2H), 4.34 *(d, J*= 13.2 Hz, 2H), 3.77 - 3.71 (m, 1H), 3.38 (s, 4H), 3.12 (d, *J* = 13.8 Hz, 3H), 3.01 - 2.91 (m, 1H), 2.65 (d, *J* = 17.4 Hz, 2H), 2.14 - 2.04 (m, 1H). LCMS (ESI) calcd. for $C_{23}H_{25}ClN_5O_2S^+$ [M+H]$^+$: 470.14, found, 470.1.

**Example 94: Preparation of 3-(5-((4-(4-chloropyridin-2-yl)piperazin-1-yl)methyl)-1-thioxoisoindolin-2-yl)piperidine-2,6-dione (GT-09218)**

With reference to the method of Scheme 4, the target compound (GT-09218) was obtained (white solid, 22 mg, yield 54 %). $^1$H NMR (400 MHz, DMSO) δ 11.14 (s, 1H), 8.12 (d, *J* = 5.4 Hz, 1H), 7.97 (d, *J* = 8.1 Hz, 2H), 7.80 (d, *J*= 8.9 Hz, 1H), 7.10 (s, 1H), 6.84 (dd, *J*= 5.4, 1.4 Hz, 1H), 4.49 (s, 2H), 4.42 (d, *J* = 13.3 Hz, 2H), 4.00 (s, 2H), 3.77 (s, 1H), 3.38 (d, *J* = 12.6 Hz, 4H), 3.10 (s, 2H), 2.99 - 2.90 (m, 1H), 2.65 (d, *J* = 17.6 Hz, 2H), 2.14 - 2.06 (m, 1H). LCMS (ESI) calcd. for $C_{23}H_{25}ClN_5O_2S^+$ [M+H]$^+$: 470.14, found, 470.1.

**Example 95: Preparation of 3-(5-((4-(3-chloropyridin-2-yl)piperazin-1-yl)methyl)-1-thioxoisoindolin-2-yl)piperidine-2,6-dione (GT-09219)**

With reference to the method of Scheme 4, the target compound (GT-09219) was obtained (white solid, 24 mg, yield 59 %). $^1$H NMR (400 MHz, DMSO) δ 11.14 (s, 1H), 8.26 (dd, *J* = 4.7, 1.5 Hz, 1H), 7.97 (d, *J* = 8.2 Hz, 1H), 7.88 - 7.81 (m, 2H), 7.09 (dd, *J* = 7.8, 4.7 Hz, 1H), 4.89 - 4.70 (m, 1H), 4.54 (d, *J* = 3.7 Hz, 2H), 3.74 - 3.60 (m, 2H), 3.52 - 3.45 (m, 1H), 3.42 (d, *J*= 10.6 Hz, 2H), 3.34 (d, *J*= 12.5 Hz, 2H), 3.22 (d, *J* = 6.7 Hz, 3H), 2.95 (dd, *J* = 12.9, 4.5 Hz, 1H), 2.65 (d, *J* = 17.4 Hz, 1H), 2.57 (d, *J* = 15.3 Hz, 1H), 2.14 - 2.06 (m, 1H). LCMS (ESI) calcd. for $C_{23}H_{25}ClN_5O_2S^+$ $[M+H]^+$: 470.14, found, 470.1.

**Example 96: Preparation of 3-(5-((4-(3-fluoropyridin-2-yl)piperazin-1-yl)methyl)-1-thioxoisoindolin-2-yl)piperidine-2,6-dione (GT-09220)**

With reference to the method of Scheme 4, the target compound (GT-09220) was obtained (white solid, 14 mg, yield 26 %). $^1$H NMR (400 MHz, DMSO) δ 11.14 (s, 1H), 8.06 (d, *J* = 4.7 Hz, 1H), 7.96 (s, 1H), 7.81 (d, *J*= 8.6 Hz, 1H), 7.60 (dd, *J*= 13.3, 8.0 Hz, 1H), 7.03 - 6.95 (m, 1H), 4.91 - 4.74 (m, 1H), 4.51 (s, 2H), 4.04 (d, *J*= 13.1 Hz, 2H), 3.77 - 3.65 (m, 2H), 3.41 (s, 4H), 3.21 (s, 2H), 2.96 (d, *J* = 12.1 Hz, 1H), 2.65 (d, *J* = 18.0 Hz, 1H), 2.59 (s, 1H), 2.14 - 2.06 (m, 1H). LCMS (ESI) calcd. for $C_{23}H_{25}FN_5O_2S^+$ $[M+H]^+$: 454.17, found, 454.1.

**Example 97: Preparation of 3-(5-((4-(6-fluoropyridin-2-yl)piperazin-1-yl)methyl)-1-thioxoisoindolin-2-yl)piperidine-2,6-dione (GT-09221)**

With reference to the method of Scheme 4, the target compound (GT-09221) was obtained (white solid, 20 mg, yield 37 %). $^1$H NMR (400 MHz, DMSO) δ 11.14 (s, 1H), 8.02 - 7.89 (m, 2H), 7.78 (dd, *J*= 14.6, 6.7 Hz, 2H), 6.79 (d, *J* = 6.1 Hz, 1H), 6.39 (d, *J*= 5.7 Hz, 1H), 4.81 (dd, *J*= 55.9, 20.3 Hz, 2H), 4.50 (s, 2H), 4.31 (d, *J* = 12.0 Hz, 2H), 3.39 (s, 3H), 3.33 - 3.30 (m, 1H), 3.29 - 3.19 (m, 1H), 3.11 (s, 2H), 3.02 - 2.90 (m, 1H), 2.65 (d, *J* = 17.4 Hz, 1H), 2.57 (d, *J* = 14.2 Hz, 1H), 2.15 - 2.04 (m, 1H). LCMS (ESI) calcd. for $C_{23}H_{25}FN_5O_2S^+$ $[M+H]^+$: 454.17, found, 454.1.

**Example 98: Preparation of 3-(5-((4-(5-fluoropyridin-2-yl)piperazin-1-yl)methyl)-1-thioxoisoindolin-2-yl)piperidine-2,6-dione (GT-09222)**

With reference to the method of Scheme 4, the target compound (GT-09222) was obtained (white solid, 17 mg, yield 32 %). $^1$H NMR (400 MHz, DMSO) δ 11.14 (s, 1H), 8.15 (d, *J* = 3.0 Hz, 1H), 7.96 (d, *J* = 8.2 Hz, 1H), 7.82 (d, *J* = 8.6 Hz, 1H), 7.64 - 7.58 (m, 1H), 6.99 (dd, *J* = 9.3, 3.4 Hz, 1H), 4.93 - 4.74 (m, 1H), 4.67 (d, *J* = 32.3 Hz, 1H), 4.50 (s, 2H), 4.27 (d, *J*= 12.5 Hz, 2H), 3.42 - 3.31 (m, 3H), 3.31 - 3.19 (m, 2H), 3.10 (d, *J* = 7.4 Hz, 2H), 2.95 (dd, *J* = 12.9, 4.5 Hz, 1H), 2.65 (d, *J* = 17.6 Hz, 1H), 2.57 (d, *J* = 13.9 Hz, 1H), 2.14 - 2.05 (m, 1H). LCMS (ESI) calcd. for $C_{23}H_{25}FN_5O_2S^+$ $[M+H]^+$: 454.17, found, 454.1.

**Example 99: Preparation of 3-(1-thioxo-5-((4-(3-(trifluoromethyl)pyridin-2-yl)piperazin-1-yl)methyl)isoindolin-2-yl)piperidine-2,6-dione (GT-09223)**

With reference to the method of Scheme 4, the target compound (GT-09223) was obtained (white solid, 13 mg, yield 22 %). $^1$H NMR (400 MHz, DMSO) δ 11.14 (s, 1H), 8.59 (d, *J* = 4.6 Hz, 1H), 8.14 (d, *J*= 7.9 Hz, 1H), 7.97 (d, *J* = 8.4 Hz, 2H), 7.84 (d, *J* = 8.1 Hz, 1H), 7.32 (dd, *J* = 7.5, 5.0 Hz, 1H), 4.89 (d, *J* = 20.4 Hz, 1H), 4.74 (d, *J* = 20.3 Hz, 1H), 4.55 (s, 2H), 3.65 - 3.59 (m, 1H), 3.50 (t, *J* = 12.7 Hz, 3H), 3.42 (d, *J* = 11.9 Hz, 3H), 3.21 (s, 2H), 2.97 (s, 1H), 2.65 (d, *J*= 18.0 Hz, 1H), 2.55 (s, 1H), 2.14 - 2.06 (m, 1H). LCMS (ESI) calcd. for $C_{24}H_{25}F_3N_5O_2S^+$ $[M+H]^+$: 504.17, found, 504.1.

**Example 100: Preparation of 3-(1-thioxo-5-((4-(6-(trifluoromethyl)pyridin-2-yl)piperazin-1-yl)methyl)isoindolin-2-yl)piperidine-2,6-dione (GT-09224)**

With reference to the method of Scheme 4, the target compound (GT-09224) was obtained (white solid, 22 mg, yield 37 %). $^1$H NMR (400 MHz, DMSO) δ 11.14 (s, 1H), 8.00 - 7.92 (m, 2H), 7.87 - 7.77 (m, 2H), 7.22 (d, *J* = 8.7 Hz, 1H), 7.16 (d, *J* = 7.3 Hz, 1H), 4.88 (d, *J* = 20.3 Hz, 1H), 4.74 (d, *J* = 20.4 Hz, 1H), 4.51 (s, 2H), 4.42 (d, *J* = 14.7 Hz, 2H), 3.42 (s, 4H), 3.33 - 3.29 (m, 1H), 3.13 (s, 2H), 2.96 (d, *J* = 12.4 Hz, 1H), 2.65 (d, *J* = 18.8 Hz, 1H), 2.55 (s, 1H), 2.14 - 2.06 (m, 1H). LCMS (ESI) calcd. for $C_{24}H_{25}F_3N_5O_2S^+$ $[M+H]^+$: 504.17, found, 504.1.

**Example 101: Preparation of 3-(1-thioxo-5-((4-(5-(trifluoromethyl)pyridin-2-yl)piperazin-1-yl)methyl)isoindolin-2-yl)piperidine-2,6-dione (GT-09225)**

With reference to the method of Scheme 4, the target compound (GT-09225) was obtained (white solid, 24 mg, yield 41 %). $^1$H NMR (400 MHz, DMSO) δ 11.14 (s, 1H), 8.48 (s, 1H), 7.97 (s, 1H), 7.91 (dd, *J* = 9.1, 2.0 Hz, 2H), 7.81 (d, *J* = 8.1 Hz, 1H), 7.07 (d, *J* = 9.1 Hz, 1H), 4.88 (d, *J* = 20.4 Hz, 1H), 4.74 (d, *J* = 20.3 Hz, 1H), 4.53 (s, 2H), 4.50 (s, 3H), 3.47 (d, *J*= 11.9 Hz, 2H), 3.40 (d, *J*= 11.4 Hz, 2H), 3.11 (s, 2H), 2.96 (d, *J* = 12.8 Hz, 1H), 2.70 - 2.57 (m, 2H), 2.14 - 2.06 (m, 1H). LCMS (ESI) calcd. for $C_{24}H_{25}F_3N_5O_2S^+$ [M+H]$^+$: 504.17, found, 504.1.

**Example 102: Preparation of 3-(5-((4-(3,5-dichloropyridin-2-yl)piperazin-1-yl)methyl)-1-thioxoisoindolin-2-yl) piperidine-2,6-dione (GT-09226)**

With reference to the method of Scheme 4, the target compound (GT-09226) was obtained (white solid, 24 mg, yield 41 %). $^1$H NMR (400 MHz, DMSO) δ 11.14 (s, 1H), 8.33 (d, *J* = 2.3 Hz, 1H), 8.13 (d, *J*= 2.3 Hz, 1H), 7.97 (d, *J* = 7.7 Hz, 2H), 7.83 (d, *J* = 8.3 Hz, 1H), 4.88 (d, *J* = 20.4 Hz, 1H), 4.74 (d, *J* = 20.3 Hz, 1H), 4.53 (s, 2H), 3.83 (d, *J* = 13.0 Hz, 2H), 3.42 - 3.30 (m, 5H), 3.21 (d, *J* = 9.9 Hz, 2H), 2.99-2.94 (m, 1H), 2.65 (d, *J* = 17.8 Hz, 2H), 2.14 - 2.06 (m, 1H). LCMS (ESI) calcd. for $C_{23}H_{24}Cl_2N_5O_2S^+$ [M+H]$^+$: 504.10, found, 504.0.

**Example 103: Preparation of 3-(5-((4-(3-fluoro-4-iodopyridin-2-yl)piperazin-1-yl)methyl)-1-thioxoisoindolin-2-yl)piperidine-2,6-dione (GT-09227)**

With reference to the method of Scheme 4, the target compound (GT-09227) was obtained (white solid, 22 mg, yield 33 %). $^1$H NMR (400 MHz, DMSO) δ 11.14 (s, 1H), 7.96 (s, 1H), 7.82 (t, *J* = 8.8 Hz, 1H), 7.72 (d, *J*= 5.1 Hz, 1H), 7.39 (dd, *J*= 5.0, 3.7 Hz, 1H), 4.88 (d, *J*= 20.4 Hz, 1H), 4.74 (d, *J*= 20.4 Hz, 1H), 4.50 (s, 2H), 4.04 (d, *J* = 13.6 Hz, 2H), 3.46 (s, 1H), 3.44 - 3.34 (m, 4H), 3.19 (s, 2H), 3.04 - 2.91 (m, 1H), 2.65 (d, *J* = 17.6 Hz, 1H), 2.57 (d, *J* = 15.4 Hz, 1H), 2.14 - 2.06 (m, 1H). LCMS (ESI) calcd. for $C_{23}H_{24}FIN_5O_2S^+$ [M+H]$^+$: 580.07, found, 580.0.

**Example 104: Preparation of 3-(5-((4-(3-chloro-5-(trifluoromethyl)pyridin-2-yl)piperazin-1-yl)methyl)-1-thioxoisoindolin-2-yl)piperidine-2,6-dione (GT-09228)**

With reference to the method of Scheme 4, the target compound (GT-09228) was obtained (white solid, 23 mg, yield 37 %). $^1$H NMR (400 MHz, DMSO) δ 11.14 (s, 1H), 8.62 (s, 1H), 8.29 (s, 1H), 7.97 (d, *J* = 7.6 Hz, 2H), 7.82 (d, *J*= 8.0 Hz, 1H), 4.88 (d, *J* = 20.3 Hz, 1H), 4.74 (d, *J* = 20.4 Hz, 1H), 4.53 (s, 2H), 4.10 (d, *J*= 13.3 Hz, 2H), 3.46 (dd, *J* = 25.0, 12.4 Hz, 5H), 3.24 (s, 2H), 2.96 (d, *J* = 12.3 Hz, 1H), 2.70 - 2.57 (m, 2H), 2.14 - 2.06 (m, 1H). LCMS (ESI) calcd. for $C_{24}H_{24}ClF_3N_5O_2S^+$ [M+H]$^+$: 538.13, found, 538.0.

**Example 105: Preparation of 3-(5-((4-(pyridin-3-yl)piperazin-1-yl)methyl)-1-thioxoisoindolin-2-yl)piperidine-2,6-dione (GT-09229)**

With reference to the method of Scheme 4, the target compound (GT-09229) was obtained (white solid, 22 mg, yield 43 %). $^1$H NMR (400 MHz, DMSO) δ 11.15 (s, 1H), 8.54 (d, *J* = 2.6 Hz, 1H), 8.26 (d, *J* = 5.2 Hz, 1H), 8.10 (dd, *J* = 8.6, 2.7 Hz, 1H), 8.01 (s, 1H), 7.96 *(d, J*= 7.9 Hz, 1H), 7.88 - 7.83 (m, 2H), 4.89 (d, *J* = 20.4 Hz, 1H), 4.74 (d, *J*= 20.4 Hz, 1H), 4.54 (s, 2H), 4.10 (s, 3H), 3.84 - 3.67 (m, 3H), 3.23 (s, 2H), 3.05 (dd, *J*= 7.3, 4.9 Hz, 1H), 2.99 - 2.91 (m, 1H), 2.65 (d, *J* = 18.0 Hz, 1H), 2.58 (d, *J* = 12.8 Hz, 1H), 2.14 - 2.06 (m, 1H). LCMS (ESI) calcd. for $C_{23}H_{26}N_5O_2S^+$ [M+H]$^+$: 436.18, found, 436.1.

**Example 106: Preparation of 3-(5-((4-(6-chloropyridin-3-yl)piperazin-1-yl)methyl)-1-thioxoisoindolin-2-yl)piperidine-2,6-dione (GT-09230)**

With reference to the method of Scheme 4, the target compound (GT-09230) was obtained (white solid, 16 mg, yield 29 %). $^1$H NMR (400 MHz, DMSO) δ 11.14 (s, 1H), 8.13 (d, *J* = 3.1 Hz, 1H), 7.99 - 7.95 (m, 1H), 7.82 (d, *J* = 8.6 Hz, 1H), 7.48 (dd, *J*= 8.9, 3.2 Hz, 1H), 7.36 (d, *J*= 8.8 Hz, 1H), 4.88 (d, *J* = 20.4 Hz, 1H), 4.74 (d, *J* = 20.4 Hz, 1H), 4.52 (s, 2H), 3.89 (d, *J* = 12.3 Hz, 2H), 3.56 - 3.49 (m, 1H), 3.39 (d, *J* = 10.7 Hz, 2H), 3.34 - 3.24 (m, 2H), 3.18 (d, *J* = 8.9 Hz, 2H), 2.96 (d, *J* = 12.2 Hz, 1H), 2.65 (d, *J* = 17.5 Hz, 1H), 2.59 (s, 1H), 2.14 - 2.06 (m, 1H). LCMS (ESI) calcd. for $C_{23}H_{25}ClN_5O_2S^+$ [M+H]$^+$: 470.14, found, 470.1.

**Example 107: Preparation of 3-(5-((4-(4,5-dichloropyridin-3-yl)piperazin-1-yl)methyl)-1-thioxoisoindolin-2-yl)piperidine-2,6-dione (GT-09231)**

With reference to the method of Scheme 4, the target compound (GT-09231) was obtained (white solid, 23 mg, yield 39 %). $^1$H NMR (400 MHz, DMSO) δ 11.14 (s, 1H), 8.51 (s, 1H), 8.40 *(d, J* = 4.1 Hz, 1H), 7.99 (d, *J* = 5.7 Hz, 1H), 7.95 (d, *J* = 11.4 Hz, 1H), 7.92 - 7.87 (m, 1H), 7.85 (d, *J* = 8.6 Hz, 1H), 4.89 (d, *J* = 20.3 Hz, 1H), 4.75 (d, *J* = 20.4 Hz, 1H), 4.57 (s, 2H), 3.54 (d, *J* = 10.9 Hz, 3H), 3.47 - 3.37 (m, 4H), 3.26 (s, 2H), 2.95 (dd, *J*= 12.8, 4.4 Hz, 1H), 2.70 - 2.57 (m, 2H), 2.15 - 2.06 (m, 1H). LCMS (ESI) calcd. for $C_{23}H_{24}Cl_2N_5O_2S^+$ $[M+H]^+$: 504.10, found, 504.1.

**Example 108: Preparation of 3-(5-((4-(2,4-dichloropyridin-3-yl)piperazin-1-yl)methyl)-1-thioxoisoindolin-2-yl)piperidine-2,6-dione (GT-09232)**

With reference to the method of Scheme 4, the target compound (GT-09232) was obtained (white solid, 11 mg, yield 19 %). $^1$H NMR (400 MHz, DMSO) δ 11.14 (s, 1H), 8.18 (s, 1H), 7.97 (s, 1H), 7.83 (d, *J* = 8.5 Hz, 1H), 7.60 (s, 1H), 4.89 (d, *J*= 20.4 Hz, 1H), 4.75 (d, *J*= 20.4 Hz, 1H), 4.57 (s, 2H), 3.75 (s, 2H), 3.39 - 3.35 (m, 2H), 3.29 - 3.09 (m, 5H), 2.97 (s, 1H), 2.65 (d, *J*= 17.0 Hz, 2H), 2.15 - 2.04 (m, 1H). LCMS (ESI) calcd. for $C_{23}H_{24}Cl_2N_5O_2S^+$ $[M+H]^+$: 504.10, found, 504.1.

**Example 109: Preparation of 3-(5-((4-(pyridin-4-yl)piperazin-1-yl)methyl)-1-thioxoisoindolin-2-yl)piperidine-2,6-dione (GT-09233)**

With reference to the method of Scheme 4, the target compound (GT-09233) was obtained (white solid, 18 mg, yield 35 %). $^1$H NMR (400 MHz, DMSO) δ 11.14 (s, 1H), 8.35 (d, *J* = 7.2 Hz, 2H), 7.96 (d, *J* = 8.0 Hz, 2H), 7.80 (d, *J* = 7.8 Hz, 1H), 7.26 (d, *J* = 7.5 Hz, 2H), 4.88 (d, *J* = 20.4 Hz, 1H), 4.73 (d, *J*= 20.3 Hz, 1H), 4.51 (s, 2H), 4.41 (s, 2H), 3.72 (s, 2H), 3.34 - 3.30 (m, 1H), 3.23 (s, 3H), 3.06 (dd, *J* = 7.3, 4.8 Hz, 1H), 2.96 (d, *J* = 12.3 Hz, 1H), 2.65 (d, *J*= 18.2 Hz, 1H), 2.57 (d, *J* = 13.7 Hz, 1H), 2.13 - 2.06 (m, 1H). LCMS (ESI) calcd. for $C_{23}H_{26}N_5O_2S^+$ $[M+H]^+$: 436.18, found, 436.1.

**Example 110: Preparation of 3-(5-((4-(2-methylpyridin-4-yl)piperazin-1-yl)methyl)-1-thioxoisoindolin-2-yl)piperidine-2,6-dione (GT-09234)**

With reference to the method of Scheme 4, the target compound (GT-09234) was obtained (white solid, 16 mg, yield 30 %). $^1$H NMR (400 MHz, DMSO) δ 11.15 (s, 1H), 8.24 (d, *J* = 7.0 Hz, 1H), 7.96 (d, *J*= 8.1 Hz, 2H), 7.80 (d, *J* = 7.9 Hz, 1H), 7.24 - 7.06 (m, 2H), 4.88 (d, *J* = 20.4 Hz, 1H), 4.73 (d, *J* = 20.4 Hz, 1H), 4.51 (s, 2H), 4.38 (s, 2H), 3.71 (s, 2H), 3.45 (s, 1H), 3.31 (s, 2H), 3.22 (s, 2H), 3.06 (dd, *J*= 7.3, 4.8 Hz, 1H), 2.96 (d, *J* = 12.2 Hz, 1H), 2.65 (d, *J* = 18.3 Hz, 1H), 2.62 - 2.54 (m, 1H), 2.14 - 2.05 (m, 1H), 1.20 (t, *J*= 7.3 Hz, 2H). LCMS (ESI) calcd. for $C_{24}H_{28}N_5O_2S^+$ $[M+H]^+$: 450.20, found, 450.1.

**Example 111: Preparation of 3-(5-((4-(3,4-difluoropyridin-2-yl)piperazin-1-yl)methyl)-1-thioxoisoindolin-2-yl)piperidine-2,6-dione (GT-09235)**

With reference to the method of Scheme 4, the target compound (GT-09235) was obtained (white solid, 18 mg, yield 33 %). $^1$H NMR (400 MHz, DMSO) δ 11.14 (s, 1H), 8.07 - 8.02 (m, 1H), 7.97 (d, *J* = 7.5 Hz, 2H), 7.81 (d, *J* = 8.7 Hz, 1H), 7.12 - 7.05 (m, 1H), 4.88 (d, *J= 20.4* Hz, 1H), 4.74 (d, *J* = 20.4 Hz, 1H), 4.51 (s, 2H), 4.10 (d, *J* = 13.7 Hz, 2H), 3.47 (s, 2H), 3.40 (d, *J*= 12.5 Hz, 3H), 3.20 (s, 2H), 2.95 (dd, *J* = 12.8, 4.5 Hz, 1H), 2.65 (d, *J* = 17.7 Hz, 2H), 2.13 - 2.07 (m, 1H). LCMS (ESI) calcd. for $C_{23}H_{24}F_2N_5O_2S^+$ $[M+H]^+$: 472.16, found, 472.1.

**Example 112: Preparation of 3-(5-((4-(3-bromopyridin-4-yl)piperazin-1-yl)methyl)-1-thioxoisoindolin-2-yl)piperidine-2,6-dione (GT-09236)**

With reference to the method of Scheme 4, the target compound (GT-09236) was obtained (white solid, 21 mg, yield 35 %). $^1$H NMR (400 MHz, DMSO) δ 11.15 (s, 1H), 8.82 (s, 1H), 8.53 (d, *J* = 6.4 Hz, 1H), 8.02 - 7.95 (m, 1H), 7.95 - 7.89 (m, 1H), 7.89 - 7.83 (m, 1H), 7.41 (dd, *J* = 6.2, 4.4 Hz, 1H), 4.89 (d, *J* = 20.4 Hz, 1H), 4.74 (d, *J* = 20.3 Hz, 1H), 4.58 (d, *J* = 7.1 Hz, 2H), 3.63 (s, 3H), 3.45 (s, 3H), 3.27 (s, 3H), 2.96 (d, *J*= 13.0 Hz, 1H), 2.84 (d, *J*= 11.8 Hz, 1H), 2.70 - 2.58 (m, 2H), 2.15 - 2.04 (m, 1H). LCMS (ESI) calcd. for $C_{23}H_{25}BrN_5O_2S^+$ $[M+H]^+$: 514.09, found, 514.1.

**Example 113: Preparation of 3-(5-((4-(3-fluoropyridin-4-yl)piperazin-1-yl)methyl)-1-thioxoisoindolin-2-yl)piperidine-2,6-dione (GT-09237)**

With reference to the method of Scheme 4, the target compound (GT-09237) was obtained (white solid, 15 mg, yield 28 %). $^1$H NMR (400 MHz, DMSO) δ 11.15 (s, 1H), 8.75 (d, *J* = 8.3 Hz, 1H), 8.37 (d, *J* = 6.9 Hz, 1H), 8.00 (s, 1H), 7.96 *(d,*

*J*= 7.9 Hz, 1H), 7.83 (d, *J* = 8.0 Hz, 1H), 7.44 (dd, *J* = 8.7, 7.1 Hz, 1H), 4.88 (d, *J*= *20.4* Hz, 1H), 4.73 (d, *J*= *20.4* Hz, 1H), 4.54 (s, 2H), 3.58 (s, 2H), 3.37 (s, 6H), 3.07-3.01 (m, 6.4 Hz, 1H), 2.99 - 2.91 (m, 1H), 2.65 (d, *J* = 18.1 Hz, 1H), 2.60 (s, 1H), 2.15 - 2.06 (m, 1H). LCMS (ESI) calcd. for $C_{23}H_{25}FN_5O_2S^+$ [M+H]$^+$: 454.17, found, 454.1.

**Example 114: Preparation of 3-(5-((4-(3-chloropyridin-4-yl)piperazin-1-yl)methyl)-1-thioxoisoindolin-2-yl)piperidine-2,6-dione (GT-09238)**

With reference to the method of Scheme 4, the target compound (GT-09238) was obtained (white solid, 11 mg, yield 19 %). $^1$H NMR (400 MHz, DMSO) δ 11.15 (s, 1H), 8.74 (s, 1H), 8.50 (d, *J* = 6.5 Hz, 1H), 7.99 (d, *J* = 13.8 Hz, 1H), 7.96 - 7.90 (m, 1H), 7.89 - 7.83 (m, 1H), 7.49 - 7.39 (m, 1H), 4.89 (d, *J* = 20.4 Hz, 1H), 4.74 (d, *J*= 20.4 Hz, 1H), 4.57 (d, *J* = 7.4 Hz, 2H), 4.33 (s, 1H), 3.71-3.66 (m, 3H), 3.43 (s, 3H), 3.31 (s, 2H), 2.96 (d, *J* = 12.5 Hz, 1H), 2.65 (d, *J* = 17.7 Hz, 1H), 2.58 (d, *J* = 17.5 Hz, 1H), 2.17 - 2.05 (m, 1H). LCMS (ESI) calcd. for $C_{23}H_{25}ClN_5O_2S^+$ [M+H]$^+$: 470.14, found, 470.1.

**Example 115: Preparation of 3-(5-((4-(2-chloro-3-fluoropyridin-4-yl)piperazin-1-yl)methyl)-1-thioxoisoindolin-2-yl)piperidine-2,6-dione (GT-09239)**

With reference to the method of Scheme 4, the target compound (GT-09239) was obtained (white solid, 13 mg, yield 23 %). $^1$H NMR (400 MHz, DMSO) δ 11.15 (s, 1H), 8.01 (d, *J* = 5.6 Hz, 1H), 7.98 (s, 1H), 7.96 - 7.88 (m, 1H), 7.84 (t, *J* = 8.9 Hz, 1H), 7.15 - 7.09 (m, 1H), 4.88 (d, *J*= 20.4 Hz, 1H), 4.74 (d, *J*= 20.4 Hz, 1H), 4.52 (s, 2H), 3.89 - 3.88 (m, 1H), 3.85 (s, 2H), 3.50 (t, *J*= 12.0 Hz, 2H), 3.39 (d, *J* = 11.6 Hz, 2H), 3.23 (s, 2H), 2.97 (s, 1H), 2.65 (d, *J* = 17.7 Hz, 1H), 2.59 (s, 1H), 2.15 - 2.05 (m, 1H). LCMS (ESI) calcd. for $C_{23}H_{24}ClFN_5O_2S^+$ [M+H]$^+$: 488.13, found, 488.1.

**Example 116: Preparation of 3-(5-((4-(2-bromo-3-fluoropyridin-4-yl)piperazin-1-yl)methyl)-1-thioxoisoindolin-2-yl)piperidine-2,6-dione (GT-09241)**

With reference to the method of Scheme 4, the target compound (GT-09241) was obtained (white solid, 11 mg, yield 18 %). $^1$H NMR (400 MHz, DMSO) δ 11.14 (s, 1H), 8.00 (d, *J* = 5.5 Hz, 1H), 7.96 (s, 1H), 7.90 (d, *J* = 7.7 Hz, 1H), 7.82 (t, *J* = 8.6 Hz, 1H), 7.16 - 7.08 (m, 1H), 4.88 (d, *J* = 20.4 Hz, 1H), 4.74 (d, *J*= 20.4 Hz, 1H), 4.52 (s, 2H), 3.86 (d, *J*= 11.7 Hz, 2H), 3.42 (dd, *J* = 22.5, 12.0 Hz, 4H), 3.23 (s, 3H), 2.96 (d, *J*= 12.2 Hz, 1H), 2.65 (d, *J*= 17.0 Hz, 1H), 2.59 (s, 1H), 2.17 - 2.02 (m, 1H). LCMS (ESI) calcd. for $C_{23}H_{24}BrFN_5O_2S^+$ [M+H]$^+$: 532.08, found, 532.0.

**Example 117: Preparation of 3-(5-((4-(3-bromo-2-chloropyridin-4-yl)piperazin-1-yl)methyl)-1-thioxoisoindolin-2-yl)piperidine-2,6-dione (GT-09240)**

With reference to the method of Scheme 4, the target compound (GT-09240) was obtained (white solid, 14 mg, yield 22 %). $^1$H NMR (400 MHz, DMSO) δ 11.14 (s, 1H), 8.25 (d, *J*= 5.4 Hz, 1H), 7.97 (d, *J* = 8.0 Hz, 2H), 7.85 (t, *J* = 8.9 Hz, 1H), 7.16 (d, *J*= 5.4 Hz, 1H), 4.89 (d, *J*= 20.4 Hz, 1H), 4.74 (d, *J* = 20.4 Hz, 1H), 4.57 (s, 2H), 3.68 (s, 2H), 3.58 - 3.53 (m, 1H), 3.45 (d, *J* = 10.3 Hz, 2H), 3.39 - 3.30 (m, 2H), 3.25 (s, 2H), 2.96 (d, *J* = 12.3 Hz, 1H), 2.65 (d, *J* = 17.2 Hz, 1H), 2.57 (d, *J* = 14.4 Hz, 1H), 2.14 - 2.06 (m, 1H). LCMS (ESI) calcd. for $C_{23}H_{24}BrClN_5O_2S^+$ [M+H]$^+$: 548.05, found, 548.0.

**Example 118: Preparation of 3-(5-((4-(2,3-dichloropyridin-4-yl)piperazin-1-yl)methyl)-1-thioxoisoindolin-2-yl)piperidine-2,6-dione (GT-09354)**

With reference to the method of Scheme 4, the target compound (GT-09354) was obtained (white solid, 13 mg, yield 24 %). $^1$H NMR (400 MHz, DMSO) δ 11.15 (s, 1H), 8.22 (d, *J* = 5.5 Hz, 1H), 7.98 (d, *J* = 7.7 Hz, 2H), 7.83 (d, *J* = 8.4 Hz, 1H), 7.21 (t, *J* = 4.8 Hz, 1H), 4.89 (d, *J*= 20.4 Hz, 1H), 4.74 (d, *J* = 20.4 Hz, 1H), 4.56 (s, 2H), 3.71 (d, *J*= 12.2 Hz, 2H), 3.51 - 3.49 (m, 1H), 3.44 (d, *J*= 11.8 Hz, 2H), 3.39 - 3.31 (m, 2H), 3.26 (s, 2H), 3.04 - 2.92 (m, 1H), 2.65 (d, *J* = 17.1 Hz, 1H), 2.57 (d, *J* = 14.4 Hz, 1H), 2.15 - 2.06 (m, 1H). LCMS (ESI) calcd. for $C_{23}H_{24}Cl_2N_5O_2S^+$ [M+H]$^+$: 504.10, found, 504.0.

**Example 119: Preparation of 3-(5-((4-(2,3-difluoropyridin-4-yl)piperazin-1-yl)methyl)-1-thioxoisoindolin-2-yl)piperidine-2,6-dione (GT-09242)**

With reference to the method of Scheme 4, the target compound (GT-09242) was obtained (white solid, 13 mg, yield 22 %). $^1$H NMR (400 MHz, DMSO) δ 11.14 (s, 1H), 7.96 (s, 1H), 7.89 (d, *J* = 4.3 Hz, 1H), 7.83 - 7.77 (m, 2H), 7.03 (t, *J*= 5.8 Hz, 1H), 4.88 (d, *J*= 20.4 Hz, 1H), 4.74 (d, *J*= 20.3 Hz, 1H), 4.52 (s, 2H), 3.90 (d, *J* = 12.5 Hz, 2H), 3.51 (s, 2H), 3.40 (d, *J* = 11.9 Hz, 2H), 3.24 (s, 3H), 2.96 (d, *J* = 12.6 Hz, 1H), 2.65 (d, *J* = 17.3 Hz, 1H), 2.59 (s, 1H), 2.15 - 2.05 (m, 1H). LCMS (ESI) calcd. for $C_{23}H_{24}F_2N_5O_2S^+$ [M+H]$^+$: 472.16, found, 472.1.

**Example 120: Preparation of 3-(5-((4-(3-chloro-5-fluoropyridin-4-yl)piperazin-1-yl)methyl)-1-thioxoisoindolin-2-yl)piperidine-2,6-dione (GT-09243)**

With reference to the method of Scheme 4, the target compound (GT-09243) was obtained (white solid, 14 mg, yield 25 %). $^1$H NMR (400 MHz, DMSO) δ 11.14 (s, 1H), 8.48 (d, *J* = 3.3 Hz, 1H), 8.42 (s, 1H), 8.02 - 7.95 (m, 1H), 7.95 - 7.89 (m, 1H), 7.89 - 7.83 (m, 1H), 4.89 (d, *J= 20.4* Hz, 1H), 4.74 (d, *J* = 20.4 Hz, 1H), 4.55 (s, 2H), 3.67 (d, *J* = 11.3 Hz, 2H), 3.60 (s, 2H), 3.42 (d, *J* = 12.5 Hz, 2H), 3.20 (s, 2H), 3.03 - 2.92 (m, 1H), 2.85 (s, 1H), 2.64 (dd, *J*= 17.4, 13.3 Hz, 2H), 2.14 - 2.06 (m, 1H). LCMS (ESI) calcd. for $C_{23}H_{24}ClFN_5O_2S^+$ [M+H]$^+$: 488.13, found, 488.1.

**Example 121: Preparation of 3-(5-((4-(4-chloro-3-fluoropyridin-2-yl)piperazin-1-yl)methyl)-1-thioxoisoindolin-2-yl)piperidine-2,6-dione (GT-09244)**

With reference to the method of Scheme 4, the target compound (GT-09244) was obtained (white solid, 15 mg, yield 26 %). $^1$H NMR (400 MHz, DMSO) δ 11.14 (s, 1H), 8.02 (d, *J* = 5.3 Hz, 1H), 7.96 (d, *J*= 7.4 Hz, 1H), 7.93 - 7.87 (m, 1H), 7.83 (t, *J* = 9.0 Hz, 1H), 7.18 (t, *J* = 4.9 Hz, 1H), 4.88 (d, *J*= 20.3 Hz, 1H), 4.74 (d, *J* = 20.2 Hz, 1H), 4.50 (s, 2H), 4.08 (d, *J* = 13.2 Hz, 2H), 3.90 (s, 1H), 3.79 - 3.73 (m, 1H), 3.43 (dd, *J* = 26.3, 12.4 Hz, 4H), 3.20 (s, 2H), 2.96 (d, *J* = 12.5 Hz, 1H), 2.72 - 2.58 (m, 2H), 2.17 - 2.05 (m, 1H). LCMS (ESI) calcd. for $C_{23}H_{24}ClFN_5O_2S^+$ [M+H]$^+$: 488.13, found, 488.1.

**Example 122: Preparation of 3-(5-((4-(3-bromo-4-chloropyridin-2-yl)piperazin-1-yl)methyl)-1-thioxoisoindolin-2-yl)piperidine-2,6-dione (GT-09245)**

With reference to the method of Scheme 4, the target compound (GT-09245) was obtained (white solid, 15 mg, yield 25 %). $^1$H NMR (400 MHz, DMSO) δ 11.15 (s, 1H), 8.25 (d, *J* = 5.2 Hz, 1H), 7.97 (d, *J*= 7.9 Hz, 1H), 7.94 - 7.88 (m, 1H), 7.85 (t, *J* = 9.4 Hz, 1H), 7.37 (d, *J* = 5.2 Hz, 1H), 4.89 (d, *J* = 20.4 Hz, 1H), 4.74 (d, *J* = 20.4 Hz, 1H), 4.55 (d, *J* = 3.5 Hz, 2H), 3.81 (d, *J*= 12.8 Hz, 2H), 3.55 - 3.51 (m, 1H), 3.39 (d, *J* = 12.2 Hz, 4H), 3.23 (s, 2H), 2.96 (d, *J* = 12.3 Hz, 1H), 2.65 (d, *J* = 17.2 Hz, 1H), 2.57 (d, *J* = 15.8 Hz, 1H), 2.17 - 2.03 (m, 1H). LCMS (ESI) calcd. for $C_{23}H_{24}BrClN_5O_2S^+$ [M+H]$^+$: 548.05, found, 548.1.

**Example 123: Preparation of 3-(5-((4-(3,5-dichloropyridin-4-yl)piperazin-1-yl)methyl)-1-thioxoisoindolin-2-yl) piperidine-2,6-dione (GT-09246)**

With reference to the method of Scheme 4, the target compound (GT-09246) was obtained (white solid, 16 mg, yield 27 %). $^1$H NMR (400 MHz, DMSO) δ 11.15 (s, 1H), 8.51 (s, 2H), 8.03 (s, 1H), 7.99 - 7.95 (m, 1H), 7.89 (d, *J* = 7.6 Hz, 1H), 4.89 (d, *J*= 20.4 Hz, 1H), 4.74 (d, *J*= 20.3 Hz, 1H), 4.57 (s, 2H), 3.80 (s, 2H), 3.53 - 3.33 (m, 5H), 3.21 (s, 2H), 2.97 (s, 1H), 2.69 - 2.57 (m, 2H), 2.17 - 2.03 (m, 1H). LCMS (ESI) calcd. for $C_{23}H_{24}Cl_2N_5O_2S^+$ [M+H]$^+$: 504.10, found, 504.1.

**Example 124: Preparation of 3-(5-((4-(2,6-dimethylpyridin-4-yl)piperazin-1-yl)methyl)-1-thioxoisoindolin-2-yl) piperidine-2,6-dione (GT-09247)**

With reference to the method of Scheme 4, the target compound (GT-09247) was obtained (white solid, 17 mg, yield 31 %). $^1$H NMR (400 MHz, DMSO) δ 11.15 (s, 1H), 7.95 (d, *J* = 8.1 Hz, 2H), 7.80 (d, *J*= 7.7 Hz, 1H), 7.05 (s, 2H), 5.96 (d, *J* = 10.2 Hz, 1H), 4.88 (d, *J* = 20.4 Hz, 1H), 4.73 (d, *J* = 20.4 Hz, 1H), 4.50 (s, 2H), 4.35 (s, 2H), 3.69 (s, 3H), 3.49 (s, 1H), 3.34 - 3.28 (m, 1H), 3.18 (d, *J* = 12.6 Hz, 3H), 3.08 - 3.02 (m, 1H), 2.97 (s, 1H), 2.65 (d, *J*= 17.8 Hz, 2H), 2.57 (d, *J*= 15.2 Hz, 1H), 2.14 - 2.05 (m, 1H), 1.20 (t, *J*= 7.3 Hz, 2H). LCMS (ESI) calcd. for $C_{25}H_{30}N_5O_2S^+$ [M+H]$^+$: 464.21, found, 464.1.

**Example 125: Preparation of 3-(5-((4-(3,6-dichloropyridazin-4-yl)piperazin-1-yl)methyl)-1-thioxoisoindolin-2-yl) piperidine-2,6-dione (GT-09248)**

With reference to the method of Scheme 4, the target compound (GT-09248) was obtained (white solid, 6 mg, yield 10 %). $^1$H NMR (400 MHz, DMSO) δ 11.14 (s, 1H), 7.98 (d, *J* = 7.8 Hz, 1H), 7.93 (s, 1H), 7.79 (d, *J*= 7.6 Hz, 1H), 7.58 (s, 1H), 4.88 (d, *J*= 20.3 Hz, 1H), 4.74 (d, *J*= 20.2 Hz, 1H), 4.54 (s, 2H), 3.90 (s, 2H), 3.42 (s, 3H), 3.25 (s, 3H), 3.03 - 2.92 (m, 1H), 2.65 (d, *J* = 16.5 Hz, 2H), 2.58 (s, 1H), 2.14 - 2.06 (m, 1H). LCMS (ESI) calcd. for $C_{22}H_{23}Cl_2N_6O_2S^+$ [M+H]$^+$: 505.10, found, 505.1.

**Example 126: Preparation of 3-(5-((4-(1,3,5-triazin-2-yl)piperazin-1-yl)methyl)-1-thioxoisoindolin-2-yl)piperidine-2,6-dione (GT-09249)**

With reference to the method of Scheme 4, the target compound (GT-09249) was obtained (white solid, 12 mg,

yield 23 %). $^1H$ NMR (400 MHz, DMSO) δ 8.70 (s, 2H), 7.89 (t, $J$ = 6.1 Hz, 2H), 7.83 (s, 1H), 4.74 (d, $J$= 20.1 Hz, 2H), 4.48 (t, $J$= 12.9 Hz, 2H), 3.54 (s, 2H), 3.46 (s, 2H), 3.23 (d, $J$= 7.1 Hz, 1H), 3.12 (s, 2H), 3.06 (dd, $J$ = 7.3, 4.9 Hz, 1H), 2.94 (dd, $J$ = 17.9, 12.8 Hz, 1H), 2.85 (s, 1H), 2.70 - 2.57 (m, 2H), 2.13 (d, $J$= 7.3 Hz, 1H). LCMS (ESI) $C_{21}H_{24}N_7O_2S^+$ [M+H]$^+$: 438.17, found, 438.1.

**Example 127: Preparation of 3-(5-((4-(perfluoropyridin-4-yl)piperazin-1-yl)methyl)-1-thioxoisoindolin-2-yl)piperidine-2,6-dione (GT-09250)**

With reference to the method of Scheme 4, the target compound (GT-09250) was obtained (white solid, 14 mg, yield 24 %). $^1H$ NMR (400 MHz, DMSO) δ 11.14 (s, 1H), 7.97 (d, $J$ = 8.0 Hz, 2H), 7.81 (s, 1H), 4.88 (d, $J$= 20.4 Hz, 1H), 4.74 (d, $J$ = 20.3 Hz, 1H), 4.53 (s, 2H), 3.81 (s, 2H), 3.73 (s, 2H), 3.40 (s, 2H), 3.22 (s, 3H), 3.01 - 2.90 (m, 1H), 2.70 - 2.57 (m, 2H), 2.13 - 2.06 (m, 1H). LCMS (ESI) $C_{23}H_{22}F_4N_5O_2S^+$ [M+H]$^+$: 508.14, found, 508.1.

**Example 128: Preparation of 3-(5-((4-(6-chloropyridazin-3-yl)piperazin-1-yl)methyl)-1-thioxoisoindolin-2-yl)piperidine-2,6-dione (GT-09251)**

With reference to the method of Scheme 4, the target compound (GT-09251) was obtained (white solid, 18 mg, yield 33 %). $^1H$ NMR (400 MHz, DMSO) δ 11.14 (s, 1H), 7.96 (s, 1H), 7.89 (d, $J$ = 4.3 Hz, 1H), 7.80 (d, $J$ = 8.9 Hz, 1H), 7.66 (d, $J$ = 9.6 Hz, 1H), 7.49 (d, $J$ = 9.6 Hz, 1H), 4.88 (d, $J$ = 20.4 Hz, 1H), 4.74 (d, $J$ = 20.4 Hz, 1H), 4.50 (s, 2H), 4.43 (d, $J$ = 12.7 Hz, 2H), 3.71 - 3.66 (m, 1H), 3.48 (s, 1H), 3.42 (d, $J$ = 13.8 Hz, 2H), 3.19 (s, 1H), 3.17 (s, 2H), 2.96 (d, $J$= 12.5 Hz, 1H), 2.71 - 2.57 (m, 2H), 2.18 - 2.03 (m, 1H). LCMS (ESI) $C_{22}H_{24}ClN_6O_2S^+$ [M+H]$^+$: 471.14, found, 471.1.

**Example 129: Preparation of 3-(5-((4-(pyrimidin-2-yl)piperazin-1-yl)methyl)-1-thioxoisoindolin-2-yl)piperidine-2,6-dione (GT-09252)**

With reference to the method of Scheme 4, the target compound (GT-09252) was obtained (white solid, 13 mg, yield 25 %). $^1H$ NMR (400 MHz, DMSO) δ 8.45 (d, $J$ = 4.7 Hz, 2H), 7.91 (dd, $J$= 18.3, 11.0 Hz, 2H), 7.83 (d, $J$ = 8.3 Hz, 1H), 6.77 (t, $J$ = 4.8 Hz, 1H), 4.69 (d, $J$ = 14.1 Hz, 2H), 4.50 (d, $J$ = 9.3 Hz, 2H), 4.03 (s, 2H), 3.83 - 3.70 (m, 1H), 3.43 (d, $J$ = 9.1 Hz, 4H), 3.16 - 2.99 (m, 3H), 2.94 - 2.79 (m, 1H), 2.62 (d, $J$ = 16.0 Hz, 1H), 2.13 (d, $J$ = 7.5 Hz, 1H). LCMS (ESI) $C_{22}H_{25}N_6O_2S^+$ [M+H]$^+$: 437.18, found, 437.1.

**Example 130: Preparation of 3-(5-((4-(pyrazin-2-yl)piperazin-1-yl)methyl)-1-thioxoisoindolin-2-yl)piperidine-2,6-dione (GT-09253)**

With reference to the method of Scheme 4, the target compound (GT-09253) was obtained (white solid, 14 mg, yield 27 %). $^1H$ NMR (400 MHz, DMSO) δ 11.14 (s, 1H), 8.42 (d, $J$ = 1.1 Hz, 1H), 8.21 - 8.14 (m, 1H), 7.97 (s, 1H), 7.95 (d, $J$ = 2.6 Hz, 1H), 7.90 (d, $J$= 3.2 Hz, 1H), 7.83 (t, $J$ = 9.2 Hz, 1H), 4.88 (d, $J$= 20.3 Hz, 1H), 4.74 (d, $J$= 20.4 Hz, 1H), 4.51 (s, 2H), 4.44 (d, $J$= 13.5 Hz, 2H), 3.46 - 3.32 (m, 4H), 3.21 (d, $J$ = 7.3 Hz, 1H), 3.15 (d, $J$ = 11.0 Hz, 2H), 2.96 (d, $J$ = 12.7 Hz, 1H), 2.65 (d, $J$ = 17.6 Hz, 1H), 2.58 (d, $J$ = 13.9 Hz, 1H), 2.15 - 2.06 (m, 1H). LCMS (ESI) $C_{22}H_{25}N_6O_2S^+$ [M+H]$^+$: 437.18, found, 437.1.

**Example 131: Preparation of 3-(5-((4-(5,6-dichloropyrimidin-4-yl)piperazin-1-yl)methyl)-1-thioxoisoindolin-2-yl)piperidine-2,6-dione (GT-09254)**

With reference to the method of Scheme 4, the target compound (GT-09254) was obtained (white solid, 11 mg, yield 19 %). $^1H$ NMR (400 MHz, DMSO) δ 11.14 (s, 1H), 8.52 (s, 1H), 7.97 (d, $J$ = 8.0 Hz, 1H), 7.92 (s, 1H), 7.78 (d, $J$ = 7.8 Hz, 1H), 5.96 (d, $J$ = 9.8 Hz, 1H), 4.88 (d, $J$ = 20.3 Hz, 1H), 4.74 (d, $J$ = 20.3 Hz, 1H), 4.51 (s, 2H), 4.36 (s, 2H), 3.54 (s, 2H), 3.40 (s, 2H), 3.21 (s, 2H), 3.02 - 2.92 (m, 1H), 2.69 - 2.57 (m, 2H), 2.14 - 2.06 (m, 1H). LCMS (ESI) calcd. for $C_{22}H_{23}Cl_2N_6O_2S^+$ [M+H]$^+$: 505.10, found, 505.1.

**Example 132: Preparation of 3-(5-((4-(5,6-dichloropyridazin-4-yl)piperazin-1-yl)methyl)-1-thioxoisoindolin-2-yl)piperidine-2,6-dione (GT-09255)**

With reference to the method of Scheme 4, the target compound (GT-09255) was obtained (white solid, 14 mg, yield 24 %). $^1H$ NMR (400 MHz, DMSO) δ 11.14 (s, 1H), 9.06 (s, 1H), 8.00 - 7.94 (m, 2H), 7.81 (d, $J$= 8.5 Hz, 1H), 4.88 (d, $J$ = 20.4 Hz, 1H), 4.74 (d, $J$ = 20.4 Hz, 1H), 4.54 (s, 2H), 3.97 (d, $J$ = 11.7 Hz, 3H), 3.56 (s, 2H), 3.25 (s, 4H), 3.03 - 2.92 (m, 1H), 2.70 - 2.57 (m, 2H), 2.14 - 2.07 (m, 1H). LCMS (ESI) calcd. for $C_{22}H_{23}Cl_2N_6O_2S^+$ [M+H]$^+$: 505.10, found, 505.1.

**Example 133: Preparation of 3-(5-((4-(isoxazol-3-yl)piperidin-1-yl)methyl)-1-thioxoisoindolin-2-yl)piperidine-2,6-dione (GT-09256)**

With reference to the method of Scheme 4, the target compound (GT-09256) was obtained (white solid, 8 mg, yield 16 %). $^1$H NMR (400 MHz, DMSO) δ 11.11 (s, 2H), 7.85 (d, *J* = 7.9 Hz, 2H), 7.60 (s, 2H), 7.49 (d, *J*= 8.0 Hz, 2H), 5.95 (dd, *J*= 13.0, 4.5 Hz, 2H), 5.43 (s, 2H), 4.81 (d, *J* = 20.0 Hz, 2H), 4.69 (s, 1H), 4.64 (s, 3H), 3.02 - 2.91 (m, 2H), 2.64 (d, *J* = 17.7 Hz, 3H), 2.54 (d, *J* = 4.4 Hz, 1H), 2.09 (d, *J* = 9.0, 2H). LCMS (ESI) calcd. for $C_{22}H_{25}N_4O_3S^+$ $[M+H]^+$: 425.16, found, 425.1.

**Example 134: Preparation of 3-(5-((4-(6-fluorobenzold]isoxazol-3-yl)piperazin-1-yl)methyl)-1-thioxoisoindolin-2-yl)piperidine-2,6-dione (GT-09257)**

With reference to the method of Scheme 4, the target compound (GT-09257) was obtained (white solid, 3 mg, yield 5 %). $^1$H NMR (400 MHz, DMSO) δ 11.14 (s, 1H), 8.07 (s, 1H), 7.99 (s, 1H), 7.90 (s, 1H), 7.78 (s, 1H), 7.61 (d, *J* = 7.2 Hz, 1H), 7.25 (s, 1H), 4.88 (d, *J* = 20.5 Hz, 1H), 4.77 (s, 1H), 4.57 (s, 1H), 4.11 (s, 2H), 3.69 (s, 1H), 3.47 (s, 4H), 3.45 - 3.41 (m, 2H), 3.26 - 3.22 (m, 1H), 2.96 (d, *J* = 13.5 Hz, 1H), 2.65 (d, *J* = 16.5 Hz, 2H), 2.09 (d, *J* = 6.5 Hz, 1H). LCMS (ESI) calcd. for $C_{25}H_{25}FN_5O_3S^+$ $[M+H]^+$: 494.17, found, 494.1.

**Example 135: Preparation of 3-(5-((4-(isoxazolo[4,5-c]pyridin-3-yl)piperazin-1-yl)methyl)-1-thioxoisoindolin-2-yl)piperidine-2,6-dione (GT-09258)**

With reference to the method of Scheme 4, the target compound (GT-09258) was obtained (white solid, 5 mg, yield 8 %). $^1$H NMR (400 MHz, DMSO) δ 11.15 (s, 1H), 9.48 (d, *J*= 5.2 Hz, 1H), 8.73 (d, *J*= 6.1 Hz, 1H), 8.00 - 7.95 (m, 1H), 7.83 (dd, *J*= 17.1, 7.2 Hz, 2H), 4.89 (d, *J*= 20.5 Hz, 1H), 4.75 (d, *J*= 20.3 Hz, 1H), 4.57 (s, 2H), 4.18 (s, 2H), 3.83 - 3.77 (m, 1H), 3.62 (s, 3H), 3.31 (s, 3H), 2.96 (d, *J*= 12.4 Hz, 1H), 2.65 (d, *J* = 16.5 Hz, 2H), 2.17 - 2.05 (m, 1H). LCMS (ESI) calcd. for $C_{24}H_{25}N_6O_3S^+$ $[M+H]^+$: 477.17, found, 477.1.

**Example 136: Preparation of 3-(5-((4-(6-fluorobenzo[d]isothiazol-3-yl)piperazin-1-yl)methyl)-1-thioxoisoindolin-2-yl)piperidine-2,6-dione (GT-09259)**

With reference to the method of Scheme 4, the target compound (GT-09259) was obtained (white solid, 4 mg, yield 7 %). $^1$H NMR (400 MHz, DMSO) δ 11.14 (s, 1H), 8.17 (s, 1H), 8.00 (d, *J* = 8.9 Hz, 2H), 7.93 (s, 1H), 7.81 (s, 1H), 7.34 (t, *J* = 7.7 Hz, 1H), 4.89 (d, *J* = 20.6 Hz, 1H), 4.78 (s, 1H), 4.58 (s, 2H), 4.04 (s, 2H), 3.44 (s, 4H), 3.29 - 3.21 (m, 3H), 3.03 - 2.92 (m, 1H), 2.69 - 2.58 (m, 2H), 2.15 - 2.08 (m, 1H). LCMS (ESI) calcd. for $C_{25}H_{25}FN_5O_2S_2^+$ $[M+H]^+$: 510.14, found, 510.1.

**Example 137: Preparation of 3-(5-((4-(6-fluoro-1H-indazol-3-yl)piperazin-1-yl)methyl)-1-thioxoisoindolin-2-yl) piperidine-2,6-dione (GT-09260)**

With reference to the method of Scheme 4, the target compound (GT-09260) was obtained (white solid, 5 mg, yield 9 %). $^1$H NMR (400 MHz, DMSO) δ 12.28 (s, 1H), 11.15 (s, 1H), 7.99 (d, *J* = 7.9 Hz, 1H), 7.94 (s, 1H), 7.82 (dd, *J* = 12.1, 6.8 Hz, 2H), 7.16 (dd, *J* = 9.8, 2.2 Hz, 1H), 6.88 (dd, *J* = 9.2, 2.2 Hz, 1H), 4.89 (d, *J* = 20.5 Hz, 1H), 4.75 (d, *J* = 20.4 Hz, 1H), 4.55 (s, 2H), 3.93 (s, 2H), 3.57 (s, 1H), 3.53 (d, *J* = 19.3 Hz, 1H), 3.31 (d, *J* = 8.4 Hz, 5H), 2.96 (d, *J* = 12.2 Hz, 1H), 2.65 (d, *J* = 16.1 Hz, 2H), 2.14 - 2.07 (m, 1H). LCMS (ESI) calcd. for $C_{25}H_{26}FN_6O_2S^+$ $[M+H]^+$: 493.18, found, 493.1.

**Example 138: Preparation of 3-(5-((4-(1H-pyrrolo[2,3-b]pyridin-3-yl)piperazin-1-yl)methyl)-1-thioxoisoindolin-2-yl)piperidine-2,6-dione (GT-09261)**

With reference to the method of Scheme 4, the target compound (GT-09261) was obtained (white solid, 5 mg, yield 8 %). $^1$H NMR (400 MHz, DMSO) δ 11.72 (s, 1H), 11.14 (s, 2H), 8.31 - 8.24 (m, 2H), 8.00 (s, 1H), 7.90 - 7.81 (m, 2H), 7.18 - 7.13 (m, 2H), 4.87 (s, 1H), 4.78 (s, 1H), 4.64 (s, 1H), 4.56 (s, 2H), 4.52 - 4.46 (m, 1H), 3.33 (s, 4H), 3.15 (s, 3H), 2.96 (d, *J*= 10.2 Hz, 2H), 2.65 (d, *J*= 16.4 Hz, 2H). LCMS (ESI) calcd. for $C_{25}H_{27}N_6O_2S^+$ $[M+H]^+$: 475.19, found, 475.1.

**Example 139: Preparation of 3-(5-((4-(5-fluoro-1H-benzo[d]imidazol-1-yl)piperidin-1-yl)methyl)-1-thioxoisoindolin-2-yl)piperidine-2,6-dione (GT-09262)**

With reference to the method of Scheme 4, the target compound (GT-09262) was obtained (white solid, 6 mg, yield 10 %). $^1$H NMR (400 MHz, DMSO) δ 11.15 (s, 1H), 9.13 (s, 1H), 8.20 (dd, *J* = 9.1, 4.6 Hz, 1H), 8.01 (d, *J* = 4.4 Hz, 1H), 7.93 (d, *J*= 10.4 Hz, 1H), 7.87 (s, 1H), 7.65 (dd, *J*= 8.9, 2.3 Hz, 1H), 7.41 (d, *J* = 9.2 Hz, 1H), 4.87 (s, 1H), 4.78 (s, 1H), 4.54 (s,

2H), 3.21 (d, *J* = 7.3 Hz, 4H), 2.98 (s, 2H), 2.66 (d, *J* = 11.1 Hz, 3H), 2.32 (s, 3H), 2.16 - 2.04 (m, 2H). LCMS (ESI) calcd. for $C_{26}H_{27}FN_5O_2S^+$ $[M+H]^+$: 492.19, found, 492.1.

**Example 140: Preparation of 3-(5-((4-(5-fluoro-2-oxo-2,3-dihydro-1H-benzo[d]imidazol-1-yl)piperidin-1-yl) methyl)-1-thioxoisoindolin-2-yl)piperidine-2,6-dione (GT-09263)**

With reference to the method of Scheme 4, the target compound (GT-09263) was obtained (white solid, 5 mg, yield 8 %). $^1$H NMR (400 MHz, DMSO) δ 11.14 (s, 1H), 11.10 (s, 1H), 8.06 - 7.96 (m, 1H), 7.93 (s, 1H), 7.80 (d, *J*= 8.1 Hz, 1H), 7.48 (s, 1H), 6.85 (d, *J*= 8.8 Hz, 2H), 4.87 (s, 1H), 4.78 (s, 1H), 4.49 (s, 3H), 3.50 (s, 3H), 3.19 (s, 3H), 2.97 (s, 1H), 2.76 (d, *J* = 9.2 Hz, 2H), 2.65 (d, *J*= 16.0 Hz, 2H), 2.08 (d, *J* = 18.1 Hz, 2H). LCMS (ESI) calcd. for $C_{26}H_{27}FN_5O_3S^+$ $[M+H]^+$: 508.18, found, 508.1.

**Example 141: Preparation of 3-(5-((4-(3-bromo-5-chloropyridin-4-yl)piperazin-1-yl)methyl)-1-thioxoisoindolin-2-yl)piperidine-2,6-dione (GT-09264)**

With reference to the method of Scheme 4, the target compound (GT-09264) was obtained (white solid, 30 mg, yield 47 %). $^1$H NMR (400 MHz, DMSO) δ 11.13 (d, *J*= 12.1 Hz, 1H), 8.63 (s, 1H), 8.53 (s, 1H), 7.97 (d, *J*= 7.6 Hz, 1H), 7.90 (s, 1H), 7.87 (d, *J*= 8.4 Hz, 1H), 4.86 (s, 1H), 4.77 (s, 1H), 4.63 (s, 1H), 4.57 (s, 2H), 3.83 (d, *J* = 11.6 Hz, 2H), 3.41 (d, *J* = 9.5 Hz, 4H), 3.22 (s, 2H), 3.03 - 2.91 (m, 1H), 2.70 - 2.57 (m, 2H), 2.14 - 2.05 (m, 1H). LCMS (ESI) calcd. for $C_{23}H_{24}BrClN_5O_2S^+$ $[M+H]^+$: 548.05, found, 548.1.

**Example 142: Preparation of 3-(5-((4-(pyridazin-3-yl)piperazin-1-yl)methyl)-1-thioxoisoindolin-2-yl)piperidine-2,6-dione (GT-09265)**

With reference to the method of Scheme 4, the target compound (GT-09265) was obtained (white solid, 16 mg, yield 31 %). $^1$H NMR (400 MHz, DMSO) δ 11.11 (s, 1H), 8.87 (d, *J* = 2.3 Hz, 1H), 7.97 (d, *J*= 3.7 Hz, 2H), 7.90 (t, *J* = 6.2 Hz, 2H), 7.84 (d, *J* = 8.1 Hz, 1H), 4.58 - 4.47 (m, 3H), 3.62 (s, 2H), 3.25 (d, *J*= 6.4 Hz, 2H), 3.06 (dd, *J* = 7.2, 4.9 Hz, 3H), 2.84 (d, *J* = 11.8 Hz, 1H), 2.62 (d, *J* = 16.5 Hz, 1H), 2.36 (s, 4H), 2.15 (d, *J* = 5.4 Hz, 1H). LCMS (ESI) calcd. for $C_{22}H_{25}N_6O_2S^+$ $[M+H]^+$: 437.18, found, 437.1.

**Example 143: Preparation of 3-(5-((4-(benzo[d]isoxazol-3-yl)piperidin-1-yl)methyl)-1-thioxoisoindolin-2-yl)piperidine-2,6-dione (GT-09266)**

With reference to the method of Scheme 4, the target compound (GT-09266) was obtained (white solid, 20 mg, yield 36 %). $^1$H NMR (400 MHz, DMSO) δ 11.15 (s, 1H), 8.14 (d, *J* = 8.0 Hz, 1H), 7.99 (d, *J= 7.3* Hz, 2H), 7.87 - 7.82 (m, 1H), 7.74 (d, *J* = 8.4 Hz, 1H), 7.66 (t, *J* = 7.7 Hz, 1H), 7.40 (t, *J* = 7.5 Hz, 1H), 5.96 (d, *J*= 12.5 Hz, 1H), 4.90 (d, *J* = 20.3 Hz, 1H), 4.78 (s, 1H), 4.63 (s, 1H), 4.52 (s, 2H), 3.53 (d, *J*= 10.5 Hz, 2H), 3.16 (d, *J*= 10.5 Hz, 2H), 2.96 (dd, *J* = 21.3, 9.6 Hz, 1H), 2.65 (d, *J*= 16.7 Hz, 2H), 2.40 (d, *J* = 12.4 Hz, 2H), 2.20 (d, *J* = 13.4 Hz, 2H), 2.13 - 2.07 (m, 1H). LCMS (ESI) calcd. for $C_{26}H_{27}N_4O_3S^+$ $[M+H]^+$: 475.18, found, 475.1.

**Example 144: Preparation of 3-(5-((4-(6-fluoro-1-methyl-1H-indol-3-yl)piperidin-1-yl)methyl)-1-thioxoisoindolin-2-yl)piperidine-2,6-dione (GT-09267)**

With reference to the method of Scheme 4, the target compound (GT-09267) was obtained (white solid, 15 mg, yield 25 %). $^1$H NMR (400 MHz, DMSO) δ 11.15 (s, 1H), 7.98 (d, *J* = 7.4 Hz, 2H), 7.83 (d, *J= 8.2* Hz, 1H), 7.68 (dd, *J*= 8.5, 5.5 Hz, 1H), 7.25 (dd, *J*= 10.4, 2.1 Hz, 1H), 7.10 (s, 1H), 6.85 (dd, *J* = 13.7, 4.7 Hz, 1H), 5.97 (d, *J*= 9.6 Hz, 1H), 4.89 (d, *J*= 20.4 Hz, 1H), 4.75 (d, *J*= 20.4 Hz, 1H), 4.47 (s, 2H), 3.69 (s, 3H), 3.45 (d, *J* = 10.7 Hz, 2H), 3.10 (d, *J*= 9.9 Hz, 2H), 3.04 - 2.95 (m, 2H), 2.65 (d, *J* = 16.8 Hz, 2H), 2.09 (d, *J* = 16.3 Hz, 5H). LCMS (ESI) calcd. for $C_{28}H_{30}FN_4O_2S^+$ $[M+H]^+$: 505.21, found, 505.2.

**Example 145: Preparation of 3-(5-((4-(1H-pyrrolo[2,3-b]pyridin-3-yl)piperidin-1-yl)methyl)-1-thioxoisoindolin-2-yl)piperidine-2,6-dione (GT-09268)**

With reference to the method of Scheme 4, the target compound (GT-09268) was obtained (white solid, 9 mg, yield 16 %). $^1$H NMR (400 MHz, DMSO) δ 12.10 (s, 1H), 11.15 (s, 1H), 8.53 (d, *J*= 7.9 Hz, 1H), 8.33 (d, *J* = 4.8 Hz, 1H), 8.05 - 7.96 (m, 1H), 7.96 - 7.88 (m, 2H), 7.40 (s, 1H), 7.29 - 7.23 (m, 1H), 4.82 - 4.69 (m, 1H), 4.65 (s, 1H), 4.49 (s, 2H), 3.49 (s, 2H), 3.21 (d, *J*= 7.3 Hz, 2H), 3.10 (d, *J*= 10.3 Hz, 2H), 3.00 - 2.92 (m, 1H), 2.66 (d, *J* = 15.7 Hz, 2H), 2.30 - 2.20 (m, 2H), 2.09 (s, 3H). LCMS (ESI) $C_{26}H_{28}N_5O_2S^+$ $[M+H]^+$: 474.20, found, 474.2.

**Example 146: Preparation of 3-(5-((4-(6-fluoro-1H-indazol-3-yl)piperidin-1-yl)methyl)-1-thioxoisoindolin-2-yl)piperidine-2,6-dione (GT-09269)**

With reference to the method of Scheme 4, the target compound (GT-09269) was obtained (white solid, 19 mg, yield 33 %). $^1$H NMR (400 MHz, DMSO) δ 11.13 (d, *J* = 13.6 Hz, 1H), 8.06 - 7.89 (m, 2H), 7.83 (dd, *J*= 12.2, 7.9 Hz, 1H), 7.66 - 7.43 (m, 1H), 7.25 (d, *J* = 9.6 Hz, 1H), 6.97 (s, 1H), 5.98 (s, 1H), 4.90 (d, *J*= 20.4 Hz, 1H), 4.74 (d, *J* = 36.7 Hz, 1H), 4.63 (s, 1H), 4.50 (d, *J* = 4.5 Hz, 1H), 3.50 (d, *J* = 10.6 Hz, 2H), 3.31 - 3.25 (m, 2H), 3.13 *(d, J*= 12.3 Hz, 1H), 2.97 (s, 1H), 2.65 *(d, J*= 16.8 Hz, 2H), 2.28 (d, *J* = 12.4 Hz, 2H), 2.12 (d, *J* = 12.4 Hz, 2H). LCMS (ESI) $C_{26}H_{27}FN_5O_2S^+$ $[M+H]^+$: 492.19, found, 492.1.

**Example 147: Preparation of 3-(5-((4-(6-fluoro-1H-indazol-3-yl)-3,6-dihydropyridin-1(2H)-yl)methyl)-1-thioxoisoindolin-2-yl)piperidine-2,6-dione (GT-09270)**

With reference to the method of Scheme 4, the target compound (GT-09270) was obtained (white solid, 21 mg, yield 37 %). $^1$H NMR (400 MHz, DMSO) δ 11.15 (s, 1H), 8.02 - 7.98 (m, 2H), 7.85 (d, *J* = 7.9 Hz, 1H), 7.34 (d, *J* = 9.4 Hz, 1H), 7.07 (s, 1H), 6.54 (s, 1H), 4.90 (d, *J* = 20.2 Hz, 1H), 4.78 (s, 1H), 4.71 (d, *J* = 17.1 Hz, 1H), 4.63 (s, 1H), 4.61 (s, 2H), 3.88 (s, 2H), 3.66 (s, 1H), 3.01 (s, 1H), 3.00 - 2.96 (m, 1H), 2.93 (d, *J* = 6.4 Hz, 1H), 2.65 (d, *J* = 17.2 Hz, 2H), 2.11 (d, *J* = 4.1 Hz, 2H). LCMS (ESI) $C_{26}H_{25}FN_5O_2S^+$ $[M+H]^+$: 490.17, found, 490.1.

**Example 148: Preparation of 3-(5-((4-(6-fluoro-3,4-dihydroquinolin-1(2H)-yl)piperidin-1-yl)methyl)-1-thioxoisoindolin-2-yl)piperidine-2,6-dione (GT-09271)**

With reference to the method of Scheme 4, the target compound (GT-09271) was obtained (white solid, 5 mg, yield 8 %). $^1$H NMR (400 MHz, DMSO) δ 11.14 (s, 1H), 7.98 (d, *J* = 7.9 Hz, 1H), 7.92 (s, 1H), 7.79 (s, 1H), 6.76 (d, *J* = 10.4 Hz, 2H), 6.72 (d, *J*= 4.9 Hz, 1H), 4.88 (d, *J* = 20.4 Hz, 1H), 4.75 (d, *J* = 20.2 Hz, 1H), 4.42 (d, *J*= 4.1 Hz, 2H), 3.89 (s, 1H), 3.70 (s, 1H), 3.46 (s, 2H), 3.09 (d, *J* = 14.7 Hz, 4H), 2.96 (d, *J* = 12.4 Hz, 1H), 2.64 (t, *J* = 6.1 Hz, 4H), 2.20 - 2.04 (m, 3H), 1.79 (s, 4H). LCMS (ESI) $C_{28}H_{32}FN_4O_2S^+$ $[M+H]^+$: 507.22, found, 507.2.

**Example 149: Preparation of 3-(5-((4-(7-fluoro-2,3-dihydro-4H-benzo[b][1,4]oxazin-4-yl)piperidin-1-yl)methyl)-1-thioxoisoindolin-2-yl)piperidine-2,6-dione (GT-09272)**

With reference to the method of Scheme 4, the target compound (GT-09272) was obtained (white solid, 9 mg, yield 15 %). $^1$H NMR (400 MHz, DMSO) δ 11.14 (s, 1H), 7.98 (d, *J* = 7.9 Hz, 1H), 7.92 (s, 1H), 7.78 (d, *J* = 8.0 Hz, 1H), 6.83 (s, 1H), 6.60 - 6.55 (m, 2H), 4.86 (s, 1H), 4.77 (s, 1H), 4.43 (d, *J*= 4.2 Hz, 2H), 4.17 (s, 2H), 3.91 (s, 1H), 3.45 (s, 2H), 3.14 (s, 3H), 3.08 (d, *J* = 11.3 Hz, 2H), 2.96 (d, *J* = 12.6 Hz, 1H), 2.65 (d, *J* = 16.3 Hz, 2H), 2.14 (s, 1H), 2.10 (d, *J*= 10.9 Hz, 2H). LCMS (ESI) $C_{27}H_{30}FN_4O_3S^+$ $[M+H]^+$: 509.20, found, 509.2.

**Example 150: Preparation of 3-(5-((4-(7-fluoro-3-oxo-2,3-dihydro-4H-benzo[b][1,4]oxazin-4-yl)piperidin-1-yl)methyl)-1-thioxoisoindolin-2-yl)piperidine-2,6-dione (GT-09273)**

With reference to the method of Scheme 4, the target compound (GT-09273) was obtained (white solid, 15 mg, yield 25 %). $^1$H NMR (400 MHz, DMSO) δ 11.08 (s, 1H), 7.91 (d, *J* = 7.8 Hz, 1H), 7.84 (s, 1H), 7.70 (d, *J*= 7.7 Hz, 1H), 7.49 (s, 1H), 6.92 (d, *J* = 9.2 Hz, 1H), 6.83 (d, *J* = 5.0 Hz, 1H), 4.82 (d, *J* = 20.2 Hz, 1H), 4.71 (s, 1H), 4.51 (s, 3H), 4.38 (s, 2H), 3.41 (s, 3H), 3.09 (d, *J* = 9.7 Hz, 2H), 2.89 (d, *J*= 12.9 Hz, 1H), 2.83 (d, *J* = 10.5 Hz, 2H), 2.58 (d, *J*= 16.9 Hz, 2H), 2.06 - 2.00 (m, 1H), 1.83 (d, *J* = 9.9 Hz, 2H). LCMS (ESI) $C_{27}H_{28}FN_4O_4S^+$ $[M+H]^+$: 523.18, found, 523.2.

**Example 151: Preparation of 3-(5-((4-(7-fluorochroman-4-yl)piperidin-1-yl)methyl)-1-thioxoisoindolin-2-yl)piperidine-2,6-dione (GT-09274)**

With reference to the method of Scheme 4, the target compound (GT-09274) was obtained (white solid, 3 mg, yield 6 %). $^1$H NMR (400 MHz, DMSO) δ 11.07 (s, 1H), 7.89 (s, 1H), 7.76 (s, 1H), 7.65 (s, 1H), 7.10 - 7.02 (m, 1H), 6.61 (d, *J* = 9.0 Hz, 1H), 6.53 (d, *J* = 10.4 Hz, 1H), 4.77 (s, 1H), 4.69 (s, 1H), 4.33 (s, 1H), 4.08 (dd, *J* = 24.4, 5.7 Hz, 2H), 3.20 - 3.08 (m, 3H), 2.88 (d, *J* = 17.9 Hz, 4H), 2.61 (s, 2H), 2.58-2.52 (m, 1H), 2.03 (s, 1H), 1.89 (s, 1H), 1.78 (s, 2H), 1.68-1.62 (m, 2H), 1.45-1.40 (m, 1H), 1.25 (s, 1H). LCMS (ESI) $C_{28}H_{31}FN_3O_3S^+$ $[M+H]^+$: 508.21, found, 508.2.

**Example 152: Preparation of 3-(5-((4-(5-fluoronaphthalen-1-yl)piperidin-1-yl)methyl)-1-thioxoisoindolin-2-yl)piperidine-2,6-dione (GT-09275)**

With reference to the method of Scheme 4, the target compound (GT-09275) was obtained (white solid, 5 mg, yield 9 %). $^1$H NMR (400 MHz, DMSO) δ 11.08 (s, 1H), 7.98 (d, *J* = 8.8 Hz, 1H), 7.91 (d, *J*= 8.5 Hz, 2H), 7.75 (s, 1H), 7.55 (t, *J* = 7.8 Hz, 1H), 7.49 (d, *J* = 6.1 Hz, 1H), 7.42 (d, *J* = 7.2 Hz, 1H), 7.29 (dd, *J* = 10.5, 7.9 Hz, 2H), 4.81 (s, 1H), 4.72 (s, 1H), 4.45 (s, 2H), 3.60 (s, 1H), 3.47 (s, 2H), 3.20 (s, 2H), 2.97 - 2.82 (m, 2H), 2.59 (d, *J* = 15.3 Hz, 2H), 2.10 - 1.96 (m, 5H). LCMS (ESI) $C_{29}H_{29}FN_3O_2S^+$ [M+H]$^+$: 502.20, found, 502.2.

**Example 153: Preparation of 3-(5-((4-(5-fluoronaphthalen-1-yl)-3,6-dihydropyridin-1(2H)-yl)methyl)-1-thioxoisoindolin-2-yl)piperidine-2,6-dione (GT-09276)**

With reference to the method of Scheme 4, the target compound (GT-09276) was obtained (white solid, 17 mg, yield 29 %). $^1$H NMR (400 MHz, DMSO) δ 11.08 (s, 1H), 7.97 (s, 1H), 7.93 (dd, *J* = 8.4, 3.3 Hz, 2H), 7.82 (d, *J* = 8.0 Hz, 1H), 7.55 (d, *J* = 8.3 Hz, 1H), 7.47 - 7.41 (m, 1H), 7.39 (t, *J* = 5.3 Hz, 1H), 7.29 (dd, *J* = 10.8, 7.6 Hz, 1H), 5.68 (s, 1H), 4.84 (d, *J* = 20.4 Hz, 1H), 4.70 (d, *J* = 20.4 Hz, 1H), 4.57 (s, 2H), 3.76 (s, 2H), 3.60 (s, 1H), 3.14 (d, *J* = 12.7 Hz, 2H), 2.98 - 2.85 (m, 2H), 2.61 (s, 1H), 2.52 (dd, *J* = 25.6, 7.6 Hz, 2H), 2.08 - 2.00 (m, 1H). LCMS (ESI) calcd. for $C_{29}H_{27}FN_3O_2S^+$ [M+H]$^+$: 500.18, found, 500.2.

**Example 154: Preparation of 3-(5-((4-(isoquinolin-5-yl)piperidin-1-yl)methyl)-1-thioxoisoindolin-2-yl)piperidine-2,6-dione (GT-09277)**

With reference to the method of Scheme 4, the target compound (GT-09277) was obtained (white solid, 13 mg, yield 23 %). $^1$H NMR (400 MHz, DMSO) δ 11.15 (s, 1H), 9.76 (s, 1H), 8.68 (d, *J* = 6.6 Hz, 1H), 8.56 (d, *J* = 6.6 Hz, 1H), 8.37 - 8.30 (m, 1H), 8.04 - 7.98 (m, 2H), 7.94 (dd, *J* = 5.5, 3.7 Hz, 2H), 7.87 (d, *J* = 8.2 Hz, 1H), 4.88 (s, 1H), 4.79 (s, 1H), 4.63 (s, 1H), 4.52 (s, 2H), 3.74 (s, 1H), 3.24 - 3.20 (m, 4H), 2.98 (s, 2H), 2.68 (s, 1H), 2.64 (s, 2H), 2.33 (d, *J* = 6.4 Hz, 1H), 2.04 (d, *J* = 12.7 Hz, 2H). LCMS (ESI) calcd. for $C_{28}H_{29}N_4O_2S^+$ [M+H]$^+$: 485.20, found, 485.2.

**Example 155: Preparation of 3-(5-((4-(7-fluoroquinolin-4-yl)piperidin-1-yl)methyl)-1-thioxoisoindolin-2-yl)piperidine-2,6-dione (GT-09278)**

With reference to the method of Scheme 4, the target compound (GT-09278) was obtained (white solid, 7 mg, yield 12 %). $^1$H NMR (400 MHz, DMSO) δ 11.15 (s, 1H), 9.00 (d, *J* = 4.8 Hz, 1H), 8.53 - 8.44 (m, 1H), 8.01 (s, 1H), 7.95 - 7.92 (m, 1H), 7.92 - 7.89 (m, 1H), 7.76 (s, 1H), 7.72 (s, 1H), 7.49 (s, 1H), 4.87 (dd, *J* = 31.5, 24.9 Hz, 1H), 4.70 (s, 1H), 4.64 (d, *J* = 3.8 Hz, 2H), 4.51 (s, 1H), 3.81 (s, 1H), 3.21 (d, *J* = 7.2 Hz, 4H), 2.89 (s, 2H), 2.68 - 2.58 (m, 3H), 2.28 (s, 1H), 2.08 (s, 2H). LCMS (ESI) calcd. for $C_{28}H_{28}FN_4O_2S^+$ [M+H]$^+$: 503.19, found, 503.2.

**Example 156: Preparation of 3-(5-((3-(7-fluoroquinolin-4-yl)azetidin-1-yl)methyl)-1-thioxoisoindolin-2-yl)piperidine-2,6-dione (GT-09279)**

With reference to the method of Scheme 4, the target compound (GT-09279) was obtained (white solid, 9 mg, yield 16 %). $^1$H NMR (400 MHz, DMSO) δ 11.14 (s, 1H), 9.12 (dd, *J* = 4.8, 2.2 Hz, 1H), 7.97 (d, *J* = 10.0 Hz, 2H), 7.90 (s, 1H), 7.87 (d, *J* = 5.8 Hz, 1H), 7.79 (d, *J* = 9.9 Hz, 1H), 7.73 (d, *J* = 5.1 Hz, 1H), 7.65 (s, 1H), 4.77 (d, *J* = 16.4 Hz, 2H), 4.57 (s, 2H), 4.45 (s, 3H), 3.23 (dd, *J* = 14.8, 7.2 Hz, 1H), 3.03 - 2.92 (m, 1H), 2.84 (d, *J* = 12.0 Hz, 1H), 2.69 - 2.57 (m, 2H), 2.14 - 2.07 (m, 1H), 1.33 (t, *J* = 6.3 Hz, 1H). LCMS (ESI) calcd. for $C_{26}H_{24}FN_4O_2S^+$ [M+H]$^+$: 475.16, found, 475.1.

**Example 157: Preparation of 3-(5-((4-(thieno[2,3-d]pyrimidin-4-yl)piperazin-1-yl)methyl)-1-thioxoisoindolin-2-yl)piperidine-2,6-dione (GT-09280)**

With reference to the method of Scheme 4, the target compound (GT-09280) was obtained (white solid, 10 mg, yield 17 %). $^1$H NMR (400 MHz, DMSO) δ 11.04 (s, 2H), 7.78 (d, *J* = 7.9 Hz, 2H), 7.53 (s, 2H), 7.42 (d, *J* = 8.0 Hz, 2H), 5.88 (dd, *J*= 12.8, 4.5 Hz, 2H), 4.74 (d, *J* = 20.1 Hz, 2H), 4.62 (s, 1H), 4.56 (s, 4H), 2.93 - 2.84 (m, 2H), 2.57 (d, *J* = 17.6 Hz, 2H), 2.47 (d, *J* = 4.5 Hz, 2H), 2.06 - 1.99 (m, 2H). LCMS (ESI) calcd. for $C_{25}H_{27}N_6O_2S_2^+$ [M+H]$^+$: 493.15, found, 493.1.

**Example 158: Preparation of 3-(5-((4-(5-methylthieno[2,3-d]pyrimidin-4-yl)piperazin-1-yl)methyl)-1-thioxoisoindolin-2-yl)piperidine-2,6-dione (GT-09281)**

With reference to the method of Scheme 4, the target compound (GT-09281) was obtained (white solid, 23 mg,

yield 39 %). $^1$H NMR (400 MHz, DMSO) δ 11.05 (s, 1H), 7.93 - 7.90 (m, 1H), 7.78 (d, *J* = 8.1 Hz, 1H), 7.53 (s, 1H), 7.42 (d, *J* = 9.6 Hz, 2H), 4.78 (d, *J* = 10.1 Hz, 1H), 4.71 (d, *J* = 6.1 Hz, 1H), 4.62 (s, 1H), 4.56 (s, 2H), 4.48 (s, 1H), 3.83 (s, 1H), 3.30 (d, *J* = 22.9 Hz, 4H), 2.88 (dd, *J* = 11.4, 6.1 Hz, 2H), 2.58 (d, *J* = 16.5 Hz, 2H), 2.48 (s, 2H), 2.40 (d, *J* = 1.2 Hz, 1H), 2.12 - 1.96 (m, 2H). LCMS (ESI) calcd. for $C_{25}H_{27}N_6O_2S_2^+$ [M+H]$^+$: 507.16, found, 507.2.

**Example 159: Preparation of 3-(5-((4-(6-methylthieno[2,3-d]pyrimidin-4-yl)piperazin-1-yl)methyl)-1-thioxoisoindolin-2-yl)piperidine-2,6-dione (GT-09282)**

With reference to the method of Scheme 4, the target compound (GT-09282) was obtained (white solid, 12 mg, yield 20 %). $^1$H NMR (400 MHz, DMSO) δ 11.15 (s, 1H), 8.47 (d, *J* = 1.2 Hz, 1H), 7.98 (d, *J* = 7.9 Hz, 1H), 7.94 (s, 1H), 7.80 (d, *J* = 8.7 Hz, 1H), 7.39 (d, *J* = 1.2 Hz, 1H), 4.86 (s, 1H), 4.77 (s, 1H), 4.67-4.62 (m, 2H), 4.53 (s, 2H), 3.65-3.61 (m, 3H), 3.23 (s, 2H), 3.03 - 2.91 (m, 2H), 2.65 (d, *J* = 16.7 Hz, 2H), 2.57 (s, 3H), 2.15 - 2.05 (m, 2H). LCMS (ESI) calcd. for $C_{25}H_{27}N_6O_2S_2^+$ [M+H]$^+$: 507.16, found, 507.2.

**Example 160: Preparation of 3-(5-((4-(6-isopropylthieno[2,3-d]pyrimidin-4-yl)piperazin-1-yl)methyl)-1-thioxoisoindolin-2-yl)piperidine-2,6-dione (GT-09284)**

With reference to the method of Scheme 4, the target compound (GT-09284) was obtained (white solid, 13 mg, yield 21 %). $^1$H NMR (400 MHz, DMSO) δ 11.08 (s, 1H), 8.40 (s, 1H), 7.92 (d, *J* = 7.9 Hz, 1H), 7.87 (s, 1H), 7.73 (d, *J* = 8.1 Hz, 1H), 7.28 (d, *J* = 2.9 Hz, 1H), 4.82 (d, *J* = 20.5 Hz, 1H), 4.68 (d, *J* = 20.3 Hz, 1H), 4.54 (d, *J* = 17.7 Hz, 2H), 4.46 (s, 2H), 3.56 (s, 2H), 3.21 - 3.10 (m, 4H), 2.93 - 2.86 (m, 1H), 2.58 (d, *J* = 16.6 Hz, 2H), 2.52 (s, 1H), 2.08 - 1.99 (m, 1H), 1.27 (d, *J* = 6.8 Hz, 7H). LCMS (ESI) calcd. for $C_{27}H_{31}N_6O_2S_2^+$ [M+H]$^+$: 535.19, found, 535.2.

**Example 161: Preparation of 3-(5-((4-(5,6-dimethylthieno[2,3-d]pyrimidin-4-yl)piperazin-1-yl)methyl)-1-thioxoisoindolin-2-yl)piperidine-2,6-dione (GT-09285)**

With reference to the method of Scheme 4, the target compound (GT-09285) was obtained (white solid, 18 mg, yield 30 %). $^1$H NMR (400 MHz, DMSO) δ 11.05 (s, 1H), 7.90 (s, 1H), 7.78 (d, *J* = 7.9 Hz, 1H), 7.53 (s, 1H), 7.43 (s, 1H), 4.78 (d, *J* = 9.8 Hz, 1H), 4.71 (d, *J* = 6.0 Hz, 1H), 4.62 (s, 1H), 4.56 (s, 2H), 4.48 (s, 1H), 3.78 (s, 1H), 3.33 - 3.31 (m, 2H), 3.29 - 3.16 (m, 4H), 2.88 (dd, *J* = 11.4, 6.1 Hz, 2H), 2.58 (d, *J* = 16.4 Hz, 2H), 2.47 (d, *J* = 4.1 Hz, 1H), 2.39 (s, 2H), 2.35 (s, 2H), 2.04 - 2.00 (m, 1H).LCMS (ESI) calcd. for $C_{26}H_{29}N_6O_2S_2^+$ [M+H]$^+$: 521.18, found, 521.3.

**Example 162: Preparation of 3-(5-((4-(5,6-dimethylthieno[2,3-d]pyrimidin-4-yl)-1,4-diazepan-1-yl)methyl)-1-thioxoisoindolin-2-yl)piperidine-2,6-dione (GT-09287)**

With reference to the method of Scheme 4, the target compound (GT-09287) was obtained (white solid, 19 mg, yield 31 %). $^1$H NMR (400 MHz, DMSO) δ 11.05 (s, 1H), 7.89 (d, *J* = 7.9 Hz, 1H), 7.78 (d, *J* = 7.9 Hz, 1H), 7.53 (s, 1H), 7.42 (d, *J* = 8.2 Hz, 1H), 4.77 (d, *J* = 5.6 Hz, 1H), 4.71 (s, 1H), 4.62 (s, 1H), 4.56 (s, 2H), 4.43 (s, 1H), 3.98 (d, *J* = 34.6 Hz, 1H), 3.79 - 3.64 (m, 2H), 3.54 (s, 2H), 3.36 - 3.23 (m, 3H), 3.09 *(d, J*= 5.9 Hz, 1H), 2.96 - 2.82 (m, 2H), 2.58 (d, *J* = 16.4 Hz, 2H), 2.48 (s, 1H), 2.35 (s, 2H), 2.27 (s, 2H), 2.06 - 1.98 (m, 2H).LCMS (ESI) calcd. for $C_{27}H_{31}N_6O_2S_2^+$ [M+H]$^+$: 535.19, found, 535.1.

**Example 163: Preparation of 3-(5-((4-(6-bromothieno[2,3-d]pyrimidin-4-yl)piperazin-1-yl)methyl)-1-thioxoisoindolin-2-yl)piperidine-2,6-dione (GT-09295)**

With reference to the method of Scheme 4, the target compound (GT-09295) was obtained (white solid, 19 mg, yield 29 %). $^1$H NMR (400 MHz, DMSO) δ 11.05 (s, 1H), 7.86 (s, 1H), 7.78 (d, *J* = 8.0 Hz, 1H), 7.73 (s, 1H), 7.53 (s, 1H), 7.42 (d, *J* = 8.1 Hz, 1H), 4.78 *(d, J*= 9.6 Hz, 1H), 4.71 (d, *J* = 3.6 Hz, 1H), 4.62 (s, 1H), 4.56 (s, 3H), 4.46 (s, 1H), 3.54 (s, 2H), 3.18 (s, 3H), 2.96 - 2.83 (m, 2H), 2.58 (d, *J* = 17.6 Hz, 2H), 2.05 - 2.00 (m, 1H). LCMS (ESI) calcd. for $C_{24}H_{24}BrN_6O_2S_2^+$ [M+H]$^+$: 571.06, found, 571.1.

**Example 164: Preparation of 3-(5-((4-(6-phenylthieno[2,3-d]pyrimidin-4-yl)piperazin-1-yl)methyl)-1-thioxoisoindolin-2-yl)piperidine-2,6-dione (GT-09296)**

With reference to the method of Scheme 4, the target compound (GT-09296) was obtained (white solid, 14 mg, yield 21 %). $^1$H NMR (400 MHz, DMSO) δ 11.08 (s, 1H), 8.44 (d, *J* = 2.9 Hz, 1H), 7.93 (t, *J* = 3.8 Hz, 2H), 7.88 (s, 1H), 7.79 (d, *J* = 7.3 Hz, 2H), 7.43 (d, *J* = 7.7 Hz, 2H), 7.37 (d, *J* = 7.3 Hz, 1H), 4.82 (d, *J* = 20.4 Hz, 1H), 4.71 (s, 1H), 4.67 (d, *J* = 7.8 Hz, 2H), 4.48 (s, 2H), 3.62 (s, 2H), 3.22 (s, 4H), 2.89 (d, *J* = 12.3 Hz, 2H), 2.58 (d, *J* = 16.4 Hz, 2H), 2.07 - 2.01 (m, 1H). LCMS (ESI) calcd. for $C_{30}H_{29}N_6O_2S_2^+$ [M+H]$^+$: 569.18, found, 569.2.

**Example 165: Preparation of 3-(5-((4-(5-phenylthieno[2,3-d]pyrimidin-4-yl)piperazin-1-yl)methyl)-1-thioxoisoindolin-2-yl)piperidine-2,6-dione (GT-09297)**

With reference to the method of Scheme 4, the target compound (GT-09297) was obtained (white solid, 14 mg, yield 21 %). $^1$H NMR (400 MHz, DMSO) δ 11.08 (s, 1H), 8.60 (s, 1H), 7.89 (d, *J* = 7.9 Hz, 1H), 7.71 (d, *J* = 7.0 Hz, 2H), 7.57 (d, *J* = 8.0 Hz, 1H), 7.40 - 7.36 (m, 2H), 7.36 - 7.29 (m, 3H), 4.80 (d, *J* = 20.4 Hz, 1H), 4.67 (d, *J* = 20.4 Hz, 1H), 4.24 (s, 2H), 3.68 (d, *J* = 12.8 Hz, 2H), 3.27 - 3.11 (m, 3H), 3.02 (s, 4H), 2.89 (d, *J* = 12.3 Hz, 1H), 2.58 (d, *J* = 16.8 Hz, 2H), 2.10 - 1.99 (m, 1H). LCMS (ESI) calcd. for $C_{30}H_{29}N_6O_2S_2^+$ [M+H]$^+$: 569.18, found, 569.2.

**Example 166: Preparation of 3-(5-((4-(2-methylthieno[2,3-d]pyrimidin-4-yl)piperazin-1-yl)methyl)-1-thioxoisoindolin-2-yl)piperidine-2,6-dione (GT-09298)**

With reference to the method of Scheme 4, the target compound (GT-09298) was obtained (white solid, 31 mg, yield 52 %). $^1$H NMR (400 MHz, DMSO) δ 11.15 (s, 1H), 7.97 (d, *J* = 7.5 Hz, 2H), 7.82 (d, *J* = 8.4 Hz, 1H), 7.68 (t, *J* = 4.3 Hz, 2H), 4.88 (d, *J* = 20.4 Hz, 1H), 4.74 (d, *J* = 20.3 Hz, 2H), 4.53 (s, 2H), 3.69 (s, 3H), 3.43 - 3.38 (m, 2H), 3.23 (dd, *J* = 14.5, 7.2 Hz, 3H), 2.96 (d, *J* = 12.4 Hz, 1H), 2.66 (d, *J* = 18.5 Hz, 2H), 2.56 (s, 3H), 2.15 - 2.07 (m, 1H). LCMS (ESI) calcd. for $C_{25}H_{27}N_6O_2S_2^+$ [M+H]$^+$: 507.16, found, 507.2.

**Example 167: Preparation of 3-(5-((4-(2-methyl-5,6,7,8-tetrahydrobenzo[4,5]thieno[2,3-d]pyrimidin-4-yl)piperazin-1-yl)methyl)-1-thioxoisoindolin-2-yl)piperidine-2,6-dione (GT-09299)**

With reference to the method of Scheme 4, the target compound (GT-09299) was obtained (white solid, 29 mg, yield 45 %). $^1$H NMR (400 MHz, DMSO) δ 11.15 (s, 1H), 8.01 (s, 1H), 7.97 (d, *J* = 7.9 Hz, 1H), 7.85 (d, *J* = 7.8 Hz, 1H), 4.89 (d, *J* = 20.4 Hz, 1H), 4.75 (d, *J* = 20.5 Hz, 1H), 4.55 (s, 2H), 3.85 (d, *J* = 12.8 Hz, 3H), 3.39 (s, 2H), 3.31 (s, 2H), 3.05 - 2.92 (m, 2H), 2.89 - 2.84 (m, 4H), 2.65 (d, *J* = 17.4 Hz, 2H), 2.58 (s, 1H), 2.57-2.52 (m, 3H), 2.14 - 2.08 (m, 1H), 1.87 (s, 2H), 1.75 (s, 2H). LCMS (ESI) calcd. for $C_{29}H_{33}N_6O_2S_2^+$ [M+H]$^+$: 561.21, found, 561.3.

**Example 168: Preparation of 3-(5-((4-(5,6,7,8-tetrahydrobenzo[4,5]thieno[2,3-d]pyrimidin-4-yl)piperazin-1-yl)methyl)-1-thioxoisoindolin-2-yl)piperidine-2,6-dione (GT-09300)**

With reference to the method of Scheme 4, the target compound (GT-09300) was obtained (white solid, 31 mg, yield 49 %). $^1$H NMR (400 MHz, DMSO) δ 11.15 (s, 1H), 8.56 (s, 1H), 8.02 - 7.95 (m, 2H), 7.84 (d, *J* = 8.0 Hz, 1H), 4.89 (d, *J* = 20.4 Hz, 1H), 4.75 (d, *J* = 20.4 Hz, 1H), 4.55 (s, 2H), 3.86 (d, *J* = 13.1 Hz, 2H), 3.49 (s, 2H), 3.39 (s, 2H), 3.33 (s, 3H), 2.97 (d, *J* = 3.9 Hz, 1H), 2.93 - 2.87 (m, 4H), 2.69 - 2.58 (m, 2H), 2.14 - 2.07 (m, 1H), 1.88 (d, *J* = 4.2 Hz, 2H), 1.77 (d, *J* = 3.3 Hz, 2H). LCMS (ESI) calcd. for $C_{28}H_{31}N_6O_2S_2^+$ [M+H]$^+$: 547.19, found, 547.2.

**Example 169: Preparation of 3-(5-((4-(6,7-dihydro-5H-cyclopenta[4,5]thieno[2,3-d]pyrimidin-4-yl)piperazin-1-yl)methyl)-1-thioxoisoindolin-2-yl)piperidine-2,6-dione (GT-09301)**

With reference to the method of Scheme 4, the target compound (GT-09301) was obtained (white solid, 29 mg, yield 47 %). $^1$H NMR (400 MHz, DMSO) δ 11.15 (s, 1H), 8.51 (s, 1H), 8.01 - 7.95 (m, 2H), 7.86 - 7.82 (m, 1H), 4.89 (d, *J* = 20.4 Hz, 1H), 4.74 (d, *J* = 20.4 Hz, 1H), 4.53 (s, 3H), 4.14 (d, *J* = 13.1 Hz, 3H), 3.42 (d, *J* = 11.7 Hz, 3H), 3.26 (s, 3H), 3.05 - 2.99 (m, 4H), 2.65 (d, *J* = 17.2 Hz, 2H), 2.42 - 2.37 (m, 2H), 2.13 - 2.08 (m, 1H). LCMS (ESI) calcd. for $C_{27}H_{29}N_6O_2S_2^+$ [M+H]$^+$: 533.18, found, 533.2.

**Example 170: Preparation of 3-(5-((4-(2-methyl-6,7-dihydro-5H-cyclopenta[4,5]thieno[2,3-d]pyrimidin-4-yl)piperazin-1-yl)methyl)-1-thioxoisoindolin-2-yl)piperidine-2,6-dione (GT-09302)**

With reference to the method of Scheme 4, the target compound (GT-09302) was obtained (white solid, 30 mg, yield 47 %). $^1$H NMR (400 MHz, DMSO) δ 11.14 (s, 1H), 8.00 (s, 1H), 7.97 (d, *J* = 7.9 Hz, 1H), 7.85 (d, *J* = 8.1 Hz, 1H), 4.89 (d, *J* = 20.3 Hz, 1H), 4.74 (d, *J* = 20.4 Hz, 1H), 4.53 (s, 3H), 4.17 (d, *J* = 11.9 Hz, 3H), 3.61 (s, 2H), 3.42 (s, 2H), 3.25 (s, 4H), 3.02 - 2.97 (m, 5H), 2.65 (d, *J* = 16.4 Hz, 2H), 2.40 - 2.36 (m, 2H), 2.13 - 2.08 (m, 1H). LCMS (ESI) calcd. for $C_{28}H_{31}N_6O_2S_2^+$ [M+H]$^+$: 547.19, found, 547.2.

**Example 171: Preparation of 3-(5-((4-(thieno[2,3-d]pyrimidin-2-yl)piperazin-1-yl)methyl)-1-thioxoisoindolin-2-yl)piperidine-2,6-dione (GT-09303)**

With reference to the method of Scheme 4, the target compound (GT-09303) was obtained (white solid, 28 mg,

yield 49 %). $^1$H NMR (400 MHz, DMSO) δ 11.15 (s, 1H), 8.97 (s, 1H), 7.97 (d, *J* = 8.2 Hz, 2H), 7.84 (s, 1H), 7.80 (d, *J* = 8.4 Hz, 1H), 7.45 *(d, J*= 5.9 Hz, 1H), 7.33 (d, *J* = 5.9 Hz, 1H), 4.88 (d, *J* = 20.4 Hz, 1H), 4.77 (s, 2H), 4.74 (s, 1H), 4.49 (s, 2H), 3.42 (d, *J*= 12.2 Hz, 3H), 3.13 (s, 2H), 3.03 - 2.89 (m, 2H), 2.65 (d, *J* = 17.1 Hz, 2H), 2.57 (d, *J* = 17.7 Hz, 1H), 2.13 - 2.08 (m, 1H). LCMS (ESI) calcd. for $C_{24}H_{25}N_6O_2S_2^+$ [M+H]$^+$: 493.15, found, 493.2.

**Example 172: Preparation of 3-(5-((4-(thieno[2,3-d]pyrimidin-4-yl)piperidin-1-yl)methyl)-1-thioxoisoindolin-2-yl)piperidine-2,6-dione (GT-09304)**

With reference to the method of Scheme 4, the target compound (GT-09304) was obtained (white solid, 23 mg, yield 40 %). $^1$H NMR (400 MHz, DMSO) δ 11.15 (s, 1H), 9.03 (s, 1H), 7.99 (t, *J* = 4.5 Hz, 2H), 7.96 - 7.89 (m, 1H), 7.87 - 7.83 (m, 1H), 7.82 (d, *J* = 6.0 Hz, 1H), 4.88 (s, 1H), 4.79 (s, 1H), 4.63 (s, 1H), 4.50 (d, *J* = 4.2 Hz, 2H), 3.35 (s, 1H), 3.19 - 3.10 (m, 2H), 3.04 - 2.89 (m, 2H), 2.66 (d, *J* = 16.3 Hz, 2H), 2.36 (d, *J* = 11.8 Hz, 2H), 2.16 - 1.99 (m, 4H). LCMS (ESI) calcd. for $C_{25}H_{26}N_5O_2S_2^+$ [M+H]$^+$: 492.15, found, 492.2.

**Example 173: Preparation of 3-(5-((4-(6-methylthieno[2,3-d]pyrimidin-4-yl)piperidin-1-yl)methyl)-1-thioxoisoindolin-2-yl)piperidine-2,6-dione (GT-09305)**

With reference to the method of Scheme 4, the target compound (GT-09305) was obtained (white solid, 21 mg, yield 36 %). $^1$H NMR (400 MHz, DMSO) δ 11.15 (s, 1H), 8.93 (s, 1H), 7.98 (s, 1H), 7.92 (s, 1H), 7.83 (d, *J* = 7.6 Hz, 1H), 7.51 (s, 1H), 4.90 (d, *J* = 20.4 Hz, 1H), 4.79 (s, 1H), 4.50 (d, *J* = 4.2 Hz, 2H), 3.49 - 3.45 (m, 2H), 3.34 (s, 1H), 3.11 (d, *J* = 10.9 Hz, 2H), 2.96 (d, *J* = 11.5 Hz, 2H), 2.67 (s, 1H), 2.62 (s, 4H), 2.32 (d, *J* = 11.9 Hz, 2H), 2.13 - 2.08 (m, 1H), 2.02 (d, *J* = 12.9 Hz, 2H). LCMS (ESI) calcd. for $C_{26}H_{28}N_5O_2S_2^+$ [M+H]$^+$: 506.17, found, 506.2.

**Example 174: Preparation of 3-(5-((4-(6-phenylthieno[2,3-d]pyrimidin-4-yl)piperidin-1-yl)methyl)-1-thioxoisoindolin-2-yl)piperidine-2,6-dione (GT-09306)**

With reference to the method of Scheme 4, the target compound (GT-09306) was obtained (white solid, 14 mg, yield 21 %). $^1$H NMR (400 MHz, DMSO) δ 11.15 (s, 1H), 9.00 (s, 1H), 8.30 (s, 1H), 8.00 (s, 1H), 7.90 (d, *J* = 7.2 Hz, 3H), 7.53 (d, *J* = 7.7 Hz, 2H), 7.48 (d, *J* = 7.1 Hz, 1H), 4.91 (d, *J* = 20.3 Hz, 1H), 4.77 (d, *J* = 20.4 Hz, 1H), 4.53 (d, *J* = 4.0 Hz, 2H), 3.59 (d, *J* = 10.8 Hz, 3H), 3.11 (d, *J* = 11.3 Hz, 2H), 2.97 (d, *J* = 12.3 Hz, 1H), 2.66 (d, *J* = 16.0 Hz, 2H), 2.58 (s, 1H), 2.38 (d, *J* = 12.8 Hz, 2H), 2.10 (d, *J* = 11.2 Hz, 3H). LCMS (ESI) calcd. for $C_{31}H_{30}N_5O_2S_2^+$ [M+H]$^+$: 568.18, found, 568.2.

**Example 175: Preparation of 3-(5-((4-(5-phenylthieno[2,3-d]pyrimidin-4-yl)piperidin-1-yl)methyl)-1-thioxoisoindolin-2-yl)piperidine-2,6-dione (GT-09307)**

With reference to the method of Scheme 4, the target compound (GT-09307) was obtained (white solid, 21 mg, yield 32 %). $^1$H NMR (400 MHz, DMSO) δ 11.16 (s, 1H), 9.06 (s, 1H), 7.98 (d, *J* = 7.9 Hz, 1H), 7.86 (s, 1H), 7.83 (s, 1H), 7.71 (d, *J* = 8.0 Hz, 1H), 7.51 - 7.49 (m, 1H), 7.48 (s, 2H), 7.43 (d, *J* = 7.6 Hz, 2H), 4.90 (d, *J* = 20.4 Hz, 1H), 4.76 (d, *J* = 20.3 Hz, 1H), 4.34 (d, *J* = 3.8 Hz, 2H), 3.34 (s, 2H), 3.18 (s, 1H), 2.97 (d, *J* = 12.4 Hz, 1H), 2.75 (s, 1H), 2.66 (d, *J* = 16.2 Hz, 2H), 2.16 (d, *J* = 8.8 Hz, 4H), 1.75 (d, *J*= *6.9* Hz, 3H). LCMS (ESI) calcd. for $C_{31}H_{30}N_5O_2S_2^+$ [M+H]$^+$: 568.18, found, 568.2.

**Example 176: Preparation of 3-(5-((4-(2-methyl-5-phenylthieno[2,3-d]pyrimidin-4-yl)piperidin-1-yl)methyl)-1-thioxoisoindolin-2-yl)piperidine-2,6-dione (GT-09308)**

With reference to the method of Scheme 4, the target compound (GT-09308) was obtained (white solid, 20 mg, yield 30 %). $^1$H NMR (400 MHz, DMSO) δ 11.16 (s, 1H), 7.98 (d, *J* = 7.9 Hz, 1H), 7.85 (s, 1H), 7.73 - 7.68 (m, 2H), 7.47 - 7.45 (m, 3H), 7.41 (dd, *J* = 5.8, 3.7 Hz, 2H), 4.90 (d, *J* = 20.4 Hz, 1H), 4.76 (d, *J* = 20.6 Hz, 1H), 4.34 (d, *J* = 3.8 Hz, 2H), 3.32 (s, 2H), 3.20 (d, *J* = 35.5 Hz, 1H), 3.04 - 2.93 (m, 1H), 2.73 (s, 1H), 2.71 (d, *J* = 6.1 Hz, 4H), 2.63 (t, *J* = 5.9 Hz, 2H), 2.15 (s, 4H), 1.75 (s, 2H). LCMS (ESI) calcd. for $C_{32}H_{32}N_5O_2S_2^+$ [M+H]$^+$: 582.20, found, 582.2.

**Example 177: Preparation of 3-(5-((4-(2-methylthieno[2,3-d]pyrimidin-4-yl)piperidin-1-yl)methyl)-1-thioxoisoindolin-2-yl)piperidine-2,6-dione (GT-09309)**

With reference to the method of Scheme 4, the target compound (GT-09309) was obtained (white solid, 18 mg, yield 30 %). $^1$H NMR (400 MHz, DMSO) δ 11.13 (d, *J* = 13.9 Hz, 1H), 7.99 (d, *J* = 7.9 Hz, 1H), 7.96 - 7.90 (m, 1H), 7.86 (d, *J* = 4.8 Hz, 1H), 7.77 (dd, *J* = 6.1, 2.2 Hz, 1H), 7.52 (d, *J* = 18.5 Hz, 1H), 4.63 (s, 1H), 4.50 (d, *J* = 4.8 Hz, 1H), 3.54 (s, 3H), 3.37 (s, 1H), 3.13 (d, *J* = 10.9 Hz, 2H), 3.04 - 2.92 (m, 1H), 2.86 (s, 1H), 2.74 - 2.65 (m, 4H), 2.62 (d, *J* = 13.7 Hz, 2H), 2.37 - 2.27 (m, 2H), 2.07 (dd, *J* = 27.1, 10.1 Hz, 3H). LCMS (ESI) calcd. for $C_{26}H_{28}N_5O_2S_2^+$ [M+H]$^+$: 506.17, found, 506.2.

**Example 178: Preparation of 3-(5-((4-(2-methyl-5,6,7,8-tetrahydrobenzo[4,5]thieno[2,3-d]pyrimidin-4-yl)piperidin-1-yl)methyl)-1-thioxoisoindolin-2-yl)piperidine-2,6-dione (GT-09310)**

With reference to the method of Scheme 4, the target compound (GT-09310) was obtained (white solid, 12 mg, yield 19 %). $^1$H NMR (400 MHz, DMSO) δ 11.15 (s, 1H), 7.94 (s, 1H), 7.89 (d, *J* = 9.1 Hz, 1H), 7.81 (d, *J* = 8.0 Hz, 1H), 4.48 (s, 2H), 3.22 (d, *J* = 11.2 Hz, 2H), 2.99 (s, 3H), 2.85 (s, 3H), 2.63 (s, 5H), 2.28 (d, *J* = 11.8 Hz, 2H), 2.11 (d, *J* = 5.7 Hz, 1H), 1.99 (d, *J* = 13.7 Hz, 3H), 1.86 (s, 6H). LCMS (ESI) calcd. for $C_{30}H_{34}N_5O_2S_2^+$ [M+H]$^+$: 560.21, found, 560.2.

**Example 179: Preparation of 3-(5-((4-(6,7-dihydro-5H-cyclopenta[4,5]thieno[2,3-d]pyrimidin-4-yl)piperidin-1-yl)methyl)-1-thioxoisoindolin-2-yl)piperidine-2,6-dione (GT-09311)**

With reference to the method of Scheme 4, the target compound (GT-09311) was obtained (white solid, 16 mg, yield 26 %). $^1$H NMR (400 MHz, DMSO) δ 11.15 (s, 1H), 8.91 (s, 1H), 7.97 (s, 1H), 7.82 (d, *J* = 8.8 Hz, 1H), 4.90 (d, *J* = 20.4 Hz, 1H), 4.78 (s, 1H), 4.48 (d, *J* = 4.7 Hz, 2H), 3.48 - 3.39 (m, 3H), 3.36 (s, 1H), 3.21 (d, *J* = 11.8 Hz, 2H), 3.13 (s, 2H), 3.06 (t, *J* = 7.2 Hz, 2H), 2.98 (s, 1H), 2.70 - 2.58 (m, 2H), 2.47 (s, 1H), 2.32 (d, *J* = 13.0 Hz, 3H), 2.14 - 2.08 (m, 1H), 2.00 (d, *J* = 13.2 Hz, 2H). LCMS (ESI) calcd. for $C_{28}H_{30}N_5O_2S_2^+$ [M+H]$^+$: 532.18, found, 532.2.

**Example 180: Preparation of 3-(5-((4-(2-methyl-6,7-dihydro-5H-cyclopenta[4,5]thieno[2,3-d]pyrimidin-4-yl)piperidin-1-yl)methyl)-1-thioxoisoindolin-2-yl)piperidine-2,6-dione (GT-09312)**

With reference to the method of Scheme 4, the target compound (GT-09312) was obtained (white solid, 18 mg, yield 28 %). $^1$H NMR (400 MHz, DMSO) δ 11.15 (s, 1H), 7.97 (d, *J* = 1.9 Hz, 1H), 7.90 (d, *J* = 3.7 Hz, 1H), 7.82 (d, *J* = 8.3 Hz, 1H), 4.89 (d, *J* = 20.4 Hz, 1H), 4.75 (d, *J* = 20.4 Hz, 1H), 4.48 (d, *J* = 4.7 Hz, 3H), 3.47 (d, *J* = 11.1 Hz, 4H), 3.27 - 3.18 (m, 3H), 3.10 (s, 3H), 3.02 (s, 3H), 2.66 (s, 4H), 2.28 (d, *J* = 12.2 Hz, 2H), 2.13 - 2.09 (m, 1H), 1.98 (d, *J* = 13.5 Hz, 3H). LCMS (ESI) calcd. for $C_{29}H_{32}N_5O_2S_2^+$ [M+H]$^+$: 546.20, found, 546.3.

**Example 181: Preparation of 3-(5-((4-(thieno[2,3-d]pyrimidin-2-yl)piperidin-1-yl)methyl)-1-thioxoisoindolin-2-yl)piperidine-2,6-dione (GT-09313)**

With reference to the method of Scheme 4, the target compound (GT-09313) was obtained (white solid, 15 mg, yield 26 %). $^1$H NMR (400 MHz, DMSO) δ 11.13 (d, *J* = 12.7 Hz, 1H), 9.29 (d, *J* = 2.4 Hz, 1H), 8.01 - 7.96 (m, 1H), 7.92 (d, *J* = 5.9 Hz, 1H), 7.84 (t, *J* = 7.1 Hz, 1H), 7.58 - 7.53 (m, 1H), 7.50 (t, *J* = 8.7 Hz, 1H), 4.85 (dd, *J* = 33.8, 20.3 Hz, 1H), 4.63 (s, 1H), 4.48 (d, *J* = 4.9 Hz, 1H), 3.49 (d, *J* = 9.8 Hz, 2H), 3.26 - 3.09 (m, 3H), 3.03 - 2.83 (m, 2H), 2.65 (d, *J* = 14.5 Hz, 2H), 2.57 (d, *J* = 19.0 Hz, 1H), 2.33 (s, 1H), 2.24 (s, 2H), 2.10 (dd, *J* = 10.9, 5.5 Hz, 2H). LCMS (ESI) calcd. for $C_{25}H_{26}N_5O_2S_2^+$ [M+H]$^+$: 492.15, found, 492.1.

**Example 182: Preparation of 3-(5-((4-(thieno[2,3-d]pyrimidin-4-yl)-3,6-dihydropyridin-1(2H)-yl)methyl)-1-thioxoisoindolin-2-yl)piperidine-2,6-dione (GT-09314)**

With reference to the method of Scheme 4, the target compound (GT-09314) was obtained (white solid, 28 mg, yield 49 %). $^1$H NMR (400 MHz, DMSO) δ 11.15 (s, 1H), 9.08 (s, 1H), 8.04 (t, *J* = 6.6 Hz, 1H), 8.00 (d, *J* = 8.0 Hz, 1H), 7.93 - 7.88 (m, 1H), 7.85 (d, *J* = 8.1 Hz, 1H), 7.78 (d, *J* = 6.0 Hz, 1H), 4.90 (d, *J* = 20.3 Hz, 1H), 4.76 (d, *J* = 20.6 Hz, 1H), 4.63 (s, 2H), 3.94 (s, 2H), 3.64 - 3.56 (m, 1H), 3.31 (s, 1H), 3.04 (t, *J* = 12.3 Hz, 2H), 2.99 - 2.80 (m, 2H), 2.71 - 2.55 (m, 2H), 2.19 - 1.97 (m, 2H). LCMS (ESI) calcd. for $C_{25}H_{24}N_5O_2S_2^+$ [M+H]$^+$: 490.14, found, 490.0.

**Example 183: Preparation of 3-(5-((4-(5-methylthieno[2,3-d]pyrimidin-4-yl)-3,6-dihydropyridin-1(2H)-yl)methyl)-1-thioxoisoindolin-2-yl)piperidine-2,6-dione (GT-09315)**

With reference to the method of Scheme 4, the target compound (GT-09315) was obtained (white solid, 5 mg, yield 9 %). $^1$H NMR (400 MHz, DMSO) δ 11.15 (s, 1H), 9.03 (s, 1H), 8.00 (d, *J* = 7.5 Hz, 1H), 7.94 (d, *J* = 7.5 Hz, 1H), 7.89 (d, *J* = 6.3 Hz, 1H), 7.65 (s, 1H), 4.90 (d, *J* = 20.3 Hz, 1H), 4.76 (d, *J* = 20.8 Hz, 1H), 4.63 (s, 3H), 3.87 (d, *J*= 31.9 Hz, 4H), 3.07 - 3.04 (m, 1H), 2.93 (s, 1H), 2.78 (d, *J* = 7.1 Hz, 1H), 2.65 (s, 2H), 2.43 (s, 2H), 2.42-2.36 (m, 2H), 2.11 (s, 1H). LCMS (ESI) calcd. for $C_{26}H_{26}N_5O_2S_2^+$ [M+H]$^+$: 504.15, found, 504.2.

**Example 184: Preparation of 3-(5-((4-(6-methylthieno[2,3-d]pyrimidin-4-yl)-3,6-dihydropyridin-1(2H)-yl)methyl)-1-thioxoisoindolin-2-yl)piperidine-2,6-dione (GT-09316)**

With reference to the method of Scheme 4, the target compound (GT-09316) was obtained (white solid, 3 mg,

yield 5 %). $^1H$ NMR (400 MHz, DMSO) δ 11.15 (s, 1H), 8.98 (s, 1H), 8.04 - 7.92 (m, 2H), 7.85 (d, *J* = 8.0 Hz, 1H), 7.48 (d, *J* = 5.0 Hz, 1H), 4.90 (d, *J* = 20.2 Hz, 1H), 4.76 (d, *J* = 20.5 Hz, 1H), 4.64 (s, 1H), 3.93 (s, 2H), 3.71 (s, 1H), 3.24 - 3.13 (m, 2H), 3.03 (s, 2H), 2.97 (d, *J* = 12.8 Hz, 1H), 2.87 (t, *J*= 6.8 Hz, 1H), 2.67 (s, 2H), 2.63 (s, 3H), 2.57 (s, 1H), 2.15 - 2.07 (m, 1H). LCMS (ESI) calcd. for $C_{26}H_{26}N_5O_2S_2^+$ [M+H]$^+$: 504.15, found, 504.2.

**Example 185: Preparation of 3-(5-((4-(6-isopropylthieno[2,3-d]pyrimidin-4-yl)-3,6-dihydropyridin-1(2H)-yl) methyl)-1-thioxoisoindolin-2-yl)piperidine-2,6-dione (GT-09317)**

With reference to the method of Scheme 4, the target compound (GT-09317) was obtained (white solid, 4 mg, yield 7 %). $^1H$ NMR (400 MHz, DMSO) δ 11.15 (s, 1H), 8.92 (d, *J* = 6.2 Hz, 1H), 8.13 (d, *J* = 6.1 Hz, 1H), 7.99 (d, *J* = 8.8 Hz, 1H), 7.86 (s, 1H), 7.47 (s, 1H), 4.90 (d, *J* = 20.1 Hz, 1H), 4.76 (d, *J* = 20.1 Hz, 1H), 4.62 (d, *J* = 9.1 Hz, 1H), 3.94 (s, 2H), 3.72 (s, 2H), 3.27 - 3.23 (m, 1H), 3.03 (s, 2H), 2.95 (s, 1H), 2.87 (s, 1H), 2.66 (*d, J* = 16.4 Hz, 2H), 2.11 (d, *J* = 4.8 Hz, 1H), 1.41 - 1.36 (m, 6H), 1.35 (s, 2H). LCMS (ESI) calcd. for $C_{28}H_{30}N_5O_2S_2^+$ [M+H]$^+$: 532.18, found, 532.2.

**Example 186: Preparation of 3-(5-((4-(5,6-dimethylthieno[2,3-d]pyrimidin-4-yl)-3,6-dihydropyridin-1(2H)-yl) methyl)-1-thioxoisoindolin-2-yl)piperidine-2,6-dione (GT-09318)**

With reference to the method of Scheme 4, the target compound (GT-09318) was obtained (white solid, 5 mg, yield 8 %). $^1H$ NMR (400 MHz, DMSO) δ 8.99 (s, 1H), 8.94 (s, 1H), 7.95 (s, 1H), 7.90 (s, 1H), 7.72 (s, 1H), 5.85 (s, 1H), 5.78 (s, 1H), 4.63 (s, 2H), 3.86 (d, *J* = 32.8 Hz, 3H), 3.39 - 3.35 (m, 2H), 3.02 (s, 2H), 2.90 (d, *J* = 17.5 Hz, 2H), 2.76 (t, *J* = 6.6 Hz, 2H), 2.66 (d, *J* = 11.2 Hz, 2H), 2.29 (s, 3H), 2.25 (s, 2H). LCMS (ESI) calcd. for $C_{27}H_{28}N_5O_2S_2^+$ [M+H]$^+$: 518.17, found, 518.2.

**Example 187: Preparation of 3-(5-((4-(6-phenylthieno[2,3-d]pyrimidin-4-yl)-3,6-dihydropyridin-1(2H)-yl) methyl)-1-thioxoisoindolin-2-yl)piperidine-2,6-dione (GT-09319)**

With reference to the method of Scheme 4, the target compound (GT-09319) was obtained (white solid, 4 mg, yield 6 %). $^1H$ NMR (400 MHz, DMSO) δ 11.16 (s, 1H), 9.06 (s, 1H), 8.12 (s, 1H), 7.96 (s, 1H), 7.90 (d, *J* = 7.1 Hz, 2H), 7.85 (s, 1H), 7.55 (d, *J* = 6.9 Hz, 2H), 7.53 - 7.47 (m, 2H), 4.91 (d, *J* = 20.7 Hz, 1H), 4.77 (d, *J* = 20.8 Hz, 1H), 4.66 (d, *J* = 21.5 Hz, 2H), 3.99 (s, 2H), 3.76 (s, 1H), 3.28 - 3.19 (m, 3H), 3.03 (d, *J* = 37.0 Hz, 3H), 2.67 (s, 2H), 2.10 (s, 1H). LCMS (ESI) calcd. for $C_{31}H_{28}N_5O_2S_2^+$ [M+H]$^+$: 566.17, found, 566.2.

**Example 188: Preparation of 3-(5-((4-(5-phenylthieno[2,3-d]pyrimidin-4-yl)-3,6-dihydropyridin-1(2H)-yl) methyl)-1-thioxoisoindolin-2-yl)piperidine-2,6-dione (GT-09320)**

With reference to the method of Scheme 4, the target compound (GT-09320) was obtained (white solid, 3 mg, yield 5 %). $^1H$ NMR (400 MHz, DMSO) δ 11.16 (s, 1H), 9.12 (s, 1H), 8.02 (d, *J* = 8.0 Hz, 1H), 7.96 (s, 1H), 7.85 (s, 1H), 7.72 (d, *J* = 7.8 Hz, 1H), 7.40 (d, *J* = 6.8 Hz, 1H), 7.33 (s, 3H), 7.08 (s, 1H), 5.27 (s, 1H), 4.83 (t, *J* = 26.4 Hz, 2H), 4.41 (s, 2H), 3.64 (s, 1H), 3.00 (dd, *J* = 15.0, 10.4 Hz, 3H), 2.73 - 2.60 (m, 6H), 2.17 - 2.10 (m, 1H). LCMS (ESI) calcd. for $C_{31}H_{28}N_5O_2S_2^+$ [M+H]$^+$: 566.17, found, 566.2.

**Example 189: Preparation of 3-(5-((4-(2-methyl-5-phenylthieno[2,3-d]pyrimidin-4-yl)-3,6-dihydropyridin-1(2H)-yl)methyl)-1-thioxoisoindolin-2-yl)piperidine-2,6-dione (GT-09321)**

With reference to the method of Scheme 4, the target compound (GT-09321) was obtained (white solid, 3 mg, yield 4 %). $^1H$ NMR (400 MHz, DMSO) δ 11.16 (s, 1H), 8.02 (d, *J* = 7.9 Hz, 1H), 7.83 (d, *J* = 13.0 Hz, 2H), 7.72 (d, *J* = 8.0 Hz, 1H), 7.31 (s, 4H), 7.07 (s, 1H), 5.27 (s, 1H), 4.86 (dd, *J* = 52.9, 20.9 Hz, 2H), 4.43 (d, *J* = 18.3 Hz, 2H), 3.78 (s, 3H), 2.98 (d, *J* = 11.7 Hz, 3H), 2.74 (s, 4H), 2.66 (d, *J* = 10.6 Hz, 4H), 2.18 - 2.10 (m, 1H). LCMS (ESI) calcd. for $C_{32}H_{30}N_5O_2S_2^+$ [M+H]$^+$: 580.18, found, 580.2.

**Example 190: Preparation of 3-(5-((4-(2-methylthieno[2,3-d]pyrimidin-4-yl)-3,6-dihydropyridin-1(2H)-yl) methyl)-1-thioxoisoindolin-2-yl)piperidine-2,6-dione (GT-09322)**

With reference to the method of Scheme 4, the target compound (GT-09322) was obtained (white solid, 4 mg, yield 7 %). $^1H$ NMR (400 MHz, DMSO) δ 11.15 (s, 1H), 8.00 (d, *J* = 7.6 Hz, 1H), 7.94 (d, *J* = 6.1 Hz, 1H), 7.91 (d, *J* = 6.1 Hz, 1H), 7.86 (d, *J* = 8.1 Hz, 1H), 7.69 (d, *J* = 6.1 Hz, 1H), 4.90 (d, *J* = 20.6 Hz, 1H), 4.76 (d, *J* = 20.3 Hz, 1H), 4.63 (s, 2H), 3.94 (s, 2H), 3.03 (s, 2H), 2.90 - 2.85 (m, 2H), 2.75 (s, 2H), 2.72 (s, 3H), 2.66 (d, *J* = 15.4 Hz, 3H), 2.11 (s, 1H). LCMS (ESI) calcd. for $C_{26}H_{26}N_5O_2S_2^+$ [M+H]$^+$: 504.15, found, 504.2.

**Example 191: Preparation of 3-(5-((4-(2-methyl-5,6,7,8-tetrahydrobenzo[4,5]thieno[2,3-d]pyrimidin-4-yl)-3,6-dihydropyridin-1(2H)-yl)methyl)-1-thioxoisoindolin-2-yl)piperidine-2,6-dione (GT-09323)**

With reference to the method of Scheme 4, the target compound (GT-09323) was obtained (white solid, 3 mg, yield 5 %). $^1$H NMR (400 MHz, DMSO) δ 11.15 (s, 1H), 8.01 (d, *J = 7.9* Hz, 1H), 7.96 (s, 1H), 7.83 (d, *J* = 7.7 Hz, 1H), 4.90 (d, *J* = 20.2 Hz, 1H), 4.77 (d, *J* = 20.6 Hz, 1H), 4.62 (d, *J* = 13.3 Hz, 2H), 3.87 (d, *J* = 26.6 Hz, 2H), 3.67 (s, 2H), 2.98 (s, 2H), 2.91-2.84 (m, 3H), 2.74 (s, 1H), 2.72-2.65 (m, 1H), 2.66 (s, 3H), 2.60 (s, 2H), 2.14-2.08 (m, 1H), 1.89-1.82 (m, 3H), 1.77 (s, 3H). LCMS (ESI) calcd. for $C_{30}H_{32}N_5O_2S_2^+$ [M+H]$^+$: 558.20, found, 558.2.

**Example 192: Preparation of 3-(5-((4-(5,6,7,8-tetrahydrobenzo[4,5]thieno[2,3-d]pyrimidin-4-yl)-3,6-dihydropyridin-1(2H)-yl)methyl)-1-thioxoisoindolin-2-yl)piperidine-2,6-dione (GT-09324)**

With reference to the method of Scheme 4, the target compound (GT-09324) was obtained (white solid, 7 mg, yield 11 %). $^1$H NMR (400 MHz, DMSO) δ 11.15 (s, 1H), 8.94 (s, 1H), 8.01 (s, 1H), 7.94 (d, *J* = 7.6 Hz, 1H), 7.89 (d, *J* = 7.4 Hz, 1H), 4.90 (d, *J* = 20.4 Hz, 1H), 4.77 (d, *J* = 20.5 Hz, 1H), 4.63 (s, 2H), 3.87 (d, *J* = 23.2 Hz, 2H), 3.67 (s, 2H), 3.02 (s, 1H), 2.91 (s, 4H), 2.80 (d, *J* = 19.7 Hz, 2H), 2.66 (d, *J* = 16.6 Hz, 2H), 2.11 (d, *J* = 5.0 Hz, 1H), 1.85 (s, 3H), 1.77 (s, 3H). LCMS (ESI) calcd. for $C_{29}H_{30}N_5O_2S_2^+$ [M+H]$^+$: 544.18, found, 544.2.

**Example 193: Preparation of 3-(5-((4-(6,7-dihydro-5H-cyclopenta[4,5]thieno[2,3-d]pyrimidin-4-yl)-3,6-dihydropyridin-1(2H)-yl)methyl)-1-thioxoisoindolin-2-yl)piperidine-2,6-dione (GT-09325)**

With reference to the method of Scheme 4, the target compound (GT-09325) was obtained (white solid, 5 mg, yield 8 %). $^1$H NMR (400 MHz, DMSO) δ 11.15 (s, 1H), 8.95 (s, 1H), 8.00 (d, *J* = 7.8 Hz, 2H), 7.87 (d, *J* = 7.9 Hz, 1H), 4.90 (d, *J* = 20.3 Hz, 1H), 4.77 (d, *J* = 20.5 Hz, 1H), 4.62 (d, *J* = 11.9 Hz, 2H), 3.91 (d, *J* = 19.7 Hz, 2H), 3.70 (s, 1H), 3.36 - 3.31 (m, 3H), 3.08 - 3.03 (m, 3H), 2.98 (s, 2H), 2.93 - 2.82 (m, 2H), 2.66 (d, *J* = 15.5 Hz, 2H), 2.40 (s, 2H), 2.15 - 2.09 (m, 1H). LCMS (ESI) calcd. for $C_{28}H_{28}N_5O_2S_2^+$ [M+H]$^+$: 530.17, found, 530.2.

**Example 194: Preparation of 3-(5-((4-(2-methyl-6,7-dihydro-5H-cyclopenta[4,5]thieno[2,3-d]pyrimidin-4-yl)-3,6-dihydropyridin-1(2H)-yl)methyl)-1-thioxoisoindolin-2-yl)piperidine-2,6-dione (GT-09326)**

With reference to the method of Scheme 4, the target compound (GT-09326) was obtained (white solid, 6 mg, yield 10 %). $^1$H NMR (400 MHz, DMSO) δ 11.15 (s, 1H), 8.01 (s, 1H), 7.97 (d, *J* = 15.4 Hz, 1H), 7.91 - 7.87 (m, 1H), 4.90 (d, *J* = 20.4 Hz, 1H), 4.76 (d, *J* = 20.7 Hz, 1H), 4.61 (d, *J* = 14.6 Hz, 2H), 3.88 (d, *J* = 24.1 Hz, 2H), 3.68 (s, 2H), 3.01 (d, *J* = 7.2 Hz, 4H), 2.98 (s, 3H), 2.85 (d, *J* = 9.1 Hz, 2H), 2.68 (s, 4H), 2.64 (s, 1H), 2.38 (s, 2H), 2.15 - 2.09 (m, 1H). LCMS (ESI) calcd. for $C_{29}H_{30}N_5O_2S_2^+$ [M+H]$^+$: 544.18, found, 544.2.

**Example 195: Preparation of 3-(5-((4-(thieno[2,3-d]pyrimidin-2-yl)-3,6-dihydropyridin-1(2H)-yl)methyl)-1-thioxoisoindolin-2-yl)piperidine-2,6-dione (GT-09327)**

With reference to the method of Scheme 4, the target compound (GT-09327) was obtained (white solid, 3 mg, yield 5 %). $^1$H NMR (400 MHz, DMSO) δ 11.15 (s, 1H), 9.34 (s, 1H), 8.01 (d, *J* = 8.0 Hz, 1H), 7.98 (s, 1H), 7.84 (d, *J* = 8.1 Hz, 1H), 7.60 (d, *J*= 6.0 Hz, 1H), 7.21 (s, 1H), 4.90 (d, *J* = 20.7 Hz, 1H), 4.77 (d, *J* = 20.1 Hz, 1H), 4.64 (s, 2H), 3.95 (s, 2H), 3.70 (s, 1H), 3.08 (s, 2H), 2.99 (s, 1H), 2.94 (t, *J* = 6.9 Hz, 2H), 2.66 (d, *J* = 16.0 Hz, 2H), 2.57 (s, 1H), 2.15 - 2.07 (m, 1H). LCMS (ESI) calcd. for $C_{25}H_{24}N_5O_2S_2^+$ [M+H]$^+$: 490.14, found, 490.1.

**Example 196: Preparation of 3-(5-((4-(2-chloro-6,7-dihydrothieno[3,2-d]pyrimidin-4-yl)piperazin-1-yl)methyl)-1-thioxoisoindolin-2-yl)piperidine-2,6-dione (GT-09328)**

With reference to the method of Scheme 4, the target compound (GT-09328) was obtained (white solid, 2 mg, yield 4 %). $^1$H NMR (400 MHz, DMSO) δ 11.15 (s, 1H), 7.97 (d, *J* = 8.0 Hz, 1H), 7.86 (s, 1H), 7.74 (s, 1H), 4.88 (d, *J* = 20.6 Hz, 1H), 4.74 (d, *J= 20.4* Hz, 1H), 4.44 (s, 3H), 3.52 (s, 3H), 3.29 - 3.26 (m, 2H), 3.24 - 3.19 (m, 4H), 3.16 - 3.06 (m, 2H), 3.04 - 2.90 (m, 2H), 2.66 (d, *J* = 17.8 Hz, 2H), 2.14 - 2.06 (m, 1H). LCMS (ESI) calcd. for $C_{24}H_{26}ClN_6O_2S_2^+$ [M+H]$^+$: 529.12, found, 529.1.

**Example 197: Preparation of 3-(5-((4-(2-morpholino-6,7-dihydrothieno[3,2-d]pyrimidin-4-yl)piperazin-1-yl)methyl)-1-thioxoisoindolin-2-yl)piperidine-2,6-dione (GT-09329)**

With reference to the method of Scheme 4, the target compound (GT-09329) was obtained (white solid, 4 mg,

yield 6 %). $^1H$ NMR (400 MHz, DMSO) δ 11.15 (s, 1H), 7.97 (d, *J* = 7.9 Hz, 1H), 7.93 (s, 1H), 7.79 (d, *J* = 7.8 Hz, 1H), 4.88 (d, *J= 20.3* Hz, 1H), 4.74 (d, *J* = 20.4 Hz, 1H), 4.49 (s, 2H), 4.46 - 4.40 (m, 1H), 3.74 (s, 2H), 3.68 (s, 3H), 3.64 (s, 8H), 3.29 (d, *J* = 7.9 Hz, 3H), 3.16 (d, *J* = 8.0 Hz, 3H), 2.99 (dd, *J* = 21.9, 9.0 Hz, 2H), 2.66 (d, *J* = 17.0 Hz, 2H), 2.14 - 2.07 (m, 1H). LCMS (ESI) calcd. for $C_{28}H_{34}N_7O_3S_2^+$ [M+H]$^+$: 580.22, found, 580.3.

**Example 198: Preparation of 3-(5-((4-(4-morpholino-6,7-dihydrothieno[3,2-d]pyrimidin-2-yl)piperazin-1-yl)methyl)-1-thioxoisoindolin-2-yl)piperidine-2,6-dione (GT-09330)**

With reference to the method of Scheme 4, the target compound (GT-09330) was obtained (white solid, 3 mg, yield 5 %). $^1H$ NMR (400 MHz, DMSO) δ 11.15 (s, 1H), 7.98 (d, *J* = 8.0 Hz, 1H), 7.90 (s, 1H), 7.77 (d, *J* = 7.9 Hz, 1H), 4.88 (d, *J* = 20.4 Hz, 1H), 4.74 (d, *J* = 20.1 Hz, 1H), 4.56 (s, 1H), 4.48 (s, 2H), 3.64 (d, *J* = 4.6 Hz, 12H), 3.25 (d, *J* = 7.9 Hz, 3H), 3.12 - 3.07 (m, 3H), 2.96 (d, *J* = 12.0 Hz, 2H), 2.66 (d, *J* = 17.*4* Hz, 3H), 2.10 (s, 1H). LCMS (ESI) calcd. for $C_{28}H_{34}N_7O_3S_2^+$ [M+H]$^+$: 580.22, found, 580.3.

**Example 199: Preparation of 3-(5-((4-(2-chlorothieno[3,2-d]pyrimidin-4-yl)piperazin-1-yl)methyl)-1-thioxoisoindolin-2-yl)piperidine-2,6-dione (GT-09331)**

With reference to the method of Scheme 4, the target compound (GT-09331) was obtained (white solid, 5 mg, yield 8 %). $^1H$ NMR (400 MHz, DMSO) δ 11.15 (s, 1H), 8.38 (d, *J* = 5.5 Hz, 1H), 7.98 (d, *J* = 7.9 Hz, 1H), 7.92 (s, 1H), 7.78 (d, *J* = 8.1 Hz, 1H), 7.47 (d, *J* = 5.5 Hz, 1H), 4.88 *(d, J=* 20.4 Hz, 1H), 4.75 (d, *J* = 20.5 Hz, 3H), 4.50 (s, 2H), 3.74 (s, 2H), 3.28 - 3.18 (m, 3H), 2.96 (d, *J* = 12.3 Hz, 1H), 2.66 (d, *J* = 16.6 Hz, 2H), 2.54 (s, 2H), 2.14 - 2.07 (m, 1H). LCMS (ESI) calcd. for $C_{24}H_{24}ClN_6O_2S_2^+$ [M+H]$^+$: 527.11, found, 527.1.

**Example 200: Preparation of 3-(5-((4-(2-morpholinothieno[3,2-d]pyrimidin-4-yl)piperazin-1-yl)methyl)-1-thioxoisoindolin-2-yl)piperidine-2,6-dione (GT-09333)**

With reference to the method of Scheme 4, the target compound (GT-09331) was obtained (white solid, 6 mg, yield 9 %). $^1H$ NMR (400 MHz, DMSO) δ 11.14 (s, 1H), 8.32 (s, 1H), 7.97 (d, *J* = 8.0 Hz, 1H), 7.93 (s, 1H), 7.79 (d, *J* = 8.2 Hz, 1H), 7.49 (s, 1H), 4.88 (d, *J* = 20.5 Hz, 1H), 4.74 (d, *J* = 20.2 Hz, 2H), 4.51 (s, 2H), 3.80 (s, 6H), 3.71 (d, *J* = 4.5 Hz, 6H), 3.51 (s, 2H), 3.22 (s, 2H), 2.99-2.93 (m, 1H), 2.70 - 2.57 (m, 2H), 2.14 - 2.06 (m, 1H). LCMS (ESI) calcd. for $C_{28}H_{32}N_7O_3S_2^+$ [M+H]$^+$: 578.20, found, 578.2.

**Example 201: Preparation of 3-(5-((4-(4-(piperazin-1-yl)thieno[3,2-d]pyrimidin-2-yl)piperazin-1-yl)methyl)-1-thioxoisoindolin-2-yl)piperidine-2,6-dione (GT-09365)**

With reference to the method of Scheme 4, the target compound (GT-09365) was obtained (white solid, 25 mg, yield 93 %). $^1H$ NMR (400 MHz, DMSO) δ 11.15 (s, 1H), 10.09 (s, 2H), 8.33 (d, *J* = 5.1 Hz, 1H), 8.01 (s, 1H), 7.95 (d, *J* = 7.9 Hz, 1H), 7.86 (d, *J* = 8.0 Hz, 1H), 7.55 (s, 1H), 4.89 (d, *J* = 20.5 Hz, 1H), 4.84 (s, 1H), 4.74 (d, *J* = 20.7 Hz, 1H), 4.56-4.51 (m, 2H), 4.21 (s, 4H), 3.65 (s, 3H), 3.29-3.21 (m, 5H), 3.18 (s, 2H), 2.95 (d, *J* = 12.6 Hz, 1H), 2.65 (d, *J* = 19.2 Hz, 2H), 2.38 (s, 2H), 2.13 - 2.07 (m, 1H). LCMS (ESI) calcd. for $C_{28}H_{33}N_8O_2S_2^+$ [M+H]$^+$: 577.22, found, 577.2.

**Example 202: Preparation of 3-(5-((4-(phenyl(pyridin-3-yl)methyl)piperazin-1-yl)methyl)-1-thioxoisoindolin-2-yl)piperidine-2,6-dione (GT-07732)**

With reference to the method of Scheme 4, the target compound GT-07732 was obtained (32 mg, white solid, yield 41%). $^1H$ NMR (400 MHz, DMSO) δ 11.35 (s, 1H), 11.14 (s, 1H), 8.93 (s, 1H), 8.74 (d, *J* = 5.0 Hz, 1H), 8.46 (s, 1H), 7.97 - 7.85 (m, 3H), 7.80 (d, *J* = 8.9 Hz, 1H), 7.46 (s, 2H), 7.37 (t, *J* = 7.5 Hz, 2H), 7.30 (d, *J* = 7.0 Hz, 1H), 6.02 - 5.86 (m, 1H), 4.96 - 4.91(m, 1H), 4.90 - 4.80 (m, 1H), 4.72 (d, *J* = 15.0 Hz, 1H), 4.46 (brs, 2H), 3.40 - 3.27 (m, 4H), 2.90 - 2.83 (m, 4H), 2.67 - 2.57 (m, 3H), 2.15 - 2.04 (m, 1H). LCMS (ESI) calcd. for $C_{30}H_{32}N_5O_2S^+$ [M+H]$^+$: 526.23, found, 526.2.

**Example 203: Preparation of 3-(5-((4-(phenyl(pyridin-4-yl)methyl)piperazin-1-yl)methyl)-1-thioxoisoindolin-2-yl)piperidine-2,6-dione (GT-07733)**

With reference to the method of Scheme 4, the target compound GT-07733 was obtained (28mg, white solid, yield 36%). $^1H$ NMR (400 MHz, DMSO) δ 11.50 (s, 1H), 11.14 (s, 1H), 8.82 (d, *J* = 6.2 Hz, 2H), 8.07 (d, *J* = 5.8 Hz, 2H), 7.94 (d, *J* = 4.9 Hz, 1H), 7.86 (d, *J* = 5.0 Hz, 1H), 7.84 - 7.77 (m, 1H), 7.45 (d, *J* = 7.4 Hz, 2H), 7.37 (t, *J* = 7.5 Hz, 2H), 7.31 (d, *J* = 7.2 Hz, 1H), 6.00 - 5.95 (m, 1H), 4.97 (s, 1H), 4.86 (d, *J* = 20.1 Hz, 1H), 4.72 (d, *J* = 20.2 Hz, 1H), 4.46 (brs, 2H), 3.40 - 3.27 (m, 4H), 2.92 - 2.71 (m, 4H), 2.70 - 2.59 (m, 3H), 2.21 - 1.99 (m, 1H).LCMS (ESI) calcd. for $C_{30}H_{32}N_5O_2S^+$ [M+H]$^+$: 526.23,

found, 526.2.

**Example 204: Preparation of 3-(5-((4-(di(pyridin-2-yl)methyl)piperazin-1-yl)methyl)-1-thioxoisoindolin-2-yl)piperidine-2,6-dione (GT-07734)**

With reference to the method of Scheme 4, the target compound GT-07734 was obtained (21 mg, white solid, yield 27%). $^1$H NMR (400 MHz, DMSO-d6) δ 11.87 (s, 1H), 11.10 (s, 1H), 8.70 (d, *J* = 4.7 Hz, 2H), 8.15 (t, *J* = 7.3 Hz, 2H), 7.91 - 7.78 (m, 5H), 7.66 - 7.59 (m, 2H), 5.83 (s, 1H), 4.56 - 4.47 (m, 6H), 3.37 (s, 4H), 3.00 (s, 4H), 2.89 - 2.76 (m, 1H), 2.67 - 2.61 (m, 1H), 2.14 - 2.08 (m, 1H). LCMS (ESI) calcd. for $C_{29}H_{31}N_6O_2S^+$ [M+H]$^+$: 527.22, found, 527.2.

**Example 205: Preparation of 3-(5-((4-(di(pyridin-3-yl)methyl)piperazin-1-yl)methyl)-1-thioxoisoindolin-2-yl)piperidine-2,6-dione (GT-07735)**

With reference to the method of Scheme 4, the target compound GT-07735 was obtained (13 mg, white solid, yield 17%). $^1$H NMR (400 MHz, DMSO-d6) δ 11.43 (s, 1H), 8.94 (s, 2H), 8.77 (d, *J* = 4.9 Hz, 2H), 8.39 (d, *J* = *7.9* Hz, 2H), 7.94 - 7.76 (m, 5H), 5.25 (s, 1H), 4.52 - 4.44 (m, 2H), 3.68 - 3.56 (m, 3H), 3.41 - 3.27 (m, 3H), 3.23 - 3.12 (m, 3H), 2.96 - 2.78 (m, 3H), 2.64 - 2.59 (m, 3H), 2.17 - 2.04 (m, 1H). LCMS (ESI) calcd. for $C_{29}H_{31}N_6O_2S^+$ [M+H]$^+$: 527.22, found, 527.2.

**Example 206: Preparation of 3-(5-((4-(bis(4-fluorophenyl)methyl)piperazin-1-yl)methyl)-1-thioxoisoindolin-2-yl)piperidine-2,6-dione (GT-07736)**

With reference to the method of Scheme 4, the target compound GT-07736 was obtained (23 mg, white solid, yield 28%). $^1$H NMR (400 MHz, DMSO-d6) δ 11.10 (s, 1H), 7.91 - 7.81 (m, 2H), 7.77 (d, *J* = 8.0 Hz, 1H), 7.52 (s, 4H), 7.26 - 7.10 (m, 4H), 4.85 (d, *J* = 20.3 Hz, 1H), 4.71 (d, *J* = 20.3 Hz, 1H), 4.45 (s, 2H), 3.52 - 3.41 (m, 3H), 3.37 - 3.29 (m, 3H), 3.24 - 3.07 (m, 2H), 3.00 - 2.78 (m, 3H), 2.70 - 2.57 (m, 2H), 2.19 - 2.02 (m, 1H). LCMS (ESI) calcd. for $C_{31}H_{31}F_2N_4O_2S^+$ [M+H]$^+$: 561.21, found, 561.1.

**Example 207: Preparation of 3-(5-((4-(bis(4-chlorophenyl)methyl)piperazin-1-yl)methyl)-1-thioxoisoindolin-2-yl)piperidine-2,6-dione (GT-07737)**

With reference to the method of Scheme 4, the target compound GT-07737 was obtained (29 mg, white solid, yield 33%). $^1$H NMR (400 MHz, DMSO-d6) δ 11.14 (s, 1H), 10.80 (s, 1H), 7.94 (d, *J* = 7.9 Hz, 1H), 7.88 (t, *J* = 6.0 Hz, 2H), 7.82 (s, 1H), 7.75 (t, *J* = 8.1 Hz, 2H), 7.49 - 7.42 (m, 5H), 6.01 - 5.87 (m, 1H), 4.85 (d, *J* = 20.4 Hz, 1H), 4.72 (d, *J* = 20.3 Hz, 1H), 4.45 (s, 2H), 3.36 - 3.27 (m, 3H), 3.17 - 3.11 (m, 4H), 2.86 - 2.78 (m, 4H), 2.67 - 2.62 (m, 1H), 2.12 - 2.03 (m, 1H). LCMS (ESI) calcd. for $C_{31}H_{31}Cl_2N_4O_2S^+$ [M+H]$^+$: 593.15, found, 593.1.

**Example 208: Preparation of 3-(5-((3-benzhydryl-3,6-diazabicyclo[3.1.1]heptan-6-yl)methyl)-1-thioxoisoindolin-2-yl)piperidine-2,6-dione (GT-07738)**

With reference to the method of Scheme 4, the target compound GT-07738 was obtained (9 mg, white solid, yield 11%). $^1$H NMR (400 MHz, DMSO-d6) δ 11.13 (s, 1H), 7.94 - 7.91 (m, 1H), 7.78 - 7.71 (m, 1H), 7.56 (d, *J* = 7.9 Hz, 1H), 7.51 - 7.45 (m, 4H), 7.39 - 7.28 (m, 5H), 7.27 - 7.19 (m, 2H), 6.04 - 5.86 (m, 1H), 4.84 (d, *J* = 20.5 Hz, 1H), 4.70 (d, *J* = 20.1 Hz, 2H), 4.34 (d, *J* = 5.5 Hz, 1H), 4.30 - 4.12 (m, 2H), 3.65 - 3.56 (m, 1H), 3.14 - 3.07 (m, 3H), 3.03 - 2.87 (m, 2H), 2.69 - 2.65 (m, 3H), 2.36 - 2.25 (m, 1H), 2.16 - 2.05 (m, 1H). LCMS (ESI) calcd. for $C_{32}H_{33}N_4O_2S^+$ [M+H]$^+$: 537.23, found, 537.1

**Example 209: Preparation of 3-(5-(((1R,4R)-5-benzhydryl-2,5-diazabicyclo[2.2.1]heptan-2-yl)methyl)-1-thioxoisoindolin-2-yl)piperidine-2,6-dione (GT-07740)**

With reference to the method of Scheme 4, the target compound GT-07740 was obtained (15 mg, white solid, yield 19%). $^1$H NMR (400 MHz, DMSO-d6) δ 11.14 (s, 1H), 8.08 - 7.94 (m, 5H), 7.49 - 7.20 (m, 8H), 6.08 - 5.86 (m, 1H), 4.86 (d, *J* = 20.4 Hz, 1H), 4.72 (d, *J* = 20.4 Hz, 1H), 4.68 - 4.51 (m, 1H), 4.49 - 4.31 (m, 1H), 4.30 - 4.02 (m, 1H), 3.36 - 3.17 (m, 3H), 3.03 - 2.91 (m, 2H), 2.68 - 2.62 (m, 2H), 2.60 - 2.54 (m, 1H), 2.42 - 2.34 (m, 2H), 2.12 - 2.07 (m, 1H). LCMS (ESI) calcd. for $C_{32}H_{33}N_4O_2S^+$ [M+H]$^+$: 537.23, found, 537.2.

**Example 210: Preparation of 3-(5-(((1S,4S)-5-benzhydryl-2,5-diazabicyclo[2.2.1]heptan-2-yl)methyl)-1-thioxoisoindolin-2-yl)piperidine-2,6-dione (GT-07739)**

With reference to the method of Scheme 4, the target compound GT-07739 was obtained (16 mg, white solid,

yield 20%). $^1$H NMR (400 MHz, DMSO-d6) δ 11.14 (s, 1H), 8.15 - 7.80 (m, 5H), 7.51 - 7.20 (m, 8H), 6.04 - 5.89 (m, 1H), 4.86 (d, *J* = 20.5 Hz, 1H), 4.72 (d, *J* = 20.3 Hz, 1H), 4.66 - 4.49 (m, 1H), 4.50 - 4.32 (m, 1H), 4.19 - 4.12 (m, 1H), 3.30 - 3.12 (m, 3H), 3.03 - 2.92 (m, 2H), 2.69 - 2.59 (m, 2H), 2.58 - 2.54 (m, 2H), 2.43 - 2.37 (m, 2H), 2.12 - 2.05 (m, 1H). LCMS (ESI) calcd. for $C_{32}H_{33}N_4O_2S^+$ [M+H]$^+$: 537.23, found, 537.2.

**Example 211: Preparation of 3-(5-((8-benzhydryl-3,8-diazabicyclo[3.2.1]octan-3-yl)methyl)-1-thioxoisoindolin-2-yl)piperidine-2,6-dione (GT-07849)**

With reference to the method of Scheme 4, the target compound GT-07849 was obtained (20 mg, white solid, yield 25%). $^1$H NMR (400 MHz, DMSO-d6) δ 11.13 (s, 1H), 7.99 - 7.84 (m, 5H), 7.52 - 7.35 (m, 8H), 6.03 - 5.90 (m, 1H), 4.75 - 4.67 (m, 1H), 4.69 - 4.43 (m, 1H), 4.52 - 4.40 (m, 1H), 3.73 - 3.65 (m, 2H), 3.29 - 3.21 (m, 2H), 3.18 - 3.06 (m, 4H), 3.00 - 2.92 (m, 1H), 2.67 - 2.61 (m, 2H), 2.16 - 2.01 (m, 5H). LCMS (ESI) calcd. for $C_{33}H_{35}N_4O_2S^+$ [M+H]$^+$: 551.25, found, 551.2.

**Example 212: Preparation of 3-(5-((3-benzhydryl-3,8-diazabicyclo[3.2.1]octan-8-yl)methyl)-1-thioxoisoindolin-2-yl)piperidine-2,6-dione (GT-07850)**

With reference to the method of Scheme 4, the target compound GT-07850 was obtained (10 mg, white solid, yield 12%). $^1$H NMR (400 MHz, DMSO-d6) δ 11.13 (s, 1H), 10.20 (s, 1H), 7.93 (d, *J* = 3.5 Hz, 1H), 7.46 (d, *J* = 7.3 Hz, 5H), 7.31 (t, *J* = 7.6 Hz, 5H), 7.20 (t, *J* = 7.3 Hz, 3H), 6.00 - 5.88 (m, 1H), 4.84 (d, *J* = 20.6 Hz, 1H), 4.71 (d, *J* = 20.2 Hz, 1H), 4.31 (d, *J* = 6.2 Hz, 2H), 3.83 (s, 2H), 2.98 - 2.89 (m, 1H), 2.70 - 2.64 (m, 3H), 2.60 - 2.55 (m, 2H), 2.33 - 2.22 (m, 5H), 2.16 - 2.03 (m, 2H). LCMS (ESI) calcd. for $C_{33}H_{35}N_4O_2S^+$ [M+H]$^+$: 551.25, found, 551.2.

**Example 213: Preparation of 3-(5-((5-benzhydryl-2,5-diazabicyclo[2.2.2]octan-2-yl)methyl)-1-thioxoisoindolin-2-yl)piperidine-2,6-dione (GT-07847)**

With reference to the method of Scheme 4, the target compound GT-07847 was obtained (15 mg, white solid, yield 19%). $^1$H NMR (400 MHz, DMSO-d6) δ 11.14 (s, 1H), 8.14 - 7.90 (m, 5H), 7.56 - 7.30 (m, 8H), 6.02 - 5.88 (m, 1H), 4.94 - 4.80 (m, 1H), 4.74 - 4.63 (m, 3H), 3.27 - 3.14 (m, 2H), 3.01 - 2.85 (m, 3H), 2.65 (d, *J* = 17.7 Hz, 2H), 2.61 - 2.55 (m, 2H), 2.21 - 1.96 (m, 4H), 1.92 - 1.68 (m, 2H). LCMS (ESI) calcd. for $C_{33}H_{35}N_4O_2S^+$ [M+H]$^+$: 551.25, found, 551.2.

**Example 214: Preparation of 3-(5-((4-benzhydryl-1,4-diazepan-1-yl)methyl)-1-thioxoisoindolin-2-yl)piperidine-2,6-dione (GT-07848)**

With reference to the method of Scheme 4, the target compound GT-07848 was obtained (19 mg, white solid, yield 24%). $^1$H NMR (400 MHz, DMSO-d6) δ 11.14 (s, 1H), 7.96 - 7.75 (m, 5H), 7.49 - 7.26 (m, 8H), 6.02 - 5.88 (m, 1H), 4.86 (d, *J* = 20.4 Hz, 1H), 4.72 (d, *J* = 20.6 Hz, 1H), 4.58 - 4.39 (m, 2H), 4.11 - 3.81 (m, 1H), 3.28 - 3.11 (m, 4H), 3.03 - 2.91 (m, 2H), 2.85 - 2.81 (m, 1H), 2.65 (d, *J* = 18.7 Hz, 2H), 2.52 - 2.50 (m, 2H), 2.13 - 1.96 (m, 3H). LCMS (ESI) calcd. for $C_{32}H_{35}N_4O_2S^+$ [M+H]$^+$: 539.25, found, 539.2.

**Example 215: Preparation of 3-(5-((4-benzhydryl-3,3-dimethylpiperazin-1-yl)methyl)-1-thioxoisoindolin-2-yl)piperidine-2,6-dione (GT-07845)**

With reference to the method of Scheme 4, the target compound GT-07845 was obtained (16 mg, white solid, yield 20%). $^1$H NMR (400 MHz, DMSO-d6) δ 11.14 (s, 1H), 10.37 (s, 1H), 7.98 - 7.93 (m, 2H), 7.39 - 7.26 (m, 8H), 7.24 - 7.17 (m, 3H), 6.03 - 5.87 (m, 1H), 4.87 (d, *J* = 20.3 Hz, 1H), 4.73 (d, *J* = 20.6 Hz, 1H), 4.54 - 4.47 (m, 1H), 4.37 - 4.31 (m, 1H), 3.35 - 3.29 (m, 1H), 3.18 - 3.07 (m, 1H), 3.06 - 2.85 (m, 4H), 2.79 - 2.59 (m, 4H), 2.15 - 2.06 (m, 1H), 1.20 (s, 3H), 1.02 (s, 3H). LCMS (ESI) calcd. for $C_{33}H_{37}N_4O_2S^+$ [M+H]$^+$: 553.26, found, 553.2.

**Example 216: Preparation of 3-(5-((4-(phenyl(pyridin-2-yl)amino)piperidin-1-yl)methyl)-1-thioxoisoindolin-2-yl) piperidine-2,6-dione (GT-07846)**

With reference to the method of Scheme 4, the target compound GT-07846 was obtained (17 mg, white solid, yield 22%). LCMS (ESI) calcd. for $C_{30}H_{32}N_5O_2S^+$ [M+H]$^+$: 526.23, found, 526.2.

**Example 217: Preparation of 3-(5-((3-((4'-chloro-5,5-dimethyl-3,4,5,6-tetrahydro-[1,1'-biphenyl]-2-yl) methyl)-3,6-diazabicyclo[3.1.1]heptan-6-yl)methyl)-1-thioxoisoindolin-2-yl)piperidine-2,6-dione (GT-08160)**

With reference to the method of Scheme 4, the target compound GT-08160 was obtained (7 mg, white solid, yield

11%). $^1$H NMR (400 MHz, DMSO-d6) δ 11.14 (s, 1H), 7.96 - 7.92 (m, 1H), 7.92 - 7.88 (m, 1H), 7.78 - 7.66 (m, 1H), 7.39 (d, *J* = 8.3 Hz, 2H), 7.10 (d, *J* = 8.5 Hz, 2H), 5.95 (d, *J* = 10.5 Hz, 1H), 4.88 - 4.84 (m, 1H), 4.74 - 4.68 (m, 1H), 4.65 - 4.60 (m, 2H), 4.43 - 4.25 (m, 1H), 4.22 - 4.00 (m, 2H), 3.33 - 3.18 (m, 1H), 3.13 - 3.05 (m, 4H), 2.80 - 2.70 (m, 1H), 2.65 (d, *J* = 17.9 Hz, 1H), 2.59 - 2.54 (m, 1H), 2.28 - 2.17 (m, 3H), 2.14 - 2.05 (m, 2H), 2.03 - 1.92 (m, 2H), 1.49 - 1.33 (m, 2H), 0.95 (d, *J* = 5.6 Hz, 6H). LCMS (ESI) calcd. for $C_{34}H_{40}ClN_4O_2S^+$ [M+H]$^+$: 603.26, found, 603.2.

**Example 218: Preparation of 3-(5-((6-((4'-chloro-5,5-dimethyl-3,4,5,6-tetrahydro-[1,1'-biphenyl]-2-yl) methyl)-3,6-diazabicyclo[3.1.1]heptan-3-yl)methyl)-1-thioxoisoindolin-2-yl)piperidine-2,6-dione (GT-08161)**

With reference to the method of Scheme 4, the target compound GT-08161 was obtained (14 mg, white solid, yield 21%). $^1$H NMR (400 MHz, DMSO-d6) δ 11.13 (s, 1H), 7.96 - 7.91(m, 1H), 7.90 - 7.80 (m, 1H), 7.48 (d, *J* = 8.4 Hz, 2H), 7.22 (d, *J* = 8.4 Hz, 2H), 7.18 - 7.14 (m, 1H), 5.95 (d, *J* = 8.9 Hz, 1H), 4.83 (d, *J* = 19.7 Hz, 1H), 4.69 (d, *J* = 19.9 Hz, 1H), 4.27 - 4.25 (m, 1H), 4.18 - 4.14 (m, 1H), 3.84 - 2.79 (m, 2H), 3.54 - 3.35 (m, 4H), 3.03 - 2.94 (m, 1H), 2.65 (d, *J* = 18.8 Hz, 2H), 2.49 - 2.41 (m, 2H), 2.30 - 2.23 (m, 3H), 2.07 - 2.02 (m, 4H), 1.45 (d, *J* = 4.4 Hz, 2H), 0.95 (s, 6H). LCMS (ESI) calcd. for $C_{34}H_{40}ClN_4O_2S^+$ [M+H]$^+$: 603.26, found, 603.2.

**Example 219: Preparation of 3-(5-((8-((4'-chloro-5,5-dimethyl-3,4,5,6-tetrahydro-[1,1'-biphenyl]-2-yl) methyl)-3,8-diazabicyclo[3.2.1]octan-3-yl)methyl)-1-thioxoisoindolin-2-yl)piperidine-2,6-dione (GT-08162)**

With reference to the method of Scheme 4, the target compound GT-08162 was obtained (23 mg, white solid, yield 34%). $^1$H NMR (400 MHz, DMSO-d6) δ 11.12 (s, 1H), 7.86 (d, *J* = 7.8 Hz, 1H), 7.69 - 7.48 (m, 2H), 7.44 (d, *J* = 8.3 Hz, 3H), 7.19 (d, *J* = 8.3 Hz, 3H), 5.94 (d, *J* = 9.4 Hz, 1H), 4.80 (d, *J* = 20.2 Hz, 1H), 4.66 (d, *J* = 20.1 Hz, 1H), 3.89 - 3.81 (m, 2H), 3.48 (brs, 2H), 3.04 - 2.78 (m, 3H), 2.73 - 2.62 (m, 3H), 2.47 - 2.42 (m, 2H), 2.35 - 2.31 (m, 3H), 2.07 (s, 4H), 1.85 - 1.81 (m, 2H), 1.47 (t, *J* = 6.0 Hz, 2H), 1.27 - 1.15 (m, 2H), 0.96 (s, 6H). LCMS (ESI) calcd. for $C_{35}H_{42}ClN_4O_2S^+$ [M+H]$^+$: 617.27, found, 617.2.

**Example 220: Preparation of 3-(5-((3-((4'-chloro-5,5-dimethyl-3,4,5,6-tetrahydro-[1,1'-biphenyl]-2-yl) methyl)-3,8-diazabicyclo[3.2.1]octan-8-yl)methyl)-1-thioxoisoindolin-2-yl)piperidine-2,6-dione (GT-08163)**

With reference to the method of Scheme 4, the target compound GT-08163 was obtained (19 mg, white solid, yield 28%). $^1$H NMR (400 MHz, DMSO-d6) δ 11.14 (s, 1H), 9.95 (s, 1H), 7.95 (d, *J* = 7.9 Hz, 1H), 7.91 (s, 1H), 7.79 (d, *J* = 7.9 Hz, 1H), 7.36 (d, *J* = 8.3 Hz, 2H), 7.05 (d, *J* = 8.1 Hz, 2H), 5.95 (d, *J* = 8.2 Hz, 1H), 4.86 (d, *J* = 20.4 Hz, 1H), 4.72 (d, *J* = 20.3 Hz, 1H), 4.28 (d, *J* = 0.6 Hz, 2H), 3.79 - 3.69 (m, 4H), 3.03 - 2.91 (m, 1H), 2.79 (s, 2H), 2.71 - 2.57 (m, 3H), 2.42 - 2.29 (m, 2H), 2.28 - 2.21 (m, 2H), 2.21 - 2.12 (m, 2H), 2.03 - 1.85 (m, 4H), 1.42 (t, *J* = 6.1 Hz, 2H), 0.94 (s, 6H). LCMS (ESI) calcd. for $C_{35}H_{42}ClN_4O_2S^+$ [M+H]$^+$: 617.27, found, 617.2.

**Example 221: Preparation of 3-(5-((4-((4'-fluoro-3,4,5,6-tetrahydro-[1,1'-biphenyl]-2-yl)methyl)piperazin-1-yl) methyl)-1-thioxoisoindolin-2-yl)piperidine-2,6-dione (GT-08164)**

With reference to the method of Scheme 4, the target compound GT-08164 was obtained (17 mg, white solid, yield 28%). $^1$H NMR (400 MHz, DMSO-d6) δ 11.14 (s, 1H), 7.93 (d, *J* = 8.1 Hz, 1H), 7.88 - 7.72 (m, 1H), 7.72 - 7.56 (m, 1H), 7.26 - 7.10 (m, 4H), 5.95 (d, *J* = 9.5 Hz, 1H), 4.84 (d, *J* = 20.4 Hz, 1H), 4.70 (d, *J* = 20.1 Hz, 1H), 4.52 - 4.16 (m, 1H), 3.79 - 3.71 (m, 4H), 3.50 - 3.10 (m, 6H), 3.03 - 2.92 (m, 2H), 2.65 (d, *J* = 18.6 Hz, 1H), 2.60 - 2.53 (m, 1H), 2.32 - 2.17 (m, 4H), 2.14 - 2.03 (m, 1H), 1.67 (s, 4H). LCMS (ESI) calcd. for $C_{31}H_{36}FN_4O_2S^+$ [M+H]$^+$: 547.25, found, 547.2.

**Example 222: Preparation of 3-(5-((4-(4'-fluoro-3,4,5,6-tetrahydro-[1,1'-biphenyl]-2-carbonyl)piperazin-1-yl) methyl)-1-thioxoisoindolin-2-yl)piperidine-2,6-dione (GT-08165)**

With reference to the method of Scheme 4, the target compound GT-08165 was obtained (23 mg, white solid, yield 37%). $^1$H NMR (400 MHz, DMSO-d6) δ 11.14 (s, 1H), 7.95 (d, *J* = 7.3 Hz, 1H), 7.80 - 7.72 (m, 1H), 7.67 - 7.60 (m, 1H), 7.34 - 7.18 (m, 2H), 7.18 - 7.06 (m, 2H), 5.97 (s, 1H), 4.84 (d, *J* = 20.3 Hz, 1H), 4.72 (d, *J* = 19.0 Hz, 1H), 4.51 - 4.36 (m, 1H), 4.36 - 4.14 (m, 1H), 3.83 - 3.67 (m, 1H), 3.23 - 3.19 (m, 1H), 3.16 - 3.02 (m, 1H), 3.02 - 2.79 (m, 3H), 2.67 - 2.57 (m, 3H), 2.46 - 2.33 (m, 3H), 2.20 - 1.96 (m, 4H), 1.80 - 1.52 (m, 4H). LCMS (ESI) calcd. for $C_{31}H_{34}FN_4O_3S^+$ [M+H]$^+$: 561.23, found, 561.2.

**Example 223: Preparation of 3-(5-((4-((4'-chloro-[1,1'-biphenyl]-2-yl)methyl)piperazin-1-yl)methyl)-1-thioxoisoindolin-2-yl)piperidine-2,6-dione (GT-08166)**

With reference to the method of Scheme 4, the target compound GT-08166 was obtained (20 mg, white solid, yield 33%). $^1$H NMR (400 MHz, DMSO-d6) δ 11.13 (s, 1H), 7.93 (d, *J* = 7.6 Hz, 1H), 7.88 - 7.74 (m, 1H), 7.74 - 7.61 (m, 1H), 7.51 (d, *J* = 8.2 Hz, 2H), 7.49 - 7.41 (m, 3H), 7.40 (d, *J* = 8.4 Hz, 2H), 7.32 - 7.24 (m, 1H), 5.95 (d, *J* = 9.2 Hz, 1H), 4.84 (d, *J* = 20.3 Hz, 1H), 4.70 (d, *J* = 20.6 Hz, 1H), 4.55 - 4.21 (m, 2H), 3.80 - 3.69 (m, 2H), 3.30 - 3.23 (m, 3H), 3.13 - 2.83 (m, 5H), 2.65 (d, *J* = 19.2 Hz, 1H), 2.55 - 2.51 (m, 2H), 2.14 - 2.04 (m, 1H). LCMS (ESI) calcd. for $C_{31}H_{32}ClN_4O_2S^+$ [M+H]$^+$: 559.19, found, 559.1.

**Example 224: Preparation of 3-(5-((4-((4-(4-chlorophenyl)-6,6-dimethyl-5,6-dihydro-2H-pyran-3-yl)methyl)piperazin-1-yl)methyl)-1-thioxoisoindolin-2-yl)piperidine-2,6-dione (GT-08167)**

With reference to the method of Scheme 4, the target compound GT-08167 was obtained (21 mg, white solid, yield 32%). $^1$H NMR (400 MHz, DMSO-d6) δ 11.14 (s, 1H), 7.93 (d, *J* = 7.5 Hz, 1H), 7.83 - 7.79 (m, 1H), 7.72 - 7.61 (m, 1H), 7.43 (d, *J* = 8.0 Hz, 2H), 7.18 (d, *J* = 7.7 Hz, 2H), 5.95 (d, *J* = 7.7 Hz, 1H), 4.84 (d, *J* = 20.0 Hz, 1H), 4.71 (d, *J= 20.6* Hz, 1H), 4.56 - 4.29 (m, 2H), 4.31 - 4.09 (m, 2H), 3.77 - 3.71 (m, 2H), 3.39 - 3.16 (m, 3H), 3.13 - 2.82 (m, 5H), 2.67 - 2.62 (m, 1H), 2.58 - 2.52 (m, 2H), 2.19 (s, 2H), 2.13 - 2.01 (m, 1H), 1.21 (s, 6H). LCMS (ESI) calcd. for $C_{32}H_{38}ClN_4O_3S^+$ [M+H]$^+$: 593.23, found, 593.2.

Example 225: Preparation of 3-(5-((4-(thiophen-2-yl)piperazin-1-yl)methyl)-1-**thioxoisoindolin-2-yl)piperidine-2,6-dione** (GT-08168)

With reference to the method of Scheme 4, the target compound GT-08168 was obtained (13 mg, white solid, yield 26%). $^1$H NMR (400 MHz, DMSO-d6) δ 11.14 (s, 1H), 9.54 (s, 1H), 7.98- 7.96 (m, 1H), 7.85 (d, *J* = 8.0 Hz, 1H), 7.80 (d, *J* = 8.0 Hz, 1H), 7.60 (s, 1H), 7.49 (d, *J* = 8.2 Hz, 1H), 6.87 - 6.76 (m, 1H), 6.71 (d, *J* = 3.7 Hz, 1H), 5.96 (d, *J* = 11.2 Hz, 1H), 4.87 (d, *J* = 20.4 Hz, 1H), 4.73 (d, *J* = 20.6 Hz, 1H), 4.53 - 4.43 (m, 2H), 3.53 - 3.46 (m, 2H), 3.47 - 3.32 (m, 3H), 3.29 - 3.12 (m, 3H), 3.04 - 2.89 (m, 2H), 2.67 - 2.63 (m, 1H), 2.58 - 2.54 (m, 1H), 2.16 - 2.02 (m, 1H). LCMS (ESI) calcd. for $C_{22}H_{25}N_4O_2S_2^+$ [M+H]$^+$: 441.14, found, 441.1.

**Example 226: Preparation of 3-(5-((4-(thiophen-3-yl)piperazin-1-yl)methyl)-1-thioxoisoindolin-2-yl)piperidine-2,6-dione (GT-08169)**

With reference to the method of Scheme 4, the target compound GT-08169 was obtained (5 mg, white solid, yield 10%). $^1$H NMR (400 MHz, DMSO-d6) δ 11.13 (s, 1H), 7.99 (d, *J* = 7.9 Hz, 1H), 7.93 (d, *J*= 7.3 Hz, 1H), 7.78 (d, *J* = 7.5 Hz, 1H), 7.46 (dd, *J* = 5.2, 3.1 Hz, 1H), 6.97 (dd, *J* = 5.3, 1.5 Hz, 1H), 6.48 (dd, *J* = 3.1, 1.5 Hz, 1H), 5.96 (d, *J* = 12.7 Hz, 1H), 4.84 (d, *J*= 18.1 Hz, 1H), 4.70 (d, *J* = 19.3 Hz, 1H), 4.53 (d, *J* = 5.1 Hz, 1H), 3.68 - 3.65 (m, 1H), 3.50 - 3.46 (m, 4H), 3.40 (d, *J* = 12.1 Hz, 1H), 3.28 - 3.12 (m, 2H), 3.11 - 2.93 (m, 2H), 2.65 (d, *J* = 17.8 Hz, 1H), 2.56 - 2.51 (m, 1H), 2.13 - 2.07 (m, 1H). LCMS (ESI) calcd. for $C_{22}H_{25}N_4O_2S_2^+$ [M+H]$^+$: 441.14, found, 441.1.

**Example 227: Preparation of 3-(5-((4-(5-methylthiophen-3-yl)piperazin-1-yl)methyl)-1-thioxoisoindolin-2-yl)piperidine-2,6-dione (GT-08170)**

With reference to the method of Scheme 4, the target compound GT-08170 was obtained (15 mg, white solid, yield 30%). $^1$H NMR (400 MHz, DMSO-d6) δ 11.44 (s, 1H), 11.14 (s, 1H), 7.98 (s, 1H), 7.90 - 7.86 (m, 1H), 7.85 (d, *J* = 8.6 Hz, 1H), 6.68 (s, 1H), 6.21 - 6.16 (m, 1H), 5.96 (d, *J* = 11.5 Hz, 1H), 4.88 (d, *J* = 20.5 Hz, 1H), 4.74 (d, *J* = 20.4 Hz, 1H), 4.55 - 4.48 (m, 2H), 3.67 - 3.53 (m, 3H), 3.41 - 3.30 (m, 4H), 3.20 - 3.15 (m, 3H), 2.67 - 2.63 (m, 2H), 2.36 (s, 3H), 2.14 - 2.06 (m, 1H). LCMS (ESI) calcd. for $C_{23}H_{27}N_4O_2S_2^+$ [M+H]$^+$: 455.16, found, 455.1.

**Example 228: Preparation of 3-(5-((4-(thiophen-2-yl)-3,6-dihydropyridin-1(2H)-yl)methyl)-1-thioxoisoindolin-2-yl)piperidine-2,6-dione (GT-08171)**

With reference to the method of Scheme 4, the target compound GT-08171 was obtained (12 mg, white solid, yield 25%). $^1$H NMR (400 MHz, DMSO-d6) δ 11.15 (s, 1H), 11.05 (s, 1H), 8.02 - 7.93 (m, 2H), 7.83 (dd, *J* = 12.9, 7.8 Hz, 1H), 7.49 (d, *J* = 5.2 Hz, 1H), 7.17 (d, *J* = 3.2 Hz, 1H), 7.09 - 7.00 (m, 1H), 6.04 (s, 1H), 5.96 (d, *J* = 13.2 Hz, 1H), 4.88 (d, *J* = 20.4 Hz, 1H), 4.76 (t, *J* = 12.9 Hz, 1H), 4.62 - 4.45 (m, 2H), 3.87 - 3.68 (m, 2H), 3.64 - 3.56 (m, 1H), 3.31 - 3.19 (m, 1H), 3.05 - 2.92 (m, 1H), 2.91 - 2.73 (m, 2H), 2.65 (d, *J* = 16.9 Hz, 1H), 2.60 - 2.53 (m, 1H), 2.13 - 2.07 (m, 1H). LCMS (ESI) calcd. for $C_{23}H_{24}N_3O_2S_2^+$ [M+H]$^+$: 438.13, found, 438.1.

**Example 229: Preparation of 3-(5-((4-(thiophen-3-yl)-3,6-dihydropyridin-1(2H)-yl)methyl)-1-thioxoisoindolin-2-yl)piperidine-2,6-dione (GT-08172)**

With reference to the method of Scheme 4, the target compound GT-08172 was obtained (16 mg, white solid, yield 33%). $^1$H NMR (400 MHz, DMSO-d6) δ 11.15 (s, 1H), 10.77 (s, 1H), 7.99 (d, *J* = 7.8 Hz, 1H), 7.93 (s, 1H), 7.80 (d, *J* = 7.4 Hz, 1H), 7.57 (dd, *J* = 12.7, 7.7 Hz, 2H), 7.37 (d, *J* = 4.8 Hz, 1H), 6.15 (s, 1H), 5.97 (d, *J* = 8.6 Hz, 1H), 4.87 (t, *J* = 20.4 Hz, 1H), 4.75 (d, *J* = 20.4 Hz, 1H), 4.57 - 4.52 (m, 2H), 3.89 - 3.69 (m, 2H), 3.65 - 3.56 (m, 1H), 3.29 - 3.16 (m, 1H), 3.01 - 2.93 (m, 1H), 2.84 - 2.76 (m, 2H), 2.65 (d, *J* = 16.4 Hz, 1H), 2.60 - 2.55 (m, 1H), 2.12 - 2.07 (m, 1H). LCMS (ESI) calcd. for $C_{23}H_{24}N_3O_2S_2^+$ [M+H]$^+$: 438.13, found, 438.1.

**Example 230: Preparation of 3-(5-((4-(5-methylthiophen-3-yl)-3,6-dihydropyridin-1(2H)-yl)methyl)-1-thioxoisoindolin-2-yl)piperidine-2,6-dione (GT-08327)**

With reference to the method of Scheme 4, the target compound GT-08327 was obtained (5 mg, white solid, yield 9%). $^1$H NMR (400 MHz, DMSO-d6) δ 11.14 (s, 1H), 10.65 (s, 1H), 7.99 (d, *J* = 7.2 Hz, 1H), 7.91 (s, 1H), 7.84 - 7.73 (m, 1H), 7.27 (s, 1H), 7.05 (s, 1H), 6.06 (s, 1H), 5.95 - 5.91 (m, 1H), 4.88 (d, *J* = 20.1 Hz, 1H), 4.75 (d, *J* = 20.1 Hz, 1H), 4.60 - 4.49 (m, 2H), 3.86 - 3.67 (m, 2H), 3.64 - 3.61 (m, 1H), 3.01 - 2.96 (m, 1H), 2.81 - 2.70 (m, 2H), 2.69 - 2.57 (m, 3H), 2.42 (s, 3H), 2.18 - 2.05 (m, 1H). LCMS (ESI) calcd. for $C_{24}H_{26}N_3O_2S_2^+$ [M+H]$^+$: 452.15, found, 452.1.

**Example 231: Preparation of 3-(5-((4-(furan-3-yl)-3,6-dihydropyridin-1(2H)-yl)methyl)-1-thioxoisoindolin-2-yl) piperidine-2,6-dione (GT-08328)**

With reference to the method of Scheme 4, the target compound GT-08328 was obtained (4 mg, white solid, yield 8%). $^1$H NMR (400 MHz, DMSO-d6) δ 11.14 (s, 1H), 10.79 (s, 1H), 7.98 (d, *J* = 7.7 Hz, 1H), 7.93 (s, 1H), 7.82 - 7.79 (m, 2H), 7.67 (s, 1H), 6.75 (s, 1H), 5.98 (s, 2H), 4.88 (d, *J* = 20.3 Hz, 1H), 4.74 (d, *J* = 20.3 Hz, 1H), 4.53 - 4.48 (m, 2H), 3.85 - 3.68 (m, 2H), 3.66 - 3.55 (m, 1H), 3.05 - 2.92 (m, 1H), 2.81 - 2.70 (m, 1H), 2.67 - 2.57 (m, 4H), 2.17 - 2.03 (m, 1H). LCMS (ESI) calcd. for $C_{23}H_{24}N_3O_3S^+$ [M+H]$^+$: 422.15, found, 422.1.

**Example 232: Preparation of 3-(5-((4-(thiophen-2-yl)piperidin-1-yl)methyl)-1-thioxoisoindolin-2-yl)piperidine-2,6-dione (GT-08329)**

With reference to the method of Scheme 4, the target compound GT-08329 was obtained (5 mg, white solid, yield 10%). LCMS (ESI) calcd. for $C_{23}H_{26}N_3O_2S_2^+$ [M+H]$^+$: 440.15, found, 440.1.

**Example 233: Preparation of 3-(5-((4-(3-methylthiophen-2-yl)piperidin-1-yl)methyl)-1-thioxoisoindolin-2-yl)piperidine-2,6-dione (GT-08323)**

With reference to the method of Scheme 4, the target compound GT-08323 was obtained (3 mg, white solid, yield 6%). $^1$H NMR (400 MHz, DMSO-d6) δ 11.14 (s, 1H), 10.33 (s, 1H), 7.99 (d, *J* = 7.8 Hz, 1H), 7.88 (s, 1H), 7.75 (d, *J* = 8.0 Hz, 1H), 7.26 (d, *J* = 4.7 Hz, 1H), 6.82 (d, *J* = 5.2 Hz, 1H), 5.97 - 5.83 (m, 1H), 4.88 (d, *J* = 20.5 Hz, 1H), 4.75 (d, *J* = 20.4 Hz, 1H), 4.46 (d, *J* = 4.7 Hz, 2H), 3.51 - 3.42 (m, 2H), 3.22 - 3.03 (m, 3H), 2.99 - 2.92 (m, 1H), 2.68 - 2.63 (m, 1H), 2.56 - 2.53(m, 2H), 2.14 (s, 3H), 2.01 - 1.97 (m, 2H), 1.94 - 1.84 (m, 2H). LCMS (ESI) calcd. for $C_{24}H_{28}N_3O_2S_2^+$ [M+H]$^+$: 454.16, found, 454.1.

**Example 234: Preparation of 3-(5-((4-(thiophen-3-yl)piperidin-1-yl)methyl)-1-thioxoisoindolin-2-yl)piperidine-2,6-dione (GT-08324)**

With reference to the method of Scheme 4, the target compound GT-08324 was obtained (6 mg, white solid, yield 12%). $^1$H NMR (400 MHz, DMSO-d6) δ 11.14 (s, 1H), 10.02 (s, 1H), 8.00 (d, *J* = 7.6 Hz, 1H), 7.87 (s, 1H), 7.74 (d, *J* = 7.7 Hz, 1H), 7.52 - 7.47 (m, 1H), 7.23 (s, 1H), 7.04 (d, *J* = 4.3 Hz, 1H), 6.02 - 5.96 (m, 1H), 4.89 (d, *J* = 21.2 Hz, 1H), 4.75 (d, *J* = 20.6 Hz, 1H), 4.49 - 4.44 (m, 2H), 3.49 - 3.44 (m, 2H), 3.16 - 3.01 (m, 3H), 2.92 - 2.83 (m, 2H), 2.16 - 2.02 (m, 4H), 1.85 - 1.78 (m, 2H). LCMS (ESI) calcd. for $C_{23}H_{26}N_3O_2S_2^+$ [M+H]$^+$: 440.15, found, 440.1.

**Example 235: Preparation of 3-(5-((4-(5-methylthiophen-3-yl)piperidin-1-yl)methyl)-1-thioxoisoindolin-2-yl)piperidine-2,6-dione (GT-08325)**

With reference to the method of Scheme 4, the target compound GT-08325 was obtained (4 mg, white solid, yield 7%). $^1$H NMR (400 MHz, DMSO-d6) δ 11.14 (s, 1H), 8.00 (d, *J* = 8.3 Hz, 1H), 7.86 (s, 1H), 7.74 (d, *J* = 4.9 Hz, 1H), 6.93 (s, 1H), 6.71 (s, 1H), 6.02 - 5.91 (m, 1H), 4.89 (d, *J* = 22.6 Hz, 1H), 4.75 (d, *J* = 20.9 Hz, 1H), 4.54 - 4.43 (m, 2H), 3.49 - 3.43 (m,

2H), 3.13 - 2.95 (m, 3H), 2.67 - 2.58 (m, 3H), 2.40 (s, 3H), 2.12 - 2.00 (m, 4H), 1.84 - 1.75 (m, 2H). LCMS (ESI) calcd. for $C_{24}H_{28}N_3O_2S_2^+$ [M+H]$^+$: 454.16, found, 454.1.

**Example 236: Preparation of 3-(5-((4-(furan-3-yl)piperidin-1-yl)methyl)-1-thioxoisoindolin-2-yl)piperidine-2,6-dione (GT-08326)**

With reference to the method of Scheme 4, the target compound GT-08326 was obtained (5 mg, white solid, yield 10%). $^1$H NMR (400 MHz, DMSO-d6) δ 11.12 (s, 1H), 10.15 (s, 1H), 7.99 (d, *J* = 7.8 Hz, 1H), 7.87 (s, 1H), 7.77 - 7.74 (m, 1H), 7.60 (s, 1H), 7.50 (s, 1H), 6.42 (s, 1H), 6.02 - 5.91 (m, 1H), 4.88 (d, *J* = 19.5 Hz, 1H), 4.75 (d, *J* = 20.1 Hz, 1H), 4.46 (d, *J* = 3.1 Hz, 2H), 3.47 - 3.42 (m, 2H), 3.13 - 2.99 (m, 3H), 2.71 - 2.66 (m, 2H), 2.10 - 2.00 (m, 4H), 1.81 - 1.71 (m, 2H). LCMS (ESI) calcd. for $C_{23}H_{26}N_3O_3S^+$ [M+H]$^+$: 424.17, found, 424.1.

**Example 237: Preparation of 3-(5-((4-phenylpiperazin-1-yl)methyl)-1-thioxoisoindolin-2-yl)piperidine-2,6-dione (GT-08205)**

With reference to the method of Scheme 4, the target compound GT-08205 was obtained (11 mg, white solid, yield 21%). $^1$H NMR (400 MHz, DMSO-d6) δ 11.42 (s, 1H), 11.15 (s, 1H), 7.99 - 7.95 (m, 2H), 7.84 - 7.81 (m, 1H), 7.32 - 7.20 (m, 2H), 6.97 (d, *J* = 8.1 Hz, 2H), 6.86 (t, *J* = 7.3 Hz, 1H), 5.96 (d, *J* = 13.0 Hz, 1H), 4.89 (d, *J* = 20.4 Hz, 1H), 4.76 (d, *J* = 20.0 Hz, 1H), 4.53 (s, 2H), 3.87 - 3.81 (m, 2H), 3.46 - 3.32 (m, 2H), 3.18 (s, 4H), 3.04 - 2.89 (m, 1H), 2.65 (d, *J* = 17.5 Hz, 1H), 2.55 - 2.51(m, 1H), 2.13 - 2.09 (m, 1H). LCMS (ESI) calcd. for $C_{24}H_{27}N_4O_2S^+$ [M+H]$^+$: 435.18, found, 435.1.

**Example 238: Preparation** of **3-(5-((4-(2-methoxyphenyl)piperazin-1-yl)methyl)-1-thioxoisoindolin-2-yl)piperidine-2,6-dione (GT-08236)**

With reference to the method of Scheme 4, the target compound GT-08236 was obtained (5 mg, white solid, yield 10%). LCMS (ESI) calcd. for $C_{25}H_{29}N_4O_3S^+$ [M+H]$^+$: 465.20, found, 465.2.

**Example 239: Preparation** of **3-(5-((4-(3-methoxyphenyl)piperazin-1-yl)methyl)-1-thioxoisoindolin-2-yl)piperidine-2,6-dione (GT-08237)**

With reference to the method of Scheme 4, the target compound GT-08237 was obtained (4 mg, white solid, yield 8%). $^1$H NMR (400 MHz, DMSO-d6) δ 11.22 (s, 1H), 11.14 (s, 1H), 8.02 - 7.93 (m, 2H), 7.80 (d, *J* = 7.8 Hz, 1H), 7.15 (t, *J* = 8.2 Hz, 1H), 6.55 (dd, *J* = 8.3, 2.1 Hz, 1H), 6.52 - 6.47 (m, 1H), 6.44 (dd, *J* = 8.1, 2.0 Hz, 1H), 5.96 (d, *J* = 8.7 Hz, 1H), 4.89 (d, *J* = 20.3 Hz, 1H), 4.74 (d, *J* = 20.4 Hz, 1H), 4.52 (s, 2H), 3.81 (d, *J* = 9.6 Hz, 2H), 3.72 (s, 3H), 3.40 - 3.32 (m, 2H), 3.21 - 3.14 (m, 4H), 3.01 - 2.94 (m, 1H), 2.65 (d, *J* = 16.4 Hz, 1H), 2.60 - 2.52 (m, 1H), 2.15 - 2.04 (m, 1H). LCMS (ESI) calcd. for $C_{25}H_{29}N_4O_3S^+$ [M+H]$^+$: 465.20, found, 465.2.

**Example 240: Preparation of 3-(1-thioxo-5-((4-(4-(trifluoromethyl)phenyl)piperazin-1-yl)methyl)isoindolin-2-yl) piperidine-2,6-dione (GT-08239)**

With reference to the method of Scheme 4, the target compound GT-08239 was obtained (5 mg, white solid, yield 10%). $^1$H NMR (400 MHz, DMSO-d6) δ 11.15 (s, 1H), 10.53 (s, 1H), 8.04 - 7.93 (m, 1H), 7.91 - 7.81 (m, 1H), 7.79 - 7.73 (m, 1H), 7.57 (d, *J* = 8.6 Hz, 2H), 7.14 (dd, *J* = 8.3 Hz, 2H), 5.97 (d, *J* = 17.1 Hz, 1H), 4.88 (d, *J* = 20.1 Hz, 1H), 4.75 (d, *J* = 20.7 Hz, 1H), 4.58 - 4.51 (m, 2H), 4.11 - 3.90 (m, 2H), 3.46 - 3.42 (m, 2H), 3.27 - 3.08 (m, 4H), 3.02 - 2.94 (m, 1H), 2.65 (d, *J* = 14.7 Hz, 1H), 2.45 - 2.37 (m, 1H), 2.18 - 2.02 (m, 1H). LCMS (ESI) calcd. for $C_{25}H_{26}F_3N_4O_2S^+$ [M+H]$^+$: 503.17, found, 503.1.

**Example** 241: **Preparation** of **3-(5-((4-(4-fluorophenyl)piperazin-1-yl)methyl)-1-thioxoisoindolin-2-yl)piperidine-2,6-dione (GT-08240)**

With reference to the method of Scheme 4, the target compound GT-08240 was obtained (7 mg, white solid, yield 13%). $^1$H NMR (400 MHz, DMSO-d6) δ 11.15 (s, 1H), 8.01 - 7.94 (m, 1H), 7.92 - 7.88 (m, 1H), 7.82 (t, *J* = 9.0 Hz, 1H), 7.11 - 7.07 (m, 2H), 7.03 - 6.93 (m, 2H), 5.96 (d, *J*= 11.4 Hz, 1H), 4.89 (d, *J* = 20.6 Hz, 1H), 4.75 (d, *J* = 20.3 Hz, 1H), 4.60 - 4.53 (m, 2H), 3.55 - 3.51 (m, 2H), 3.45 - 3.32 (m, 2H), 3.18 - 3.03 (m, 4H), 3.02 - 2.92 (m, 2H), 2.73 - 2.64 (m, 1H), 2.62 - 2.54 (m, 1H), 2.20 - 2.02 (m, 1H). LCMS (ESI) calcd. for $C_{24}H_{26}FN_4O_2S^+$ [M+H]$^+$: 453.18, found, 453.1.

**Example 242: Preparation of 3-(5-((4-(2-chlorophenyl)piperazin-1-yl)methyl)-1-thioxoisoindolin-2-yl)piperidine-2,6-dione (GT-08241)**

With reference to the method of Scheme 4, the target compound GT-08241 was obtained (3 mg, white solid, yield 6%). $^1$H NMR (400 MHz, DMSO-d6) δ 11.14 (s, 1H), 10.91 (s, 1H), 8.03 - 7.86 (m, 2H), 7.86 - 7.77 (m, 1H), 7.48 - 7.42 (m, 1H), 7.37 - 7.31 (m, 1H), 7.25 - 7.17 (m, 1H), 7.14 - 7.03 (m, 1H), 5.96 (d, *J* = 12.3 Hz, 1H), 4.88 (d, *J* = 22.0 Hz, 1H), 4.74 (d, *J* = 20.8 Hz, 1H), 4.64 - 4.52 (m, 2H), 3.52 - 3.40 (m, 4H), 3.24 - 3.07 (m, 4H), 3.05 - 2.98 (m, 1H), 2.67 - 2.63 (m, 1H), 2.58 - 2.54 (m, 1H), 2.15 - 2.04 (m, 1H). LCMS (ESI) calcd. for $C_{24}H_{26}ClN_4O_2S^+$ [M+H]$^+$: 469.15, found, 469.1.

**Example 243: Preparation of 3-(5-((4-(2-bromophenyl)piperazin-1-yl)methyl)-1-thioxoisoindolin-2-yl)piperidine-2,6-dione (GT-08242)**

With reference to the method of Scheme 4, the target compound GT-08242 was obtained (7 mg, white solid, yield 14%). $^1$H NMR (400 MHz, DMSO-d6) δ 11.14 (s, 1H), 10.93 (s, 1H), 8.01 - 7.94 (m, 2H), 7.82 (t, *J* = 9.2 Hz, 1H), 7.63 (d, *J* = 7.5 Hz, 2H), 7.41 - 7.37 (m, 1H), 7.20 (d, *J* = 7.4 Hz, 1H), 7.05 (t, *J* = 7.9 Hz, 1H), 5.97 (d, *J* = 15.2 Hz, 1H), 4.89 (d, *J* = 20.0 Hz, 1H), 4.75 (d, *J* = 20.7 Hz, 1H), 4.67 - 4.51 (m, 2H), 3.47 (d, *J* = 6.2 Hz, 2H), 3.19 - 3.12 (m, 4H), 3.03 - 2.87 (m, 3H), 2.67 - 2.63 (m, 1H), 2.59 - 2.52 (m, 1H), 2.14 -2.06 (m, 1H). LCMS (ESI) calcd. for $C_{24}H_{26}BrN_4O_2S^+$ [M+H]$^+$: 513.10, found, 513.1.

**Example 244: Preparation of 3-(5-((4-(3-bromophenyl)piperazin-1-yl)methyl)-1-thioxoisoindolin-2-yl)piperidine-2,6-dione (GT-08238)**

With reference to the method of Scheme 4, the target compound GT-08238 was obtained (5 mg, white solid, yield 10%). $^1$H NMR (400 MHz, DMSO-d6) δ 11.14 (s, 1H), 10.61 (s, 1H), 8.00 (d, *J* = 8.6 Hz, 1H), 7.90 - 7.85 (m, 2H), 7.77 (t, *J* = 8.6 Hz, 1H), 7.25 - 7.13 (m, 3H), 5.96 (d, *J* = 8.6 Hz, 1H), 4.88 (d, *J* = 20.2 Hz, 1H), 4.75 (d, *J* = 18.1 Hz, 1H), 4.54 (s, 2H), 3.96 - 3.82 (m, 2H), 3.18 - 3.12 (m, 5H), 3.01 - 2.95 (m, 2H), 2.67 - 2.62 (m, 1H), 2.60 - 2.55 (m, 1H), 2.13 - 2.05 (m, 1H). LCMS (ESI) calcd. for $C_{24}H_{26}BrN_4O_2S^+$ [M+H]$^+$: 513.10, found, 513.1.

**Example 245: Preparation of 3-(5-((4-(4-bromophenyl)piperazin-1-yl)methyl)-1-thioxoisoindolin-2-yl)piperidine-2,6-dione (GT-08600)**

With reference to the method of Scheme 4, the target compound GT-08600 was obtained (11 mg, white solid, yield 18%). $^1$H NMR (400 MHz, DMSO-d6) δ 11.42 (s, 1H), 11.15 (s, 1H), 7.97 (d, *J* = 8.0 Hz, 2H), 7.81 (d, *J* = 7.9 Hz, 1H), 7.40 (d, *J* = 8.9 Hz, 2H), 6.95 (t, *J* = 6.2 Hz, 2H), 5.96 (d, *J* = 7.9 Hz, 1H), 4.88 (d, *J* = 20.4 Hz, 1H), 4.74 (d, *J* = 20.3 Hz, 1H), 4.52 (s, 2H), 3.80 (d, *J* = 8.5 Hz, 2H), 3.37 - 3.28 (m, 2H), 3.17 (brs, 4H), 3.04 - 2.92 (m, 1H), 2.65 (d, *J* = 17.4 Hz, 1H), 2.58 - 2.55 (m, 1H), 2.16 - 2.09 (m, 1H). LCMS (ESI) calcd. for $C_{24}H_{26}BrN_4O_2S^+$ [M+H]$^+$: 513.10, found, 513.1.

**Example 246: Preparation of 3-(1-thioxo-5-((4-(o-tolyl)piperazin-1-yl)methyl)isoindolin-2-yl)piperidine-2,6-dione (GT-08601)**

With reference to the method of Scheme 4, the target compound GT-08601 was obtained (15 mg, white solid, yield 28%). $^1$H NMR (400 MHz, DMSO-d6) δ 11.14 (s, 1H), 11.00 (s, 1H), 8.02 - 7.89 (m, 2H), 7.83 (t, *J* = 8.2 Hz, 1H), 7.18 (t, *J* = 8.1 Hz, 2H), 7.07 - 6.97 (m, 2H), 5.97 (d, *J* = 9.1 Hz, 1H), 4.89 (d, *J* = 20.3 Hz, 1H), 4.75 (d, *J* = 20.*3* Hz, 1H), 4.56 (d, *J* = 4.5 Hz, 2H), 3.40 (d, *J* = 10.8 Hz, 2H), 3.33 - 3.22 (m, 2H), 3.22 - 3.06 (m, 4H), 3.03 - 2.95 (m, 1H), 2.65 (d, *J* = 16.1 Hz, 1H), 2.59 - 2.56 (m, 1H), 2.25 (s, 3H), 2.15 - 2.05 (m, 1H). LCMS (ESI) calcd. for $C_{25}H_{29}N_4O_2S^+$ [M+H]$^+$: 449.20, found, 449.2.

**Example 247: Preparation of 3-(1-thioxo-5-((4-(m-tolyl)piperazin-1-yl)methyl)isoindolin-2-yl)piperidine-2,6-dione (GT-08602)**

With reference to the method of Scheme 4, the target compound GT-08602 was obtained (15 mg, white solid, yield 28%). $^1$H NMR (400 MHz, DMSO-d6) δ 11.28 (s, 1H), 11.14 (s, 1H), 7.99 - 7.90 (m, 2H), 7.83 (t, *J*= 8.4 Hz, 1H), 7.13 (t, *J*= 7.8 Hz, 1H), 6.84 - 6.72 (m, 2H), 6.68 (d, *J* = 7.4 Hz, 1H), 5.96 (d, *J*= 9.5 Hz, 1H), 4.89 (d, *J= 20.3* Hz, 1H), 4.74 (d, *J*= 20.3 Hz, 1H), 4.52 (s, 2H), 3.60 - 3.50 (m, 1H), 3.45 - 3.32 (m, 2H), 3.23 - 3.09 (m, 5H), 3.04 - 2.91 (m, 1H), 2.65 (d, *J*= 17.2 Hz, 1H), 2.59 - 2.55 (m, 1H), 2.26 (s, 3H), 2.16 - 2.06 (m, 1H). LCMS (ESI) calcd. for $C_{25}H_{29}N_4O_2S^+$ [M+H]$^+$: 449.20, found, 449.2.

**Example 248: Preparation of 3-(1-thioxo-5-((4-(p-tolyl)piperazin-1-yl)methyl)isoindolin-2-yl)piperidine-2,6-dione (GT-08603)**

With reference to the method of Scheme 4, the target compound GT-08603 was obtained (14 mg, white solid, yield 26%). $^1$H NMR (400 MHz, DMSO-d6) δ 11.23 (s, 1H), 11.14 (s, 1H), 8.05 - 7.89 (m, 2H), 7.82 (t, *J* = 8.5 Hz, 1H), 7.07 (d, *J* = 8.4 Hz, 2H), 6.87 (d, *J* = 8.5 Hz, 2H), 5.96 (d, *J* = 8.8 Hz, 1H), 4.89 (d, *J*= 20.4 Hz, 1H), 4.74 (d, *J* = 20.3 Hz, 1H), 4.52 (s, 2H), 3.72 (d, *J* = 12.2 Hz, 2H), 3.39 (d, *J* = 11.2 Hz, 2H), 3.27 - 3.05 (m, 4H), 3.04 - 2.91 (m, 1H), 2.65 (d, *J* = 17.1 Hz, 1H), 2.57 - 2.51 (m, 1H), 2.21 (s, 3H), 2.16 - 2.04 (m, 1H). LCMS (ESI) calcd. for $C_{25}H_{29}N_4O_2S^+$ $[M+H]^+$: 449.20, found, 449.2.

**Example 249: Preparation of 3-(5-((4-(2,4-dichlorophenyl)piperazin-1-yl)methyl)-1-thioxoisoindolin-2-yl)piperidine-2,6-dione (GT-08604)**

With reference to the method of Scheme 4, the target compound GT-08604 was obtained (13 mg, white solid, yield 22%). $^1$H NMR (400 MHz, DMSO-d6) δ 11.29 (s, 1H), 11.14 (s, 1H), 7.95 (t, *J* = 15.9 Hz, 2H), 7.83 (d, *J* = 7.8 Hz, 1H), 7.60 (s, 1H), 7.41 (dd, *J* = 8.6, 2.2 Hz, 1H), 7.21 (d, *J* = 8.6 Hz, 1H), 5.96 (d, *J*= 8.5 Hz, 1H), 4.89 (d, *J* = 20.3 Hz, 1H), 4.74 (d, *J* = 20.4 Hz, 1H), 4.56 (s, 2H), 3.50 - 3.38 (m, 4H), 3.25 - 3.17 (m, 4H), 3.05 - 2.91 (m, 1H), 2.65 (d, *J* = 17.3 Hz, 1H), 2.61 - 2.53 (m, 1H), 2.18 - 2.04 (m, 1H). LCMS (ESI) calcd. for $C_{24}H_{25}Cl_2N_4O_2S^+$ $[M+H]^+$: 503.11, found, 503.1.

**Example 250: Preparation of 3-(5-((4-(2,5-dichlorophenyl)piperazin-1-yl)methyl)-1-thioxoisoindolin-2-yl)piperidine-2,6-dione (GT-08611)**

With reference to the method of Scheme 4, the target compound GT-08611 was obtained (11 mg, white solid, yield 19%). $^1$H NMR (400 MHz, DMSO-d6) δ 11.43 (s, 1H), 11.14 (s, 1H), 8.06 - 7.89 (m, 2H), 7.84 *(d, J*= 7.9 Hz, 1H), 7.48 (d, *J*= 8.5 Hz, 1H), 7.22 (d, *J* = 2.2 Hz, 1H), 7.18 *(d, J*= 8.5 Hz, 1H), 5.96 (d, *J* = 8.1 Hz, 1H), 4.89 (d, *J* = 20.4 Hz, 1H), 4.75 (d, *J* = 20.4 Hz, 1H), 4.56 (s, 2H), 3.51 - 3.42 (m, 4H), 3.28 - 3.17 (m, 4H), 3.06 - 2.91 (m, 1H), 2.65 (d, *J*= 17.2 Hz, 1H), 2.62 - 2.53 (m, 1H), 2.18 - 2.03 (m, 1H). LCMS (ESI) calcd. for $C_{24}H_{25}Cl_2N_4O_2S^+$ $[M+H]^+$: 503.11, found, 503.1.

**Example 251: Preparation of 3-(5-((4-(3,4-dichlorophenyl)piperazin-1-yl)methyl)-1-thioxoisoindolin-2-yl)piperidine-2,6-dione (GT-08612)**

With reference to the method of Scheme 4, the target compound GT-08612 was obtained (10 mg, white solid, yield 17%). $^1$H NMR (400 MHz, DMSO-d6) δ 11.41 (s, 1H), 11.14 (s, 1H), 7.98 - 7.89 (m, 2H), 7.86 - 7.74 (m, 1H), 7.45 (d, *J* = 9.0 Hz, 1H), 7.22 (d, *J* = 2.7 Hz, 1H), 6.98 (dd, *J*= 9.0, 2.9 Hz, 1H), 5.96 (d, *J* = 8.5 Hz, 1H), 4.88 (d, *J* = 20.3 Hz, 1H), 4.74 (d, *J*= 20.4 Hz, 1H), 4.52 (s, 2H), 3.88 (d, *J* = 12.2 Hz, 2H), 3.48 - 3.39 (m, 2H), 3.29 - 3.11 (m, 4H), 3.03 - 2.93 (m, 1H), 2.65 (d, *J* = 17.1 Hz, 1H), 2.58 - 2.54 (m, 1H), 2.13 - 2.07 (m, 1H). LCMS (ESI) calcd. for $C_{24}H_{25}Cl_2N_4O_2S^+$ $[M+H]^+$: 503.11, found, 503.1.

**Example 252: Preparation of 3-(5-((4-(3,5-dichlorophenyl)piperazin-1-yl)methyl)-1-thioxoisoindolin-2-yl)piperidine-2,6-dione (GT-08624)**

With reference to the method of Scheme 4, the target compound GT-08624 was obtained (10 mg, white solid, yield 17%). $^1$H NMR (400 MHz, DMSO-d6) δ 11.34 (s, 1H), 11.14 (s, 1H), 8.07 - 7.90 (m, 2H), 7.79 (d, *J* = 6.7 Hz, 1H), 7.02 (s, 2H), 6.96 (s, 1H), 5.96 (d, *J* = 9.2 Hz, 1H), 4.88 (d, *J* = 20.4 Hz, 1H), 4.74 (d, *J* = 20.2 Hz, 1H), 4.51 (s, 2H), 3.95 (d, *J* = 12.1 Hz, 2H), 3.30 - 3.26 (m, 4H), 3.20 - 3.10 (m, 2H), 3.04 - 2.90 (m, 1H), 2.65 (d, *J* = 16.9 Hz, 1H), 2.62 - 2.54 (m, 1H), 2.17 - 2.09 (m, 1H). LCMS (ESI) calcd. for $C_{24}H_{25}Cl_2N_4O_2S^+$ $[M+H]^+$: 503.11, found, 503.1.

**Example 253: Preparation of 3-(5-((4-(2,3-difluorophenyl)piperazin-1-yl)methyl)-1-thioxoisoindolin-2-yl)piperidine-2,6-dione (GT-08210)**

With reference to the method of Scheme 4, the target compound GT-08210 was obtained (5 mg, white solid, yield 9%). $^1$H NMR (400 MHz, DMSO-d6) δ 11.21 (s, 1H), 11.14 (s, 1H), 8.06 - 7.89 (m, 2H), 7.84 - 7.80 (m, 1H), 7.20 - 7.00 (m, 2H), 6.92 (t, *J* = 7.7 Hz, 1H), 5.96 (d, *J*= 8.4 Hz, 1H), 4.94 - 4.81 (m, 1H), 4.75 (d, *J* = 20.3 Hz, 1H), 4.55 (s, 2H), 3.52 (d, *J*= 10.0 Hz, 2H), 3.47 - 3.40 (m, 2H), 3.25 - 3.19 (m, 4H), 3.05 - 2.89 (m, 1H), 2.67 - 2.61 (m, 1H), 2.61 - 2.53 (m, 1H), 2.13 - 2.06 (m, 1H). LCMS (ESI) calcd. for $C_{24}H_{25}F_2N_4O_2S^+$ $[M+H]^+$: 471.17, found, 471.1.

**Example 254: Preparation of 3-(5-((4-(2,4-difluorophenyl)piperazin-1-yl)methyl)-1-thioxoisoindolin-2-yl)piperidine-2,6-dione (GT-08613)**

With reference to the method of Scheme 4, the target compound GT-08613 was obtained (12 mg, white solid, yield 22%). $^1$H NMR (400 MHz, DMSO-d6) δ 11.32 (s, 1H), 11.14 (s, 1H), 7.99 - 7.91 (m, 2H), 7.82 (d, *J*= 7.4 Hz, 1H), 7.27 - 7.22 (m, 1H), 7.20 - 7.09 (m, 1H), 7.03 (t, *J* = 7.3 Hz, 1H), 5.96 (d, *J* = 8.9 Hz, 1H), 4.88 (d, *J* = 20.4 Hz, 1H), 4.74 (d, *J* = 20.5 Hz, 1H), 4.53 (s, 2H), 3.51 - 3.46 (m, 4H), 3.27 - 3.18 (m, 4H), 3.03 - 2.91 (m, 1H), 2.65 (d, *J* = 17.5 Hz, 1H), 2.57 (d, *J* = 13.8 Hz, 1H), 2.16 - 2.01 (m, 1H). LCMS (ESI) calcd. for $C_{24}H_{25}F_2N_4O_2S^+$ [M+H]$^+$: 471.17, found, 471.1.

**Example 255: Preparation of 3-(5-((4-(2,5-difluorophenyl)piperazin-1-yl)methyl)-1-thioxoisoindolin-2-yl)piperidine-2,6-dione (GT-08625)**

With reference to the method of Scheme 4, the target compound GT-08625 was obtained (13 mg, white solid, yield 24%). $^1$H NMR (400 MHz, DMSO-d6) δ 11.19 (s, 1H), 11.14 (s, 1H), 8.03 - 7.86 (m, 2H), 7.80 (d, *J* = 7.6 Hz, 1H), 7.28 - 7.14 (m, 1H), 7.05 - 6.92 (m, 1H), 6.93 - 6.75 (m, 1H), 5.96 (d, *J* = 8.7 Hz, 1H), 4.88 (d, *J* = 20.4 Hz, 1H), 4.74 (d, *J* = 20.3 Hz, 1H), 4.54 (s, 2H), 3.53 (d, *J*= 10.3 Hz, 2H), 3.45 - 3.38 (m, 2H), 3.27 - 3.10 (m, 4H), 3.02 - 2.89 (m, 1H), 2.65 (d, *J*= 16.3 Hz, 1H), 2.57 - 2.51 (m, 1H), 2.18 - 2.09 (m, 1H). LCMS (ESI) calcd. for $C_{24}H_{25}F_2N_4O_2S^+$ [M+H]$^+$: 471.17, found, 471.1.

**Example 256: Preparation of 3-(5-((4-(2,6-difluorophenyl)piperazin-1-yl)methyl)-1-thioxoisoindolin-2-yl)piperidine-2,6-dione (GT-08626)**

With reference to the method of Scheme 4, the target compound GT-08626 was obtained (13 mg, white solid, yield 24%). $^1$H NMR (400 MHz, DMSO-d6) δ 11.20 (s, 1H), 11.14 (s, 1H), 7.99 - 7.91 (m, 2H), 7.82 (d, *J*= *7.7* Hz, 1H), 7.15 (d, *J*= 7.6 Hz, 1H), 7.08 (t, *J* = 8.4 Hz, 2H), 5.96 (d, *J*= 8.4 Hz, 1H), 4.89 (d, *J*= 20.3 Hz, 1H), 4.74 (d, *J* = 20.3 Hz, 1H), 4.54 (s, 2H), 3.62 - 3.48 (m, 2H), 3.43 - 3.40 (m, 2H), 3.29 - 3.25 (m, 2H), 3.24 - 3.10 (m, 2H), 3.05 - 2.90 (m, 1H), 2.65 (d, *J* = 17.3 Hz, 1H), 2.59 - 2.53 (m, 1H), 2.16 - 2.03 (m, 1H). LCMS (ESI) calcd. for $C_{24}H_{25}F_2N_4O_2S^+$ [M+H]$^+$: 471.17, found, 471.1.

**Example 257: Preparation of 3-(5-((4-(3,4-difluorophenyl)piperazin-1-yl)methyl)-1-thioxoisoindolin-2-yl)piperidine-2,6-dione (GT-08627)**

With reference to the method of Scheme 4, the target compound GT-08627 was obtained (14 mg, white solid, yield 25%). $^1$H NMR (400 MHz, DMSO-d6) δ 11.22 (s, 1H), 11.14 (s, 1H), 8.04 - 7.86 (m, 2H), 7.80 (d, *J*= *7.6* Hz, 1H), 7.30 (dd, *J*= 19.6, 9.6 Hz, 1H), 7.16 - 7.01 (m, 1H), 6.77 (d, *J*= 8.7 Hz, 1H), 5.96 (d, *J*= 7.9 Hz, 1H), 4.88 (d, *J* = 20.3 Hz, 1H), 4.74 (d, *J*= *20.3* Hz, 1H), 4.52 (s, 2H), 3.79 (d, *J*= 7.0 Hz, 2H), 3.49 - 3.38 (m, 2H), 3.22 - 3.11 (m, 4H), 3.05 - 2.90 (m, 1H), 2.65 (d, *J* = 16.6 Hz, 1H), 2.59 - 2.54 (m, 1H), 2.17 - 2.03 (m, 1H). LCMS (ESI) calcd. for $C_{24}H_{25}F_2N_4O_2S^+$ [M+H]$^+$: 471.17, found, 471.1.

**Example 258: Preparation of 3-(5-((4-(3,5-difluorophenyl)piperazin-1-yl)methyl)-1-thioxoisoindolin-2-yl)piperidine-2,6-dione (GT-08628)**

With reference to the method of Scheme 4, the target compound GT-08628 was obtained (13 mg, white solid, yield 24%). $^1$H NMR (400 MHz, DMSO-d6) δ 11.27 (s, 1H), 11.14 (s, 1H), 7.99 - 7.87 (m, 2H), 7.79 (d, *J* = 7.5 Hz, 1H), 6.70 (d, *J* = 9.4 Hz, 2H), 6.58 (t, *J* = 9.3 Hz, 1H), 5.96 (d, *J* = 8.7 Hz, 1H), 4.88 (d, *J* = 20.3 Hz, 1H), 4.74 (d, *J*= 20.3 Hz, 1H), 4.51 (s, 2H), 3.92 (d, *J*= 12.8 Hz, 2H), 3.49 - 3.37 (m, 2H), 3.24 (t, *J* = 12.6 Hz, 2H), 3.19 - 3.07 (m, 2H), 3.05 - 2.91 (m, 1H), 2.65 (d, *J* = 16.9 Hz, 1H), 2.59 - 2.54 (m, 1H), 2.13 - 2.06 (m, 1H). LCMS (ESI) calcd. for $C_{24}H_{25}F_2N_4O_2S^+$ [M+H]$^+$: 471.17, found, 471.1.

**Example 259: Preparation of 3-(5-((4-(2,4-dimethylphenyl)piperazin-1-yl)methyl)-1-thioxoisoindolin-2-yl)piperidine-2,6-dione (GT-08614)**

With reference to the method of Scheme 4, the target compound GT-08614 was obtained (11 mg, white solid, yield 20%). $^1$H NMR (400 MHz, DMSO-d6) δ 11.14 (s, 1H), 11.00 (s, 1H), 8.02 - 7.89 (m, 2H), 7.83 (t, *J* = 8.2 Hz, 1H), 7.00 (s, 1H), 6.97 (d, *J* = 8.5 Hz, 1H), 6.92 (dd, *J* = 8.1, 3.2 Hz, 1H), 5.96 (d, *J* = 8.8 Hz, 1H), 4.89 (d, *J* = 20.4 Hz, 1H), 4.75 (d, *J*= 20.5 Hz, 1H), 4.55 (d, *J* = 4.5 Hz, 2H), 3.37 - 3.32 (m, 2H), 3.28 - 3.19 (m, 2H), 3.14 - 3.10 (m, 4H), 3.01 - 2.88 (m, 1H), 2.68 - 2.61 (m, 1H), 2.55 - 2.51 (m, 1H), 2.21 (s, 6H), 2.21 - 2.07 (m, 1H). LCMS (ESI) calcd. for $C_{26}H_{31}N_4O_2S^+$ [M+H]$^+$: 463.22, found, 463.1.

**Example 260: Preparation of 3-(5-((4-(5-chloro-2-methylphenyl)piperazin-1-yl)methyl)-1-thioxoisoindolin-2-yl) piperidine-2,6-dione (GT-08615)**

With reference to the method of Scheme 4, the target compound GT-08615 was obtained (11 mg, white solid, yield 19%). $^1H$ NMR (400 MHz, DMSO-d6) δ 11.14 (s, 1H), 11.00 (s, 1H), 8.03 - 7.87 (m, 2H), 7.82 (t, *J* = 8.2 Hz, 1H), 7.21 (d, *J* = 8.1 Hz, 1H), 7.07 *(d, J* = 8.3 Hz, 1H), 7.03 (s, 1H), 5.97 (d, *J* = 9.1 Hz, 1H), 4.89 (d, *J* = 20.6 Hz, 1H), 4.75 (d, *J* = 20.2 Hz, 1H), 4.56 (s, 2H), 3.50 - 3.38 (m, 2H), 3.28 - 3.17 (m, 4H), 3.14 - 3.05 (m, 2H), 3.02 - 2.91 (m, 1H), 2.73 - 2.62 (m, 1H), 2.59 - 2.53 (m, 1H), 2.23 (s, 3H), 2.16 - 2.04 (m, 1H). LCMS (ESI) calcd. for $C_{25}H_{28}ClN_4O_2S^+$ [M+H]$^+$: 483.16, found, 483.1.

**Example 261: Preparation of 3-(5-((4-phenylpiperidin-1-yl)methyl)-1-thioxoisoindolin-2-yl)piperidine-2,6-dione (GT-08616)**

With reference to the method of Scheme 4, the target compound GT-08616 was obtained (5 mg, white solid, yield 10%). $^1H$ NMR (400 MHz, DMSO-d6) δ 11.14 (s, 1H), 10.48 (s, 1H), 7.99 (d, *J* = 8.0 Hz, 1H), 7.92 (s, 1H), 7.78 (d, *J* = 7.8 Hz, 1H), 7.33 (t, *J*= 7.2 Hz, 2H), 7.22 (d, *J* = 7.9 Hz, 3H), 5.97 (d, *J* = 14.2 Hz, 1H), 4.89 (d, *J* = 20.8 Hz, 1H), 4.75 (d, *J* = 20.2 Hz, 1H), 4.48 (d, *J* = 3.5 Hz, 2H), 3.50 - 3.44 (m, 2H), 3.31 - 3.24 (m, 2H), 3.16 - 2.91 (m, 4H), 2.86 - 2.74 (m, 1H), 2.69 - 2.54 (m, 3H), 2.15 - 2.09 (m, 1H). LCMS (ESI) calcd. for $C_{25}H_{28}N_3O_2S^+$ [M+H]$^+$: 434.19, found, 434.1.

**Example 262: Preparation of 3-(5-((4-(3-bromophenyl)piperidin-1-yl)methyl)-1-thioxoisoindolin-2-yl)piperidine-2,6-dione (GT-08617)**

With reference to the method of Scheme 4, the target compound GT-08617 was obtained (7 mg, white solid, yield 12%). $^1H$ NMR (400 MHz, DMSO-d6) δ 11.14 (s, 1H), 10.65 (s, 1H), 7.98 (d, *J*= 7.6 Hz, 1H), 7.93 (s, 1H), 7.79 (d, *J*= 7.5 Hz, 1H), 7.47 - 7.39 (m, 2H), 7.31 (t, *J*= 7.7 Hz, 1H), 7.24 (d, *J*= 7.5 Hz, 1H), 5.96 (d, *J* = 8.2 Hz, 1H), 4.89 (d, *J= 20.3* Hz, 1H), 4.75 (d, *J*= 20.6 Hz, 1H), 4.47 (s, 2H), 3.53 - 3.41 (m, 2H), 3.31 - 3.23 (m, 2H), 3.15 - 2.91 (m, 4H), 2.89 - 2.74 (m, 2H), 2.67 - 2.62 (m, 1H), 2.61 - 2.54 (m, 1H), 2.14 - 2.06 (m, 1H). LCMS (ESI) calcd. for $C_{25}H_{27}BrN_3O_2S^+$ [M+H]$^+$: 512.10, found, 512.0.

**Example 263: Preparation of 3-(5-((4-(4-bromophenyl)piperidin-1-yl)methyl)-1-thioxoisoindolin-2-yl)piperidine-2,6-dione (GT-08618)**

With reference to the method of Scheme 4, the target compound GT-08618 was obtained (8 mg, white solid, yield 13%). $^1H$ NMR (400 MHz, DMSO-d6) δ 11.14 (s, 1H), 10.72 (s, 1H), 8.06 - 7.85 (m, 2H), 7.79 (d, *J* = 8.0 Hz, 1H), 7.53 (t, *J*= 8.3 Hz, 2H), 7.19 (d, *J*= 8.4 Hz, 2H), 5.96 (d, *J*= 10.1 Hz, 1H), 4.88 (d, *J* = 20.3 Hz, 1H), 4.75 (d, *J* = 20.1 Hz, 1H), 4.47 (d, *J* = 3.8 Hz, 2H), 3.45 (d, *J*= 10.4 Hz, 2H), 3.15 - 2.90 (m, 3H), 2.84 - 2.79 (m, 1H), 2.65 (d, *J*= 17.3 Hz, 1H), 2.60 - 2.53 (m, 1H), 2.15 - 2.08 (m, 1H), 2.05 - 1.92 (m, 3H). LCMS (ESI) calcd. for $C_{25}H_{27}BrN_3O_2S^+$ [M+H]$^+$: 512.10, found, 512.0.

**Example 264: Preparation of 3-(5-((4-(4-chlorophenyl)piperidin-1-yl)methyl)-1-thioxoisoindolin-2-yl)piperidine-2,6-dione (GT-08619)**

With reference to the method of Scheme 4, the target compound GT-08619 was obtained (7 mg, white solid, yield 13%). $^1H$ NMR (400 MHz, DMSO-d6) δ 11.14 (s, 1H), 10.74 (s, 1H), 8.08 - 7.86 (m, 2H), 7.80 (t, *J* = 8.9 Hz, 1H), 7.39 (d, *J* = 8.4 Hz, 2H), 7.25 (d, *J* = 8.5 Hz, 2H), 5.96 (d, *J* = 7.8 Hz, 1H), 4.88 (d, *J* = 20.5 Hz, 1H), 4.75 (d, *J*= 20.2 Hz, 1H), 4.47 (s, 2H), 3.45 (d, *J* = 10.5 Hz, 2H), 3.16 - 2.91 (m, 3H), 2.88 - 2.76 (m, 1H), 2.65 (d, *J* = 16.9 Hz, 1H), 2.59 - 2.53 (m, 1H), 2.16 - 2.07 (m, 1H), 2.01 - 1.89 (m, 3H). LCMS (ESI) calcd. for $C_{25}H_{27}ClN_3O_2S^+$ [M+H]$^+$: 468.15, found, 468.1.

**Example 265: Preparation of 3-(5-((4-(2-fluorophenyl)piperidin-1-yl)methyl)-1-thioxoisoindolin-2-yl)piperidine-2,6-dione (GT-08620)**

With reference to the method of Scheme 4, the target compound GT-08620 was obtained (9 mg, white solid, yield 17%). $^1H$ NMR (400 MHz, DMSO-d6) δ 11.14 (s, 1H), 10.66 (s, 1H), 7.98 (d, *J*= 7.9 Hz, 1H), 7.92 (s, 1H), 7.78 (d, *J* = 7.7 Hz, 1H), 7.28 (t, *J*= 6.6 Hz, 2H), 7.22 - 7.15 (m, 2H), 5.96 (d, *J* = 8.6 Hz, 1H), 4.89 (d, *J* = 20.4 Hz, 1H), 4.75 (d, *J* = 20.3 Hz, 1H), 4.46 (s, 2H), 3.46 (d, *J*= 10.8 Hz, 2H), 3.20 - 3.04 (m, 4H), 3.04 - 2.91 (m, 1H), 2.65 (d, *J* = 16.7 Hz, 1H), 2.57 - 2.51 (m, 1H), 2.20 - 2.05 (m, 3H), 1.92 (d, *J* = 13.2 Hz, 2H). LCMS (ESI) calcd. for $C_{25}H_{27}FN_3O_2S^+$ [M+H]$^+$: 452.18, found, 452.1.

**Example 266: Preparation of 3-(5-((4-(4-fluorophenyl)piperidin-1-yl)methyl)-1-thioxoisoindolin-2-yl)piperidine-2,6-dione (GT-08621)**

With reference to the method of Scheme 4, the target compound GT-08621 was obtained (10 mg, white solid,

yield 19%). $^1$H NMR (400 MHz, DMSO-d6) δ 11.14 (s, 1H), 10.75 (s, 1H), 7.98 (d, *J*= 7.9 Hz, 1H), 7.94 (s, 1H), 7.79 (d, *J* = 8.4 Hz, 1H), 7.30 - 7.23 (m, 2H), 7.15 (t, *J* = 8.8 Hz, 2H), 5.96 (d, *J* = 8.6 Hz, 1H), 4.89 (d, *J* = 20.3 Hz, 1H), 4.75 (d, *J* = 20.3 Hz, 1H), 4.47 (d, *J* = 4.0 Hz, 1H), 3.45 (d, *J* = 10.7 Hz, 2H), 3.32 - 3.21 (m, 2H), 3.13 - 2.91 (m, 2H), 3.07 - 2.94 (m, 1H), 2.65 (d, *J* = 16.7 Hz, 1H), 2.62 - 2.53 (m, 1H), 2.16 - 2.07 (m, 1H), 2.02 - 1.989 (m, 3H). LCMS (ESI) calcd. for $C_{25}H_{27}FN_3O_2S^+$ [M+H]$^+$: 452.18, found, 452.1.

**Example 267: Preparation of 3-(1-thioxo-5-((4-(2-(trifluoromethyl)phenyl)piperidin-1-yl)methyl)isoindolin-2-yl) piperidine-2,6-dione (GT-08622)**

With reference to the method of Scheme 4, the target compound GT-08622 was obtained (8 mg, white solid, yield 14%). $^1$H NMR (400 MHz, DMSO-d6) δ 11.14 (s, 1H), 10.66 (s, 1H), 7.99 (d, *J*= 7.8 Hz, 1H), 7.94 (s, 1H), 7.80 (d, *J* = 7.6 Hz, 1H), 7.65 - 7.53 (m, 4H), 5.96 (d, *J* = 8.3 Hz, 1H), 4.89 (d, *J*= 20.5 Hz, 1H), 4.75 (d, *J* = 20.2 Hz, 1H), 4.49 (d, *J* = 3.7 Hz, 2H), 3.48 (d, *J*= 13.9 Hz, 2H), 3.18 - 3.03 (m, 3H), 3.03 - 2.89 (m, 3H), 2.65 (d, *J* = 16.3 Hz, 2H), 2.60 - 2.54 (m, 1H), 2.16 - 1.99 (m, 5H). LCMS (ESI) calcd. for $C_{26}H_{27}F_3N_3O_2S^+$ [M+H]$^+$: 502.18, found, 502.1.

**Example 268: Preparation of 3-(1-thioxo-5-((4-(3-(trifluoromethyl)phenyl)piperidin-1-yl)methyl)isoindolin-2-yl) piperidine-2,6-dione (GT-08623)**

With reference to the method of Scheme 4, the target compound GT-08623 was obtained (9 mg, white solid, yield 15%). $^1$H NMR (400 MHz, DMSO-d6) δ 11.14 (s, 1H), 10.78 (s, 1H), 7.99 (d, *J*= 8.1 Hz, 1H), 7.91 (s, 1H), 7.78 (d, *J*= 7.8 Hz, 1H), 7.70 (d, *J* = 7.7 Hz, 2H), 7.54 (d, *J= 7.6* Hz, 1H), 7.46 (t, *J* = 7.3 Hz, 1H), 5.97 (d, *J* = 8.7 Hz, 1H), 4.88 (d, *J* = 20.5 Hz, 1H), 4.75 (d, *J* = 20.3 Hz, 1H), 4.47 (s, 2H), 3.46 (d, *J*= 10.8 Hz, 2H), 3.21 - 3.07 (m, 3H), 3.01 - 2.93 (m, 1H), 2.65 (d, *J*= 16.5 Hz, 1H), 2.56 - 2.51 (m, 1H), 2.27 - 2.19 (m, 2H), 2.11 - 2.07 (m, 1H), 1.87 (d, *J* = 12.6 Hz, 2H). LCMS (ESI) calcd. for $C_{26}H_{27}F_3N_3O_2S^+$ [M+H]$^+$: 502.18, found, 502.1.

**Example 269: Preparation of 3-(5-((4-(2-chloro-3-fluorophenyl)piperidin-1-yl)methyl)-1-thioxoisoindolin-2-yl) piperidine-2,6-dione (GT-08330)**

With reference to the method of Scheme 4, the target compound GT-08330 was obtained (4 mg, white solid, yield 7%). $^1$H NMR (400 MHz, DMSO-d6) δ 11.14 (s, 1H), 10.57 (s, 1H), 7.99 (d, *J*= 7.9 Hz, 1H), 7.90 (s, 1H), 7.77 (d, *J* = 7.3 Hz, 1H), 7.45 - 7.38 (m, 1H), 7.35 - 7.32 (m, 1H), 7.17 (d, *J* = 9.4 Hz, 1H), 6.04 - 5.89 (m, 1H), 4.89 (d, *J*= 21.0 Hz, 1H), 4.75 (d, *J*= 20.2 Hz, 1H), 4.48 (s, 2H), 3.49 - 3.46 (m, 2H), 3.21 - 3.15 (m, 3H), 2.97 - 2.94 (m, 1H), 2.67 - 2.63 (m, 2H), 2.12 - 1.95 (m, 5H). LCMS (ESI) calcd. for $C_{25}H_{26}ClFN_3O_2S^+$ [M+H]$^+$: 486.14, found, 486.1.

**Example 270: Preparation of 3-(5-((4-(2,3-difluorophenyl)piperidin-1-yl)methyl)-1-thioxoisoindolin-2-yl)piperidine-2,6-dione (GT-08331)**

With reference to the method of Scheme 4, the target compound GT-08331 was obtained (5 mg, white solid, yield 9%). $^1$H NMR (400 MHz, DMSO-d6) δ 11.14 (s, 1H), 10.45 (s, 1H), 7.99 (d, *J*= 8.0 Hz, 1H), 7.90 (s, 1H), 7.77 (d, *J*= 8.3 Hz, 1H), 7.37 - 7.28 (m, 1H), 7.24 - 7.18 (m, 1H), 7.12 - 7.06 (m, 1H), 6.08 - 5.86 (m, 1H), 4.89 (d, *J*= 20.0 Hz, 1H), 4.75 (d, *J* = 21.1 Hz, 1H), 4.53 - 4.41 (m, 2H), 3.51 - 3.43 (m, 2H), 3.16 - 3.09 (m, 3H), 2.98 - 2.94 (m, 1H), 2.67 - 2.63 (m, 2H), 2.13 - 2.04 (m, 3H), 1.99 - 1.94 (m, 2H). LCMS (ESI) calcd. for $C_{25}H_{26}F_2N_3O_2S^+$ [M+H]$^+$: 470.17, found, 470.1.

**Example 271: Preparation of 3-(5-((4-(2-chloro-3-fluorophenyl)-3,6-dihydropyridin-1(2H)-yl)methyl)-1-thioxoisoindolin-2-yl)piperidine-2,6-dione (GT-08206)**

With reference to the method of Scheme 4, the target compound GT-08206 was obtained (6 mg, white solid, yield 11%). $^1$H NMR (400 MHz, DMSO-d6) δ 11.15 (s, 1H), 11.00 (s, 1H), 8.06 - 7.90 (m, 2H), 7.82 (d, *J* = 8.0 Hz, 1H), 7.41 (t, *J*= 6.4 Hz, 2H), 7.14 (dd, *J*= 6.0, 2.5 Hz, 1H), 5.97 (d, *J*= 8.9 Hz, 1H), 5.78 (s, 1H), 4.89 (d, *J* = 20.5 Hz, 1H), 4.75 (d, *J* = 20.6 Hz, 1H), 4.59 (s, 2H), 3.91 - 3.70 (m, 2H), 3.65 - 3.60 (m, 1H), 3.30 - 3.19 (m, 2H), 3.02 - 2.95 (m, 1H), 2.94 - 2.78 (m, 1H), 2.65 (d, *J* = 16.2 Hz, 1H), 2.59 - 2.54 (m, 1H), 2.13 - 2.06 (m, 1H). LCMS (ESI) calcd. for $C_{25}H_{24}ClFN_3O_2S^+$ [M+H]$^+$: 484.13, found, 484.1.

**Example 272: Preparation of 3-(5-((4-(3,4-dichloropyridin-2-yl)piperidin-1-yl)methyl)-1-thioxoisoindolin-2-yl)piperidine-2,6-dione (GT-08488)**

With reference to the method of Scheme 4, the target compound GT-08488 was obtained (13 mg, white solid, yield 22%). $^1$H NMR (400 MHz, DMSO-d6) δ 11.15 (s, 1H), 11.00 (s, 1H), 8.48 (d, *J*= 5.2 Hz, 1H), 8.00 - 7.94 (m, 1H), 7.91 -

7.87 (m, 1H), 7.82 (t, $J$ = 9.0 Hz, 1H), 7.65 (d, $J$ = 5.2 Hz, 1H), 5.96 (d, $J$ = 10.8 Hz, 1H), 4.89 (d, $J$ = 20.4 Hz, 1H), 4.75 (d, $J$ = 20.3 Hz, 1H), 4.53 - 4.45 (m, 2H), 3.51 - 3.43 (m, 4H), 3.19 - 3.11 (m, 2H), 3.05 - 2.92 (m, 1H), 2.65 (d, $J$= 17.1 Hz, 1H), 2.24 - 2.11 (m, 4H), 1.97 (d, $J$ = 13.8 Hz, 2H). LCMS (ESI) calcd. for $C_{24}H_{25}Cl_2N_4O_2S^+$ [M+H]$^+$: 503.11, found, 503.1.

**Example 273: Preparation of 3-(5-((4-(2,3-dichloropyridin-4-yl)piperidin-1-yl)methyl)-1-thioxoisoindolin-2-yl)piperidine-2,6-dione (GT-08475)**

With reference to the method of Scheme 4, the target compound GT-08475 was obtained (9 mg, white solid, yield 15%). $^1$H NMR (400 MHz, DMSO-d6) δ 11.19 (s, 1H), 11.15 (s, 1H), 8.39 (d, $J$ = 5.0 Hz, 1H), 8.04 - 7.91 (m, 2H), 7.80 (d, $J$= 8.2 Hz, 1H), 7.37 (d, $J$= 5.1 Hz, 1H), 5.96 (d, $J$ = 8.9 Hz, 1H), 4.89 (d, $J$ = 20.3 Hz, 1H), 4.75 (d, $J$ = 20.3 Hz, 1H), 4.47 (d, $J$ = 4.5 Hz, 2H), 3.61 - 3.56 (m, 2H), 3.30 (t, $J$ = 12.0 Hz, 1H), 3.20 - 3.12 (m, 2H), 3.05 - 2.90 (m, 1H), 2.65 (d, $J$ = 17.3 Hz, 1H), 2.61 - 2.54 (m, 1H), 2.20 - 2.05 (m, 3H), 2.04 - 1.90 (m, 2H). LCMS (ESI) calcd. for $C_{24}H_{25}Cl_2N_4O_2S^+$ [M+H]$^+$: 503.11, found, 503.1.

**Example 274: Preparation of 3-(5-((4-(2,3-difluoropyridin-4-yl)piperidin-1-yl)methyl)-1-thioxoisoindolin-2-yl)piperidine-2,6-dione (GT-08473)**

With reference to the method of Scheme 4, the target compound GT-08473 was obtained (7 mg, white solid, yield 13%). $^1$H NMR (400 MHz, DMSO-d6) δ 11.14 (s, 1H), 10.83 (s, 1H), 8.04 (d, $J$ = 5.1 Hz, 1H), 7.98 (d, $J$= 7.8 Hz, 1H), 7.92 (s, 1H), 7.79 (d, $J$ = 8.1 Hz, 1H), 7.29 (t, $J$ = 4.7 Hz, 1H), 5.96 (d, $J$ = 9.6 Hz, 1H), 4.89 (d, $J$ = 20.5 Hz, 1H), 4.75 (d, $J$= 20.4 Hz, 1H), 4.47 (d, $J$= 4.2 Hz, 2H), 3.48 (d, $J$= 11.0 Hz, 2H), 3.28 - 3.20 (m, 2H), 3.20 - 3.06 (m, 2H), 3.04 - 2.90 (m, 1H), 2.65 (d, $J$ = 16.7 Hz, 1H), 2.60 - 2.54 (m, 1H), 2.18 - 2.11 (m, 3H), 2.04 - 1.93 (m, 2H). LCMS (ESI) calcd. for $C_{25}H_{25}F_2N_4O_2S^+$ [M+H]$^+$: 471.17, found, 471.1.

**Example 275: Preparation of 3-(5-((4,5-dichloro-3',6'-dihydro-[3,4'-bipyridin]-1'(2'H)-yl)methyl)-1-thioxoisoindolin-2-yl)piperidine-2,6-dione (GT-08489)**

With reference to the method of Scheme 4, the target compound GT-08489 was obtained (17 mg, white solid, yield 29%). $^1$H NMR (400 MHz, DMSO-d6) δ 11.55 (s, 1H), 11.15 (s, 1H), 8.75 (d, $J$ = 1.0 Hz, 1H), 8.40 (s, 1H), 8.06 - 7.83 (m, 3H), 6.33 (s, 1H), 5.91 (s, 1H), 4.89 (d, $J$= 20.3 Hz, 1H), 4.75 (d, $J$ = 20.3 Hz, 1H), 4.69 - 4.51 (m, 2H), 3.81 - 3.78 (m, 2H), 3.70 - 3.53 (m, 1H), 3.35 - 3.16 (m, 1H), 3.05 - 2.81 (m, 3H), 2.67 - 2.51 (m, 3H), 2.22 - 2.01 (m, 1H). LCMS (ESI) calcd. for $C_{24}H_{23}Cl_2N_4O_2S^+$ [M+H]$^+$: 501.19, found, 501.1.

**Example 276: Preparation of 3-(5-((5-chloro-3',6'-dihydro-[3,4'-bipyridin]-1'(2'H)-yl)methyl)-1-thioxoisoindolin-2-yl)piperidine-2,6-dione (GT-08490)**

With reference to the method of Scheme 4, the target compound GT-08490 was obtained (17 mg, white solid, yield 31%). $^1$H NMR (400 MHz, DMSO-d6) δ 11.30 (s, 1H), 11.15 (s, 1H), 8.69 (d, $J$ = 2.0 Hz, 1H), 8.59 (d, $J$ = 2.2 Hz, 1H), 8.06 (s, 1H), 7.92 (d, $J$ = 10.2 Hz, 1H), 7.90 - 7.82 (m, 1H), 6.40 (s, 1H), 5.97 (d, $J$= 11.5 Hz, 1H), 4.89 (d, $J$= *20.4* Hz, 1H), 4.75 (d, $J$= 20.2 Hz, 1H), 4.67 - 4.50 (m, 2H), 4.05 - 3.90 (m, 2H), 3.70 - 3.57 (m, 1H), 3.32 - 3.19 (m, 1H), 3.05 - 2.78 (m, 3H), 2.67 - 2.51 (m, 2H), 2.22 - 2.04 (m, 1H). LCMS (ESI) calcd. for $C_{24}H_{24}ClN_4O_2S^+$ [M+H]$^+$: 467.13, found, 467.1.

**Example 277: Preparation of 3-(5-((2',3'-dichloro-3,6-dihydro-[4,4'-bipyridin]-1(2H)-yl)methyl)-1-thioxoisoindolin-2-yl)piperidine-2,6-dione (GT-08482)**

With reference to the method of Scheme 4, the target compound GT-08482 was obtained (15 mg, white solid, yield 26%). $^1$H NMR (400 MHz, DMSO-d6) δ 11.47 (s, 1H), 11.14 (s, 1H), 8.40 (d, $J$= 4.9 Hz, 1H), 7.98 (d, $J$ = 7.7 Hz, 2H), 7.88 - 7.81 (m, 1H), 7.35 (dd, $J$ = 4.9, 2.8 Hz, 1H), 6.03 - 5.85 (m, 2H), 4.89 (d, $J$ = 20.3 Hz, 1H), 4.75 (d, $J$= 20.5 Hz, 1H), 4.59 (brs, 2H), 3.91 - 3.71 (m, 2H), 3.61 (brs, 1H), 3.25 - 3.20 (m, 1H), 3.02 - 2.93 (m, 2H), 2.65 (d, $J$= 18.2 Hz, 1H), 2.51 - 2.48 (m, 2H), 2.15 - 2.06 (m, 1H). LCMS (ESI) calcd. for $C_{24}H_{23}Cl_2N_4O_2S^+$ [M+H]$^+$: 501.19, found, 501.1.

**Example 278: Preparation of 3-(5-((3-fluoro-3',6'-dihydro-[2,4'-bipyridin]-1'(2'H)-yl)methyl)-1-thioxoisoindolin-2-yl)piperidine-2,6-dione (GT-08491)**

With reference to the method of Scheme 4, the target compound GT-08491 was obtained (14 mg, white solid, yield 26%). $^1$H NMR (400 MHz, DMSO-d6) δ 11.25 (s, 1H), 11.15 (s, 1H), 8.46 (d, $J$= 4.5 Hz, 1H), 7.98 (d, $J$ = 7.6 Hz, 1H), 7.85 (t, $J$ = 7.1 Hz, 1H), 7.77 (dd, $J$= 12.2, 8.4 Hz, 1H), 7.46 - 7.42 (m, 1H), 6.55 (s, 1H), 5.97 (d, $J$ = 9.7 Hz, 1H), 4.89 (d, $J$ = 20.4 Hz, 1H), 4.75 (d, $J$= 20.4 Hz, 1H), 4.66 - 4.49 (m, 2H), 3.85 - 3.75 (m, 2H), 3.65 (d, $J$= 10.7 Hz, 1H), 3.35 - 3.18 (m, 1H),

3.04 - 2.88 (m, 3H), 2.65 (d, $J$ = 16.5 Hz, 1H), 2.60 - 2.55 (m, 1H), 2.16 - 2.04 (m, 1H). LCMS (ESI) calcd. for $C_{24}H_{24}FN_4O_2S^+$ $[M+H]^+$: 451.16, found, 451.1.

**Example 279: Preparation of 3-(5-((2',3'-difluoro-3,6-dihydro-[4,4'-bipyridin]-1(2H)-yl)methyl)-1-thioxoisoindolin-2-yl)piperidine-2,6-dione (GT-08474)**

With reference to the method of Scheme 4, the target compound GT-08474 was obtained (17 mg, white solid, yield 31%). $^1H$ NMR (400 MHz, DMSO-d6) δ 11.19 (s, 1H), 11.15 (s, 1H), 8.05 (d, $J$ = 5.1 Hz, 1H), 8.03 - 7.89 (m, 2H), 7.82 (d, $J$ = 7.5 Hz, 1H), 7.41 (t, $J$= 5.1 Hz, 1H), 6.34 (s, 1H), 5.96 (d, $J$ = 8.9 Hz, 1H), 4.89 (d, $J$ = 20.3 Hz, 1H), 4.75 (d, $J$ = 20.4 Hz, 1H), 4.63 - 4.53 (m, 2H), 3.98 - 3.73 (m, 2H), 3.72 - 3.56 (m, 1H), 3.44 - 3.41 (m, 2H), 3.27 - 3.17 (m, 1H), 3.07 - 2.91 (m, 2H), 2.78 - 2.54 (m, 3H), 2.17 - 2.00 (m, 1H). LCMS (ESI) calcd. for $C_{24}H_{23}F_2N_4O_2S^+$ $[M+H]^+$: 469.15, found, 469.1.

**Example 280: Preparation of 3-(5-((6-(2,3-dichlorophenyl)-3,6-diazabicyclo[3.1.1]heptan-3-yl)methyl)-1-thioxoisoindolin-2-yl)piperidine-2,6-dione (GT-08476)**

With reference to the method of Scheme 4, the target compound GT-08476 was obtained (12 mg, white solid, yield 20%). $^1H$ NMR (400 MHz, DMSO-d6) δ 11.92 (s, 1H), 11.16 (s, 1H), 7.79 - 7.72 (m, 2H), 7.61 (d, $J$= 8.0 Hz, 1H), 6.96 (t, $J$= 7.5 Hz, 1H), 6.81 (d, $J$= 8.1 Hz, 1H), 6.48 (d, $J$= 8.2 Hz, 1H), 5.98 (d, $J$= 10.7 Hz, 1H), 4.81 - 4.53 (m, 4H), 4.27 (s, 2H), 3.62 - 3.50 (m, 2H), 3.49 - 3.42 (m, 2H), 3.05 - 2.92 (m, 1H), 2.81 - 2.57 (m, 4H), 2.19 - 2.03 (m, 2H). LCMS (ESI) calcd. for $C_{25}H_{25}Cl_2N_4O_2S^+$ $[M+H]^+$: 515.11, found, 515.1.

**Example 281: Preparation of 3-(5-(((1R,4R)-5-(2,3-dichlorophenyl)-2,5-diazabicyclo[2.2.1]heptan-2-yl) methyl)-1-thioxoisoindolin-2-yl)piperidine-2,6-dione (GT-08477)**

With reference to the method of Scheme 4, the target compound GT-08477 was obtained (14 mg, white solid, yield 23%). $^1H$ NMR (400 MHz, DMSO-d6) δ 11.14 (s, 1H), 10.45 (s, 1H), 8.03 - 7.96 (m, 2H), 7.85 (t, $J$ = 9.5 Hz, 1H), 7.16 (d, $J$ = 6.5 Hz, 1H), 7.11 (d, $J$ = 7.7 Hz, 1H), 6.98 (d, $J$ = 7.9 Hz, 1H), 5.96 (d, $J$= 9.7 Hz, 1H), 4.87 (dd, $J$= 20.4, 4.6 Hz, 1H), 4.79 - 4.63 (m, 2H), 4.54 (s, 1H), 4.51 - 4.42 (m, 1H), 4.38 (s, 1H), 3.91 (d, $J$ = 11.4 Hz, 1H), 3.77 (d, $J$ = 11.0 Hz, 1H), 3.68 - 3.55 (m, 1H), 3.24 (d, $J$ = 11.0 Hz, 1H), 3.03 - 2.91 (m, 1H), 2.69 - 2.54 (m, 3H), 2.19 - 2.08 (m, 2H). LCMS (ESI) calcd. for $C_{25}H_{25}Cl_2N_4O_2S^+$ $[M+H]^+$: 515.11, found, 515.1.

**Example 282: Preparation of 3-(5-((5-(2,3-dichlorophenyl)-2,5-diazabicyclo[2.2.2]octan-2-yl)methyl)-1-thioxoisoindolin-2-yl)piperidine-2,6-dione** (GT-08478)

With reference to the method of Scheme 4, the target compound GT-08478 was obtained (12 mg, white solid, yield 19%). $^1H$ NMR (400 MHz, DMSO-d6) δ 11.14 (s, 1H), 10.83 (s, 1H), 8.04 (d, $J$= 10.3 Hz, 1H), 7.99 - 7.95 (m, 1H), 7.95 - 7.88 (m, 1H), 7.34 (d, $J$= 7.3 Hz, 1H), 7.30 - 7.22 (m, 2H), 5.96 (d, $J$ = 12.6 Hz, 1H), 4.99 - 4.83 (m, 1H), 4.81 - 4.59 (m, 3H), 3.88 - 3.65 (m, 4H), 3.54 - 3.45 (m, 1H), 3.23 - 3.15 (m, 1H), 3.05 - 2.95 (m, 1H), 2.67 - 2.61 (m, 2H), 2.47 - 2.39 (m, 1H), 2.18 - 2.07 (m, 2H), 2.02 - 1.87 (m, 2H). LCMS (ESI) calcd. for $C_{26}H_{27}Cl_2N_4O_2S^+$ $[M+H]^+$: 529.12, found, 529.1.

**Example 283: Preparation of 3-(5-((8-(2,3-dichlorophenyl)-3,8-diazabicyclo[3.2.1]octan-3-yl)methyl)-1-thioxoisoindolin-2-yl)piperidine-2,6-dione (GT-08479)**

With reference to the method of Scheme 4, the target compound GT-08479 was obtained (13 mg, white solid, yield 21%). $^1H$ NMR (400 MHz, DMSO-d6) δ 11.14 (s, 1H), 10.32 (s, 1H), 8.06 (s, 1H), 7.97 (d, $J$= 8.1 Hz, 1H), 7.91 (d, $J$= 7.2 Hz, 1H), 7.28 - 7.19 (m, 2H), 7.10 - 6.99 (m, 1H), 6.02 - 5.95 (m, 1H), 4.88 (d, $J$ = 20.6 Hz, 1H), 4.74 (d, $J$ = 20.1 Hz, 1H), 4.53 (s, 2H), 4.16 (s, 2H), 3.01 - 2.96 (m, 1H), 2.67 - 2.57 (m, 3H), 2.47 - 2.38 (m, 1H), 2.22 - 2.08 (m, 4H), 2.05 - 1.87 (m, 3H). LCMS (ESI) calcd. for $C_{26}H_{27}Cl_2N_4O_2S^+$ $[M+H]^+$: 529.12, found, 529.1.

**Example 284: Preparation of 3-(5-((3-(2,3-dichlorophenyl)-3,8-diazabicyclo[3.2.1]octan-8-yl)methyl)-1-thioxoisoindolin-2-yl)piperidine-2,6-dione (GT-08480)**

With reference to the method of Scheme 4, the target compound GT-08480 was obtained (14 mg, white solid, yield 23%). $^1H$ NMR (400 MHz, DMSO-d6) δ 11.14 (s, 1H), 8.06 (d, $J$ = 13.1 Hz, 1H), 8.00 (d, $J$ = 8.0 Hz, 1H), 7.95 - 7.89 (m, 1H), 7.43 - 7.35 (m, 2H), 7.17 (dd, $J$= 7.8, 1.7 Hz, 1H), 5.97 (d, $J$ = 13.4 Hz, 1H), 4.89 (d, $J$ = 20.5 Hz, 1H), 4.75 (d, $J$= 20.4 Hz, 1H), 4.41 (d, $J$ = 5.4 Hz, 2H), 3.99 (brs, 2H), 3.58 - 3.43 (m, 2H), 3.27 - 3.14 (m, 4H), 3.06 - 2.90 (m, 1H), 2.72 - 2.56 (m, 3H), 2.39 - 2.29 (m, 4H), 2.16 - 2.06 (m, 2H). LCMS (ESI) calcd. for $C_{26}H_{27}Cl_2N_4O_2S^+$ $[M+H]^+$: 529.12, found, 529.1.

**Example 285: Preparation of 3-(5-((4-(2,3-dichlorophenyl)-1,4-diazepan-1-yl)methyl)-1-thioxoisoindolin-2-yl)piperidine-2,6-dione (GT-08481)**

With reference to the method of Scheme 4, the target compound GT-08481 was obtained (11 mg, white solid, yield 18%). $^1H$ NMR (400 MHz, DMSO-d6) δ 11.14 (s, 1H), 10.94 (s, 1H), 8.02 - 7.94 (m, 2H), 7.85 (t, *J* = 8.2 Hz, 1H), 7.30 (d, *J* = 4.7 Hz, 2H), 7.26 - 7.19 (m, 1H), 5.96 (d, *J* = 9.6 Hz, 1H), 4.88 (d, *J* = 20.5 Hz, 1H), 4.74 (d, *J*= 20.0 Hz, 1H), 4.57 - 3.49 (m, 2H), 3.57 - 3.51 (m, 4H), 3.44 - 3.40 (m, 2H), 3.27 - 3.18 (m, 1H), 3.02 - 2.93 (m, 1H), 2.65 (d, *J*= 17.1 Hz, 1H), 2.63 - 2.54 (m, 2H), 2.29 - 2.16 (m, 2H), 2.14 - 2.04 (m, 1H). LCMS (ESI) calcd. for $C_{25}H_{27}Cl_2N_4O_2S^+$ $[M+H]^+$: 517.12, found, 517.1.

**Example 286: Preparation of 3-(4-((4-benzhydrylpiperazin-1-yl)methyl)-1-thioxoisoindolin-2-yl)piperidine-2,6-dione (GT-08191)**

With reference to the method of Scheme 4, the target compound GT-08191 was obtained (28 mg, white solid, yield 46%). $^1H$ NMR (400 MHz, DMSO-d6) δ 11.19 (s, 1H), 8.04 - 7.83 (m, 3H), 7.73 - 7.52 (m, 3H), 7.50 - 7.12 (m, 7H), 5.99 (d, *J* = 8.6 Hz, 1H), 5.33 (d, *J* = 21.6 Hz, 1H), 4.90 (d, *J*= 20.4 Hz, 1H), 4.59 - 4.29 (m, 2H), 4.09 - 3.86 (m, 6H), 3.58 - 3.25 (m, 4H), 3.04 - 2.95 (m, 1H), 2.74 - 2.65 (m, 1H), 2.12 - 2.07 (m, 1H). LCMS (ESI) calcd. for $C_{31}H_{33}N_4O_2S^+$ $[M+H]^+$: 525.23, found, 525.2.

**Example 287: Preparation of 3-(4-((4-(phenyl(pyridin-2-yl)methyl)piperazin-1-yl)methyl)-1-thioxoisoindolin-2-yl)piperidine-2,6-dione (GT-08192)**

With reference to the method of Scheme 4, the target compound GT-08192 was obtained (12 mg, white solid, yield 20%). $^1H$ NMR (400 MHz, DMSO-d6) δ 11.20 (s, 1H), 8.69 (d, *J* = 4.5 Hz, 1H), 8.10 - 8.03 (m, 1H), 8.00 - 7.88 (m, 2H), 7.77 (d, *J* = 7.8 Hz, 1H), 7.69 - 7.57 (m, 3H), 7.57 - 7.49 (m, 1H), 7.46 - 7.31 (m, 3H), 5.99 (d, *J*= 8.4 Hz, 1H), 5.60 - 5.47 (m, 2H), 4.91 (t, *J*= 17.3 Hz, 1H), 4.56 - 4.38 (m, 2H), 3.47 - 3.22 (m, 4H), 3.18 - 2.82 (m, 6H), 2.69 (d, *J* = 16.7 Hz, 1H), 2.21 - 2.04 (m, 1H). LCMS (ESI) calcd. for $C_{30}H_{32}N_5O_2S^+$ $[M+H]^+$: 526.23, found, 526.2.

**Example 288: Preparation of 3-(4-((4-(phenyl(pyridin-3-yl)methyl)piperazin-1-yl)methyl)-1-thioxoisoindolin-2-yl)piperidine-2,6-dione (GT-08193)**

With reference to the method of Scheme 4, the target compound GT-08193 was obtained (8 mg, white solid, yield 13%). $^1H$ NMR (400 MHz, DMSO-d6) δ 11.20 (s, 1H), 9.44 - 9.34 (m, 1H), 9.33 - 9.21 (m, 2H), 9.11 - 8.99 (m, 1H), 8.19 - 8.06 (m, 1H), 8.03 - 7.90 (m, 1H), 7.73 - 7.65 (m, 2H), 7.52 - 7.36 (m, 4H), 6.17 - 5.97 (m, 2H), 5.03 - 4.99 (m, 1H), 4.89 - 4.76 (m, 1H), 3.44 - 3.30 (m, 2H), 3.22 - 3.04 (m, 4H), 2.79 - 2.61 (m, 3H), 2.64 - 2.53 (m, 4H), 2.19 - 2.01 (m, 1H). LCMS (ESI) calcd. for $C_{30}H_{32}N_5O_2S^+$ $[M+H]^+$: 526.23, found, 526.2.

**Example 289: Preparation of 3-(4-((4-(phenyl(pyridin-4-yl)methyl)piperazin-1-yl)methyl)-1-thioxoisoindolin-2-yl)piperidine-2,6-dione (GT-08204)**

With reference to the method of Scheme 4, the target compound GT-08204 was obtained (20 mg, white solid, yield 33%). $^1H$ NMR (400 MHz, DMSO-d6) δ 11.20 (s, 1H), 8.85 (d, *J* = 6.1 Hz, 2H), 8.14 - 8.10 (m, 2H), 8.02 - 7.96 (m, 1H), 7.83 - 7.80 (m, 1H), 7.68 - 7.63 (m, 1H), 7.48 - 7.46 (m, 2H), 7.38 (t, *J* = 7.5 Hz, 2H), 7.32 - 7.28 (m, 1H), 6.01 - 5.95 (m, 1H), 5.44 (d, *J* = 19.6 Hz, 1H), 5.13 - 4.86 (m, 2H), 4.55 - 4.36 (m, 2H), 3.41 - 3.23 (m, 6H), 2.88 - 2.66 (m, 5H), 2.19 - 2.08 (m, 1H). LCMS (ESI) calcd. for $C_{30}H_{32}N_5O_2S^+$ $[M+H]^+$: 526.23, found, 526.2.

**Example 290: Preparation of 3-(4-((4-(di(pyridin-2-yl)methyl)piperazin-1-yl)methyl)-1-thioxoisoindolin-2-yl)piperidine-2,6-dione (GT-08201)**

With reference to the method of Scheme 4, the target compound GT-08201 was obtained (14 mg, white solid, yield 23%). $^1H$ NMR (400 MHz, DMSO-d6) δ 10.99 (s, 1H), 8.71 (d, *J* = 4.7 Hz, 2H), 8.20 - 8.10 (m, 3H), 7.91 - 7.85 (m, 3H), 7.68 - 7.62 (m, 3H), 6.62 (s, 1H), 5.85 (s, 1H), 4.71 - 4.44 (m, 3H), 3.48 (brs, 5H), 3.42 - 3.32 (m, 2H), 3.01 (brs, 5H), 2.64 (d, *J*= 16.7 Hz, 1H), 2.19 - 2.09 (m, 1H). LCMS (ESI) calcd. for $C_{29}H_{31}N_6O_2S^+$ $[M+H]^+$: 527.22, found, 527.2.

**Example 291: Preparation of 3-(4-((4-(bis(4-fluorophenyl)methyl)piperazin-1-yl)methyl)-1-thioxoisoindolin-2-yl) piperidine-2,6-dione (GT-08202)**

With reference to the method of Scheme 4, the target compound GT-08202 was obtained (14 mg, white solid, yield 22%). $^1H$ NMR (400 MHz, DMSO-d6) δ 11.20 (s, 1H), 7.97 - 7.88 (m, 2H), 7.73 - 7.61 (m, 2H), 7.56 - 7.44 (m, 3H), 7.28 -

7.07 (m, 4H), 6.07 - 5.88 (m, 1H), 5.36 - 5.19 (m, 1H), 4.88 (d, *J* = 21.0 Hz, 1H), 4.68 - 4.15 (m, 3H), 3.40 - 3.14 (m, 4H), 3.07 - 2.94 (m, 1H), 2.89 - 2.80 (m, 2H), 2.76 - 2.63 (m, 2H), 2.48 - 2.29 (m, 2H), 2.19 - 2.04 (m, 1H). LCMS (ESI) calcd. for $C_{31}H_{31}F_2N_4O_2S^+$ [M+H]$^+$: 561.21, found, 561.2.

**Example 292: Preparation of 3-(4-((4-(bis(4-chlorophenyl)methyl)piperazin-1-yl)methyl)-1-thioxoisoindolin-2-yl)piperidine-2,6-dione (GT-08203)**

With reference to the method of Scheme 4, the target compound GT-08203 was obtained (22 mg, white solid, yield 32%). $^1$H NMR (400 MHz, DMSO-d6) δ 11.20 (s, 1H), 7.97 - 7.89 (m, 2H), 7.72 - 7.64 (m, 2H), 7.55 - 7.47 (m, 3H), 7.44 - 7.33 (m, 4H), 5.99 (d, *J* = 9.9 Hz, 1H), 5.31 (d, *J* = 21.2 Hz, 1H), 4.89 (d, *J* = 20.4 Hz, 1H), 4.70 - 4.57 (m, 1H), 4.41 - 4.29 (m, 2H), 3.42 - 3.13 (m, 4H), 3.05 - 2.92 (m, 1H), 2.92 - 2.75 (m, 2H), 2.74 - 2.62 (m, 2H), 2.49 - 2.36 (m, 2H), 2.18 - 2.05 (m, 1H). LCMS (ESI) calcd. for $C_{31}H_{31}Cl_2N_4O_2S^+$ [M+H]$^+$: 593.15, found, 593.1.

**Example 293: Preparation of 3-(4-(((1R,4R)-5-benzhydryl-2,5-diazabicyclo[2.2.1]heptan-2-yl)methyl)-1-thioxoisoindolin-2-yl)piperidine-2,6-dione (GT-08208)**

With reference to the method of Scheme 4, the target compound GT-08208 was obtained (10 mg, white solid, yield 16%). $^1$H NMR (400 MHz, DMSO-d6) δ 11.19 (s, 1H), 8.11 - 7.91 (m, 3H), 7.78 - 7.53 (m, 4H), 7.46 - 7.21 (m, 6H), 6.06 - 6.00 (m, 1H), 5.35 - 5.05 (m, 1H), 4.87 (d, *J* = 19.4 Hz, 1H), 4.67 - 4.52 (m, 2H), 4.41 - 4.10 (m, 2H), 3.61 - 3.54 (m, 4H), 3.08 - 2.95 (m, 2H), 2.77 - 2.63 (m, 2H), 2.49 - 2.38 (m, 2H), 2.17 - 2.08 (m, 1H). LCMS (ESI) calcd. for $C_{32}H_{33}N_4O_2S^+$ [M+H]$^+$: 537.23, found, 537.2.

**Example 294: Preparation of 3-(4-(((1S,4S)-5-benzhydryl-2,5-diazabicyclo[2.2.1]heptan-2-yl)methyl)-1-thioxoisoindolin-2-yl)piperidine-2,6-dione (GT-08207)**

With reference to the method of Scheme 4, the target compound GT-08207 was obtained (8 mg, white solid, yield 10%). $^1$H NMR (400 MHz, DMSO-d6) δ 11.19 (s, 1H), 8.07 - 7.91 (m, 3H), 7.76 - 7.57 (m, 4H), 7.44 - 7.21 (m, 6H), 6.05 - 5.99 (m, 1H), 5.34 - 5.10 (m, 1H), 4.97 - 4.81 (m, 1H), 4.65 - 4.45 (m, 2H), 4.39 - 4.07 (m, 2H), 3.66 - 3.47 (m, 4H), 3.09 - 2.93 (m, 2H), 2.76 - 2.63 (m, 2H), 2.56 - 2.52 (m, 2H), 2.18 - 2.06 (m, 1H). LCMS (ESI) calcd. for $C_{32}H_{33}N_4O_2S^+$ [M+H]$^+$: 537.23, found, 537.2.

**Example 295: Preparation of 3-(4-((8-benzhydryl-3,8-diazabicyclo[3.2.1]octan-3-yl)methyl)-1-thioxoisoindolin-2-yl)piperidine-2,6-dione (GT-08234)**

With reference to the method of Scheme 4, the target compound GT-08234 was obtained (15 mg, white solid, yield 24%). $^1$H NMR (400 MHz, DMSO-d6) δ 11.15 (s, 1H), 10.82 (s, 1H), 8.08 - 7.93 (m, 4H), 7.86 - 7.81 (m, 1H), 7.58 - 7.50 (m, 2H), 7.49 - 7.41 (m, 4H), 7.39 - 7.36 (m, 2H), 6.00 (d, *J* = 7.4 Hz, 1H), 5.39 (d, *J* = 8.8 Hz, 1H), 4.98 - 4.74 (m, 2H), 3.81 - 3.62 (m, 4H), 3.35 - 3.17 (m, 2H), 3.01 - 2.95 (m, 1H), 2.68 - 2.51 (m, 4H), 2.37 - 2.24 (m, 2H), 2.17 - 2.08 (m, 2H), 2.05 - 1.92 (m, 1H). LCMS (ESI) calcd. for $C_{33}H_{35}N_4O_2S^+$ [M+H]$^+$: 551.25, found, 551.2.

**Example 296: Preparation of 3-(4-((3-benzhydryl-3,8-diazabicyclo[3.2.1]octan-8-yl)methyl)-1-thioxoisoindolin-2-yl)piperidine-2,6-dione (GT-08235)**

With reference to the method of Scheme 4, the target compound GT-08235 was obtained (8 mg, white solid, yield 13%). $^1$H NMR (400 MHz, DMSO-d6) δ 11.19 (s, 1H), 8.00 - 7.86 (m, 2H), 7.73 - 7.66 (m, 2H), 7.55 - 7.50 (m, 1H), 7.48 (d, *J*= 7.1 Hz, 3H), 7.31 (t, *J*= 7.5 Hz, 3H), 7.20 (t, *J* = 7.3 Hz, 2H), 6.00 (d, *J* = 12.3 Hz, 1H), 5.27 - 5.04 (m, 1H), 4.91 (d, *J*= 20.2 Hz, 1H), 4.42 - 4.39 (m, 2H), 4.28 (d, *J*= 5.4 Hz, 1H), 4.09 - 3.88 (m, 2H), 3.35 - 3.23 (m, 2H), 3.02 - 2.95 (m, 1H), 2.75 - 2.60 (m, 3H), 2.49 - 2.44 (m, 3H), 2.37 - 2.21 (m, 3H), 2.14 - 2.06 (m, 1H). LCMS (ESI) calcd. for $C_{33}H_{35}N_4O_2S^+$ [M+H]$^+$: 551.25, found, 551.2.

**Example 297: Preparation of 3-(4-((5-benzhydryl-2,5-diazabicyclo[2.2.2]octan-2-yl)methyl)-1-thioxoisoindolin-2-yl)piperidine-2,6-dione (GT-08233)**

With reference to the method of Scheme 4, the target compound GT-08233 was obtained (10 mg, white solid, yield 16%). $^1$H NMR (400 MHz, DMSO-d6) δ 11.19 (s, 1H), 8.24 - 7.85 (m, 3H), 7.78 - 7.50 (m, 4H), 7.47 - 7.32 (m, 5H), 7.24 - 7.17 (m, 1H), 6.12 - 5.88 (m, 1H), 5.27 - 5.07 (m, 1H), 5.05 - 4.83 (m, 1H), 4.71 - 4.52 (m, 1H), 4.49 - 4.22 (m, 1H), 3.66 - 3.51 (m, 2H), 3.12 - 2.94 (m, 2H), 2.94 - 2.80 (m, 1H), 2.78 - 2.57 (m, 3H), 2.30 - 1.99 (m, 3H), 1.98 - 1.67 (m, 2H), 1.41 - 1.10 (m, 2H). LCMS (ESI) calcd. for $C_{33}H_{35}N_4O_2S^+$ [M+H]$^+$: 551.25, found, 551.2.

**Example 298: Preparation of 3-(4-((4-benzhydryl-3,3-dimethylpiperazin-1-yl)methyl)-1-thioxoisoindolin-2-yl)piperidine-2,6-dione (GT-08231)**

With reference to the method of Scheme 4, the target compound GT-08231 was obtained (8 mg, white solid, yield 13%). $^1H$ NMR (400 MHz, DMSO-d6) δ 11.21 (s, 1H), 10.25 (s, 1H), 8.10 - 8.03 (m, 1H), 7.97 (d, *J*= 7.7 Hz, 1H), 7.66 (t, *J* = 7.6 Hz, 1H), 7.39 - 7.28 (m, 8H), 7.24 - 7.18 (m, 2H), 6.07 - 5.93 (m, 1H), 5.48 (s, 1H), 5.19 - 5.04 (m, 1H), 4.88 (d, *J*= 19.7 Hz, 1H), 4.47 - 4.33 (m, 2H), 3.23 - 3.16 (m, 2H), 3.08 - 2.96 (m, 2H), 2.78 - 2.67 (m, 3H), 2.49 - 2.36 (m, 2H), 2.20 - 2.05 (m, 1H), 1.60 - 1.47 (m, 1H), 1.20 (s, 3H), 1.05 (s, 3H). LCMS (ESI) calcd. for $C_{33}H_{37}N_4O_2S^+$ [M+H]$^+$: 553.26, found, 553.2.

**Example 299: Preparation of 3-(4-(((R)-4-benzhydryl-2-methylpiperazin-1-yl)methyl)-1-thioxoisoindolin-2-yl)piperidine-2,6-dione (GT-08232)**

With reference to the method of Scheme 4, the target compound GT-08232 was obtained (7 mg, white solid, yield 11%). $^1H$ NMR (400 MHz, DMSO-d6) δ 11.19 (s, 1H), 8.10 - 7.92 (m, 2H), 7.92 - 7.82 (m, 1H), 7.74 - 7.58 (m, 2H), 7.49 - 7.41 (m, 3H), 7.37 - 7.30 (m, 3H), 7.27 - 7.20 (m, 2H), 6.06 - 5.87 (m, 1H), 5.01 - 4.77 (m, 2H), 4.48 - 4.43 (m, 1H), 4.26 - 4.09 (m, 1H), 3.64 - 3.55 (m, 2H), 3.26 - 3.03 (m, 3H), 3.03 - 2.82 (m, 3H), 2.73 - 2.67 (m, 2H), 2.49 - 2.36 (m, 1H), 2.19 - 2.07 (m, 1H), 1.56 - 1.35 (m, 3H). LCMS (ESI) calcd. for $C_{32}H_{35}N_4O_2S^+$ [M+H]$^+$: 539.25, found, 539.2.

**Example 300: Preparation of 3-(4-((4-(diphenylamino)piperidin-1-yl)methyl)-1-thioxoisoindolin-2-yl)piperidine-2,6-dione (GT-08209)**

With reference to the method of Scheme 4, the target compound GT-08209 was obtained (5 mg, white solid, yield 10%). LCMS (ESI) calcd. for $C_{31}H_{33}N_4O_2S^+$ [M+H]$^+$: 525.23, found, 525.1.

**Example 301: Preparation of 3-(4-((4-(phenyl(pyridin-2-yl)amino)piperidin-1-yl)methyl)-1-thioxoisoindolin-2-yl)piperidine-2,6-dione (GT-08243)**

With reference to the method of Scheme 4, the target compound GT-08243 was obtained (14 mg, white solid, yield 23%). $^1H$ NMR (400 MHz, DMSO-d6) δ 11.20 (s, 1H), 10.26 (s, 1H), 8.11 (dd, *J* = 5.4, 1.2 Hz, 1H), 7.97 (d, *J*= 7.7 Hz, 1H), 7.89 (d, *J* = 7.7 Hz, 1H), 7.81 - 7.78 (m, 1H), 7.75 - 7.68 (m, 1H), 7.65 (t, *J* = 7.6 Hz, 1H), 7.58 - 7.54 (m, 2H), 7.50 (d, *J* = 7.2 Hz, 1H), 7.25 (d, *J* = 7.6 Hz, 1H), 6.83 - 6.75 (m, 1H), 6.00 (d, *J* = 8.0 Hz, 1H), 5.18 (d, *J* = 20.2 Hz, 1H), 5.02 - 4.98 (m, 1H), 4.86 (d, *J* = 20.4 Hz, 1H), 4.46 - 4.24 (m, 1H), 3.41 - 3.20 (m, 6H), 3.08 - 2.92 (m, 1H), 2.73 - 2.64 (m, 1H), 2.44 - 2.34 (m, 1H), 2.21 - 2.03 (m, 2H), 1.82 - 1.68 (m, 1H). LCMS (ESI) calcd. for $C_{30}H_{32}N_5O_2S^+$ [M+H]$^+$: 526.23, found, 526.2.

**Example 302: Preparation of 3-(4-((4-((4'-chloro-5,5-dimethyl-3,4,5,6-tetrahydro-[1,1'-biphenyl]-2-yl)methyl)piperazin-1-yl)methyl)-1-thioxoisoindolin-2-yl)piperidine-2,6-dione (GT-08303)**

With reference to the method of Scheme 4, the target compound GT-08303 was obtained (22 mg, white solid, yield 32%). $^1H$ NMR (400 MHz, DMSO-d6) δ 11.17 (s, 1H), 7.93 (d, *J* = 7.0 Hz, 1H), 7.88 - 7.70 (m, 1H), 7.62 (d, *J* = 6.8 Hz, 1H), 7.41 (d, *J*= 8.0 Hz, 2H), 7.11 (d, *J* = 8.2 Hz, 2H), 5.99 (d, *J* = 9.6 Hz, 1H), 5.36 - 5.01 (m, 1H), 4.81 (d, *J* = 18.3 Hz, 1H), 4.55 - 3.91 (m, 1H), 3.66 - 3.61 (m, 2H), 3.38 - 3.21 (m, 7H), 3.04 - 2.95 (m, 3H), 2.68 (d, *J*= 16.5 Hz, 1H), 2.41 - 2.21 (m, 3H), 2.17 - 2.06 (m, 1H), 2.01 (s, 2H), 1.45 (t, *J* = 5.9 Hz, 2H), 0.95 (s, 6H). LCMS (ESI) calcd. for $C_{33}H_{40}ClN_4O_2S^+$ [M+H]$^+$: 591.26, found, 591.2.

**Example 303: Preparation of 3-(4-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-thioxoisoindolin-2-yl)piperidine-2,6-dione (GT-08271)**

With reference to the method of Scheme 4, the target compound GT-08271 was obtained (8 mg, white solid, yield 10%). $^1H$ NMR (400 MHz, DMSO-d6) δ 11.13 (s, 1H), 7.83 (d, *J* = 8.3 Hz, 2H), 7.56 - 7.53 (m, 2H), 7.47 (d, J = 8.3Hz, 2H), 7.28 (d, *J*= 8.1 Hz, 1H), 7.21 (d, *J*= 8.3 Hz, 2H), 5.98 (d, *J*= 8.0 Hz, 1H), 4.75 (d, *J*= 19.4 Hz, 1H), 4.63 (d, *J*= 21.0 Hz, 1H), 4.32 - 4.17 (m, 1H), 4.15 - 3.96 (m, 1H), 3.86 - 3.70 (m, 2H), 3.30 - 3.23 (m, 2H), 3.02 - 2.94 (m, 2H), 2.67 - 2.63 (m, 2H), 2.26 - 2.15 (m, 4H), 2.12 - 2.01 (m, 5H), 1.48 - 1.39 (m, 3H), 0.95 (s, 6H). LCMS (ESI) calcd. for $C_{34}H_{40}ClN_4O_2S^+$ [M+H]$^+$: 603.26, found, 603.2.

**Example 304: Preparation of 3-(4-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-thioxoisoindolin-2-yl)piperidine-2,6-dione (GT-08291)**

With reference to the method of Scheme 4, the target compound GT-08291 was obtained (17 mg, white solid,

yield 24%). $^1$H NMR (400 MHz, DMSO-d6) δ 11.14 (s, 1H), 7.89 - 7.84 (m, 1H), 7.63 - 7.50 (m, 2H), 7.44 (d, *J* = 8.4 Hz, 2H), 7.24 - 7.14 (m, 2H), 5.99 (d, *J* = 5.7 Hz, 1H), 5.22 - 5.18 (m, 1H), 5.11 - 5.09 (m, 1H), 4.92 - 4.64 (m, 2H), 4.08 - 3.75 (m, 4H), 3.55 - 3.39 (m, 2H), 3.02 - 2.79 (m, 2H), 2.65 (d, *J* = 17.6 Hz, 1H), 2.63 - 2.56 (m, 2H), 2.45 - 2.34 (m, 2H), 2.22 - 1.92 (m, 4H), 1.87 - 1.61 (m, 1H), 1.47 (t, *J*= 5.8 Hz, 2H), 1.29 - 1.10 (m, 2H), 0.96 (s, 6H). LCMS (ESI) calcd. for $C_{35}H_{42}ClN_4O_2S^+$ $[M+H]^+$: 617.27, found, 617.2.

**Example 305: Preparation of 3-(4-((3-((4'-chloro-5,5-dimethyl-3,4,5,6-tetrahydro-[1,1'-biphenyl]-2-yl) methyl)-3,8-diazabicyclo[3.2.1]octan-8-yl)methyl)-1-thioxoisoindolin-2-yl)piperidine-2,6-dione (GT-08292)**

With reference to the method of Scheme 4, the target compound GT-08292 was obtained (9 mg, white solid, yield 13%). $^1$H NMR (400 MHz, DMSO-d6) δ 11.19 (s, 1H), 9.79 (s, 1H), 7.98 (d, *J*= 7.6 Hz, 1H), 7.88 (d, *J* = 7.2 Hz, 1H), 7.65 (t, *J* = 7.6 Hz, 1H), 7.35 (d, *J* = 8.2 Hz, 2H), 7.05 (d, *J* = 7.9 Hz, 2H), 6.00 (d, *J* = 13.4 Hz, 1H), 5.12 (d, *J*= 20.3 Hz, 1H), 4.89 (d, *J*= 20.4 Hz, 1H), 4.25 (d, *J* = 1.7 Hz, 2H), 3.97 (brs, 2H), 3.02 - 2.95 (m, 1H), 2.81 (s, 2H), 2.73 - 2.61 (m, 3H), 2.44 - 2.40 (m, 2H), 2.32 - 2.05 (m, 6H), 1.97 (brs, 4H), 1.43 (t, *J* = 6.2 Hz, 2H), 0.94 (s, 6H). LCMS (ESI) calcd. for $C_{35}H_{42}ClN_4O_2S^+$ $[M+H]^+$: 617.27, found, 617.2.

**Example 306: Preparation of 3-(4-((5-((4'-chloro-5,5-dimethyl-3,4,5,6-tetrahydro-[1,1'-biphenyl]-2-yl) methyl)-2,5-diazabicyclo[2.2.2]octan-2-yl)methyl)-1-thioxoisoindolin-2-yl)piperidine-2,6-dione (GT-08302)**

With reference to the method of Scheme 4, the target compound GT-08302 was obtained (8 mg, white solid, yield 11%). $^1$H NMR (400 MHz, DMSO-d6) δ 11.16 (s, 1H), 8.08 - 7.73 (m, 2H), 7.65 - 7.54 (m, 1H), 7.45 - 7.31 (m, 2H), 7.21 (brs, 2H), 6.12 - 5.87 (m, 1H), 5.15 (d, *J* = 20.0 Hz, 1H), 5.00 (d, *J* = 20.5 Hz, 1H), 4.93 - 4.59 (m, 2H), 4.45 - 4.20 (m, 1H), 3.99 - 3.61 (m, 3H), 3.36 - 3.14 (m, 5H), 3.04 - 2.99 (m, 1H), 2.66 (d, *J* = 13.5 Hz, 1H), 2.47 - 2.18 (m, 4H), 2.17 - 1.87 (m, 4H), 1.45 (s, 2H), 1.35 - 1.12 (m, 1H), 0.95 (s, 6H). LCMS (ESI) calcd. for $C_{35}H_{42}ClN_4O_2S^+$ $[M+H]^+$: 617.27, found, 617.2.

**Example 307: Preparation of 3-(4-((4-((4'-fluoro-3,4,5,6-tetrahydro-[1,1'-biphenyl]-2-yl)methyl)piperazin-1-yl) methyl)-1-thioxoisoindolin-2-yl)piperidine-2,6-dione (GT-08293)**

With reference to the method of Scheme 4, the target compound GT-08293 was obtained (19 mg, white solid, yield 30%). $^1$H NMR (400 MHz, DMSO-d6) δ 11.18 (s, 1H), 7.93 (d, *J* = 7.0 Hz, 1H), 7.88 - 7.71 (m, 1H), 7.61 (t, *J* = 7.5 Hz, 1H), 7.22 - 7.10 (m, 4H), 5.99 (d, *J* = 13.9 Hz, 1H), 5.32 - 5.03 (m, 1H), 4.80 (d, *J*= 19.5 Hz, 1H), 4.45 - 4.01 (m, 1H), 3.33 - 3.18 (m, 4H), 3.01 - 2.91 (m, 2H), 2.89 - 2.81 (m, 1H), 2.67 (d, *J*= 16.8 Hz, 1H), 2.46 - 2.41 (m, 1H), 2.36 - 2.17 (m, 6H), 2.15 - 2.04 (m, 2H), 1.73 - 1.57 (m, 6H). LCMS (ESI) calcd. for $C_{31}H_{36}FN_4O_2S^+$ $[M+H]^+$: 547.25, found, 547.2.

**Example 308: Preparation of 3-(4-((4-(4'-fluoro-3,4,5,6-tetrahydro-[1,1'-biphenyl]-2-carbonyl)piperazin-1-yl) methyl)-1-thioxoisoindolin-2-yl)piperidine-2,6-dione (GT-08294)**

With reference to the method of Scheme 4, the target compound GT-08294 was obtained (17 mg, white solid, yield 26%). $^1$H NMR (400 MHz, DMSO-d6) δ 11.20 (s, 1H), 10.79 (s, 1H), 7.96 (s, 1H), 7.86 (s, 1H), 7.64 (s, 1H), 7.35 - 7.18 (m, 2H), 7.18 - 7.05 (m, 2H), 5.99 (s, 1H), 5.18 (d, *J* = 16.3 Hz, 1H), 4.83 (d, *J* = 18.3 Hz, 1H), 4.48 - 4.19 (m, 2H), 3.86 - 3.64 (m, 1H), 3.04 - 2.95 (m, 3H), 2.74 - 2.54 (m, 4H), 2.45 - 2.34 (m, 3H), 2.20 - 2.00 (m, 4H), 1.79 - 1.56 (m, 5H). LCMS (ESI) calcd. for $C_{31}H_{34}FN_4O_3S^+$ $[M+H]^+$: 561.23, found, 561.2.

**Example 309: Preparation of 3-(4-((4-((4'-chloro-[1,1'-biphenyl]-2-yl)methyl)piperazin-1-yl)methyl)-1-thioxoisoindolin-2-yl)piperidine-2,6-dione (GT-08295)**

With reference to the method of Scheme 4, the target compound GT-08295 was obtained (20 mg, white solid, yield 31%). $^1$H NMR (400 MHz, DMSO-d6) δ 11.17 (s, 1H), 7.93 (d, *J* = 6.8 Hz, 1H), 7.65 - 7.58 (m, 1H), 7.55 - 7.50 (m, 3H), 7.49 - 7.43 (m, 2H), 7.43 - 7.38 (m, 3H), 7.33 - 7.25 (m, 1H), 5.98 (d, *J*= 11.5 Hz, 1H), 5.37 - 5.01 (m, 1H), 4.81 (d, *J* = 13.4 Hz, 1H), 4.27 - 4.11 (m, 4H), 3.73 - 3.58 (m, 2H), 3.25 - 3.08 (m, 4H), 3.06 - 2.91 (m, 3H), 2.68 (d, *J*= 17.1 Hz, 1H), 2.45 - 2.39 (m, 1H), 2.12 - 2.05 (m, 1H). LCMS (ESI) calcd. for $C_{31}H_{32}ClN_4O_2S^+$ $[M+H]^+$: 559.19, found, 559.1.

**Example 310: Preparation of 3-(4-((4-((4-(4-chlorophenyl)-6,6-dimethyl-5,6-dihydro-2H-pyran-3-yl)methyl)piperazin-1-yl)methyl)-1-thioxoisoindolin-2-yl)piperidine-2,6-dione (GT-08296)**

With reference to the method of Scheme 4, the target compound GT-08296 was obtained (17 mg, white solid, yield 25%). $^1$H NMR (400 MHz, DMSO-d6) δ 11.18 (s, 1H), 7.94 (d, *J* = 8.9 Hz, 1H), 7.90 - 7.72 (m, 1H), 7.62 (t, *J* = 7.5 Hz, 1H), 7.43 (d, *J* = 8.2 Hz, 2H), 7.19 (d, *J* = 7.9 Hz, 2H), 5.99 (d, *J* = 7.4 Hz, 1H), 5.27 - 5.03 (m, 1H), 4.81 (d, *J* = 18.7 Hz, 1H),

4.37 - 4.20 (m, 2H), 3.62 - 3.49 (m, 4H), 3.32 - 3.06 (m, 6H), 3.04 - 2.85 (m, 3H), 2.69 (d, *J* = 14.6 Hz, 1H), 2.43 - 2.38 (m, 1H), 2.20 (s, 2H), 2.15 - 2.05 (m, 1H), 1.21 (s, 6H). LCMS (ESI) calcd. for $C_{32}H_{38}ClN_4O_3S^+$ [M+H]$^+$: 593.23, found, 593.2.

**Example 311: Preparation of 3-(4-((4-(thiophen-2-yl)piperazin-1-yl)methyl)-1-thioxoisoindolin-2-yl)piperidine-2,6-dione (GT-08297)**

With reference to the method of Scheme 4, the target compound GT-08297 was obtained (16 mg, white solid, yield 31%). $^1$H NMR (400 MHz, DMSO-d6) δ 11.54 (s, 1H), 11.21 (s, 1H), 8.02 - 7.97 (m, 2H), 7.80 (d, *J*= 7.5 Hz, 1H), 7.68 (t, *J*= 7.5 Hz, 1H), 7.61 (d, *J*= 7.4 Hz, 1H), 7.53 (t, *J*= 7.6 Hz, 1H), 6.06 - 5.93 (m, 1H), 5.40 (d, *J* = 20.1 Hz, 1H), 4.91 (d, *J* = 20.4 Hz, 1H), 4.79 (d, *J* = 21.7 Hz, 1H), 4.59 - 4.36 (m, 1H), 3.66 - 3.58 (m, 2H), 3.33 - 3.20 (m, 4H), 3.04 - 2.92 (m, 1H), 2.69 (d, *J* = 16.4 Hz, 1H), 2.47 - 2.36 (m, 1H), 2.21 - 2.03 (m, 1H). LCMS (ESI) calcd. for $C_{22}H_{25}N_4O_2S_2^+$ [M+H]$^+$: 441.14, found, 441.1.

**Example 312: Preparation of 3-(4-((4-(5-methylthiophen-2-yl)piperazin-1-yl)methyl)-1-thioxoisoindolin-2-yl)piperidine-2,6-dione (GT-08305)**

With reference to the method of Scheme 4, the target compound GT-08305 was obtained (15 mg, white solid, yield 28%). $^1$H NMR (400 MHz, DMSO-d6) δ 11.19 (s, 1H), 8.05 - 7.91 (m, 2H), 7.68 (d, *J* = 7.7 Hz, 1H), 6.45 (d, *J* = 3.5 Hz, 1H), 6.05 (d, *J*= 3.5 Hz, 1H), 5.99 - 5.89 (m, 1H), 5.39 (d, *J*= 20.1 Hz, 1H), 4.86 (d, *J* = 20.8 Hz, 1H), 4.64 - 4.16 (m, 2H), 3.73 - 3.60 (m, 4H), 3.39 - 3.12 (m, 4H), 3.06 - 2.90 (m, 1H), 2.69 (d, *J* = 16.7 Hz, 1H), 2.50 (s, 3H), 2.48 - 2.39 (m, 1H), 2.18 - 2.04 (m, 1H). LCMS (ESI) calcd. for $C_{23}H_{27}N_4O_2S_2^+$ [M+H]$^+$: 455.16, found, 455.1.

Example 313: Preparation of 3-(4-((4-(thiophen-3-yl)piperazin-1-yl)methyl)-1-thioxoisoindolin-2-yl)piperidine-2,6-dione (GT-08313)

With reference to the method of Scheme 4, the target compound GT-08313 was obtained (4 mg, white solid, yield 8%). LCMS (ESI) calcd. for $C_{22}H_{25}N_4O_2S_2^+$ [M+H]$^+$: 441.14, found, 441.1.

**Example 314: Preparation of 3-(4-((4-(4-methylthiophen-3-yl)piperazin-1-yl)methyl)-1-thioxoisoindolin-2-yl)piperidine-2,6-dione (GT-08306)**

With reference to the method of Scheme 4, the target compound GT-08306 was obtained (17 mg, white solid, yield 32%). $^1$H NMR (400 MHz, DMSO-d6) δ 11.36 (s, 1H), 11.21 (s, 1H), 8.05 (d, *J*= 7.6 Hz, 1H), 8.00 (d, *J*= 7.7 Hz, 1H), 7.68 (t, *J*= 7.7 Hz, 1H), 7.13 (d, *J*= 2.3 Hz, 1H), 6.80 (d, *J*= 3.3 Hz, 1H), 6.02 (d, *J* = 8.5 Hz, 1H), 5.39 (d, *J* = 20.4 Hz, 1H), 4.94 (d, *J*= 20.3 Hz, 1H), 4.61 - 4.41 (m, 2H), 3.36 - 3.28 (m, 6H), 3.20 - 2.90 (m, 4H), 2.73 - 2.67 (m, 1H), 2.47 - 2.38 (m, 1H), 2.11 (s, 3H). LCMS (ESI) calcd. for $C_{23}H_{27}N_4O_2S_2^+$ [M+H]$^+$: 455.16, found, 455.1.

**Example 315: Preparation of 3-(4-((4-(5-methylthiophen-3-yl)piperazin-1-yl)methyl)-1-thioxoisoindolin-2-yl)piperidine-2,6-dione (GT-08298)**

With reference to the method of Scheme 4, the target compound GT-08298 was obtained (17 mg, white solid, yield 32%). $^1$H NMR (400 MHz, DMSO-d6) δ 11.48 (s, 1H), 11.21 (s, 1H), 8.08 - 7.96 (m, 2H), 7.72 - 7.66 (m, 1H), 6.69 (s, 1H), 6.19 (s, 1H), 6.07 - 5.90 (m, 1H), 5.42 (d, *J* = 20.4 Hz, 1H), 4.91 (d, *J* = 20.8 Hz, 1H), 4.60 - 4.32 (m, 2H), 3.63 - 3.59 (m, 2H), 3.53 - 3.42 (m, 2H), 3.04 - 2.93 (m, 1H), 2.69 (d, *J*= 18.0 Hz, 1H), 2.45 - 2.40 (m, 1H), 2.37 (s, 3H), 2.16 - 2.07 (m, 1H). LCMS (ESI) calcd. for $C_{23}H_{27}N_4O_2S_2^+$ [M+H]$^+$: 455.16, found, 455.1.

**Example 316: Preparation of 3-(4-((4-(thiophen-2-yl)-3,6-dihydropyridin-1(2H)-yl)methyl)-1-thioxoisoindolin-2-yl)piperidine-2,6-dione (GT-08299)**

With reference to the method of Scheme 4, the target compound GT-08299 was obtained (16 mg, white solid, yield 31%). $^1$H NMR (400 MHz, DMSO-d6) δ 11.20 (s, 1H), 10.99 (s, 1H), 8.07 - 7.96 (m, 2H), 7.69 (t, *J* = 7.4 Hz, 1H), 7.49 (d, *J* = 4.8 Hz, 1H), 7.18 (s, 1H), 7.10 - 7.00 (m, 1H), 6.06 (s, 1H), 6.04 - 5.94 (m, 1H), 5.36 - 5.13 (m, 1H), 4.99 - 4.86 (m, 1H), 4.69 - 4.30 (m, 2H), 3.94 - 3.85 (m, 2H), 3.72 - 3.60 (m, 1H), 3.12 - 2.97 (m, 1H), 2.95 - 2.77 (m, 3H), 2.75 - 2.65 (m, 1H), 2.46 - 2.35 (m, 1H), 2.13 - 2.01 (m, 1H). LCMS (ESI) calcd. for $C_{23}H_{24}N_3O_2S_2^+$ [M+H]$^+$: 438.13, found, 438.1.

**Example 317: Preparation of 3-(4-((4-(5-methylthiophen-2-yl)-3,6-dihydropyridin-1(2H)-yl)methyl)-1-thioxoisoindolin-2-yl)piperidine-2,6-dione (GT-08307)**

With reference to the method of Scheme 4, the target compound GT-08307 was obtained (14 mg, white solid,

yield 26%). $^1$H NMR (400 MHz, DMSO-d6) δ 11.20 (s, 1H), 10.90 (s, 1H), 8.08 - 7.91 (m, 2H), 7.69 (t, $J$ = 7.6 Hz, 1H), 6.95 (d, $J$= 2.8 Hz, 1H), 6.74 (d, $J$= 2.3 Hz, 1H), 6.10 - 5.84 (m, 3H), 5.37 - 5.12 (m, 1H), 4.92 (dd, $J$= 20.9, 7.3 Hz, 1H), 4.61 - 4.41 (m, 2H), 3.91 - 3.78 (m, 2H), 3.67 - 3.63 (m, 1H), 3.04 - 2.93 (m, 2H), 2.75 - 2.63 (m, 3H), 2.41 (s, 3H), 2.20 - 2.01 (m, 1H). LCMS (ESI) calcd. for $C_{24}H_{26}N_3O_2S_2^+$ [M+H]$^+$: 452.15, found, 452.1.

Example 318: Preparation of 3-(4-((4-(thiophen-3-yl)-3,6-dihydropyridin-1(2H)-yl)methyl)-1-thioxoisoindolin-2-yl)piperidine-2,6-dione (GT-08300)

With reference to the method of Scheme 4, the target compound GT-08300 was obtained (14 mg, white solid, yield 27%). $^1$H NMR (400 MHz, DMSO-d6) δ 11.20 (s, 1H), 11.12 (s, 1H), 8.10 - 7.97 (m, 2H), 7.69 (t, $J$= 7.2 Hz, 1H), 7.55 (dd, $J$ = 9.9, 6.1 Hz, 2H), 7.37 (d, $J$ = 4.6 Hz, 1H), 6.16 (s, 1H), 6.09 - 5.92 (m, 1H), 5.43 - 5.19 (m, 1H), 4.93 (dd, $J$ = 20.0, 5.0 Hz, 1H), 4.60 - 4.42 (m, 2H), 3.97 - 3.79 (m, 1H), 3.63 - 2.57 (m, 1H), 3.07 - 2.93 (m, 3H), 2.78 - 2.67 (m, 3H), 2.48 - 2.38 (m, 1H), 2.19 - 2.07 (m, 1H). LCMS (ESI) calcd. for $C_{23}H_{24}N_3O_2S_2^+$ [M+H]$^+$: 438.13, found, 438.1.

**Example 319: Preparation of 3-(4-((4-(4-methylthiophen-3-yl)-3,6-dihydropyridin-1(2H)-yl)methyl)-1-thioxoisoindolin-2-yl)piperidine-2,6-dione (GT-08308)**

With reference to the method of Scheme 4, the target compound GT-08308 was obtained (16 mg, white solid, yield 30%). $^1$H NMR (400 MHz, DMSO-d6) δ 11.21 (s, 1H), 10.95 (s, 1H), 8.00 (t, $J$ = 10.4 Hz, 2H), 7.69 (t, $J$= 7.6 Hz, 1H), 7.42 (brs, 1H), 7.20 (brs, 1H), 6.10 - 5.91 (m, 1H), 5.80 (s, 1H), 5.39 - 5.15 (m, 1H), 5.04 - 4.88 (m, 1H), 4.61 - 4.41 (m, 2H), 3.91 - 3.79 (m, 2H), 3.67 - 3.59 (m, 1H), 3.09 - 2.81 (m, 3H), 2.72 - 2.60 (m, 3H), 2.23 (s, 3H), 2.15 - 2.10 (m, 1H). LCMS (ESI) calcd. for $C_{24}H_{26}N_3O_2S_2^+$ [M+H]$^+$: 452.15, found, 452.1.

**Example 320: Preparation of 3-(4-((4-(5-methylthiophen-3-yl)-3,6-dihydropyridin-1(2H)-yl)methyl)-1-thioxoisoindolin-2-yl)piperidine-2,6-dione (GT-08318)**

With reference to the method of Scheme 4, the target compound GT-08318 was obtained (12 mg, white solid, yield 23%). $^1$H NMR (400 MHz, DMSO-d6) δ 11.20 (s, 1H), 10.84 (s, 1H), 8.02 - 7.97 (m, 2H), 7.69 (t, $J$ = 7.8 Hz, 1H), 7.27 (s, 1H), 7.05 (s, 1H), 6.12 - 5.96 (m, 2H), 5.23 (dd, $J$ = 29.8, 20.5 Hz, 1H), 4.92 (dd, $J$= 20.2, 5.6 Hz, 1H), 4.67 - 4.46 (m, 2H), 3.96 - 3.75 (m, 2H), 3.70 - 3.57 (m, 1H), 3.06 - 2.95 (m, 1H), 2.89 - 2.77 (m, 1H), 2.77 - 2.64 (m, 3H), 2.42 (s, 3H), 2.21 - 2.03 (m, 2H). LCMS (ESI) calcd. for $C_{24}H_{26}N_3O_2S_2^+$ [M+H]$^+$: 452.15, found, 452.1.

**Example 321: Preparation of 3-(4-((4-(furan-2-yl)-3,6-dihydropyridin-1(2H)-yl)methyl)-1-thioxoisoindolin-2-yl) piperidine-2,6-dione (GT-08311)**

With reference to the method of Scheme 4, the target compound GT-08311 was obtained (14 mg, white solid, yield 28%). $^1$H NMR (400 MHz, DMSO-d6) δ 11.26 (s, 1H), 11.20 (s, 1H), 8.04 - 7.99 (m, 2H), 7.70 - 7.67 (m, 2H), 6.53 (d, $J$ = 3.2 Hz, 2H), 6.09 (s, 1H), 6.03 - 6.00 (m, 1H), 5.37 - 3.24 (m, 1H), 4.99 - 4.84 (m, 1H), 4.65 - 4.37 (m, 2H), 3.93 - 3.77 (m, 2H), 3.68 - 3.61 (m, 1H), 3.08 - 2.93 (m, 1H), 2.91 - 2.83 (m, 1H), 2.73 - 2.54 (m, 3H), 2.47 - 2.41 (m, 1H), 2.19 - 2.07 (m, 1H). LCMS (ESI) calcd. for $C_{23}H_{24}N_3O_3S^+$ [M+H]$^+$: 422.15, found, 422.1.

**Example 322: Preparation of 3-(4-((4-(furan-3-yl)-3,6-dihydropyridin-1(2H)-yl)methyl)-1-thioxoisoindolin-2-yl) piperidine-2,6-dione (GT-08319)**

With reference to the method of Scheme 4, the target compound GT-08319 was obtained (14 mg, white solid, yield 28%). $^1$H NMR (400 MHz, DMSO-d6) δ 11.20 (s, 1H), 10.91 (s, 1H), 8.01 - 7.97 (m, 2H), 7.83 (s, 1H), 7.74 - 7.63 (m, 2H), 6.75 (s, 1H), 6.10 - 5.93 (m, 2H), 5.24 (dd, $J$ = 26.7, 19.8 Hz, 1H), 5.01 - 4.85 (m, 1H), 4.62 - 4.41 (m, 2H), 3.96 - 3.73 (m, 2H), 3.63 - 2.57 (m, 1H), 3.03 - 2.89 (m, 1H), 2.87 - 2.75 (m, 1H), 2.74 - 2.62 (m, 2H), 2.48 - 2.35 (m, 2H), 2.20 - 2.05 (m, 1H). LCMS (ESI) calcd. for $C_{23}H_{24}N_3O_3S^+$ [M+H]$^+$: 422.15, found, 422.1.

**Example 323: Preparation of 3-(4-((4-(thiophen-2-yl)piperidin-1-yl)methyl)-1-thioxoisoindolin-2-yl)piperidine-2,6-dione (GT-08320)**

With reference to the method of Scheme 4, the target compound GT-08320 was obtained (14 mg, white solid, yield 27%). $^1$H NMR (400 MHz, DMSO-d6) δ 11.21 (s, 1H), 10.88 (s, 1H), 8.00 (d, $J$= 7.6 Hz, 2H), 7.68 (t, $J$ = 7.6 Hz, 1H), 7.38 (d, $J$ = 5.0 Hz, 1H), 6.97 (dd, $J$= 4.9, 3.6 Hz, 1H), 6.90 (d, $J$ = 3.3 Hz, 1H), 6.12 - 5.93 (m, 2H), 5.30 (d, $J$ = 20.3 Hz, 1H), 4.91 (d, $J$= 20.3 Hz, 1H), 4.50 - 4.32 (m, 2H), 3.52 - 2.42 (m, 2H), 3.23 - 3.17 (m, 3H), 3.02 - 2.93 (m, 1H), 2.69 (d, $J$ = 17.6 Hz, 2H), 2.20 - 1.98 (m, 6H). LCMS (ESI) calcd. for $C_{23}H_{26}N_3O_2S_2^+$ [M+H]$^+$: 440.15, found, 440.1.

**Example 324: Preparation of 3-(4-((4-(5-methylthiophen-2-yl)piperidin-1-yl)methyl)-1-thioxoisoindolin-2-yl)piperidine-2,6-dione (GT-08312)**

With reference to the method of Scheme 4, the target compound GT-08312 was obtained (14 mg, white solid, yield 26%). $^1$H NMR (400 MHz, DMSO-d6) δ 11.21 (s, 1H), 10.68 (s, 1H), 7.98 (dd, *J* = 12.4, 7.8 Hz, 2H), 7.68 (t, *J* = 7.7 Hz, 1H), 6.64 (dd, *J* = 15.2, 6.0 Hz, 2H), 6.04 - 5.98 (m, 1H), 5.26 (d, *J* = 20.3 Hz, 1H), 4.90 (d, *J* = 20.2 Hz, 1H), 4.47 - 4.29 (m, 2H), 3.54 - 3.40 (m, 2H), 3.20 - 3.13 (m, 2H), 3.04 - 2.89 (m, 2H), 2.76 - 2.64 (m, 1H), 2.38 (s, 3H), 2.23 - 1.88 (m, 6H). LCMS (ESI) calcd. for $C_{24}H_{28}N_3O_2S_2^+$ [M+H]$^+$: 454.16, found, 454.1.

**Example 325: Preparation of 3-(4-((4-(3-methylthiophen-2-yl)piperidin-1-yl)methyl)-1-thioxoisoindolin-2-yl)piperidine-2,6-dione (GT-08314)**

With reference to the method of Scheme 4, the target compound GT-08314 was obtained (9 mg, white solid, yield 17%). $^1$H NMR (400 MHz, DMSO-d6) δ 11.21 (s, 1H), 10.59 (s, 1H), 8.00 (d, *J* = 7.6 Hz, 1H), 7.96 (d, *J* = 7.7 Hz, 1H), 7.69 (t, *J* = 7.7 Hz, 1H), 7.26 (d, *J* = 5.0 Hz, 1H), 6.83 (d, *J* = 5.0 Hz, 1H), 6.12 - 5.92 (m, 1H), 5.22 (d, *J* = 20.3 Hz, 1H), 4.91 (d, *J* = 20.2 Hz, 1H), 4.51 - 4.27 (m, 2H), 3.57 - 3.42 (m, 2H), 3.25 - 3.12 (m, 3H), 3.04 - 2.96 (m, 1H), 2.69 (d, *J* = 19.1 Hz, 1H), 2.49 - 2.36 (m, 3H), 2.17 (s, 3H), 2.01 - 1.98 (m, 4H). LCMS (ESI) calcd. for $C_{24}H_{28}N_3O_2S_2^+$ [M+H]$^+$: 454.16, found, 454.1.

**Example 326: Preparation of 3-(4-((4-(thiophen-3-yl)piperidin-1-yl)methyl)-1-thioxoisoindolin-2-yl)piperidine-2,6-dione (GT-08315)**

With reference to the method of Scheme 4, the target compound GT-08315 was obtained (12 mg, white solid, yield 23%). $^1$H NMR (400 MHz, DMSO-d6) δ 11.21 (s, 1H), 10.79 (s, 1H), 8.00 (d, *J* = 7.6 Hz, 2H), 7.68 (t, *J* = 7.7 Hz, 1H), 7.50 (dd, *J* = 4.8, 2.9 Hz, 1H), 7.22 (s, 1H), 7.05 (dd, *J* = 5.0, 1.2 Hz, 1H), 6.09 - 5.93 (m, 1H), 5.30 (d, *J* = 20.3 Hz, 1H), 4.91 (d, *J* = 20.4 Hz, 1H), 4.53 - 4.27 (m, 2H), 3.46 (dd, *J* = 28.5, 12.0 Hz, 2H), 3.23 - 3.11 (m, 2H), 3.03 - 2.95 (m, 3H), 2.69 (d, *J* = 18.8 Hz, 1H), 2.16 - 1.97 (m, 5H). LCMS (ESI) calcd. for $C_{23}H_{26}N_3O_2S_2^+$ [M+H]$^+$: 440.15, found, 440.1.

**Example 327: Preparation of 3-(4-((4-(5-methylthiophen-3-yl)piperidin-1-yl)methyl)-1-thioxoisoindolin-2-yl)piperidine-2,6-dione (GT-08316)**

With reference to the method of Scheme 4, the target compound GT-08316 was obtained (11 mg, white solid, yield 21%). $^1$H NMR (400 MHz, DMSO-d6) δ 11.21 (s, 1H), 10.68 (s, 1H), 7.99 (t, *J* = 7.6 Hz, 2H), 7.68 (t, *J* = 7.7 Hz, 1H), 6.92 (s, 1H), 6.72 (s, 1H), 6.05 - 5.97 (m, 1H), 5.27 (d, *J* = 20.4 Hz, 1H), 4.90 (d, *J* = 19.7 Hz, 1H), 4.50 - 4.30 (m, 2H), 3.57 - 3.41 (m, 2H), 3.19 - 3.10 (m, 3H), 3.03 - 2.97 (m, 1H), 2.78 - 2.66 (m, 2H), 2.40 (s, 3H), 2.21 - 2.09 (m, 2H), 2.01 - 1.92 (m, 3H). LCMS (ESI) calcd. for $C_{24}H_{28}N_3O_2S_2^+$ [M+H]$^+$: 454.16, found, 454.1.

**Example 328: Preparation of 3-(4-((4-(4-methylthiophen-3-yl)piperidin-1-yl)methyl)-1-thioxoisoindolin-2-yl)piperidine-2,6-dione (GT-08309)**

With reference to the method of Scheme 4, the target compound GT-08309 was obtained (12 mg, white solid, yield 22%). $^1$H NMR (400 MHz, DMSO-d6) δ 11.21 (s, 1H), 10.84 (s, 1H), 8.00 (d, *J* = 7.5 Hz, 2H), 7.68 (t, *J* = 7.6 Hz, 1H), 7.12 (dd, *J* = 14.6, 5.4 Hz, 2H), 6.05 - 5.98 (m, 1H), 5.29 (d, *J* = 19.9 Hz, 1H), 4.91 (d, *J* = 20.9 Hz, 1H), 4.67 (d, *J* = 22.5 Hz, 1H), 4.52 - 4.30 (m, 2H), 3.58 - 3.42 (m, 3H), 3.24 - 3.10 (m, 3H), 3.08 - 2.92 (m, 2H), 2.82 - 2.79 (m, 1H), 2.70 (d, *J* = 18.9 Hz, 2H), 2.17 (s, 3H), 2.01 - 1.98 (m, 1H). LCMS (ESI) calcd. for $C_{24}H_{28}N_3O_2S_2^+$ [M+H]$^+$: 454.16, found, 454.1.

**Example 329: Preparation of 3-(4-((4-(furan-2-yl)piperidin-1-yl)methyl)-1-thioxoisoindolin-2-yl)piperidine-2,6-dione (GT-08310)**

With reference to the method of Scheme 4, the target compound GT-08310 was obtained (7 mg, white solid, yield 14%). $^1$H NMR (400 MHz, DMSO-d6) δ 11.21 (s, 1H), 10.80 (s, 1H), 8.01 - 7.97 (m, 2H), 7.68 (t, *J* = 7.6 Hz, 1H), 7.56 (s, 1H), 6.38 (s, 1H), 6.14 (d, *J* = 2.5 Hz, 1H), 6.01 (d, *J* = 9.6 Hz, 1H), 5.27 (dd, *J* = 22.7, 12.7 Hz, 1H), 4.90 (d, *J* = 20.2 Hz, 1H), 4.53 - 4.26 (m, 2H), 3.57 - 3.41 (m, 2H), 3.25 - 3.12 (m, 2H), 3.08 - 2.86 (m, 3H), 2.69 (d, *J* = 17.6 Hz, 1H), 2.15 - 2.09 (m, 3H), 2.01 - 1.92 (m, 2H). LCMS (ESI) calcd. for $C_{23}H_{26}N_3O_3S^+$ [M+H]$^+$: 424.17, found, 424.1.

**Example 330: Preparation of 3-(4-((4-(furan-3-yl)piperidin-1-yl)methyl)-1-thioxoisoindolin-2-yl)piperidine-2,6-dione (GT-08317)**

With reference to the method of Scheme 4, the target compound GT-08317 was obtained (7 mg, white solid, yield

14%). $^1$H NMR (400 MHz, DMSO-d6) δ 11.21 (s, 1H), 10.66 (s, 1H), 7.99 (t, *J*= 7.8 Hz, 2H), 7.68 (t, *J* = 7.6 Hz, 2H), 7.60 (s, 1H), 7.50 (s, 1H), 6.09 - 5.94 (m, 1H), 5.26 (d, *J* = 20.5 Hz, 1H), 4.90 (d, *J* = 20.1 Hz, 1H), 4.52 - 4.26 (m, 2H), 3.49 - 3.40 (m, 2H), 3.19 - 3.13 (m, 2H), 3.02 - 2.93 (m, 1H), 2.72 - 2.67 (m, 2H), 2.25 - 1.99 (m, 4H), 1.92 - 1.84 (m, 2H). LCMS (ESI) calcd. for $C_{23}H_{26}N_3O_3S^+$ [M+H]$^+$: 424.17, found, 424.1.

**Example 331: Preparation of 3-(4-((4-(2,3-dichlorophenyl)piperazin-1-yl)methyl)-1-thioxoisoindolin-2-yl)piperidine-2,6-dione (GT-08258)**

With reference to the method of Scheme 4, the target compound GT-08258 was obtained (6 mg, white solid, yield 11%). $^1$H NMR (400 MHz, DMSO-d6) δ 11.22 (s, 1H), 11.09 (s, 1H), 8.02 (t, *J*= 7.1 Hz, 2H), 7.69 (t, *J* = 7.7 Hz, 1H), 7.46 - 7.30 (m, 2H), 7.19 (d, *J* = 6.8 Hz, 1H), 6.02 (d, *J* = 13.1 Hz, 1H), 5.31 (d, *J*= 20.3 Hz, 1H), 4.94 (d, *J* = 20.8 Hz, 1H), 4.53 (s, 2H), 3.56 - 3.41 (m, 4H), 3.30 - 3.14 (m, 4H), 3.04 - 2.93 (m, 1H), 2.69 (d, *J* = 19.0 Hz, 1H), 2.44 - 2.33 (m, 1H), 2.19 - 2.05 (m, 1H). LCMS (ESI) calcd. for $C_{24}H_{25}Cl_2N_4O_2S^+$ [M+H]$^+$: 503.11, found, 503.1.

**Example 332: Preparation of 3-(4-((4-phenylpiperazin-1-yl)methyl)-1-thioxoisoindolin-2-yl)piperidine-2,6-dione (GT-08259)**

With reference to the method of Scheme 4, the target compound GT-08259 was obtained (12 mg, white solid, yield 23%). $^1$H NMR (400 MHz, DMSO-d6) δ 11.49 (s, 1H), 11.21 (s, 1H), 8.04 (d, *J* = 5.9 Hz, 1H), 8.00 (d, *J*= *7.7* Hz, 1H), 7.68 (t, *J* = 7.7 Hz, 1H), 7.28 - 7.23 (m, 2H), 6.98 (d, *J*= 7.9 Hz, 2H), 6.86 (t, *J* = 7.3 Hz, 1H), 6.02 (d, *J* = 8.3 Hz, 1H), 5.41 (dd, *J* = 22.2, 4.9 Hz, 1H), 4.93 (d, *J* = 20.4 Hz, 1H), 4.54 - 4.43 (m, 2H), 3.60 - 3.49 (m, 2H), 3.42 - 3.21 (m, 6H), 3.06 - 2.94 (m, 1H), 2.69 (d, *J*= 16.9 Hz, 1H), 2.48 - 2.39 (m, 1H), 2.19 - 2.08 (m, 1H). LCMS (ESI) calcd. for $C_{24}H_{27}N_4O_2S^+$ [M+H]$^+$: 435.18, found, 435.1.

**Example 333: Preparation of 3-(4-((4-(2-methoxyphenyl)piperazin-1-yl)methyl)-1-thioxoisoindolin-2-yl)piperidine-2,6-dione (GT-08260)**

With reference to the method of Scheme 4, the target compound GT-08260 was obtained (15 mg, white solid, yield 27%). $^1$H NMR (400 MHz, DMSO-d6) δ 11.42 (s, 1H), 11.21 (s, 1H), 8.00 (d, *J*= 7.2 Hz, 1H), 7.93 (d, *J* = 7.5 Hz, 1H), 7.70 (t, *J* = 7.8 Hz, 1H), 7.04 - 7.01 (m, 2H), 6.95 - 6.88 (m, 2H), 6.02 (d, *J* = 9.7 Hz, 1H), 5.42 *(d, J*= 20.4 Hz, 1H), 4.94 (d, *J* = 20.4 Hz, 1H), 4.69 - 4.44 (m, 2H), 3.79 (s, 3H), 3.58 - 3.40 (m, 4H), 3.39 - 3.28 (m, 2H), 3.25 - 3.10 (m, 2H), 3.05 - 2.83 (m, 2H), 2.74 - 2.58 (m, 1H), 2.23 - 2.04 (m, 1H). LCMS (ESI) calcd. for $C_{25}H_{29}N_4O_3S^+$ [M+H]$^+$: 465.20, found, 465.1.

**Example 334: Preparation of 3-(4-((4-(3-methoxyphenyl)piperazin-1-yl)methyl)-1-thioxoisoindolin-2-yl)piperidine-2,6-dione (GT-08304)**

With reference to the method of Scheme 4, the target compound GT-08304 was obtained (12 mg, white solid, yield 22%). $^1$H NMR (400 MHz, DMSO-d6) δ 11.32 (s, 1H), 11.21 (s, 1H), 8.06 - 7.96 (m, 2H), 7.69 (t, *J* = 7.7 Hz, 1H), 7.16 (t, *J* = 8.2 Hz, 1H), 6.56 (dd, *J* = 8.2, 2.0 Hz, 1H), 6.50 (t, *J*= 2.2 Hz, 1H), 6.44 (dd, *J* = 8.1, 2.1 Hz, 1H), 6.02 (d, *J* = 9.0 Hz, 1H), 5.37 (d, *J*= 19.4 Hz, 1H), 4.92 (d, *J*= 20.4 Hz, 1H), 4.58 - 4.39 (m, 2H), 3.82 (d, *J*= 10.2 Hz, 2H), 3.73 (s, 3H), 3.49 - 3.46 (m, 2H), 3.33 - 3.13 (m, 4H), 3.06 - 2.94 (m, 1H), 2.69 (d, *J* = 17.4 Hz, 1H), 2.47 - 2.41 (m, 1H), 2.18 - 2.07 (m, 1H). LCMS (ESI) calcd. for $C_{25}H_{29}N_4O_3S^+$ [M+H]$^+$: 465.20, found, 465.1.

**Example 335: Preparation of 3-(4-((4-(4-methoxyphenyl)piperazin-1-yl)methyl)-1-thioxoisoindolin-2-yl)piperidine-2,6-dione (GT-08495)**

With reference to the method of Scheme 4, the target compound GT-08495 was obtained (18 mg, white solid, yield 33%). $^1$H NMR (400 MHz, DMSO-d6) δ 11.28 (s, 1H), 11.21 (s, 1H), 8.01 (t, *J* = 7.6 Hz, 2H), 7.68 (t, *J* = 7.7 Hz, 1H), 6.94 (d, *J* = 8.8 Hz, 2H), 6.86 (d, *J* = 9.1 Hz, 2H), 6.02 (d, *J* = 8.5 Hz, 1H), 5.37 (d, *J*= 20.4 Hz, 1H), 4.92 (d, *J* = 20.4 Hz, 1H), 4.59 - 4.39 (m, 2H), 3.72 (s, 3H), 3.63 - 3.60 (m, 3H), 3.40 - 3.26 (m, 3H), 3.22 - 3.08 (m, 2H), 3.06 - 2.92 (m, 1H), 2.69 (d, *J* = 18.0 Hz, 1H), 2.45 - 2.32 (m, 1H), 2.18 - 2.08 (m, 1H). LCMS (ESI) calcd. for $C_{25}H_{29}N_4O_3S^+$ [M+H]$^+$: 465.20, found, 465.1.

**Example 336: Preparation of 3-(1-thioxo-4-((4-(4-(trifluoromethyl)phenyl)piperazin-1-yl)methyl)isoindolin-2-yl)piperidine-2,6-dione (GT-08262)**

With reference to the method of Scheme 4, the target compound GT-08262 was obtained (11 mg, white solid, yield 22%). $^1$H NMR (400 MHz, DMSO-d6) δ 11.35 (s, 1H), 11.21 (s, 1H), 8.00 (d, *J*= 7.2 Hz, 2H), 7.69 (t, *J* = 7.5 Hz, 1H), 7.57 (d, *J* = 8.5 Hz, 3H), 7.13 (d, *J* = 8.4 Hz, 2H), 6.02 (d, *J* = 9.7 Hz, 1H), 5.35 (d, *J*= 21.2 Hz, 1H), 4.91 (d, *J* = 20.4 Hz, 1H),

4.59 - 4.39 (m, 2H), 4.01 (d, *J* = 12.3 Hz, 2H), 3.55 - 3.49 (m, 2H), 3.33 - 3.18 (m, 4H), 3.06 - 2.93 (m, 1H), 2.69 (d, *J* = 15.8 Hz, 1H), 2.48 - 2.39 (m, 1H), 2.18 - 2.07 (m, 1H). LCMS (ESI) calcd. for $C_{25}H_{26}F_3N_4O_2S^+$ [M+H]$^+$: 503.17, found, 503.1.

**Example 337: Preparation of 3-(4-((4-(3-fluorophenyl)piperazin-1-yl)methyl)-1-thioxoisoindolin-2-yl)piperidine-2,6-dione (GT-08263)**

With reference to the method of Scheme 4, the target compound GT-08263 was obtained (15 mg, white solid, yield 28%). $^1$H NMR (400 MHz, DMSO-d6) δ 11.42 (s, 1H), 11.21 (s, 1H), 8.00 (d, *J* = 7.6 Hz, 2H), 7.68 (t, *J*= 7.7 Hz, 1H), 7.30 - 7.24 (m, 1H), 6.82 (t, *J* = 9.9 Hz, 2H), 6.63 (t, *J*= 8.3 Hz, 1H), 6.02 (d, *J* = 7.9 Hz, 1H), 5.44 - 5.28 (m, 1H), 4.91 (d, *J* = 20.3 Hz, 1H), 4.60 - 4.37 (m, 2H), 3.88 (d, *J*= 9.8 Hz, 2H), 3.56 - 3.44 (m, 2H), 3.31 - 3.22 (m, 4H), 3.06 - 2.94 (m, 1H), 2.69 (d, *J*= 17.0 Hz, 1H), 2.47 - 2.39 (m, 1H), 2.19 - 2.09 (m, 1H). LCMS (ESI) calcd. for $C_{24}H_{26}FN_4O_2S^+$ [M+H]$^+$: 453.18, found, 453.1.

**Example 338: Preparation of 3-(4-((4-(4-fluorophenyl)piperazin-1-yl)methyl)-1-thioxoisoindolin-2-yl)piperidine-2,6-dione (GT-08264)**

With reference to the method of Scheme 4, the target compound GT-08264 was obtained (15 mg, white solid, yield 28%). $^1$H NMR (400 MHz, DMSO-d6) δ 11.45 (s, 1H), 11.21 (s, 1H), 8.00 (d, *J* = 7.6 Hz, 1H), 7.93 (d, *J*= 7.6 Hz, 1H), 7.71 (t, *J* = 8.1 Hz, 1H), 7.12 - 7.08 (m, 2H), 7.05 - 6.95 (m, 2H), 6.02 (d, *J* = 10.4 Hz, 1H), 5.40 (d, *J*= 21.0 Hz, 1H), 4.92 (d, *J*= 20.2 Hz, 1H), 4.69 - 4.47 (m, 2H), 3.73 - 3.70 (m, 2H), 3.59 - 3.41 (m, 2H), 3.39 - 3.14 (m, 4H), 3.06 - 2.84 (m, 1H), 2.73 - 2.67 (m, 1H), 2.47 - 2.37 (m, 1H), 2.18 - 2.12 (m, 1H). LCMS (ESI) calcd. for $C_{24}H_{26}FN_4O_2S^+$ [M+H]$^+$: 453.18, found, 453.1.

**Example 339: Preparation of 3-(4-((4-(2-chlorophenyl)piperazin-1-yl)methyl)-1-thioxoisoindolin-2-yl)piperidine-2,6-dione (GT-08265)**

With reference to the method of Scheme 4, the target compound GT-08265 was obtained (14 mg, white solid, yield 25%). $^1$H NMR (400 MHz, DMSO-d6) δ 11.29 (s, 1H), 11.21 (s, 1H), 8.02 (dd, *J* = 16.8, 7.4 Hz, 2H), 7.69 (t, *J* = 7.6 Hz, 1H), 7.45 (d, *J*= 7.7 Hz, 1H), 7.34 (t, *J* = 7.5 Hz, 1H), 7.19 (d, *J* = 7.8 Hz, 1H), 7.11 (t, *J*= 7.3 Hz, 1H), 6.02 (d, *J* = 9.3 Hz, 1H), 5.35 (d, *J* = 20.2 Hz, 1H), 4.94 (d, *J* = 20.7 Hz, 1H), 4.59 - 4.42 (m, 2H), 3.59 - 3.41 (m, 4H), 3.31 - 3.16 (m, 4H), 3.03 - 2.97 (m, 1H), 2.75 - 2.65 (m, 1H), 2.46 - 2.37 (m, 1H), 2.18 - 2.12 (m, 1H). LCMS (ESI) calcd. for $C_{24}H_{26}ClN_4O_2S^+$ [M+H]$^+$: 469.15, found, 469.1.

**Example 340: Preparation of 3-(4-((4-(4-chlorophenyl)piperazin-1-yl)methyl)-1-thioxoisoindolin-2-yl)piperidine-2,6-dione (GT-08266)**

With reference to the method of Scheme 4, the target compound GT-08266 was obtained (13 mg, white solid, yield 24%). $^1$H NMR (400 MHz, DMSO-d6) δ 11.36 (s, 1H), 11.21 (s, 1H), 8.00 (d, *J* = 7.6 Hz, 2H), 7.68 (t, *J* = 7.6 Hz, 1H), 7.29 (d, *J* = 8.9 Hz, 2H), 7.00 (d, *J* = 9.0 Hz, 2H), 6.02 (d, *J* = 7.9 Hz, 1H), 5.36 (d, *J* = 20.9 Hz, 1H), 4.91 (d, *J= 20.3* Hz, 1H), 4.59 - 4.28 (m, 2H), 3.81 (d, *J* = 10.3 Hz, 2H), 3.58 - 3.44 (m, 2H), 3.30 - 3.19 (m, 4H), 3.08 - 2.95 (m, 2H), 2.75 - 2.66 (m, 1H), 2.48 - 2.40 (m, 1H), 2.15 - 2.11 (m, 1H). LCMS (ESI) calcd. for $C_{24}H_{26}ClN_4O_2S^+$ [M+H]$^+$: 469.15, found, 469.1.

**Example 341: Preparation of 3-(4-((4-(2-bromophenyl)piperazin-1-yl)methyl)-1-thioxoisoindolin-2-yl)piperidine-2,6-dione (GT-08267)**

With reference to the method of Scheme 4, the target compound GT-082670 was obtained (16 mg, white solid, yield 27%). $^1$H NMR (400 MHz, DMSO-d6) δ 11.30 (s, 1H), 11.21 (s, 1H), 8.06 - 8.00 (m, 2H), 7.69 (t, *J*= 7.6 Hz, 1H), 7.63 (d, *J* = 8.0 Hz, 1H), 7.39 (t, *J* = 7.7 Hz, 1H), 7.20 (d, *J* = 7.9 Hz, 1H), 7.05 (t, *J*= 7.2 Hz, 1H), 6.02 (d, *J*= 14.1 Hz, 1H), 5.33 - 5.26 (m, 1H), 4.94 (d, *J*= 20.3 Hz, 1H), 4.53 (s, 2H), 3.57 - 3.45 (m, 4H), 3.31 - 3.13 (m, 4H), 3.02 - 2.93 (m, 1H), 2.75 - 2.66 (m, 1H), 2.45 - 2.41 (m, 1H), 2.19 - 2.07 (m, 1H). LCMS (ESI) calcd. for $C_{24}H_{26}BrN_4O_2S^+$ [M+H]$^+$: 513.10, found, 513.1.

**Example 342: Preparation of 3-(4-((4-(3-bromophenyl)piperazin-1-yl)methyl)-1-thioxoisoindolin-2-yl)piperidine-2,6-dione (GT-08261)**

With reference to the method of Scheme 4, the target compound GT-08261 was obtained (12 mg, white solid, yield 20%). $^1$H NMR (400 MHz, DMSO-d6) δ 11.26 (s, 1H), 11.21 (s, 1H), 8.00 (d, *J*= 7.0 Hz, 2H), 7.69 (t, *J* = 7.4 Hz, 1H), 7.22 - 7.16 (m, 2H), 6.99 (t, *J* = 7.0 Hz, 2H), 6.02 (d, *J* = 8.8 Hz, 1H), 5.33 (d, *J= 20.2* Hz, 1H), 4.91 (d, *J*= 20.6 Hz, 1H), 4.59 - 4.38 (m, 2H), 3.95 - 3.80 (m, 2H), 3.54 - 3.44 (m, 2H), 3.30 - 3.16 (m, 4H), 3.06 - 2.93 (m, 1H), 2.69 (d, *J*= 17.4 Hz, 1H), 2.43 - 2.33 (m, 1H), 2.17 - 2.10 (m, 1H). LCMS (ESI) calcd. for $C_{24}H_{26}BrN_4O_2S^+$ [M+H]$^+$: 513.10, found, 513.1.

**Example 343: Preparation of 3-(4-((4-(4-bromophenyl)piperazin-1-yl)methyl)-1-thioxoisoindolin-2-yl)piperidine-2,6-dione (GT-08496)**

With reference to the method of Scheme 4, the target compound GT-08496 was obtained (12 mg, white solid, yield 20%). $^1$H NMR (400 MHz, DMSO-d6) δ 11.40 (s, 1H), 11.21 (s, 1H), 8.00 (d, *J* = 7.6 Hz, 2H), 7.68 (t, *J* = 7.7 Hz, 1H), 7.40 (d, *J* = 8.9 Hz, 2H), 6.95 (t, *J* = 8.9 Hz, 2H), 6.02 (d, *J* = 8.6 Hz, 1H), 5.38 (d, *J* = 20.6 Hz, 1H), 4.91 (d, *J* = 20.3 Hz, 1H), 4.60 - 4.38 (m, 2H), 3.81 (d, *J* = 9.5 Hz, 2H), 3.57 - 3.47 (m, 1H), 3.33 - 3,18 (m, 5H), 3.07 - 2.94 (m, 1H), 2.69 (d, *J* = 16.6 Hz, 1H), 2.44 (d, *J*= 13.0 Hz, 1H), 2.21 - 2.05 (m, 1H). LCMS (ESI) calcd. for $C_{24}H_{26}BrN_4O_2S^+$ [M+H]$^+$: 513.10, found, 513.1.

**Example 344: Preparation of 3-(1-thioxo-4-((4-(o-tolyl)piperazin-1-yl)methyl)isoindolin-2-yl)piperidine-2,6-dione (GT-08497)**

With reference to the method of Scheme 4, the target compound GT-08497 was obtained (11 mg, white solid, yield 21%). $^1$H NMR (400 MHz, DMSO-d6) δ 11.43 (s, 1H), 11.22 (s, 1H), 8.08 (d, *J= 7.6* Hz, 1H), 8.00 (d, *J* = 7.7 Hz, 1H), 7.69 (t, *J* = 7.8 Hz, 1H), 7.19 (d, *J* = 7.5 Hz, 2H), 7.02 (d, *J* = 7.5 Hz, 2H), 6.02 (d, *J* = 8.2 Hz, 1H), 5.43 (d, *J* = 20.2 Hz, 1H), 4.95 (d, *J* = 20.4 Hz, 1H), 4.61 - 4.43 (m, 2H), 3.49 (d, *J* = 11.3 Hz, 1H), 3.42 - 3.33 (m, 4H), 3.21 - 3.14 (m, 3H), 3.07 - 2.93 (m, 1H), 2.70 (d, *J* = 18.2 Hz, 1H), 2.42 - 2.33 (m, 1H), 2.27 (s, 3H), 2.18 - 2.12 (m, 1H). LCMS (ESI) calcd. for $C_{25}H_{29}N_4O_2S^+$ [M+H]$^+$: 449.20, found, 449.1.

**Example 345: Preparation of 3-(1-thioxo-4-((4-(m-tolyl)piperazin-1-yl)methyl)isoindolin-2-yl)piperidine-2,6-dione (GT-08498)**

With reference to the method of Scheme 4, the target compound GT-08498 was obtained (14 mg, white solid, yield 26%). $^1$H NMR (400 MHz, DMSO-d6) δ 11.61 (s, 1H), 11.21 (s, 1H), 8.00 (d, *J*= 7.7 Hz, 1H), 7.69 (d, *J* = 8.0 Hz, 1H), 7.13 (t, *J* = 7.8 Hz, 1H), 6.83 - 6.76 (m, 2H), 6.68 (d, *J*= 7.4 Hz, 2H), 6.02 (d, *J* = 8.3 Hz, 1H), 5.46 (d, *J*= 20.0 Hz, 1H), 4.93 (d, *J= 20.3* Hz, 1H), 4.70 - 4.53 (m, 1H), 4.53 - 4.42 (m, 2H), 3.50 - 3.42 (m, 2H), 3.30 - 3.21 (m, 5H), 3.06 - 2.94 (m, 1H), 2.69 (d, *J* = 16.2 Hz, 1H), 2.45 (d, *J= 12.9* Hz, 1H), 2.25 (s, 3H), 2.15 - 1.97 (m, 1H). LCMS (ESI) calcd. for $C_{25}H_{29}N_4O_2S^+$ [M+H]$^+$: 449.20, found, 449.1.

**Example 346: Preparation of 3-(1-thioxo-4-((4-(p-tolyl)piperazin-1-yl)methyl)isoindolin-2-yl)piperidine-2,6-dione (GT-08499)**

With reference to the method of Scheme 4, the target compound GT-08499 was obtained (12 mg, white solid, yield 23%). $^1$H NMR (400 MHz, DMSO-d6) δ 11.21 (s, 1H), 11.09 (s, 1H), 8.01 - 7.97 (m, 2H), 7.69 (t, *J* = 7.7 Hz, 1H), 7.07 (d, *J* = 8.4 Hz, 3H), 6.88 (d, *J* = 8.5 Hz, 2H), 6.02 (d, *J* = 7.6 Hz, 1H), 5.32 (d, *J* = 20.3 Hz, 1H), 4.92 (d, *J= 20.2* Hz, 1H), 4.55 - 4.43 (m, 2H), 3.73 (d, *J* = 11.8 Hz, 2H), 3.44 - 3.36 (m, 1H), 3.33 - 3.24 (m, 2H), 3.18 - 3.09 (m, 3H), 3.05 - 2.92 (m, 1H), 2.69 (d, *J* = 17.7 Hz, 1H), 2.47 - 2.41 (m, 1H), 2.21 (s, 3H), 2.17 - 2.07 (m, 1H). LCMS (ESI) calcd. for $C_{25}H_{29}N_4O_2S^+$ [M+H]$^+$: 449.20, found, 449.1.

**Example 347: Preparation of 3-(4-((4-(2,4-dichlorophenyl)piperazin-1-yl)methyl)-1-thioxoisoindolin-2-yl)piperidine-2,6-dione (GT-08500)**

With reference to the method of Scheme 4, the target compound GT-08500 was obtained (10 mg, white solid, yield 17%). $^1$H NMR (400 MHz, DMSO-d6) δ 11.21 (s, 1H), 11.11 (s, 1H), 8.00 (d, *J* = 6.4 Hz, 2H), 7.69 (t, *J* = 7.5 Hz, 1H), 7.61 (s, 1H), 7.41 (d, *J* = 6.6 Hz, 1H), 7.21 (d, *J* = 8.7 Hz, 1H), 6.02 (d, *J* = 12.2 Hz, 1H), 5.31 (d, *J*= 19.8 Hz, 1H), 4.93 (d, *J* = 20.1 Hz, 1H), 4.52 (s, 2H), 3.59 - 3.40 (m, 4H), 3.31 - 3.11 (m, 4H), 3.05 - 2.95 (m, 1H), 2.76 - 2.65 (m, 1H), 2.46 - 2.38 (m, 1H), 2.19 - 2.07 (m, 1H). LCMS (ESI) calcd. for $C_{24}H_{25}Cl_2N_4O_2S^+$ [M+H]$^+$: 503.11, found, 503.1.

**Example 348: Preparation of 3-(4-((4-(2,5-dichlorophenyl)piperazin-1-yl)methyl)-1-thioxoisoindolin-2-yl)piperidine-2,6-dione (GT-08501)**

With reference to the method of Scheme 4, the target compound GT-08501 was obtained (7 mg, white solid, yield 12%). $^1$H NMR (400 MHz, DMSO-d6) δ 11.21 (s, 1H), 11.16 (s, 1H), 8.01 (t, *J*= 7.5 Hz, 2H), 7.69 (t, *J= 7.5* Hz, 1H), 7.48 (d, *J* = 8.5 Hz, 1H), 7.22 (s, 1H), 7.18 (d, *J* = 8.0 Hz, 2H), 6.02 (d, *J* = 9.8 Hz, 1H), 5.32 (d, *J= 20.3* Hz, 1H), 4.93 (d, *J= 20.2* Hz, 1H), 4.53 (s, 2H), 3.61 - 3.42 (m, 4H), 3.31 - 3.11 (m, 4H), 3.08 - 2.92 (m, 1H), 2.69 (d, *J* = 18.9 Hz, 1H), 2.42 - 2.31 (m, 1H), 2.19 - 2.07 (m, 1H). LCMS (ESI) calcd. for $C_{24}H_{25}Cl_2N_4O_2S^+$ [M+H]$^+$: 503.11, found, 503.1.

**Example 349: Preparation of 3-(4-((4-(3,4-dichlorophenyl)piperazin-1-yl)methyl)-1-thioxoisoindolin-2-yl)piperidine-2,6-dione (GT-08502)**

With reference to the method of Scheme 4, the target compound GT-08502 was obtained (10 mg, white solid, yield 17%). $^1$H NMR (400 MHz, DMSO-d6) δ 11.21 (s, 1H), 11.16 (s, 1H), 7.99 (t, *J* = 8.6 Hz, 2H), 7.69 (t, *J*= 7.2 Hz, 1H), 7.46 (d, *J* = 8.6 Hz, 1H), 7.23 (s, 1H), 6.99 (d, *J* = 9.3 Hz, 1H), 6.02 (d, *J* = 10.8 Hz, 1H), 5.32 (d, *J* = 20.0 Hz, 1H), 4.90 (d, *J* = 20.1 Hz, 1H), 4.57 - 4.35 (m, 2H), 4.01 - 3.79 (m, 2H), 3.56 - 3.41 (m, 2H), 3.33 - 3.25 (m, 4H), 3.07 - 2.93 (m, 1H), 2.69 (d, *J*= 15.5 Hz, 1H), 2.41- 2.33 (m, 1H), 2.19 - 2.05 (m, 1H). LCMS (ESI) calcd. for $C_{24}H_{25}Cl_2N_4O_2S^+$ [M+H]$^+$: 503.11, found, 503.1.

**Example 350: Preparation of 3-(4-((4-(3,5-dichlorophenyl)piperazin-1-yl)methyl)-1-thioxoisoindolin-2-yl)piperidine-2,6-dione (GT-08629)**

With reference to the method of Scheme 4, the target compound GT-08629 was obtained (21 mg, white solid, yield 36%). $^1$H NMR (400 MHz, DMSO-d6) δ 11.21 (s, 1H), 11.16 (s, 1H), 8.09 - 7.90 (m, 2H), 7.69 (t, *J* = 6.4 Hz, 1H), 7.03 (s, 2H), 6.95 (s, 1H), 6.02 (d, *J* = 9.3 Hz, 1H), 5.31 (d, *J* = 20.2 Hz, 1H), 4.90 (d, *J*= 19.9 Hz, 1H), 4.56 - 4.36 (m, 2H), 3.97 (d, *J*= 11.6 Hz, 2H), 3.46 (d, *J* = 10.8 Hz, 2H), 3.32 - 3.12 (m, 4H), 3.03 - 2.95 (m, 1H), 2.69 (d, *J* = 16.1 Hz, 1H), 2.48 - 2.39 (m, 1H), 2.15 - 2.11 (m, 1H). LCMS (ESI) calcd. for $C_{24}H_{25}Cl_2N_4O_2S^+$ [M+H]$^+$: 503.11, found, 503.1.

**Example 351: Preparation of 3-(4-((4-(2,3-difluorophenyl)piperazin-1-yl)methyl)-1-thioxoisoindolin-2-yl)piperidine-2,6-dione (GT-08268)**

With reference to the method of Scheme 4, the target compound GT-08268 was obtained (16 mg, white solid, yield 29%). $^1$H NMR (400 MHz, DMSO-d6) δ 11.29 (s, 1H), 11.21 (s, 1H), 8.00 (d, *J*= 6.7 Hz, 2H), 7.69 (t, *J* = 7.4 Hz, 1H), 7.17 - 7.03 (m, 2H), 6.92 (t, *J* = 6.9 Hz, 1H), 6.02 (d, *J* = 9.9 Hz, 1H), 5.33 *(d, J= 19.0* Hz, 1H), 4.93 (d, *J*= 19.8 Hz, 1H), 4.50 (s, 2H), 3.52 (t, *J*= 10.4 Hz, 3H), 3.47 - 3.39 (m, 2H), 3.33 - 3.19 (m, 4H), 3.05 - 2.95 (m, 1H), 2.75 - 2.64 (m, 1H), 2.43 - 2.31 (m, 1H), 2.16 - 2.03 (m, 1H). LCMS (ESI) calcd. for $C_{24}H_{25}F_2N_4O_2S^+$ [M+H]$^+$: 471.17, found, 471.1.

**Example 352: Preparation of 3-(4-((4-(2,4-difluorophenyl)piperazin-1-yl)methyl)-1-thioxoisoindolin-2-yl)piperidine-2,6-dione (GT-08503)**

With reference to the method of Scheme 4, the target compound GT-08503 was obtained (10 mg, white solid, yield 18%). $^1$H NMR (400 MHz, DMSO-d6) δ 11.44 (s, 1H), 11.21 (s, 1H), 8.02 (dd, *J* = 18.9, 7.4 Hz, 2H), 7.68 (t, *J*= 7.6 Hz, 1H), 7.25 (t, *J* = 9.5 Hz, 1H), 7.13 (dd, *J* = 15.1, 9.2 Hz, 1H), 7.03 (t, *J* = 7.5 Hz, 1H), 6.02 (d, *J*= 8.3 Hz, 1H), 5.38 (d, *J* = 20.2 Hz, 1H), 4.93 (d, *J*= 20.3 Hz, 1H), 4.62 - 4.40 (m, 2H), 3.59 - 3.41 (m, 4H), 3.34 - 3.17 (m, 4H), 3.09 - 2.94 (m, 1H), 2.69 (d, *J* = 18.1 Hz, 1H), 2.42 - 2.39 (m, 1H), 2.21 - 2.05 (m, 1H). LCMS (ESI) calcd. for $C_{24}H_{25}F_2N_4O_2S^+$ [M+H]$^+$: 471.17, found, 471.1.

**Example 353: Preparation of 3-(4-((4-(2,5-difluorophenyl)piperazin-1-yl)methyl)-1-thioxoisoindolin-2-yl)piperidine-2,6-dione (GT-08593)**

With reference to the method of Scheme 4, the target compound GT-08593 was obtained (15 mg, white solid, yield 27%). $^1$H NMR (400 MHz, DMSO-d6) δ 11.34 (s, 1H), 11.21 (s, 1H), 8.06 -7.99 (m, 2H), 7.68 *(t, J*= 7.4 Hz, 1H), 7.33 - 7.13 (m, 1H), 7.09 - 6.92 (m, 1H), 6.83 *(t, J*= 8.1 Hz, 1H), 6.01 *(d, J*= 8.7 Hz, 1H), 5.35 (d, *J* = 20.1 Hz, 1H), 4.92 (d, *J* = 20.2 Hz, 1H), 4.62 - 4.39 (m, 2H), 3.57 - 3.47 (m, 4H), 3.35 - 3.20 (m, 4H), 3.09 - 2.93 (m, 1H), 2.69 (d, *J* = 17.9 Hz, 1H), 2.49 - 2.39 (m, 1H), 2.20 - 2.04 (m, 1H). LCMS (ESI) calcd. for $C_{24}H_{25}F_2N_4O_2S^+$ [M+H]$^+$: 471.17, found, 471.1.

**Example 354: Preparation of 3-(4-((4-(2,6-difluorophenyl)piperazin-1-yl)methyl)-1-thioxoisoindolin-2-yl)piperidine-2,6-dione (GT-08630)**

With reference to the method of Scheme 4, the target compound GT-08630 was obtained (15 mg, white solid, yield 27%). $^1$H NMR (400 MHz, DMSO-d6) δ 11.21 (s, 1H), 11.13 (s, 1H), 8.11 - 7.77 (m, 2H), 7.68 *(t, J*= 7.6 Hz, 1H), 7.26 - 7.13 (m, 1H), 7.13 - 7.06 (m, 2H), 6.02 (d, *J* = 9.7 Hz, 1H), 5.33 (d, *J* = 20.4 Hz, 1H), 4.93 (d, *J*= 20.2 Hz, 1H), 4.50 (brs, 2H), 3.70 - 3.55 (m, 2H), 3.47 - 3.45 (m, 2H), 3.31 - 3.20 (m, 4H), 3.05 - 2.88 (m, 1H), 2.75 - 2.64 (m, 1H), 2.62 - 2.59 (m, 1H), 2.18 - 2.06 (m, 1H). LCMS (ESI) calcd. for $C_{24}H_{25}F_2N_4O_2S^+$ [M+H]$^+$: 471.17, found, 471.1.

**Example 355: Preparation of 3-(4-((4-(3,4-difluorophenyl)piperazin-1-yl)methyl)-1-thioxoisoindolin-2-yl)piperidine-2,6-dione (GT-08631)**

With reference to the method of Scheme 4, the target compound GT-08631 was obtained (14 mg, white solid, yield 25%). $^1$H NMR (400 MHz, DMSO-d6) δ 11.39 (s, 1H), 11.21 (s, 1H), 8.00 (d, *J* = 7.6 Hz, 2H), 7.68 (t, *J*= 7.7 Hz, 1H), 7.30 (dd, *J*= 19.7, 9.5 Hz, 1H), 7.12 - 7.06 (m, 1H), 6.78 (d, *J* = 9.1 Hz, 1H), 6.02 (d, *J* = 8.9 Hz, 1H), 5.37 *(d, J*= 19.7 Hz, 1H), 4.91 (d, *J* = 20.7 Hz, 1H), 4.65 - 4.42 (m, 2H), 3.80 (d, *J* = 9.3 Hz, 2H), 3.59 - 3.46 (m, 2H), 3.32 - 3.19 (m, 4H), 3.09 - 2.91 (m, 1H), 2.69 (d, *J*= 17.5 Hz, 1H), 2.48 - 2.38 (m, 1H), 2.18 - 2.05 (m, 1H). LCMS (ESI) calcd. for $C_{24}H_{25}F_2N_4O_2S^+$ [M+H]$^+$: 471.17, found, 471.1.

**Example 356: Preparation of 3-(4-((4-(3,5-difluorophenyl)piperazin-1-yl)methyl)-1-thioxoisoindolin-2-yl)piperidine-2,6-dione (GT-08632)**

With reference to the method of Scheme 4, the target compound GT-08632 was obtained (17 mg, white solid, yield 31%). $^1$H NMR (400 MHz, DMSO-d6) δ 11.28 (s, 1H), 11.21 (s, 1H), 8.01 - 7.97 (m, 2H), 7.69 (t, *J*= 7.4 Hz, 1H), 6.71 (d, *J* = 9.9 Hz, 2H), 6.58 (t, *J*= 9.0 Hz, 1H), 6.02 (d, *J*= 10.1 Hz, 1H), 5.32 (d, *J* = 21.0 Hz, 1H), 4.90 (d, *J* = 19.4 Hz, 1H), 4.58 - 4.25 (m, 2H), 3.94 (d, *J*= 11.0 Hz, 2H), 3.53 - 3.43 (m, 2H), 3.24 - 2.18 (m, 4H), 3.09 - 2.93 (m, 1H), 2.67 (t, *J* = 18.4 Hz, 1H), 2.47 - 2.36 (m, 1H), 2.22 - 2.07 (m, 1H). LCMS (ESI) calcd. for $C_{24}H_{25}F_2N_4O_2S^+$ [M+H]$^+$: 471.17, found, 471.1.

**Example 357: Preparation of 3-(4-((4-(2,4-dimethylphenyl)piperazin-1-yl)methyl)-1-thioxoisoindolin-2-yl)piperidine-2,6-dione (GT-08504)**

With reference to the method of Scheme 4, the target compound GT-08504 was obtained (9 mg, white solid, yield 17%). $^1$H NMR (400 MHz, DMSO-d6) δ 11.21 (s, 1H), 11.09 (s, 1H), 8.04 (d, *J= 7.6* Hz, 1H), 8.00 (d, *J* = 7.7 Hz, 1H), 7.69 (t, *J* = 7.6 Hz, 1H), 7.00 (s, 1H), 6.97 (d, *J* = 8.2 Hz, 1H), 6.91 (d,*J* = 8.1 Hz, 1H), 6.02 (d, *J* = 8.3 Hz, 1H), 5.34 (d, *J* = 20.5 Hz, 1H), 4.94 (d, *J* = 20.3 Hz, 1H), 4.58 - 4.38 (m, 2H), 3.54 - 3.45 (m, 2H), 3.27 - 3.05 (m, 6H), 3.05 - 2.95 (m, 1H), 2.70 (d, *J*= 17.5 Hz, 1H), 2.45- 2.39 (m, 1H), 2.23 (s, 3H), 2.21 (s, 3H), 2.18 - 2.12 (m, 1H). LCMS (ESI) calcd. for $C_{26}H_{31}N_4O_2S^+$ [M+H]$^+$: 463.22, found, 463.2.

**Example 358: Preparation of 3-(4-((4-(5-chloro-2-methylphenyl)piperazin-1-yl)methyl)-1-thioxoisoindolin-2-yl) piperidine-2,6-dione (GT-08505)**

With reference to the method of Scheme 4, the target compound GT-08505 was obtained (11 mg, white solid, yield 19%). $^1$H NMR (400 MHz, DMSO-d6) δ 11.22 (s, 1H), 11.04 (s, 1H), 8.10 - 7.95 (m, 2H), 7.69 (t, *J* = 7.4 Hz, 1H), 7.22 (d, *J* = 8.0 Hz, 1H), 7.07 (d, *J*= 8.1 Hz, 1H), 7.02 (s, 1H), 6.03 (d, *J* = 15.9 Hz, 1H), 5.32 (d, *J* = 20.3 Hz, 1H), 4.94 (d, *J* = 20.0 Hz, 1H), 4.62 - 4.36 (m, 2H), 3.49 (d, *J* = 10.0 Hz, 1H), 3.44 - 3.38 (m, 2H), 3.29 - 3.09 (m, 5H), 3.07 - 2.93 (m, 1H), 2.70 (d, *J* = 18.2 Hz, 1H), 2.47 - 2.38 (m, 1H), 2.24 (s, 3H), 2.18 - 2.06 (m, 1H). LCMS (ESI) calcd. for $C_{25}H_{28}ClN_4O_2S^+$ [M+H]$^+$: 483.16, found, 483.1.

**Example 359: Preparation of 3-(4-((4-phenylpiperidin-1-yl)methyl)-1-thioxoisoindolin-2-yl)piperidine-2,6-dione (GT-08506)**

With reference to the method of Scheme 4, the target compound GT-08506 was obtained (10 mg, white solid, yield 20%). $^1$H NMR (400 MHz, DMSO-d6) δ 11.21 (s, 1H), 10.83 (s, 1H), 8.00 (d, *J*= 7.7 Hz, 2H), 7.69 *(t, J*= 7.7 Hz, 1H), 7.38 - 7.29 (m, 2H), 7.28 - 7.17 (m, 3H), 6.02 (d, *J* = 8.0 Hz, 1H), 5.31 (d, *J* = 20.4 Hz, 1H), 4.92 (d, *J* = 20.3 Hz, 1H), 4.51 - 4.33 (m, 2H), 3.49 (dd, *J* = 30.2, 11.2 Hz, 2H), 3.26 - 3.10 (m, 2H), 3.08 - 2.92 (m, 1H), 2.80 (t, *J* = 11.8 Hz, 1H), 2.69 (d, *J*= 18.9 Hz, 1H), 2.43 - 2.33 (m, 1H), 2.22 - 2.04 (m, 3H), 1.95 (d, *J* = 12.5 Hz, 2H). LCMS (ESI) calcd. for $C_{25}H_{28}N_3O_2S^+$ [M+H]$^+$: 434.19, found, 434.1.

**Example 360: Preparation of 3-(4-((4-(3-bromophenyl)piperidin-1-yl)methyl)-1-thioxoisoindolin-2-yl)piperidine-2,6-dione (GT-08507)**

With reference to the method of Scheme 4, the target compound GT-08507 was obtained (7 mg, white solid, yield 12%). $^1$H NMR (400 MHz, DMSO-d6) δ 11.21 (s, 1H), 10.67 (s, 1H), 8.06 - 7.95 (m, 2H), 7.69 (t, *J*= 7.7 Hz, 1H), 7.44 (d, *J* = 8.3 Hz, 2H), 7.31 (t, *J* = 7.6 Hz, 1H), 7.25 (d, *J*= 7.7 Hz, 1H), 6.03 (d, *J*= 15.8 Hz, 1H), 5.27 (d, *J* = 20.4 Hz, 1H), 4.91 (d, *J* = 20.3 Hz, 1H), 4.53 - 4.33 (m, 2H), 3.49 (dd, *J* = 28.6, 11.0 Hz, 2H), 3.23 - 3.13 (m, 2H), 3.07 - 2.95 (m, 1H), 2.93 - 2.77 (m, 1H), 2.69 (d, *J*= 18.7 Hz, 1H), 2.46 - 2.39 (m, 1H), 2.21 - 2.03 (m, 3H), 1.97 (d, *J* = 13.0 Hz, 2H). LCMS (ESI) calcd. for $C_{25}H_{27}BrN_3O_2S^+$ [M+H]$^+$: 512.10, found, 512.1.

**Example 361: Preparation of 3-(4-((4-(4-bromophenyl)piperidin-1-yl)methyl)-1-thioxoisoindolin-2-yl)piperidine-2,6-dione (GT-08592)**

With reference to the method of Scheme 4, the target compound GT-08592 was obtained (6 mg, white solid, yield 10%). $^1$H NMR (400 MHz, DMSO-d6) δ 11.21 (s, 1H), 10.69 (s, 1H), 7.99 (dd, *J* = 9.8, 7.8 Hz, 2H), 7.69 (t, *J* = 7.6 Hz, 1H), 7.53 (d, *J* = 8.4 Hz, 2H), 7.20 (d, *J* = 8.5 Hz, 2H), 6.02 (d, *J* = 11.9 Hz, 1H), 5.27 (d, *J* = 20.4 Hz, 1H), 4.91 (d, *J* = 20.4 Hz, 1H), 4.51 - 4.33 (m, 2H), 3.49 (dd, *J* = 26.9, 12.2 Hz, 2H), 3.20 - 3.12 (m, 2H), 3.04 - 2.96 (m, 1H), 2.88 - 2.76 (m, 1H), 2.74 - 2.65 (m, 1H), 2.45 - 2.39 (m, 1H), 2.15 - 2.04 (m, 3H), 1.95 (d, *J* = 12.4 Hz, 2H). LCMS (ESI) calcd. for $C_{25}H_{27}BrN_3O_2S^+$ [M+H]$^+$: 512.10, found, 512.1.

**Example 362: Preparation of 3-(4-((4-(4-chlorophenyl)piperidin-1-yl)methyl)-1-thioxoisoindolin-2-yl)piperidine-2,6-dione (GT-08594)**

With reference to the method of Scheme 4, the target compound GT-08594 was obtained (7 mg, white solid, yield 13%). $^1$H NMR (400 MHz, DMSO-d6) δ 11.21 (s, 1H), 10.70 (s, 1H), 8.04 - 7.93 (m, 2H), 7.69 (t, *J* = 7.6 Hz, 1H), 7.39 (d, *J* = 8.3 Hz, 2H), 7.26 (d, *J* = 8.4 Hz, 2H), 6.02 (d, *J* = 9.0 Hz, 1H), 5.27 (d, *J*= 20.4 Hz, 1H), 4.91 (d, *J= 20.4* Hz, 1H), 4.52 - 4.33 (m, 2H), 3.47 - 3.41 (m, 2H), 3.20 - 3.13 (m, 2H), 3.04 - 2.96 (m, 2H), 2.89 - 2.80 (m, 1H), 2.70 (d, *J* = 19.2 Hz, 1H), 2.44 - 2.41 (m, 1H), 2.20 - 2.02 (m, 3H), 1.95 (d, *J* = 13.8 Hz, 2H). LCMS (ESI) calcd. for $C_{25}H_{27}ClN_3O_2S^+$ [M+H]$^+$: 468.15, found, 468.1.

**Example 363: Preparation of 3-(4-((4-(2-fluorophenyl)piperidin-1-yl)methyl)-1-thioxoisoindolin-2-yl)piperidine-2,6-dione (GT-08595)**

With reference to the method of Scheme 4, the target compound GT-08595 was obtained (7 mg, white solid, yield 13%). $^1$H NMR (400 MHz, DMSO-d6) δ 11.21 (s, 1H), 10.67 (s, 1H), 8.01 - 7.97 (m, 2H), 7.69 (t, *J* = 7.7 Hz, 1H), 7.28 (t, *J* = 7.9 Hz, 2H), 7.24 - 7.14 (m, 2H), 6.02 (d, *J* = 11.7 Hz, 1H), 5.25 (d, *J= 20.4* Hz, 1H), 4.92 (d, *J*= 20.2 Hz, 1H), 4.45 - 4.41 (m, 2H), 3.57 - 3.44 (m, 2H), 3.27 - 3.17 (m, 2H), 3.11 (t, *J* = 12.3 Hz, 1H), 3.04 - 2.95 (m, 1H), 2.70 (d, *J* = 19.3 Hz, 1H), 2.43 - 2.33 (m, 1H), 2.22 - 2.09 (m, 3H), 1.98 - 1.87 (m, 2H). LCMS (ESI) calcd. for $C_{25}H_{27}FN_3O_2S^+$ [M+H]$^+$: 452.18, found, 452.1.

**Example 364: Preparation of 3-(4-((4-(4-fluorophenyl)piperidin-1-yl)methyl)-1-thioxoisoindolin-2-yl)piperidine-2,6-dione (GT-08596)**

With reference to the method of Scheme 4, the target compound GT-08596 was obtained (7 mg, white solid, yield 13%). $^1$H NMR (400 MHz, DMSO-d6) δ 11.21 (s, 1H), 10.83 (s, 1H), 8.00 (d, *J= 7.6* Hz, 2H), 7.69 (t, *J* = 7.7 Hz, 1H), 7.27 (dd, *J* = 8.5, 5.6 Hz, 2H), 7.16 (t, *J* = 8.8 Hz, 2H), 6.02 (d, *J* = 7.2 Hz, 1H), 5.31 (d, *J*= 20.4 Hz, 1H), 4.91 (d, *J* = 20.1 Hz, 1H), 4.51 - 4.31 (m, 2H), 3.48 (dd, *J*= 31.8, 11.3 Hz, 2H), 3.23 - 3.09 (m, 2H), 3.08 - 2.94 (m, 1H), 2.82 (t, *J*= 11.8 Hz, 1H), 2.69 (d, *J*= 18.7 Hz, 1H), 2.43 - 2.41(m, 1H), 2.19 -2.04 (m, 3H), 1.94 (d, *J*= 12.4 Hz, 2H). LCMS (ESI) calcd. for $C_{25}H_{27}FN_3O_2S^+$ [M+H]$^+$: 452.18, found, 452.1.

**Example 365: Preparation of 3-(1-thioxo-4-((4-(2-(trifluoromethyl)phenyl)piperidin-1-yl)methyl)isoindolin-2-yl)piperidine-2,6-dione (GT-08598)**

With reference to the method of Scheme 4, the target compound GT-08598 was obtained (10 mg, white solid, yield 17%). $^1$H NMR (400 MHz, DMSO-d6) δ 11.21 (s, 1H), 10.88 (s, 1H), 8.00 (d, *J* = 7.7 Hz, 2H), 7.69 (t, *J* = 7.6 Hz, 1H), 7.65 - 7.52 (m, 4H), 6.03 (d, *J* = 9.9 Hz, 1H), 5.33 (d, *J* = 20.4 Hz, 1H), 4.91 (d, *J* = 20.0 Hz, 1H), 4.56 - 4.34 (m, 2H), 3.50 (dd, *J*= 37.4, 11.4 Hz, 2H), 3.26 - 3.15 (m, 2H), 3.04 - 2.93 (m, 2H), 2.69 (d, *J* = 18.7 Hz, 1H), 2.44 - 2.33 (m, 1H), 2.25 - 2.07 (m, 3H), 2.00 (d, *J* = 12.1 Hz, 2H). LCMS (ESI) calcd. for $C_{26}H_{27}F_3N_3O_2S^+$ [M+H]$^+$: 502.18, found, 502.1.

**Example 366: Preparation of 3-(1-thioxo-4-((4-(3-(trifluoromethyl)phenyl)piperidin-1-yl)methyl)isoindolin-2-yl)piperidine-2,6-dione (GT-08599)**

With reference to the method of Scheme 4, the target compound GT-08599 was obtained (6 mg, white solid, yield 10%). $^1$H NMR (400 MHz, DMSO-d6) δ 11.22 (s, 1H), 10.97 (s, 1H), 8.00 (d, *J*= 7.6 Hz, 2H), 7.76 - 7.66 (m, 3H), 7.55 (d, *J* = 7.7 Hz, 1H), 7.46 (t, *J= 7.5* Hz, 1H), 6.01 (d, *J*= 13.6 Hz, 1H), 5.30 *(d, J= 20.2* Hz, 1H), 4.92 (d, *J* = 20.5 Hz, 1H), 4.53 - 4.31 (m, 2H), 3.48 (dd, *J*= 32.5, 11.4 Hz, 2H), 3.28 - 3.19 (m, 2H), 3.20 - 3.10 (m, 1H), 3.05 - 2.97 (m, 1H), 2.70 (d, *J* = 18.8 Hz, 1H), 2.35 - 2.27 (m, 2H), 2.21 - 2.09 (m, 1H), 1.86 (d, *J*= 13.7 Hz, 2H). LCMS (ESI) calcd. for $C_{26}H_{27}F_3N_3O_2S^+$ [M+H]$^+$: 502.18, found, 502.1.

**Example 367: Preparation of 3-(4-((4-(2,3-dichlorophenyl)piperidin-1-yl)methyl)-1-thioxoisoindolin-2-yl)piperidine-2,6-dione (GT-08269)**

With reference to the method of Scheme 4, the target compound GT-08269 was obtained (4 mg, white solid, yield 7 %). $^1$H NMR (400 MHz, DMSO-d6) δ 11.21 (s, 1H), 10.75 (s, 1H), 8.04 - 7.94 (m, 2H), 7.69 (t, *J*= 7.7 Hz, 1H), 7.56 (d, *J* = 7.7 Hz, 1H), 7.40 *(t, J*= 7.8 Hz, 1H), 7.30 (d, *J* = 8.1 Hz, 1H), 6.02 (d, *J*= *9.0* Hz, 1H), 5.26 (d, *J*= 20.3 Hz, 1H), 4.91 (d, *J* = 20.3 Hz, 1H), 4.50 - 4.35 (m, 2H), 3.56 - 3.47 (m, 3H), 3.11 - 3.05 (m, 1H), 3.03 - 2.94 (m, 1H), 2.70 (d, *J*= 23.8 Hz, 2H), 2.45 - 2.41 (m, 2H), 2.19 - 2.02 (m, 4H), 1.97 (d, *J* = 12.3 Hz, 2H). LCMS (ESI) calcd. for $C_{25}H_{26}Cl_2N_3O_2S^+$ $[M+H]^+$: 502.11, found, 502.1.

**Example 368: Preparation of 3-(4-((4-(2-chloro-3-fluorophenyl)piperidin-1-yl)methyl)-1-thioxoisoindolin-2-yl)piperidine-2,6-dione (GT-08321)**

With reference to the method of Scheme 4, the target compound GT-08321 was obtained (14 mg, white solid, yield 24%). $^1$H NMR (400 MHz, DMSO-d6) δ 11.21 (s, 1H), 11.05 (s, 1H), 8.04 - 7.95 (m, 2H), 7.69 (t, *J*= 7.6 Hz, 1H), 7.45 - 7.40 (m, 1H), 7.32 (t, *J*= 8.5 Hz, 1H), 7.17 (d, *J* = 8.0 Hz, 1H), 6.02 (d, *J*= 8.5 Hz, 1H), 5.32 (d, *J* = 20.2 Hz, 1H), 4.92 (d, *J*= 20.4 Hz, 1H), 4.53 - 4.31 (m, 2H), 3.50 (dd,*J* = 31.4, 11.6 Hz, 2H), 3.25 - 3.17 (m, 3H), 3.07 - 2.95 (m, 1H), 2.70 (d, *J* = 18.8 Hz, 1H), 2.21 - 2.11 (m, 4H), 1.94 (d, *J* = 12.9 Hz, 2H). L LCMS (ESI) calcd. for $C_{25}H_{26}ClFN_3O_2S^+$ $[M+H]^+$: 486.14, found, 486.1.

**Example 369: Preparation of 3-(4-((4-(2,3-difluorophenyl)piperidin-1-yl)methyl)-1-thioxoisoindolin-2-yl)piperidine-2,6-dione (GT-08322)**

With reference to the method of Scheme 4, the target compound GT-08322 was obtained (13 mg, white solid, yield 24%). $^1$H NMR (400 MHz, DMSO-d6) δ 11.21 (s, 1H), 10.90 (s, 1H), 8.00 (dd, *J* = 7.5, 2.6 Hz, 2H), 7.69 *(t, J*= 7.6 Hz, 1H), 7.32 (dd, *J* = 17.6, 8.4 Hz, 1H), 7.22 (dd, *J* = 13.1, 7.5 Hz, 1H), 7.10 (t, *J* = 6.8 Hz, 1H), 6.02 (d, *J* = 9.8 Hz, 1H), 5.29 (d, *J* = 20.0 Hz, 1H), 4.91 (d, *J* = 20.4 Hz, 1H), 4.52 - 4.25 (m, 2H), 3.49 (dd, *J* = 26.7, 9.8 Hz, 2H), 3.25 - 3.16 (m, 4H), 3.04 - 2.96 (m, 1H), 2.69 (d, *J* = 18.4 Hz, 1H), 2.25 - 2.07 (m, 4H), 1.94 (d, *J* = 13.5 Hz, 2H). LCMS (ESI) calcd. for $C_{25}H_{26}F_2N_3O_2S^+$ $[M+H]^+$: 470.17, found, 470.1.

**Example 370: Preparation of 3-(4-((4-(2,3-dichlorophenyl)-3,6-dihydropyridin-1(2H)-yl)methyl)-1-thioxoisoindolin-2-yl)piperidine-2,6-dione (GT-08270)**

With reference to the method of Scheme 4, the target compound GT-08270 was obtained (7 mg, white solid, yield 12%). $^1$H NMR (400 MHz, DMSO-d6) δ 11.21 (s, 1H), 10.94 (s, 1H), 8.08 - 7.95 (m, 2H), 7.71 (d, *J* = 7.8 Hz, 1H), 7.63 (d, *J* = 8.0 Hz, 1H), 7.40 (t, *J* = 7.9 Hz, 1H), 7.24 (d, *J* = 7.6 Hz, 1H), 6.03 (d, *J* = 8.9 Hz, 1H), 5.76 (s, 1H), 5.20 (d, *J*= 21.1 Hz, 1H), 4.94 (d, *J*= 19.7 Hz, 1H), 4.62 - 4.44 (m, 2H), 3.97 - 3.78 (m, 2H), 3.70 - 3.57 (m, 1H), 3.11 - 3.03 (m, 1H), 3.02 - 2.95 (m, 1H), 2.93 - 2.81 (m, 1H), 2.75 - 2.65 (m, 1H), 2.62 - 2.58 (m, 1H), 2.43 - 2.38 (m, 1H), 2.20 - 2.09 (m, 1H). LCMS (ESI) calcd. for $C_{25}H_{24}Cl_2N_3O_2S^+$ $[M+H]^+$: 500.10, found, 500.1.

**Example 371: Preparation of 3-(4-((4-(2-chloro-3-fluorophenyl)-3,6-dihydropyridin-1(2H)-yl)methyl)-1-thioxoisoindolin-2-yl)piperidine-2,6-dione (GT-08301)**

With reference to the method of Scheme 4, the target compound GT-08301 was obtained (6 mg, white solid, yield 11%). $^1$H NMR (400 MHz, DMSO-d6) δ 11.21 (s, 1H), 11.06 (s, 1H), 8.01 (d, *J*= 7.4 Hz, 2H), 7.70 (t, *J* = 7.5 Hz, 1H), 7.41 (t, *J* = 6.3 Hz, 2H), 7.13 (d, *J* = 6.2 Hz, 1H), 6.02 (d, *J* = 13.5 Hz, 1H), 5.79 (s, 1H), 5.26 (t, *J* = 21.6 Hz, 1H), 4.94 (d, *J* = 21.2 Hz, 1H), 4.65 - 4.43 (m, 2H), 3.96 - 3.82 (m, 2H), 3.70 - 3.59 (m, 1H), 3.12 - 2.84 (m, 3H), 2.77 - 2.66 (m, 1H), 2.61 - 2.55 (m, 2H), 2.19 - 2.09 (m, 1H). LCMS (ESI) calcd. for $C_{25}H_{24}ClFN_3O_2S^+$ $[M+H]^+$: 484.13, found, 484.1.

**Example 372: Preparation of 3-(4-((4-(pyridin-2-yl)piperazin-1-yl)methyl)-1-thioxoisoindolin-2-yl)piperidine-2,6-dione (GT-08691)**

With reference to the method of Scheme 4, the target compound GT-08691 was obtained (21 mg, white solid, yield 41%). $^1$H NMR (400 MHz, DMSO-d6) δ 11.93 (s, 1H), 11.21 (s, 1H), 8.15 - 8.12 (m, 1H), 8.06 - 7.98 (m, 1H), 7.98 - 7.92 (m, 2H), 7.72 - 7.66 (m, 1H), 7.29 - 7.21 (m, 1H), 7.01 - 6.90 (m, 1H), 6.02 (d, *J* = 8.7 Hz, 1H), 5.44 (d, *J* = 20.4 Hz, 1H), 4.91 (d, *J* = 20.3 Hz, 1H), 4.63 - 4.42 (m, 4H), 3.59 - 3.46 (m, 2H), 3.46 - 3.22 (m, 4H), 3.08 - 2.93 (m, 1H), 2.75 - 2.63 (m, 1H), 2.43 - 2.40 (m, 1H), 2.20 - 2.04 (m, 1H). LCMS (ESI) calcd. for $C_{23}H_{26}N_5O_2S^+$ $[M+H]^+$: 436.18, found, 436.1.

**Example 373: Preparation of 3-(4-((4-(6-methylpyridin-2-yl)piperazin-1-yl)methyl)-1-thioxoisoindolin-2-yl)piperidine-2,6-dione (GT-08692)**

With reference to the method of Scheme 4, the target compound GT-08692 was obtained (18 mg, white solid, yield 34%). $^1$H NMR (400 MHz, DMSO-d6) δ 11.88 (s, 1H), 11.21 (s, 1H), 8.02 - 7.99 (m, 2H), 7.79 (s, 1H), 7.68 (t, *J*= 7.7 Hz, 1H), 7.02 (d, *J* = 7.5 Hz, 1H), 6.80 (d, *J*= 7.1 Hz, 1H), 6.02 (d, *J* = 8.2 Hz, 1H), 5.45 (d, *J* = 20.4 Hz, 1H), 4.91 (d, *J* = 20.4 Hz, 1H), 4.62 - 4.43 (m, 4H), 4.01 - 3.85 (m, 2H), 3.42 - 3.16 (m, 4H), 3.09 - 2.91 (m, 1H), 2.69 (d, *J* = 16.4 Hz, 1H), 2.49 (s, 3H), 2.48 - 2.35 (m, 1H), 2.19 - 2.03 (m, 1H). LCMS (ESI) calcd. for $C_{24}H_{28}N_5O_2S^+$ $[M+H]^+$: 450.20, found, 450.1.

**Example 374: Preparation of 3-(4-((4-(6-chloropyridin-2-yl)piperazin-1-yl)methyl)-1-thioxoisoindolin-2-yl)piperidine-2,6-dione (GT-08693)**

With reference to the method of Scheme 4, the target compound GT-08693 was obtained (10 mg, white solid, yield 18%). $^1$H NMR (400 MHz, DMSO-d6) δ 11.32 (s, 1H), 11.20 (s, 1H), 7.99 (t, *J*= 7.7 Hz, 2H), 7.70 - 7.62 (m, 2H), 6.89 (d, *J*= 8.1 Hz, 1H), 6.78 (d, *J* = 7.4 Hz, 1H), 6.02 (d, *J* = 9.4 Hz, 1H), 5.32 (d, *J*= *20.4* Hz, 1H), 4.90 (d, *J*= 19.4 Hz, 1H), 4.52 - 4.41 (m, 2H), 4.35 (d, *J* = 12.0 Hz, 2H), 3.55 - 3.45 (m, 1H), 3.45 - 3.41 (m, 4H), 3.24 - 3.14 (m, 2H), 3.06 - 2.92 (m, 1H), 2.69 (d, *J* = 16.0 Hz, 1H), 2.48 - 2.35 (m, 1H), 2.16 - 2.07 (m, 1H). LCMS (ESI) calcd. for $C_{23}H_{25}ClN_5O_2S^+$ $[M+H]^+$: 470.14, found, 470.1.

**Example 375: Preparation of 3-(4-((4-(5-chloropyridin-2-yl)piperazin-1-yl)methyl)-1-thioxoisoindolin-2-yl)piperidine-2,6-dione (GT-08694)**

With reference to the method of Scheme 4, the target compound GT-08694 was obtained (9 mg, white solid, yield 16%). $^1$H NMR (400 MHz, DMSO-d6) δ 11.57 (s, 1H), 11.20 (s, 1H), 8.18 (d, *J*= 2.5 Hz, 1H), 8.06 - 7.96 (m, 2H), 7.76 - 7.64 (m, 2H), 6.98 (d, *J* = 9.1 Hz, 1H), 6.01 (d, *J* = 9.0 Hz, 1H), 5.39 (d, *J*= 20.5 Hz, 1H), 4.90 (d, *J* = 20.4 Hz, 1H), 4.51 - 4.34 (m, 4H), 3.50 - 3.36 (m, 4H), 3.26 - 3.14 (m, 2H), 3.03 - 2.95 (m, 1H), 2.69 (d, *J* = 16.7 Hz, 1H), 2.47 - 2.38 (m, 1H), 2.19 - 2.05 (m, 1H). LCMS (ESI) calcd. for $C_{23}H_{25}ClN_5O_2S^+$ $[M+H]^+$: 470.14, found, 470.1.

**Example 376: Preparation of 3-(4-((4-(4-chloropyridin-2-yl)piperazin-1-yl)methyl)-1-thioxoisoindolin-2-yl)piperidine-2,6-dione (GT-08695)**

With reference to the method of Scheme 4, the target compound GT-08695 was obtained (20 mg, white solid, yield 36%). $^1$H NMR (400 MHz, DMSO-d6) δ 11.67 (s, 1H), 11.20 (s, 1H), 8.12 (d, *J*= 5.5 Hz, 1H), 8.01 (t, *J* = 7.6 Hz, 2H), 7.68 (t, *J*= 7.7 Hz, 1H), 7.11 (s, 1H), 6.85 (d, *J*= 5.4 Hz, 1H), 6.01 (d, *J* = 8.5 Hz, 1H), 5.40 (d, *J* = 21.7 Hz, 1H), 4.90 (d, *J* = 20.3 Hz, 1H), 4.51 - 4.40 (m, 4H), 3.52 - 3.42 (m, 4H), 3.23 - 3.15 (m, 2H), 3.06 - 2.93 (m, 1H), 2.69 (d, *J* = 16.6 Hz, 1H), 2.46 - 2.40 (m, 1H), 2.17 - 2.10 (m, 1H). LCMS (ESI) calcd. for $C_{23}H_{25}ClN_5O_2S^+$ $[M+H]^+$: 470.14, found, 470.1.

**Example 377: Preparation of 3-(4-((4-(3-chloropyridin-2-yl)piperazin-1-yl)methyl)-1-thioxoisoindolin-2-yl)piperidine-2,6-dione (GT-08696)**

With reference to the method of Scheme 4, the target compound GT-08696 was obtained (18 mg, white solid, yield 33%). $^1$H NMR (400 MHz, DMSO-d6) δ 11.43 (s, 1H), 11.21 (s, 1H), 8.27 (d, *J*= 4.7 Hz, 1H), 8.01 (t, *J* = 8.6 Hz, 2H), 7.87 (d, *J* = 7.8 Hz, 1H), 7.69 (t, *J* = 7.9 Hz, 1H), 7.10 (dd, *J* = 7.7, 4.6 Hz, 1H), 6.02 (d, *J* = 7.8 Hz, 1H), 5.45 - 5.31 (m, 1H), 4.93 (d, *J* = 20.4 Hz, 1H), 4.60 - 4.43 (m, 2H), 3.90 - 3.82 (m, 4H), 3.40 - 3.27 (m, 4H), 3.07 - 2.93 (m, 1H), 2.69 (d, *J* = 17.8 Hz, 1H), 2.45 - 2.36 (m, 1H), 2.20 - 2.06 (m, 1H). LCMS (ESI) calcd. for $C_{23}H_{25}ClN_5O_2S^+$ $[M+H]^+$: 470.14, found, 470.1.

**Example 378: Preparation of 3-(4-((4-(3-fluoropyridin-2-yl)piperazin-1-yl)methyl)-1-thioxoisoindolin-2-yl)piperidine-2,6-dione (GT-08697)**

With reference to the method of Scheme 4, the target compound GT-08697 was obtained (13 mg, white solid, yield 24%). $^1$H NMR (400 MHz, DMSO-d6) δ 11.68 (s, 1H), 11.21 (s, 1H), 8.09 - 8.06 (m, 1H), 8.02 (dd, *J* = 20.4, 8.5 Hz, 2H), 7.67 (t, *J* = 7.7 Hz, 1H), 7.60 (dd, *J* = 12.8, 7.3 Hz, 2H), 7.01 - 6.97 (m, 1H), 6.01 (d, *J* = 8.1 Hz, 1H), 5.44 (d, *J* = 20.5 Hz, 1H), 4.92 (d, *J*= *20.3* Hz, 1H), 4.54 - 4.42 (m, 2H), 3.60 - 3.45 (m, 4H), 3.42 - 3.24 (m, 4H), 3.07 - 2.94 (m, 1H), 2.69 (d, *J* = 17.3 Hz, 1H), 2.47 - 2.37 (m, 1H), 2.18 - 2.11 (m, 1H). LCMS (ESI) calcd. for $C_{23}H_{25}FN_5O_2S^+$ $[M+H]^+$: 454.17, found, 454.1.

**Example 379: Preparation of 3-(4-((4-(6-fluoropyridin-2-yl)piperazin-1-yl)methyl)-1-thioxoisoindolin-2-yl)piperidine-2,6-dione (GT-08698)**

With reference to the method of Scheme 4, the target compound GT-08698 was obtained (9 mg, white solid, yield 17%). $^1$H NMR (400 MHz, DMSO-d6) δ 11.30 (s, 1H), 11.20 (s, 1H), 7.99 (t, *J*= 6.7 Hz, 2H), 7.79 - 7.66 (m, 2H), 6.79 (d, *J*= *7.6* Hz, 1H), 6.39 (d, *J*= 6.7 Hz, 1H), 6.01 (d, *J* = 9.8 Hz, 1H), 5.32 (d, *J* = 19.0 Hz, 1H), 4.90 (d, *J*= 19.7 Hz, 1H), 4.54 - 4.44 (m, 2H), 4.38 - 4.25 (m, 2H), 3.58 - 3.39 (m, 4H), 3.24 - 3.09 (m, 2H), 3.08 - 2.92 (m, 1H), 2.69 (d, *J*= 16.1 Hz, 1H), 2.45 - 2.33 (m, 1H), 2.18 - 2.04 (m, 1H). LCMS (ESI) calcd. for $C_{23}H_{25}FN_5O_2S^+$ $[M+H]^+$: 454.17, found, 454.1.

**Example 380: Preparation of 3-(4-((4-(5-fluoropyridin-2-yl)piperazin-1-yl)methyl)-1-thioxoisoindolin-2-yl)piperidine-2,6-dione (GT-08699)**

With reference to the method of Scheme 4, the target compound GT-08699 was obtained (11 mg, white solid, yield 21%). $^1$H NMR (400 MHz, DMSO-d6) δ 11.58 (s, 1H), 11.20 (s, 1H), 8.16 (d, *J* = 2.9 Hz, 1H), 8.01 (dd, *J* = 10.3, 7.7 Hz, 2H), 7.68 (t, *J* = 7.7 Hz, 1H), 7.61 (td, *J* = 8.8, 3.1 Hz, 1H), 6.99 (dd, *J* = 9.2, 3.2 Hz, 1H), 6.01 (d, *J*= 7.9 Hz, 1H), 5.41 (d, *J* = 20.4 Hz, 1H), 4.91 (d, *J* = 20.4 Hz, 1H), 4.51 - 4.40 (m, 2H), 4.29 (d, *J*= 12.5 Hz, 2H), 3.44 - 3.36 (m, 4H), 3.24 - 3.21 (m, 2H), 3.07 - 2.93 (m, 1H), 2.69 (d, *J= 16.3 Hz,* 1H), 2.42 - 2.33 (m, 1H), 2.17 - 2.07 (m, 1H). LCMS (ESI) calcd. for $C_{23}H_{25}FN_5O_2S^+$ $[M+H]^+$: 454.17, found, 454.1.

**Example 381: Preparation of 3-(1-thioxo-4-((4-(3-(trifluoromethyl)pyridin-2-yl)piperazin-1-yl)methyl)isoindolin-2-yl)piperidine-2,6-dione (GT-08700)**

With reference to the method of Scheme 4, the target compound GT-08700 was obtained (22 mg, white solid, yield 37%). $^1$H NMR (400 MHz, DMSO-d6) δ 11.49 (s, 1H), 11.21 (s, 1H), 8.59 (d, *J*= 4.6 Hz, 1H), 8.14 (d, *J*= 7.8 Hz, 1H), 8.06 (d, *J*= 7.6 Hz, 1H), 8.00 (d, *J*= 7.7 Hz, 1H), 7.68 (t, *J*= 7.7 Hz, 1H), 7.31 (dd, *J* = 7.5, 4.9 Hz, 1H), 6.02 (d, *J* = 9.4 Hz, 1H), 5.42 (d, *J* = 20.3 Hz, 1H), 4.93 (d, *J* = 20.3 Hz, 1H), 4.61 - 4.43 (m, 2H), 3.56 - 3.47 (m, 5H), 3.47 - 3.38 (m, 1H), 3.37 - 3.21 (m, 2H), 3.04 - 2.92 (m, 1H), 2.70 (d, *J* = 18.8 Hz, 1H), 2.44 - 2.36 (m, 1H), 2.21 - 2.04 (m, 1H). LCMS (ESI) calcd. for $C_{24}H_{25}F_3N_5O_2S^+$ $[M+H]^+$: 504.17, found, 504.1.

**Example 382: Preparation of 3-(1-thioxo-4-((4-(6-(trifluoromethyl)pyridin-2-yl)piperazin-1-yl)methyl)isoindolin-2-yl)piperidine-2,6-dione (GT-08701)**

With reference to the method of Scheme 4, the target compound GT-08701 was obtained (17 mg, white solid, yield 29%). $^1$H NMR (400 MHz, DMSO-d6) δ 11.20 (s, 1H), 8.05 - 7.91 (m, 2H), 7.84 (t, *J* = 6.3 Hz, 1H), 7.69 (t, *J*= 6.5 Hz, 1H), 7.23 (d, *J*= 8.2 Hz, 1H), 7.16 (d, *J*= 6.8 Hz, 1H), 6.02 (d, *J* = 12.1 Hz, 1H), 5.35 - 5.24 (m, 1H), 4.91 (d, *J* = 20.5 Hz, 1H), 4.57 - 4.34 (m, 4H), 3.63 - 3.39 (m, 4H), 3.25 - 3.21 (m, 2H), 3.01 - 2.95 (m, 1H), 2.69 (d, *J* = 14.5 Hz, 1H), 2.43 - 2.34 (m, 1H), 2.16 - 2.07 (m, 1H). LCMS (ESI) calcd. for $C_{24}H_{25}F_3N_5O_2S^+$ $[M+H]^+$: 504.17, found, 504.1.

**Example 383: Preparation of 3-(1-thioxo-4-((4-(5-(trifluoromethyl)pyridin-2-yl)piperazin-1-yl)methyl)isoindolin-2-yl)piperidine-2,6-dione (GT-08702)**

With reference to the method of Scheme 4, the target compound GT-08702 was obtained (20 mg, white solid, yield 34%). $^1$H NMR (400 MHz, DMSO-d6) δ 11.69 (s, 1H), 11.20 (s, 1H), 8.48 (s, 1H), 8.06 - 7.96 (m, 2H), 7.92 (dd, *J* = 8.9, 2.3 Hz, 1H), 7.68 (t, *J*= 7.7 Hz, 1H), 7.08 (d, *J* = 9.0 Hz, 1H), 6.01 (d, *J* = 8.8 Hz, 1H), 5.39 (d, *J* = 20.4 Hz, 1H), 4.90 (d, *J* = 20.3 Hz, 1H), 4.58 - 4.41 (m, 4H), 3.56 - 3.45 (m, 4H), 3.24 - 3.14 (m, 2H), 3.07 - 2.91 (m, 1H), 2.69 (d, *J*= 16.8 Hz, 1H), 2.42 - 2.33 (m, 1H), 2.19 - 2.06 (m, 1H). LCMS (ESI) calcd. for $C_{24}H_{25}F_3N_5O_2S^+$ $[M+H]^+$: 504.17, found, 504.1.

**Example 384: Preparation of 3-(4-((4-(3,5-dichloropyridin-2-yl)piperazin-1-yl)methyl)-1-thioxoisoindolin-2-yl)piperidine-2,6-dione (GT-08703)**

With reference to the method of Scheme 4, the target compound GT-08703 was obtained (17 mg, white solid, yield 29%). $^1$H NMR (400 MHz, DMSO-d6) δ 11.39 (s, 1H), 11.21 (s, 1H), 8.33 (d, *J*= 2.2 Hz, 1H), 8.14 (d, *J*= 2.2 Hz, 1H), 8.01 (t, *J*= 7.1 Hz, 2H), 7.68 (t, *J*= 7.7 Hz, 1H), 6.02 (d, *J*= 9.3 Hz, 1H), 5.38 (d, *J*= 20.2 Hz, 1H), 4.92 (d, *J*= *20.3* Hz, 1H), 4.62 - 4.41 (m, 2H), 3.84 (d, *J* = 12.5 Hz, 2H), 3.63 - 3.45 (m, 4H), 3.31 - 3.25 (m, 2H), 3.08 - 2.92 (m, 1H), 2.69 (d, *J* = 17.5 Hz, 1H), 2.48 - 2.38 (m, 1H), 2.18 - 2.04 (m, 1H). LCMS (ESI) calcd. for $C_{23}H_{24}Cl_2N_5O_2S^+$ $[M+H]^+$: 504.10, found, 504.0.

**Example 385: Preparation of 3-(4-((4-(3,4-difluoropyridin-2-yl)piperazin-1-yl)methyl)-1-thioxoisoindolin-2-yl)piperidine-2,6-dione (GT-08728)**

With reference to the method of Scheme 4, the target compound GT-08728 was obtained (22 mg, white solid, yield 40%). $^1$H NMR (400 MHz, DMSO-d6) δ 11.50 (s, 1H), 11.21 (s, 1H), 8.12 - 7.99 (m, 3H), 7.68 (t, *J* = 7.7 Hz, 1H), 7.11 - 7.05 (m, 1H), 6.01 (d, *J* = 9.4 Hz, 1H), 5.38 (d, *J* = 20.5 Hz, 1H), 4.91 (d, *J* = 20.3 Hz, 1H), 4.59 - 4.37 (m, 2H), 4.11 (d, *J* = 13.7 Hz, 2H), 3.54 - 3.50 (m, 2H), 3.40 (d, *J* = 10.5 Hz, 1H), 3.35 - 3.23 (m, 3H), 3.05 - 2.94 (m, 1H), 2.69 (d, *J* = 16.8 Hz, 1H), 2.45 - 2.38 (m, 1H), 2.18 - 2.11 (m, 1H). LCMS (ESI) calcd. for $C_{23}H_{24}F_2N_5O_2S^+$ [M+H]$^+$: 472.16, found, 472.1.

**Example 386: Preparation of 3-(4-((4-(4-chloro-3-fluoropyridin-2-yl)piperazin-1-yl)methyl)-1-thioxoisoindolin-2-yl)piperidine-2,6-dione (GT-08803)**

With reference to the method of Scheme 4, the target compound GT-08803 was obtained (11 mg, white solid, yield 19%). $^1$H NMR (400 MHz, DMSO-d6) δ 11.43 (s, 1H), 11.12 (s, 1H), 8.04 - 8.00 (m, 2H), 7.80 (d, *J* = 7.3 Hz, 1H), 7.61 (d, *J* = 7.3 Hz, 1H), 7.53 (t, *J* = *7.5* Hz, 1H), 6.06 - 5.95 (m, 1H), 5.36 *(d, J* = *21.2* Hz, 1H), 4.91 (d, *J* = 22.5 Hz, 1H), 4.56 - 4.40 (m, 2H), 4.10 (d, *J* = 12.9 Hz, 3H), 3.49 - 3.36 (m, 4H), 3.31 - 3.22 (m, 2H), 3.01 - 2.94 (m, 1H), 2.67 - 2.62 (m, 1H), 2.45 - 2.33 (m, 2H), 2.13 - 2.09 (m, 1H). LCMS (ESI) calcd. for $C_{23}H_{24}ClFN_5O_2S^+$ [M+H]$^+$: 488.13, found, 488.1.

**Example 387: Preparation of 3-(4-((4-(3-fluoro-4-iodopyridin-2-yl)piperazin-1-yl)methyl)-1-thioxoisoindolin-2-yl)piperidine-2,6-dione (GT-08704)**

With reference to the method of Scheme 4, the target compound GT-08704 was obtained (15 mg, white solid, yield 22%). $^1$H NMR (400 MHz, DMSO-d6) δ 11.20 (s, 1H), 7.99 (t, *J* = 7.6 Hz, 2H), 7.73 (d, *J* = 5.1 Hz, 1H), 7.68 *(t, J* = 7.6 Hz, 1H), 7.39 (dd, *J* = 5.0, 3.8 Hz, 1H), 6.01 *(d, J* = 11.6 Hz, 1H), 5.31 (d, *J* = *19.9* Hz, 1H), 4.91 (d, *J* = 20.4 Hz, 1H), 4.57 - 4.38 (m, 2H), 4.05 (d, *J* = 12.8 Hz, 2H), 3.44 - 3.36 (m, 4H), 3.32 - 3.23 (m, 2H), 3.03 - 2.95 (m, 1H), 2.69 (d, *J* = 16.3 Hz, 1H), 2.43 - 2.35 (m, 1H), 2.19 - 2.08 (m, 1H). LCMS (ESI) calcd. for $C_{23}H_{24}FIN_5O_2S^+$ [M+H]$^+$: 580.17, found, 580.0.

**Example 388: Preparation of 3-(4-((4-(3-bromo-4-chloropyridin-2-yl)piperazin-1-yl)methyl)-1-thioxoisoindolin-2-yl)piperidine-2,6-dione (GT-08804)**

With reference to the method of Scheme 4, the target compound GT-08804 was obtained (12 mg, white solid, yield 19%). $^1$H NMR (400 MHz, DMSO-d6) δ 11.45 (s, 1H), 11.21 (s, 1H), 8.25 (d, *J* = 5.2 Hz, 1H), 8.02 (dd, *J* = 13.2, 7.7 Hz, 2H), 7.68 (t, *J* = 7.8 Hz, 1H), 7.37 (d, *J* = 5.2 Hz, 2H), 6.02 (d, *J* = 9.2 Hz, 1H), 5.41 (d, *J* = 20.5 Hz, 1H), 4.93 (d, *J* = *20.3* Hz, 1H), 4.63 - 4.50 (m, 2H), 3.88 - 3.80 (m, 2H), 3.53 - 3.38 (m, 6H), 3.03 - 2.95 (m, 1H), 2.69 (d, *J* = 18.3 Hz, 1H), 2.45 - 2.38 (m, 1H), 2.17 - 2.11 (m, 1H). LCMS (ESI) calcd. for $C_{23}H_{24}BrClN_5O_2S^+$ [M+H]$^+$: 548.05, found, 548.0.

**Example 389: Preparation of 3-(4-((4-(3-chloro-5-(trifluoromethyl)pyridin-2-yl)piperazin-1-yl)methyl)-1-thioxoisoindolin-2-yl)piperidine-2,6-dione (GT-08705)**

With reference to the method of Scheme 4, the target compound GT-08705 was obtained (17 mg, white solid, yield 27%). $^1$H NMR (400 MHz, DMSO-d6) δ 11.21 (s, 1H), 8.63 (s, 1H), 8.30 (s, 1H), 8.00 (d, *J* = 6.4 Hz, 2H), 7.69 (t, *J* = 7.5 Hz, 1H), 6.01 (d, *J* = 13.8 Hz, 1H), 5.33 (d, *J* = 17.8 Hz, 1H), 4.92 (d, *J* = 20.5 Hz, 1H), 4.62 - 4.40 (m, 2H), 4.12 (d, *J* = 15.0 Hz, 2H), 3.63 - 3.40 (m, 6H), 3.03 - 2.95 (m, 1H), 2.69 (d, *J* = 16.1 Hz, 1H), 2.41 - 2.30 (m, 1H), 2.19 - 2.06 (m, 1H). LCMS (ESI) calcd. for $C_{24}H_{24}ClF_3N_5O_2S^+$ [M+H]$^+$: 583.13, found, 583.0.

**Example 390: Preparation of 3-(4-((4-(pyridin-3-yl)piperazin-1-yl)methyl)-1-thioxoisoindolin-2-yl)piperidine-2,6-dione (GT-08706)**

With reference to the method of Scheme 4, the target compound GT-08706 was obtained (15 mg, white solid, yield 29%). $^1$H NMR (400 MHz, DMSO-d6) δ 11.88 (s, 1H), 11.21 (s, 1H), 8.54 (d, *J* = 2.6 Hz, 1H), 8.26 (d, *J* = 5.2 Hz, 1H), 8.08 (dd, *J* = 8.7, 2.3 Hz, 1H), 8.04 - 7.96 (m, 2H), 7.84 (dd, *J* = 8.8, 5.3 Hz, 1H), 7.68 (t, *J* = 7.7 Hz, 1H), 6.02 (d, *J* = 8.2 Hz, 1H), 5.45 (d, *J* = *20.4* Hz, 1H), 4.91 (d, *J* = 20.6 Hz, 1H), 4.54 - 4.41 (m, 2H), 4.23 - 4.00 (m, 2H), 3.78 - 3.53 (m, 6H), 3.06 - 2.95 (m, 1H), 2.69 (d, *J* = 15.9 Hz, 1H), 2.47 - 2.41 (m, 1H), 2.18 - 2.09 (m, 1H). LCMS (ESI) calcd. for $C_{23}H_{26}N_5O_2S^+$ [M+H]$^+$: 436.18, found, 436.1.

**Example 391: Preparation of 3-(4-((4-(6-chloropyridin-3-yl)piperazin-1-yl)methyl)-1-thioxoisoindolin-2-yl)piperidine-2,6-dione (GT-08723)**

With reference to the method of Scheme 4, the target compound GT-08723 was obtained (17 mg, white solid, yield 31%). $^1$H NMR (400 MHz, DMSO-d6) δ 11.55 (s, 1H), 11.21 (s, 1H), 8.14 (d, *J*= 3.0 Hz, 1H), 8.05 - 7.98 (m, 1H), 7.73 - 7.65 (m, 1H), 7.51 - 7.47 (m, 1H), 7.36 (d, *J* = 8.8 Hz, 1H), 6.02 (d, *J* = 9.4 Hz, 1H), 5.39 (d, *J= 20.4* Hz, 1H), 4.91 (d, *J*= 20.3 Hz, 1H), 4.55 - 4.42 (m, 2H), 3.97 - 3.88 (m, 2H), 3.51 - 3.47 (m, 2H), 3.36 - 3.30 (m, 4H), 3.08 - 2.94 (m, 1H), 2.69 (d, *J* = 16.7 Hz, 1H), 2.43 - 2.37 (m, 1H), 2.17 - 2.11 (m, 1H). LCMS (ESI) calcd. for $C_{23}H_{25}ClN_5O_2S^+$ [M+H]$^+$: 470.14, found, 470.1.

**Example 392: Preparation of 3-(4-((4-(4,5-dichloropyridin-3-yl)piperazin-1-yl)methyl)-1-thioxoisoindolin-2-yl) piperidine-2,6-dione (GT-08724)**

With reference to the method of Scheme 4, the target compound GT-08724 was obtained (19 mg, white solid, yield 32%). $^1$H NMR (400 MHz, DMSO-d6) δ 11.30 (s, 1H), 11.21 (s, 1H), 8.51 (s, 1H), 8.41 (s, 1H), 8.08 - 7.94 (m, 2H), 7.69 (t, *J* = 7.6 Hz, 1H), 6.02 (d, *J* = 9.1 Hz, 1H), 5.33 (d, *J* = 20.2 Hz, 1H), 4.93 (d, *J* = 20.1 Hz, 1H), 4.53 (s, 2H), 3.66 - 3.62 (m, 4H), 3.40 - 3.28 (m, 4H), 3.06 - 2.93 (m, 1H), 2.70 (d, *J* = 19.7 Hz, 1H), 2.44 - 2.41 (m, 1H), 2.15 - 2.07 (m, 1H). LCMS (ESI) calcd. for $C_{23}H_{24}Cl_2N_5O_2S^+$ [M+H]$^+$: 504.10, found, 504.0.

**Example 393: Preparation of 3-(4-((4-(2,4-dichloropyridin-3-yl)piperazin-1-yl)methyl)-1-thioxoisoindolin-2-yl) piperidine-2,6-dione (GT-08725)**

With reference to the method of Scheme 4, the target compound GT-08725 was obtained (13 mg, white solid, yield 22%). $^1$H NMR (400 MHz, DMSO-d6) δ 11.31 (s, 1H), 11.21 (s, 1H), 8.18 (s, 1H), 8.07 (d, *J*= 6.0 Hz, 1H), 8.00 (d, *J= 7.5* Hz, 1H), 7.77 - 7.62 (m, 2H), 6.02 (d, *J* = 9.9 Hz, 1H), 5.35 (d, *J* = 21.1 Hz, 1H), 4.94 (d, *J*= 20.6 Hz, 1H), 4.53 (s, 2H), 3.95 - 3.75 (m, 2H), 3.54 - 3.47 (m, 1H), 3.31 - 3.25 (m, 2H), 3.25 - 3.09 (m, 3H), 3.06 - 2.93 (m, 1H), 2.70 (d, *J*= 18.2 Hz, 1H), 2.47 - 2.40 (m, 1H), 2.20 - 2.07 (m, 1H). LCMS (ESI) calcd. for $C_{23}H_{24}Cl_2N_5O_2S^+$ [M+H]$^+$: 504.10, found, 504.0.

**Example 394: Preparation of 3-(4-((4-(pyridin-4-yl)piperazin-1-yl)methyl)-1-thioxoisoindolin-2-yl)piperidine-2,6-dione (GT-08726)**

With reference to the method of Scheme 4, the target compound GT-08726 was obtained (15 mg, white solid, yield 29%). $^1$H NMR (400 MHz, DMSO-d6) δ 14.08 (s, 1H), 12.20 (s, 1H), 11.20 (s, 1H), 8.36 *(d, J*= 6.7 Hz, 2H), 7.99 (d, *J* = 7.4 Hz, 2H), 7.67 *(t, J*= 7.5 Hz, 1H), 7.26 *(d, J*= 7.3 Hz, 2H), 6.01 *(d, J*= 8.5 Hz, 1H), 5.56 - 5.31 (m, 1H), 4.90 (d, *J= 20.3* Hz, 1H), 4.67 - 4.20 (m, 4H), 3.91 - 3.67 (m, 2H), 3.25 - 3.21 (m, 4H), 3.07 - 2.99 (m, 1H), 2.69 (d, *J* = 15.8 Hz, 1H), 2.45 - 2.35 (m, 1H), 2.18 - 2.05 (m, 1H). LCMS (ESI) calcd. for $C_{23}H_{26}N_5O_2S^+$ [M+H]$^+$: 436.18, found, 436.1.

**Example 395: Preparation of 3-(4-((4-(2-methylpyridin-4-yl)piperazin-1-yl)methyl)-1-thioxoisoindolin-2-yl)piperidine-2,6-dione (GT-08727)**

With reference to the method of Scheme 4, the target compound GT-08727 was obtained (16 mg, white solid, yield 30%). $^1$H NMR (400 MHz, DMSO-d6) δ 13.97 (s, 1H), 12.11 (s, 1H), 11.20 (s, 1H), 8.25 (s, 1H), 7.98 (s, 2H), 7.67 (s, 1H), 7.19 (s, 1H), 7.13 (d, *J* = 7.0 Hz, 1H), 6.01 (d, *J* = 8.8 Hz, 1H), 5.52 - 5.39 (m, 1H), 4.89 (d, *J* = 20.2 Hz, 1H), 4.54 - 4.45 (m, 1H), 4.50 - 4.23 (m, 3H), 3.86 - 3.68 (m, 2H), 3.34 - 3.17 (m, 4H), 3.02 - 2.90 (m, 1H), 2.69 (d, *J* = 14.9 Hz, 1H), 2.46 (s, 3H), 2.44 - 2.37 (m, 1H), 2.17 - 2.05 (m, 1H). LCMS (ESI) calcd. for $C_{24}H_{28}N_5O_2S^+$ [M+H]$^+$: 450.20, found, 450.1.

**Example 396: Preparation of 3-(4-((4-(3-bromopyridin-4-yl)piperazin-1-yl)methyl)-1-thioxoisoindolin-2-yl)piperidine-2,6-dione (GT-08729)**

With reference to the method of Scheme 4, the target compound GT-08729 was obtained (21 mg, white solid, yield 35%). $^1$H NMR (400 MHz, DMSO-d6) δ 11.75 (s, 1H), 11.21 (s, 1H), 8.78 (s, 1H), 8.51 (d, *J*= 6.0 Hz, 1H), 8.04 - 7.96 (m, 2H), 7.69 (t, *J*= 7.6 Hz, 1H), 7.36 *(t, J*= 5.0 Hz, 1H), 6.02 (d, *J*= 11.4 Hz, 1H), 5.42 (d, *J*= 17.1 Hz, 1H), 4.93 (d, *J* = 20.1 Hz, 1H), 4.62 - 4.44 (m, 2H), 3.98 - 3.92 (m, 2H), 3.45 - 3.24 (m, 6H), 3.03 - 2.95 (m, 1H), 2.69 (d, *J*= 17.9 Hz, 1H), 2.46 - 2.37 (m, 1H), 2.19 - 2.06 (m, 1H). LCMS (ESI) calcd. for $C_{23}H_{25}BrN_5O_2S^+$ [M+H]$^+$: 514.09, found, 514.0.

**Example 397: Preparation of 3-(4-((4-(3-fluoropyridin-4-yl)piperazin-1-yl)methyl)-1-thioxoisoindolin-2-yl)piperidine-2,6-dione (GT-08730)**

With reference to the method of Scheme 4, the target compound GT-08730 was obtained (14 mg, white solid,

yield 28%). $^1$H NMR (400 MHz, DMSO-d6) δ 11.77 (s, 1H), 11.20 (s, 1H), 8.69 (d, *J* = 8.2 Hz, 1H), 8.35 (d, *J*= 6.5 Hz, 1H), 8.07 - 7.90 (m, 2H), 7.67 (t, *J*= 7.7 Hz, 1H), 7.45 - 7.32 (m, 1H), 6.02 (d, *J* = 10.3 Hz, 1H), 5.44 - 5.25 (m, 1H), 4.90 (d, *J*= 20.3 Hz, 1H), 4.61 - 4.31 (m, 2H), 4.28 - 4.04 (m, 2H), 3.84 - 3.71 (m, 2H), 3.31 - 3.14 (m, 4H), 3.03 - 2.95 (m, 2H), 2.69 (d, *J* = 15.3 Hz, 2H), 2.44 - 2.37 (m, 1H), 2.18 - 2.07 (m, 1H). LCMS (ESI) calcd. for $C_{23}H_{25}FN_5O_2S^+$ [M+H]$^+$: 454.17, found, 454.1.

**Example 398: Preparation of 3-(4-((4-(3-chloropyridin-4-yl)piperazin-1-yl)methyl)-1-thioxoisoindolin-2-yl)piperidine-2,6-dione (GT-08731)**

With reference to the method of Scheme 4, the target compound GT-08731 was obtained (19 mg, white solid, yield 34%). $^1$H NMR (400 MHz, DMSO-d6) δ 11.94 (s, 1H), 11.21 (s, 1H), 8.71 (s, 1H), 8.48 (d, *J*= 6.3 Hz, 1H), 8.04 - 7.99 (m, 2H), 7.68 (t, *J*= 7.6 Hz, 1H), 7.41 (d, *J*= 6.0 Hz, 1H), 6.02 (d, *J* = 8.0 Hz, 1H), 5.45 (d, *J* = 19.8 Hz, 1H), 4.93 (d, *J* = 20.5 Hz, 1H), 4.51 (dd, *J* = 33.6, 12.7 Hz, 2H), 4.15 - 3.96 (m, 4H), 3.44 - 3.28 (m, 4H), 3.07 - 2.93 (m, 1H), 2.69 (d, *J*= 17.3 Hz, 1H), 2.47 - 2.36 (m, 1H), 2.20 - 2.06 (m, 1H). LCMS (ESI) calcd. for $C_{23}H_{25}ClN_5O_2S^+$ [M+H]$^+$: 470.14, found, 470.1.

**Example 399: Preparation of 3-(4-((4-(2-chloro-3-fluoropyridin-4-yl)piperazin-1-yl)methyl)-1-thioxoisoindolin-2-yl)piperidine-2,6-dione (GT-08732)**

With reference to the method of Scheme 4, the target compound GT-08732 was obtained (6 mg, white solid, yield 11%). $^1$H NMR (400 MHz, DMSO-d6) δ 11.52 (s, 1H), 11.21 (s, 1H), 8.08 - 7.97 (m, 3H), 7.68 (t, *J*= 7.7 Hz, 1H), 7.13 (t, *J* = 6.1 Hz, 1H), 6.02 (d, *J* = 12.4 Hz, 1H), 5.35 (d, *J* = 20.4 Hz, 1H), 4.91 (d, *J*= 20.2 Hz, 1H), 4.59 - 4.38 (m, 2H), 3.88 (d, *J*= 12.7 Hz, 2H), 3.52 - 3.43 (m, 4H), 3.39 - 3.31 (m, 4H), 2.69 (d, *J* = 18.0 Hz, 1H), 2.42 - 2.38 (m, 1H), 2.17 - 2.11 (m, 1H). LCMS (ESI) calcd. for $C_{23}H_{24}ClFN_5O_2S^+$ [M+H]$^+$: 488.13, found, 488.1.

**Example 400: Preparation of 3-(4-((4-(2-bromo-3-fluoropyridin-4-yl)piperazin-1-yl)methyl)-1-thioxoisoindolin-2-yl)piperidine-2,6-dione (GT-08733)**

With reference to the method of Scheme 4, the target compound GT-08733 was obtained (10 mg, white solid, yield 16%). $^1$H NMR (400 MHz, DMSO-d6) δ 11.64 (s, 1H), 11.21 (s, 1H), 8.04 - 7.97 (m, 3H), 7.68 (t, *J* = 7.7 Hz, 1H), 7.18 - 7.05 (m, 1H), 6.02 *(d, J*= 10.6 Hz, 1H), 5.38 (d, *J* = 21.0 Hz, 1H), 4.91 (d, *J* = 20.0 Hz, 1H), 4.55 - 4.36 (m, 2H), 3.87 (d, *J* = 12.8 Hz, 2H), 3.48 - 3.42 (m, 2H), 3.39 - 3.25 (m, 4H), 3.07 - 2.94 (m, 1H), 2.69 (d, *J*= 17.2 Hz, 1H), 2.43 - 2.37 (m, 1H), 2.18 - 2.07 (m, 1H). LCMS (ESI) calcd. for $C_{23}H_{24}BrFN_5O_2S^+$ [M+H]$^+$: 532.08, found, 532.0.

**Example 401: Preparation of 3-(4-((4-(3-bromo-2-chloropyridin-4-yl)piperazin-1-yl)methyl)-1-thioxoisoindolin-2-yl)piperidine-2,6-dione (GT-08734)**

With reference to the method of Scheme 4, the target compound GT-08734 was obtained (14 mg, white solid, yield 22%). $^1$H NMR (400 MHz, DMSO-d6) δ 11.35 (s, 1H), 11.21 (s, 1H), 8.26 (d, *J*= 5.4 Hz, 1H), 8.00 (d, *J* = 7.4 Hz, 2H), 7.70 (d, *J* = 7.6 Hz, 1H), 7.17 (d, *J* = 5.3 Hz, 1H), 6.00 (t, *J* = 14.8 Hz, 1H), 5.34 (d, *J*= 20.1 Hz, 1H), 4.93 (d, *J* = 20.8 Hz, 1H), 4.53 (s, 2H), 3.75 - 3.68 (m, 2H), 3.62 - 3.44 (m, 6H), 3.04 - 2.94 (m, 1H), 2.69 (d, *J* = 18.0 Hz, 1H), 2.47 - 2.40 (m, 1H), 2.14 - 2.04 (m, 1H). LCMS (ESI) calcd. for $C_{23}H_{24}BrClN_5O_2S^+$ [M+H]$^+$: 550.05, found, 550.0.

**Example 402: Preparation of 3-(4-((4-(2,3-dichloropyridin-4-yl)piperazin-1-yl)methyl)-1-thioxoisoindolin-2-yl) piperidine-2,6-dione (GT-08735)**

With reference to the method of Scheme 4, the target compound GT-08735 was obtained (17 mg, white solid, yield 29%). $^1$H NMR (400 MHz, DMSO-d6) δ 11.37 (s, 1H), 11.21 (s, 1H), 8.22 (d, *J*= 5.4 Hz, 1H), 8.00 (d, *J* = 5.6 Hz, 2H), 7.69 (t, *J*= 6.3 Hz, 1H), 7.20 (d, *J*= 5.2 Hz, 1H), 6.11 - 5.89 (m, 1H), 5.37 - 5.31 (m, 1H), 4.92 (d, *J* = 20.2 Hz, 1H), 4.61 - 4.42 (m, 2H), 3.79 - 3.65 m, 2H), 3.60 - 3.41 (m, 6H), 3.07 - 2.94 (m, 1H), 2.69 (d, *J* = 18.2 Hz, 1H), 2.42 - 2.33 (m, 2H), 2.14 - 2.09 (m, 1H). LCMS (ESI) calcd. for $C_{23}H_{24}Cl_2N_5O_2S^+$ [M+H]$^+$: 504.10, found, 504.0.

**Example 403: Preparation of 3-(4-((4-(2,3-difluoropyridin-4-yl)piperazin-1-yl)methyl)-1-thioxoisoindolin-2-yl)piperidine-2,6-dione (GT-08736)**

With reference to the method of Scheme 4, the target compound GT-08736 was obtained (15 mg, white solid, yield 27%). $^1$H NMR (400 MHz, DMSO-d6) δ 11.63 (s, 1H), 11.20 (s, 1H), 7.99 (d, *J* = 7.1 Hz, 2H), 7.80 (d, *J*= 5.6 Hz, 1H), 7.68 (t, *J*= 7.0 Hz, 1H), 7.04 (t, *J*= 5.6 Hz, 1H), 6.01 (d, *J* = 9.1 Hz, 1H), 5.37 (d, *J*= 19.7 Hz, 1H), 4.91 (d, *J*= 19.7 Hz, 1H), 4.59 - 4.34 (m, 2H), 3.98 - 3.83 (m, 2H), 3.62 - 3.49 (m, 2H), 3.34 - 3.17 (m, 4H), 3.05 - 2.92 (m, 1H), 2.69 (d, *J* = 16.6 Hz, 1H), 2.46 - 2.36 (m, 1H), 2.15 - 2.07 (m, 1H). LCMS (ESI) calcd. for $C_{23}H_{24}F_2N_5O_2S^+$ [M+H]$^+$: 472.16, found, 472.1.

**Example 404: Preparation of 3-(4-((4-(3-bromo-5-chloropyridin-4-yl)piperazin-1-yl)methyl)-1-thioxoisoindolin-2-yl)piperidine-2,6-dione (GT-08801)**

With reference to the method of Scheme 4, the target compound GT-08801 was obtained (10 mg, white solid, yield 16%). $^1$H NMR (400 MHz, DMSO-d6) δ 11.54 (s, 1H), 11.21 (s, 1H), 8.64 (s, 1H), 8.54 (s, 1H), 8.07 (d, *J* = 7.7 Hz, 1H), 8.00 (d, *J* = 7.5 Hz, 1H), 7.73 - 7.65 (m, 1H), 6.02 (d, *J* = 8.4 Hz, 1H), 5.40 (d, *J*= 20.8 Hz, 1H), 4.94 (d, *J*= 20.4 Hz, 1H), 4.66 - 4.53 (m, 3H), 3.60 - 3.50 (m, 2H), 3.49 - 3.37 (m, 5H), 3.05 - 2.96 (m, 1H), 2.69 (d, *J*= 17.5 Hz, 1H), 2.42 - 2.37 (m, 1H), 2.18 - 2.08 (m 1H). LCMS (ESI) calcd. for $C_{23}H_{24}BrClN_5O_2S^+$ [M+H]$^+$: 548.05, found, 548.0.

**Example 405: Preparation of 3-(4-((4-(3-chloro-5-fluoropyridin-4-yl)piperazin-1-yl)methyl)-1-thioxoisoindolin-2-yl)piperidine-2,6-dione (GT-08802)**

With reference to the method of Scheme 4, the target compound GT-08802 was obtained (14 mg, white solid, yield 24%). $^1$H NMR (400 MHz, DMSO-d6) δ 11.81 (s, 1H), 11.21 (s, 1H), 8.49 (s, 1H), 8.43 (s, 1H), 8.08 (d, *J*= 7.6 Hz, 1H), 8.00 (d, *J*= 7.7 Hz, 1H), 7.72 - 7.65 (m, 1H), 6.02 (d, *J*= 9.1 Hz, 1H), 5.45 (d, *J* = 20.5 Hz, 1H), 4.93 (d, *J* = 20.4 Hz, 1H), 4.61 - 4.49 (m, 3H), 3.73 - 3.62 (m, 2H), 3.60 - 3.47 (m, 5H), 3.04 - 2.95 (m, 1H), 2.69 (d, *J* = 18.5 Hz, 1H), 2.45 - 2.37 (m, 1H), 2.16 - 2.11 (m, 1H). LCMS (ESI) calcd. for $C_{23}H_{24}ClFN_5O_2S^+$ [M+H]$^+$: 488.13, found, 488.1.

**Example 406: Preparation of 3-(4-((4-(3,5-dichloropyridin-4-yl)piperazin-1-yl)methyl)-1-thioxoisoindolin-2-yl)piperidine-2,6-dione (GT-08805)**

With reference to the method of Scheme 4, the target compound GT-08805 was obtained (13 mg, white solid, yield 22%). $^1$H NMR (400 MHz, DMSO-d6) δ 11.64 (s, 1H), 11.21 (s, 1H), 8.09 (d, *J* = 6.3 Hz, 1H), 8.00 (d, *J*= 7.7 Hz, 1H), 7.93 (d, *J*= *7.6* Hz, 1H), 7.69 (dd, *J*= 16.4, 8.5 Hz, 2H), 6.02 (d, *J*= 8.8 Hz, 1H), 5.44 (d, *J*= 19.8 Hz, 1H), 4.94 (d, *J*= *20.3* Hz, 1H), 4.60 - 4.49 (m, 2H), 3.53 - 3.38 (m, 6H), 3.37 - 3.23 (m, 4H), 3.03 - 2.95 (m, 1H), 2.69 (d, *J* = 17.6 Hz, 1H), 2.44 - 2.33 (m, 1H), 2.15 - 2.11 (m, 1H). LCMS (ESI) calcd. for $C_{23}H_{24}Cl_2N_5O_2S^+$ [M+H]$^+$: 504.10, found, 504.0.

**Example 407: Preparation of 3-(4-((4-(2,6-dimethylpyridin-4-yl)piperazin-1-yl)methyl)-1-thioxoisoindolin-2-yl)piperidine-2,6-dione (GT-08806)**

With reference to the method of Scheme 4, the target compound GT-08806 was obtained (19 mg, white solid, yield 35%). $^1$H NMR (400 MHz, DMSO-d6) δ 12.20 (s, 1H), 11.21 (s, 1H), 8.03 - 7.98 (m, 3H), 7.72 - 7.65 (m, 2H), 6.01 (d, *J*= 7.9 Hz, 1H), 5.49 (d, *J*= 20.5 Hz, 1H), 4.90 (d, *J*= 20.4 Hz, 1H), 4.47 - 4.30 (m, 4H), 3.81 - 3.68 (m, 2H), 3.38 - 3.26 (m, 4H), 3.02 - 2.91 (m, 1H), 2.72 (d, *J*= 10.8 Hz, 1H), 2.48 (s, 6H), 2.41 - 2.33 (m, 2H), 2.14 - 2.05 (m, 1H). LCMS (ESI) calcd. for $C_{25}H_{30}N_5O_2S^+$ [M+H]$^+$: 464.21, found, 464.2.

**Example 408: Preparation of 3-(4-((4-(perfluoropyridin-4-yl)piperazin-1-yl)methyl)-1-thioxoisoindolin-2-yl)piperidine-2,6-dione (GT-08809)**

With reference to the method of Scheme 4, the target compound GT-08809 was obtained (13 mg, white solid, yield 22%). $^1$H NMR (400 MHz, DMSO-d6) δ 11.33 (s, 1H), 11.20 (s, 1H), 7.99 (s, 2H), 7.69 (s, 1H), 6.03 (s, 1H), 5.30 (d, *J* = 21.4 Hz, 1H), 4.90 (d, *J* = 21.7 Hz, 1H), 4.63 - 4.41 (m, 2H), 3.96 - 3.64 (m, 4H), 3.65 - 3.47 (m, 2H), 3.23 - 3.17 (m, 2H), 3.04 - 2.95 (m, 1H), 2.69 (d, *J* = 17.0 Hz, 2H), 2.42 - 2.35 (m, 1H), 2.23 - 2.04 (m, 1H). LCMS (ESI) calcd. for $C_{23}H_{22}F_4N_5O_2S^+$ [M+H]$^+$: 508.14, found, 508.1.

**Example 409: Preparation of 3-(4-((4-(pyridazin-3-yl)piperazin-1-yl)methyl)-1-thioxoisoindolin-2-yl)piperidine-2,6-dione (GT-08810)**

With reference to the method of Scheme 4, the target compound GT-08810 was obtained (14 mg, white solid, yield 27%). $^1$H NMR (400 MHz, DMSO-d6) δ 11.94 (s, 1H), 11.21 (s, 1H), 8.83 (d, *J* = 4.1 Hz, 1H), 8.08 - 7.97 (m, 2H), 7.90 - 7.82 (m, 2H), 7.68 (t, *J* = 7.7 Hz, 1H), 6.02 (d, *J* = 8.7 Hz, 1H), 5.44 (d, *J* = 20.2 Hz, 1H), 4.91 (d, *J*= 20.3 Hz, 1H), 4.54 - 4.41 (m, 4H), 3.60 - 3.48 (m, 2H), 3.42 - 3.19 (m, 4H), 3.07 - 2.90 (m, 1H), 2.69 (d, *J* = 17.2 Hz, 1H), 2.48 - 2.38 (m, 1H), 2.14 - 2.09 (m, 1H). LCMS (ESI) calcd. for $C_{22}H_{25}N_6O_2S^+$ [M+H]$^+$: 437.18, found, 437.1.

**Example 410: Preparation of 3-(4-((4-(6-chloropyridazin-3-yl)piperazin-1-yl)methyl)-1-thioxoisoindolin-2-yl)piperidine-2,6-dione (GT-08811)**

With reference to the method of Scheme 4, the target compound GT-08811 was obtained (19 mg, white solid,

yield 34%). $^1$H NMR (400 MHz, DMSO-d6) δ 11.86 (s, 1H), 11.21 (s, 1H), 8.04 - 7.97 (m, 2H), 7.66 (d, *J* = 9.7 Hz, 2H), 7.50 (d, *J*= 9.6 Hz, 1H), 6.01 (d, *J*= 8.4 Hz, 1H), 5.43 (d, *J*= 20.3 Hz, 1H), 4.91 (d, *J* = 20.2 Hz, 1H), 4.53 - 4.44 (m, 4H), 3.63 - 3.51 (m, 4H), 3.31 - 3.19 (m, 2H), 3.06 - 2.93 (m, 1H), 2.69 (d, *J* = 16.6 Hz, 1H), 2.45 - 2.40 (m, 1H), 2.18 - 2.06 (m, 1H). LCMS (ESI) calcd. for $C_{22}H_{24}ClN_6O_2S^+$ [M+H]$^+$: 471.14, found, 471.1.

**Example 411: Preparation of 3-(4-((4-(pyrimidin-2-yl)piperazin-1-yl)methyl)-1-thioxoisoindolin-2-yl)piperidine-2,6-dione (GT-08812)**

With reference to the method of Scheme 4, the target compound GT-08812 was obtained (15 mg, white solid, yield 29%). $^1$H NMR (400 MHz, DMSO-d6) δ 11.50 (s, 1H), 11.20 (s, 1H), 8.45 (d, *J*= 4.7 Hz, 2H), 8.00 (d, *J* = 7.7 Hz, 2H), 7.68 (t, *J* = 7.7 Hz, 1H), 6.76 (t, *J*= 4.7 Hz, 1H), 6.01 (d, *J* = 8.9 Hz, 1H), 5.31 (t, *J* = 16.1 Hz, 1H), 4.90 (d, *J* = 20.4 Hz, 1H), 4.71 (d, *J* = 13.2 Hz, 2H), 4.49 - 4.40 (m, 2H), 3.51 - 3.45 (m, 4H), 3.23 - 3.08 (m, 2H), 3.08 - 2.92 (m, 1H), 2.69 (d, *J* = 15.0 Hz, 1H), 2.45 - 2.38 (m, 1H), 2.16 - 2.11 (m, 1H). LCMS (ESI) calcd. for $C_{22}H_{25}N_6O_2S^+$ [M+H]$^+$: 437.18, found, 437.1.

**Example 412: Preparation of 3-(4-((4-(pyrazin-2-yl)piperazin-1-yl)methyl)-1-thioxoisoindolin-2-yl)piperidine-2,6-dione (GT-08813)**

With reference to the method of Scheme 4, the target compound GT-08813 was obtained (14 mg, white solid, yield 27%). $^1$H NMR (400 MHz, DMSO-d6) δ 11.60 (s, 1H), 11.20 (s, 1H), 8.43 (s, 1H), 8.17 (d, *J* = 1.5 Hz, 1H), 8.01 (dd, *J* = 7.7, 2.8 Hz, 2H), 7.95 (d, *J*= 2.6 Hz, 1H), 7.68 (t, *J* = 7.6 Hz, 1H), 6.02 (d, *J* = 9.1 Hz, 1H), 5.37 (d, *J*= 20.2 Hz, 1H), 4.90 (d, *J*= 20.3 Hz, 1H), 4.55 - 4.39 (m, 4H), 3.50 - 3.31 (m, 4H), 3.25 - 3.19 (m, 2H), 3.04 - 2.93 (m, 1H), 2.69 (d, *J*= 17.5 Hz, 1H), 2.45 - 2.37 (m, 1H), 2.18 - 2.05 (m, 1H). LCMS (ESI) calcd. for $C_{22}H_{25}N_6O_2S^+$ [M+H]$^+$: 437.18, found, 437.1.

**Example 413: Preparation of 3-(4-((4-(5,6-dichloropyrimidin-4-yl)piperazin-1-yl)methyl)-1-thioxoisoindolin-2-yl)piperidine-2,6-dione (GT-08814)**

With reference to the method of Scheme 4, the target compound GT-08814 was obtained (13 mg, white solid, yield 22%). $^1$H NMR (400 MHz, DMSO-d6) δ 11.60 (s, 1H), 11.20 (s, 1H), 8.52 (s, 1H), 7.99 (d, *J* = 7.2 Hz, 2H), 7.68 (t, *J*= 7.6 Hz, 1H), 6.02 (d, *J* = 12.4 Hz, 1H), 5.43 - 5.31 (m, 1H), 4.90 (d, *J* = 19.1 Hz, 1H), 4.54 - 4.34 (m, 3H), 3.77 - 3.51 (m, 3H), 3.34 - 3.15 (m, 4H), 3.07 - 2.91 (m, 1H), 2.69 (d, *J* = 16.3 Hz, 1H), 2.46 - 2.35 (m, 1H), 2.18 - 2.05 (m, 1H). LCMS (ESI) calcd. for $C_{22}H_{23}Cl_2N_6O_2S^+$ [M+H]$^+$: 505.10, found, 505.0.

**Example 414: Preparation of 3-(4-((4-(3,6-dichloropyridazin-4-yl)piperazin-1-yl)methyl)-1-thioxoisoindolin-2-yl)piperidine-2,6-dione (GT-08807)**

With reference to the method of Scheme 4, the target compound GT-08807 was obtained (4 mg, white solid, yield 7%). $^1$H NMR (400 MHz, DMSO-d6) δ 11.45 (s, 1H), 11.21 (s, 1H), 8.05 - 7.93 (m, 2H), 7.74 - 7.66 (m, 1H), 7.59 (s, 1H), 6.02 (d, *J* = 9.2 Hz, 1H), 5.46 - 5.16 (m, 1H), 4.91 (d, *J* = 20.8 Hz, 1H), 4.65 - 4.40 (m, 2H), 4.02 - 3.87 (m, 2H), 3.63 - 3.53 (m, 2H), 3.34 - 3.16 (m, 4H), 3.03 - 2.95 (m, 1H), 2.69 (d, *J*= 17.8 Hz, 1H), 2.42 - 2.33 (m, 2H), 2.17 - 2.09 (m, 1H). LCMS (ESI) calcd. for $C_{22}H_{23}Cl_2N_6O_2S^+$ [M+H]$^+$: 505.10, found, 505.0.

**Example 415: Preparation of 3-(4-((4-(5,6-dichloropyridazin-4-yl)piperazin-1-yl)methyl)-1-thioxoisoindolin-2-yl)piperidine-2,6-dione (GT-08815)**

With reference to the method of Scheme 4, the target compound GT-08815 was obtained (12 mg, white solid, yield 20%). $^1$H NMR (400 MHz, DMSO-d6) δ 11.65 (s, 1H), 11.21 (s, 1H), 9.07 (s, 1H), 8.02 (d, *J* = 8.9 Hz, 2H), 7.69 (t, *J* = 7.7 Hz, 1H), 6.02 (d, *J*= 8.4 Hz, 1H), 5.37 (d, *J* = 22.0 Hz, 1H), 4.92 (d, *J* = 20.3 Hz, 1H), 4.59 - 4.44 (m, 2H), 4.04 - 3.94 (m, 2H), 3.46 - 3.23 (m, 6H), 3.06 - 2.95 (m, 1H), 2.69 (d, *J*= 18.0 Hz, 1H), 2.46 - 2.37 (m, 1H), 2.16 - 2.07 (m, 1H). LCMS (ESI) calcd. for $C_{22}H_{23}Cl_2N_6O_2S^+$ [M+H]$^+$: 505.10, found, 505.0.

**Example 416: Preparation of 3-(4-((4-(1,3,5-triazin-2-yl)piperazin-1-yl)methyl)-1-thioxoisoindolin-2-yl)piperidine-2,6-dione (GT-08808)**

With reference to the method of Scheme 4, the target compound GT-08808 was obtained (17 mg, white solid, yield 33%). $^1$H NMR (400 MHz, DMSO-d6) δ 11.71 (s, 1H), 10.98 (s, 1H), 8.72 (s, 2H), 8.14 (dd, *J*= 22.9, 7.7 Hz, 1H), 8.05 - 7.90 (m, 1H), 7.70 (t, *J*= 7.7 Hz, 1H), 6.01 (d, *J*= 14.6 Hz, 1H), 5.37 (d, *J*= *20.4* Hz, 1H), 4.90 (d, *J* = 20.0 Hz, 1H), 4.83 - 4.68 (m, 2H), 4.61 - 4.45 (m, 2H), 3.73 - 3.41 (m, 4H), 3.31 - 3.16 (m, 2H), 2.89 - 2.82 (m, 1H), 2.70 - 2.57 (m, 1H), 2.47 - 2.38 (m, 1H), 2.21 - 2.06 (m, 1H). LCMS (ESI) calcd. for $C_{21}H_{24}N_7O_2S^+$ [M+H]$^+$: 438.17, found, 438.1.

**Example 417: Preparation of 3-(4-((4-(3,4-dichloropyridin-2-yl)piperidin-1-yl)methyl)-1-thioxoisoindolin-2-yl)piperidine-2,6-dione (GT-08457)**

With reference to the method of Scheme 4, the target compound GT-08457 was obtained (7 mg, white solid, yield 12%). $^1$H NMR (400 MHz, DMSO-d6) δ 11.21 (s, 1H), 10.74 (s, 1H), 8.48 (d, *J*= 5.2 Hz, 1H), 8.00 (dd, *J*= 7.6, 3.5 Hz, 2H), 7.70 (d, *J* = 7.7 Hz, 1H), 7.66 (d, *J*= 5.2 Hz, 1H), 6.02 (d, *J* = 9.2 Hz, 1H), 5.24 (d, *J* = 20.1 Hz, 1H), 4.91 (d, *J= 20.3* Hz, 1H), 4.51 - 4.35 (m, 2H), 3.31 - 3.23 (m, 2H), 3.06 - 2.92 (m, 1H), 2.70 (d, *J* = 19.6 Hz, 1H), 2.44 - 2.41 (m, 3H), 2.28 - 2.08 (m, 4H), 1.98 (d, *J* = 13.1 Hz, 2H). LCMS (ESI) calcd. for $C_{24}H_{25}Cl_2N_4O_2S^+$ [M+H]$^+$: 503.11, found, 503.0.

**Example 418: Preparation of 3-(4-((4-(2,3-dichloropyridin-4-yl)piperidin-1-yl)methyl)-1-thioxoisoindolin-2-yl)piperidine-2,6-dione (GT-08458)**

With reference to the method of Scheme 4, the target compound GT-08458 was obtained (6 mg, white solid, yield 10%). $^1$H NMR (400 MHz, DMSO-d6) δ 11.21 (s, 1H), 10.97 (s, 1H), 8.39 (d, *J*= 4.9 Hz, 1H), 7.99 (t, *J* = 6.4 Hz, 2H), 7.69 (t, *J* = 7.6 Hz, 1H), 7.36 (d, *J* = 5.0 Hz, 1H), 6.11 - 5.93 (m, 1H), 5.29 (d, *J* = 21.1 Hz, 1H), 4.91 (d, *J*= 20.6 Hz, 1H), 4.54 - 4.30 (m, 2H), 3.52 (dd, *J*= 27.1, 11.7 Hz, 2H), 3.29 - 3.15 (m, 3H), 3.03 - 2.90 (m, 1H), 2.69 (d, *J* = 18.0 Hz, 1H), 2.25 - 2.04 (m, 4H), 1.99 (d, *J* = 12.2 Hz, 2H). LCMS (ESI) calcd. for $C_{24}H_{25}Cl_2N_4O_2S^+$ [M+H]$^+$: 503.11, found, 503.0.

**Example 419: Preparation of 3-(4-((4-(2,3-difluoropyridin-4-yl)piperidin-1-yl)methyl)-1-thioxoisoindolin-2-yl)piperidine-2,6-dione (GT-08459)**

With reference to the method of Scheme 4, the target compound GT-08459 was obtained (6 mg, white solid, yield 11%). $^1$H NMR (400 MHz, DMSO-d6) δ 11.21 (s, 1H), 11.04 (s, 1H), 8.04 (d, *J*= 5.0 Hz, 1H), 8.00 (d, *J*= 7.7 Hz, 2H), 7.69 (t, *J* = 7.7 Hz, 1H), 7.29 (t, *J*= 4.8 Hz, 1H), 6.02 (d, *J* = 8.3 Hz, 1H), 5.31 (d, *J*= 20.3 Hz, 1H), 4.91 (d, *J= 20.3* Hz, 1H), 4.49 - 4.37 (m, 2H), 3.51 (dd, *J*= 31.0, 11.2 Hz, 2H), 3.24 (d, *J* = 8.9 Hz, 3H), 3.06 - 2.93 (m, 1H), 2.70 (d, *J* = 18.4 Hz, 1H), 2.48 - 2.37 (m, 1H), 2.30 - 2.09 (m, 3H), 1.99 (d, *J*= 13.3 Hz, 2H). LCMS (ESI) calcd. for $C_{24}H_{25}F_2N_4O_2S^+$ [M+H]$^+$: 471.17, found, 471.1.

**Example 420: Preparation of 3-(4-((4,5-dichloro-3',6'-dihydro-[3,4'-bipyridin]-1'(2'H)-yl)methyl)-1-thioxoisoindolin-2-yl)piperidine-2,6-dione (GT-08460)**

With reference to the method of Scheme 4, the target compound GT-08460 was obtained (5 mg, white solid, yield 9%). $^1$H NMR (400 MHz, DMSO-d6) δ 11.22 (s, 1H), 8.76 (s, 1H), 8.38 (s, 1H), 7.80 (d, *J= 7.6* Hz, 1H), 7.70 (t, *J*=7.8 Hz, 1H), 7.61 (d, *J*=7.5 Hz, 1H), 6.06 - 5.97 (m, 2H), 5.93 (s, 1H), 4.86 (d, *J* = 20.2 Hz, 1H), 4.71 (d, *J* = 20.1 Hz,, 1H), 4.60 - 4.48 (m, 2H), 3.97 - 3.88 (m, 2H), 3.71 - 3.61 (m, 4H), 3.01 - 2.92 (m, 1H), 2.67 - 2.59 (m, 2H), 2.15 - 2.04 (m, 1H). LCMS (ESI) calcd. for $C_{24}H_{23}Cl_2N_4O_2S^+$ [M+H]$^+$: 501.19, found, 501.1.

**Example 421: Preparation of 3-(4-((5-chloro-3',6'-dihydro-[3,4'-bipyridin]-1'(2'H)-yl)methyl)-1-thioxoisoindolin-2-yl)piperidine-2,6-dione (GT-08461)**

With reference to the method of Scheme 4, the target compound GT-08461 was obtained (17 mg, white solid, yield 31%). $^1$H NMR (400 MHz, DMSO-d6) δ 11.39 (s, 1H), 11.21 (s, 1H), 8.69 (d, *J*= 4.3 Hz, 1H), 8.60 (d, *J* = 2.1 Hz, 1H), 8.08 - 8.02 (m, 2H), 8.01 (d, *J= 7.5* Hz, 1H), 7.70 (d, *J*= 7.7 Hz, 1H), 6.41 (s, 1H), 6.02 (d, *J* = 8.2 Hz, 1H), 5.35 (dd, *J* = 30.5, 20.2 Hz, 1H), 4.93 (d, *J* = 20.8 Hz, 1H), 4.65 - 4.51 (m, 2H), 3.95 - 3.86 (m, 1H), 3.72 - 3.65 (m, 1H), 3.43 - 3.35 (m, 1H), 3.03 - 2.96 (m, 2H), 2.86 - 2.81 (m, 2H), 2.69 (d, *J* = 17.1 Hz, 1H), 2.46 - 2.41 (m, 1H), 2.20 - 2.09 (m, 1H). LCMS (ESI) calcd. for $C_{24}H_{24}ClN_4O_2S^+$ [M+H]$^+$: 467.13, found, 467.1.

**Example 422: Preparation of 3-(4-((2',3'-dichloro-3,6-dihydro-[4,4'-bipyridin]-1(2H)-yl)methyl)-1-thioxoisoindolin-2-yl)piperidine-2,6-dione (GT-08462)**

With reference to the method of Scheme 4, the target compound GT-08462 was obtained (7 mg, white solid, yield 12%). $^1$H NMR (400 MHz, DMSO-d6) δ 11.22 (s, 1H), 10.97 (s, 1H), 8.41 (d, *J*= 7.4 Hz, 1H), 8.02 - 7.95 (m, 2H), 7.69 (t, *J* = 11.6 Hz, 1H), 7.62 - 7.55 (m, 1H), 7.34 (d, *J* = 4.8 Hz, 1H), 6.11 - 5.96 (m, 1H), 5.94 (s, 1H), 5.23 (t, *J* = 20.9 Hz, 1H), 4.93 (d, *J* = 20.3 Hz, 1H), 4.64 - 4.46 (m, 2H), 4.02 - 3.78 (m, 2H), 3.71 - 3.62 (m, 1H), 3.04 - 2.95 (m, 2H), 2.70 (d, *J* = 19.5 Hz, 1H), 2.62 - 2.56 (m, 2H), 2.47 - 2.38 (m, 1H), 2.21 - 2.09 (m, 1H). LCMS (ESI) calcd. for $C_{24}H_{23}Cl_2N_4O_2S^+$ [M+H]$^+$: 501.09, found, 501.0.

**Example 423: Preparation of 3-(4-((3-fluoro-3',6'-dihydro-[2,4'-bipyridin]-1'(2'H)-yl)methyl)-1-thioxoisoindolin-2-yl)piperidine-2,6-dione (GT-08463)**

With reference to the method of Scheme 4, the target compound GT-08463 was obtained (16 mg, white solid, yield 30%). $^1H$ NMR (400 MHz, DMSO-d6) δ 11.21 (s, 1H), 11.17 (s, 1H), 8.46 (d, *J* = 4.5 Hz, 1H), 8.06 - 8.02 (m, 2H), 7.80 - 7.74 (m, 1H), 7.70 (t, *J* = 7.9 Hz, 1H), 7.46 - 7.42 (m, 1H), 6.56 (s, 1H), 6.02 (d, *J* = 8.8 Hz, 1H), 5.37 - 5.24 (m, 1H), 4.94 (d, *J* = 20.1 Hz, 1H), 4.63 - 4.48 (m, 2H), 4.05 - 3.95 (m, 3H), 3.37 (brs, 1H), 3.08 - 2.90 (m, 4H), 2.70 (d, *J* = 19.1 Hz, 1H), 2.43 - 2.35 (m, 1H), 2.18 - 2.08 (m, 1H). LCMS (ESI) calcd. for $C_{24}H_{24}FN_4O_2S^+$ $[M+H]^+$: 451.16, found, 451.1.

**Example 424: Preparation of 3-(4-((2',3'-difluoro-3,6-dihydro-[4,4'-bipyridin]-1(2H)-yl)methyl)-1-thioxoisoindolin-2-yl)piperidine-2,6-dione (GT-08464)**

With reference to the method of Scheme 4, the target compound GT-08464 was obtained (15 mg, white solid, yield 27%). $^1H$ NMR (400 MHz, DMSO-d6) δ 11.31 (s, 1H), 11.21 (s, 1H), 8.05 - 8.00 (m, 3H), 7.69 (t, *J* = 7.5 Hz, 1H), 7.41 (t, *J* = 5.0 Hz, 1H), 6.35 (s, 1H), 6.02 (d, *J* = 8.1 Hz, 1H), 5.41 - 5.21 (m, 1H), 4.93 (d, *J* = 19.9 Hz, 1H), 4.64 - 4.46 (m, 2H), 3.98 - 3.93 (m, 2H), 3.76 - 3.58 (m, 1H), 3.29 - 3.26 (m, 1H), 3.07 - 2.96 (m, 2H), 2.70 (d, *J* = 17.6 Hz, 2H), 2.45 - 2.41 (m, 1H), 2.19 - 2.07 (m, 1H). LCMS (ESI) calcd. for $C_{24}H_{23}F_2N_4O_2S^+$ $[M+H]^+$: 469.15, found, 469.1.

**Example 425: Preparation of 3-(4-((3-(2,3-dichlorophenyl)-3,6-diazabicyclo[3.1.1]heptan-6-yl)methyl)-1-thioxoisoindolin-2-yl)piperidine-2,6-dione (GT-08465)**

With reference to the method of Scheme 4, the target compound GT-08465 was obtained (10 mg, white solid, yield 17%). $^1H$ NMR (400 MHz, DMSO-d6) δ 11.21 (s, 1H), 11.19 (s, 1H), 7.99 - 7.94 (m, 2H), 7.69 - 7.63 (m, 1H), 7.58 (dd, *J* = 7.2, 2.4 Hz, 1H), 7.41 - 7.35 (m, 2H), 6.02 (d, *J* = 11.4 Hz, 1H), 5.29 (d, *J* = 20.4 Hz, 1H), 4.95 (d, *J* = 20.3 Hz, 1H), 4.89 - 4.73 (m, 2H), 4.52 - 4.34 (m, 2H), 4.07 (d, *J* = 12.3 Hz, 1H), 3.96 - 3.80 (m, 1H), 3.75 (d, *J* = 11.3 Hz, 1H), 3.55 - 3.42 (m, 1H), 3.09 - 2.92 (m, 2H), 2.69 (d, *J* = 14.7 Hz, 2H), 2.47 - 2.42 (m, 1H), 2.18 - 2.08 (m, 1H). LCMS (ESI) calcd. for $C_{25}H_{25}Cl_2N_4O_2S^+$ $[M+H]^+$: 515.11, found, 515.0.

**Example 426: Preparation of 3-(4-((6-(2,3-dichlorophenyl)-3,6-diazabicyclo[3.1.1]heptan-3-yl)methyl)-1-thioxoisoindolin-2-yl)piperidine-2,6-dione (GT-08466)**

With reference to the method of Scheme 4, the target compound GT-08466 was obtained (14 mg, white solid, yield 23%). $^1H$ NMR (400 MHz, DMSO-d6) δ 11.87 (s, 1H), 11.21 (s, 1H), 8.02 - 7.76 (m, 2H), 7.65 - 7.48 (m, 1H), 7.14 (t, *J* = 8.1 Hz, 1H), 6.99 (d, *J* = 7.9 Hz, 1H), 6.68 (d, *J* = 7.9 Hz, 1H), 6.08 - 5.88 (m, 1H), 5.02 (d, *J* = 20.1 Hz, 1H), 4.74 (d, *J* = 19.5 Hz, 1H), 4.63 (brs, 2H), 4.45 - 4.17 (m, 2H), 3.76 - 3.58 (m, 2H), 3.55 - 3.45 (m, 2H), 3.10 - 2.93 (m, 2H), 2.74 - 2.66 (m, 3H), 2.19 - 2.09 (m, 1H). LCMS (ESI) calcd. for $C_{25}H_{25}Cl_2N_4O_2S^+$ $[M+H]^+$: 515.11, found, 515.0.

**Example 427: Preparation of 3-(4-(((1R,4R)-5-(2,3-dichlorophenyl)-2,5-diazabicyclo[2.2.1]heptan-2-yl)methyl)-1-thioxoisoindolin-2-yl)piperidine-2,6-dione (GT-08467)**

With reference to the method of Scheme 4, the target compound GT-08467 was obtained (13 mg, white solid, yield 22%). $^1H$ NMR (400 MHz, DMSO-d6) δ 11.20 (s, 1H), 10.64 (s, 1H), 8.04 (d, *J* = 7.5 Hz, 1H), 7.98 (d, *J* = 7.3 Hz, 1H), 7.74 - 7.62 (m, 1H), 7.18 (td, *J* = 8.1, 3.9 Hz, 1H), 7.14 - 7.06 (m, 1H), 7.01 (dd, *J* = 7.6, 5.0 Hz, 1H), 6.01 (d, *J* = 9.1 Hz, 1H), 5.31 - 5.14 (m, 1H), 4.90 (dd, *J* = 20.3, 6.5 Hz, 1H), 4.65 (dd, *J* = 13.3, 5.0 Hz, 1H), 4.55 (s, 1H), 4.51 - 4.38 (m, 2H), 3.96 - 3.87 (m, 1H), 3.87 - 3.61 (m, 2H), 3.05 - 2.92 (m, 1H), 2.71 - 2.60 (m, 2H), 2.45 - 2.34 (m, 1H), 2.22 - 2.08 (m, 2H). LCMS (ESI) calcd. for $C_{25}H_{25}Cl_2N_4O_2S^+$ $[M+H]^+$: 515.11, found, 515.0.

**Example 428: Preparation of 3-(4-(((1S,4S)-5-(2,3-dichlorophenyl)-2,5-diazabicyclo[2.2.1]heptan-2-yl)methyl)-1-thioxoisoindolin-2-yl)piperidine-2,6-dione (GT-08468)**

With reference to the method of Scheme 4, the target compound GT-08468 was obtained (12 mg, white solid, yield 20%). $^1H$ NMR (400 MHz, DMSO-d6) δ 11.20 (s, 1H), 10.49 (s, 1H), 8.02 (d, *J* = 7.7 Hz, 1H), 7.98 (d, *J* = 7.6 Hz, 1H), 7.67 (t, *J* = 6.7 Hz, 1H), 7.18 (dd, *J* = 8.1, 3.0 Hz, 1H), 7.11 (d, *J* = 8.3 Hz, 1H), 7.05 - 6.97 (m, 1H), 6.01 (d, *J* = 14.3 Hz, 1H), 5.20 (dd, *J* = 20.2, 14.6 Hz, 1H), 4.90 (dd, *J* = 20.3, 5.2 Hz, 1H), 4.65 (dd, *J* = 13.6, 4.9 Hz, 1H), 4.56 (s, 1H), 4.51 - 4.38 (m, 2H), 3.91 (d, *J* = 10.9 Hz, 1H), 3.87 - 3.69 (m, 2H), 3.28 - 3.17 (m, 1H), 3.08 - 2.93 (m, 1H), 2.69 (d, *J* = 15.6 Hz, 1H), 2.61 (d, *J* = 11.5 Hz, 1H), 2.42 - 2.37 (m, 1H), 2.19 - 2.05 (m, 2H). LCMS (ESI) calcd. for $C_{25}H_{25}Cl_2N_4O_2S^+$ $[M+H]^+$: 515.11, found, 515.0.

**Example 429: Preparation of 3-(4-((5-(2,3-dichlorophenyl)-2,5-diazabicyclo[2.2.2]octan-2-yl)methyl)-1-thioxoisoindolin-2-yl)piperidine-2,6-dione (GT-08469)**

With reference to the method of Scheme 4, the target compound GT-08469 was obtained (15 mg, white solid, yield 24%). $^1$H NMR (400 MHz, DMSO-d6) δ 11.21 (s, 1H), 11.04 (s, 1H), 8.14 - 8.02 (m, 1H), 7.99 (d, *J* = 7.6 Hz, 1H), 7.72 - 7.64 (m, 1H), 7.37 (t, *J* = 6.3 Hz, 1H), 7.35 - 7.30 (m, 1H), 7.30 - 7.25 (m, 1H), 6.02 (d, *J* = 12.1 Hz, 1H), 4.96 - 4.91 (m, 1H), 4.73 - 4.56 (m, 2H), 4.03 - 3.86 (m, 1H), 3.82 (s, 2H), 3.63 (t, *J* = 9.6 Hz, 1H), 3.58 - 3.50 (m, 1H), 3.07 - 2.94 (m, 1H), 2.69 (d, *J* = 17.4 Hz, 1H), 2.45 - 2.31 (m, 2H), 2.21 - 2.05 (m, 3H), 2.04 - 1.78 (m, 3H). LCMS (ESI) calcd. for $C_{26}H_{27}Cl_2N_4O_2S^+$ $[M+H]^+$: 529.12, found, 529.1.

**Example 430: Preparation of 3-(4-((8-(2,3-dichlorophenyl)-3,8-diazabicyclo[3.2.1]octan-3-yl)methyl)-1-thioxoisoindolin-2-yl)piperidine-2,6-dione (GT-08470)**

With reference to the method of Scheme 4, the target compound GT-08470 was obtained (18 mg, white solid, yield 29%). $^1$H NMR (400 MHz, DMSO-d6) δ 11.20 (s, 1H), 10.53 (s, 1H), 8.20 (d, *J* = 7.1 Hz, 1H), 7.99 (d, *J* = 7.3 Hz, 1H), 7.69 (t, *J* = 7.5 Hz, 1H), 7.26 (brs, 2H), 7.06 (bs, 1H), 6.01 (d, *J* = 8.1 Hz, 1H), 5.18 (d, *J* = 20.4 Hz, 1H), 4.92 (d, *J* = 19.9 Hz, 1H), 4.48 (brs, 1H), 4.17 (s, 2H), 3.94 (brs, 1H), 3.57 - 3.44 (m, 2H), 3.03 - 2.95 (m, 1H), 2.74 - 2.56 (m, 2H), 2.28 - 2.21 (m, 1H), 2.15 - 2.11 (m, 1H), 2.07 - 1.94 (m, 2H), 1.88 - 1.68 (m, 1H). LCMS (ESI) calcd. for $C_{26}H_{27}Cl_2N_4O_2S^+$ $[M+H]^+$: 529.12, found, 529.1.

**Example 431: Preparation of 3-(4-((3-(2,3-dichlorophenyl)-3,8-diazabicyclo[3.2.1]octan-8-yl)methyl)-1-thioxoisoindolin-2-yl)piperidine-2,6-dione (GT-08471)**

With reference to the method of Scheme 4, the target compound GT-08471 was obtained (12 mg, white solid, yield 19%). $^1$H NMR (400 MHz, DMSO-d6) δ 11.21 (s, 1H), 11.01 (s, 1H), 8.06 (d, *J* = 7.6 Hz, 1H), 8.01 (d, *J* = 7.6 Hz, 1H), 7.69 (t, *J* = 7.7 Hz, 1H), 7.40 - 7.33 (m, 2H), 7.17 (d, *J* = 6.3 Hz, 1H), 6.02 (d, *J* = 8.4 Hz, 1H), 5.40 (d, *J* = 20.4 Hz, 1H), 4.96 (d, *J* = 20.2 Hz, 1H), 4.44 - 4.33 (m, 2H), 4.16 (d, *J* = 26.4 Hz, 2H), 3.69 (dd, *J* = 16.8, 12.5 Hz, 2H), 3.21 (d, *J* = 12.0 Hz, 3H), 3.09 - 2.93 (m, 1H), 2.69 (d,*J* = 17.9 Hz, 1H), 2.44 - 2.26 (m, 5H), 2.15 - 2.09 (m, 1H). LCMS (ESI) calcd. for $C_{26}H_{27}Cl_2N_4O_2S^+$ $[M+H]^+$: 529.12, found, 529.1.

**Example 432: Preparation of 3-(4-((4-(2,3-dichlorophenyl)-1,4-diazepan-1-yl)methyl)-1-thioxoisoindolin-2-yl)piperidine-2,6-dione (GT-08472)**

With reference to the method of Scheme 4, the target compound GT-08472 was obtained (15 mg, white solid, yield 25%). $^1$H NMR (400 MHz, DMSO-d6) δ 11.21 (s, 1H), 11.00 (s, 1H), 8.05 (d, *J* = 7.6 Hz, 1H), 8.00 (d, *J* = 7.7 Hz, 1H), 7.69 (t, *J* = 7.8 Hz, 1H), 7.34 - 7.27 (m, 2H), 7.23 (dd, *J* = 9.1, 4.7 Hz, 1H), 6.02 (d, *J* = 10.6 Hz, 1H), 5.26 (dd, *J* = 20.4, 6.1 Hz, 1H), 4.90 (d, *J* = 20.3 Hz, 1H), 4.53 (d, *J* = 4.5 Hz, 2H), 3.66 - 3.56 (m, 2H), 3.53 - 3.40 (m, 4H), 3.29 - 3.19 (m, 2H), 3.06 - 2.94 (m, 1H), 2.69 (d, *J* = 16.8 Hz, 1H), 2.45 - 2.38 (m, 1H), 2.34 - 2.11 (m, 3H). LCMS (ESI) calcd. for $C_{25}H_{27}Cl_2N_4O_2S^+$ $[M+H]^+$: 517.11, found, 517.1.

**Example 433: Preparation of 3-(4-((4-(benzo[d]isoxazol-3-yl)piperidin-1-yl)methyl)-1-thioxoisoindolin-2-yl)piperidine-2,6-dione (GT-08816)**

With reference to the method of Scheme 4, the target compound GT-08816 was obtained (6 mg, white solid, yield 11%). $^1$H NMR (400 MHz, DMSO-d6) δ 11.12 (s, 1H), 10.95 (s, 1H), 8.16 (d, *J* = 7.9 Hz, 1H), 7.80 (d, *J* = 7.6 Hz, 1H), 7.73 (t, *J* = 9.2 Hz, 2H), 7.61 (d, *J* = 7.1 Hz, 1H), 7.53 (t, *J* = 7.5 Hz, 1H), 7.40 (t, *J* = 7.5 Hz, 1H), 6.11 - 5.93 (m, 2H), 5.35 (d, *J* = 20.0 Hz, 1H), 4.85 (d, *J* = 20.2 Hz, 1H), 4.73 (d, *J* = 20.3 Hz, 1H), 4.57 - 4.39 (m, 2H), 3.56 - 3.44 (m, 2H), 3.03 - 2.91 (m, 2H), 2.75 - 2.62 (m, 3H), 2.41 - 2.36 (m, 1H), 2.16 - 2.04 (m, 2H). LCMS (ESI) calcd. for $C_{26}H_{27}N_4O_3S^+$ $[M+H]^+$: 475.18, found, 475.1.

**Example 434: Preparation of 3-(4-((4-(isoxazol-3-yl)piperidin-1-yl)methyl)-1-thioxoisoindolin-2-yl)piperidine-2,6-dione (GT-08817)**

With reference to the method of Scheme 4, the target compound GT-08817 was obtained (6 mg, white solid, yield 12%). $^1$H NMR (400 MHz, DMSO-d6) δ 11.21 (s, 1H), 10.79 (s, 1H), 8.85 (s, 1H), 7.99 (t, *J* = 8.9 Hz, 2H), 7.69 (t, *J* = 7.7 Hz, 1H), 6.54 (d, *J* = 1.5 Hz, 1H), 6.02 (d, *J* = 9.4 Hz, 1H), 5.28 (d, *J* = 19.2 Hz, 1H), 4.91 (d, *J* = 20.2 Hz, 1H), 4.54 - 4.32 (m, 2H), 3.49 (dd, *J* = 27.4, 12.0 Hz, 2H), 3.23 - 3.15 (m, 2H), 3.08 - 2.93 (m, 2H), 2.69 (d, *J* = 17.9 Hz, 1H), 2.45 - 2.31 (m, 1H), 2.23 - 1.96 (m, 5H). LCMS (ESI) calcd. for $C_{22}H_{25}N_4O_3S^+$ $[M+H]^+$: 425.16, found, 425.1.

**Example 435: Preparation of 3-(4-((4-(6-fluorobenzo[d]isoxazol-3-yl)piperazin-1-yl)methyl)-1-thioxoisoindolin-2-yl)piperidine-2,6-dione (GT-08818)**

With reference to the method of Scheme 4, the target compound GT-08818 was obtained (6 mg, white solid, yield 10%). $^1$H NMR (400 MHz, DMSO-d6) δ 11.33 (s, 1H), 11.21 (s, 1H), 8.13 - 7.92 (m, 3H), 7.71 (dd, *J* = 21.9, 6.5 Hz, 1H), 7.61 (d, *J* = 8.7 Hz, 1H), 7.26 (t, *J* = 11.3 Hz, 1H), 6.02 (d, *J* = 13.3 Hz, 1H), 5.34 (d, *J* = 19.2 Hz, 1H), 4.92 (d, *J* = 20.8 Hz, 1H), 4.63 - 4.42 (m, 2H), 4.22 - 4.02 (m, 2H), 3.62 - 3.49 (m, 4H), 3.27 - 3.18 (m, 2H), 3.03 - 2.95 (m, 1H), 2.69 (d, *J* = 16.3 Hz, 1H), 2.44 - 2.36 (m, 1H), 2.21 - 2.06 (m, 1H). LCMS (ESI) calcd. for $C_{25}H_{25}FN_5O_3S^+$ [M+H]$^+$: 494.17, found, 494.1.

**Example 436: Preparation of 3-(4-((4-(isoxazolo[4,5-c]pyridin-3-yl)piperazin-1-yl)methyl)-1-thioxoisoindolin-2-yl)piperidine-2,6-dione (GT-08838)**

With reference to the method of Scheme 4, the target compound GT-08838 was obtained (18 mg, white solid, yield 32%). $^1$H NMR (400 MHz, DMSO-d6) δ 12.05 (s, 1H), 11.21 (s, 1H), 9.66 (s, 1H), 8.82 (d, *J* = 6.4 Hz, 1H), 8.02 (dd, *J* = 12.1, 7.6 Hz, 3H), 7.69 (t, *J* = 7.7 Hz, 1H), 6.02 (d, *J* = 8.9 Hz, 1H), 5.51 (d, *J* = 20.3 Hz, 1H), 4.94 (d, *J* = 20.3 Hz, 1H), 4.73 - 4.45 (m, 3H), 3.84 - 3.64 (m, 3H), 3.47 - 3.36 (m, 4H), 3.07 - 2.94 (m, 1H), 2.69 (d, *J* = 16.7 Hz, 1H), 2.48 - 2.38 (m, 1H), 2.20 - 2.06 (m, 1H). LCMS (ESI) calcd. for $C_{24}H_{25}N_6O_3S^+$ [M+H]$^+$: 477.17, found, 477.1.

**Example 437: Preparation of 3-(4-((4-(6-fluorobenzo[d]isothiazol-3-yl)piperazin-1-yl)methyl)-1-thioxoisoindolin-2-yl)piperidine-2,6-dione (GT-08839)**

With reference to the method of Scheme 4, the target compound GT-08839 was obtained (16 mg, white solid, yield 27%). $^1$H NMR (400 MHz, DMSO-d6) δ 11.26 (s, 1H), 11.21 (s, 1H), 8.19 - 8.16 (m, 1H), 8.02 - 7.95 (m, 3H), 7.70 (t, *J* = 7.1 Hz, 2H), 7.36 (dd, *J* = 8.7, 6.6 Hz, 1H), 6.02 (dd, *J* = 16.0, 4.8 Hz, 1H), 5.36 (d, *J* = 20.4 Hz, 1H), 4.94 (d, *J* = 20.7 Hz, 1H), 4.62 - 4.44 (m, 2H), 4.06 (d, *J* = 12.4 Hz, 2H), 3.63 - 3.52 (m, 7H), 3.03 - 2.94 (m, 1H), 2.67 (d, *J* = 1.3 Hz, 2H), 2.45 - 2.34 (m, 1H), 2.20 - 2.09 (m, 1H). LCMS (ESI) calcd. for $C_{25}H_{25}FN_5O_3S_2^+$ [M+H]$^+$: 510.14, found, 510.1.

**Example 438: Preparation of 3-(4-((4-(6-fluoro-1H-indazol-3-yl)piperazin-1-yl)methyl)-1-thioxoisoindolin-2-yl) piperidine-2,6-dione (GT-08840)**

With reference to the method of Scheme 4, the target compound GT-08840 was obtained (17 mg, white solid, yield 29%). $^1$H NMR (400 MHz, DMSO-d6) δ 11.21 (s, 1H), 11.12 (s, 1H), 8.00 (t, *J*= 6.9 Hz, 2H), 7.83 (dd, *J* = 9.0, 5.4 Hz, 1H), 7.70 (t, *J* = 7.6 Hz, 1H), 7.16 (dd, *J* = 9.7, 2.1 Hz, 1H), 6.89 (td, *J* = 9.1, 2.2 Hz, 1H), 6.02 (d, *J* = 9.2 Hz, 1H), 5.33 (d, *J* = 20.7 Hz, 1H), 4.94 (d, *J* = 20.5 Hz, 1H), 4.57 - 4.44 (m, 2H), 3.95 (d, *J* = 10.5 Hz, 2H), 3.41 - 3.25 (m, 6H), 3.07 - 2.93 (m, 1H), 2.69 (d, *J* = 18.8 Hz, 1H), 2.46 - 2.37 (m, 1H), 2.17 - 2.06 (m, 1H). LCMS (ESI) calcd. for $C_{25}H_{26}FN_6O_2S^+$ [M+H]$^+$: 493.18, found, 493.1.

**Example 439: Preparation of 3-(4-((4-(1H-pyrrolo[2,3-b]pyridin-3-yl)piperazin-1-yl)methyl)-1-thioxoisoindolin-2-yl)piperidine-2,6-dione (GT-08841)**

With reference to the method of Scheme 4, the target compound GT-08841 was obtained (12 mg, white solid, yield 22%). $^1$H NMR (400 MHz, DMSO-d6) δ 11.46 (s, 1H), 11.21 (s, 1H), 8.29 (t, *J* = 7.0 Hz, 1H), 8.14 - 7.85 (m, 3H), 7.69 (t, *J* = *7.6* Hz, 1H), 7.20 - 7.12 (m, 1H), 6.02 (d, *J* = 5.3 Hz, 1H), 5.42 (d, *J* = 20.5 Hz, 1H), 4.95 (d, *J* = 20.4 Hz, 1H), 4.60 - 4.43 (m, 2H), 3.42 - 3.35 (m, 5H), 3.31 - 3.12 (m, 3H), 3.04 - 2.95 (m, 1H), 2.69 (d, *J* = 17.1 Hz, 1H), 2.44 - 2.33 (m, 1H), 2.23 - 2.07 (m, 1H). LCMS (ESI) calcd. for $C_{25}H_{27}N_6O_2S^+$ [M+H]$^+$: 475.19, found, 475.1.

**Example 440: Preparation of 3-(4-((4-(6-fluoro-1H-indol-3-yl)piperidin-1-yl)methyl)-1-thioxoisoindolin-2-yl)piperidine-2,6-dione (GT-08492)**

With reference to the method of Scheme 4, the target compound GT-08492 was obtained (17 mg, white solid, yield 30%). $^1$H NMR (400 MHz, DMSO-d6) δ 11.22 (s, 1H), 10.51 (s, 1H), 8.02 - 7.97 (m, 2H), 7.72 - 7.67 (m, 1H), 7.53 (t, *J* = 7.6 Hz, 1H), 7.16 - 7.08 (m, 2H), 6.88 - 6.78 (m, 1H), 6.10 - 5.89 (m, 1H), 5.26 (d, *J* = 20.2 Hz, 1H), 4.93 (d, *J* = 20.2 Hz, 1H), 4.54 - 4.37 (m, 1H), 3.59 - 3.45 (m, 2H), 3.25 - 3.18 (m, 2H), 3.03 - 2.95 (m, 1H), 2.73 - 2.62 (m, 1H), 2.57 - 2.51 (m, 2H), 2.15 - 2.04 (m, 4H), 1.35 - 1.17(m, 2H). LCMS (ESI) calcd. for $C_{27}H_{28}FN_4O_2S^+$ [M+H]$^+$: 491.19, found, 491.1.

**Example 441: Preparation of 3-(4-((4-(6-fluoro-1-methyl-1H-indol-3-yl)piperidin-1-yl)methyl)-1-thioxoisoindolin-2-yl)piperidine-2,6-dione (GT-08844)**

With reference to the method of Scheme 4, the target compound GT-08844 was obtained (9 mg, white solid, yield 15%). $^1$H NMR (400 MHz, DMSO-d6) δ 11.22 (s, 1H), 10.74 (s, 1H), 8.00 (t, *J* = 6.3 Hz, 2H), 7.70 (t, *J* = 7.3 Hz, 2H), 7.26 (d, *J* = 10.3 Hz, 1H), 7.11 (s, 1H), 6.86 (t, *J* = 9.2 Hz, 1H), 6.02 (d, *J* = 10.1 Hz, 1H), 5.31 - 5.25 (m, 1H), 4.93 (d, *J* = 20.2 Hz, 1H), 4.52 - 4.39 (m, 2H), 3.69 (s, 3H), 3.55 - 3.45 (m, 2H), 3.25 - 3.18 (m, 2H), 3.08 - 2.95 (m, 2H), 2.70 (d, *J* = 21.2 Hz, 1H), 2.47 - 2.41 (m, 1H), 2.19 - 2.06 (m, 5H). LCMS (ESI) calcd. for $C_{28}H_{30}FN_4O_2S^+$ [M+H]$^+$: 505.21, found, 505.2.

**Example 442: Preparation of 3-(4-((4-(6-fluoro-1H-indazol-3-yl)piperidin-1-yl)methyl)-1-thioxoisoindolin-2-yl)piperidine-2,6-dione (GT-08849)**

With reference to the method of Scheme 4, the target compound GT-08849 was obtained (12 mg, white solid, yield 21%). $^1$H NMR (400 MHz, DMSO-d6) δ 12.84 (s, 1H), 11.22 (s, 1H), 8.02 - 7.94 (m, 2H), 7.70 (t, *J* = 7.6 Hz, 1H), 7.53 (t, *J* = 7.5 Hz, 1H), 7.25 (dd, *J* = 9.7, 2.0 Hz, 1H), 7.02 - 6.93 (m, 1H), 6.11 - 5.97 (m, 1H), 5.32 (d, *J* = 20.0 Hz, 1H), 4.86 (d, *J* = 20.3 Hz, 1H), 4.78 - 4.68 (m, 1H), 4.55 - 4.39 (m, 2H), 3.59 - 3.48 (m, 2H), 3.26 - 3.17 (m, 2H), 3.01 - 2.92 (m, 1H), 2.70 (d, *J* = 21.8 Hz, 1H), 2.38 - 2.29 (m, 2H), 2.19 - 2.06 (m, 4H). LCMS (ESI) calcd. for $C_{26}H_{27}FN_5O_2S^+$ [M+H]$^+$: 492.19, found, 492.2.

**Example 443: Preparation of 3-(4-((4-(5-fluoroindolin-1-yl)piperidin-1-yl)methyl)-1-thioxoisoindolin-2-yl)piperidine-2,6-dione (GT-08845)**

With reference to the method of Scheme 4, the target compound GT-08845 was obtained (7 mg, white solid, yield 12%). $^1$H NMR (400 MHz, DMSO-d6) δ 11.20 (s, 2H), 11.00 (s, 2H), 7.99 (dd, *J* = 7.5, 3.5 Hz, 1H), 7.69 (dd, *J* = 16.3, 8.4 Hz, 2H), 6.91 (d, *J* = *9.1* Hz, 1H), 6.81 (t, *J* = 9.0 Hz, 1H), 6.51 - 6.47 (m, 1H), 6.00 (d, *J* = 9.5 Hz, 1H), 5.31 (d, *J* = 20.3 Hz, 1H), 4.90 (d, *J* = 20.4 Hz, 1H), 4.47 - 4.36 (m, 2H), 3.31 - 3.27 (m, 4H), 3.21 - 3.15 (m, 2H), 2.69 (d, *J* = 18.6 Hz, 1H), 2.41 - 2.33 (m, 1H), 2.15 - 2.08 (m, 4H), 1.82 (d, *J* = 13.2 Hz, 2H), 1.35 - 1.21 (m, 2H). LCMS (ESI) calcd. for $C_{27}H_{30}FN_4O_2S^+$ [M+H]$^+$: 493.21, found, 493.2.

**Example 444: Preparation of 3-(4-((4-(5-fluoro-1H-benzo[d]imidazol-1-yl)piperidin-1-yl)methyl)-1-thioxoisoindolin-2-yl)piperidine-2,6-dione (GT-08842)**

With reference to the method of Scheme 4, the target compound GT-08842 was obtained (12 mg, white solid, yield 21%). $^1$H NMR (400 MHz, DMSO-d6) δ 11.73 (s, 1H), 11.22 (s, 1H), 9.14 (s, 1H), 8.29 (s, 1H), 8.03 (dd, *J* = 12.1, 7.6 Hz, 2H), 7.67 (dd, *J* = 13.4, 8.3 Hz, 2H), 7.38 (t, *J* = 8.5 Hz, 1H), 6.03 (d, *J* = 8.0 Hz, 1H), 5.48 (d, *J* = 20.5 Hz, 1H), 4.94 (d, *J* = 20.1 Hz, 2H), 4.61 - 4.43 (m, 2H), 3.42 - 3.30 (m, 6H), 3.04 - 2.95 (m, 1H), 2.74 - 2.67 (m, 1H), 2.37 - 2.23 (m, 3H), 2.20 - 2.12 (m, 1H). LCMS (ESI) calcd. for $C_{26}H_{27}FN_5O_2S^+$ [M+H]$^+$: 492.19, found, 492.1.

**Example 445: Preparation of 3-(4-((4-(5-fluoro-2-oxo-2,3-dihydro-1H-benzo[d]imidazol-1-yl)piperidin-1-yl)methyl)-1-thioxoisoindolin-2-yl)piperidine-2,6-dione (GT-08843)**

With reference to the method of Scheme 4, the target compound GT-08843 was obtained (17 mg, white solid, yield 29%). $^1$H NMR (400 MHz, DMSO-d6) δ 11.22 (s, 1H), 11.11 (s, 1H), 11.08 (s, 1H), 7.99 (dd, *J* = 12.8, 7.7 Hz, 2H), 7.70 (t, *J* = 7.6 Hz, 1H), 7.58 (dd, *J* = 8.6, 4.5 Hz, 1H), 6.92 - 6.72 (m, 2H), 6.03 (d, *J* = 8.9 Hz, 1H), 5.37 (d, *J* = 20.2 Hz, 1H), 4.92 (d, *J* = 20.0 Hz, 1H), 4.51 (dd, *J* = 24.2, 9.6 Hz, 2H), 4.40 (dd, *J* = 13.3, 5.2 Hz, 1H), 3.59 (d, *J* = 11.5 Hz, 1H), 3.49 (d, *J* = 11.3 Hz, 1H), 3.30 - 3.19 (m, 2H), 3.08 - 2.95 (m, 1H), 2.92 - 2.77 (m, 2H), 2.70 (d, *J* = 19.2 Hz, 1H), 2.48 - 2.38 (m, 1H), 2.21 - 2.08 (m, 1H), 1.87 (d, *J* = 12.2 Hz, 2H). LCMS (ESI) calcd. for $C_{26}H_{27}FN_5O_3S^+$ [M+H]$^+$: 508.18, found, 508.1.

**Example 446: Preparation of 3-(4-((4-(1H-pyrrolo[2,3-b]pyridin-3-yl)piperidin-1-yl)methyl)-1-thioxoisoindolin-2-yl)piperidine-2,6-dione (GT-08848)**

With reference to the method of Scheme 4, the target compound GT-08848 was obtained (5 mg, white solid, yield 9%). $^1$H NMR (400 MHz, DMSO-d6) δ 11.70 (s, 2H), 11.20 (s, 1H), 8.30 (d, *J* = 7.7 Hz, 1H), 8.25 (d, *J* = 4.2 Hz, 1H), 8.04 - 7.98 (m, 2H), 7.75 - 7.70 (m, 1H), 7.32 (s, 1H), 7.12 (dd, *J* = 7.6, 4.4 Hz, 1H), 6.03 (d, *J* = 7.9 Hz, 1H), 5.31 (d, *J* = 20.4 Hz, 1H), 4.93 (d, *J*= 20.5 Hz, 1H), 4.59 - 4.44 (m, 3H), 3.27 - 3.23 (m, 4H), 2.71 (d, *J* = 14.4 Hz, 1H), 2.39 (d, *J* = 7.8 Hz, 1H), 2.27 - 2.15 (m, 6H). LCMS (ESI) calcd. for $C_{26}H_{28}N_5O_2S^+$ [M+H]$^+$: 474.20, found, 474.2.

**Example 447: Preparation of 3-(4-((4-(1H-pyrrolo[2,3-b]pyridin-3-yl)-3,6-dihydropyridin-1(2H)-yl)methyl)-1-thioxoisoindolin-2-yl)piperidine-2,6-dione (GT-08494)**

With reference to the method of Scheme 4, the target compound GT-08494 was obtained (6 mg, white solid, yield 11%). $^1$H NMR (400 MHz, DMSO-d6) δ 12.03 (s, 1H), 11.20 (s, 1H), 8.38 - 8.24 (m, 2H), 8.07 - 7.98 (m, 1H), 7.83 - 7.63 (m, 3H), 7.24 - 7.14 (m, 1H), 6.19 s, 1H), 6.03 (d, *J* = 11.1 Hz, 1H), 4.95 (dd, *J* = 20.0, 7.3 Hz, 1H), 4.76 - 4.51 (m, 2H), 4.02 - 3.88 (m, 1H), 3.39 - 3.27 (m, 3H), 3.10 - 3.05 (m, 1H), 3.02 - 2.87 (m, 2H), 2.72 - 2.61 (m, 2H), 2.48 - 2.39 (m, 1H), 2.20 - 2.05 (m, 1H). LCMS (ESI) calcd. for $C_{26}H_{26}N_5O_2S^+$ [M+H]$^+$: 472.18, found, 472.1.

**Example 448: Preparation of 3-(4-((4-(6-fluoro-1-methyl-1H-indol-3-yl)-3,6-dihydropyridin-1(2H)-yl)methyl)-1-thioxoisoindolin-2-yl)piperidine-2,6-dione (GT-08493)**

With reference to the method of Scheme 4, the target compound GT-08493 was obtained (17 mg, white solid, yield 29%). LCMS (ESI) calcd. for $C_{28}H_{28}FN_4O_2S^+$ [M+H]$^+$: 503.19, found, 503.1.

**Example 449: Preparation of 3-(4-((4-(6-fluoro-1H-indazol-3-yl)-3,6-dihydropyridin-1(2H)-yl)methyl)-1-thioxoisoindolin-2-yl)piperidine-2,6-dione (GT-08850)**

With reference to the method of Scheme 4, the target compound GT-08850 was obtained (14 mg, white solid, yield 24%). $^1$H NMR (400 MHz, DMSO-d6) δ 11.12 (s, 1H), 10.68 (s, 1H), 8.09 - 7.99 (m, 2H), 7.72 (t, *J* = 7.6 Hz, 1H), 7.53 (t, *J* = 7.6 Hz, 1H), 7.35 (d, *J* = 9.1 Hz, 1H), 7.09 (dt, *J* = 15.6, 8.1 Hz, 1H), 6.57 (s, 1H), 6.07 - 5.95 (m, 1H), 5.17 (d, *J* = 21.5 Hz, 1H), 4.96 (dd, *J* = 18.3, 8.0 Hz, 1H), 4.85 (d, *J* = 20.2 Hz, 1H), 4.73 (d, *J* = 20.3 Hz, 1H), 4.61 - 4.55 (m, 2H), 4.04 - 3.91 (m, 2H), 3.79 - 3.64 (m, 1H), 3.03 - 2.94 (m, 2H), 2.64 (d, *J* = 20.8 Hz, 1H), 2.20 - 2.07 (m, 2H). LCMS (ESI) calcd. for $C_{26}H_{25}FN_5O_2S^+$ [M+H]$^+$: 490.17, found, 490.2.

**Example 450: Preparation of 3-(4-((4-(6-fluoro-3,4-dihydroquinolin-1(2H)-yl)piperidin-1-yl)methyl)-1-thioxoisoindolin-2-yl)piperidine-2,6-dione (GT-08851)**

With reference to the method of Scheme 4, the target compound GT-08851 was obtained (7 mg, white solid, yield 12%). $^1$H NMR (400 MHz, DMSO-d6) δ 11.20 (s, 1H), 10.96 (s, 1H), 7.99 (t, *J* = 8.3 Hz, 2H), 7.68 (t, *J* = 7.6 Hz, 1H), 6.87 - 6.74 (m, 3H), 6.00 (d, *J* = 17.3 Hz, 1H), 5.29 (d, *J* = 20.3 Hz, 1H), 4.90 (d, *J* = 20.3 Hz, 1H), 4.41 - 4.33 (m, 2H), 3.95 - 3.86 (m, 1H), 3.46 - 3.31 (m, 2H), 3.27 - 3.20 (m, 2H), 3.07 - 2.96 (m, 2H), 2.65 - 2.58 (m, 3H), 2.28 - 2.11 (m, 4H), 1.84 - 1.72 (m, 6H). LCMS (ESI) calcd. for $C_{28}H_{32}FN_4O_2S^+$ [M+H]$^+$: 507.22, found, 507.2.

**Example 451: Preparation of 3-(4-((4-(7-fluoro-2,3-dihydro-4H-benzo[b][1,4]oxazin-4-yl)piperidin-1-yl)methyl)-1-thioxoisoindolin-2-yl)piperidine-2,6-dione (GT-08852)**

With reference to the method of Scheme 4, the target compound GT-08852 was obtained (9 mg, white solid, yield 15%). $^1$H NMR (400 MHz, DMSO-d6) δ 11.20 (s, 1H), 10.98 (s, 1H), 8.04 - 7.96 (m, 2H), 7.68 (t, *J* = 7.7 Hz, 1H), 6.84 - 2.80 (m, 1H), 6.64 - 6.57 (m, 2H), 6.01 (d, *J* = 10.3 Hz, 1H), 5.29 (d, *J* = 20.3 Hz, 1H), 4.90 (d, *J* = 20.3 Hz, 1H), 4.47 - 4.36 (m, 2H), 4.23 - 4.10 (m, 2H), 3.93 - 3.83 (m, 1H), 3.57 - 3.51 (m, 2H), 3.27 - 3.11 (m, 6H), 2.69 (d, *J* = 17.1 Hz, 1H), 2.26 - 2.08 (m, 4H), 1.81 - 1.78 (m, 2H). LCMS (ESI) calcd. for $C_{27}H_{30}FN_4O_3S^+$ [M+H]$^+$: 509.20, found, 509.2.

**Example 452: Preparation of 3-(4-((4-(7-fluoro-3-oxo-2,3-dihydro-4H-benzo[b][1,4]oxazin-4-yl)piperidin-1-yl)methyl)-1-thioxoisoindolin-2-yl)piperidine-2,6-dione (GT-08853)**

With reference to the method of Scheme 4, the target compound GT-08853 was obtained (11 mg, white solid, yield 18%). $^1$H NMR (400 MHz, DMSO-d6) δ 11.12 (s, 1H), 10.46 (s, 1H), 8.01 (d, *J* = 7.4 Hz, 1H), 7.80 (d, *J* = 7.4 Hz, 1H), 7.61 (d, *J* = 7.4 Hz, 1H), 7.54 (q, *J* = 7.6 Hz, 2H), 7.00 (dd, *J* = 9.1, 2.7 Hz, 1H), 6.11 - 5.91 (m, 2H), 5.45 - 5.26 (m, 1H), 4.85 (d, *J* = 20.5 Hz, 1H), 4.73 (d, *J* = 20.4 Hz, 1H), 4.59 (s, 2H), 4.45 - 4.37 (m, 2H), 3.60 - 3.44 (m, 2H), 3.04 - 2.92 (m, 3H), 2.68 - 2.57 (m, 2H), 2.18 - 2.06 (m, 2H), 1.92 (d, *J* = 10.7 Hz, 2H). LCMS (ESI) calcd. for $C_{27}H_{28}FN_4O_4S^+$ [M+H]$^+$: 523.18, found, 523.2.

**Example 453: Preparation of 3-(4-((4-(7-fluorochroman-4-yl)piperidin-1-yl)methyl)-1-thioxoisoindolin-2-yl)piperidine-2,6-dione (GT-08854)**

With reference to the method of Scheme 4, the target compound GT-08854 was obtained (7 mg, white solid, yield 12%). $^1$H NMR (400 MHz, DMSO-d6) δ 11.19 (s, 1H), 10.46 (s, 1H), 7.99 (d, *J*=7.9 Hz, 1H), 7.92 (d, *J* = 7.5 Hz, 1H), 7.67 (t, *J*

=7.6 Hz, 1H), 7.13 (d, *J* = 9.3 Hz, 1H), 6.68 (t, *J* = 8.5 Hz, 1H), 6.60 (dd, *J* = 10.4, 2.5 Hz, 1H), 6.00 (d, *J* = 7.6 Hz, 1H), 5.16 (d, *J* = 20.6 Hz, 1H), 4.87 (d, *J* = 19.7 Hz, 1H), 4.35 (s, 2H), 4.24 - 4.06 (m, 2H), 3.46 - 3.40 (m, 2H), 3.08 - 2.95 (m, 3H), 2.70 - 2.67 (m, 2H), 2.43 - 2.35 (m, 1H), 2.16 - 2.10 (m, 1H), 2.01 - 1.82 (m, 4H), 1.71 - 1.61 (m, 3H). LCMS (ESI) calcd. for $C_{28}H_{31}FN_3O_3S^+$ $[M+H]^+$: 508.21, found, 508.2.

**Example 454: Preparation of 3-(4-((4-(5-fluoronaphthalen-1-yl)piperidin-1-yl)methyl)-1-thioxoisoindolin-2-yl)piperidine-2,6-dione (GT-08855)**

With reference to the method of Scheme 4, the target compound GT-08855 was obtained (10 mg, white solid, yield 17%). $^1H$ NMR (400 MHz, DMSO) δ 11.22 (s, 1H), 8.14 - 7.96 (m, 4H), 7.71 (t, J = 6.8 Hz, 1H), 7.66 - 7.54 (m, 2H), 7.48 (d, J = 6.9 Hz, 1H), 7.40 - 7.35 (m, 1H), 6.03 (d, J = 9.8 Hz, 1H), 5.28 (d, J = 19.3 Hz, 1H), 4.95 (d, J = 21.1 Hz, 1H), 4.60 - 4.38 (m, 2H), 3.77 - 3.50 (m, 4H), 3.10 - 2.98 (m, 1H), 2.73 - 2.68 (m, 2H), 2.29 - 2.14 (m, 4H), 2.09 - 2.04 (m, 3H). LCMS (ESI) calcd. for $C_{29}H_{29}FN_3O_2S^+$ $[M+H]^+$: 502.20, found, 502.2.

**Example 455: Preparation of 3-(4-((4-(7-fluoroquinolin-4-yl)piperidin-1-yl)methyl)-1-thioxoisoindolin-2-yl)piperidine-2,6-dione (GT-08858)**

With reference to the method of Scheme 4, the target compound GT-08858 was obtained (10 mg, white solid, yield 17%). $^1H$ NMR (400 MHz, DMSO-d6) δ 11.33 (s, 1H), 11.21 (s, 1H), 9.02 (d, *J* = 4.9 Hz, 1H), 8.50 (dd, *J* = 9.4, 6.0 Hz, 1H), 8.09 - 8.00 (m, 2H), 7.94 - 7.88 (m, 1H), 7.74 - 7.65 (m, 2H), 7.52 (t, *J* = 5.3 Hz, 1H), 6.02 (d, *J* = 7.4 Hz, 1H), 5.38 (d, *J* = 20.3 Hz, 1H), 4.95 (d, *J* = 20.1 Hz, 1H), 4.47 (dd, *J* = 18.8, 14.0 Hz, 1H), 3.83 (t, *J* = 13.1 Hz, 1H), 3.38 - 3.32 (m, 5H), 3.04 - 2.97 (m, 1H), 2.70 (d, *J* = 20.9 Hz, 1H), 2.41 - 2.26 (m, 3H), 2.24 - 2.14 (m, 1H), 2.07 (d, *J* = 13.1 Hz, 2H). LCMS (ESI) calcd. for $C_{28}H_{28}FN_4O_2S^+$ $[M+H]^+$: 503.19, found, 503.2.

**Example 456: Preparation of 3-(4-((4-(isoquinolin-5-yl)piperidin-1-yl)methyl)-1-thioxoisoindolin-2-yl)piperidine-2,6-dione (GT-08857)**

With reference to the method of Scheme 4, the target compound GT-08857 was obtained (12 mg, white solid, yield 21%). $^1H$ NMR (400 MHz, DMSO-d6) δ 11.32 (s, 1H), 11.22 (s, 1H), 9.75 (s, 1H), 8.70 (d, *J* = 6.6 Hz, 1H), 8.55 (t, *J* = 8.0 Hz, 1H), 8.33 (dd, *J* = 9.1, 3.3 Hz, 1H), 8.07 - 7.99 (m, 2H), 7.93 (d, *J* = 5.8 Hz, 2H), 7.71 (t, *J* = 7.8 Hz, 1H), 6.03 (d, *J* = 7.3 Hz, 1H), 5.40 (d, *J* = 20.3 Hz, 1H), 4.95 (d, *J* = 20.2 Hz, 1H), 4.65 - 4.44 (m, 2H), 3.85 - 3.80 (m, 2H), 3.68 - 3.62 (m, 5H), 2.70 (d, *J* = 20.5 Hz, 1H), 2.43 - 2.34 (m, 2H), 2.18 - 2.13 (m, 1H), 2.05 (d, *J* = 13.0 Hz, 2H). LCMS (ESI) calcd. for $C_{28}H_{29}N_4O_2S^+$ $[M+H]^+$: 485.20, found, 485.2.

**Example 457: Preparation of 3-(4-((4-(5-fluoronaphthalen-1-yl)-3,6-dihydropyridin-1(2H)-yl)methyl)-1-thioxoisoindolin-2-yl)piperidine-2,6-dione (GT-08856)**

With reference to the method of Scheme 4, the target compound GT-08856 was obtained (11 mg, white solid, yield 19%). $^1H$ NMR (400 MHz, DMSO-d6) δ 11.23 (s, 1H), 11.08 (s, 1H), 8.03 (d, *J*= 7.8 Hz, 3H), 7.98 (d, *J* = 7.8 Hz, 1H), 7.73 (d, *J* = 7.3 Hz, 1H), 7.63 (t, *J* = 7.7 Hz, 1H), 7.48 (dd, *J* = 22.4, 7.4 Hz, 2H), 7.42 - 7.29 (m, 1H), 6.04 (d, *J* = 10.5 Hz, 1H), 5.77 (s, 1H), 5.37 - 5.24 (m, 1H), 4.98 (dd, *J* = 20.4, 5.0 Hz, 1H), 4.71 - 4.49 (m, 2H), 4.05 - 3.86 (m, 2H), 3.79 - 3.66 (m, 1H), 3.60 - 3.46 (m, 1H), 3.06 - 2.94 (m, 2H), 2.73 - 2.67 (m, 1H), 2.65 - 2.53 (m, 2H), 2.21 - 2.09 (m, 1H). LCMS (ESI) calcd. for $C_{29}H_{27}FN_3O_2S^+$ $[M+H]^+$: 500.18, found, 500.2.

**Example 458: Preparation of 3-(4-((4-(7-fluoroquinolin-4-yl)-3,6-dihydropyridin-1(2H)-yl)methyl)-1-thioxoisoindolin-2-yl)piperidine-2,6-dione (GT-08633)**

With reference to the method of Scheme 4, the target compound GT-08633 was obtained (16 mg, white solid, yield 27%). $^1H$ NMR (400 MHz, DMSO-d6) δ 11.94 (s, 1H), 11.23 (s, 1H), 9.10 (s, 1H), 8.59 - 8.54 (m, 1H), 8.12 (t, *J* = 8.0 Hz, 1H), 8.07 - 7.92 (m, 2H), 7.75 - 7.61 (m, 3H), 6.04 (d, *J* = 13.4 Hz, 1H), 5.94 (s, 1H), 5.54 (dd, *J* = 40.8, 20.6 Hz, 1H), 4.98 (dd, *J* = 20.3, 8.0 Hz, 1H), 4.77 - 4.53 (m, 2H), 3.80 (d, *J* = 19.0 Hz, 2H), 3.59 - 3.46 (m, 1H), 3.43 - 3.28 (m, 1H), 3.28 - 3.07 (m, 1H), 3.08 - 2.95 (m, 1H), 2.70 (d, *J* = 16.5 Hz, 2H), 2.45 - 2.33 (m, 1H), 2.22 - 2.05 (m, 1H). LCMS (ESI) calcd. for $C_{28}H_{26}FN_4O_2S^+$ $[M+H]^+$: 501.18, found, 501.1.

**Example 459: Preparation of 3-(4-((4-(isoquinolin-5-yl)-3,6-dihydropyridin-1(2H)-yl)methyl)-1-thioxoisoindolin-2-yl)piperidine-2,6-dione (GT-08634)**

With reference to the method of Scheme 4, the target compound GT-08634 was obtained (7 mg, white solid, yield

12%). $^{1}$H NMR (400 MHz, DMSO-d6) δ 11.76 (s, 1H), 11.23 (s, 1H), 9.76 (s, 1H), 8.62 (s, 2H), 8.50 - 8.35 (m, 1H), 8.10 (d, *J* = 7.1 Hz, 1H), 8.03 (d, *J* = 7.7 Hz, 1H), 7.94 (d, *J* = 4.9 Hz, 2H), 7.71 (t, *J* = 7.7 Hz, 1H), 6.05 (d, *J* = 14.3 Hz, 1H), 5.82 (s, 1H), 5.59 - 5.44 (m, 1H), 4.98 (dd, *J* = 20.2, 7.3 Hz, 1H), 4.75 - 4.48 (m, 2H), 3.96 (brs, 2H), 3.22 - 3.08 (m, 1H), 3.06 - 2.96 (m, 2H), 2.72 - 2.62 (m, 3H), 2.49 - 2.39 (m, 1H), 2.19 - 2.11 (m, 1H). LCMS (ESI) calcd. for $C_{28}H_{27}N_4O_2S^+$ [M+H]$^+$: 483.18, found, 483.1.

**Example 460: Preparation of 3-(4-((3-(7-fluorochroman-4-yl)azetidin-1-yl)methyl)-1-thioxoisoindolin-2-yl)piperidine-2,6-dione (GT-08859)**

With reference to the method of Scheme 4, the target compound GT-08859 was obtained (3 mg, white solid, yield 6%). $^{1}$H NMR (400 MHz, DMSO-d6) δ 11.21 (s, 1H), 10.72 (s, 1H), 7.97 (d, *J* = 7.3 Hz, 1H), 7.91 - 7.84 (m, 1H), 7.72 - 7.62 (m, 1H), 7.11 - 7.07 (m, 1H), 6.67 - 6.61 (m, 2H), 6.07 - 5.98 (m, 1H), 5.12 (d, *J* = 20.1 Hz, 1H), 4.89 (d, *J* = 21.1 Hz, 1H), 4.56 - 4.44 (m, 2H), 4.20 - 4.05 (m, 3H), 3.46 - 3.38 (m, 2H), 3.24 - 3.13 (m, 2H), 3.03 - 2.90 (m, 2H), 2.71 - 2.67 (m, 1H), 2.18 - 2.08 (m, 1H), 2.00 - 1.86 (m, 2H), 1.73 - 1.64 (m, 1H). LCMS (ESI) calcd. for $C_{26}H_{27}FN_3O_3S^+$ [M+H]$^+$: 480.18, found, 480.2.

**Example 461: Preparation of 3-(4-((3-(7-fluoroquinolin-4-yl)azetidin-1-yl)methyl)-1-thioxoisoindolin-2-yl)piperidine-2,6-dione (GT-08860)**

With reference to the method of Scheme 4, the target compound GT-08860 was obtained (14 mg, white solid, yield 25%). $^{1}$H NMR (400 MHz, DMSO-d6) δ 11.79 (s, 1H), 11.20 (s, 2H), 9.03 (t, *J* = 4.6 Hz, 1H), 8.00 - 7.92 (m, 4H), 7.71 - 7.58 (m, 3H), 6.09 - 5.94 (m, 1H), 5.32 - 5.25 (m, 1H), 4.90 (d, *J* = 19.9 Hz, 1H), 4.82 - 4.62 (m, 4H), 4.60 - 4.46 (m, 4H), 3.03 - 2.94 (m, 1H), 2.69 (d, *J* = 17.6 Hz, 1H), 2.18 - 2.07 (m, 1H). LCMS (ESI) calcd. for $C_{26}H_{24}FN_4O_2S^+$ [M+H]$^+$: 475.16, found, 475.2.

**Example 462: Preparation of 3-(4-((3-(5-fluoroindolin-1-yl)azetidin-1-yl)methyl)-1-thioxoisoindolin-2-yl)piperidine-2,6-dione (GT-08635)**

With reference to the method of Scheme 4, the target compound GT-08635 was obtained (13 mg, white solid, yield 24%). $^{1}$H NMR (400 MHz, DMSO-d6) δ 11.20 (s, 1H), 11.12 (s, 1H), 7.97 (d, *J* = 7.7 Hz, 1H), 7.96 - 7.89 (m, 1H), 7.70 - 7.59 (m, 2H), 6.98 (dd, *J* = 8.4, 1.8 Hz, 1H), 6.84 - 6.79 (m, 1H), 6.08 - 5.93 (m, 1H), 4.85 - 4.75 (m, 2H), 4.59 - 4.51 (m, 1H), 4.42 - 4.34 (m, 2H), 3.49 - 3.42 (m, 4H), 3.06 - 2.87 (m, 4H), 2.71 - 2.62 (m, 1H), 2.46 - 2.37 (m, 1H), 2.17 - 2.02 (m, 2H). LCMS (ESI) calcd. for $C_{25}H_{26}FN_4O_2S^+$ [M+H]$^+$: 465.18, found, 465.1.

**Example 463: Preparation of 3-(4-((3-(5-fluoro-1H-benzo[d]imidazol-1-yl)azetidin-1-yl)methyl)-1-thioxoisoindolin-2-yl)piperidine-2,6-dione (GT-08861)**

With reference to the method of Scheme 4, the target compound GT-08861 was obtained (16 mg, white solid, yield 29%). $^{1}$H NMR (400 MHz, DMSO-d6) δ 11.19 (s, 1H), 8.97 (s, 1H), 7.95 - 7.90 (m, 2H), 7.63 - 7.58 (m, 2H), 7.38 - 7.21 (m, 2H), 6.08 - 5.92 (m, 1H), 5.90 - 5.72 (m, 1H), 5.56 - 5.40 (m, 1H), 4.82 (d, *J* = 20.1 Hz, 1H), 4.34 - 4.20 (m, 4H), 3.94 - 3.88 (m, 2H), 3.05 - 2.94 (m, 1H), 2.69 (d, *J* = 17.0 Hz, 2H), 2.46 - 2.37 (m, 1H), 2.16 - 2.10 (m, 1H). LCMS (ESI) calcd. for $C_{24}H_{23}FN_5O_2S^+$ [M+H]$^+$: 464.16, found, 464.1.

**Example 464: Preparation of 3-(4-((4-(benzo[b]thiophen-4-yl)piperazin-1-yl)methyl)-1-thioxoisoindolin-2-yl)piperidine-2,6-dione (GT-08364)**

With reference to the method of Scheme 4, the target compound GT-08364 was obtained (14 mg, white solid, yield 24%). $^{1}$H NMR (400 MHz, DMSO-d6) δ 11.27 (s, 1H), 11.22 (s, 1H), 8.03 (dd, *J* = 12.3, 7.6 Hz, 2H), 7.77 (d, *J* = 5.5 Hz, 1H), 7.73 - 7.66 (m, 2H), 7.49 (d, *J* = 5.5 Hz, 1H), 7.31 (t, *J* = 7.9 Hz, 1H), 6.95 (d, *J* = 7.6 Hz, 1H), 6.03 (d, *J* = 9.1 Hz, 1H), 5.36 (d, *J* = 20.3 Hz, 1H), 4.96 (d, *J* = 20.2 Hz, 1H), 4.55 (s, 2H), 3.56 - 3.41 (m, 6H), 3.33 - 3.27 (m, 3H), 3.07 - 2.94 (m, 1H), 2.70 (d, *J* = 18.3 Hz, 1H), 2.22 - 2.08 (m, 1H). LCMS (ESI) calcd. for $C_{26}H_{27}N_4O_2S_2^+$ [M+H]$^+$: 491.16, found, 491.1.

**Example 465: Preparation of 3-(1-thioxo-4-((3-(trifluoromethyl)-5,6-dihydro-[1,2,4]triazolo[4,3-a]pyrazin-7(8H)-yl)methyl)isoindolin-2-yl)piperidine-2,6-dione (GT-08365)**

With reference to the method of Scheme 4, the target compound GT-08365 was obtained (8 mg, white solid, yield 15%). $^{1}$H NMR (400 MHz, DMSO-d6) δ 11.12 (s, 1H), 7.88 (d, *J* = 7.6 Hz, 1H), 7.69 (d, *J* = 7.2 Hz, 1H), 7.57 (t, *J* = 7.5 Hz, 1H), 6.00 - 5.92 (m, 1H), 4.93 (d, *J* = 20.5 Hz, 1H), 4.78 (d, *J* = 20.5 Hz, 1H), 4.20 (brs, 2H), 4.01 (brs, 4H), 3.03 (brs, 2H), 2.99 - 2.87 (m, 1H), 2.64 (d, *J* = 19.1 Hz, 2H), 2.17 - 2.01 (m, 1H). LCMS (ESI) calcd. for $C_{20}H_{20}F_3N_6O_2S^+$ [M+H]$^+$: 465.13, found,

465.1.

**Example 466: Preparation of 3-(4-((4-(2-((2,4-dimethylphenyl)thio)phenyl)piperazin-1-yl)methyl)-1-thioxoisoindolin-2-yl)piperidine-2,6-dione (GT-08332)**

With reference to the method of Scheme 4, the target compound GT-08332 was obtained (18 mg, white solid, yield 27%). $^1$H NMR (400 MHz, DMSO-d6) δ 11.21 (s, 1H), 11.15 (s, 1H), 8.06 (d, *J* = 7.4 Hz, 1H), 8.01 (d, *J* = 7.6 Hz, 1H), 7.69 (t, *J* = 7.6 Hz, 1H), 7.35 (d, *J* = 7.8 Hz, 1H), 7.25 (s, 1H), 7.13 (dd, *J* = 10.3, 6.1 Hz, 3H), 7.03 - 6.90 (m, 1H), 6.39 (d, *J* = 7.8 Hz, 1H), 6.02 (d, *J* = 9.2 Hz, 1H), 5.34 (d, *J* = 20.4 Hz, 1H), 4.95 (d, *J* = 20.5 Hz, 1H), 4.54 (s, 2H), 3.52 (dd, *J* = 30.2, 9.8 Hz, 2H), 3.38 - 3.34 (m, 2H), 3.33 - 3.16 (m, 4H), 3.07 - 2.93 (m, 1H), 2.76 - 2.63 (m, 1H), 2.47 - 2.40 (m, 1H), 2.33 (s, 3H), 2.24 (s, 3H), 2.18 - 2.11 (m, 1H). LCMS (ESI) calcd. for $C_{32}H_{35}N_4O_2S_2^+$ [M+H]$^+$: 571.22, found, 571.1.

**Example 467: Preparation of 3-(4-((6,7-dihydrothieno[3,2-c]pyridin-5(4H)-yl)methyl)-1-thioxoisoindolin-2-yl)piperidine-2,6-dione (GT-08333)**

With reference to the method of Scheme 4, the target compound GT-08333 was obtained (13 mg, white solid, yield 27%). $^1$H NMR (400 MHz, DMSO-d6) δ 11.20 (s, 1H), 11.10 (s, 1H), 8.02 - 7.98 (m, 2H), 7.70 (t, *J* = 7.2 Hz, 1H), 7.46 (d, *J* = 3.9 Hz, 1H), 6.90 (d, *J* = 4.3 Hz, 1H), 6.02 (d, *J* = 9.6 Hz, 1H), 5.26 (d, *J* = 20.6 Hz, 1H), 5.11 (d, *J* = 20.1 Hz, 1H), 4.91 (dd, *J* = 19.8, 8.1 Hz, 1H), 4.65 (brs, 1H), 4.54 (brs, 1H), 4.29 (s, 2H), 3.79 (s, 1H), 3.50 (s, 1H), 3.27 - 3.07 (m, 2H), 3.07 - 2.92 (m, 1H), 2.69 (d, *J* = 17.2 Hz, 1H), 2.20 - 2.07 (m, 1H). LCMS (ESI) calcd. for $C_{21}H_{22}N_3O_2S_2^+$ [M+H]$^+$: 412.11, found, 412.1.

**Example 468: Preparation of 3-(4-((4-(benzo[d]isothiazol-3-yl)piperazin-1-yl)methyl)-1-thioxoisoindolin-2-yl)piperidine-2,6-dione (GT-08366)**

With reference to the method of Scheme 4, the target compound GT-08366 was obtained (15 mg, white solid, yield 26%). $^1$H NMR (400 MHz, DMSO-d6) δ 11.31 (s, 1H), 11.21 (s, 1H), 8.12 (t, *J* = 8.1 Hz, 2H), 8.01 (d, *J* = 7.7 Hz, 2H), 7.70 (t, *J* = 7.7 Hz, 1H), 7.60 (t, *J* = 8.0 Hz, 1H), 7.47 (t, *J* = 7.6 Hz, 1H), 6.13 - 5.90 (m, 1H), 5.36 (d, *J* = 20.2 Hz, 1H), 4.94 (d, *J* = 20.1 Hz, 1H), 4.62 - 4.46 (m, 2H), 4.09 (d, *J* = 11.7 Hz, 2H), 3.64 - 3.52 (m, 3H), 3.38 - 3.23 (m, 4H), 3.05 - 2.92 (m, 1H), 2.70 (d, *J* = 19.2 Hz, 1H), 2.18 - 2.08 (m, 1H). LCMS (ESI) calcd. for $C_{25}H_{26}N_5O_2S_2^+$ [M+H]$^+$: 492.15, found, 492.1.

**Example 469: Preparation of (S)-3-(4-((4-(morpholinomethyl)benzyl)oxy)-1-thioxoisoindolin-2-yl)piperidine-2,6-dione (GT-05712)**

With reference to the method of Scheme 5, the target compound GT-05712 was obtained (23 mg, white solid, yield 45%). $^1$H NMR (400 MHz, DMSO-d6) δ 11.09 (s, 1H), 7.51 (d, *J* = 6.3 Hz, 2H), 7.45 (d, *J* = 8.0 Hz, 2H), 7.40 - 7.28 (m, 3H), 5.95 (s, 1H), 5.24 (s, 2H), 4.80 (d, *J* = 20.5 Hz, 1H), 4.61 (d, *J* = 20.3 Hz, 1H), 3.61 - 3.54 (m, 4H), 3.46 (s, 2H), 2.95 (s, 1H), 2.61 (d, *J* = 14.2 Hz, 2H), 2.34 (s, 4H), 2.02 (d, *J* = 5.5 Hz, 1H). LCMS (ESI) calcd. for $C_{25}H_{28}N_3O_4S^+$ [M+H]$^+$: 466.18, found, 466.2.

**Example 470: Preparation of (S)-4-(4-(4-(((2-(2,6-dioxopiperidin-3-yl)-1-thioxoisoindolin-4-yl)oxy)methyl)benzyl)piperazin-1-yl)-3-fluorobenzonitrile (GT-05957)**

With reference to the method of Scheme 5, the target compound GT-05957 was obtained (36 mg, white solid, yield 20%). $^1$H NMR (400 MHz, DMSO-d6) δ 11.09 (s, 1H), 7.68 (dd, *J* = 13.4, 1.8 Hz, 1H), 7.56 (dd, *J* = 8.5, 1.7 Hz, 1H), 7.53 - 7.49 (m, 2H), 7.46 (d, *J* = 7.9 Hz, 2H), 7.36 (t, *J* = 7.2 Hz, 4H), 7.11 (t, *J* = 8.8 Hz, 1H), 5.93 (d, *J* = 9.1 Hz, 1H), 5.25 (s, 2H), 4.81 (d, *J* = 20.4 Hz, 1H), 4.62 (d, *J* = 20.4 Hz, 1H), 3.54 (s, 2H), 3.44 - 3.38 (m, 2H), 3.33 - 3.30 (m, 2H), 3.17 (d, *J* = 4.7 Hz, 4H), 2.94 (td, *J* = 14.2, 7.8 Hz, 1H), 2.61 (d, *J* = 15.9 Hz, 2H), 2.06 (dd, *J* = 6.2, 4.2 Hz, 1H). LCMS (ESI) calcd. for $C_{32}H_{31}FN_5O_3S^+$ [M+H]$^+$: 584.21, found, 584.2.

**Biological activity assay**

**Reagents and Materials:**

| Reagents and materials | Suppliers or Source |
|---|---|
| Human multiple myeloma cell line: MM.1S | ATCC (American Type Culture Collection) |

(continued)

| | |
|---|---|
| Human multiple myeloma cell line resistant to dexamethasone: MM.1R | ATCC |
| Human mantle cell lymphoma cell line: JEKO-1 | ATCC |
| Human non-small cell lung cancer cell line: NCI-H1975 | ATCC |
| Human breast cancer cell line: MCF-7 | ATCC |
| Human T lymphocytic leukemia cell line: Jurkat | ATCC |
| Human T cell acute lymphoblastic leukemia cell line: CCRF-CEM | Dalian Meilun Biotech Co., Ltd. |
| Human multiple myeloma peripheral blood B lymphocyte cell line: RPMI-8226 | ATCC |
| Human acute T lymphocytic leukemia cell line: Molt4 | Dalian Meilun Biotech Co., Ltd. |
| Human myeloid monocytic leukemia cell line: MV-4-11 | ATCC |
| Human acute myeloid leukemia cell line: Kasumi-1 | ATCC |
| Human monocytic leukemia cell line: THP-1 | ATCC |
| Human colon cancer cell line: SW620 | ATCC |
| Human malignant melanoma cell line: A375 | Dalian Meilun Biotech Co., Ltd. |
| Human gastric cancer cell line: MGC803 | Dalian Meilun Biotech Co., Ltd. |
| Human diffuse large B-cell lymphoma cell line: TMD8 | Kyinno Biotechnology Co., Ltd |
| RPMI-1640 Medium | ATCC |
| EMEM Medium | ATCC |
| L-15 Medium | ATCC |
| DMEM high-glucose medium | Dalian Meilun Biotech Co., Ltd. |
| IMDM Medium | Gibco |
| Meilun Cell Counting Kit-8 | Dalian Meilun Biotech Co., Ltd. |
| Fetal bovine serum (FBS) | Sunrise Science Products Company |
| Penicillin-Streptomycin | Beijing TransGen Biotech Company |
| CellTiter-Meiluncell Luminescent Cell Viability Assay Kit | Dalian Meilun Biotech Co., Ltd. |
| Mycoplasma detection kit | Beijing TransGen Biotech Company |
| STR genotype detection | Shanghai Biowing Applied Biotechnology Co. Ltd. |

**Methods:**

**Cell cultures**

The tumor cells used herein were cultured in a cell culture medium listed in table 2 at 37°C in an incubator with 5% $CO_2$. Prior to experimentation, all cell lines used were correctly identified by STR profiling, and tested negative for mycoplasma contamination using a mycoplasma detection kit through routine screenings.

Table 2. Tumor cells and cell culture medium used in the present disclosure

| Cell line | Cell culture medium used |
|---|---|
| MM.1S | RPMI 1640 culture medium supplemented with 10% FBS, as well as penicillin at a final concentration of 100 U/mL and streptomycin at a final concentration of 100 μg/mL |
| MM.1R | |
| JEKO-1 | |
| NCI-H1975 | |
| Jurkat | |
| CCRF-CEM | |
| RPMI-8226 | |
| Molt4 | |
| THP-1 | |
| TMD8 | |
| MCF-7 | EMEM culture medium supplemented with 10% FBS, 10 μg/mL insulin, and penicillin at a final concentration of 100 U/mL and streptomycin at a final concentration of 100 μg/mL |
| MV-4-11 | IMDM culture medium supplemented with 10% FBS, 10 μg/mL insulin, and penicillin at a final concentration of 100 U/mL and streptomycin at a final concentration of 100 μg/mL |
| Kasumi-1 | RPMI-1640 culture medium supplemented with 10% FBS, 2.5% horse serum, as well as penicillin at a final concentration of 100 U/mL and streptomycin at a final concentration of 100 μg/mL |
| SW620 | L-15 culture medium supplemented with 10% FBS, as well as penicillin at a final concentration of 100 U/mL and streptomycin at a final concentration of 100 μg/mL |
| A375 | DMEM high-glucose medium supplemented with 10% FBS, as well as penicillin at a final concentration of 100 U/mL and streptomycin at a final concentration of 100 μg/mL |

**I. Determination of half inhibitory concentration ($IC_{50}$) of the compounds against tumor cells:**

The $IC_{50}$ values of the compounds of the present disclosure (including the compounds in Table 1 and Examples) were determined using the CellTiter-Meiluncell Luminescent Cell Viability Assay Kit from Dalian Meilun Biotech Co., Ltd. The detailed procedure is as follows: Tumor cells were seeded in 96-well cell culture plates at the densities specified in Table 3. On the following day, the test compounds of the present disclosure were subjected to serial dilution. The dilution can be performed according to one of the following two methods:

(1) The first dilution method: The test compounds of the present disclosure (including the compounds in Table 1 and Examples) were subjected to 5-fold serial dilutions starting from a highest concentration of 2000 μM, resulting in a total of 9 concentrations from high to low (2000, 400, 80, 16, 3.2, 0.64, 0.128, 0.0256, 0.00512 μM). Subsequently, 0.5 μL of the test compound dilution was transferred into the 96-well plate containing the cells, with each well containing 100 μL of cell culture medium. This resulted in final test compound concentrations of 10, 2, 0.4, 0.08, 0.016, 0.0032, 0.00064, 0.000128, and 0.0000256 μM, respectively, or

(2) As an alternative, a second dilution method can be used: The test compounds of the present disclosure (including the compounds in Table 1 and Examples) were subjected to a 50-fold gradient dilution starting from a highest concentration of 100 μM, resulting in 2 concentrations (100 μM, 2 μM). Into the 96-well plate, the diluted test compounds and corresponding detection reagents were added, resulting in final test compound concentrations of 500 nM and 10 nM, respectively. The experimental procedure is described below using the diluted 2 μM test compound as an example.

0.5 μL of the aforementioned diluted compound of the present disclosure (including the compounds in Table 1 and Examples) was added to the seeded cells in 100 μL of medium, yielding a final test compound concentration of 10 nM. After the cells were treated with the compounds of the present disclosure for a specified period, the cell viability assay reagent was added to the culture medium according to the kit's instructions to determine cell viability. The negative control was DMSO, and the reference controls were corresponding commercial inhibitors immunomodulatory agents (including lenalidomide and pomalidomide). All controls were treated using the same procedure as the compounds of the present

disclosure. The growth inhibition curves for the compounds of the present disclosure were plotted using Prism GraphPad software, and the $IC_{50}$ values were calculated therefrom.

Table 3. Seeding protocol and treatment time for tumor cells

| Cells | Cells seeding protocol | The times for treating cells with the compounds of the present disclosure (hours) |
|---|---|---|
| MM.1S | cells were seeded at a density of 10,000 cells per well in 100 μL of serum-containing RPMI-1640 medium per well | 96 hours |
| MM.1R | | |
| Jurkat | | |
| RPMI-8226 | | |
| THP-1 | | |
| Molt4 | | |
| TMD8 | | |
| A375 | cells were seeded at a density of 4,000 cells per well in 100 μL of serum-containing DMEM medium per well | 96 hours |
| JEKO-1 | cells were seeded at a density of 4,000 cells per well in 100 μL of serum-containing RPMI-1640 medium per well | 96 hours |
| NCI-H1975 | | |
| CCRF-CEM | | |
| Kasumi-1 | cells were seeded at a density of 10,000 cells per well in 100 μL of serum-containing RPMI-1640 medium per well | 96 hours |
| SW620 | cells were seeded at a density of 4,000 cells per well in 100 μL of serum-containing L-15 medium per well | 96 hours |
| MV-4-11 | cells were seeded at a density of 10,000 cells per well in 100 μL of serum-containing IMDM medium per well | 96 hours |
| MCF-7 | cells were seeded at a density of 4,000 cells per well in 100 μL of serum-containing EMEM medium per well | 96 hours |

The results of tumor cell proliferation inhibition are presented in Tables I-1 through I-9 below.

Table I-1. The Inhibitory activity (Inhibition rate %) of the compounds of the present disclosure on the proliferation of Human multiple myeloma cell (MM.1S)

| Comp. No. /names | Inhibition Rate at 500 nM (%) | Inhibition Rate at 10 nM (%) | Comp. No. /name s | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| lenalidomid e | 1a5 | 1a3 | GT-08490 | 1a10 | 1a7 | GT-08965 | 1a6 | 1a5 |
| GT-07736 | 1a10 | 1a3 | GT-08491 | 1a10 | 1a3 | GT-09245 | 1a9 | 1a6 |
| GT-07846 | 1a10 | 1a8 | GT-08501 | 1a8 | 1a7 | GT-09247 | la7 | 1a7 |
| GT-07850 | la7 | 1a5 | GT-08504 | 1a7 | 1a5 | GT-09250 | la7 | 1a5 |
| GT-08205 | la8 | 1a3 | GT-08595 | 1a7 | 1a6 | GT-09260 | 1a10 | 1a3 |
| GT-08210 | 1a10 | 1a4 | GT-08596 | 1a7 | 1a6 | GT-09261 | 1a6 | 1a6 |
| GT-08206 | 1a10 | 1a7 | GT-08600 | 1a8 | 1a5 | GT-09264 | 1a6 | 1a5 |

(continued)

| Comp. No. /names | Inhibition Rate at 500 nM (%) | Inhibition Rate at 10 nM (%) | Comp. No. /name s | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| GT-08266 | 1a7 | 1a6 | GT-08602 | 1a10 | 1a3 | GT-09267 | 1a10 | 1a9 |
| GT-08193 | 1a6 | 1a5 | GT-08603 | 1a7 | 1a5 | GT-09268 | la7 | 1a5 |
| GT-08237 | 1a6 | 1la6 | GT-08611 | 1a7 | 1a4 | GT-09269 | 1a10 | 1a2 |
| GT-08238 | 1a9 | 1a9 | GT-08615 | 1a6 | 1a3 | GT-09270 | 1a10 | 1a7 |
| GT-08240 | 1a7 | 1a7 | GT-08616 | 1a7 | 1a4 | GT-09274 | 1a6 | 1a5 |
| GT-08241 | 1a9 | 1a4 | GT-08617 | 1a10 | 1a5 | GT-09276 | 1a10 | 1a9 |
| GT-08242 | la8 | 1a5 | GT-08619 | 1a6 | 1a4 | GT-09296 | la7 | 1a6 |
| GT-08364 | la8 | 1a6 | GT-08620 | 1a9 | 1a6 | GT-05712 | 1a9 | 1a7 |
| GT-08463 | la5 | 1a6 | GT-08622 | 1a9 | 1a3 | GT-05957 | 1a10 | 1a10 |
| GT-08464 | la5 | 1a7 | GT-08623 | 1a6 | 1a5 | GT-08166 | 1a10 | 1a6 |
| GT-08465 | la5 | 1a6 | GT-08624 | 1a10 | 1a4 | GT-08171 | 1a10 | 1a7 |
| GT-08466 | la7 | 1a4 | GT-08695 | 1a6 | 1a4 | GT-08172 | la10 | 1a6 |
| GT-08467 | la7 | 1a3 | GT-08817 | 1a6 | 1a4 | GT-08323 | la7 | 1a1 |
| GT-08468 | 1a7 | 1a6 | GT-09215 | 1a7 | 1a5 | GT-08324 | 1a10 | 1a4 |
| GT-08471 | 1a6 | 1a6 | GT-09217 | 1a8 | 1a5 | GT-08325 | 1a10 | 1a5 |
| GT-08476 | 1a6 | 1a2 | GT-08845 | 1a6 | 1a4 | GT-08327 | 1a10 | 1a6 |
| GT-08477 | 1a6 | la5 | GT-08853 | 1a6 | 1a5 | GT-08328 | 1a10 | 1a7 |
| GT-08478 | 1a7 | 1a6 | GT-08856 | 1a6 | 1a4 | GT-08329 | la10 | 1a3 |
| GT-08480 | 1a10 | 1a4 | GT-08859 | 1a7 | 1a5 | GT-08330 | 1a10 | 1a4 |
| GT-08481 | 1a9 | 1a5 | GT-08920 | 1a6 | 1a5 | GT-08331 | 1a10 | 1a3 |
| GT-08482 | 1a10 | 1a7 | GT-08947 | 1a6 | 1a5 | GT-08332 | 1a10 | 1a3 |
| GT-08488 | la8 | 1a3 | GT-08956 | 1a6 | 1a4 | GT-09317 | 1a8 | 1a3 |
| GT-08489 | 1a10 | 1a3 | GT-08962 | 1a6 | 1a5 | | | |
| Note: in table I-1, the symbols 1a1, 1a2, 1a3, 1a4, 1a5, 1a6, 1a7, 1a8, 1a9, and 1a10 are defined as follows: 0 < 1a1 < 5%, 5% ≤ 1a2 < 10%, 10% ≤ la3 < 20%, 20% ≤ 1a4 < 30%, 30% ≤ la5 < 40%, 40% ≤ 1a6 < 50%, 50% ≤ la7 < 60%, 60% ≤ la8 < 70%, 70% ≤ la9 < 80%, 80% ≤ 1a10 ≤ 100%. | | | | | | | | |

Table I-2. The Inhibitory activity (Inhibition rate %) of the compounds of the present disclosure on the proliferation of Human multiple myeloma peripheral blood B lymphocyte cell (RPMI-8226)

| Comp. No. /names | Inhibition Rate at 500 nM (%) | Inhibition Rate at 10 nM (%) | Comp. No. /names | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| pomalid omide | 1b5 | 1b3 | GT-08482 | 1b8 | 1b3 | GT-08166 | 1b8 | 1b3 |
| lenalido mide | 1b3 | 1b2 | GT-08489 | 1b9 | 1b1 | GT-08171 | 1b10 | 1b4 |
| GT-07846 | 1b8 | 1b4 | GT-08490 | 1b9 | 1b4 | GT-08172 | 1b10 | 1b4 |
| GT-08204 | 1b9 | 1b8 | GT-08491 | 1b9 | 1b3 | GT-08325 | 1b8 | 1b4 |
| GT-08202 | 1b6 | 1b4 | GT-08602 | 1b9 | 1b3 | GT-08327 | 1b10 | 1b3 |
| GT-08207 | 1b8 | 1b7 | GT-08617 | 1b7 | 1b3 | GT-08329 | 1b9 | 1b3 |

(continued)

| Comp. No. /names | Inhibition Rate at 500 nM (%) | Inhibition Rate at 10 nM (%) | Comp. No. /names | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| GT-08206 | 1b10 | 1b10 | GT-08624 | 1b8 | 1b3 | GT-08330 | 1b9 | 1b3 |
| GT-08260 | 1b9 | 1b9 | GT-09260 | 1b6 | 1b2 | GT-08331 | 1b6 | 1b3 |
| GT-08293 | 1b6 | 1b4 | GT-09270 | 1b9 | 1b4 | GT-08364 | 1b6 | 1b4 |
| GT-08294 | 1b6 | 1b4 | GT-09276 | 1b9 | 1b4 | GT-09319 | 1b7 | 1b2 |
| GT-08238 | 1b7 | 1b4 | GT-05957 | 1b8 | 1b7 | | | |
| Note: in table I-2, the symbols 1b1, 1b2, 1b3, 1b4, 1b5, 1b6, 1b7, 1b8, 1b9, and 1b10 are defined as follows: 0 < 1b1 < 5%, 5% ≤ 1b2 < 10%, 10% ≤ lb3 < 20%, 20% < 1b4 < 30%, 30% ≤ 1b5 < 40%, 40% ≤ lb6 < 50%, 50% ≤ lb7 < 60%, 60% ≤ 1b8 < 70%, 70% ≤ lb9 < 80%, 80% ≤ 1b10 ≤ 100%. | | | | | | | | |

Table I-3. The Inhibitory activity (Inhibition rate %) of the compounds of the present disclosure on the proliferation of Human acute T lymphocytic leukemia cell (Molt4)

| Comp. No. /names | Inhibition Rate at 500 nM (%) | Inhibition Rate at 10 nM (%) | Comp. No. /names | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| lenalido mide | 1c2 | 1c2 | GT-08603 | 1c7 | 1c2 | GT-09276 | 1c10 | 1c10 |
| GT-07736 | 1c7 | 1c4 | GT-08611 | 1c10 | 1c2 | GT-08166 | 1c11 | 1c4 |
| GT-07846 | 1c11 | 1c10 | GT-08612 | 1c8 | 1c4 | GT-08170 | 1c9 | 1c3 |
| GT-08205 | 1c9 | 1c2 | GT-08615 | 1c8 | 1c3 | GT-08171 | 1c11 | 1c11 |
| GT-08210 | 1c11 | 1c4 | GT-08616 | 1c7 | 1c4 | GT-08172 | 1c11 | 1c9 |
| GT-08206 | 1c11 | 1c10 | GT-08617 | 1c11 | 1c5 | GT-08324 | 1c11 | 1c4 |
| GT-08236 | 1c7 | 1c4 | GT-08622 | 1c11 | 1c1 | GT-08325 | 1c11 | 1c4 |
| GT-08238 | 1c11 | 1c6 | GT-08960 | 1c7 | 1c5 | GT-08327 | 1c11 | 1c4 |
| GT-08480 | 1c11 | 1c2 | GT-09240 | 1c6 | 1c8 | GT-08328 | 1c11 | 1c4 |
| GT-08482 | 1c11 | 1c1 | GT-09245 | 1c8 | 1c4 | GT-08329 | 1c11 | 1c4 |
| GT-08488 | 1c10 | 1c4 | GT-09260 | 1c11 | 1c5 | GT-08330 | 1c11 | 1c1 |
| GT-08489 | 1c11 | 1c2 | GT-09267 | 1c11 | 1c11 | GT-08331 | 1c11 | 1c4 |
| GT-08490 | 1c11 | 1c2 | GT-09268 | 1c7 | 1c7 | GT-08332 | 1c11 | 1c1 |
| GT-08491 | 1c11 | 1c5 | GT-09269 | 1c11 | 1c6 | | | |
| GT-08602 | 1c11 | 1c1 | GT-09270 | 1c11 | 1c10 | | | |
| Note: in table I-3, the symbols 1c1, 1c2, 1c3, 1c4, 1c5, 1c6, 1c7, 1c8, 1c9, 1c10, and 1c11 are defined as follows: -5% < 1c1 < 0, 0 < 1c2 < 5%, 5% ≤ 1c3 < 10%, 10% ≤ 1c4 < 20%, 20% ≤ 1c5 < 30%, 30% ≤ 1c6 < 40%, 40% ≤ 1c7 < 50%, 50% ≤ 1c8 < 60%, 60% ≤ 1c9 < 70%, 70% ≤ 1c10 < 80%, 80% ≤ 1c11 ≤ 100%. | | | | | | | | |

Table I-4. The Inhibitory activity (Inhibition rate %) of the compounds of the present disclosure on the proliferation of Human myeloid monocytic leukemia cell (MV-4-11)

| Comp. No. /names | Inhibition Rate at 500 nM (%) | Inhibition Rate at 10 nM (%) | Comp . No. /name s | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| pomalidomid e | 1d4 | 1d5 | GT-08492 | 1d8 | 1d7 | GT-09245 | 1d10 | 1d9 |

(continued)

| Comp. No. /names | Inhibition Rate at 500 nM (%) | Inhibition Rate at 10 nM (%) | Comp . No. /name s | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| lenalidomide | 1d4 | 1d4 | GT-08500 | 1d8 | 1d7 | GT-09247 | 1d8 | 1d8 |
| GT-07736 | 1d8 | 1d4 | GT-08501 | 1d8 | 1d8 | GT-09250 | 1d8 | 1d7 |
| GT-07846 | 1d10 | 1d4 | GT-08504 | 1d8 | 1d8 | GT-09260 | 1d10 | 1d5 |
| GT-07850 | 1d8 | 1d8 | GT-08506 | 1d8 | 1d7 | GT-09261 | 1d8 | 1d8 |
| GT-08202 | 1d8 | 1d7 | GT-08600 | 1d10 | 1d8 | GT-09264 | 1d8 | 1d7 |
| GT-08205 | 1d10 | 1d3 | GT-08602 | 1d10 | 1d5 | GT-09267 | 1d10 | 1d10 |
| GT-08210 | 1d10 | 1d3 | GT-08603 | 1d10 | 1d8 | GT-09268 | 1d9 | 1d3 |
| GT-08206 | 1d10 | 1d10 | GT-08611 | 1d10 | 1d5 | GT-09269 | 1d10 | 1d4 |
| GT-08262 | 1d8 | 1d6 | GT-08491 | 1d10 | 1d5 | GT-09270 | 1d10 | 1d10 |
| GT-08263 | 1d10 | 1d9 | GT-09242 | 1d7 | 1d9 | GT-09271 | 1d8 | 1d8 |
| GT-08266 | 1d10 | 1d9 | GT-08616 | 1d9 | 1d1 | GT-09276 | 1d10 | 1d10 |
| GT-08268 | 1d9 | 1d9 | GT-08617 | 1d10 | 1d3 | GT-09281 | 1d8 | 1d6 |
| GT-08301 | 1d8 | 1d8 | GT-08620 | 1d9 | 1d7 | GT-05957 | 1d8 | 1d6 |
| GT-08292 | 1d9 | 1d9 | GT-08622 | 1d10 | 1d4 | GT-08166 | 1d10 | 1d7 |
| GT-08294 | 1d9 | 1d8 | GT-08624 | 1d10 | 1d5 | GT-08170 | 1d9 | 1d6 |
| GT-08303 | 1d8 | 1d8 | GT-08817 | 1d7 | 1d8 | GT-08171 | 1d10 | 1d9 |
| GT-08236 | 1d9 | 1d4 | GT-09214 | 1d8 | 1d4 | GT-08172 | 1d10 | 1d9 |
| GT-08238 | 1d10 | 1d3 | GT-09215 | 1d10 | 1d8 | GT-08310 | 1d9 | 1d8 |
| GT-08240 | 1d8 | 1d2 | GT-09217 | 1d10 | 1d8 | GT-08314 | 1d9 | 1d8 |
| GT-08468 | 1d8 | 1d8 | GT-09220 | 1d8 | 1d7 | GT-08316 | 1d8 | 1d5 |
| GT-08471 | 1d8 | 1d8 | GT-09224 | 1d8 | 1d1 | GT-08323 | 1d8 | 1d1 |
| GT-08473 | 1d8 | 1d8 | GT-09226 | 1d8 | 1d7 | GT-08324 | 1d10 | 1d7 |
| GT-08480 | 1d10 | 1d6 | GT-09229 | 1d8 | 1d8 | GT-08325 | 1d11 | 1d9 |
| GT-08481 | 1d9 | 1d5 | GT-09231 | 1d8 | 1d7 | GT-08327 | 1d10 | 1d8 |
| GT-08482 | 1d10 | 1d8 | GT-08840 | 1d8 | 1d8 | GT-08328 | 1d10 | 1d8 |
| GT-08488 | 1d10 | 1d5 | GT-08856 | 1d8 | 1d8 | GT-08329 | 1d10 | 1d1 |
| GT-08489 | 1d10 | 1d7 | GT-08956 | 1d8 | 1d5 | GT-08330 | 1d10 | 1d4 |
| GT-08490 | 1d10 | 1d8 | GT-08970 | 1d8 | 1d5 | GT-08332 | 1d9 | 1d1 |
| Note: in table I-4, the symbols 1d1, 1d2, 1d3, 1d4, 1d5, 1d6, 1d7, 1d8, 1d9, 1d10, and 1d11 are defined as follows: -5% < 1d1 < 0, 0 < 1d2 < 5%, 5% ≤ 1d3 < 10%, 10% ≤ 1d4 < 18%, 18% ≤ 1d5 < 25%, 25% ≤ 1d6 < 30%, 30% ≤ 1d7 < 40%, 40% ≤ 1d8 < 60%, 60% < 1d9 < 80%, 80% ≤ 1d10 ≤ 100%, 100% < 1d11 ≤ 110%. | | | | | | | | |

Table I-5. The Inhibitory activity (Inhibition rate %) of the compounds of the present disclosure on the proliferation of Human breast cancer cell (MCF-7)

| Comp. No. /names | Inhibition Rate at 500 nM (%) | Inhibition Rate at 10 nM (%) | Comp. No. /names | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| pomalido mide | 1e2 | 1e3 | GT-08853 | 1e7 | 1e3 | GT-09271 | 1e7 | 1e5 |

(continued)

| Comp. No. /names | Inhibition Rate at 500 nM (%) | Inhibition Rate at 10 nM (%) | Comp. No. /names | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| lenalidom ide | 1e3 | 1e2 | GT-08859 | 1e7 | 1e8 | GT-09276 | 1e11 | 1e6 |
| GT-07846 | 1e7 | 1ed | GT-08861 | 1e7 | 1e6 | GT-08166 | 1e9 | 1el |
| GT-08206 | 1e11 | 1e2 | GT-08920 | 1e6 | 1e8 | GT-08171 | 1e10 | 1e5 |
| GT-08266 | 1e7 | 1e7 | GT-08923 | 1e10 | 1e10 | GT-08172 | 1e10 | 1e8 |
| GT-08489 | 1e8 | 1ed | GT-08925 | 1e9 | 1e9 | GT-08327 | 1e10 | 1e2 |
| GT-08490 | 1e8 | 1e6 | GT-08928 | 1e9 | 1e7 | GT-08330 | 1e8 | 1e4 |
| GT-08491 | 1e7 | 1ed | GT-08931 | 1e9 | 1e6 | GT-09317 | 1e11 | 1e3 |
| GT-08504 | 1e7 | 1e5 | GT-08934 | 1e7 | 1e6 | GT-09319 | 1e10 | 1e5 |
| GT-08840 | 1e9 | 1e8 | GT-08937 | 1e7 | 1e6 | GT-09329 | 1e7 | 1e5 |
| GT-08843 | 1e9 | 1e9 | GT-09247 | 1e7 | 1e5 | GT-09331 | 1e7 | 1e5 |
| GT-08845 | 1e9 | 1e8 | GT-09267 | 1e10 | 1e3 | | | |
| GT-08850 | 1e8 | 1e7 | GT-09270 | 1e10 | 1e4 | | | |
| Note: in table I-5, the symbols 1e1, 1e2, 1e3, 1e4, 1e5, 1e6, 1e7, 1e8, 1e9, 1e10, and 1e11 are defined as follows: -5% < 1e1 < 0, 0 < 1e2 < 5%, 5% ≤ 1e3 < 10%, 10% ≤ 1e4 < 20%, 20% ≤ 1e5 < 30%, 30% ≤ 1e6 < 40%, 40% ≤ 1e7 < 50%, 50% ≤ 1e8 < 60%, 60% ≤ 1e9 < 70%, 70% ≤ 1e10 < 80%, 80% ≤ 1e11 ≤ 100%. | | | | | | | | |

Table I-6. The Inhibitory activity (Inhibition rate %) of the compounds of the present disclosure on the proliferation of Human T cell acute lymphoblastic leukemia cell (CCRF-CEM)

| Comp. No. /names | Inhibition Rate at 500 nM (%) | Inhibition Rate at 10 nM (%) | Comp. No. /names | Inhibition Rate at 500 nM (%) | Inhibition Rate at 10 nM (%) | Comp. No. /names | Inhibition Rate at 500 nM (%) | Inhibition Rate at 10 nM (%) |
|---|---|---|---|---|---|---|---|---|
| pomalido mide | 1f2 | 1f2 | GT-08166 | 1f11 | 1f4 | GT-08328 | 1f11 | 1f4 |
| lenalido mide | 1f4 | 1f4 | GT-08171 | 1f11 | 1f10 | GT-08329 | 1f11 | 1f4 |
| GT-07846 | 1f11 | 1f7 | GT-08172 | 1f11 | 1f9 | GT-08330 | 1f11 | 1f3 |
| GT-08205 | 1f9 | 1f4 | GT-08324 | 1f11 | 1f3 | GT-08331 | 1f11 | 1f3 |
| GT-08206 | 1f11 | 1f11 | GT-08325 | 1f11 | 1f4 | GT-08332 | 1f11 | 1f1 |
| GT-08260 | 1f7 | 1f5 | GT-08327 | 1f11 | 1f6 | | | |
| Note: in table I-6, the symbols 1f1, 1f2, 1f3, 1f4, 1f5, 1f6, 1f7, 1f8, 1f9, 1f10, and 1f11 are defined as follows: -5% < 1f1 < 0, 0 < 1f2 < 5%, 5% ≤ 1f3 < 10%, 10% ≤ 1f4 < 20%, 20% ≤ 1f5 < 30%, 30% ≤ 1f6 < 40%, 40% ≤ 1f7 < 50%, 50% ≤ 1f8 < 60%, 60% ≤ 1f9 < 70%, 70% ≤ 1f10 < 80%, 80% ≤ 1f11 ≤ 100%. | | | | | | | | |

Table I-7. The Inhibitory activity (Inhibition rate %) of the compounds of the present disclosure against tumor cells proliferation

| Comp. No. /names | Kasumi-1 | | Jurkat | |
|---|---|---|---|---|
| | Inhibition Rate at 500 nM (%) | Inhibition Rate at 10 nM (%) | Inhibition Rate at 500 nM (%) | Inhibition Rate at 10 nM (%) |
| pomalidomide | 1g2 | 1g1 | 1g5 | 1g5 |
| lenalidomide | 1g3 | 1g4 | 1g5 | 1g6 |

(continued)

| Comp. No. /names | Kasumi-1 | | Jurkat | |
|---|---|---|---|---|
| | Inhibition Rate at 500 nM (%) | Inhibition Rate at 10 nM (%) | Inhibition Rate at 500 nM (%) | Inhibition Rate at 10 nM (%) |
| GT-07846 | 1g12 | 1g8 | 1g12 | 1g7 |
| GT-08205 | 1g8 | 1g5 | 1g7 | 1g4 |
| GT-08210 | 1g12 | 1g6 | 1g10 | 1g6 |
| GT-08206 | 1g12 | 1g10 | 1g12 | 1g9 |
| GT-08263 | 1g10 | 1g9 | 1g7 | 1g6 |
| GT-08266 | 1g9 | 1g9 | - | - |
| GT-08268 | 1g9 | 1g9 | - | - |
| GT-08301 | 1g9 | 1g9 | 1g7 | 1g6 |
| GT-08292 | 1g9 | 1g10 | 1g7 | 1g7 |
| GT-08166 | 1g12 | 1g5 | 1g12 | 1g5 |
| GT-08171 | 1g12 | 1g7 | 1g12 | 1g8 |
| GT-08172 | 1g12 | 1g6 | 1g12 | 1g9 |
| GT-08324 | 1g10 | 1g3 | 1g8 | 1g2 |
| GT-08325 | 1g12 | 1g3 | 1g11 | 1g2 |
| GT-08327 | 1g12 | 1g7 | 1g12 | 1g6 |
| GT-08328 | 1g10 | 1g4 | 1g8 | 1g3 |
| GT-08329 | 1g12 | 1g4 | 1g11 | 1g5 |
| GT-08330 | 1g12 | 1g4 | 1g11 | 1g2 |
| GT-08331 | 1g11 | 1g4 | 1g9 | 1g4 |
| GT-08332 | 1g12 | 1g2 | 1g12 | 1g3 |
| Note: in table I-7, the symbols 1g1, 1g2, 1g3, 1g4, 1g5, 1g6, 1g7, 1g8, 1g9, 1g10, 1g11, and 1g12 are defined as follows: -10% < 1g1 ≤ -5%, -5% < 1g2 < 0, 0 < 1g3 < 5%, 5% ≤ 1g4 < 10%, 10% ≤ 1g5 < 20%, 20% ≤ 1g6 < 30%, 30% ≤ 1g7 < 40%, 40% ≤ 1g8 < 50%, 50% ≤ 1g9 < 60%, 60% ≤ 1g10 < 70%, 70% ≤ 1g11 < 80%, 80% ≤ 1g12 < 100%. The symbol "-" indicates "not detected". | | | | |

Table I-8. The Inhibitory activity (Inhibition rate %) of the compounds of the present disclosure against tumor cells proliferation

| Comp. No. /names | THP-1 | | SW620 | | A375 | |
|---|---|---|---|---|---|---|
| | Inhibition Rate at 500 nM (%) | Inhibition Rate at 10 nM (%) | Inhibition Rate at 500 nM (%) | Inhibition Rate at 10 nM (%) | Inhibition Rate at 500 nM (%) | Inhibition Rate at 10 nM (%) |
| pomalidomide | 1h5 | 1h5 | 1h6 | 1h6 | 1h4 | 1h5 |
| lenalidomide | 1h4 | 1h3 | 1h4 | 1h4 | 1h4 | 1h4 |
| GT-09314 | 1h12 | 1h4 | 1h12 | 1h5 | 1h8 | 1h4 |
| GT-09316 | 1h12 | 1h4 | 1h12 | 1h5 | 1h12 | 1h7 |
| GT-09317 | 1h13 | 1h12 | 1h13 | 1h12 | 1h12 | 1h9 |
| GT-09318 | 1h12 | 1h5 | 1h12 | 1h5 | 1h10 | 1h6 |
| GT-09319 | 1h12 | 1h12 | 1h12 | 1h11 | 1h12 | 1h8 |
| GT-09322 | 1h12 | 1h5 | 1h12 | 1h4 | 1h12 | 1h5 |

(continued)

| Comp. No. /names | THP-1 | | SW620 | | A375 | |
|---|---|---|---|---|---|---|
| | Inhibition Rate at 500 nM (%) | Inhibition Rate at 10 nM (%) | Inhibition Rate at 500 nM (%) | Inhibition Rate at 10 nM (%) | Inhibition Rate at 500 nM (%) | Inhibition Rate at 10 nM (%) |
| GT-09324 | 1h12 | 1h5 | 1h12 | 1h6 | 1h12 | 1h7 |
| GT-09325 | 1h12 | 1h4 | 1h12 | 1h1 | 1h12 | 1h6 |
| GT-09327 | 1h12 | 1h6 | 1h12 | 1h7 | 1h12 | 1h6 |
| GT-09331 | - | - | - | - | 1h9 | 1h7 |
| Note: in table I-8, the symbols 1h1, 1h2, 1h3, 1h4, 1h5, 1h6, 1h7, 1h8, 1h9, 1h10, 1h11, 1h12, and 1h13 are defined as follows: -15% < 1h1 ≤ -5%, -5% < 1h2 < 0, 0 < 1h3 < 5%, 5% ≤ 1h4 < 10%, 10% ≤ 1h5 < 20%, 20% ≤ 1h6 < 30%, 30% ≤ 1h7 < 40%, 40% ≤ 1h8 < 50%, 50% ≤ 1h9 < 60%, 60% ≤ 1h10 < 70%, 70% ≤ 1h11 < 80%, 80% ≤ 1h12 ≤ 100%, 100% < 1h13 ≤ 110%. The symbol "-" indicates "not detected". | | | | | | |

Table I-9: Inhibitory activity ($IC_{50}$, nM) of the compounds of the present disclosure on tumor cell proliferation

| Comp. No. /names | MM.1S | Jurkat | H1975 | JEKO-1 | MGC80 3 | MCF7 | RPMI-8226 | TMD8 |
|---|---|---|---|---|---|---|---|---|
| lenalidomide | G | G | G | G | G | G | G | G |
| pomalidomide | D | G | G | F | G | G | E | G |
| GT-05038 | C | D | D | - | - | - | - | - |
| GT-05088 | A | B | B | A | C | C | - | - |
| GT-05092 | A | B | B | A | C | C | - | - |
| GT-05103 | A | - | - | - | - | - | - | - |
| GT-05104 | A++ | C | C | C | B | - | - | - |
| GT-05106 | A++ | A++ | A+ | A++ | A+ | A | - | - |
| GT-05109 | A++ | A++ | A+ | A+ | A | A | - | - |
| GT-05111 | A | B | B | B | C | C | - | - |
| GT-05112 | B | - | - | - | - | - | - | - |
| GT-05145 | A++ | A++ | A+ | A++ | A | A | - | - |
| GT-05146 | C | - | - | - | - | - | - | - |
| GT-05147 | A | C | - | C | - | - | - | - |
| GT-05149 | A++ | A+ | A | A+ | A | A | - | - |
| GT-05150 | A+ | A | B | B | C | C | - | - |
| GT-04474 | - | B | - | - | - | - | - | - |
| GT-05182 | C | - | - | - | - | - | - | - |
| GT-05183 | B | - | - | - | - | - | - | - |
| GT-05243 | A++ | - | - | - | - | - | A | C |
| GT-05244 | C | - | - | - | - | - | - | - |
| GT-05245 | A | - | - | - | - | - | - | - |
| GT-05247 | A++ | - | - | - | - | - | A | C |
| GT-05248 | B | - | - | - | - | - | - | - |
| Note: in table I-9, the symbols A++, A+, A, B, C, D, E, F, and G are defined as follows: 0 nM < A++ ≤ 5 nM; 5 nM < A+ ≤ 10 nM; 10 nM < A ≤ 50 nM; 50 nM < B ≤ 100 nM; 100 nM < C ≤ 500 nM; 500 nM < D ≤ 1000 nM; 1000 nM < E ≤ 5000 nM; 5000 nM < F ≤ 10000 nM; 10000 nM < G. The symbol "-" indicates "not detected". | | | | | | | | |

The experimental results demonstrated that the compounds of the present disclosure (including those listed in Table 1 and in Examples) can effectively inhibit the proliferation of tumor cells proliferation (as shown in Tables I-1 through I-9), which were superior to those of the corresponding positive control drugs.

## II. Western Blot

Conventional Western blot assay was performed to evaluate the effects of the compounds of the present disclosure on the expression of target proteins in cells.

(1) Cell Seeding and Protein Harvesting: Human peripheral blood mononuclear cells (hPBMCs) at a density of $1\times10^6$ cells/mL were thawed according to the PBMC Recovery Protocol (Milecell Biotechnologies Inc, Shanghai). The cells were then treated with the compounds of the present disclosure (including the compounds listed in Table 1 and the Examples compounds) at concentrations of 50 nM and 500 nM. Controls included a negative control (DMSO) and a positive control (the commercial immunomodulatory drug lenalidomide), both processed following the identical experimental protocol as the compounds of the present disclosure. After 24 hours of compounds treatment, cells were harvested and lysed in RIPA protein lysate containing protease inhibitors on ice for 30 min, followed by centrifugation. The supernatant was aspirated as the extracted total cell protein. The protein concentration of the supernatant was determined using the conventional BCA (Bicinchoninic Acid) assay.

(2) To the collected supernatant, 5X SDS sample loading buffer was added, followed by denaturation at 95°C for 5 minutes. The denatured samples were then separated by SDS-PAGE electrophoresis on a polyacrylamide gel, transferred onto a nitrocellulose membrane (0.45 μm NC membrane), blocked with blocking buffer at room temperature for 1 hour, and subjected to antibody incubation and development procedures according to the antibody manufacturer's instructions (Cell Signaling Technology Company).

Half-Maximal Degradation Concentration value (the drug concentration required for degrading proteins by 50%, abbreviated as $DC_{50}$) reads method: comparing the grayscale values of Western blot bands from drug-treated samples with those from blank DMSO-treated controls, and identifying the drug concentration range at which the grayscale value is half that of the DMSO-treated control bands.

$DC_{50}$ values can be calculated using ImageJ software to measure grayscale values of Western blot bands from drug-treated samples. The concentration-response curve is fitted to estimate the drug concentration corresponding to half the grayscale value.

Target protein remaining (%) refers to the percentage of target protein remaining in cells after treatment with protein degrader compounds.

Calculation method for target protein remaining (%): Use ImageJ software to measure grayscale values of Western blot bands from drug (protein degrader)-treated samples. The grayscale value of drug-treated bands is divided by that of internal control protein bands, and the resulting quotient is normalized to obtain the relative percentage of remaining target protein after protein degrader treatment.

$$\text{Target protein degradation rate (\%)} = 1 - \text{target protein remaining (\%)}.$$

The effects of the compounds of the present disclosure (including the compounds listed in Table 1 and the Examples compounds) on substrate proteins expression in cells were shown in Table II-1below.

Table II-1 Degradation of substrate proteins WEE1, IKZF1, IKZF2, IKZF3, CK1α and GSPT1 by the compounds of the present disclosure

| Comp. No./names | Wee1 | IKZF 1 | IKZF2 | IKZF3 | GSPT1 | CK1α |
|---|---|---|---|---|---|---|
| Lenalidomide | B | A | B | A | B | B |
| GT-04474 | A | A | A | A | A | B |
| GT-04509 | A | B | A | B | B | B |
| GT-05035 | A | A | A | A | A | B |
| GT-05038 | B | A | A | A | A | B |
| GT-05041 | B | A | B | B | A | B |

(continued)

| Comp. No./names | Wee1 | IKZF 1 | IKZF2 | IKZF3 | GSPT1 | CK1α |
|---|---|---|---|---|---|---|
| GT-05088 | A | A | A | A | A | B |
| GT-05089 | B | B | B | B | A | B |
| GT-05092 | A | A | A | A | A | B |
| GT-05103 | B | B | B | B | A | B |
| GT-05104 | A | B | B | B | A | B |
| GT-05106 | A | A | A | A | A | B |
| GT-05109 | A | A | A | A | A | A |
| GT-05110 | B | B | B | B | A | B |
| GT-05111 | A | A | A | A | A | A |
| GT-05112 | B | A | B | A | A | B |
| GT-05113 | B | A | B | A | A | B |
| GT-05145 | A | A | A | A | A | A |
| GT-05146 | A | A | B | B | A | B |
| GT-05147 | A | A | A | A | A | B |
| GT-05148 | B | B | B | B | A | B |
| GT-05149 | A | A | A | A | A | B |
| GT-05150 | A | A | A | B | A | B |
| GT-05151 | A | A | A | A | A | B |
| GT-05182 | A | B | B | B | A | B |
| GT-05183 | B | B | B | A | A | B |
| GT-05243 | A | B | A | A | A | B |
| GT-05244 | B | B | A | B | A | B |
| GT-05245 | A | B | A | A | A | B |
| GT-05246 | A | B | B | B | A | B |
| GT-05247 | A | B | A | A | A | A |
| GT-05248 | A | A | A | A | A | B |
| GT-07732 | A | B | B | A | A | B |
| GT-07733 | A | B | B | A | A | B |
| GT-07734 | A | B | B | A | A | B |
| GT-07735 | A | B | B | A | A | B |
| GT-07736 | A | A | B | A | A | B |
| GT-07737 | B | A | A | A | A | B |
| GT-07738 | B | A | A | B | A | B |
| GT-07739 | B | A | A | B | A | B |
| GT-07740 | B | A | A | B | B | B |
| GT-07845 | A | A | A | B | B | B |
| GT-07846 | A | A | A | A | A | B |
| GT-07847 | A | B | A | A | A | B |
| GT-07848 | B | B | A | A | A | B |

(continued)

| Comp. No./names | Wee1 | IKZF 1 | IKZF2 | IKZF3 | GSPT1 | CK1α |
|---|---|---|---|---|---|---|
| GT-07850 | A | B | A | B | A | B |
| GT-08204 | A | B | B | B | B | B |
| GT-08202 | A | A | A | A | A | B |
| GT-08203 | A | B | A | A | A | B |
| GT-08207 | A | A | B | B | A | B |
| GT-08208 | A | B | A | B | A | B |
| GT-08209 | A | B | B | B | A | B |
| GT-08205 | A | B | A | A | A | B |
| GT-08210 | A | A | A | A | A | B |
| GT-08206 | A | A | A | A | A | A |
| GT-08258 | A | B | B | B | A | B |
| GT-08259 | A | B | B | B | A | B |
| GT-08260 | A | B | B | B | A | B |
| GT-08304 | A | A | B | B | B | B |
| GT-08262 | A | B | B | B | A | A |
| GT-08263 | A | B | B | B | A | A |
| GT-08264 | A | B | B | A | A | A |
| GT-08265 | A | B | B | B | A | B |
| GT-08266 | A | B | B | B | A | B |
| GT-08267 | A | B | B | B | A | B |
| GT-08268 | A | B | B | B | A | B |
| GT-08269 | A | B | B | B | A | B |
| GT-08270 | A | B | B | B | A | B |
| GT-08301 | A | B | B | B | A | A |
| GT-08271 | A | B | B | B | A | A |
| GT-08291 | A | B | B | B | A | A |
| GT-08292 | A | B | B | B | A | A |
| GT-08302 | A | B | B | B | B | B |
| GT-08293 | A | B | B | B | B | B |
| GT-08294 | B | B | B | B | B | A |
| GT-08298 | A | B | B | B | B | B |
| GT-08300 | B | B | A | B | B | B |
| GT-08191 | B | B | B | B | A | B |
| GT-08193 | A | B | A | B | A | B |
| GT-08231 | A | B | A | B | A | B |
| GT-08232 | A | B | A | B | A | B |
| GT-08235 | B | B | B | B | A | B |
| GT-08236 | B | B | B | B | A | B |
| GT-08237 | B | B | B | B | A | B |

(continued)

| Comp. No./names | Wee1 | IKZF 1 | IKZF2 | IKZF3 | GSPT1 | CK1α |
|---|---|---|---|---|---|---|
| GT-08238 | B | B | B | B | A | B |
| GT-08240 | B | B | B | B | A | B |
| GT-08241 | B | B | B | B | A | B |
| GT-08242 | B | B | B | B | A | B |
| GT-08364 | A | B | B | B | A | B |
| GT-08365 | B | B | B | B | A | B |
| GT-08366 | A | B | B | B | B | B |
| GT-08457 | A | B | B | B | B | B |
| GT-08458 | A | B | B | B | B | B |
| GT-08459 | A | B | B | B | B | B |
| GT-08460 | A | B | B | A | B | B |
| GT-08461 | A | B | B | A | B | B |
| GT-08462 | A | B | B | B | B | B |
| GT-08463 | A | B | B | B | A | B |
| GT-08464 | A | B | B | B | A | B |
| GT-08465 | B | B | B | B | A | B |
| GT-08466 | B | B | A | B | A | B |
| GT-08467 | B | B | A | B | A | B |
| GT-08468 | B | B | B | B | A | B |
| GT-08469 | B | B | B | B | A | B |
| GT-08470 | B | B | B | B | A | B |
| GT-08471 | B | B | B | B | A | B |
| GT-08472 | B | B | B | B | A | B |
| GT-08474 | B | B | B | B | A | B |
| GT-08475 | B | B | B | B | A | B |
| GT-08476 | B | B | B | A | B | B |
| GT-08477 | B | B | B | A | A | B |
| GT-08478 | B | B | B | B | A | B |
| GT-08479 | B | B | B | B | A | B |
| GT-08480 | B | B | B | B | A | B |
| GT-08481 | B | B | B | B | A | B |
| GT-08482 | A | B | B | B | A | B |
| GT-08488 | B | B | B | B | A | B |
| GT-08489 | A | B | A | A | A | B |
| GT-08490 | A | B | A | A | A | B |
| GT-08491 | A | B | B | B | A | B |
| GT-08492 | A | B | B | B | B | B |
| GT-08493 | B | B | A | B | A | B |
| GT-08494 | A | A | A | B | B | B |

(continued)

| Comp. No./names | Wee1 | IKZF 1 | IKZF2 | IKZF3 | GSPT1 | CK1α |
|---|---|---|---|---|---|---|
| GT-08495 | A | A | A | B | A | B |
| GT-08496 | A | A | A | B | A | B |
| GT-08497 | A | A | A | B | A | B |
| GT-08498 | A | B | A | B | A | B |
| GT-08499 | A | B | A | B | A | B |
| GT-08500 | A | B | B | B | A | B |
| GT-08501 | A | A | A | B | A | B |
| GT-08502 | A | B | A | B | A | B |
| GT-08503 | A | B | B | B | A | B |
| GT-08504 | A | B | B | A | A | B |
| GT-08505 | A | B | B | A | B | B |
| GT-08506 | A | B | A | A | A | B |
| GT-08507 | A | B | B | A | B | B |
| GT-08592 | A | B | B | B | B | B |
| GT-08593 | A | B | B | B | B | B |
| GT-08594 | A | B | A | B | B | B |
| GT-08595 | A | B | A | B | B | B |
| GT-08596 | A | B | A | B | B | B |
| GT-08598 | A | B | B | B | A | B |
| GT-08599 | A | B | B | B | B | B |
| GT-08600 | B | B | B | B | A | B |
| GT-08601 | B | B | B | B | A | B |
| GT-08602 | A | B | B | B | A | B |
| GT-08603 | B | B | B | B | A | B |
| GT-08604 | B | B | B | B | A | B |
| GT-08611 | B | B | B | B | A | B |
| GT-08612 | B | B | B | B | A | B |
| GT-08613 | B | B | B | B | A | B |
| GT-08615 | B | B | B | B | A | B |
| GT-08616 | B | B | B | B | A | B |
| GT-08617 | B | B | B | B | A | B |
| GT-08618 | B | B | B | B | A | B |
| GT-08619 | B | B | B | B | A | B |
| GT-08620 | B | B | A | B | A | B |
| GT-08621 | B | B | A | B | A | B |
| GT-08622 | B | B | A | B | A | B |
| GT-08623 | B | B | A | B | B | B |
| GT-08624 | B | B | B | B | A | B |
| GT-08625 | B | B | B | B | A | A |

(continued)

| Comp. No./names | Wee1 | IKZF 1 | IKZF2 | IKZF3 | GSPT1 | CK1α |
|---|---|---|---|---|---|---|
| GT-08626 | B | B | B | B | A | B |
| GT-08627 | B | B | B | B | A | B |
| GT-08628 | B | B | B | B | A | B |
| GT-08629 | A | B | B | B | B | A |
| GT-08630 | A | B | B | B | A | B |
| GT-08631 | A | B | B | B | B | B |
| GT-08632 | A | B | B | B | B | B |
| GT-08633 | A | B | B | B | B | B |
| GT-08634 | A | B | B | B | B | B |
| GT-08635 | A | B | B | B | B | B |
| GT-08691 | A | B | B | B | B | B |
| GT-08692 | A | B | B | B | B | B |
| GT-08693 | A | B | B | B | B | B |
| GT-08694 | A | B | B | B | A | B |
| GT-08695 | A | B | B | B | B | B |
| GT-08696 | A | B | B | B | B | B |
| GT-08697 | A | B | B | B | B | B |
| GT-08698 | A | B | B | B | A | B |
| GT-08699 | A | B | B | B | B | B |
| GT-08701 | A | B | B | B | B | B |
| GT-08702 | A | B | B | B | B | B |
| GT-08703 | A | B | B | B | B | B |
| GT-08704 | A | B | A | A | B | B |
| GT-08705 | A | B | B | B | B | B |
| GT-08706 | A | B | A | A | A | B |
| GT-08723 | A | B | A | A | A | B |
| GT-08724 | A | B | B | B | B | B |
| GT-08725 | A | B | B | B | A | B |
| GT-08726 | A | B | A | A | A | B |
| GT-08728 | A | B | B | B | B | B |
| GT-08729 | A | B | B | B | B | B |
| GT-08730 | A | B | B | B | B | B |
| GT-08731 | A | B | B | B | A | B |
| GT-08732 | A | B | B | B | B | B |
| GT-08733 | A | B | B | B | A | B |
| GT-08734 | A | B | B | B | A | B |
| GT-08735 | A | B | B | B | B | B |
| GT-08801 | A | B | B | B | B | B |
| GT-08802 | A | B | B | B | B | B |

(continued)

| Comp. No./names | Wee1 | IKZF 1 | IKZF2 | IKZF3 | GSPT1 | CK1α |
|---|---|---|---|---|---|---|
| GT-08803 | A | B | B | B | B | B |
| GT-08804 | A | B | B | B | B | B |
| GT-08805 | A | B | B | B | B | B |
| GT-08806 | A | B | B | B | B | B |
| GT-08807 | A | B | B | B | B | B |
| GT-08808 | A | B | B | B | B | B |
| GT-08809 | A | A | A | A | A | B |
| GT-08810 | A | B | B | B | B | B |
| GT-08811 | A | A | A | A | A | A |
| GT-08812 | A | B | B | B | B | B |
| GT-08813 | A | B | B | B | B | B |
| GT-08814 | B | B | A | B | B | B |
| GT-08815 | A | B | B | B | B | B |
| GT-08817 | A | B | B | B | B | B |
| GT-08818 | A | B | B | B | A | B |
| GT-09214 | B | B | B | B | A | B |
| GT-09215 | B | B | B | B | A | B |
| GT-09216 | B | B | B | B | A | B |
| GT-09217 | B | B | B | B | A | B |
| GT-09218 | B | B | B | B | A | B |
| GT-09219 | B | B | A | B | A | B |
| GT-09220 | A | A | A | B | B | B |
| GT-09221 | A | A | B | B | B | B |
| GT-09222 | A | A | B | B | B | B |
| GT-09223 | A | B | B | B | B | A |
| GT-09224 | B | B | B | A | A | A |
| GT-09225 | B | B | B | A | A | A |
| GT-09227 | B | B | B | B | A | A |
| GT-09228 | B | B | B | B | B | A |
| GT-09230 | B | B | B | B | A | A |
| GT-09231 | B | A | A | A | A | A |
| GT-09232 | A | A | A | A | A | A |
| GT-09233 | A | A | A | A | A | A |
| GT-09234 | A | A | A | A | A | A |
| GT-09235 | B | B | A | A | A | A |
| GT-09238 | B | B | A | A | A | A |
| GT-08838 | A | B | B | B | A | B |
| GT-08839 | A | B | B | B | B | B |
| GT-08840 | A | B | B | B | B | B |

(continued)

| Comp. No./names | Wee1 | IKZF 1 | IKZF2 | IKZF3 | GSPT1 | CK1α |
|---|---|---|---|---|---|---|
| GT-08841 | A | B | B | B | A | B |
| GT-08842 | A | B | B | B | B | B |
| GT-08843 | A | B | B | B | B | B |
| GT-08844 | A | B | B | B | B | B |
| GT-08845 | A | B | B | B | A | B |
| GT-08848 | A | B | B | B | B | B |
| GT-08849 | A | B | B | B | A | B |
| GT-08850 | B | B | B | B | A | B |
| GT-08851 | A | B | B | B | A | B |
| GT-08852 | A | B | B | B | B | B |
| GT-08853 | B | B | B | A | B | B |
| GT-08855 | A | B | B | B | A | B |
| GT-08857 | A | B | B | A | B | B |
| GT-08858 | A | B | B | B | B | B |
| GT-08859 | B | B | B | B | A | B |
| GT-08860 | A | B | B | B | A | B |
| GT-08861 | A | B | B | B | B | B |
| GT-08912 | A | B | B | B | B | B |
| GT-08913 | A | B | B | B | B | B |
| GT-08914 | A | B | B | B | B | B |
| GT-08915 | A | B | B | B | B | B |
| GT-08916 | A | B | B | B | A | B |
| GT-08917 | A | B | B | B | A | B |
| GT-08918 | A | B | B | B | B | B |
| GT-08919 | A | B | B | B | B | B |
| GT-08922 | A | B | B | B | B | B |
| GT-08923 | A | B | B | B | B | B |
| GT-08924 | A | B | A | B | B | B |
| GT-08925 | A | B | A | A | B | B |
| GT-08926 | A | B | B | B | B | B |
| GT-08927 | A | B | A | A | B | B |
| GT-08928 | A | B | B | B | B | B |
| GT-08929 | A | B | B | B | B | B |
| GT-08930 | A | B | B | B | B | B |
| GT-08932 | A | B | B | B | B | B |
| GT-08933 | A | A | B | B | A | B |
| GT-08934 | A | A | B | A | A | B |
| GT-08935 | A | A | B | A | A | B |
| GT-08936 | A | A | B | B | A | B |

(continued)

| Comp. No./names | Wee1 | IKZF 1 | IKZF2 | IKZF3 | GSPT1 | CK1α |
|---|---|---|---|---|---|---|
| GT-08937 | A | B | B | B | A | B |
| GT-08938 | A | B | B | B | B | B |
| GT-08947 | A | B | B | B | B | B |
| GT-08954 | A | B | B | B | A | B |
| GT-08955 | A | B | B | B | B | B |
| GT-08956 | A | B | B | B | A | B |
| GT-08957 | A | B | B | B | B | B |
| GT-08958 | A | B | B | B | B | B |
| GT-08959 | A | B | B | B | B | B |
| GT-08961 | A | B | B | B | B | B |
| GT-08962 | A | B | B | B | A | B |
| GT-08963 | A | B | B | B | B | B |
| GT-08964 | A | B | B | B | B | B |
| GT-08966 | B | B | B | B | A | B |
| GT-08968 | A | B | B | B | B | B |
| GT-08970 | A | B | B | B | B | B |
| GT-08971 | A | B | B | B | B | B |
| GT-09241 | B | B | B | B | A | B |
| GT-09240 | B | A | B | B | A | B |
| GT-09244 | B | B | B | B | A | B |
| GT-09245 | B | A | A | B | A | B |
| GT-09252 | B | B | A | B | B | B |
| GT-09259 | B | B | B | B | A | B |
| GT-09260 | B | B | B | B | A | B |
| GT-09261 | B | B | B | B | A | B |
| GT-09262 | B | B | B | B | A | B |
| GT-09263 | B | B | B | B | A | B |
| GT-09264 | B | B | B | B | A | B |
| GT-09266 | B | B | B | B | A | B |
| GT-09267 | A | A | A | A | A | B |
| GT-09268 | B | B | B | B | A | B |
| GT-09269 | B | B | B | B | A | B |
| GT-09270 | A | A | B | B | B | A |
| GT-09275 | B | B | B | B | A | B |
| GT-09276 | A | B | A | A | A | A |
| GT-09032 | A | B | B | B | B | B |
| GT-09033 | A | B | B | B | B | B |
| GT-05563 | A | A | A | A | B | B |
| GT-05566 | A | A | A | A | B | B |

(continued)

| Comp. No./names | Wee1 | IKZF 1 | IKZF2 | IKZF3 | GSPT1 | CK1α |
|---|---|---|---|---|---|---|
| GT-06977 | A | B | B | A | B | B |
| GT-04997 | A | B | B | B | B | B |
| GT-05031 | B | B | B | A | A | B |
| GT-05032 | B | B | B | A | A | B |
| GT-05033 | B | B | B | A | A | B |
| GT-05712 | B | A | B | A | A | B |
| GT-05957 | B | A | A | A | A | B |
| GT-08160 | A | B | A | A | A | B |
| GT-08161 | A | B | A | A | A | B |
| GT-08162 | A | B | B | B | B | B |
| GT-08163 | A | A | B | B | A | B |
| GT-08164 | A | A | A | A | A | B |
| GT-08166 | A | B | A | A | A | B |
| GT-08167 | A | B | A | A | A | B |
| GT-08168 | A | B | B | B | A | B |
| GT-08169 | A | B | B | B | A | B |
| GT-08170 | B | B | A | A | A | A |
| GT-08171 | A | A | A | A | A | A |
| GT-08172 | A | A | A | A | A | A |
| GT-08313 | A | B | A | B | B | B |
| GT-08305 | B | B | A | B | B | B |
| GT-08306 | A | B | B | B | B | B |
| GT-08307 | A | B | B | B | B | B |
| GT-08308 | A | B | B | B | B | B |
| GT-08309 | A | B | B | B | B | B |
| GT-08310 | A | B | B | B | B | B |
| GT-08311 | A | B | B | B | B | B |
| GT-08312 | A | B | B | B | B | B |
| GT-08314 | A | B | B | B | B | B |
| GT-08315 | A | B | B | B | B | B |
| GT-08316 | A | B | B | B | B | B |
| GT-08317 | A | B | A | A | B | B |
| GT-08318 | A | B | B | A | B | B |
| GT-08319 | A | B | B | A | B | B |
| GT-08320 | A | B | B | A | B | B |
| GT-08321 | A | B | B | A | B | B |
| GT-08322 | A | B | A | B | A | A |
| GT-08323 | B | B | B | B | A | A |
| GT-08324 | A | A | A | B | A | A |

(continued)

| Comp. No./names | Wee1 | IKZF 1 | IKZF2 | IKZF3 | GSPT1 | CK1α |
|---|---|---|---|---|---|---|
| GT-08325 | A | B | B | B | A | A |
| GT-08326 | B | B | A | B | A | A |
| GT-08327 | A | A | A | A | A | A |
| GT-08328 | A | B | B | B | A | A |
| GT-08329 | A | B | B | B | A | A |
| GT-08330 | A | A | A | A | A | A |
| GT-08331 | A | A | A | B | A | A |
| GT-08332 | A | A | A | B | A | A |
| GT-08333 | A | B | B | B | A | A |
| GT-09278 | A | A | B | A | B | B |
| GT-09299 | B | B | A | A | B | B |
| GT-09300 | B | A | A | A | B | B |
| GT-09301 | B | A | A | A | B | B |
| GT-09307 | A | A | B | A | A | A |
| GT-09309 | B | B | B | B | A | B |
| GT-09310 | B | B | B | A | A | B |
| GT-09311 | A | A | B | A | A | B |
| GT-09312 | B | B | B | A | A | B |
| GT-09313 | B | A | B | B | A | B |
| GT-09314 | A | A | B | A | A | B |
| GT-09315 | B | A | B | A | A | B |
| GT-09316 | B | A | B | A | A | B |
| GT-09317 | A | A | A | A | A | B |
| GT-09318 | A | B | B | A | A | B |
| GT-09319 | A | A | A | A | A | B |
| GT-09320 | A | B | B | B | A | B |
| GT-09321 | A | B | B | B | A | B |
| GT-09322 | A | A | B | B | A | B |
| GT-09324 | A | B | B | B | A | B |
| GT-09325 | A | B | B | B | A | B |
| GT-09326 | A | B | B | B | A | B |
| GT-09327 | A | B | A | B | A | B |
| GT-09328 | B | B | A | B | B | B |
| GT-09329 | B | B | A | B | A | B |
| GT-09331 | B | B | B | B | A | B |
| GT-09333 | B | A | B | A | A | B |
| GT-09365 | B | A | B | B | A | A |
| Note: In Table II-1, "A" is defined as 0% ≤ substrate protein remaining % ≤ 80%, and "B" is defined as 80%< substrate protein remaining %≤ 100%. | | | | | | |

The degradation effects of the compounds of the present invention on the target substrate proteins were shown in Table II-1 and Figure 1. From the experimental results in Table II-1 and Figure 1, it can be concluded that the compounds of the present invention significantly degrade the target substrate proteins (e.g., proteins WEE1, IKZF1/2/3, CK1α, GSPT1, and ZFP91 etc.). Compared to lenalidomide, the compounds of the present invention exhibited significantly enhanced efficiency in inducing substrate protein degradation and also showed selective induction of degradation, thereby potentially enabling their use in treating indications associated with these substrate proteins.

**III.** Determination of CRBN-binding affinity of compounds:

The CEREBLON BINDING kits (specification: 10,000 tests; product number: Catalog # 64BDCRBNPEH; CISBIO company) was used to determine the CRBN binding ability of the compounds to be tested through a HTRF method (homogeneous time-resolved fluorescence). The specific methods are as follows:

1. According to the instructions of the CEREBLON BINDING KITS, serial dilutions were performed for the test compounds of the present disclosure (including the compounds in Table 1 and Examples) and for lenalidomide. Specifically, diluent #9 (1X) solution was used as the diluent, and multiple concentration gradients were set. The dilution operation can be performed according to one of the following two methods:

(1) The first dilution method: The test compounds of the present disclosure (including the compounds in Table 1 and Examples) were subjected to 5-fold serial dilutions starting from a highest concentration of 40 μM, resulting in a total of 7 concentrations from high to low (40, 8, 1.6, 0.32, 0.064, 0.0128, 0.00256 μM). Subsequently, the diluted test compounds and corresponding detection reagents were added to each well of a 96-well plate, resulting in final test compound concentrations of 10, 2, 0.4, 0.08, 0.016, 0.0032, and 0.00064 μM, respectively.

(2) As an alternative, a second dilution method can be used: The test compounds of the present disclosure (including the compounds in Table 1 and Examples) were subjected to 4-fold gradient dilutions starting from a highest concentration of 8 μM, resulting in 2 concentrations (8 μM, 2 μM). Into the 96-well plate, the diluted test compounds and corresponding detection reagents were added, resulting in final test compound concentrations of 2 μM and 0.5 μM, respectively. The experimental procedure is described below using the diluted 8 μM test compound and lenalidomide solutions as examples.

2. 2.5 μL of the above 8 μM of the tested compounds and lenalidomide solution, as well as the same volume of diluent #9 (1X) solution (solvent control group, Std0) were added to each well of a 96-well plate, respectively. Then, 2.5 μL of human Cereblon WT GST-tagged protein solution was added to each well. Finally, 5 μL of the thoroughly mixed Thalidomide-Re reagent and GST Eu antibody working solution were added to each of the aforementioned wells. The final concentration of the tested compounds and lenalidomide in each well was 2 μM.
3. The blank control wells were sequentially added with 2.5 μL of diluent #9 (1X) solution, 2.5 μL of PROTAC binding buffer, and 5 μL of thoroughly mixed Thalidomide-Re reagent and GST Eu antibody working solution.
4. After sealing and incubating the solutions in the aforementioned wells at room temperature for 3 hours, the absorbance values at emission wavelengths of 620 nm and 665 nm were detected by the HTRF method using a Spark microplate reader (V3.1 SP1).

The corresponding CRBN binding inhibition rate was calculated using the following method:

$$R_{compound} = OD\ 665\ nm_{compound} / OD\ 620\ nm_{compound} - OD\ 665\ nm_{control} / OD\ 620\ nm_{control}$$

$$R_{Std0} = OD\ 665\ nm_{Std0} / OD\ 620\ nm_{Std0} - OD\ 665\ nm_{control} / OD\ 620\ nm_{control}$$

$$\text{inhibition rate (\%)} = (1 - R_{compound} / R_{Std0}) * 100$$

Note: OD 665 nm $_{compound}$ is the absorbance value of each well of the compounds to be tested of the present disclosure at an emission wavelength of 665 nm.
OD 620 nm $_{compound}$ is the absorbance value of each well of the compounds to be tested of the present disclosure at an emission wavelength of 620 nm.
OD 665 nm $_{control}$ is the absorbance value of the blank control well at an emission wavelength of 665 nm.
OD 620 nm $_{control}$ is the absorbance value of the blank control well at an emission wavelength of 620 nm.
OD 665 nm $_{Std0}$ is the absorbance value of the solvent control well at an emission wavelength of 665 nm.

OD 620 nm $_{Std0}$ is the absorbance value of the solvent control well at an emission wavelength of 620 nm.

The test results were shown in Tables III-1 and III-2 below.

Table III-1. HTRF inhibitory activity ($IC_{50}$, μM) of the compounds of the present disclosure (including but not limited to the compounds in Table 1 and the Examples compounds)

| Comp. No. | HTRF $IC_{50}$ (μM) | Comp. No. | HTRF $IC_{50}$ (μM) | Comp. No. | HTRF $IC_{50}$ (μM) | Comp. No. | HTRF $IC_{50}$ (μM) |
|---|---|---|---|---|---|---|---|
| Lenalidomide | e | GT-05093 | a1 | GT-05148 | a | GT-05563 | a1 |
| GT-04474 | b | GT-05103 | a | GT-05149 | a | GT-05566 | a |
| GT-05035 | a | GT-05104 | b | GT-05150 | b | GT-06977 | a1 |
| GT-05038 | b | GT-05106 | a | GT-05151 | b | GT-06981 | a1 |
| GT-05039 | a | GT-05109 | b | GT-05182 | a | GT-04997 | a |
| GT-05040 | a | GT-05110 | a | GT-05183 | a | GT-05031 | a |
| GT-05041 | d | GT-05111 | a | GT-05243 | a1 | GT-05032 | a |
| GT-05088 | a | GT-05112 | a | GT-05244 | a | GT-05033 | a |
| GT-05089 | a1 | GT-05113 | a1 | GT-05245 | a1 | GT-05712 | a1 |
| GT-05090 | a | GT-05145 | a1 | GT-05246 | a | GT-05957 | a |
| GT-05091 | a1 | GT-05146 | a | GT-05247 | a | | |
| GT-05092 | b | GT-05147 | a1 | GT-05248 | a1 | | |
| Note: in Table III-1, the symbols a1, a, b, c, d, and e are defined as follows: 0<a1≤0.1μM, 0.1μM<a≤0.5μM, 0.5μM<b≤1μM, 1μM<c≤1.5μM, 1.5μM<d<1.9μM, 1.9μM ≤ e ≤10μM, 10μM < f. | | | | | | | |

Table III-2: Screening of CRBN Binding Affinity of the Compounds of the Present Invention (including but not limited to the compounds in Table 1 and Examples Compounds) at Concentrations of 2 μM and 0.5 μM

| Comp. No. | Inhibition Rate at 2 μM (%) | Inhibition Rate at 0.5 μM (%) | Comp. No. | Inhibition Rate at 2 μM (%) | Inhibition Rate at 0.5 μM (%) | Comp. No. | Inhibition Rate at 2 μM (%) | Inhibition Rate at 0.5 μM (%) |
|---|---|---|---|---|---|---|---|---|
| Lenalidomide | a4 | a2 | GT-08627 | a7 | a6 | GT-08965 | a7 | a7 |
| GT-07732 | a7 | a5 | GT-08628 | a7 | a5 | GT-08966 | a7 | a7 |
| GT-07733 | a7 | a6 | GT-08629 | a6 | a4 | GT-08968 | a7 | a6 |
| GT-07734 | a7 | a6 | GT-08630 | a6 | a5 | GT-08969 | a7 | a6 |
| GT-07735 | a7 | a5 | GT-08631 | a7 | a6 | GT-08970 | a7 | a6 |
| GT-07736 | a6 | a2 | GT-08632 | a7 | a6 | GT-08971 | a7 | a7 |
| GT-07737 | a5 | a3 | GT-08634 | a7 | a6 | GT-09239 | a7 | a6 |
| GT-07738 | a7 | a5 | GT-08635 | a7 | a7 | GT-09241 | a7 | a6 |
| GT-07739 | a7 | a6 | GT-08691 | a7 | a6 | GT-09240 | a7 | a7 |
| GT-07740 | a7 | a5 | GT-08692 | a7 | a5 | GT-09354 | a7 | a6 |
| GT-07845 | a6 | a4 | GT-08693 | a7 | a6 | GT-09242 | a7 | a6 |
| GT-07846 | a7 | a5 | GT-08694 | a6 | a4 | GT-09243 | a7 | a6 |
| GT-07847 | a7 | a5 | GT-08695 | a7 | a6 | GT-09244 | a7 | a6 |
| GT-07848 | a7 | a3 | GT-08696 | a7 | a6 | GT-09245 | a7 | a6 |
| GT-07849 | a6 | a3 | GT-08697 | a7 | a6 | GT-09246 | a7 | a7 |
| GT-07850 | a7 | a4 | GT-08698 | a7 | a6 | GT-09247 | a7 | a6 |

(continued)

| Comp. No. | Inhibition Rate at 2 µM (%) | Inhibition Rate at 0.5 µM (%) | Comp. No. | Inhibition Rate at 2 µM (%) | Inhibition Rate at 0.5 µM (%) | Comp. No. | Inhibition Rate at 2 µM (%) | Inhibition Rate at 0.5 µM (%) |
|---|---|---|---|---|---|---|---|---|
| GT-08201 | a7 | a4 | GT-08699 | a7 | a5 | GT-09248 | a7 | a6 |
| GT-08204 | a7 | a4 | GT-08700 | a7 | a5 | GT-09249 | a7 | a6 |
| GT-08202 | a6 | a3 | GT-08701 | a7 | a4 | GT-09250 | a7 | a6 |
| GT-08203 | a5 | a3 | GT-08703 | a6 | a5 | GT-09251 | a7 | a6 |
| GT-08207 | a6 | a4 | GT-08704 | a7 | a4 | GT-09252 | a7 | a6 |
| GT-08208 | a7 | a4 | GT-08705 | a6 | a3 | GT-09253 | a7 | a6 |
| GT-08209 | a7 | a6 | GT-08706 | a7 | a5 | GT-09254 | a7 | a6 |
| GT-08205 | a7 | a6 | **GT-**08723 | a7 | a6 | GT-09255 | a7 | a7 |
| GT-08210 | a7 | a6 | GT-08724 | a7 | a6 | GT-09256 | a7 | a6 |
| GT-08206 | a7 | a4 | GT-08726 | a7 | a6 | GT-09257 | a7 | a6 |
| GT-08259 | a6 | a4 | GT-08727 | a7 | a5 | GT-09258 | a7 | a6 |
| GT-08260 | a7 | a6 | GT-08728 | a7 | a5 | GT-09259 | a7 | a5 |
| GT-08304 | a6 | a6 | GT-08729 | a7 | a6 | GT-09260 | a7 | a6 |
| GT-08261 | a6 | a3 | GT-08730 | a7 | a6 | GT-09261 | a7 | a5 |
| GT-08262 | a6 | a3 | GT-08731 | a7 | a6 | GT-09262 | a7 | a5 |
| GT-08263 | a7 | a5 | GT-08732 | a7 | a6 | GT-09263 | a7 | a6 |
| GT-08264 | a7 | a4 | GT-08733 | a7 | a6 | GT-09264 | a7 | a7 |
| GT-08265 | a5 | a4 | GT-08734 | a7 | a6 | GT-09266 | a7 | a6 |
| GT-08266 | a6 | a3 | GT-08735 | a7 | a6 | GT-09267 | a7 | a5 |
| GT-08268 | a6 | a4 | GT-08736 | a7 | a6 | GT-09268 | a7 | a5 |
| GT-08269 | a5 | a3 | GT-08801 | a7 | a6 | GT-09269 | a7 | a7 |
| GT-08271 | a6 | a3 | GT-08802 | a7 | a5 | GT-09270 | a7 | a6 |
| GT-08291 | a6 | a2 | GT-08803 | a7 | a6 | GT-09271 | a7 | a6 |
| GT-08292 | a6 | a3 | GT-08804 | a6 | a4 | GT-09272 | a7 | a6 |
| GT-08302 | a7 | a3 | GT-08805 | a6 | a6 | GT-09273 | a7 | a6 |
| GT-08293 | a6 | a3 | GT-08806 | a7 | a5 | GT-09274 | a7 | a6 |
| GT-08294 | a7 | a3 | GT-08807 | a7 | a6 | GT-09275 | a6 | a3 |
| GT-08295 | a6 | a3 | GT-08808 | a7 | a6 | GT-09276 | a7 | a4 |
| GT-08296 | a6 | a4 | GT-08809 | a7 | a5 | GT-09279 | a7 | a6 |
| GT-08303 | a6 | a3 | GT-08810 | a7 | a6 | GT-09280 | a7 | a7 |
| GT-08297 | a7 | a6 | GT-08811 | a7 | a6 | GT-09281 | a7 | a7 |
| GT-08298 | a7 | a6 | GT-08812 | a7 | a6 | GT-09282 | a7 | a6 |
| GT-08299 | a6 | a5 | GT-08813 | a7 | a6 | GT-09284 | a7 | a4 |
| GT-08300 | a6 | a4 | GT-08814 | a7 | a6 | GT-09285 | a7 | a7 |
| GT-08191 | a6 | a3 | GT-08815 | a7 | a6 | GT-09287 | a7 | a7 |
| GT-08192 | a7 | a6 | GT-08816 | a7 | a6 | GT-09295 | a7 | a7 |
| GT-08193 | a5 | a3 | GT-08817 | a7 | a5 | GT-09296 | a7 | a6 |

(continued)

| Comp. No. | Inhibition Rate at 2 μM (%) | Inhibition Rate at 0.5 μM (%) | Comp. No. | Inhibition Rate at 2 μM (%) | Inhibition Rate at 0.5 μM (%) | Comp. No. | Inhibition Rate at 2 μM (%) | Inhibition Rate at 0.5 μM (%) |
|---|---|---|---|---|---|---|---|---|
| GT-08231 | a6 | a5 | GT-08818 | a6 | a5 | GT-09297 | a7 | a6 |
| GT-08232 | a7 | a4 | GT-09214 | a7 | a6 | GT-09032 | a7 | a4 |
| GT-08243 | a6 | a2 | GT-09215 | a7 | a6 | GT-09033 | a7 | a6 |
| GT-08233 | a7 | a5 | GT-09216 | a7 | a6 | GT-08160 | a7 | a5 |
| GT-08234 | a6 | a4 | GT-09217 | a7 | a6 | GT-08161 | a7 | a4 |
| GT-08235 | a7 | a4 | GT-09218 | a7 | a6 | GT-08162 | a6 | a3 |
| GT-08236 | a7 | a4 | GT-09219 | a7 | a6 | GT-08163 | a6 | a5 |
| GT-08237 | a7 | a5 | GT-09220 | a7 | a6 | GT-08164 | a7 | a4 |
| GT-08238 | a7 | a5 | GT-09221 | a7 | a7 | GT-08165 | a7 | a6 |
| GT-08239 | a7 | a4 | GT-09222 | a7 | a6 | GT-08166 | a6 | a2 |
| GT-08240 | a7 | a5 | GT-09223 | a7 | a6 | GT-08167 | a7 | a4 |
| GT-08241 | a7 | a4 | GT-09224 | a7 | a6 | GT-08168 | a7 | a7 |
| GT-08242 | a7 | a4 | GT-09225 | a7 | a6 | GT-08169 | a7 | a7 |
| GT-08364 | a7 | a4 | GT-09226 | a7 | a6 | GT-08170 | a7 | a6 |
| GT-08365 | a6 | a4 | GT-09227 | a7 | a6 | GT-08171 | a7 | a6 |
| GT-08366 | a7 | a6 | GT-09228 | a7 | a4 | GT-08172 | a7 | a7 |
| GT-08458 | a7 | a5 | GT-09229 | a7 | a6 | GT-08313 | a6 | a4 |
| GT-08459 | a7 | a6 | GT-09230 | a7 | a6 | GT-08305 | a7 | a6 |
| GT-08460 | a7 | a6 | GT-09231 | a7 | a6 | GT-08306 | a7 | a5 |
| GT-08461 | a7 | a6 | GT-09232 | a7 | a6 | GT-08307 | a7 | a5 |
| GT-08462 | a7 | a6 | GT-09233 | a7 | a6 | GT-08308 | a6 | a5 |
| GT-08463 | a6 | a5 | GT-09234 | a7 | a6 | GT-08309 | a7 | a5 |
| GT-08464 | a6 | a6 | GT-09235 | a7 | a6 | GT-08310 | a6 | a3 |
| GT-08465 | a6 | a4 | GT-09236 | a7 | a6 | GT-08311 | a7 | a6 |
| GT-08466 | a6 | a3 | GT-09237 | a7 | a6 | GT-08312 | a7 | a5 |
| GT-08467 | a6 | a3 | GT-09238 | a7 | a6 | GT-08314 | a6 | a4 |
| GT-08468 | a7 | a4 | **GT-**08838 | a7 | a5 | GT-08315 | a6 | a4 |
| GT-08469 | a6 | a4 | GT-08839 | a7 | a6 | GT-08316 | a6 | a4 |
| GT-08470 | a5 | a2 | GT-08840 | a7 | a6 | GT-08317 | a6 | a4 |
| GT-08471 | a6 | a3 | GT-08841 | a7 | a6 | GT-08318 | a6 | a4 |
| GT-08472 | a7 | a4 | GT-08842 | a7 | a4 | GT-08319 | a7 | a4 |
| GT-08473 | a7 | a6 | GT-08843 | a7 | a6 | GT-08320 | a6 | a4 |
| GT-08474 | a7 | a7 | GT-08844 | a6 | a4 | GT-08322 | a5 | a3 |
| GT-08475 | a7 | a6 | GT-08845 | a7 | a4 | GT-08323 | a7 | a4 |
| GT-08476 | a7 | a6 | GT-08848 | a7 | a3 | GT-08324 | a7 | a6 |
| GT-08477 | a7 | a5 | GT-08849 | a7 | a6 | GT-08325 | a7 | a6 |
| GT-08478 | a7 | a4 | GT-08850 | a7 | a6 | GT-08326 | a7 | a4 |

(continued)

| Comp. No. | Inhibition Rate at 2 μM (%) | Inhibition Rate at 0.5 μM (%) | Comp. No. | Inhibition Rate at 2 μM (%) | Inhibition Rate at 0.5 μM (%) | Comp. No. | Inhibition Rate at 2 μM (%) | Inhibition Rate at 0.5 μM (%) |
|---|---|---|---|---|---|---|---|---|
| GT-08479 | a6 | a4 | GT-08851 | a6 | a4 | GT-08327 | a7 | a5 |
| GT-08480 | a7 | a4 | GT-08852 | a7 | a4 | GT-08328 | a7 | a6 |
| GT-08481 | a7 | a6 | GT-08853 | a7 | a6 | GT-08329 | a7 | a4 |
| GT-08482 | a7 | a6 | GT-08854 | a7 | a4 | GT-08330 | a6 | a4 |
| GT-08488 | a7 | a6 | GT-08855 | a6 | a3 | GT-08331 | a7 | a4 |
| GT-08489 | a7 | a6 | GT-08856 | a5 | a3 | GT-08333 | a7 | a6 |
| GT-08490 | a7 | a6 | GT-08857 | a7 | a6 | GT-09034 | a6 | a4 |
| GT-08491 | a7 | a7 | GT-08858 | a7 | a6 | GT-09035 | a7 | a6 |
| GT-08492 | a7 | a6 | GT-08859 | a7 | a5 | GT-09036 | a7 | a6 |
| GT-08493 | a7 | a4 | GT-08860 | a7 | a6 | GT-09265 | a7 | a6 |
| GT-08494 | a7 | a6 | GT-08861 | a7 | a4 | GT-09277 | a7 | a4 |
| GT-08495 | a6 | a5 | GT-08912 | a7 | a6 | GT-09278 | a6 | a3 |
| GT-08496 | a7 | a4 | GT-08913 | a7 | a6 | GT-09299 | a7 | a5 |
| GT-08498 | a6 | a4 | GT-08914 | a7 | a6 | GT-09300 | a7 | a5 |
| GT-08499 | a5 | a3 | GT-08916 | a7 | a4 | GT-09301 | a7 | a6 |
| GT-08500 | a6 | a4 | GT-08917 | a7 | a5 | GT-09303 | a7 | a6 |
| GT-08501 | a5 | a3 | GT-08918 | a7 | a6 | GT-09304 | a7 | a6 |
| GT-08502 | a6 | a4 | GT-08919 | a6 | a2 | GT-09305 | a7 | a6 |
| GT-08503 | a6 | a4 | GT-08920 | a7 | a4 | GT-09306 | a7 | a4 |
| GT-08504 | a6 | a3 | GT-08921 | a7 | a3 | GT-09307 | a7 | a6 |
| GT-08505 | a6 | a4 | GT-08922 | a7 | a6 | GT-09309 | a7 | a6 |
| GT-08507 | a6 | a4 | GT-08923 | a7 | a3 | GT-09310 | a7 | a5 |
| GT-08592 | a6 | a4 | GT-08924 | a7 | a4 | GT-09311 | a7 | a6 |
| GT-08593 | a6 | a4 | GT-08925 | a7 | a6 | GT-09312 | a7 | a6 |
| GT-08594 | a5 | a3 | GT-08926 | a7 | a5 | GT-09313 | a7 | a6 |
| GT-08598 | a6 | a4 | GT-08927 | a7 | a6 | GT-09314 | a7 | a7 |
| GT-08599 | a6 | a3 | GT-08928 | a7 | a6 | GT-09315 | a7 | a6 |
| GT-08600 | a7 | a5 | GT-08929 | a6 | a3 | GT-09316 | a7 | a6 |
| GT-08601 | a7 | a4 | GT-08930 | a7 | a5 | GT-09317 | a7 | a4 |
| GT-08602 | a7 | a4 | GT-08931 | a7 | a4 | GT-09318 | a7 | a5 |
| GT-08603 | a7 | a5 | GT-08932 | a7 | a6 | GT-09319 | a7 | a6 |
| GT-08604 | a6 | a3 | GT-08933 | a6 | a5 | GT-09320 | a7 | a6 |
| GT-08611 | a6 | a3 | GT-08934 | a5 | a4 | GT-09321 | a7 | a5 |
| GT-08612 | a6 | a3 | GT-08935 | a7 | a6 | GT-09322 | a7 | a6 |
| GT-08613 | a7 | a6 | GT-08936 | a7 | a6 | GT-09323 | a7 | a4 |
| GT-08614 | a6 | a3 | GT-08937 | a7 | a6 | GT-09324 | a7 | a6 |
| GT-08615 | a6 | a3 | GT-08947 | a7 | a6 | GT-09325 | a7 | a6 |

(continued)

| Comp. No. | Inhibition Rate at 2 $\mu$M (%) | Inhibition Rate at 0.5 $\mu$M (%) | Comp. No. | Inhibition Rate at 2 $\mu$M (%) | Inhibition Rate at 0.5 $\mu$M (%) | Comp. No. | Inhibition Rate at 2 $\mu$M (%) | Inhibition Rate at 0.5 $\mu$M (%) |
|---|---|---|---|---|---|---|---|---|
| GT-08616 | a7 | a5 | GT-08954 | a6 | a4 | GT-09326 | a7 | a5 |
| GT-08617 | a7 | a4 | GT-08955 | a6 | a4 | GT-09327 | a7 | a6 |
| GT-08618 | a6 | a3 | GT-08956 | a6 | a5 | GT-09328 | a7 | a5 |
| GT-08619 | a7 | a4 | GT-08957 | a7 | a6 | GT-09329 | a7 | a6 |
| GT-08620 | a7 | a5 | GT-08958 | a7 | a6 | GT-09330 | a7 | a6 |
| GT-08621 | a7 | a5 | GT-08959 | a7 | a6 | GT-09331 | a7 | a7 |
| GT-08622 | a7 | a4 | GT-08960 | a7 | a5 | GT-09333 | a7 | a6 |
| GT-08623 | a7 | a4 | GT-08961 | a7 | a4 | GT-09365 | a7 | a6 |
| GT-08624 | a7 | a3 | GT-08962 | a7 | a4 | GT-09308 | a7 | a4 |
| GT-08625 | a7 | a6 | GT-08963 | a7 | a6 | | | |
| GT-08626 | a7 | a6 | GT-08964 | a7 | a5 | | | |
| Note: in table III-2, the symbols a1, a2, a3, a4, a5, a6, and a7 are defined as follows: 0% < a ≤ 10%, 10% < a1 ≤ 20%, 20% < a2 ≤ 30%, 30% < a3 ≤ 40%, 40% < a4 ≤ 53%, 53% < a5 ≤ 60%, 60% < a6 ≤ 80%, 80% < a7 ≤100%. | | | | | | | | |

The results in Table III-1 and Table III-2 demonstrate that the compounds of the present invention (including but not limited to the compounds in Table 1 and Examples) exhibit lower or comparable $IC_{50}$ values, or higher inhibition rates compared to lenalidomide, indicating stronger binding affinity to CRBN.

**IV. TNF-$\alpha$ Activity Inhibition Assay**

PBMCs cells were cultured at 37°C with 5% $CO_2$ and seeded in 96-well plates at $1\times10^7$ cells/well. The compounds to be tested (including those listed in Table 1 and Examples compounds) were dissolved in DMSO and diluted to appropriate concentrations, ensuring that the final DMSO concentration in cell culture did not exceed 0.5%. After 1-hour incubation in medium with or without the compounds, the cells were stimulated with Lipopolysaccharide (LPS, 1 ng/ml) and continued to be cultured for 18-20 hours. Culture supernatants were then collected, diluted with serum-free medium, and analyzed for TNF-$\alpha$ levels using an ELISA kit. $IC_{50}$ values were calculated using GraphPad Prism 7.0.

TNF-$\alpha$ downregulation is a key mechanism for the anti-tumor action of immunomodulators. The compounds of the present disclosure demonstrated dose-dependent inhibition of TNF-$\alpha$ levels.

**V. Pharmacokinetic Study of the compounds of the present disclosure**

The pharmacokinetic properties of the compounds of the present disclosure were evaluated using conventional pharmacokinetic experimental methods.

The test compounds of the present disclosure (including the compounds in Table 1 and the Examples compounds) were orally administered to experimental animals to evaluate their pharmacokinetic properties. The experimental animals used may be those commonly used in pharmacokinetic experiments, e.g., adult and healthy rodents (e.g., mice, rats (such as Sprague-Dawley (SD) rats), guinea pigs) or non-rodents (rabbits, dogs, and monkeys). The experimental animals used in this study were mice.

The experimental animals were divided into two groups: a control group (for collecting blank plasma) and an oral administration group (administered at a dose of 10 mg/kg). A test solution of the compound of the present disclosure (concentration: 1 mg/mL) was prepared by adding an appropriate amount of the test compound to a conventional vehicle (e.g., 5% DMSO + 10% Solutol + 85% saline). The solution was orally administered to the experimental animals at a dose of 10 mg/kg. Blood samples were collected at predetermined time points after administration, such as 0.25 h, 0.5 h, 1 h, 2 h, 4 h, 8 h, and 24 h post-dose. Blank plasma was collected from the control group.

Prior to analysis, plasma samples were processed as follows: To 10 $\mu$L of plasma sample were added 10 $\mu$L of 50% acetonitrile and 200 $\mu$L of internal standard solution (containing 50 ng/mL dexamethasone in acetonitrile). For blank control plasma samples, no internal standard was added, instead an equivalent volume of acetonitrile was supplemented.

The processed samples were vortex-mixed, centrifuged, and the supernatant was subjected to LC-MS/MS analysis.

After preparation of calibration curves and QC samples, the concentrations of compounds in plasma samples were determined by LC-MS/MS analysis. The pharmacokinetic parameters of the test compounds were calculated using the pharmacokinetic calculation software WinNonlin v8.1 with non-compartmental analysis based on the plasma concentration data obtained from LC-MS/MS.

The results demonstrated that the compounds of the present disclosure administered via oral route exhibited favorable drug metabolism and pharmacokinetic (DMPK) properties, indicating their potential as therapeutic agents for cancer patients.

## VI. Effects of the compounds of the present disclosure on TNF-α, IL-10, IL-6, IL-1β, IFN-γ, GM-CSF, IL-2, and IL-12p40 release from PBMCs induced by Lipopolysaccharide (LPS) or Phytohemagglutinin (PHA)

The following materials were used in this study:

VI.1 Reagents and Consumable materials

| Name Supplier or Source |
|---|
| Human PBMC cell Shanghai Sai Li |
| Lipopolysaccharide (LPS) Sigma |
| Phytohemagglutinin (PHA) MCE |
| RPMI 1640 Medium Gibco |
| Human TNF-alpha DuoSet ELISA R&D Systems |
| Human IL-6 DuoSet ELISA R&D Systems |
| Human IL-10 DuoSet ELISA R&D Systems |
| Human IFN-gamma DuoSet ELISA R&D Systems |
| Human IL-2 DuoSet ELISA R&D Systems |
| Human IL-1 beta ELISA Kit Abclonal |
| Human GM-CSF ELISA Kit Abclonal |
| Human IL-12/IL-23 p40 ELISA Kit Abclonal |

VI.2 Methods

VI.2.1 Cell Thawing and Plating

A complete medium was prepared and pre-warmed to 37°C. Cryovials containing the cells were retrieved from liquid nitrogen, and the area immediately below the cap-notch interface was firmly gripped with forceps. The cryovials were immersed in a 37°C water bath, and shook occasionally to facilitate thawing. The cell suspension was aspirated and transferred to a centrifuge tube containing 10 mL complete medium, followed by thorough mixing and centrifugation at 400 × g for 10 min at room temperature. The supernatant was discarded, and the pellet was resuspended in fresh complete medium. After cell counting and the cell density adjustment, the cell suspension was seeded in 96-well plates at a density of $1\times10^5$ cells/well (75 μL/well) and incubated in an incubator for 2 h at 37°C with 5% $CO_2$ under high humidity conditions.

VI.2.2 Compound Preparation and Addition to Cell Plates

1) The test compounds were prepared as 10 mM stock solutions in DMSO.
2) The test compounds were serially diluted 5-fold in DMSO starting from 1 mM (highest concentration) to generate 9 concentration gradients.
3) The test compounds were further diluted in culture medium to five times their final working concentration intended for use.
4) The diluted compounds were added to designated wells according to the plate layout at 25 μL/well, achieving final concentrations starting from 1 μM with 5-fold serial dilution (9 concentration points in total).

5) After 1 h incubation, LPS or PHA (25 μL/well) was added to the cell wells to reach final concentrations of: 1 μg/mL LPS (for TNF-α, IL-10, IL-6, GM-CSF and IL-12p40), 5 μg/mL LPS (IL-1β), 50 μg/mL LPS (IFN-γ), or 1 μg/mL PHA (IL-2).

6) The plates were incubated in an incubator for 24 h at 37°C with 5% $CO_2$ under high humidity.

Cell plate layout:

<table>
<tr><td></td><td>1</td><td>2</td><td>3</td><td>4</td><td>5</td><td>6</td><td>7</td><td>8</td><td>9</td><td>10</td><td>11</td><td>12</td></tr>
<tr><td>A</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td></tr>
<tr><td>B</td><td>$H_2O$</td><td colspan="9" rowspan="2">Cmpd 1# (1 μM Starting, 5-fold dilution, 9 points)</td><td rowspan="4">LPS/PHA</td><td>$H_2O$</td></tr>
<tr><td>C</td><td>$H_2O$</td><td>$H_2O$</td></tr>
<tr><td>D</td><td>$H_2O$</td><td colspan="9" rowspan="2">Cmpd 2# (1 μM Starting, 5-fold dilution, 9 points)</td><td>$H_2O$</td></tr>
<tr><td>E</td><td>$H_2O$</td><td>$H_2O$</td></tr>
<tr><td>F</td><td>$H_2O$</td><td colspan="9" rowspan="2">Cmpd 3# (1 μM Starting, 5-fold dilution, 9 points)</td><td>Blank</td><td>$H_2O$</td></tr>
<tr><td>G</td><td>$H_2O$</td><td></td><td>$H_2O$</td></tr>
<tr><td>H</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td><td>$H_2O$</td></tr>
</table>

VI.2.3 Detection

(1) The cell plate was centrifuged and the culture supernatant was aspirated. TNF-α, IL-10, IL-6, IL-1β, IFN-γ, GM-CSF, IL-2, and IL-12p40 cytokines in the culture supernatant were detected according to the ELISA kit instructions. The concentrations of TNF-α, IL-10, IL-6, IL-1β, IFN-γ, GM-CSF, IL-2, and IL-12p40 in the samples were calculated based on the standard curve. Inhibition rate % = [(Ac-As)/(Ac-Ab)]×100%

As: OA of the samples (cells + LPS/PHA + test compound)
Ac: OA of positive cell control (cells + LPS/PHA + DMSO)
Ab: OA of blank control (cells + culture medium + DMSO)

(2) $IC_{50}$ value was calculated as follows: using GraphPad Prism 6 software and applying the calculation formula XY-analysis/Nonlinear regression (curve fit)/Dose response-Inhibition/log (inhibitor) vs. response-Variable slope (four parameters) to perform $IC_{50}$ curve fitting and determine the $IC_{50}$ values.

The experimental results demonstrated that the compounds of the present disclosure can modulate the production levels of cytokines associated with autoimmune diseases and can be used for the treatment of autoimmune diseases.

**VII. Efficacy experiment of the compounds of the present disclosure on Psoriasis-like skin lesions induced by Imiquimod in Mice**

The compounds of the present disclosure (test compounds, including the compounds in Table 1 and Examples) were administered orally via gavage once daily for 7 consecutive days to mice with psoriasis-like skin lesions induced by imiquimod, to investigate their efficacy on such lesions.

A total of 60 female C57BL/6J mice (purchased from Shanghai SLAC Laboratory Animal Co., Ltd.) were randomly divided into 6 groups (n=10 per group) based on body weight:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 7 days |
| 2 | Model: Vehicle | 10 | NA | 10 | NA | ig. qd. 7 days |
| 3 | Positive Control | 10 | 3 | 10 | 0.3 | sc. qd. 7 days |
| 4 | Low-dose test compounds group | 10 | 3 | 10 | 0.3 | ig. qd. 7 days |
| 5 | Medium-dose test compounds group | 10 | 10 | 10 | 1 | ig. qd. 7 days |
| 6 | High-dose test compounds group | 10 | 30 | 10 | 3 | ig. qd. 7 days |
| Note: ig., oral gavage; sc., subcutaneous injection; qd., once daily. | | | | | | |

After grouping the experimental animals, the backs of all mice were shaved. Except for the Control group, the remaining groups were topically treated with imiquimod cream once daily for 7 consecutive days. Simultaneously, each group was administered with a vehicle control, a positive control drug, or the test compounds, according to the above table, once daily for 7 consecutive days.

All clinical symptoms of each animal were observed at the beginning and throughout the experiment. The animals' weights were measured and recorded every 2 days.

Scoring

Gross Observation Scoring of Skin Lesions (PASI Method): during the last 3 days of modeling and drug administration, the changes in skin lesions of mice in each group were observed once daily. PASI Method: the erythema, scaling, and thickening/infiltration of the lesions were scored separately, and the scores were summed to obtain the total score. PASI Scoring Criteria: 0 = none; 1 = mild; 2 = moderate; 3 = severe; 4 = very severe.

The skin lesions were photographed once daily during the last 3 days of modeling and drug administration.

After the experiment, the mice were euthanized by $CO_2$ inhalation. Three different areas of skin from the lesion site on the back were collected using a 6 mm punch, weighed, and recorded, and the average weight was calculated.

The skin tissue from the lesion site was homogenized and the levels of IL-23p19, IL-12p40, IL-17, and **TNF-$\alpha$** were determined.

The local lesion tissue was fixed, stained with HE, and subjected to pathological scoring (based on epidermal thickness, degree of epidermal keratinization, thickness of the basal layer, and degree of dermal inflammatory cell infiltration). Scoring Criteria: 0 = none; 1 = mild; 2 = moderate; 3 = severe; 4 = very severe.

Experimental data were presented as Mean $\pm$ SD. Statistical analysis between two groups was performed using SPSS, with $p < 0.05$ considered statistically significant.

The experimental results demonstrated that the compounds of the present disclosure exhibited ameliorative effects on psoriasis-like skin lesions on the back of mice induced by imiquimod and can be used for the treatment of psoriasis.

**VIII. Efficacy Experiment of the compounds of the present disclosure on the CIA Model in Rats Induced by Type II Collagen**

The compounds of the present disclosure (test compounds, including the compounds in Tables 1, 2, and Examples) were administered orally via gavage once daily for 21 consecutive days to Wistar rats with collagen-induced arthritis (CIA) that was induced using bovine type II collagen (Chondrex, Inc.), in order to investigate their efficacy in the rat arthritis model.

Preparation of the Modeling Agent:

10 mg of CII (Immunization Grade Bovine Type II Collagen, purchased from Chondrex, Inc.) was dissolved in 5 mL of 0.05M acetic acid solution, allowing it to be fully dissolved, thereby preparing a solution with a concentration of 2 mg/mL, which was then stored at 4°C in the dark overnight. On the day of the experiment, the 2 mg/mL CII solution was mixed with an equal volume of a 4 mg/mL solution of Complete Freund's Adjuvant (CFA, also purchased from Chondrex, Inc.) on ice, and was thoroughly emulsified into an emulsion.

On day 0, 10 Wistar rats (purchased from Beijing Vital River Laboratory Animal Technology Co., Ltd.) were randomly selected as the blank control group and were not immunized. The remaining 100 Wistar rats were intradermally injected at the base of the tail with an emulsion prepared by mixing equal volumes of CII (2 mg/mL) and CFA (4 mg/mL) for the first immunization. A second booster immunization was administered to them on day 7. Between days 10 and 14, i.e., 3 to 7 days after the second booster immunization, 50 rats with an arthritis index (AI) score ranging from 1 to 2 were selected from the modeled animals and were divided into 5 groups based on their body weight, foot volume, and AI score, with 10 rats per group:

| Group | Drug | Number of Animals | Dose (mg/kg) | Administration volume (mL/kg) | Administration concentration (mg/mL) | Route and Duration |
|---|---|---|---|---|---|---|
| 1 | Control: Vehicle | 10 | NA | 10 | NA | ig. qd. 21 days |
| 2 | Model: Vehicle | 10 | NA | 10 | NA | ig. qd. 21 days |
| 3 | Positive Control | 10 | 0.1 | 10 | 0.01 | sc. qd. 21 days |
| 4 | Low-dose test compounds group | 10 | 3 | 10 | 0.3 | ig. qd. 21 days |
| 5 | Medium-dose test compounds group | 10 | 10 | 10 | 1 | ig. qd. 21 days |
| 6 | High-dose test compounds group | 10 | 30 | 10 | 3 | ig. qd. 21 days |
| Note: ig. = oral gavage; sc. = subcutaneous injection | | | | | | |

Administration commenced between 3 and 7 days after the second booster immunization, once the AI score of the affected paws reached 1-2. According to the table above, animals in each group received daily oral gavage (or subcutaneous injection) of either the vehicle control, positive control drug, or the test compounds for 21 consecutive days.

Beginning on day 3 after the second booster immunization, the volume of both hind paws (or toes) was measured and recorded twice weekly.

Foot Volume Measurement: Before the measurement, a line was marked on the rat's ankle joint with a marker pen to indicate the position. After clean water was added to the instrument, the instrument's reading was reset to zero in preparation for the measurement. The hind limb of the rat was placed in the water so that the marked line at the ankle joint was at the surface of the liquid. The reading obtained by pressing the foot pedal at this time was the foot volume of the rat. After the measurement was completed, the foot pedal was pressed again to reset the instrument to zero, preparing it for the measurement of the next rat.

Scoring

Arthritis Index (AI): The foot volume was measured twice a week, and the swelling of the four toes was scored simultaneously. Each foot was scored on a scale from 0 to 4, with a maximum score of 16 for each rat.

Arthritis Index (AI) Scoring Criteria:

| Score | Degree |
|---|---|
| 0 | No swelling, normal appearance |
| 1 | Mild swelling or redness of the ankle, wrist, or finger joints |
| 2 | Moderate swelling or redness of the ankle, wrist, or finger joints |
| 3 | Severe swelling or redness of the ankle, wrist, or finger joints |
| 4 | Very severe swelling or redness of the ankle, wrist, or finger joints |

The day of starting drug administration is recorded as Day 0. Five days after starting drug administration, i.e., 2 hours after Day 5 administration, blood was collected from the jugular vein of the experimental animals, allowed to stand for more than 30 minutes, centrifuged to separate the serum, which was stored at -80°C. The levels of TNF-$\alpha$, IL-1$\beta$, and IL-6 in serum were measured by ELISA.

After the experiment, the animals were euthanized by $CO_2$ inhalation. The spleen and thymus of the animals were collected and weighed, and the organ coefficients were calculated.

Pathological Examination: One side of the foot joint tissue was fixed in 10% neutral formalin solution, followed by decalcification and paraffin embedding to prepare pathological sections. The sections were then stained with hematoxylin and eosin (HE) for pathological observation.

Observation Indices:

1. Infiltration of synovial inflammatory cells (lymphocytes, plasma cells);
2. Synovial tissue hyperplasia;
3. Synovial pannus;
4. Fibrinoid necrosis on the synovial surface.

Scoring and Counting Method: 0, 1, 2, 3, and 4 levels, where "0" indicates no observation; "1" is mild; "2" is moderate; "3" is severe; and "4" is extremely severe.

Experimental data were presented as Mean $\pm$ SEM. Data between two groups were analyzed using Excel-T test, with $p<0.05$ considered statistically significant. Scoring data were analyzed using the non-parametric Mann-Whitney U test in SPSS, with $p<0.05$ considered statistically significant.

The experimental results demonstrated that the compounds of the present disclosure regulated the serum inflammatory cytokines levels in the bovine type II collagen-induced rat model of collagen-induced arthritis (CIA), significantly ameliorated limb swelling in the rats, and thus can be applicable for the treatment of arthritis.

The above descriptions represent only preferred embodiments of the present invention, but the scope of protection is not limited thereto. Any person skilled in the art, within the technical scope disclosed by the invention, may make equivalent replacements or modifications based on the technical solutions and inventive concepts of the present invention, and such changes shall fall within the protection scope of the invention.

## Claims

**1.** A compound of Formula (I)

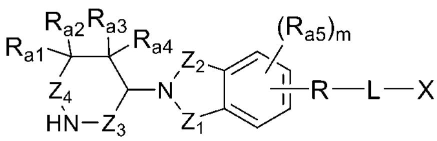

(I)

or salts, enantiomers, stereoisomers, solvates, or polymorphs thereof,

wherein $Z_1$ represents C(O), C(S), $CH_2$ or $CD_2$; $Z_2$, $Z_3$, and $Z_4$ each independently represent C(O) or C(S), wherein at least one of $Z_1$, $Z_2$, $Z_3$, and $Z_4$ represents C(S);

$R_{a1}$, $R_{a2}$, $R_{a3}$, and $R_{a4}$ each independently represent hydrogen, deuterium, halogen, $C_{1-6}$ alkyl, halogenated $C_{1-6}$ alkyl, deuterated $C_{1-6}$ alkyl, $C_{1-6}$ alkoxy, deuterated $C_{1-6}$ alkoxy or halogenated $C_{1-6}$ alkoxy;
$(R_{a5})_m$ indicates that the isoindoline ring to which it is attached is optionally substituted with m $R_{a5}$, with each $R_{a5}$ being identical or different and each independently representing deuterium, halogen, hydroxy, mercapto, nitro, amino, cyano, $C_{1-6}$ alkyl, halogenated $C_{1-6}$ alkyl, deuterated $C_{1-6}$ alkyl, $C_{1-6}$ alkoxy, deuterated $C_{1-6}$ alkoxy, halogenated $C_{1-6}$ alkoxy, $C_{2-6}$ alkenyl, or $C_{2-6}$ alkynyl;
m represents an integer of 0, 1, 2 or 3;
R represents O, S, S(O), $S(O)_2$, alkenylene, alkynylene or $N(R_{a6})$, wherein $R_{a6}$ represents H or $C_{1-3}$ alkyl; or R represents a bond; or
R represents:

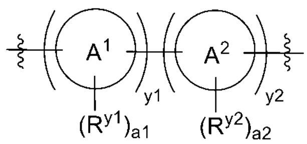

wherein each ring $A^1$ is identical or different and each independently represents nitrogen-containing heterocyclylene, arylene or heteroarylene, y1 represents an integer of 1 or 2, and $(R^{y1})_{a1}$ indicates that each ring $A^1$ is independently optionally substituted with a1 $R^{y1}$ groups, with each $R^{y1}$ being independently deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a1 represents an integer of 0-20;
each ring $A^2$ is identical or different and each independently represents heterocyclylene, cycloalkylene, arylene or heteroarylene, y2 represents an integer of 0 or 1, and $(R^{y2})_{a2}$ indicates that each ring $A^2$ is independently optionally substituted with a2 $R^{y2}$ groups, with each $R^{y2}$ being independently deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a2 represents an integer of 0-20;

L represents :

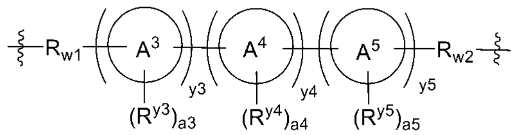

wherein $R_{w1}$ is linked to R, and $R_{w2}$ is linked to X;
$R_{w2}$ represents a bond, C(O), C(O)NH, NHC(O) or $N(R_{a6})$, wherein $R_{a6}$ represents H or $C_{1-3}$ alkyl;
each ring $A^3$ is identical or different and each independently represents heterocyclylene, cycloalkylene, arylene, or heteroarylene, y3 represents an integer of 0, 1, 2 or 3, and $(R^{y3})_{a3}$ indicates that each ring $A^3$ is independently optionally substituted with a3 $R^{y3}$ substituents, wherein each $R^{y3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a3 represents an integer of 0-20;
each ring $A^4$ is identical or different and each independently represents heterocyclylene, cycloalkylene, arylene, or heteroarylene, y4 represents an integer of 0, 1, 2 or 3, and $(R^{y4})_{a4}$ indicates that each ring $A^4$ is independently optionally substituted with a4 $R^{y4}$ substituents, wherein each $R^{y4}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a4 represents an integer of 0-20;
each ring $A^5$ is identical or different and each independently represents heterocyclylene, cycloalkylene, arylene, or heteroarylene, y5 represents an integer of 0, 1, 2 or 3, and $(R^{y5})_{a5}$ indicates that each ring $A^5$ is independently optionally substituted with a5 $R^{y5}$ substituents, wherein each $R^{y5}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a5 represents an integer of 0-20;
$R_{w1}$ represents a bond or an optionally substituted linear or branched $C_{1-20}$ alkylene, wherein any one or more methylene groups in the main carbon chain skeleton of the linear or branched $C_{1-20}$ alkylene are optionally replaced by one or more groups selected from $R_a$, $R_b$, and any combination thereof; wherein each $R_a$ is independently selected from the group consisting of: O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, wherein each $R_{a7}$ independently

represents H or $C_{1-6}$ alkyl, and when any two or more methylene in the main carbon chain skeleton of the linear or branched $C_{1-20}$ alkylene are replaced by two or more $R_a$ groups, the $R_a$ groups are not directly connected to each other; and wherein each $R_b$ is independently selected from the group consisting of: optionally substituted cycloalkylene, optionally substituted arylene, optionally substituted heterocyclylene, optionally substituted heteroarylene, alkynylene, alkenylene, and any combination thereof;

X represents :

$NR_1R_2$, $C(O)NR_3R_4$, $NHC(O)R_5$, $NHC(O)NR_6R_7$, $OR_8$, $SR_9$, $S(O)_2NR_{10}R_{11}$ or $N(R_{12})S(O)_2R_{13}$, wherein $R_1$, $R_3$, $R_4$, $R_5$, $R_6$, $R_7$, $R_9$, $R_{10}$, $R_{11}$, and $R_{12}$ each independently represent H, optionally substituted linear or branched alkyl, optionally substituted cycloalkyl, optionally substituted aryl, optionally substituted heterocyclyl or optionally substituted heteroaryl, and wherein $R_2$, $R_8$, and $R_{13}$ each independently represent optionally substituted linear or branched alkyl, optionally substituted cycloalkyl, optionally substituted aryl, optionally substituted heterocyclyl or optionally substituted heteroaryl, or wherein $R_3$ and $R_4$, together with the nitrogen atom to which they are attached, form an optionally substituted 4- to 30-membered nitrogen-containing heterocyclyl;

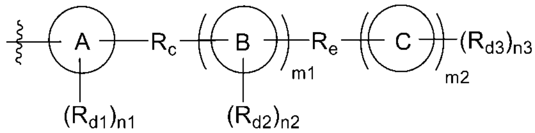

wherein ring A represents heterocyclylene, cycloalkylene, arylene or heteroarylene, and $(R_{d1})_{n1}$ indicates that ring A is optionally substituted with n1 $R_{d1}$ groups, with each $R_{d1}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n1 represents an integer of 0-20; $R_c$ represents $NR_{c1}$, C(O), $CR_{c2}R_{c3}$ or a bond, wherein $R_{c1}$ represents H, optionally substituted linear or branched alkyl, optionally substituted cycloalkyl, optionally substituted aryl, optionally substituted heterocyclyl or optionally substituted heteroaryl, and $R_{c2}$ and $R_{c3}$ each independently represent H, halogen, optionally substituted linear or branched alkyl, optionally substituted cycloalkyl, optionally substituted aryl, optionally substituted heterocyclyl or optionally substituted heteroaryl;
each ring B is identical or different and each independently represents heterocyclylene, cycloalkylene, arylene, or heteroarylene, m1 represents an integer of 0, 1, 2 or 3, and $(R_{a2})_{n2}$ indicates that each ring B is independently optionally substituted with n2 $R_{d2}$ groups, with each $R_{d2}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n2 represents an integer of 0-20; $R_e$ represents S, O, $CR_{e1}R_{e2}$ or a bond, wherein $R_{e1}$ and $R_{e2}$ each independently represent H, halogen, optionally substituted linear or branched alkyl, optionally substituted cycloalkyl, optionally substituted aryl, optionally substituted heterocyclyl or optionally substituted heteroaryl; and
each ring C is identical or different and each independently represents heterocyclyl, cycloalkyl, aryl, or heteroaryl, m2 represents an integer of 0, 1, 2 or 3, and $(R_{d3})_{n3}$ indicates that each ring C is independently optionally substituted with n3 $R_{d3}$ groups, with each $R_{d3}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n3 represents an integer of 0-20; or

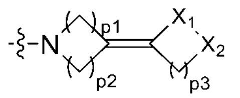

wherein $-X_1-X_2-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;
p1, p2, and p3 each independently represent an integer of 1, 2, 3 or 4; and
the ring containing $-X_1-X_2-$ is optionally substituted with one or more substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, $C_{2-6}$ alkenyl, and any combination thereof; or

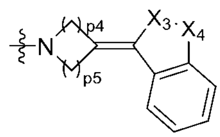

wherein $-X_3-X_4-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;
p4 and p5 each independently represent an integer of 1, 2, 3 or 4; and
the benzo-fused ring containing $-X_3-X_4-$ is optionally substituted with one or more substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, $C_{2-6}$ alkenyl, and any combination thereof.

**2.** The compound of Formula (I) or salts, enantiomers, stereoisomers, solvates, or polymorphs thereof as claimed in claim 1, wherein

(i) $R_{a1}$, $R_{a2}$, $R_{a3}$, and $R_{a4}$ each independently represent H; and/or
(ii) $Z_1$ represents C(O), C(S), $CH_2$ or $CD_2$; $Z_2$, $Z_3$, and $Z_4$ each independently represent C(O) or C(S), wherein at least two of $Z_1$, $Z_2$, $Z_3$, and $Z_4$ represent C(S); or

at least three of $Z_1$, $Z_2$, $Z_3$, and $Z_4$ represent C(S); or
$Z_1$, $Z_2$, $Z_3$, and $Z_4$ all represent C(S); and/or

(iii) R represents O, S, S(O), $S(O)_2$, $C_{2-6}$ alkenylene, $C_{2-6}$ alkynylene or $N(R_{a6})$, wherein $R_{a6}$ represents H or $C_{1-3}$ alkyl; preferably, R represents O, S, S(O), $S(O)_2$, $C_{2-4}$ alkenylene, $C_{2-4}$ alkynylene or $N(R_{a6})$, wherein $R_{a6}$ represents H or $C_{1-3}$ alkyl; or

R represents a bond; or
R represents :

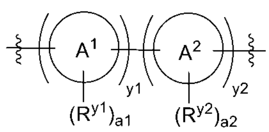

wherein each ring $A^1$ is identical or different and each independently represents 4- to 30-membered nitrogen-containing heterocyclylene, $C_{5-30}$ arylene or 5- to 30-membered heteroarylene (preferably, 4- to 20-membered nitrogen-containing heterocyclylene, $C_{5-20}$ arylene or 5- to 20-membered heteroarylene; or more preferably, 4- to 15-membered nitrogen-containing heterocyclylene, $C_{5-15}$ arylene or 5- to 15-membered heteroarylene), y1 represents an integer of 1 or 2, and $(R^{y1})_{a1}$ indicates that each ring $A^1$ is independently optionally substituted with a1 $R^{y1}$ groups, with each $R^{y1}$ being independently deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a1 represents an integer of 0-20;
each ring $A^2$ is identical or different and each independently represents 4- to 30-membered heterocyclylene, $C_{3-30}$ cycloalkylene, $C_{5-30}$ arylene or 5- to 30-membered heteroarylene (preferably, 4- to 20-membered heterocyclylene, $C_{3-20}$ cycloalkylene, $C_{5-20}$ arylene or 5- to 20-membered heteroarylene; or more preferably, 4- to 15-membered heterocyclylene, $C_{3-15}$ cycloalkylene, $C_{5-15}$ arylene or 5- to 15-membered heteroarylene), y2 represents an integer of 0 or 1, and $(R^{y2})_{a2}$ indicates that each ring $A^2$ is independently optionally substituted with a2 $R^{y2}$ groups, with each $R^{y2}$ being independently deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a2 represents an integer of 0-20; and/or

(iv) L represents :

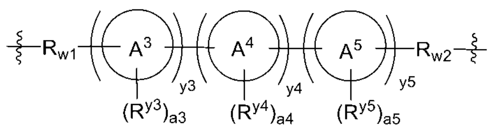

wherein $R_{w1}$ is linked to R, and $R_{w2}$ is linked to X;

$R_{w2}$ represents a bond, C(O), C(O)NH, NHC(O) or N($R_{a6}$), wherein $R_{a6}$ represents H or $C_{1-3}$ alkyl;

each ring $A^3$ is identical or different and each independently represents 4- to 30-membered heterocyclylene, $C_{3-30}$ cycloalkylene, $C_{5-30}$ arylene or 5- to 30-membered heteroarylene (preferably, 4- to 20-membered heterocyclylene, $C_{3-20}$ cycloalkylene, $C_{5-20}$ arylene or 5- to 20-membered heteroarylene; or more preferably, 4- to 15-membered heterocyclylene, $C_{3-15}$ cycloalkylene, $C_{5-15}$ arylene or 5- to 15-membered heteroarylene), y3 represents an integer of 0, 1, 2 or 3, and $(R^{y3})_{a3}$ indicates that each ring $A^3$ is independently optionally substituted with a3 $R^{y3}$ substituents, wherein each $R^{y3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a3 represents an integer of 0-20;

each ring $A^4$ is identical or different and each independently represents 4- to 30-membered heterocyclylene, $C_{3-30}$ cycloalkylene, $C_{5-30}$ arylene or 5- to 30-membered heteroarylene (preferably, 4- to 20-membered heterocyclylene, $C_{3-20}$ cycloalkylene, $C_{5-20}$ arylene or 5- to 20-membered heteroarylene; or more preferably, 4- to 15-membered heterocyclylene, $C_{3-15}$ cycloalkylene, $C_{5-15}$ arylene or 5- to 15-membered heteroarylene), y4 represents an integer of 0, 1, 2 or 3, and $(R^{y4})_{a4}$ indicates that each ring $A^4$ is independently optionally substituted with a4 $R^{y4}$ substituents, wherein each $R^{y4}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a4 represents an integer of 0-20;

each ring $A^5$ is identical or different and each independently represents 4- to 30-membered heterocyclylene, $C_{3-30}$ cycloalkylene, $C_{5-30}$ arylene or 5- to 30-membered heteroarylene (preferably, 4- to 20-membered heterocyclylene, $C_{3-20}$ cycloalkylene, $C_{5-20}$ arylene or 5- to 20-membered heteroarylene; or more preferably, 4- to 15-membered heterocyclylene, $C_{3-15}$ cycloalkylene, $C_{5-15}$ arylene or 5- to 15-membered heteroarylene), y5 represents an integer of 0, 1, 2 or 3, and $(R^{y5})_{a5}$ indicates that each ring $A^5$ is independently optionally substituted with a5 $R^{y5}$ substituents, wherein each $R^{y5}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a5 represents an integer of 0-20;

$R_{w1}$ represents a bond or an optionally substituted linear or branched $C_{1-20}$ alkylene, wherein any one or more methylene groups in the main carbon chain skeleton of the linear or branched $C_{1-20}$ alkylene are optionally replaced by one or more groups selected from $R_a$, $R_b$, and any combination thereof; wherein each $R_a$ is independently selected from the group consisting of: O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, wherein each $R_{a7}$ independently represent H or $C_{1-6}$ alkyl; and when any two or more methylene in the main carbon chain skeleton of the linear or branched $C_{1-20}$ alkylene are replaced by two or more $R_a$ groups, the $R_a$ groups are not directly connected to each other; and wherein each $R_b$ is independently selected from the group consisting of: optionally substituted $C_{3-30}$ cycloalkylene, optionally substituted $C_{5-30}$ arylene, optionally substituted 4- to 30-membered heterocyclylene, optionally substituted 5- to 30-membered heteroarylene, $C_{2-6}$ alkenylene or $C_{2-6}$ alkynylene (preferably, optionally substituted $C_{3-20}$ cycloalkylene, optionally substituted $C_{5-20}$ arylene, optionally substituted 4- to 20-membered heterocyclylene, optionally substituted 5- to 20-membered heteroarylene, $C_{2-4}$ alkenylene or $C_{2-4}$ alkynylene; or more preferably, optionally substituted $C_{3-15}$ cycloalkylene, optionally substituted $C_{5-15}$ arylene, optionally substituted 4- to 15-membered heterocyclylene, optionally substituted 5- to 15-membered heteroarylene, $C_{2-4}$ alkenylene or $C_{2-4}$ alkynylene), and any combination thereof; and/or

(v) X represents :

$NR_1R_2$, $C(O)NR_3R_4$, $NHC(O)R_5$, $NHC(O)NR_6R_7$, $OR_8$, $SR_9$, $S(O)_2NR_{10}R_{11}$, or $N(R_{12})S(O)_2R_{13}$, wherein $R_1$, $R_3$, $R_4$, $R_8$, $R_6$, $R_7$, $R_9$, $R_{10}$, $R_{11}$, and $R_{12}$ each independently represent H, optionally substituted linear or branched $C_{1-10}$ alkyl (preferably, optionally substituted linear or branched $C_{1-6}$ alkyl), optionally substituted $C_{3-30}$ cycloalkyl (preferably, optionally substituted $C_{3-20}$ cycloalkyl; more preferably, optionally substituted $C_{3-15}$ cycloalkyl), optionally substituted $C_{5-30}$ aryl (preferably, optionally substituted $C_{5-20}$ aryl; more preferably, optionally substituted $C_{5-15}$ aryl), optionally substituted 4- to 30-membered heterocyclyl (preferably, optionally substituted 4- to 20-membered heterocyclyl; more preferably, optionally substituted 4- to 15-membered heterocyclyl), or optionally substituted 5- to 30-membered heteroaryl (preferably, optionally

substituted 5- to 20-membered heteroaryl; more preferably, optionally substituted 5- to 15-membered heteroaryl), and $R_2$, $R_8$, and $R_{13}$ each independently represent optionally substituted linear or branched $C_{1-10}$ alkyl (preferably, optionally substituted linear or branched $C_{1-6}$ alkyl), optionally substituted $C_{3-30}$ cycloalkyl (preferably, optionally substituted $C_{3-20}$ cycloalkyl; more preferably, optionally substituted $C_{3-15}$ cycloalkyl), optionally substituted $C_{5-30}$ aryl (preferably, optionally substituted $C_{5-20}$ aryl; more preferably, optionally substituted $C_{5-15}$ aryl), optionally substituted 4- to 30-membered heterocyclyl (preferably, optionally substituted 4- to 20-membered heterocyclyl; more preferably, optionally substituted 4- to 15-membered heterocyclyl), or optionally substituted 5- to 30-membered heteroaryl (preferably, optionally substituted 5- to 20-membered heteroaryl; more preferably, optionally substituted 5- to 15-membered heteroaryl); or wherein $R_3$ and $R_4$, together with the nitrogen atom to which they are attached, form an optionally substituted 4- to 30-membered nitrogen-containing heterocyclyl; or

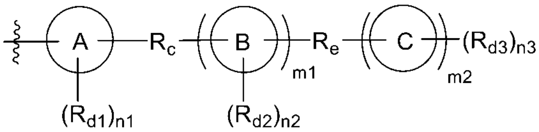

wherein ring A represents 4- to 30-membered heterocyclylene (preferably, 4- to 20-membered heterocyclylene; more preferably, 4- to 15-membered heterocyclylene), $C_{3-30}$ cycloalkylene (preferably, $C_{3-20}$ cycloalkylene; more preferably, $C_{3-15}$ cycloalkylene), $C_{5-30}$ arylene (preferably, $C_{5-20}$ arylene; more preferably, $C_{5-15}$ arylene) or 5- to 30-membered heteroarylene (preferably, 5- to 20-membered heteroarylene; more preferably, 5- to 15-membered heteroarylene), and $(R_{d1})_{n1}$ indicates that ring A is optionally substituted with n1 $R_{d1}$ groups, with each $R_{d1}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n1 represents an integer of 0-20;

$R_c$ represents $NR_{c1}$, C(O), $CR_{c2}R_{c3}$ or a bond, wherein $R_{c1}$ represents H, optionally substituted linear or branched $C_{1-10}$ alkyl (preferably, optionally substituted linear or branched $C_{1-6}$ alkyl), optionally substituted $C_{3-30}$ cycloalkyl (preferably, optionally substituted $C_{3-20}$ cycloalkyl; more preferably, optionally substituted $C_{3-15}$ cycloalkyl), optionally substituted $C_{5-30}$ aryl (preferably, optionally substituted $C_{5-20}$ aryl; more preferably, optionally substituted $C_{5-15}$ aryl), optionally substituted 4- to 30-membered heterocyclyl (preferably, optionally substituted 4- to 20-membered heterocyclyl; more preferably, optionally substituted 4- to 15-membered heterocyclyl) or optionally substituted 5- to 30-membered heteroaryl (preferably, optionally substituted 5- to 20-membered heteroaryl; more preferably, optionally substituted 5- to 15-membered heteroaryl), and $R_{c2}$ and $R_{c3}$ each independently represent H, halogen, optionally substituted linear or branched $C_{1-10}$ alkyl (preferably, optionally substituted linear or branched $C_{1-6}$ alkyl), optionally substituted $C_{3-30}$ cycloalkyl (preferably, optionally substituted $C_{3-20}$ cycloalkyl; more preferably, optionally substituted $C_{3-15}$ cycloalkyl), optionally substituted $C_{5-30}$ aryl (preferably, optionally substituted $C_{5-20}$ aryl; more preferably, optionally substituted $C_{5-15}$ aryl), optionally substituted 4- to 30-membered heterocyclyl (preferably, optionally substituted 4- to 20-membered heterocyclyl; more preferably, optionally substituted 4- to 15-membered heterocyclyl), or optionally substituted 5- to 30-membered heteroaryl (preferably, optionally substituted 5- to 20-membered heteroaryl; more preferably, optionally substituted 5- to 15-membered heteroaryl);

each ring B is identical or different and each independently represents 4- to 30-membered heterocyclylene (preferably, 4- to 20-membered heterocyclylene; more preferably, 4- to 15-membered heterocyclylene), $C_{3-30}$ cycloalkylene (preferably, $C_{3-20}$ cycloalkylene; more preferably, $C_{3-15}$ cycloalkylene), $C_{5-30}$ arylene (preferably, $C_{5-20}$ arylene; more preferably, $C_{5-15}$ arylene), or 5- to 30-membered heteroarylene (preferably, 5- to 20-membered heteroarylene; more preferably, 5- to 15-membered heteroarylene), m1 represents an integer of 0, 1, 2 or 3, and $(R_{d2})_{n2}$ indicates that each ring B is independently optionally substituted with n2 $R_{d2}$ groups, with each $R_{d2}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n2 represents an integer of 0-20;

$R_e$ represents S, O, $CR_{e1}R_{e2}$ or a bond, wherein $R_{e1}$ and $R_{e2}$ each independently represent H, halogen, optionally substituted linear or branched $C_{1-10}$ alkyl (preferably, optionally substituted linear or branched $C_{1-6}$ alkyl), optionally substituted $C_{3-30}$ cycloalkyl (preferably, optionally substituted $C_{3-20}$ cycloalkyl; more preferably, optionally substituted $C_{3-15}$ cycloalkyl), optionally substituted $C_{5-30}$ aryl (preferably, optionally substituted $C_{5-20}$ aryl; more preferably, optionally substituted $C_{5-15}$ aryl), optionally substituted 4- to 30-membered heterocyclyl (preferably, optionally substituted 4- to 20-membered heterocyclyl; more preferably, optionally substituted 4- to 15-membered heterocyclyl), or optionally substituted 5- to 30-membered

heteroaryl (preferably, optionally substituted 5- to 20-membered heteroaryl; more preferably, optionally substituted 5- to 15-membered heteroaryl); and

each ring C is identical or different and each independently represents 4- to 30-membered heterocyclyl (preferably, 4- to 20-membered heterocyclyl; more preferably, 4- to 15-membered heterocyclyl), $C_{3-30}$ cycloalkyl (preferably, $C_{3-20}$ cycloalkyl; more preferably, $C_{3-15}$ cycloalkyl), $C_{5-30}$ aryl (preferably, $C_{5-20}$ aryl; more preferably, $C_{5-15}$ aryl), or 5- to 30-membered heteroaryl (preferably, 5- to 20-membered heteroaryl; more preferably, 5- to 15-membered heteroaryl), m2 represents an integer of 0, 1, 2 or 3, and $(R_{d3})_{n3}$ indicates that each ring C is independently optionally substituted with n3 $R_{d3}$ groups, with each $R_{d3}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n3 represents an integer of 0-20; or

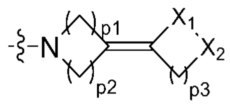

wherein $-X_1-X_2-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;

p1, p2, and p3 each independently represent an integer of 1, 2 or 3; and

the ring containing $-X_1-X_2-$ is optionally substituted with one or more substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, $C_{2-6}$ alkenyl, and any combination thereof; or

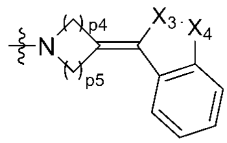

wherein $-X_3-X_4-$ represents $-(CH_2)_{1-6}-$, $-(CH_2)_{1-5}O-$, $-CH_2O(CH_2)_{1-4}-$, $-(CH_2)_{1-4}OCH_2-$, $-(CH_2)_{1-5}NH-$, $-CH_2NH(CH_2)_{1-4}-$, or $-(CH_2)_{1-4}NHCH_2-$;

p4 and p5 each independently represent an integer of 1, 2 or 3; and

the benzo-fused ring containing $-X_3-X_4-$ is optionally substituted with one or more substituents selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, $C_{2-6}$ alkenyl, and any combination thereof.

**3.** The compound of Formula (I) or salts, enantiomers, stereoisomers, solvates, or polymorphs thereof as claimed in claim 1 or 2, wherein

(i) R represents O, S, S(O), $S(O)_2$, vinylene, ethynylene, NH or $N(CH_3)$; or R represents a bond; or
(ii) each ring $A^1$ independently represents:

piperidinylene, piperazinylene, morpholinylene, azetidinylene, pyrrolidinylene, imidazolidinylene, pyrazolidylene, oxazolidinylene, thiazolidinylene, thiomorpholinylene, azacycloheptylene, diazacycloheptanylene, azacyclooctylene, diazacyclooctylene, azabicyclo[3.1.1]heptanylene, azabicyclo[2.2.1]heptanylene, azabicyclo[3.2.1]octanylene, azabicyclo[2.2.2]octanylene, diazabicyclo[3.1.1]heptanylene, diazabicyclo[2.2.1]heptanylene, diazabicyclo[3.2.1]octanylene, diazabicyclo[2.2.2]octanylene, 3-azaspiro[5.5]undecanylene, 8-azaspiro[4.5]decanylene, 2-oxa-8-azaspiro[4.5]decanylene, 3-methyl-2-oxa-8-azaspiro[4.5]decanylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene,

or imidazo[2,1-b]thiazolylene; or preferably,

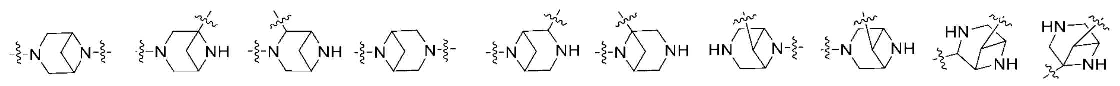

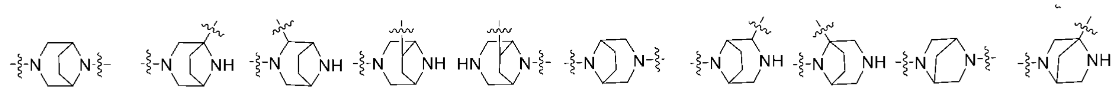

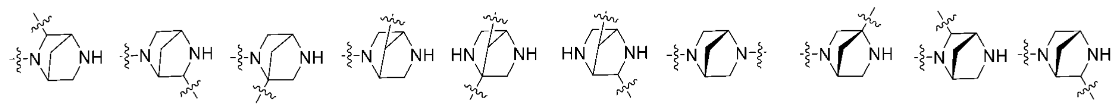

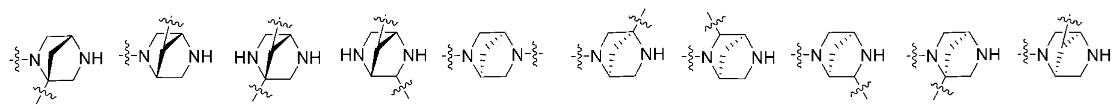

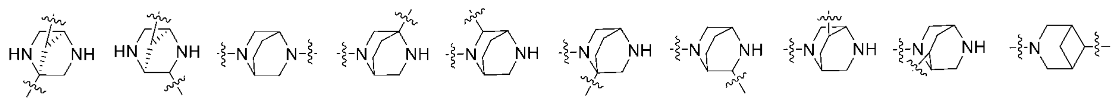

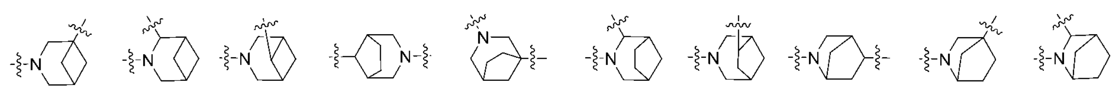

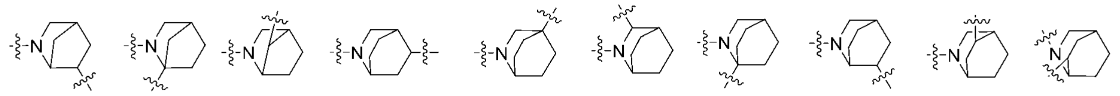

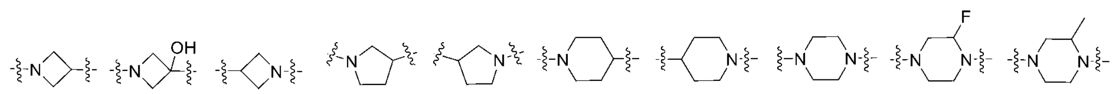

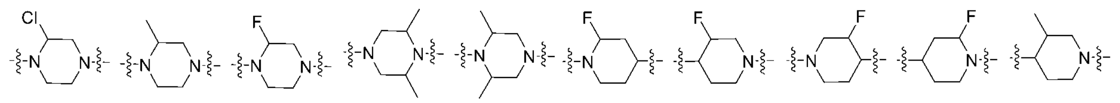

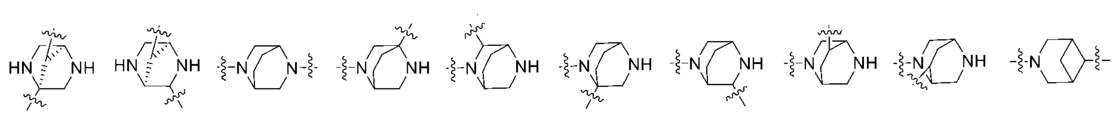

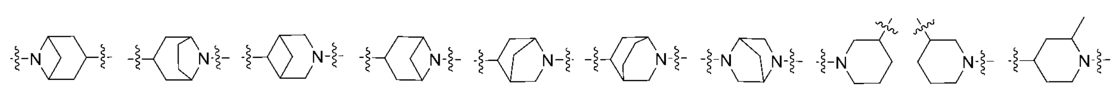

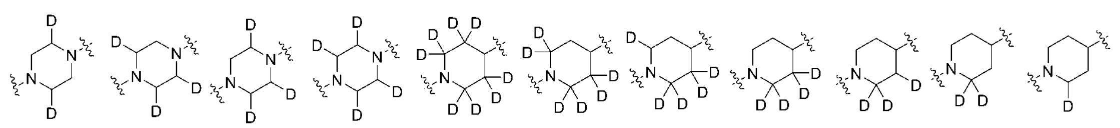

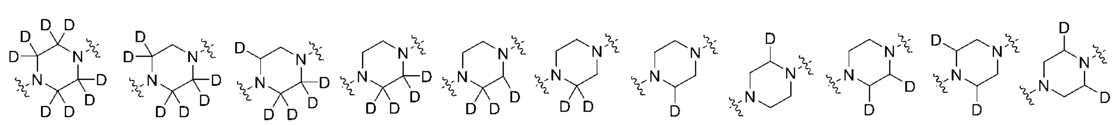

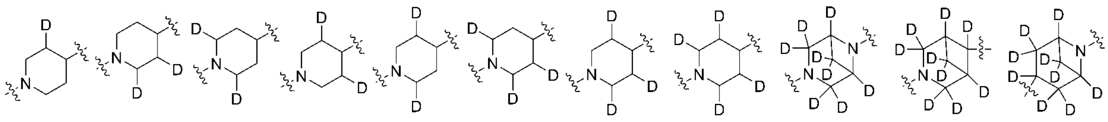

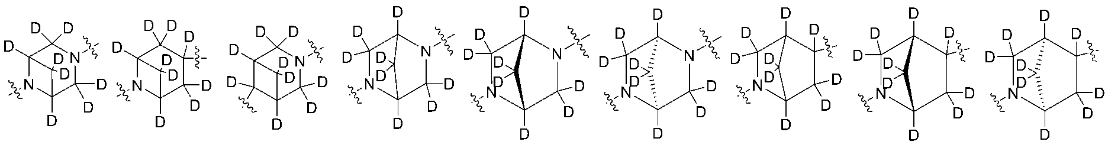

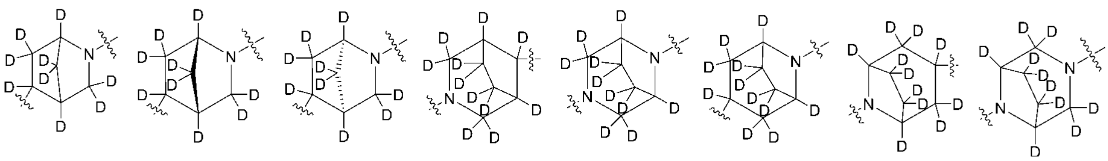

or

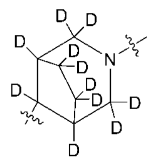

;

each ring $A^1$ is independently optionally substituted with a1 $R^{y1}$ groups, wherein each $R^{y1}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl; and a1 represents an integer of 0-20;
and/or

each ring $A^2$ independently represents:

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, $C_{5-15}$ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene; or each ring $A^2$ independently represents:

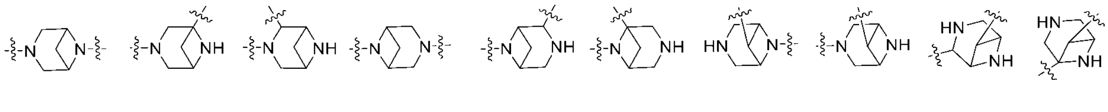

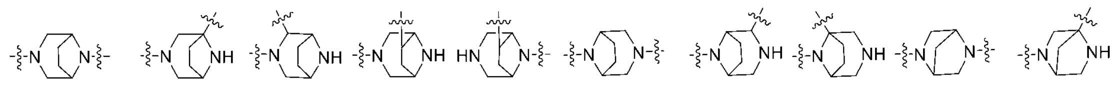

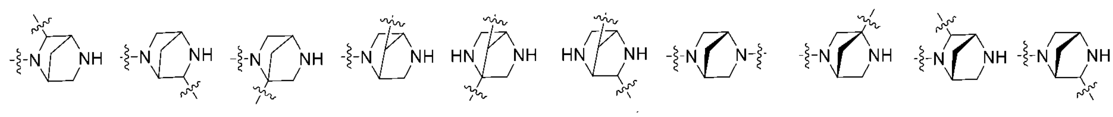

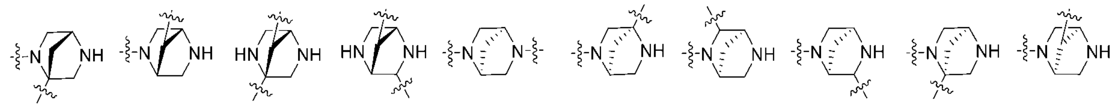

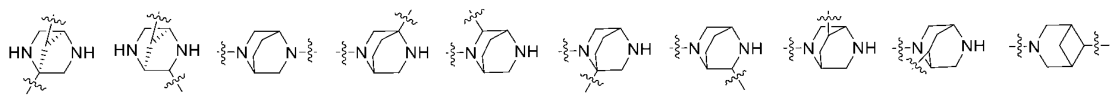

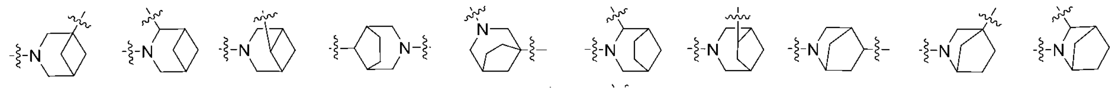

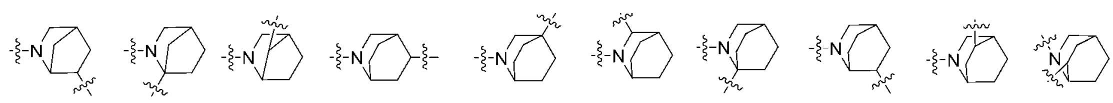

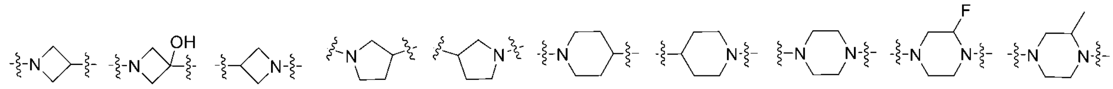

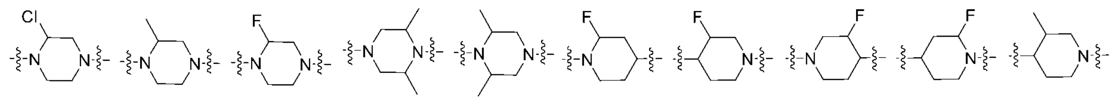

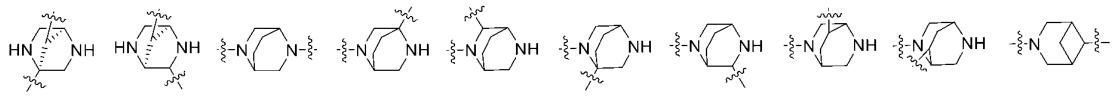

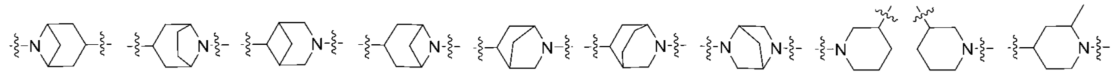

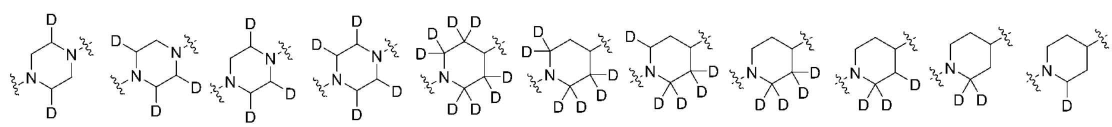

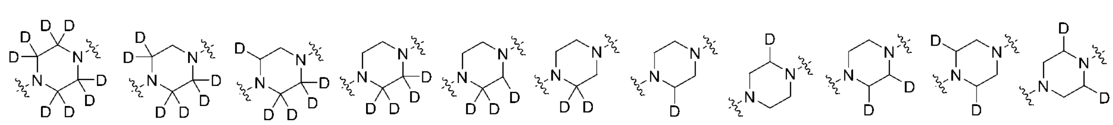

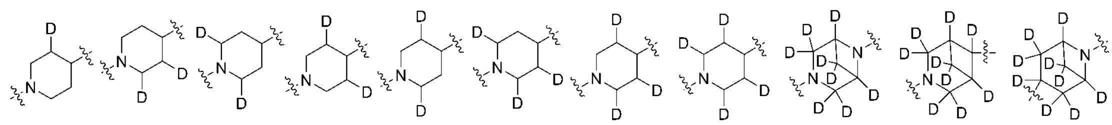

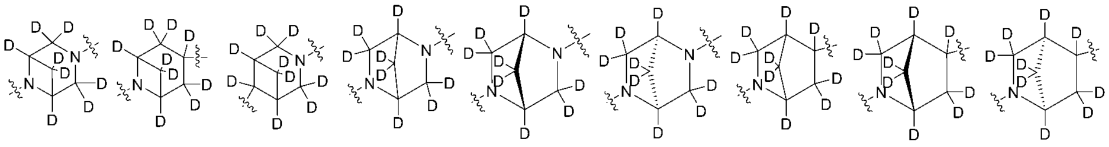

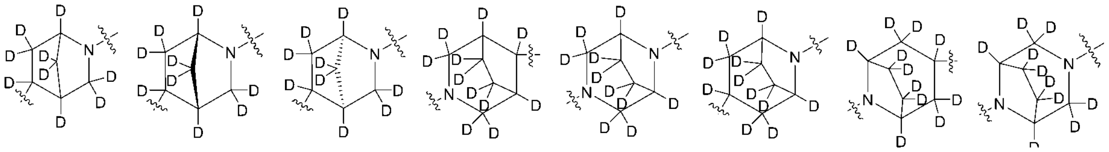

or

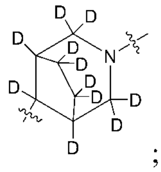

;

each ring $A^2$ is independently optionally substituted with a2 $R^{y2}$ groups, wherein each $R^{y2}$ is independently deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl, and a2 represents an integer of 0-20; preferably, R represents :

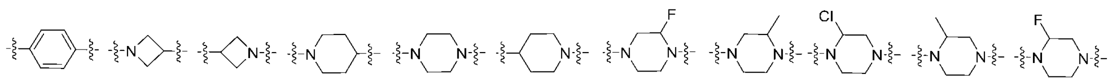

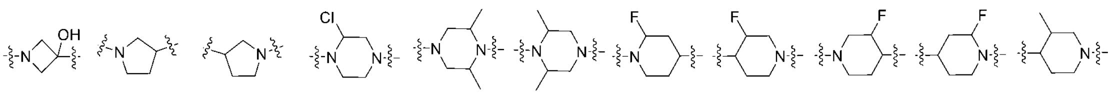

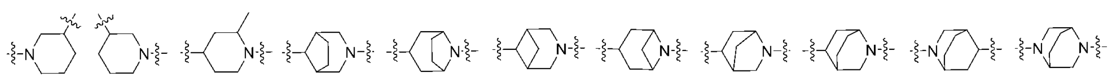

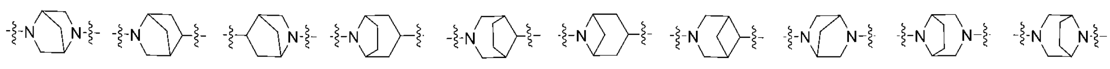

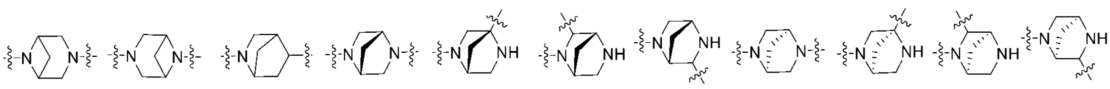

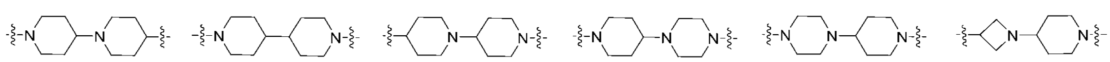

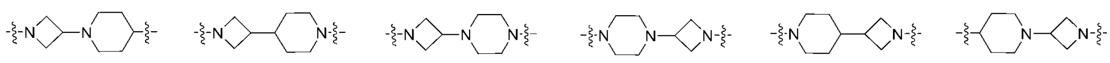

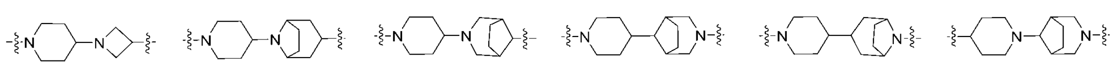

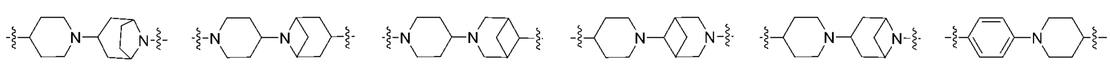

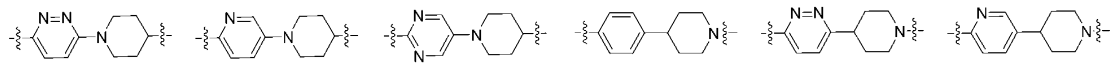

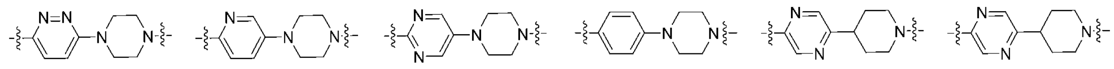

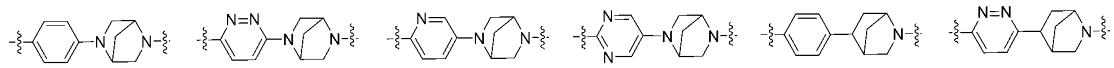

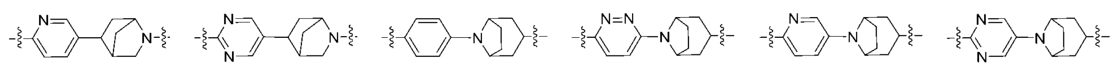

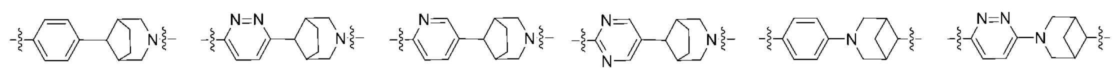

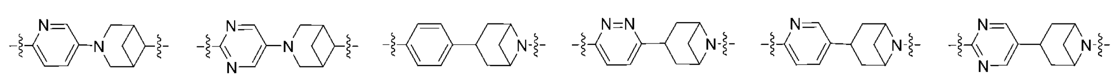

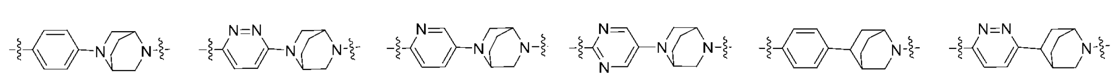

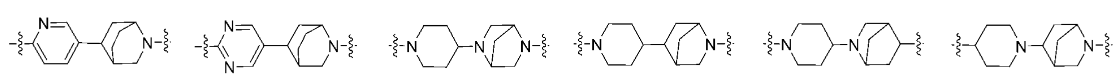

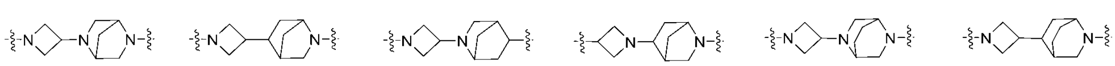

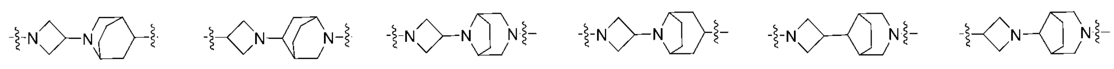

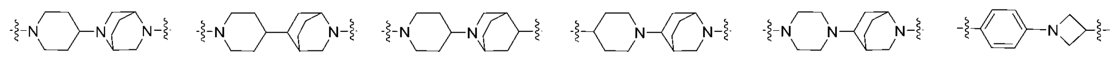

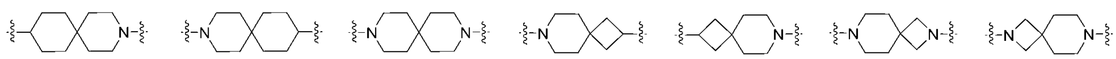

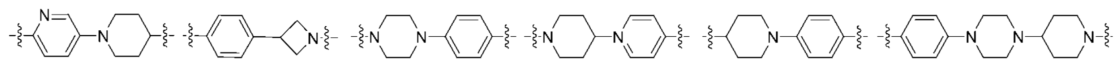

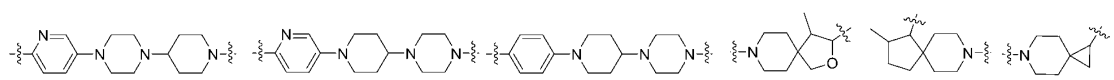

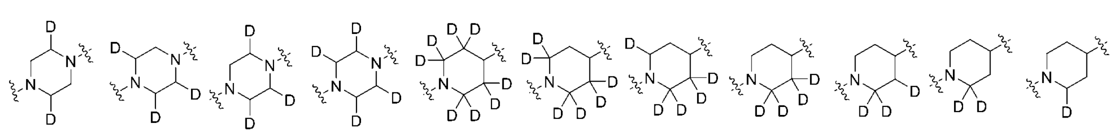

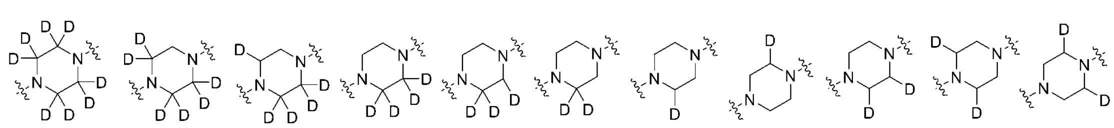

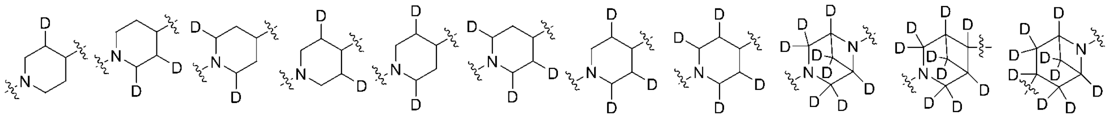

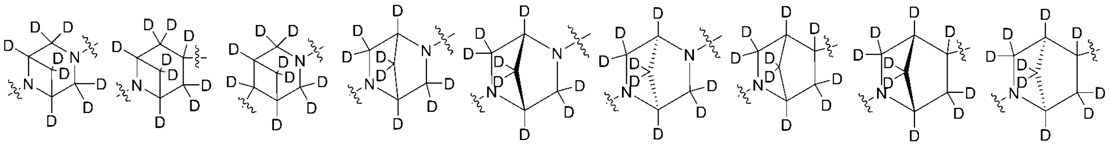

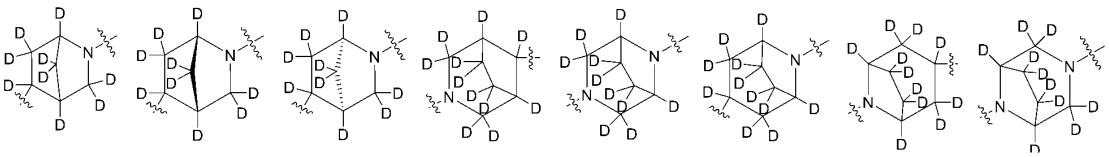

or

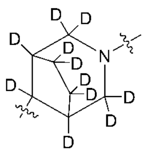

;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl, and $C_{2-6}$ alkenyl;
and/or

(iii) ring $A^2$, ring $A^4$, and ring $A^5$ each independently represent:

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, morpholinylene, thiomorpholinylene, azacycloheptanylene, azacyclooctylene, dioxacyclohexylene, azacycloheptanylene, azacyclooctylene, diazacycloheptanylene, diazacyclooctylene, 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, 5- to 20-membered azaspirocycloalkylene, cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, 2,3-dihydro-1H-indenylene, $C_{5-15}$ spiro-cycloalkylene, adamantanylene, noradamantanylene, norbornylene, phenylene, naphthylene, furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, indolylene, isoindolylene, benzofuranylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, quinolinylene, isoquinolinylene, naphthyridinylene, cinnolinylene, quinazolinylene, quinoxalinylene, phthalazinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, or imidazo[2,1-b]thiazolylene; or ring $A^3$, ring $A^4$, and ring $A^5$ each independently represent:

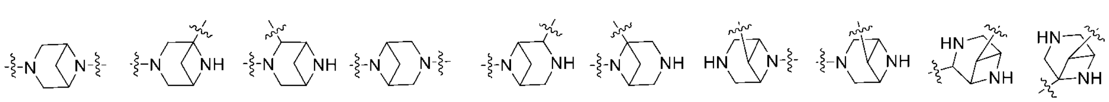

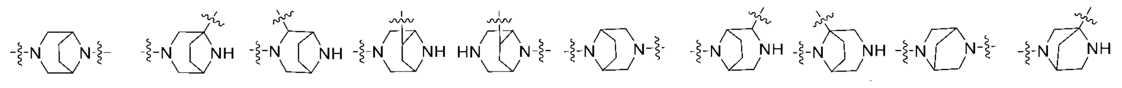

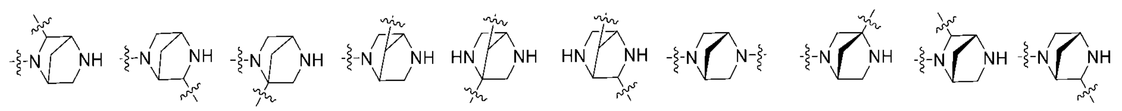

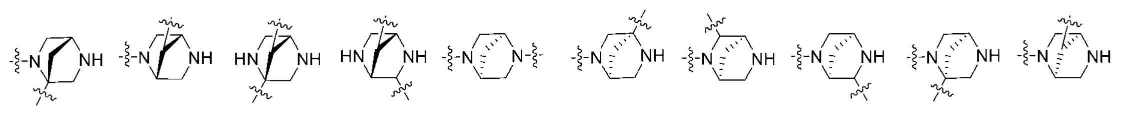

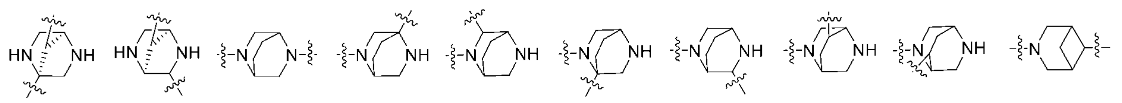

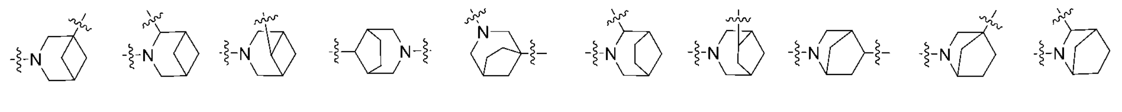

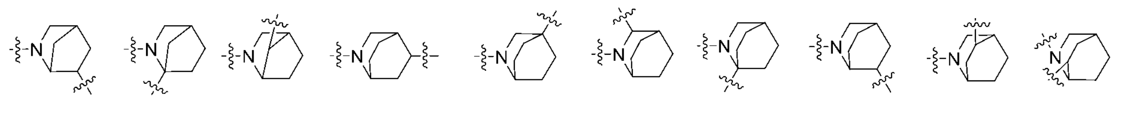

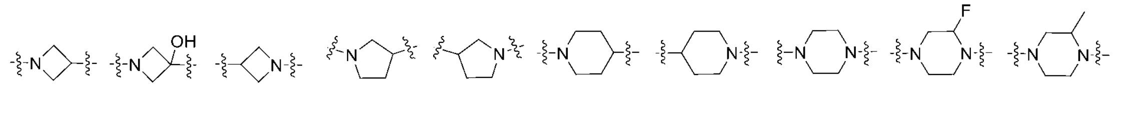

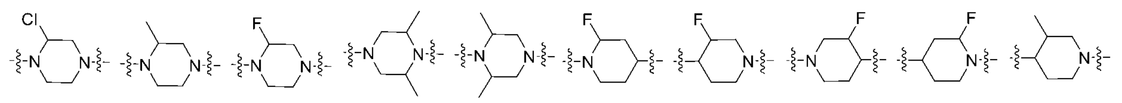

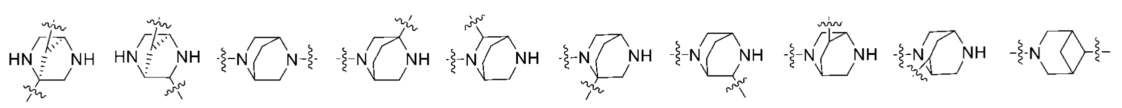

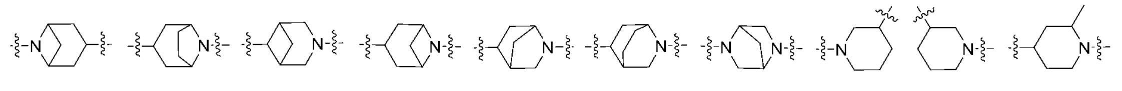

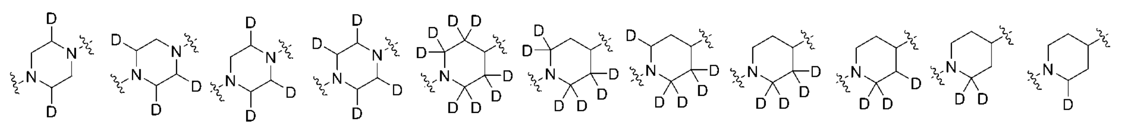

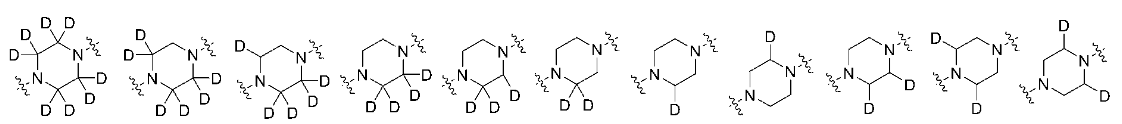

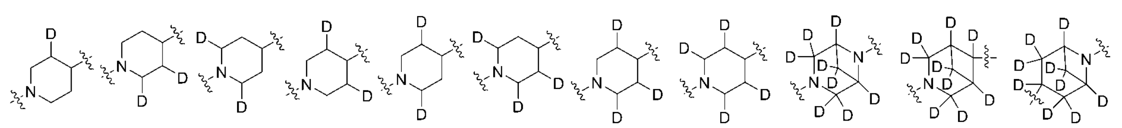

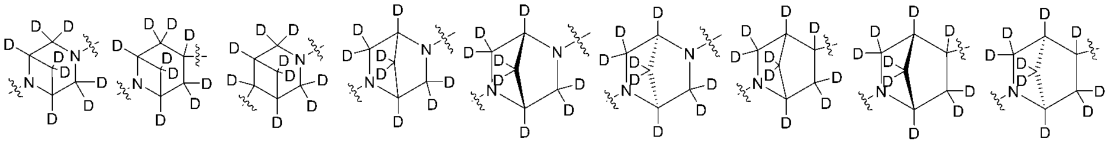

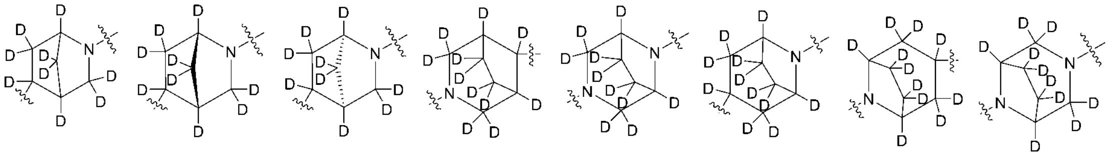

or

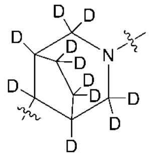

;

and

y3 represents an integer of 0, 1, 2 or 3; and each ring $A^3$ is independently optionally substituted with a3 $R^{y3}$ substituents, wherein each $R^{y3}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl; and a3 represents an integer of 0-20;

y4 represents an integer of 0, 1, 2 or 3; and each ring $A^4$ is independently optionally substituted with a4 $R^{y4}$ substituents, wherein each $R^{y4}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl or $C_{2-6}$ alkenyl; and a4 represents an integer of 0-20; or

y5 represents an integer of 0, 1, 2 or 3; and each ring $A^5$ is independently optionally substituted with a5 $R^{y5}$ substituents, wherein each $R^{y5}$ independently represents deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, carboxyl,, $C_{2-6}$ alkynyl or $C_{2-6}$ alkenyl; and a5 represents an integer of 0-20.

4. The compound of Formula (I) or salts, enantiomers, stereoisomers, solvates, or polymorphs thereof as claimed in claim 1, wherein the

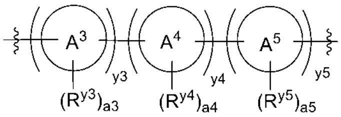

moiety in the general formula of L represents:

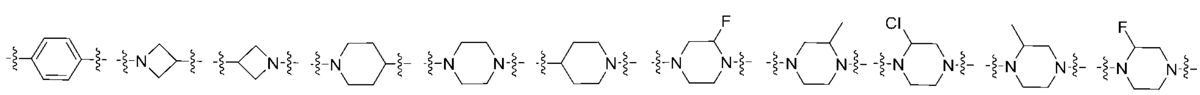

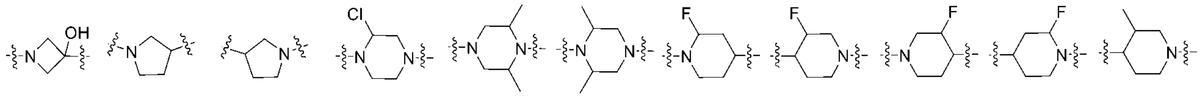

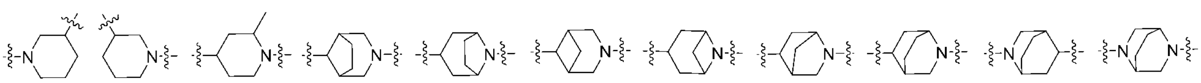

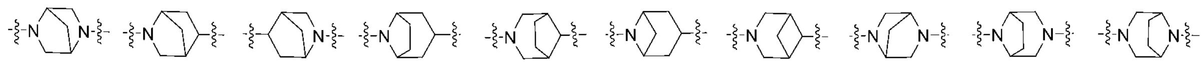

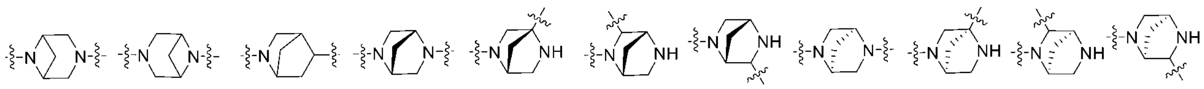

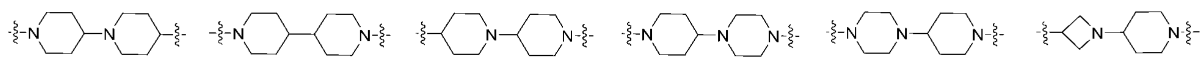

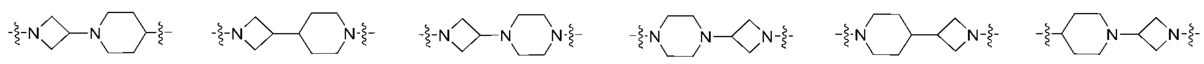

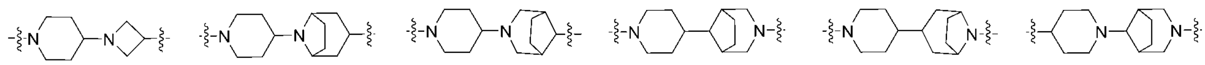

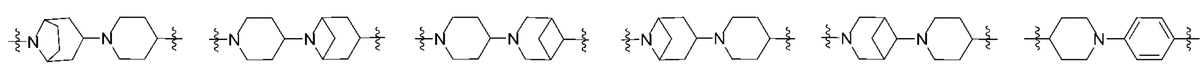

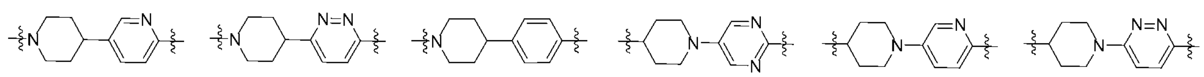

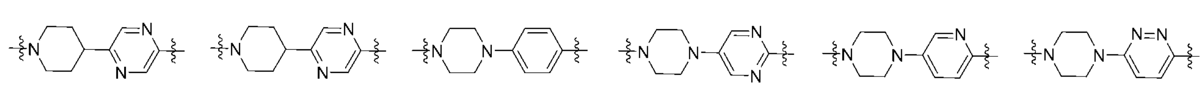

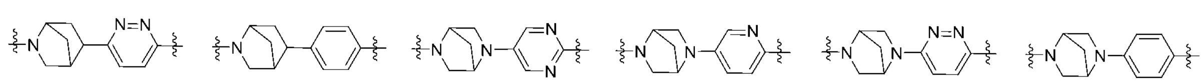

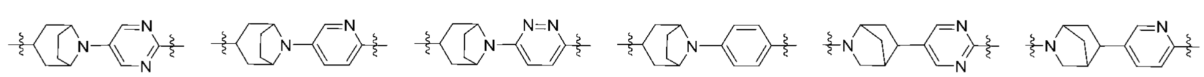

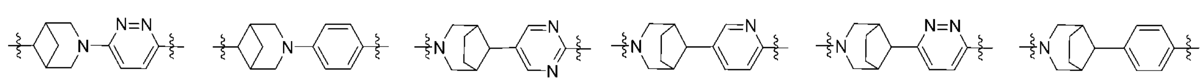

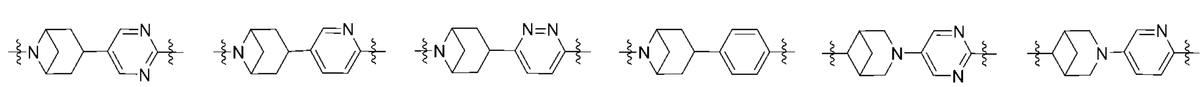

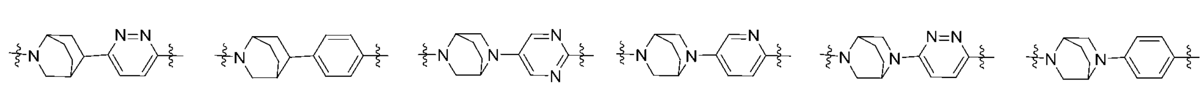

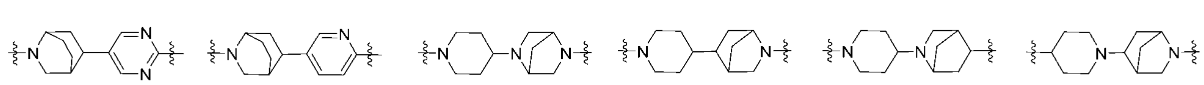

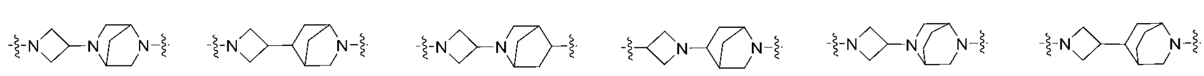

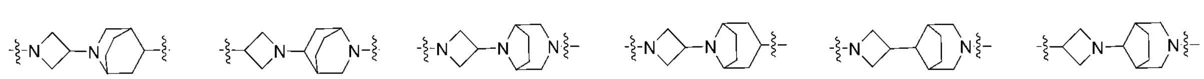

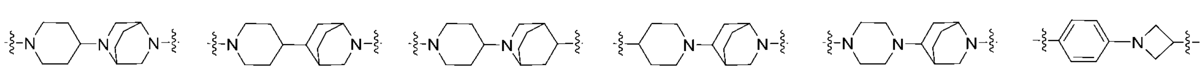

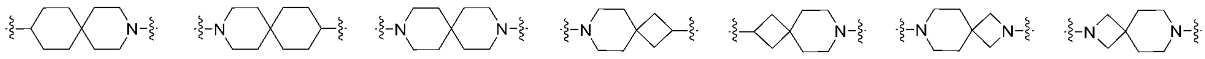
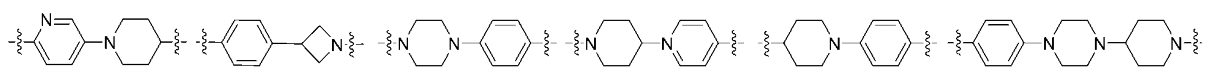
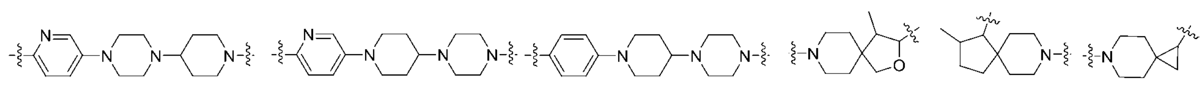
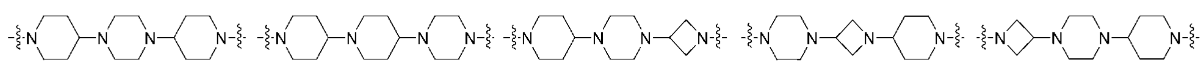
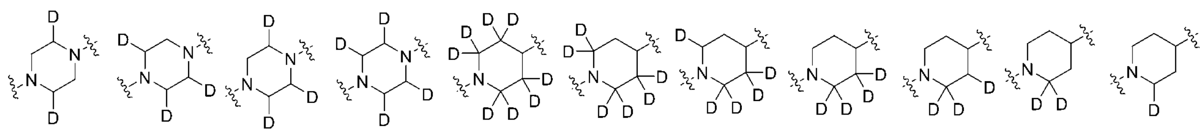
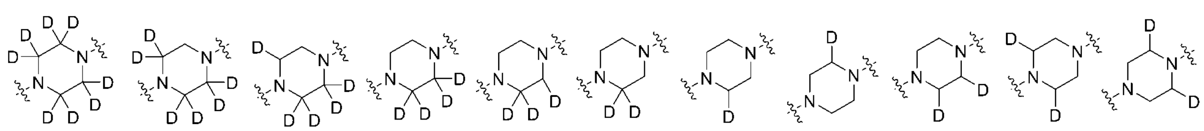
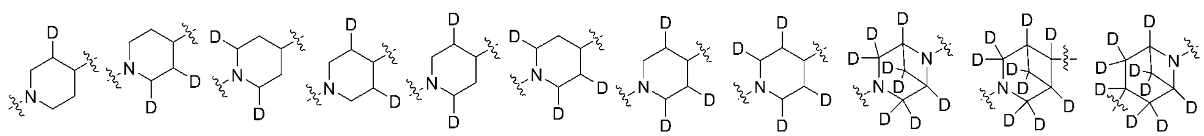
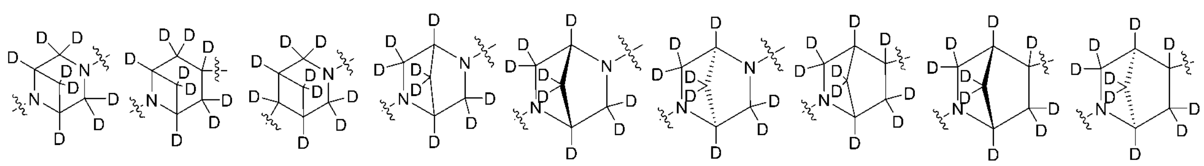
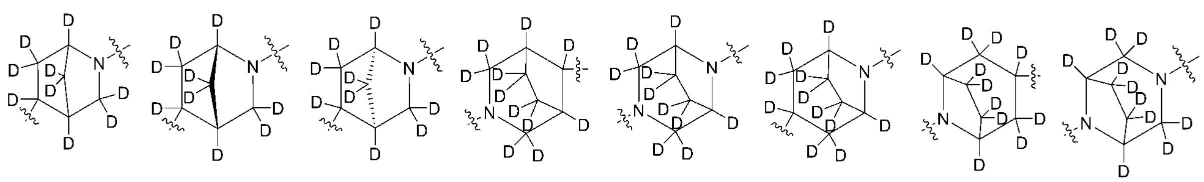

or

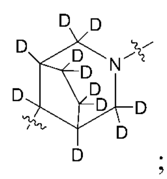

;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, $C_{2-6}$ alkynyl, and $C_{2-6}$ alkenyl;
and/or

$R_{w1}$ represents a bond, or

$R_{w1}$ represents :

$-C_{1-15}$ alkylene-;
$-(C(R_{a8})(R_{a9}))_{r1}-(R_a(C(Ra_{10})(R_{a11}))_{r2})_{s1}-*$;
$-(C(R_{a8})(R_{a9}))_{r1}-(R_a(C(Ra_{10})(R_{a11}))_{r2})_{s1}-(R_a(C(R_{a12})(R_{a13}))_{r3})_{s2}-*$;
$-(C(R_{a8})(R_{a9}))_{r1}-(R_a(C(R_{a10})(R_{a11}))_{r2})_{s1}-(R_a(C(R_{a12})(R_{a13}))_{r3})_{s2}-(R_a(C(R_{a14})(R_{a15}))_{r4})_{s3}-*$;
$-(C(R_{a8})(R_{a9}))_{r1}-((C(R_{a10})(R_{a11}))_{r2}R_a)_{s1}-*$;
$-(C(R_{a8})(R_{a9}))_{r1}-((C(R_{a10})(R_{a11}))_{r2}R_a)_{s1}-((C(R_{a12})(R_{a13}))_{r3}R_a)_{s2}-*$;
$-(C(R_{a8})(R_{a9}))_{r1}-((C(Ra_{10})(R_{a11}))_{r2}R_a)_{s1}-((C(Ra_{12})(R_{a13}))_{r3}R_a)_{s2}-((C(Ra_{14})(Ra_{15}))_{r4}R_a)_{s3}-*$;
$-(C(R_{a8})(R_{a9}))_{r1}-(R_b(C(R_{a10})(R_{a11}))_{r2})_{s1}-*$;
$-(C(R_{a8})(R_{a9}))_{r1}-(R_b(C(R_{a10})(R_{a11}))_{r2})_{s1}-(R_b(C(R_{a12})(R_{a13}))_{r3})_{s2}-*$;
$-(C(R_{a8})(R_{a9}))_{r1}-(R_b(C(R_{a10})(R_{a11}))_{r2})_{s1}-(R_b(C(R_{a12})(R_{a13}))_{r3})_{s2}-(R_b(C(R_{a14})(R_{a15}))_{r4})_{s3}-*$;
$-(C(R_{a8})(R_{a9}))_{r1}-((C(R_{a10})(R_{a11}))_{r2}R_b)_{s1}-*$;
$-(C(R_{a8})(R_{a9}))_{r1}-((C(Ra_{10})(R_{a11}))_{r2}R_b)_{s1}-((C(R_{a12})(R_{a13}))_{r3}R_b)_{s2}-*$;
$-(C(R_{a8})(R_{a9}))_{r1}-((C(R_{a10})(R_{a11}))_{r2}R_b)_{s1}-((C(R_{a12})(R_{a13}))_{r3}R_b)_{s2}-((C(R_{a14})(R_{a15}))_{r4}R_b)_{s3}-*$;
$-(C(R_{a8})(R_{a9}))_{r1}-(R_a-R_b-(C(R_{a10})(R_{a11}))_{r2})_{s1}-*$;
$-(C(R_{a8})(R_{a9}))_{r1}-(R_a(C(R_{a10})(R_{a11}))_{r2})_{s1}-(R_b(C(R_{a12})(R_{a13}))_{r3})_{s2}-*$;
$-(C(R_{a8})(R_{a9}))_{r1}-(R_b-R_a-(C(R_{a10})(R_{a11}))_{r2})_{s1}-*$;
$-(C(R_{a8})(R_{a9}))_{r1}-(R_b(C(R_{a10})(R_{a11}))_{r2})_{s1}-(R_a(C(R_{a12})(R_{a13}))_{r3})_{s2}-*$;
$-(C(R_{a8})(R_{a9}))_{r1}-((C(R_{a10})(R_{a11}))_{r2}-R_a-R_b)_{s1}-*$;
$-(C(R_{a8})(R_{a9}))_{r1}-((C(R_{a10})(R_{a11}))_{r2}R_a)_{s1}-((C(R_{a12})(R_{a13}))_{r3}R_b)_{s2}-*$;
$-(C(R_{a8})(R_{a9}))_{r1}-((C(R_{a10})(R_{a11}))_{r2}-R_b-R_a)_{s1}-*$; or
$-(C(R_{a8})(R_{a9}))_{r1}-((C(R_{a10})(R_{a11}))_{r2}R_b)_{s1}-((C(R_{a12})(R_{a13}))_{r3}R_a)_{s2}-*$;

wherein each $R_a$ is independently selected from the group consisting of O, C(O), OC(O), S, S(O), $S(O)_2$, $S(O)_2N(R_{a7})$, $N(R_{a7})S(O)_2$, $C(O)N(R_{a7})$, $N(R_{a7})C(O)$, $N(R_{a7})$, and $N(R_{a7})C(O)N(R_{a7})$, where each $R_{a7}$ independently represents H or $C_{1-6}$ alkyl; and each $R_b$ is independently selected from the group consisting of optionally substituted cycloalkylene (e.g., optionally substituted $C_{3-20}$ cycloalkylene), optionally substituted arylene (e.g., optionally substituted $C_{5-20}$ arylene), optionally substituted heterocyclylene (e.g., optionally substituted 4- to 20-membered heterocyclylene), optionally substituted heteroarylene (e.g., 5- to 20-membered heteroarylene), alkynylene (e.g., $C_{2-6}$ alkynylene), alkenylene (e.g., $C_{2-6}$ alkenylene), and any combination thereof, wherein the cycloalkylene, arylene, heterocyclylene, and heteroarylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;

$R_{a8}$, $R_{a9}$, $R_{a10}$, $R_{a11}$, $R_{a12}$, $R_{a13}$, $R_{a14}$, and $R_{a15}$ each independently represent H, deuterium, hydroxy, amino, mercapto, nitro, halogen, cyano, oxo, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; and

r1, r2, r3, r4, s1, s2, and s3 each independently represent an integer of 1, 2, 3, 4, 5, 6, 7, 8, 9 or 10;

symbol * indicates the point of attachment to R; and

any one or more hydrogens on the main carbon chain skeleton of said $C_{1-15}$ alkylene are optionally replaced by a substituent selected from: deuterium, hydroxy, amino, mercapto, nitro, halogen, cyano, oxo, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.

**5.** The compound of Formula (I) or salts, enantiomers, stereoisomers, solvates, or polymorphs thereof as claimed in claim 1, wherein the $R_{w1}$ represents a structure of the following Formulae:

$-(C(R_{a8})(R_{a9}))_{r1}-(O(C(R_{a10})(R_{a11}))_{r2})_{s1}-*$;
$-(C(R_{a8})(R_{a9}))_{r1}-(O(C(R_{a10})(R_{a11}))_{r2})_{s1}-(O(C(R_{a12})(R_{a13}))_{r3})_{s2}-*$;
$-(C(R_{a8})(R_{a9}))_{r1}-(O(C(R_{a10})(R_{a11}))_{r2})_{s1}-(O(C(R_{a12})(R_{a13}))_{r3})_{s2}-(O(C(R_{a14})(R_{a15}))_{r4})_{s3}-*$;
$-(C(R_{a8})(R_{a9}))_{r1}-((C(R_{a10})(R_{a11}))_{r2}O)_{s1}-*$;
$-(C(R_{a8})(R_{a9}))_{r1}-((C(R_{a10})(R_{a11}))_{r2}O)_{s1}-((C(R_{a12})(R_{a13}))_{r3}O)_{s2}-*$;
$-(C(R_{a8})(R_{a9}))_{r1}-((C(R_{a10})(R_{a11}))_{r2}O)_{s1}-((C(R_{a12})(R_{a13}))_{r3}O)_{s2}-((C(R_{a14})(R_{a15}))_{r4}O)_{s3}-*$;
$-(C(R_{a8})(R_{a9}))_{r1}-(N(R_{a7})(C(R_{a10})(R_{a11}))_{r2})_{s1}-*$;
$-(C(R_{a8})(R_{a9}))_{r1}-(N(R_{a7})(C(R_{a10})(R_{a11}))_{r2})_{s1}-(N(R_{a7})(C(R_{a12})(R_{a13}))_{r3})_{s2}-*$;
$-(C(R_{a8})(R_{a9}))_{r1}-(N(R_{a7})(C(R_{a10})(R_{a11}))_{r2})_{s1}-(N(R_{a7})(C(R_{a12})(R_{a13}))_{r3})_{s2}-(N(R_{a7})(C(R_{a14})(R_{a15}))_{r4})_{s3}-*$;

$-(C(R_{a8})(R_{a9}))_{r1}-((C(R_{a10})(R_{a11}))_{r2}N(R_{a7}))_{s1}-*$;
$-(C(R_{a8})(R_{a9}))_{r1}-((C(R_{a10})(R_{a11}))_{r2}N(R_{a7}))_{s1}-((C(R_{a12})(R_{a13}))_{r3}N(R_{a7}))_{s2}-*$;
$-(C(R_{a8})(R_{a9}))_{r1}-((C(R_{a10})(R_{a11}))_{r2}N(R_{a7}))_{s1}-((C(R_{a12})(R_{a13}))_{r3}N(R_{a7}))_{s2}-((C(R_{a14})(R_{a15}))_{r4}N(R_{a7}))_{s3}-*$;
$-(C(R_{a8})(R_{a9}))_{r1}-(C(O)N(R_{a7})-(C(R_{a10})(R_{a11}))_{r2})_{s1}-*$;
$-(C(R_{a8})(R_{a9}))_{r1}-(C(O)N(R_{a7})-(C(R_{a10})(R_{a11}))_{2})_{s1}-(C(O)N(R_{a7})-(C(R_{a12})(R_{a13}))_{s3})_{s2}-*$;
$-(C(R_{a8})(R_{a9}))_{r1}-(C(O)N(R_{a7})-(C(R_{a10})(R_{a11}))_{r2})_{s1}-(C(O)N(R_{a7})-(C(R_{a12})(R_{a13}))_{r3})_{s2}-(C(O)N(R_{a7})-(C(R_{a14})(R_{a15}))_{r4})_{s3}-*$;
$-(C(R_{a8})(R_{a9}))_{r1}-((C(R_{a10)}(R_{a11}))_{2}-C(O)N(R_{a7}))_{s1}-*$;
$-(C(R_{a8})(R_{a9}))_{r1}-((C(R_{a10})(R_{a11}))_{r2}-C(O)N(R_{a7}))_{s1}-((C(R_{a12})(R_{a13}))_{r3}-C(O)N(R_{a7}))_{s2}-*$;
$-(C(R_{a8})(R_{a9}))_{r1}-((C(R_{a10})(R_{a11}))_{r2}-C(O)N(R_{a7}))_{s1}-((C(R_{a12})(R_{a13}))_{r3}-C(O)N(R_{a7}))_{s2}-((C(R_{a14})(R_{a15}))_{r4}-C(O)N(R_{a7}))_{s3}-*$;
$-(C(R_{a8})(R_{a9}))_{r1}-(C(O)N(R_{a7})-(C(R_{a10})(R_{a11}))_{r2})_{s1}-(O-(C(R_{a12})(R_{a13}))_{r3})_{s2}-*$;
$-(C(R_{a8})(R_{a9}))_{r1}-C(O)N(R_{a7})-(C(R_{a10})(R_{a11}))_{r2}-(O(C(R_{a12})(R_{a13}))_{r3})_{s1}-*$;
$-(C(R_{a8})(R_{a9}))_{r1}-((C(R_{a10})(R_{a11}))_{2}-C(O)N(R_{a7}))_{s1}-( (C(R_{a12})(R_{a13}))_{r3}O)_{s2}-*$;
$-(C(R_{a8})(R_{a9}))_{r1}-(C(R_{a10})(R_{a11}))_{r2}-C(O)N(R_{a7})-((C(R_{a12})(R_{a13}))_{r3}O)_{s1}-*$;
$-(C(R_{a8})(R_{a9}))_{r1}-(N(R_{a7})C(O)-(C(R_{a10})(R_{a11}))_{r2})_{s1}-*$;
$-(C(R_{a8})(R_{a9}))_{r1}-(N(R_{a7})C(O)-(C(R_{a10})(R_{a11}))_{r2})_{s1}-(O-(C(R_{a12})(R_{a13})))_{r3})_{s2}-*$;
$-(C(R_{a8})(R_{a9}))_{r1}-(N(R_{a7})C(O)-(C(R_{a10})(R_{a11}))_{r2})_{s1}-(O-(C(R_{a12})(R_{a13}))_{r3})_{s2}-(O-(C(R_{a14})(R_{a15}))_{r4})_{s3}-*$;
$-(C(R_{a8})(R_{a9}))_{r1}-N(R_{a7})C(O)-(C(R_{a10})(R_{a11}))_{r2}-(O(C(R_{a12})(R_{a13}))_{r3})_{s1}-*$;
$-(C(R_{a8})(R_{a9}))_{r1}-N(R_{a7})C(O)N(R_{a7})-(C(R_{a10})(R_{a11}))_{r2}-*$;
$-(C(R_{a8})(R_{a9}))_{r1}-((C(R_{a10})(R_{a11}))_{r2}-N(R_{a7})C(O))_{s1}-*$;
$-(C(R_{a8})(R_{a9}))_{r1}-((C(R_{a10})(R_{a11}))_{r2}-N(R_{a7})C(O))_{s1}-((C(R_{a12})(R_{a13})))_{r3}-O)_{s2}-*$;
$-(C(R_{a8})(R_{a9}))_{r1}-((C(R_{a10})(R_{a11}))_{r2}-N(R_{a7})C(O))_{s1}-((C(R_{a12})(R_{a13}))_{r3}O)_{s2}-((C(R_{a14})(R_{a15}))_{r4}O)_{s3}-*$;
$-(C(R_{a8})(R_{a9}))_{r1}- (C(R_{a10})(R_{a11}))_{r2}-N(R_{a7})C(O)-((C(R_{a12})(R_{a13}))_{r3}O)_{s1}-*$;
$-(C(R_{a8})(R_{a9}))_{r1}-$(arylene$-(C(R_{a10})(R_{a11}))_{r2})_{s1}-*$;
$-(C(R_{a8})(R_{a9}))_{r1}-$(arylene$-(C(R_{a10})(R_{a11}))_{r2})_{s1}-$ arylene$-(C(R_{a12})(R_{a13}))_{r3}-*$;
$-(C(R_{a8})(R_{a9}))_{r1}-((C(R_{a10})(R_{a11}))_{r2}-$ arylene$)_{s1}-*$;
$-(C(R_{a8})(R_{a9}))_{r1}-((C(R_{a10})(R_{a11}))_{r2}-$ arylene$)_{s1}-(C(R_{a12})(R_{a13}))_{r3}-$ arylene-*;
$-(C(R_{a8})(R_{a9}))_{r1}-$(heterocyclylene$-(C(R_{a10})(R_{a11}))_{r2})_{s1}-*$;
$-(C(R_{a8})(R_{a9}))_{r1}-$(heterocyclylene$-(C(R_{a10})(R_{a11}))_{r2})_{s1}-$(heterocyclylene$-(C(R_{a12})(R_{a13}))_{r3})_{s2}-*$;
$-(C(R_{a8})(R_{a9}))_{r1}-((C(R_{a10})(R_{a11}))_{r2}-$ heterocyclylene$)_{s1}-*$;
$-(C(R_{a8})(R_{a9}))_{r1}-((C(R_{a10})(R_{a11}))_{r2}-$ heterocyclylene$)_{s1}-((C(R_{a12})(R_{a13}))_{r3}-$ heterocyclylene$)_{s2}-*$;
$-(C(R_{a8})(R_{a9}))_{r1}-$(heteroarylene$-(C(R_{a10})(R_{a11}))_{r2})_{s1}-*$;
$-(C(R_{a8})(R_{a9}))_{r1}-$(heteroarylene$-(C(R_{a10})(R_{a11}))_{r2})_{s1}-($ heteroarylene$-(C(R_{a12})(R_{a13}))_{r3})_{s2}-*$;
$-(C(R_{a8})(R_{a9}))_{r1}-((C(R_{a10})(R_{a11}))_{r2}-$ heteroarylene$)_{s1}-*$;
$-(C(R_{a8})(R_{a9}))_{r1}-((C(R_{a10})(R_{a11}))_{r2}-$ heteroarylene$)_{s1}-((C(R_{a12})(R_{a13}))_{r3}-$ heteroarylene$)_{s2}-*$;
$-(C(R_{a8})(R_{a9}))_{r1}-($ cycloalkylene$-(C(R_{a10})(R_{a11}))_{r2})_{s1}-*$;
$-(C(R_{a8})(R_{a9}))_{r1}-$(cycloalkylene$-(C(R_{a10})(R_{a11}))_{r2})_{s1}-$(cycloalkylene$-(C(R_{a12})(R_{a13}))_{r3})_{s2}-*$;
$-(C(R_{a8})(R_{a9}))_{r1}-((C(R_{a10})(R_{a11}))_{r2}-$ cycloalkylene$)_{s1}-*$; or
$-(C(R_{a8})(R_{a9}))_{r1}-((C(R_{a10})(R_{a11}))_{r2}-$ cycloalkylene$)_{s1}-((C(R_{a12})(R_{a13}))_{r3}-$ cycloalkylene$)_{s2}-*$;
wherein each $R_{a7}$ independently represents H or $C_{1-6}$ alkyl;

the cycloalkylene (e.g., $C_{3-20}$ cycloalkylene), arylene (e.g., $C_{5-20}$ arylene), heterocyclylene (e.g., 4- to 20-membered heterocyclylene), and heteroarylene (e.g., 5- to 20-membered heteroarylene) are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;

$R_{a8}$, $R_{a9}$, $R_{a10}$, $R_{a11}$, $R_{a12}$, $R_{a13}$, $R_{a14}$, and $R_{a15}$ each independently represent H, deuterium, hydroxy, amino, mercapto, nitro, halogen, cyano, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; and

r1, r2, r3, r4, s1, s2, and s3 each independently represent an integer of 1, 2, 3, 4, 5, 6, 7, 8, 9 or 10; and symbol * indicates the point of attachment to R.

**6.** The compound of Formula (I) or salts, enantiomers, stereoisomers, solvates, or polymorphs thereof as claimed in claim 1, wherein $R_{w1}$ represents:
$-CH_2-$, $-(CH_2)_2-$, $-(CH_2)_3-$, $-(CH_2)_4-$, $-(CH_2)_5-$, $-(CH_2)_6-$, $-(CH_2)_7-$, $-(CH_2)_8-$, $-(CH_2)_9-$, $-(CH_2)_{10}-$, $-(CH_2)_{11}-$, $-(CH_2)_{12}-$, $-(CH_2)_{13}-$, $-(CH_2)_{14}-$, $-(CH_2)_{15}-$; $-O-CH_2-$, $-O-(CH_2)_2-$, $-O-(CH_2)_3-$, $-O-(CH_2)_4-$, $-O-(CH_2)_5-$, $-O-(CH_2)_6-$, $-O-(CH_2)_7-$,

-O-(CH$_2$)$_8$-, -O-(CH$_2$)$_9$-, -O-(CH$_2$)$_{10}$-, -(CH$_2$)$_1$-O-, -(CH$_2$)$_2$-O-, -(CH$_2$)$_3$-O-, -(CH$_2$)$_4$-O-, -(CH$_2$)$_5$-O-, -(CH$_2$)$_6$-O-, -(CH$_2$)$_7$-O-, -(CH$_2$)$_8$-O-, -(CH$_2$)$_9$-O-, -(CH$_2$)$_{10}$-O-, -CH$_2$-O-CH$_2$-, - CH$_2$-O-(CH$_2$)$_2$-, -(CH$_2$)$_1$-O-(CH$_2$)$_3$-, -(CH$_2$)$_1$-O-(CH$_2$)$_4$-, -(CH$_2$)$_1$-O-(CH$_2$)$_5$-, -(CH$_2$)$_1$-O-(CH$_2$)$_6$-, -(CH$_2$)$_1$-O-(CH$_2$)$_7$-, -(CH$_2$)$_1$-O-(CH$_2$)$_8$-, -(CH$_2$)$_1$-O-(CH$_2$)$_9$-, -(CH$_2$)$_1$-O-(CH$_2$)$_{10}$-, -(CH$_2$)$_2$-O-(CH$_2$)$_1$-, -(CH$_2$)$_2$-O-(CH$_2$)$_2$-, -(CH$_2$)$_2$-O-(CH$_2$)$_3$-, -(CH$_2$)$_2$-O-(CH$_2$)$_4$-, -(CH$_2$)$_2$-O-(CH$_2$)$_5$-, -(CH$_2$)$_2$-O-(CH$_2$)$_6$-, -(CH$_2$)$_2$-O-(CH$_2$)$_7$-, -(CH$_2$)$_2$-O-(CH$_2$)$_8$-, -(CH$_2$)$_2$-O-(CH$_2$)$_9$-, -(CH$_2$)$_2$-O-(CH$_2$)$_{10}$-, -(CH$_2$)$_3$-O-(CH$_2$)$_1$-, -(CH$_2$)$_3$-O-(CH$_2$)$_2$-, - (CH$_2$)$_3$-O-(CH$_2$)$_3$-, -(CH$_2$)$_3$-O-(CH$_2$)$_4$-, -(CH$_2$)$_3$-O-(CH$_2$)$_5$-, -(CH$_2$)$_3$-O-(CH$_2$)$_6$-, -(CH$_2$)$_3$-O-(CH$_2$)$_7$-, -(CH$_2$)$_4$-O-(CH$_2$)$_1$-, -(CH$_2$)$_4$-O-(CH$_2$)$_2$-, -(CH$_2$)$_4$-O-(CH$_2$)$_3$-, -(CH$_2$)$_4$-O-(CH$_2$)$_4$-, -(CH$_2$)$_4$-O-(CH$_2$)$_5$-, -(CH$_2$)$_4$-O-(CH$_2$)$_6$-, -(CH$_2$)$_5$-O-(CH$_2$)$_1$-, -(CH$_2$)$_5$-O-(CH$_2$)$_2$-, -(CH$_2$)$_5$-O-(CH$_2$)$_3$-, -(CH$_2$)$_5$-O-(CH$_2$)$_4$-, -(CH$_2$)$_5$-O-(CH$_2$)$_5$-, -(CH$_2$)$_6$-O-(CH$_2$)$_1$-, -(CH$_2$)$_6$-O-(CH$_2$)$_2$-, -(CH$_2$)$_6$-O-(CH$_2$)$_3$-, -(CH$_2$)$_6$-O-(CH$_2$)$_4$-, -(CH$_2$)$_7$-O-(CH$_2$)$_1$-, -(CH$_2$)$_7$-O-(CH$_2$)$_2$-, -(CH$_2$)$_7$-O-(CH$_2$)$_3$-, -(CH$_2$)$_8$-O-(CH$_2$)$_1$-, -(CH$_2$)$_8$-O-(CH$_2$)$_2$-, -CH(CH$_3$)-O-(CH$_2$)$_1$-, -CH(CH$_3$)-O-(CH$_2$)$_2$-, -CH(CH$_3$)-O-(CH$_2$)$_3$-, -CH(CH$_3$)-O-(CH$_2$)$_4$-, -CH(CH$_3$)-O-(CH$_2$)$_5$-, -CH(CH$_3$)-O-(CH$_2$)$_6$-, -CH(CH$_3$)-O-(CH$_2$)$_7$-, -CH(CH$_3$)-O-(CH$_2$)$_8$-, -CH(CH$_3$)-O-(CH$_2$)$_9$-, -CH(CH$_3$)-O-(CH$_2$)$_{10}$-, - (O(CH$_2$)$_2$)$_2$-, -(O(CH$_2$)$_2$)$_3$-, -(O(CH$_2$)$_2$)$_4$-, -(O(CH$_2$)$_2$)$_5$-, -(O(CH$_2$)$_2$)$_6$-, -(O(CH$_2$)$_2$)$_7$-, -CH$_2$-(O(CH$_2$)$_2$)$_2$-, - CH$_2$-(O(CH$_2$)$_2$)$_3$-, -CH$_2$-(O(CH$_2$)$_2$)$_4$-, -CH$_2$-(O(CH$_2$)$_2$)$_5$-, -CH$_2$-(O(CH$_2$)$_2$)$_6$-, -CH$_2$-(O(CH$_2$)$_2$)$_7$-, -CH$_2$-(O(CH$_2$)$_2$)$_1$-OCH$_2$-, -CH$_2$-(O(CH$_2$)$_2$)$_2$-OCH$_2$-, -CH$_2$-(O(CH$_2$)$_2$)$_3$-OCH$_2$-, -CH$_2$-(O(CH$_2$)$_2$)$_4$-OCH$_2$-, -CH$_2$-(O(CH$_2$)$_2$)$_5$-OCH$_2$-, -(CH$_2$)$_2$-(O(CH$_2$)$_2$)$_2$-, -(CH$_2$)$_2$-(O(CH$_2$)$_2$)$_3$-, -(CH$_2$)$_2$-(O(CH$_2$)$_2$)$_4$-, -(CH$_2$)$_2$-(O(CH$_2$)$_2$)$_5$-, - (CH$_2$)$_2$-(O(CH$_2$)$_2$)$_6$-, -(CH$_2$)$_3$-(O(CH$_2$)$_2$)$_2$-, -(CH$_2$)$_3$-(O(CH$_2$)$_2$)$_3$-, -(CH$_2$)$_3$-(O(CH$_2$)$_2$)$_4$-, -(CH$_2$)$_3$-(O(CH$_2$)$_2$)$_5$-, -(CH$_2$)$_4$-(O(CH$_2$)$_2$)$_2$-, -(CH$_2$)$_4$-(O(CH$_2$)$_2$)$_3$-, -(CH$_2$)$_4$-(O(CH$_2$)$_2$)$_4$-, -(CH$_2$)$_4$-(O(CH$_2$)$_2$)$_5$-, -CH$_2$-(O(CH$_2$)$_3$)$_2$-, - CH$_2$-(O(CH$_2$)$_3$)$_3$-, -CH$_2$-(O(CH$_2$)$_3$)$_4$-, -(CH$_2$)$_2$-(O(CH$_2$)$_3$)$_2$-, -(CH$_2$)$_2$-(O(CH$_2$)$_3$)$_3$-, -(CH$_2$)$_2$-(O(CH$_2$)$_3$)$_4$-, - (CH$_2$)$_3$-(O(CH$_2$)$_3$)$_2$-, -(CH$_2$)$_3$-(O(CH$_2$)$_3$)$_3$-, -(CH$_2$)$_3$-(O(CH$_2$)$_3$)$_4$-, -CH$_2$-O-(CH$_2$)$_2$-O-(CH$_2$)$_3$-, -CH$_2$-(O(CH$_2$)$_2$)$_2$-(O(CH$_2$)$_3$)$_2$-, (CH$_2$)$_2$-O-(CH$_2$)$_2$-O-(CH$_2$)$_3$-, -(CH$_2$)$_2$-(O(CH$_2$)$_2$)$_2$-(O(CH$_2$)$_3$)$_2$-, -(CH$_2$)$_3$-O-(CH$_2$)$_2$-O-(CH$_2$)$_3$-, -(CH$_2$)$_3$-(O(CH$_2$)$_2$)$_2$-(O(CH$_2$)$_3$)$_2$-, -CH$_2$-O-(CH$_2$)$_3$-O-(CH$_2$)$_2$-, -CH$_2$-(O(CH$_2$)$_3$)$_2$-(O(CH$_2$)$_2$)$_2$-, - (CH$_2$)$_2$-O-(CH$_2$)$_3$-O-(CH$_2$)$_2$-, -(CH$_2$)$_2$-(O(CH$_2$)$_3$)$_2$-(O(CH$_2$)$_2$)$_2$-, -(CH$_2$)$_3$-O-(CH$_2$)$_3$-O-(CH$_2$)$_2$-, -(CH$_2$)$_3$-(O(CH$_2$)$_3$)$_2$-(O(CH$_2$)$_2$)$_2$-, -CH$_2$-O-(CH$_2$)$_2$-O-CH$_2$-, -(CH$_2$)$_2$-O-(CH$_2$)$_2$-O-CH$_2$-, -(CH$_2$)$_2$-(O(CH$_2$)$_2$)$_2$-O-(CH$_2$)$_3$-, -(CH$_2$)$_2$-(O(CH$_2$)$_2$)$_3$-O-(CH$_2$)$_3$-, -(CH$_2$)$_2$-(O(CH$_2$)$_2$)$_4$-O-(CH$_2$)$_3$-, -(CH$_2$)$_5$-(O(CH$_2$)$_2$)$_2$-O-(CH$_2$)$_5$-, - (CH$_2$)$_5$-(O(CH$_2$)$_2$)$_2$-O-(CH$_2$)$_6$-, -(CH$_2$)$_1$-N(R$_{a7}$)-(CH$_2$)$_1$-, -(CH$_2$)$_1$-N(R$_{a7}$)-(CH$_2$)$_2$-, -(CH$_2$)$_1$-N(R$_{a7}$)-(CH$_2$)$_3$-, - (CH$_2$)$_1$-N(R$_{a7}$)-(CH$_2$)$_4$-, -(CH$_2$)$_1$-N(R$_{a7}$)-(CH$_2$)$_5$-, -(CH$_2$)$_1$-N(R$_{a7}$)-(CH$_2$)$_6$-, -(CH$_2$)$_1$-N(R$_{a7}$)-(CH$_2$)$_7$-, -(CH$_2$)$_1$-N(R$_{a7}$)-(CH$_2$)$_8$-, -(CH$_2$)$_1$-N(R$_{a7}$)-(CH$_2$)$_9$-, -(CH$_2$)$_1$-N(R$_{a7}$)-(CH$_2$)$_{10}$-, -N(R$_{a7}$)-(CH$_2$)$_1$-, -N(R$_{a7}$)-(CH$_2$)$_2$-, - N(R$_{a7}$)-(CH$_2$)$_3$-, -N(R$_{a7}$)-(CH$_2$)$_4$-, -N(R$_{a7}$)-(CH$_2$)$_5$-, -N(R$_{a7}$)-(CH$_2$)$_6$-, -N(R$_{a7}$)-(CH$_2$)$_7$-, -N(R$_{a7}$)-(CH$_2$)$_8$-, - N(R$_{a7}$)-(CH$_2$)$_9$-, -N(R$_{a7}$)-(CH$_2$)$_{10}$-, -(CH$_2$)$_2$-N(R$_{a7}$)-(CH$_2$)$_1$-, -(CH$_2$)$_2$-N(R$_{a7}$)-(CH$_2$)$_2$-, -(CH$_2$)$_2$-N(R$_{a7}$)-(CH2)3-, -(CH$_2$)$_2$-N(R$_{a7}$)-(CH$_2$)$_4$-, -(CH$_2$)$_2$-N(R$_{a7}$)-(CH$_2$)$_5$-, -(CH$_2$)$_2$-N(R$_{a7}$)-(CH$_2$)$_6$-, -(CH$_2$)$_2$-N(R$_{a7}$)-(CH$_2$)$_7$-, -(CH$_2$)$_2$-N(R$_{a7}$)-(CH$_2$)$_8$-, -(CH$_2$)$_2$-N(R$_{a7}$)-(CH$_2$)$_9$-, -(CH$_2$)$_2$-N(R$_{a7}$)-(CH$_2$)$_{10}$-, -(CH$_2$)$_3$-N(R$_{a7}$)-(CH$_2$)$_1$-, - (CH$_2$)$_3$-N(R$_{a7}$)-(CH$_2$)$_2$-, -(CH$_2$)$_3$-N(R$_{a7}$)-(CH$_2$)$_3$-, -(CH$_2$)$_4$-N(R$_{a7}$)-(CH$_2$)$_1$-, -(CH$_2$)$_4$-N(R$_{a7}$)-(CH$_2$)$_2$-, -(CH$_2$)$_4$-N(R$_{a7}$)-(CH$_2$)$_3$-, -(CH$_2$)$_4$-N(R$_{a7}$)-(CH$_2$)$_4$-, -(CH$_2$)$_5$-N(R$_{a7}$)-(CH$_2$)$_1$-, -(CH$_2$)$_5$-N(R$_{a7}$)-(CH$_2$)$_2$-, -(CH$_2$)$_5$-N(R$_{a7}$)-(CH2)3-, -(CH$_2$)$_5$-N(R$_{a7}$)-(CH$_2$)$_4$-, -(CH$_2$)$_5$-N(R$_{a7}$)-(CH$_2$)$_5$-, -(CH$_2$)$_6$-N(R$_{a7}$)-(CH$_2$)$_1$-, -(CH$_2$)$_6$-N(R$_{a7}$)-(CH$_2$)$_2$-, -(CH$_2$)$_6$-N(R$_{a7}$)-(CH$_2$)$_3$-, -(CH$_2$)$_7$-N(R$_{a7}$)-(CH$_2$)$_1$-, -(CH$_2$)$_7$-N(R$_{a7}$)-(CH$_2$)$_2$-, -(CH$_2$)$_7$-N(R$_{a7}$)-(CH$_2$)$_3$-, - (CH$_2$)$_8$-N(R$_{a7}$)-(CH$_2$)$_1$-, -(CH$_2$)$_8$-N(R$_{a7}$)-(CH$_2$)$_2$-, -(CH$_2$)$_8$-N(R$_{a7}$)-(CH$_2$)$_3$-, -CH(CH$_3$)-N(R$_{a7}$)-(CH$_2$)$_1$-, - CH(CH$_3$)-N(R$_{a7}$)-(CH$_2$)$_2$-, -CH(CH$_3$)-N(R$_{a7}$)-(CH$_2$)$_3$-, -CH(CH$_3$)-N(R$_{a7}$)-(CH$_2$)$_4$-, -CH(CH$_3$)-N(R$_{a7}$)-(CH$_2$)$_5$-, -CH(CH$_3$)-N(R$_{a7}$)-(CH$_2$)$_6$-, -CH(CH$_3$)-N(R$_{a7}$)-(CH$_2$)$_7$-, -CH(CH$_3$)-N(R$_{a7}$)-(CH$_2$)$_8$-, -CH(CH$_3$)-N(R$_{a7}$)-(CH$_2$)$_9$-, -CH(CH$_3$)-N(R$_{a7}$)-(CH$_2$)$_{10}$-, -(CH$_2$)$_1$-N(R$_{a7}$)-, -(CH$_2$)$_2$-N(R$_{a7}$)-, -(CH$_2$)$_3$-N(R$_{a7}$)-, -(CH$_2$)$_4$-N(R$_{a7}$)-, -(CH$_2$)$_5$-N(R$_{a7}$)-, -(CH$_2$)$_6$-N(R$_{a7}$)-, -(CH$_2$)$_7$-N(R$_{a7}$)-, -(CH$_2$)$_8$-N(R$_{a7}$)-, -(CH$_2$)$_9$-N(R$_{a7}$)-, -(CH$_2$)$_{10}$-N(R$_{a7}$)-, -C(O)NHCH$_2$-, -C(O)NH(CH$_2$)$_2$-, -C(O)NH(CH$_2$)$_3$-, -C(O)NH(CH$_2$)$_4$-, -C(O)NH(CH$_2$)$_5$-, - CH$_2$C(O)NHCH$_2$-, -(CH$_2$)$_2$C(O)NH(CH$_2$)$_2$-, -(CH$_2$)$_2$C(O)NH(CH$_2$)$_3$-, -(CH$_2$)$_2$C(O)NH(CH$_2$)$_4$-, - (CH$_2$)$_2$C(O)NH(CH$_2$)$_5$-, -(CH$_2$)$_3$C(O)NH(CH$_2$)$_3$-, -(CH$_2$)$_3$C(O)NH(CH$_2$)$_4$-, -(CH$_2$)$_4$C(O)NH(CH$_2$)$_4$-, - (CH$_2$)$_5$C(O)NH(CH$_2$)$_5$-, -(CH$_2$)$_6$C(O)NH(CH$_2$)$_7$-, -(CH$_2$)$_6$C(O)NH(CH$_2$)$_6$-, -(CH$_2$)$_7$C(O)NH(CH$_2$)$_7$-, - (CH$_2$)$_2$C(O)NH(CH$_2$)$_2$-O-(CH$_2$)$_2$-, -NHC(O)CH$_2$-, -NHC(O)(CH$_2$)$_2$-, -NHC(O)(CH$_2$)$_3$-, -NHC(O)(CH$_2$)$_4$-, - NHC(O)(CH$_2$)$_5$-, -CH$_2$NHC(O)CH$_2$-, -(CH$_2$)$_2$NHC(O)(CH$_2$)$_2$-, -(CH$_2$)$_2$NHC(O)(CH$_2$)$_3$-, - (CH$_2$)$_2$NHC(O)(CH$_2$)$_4$-, -(CH$_2$)$_2$NHC(O)(CH$_2$)$_5$-, -(CH$_2$)$_3$NHC(O)(CH$_2$)$_3$-, -(CH$_2$)$_3$NHC(O)(CH$_2$)$_4$-, - (CH$_2$)$_4$NHC(O)(CH$_2$)$_4$-, -(CH$_2$)$_5$NHC(O)(CH$_2$)$_5$-, -(CH$_2$)$_6$NHC(O)(CH$_2$)$_7$-, -(CH$_2$)$_6$NHC(O)(CH$_2$)$_6$-, - (CH$_2$)$_7$NHC(O)(CH$_2$)$_7$-, -(CH$_2$)$_4$NHC(O)(CH$_2$)$_8$-, -(CH$_2$)$_2$NHC(O)(CH$_2$)$_2$-O-(CH$_2$)$_2$-, -(CH$_2$)$_4$NHC(O)CH$_2$-, -phenylene-CH$_2$-, -phenylene-(CH$_2$)$_2$-, -phenylene-(CH$_2$)$_3$-, -phenylene-(CH$_2$)$_4$-, -phenylene-(CH$_2$)$_5$-, - phenylene-(CH$_2$)$_6$-, -phenylene-(CH$_2$)$_7$-, -phenylene-(CH$_2$)$_8$-, -CH$_2$-phenylene-CH$_2$-, -CH$_2$-phenylene-(CH$_2$)$_2$-, -CH$_2$-phenylene-(CH$_2$)$_3$-, -CH$_2$-phenylene-(CH$_2$)$_4$-, -CH$_2$-phenylene-(CH$_2$)$_5$-, -CH$_2$-phenylene-(CH$_2$)$_6$-, -CH$_2$-phenylene-(CH$_2$)$_7$-, -CH$_2$-phenylene-(CH$_2$)$_8$-, -(CH$_2$)$_2$-phenylene-(CH$_2$)$_1$-, -(CH$_2$)$_2$-phenylene-(CH$_2$)$_2$-, -(CH$_2$)$_2$-phenylene-(CH$_2$)$_3$-, -(CH$_2$)$_2$-phenylene-(CH$_2$)$_4$-, -(CH$_2$)$_2$-phenylene-(CH$_2$)$_5$-, - (CH$_2$)$_2$-phenylene-(CH$_2$)$_6$-, -(CH$_2$)$_2$-phenylene-(CH$_2$)$_7$-, -(CH$_2$)$_2$-phenylene-(CH$_2$)$_8$-, -(CH$_2$)$_3$-phenylene-CH$_2$-, -(CH$_2$)$_3$-phenylene-(CH$_2$)$_2$-, -(CH$_2$)$_3$-phenylene-(CH$_2$)$_3$-, -(CH$_2$)$_3$-phenylene-(CH$_2$)$_4$-, -(CH$_2$)$_3$-phenylene-(CH$_2$)$_5$-, -(CH$_2$)$_3$-phenylene-(CH$_2$)$_6$-, -(CH$_2$)$_3$-phenylene-(CH$_2$)$_7$-, -(CH$_2$)$_3$-phenyle-

ne-$(CH_2)_8$-, - $(CH_2)_4$-phenylene-$CH_2$-, -$(CH_2)_4$-phenylene-$(CH_2)_2$-, -$(CH_2)_4$-phenylene-$(CH_2)_3$-, -$(CH_2)_4$-phenylene-$(CH_2)_4$-, -$(CH_2)_4$-phenylene-$(CH_2)_5$-, -$(CH_2)_4$-phenylene-$(CH_2)_6$-, -$(CH_2)_4$-phenylene-$(CH_2)_7$-, -$(CH_2)_4$-phenylene-$(CH_2)_8$-, -$(CH_2)_5$-phenylene-$(CH_2)_1$-, -$(CH_2)_5$-phenylene-$(CH_2)_2$-, -$(CH_2)_5$-phenylene-$(CH_2)_3$-, - $(CH_2)_5$-phenylene-$(CH_2)_4$-, -$(CH_2)_5$-phenylene-$(CH_2)_5$-, -$(CH_2)_5$-phenylene-$(CH_2)_6$-, -$(CH_2)_5$-phenylene-$(CH_2)_7$-, -$(CH_2)_5$-phenylene-$(CH_2)_9$-, -$(CH_2)_6$-phenylene-$(CH_2)_1$-, -$(CH_2)_6$-phenylene-$(CH_2)_2$-, -$(CH_2)_6$-phenylene-$(CH_2)_3$-, -$(CH_2)_6$-phenylene-$(CH_2)_4$-, -$(CH_2)_6$-phenylene-$(CH_2)_5$-, -$(CH_2)_6$-phenylene-$(CH_2)_6$-, - $(CH_2)_6$-phenylene-$(CH_2)_7$-, -$(CH_2)_6$-phenylene-$(CH_2)_8$-, -$(CH_2)_7$-phenylene-$(CH_2)_1$-, -$(CH_2)_7$-phenylene-$(CH_2)_2$-, -$(CH_2)_7$-phenylene-$(CH_2)_3$-, -$(CH_2)_7$-phenylene-$(CH_2)_4$-, -$(CH_2)_7$-phenylene-$(CH_2)_8$-, -$(CH_2)_9$-phenylene-$CH_2$-, -$(CH_2)_8$-phenylene-$(CH_2)_2$-, -$(CH_2)_8$-phenylene-$(CH_2)_3$-, -$(CH_2)_8$-phenylene-$(CH_2)_4$-, - $(CH_2)_8$-phenylene-$(CH_2)_5$-, -$(CH_2)_8$-phenylene-$(CH_2)_6$-, -$(CH_2)_8$-phenylene-$(CH_2)_7$-, -$N(R_{a7})$-$CH_2$-phenylene-$CH_2$-, -$N(R_{a7})$-$CH_2$-phenylene-$(CH_2)_2$-, - $N(R_{a7})$-$CH_2$-phenylene-$(CH_2)_3$-, -$N(R_{a7})$-$CH_2$-phenylene-$(CH_2)_4$-, -$N(R_{a7})$-$CH_2$-phenylene-$(CH_2)_5$-, -$N(R_{a7})$-$CH_2$-phenylene-$(CH_2)_6$-, -$N(R_{a7})$-$CH_2$-phenylene-$(CH_2)_7$-, -$N(R_{a7})$-$CH_2$-phenylene-$(CH_2)_8$-, -$CH_2$-$N(R_{a7})$-$CH_2$-phenylene-$CH_2$-, -$CH_2$-$N(R_{a7})$-$CH_2$-phenylene-$(CH_2)_2$-, -$CH_2$-$N(R_{a7})$-$CH_2$-phenylene-$(CH_2)_3$-, -$CH_2$-$N(R_{a7})$-$CH_2$-phenylene-$(CH_2)_4$-, - $CH_2$-$N(R_{a7})$-$CH_2$-phenylene-$(CH_2)_5$-, -$CH_2$-$N(R_{a7})$-$CH_2$-phenylene-$(CH_2)_6$-, -$CH_2$-$N(R_{a7})$-$CH_2$-phenylene-$(CH_2)_7$-, -$CH_2$-$N(R_{a7})$-$CH_2$-phenylene-$(CH_2)_8$-, -$CH_2$-$N(R_{a7})$-$(CH_2)_2$-phenylene-$CH_2$-, -$CH_2$-$N(R_{a7})$-$(CH_2)_2$-phenylene-$(CH_2)_2$-, -$CH_2$-$N(R_{a7})$-$(CH_2)_2$-phenylene-$(CH_2)_3$-, -$CH_2$-$N(R_{a7})$-$(CH_2)_2$-phenylene-$(CH_2)_4$-, - $CH_2$-$N(R_{a7})$-$(CH_2)_2$-phenylene-$(CH_2)_5$-, -$CH_2$-$N(R_{a7})$-$(CH_2)_2$-phenylene-$(CH_2)_6$-, -$CH_2$-$N(R_{a7})$-$(CH_2)_2$-phenylene-$(CH_2)_7$-, -$CH_2$-$N(R_{a7})$-$(CH_2)_2$-phenylene-$(CH_2)_8$-, -$(CH_2)_2$-$N(R_{a7})$-$CH_2$-phenylene-$CH_2$-, - $(CH_2)_2$-$N(R_{a7})$-$CH_2$-phenylene-$(CH_2)_2$-, -$(CH_2)_2$-$N(R_{a7})$-$CH_2$-phenylene-$(CH_2)_3$-, -$(CH_2)_2$-$N(R_{a7})$-$CH_2$-phenylene-$(CH_2)_4$-, -$(CH_2)_2$-$N(R_{a7})$-$CH_2$-phenylene-$(CH_2)_5$-, -$(CH_2)_2$-$N(R_{a7})$-$CH_2$-phenylene-$(CH_2)_6$-, - $(CH_2)_2$-$N(R_{a7})$-$CH_2$-phenylene-$(CH_2)_7$-, -$(CH_2)_2$-$N(R_{a7})$-$CH_2$-phenylene-$(CH_2)_8$-, -$(CH_2)_3$-$N(R_{a7})$-$CH_2$-phenylene-$CH_2$-, -$(CH_2)_3$-$N(R_{a7})$-$CH_2$-phenylene-$(CH_2)_2$-, -$(CH_2)_3$-$N(R_{a7})$-$CH_2$-phenylene-$(CH_2)_3$-, - $(CH_2)_3$-$N(R_{a7})$-$CH_2$-phenylene-$(CH_2)_8$-, -$(CH_2)_4$-$N(R_{a7})$-$CH_2$-phenylene-$CH_2$-, -$(CH_2)_4$-$N(R_{a7})$-$CH_2$-phenylene-$(CH_2)_2$-, -$(CH_2)_4$-$N(R_{a7})$-$CH_2$-phenylene-$(CH_2)_3$-, -$(CH_2)_4$-$N(R_{a7})$-$CH_2$-phenylene-$(CH_2)_8$-, - $(CH_2)_5$-$N(R_{a7})$-$CH_2$-phenylene-$(CH_2)_3$-, -$(CH_2)_5$-$N(R_{a7})$-$CH_2$-phenylene-$(CH_2)_8$-, -$(CH_2)_6$-$N(R_{a7})$-$CH_2$-phenylene-$(CH_2)_3$-, -$(CH_2)_6$-$N(R_{a7})$-$CH_2$-phenylene-$(CH_2)_8$-, -$(CH_2)_7$-$N(R_{a7})$-$CH_2$-phenylene-$(CH_2)_3$-, - $(CH_2)_8$-$N(R_{a7})$-$CH_2$-phenylene-$CH_2$-, -$(CH_2)_8$-$N(R_{a7})$-$CH_2$-phenylene-$(CH_2)_2$-, -$(CH_2)_8$-$N(R_{a7})$-$CH_2$-phenylene-$(CH_2)_3$-, -$(CH_2)_8$-$N(R_{a7})$-$CH_2$-phenylene-$(CH_2)_4$-, -$(CH_2)_8$-$N(R_{a7})$-$CH_2$-phenylene-$(CH_2)_5$-, - $(CH_2)_8$-$N(R_{a7})$-$CH_2$-phenylene-$(CH_2)_6$-, -piperidinylene-$CH_2$-, -piperidinylene-$(CH_2)_2$-, -piperidinylene-$(CH_2)_3$-, -piperidinylene-$(CH_2)_4$-, -piperidinylene-$(CH_2)_5$-, -piperidinylene-$(CH_2)_6$-, -piperidinylene-$(CH_2)_7$-, -piperidinylene-$(CH_2)_8$-, -$CH_2$-piperidinylene-$CH_2$-, -$CH_2$-piperidinylene-$(CH_2)_2$-, -$CH_2$-piperidinylene-$(CH_2)_3$-, -$CH_2$-piperidinylene-$(CH_2)_4$-, -$CH_2$-piperidinylene-$(CH_2)_5$-, -$CH_2$-piperidinylene-$(CH_2)_6$-, -$CH_2$-piperidinylene-$(CH_2)_7$-, -$CH_2$-piperidinylene-$(CH_2)_8$-, -$(CH_2)_2$-piperidinylene-$(CH_2)_1$-, - $(CH_2)_2$-piperidinylene-$(CH_2)_2$-, -$(CH_2)_2$-piperidinylene-$(CH_2)_3$-, -$(CH_2)_2$-piperidinylene-$(CH_2)_4$-, -$(CH_2)_2$-piperidinylene-$(CH_2)_5$-, -$(CH_2)_2$-piperidinylene-$(CH_2)_6$-, -$(CH_2)_2$-piperidinylene-$(CH_2)_7$-, -$(CH_2)_2$-piperidinylene-$(CH_2)_8$-, -$(CH_2)_3$-piperidinylene-$CH_2$-, -$(CH_2)_3$-piperidinylene-$(CH_2)_2$-, -$(CH_2)_3$-piperidinylene-$(CH_2)_3$-, -$(CH_2)_3$-piperidinylene-$(CH_2)_4$-, -$(CH_2)_3$-piperidinylene-$(CH_2)_5$-, -$(CH_2)_3$-piperidinylene-$(CH_2)_6$-, -$(CH_2)_3$-piperidinylene-$(CH_2)_7$-, -$(CH_2)_3$-piperidinylene-$(CH_2)_8$-, -$(CH_2)_4$-piperidinylene-$CH_2$-, -$(CH_2)_4$-piperidinylene-$(CH_2)_2$-, -$(CH_2)_4$-piperidinylene-$(CH_2)_3$-, -$(CH_2)_4$-piperidinylene-$(CH_2)_4$-, -$(CH_2)_4$-piperidinylene-$(CH_2)_5$-, -$(CH_2)_4$-piperidinylene-$(CH_2)_6$-, -$(CH_2)_4$-piperidinylene-$(CH_2)_7$-, -$(CH_2)_4$-piperidinylene-$(CH_2)_8$-, -$(CH_2)_5$-piperidinylene-$(CH_2)_1$-, -$(CH_2)_5$-piperidinylene-$(CH_2)_2$-, -$(CH_2)_5$-piperidinylene-$(CH_2)_3$-, -$(CH_2)_5$-piperidinylene-$(CH_2)_4$-, -$(CH_2)_5$-piperidinylene-$(CH_2)_5$-, -$(CH_2)_5$-piperidinylene-$(CH_2)_6$-, -$(CH_2)_5$-piperidinylene-$(CH_2)_7$-, -$(CH_2)_5$-piperidinylene-$(CH_2)_8$-, -$(CH_2)_6$-piperidinylene-$(CH_2)_1$-, -$(CH_2)_6$-piperidinylene-$(CH_2)_2$-, -$(CH_2)_6$-piperidinylene-$(CH_2)_3$-, -$(CH_2)_6$-piperidinylene-$(CH_2)_4$-, -$(CH_2)_6$-piperidinylene-$(CH_2)_5$-, -$(CH_2)_6$-piperidinylene-$(CH_2)_6$-, -$(CH_2)_6$-piperidinylene-$(CH_2)_7$-, -$(CH_2)_6$-piperidinylene-$(CH_2)_8$-, -$(CH_2)_7$-piperidinylene-$(CH_2)_1$-, -$(CH_2)_7$-piperidinylene-$(CH_2)_2$-, -$(CH_2)_7$-piperidinylene-$(CH_2)_3$-, -$(CH_2)_7$-piperidinylene-$(CH_2)_4$-, -$(CH_2)_7$-piperidinylene-$(CH_2)_8$-, -$(CH_2)_8$-piperidinylene-$CH_2$-, -$(CH_2)_8$-piperidinylene-$(CH_2)_2$-, -$(CH_2)_8$-piperidinylene-$(CH_2)_3$-, -$(CH_2)_8$-piperidinylene-$(CH_2)_4$-, -$(CH_2)_8$-piperidinylene-$(CH_2)_5$-, -$(CH_2)_8$-piperldinylene-$(CH_2)_6$-, -$(CH_2)_8$-piperidinylene-$(CH_2)_7$-, -$N(R_{a7})$-$CH_2$-piperidinylene-$CH_2$-, -$N(R_{a7})$-$CH_2$-piperidinylene-$(CH_2)_2$-, -$N(R_{a7})$-$CH_2$-piperidinylene-$(CH_2)_3$-, -$N(R_{a7})$-$CH_2$-piperidinylene-$(CH_2)_4$-, -$N(R_{a7})$-$CH_2$-piperidinylene-$(CH_2)_5$-, -$N(R_{a7})$-$CH_2$-piperidinylene-$(CH_2)_6$-, - $N(R_{a7})$-$CH_2$-piperidinylene-$(CH_2)_7$-, -$N(R_{a7})$-$CH_2$-piperidinylene-$(CH_2)_8$-, -$CH_2$-$N(R_{a7})$-$CH_2$-piperidinylene-$CH_2$-, -$CH_2$-$N(R_{a7})$-$CH_2$-piperidinylene-$(CH_2)_2$-, -$CH_2$-$N(R_{a7})$-$CH_2$-piperidinylene-$(CH_2)_3$-, -$CH_2$-$N(R_{a7})$-$CH_2$-piperidinylene-$(CH_2)_4$-, -$CH_2$-$N(R_{a7})$-$CH_2$-piperidinylene-$(CH_2)_5$-, -$CH_2$-$N(R_{a7})$-$CH_2$-piperidinylene-$(CH_2)_6$-, -$CH_2$-$N(R_{a7})$-$CH_2$-piperidinylene-$(CH_2)_7$-, -$CH_2$-$N(R_{a7})$-$CH_2$-piperidinylene-$(CH_2)_8$-, -$CH_2$-$N(R_{a7})$-$(CH_2)_2$-piperidinylene-$CH_2$-, -$CH_2$-$N(R_{a7})$-$(CH_2)_2$-piperidinylene-$(CH_2)_2$-, -$CH_2$-$N(R_{a7})$-$(CH_2)_2$-piperidinylene-$(CH_2)_3$-, -$CH_2$-$N(R_{a7})$-$(CH_2)_2$-piperidinylene-$(CH_2)_4$-, -$CH_2$-$N(R_{a7})$-$(CH_2)_2$-piperidinylene-$(CH_2)_5$-, -$CH_2$-$N(R_{a7})$-$(CH_2)_2$-piperidinylene-$(CH_2)_6$-, -$CH_2$-$N(R_{a7})$-$(CH_2)_2$-piperidinylene-$(CH_2)_7$-, -$CH_2$-$N(R_{a7})$-$(CH_2)_2$-piperidinylene-$(CH_2)_8$-, -$(CH_2)_2$-$N(R_{a7})$-$CH_2$-piperidinylene-$CH_2$-, -$(CH_2)_2$-$N(R_{a7})$-$CH_2$-piperidinylene-$(CH_2)_2$-, -$(CH_2)_2$-$N(R_{a7})$-$CH_2$-piperidinylene-$(CH_2)_3$-, -$(CH_2)_2$-$N(R_{a7})$-$CH_2$-piperidinylene-$(CH_2)_4$-, -$(CH_2)_2$-$N(R_{a7})$-$CH_2$-piperidinylene-$(CH_2)_5$-, -$(CH_2)_2$-$N(R_{a7})$-$CH_2$-piperidinylene-$(CH_2)_6$-, -$(CH_2)_2$-$N(R_{a7})$-$CH_2$-pi-

peridinylene-$(CH_2)_7$-, -$(CH_2)_2$-N($R_{a7}$)-$CH_2$-piperidinylene-$(CH_2)_8$-, - $(CH_2)_3$-N($R_{a7}$)-$CH_2$-piperidinylene-$CH_2$-, -$(CH_2)_3$-N($R_{a7}$)-$CH_2$-piperidinylene-$(CH_2)_2$-, -$(CH_2)_3$-N($R_{a7}$)-$CH_2$-piperidinylene-$(CH_2)_3$-, -$(CH_2)_3$-N($R_{a7}$)-$CH_2$-piperidinylene-$(CH_2)_8$-, -$(CH_2)_4$-N($R_{a7}$)-$CH_2$-piperidinylene-$CH_2$-, -$(CH_2)_4$-N($R_{a7}$)-$CH_2$-piperidinylene-$(CH_2)_2$-, -$(CH_2)_4$-N($R_{a7}$)-$CH_2$-piperidinylene-$(CH_2)_3$-, -$(CH_2)_4$-N($R_{a7}$)-$CH_2$-piperidinylene-$(CH_2)_8$-, -$(CH_2)_5$-N($R_{a7}$)-$CH_2$-piperidinylene-$(CH_2)_3$-, - $(CH_2)_5$-N($R_{a7}$)-$CH_2$-piperidinylene-$(CH_2)_8$-, -$(CH_2)_6$-N($R_{a7}$)-$CH_2$-piperidinylene-$(CH_2)_3$-, -$(CH_2)_6$-N($R_{a7}$)-$CH_2$-piperidinylene-$(CH_2)_8$-, -$(CH_2)_7$-N($R_{a7}$)-$CH_2$-piperidinylene-$(CH_2)_3$-, -$(CH_2)_8$-N($R_{a7}$)-$CH_2$-piperidinylene-$CH_2$-, -$(CH_2)_8$-N($R_{a7}$)-$CH_2$-piperidinylene-$(CH_2)_2$-, -$(CH_2)_8$-N($R_{a7}$)-$CH_2$-piperidinylene-$(CH_2)_3$-, -$(CH_2)_8$-N($R_{a7}$)-$CH_2$-piperidinylene-$(CH_2)_4$-, -$(CH_2)_8$-N($R_{a7}$)-$CH_2$-piperidinylene-$(CH_2)_5$-, - $(CH_2)_8$-N($R_{a7}$)-$CH_2$-piperidinylene-$(CH_2)_6$-, -piperazinylene-$CH_2$-, -piperazinylene-$(CH_2)_2$-, - piperazinylene-$(CH_2)_3$-, -piperazinylene-$(CH_2)_4$-, -piperazinylene-$(CH_2)_5$-, -piperazinylene-$(CH_2)_6$-, - piperazinylene-$(CH_2)_7$-, -piperazinylene-$(CH_2)_8$-, -$CH_2$-piperazinylene-$CH_2$-, -$CH_2$-piperazinylene-$(CH_2)_2$-, -$CH_2$-piperazinylene-$(CH_2)_3$-, -$CH_2$-piperazinylene-$(CH_2)_4$-, -$CH_2$-piperazinylene-$(CH_2)_5$-, -$CH_2$-piperazinylene-$(CH_2)_6$-, -$CH_2$-piperazinylene-$(CH_2)_7$-, -$CH_2$-piperazinylene-$(CH_2)_8$-, -$(CH_2)_2$-piperazinylene-$(CH_2)_1$-, -$(CH_2)_2$-piperazinylene-$(CH_2)_2$-, -$(CH_2)_2$-piperazinylene-$(CH_2)_3$-, -$(CH_2)_2$-piperazinylene-$(CH_2)_4$-, -$(CH_2)_2$-piperazinylene-$(CH_2)_5$-, -$(CH_2)_2$-piperazinylene-$(CH_2)_6$-, -$(CH_2)_2$-piperazinylene-$(CH_2)_7$-, -$(CH_2)_2$-piperazinylene-$(CH_2)_8$-, -$(CH_2)_3$-piperazinylene-$CH_2$-, -$(CH_2)_3$-piperazinylene-$(CH_2)_2$-, -$(CH_2)_3$-piperazinylene-$(CH_2)_3$-, -$(CH_2)_3$-piperazinylene-$(CH_2)_4$-, -$(CH_2)_3$-piperazinylene-$(CH_2)_5$-, -$(CH_2)_3$-piperazinylene-$(CH_2)_6$-, -$(CH_2)_3$-piperazinylene-$(CH_2)_7$-, -$(CH_2)_3$-piperazinylene-$(CH_2)_8$-, -$(CH_2)_4$-piperazinylene-$CH_2$-, -$(CH_2)_4$-piperazinylene-$(CH_2)_2$-, -$(CH_2)_4$-piperazinylene-$(CH_2)_3$-, -$(CH_2)_4$-piperazinylene-$(CH_2)_4$-, -$(CH_2)_4$-piperazinylene-$(CH_2)_5$-, -$(CH_2)_4$-piperazinylene-$(CH_2)_6$-, -$(CH_2)_4$-piperazinylene-$(CH_2)_7$-, -$(CH_2)_4$-piperazinylene-$(CH_2)_9$-, -$(CH_2)_5$-piperazinylene-$(CH_2)_1$-, -$(CH_2)_5$-piperazinylene-$(CH_2)_2$-, -$(CH_2)_5$-piperazinylene-$(CH_2)_3$-, -$(CH_2)_5$-piperazinylene-$(CH_2)_4$-, -$(CH_2)_5$-piperazinylene-$(CH_2)_5$-, -$(CH_2)_5$-piperazinylene-$(CH_2)_6$-, -$(CH_2)_5$-piperazinylene-$(CH_2)_7$-, -$(CH_2)_5$-piperazinylene-$(CH_2)_8$-, -$(CH_2)_6$-piperazinylene-$(CH_2)_1$-, -$(CH_2)_6$-piperazinylene-$(CH_2)_2$-, -$(CH_2)_6$-piperazinylene-$(CH_2)_3$-, -$(CH_2)_6$-piperazinylene-$(CH_2)_4$-, -$(CH_2)_6$-piperazinylene-$(CH_2)_5$-, -$(CH_2)_6$-piperazinylene-$(CH_2)_6$-, -$(CH_2)_6$-piperazinylene-$(CH_2)_7$-, -$(CH_2)_6$-piperazinylene-$(CH_2)_8$-, -$(CH_2)_7$-piperazinylene-$(CH_2)_1$-, -$(CH_2)_7$-piperazinylene-$(CH_2)_2$-, -$(CH_2)_7$-piperazinylene-$(CH_2)_3$-, -$(CH_2)_7$-piperazinylene-$(CH_2)_4$-, -$(CH_2)_8$-piperazinylene-$CH_2$-, -$(CH_2)_8$-piperazinylene-$(CH_2)_2$-, -$(CH_2)_8$-piperazinylene-$(CH_2)_3$-, -$(CH_2)_8$-piperazinylene-$(CH_2)_4$-, -$(CH_2)_8$-piperazinylene-$(CH_2)_5$-, or -$(CH_2)_8$-piperazinylene-$(CH_2)_6$-;

wherein each $R_{a7}$ independently represents H or $C_{1-6}$ alkyl (e.g., methyl, ethyl, propyl, or isopropyl);
the phenylene is optionally substituted with a substituent selected from the group consisting of deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; and
the piperazinylene and piperidinylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof.

**7.** The compound of Formula (I) or salts, enantiomers, stereoisomers, solvates, or polymorphs thereof as claimed in claim 1, wherein L represents:

a bond, C(O), C(O)NH, NHC(O), -$CH_2$-, -$(CH_2)_2$-, -$(CH_2)_3$-, -$(CH_2)_4$-, -$(CH_2)_5$-, -$(CH_2)_6$-, -$(CH_2)_7$-, - $(CH_2)_8$-, -$(CH_2)_9$-, -$(CH_2)_{10}$-, -$(CH_2)_{11}$-, -$(CH_2)_{12}$-, -$(CH_2)_{13}$-, -$(CH_2)_{14}$-, -$(CH_2)_{15}$-; -O-$CH_2$-, -O-$(CH_2)_2$-, -O-$(CH_2)_3$-, -O-$(CH_2)_4$-, -O-$(CH_2)_5$-, -O-$(CH_2)_6$-, -O-$(CH_2)_7$-, -O-$(CH_2)_8$-, -O-$(CH_2)_9$-, -O-$(CH_2)_{10}$-, -$(CH_2)_1$-O-, -$(CH_2)_2$-O-, -$(CH_2)_3$-O-, -$(CH_2)_4$-O-, -$(CH_2)_5$-O-, -$(CH_2)_6$-O-, -$(CH_2)_7$-O-, -$(CH_2)_8$-O-, -$(CH_2)_9$-O-, -$(CH_2)_{10}$-O-, -$CH_2$-O-$CH_2$-, -$CH_2$-O-$(CH_2)_2$-, -$(CH_2)_1$-O-$(CH_2)_3$-, -$(CH_2)_1$-O-$(CH_2)_4$-, -$(CH_2)_1$-O-$(CH_2)_5$-, -$(CH_2)_1$-O-$(CH_2)_6$-, -$(CH_2)_1$-O-$(CH_2)_7$-, -$(CH_2)_1$-O-$(CH_2)_8$-, -$(CH_2)_2$-O-$(CH_2)_1$-, -$(CH_2)_2$-O-$(CH_2)_2$-, -$(CH_2)_2$-O-$(CH_2)_3$-, -$(CH_2)_2$-O-$(CH_2)_4$-, -$(CH_2)_2$-O-$(CH_2)_5$-, -$(CH_2)_2$-O-$(CH_2)_6$-, -$(CH_2)_2$-O-$(CH_2)_7$-, -$(CH_2)_2$-O-$(CH_2)_8$-, - $(CH_2)_3$-O-$(CH_2)_1$-, -$(CH_2)_3$-O-$(CH_2)_2$-, -$(CH_2)_3$-O-$(CH_2)_3$-, -$(CH_2)_3$-O-$(CH_2)_4$-, -$(CH_2)_3$-O-$(CH_2)_5$-, -$(CH_2)_3$-O-$(CH_2)_6$-, -$(CH_2)_3$-O-$(CH_2)_7$-, -$(CH_2)_4$-O-$(CH_2)_1$-, -$(CH_2)_4$-O-$(CH_2)_2$-, -$(CH_2)_4$-O-$(CH_2)_3$-, -$(CH_2)_4$-O-$(CH_2)_4$-, -$(CH_2)_4$-O-$(CH_2)_5$-, -$(CH_2)_4$-O-$(CH_2)_6$-, -$(CH_2)_5$-O-$(CH_2)_1$-, -$(CH_2)_5$-O-$(CH_2)_2$-, -$(CH_2)_5$-O-$(CH_2)_3$-, -$(CH_2)_5$-O-$(CH_2)_4$-, -$(CH_2)_5$-O-$(CH_2)_5$-, -$(CH_2)_6$-O-$(CH_2)_1$-, -$(CH_2)_6$-O-$(CH_2)_2$-, -$(CH_2)_6$-O-$(CH_2)_3$-, - $(CH_2)_6$-O-$(CH_2)_4$-, -$(CH_2)_7$-O-$(CH_2)_1$-, -$(CH_2)_7$-O-$(CH_2)_2$-, -$(CH_2)_7$-O-$(CH_2)_3$-, -$(CH_2)_8$-O-$(CH_2)_1$-, -$(CH_2)_8$-O-$(CH_2)_2$-, -CH($CH_3$)-O-$(CH_2)_1$-, -CH($CH_3$)-O-$(CH_2)_2$-, -CH($CH_3$)-O-$(CH_2)_3$-, -CH($CH_3$)-O-$(CH_2)_4$-, - CH($CH_3$)-O-$(CH_2)_5$-, -CH($CH_3$)-O-$(CH_2)_6$-, -CH($CH_3$)-O-$(CH_2)_7$-, -CH($CH_3$)-O-$(CH_2)_8$-, -$(O(CH_2)_2)_2$-, - $(O(CH_2)_2)_3$-, -$(O(CH_2)_2)_4$-, -$(O(CH_2)_2)_5$-, -$(O(CH_2)_2)_6$-, -$CH_2$-$(O(CH_2)_2)_2$-, -$CH_2$-$(O(CH_2)_2)_3$-, -$CH_2$-$(O(CH_2)_2)_4$-, -$CH_2$-$(O(CH_2)_2)_5$-, -$CH_2$-$(O(CH_2)_2)_6$-, -$CH_2$-$(O(CH_2)_2)_1$-$OCH_2$-, -$CH_2$-$(O(CH_2)_2)_2$-$OCH_2$-, - $CH_2$-$(O(CH_2)_2)_3$-$OCH_2$-, -$CH_2$-$(O(CH_2)_2)_4$-$OCH_2$-, -$CH_2$-$(O(CH_2)_2)_5$-$OCH_2$-, -$CH_2$-$(O(CH_2)_2)_6$-$OCH_2$-, -

$(CH_2)_2-(O(CH_2)_2)_2-$, $-(CH_2)_2-(O(CH_2)_2)_3-$, $-(CH_2)_2-(O(CH_2)_2)_4-$, $-(CH_2)_2-(O(CH_2)_2)_5-$, $-(CH_2)_2-(O(CH_2)_2)_6-$, $-(CH_2)_3-(O(CH_2)_2)_2-$, $-(CH_2)_3-(O(CH_2)_2)_3-$, $-(CH_2)_3-(O(CH_2)_2)_4-$, $-(CH_2)_3-(O(CH_2)_2)_5-$, $-(CH_2)_4-(O(CH_2)_2)_2-$, $-(CH_2)_4-(O(CH_2)_2)_3-$, $-(CH_2)_4-(O(CH_2)_2)_4-$, $-(CH_2)_4-(O(CH_2)_2)_5-$, $-CH_2-(O(CH_2)_3)_2-$, $-CH_2-(O(CH_2)_3)_3-$, $-CH_2-(O(CH_2)_3)_4-$, $-(CH_2)_2-(O(CH_2)_3)_2-$, $-(CH_2)_2-(O(CH_2)_3)_3-$, $-(CH_2)_2-(O(CH_2)_3)_4-$, $-(CH_2)_3-(O(CH_2)_3)_2-$, $-(CH_2)_3-(O(CH_2)_3)_3-$, $-CH_2-O-(CH_2)_2-O-(CH_2)_3-$, $-CH_2-(O(CH_2)_2)_2-(O(CH_2)_3)_2-$, $(CH_2)_2-O-(CH_2)_2-O-(CH_2)_3-$, $-(CH_2)_2-(O(CH_2)_2)_2-(O(CH_2)_3)_2-$, $-(CH_2)_3-O-(CH_2)_2-O-(CH_2)_3-$, $-(CH_2)_3-(O(CH_2)_2)_2-(O(CH_2)_3)_2-$, $-CH_2-O-(CH_2)_3-O-(CH_2)_2-$, $-CH_2-(O(CH_2)_3)_2-(O(CH_2)_2)_2-$, $(CH_2)_1-N(R_{a7})-(CH_2)_1-$, $-(CH_2)_1-N(R_{a7})-(CH_2)_2-$, $-(CH_2)_1-N(R_{a7})-(CH_2)_3-$, $-(CH_2)_1-N(R_{a7})-(CH_2)_4-$, $-(CH_2)_1-N(R_{a7})-(CH_2)_5-$, $-(CH_2)_1-N(R_{a7})-(CH_2)_6-$, $-N(R_{a7})-(CH_2)_1-$, $-N(R_{a7})-(CH_2)_2-$, $-N(R_{a7})-(CH_2)_3-$, $-N(R_{a7})-(CH_2)_4-$, $-N(R_{a7})-(CH_2)_5-$, $-N(R_{a7})-(CH_2)_6-$, $-(CH_2)_2-N(R_{a7})-(CH_2)_1-$, $-(CH_2)_2-N(R_{a7})-(CH_2)_2-$, $-(CH_2)_2-N(R_{a7})-(CH_2)_3-$, $-(CH_2)_2-N(R_{a7})-(CH_2)_4-$, $-(CH_2)_2-N(R_{a7})-(CH_2)_5-$, $-(CH_2)_2-N(R_{a7})-(CH_2)_6-$, $-(CH_2)_3-N(R_{a7})-(CH_2)_1-$, $-(CH_2)_3-N(R_{a7})-(CH_2)_2-$, $-(CH_2)_3-N(R_{a7})-(CH_2)_3-$, $-(CH_2)_4-N(R_{a7})-(CH_2)_1-$, $-(CH_2)_4-N(R_{a7})-(CH_2)_2-$, $-(CH_2)_4-N(R_{a7})-(CH_2)_3-$, $-(CH_2)_4-N(R_{a7})-(CH_2)_4-$, $-(CH_2)_5-N(R_{a7})-(CH_2)_1-$, $-(CH_2)_5-N(R_{a7})-(CH_2)_2-$, $-(CH_2)_5-N(R_{a7})-(CH_2)_3-$, $-(CH_2)_5-N(R_{a7})-(CH_2)_4-$, $-(CH_2)_5-N(R_{a7})-(CH_2)_5-$, $-(CH_2)_6-N(R_{a7})-(CH_2)_1-$, $-(CH_2)_6-N(R_{a7})-(CH_2)_2-$, $-(CH_2)_6-N(R_{a7})-(CH_2)_3-$, $-(CH_2)_7-N(R_{a7})-(CH_2)_1-$, $-(CH_2)_7-N(R_{a7})-(CH_2)_2-$, $-(CH_2)_7-N(R_{a7})-(CH_2)_3-$, $-(CH_2)_8-N(R_{a7})-(CH_2)_1-$, $-(CH_2)_8-N(R_{a7})-(CH_2)_2-$, $-(CH_2)_8-N(R_{a7})-(CH_2)_3-$, $-CH(CH_3)-N(R_{a7})-(CH_2)_1-$, $-CH(CH_3)-N(R_{a7})-(CH_2)_2-$, $-CH(CH_3)-N(R_{a7})-(CH_2)_3-$, $-CH(CH_3)-N(R_{a7})-(CH_2)_4-$, $-CH(CH_3)-N(R_{a7})-(CH_2)_5-$, $-CH(CH_3)-N(R_{a7})-(CH_2)_6-$, $-(CH_2)_1-N(R_{a7})-$, $-(CH_2)_2-N(R_{a7})-$, $-(CH_2)_3-N(R_{a7})-$, $-(CH_2)_4-N(R_{a7})-$, $-(CH_2)_5-N(R_{a7})-$, $-(CH_2)_6-N(R_{a7})-$, $-(CH_2)_7-N(R_{a7})-$, $-(CH_2)_8-N(R_{a7})-$, $-(CH_2)_9-N(R_{a7})-$, $-(CH_2)_{10}-N(R_{a7})-$, $-C(O)NHCH_2-$, $-C(O)NH(CH_2)_2-$, $-C(O)NH(CH_2)_3-$, $-C(O)NH(CH_2)_4-$, $-C(O)NH(CH_2)_5-$, $-CH_2C(O)NHCH_2-$, $-(CH_2)_2C(O)NH(CH_2)_2-$, $-(CH_2)_2C(O)NH(CH_2)_3-$, $-(CH_2)_2C(O)NH(CH_2)_4-$, $-(CH_2)_2C(O)NH(CH_2)_5-$, $-(CH_2)_3C(O)NH(CH_2)_3-$, $-(CH_2)_3C(O)NH(CH_2)_4-$, $-(CH_2)_4C(O)NH(CH_2)_4-$, $-(CH_2)_5C(O)NH(CH_2)_5-$, $-(CH_2)_2C(O)NH(CH_2)_2-O-(CH_2)_2-$, $-NHC(O)CH_2-$, $-NHC(O)(CH_2)_2-$, $-NHC(O)(CH_2)_3-$, $-NHC(O)(CH_2)_4-$, $-NHC(O)(CH_2)_5-$, $-CH_2NHC(O)CH_2-$, $-(CH_2)_2NHC(O)(CH_2)_2-$, $-(CH_2)_2NHC(O)(CH_2)_3-$, $-(CH_2)_2NHC(O)(CH_2)_4-$, $-(CH_2)_2NHC(O)(CH_2)_5-$, $-(CH_2)_3NHC(O)(CH_2)_3-$, $-(CH_2)_3NHC(O)(CH_2)_4-$, $-(CH_2)_4NHC(O)(CH_2)_4-$, $-(CH_2)_5NHC(O)(CH_2)_5-$, $-(CH_2)_2NHC(O)(CH_2)_2-O-(CH_2)_2-$, $-(CH_2)_4NHC(O)CH_2-$, $-CH_2$-phenylene-$CH_2-$, $-CH_2$-phenylene-$(CH_2)_2-$, $-CH_2$-phenylene-$(CH_2)_3-$, $-CH_2$-phenylene-$(CH_2)_4-$, $-CH_2$-phenylene-$(CH_2)_5-$, $-CH_2$-phenylene-$(CH_2)_6-$, $-(CH_2)_2$-phenylene-$(CH_2)_1-$, $-(CH_2)_2$-phenylene-$(CH_2)_2-$, $-(CH_2)_2$-phenylene-$(CH_2)_3-$, $-(CH_2)_2$-phenylene-$(CH_2)_4-$, $-(CH_2)_2$-phenylene-$(CH_2)_5-$, $-(CH_2)_3$-phenylene-$CH_2-$, $-(CH_2)_3$-phenylene-$(CH_2)_2-$, $-(CH_2)_3$-phenylene-$(CH_2)_3-$, $-(CH_2)_3$-phenylene-$(CH_2)_4-$, $-(CH_2)_3$-phenylene-$(CH_2)_5-$, $-(CH_2)_4$-phenylene-$CH_2-$, $-(CH_2)_4$-phenylene-$(CH_2)_2-$, $-(CH_2)_4$-phenylene-$(CH_2)_3-$, $-(CH_2)_4$-phenylene-$(CH_2)_4-$, $-(CH_2)_4$-phenylene-$(CH_2)_5-$, $-CH_2$-piperidinylene-$CH_2-$, $-CH_2$-piperidinylene-$(CH_2)_2-$, $-CH_2$-piperidinylene-$(CH_2)_3-$, $-CH_2$-piperidinylene-$(CH_2)_4-$, $-CH_2$-piperidinylene-$(CH_2)_5-$, $-CH_2$-piperidinylene-$(CH_2)_6-$, $-(CH_2)_2$-piperidinylene-$(CH_2)_1-$, $-(CH_2)_2$-piperidinylene-$(CH_2)_2-$, $-(CH_2)_2$-piperidinylene-$(CH_2)_3-$, $-(CH_2)_2$-piperidinylene-$(CH_2)_4-$, $-(CH_2)_2$-piperidinylene-$(CH_2)_5-$, $-(CH_2)_3$-piperidinylene-$CH_2-$, $-(CH_2)_3$-piperidinylene-$(CH_2)_2-$, $-(CH_2)_3$-piperidinylene-$(CH_2)_3-$, $-(CH_2)_3$-piperidinylene-$(CH_2)_4-$, $-(CH_2)_3$-piperidinylene-$(CH_2)_5-$, $-CH_2$-piperazinylene-$CH_2-$, $-CH_2$-piperazinylene-$(CH_2)_2-$, $-CH_2$-piperazinylene-$(CH_2)_3-$, $-CH_2$-piperazinylene-$(CH_2)_4-$, $-CH_2$-piperazinylene-$(CH_2)_5-$, $-(CH_2)_2$-piperazinylene-$(CH_2)_1-$, $-(CH_2)_2$-piperazinylene-$(CH_2)_2-$, $-(CH_2)_2$-piperazinylene-$(CH_2)_3-$, $-(CH_2)_2$-piperazinylene-$(CH_2)_4-$, $-(CH_2)_2$-piperazinylene-$(CH_2)_5-$, $-(CH_2)_3$-piperazinylene-$CH_2-$, $-(CH_2)_3$-piperazinylene-$(CH_2)_2-$, $-(CH_2)_3$-piperazinylene-$(CH_2)_3-$, $-(CH_2)_3$-piperazinylene-$(CH_2)_4-$, $-(CH_2)_3$-piperazinylene-$(CH_2)_5-$, $-(CH_2)_4$-piperazinylene-$CH_2-$, $-(CH_2)_4$-piperazinylene-$(CH_2)_2-$, $-(CH_2)_4$-piperazinylene-$(CH_2)_3-$, $-(CH_2)_4$-piperazinylene-$(CH_2)_4-$, $-(CH_2)_4$-piperazinylene-$(CH_2)_5-$, $-(CH_2)_8$-piperazinylene-$CH_2-$, $-(CH_2)_8$-piperazinylene-$(CH_2)_2-$, $-(CH_2)_8$-piperazinylene-$(CH_2)_3-$, $-(CH_2)_8$-piperazinylene-$(CH_2)_4-$, or $-(CH_2)_8$-piperazinylene-$(CH_2)_5-$;

wherein each $R_{a7}$ independently represents H or $C_{1-6}$ alkyl (e.g., methyl, ethyl, propyl, or isopropyl);

the phenylene is optionally substituted with a substituent selected from the group consisting of deuterium, hydroxy, amino, mercapto, nitro, halogen, cyano, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof;

the piperazinylene and piperidinylene are each independently optionally substituted with a substituent selected from the group consisting of deuterium, hydroxy, amino, mercapto, nitro, halogen, cyano, oxo, $C_{1-6}$ alkyl, halogenated $C_{1-6}$ alkyl, deuterated $C_{1-6}$ alkyl, $C_{1-6}$ alkoxy, halogenated $C_{1-6}$ alkoxy, $C_{3-6}$ cycloalkyl, halogenated $C_{3-6}$ cycloalkyl, deuterated $C_{3-6}$ cycloalkyl, 4- to 10-membered heterocyclyl, and any combination thereof; or L represents:

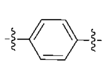 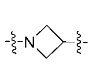 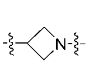 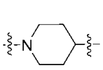 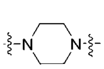 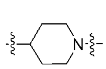 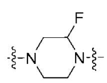 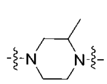 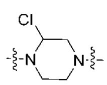 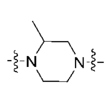 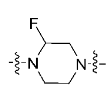

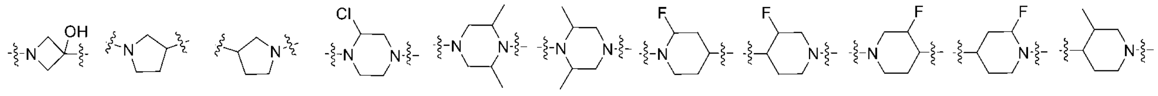

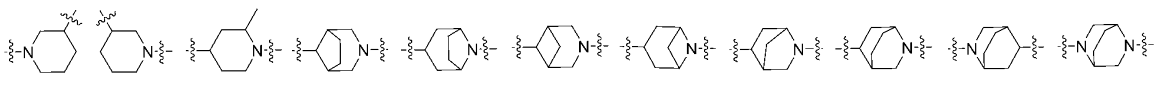

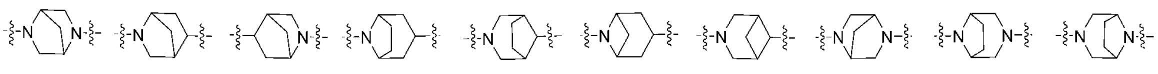

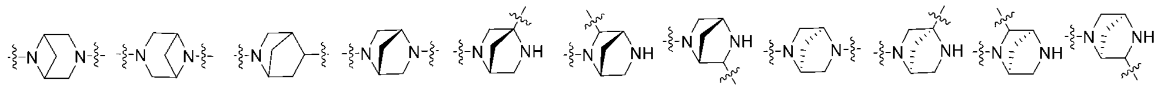

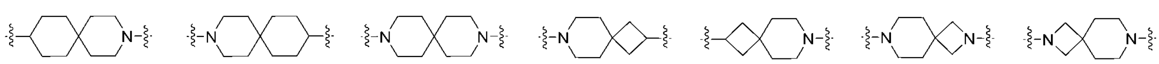

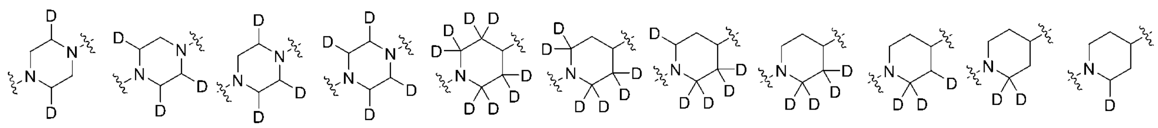

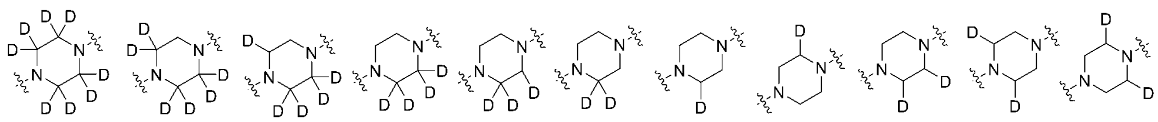

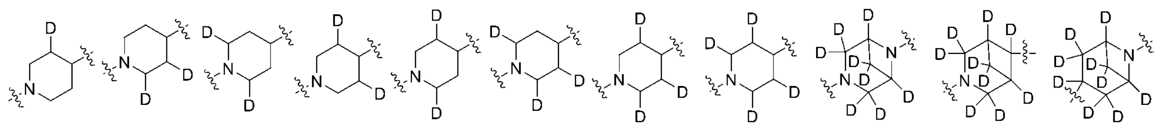

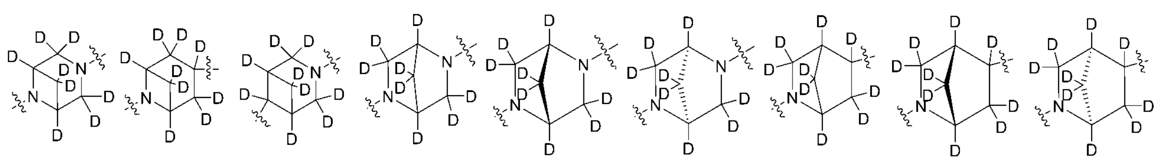

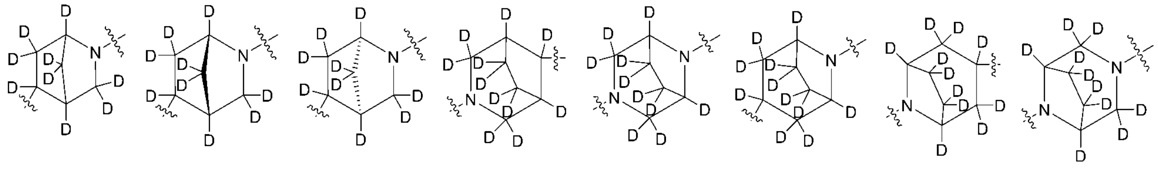

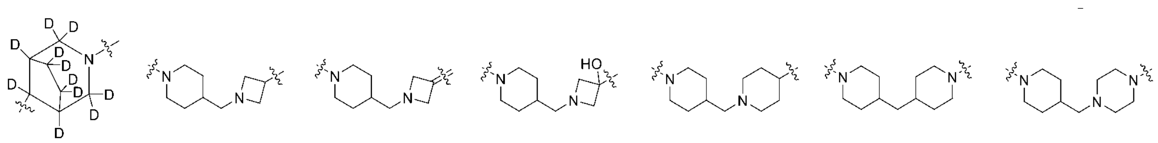

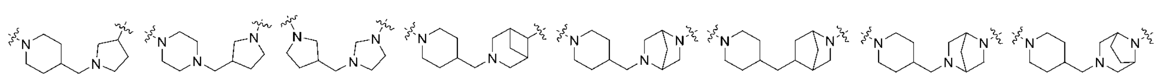

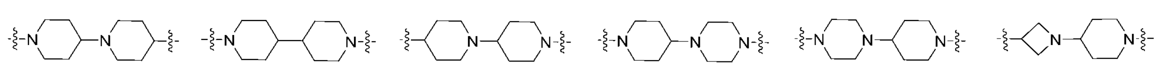

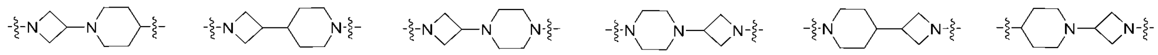
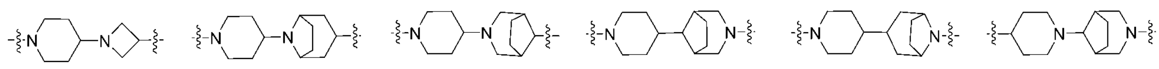
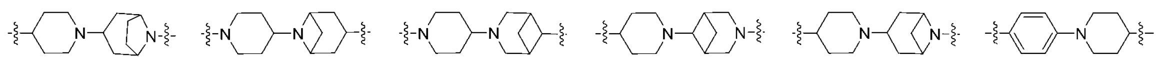
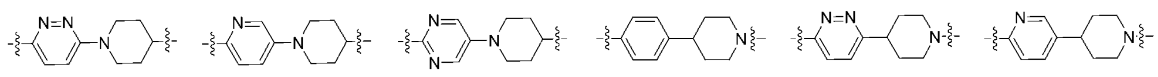
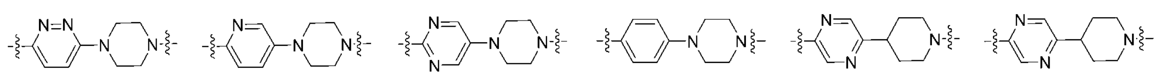
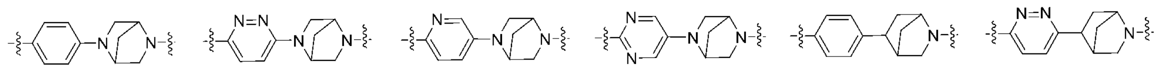
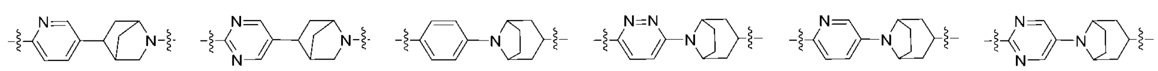
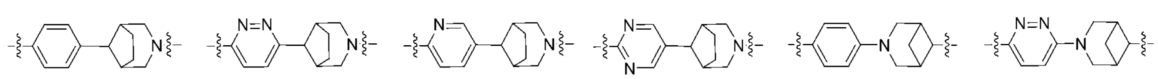
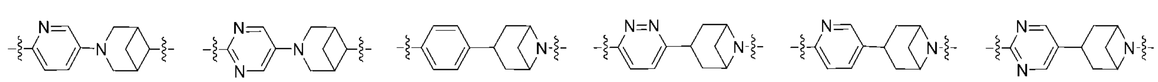
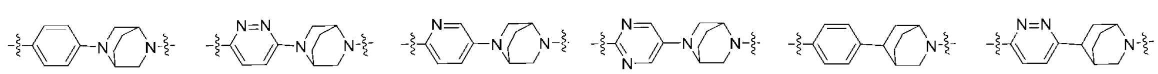
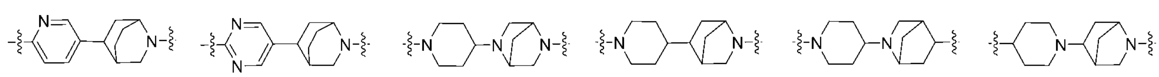
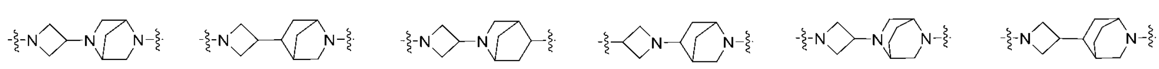
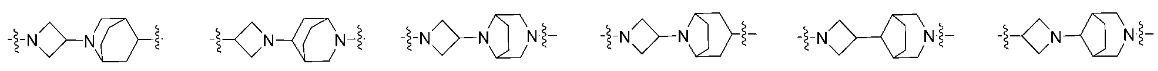
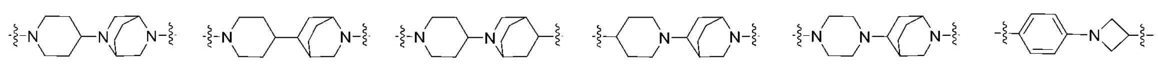
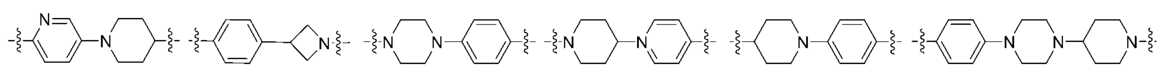
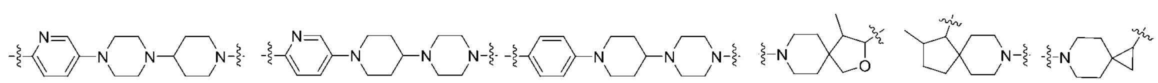
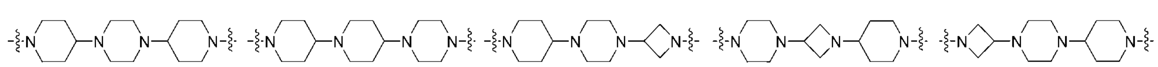

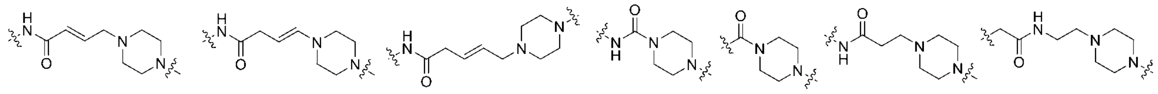

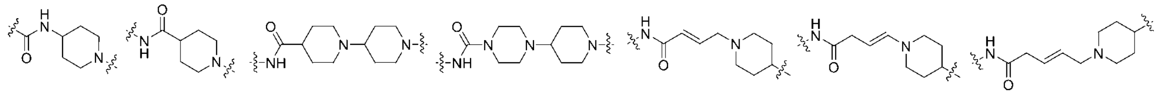

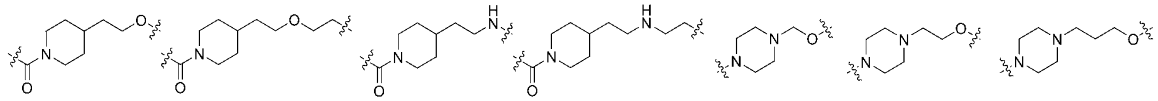

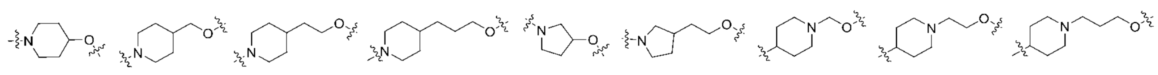

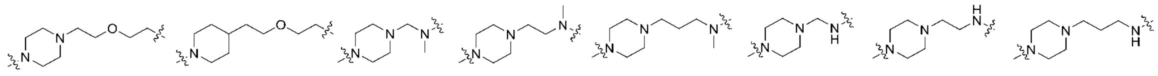

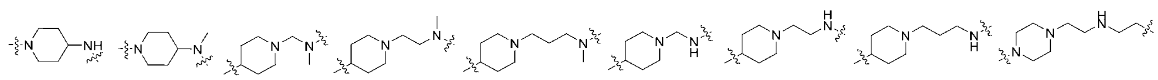

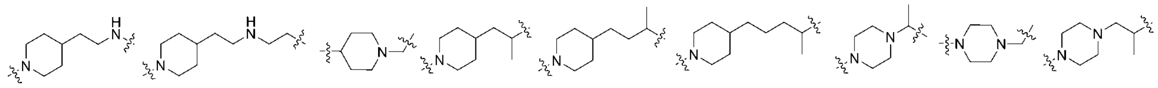

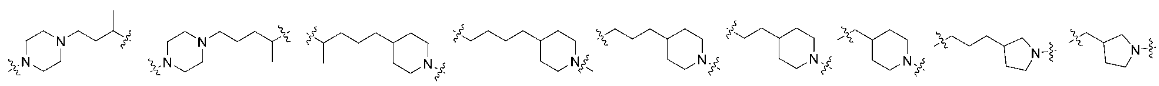

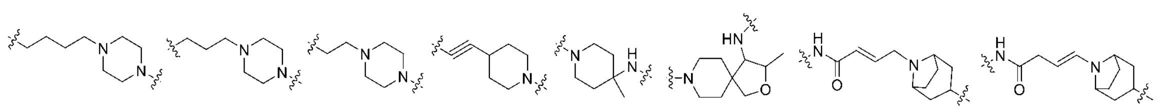

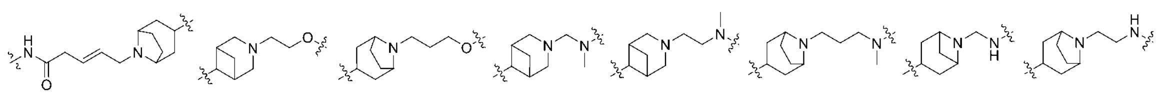

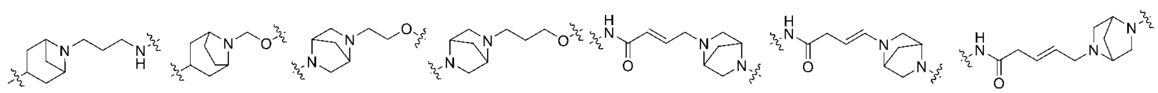

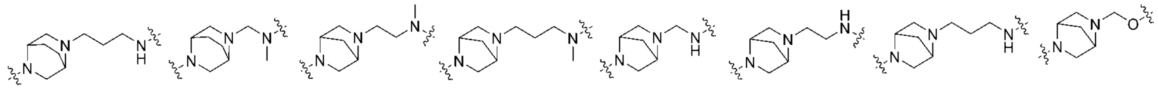

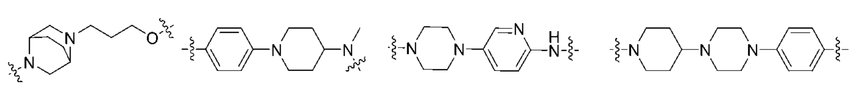 或 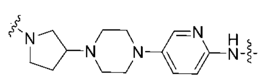;

wherein the groups are optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, and $C_{2-6}$ alkenyl.

**8.** The compound of Formula (I) or salts, enantiomers, stereoisomers, solvates, or polymorphs thereof as claimed in claim 1, wherein X represents:

$NR_1R_2$, $C(O)NR_3R_4$, $NHC(O)R_5$, $NHC(O)NR_6R_7$, $OR_8$, $SR_9$, $S(O)_2NR_{20}R_{22}$ or $N(R_{12})S(O)_2R_{13}$, wherein $R_1$, $R_3$, $R_4$, $R_5$, $R_6$, $R_7$, $R_9$, $R_{10}$, $R_{11}$, and $R_{12}$ each independently represent H, optionally substituted linear or branched $C_{1-10}$ alkyl, optionally substituted $C_{3-30}$ cycloalkyl, optionally substituted $C_{5-30}$ aryl, optionally substituted 4- to 30-membered heterocyclyl, or optionally substituted 5- to 30-membered heteroaryl; and $R_2$, $R_8$, and $R_{13}$ each independently represent optionally substituted linear or branched $C_{1-10}$ alkyl, optionally substituted $C_{3-30}$ cycloalkyl, optionally substituted $C_{5-30}$ aryl, optionally substituted 4- to 30-membered heterocyclyl, or optionally substituted 5- to 30-membered heteroaryl; or wherein $R_3$ and $R_4$, together with the nitrogen atom to which they are attached, form an optionally substituted 4- to 30-membered nitrogen-containing heterocyclyl; or

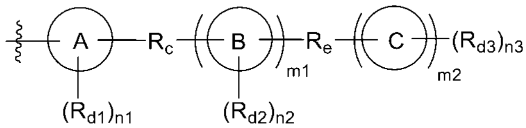

wherein ring A represents 4- to 30-membered heterocyclylene, $C_{3-30}$ cycloalkylene, $C_{5-30}$ arylene or 5- to 30-membered heteroarylene, and $(R_{d1})_{n1}$ indicates that ring A is optionally substituted with n1 $R_{d1}$ groups, with each $R_{d1}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl, and n1 represents an integer of 0-20;

$R_c$ represents $NR_{c1}$, C(O), $CR_{c2}R_{c3}$ or a bond, wherein $R_{c1}$ represents H, optionally substituted linear or branched $C_{1-10}$ alkyl, optionally substituted $C_{3-30}$ cycloalkyl, optionally substituted $C_{5-30}$ aryl, optionally substituted 4- to 30-membered heterocyclyl or optionally substituted 5- to 30-membered heteroaryl; $R_{c2}$ and $R_{c3}$ each independently represent H, halogen, optionally substituted linear or branched $C_{1-10}$ alkyl, optionally substituted $C_{3-30}$ cycloalkyl, optionally substituted $C_{5-30}$ aryl, optionally substituted 4- to 30-membered heterocyclyl, or optionally substituted 5- to 30-membered heteroaryl;

each ring B is identical or different and each independently represents 4- to 30-membered heterocyclylene, $C_{3-30}$ cycloalkylene, $C_{5-30}$ arylene or 5- to 30-membered heteroarylene; m1 represents an integer of 0, 1, 2 or 3; and $(R_{d2})_{n2}$ indicates that each ring B is independently optionally substituted with n2 $R_{d2}$ groups, with each $R_{d2}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl; and n2 represents an integer of 0-20;

$R_e$ represents S, O, $CR_{e1}R_{e2}$ or a bond, wherein $R_{e1}$ and $R_{e2}$ each independently represent H, halogen, optionally substituted linear or branched $C_{1-19}$ alkyl, optionally substituted $C_{3-30}$ cycloalkyl, optionally substituted $C_{5-30}$ aryl, optionally substituted 4- to 30-membered heterocyclyl, or optionally substituted 5- to 30-membered heteroaryl; and

each ring C is identical or different and each independently represents 4- to 30-membered heterocyclyl, $C_{3-30}$ cycloalkyl, $C_{5-30}$ aryl or 5- to 30-membered heteroaryl; m2 represents an integer of 0, 1, 2 or 3; and $(R_{d3})_{n3}$ indicates that each ring C is independently optionally substituted with n3 $R_{d3}$ groups, with each $R_{d3}$ independently representing deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl or $C_{2-6}$ alkenyl; and n3 represents an integer of 0-20;

wherein each linear or branched $C_{1-10}$ alkyl is independently optionally substituted with a substituent selected from the group consisting of deuterium, halogen, amino, hydroxy, mercapto, cyano, nitro, oxo, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, optionally substituted $C_{3-30}$ cycloalkyl, 4- to 10-membered heterocyclyl, $C_{2-6}$ alkynyl, and $C_{2-6}$ alkenyl; and

each of $C_{3-30}$ cycloalkylene, $C_{5-30}$ arylene, 4- to 30-membered heterocyclylene, 5- to 30-membered heteroarylene, $C_{3-30}$ cycloalkyl, $C_{5-30}$ aryl, 4- to 30-membered heterocyclyl, and 5- to 30-membered heteroaryl is independently optionally substituted with a substituent selected from the group consisting of deuterium, $C_{1-6}$ alkyl, deuterated $C_{1-6}$ alkyl, halogenated $C_{1-6}$ alkyl, $C_{1-6}$ alkoxy, halogenated $C_{1-6}$ alkoxy, halogen, amino, hydroxy, mercapto, cyano, oxo, $C_{2-6}$ alkynyl, and $C_{2-6}$ alkenyl.

**9.** The compound of Formula (I) or salts, enantiomers, stereoisomers, solvates, or polymorphs thereof as claimed in claim 1, wherein

(i) $R_1$, $R_3$, $R_4$, $R_5$, $R_6$, $R_7$, $R_9$, $R_{10}$, $R_{11}$, $R_{12}$, and $R_{c1}$ each independently represent:

H or optionally substituted linear or branched $C_{1-10}$ alkyl;

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbomanyl, bicyclo[2.2.1]heptanyl, 2-oxobicyclo[2.2.1]heptanyl, or bicyclo[2.2.1]heptenyl), each of which is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenyl or naphthyl, each of which is optionally substituted with one or more (e.g., 1-10) substituents selected from deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; and/or

(ii) $R_2$, $R_8$, and $R_{13}$ each independently represent: optionally substituted linear or branched $C_{1-10}$ alkyl:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbomanyl, bicyclo[2.2.1]heptanyl, 2-oxobicyclo[2.2.1]heptanyl, or bicyclo[2.2.1]heptenyl), each of which is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any

combination thereof;

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenyl or naphthyl, each of which is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; and/or

(iii) $R_{c2}$, $R_{c3}$, $R_{e1}$, and $R_{e2}$ each independently represent:

H, halogen, or optionally substituted linear or branched $C_{1-10}$ alkyl; or

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbomanyl, bicyclo[2.2.1]heptanyl, 2-oxobicyclo[2.2.1]heptanyl, or bicyclo[2.2.1]heptenyl), each of which is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenyl or naphthyl, each of which is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; and/or

(iv) $R_3$ and $R_4$, together with the nitrogen atom to which they are attached, form:
azetidinyl, pyrrolidinyl, imidazolidinyl, pyrazolidyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, diazacycloheptanyl, diazacyclooctyl, nitrogen-containing bridged heterocyclyl (e.g., 6- to 20-membered nitrogen-containing bridged heterocyclyl, such as 3-azabicyclo[3.1.0]hexanyl, 3-azabicyclo[3.1.1]heptanyl, 2-azabicyclo[2.2.1]heptanyl, 6-azabicyclo[3.1.1]heptanyl, 2-azabicyclo[2.2.2]octanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 2,5-diazabicyclo[2.2.2]octanyl, and quinuclidinyl), or azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; and/or
(v) ring A and ring B each independently represent:

cyclopropylene, cyclobutylene, cyclopentylene, cyclopentenylene, cyclohexylene, cyclohexenylene, cycloheptylene, cyclooctylene, decalinylene, octahydropentalenylene, octahydro-1H-indenylene, spiro-cycloalkylene (e.g., $C_5$-$C_{20}$ spiro-cycloalkylene, such as spiro[3.3]heptylene, spiro[2.5]octylene, spiro[3.5]nonylene, spiro[4.4]nonylene, spiro[4.5]decylene, and spiro[5.5]undecylene), p-menthanylene, m-menthanylene, or bridged cycloalkylene (e.g., $C_6$-$C_{20}$ bridged cycloalkylene, such as adamantanylene, noradamantanylene, bornylene, norbomylene, bicyclo[2.2.1]heptanylene, 2-oxobicyclo[2.2.1]heptanylene, or bicyclo[2.2.1]heptenylene), each of which is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

azetidinylene, oxetanylene, pyrrolidinylene, imidazolidinylene, pyrazolidylene, tetrahydrofuranylene, tetrahydropyranylene, tetrahydrothienylene, tetrahydrothiopyranylene, oxazolidinylene, thiazolidinylene, piperidinylene, piperazinylene, tetrahydropyridinylene, dihydroxy-piperidinylene, difluoro-piperidinylene, morpholinylene, thiomorpholinylene, azacycloheptanylene, azacyclooctylene, dioxacyclohexylene, azacycloheptanylene, azacyclooctylene, diazacycloheptanylene (e.g., 1,4-diazacycloheptanylene, 4,5-diazacycloheptanylene, 1,3-diazacycloheptanylene), diazacyclooctylene, bridged heterocyclylene (e.g., 6- to 20-membered bridged heterocyclylene, such as 6-azabicyclo[3.1.1]heptanylene, 2,5-diazabicyclo[2.2.1]heptanylene, 3,6-diazabicyclo[3.1.1]heptanylene, 3-azabicyclo[3.2.1]octanylene, 3,8-diazabicyclo[3.2.1]octanylene, 3,8-diazabicyclo[3.2.1]octanylene, 2,5-diazabicyclo[2.2.2]octanylene, and quinuclidinylene), azaspirocycloalkylene (e.g., 5- to 20-membered azaspirocycloalkylene, such as 2,6-diazaspiro[3.3]heptanylene, 2,7-diazaspiro[3.5]nonanylene, 2,8-diazaspiro[4.5]decanylene, 3,9-diazaspiro[5.5]undecanylene, 3-azaspiro[5.5]undecanylene, and 7-azaspiro[3.5]nonanylene), or octahydropyrrolo[3,4-c]pyrrolylene, each of which is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenylene or naphthylene, each of which is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

furanylene, oxazolylene, isoxazolylene, oxadiazolylene, thienylene, thiazolylene, isothiazolylene, thiadiazolylene, pyrrolylene, imidazolylene, pyrazolylene, triazolylene, pyridylene, pyrimidinylene, pyridazinylene, pyrazinylene, triazinylene, indolylene, isoindolylene, isoindolinylene, benzofuranylene, chromanylene, isobenzofuranylene, benzothienylene, indazolylene, benzimidazolylene, benzoxazolylene, benzisoxazolylene, benzothiazolylene, benzisothiazolylene, benzotriazolylene, benzo[2,1,3]oxadiazolylene, benzo[2,1,3]thiadiazolylene, benzo[1,2,3]thiadiazolylene, 2-oxo-2,3-dihydro-1H-benzo[d]imidazolylene, benzo[b][1,4]oxazinylene, 3,4-dihydro-2H-benzo[b][1,4]oxazinylene, quinolinylene, isoquinolinylene, 1,2,3,4-tetrahydroquinolinylene, naphthyridinylene, cinnolinylene, quinazolinylene, quinoxalinylene, 1,2,3,4-tetrahydroquinoxalinylene, phthalazinylene, 5,6,7,8-tetrahydro-[1,2,4]triazolo[4,3-a]pyrazinylene, 4,5,6,7-tetrahydrothieno[3,2-c]pyridinylene, 5-oxo-6,7-dihydrothieno[3,2-d]pyrimidinylene, thieno[2,3-d]pyrimidinylene, thieno[3,2-d]pyrimidinylene, isoxazolo[4,5-c]pyridinylene, isoxazolo[4,5-c]pyrimidinylene, isoxazolo[4,5-d]pyrimidinylene, pyrazolo[1,5-a]pyridylene, pyrazolo[1,5-a]pyrimidinylene, imidazo[1,2-a]pyridylene, 1H-pyrrolo[3,2-b]pyridylene, 1H-pyrrolo[2,3-b]pyridylene, pyrrolo[2,1-b]thiazolylene, imidazo[2,1-b]thiazolylene, 5,6,7,8-tetrahydrobenzo[4,5]thieno[2,3-d]pyrimidinylene, or 6,7-dihydro-5H-cyclopenta[4,5]thieno[2,3-d]pyrimidinylene, each of which is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; and/or

(vi) each rinC is identical or different and each independently represents:

cyclopropyl, cyclobutyl, cyclopentyl, cyclopentenyl, cyclohexyl, cyclohexenyl, cycloheptyl, cyclooctyl, decalinyl, octahydropentalenyl, octahydro-1H-indenyl, spiro-cycloalkyl (e.g., $C_5$-$C_{20}$ spiro-cycloalkyl, such as spiro[3.3]heptanyl, spiro[2.5]octanyl, spiro[3.5]nonanyl, spiro[4.4]nonanyl, spiro[4.5]decanyl, and spiro[5.5]undecanyl), p-menthanyl, m-menthanyl, or bridged cycloalkyl (e.g., $C_6$-$C_{20}$ bridged cycloalkyl, such as adamantanyl, noradamantanyl, bornyl, norbomanyl, bicyclo[2.2.1]heptanyl, 2-oxobicyclo[2.2.1]heptanyl, or bicyclo[2.2.1]heptenyl), each of which is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

azetidinyl, oxetanyl, pyrrolidinyl, imidazolidinyl, pyrazolidyl, tetrahydrofuranyl, tetrahydropyranyl, tetrahydrothienyl, tetrahydrothiopyranyl, oxazolidinyl, thiazolidinyl, piperidinyl, piperazinyl, tetrahydropyridinyl, dihydroxy-piperidinyl, difluoro-piperidinyl, morpholinyl, thiomorpholinyl, azacycloheptyl, azacyclooctyl, dioxacyclohexyl, azacycloheptyl, azacyclooctyl, diazacycloheptanyl (e.g., 1,4-diazacycloheptanyl, 4,5-diazacycloheptanyl, 1,3-diazacycloheptanyl), diazacyclooctyl, bridged heterocyclyl (e.g., 6- to 20-membered bridged heterocyclyl, such as 6-azabicyclo[3.1.1]heptanyl, 2,5-diazabicyclo[2.2.1]heptanyl, 3,6-diazabicyclo[3.1.1]heptanyl, 3-azabicyclo[3.2.1]octanyl, 3,8-diazabicyclo[3.2.1]octanyl, 3,8-diazabicyclo[3.2.1]octanyl, 2,5-diazabicyclo[2.2.2]octanyl, and quinuclidinyl), and azaspirocycloalkyl (e.g., 5- to 20-membered azaspirocycloalkyl, such as 2,6-diazaspiro[3.3]heptanyl, 2,7-diazaspiro[3.5]nonanyl, 2,8-diazaspiro[4.5]decanyl, 3,9-diazaspiro[5.5]undecanyl, 3-azaspiro[5.5]undecanyl, and 7-azaspiro[3.5]nonanyl), or octahydropyrrolo[3,4-c]pyrrolyl, each of which is optionally substituted with one or more (e.g., 1-10) substituents selected from the group consisting of: deuterium, halogen, hydroxy, mercapto, nitro, amino, cyano, oxo, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof;

phenyl or naphthyl, each of which is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof; or

furanyl, oxazolyl, isoxazolyl, oxadiazolyl, thienyl, thiazolyl, isothiazolyl, thiadiazolyl, pyrrolyl, imidazolyl, pyrazolyl, triazolyl, pyridyl, pyrimidinyl, pyridazinyl, pyrazinyl, triazinyl, indolyl, isoindolyl, isoindolinyl, benzofuranyl, chromanyl, isobenzofuranyl, benzothienyl, indazolyl, benzimidazolyl, benzoxazolyl, benzisoxazolyl, benzothiazolyl, benzisothiazolyl, benzotriazolyl, benzo[2,1,3]oxadiazolyl, benzo[2,1,3]thiadiazolyl, benzo[1,2,3]thiadiazolyl, 2-oxo-2,3-dihydro-1H-benzo[d]imidazolyl, benzo[b][1,4]oxazinyl, 3,4-dihydro-2H-benzo[b][1,4]oxazinyl,quinolinyl, isoquinolinyl, 1,2,3,4-tetrahydroquinolinyl, naphthyridinyl, cinnolinyl, quinazolinyl, quinoxalinyl, 1,2,3,4-tetrahydroquinoxalinyl, phthalazinyl, 5,6,7,8-tetrahydro-[1,2,4]triazolo[4,3-a]pyrazinyl, 4,5,6,7-tetrahydrothieno[3,2-c]pyridinyl, 5-oxo-6,7-dihydrothieno[3,2-d]pyrimidinyl, thieno[2,3-d]pyrimidinyl, thieno[3,2-d]pyrimidinyl, isoxazolo[4,5-c]pyridinyl, isoxazolo[4,5-c]pyrimidinyl, isoxazolo[4,5-d]pyrimidinyl, pyrazolo[1,5-a]pyridinyl, pyrazolo[1,5-a]pyrimidinyl, imidazo[1,2-a]pyridinyl, 1H-pyrrolo[3,2-b]pyridinyl, 1H-pyrrolo[2,3-b]pyridinyl, pyrrolo[2,1-b]thiazolyl, imidazo[2,1-b]thiazolyl, 5,6,7,8-tetrahydrobenzo[4,5]thieno[2,3-d]pyrimidinyl, or 6,7-dihydro-5H-cyclopenta[4,5]thieno[2,3-d]pyrimidinyl, each of which is optionally substituted with one or more (e.g., 1-10) substituents selected from the group consisting of: deuterium, halogen, hydroxy, mercapto, nitro, amino, cyano, $C_{1-6}$ alkyl, halogenated $C_{1-6}$ alkyl, deuterated $C_{1-6}$ alkyl, hydroxy-substituted $C_{1-6}$ alkyl, amino-substituted $C_{1-6}$ alkyl, $C_{1-6}$ alkoxy, deuterated $C_{1-6}$ alkoxy, halogenated $C_{1-6}$ alkoxy, $C_{2-6}$ alkenyl, $C_{2-6}$ alkynyl, and any combination thereof.

**10.** The compound of Formula (I) or salts, enantiomers, stereoisomers, solvates, or polymorphs thereof as claimed in claim 1, wherein X represents :

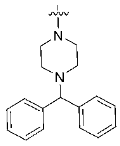 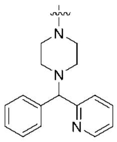 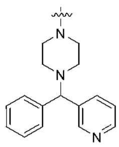 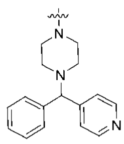 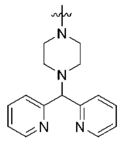 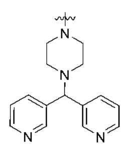 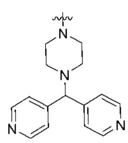 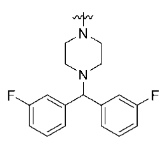 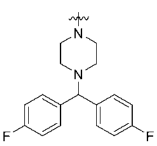

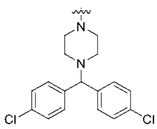 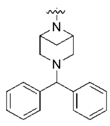 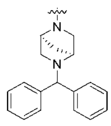 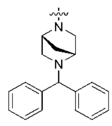 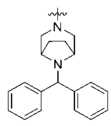 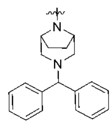 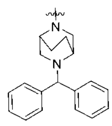 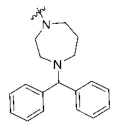 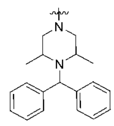 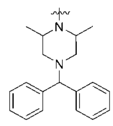

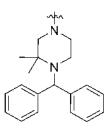 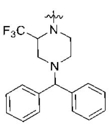 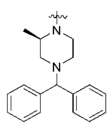 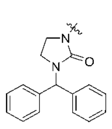 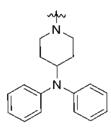 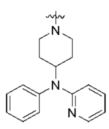 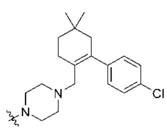 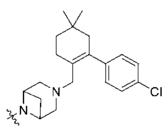 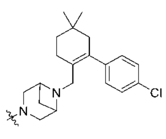

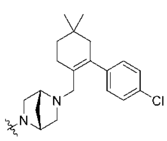 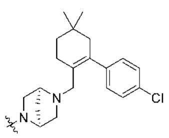 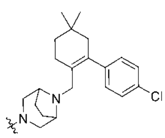 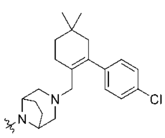 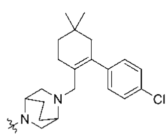 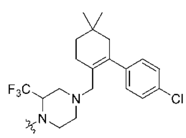 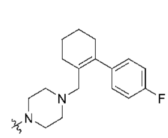

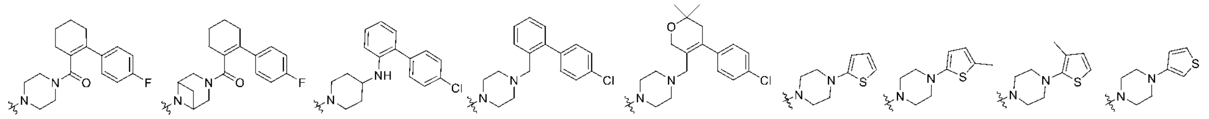
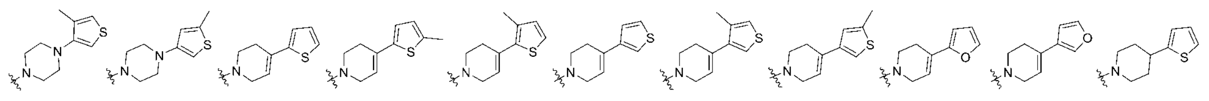
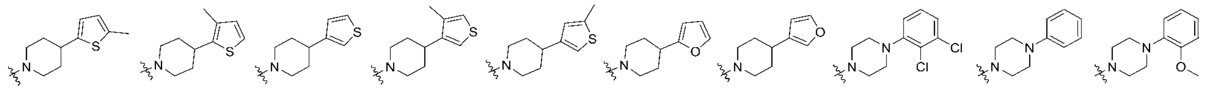
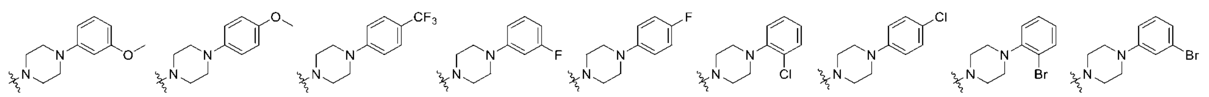
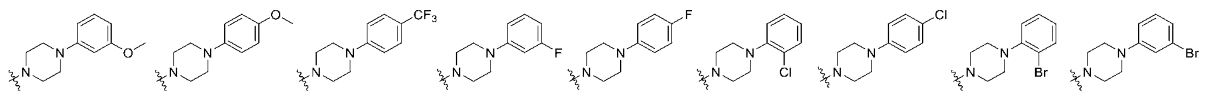
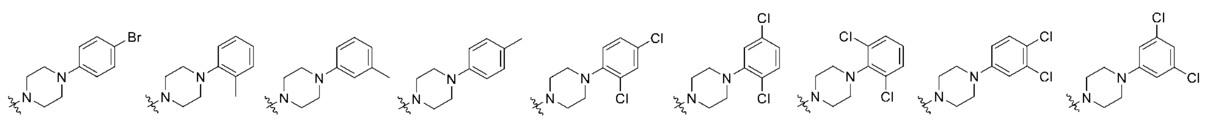
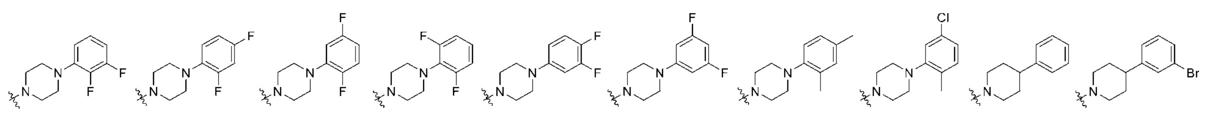
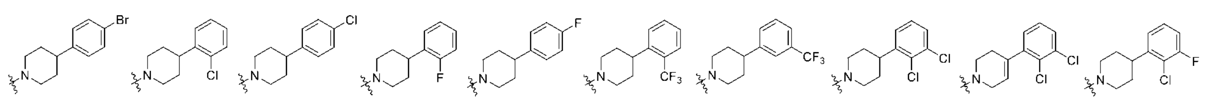
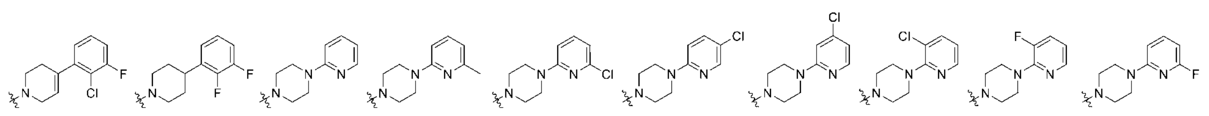
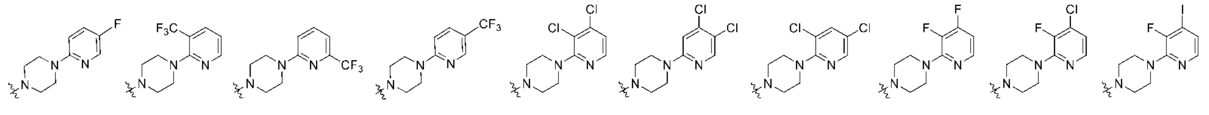
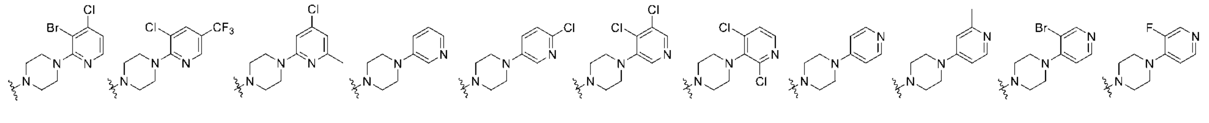
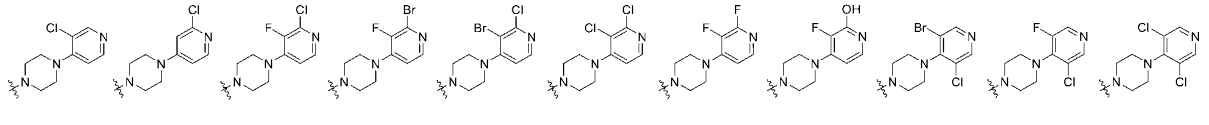

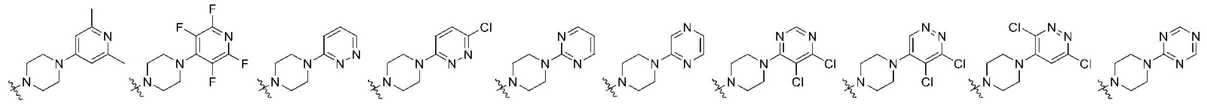

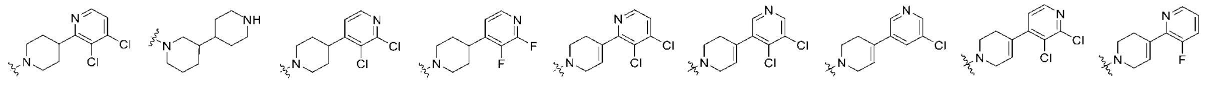

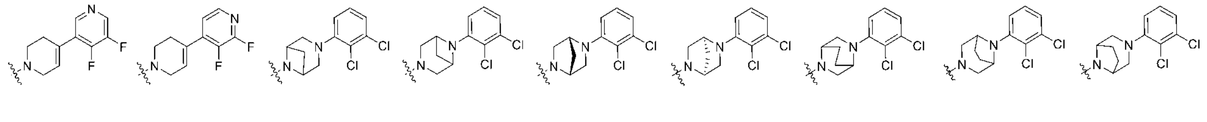

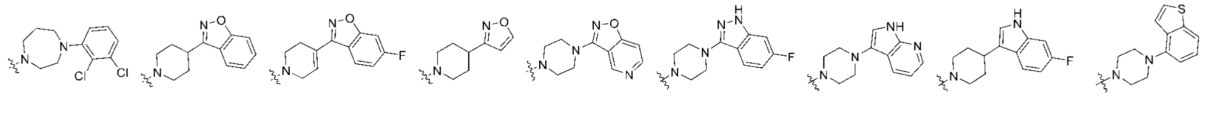

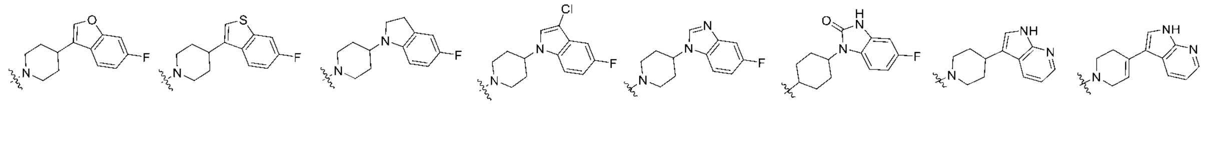

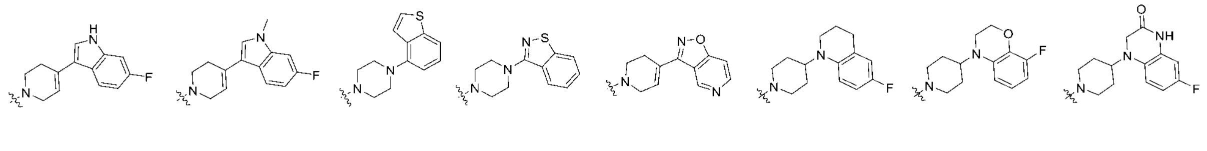

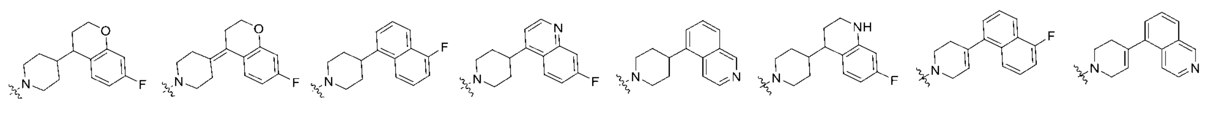

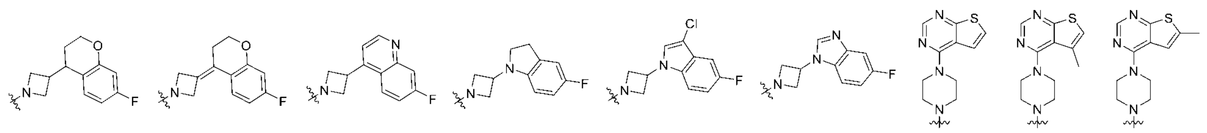

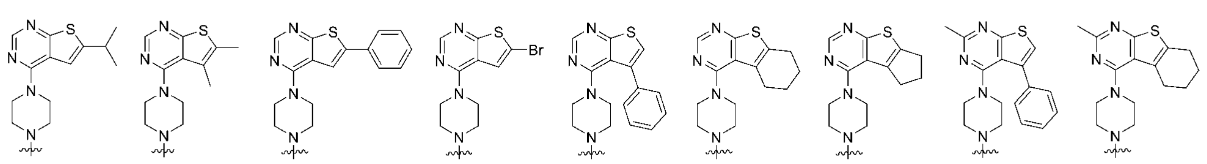

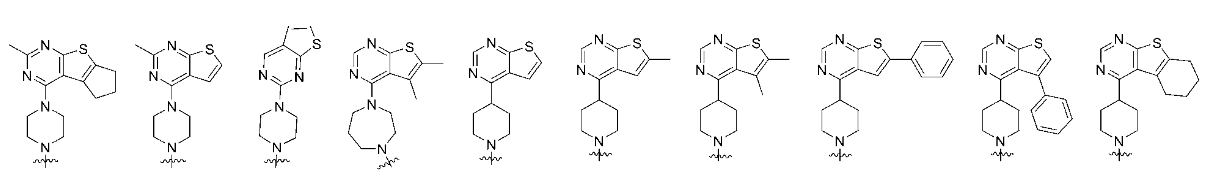

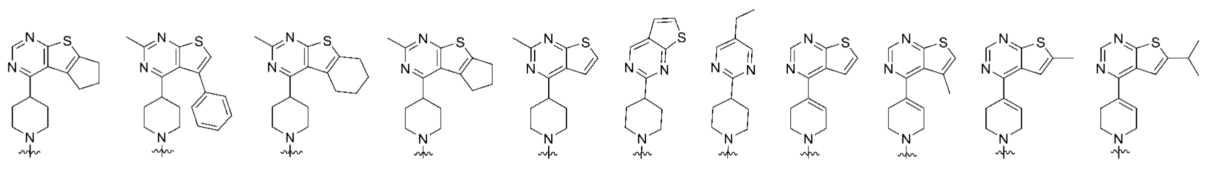

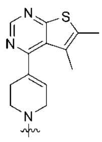 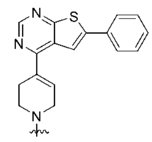 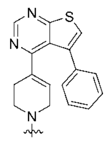 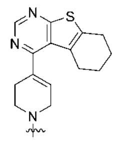 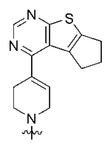 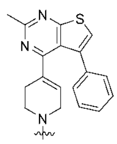 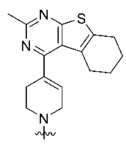 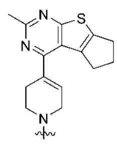 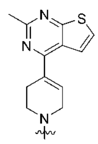 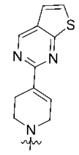

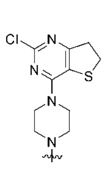 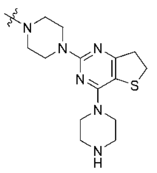 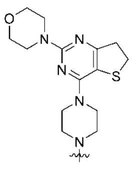 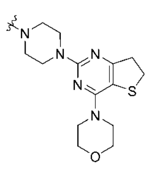 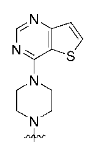 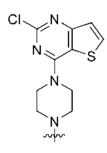 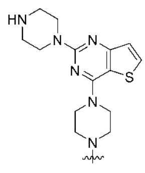 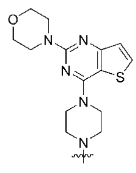 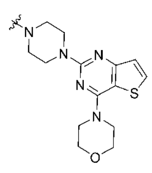

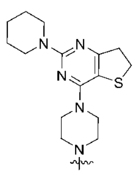 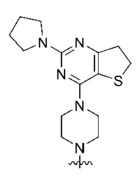 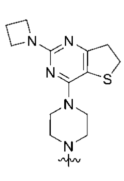 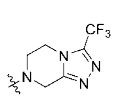 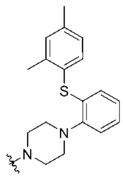 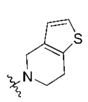 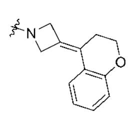 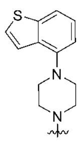 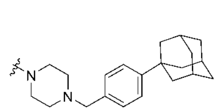

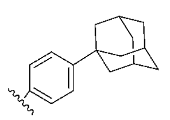 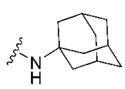 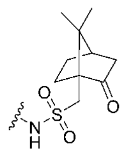 or 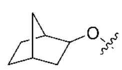 .

**11.** The compound of Formula (I) or salts, enantiomers, stereoisomers, solvates, or polymorphs thereof as claimed in claim 1, which is selected from:

3-(5-((4-benzhydrylpiperazin-1-yl)methyl)-1-thioxoisoindolin-2-yl)piperidine-2,6-dione;
3-(5-((4-(phenyl(pyridin-2-yl)methyl)piperazin-1-yl)methyl)-1-thioxoisoindolin-2-yl)piperidine-2,6-dione;
3-(5-((4-(phenyl(pyridin-3-yl)methyl)piperazin-1-yl)methyl)-1-thioxoisoindolin-2-yl)piperidine-2,6-dione;
3-(5-((4-(phenyl(pyridin-4-yl)methyl)piperazin-1-yl)methyl)-1-thioxoisoindolin-2-yl)piperidine-2,6-dione;
3-(5-((4-(di(pyridin-2-yl)methyl)piperazin-1-yl)methyl)-1-thioxoisoindolin-2-yl)piperidine-2,6-dione;
3-(5-((4-(di(pyridin-3-yl)methyl)piperazin-1-yl)methyl)-1-thioxoisoindolin-2-yl)piperidine-2,6-dione;
3-(5-((4-(bis(3-fluorophenyl)methyl)piperazin-1-yl)methyl)-1-thioxoisoindolin-2-yl)piperidine-2,6-dione;
3-(5-((4-(bis(4-fluorophenyl)methyl)piperazin-1-yl)methyl)-1-thioxoisoindolin-2-yl)piperidine-2,6-dione;
3-(5-((4-(bis(4-chlorophenyl)methyl)piperazin-1-yl)methyl)-1-thioxoisoindolin-2-yl)piperidine-2,6-dione;
3-(5-((3-benzhydryl-3,6-diazabicyclo[3.1.1]heptan-6-yl)methyl)-1-thioxoisoindolin-2-yl)piperidine-2,6-dione;
3-(5-((5-benzhydryl-2,5-diazabicyclo[2.2.1]heptan-2-yl)methyl)-1-thioxoisoindolin-2-yl)piperidine-2,6-dione;
3-(5-(((1R,4R)-5-benzhydryl-2,5-diazabicyclo[2.2.1]heptan-2-yl)methyl)-1-thioxoisoindolin-2-yl)piperidine-2,6-dione;
3-(5-(((1S,4S)-5-benzhydryl-2,5-diazabicyclo[2.2.1]heptan-2-yl)methyl)-1-thioxoisoindolin-2-yl)piperidine-2,6-dione;
3-(5-((8-benzhydryl-3,8-diazabicyclo[3.2.1]octan-3-yl)methyl)-1-thioxoisoindolin-2-yl)piperidine-2,6-dione;
3-(5-((3-benzhydryl-3,8-diazabicyclo[3.2.1]octan-8-yl)methyl)-1-thioxoisoindolin-2-yl)piperidine-2,6-dione;
3-(5-((5-benzhydryl-2,5-diazabicyclo[2.2.2]octan-2-yl)methyl)-1-thioxoisoindolin-2-yl)piperidine-2,6-dione;
3-(5-((4-benzhydryl-1,4-diazepan-1-yl)methyl)-1-thioxoisoindolin-2-yl)piperidine-2,6-dione;
3-(5-((4-benzhydryl-3,5-dimethylpiperazin-1-yl)methyl)-1-thioxoisoindolin-2-yl)piperidine-2,6-dione;
3-(5-((4-benzhydryl-2,6-dimethylpiperazin-1-yl)methyl)-1-thioxoisoindolin-2-yl)piperidine-2,6-dione;
3-(5-((4-benzhydryl-3,3-dimethylpiperazin-1-yl)methyl)-1-thioxoisoindolin-2-yl)piperidine-2,6-dione;
3-(5-((4-benzhydryl-2-(trifluoromethyl)piperazin-1-yl)methyl)-1-thioxoisoindolin-2-yl)piperidine-2,6-dione;
3-(5-(((R)-4-benzhydryl-2-methylpiperazin-1-yl)methyl)-1-thioxoisoindolin-2-yl)piperidine-2,6-dione;
3-(5-((3-benzhydryl-2-oxoimidazolidin-1-yl)methyl)-1-thioxoisoindolin-2-yl)piperidine-2,6-dione;
3-(5-((4-(diphenylamino)piperidin-1-yl)methyl)-1-thioxoisoindolin-2-yl)piperidine-2,6-dione;
3-(5-((4-(phenyl(pyridin-2-yl)amino)piperidin-1-yl)methyl)-1-thioxoisoindolin-2-yl)piperidine-2,6-dione;
3-(5-((4-((4'-chloro-5,5-dimethyl-3,4,5,6-tetrahydro-[1,1'-biphenyl]-2-yl)methyl)piperazin-1-yl)methyl)-1-thioxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-thioxoisoindolin-2-yl)piperidine-2,6-dione;
3-(5-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-thioxoisoindolin-2-yl)piperidine-2,6-dione;
3-(5-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-thioxoisoindolin-2-yl)piperidine-2,6-dione;
3-(5-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-thioxoisoindolin-2-yl)piperidine-2,6-dione;
3-(5-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-thioxoisoindolin-2-yl)piperidine-2,6-dione;
3-(5-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-thioxoisoindolin-2-yl)piperidine-2,6-dione;
3-(5-((4-((4'-chloro-5,5-dimethyl-3,4,5,6-tetrahydro-[1,1'-biphenyl]-2-yl)methyl)-2-(trifluoromethyl)piperazin-1-yl)methyl)-1-thioxoisoindolin-2-yl)piperidine-2,6-dione;
3-(5-((4-((4'-fluoro-3,4,5,6-tetrahydro-[1,1'-biphenyl]-2-yl)methyl)piperazin-1-yl)methyl)-1-thioxoisoindolin-2-yl)piperidine-2,6-dione;
3-(5-((4-(4'-fluoro-3,4,5,6-tetrahydro-[1,1'-biphenyl]-2-carbonyl)piperazin-1-yl)methyl)-1-thioxoisoindolin-2-yl) piperidine-2,6-dione;
3-(5-((3-(4'-fluoro-3,4,5,6-tetrahydro-[1,1'-biphenyl]-2-carbonyl)-3,6-diazabicyclo[3.1.1]heptan-6-yl)methyl)-1-thioxoisoindolin-2-yl)piperidine-2,6-dione;
3-(5-((4-((4'-chloro-[1,1'-biphenyl]-2-yl)amino)piperidin-1-yl)methyl)-1-thioxoisoindolin-2-yl)piperidine-2,6-dione;
3-(5-((4-((4'-chloro-[1,1'-biphenyl]-2-yl)methyl)piperazin-1-yl)methyl)-1-thioxoisoindolin-2-yl)piperidine-2,6-dione;
3-(5-((4-((4-(4-chlorophenyl)-6,6-dimethyl-5,6-dihydro-2H-pyran-3-yl)methyl)piperazin-1-yl)methyl)-1-thioxoisoindolin-2-yl)piperidine-2,6-dione;
3-(5-((4-(thiophen-2-yl)piperazin-1-yl)methyl)-1-thioxoisoindolin-2-yl)piperidine-2,6-dione;
3-(5-((4-(5-methylthiophen-2-yl)piperazin-1-yl)methyl)-1-thioxoisoindolin-2-yl)piperidine-2,6-dione;
3-(5-((4-(3-methylthiophen-2-yl)piperazin-1-yl)methyl)-1-thioxoisoindolin-2-yl)piperidine-2,6-dione;
3-(5-((4-(thiophen-3-yl)piperazin-1-yl)methyl)-1-thioxoisoindolin-2-yl)piperidine-2,6-dione;
3-(5-((4-(4-methylthiophen-3-yl)piperazin-1-yl)methyl)-1-thioxoisoindolin-2-yl)piperidine-2,6-dione;
3-(5-((4-(5-methylthiophen-3-yl)piperazin-1-yl)methyl)-1-thioxoisoindolin-2-yl)piperidine-2,6-dione;
3-(5-((4-(thiophen-2-yl)-3,6-dihydropyridin-1 (2H)-yl)methyl)-1-thioxoisoindolin-2-yl)piperidine-2,6-dione;
3-(5-((4-(5-methylthiophen-2-yl)-3,6-dihydropyridin-1(2H)-yl)methyl)-1-thioxoisoindolin-2-yl)piperidine-2,6-dione;
3-(5-((4-(3-methylthiophen-2-yl)-3,6-dihydropyridin-1(2H)-yl)methyl)-1-thioxoisoindolin-2-yl)piperidine-2,6-dione;
3-(5-((4-(thiophen-3-yl)-3,6-dihydropyridin-1(2H)-yl)methyl)-1-thioxoisoindolin-2-yl)piperidine-2,6-dione;
3-(5-((4-(4-methylthiophen-3-y1)-3,6-dihydropyridin-1(2H)-yl)methyl)-1-thioxoisoindolin-2-yl)piperidine-2,6-dione;
3-(5-((4-(5-methylthiophen-3-yl)-3,6-dihydropyridin-1(2H)-yl)methyl)-1-thioxoisoindolin-2-yl)piperidine-2,6-dione;
3-(5-((4-(furan-2-yl)-3,6-dihydropyridin-1(2H)-yl)methyl)-1-thioxoisoindolin-2-yl)piperidine-2,6-dione;
3-(5-((4-(furan-3-yl)-3,6-dihydropyridin-1(2H)-yl)methyl)-1-thioxoisoindolin-2-yl)piperidine-2,6-dione;
3-(5-((4-(thiophen-2-yl)piperidin-1-yl)methyl)-1-thioxoisoindolin-2-yl)piperidine-2,6-dione;
3-(5-((4-(5-methylthiophen-2-yl)piperidin-1-yl)methyl)-1-thioxoisoindolin-2-yl)piperidine-2,6-dione;
3-(5-((4-(3-methylthiophen-2-yl)piperidin-1-yl)methyl)-1-thioxoisoindolin-2-yl)piperidine-2,6-dione;
3-(5-((4-(thiophen-3-yl)piperidin-1-yl)methyl)-1-thioxoisoindolin-2-yl)piperidine-2,6-dione;
3-(5-((4-(5-methylthiophen-3-yl)piperidin-1-yl)methyl)-1-thioxoisoindolin-2-yl)piperidine-2,6-dione;
3-(5-((4-(4-methylthiophen-3-yl)piperidin-1-yl)methyl)-1-thioxoisoindolin-2-yl)piperidine-2,6-dione;
3-(5-((4-(furan-2-yl)piperidin-1-yl)methyl)-1-thioxoisoindolin-2-yl)piperidine-2,6-dione;
3-(5-((4-(furan-3-yl)piperidin-1-yl)methyl)-1-thioxoisoindolin-2-yl)piperidine-2,6-dione;
3-(5-((4-(2,3-dichlorophenyl)piperazin-1-yl)methyl)-1-thioxoisoindolin-2-yl)piperidine-2,6-dione;
3-(5-((4-phenylpiperazin-1-yl)methyl)-1-thioxoisoindolin-2-yl)piperidine-2,6-dione;
3-(5-((4-(2-methoxyphenyl)piperazin-1-yl)methyl)-1-thioxoisoindolin-2-yl)piperidine-2,6-dione;
3-(5-((4-(3-methoxyphenyl)piperazin-1-yl)methyl)-1-thioxoisoindolin-2-yl)piperidine-2,6-dione;
3-(5-((4-(4-methoxyphenyl)piperazin-1-yl)methyl)-1-thioxoisoindolin-2-yl)piperidine-2,6-dione;

3-(1-thioxo-5-((4-(4-(trifluoromethyl)phenyl)piperazin-1-yl)methyl)isoindolin-2-yl)piperidine-2,6-dione;
3-(5-((4-(3-fluorophenyl)piperazin-1-yl)methyl)-1-thioxoisoindolin-2-yl)piperidine-2,6-dione;
3-(5-((4-(4-fluorophenyl)piperazin-1-yl)methyl)-1-thioxoisoindolin-2-yl)piperidine-2,6-dione;
3-(5-((4-(2-chlorophenyl)piperazin-1-yl)methyl)-1-thioxoisoindolin-2-yl)piperidine-2,6-dione;
3-(5-((4-(4-chlorophenyl)piperazin-1-yl)methyl)-1-thioxoisoindolin-2-yl)piperidine-2,6-dione;
3-(5-((4-(2-bromophenyl)piperazin-1-yl)methyl)-1-thioxoisoindolin-2-yl)piperidine-2,6-dione;
3-(5-((4-(3-bromophenyl)piperazin-1-yl)methyl)-1-thioxoisoindolin-2-yl)piperidine-2,6-dione;
3-(5-((4-(4-bromophenyl)piperazin-1-yl)methyl)-1-thioxoisoindolin-2-yl)piperidine-2,6-dione;
3-(1-thioxo-5-((4-(o-tolyl)piperazin-1-yl)methyl)isoindolin-2-yl)piperidine-2,6-dione;
3-(1-thioxo-5-((4-(m-tolyl)piperazin-1-yl)methyl)isoindolin-2-yl)piperidine-2,6-dione;
3-(1-thioxo-5-((4-(p-tolyl)piperazin-1-yl)methyl)isoindolin-2-yl)piperidine-2,6-dione;
3-(5-((4-(2,4-dichlorophenyl)piperazin-1-yl)methyl)-1-thioxoisoindolin-2-yl)piperidine-2,6-dione;
3-(5-((4-(2,5-dichlorophenyl)piperazin-1-yl)methyl)-1-thioxoisoindolin-2-yl)piperidine-2,6-dione;
3-(5-((4-(2,6-dichlorophenyl)piperazin-1-yl)methyl)-1-thioxoisoindolin-2-yl)piperidine-2,6-dione;
3-(5-((4-(3,4-dichlorophenyl)piperazin-1-yl)methyl)-1-thioxoisoindolin-2-yl)piperidine-2,6-dione;
3-(5-((4-(3,5-dichlorophenyl)piperazin-1-yl)methyl)-1-thioxoisoindolin-2-yl)piperidine-2,6-dione;
3-(5-((4-(2,3-difluorophenyl)piperazin-1-yl)methyl)-1-thioxoisoindolin-2-yl)piperidine-2,6-dione;
3-(5-((4-(2,4-difluorophenyl)piperazin-1-yl)methyl)-1-thioxoisoindolin-2-yl)piperidine-2,6-dione;
3-(5-((4-(2,5-difluorophenyl)piperazin-1-yl)methyl)-1-thioxoisoindolin-2-yl)piperidine-2,6-dione;
3-(5-((4-(2,6-difluorophenyl)piperazin-1-yl)methyl)-1-thioxoisoindolin-2-yl)piperidine-2,6-dione;
3-(5-((4-(3,4-difluorophenyl)piperazin-1-yl)methyl)-1-thioxoisoindolin-2-yl)piperidine-2,6-dione;
3-(5-((4-(3,5-difluorophenyl)piperazin-1-yl)methyl)-1-thioxoisoindolin-2-yl)piperidine-2,6-dione;
3-(5-((4-(2,4-dimethylphenyl)piperazin-1-yl)methyl)-1-thioxoisoindolin-2-yl)piperidine-2,6-dione;
3-(5-((4-(5-chloro-2-methylphenyl)piperazin-1-yl)methyl)-1-thioxoisoindolin-2-yl)piperidine-2,6-dione;
3-(5-((4-phenylpiperidin-1-yl)methyl)-1-thioxoisoindolin-2-yl)piperidine-2,6-dione;
3-(5-((4-(3-bromophenyl)piperidin-1-yl)methyl)-1-thioxoisoindolin-2-yl)piperidine-2,6-dione;
3-(5-((4-(4-bromophenyl)piperidin-1-yl)methyl)-1-thioxoisoindolin-2-yl)piperidine-2,6-dione;
3-(5-((4-(2-chlorophenyl)piperidin-1-yl)methyl)-1-thioxoisoindolin-2-yl)piperidine-2,6-dione;
3-(5-((4-(4-chlorophenyl)piperidin-1-yl)methyl)-1-thioxoisoindolin-2-yl)piperidine-2,6-dione;
3-(5-((4-(2-fluorophenyl)piperidin-1-yl)methyl)-1-thioxoisoindolin-2-yl)piperidine-2,6-dione;
3-(5-((4-(4-fluorophenyl)piperidin-1-yl)methyl)-1-thioxoisoindolin-2-yl)piperidine-2,6-dione;
3-(1-thioxo-5-((4-(2-(trifluoromethyl)phenyl)piperidin-1-yl)methyl)isoindolin-2-yl)piperidine-2,6-dione;
3-(1-thioxo-5-((4-(3-(trifluoromethyl)phenyl)piperidin-1-yl)methyl)isoindolin-2-yl)piperidine-2,6-dione;
3-(5-((4-(2,3-dichlorophenyl)piperidin-1-yl)methyl)-1-thioxoisoindolin-2-yl)piperidine-2,6-dione;
3-(5-((4-(2-chloro-3-fluorophenyl)piperidin-1-yl)methyl)-1-thioxoisoindolin-2-yl)piperidine-2,6-dione;
3-(5-((4-(2,3-difluorophenyl)piperidin-1-yl)methyl)-1-thioxoisoindolin-2-yl)piperidine-2,6-dione;
3-(5-((4-(2,3-dichlorophenyl)-3,6-dihydropyridin-1(2H)-yl)methyl)-1-thioxoisoindolin-2-yl)piperidine-2,6-dione;
3-(5-((4-(2-chloro-3-fluorophenyl)-3,6-dihydropyridin-1(2H)-yl)methyl)-1-thioxoisoindolin-2-yl)piperidine-2,6-dione;
3-(5-((4-(pyridin-2-yl)piperazin-1-yl)methyl)-1-thioxoisoindolin-2-yl)piperidine-2,6-dione;
3-(5-((4-(6-methylpyridin-2-yl)piperazin-1-yl)methyl)-1-thioxoisoindolin-2-yl)piperidine-2,6-dione;
3-(5-((4-(6-chloropyridin-2-yl)piperazin-1-yl)methyl)-1-thioxoisoindolin-2-yl)piperidine-2,6-dione;
3-(5-((4-(5-chloropyridin-2-yl)piperazin-1-yl)methyl)-1-thioxoisoindolin-2-yl)piperidine-2,6-dione;
3-(5-((4-(4-chloropyridin-2-yl)piperazin-1-yl)methyl)-1-thioxoisoindolin-2-yl)piperidine-2,6-dione;
3-(5-((4-(3-chloropyridin-2-yl)piperazin-1-yl)methyl)-1-thioxoisoindolin-2-yl)piperidine-2,6-dione;
3-(5-((4-(3-fluoropyridin-2-yl)piperazin-1-yl)methyl)-1-thioxoisoindolin-2-yl)piperidine-2,6-dione;
3-(5-((4-(6-fluoropyridin-2-yl)piperazin-1-yl)methyl)-1-thioxoisoindolin-2-yl)piperidine-2,6-dione;
3-(5-((4-(5-fluoropyridin-2-yl)piperazin-1-yl)methyl)-1-thioxoisoindolin-2-yl)piperidine-2,6-dione;
3-(1-thioxo-5-((4-(3-(trifluoromethyl)pyridin-2-yl)piperazin-1-yl)methyl)isoindolin-2-yl)piperidine-2,6-dione;
3-(1-thioxo-5-((4-(6-(trifluoromethyl)pyridin-2-yl)piperazin-1-yl)methyl)isoindolin-2-yl)piperidine-2,6-dione;
3-(1-thioxo-5-((4-(5-(trifluoromethyl)pyridin-2-yl)piperazin-1-yl)methyl)isoindolin-2-yl)piperidine-2,6-dione;
3-(5-((4-(3,4-dichloropyridin-2-yl)piperazin-1-yl)methyl)-1-thioxoisoindolin-2-yl)piperidine-2,6-dione;
3-(5-((4-(3,5-dichloropyridin-2-yl)piperazin-l-yl)methyl)-1-thioxoisoindolin-2-yl)piperidine-2,6-dione;
3-(5-((4-(4,5-dichloropyridin-2-yl)piperazin-1-yl)methyl)-1-thioxoisoindolin-2-yl)piperidine-2,6-dione;
3-(5-((4-(3,4-difluoropyridin-2-yl)piperazin-l-yl)methyl)-1-thioxoisoindolin-2-yl)piperidine-2,6-dione;
3-(5-((4-(4-chloro-3-fluoropyridin-2-yl)piperazin-1-yl)methyl)-1-thioxoisoindolin-2-yl)piperidine-2,6-dione;
3-(5-((4-(3-fluoro-4-iodopyridin-2-yl)piperazin-1-yl)methyl)-1-thioxoisoindolin-2-yl)piperidine-2,6-dione;
3-(5-((4-(3-bromo-4-chloropyridin-2-yl)piperazin-1-yl)methyl)-1-thioxoisoindolin-2-yl)piperidine-2,6-dione;

3-(5-((4-(3-chloro-5-(trifluoromethyl)pyridin-2-yl)piperazin-1-yl)methyl)-1-thioxoisoindolin-2-yl)piperidine-2,6-dione;
3-(5-((4-(4-chloro-6-methylpyridin-2-yl)piperazin-1-yl)methyl)-1-thioxoisoindolin-2-yl)piperidine-2,6-dione;
3-(5-((4-(pyridin-3-yl)piperazin-1-yl)methyl)-1-thioxoisoindolin-2-yl)piperidine-2,6-dione;
3-(5-((4-(6-chloropyridin-3-yl)piperazin-1-yl)methyl)-1-thioxoisoindolin-2-yl)piperidine-2,6-dione;
3-(5-((4-(4,5-dichloropyridin-3-yl)piperazin-1-yl)methyl)-1-thioxoisoindolin-2-yl)piperidine-2,6-dione;
3-(5-((4-(2,4-dichloropyridin-3-yl)piperazin-1-yl)methyl)-1-thioxoisoindolin-2-yl)piperidine-2,6-dione;
3-(5-((4-(pyridin-4-yl)piperazin-1-yl)methyl)-1-thioxoisoindolin-2-yl)piperidine-2,6-dione;
3-(5-((4-(2-methylpyridin-4-yl)piperazin-1-yl)methyl)-1-thioxoisoindolin-2-yl)piperidine-2,6-dione;
3-(5-((4-(3-bromopyridin-4-yl)piperazin-1-yl)methyl)-1-thioxoisoindolin-2-yl)piperidine-2,6-dione;
3-(5-((4-(3-fluoropyridin-4-yl)piperazin-1-yl)methyl)-1-thioxoisoindolin-2-yl)piperidine-2,6-dione;
3-(5-((4-(3-chloropyridin-4-yl)piperazin-1-yl)methyl)-1-thioxoisoindolin-2-yl)piperidine-2,6-dione;
3-(5-((4-(2-chloropyridin-4-yl)piperazin-1-yl)methyl)-1-thioxoisoindolin-2-yl)piperidine-2,6-dione;
3-(5-((4-(2-chloro-3-fluoropyridin-4-yl)piperazin-1-yl)methyl)-1-thioxoisoindolin-2-yl)piperidine-2,6-dione;
3-(5-((4-(2-bromo-3-fluoropyridin-4-yl)piperazin-1-yl)methyl)-1-thioxoisoindolin-2-yl)piperidine-2,6-dione;
3-(5-((4-(3-bromo-2-chloropyridin-4-yl)piperazin-1-yl)methyl)-1-thioxoisoindolin-2-yl)piperidine-2,6-dione;
3-(5-((4-(2,3-dichloropyridin-4-yl)piperazin-1-yl)methyl)-1-thioxoisoindolin-2-yl)piperidine-2,6-dione;
3-(5-((4-(2,3-difluoropyridin-4-yl)piperazin-1-yl)methyl)-1-thioxoisoindolin-2-yl)piperidine-2,6-dione;
3-(5-((4-(3-fluoro-2-hydroxypyridin-4-yl)piperazin-1-yl)methyl)-1-thioxoisoindolin-2-yl)piperidine-2,6-dione;
3-(5-((4-(3-bromo-5-chloropyridin-4-yl)piperazin-1-yl)methyl)-1-thioxoisoindolin-2-yl)piperidine-2,6-dione;
3-(5-((4-(3-chloro-5-fluoropyridin-4-yl)piperazin-1-yl)methyl)-1-thioxoisoindolin-2-yl)piperidine-2,6-dione;
3-(5-((4-(3,5-dichloropyridin-4-yl)piperazin-1-yl)methyl)-1-thioxoisoindolin-2-yl)piperidine-2,6-dione;
3-(5-((4-(2,6-dimethylpyridin-4-yl)piperazin-1-yl)methyl)-1-thioxoisoindolin-2-yl)piperidine-2,6-dione;
3-(5-((4-(perfluoropyridin-4-yl)piperazin-1-yl)methyl)-1-thioxoisoindolin-2-yl)piperidine-2,6-dione;
3-(5-((4-(pyridazin-3-yl)piperazin-1-yl)methyl)-1-thioxoisoindolin-2-yl)piperidine-2,6-dione;
3-(5-((4-(6-chloropyridazin-3-yl)piperazin-1-yl)methyl)-1-thioxoisoindolin-2-yl)piperidine-2,6-dione;
3-(5-((4-(pyrimidin-2-yl)piperazin-1-yl)methyl)-1-thioxoisoindolin-2-yl)piperidine-2,6-dione;
3-(5-((4-(pyrazin-2-yl)piperazin-1-yl)methyl)-1-thioxoisoindolin-2-yl)piperidine-2,6-dione;
3-(5-((4-(5,6-dichloropyrimidin-4-yl)piperazin-1-yl)methyl)-1-thioxoisoindolin-2-yl)piperidine-2,6-dione;
3-(5-((4-(3,6-dichloropyridazin-4-yl)piperazin-1-yl)methyl)-1-thioxoisoindolin-2-yl)piperidine-2,6-dione;
3-(5-((4-(5,6-dichloropyridazin-4-yl)piperazin-1-yl)methyl)-1-thioxoisoindolin-2-yl)piperidine-2,6-dione;
3-(5-((4-(1,3,5-triazin-2-yl)piperazin-1-yl)methyl)-1-thioxoisoindolin-2-yl)piperidine-2,6-dione;
3-(5-((4-(3,4-dichloropyridin-2-yl)piperidin-l-yl)methyl)-1-thioxoisoindolin-2-yl)piperidine-2,6-dione;
3-(5-((4-(2,3-dichloropyridin-4-yl)piperidin-1-yl)methyl)-1-thioxoisoindolin-2-yl)piperidine-2,6-dione;
3-(5-((4-(2,3-difluoropyridin-4-yl)piperidin-1-yl)methyl)-1-thioxoisoindolin-2-yl)piperidine-2,6-dione;
3-(5-([3,4'-bipiperidin]-1-ylmethyl)-1-thioxoisoindolin-2-yl)piperidine-2,6-dione;
3-(5-((3,4-dichloro-3',6'-dihydro-[2,4'-bipyridin]-1'(2'H)-yl)methyl)-1-thioxoisoindolin-2-yl)piperidine-2,6-dione;
3-(5-((4,5-dichloro-3',6'-dihydro-[3,4'-bipyridin]-1'(2'H)-yl)methyl)-1-thioxoisoindolin-2-yl)piperidine-2,6-dione;
3-(5-((5-chloro-3',6'-dihydro-[3,4'-bipyridin]-1'(2'H)-yl)methyl)-1-thioxoisoindolin-2-yl)piperidine-2,6-dione;
3-(5-((2',3'-dichloro-3,6-dihydro-[4,4'-bipyridin]-1(2H)-yl)methyl)-1-thioxoisoindolin-2-yl)piperidine-2,6-dione;
3-(5-((3-fluoro-3',6'-dihydro-[2,4'-bipyridin]-1'(2'H)-yl)methyl)-1-thioxoisoindolin-2-yl)piperidine-2,6-dione;
3-(5-((2',3'-difluoro-3,6-dihydro-[4,4'-bipyridin]-1(2H)-yl)methyl)-1-thioxoisoindolin-2-yl)piperidine-2,6-dione;
3-(5-((3-(2,3-dichlorophenyl)-3,6-diazabicyclo[3.1.1]heptan-6-yl)methyl)-1-thioxoisoindolin-2-yl)piperidine-2,6-dione;
3-(5-((6-(2,3-dichlorophenyl)-3,6-diazabicyclo[3.1.1]heptan-3-yl)methyl)-1-thioxoisoindolin-2-yl)piperidine-2,6-dione;
3-(5-(((1R,4R)-5-(2,3-dichlorophenyl)-2,5-diazabicyclo[2.2.1]heptan-2-yl)methyl)-1-thioxoisoindolin-2-yl)piperidine-2,6-dione;
3-(5-(((1S,4S)-5-(2,3-dichlorophenyl)-2,5-diazabicyclo[2.2.1]heptan-2-yl)methyl)-1-thioxoisoindolin-2-yl)piperidine-2,6-dione;
3-(5-((5-(2,3-dichlorophenyl)-2,5-diazabicyclo[2.2.2]octan-2-yl)methyl)-1-thioxoisoindolin-2-yl)piperidine-2,6-dione;
3-(5-((8-(2,3-dichlorophenyl)-3,8-diazabicyclo[3.2.1]octan-3-yl)methyl)-1-thioxoisoindolin-2-yl)piperidine-2,6-dione;
3-(5-((3-(2,3-dichlorophenyl)-3,8-diazabicyclo[3.2.1]octan-8-yl)methyl)-1-thioxoisoindolin-2-yl)piperidine-2,6-dione;
3-(5-((4-(2,3-dichlorophenyl)-1,4-diazepan-1-yl)methyl)-1-thioxoisoindolin-2-yl)piperidine-2,6-dione;
3-(5-((4-(6-fluorobenzo[d]isoxazol-3-yl)piperidin-1-yl)methyl)-1-thioxoisoindolin-2-yl)piperidine-2,6-dione;

3-(5-((4-(benzo[d]isoxazol-3-yl)piperidin-1-yl)methyl)-1-thioxoisoindolin-2-yl)piperidine-2,6-dione;
3-(5-((4-(isoxazol-3-yl)piperidin-1-yl)methyl)-1-thioxoisoindolin-2-yl)piperidine-2,6-dione;
3-(5-((4-(6-fluorobenzo[d]isoxazol-3-yl)piperazin-1-yl)methyl)-1-thioxoisoindolin-2-yl)piperidine-2,6-dione;
3-(5-((4-(isoxazolo[4,5-c]pyridin-3-yl)piperazin-1-yl)methyl)-1-thioxoisoindolin-2-yl)piperidine-2,6-dione;
3-(5-((4-(6-fluorobenzo[d]isothiazol-3-yl)piperazin-1-yl)methyl)-1-thioxoisoindolin-2-yl)piperidine-2,6-dione;
3-(5-((4-(6-fluoro-1H-indazol-3-yl)piperazin-1-yl)methyl)-1-thioxoisoindolin-2-yl)piperidine-2,6-dione;
3-(5-((4-(1H-pyrrolo[2,3-b]pyridin-3-yl)piperazin-1-yl)methyl)-1-thioxoisoindolin-2-yl)piperidine-2,6-dione;
3-(5-((4-(6-fluoro-1H-indol-3-yl)piperidin-1-yl)methyl)-1-thioxoisoindolin-2-yl)piperidine-2,6-dione;
3-(5-((4-(6-fluoro-1-methyl-1H-indol-3-yl)piperidin-1-yl)methyl)-1-thioxoisoindolin-2-yl)piperidine-2,6-dione;
3-(5-((4-(6-fluoro-1H-indazol-3-yl)piperidin-1-yl)methyl)-1-thioxoisoindolin-2-yl)piperidine-2,6-dione;
3-(5-((4-(5-fluoroindolin-1-yl)piperidin-1-yl)methyl)-1-thioxoisoindolin-2-yl)piperidine-2,6-dione;
3-(5-((4-(5-fluoro-1H-benzo[d]imidazol-1-yl)piperidin-1-yl)methyl)-1-thioxoisoindolin-2-yl)piperidine-2,6-dione;
3-(5-((4-(5-fluoro-2-oxo-2,3-dihydro-1H-benzo[d]imidazol-1-yl)piperidin-1-yl)methyl)-1-thioxoisoindolin-2-yl)piperidine-2,6-dione;
3-(5-((4-(1H-pyrrolo[2,3-b]pyridin-3-yl)piperidin-1-yl)methyl)-1-thioxoisoindolin-2-yl)piperidine-2,6-dione;
3-(5-((4-(1H-pyrrolo[2,3-b]pyridin-3-yl)-3,6-dihydropyridin-1(2H)-yl)methyl)-1-thioxoisoindolin-2-yl)piperidine-2,6-dione;
3-(5-((4-(6-fluoro-1H-indol-3-yl)-3,6-dihydropyridin-1(2H)-yl)methyl)-1-thioxoisoindolin-2-yl)piperidine-2,6-dione;
3-(5-((4-(6-fluoro-1-methyl-1H-indol-3-yl)-3,6-dihydropyridin-1(2H)-yl)methyl)-1-thioxoisoindolin-2-yl)piperidine-2,6-dione;
3-(5-((4-(6-fluoro-1H-indazol-3-yl)-3,6-dihydropyridin-1(2H)-yl)methyl)-1-thioxoisoindolin-2-yl)piperidine-2,6-dione;
3-(5-((4-(6-fluoro-3,4-dihydroquinolin-1(2H)-yl)piperidin-1-yl)methyl)-1-thioxoisoindolin-2-yl)piperidine-2,6-dione;
3-(5-((4-(7-fluoro-2,3-dihydro-4H-benzo[b][1,4]oxazin-4-yl)piperidin-1-yl)methyl)-1-thioxoisoindolin-2-yl)piperidine-2,6-dione;
3-(5-((4-(7-fluoro-3-oxo-2,3-dihydro-4H-benzo[b][1,4]oxazin-4-yl)piperidin-1-yl)methyl)-1-thioxoisoindolin-2-yl)piperidine-2,6-dione;
3-(5-((4-(7-fluorochroman-4-yl)piperidin-1-yl)methyl)-1-thioxoisoindolin-2-yl)piperidine-2,6-dione;
3-(5-((4-(7-fluorochroman-4-ylidene)piperidin-1-yl)methyl)-1-thioxoisoindolin-2-yl)piperidine-2,6-dione;
3-(5-((4-(5-fluoronaphthalen-1-yl)piperidin-1-yl)methyl)-1-thioxoisoindolin-2-yl)piperidine-2,6-dione;
3-(5-((4-(7-fluoroquinolin-4-yl)piperidin-1-yl)methyl)-1-thioxoisoindolin-2-yl)piperidine-2,6-dione;
3-(5-((4-(isoquinolin-5-yl)piperidin-1-yl)methyl)-1-thioxoisoindolin-2-yl)piperidine-2,6-dione;
3-(5-((4-(5-fluoronaphthalen-1-yl)-3,6-dihydropyridin-1(2H)-yl)methyl)-1-thioxoisoindolin-2-yl)piperidine-2,6-dione;
3-(5-((4-(7-fluoroquinolin-4-yl)-3,6-dihydropyridin-1(2H)-yl)methyl)-1-thioxoisoindolin-2-yl)piperidine-2,6-dione;
3-(5-((4-(isoquinolin-5-y1)-3,6-dihydropyridin-1(2H)-yl)methyl)-1-thioxoisoindolin-2-yl)piperidine-2,6-dione;
3-(5-((3-(7-fluorochroman-4-yl)azetidin-1-yl)methyl)-1-thioxoisoindolin-2-yl)piperidine-2,6-dione;
3-(5-((3-(7-fluorochroman-4-ylidene)azetidin-1-yl)methyl)-1-thioxoisoindolin-2-yl)piperidine-2,6-dione;
3-(5-((3-(7-fluoroquinolin-4-yl)azetidin-1-yl)methyl)-1-thioxoisoindolin-2-yl)piperidine-2,6-dione;
3-(5-((3-(5-fluoroindolin-1-yl)azetidin-1-yl)methyl)-1-thioxoisoindolin-2-yl)piperidine-2,6-dione;
3-(5-((3-(5-fluoro-1H-benzo[d]imidazol-1-yl)azetidin-1-yl)methyl)-1-thioxoisoindolin-2-yl)piperidine-2,6-dione;
3-(5-((4-(thieno[2,3-d]pyrimidin-4-yl)piperazin-1-yl)methyl)-1-thioxoisoindolin-2-yl)piperidine-2,6-dione;
3-(5-((4-(5-methylthieno[2,3-d]pyrimidin-4-yl)piperazin-1-yl)methyl)-1-thioxoisoindolin-2-yl)piperidine-2,6-dione;
3-(5-((4-(6-methylthieno[2,3-d]pyrimidin-4-yl)piperazin-1-yl)methyl)-1-thioxoisoindolin-2-yl)piperidine-2,6-dione;
3-(5-((4-(6-isopropylthieno[2,3-d]pyrimidin-4-yl)piperazin-1-yl)methyl)-1-thioxoisoindolin-2-yl)piperidine-2,6-dione;
3-(5-((4-(5,6-dimethylthieno[2,3-d]pyrimidin-4-yl)piperazin-1-yl)methyl)-1-thioxoisoindolin-2-yl)piperidine-2,6-dione;
3-(5-((4-(6-phenylthieno[2,3-d]pyrimidin-4-yl)piperazin-1-yl)methyl)-1-thioxoisoindolin-2-yl)piperidine-2,6-dione;
3-(5-((4-(6-bromothieno[2,3-d]pyrimidin-4-yl)piperazin-1-yl)methyl)-1-thioxoisoindolin-2-yl)piperidine-2,6-dione;
3-(5-((4-(5-phenylthieno[2,3-d]pyrimidin-4-yl)piperazin-1-yl)methyl)-1-thioxoisoindolin-2-yl)piperidine-2,6-dione;
3-(5-((4-(5,6,7,8-tetrahydrobenzo[4,5]thieno[2,3-d]pyrimidin-4-yl)piperazin-1-yl)methyl)-1-thioxoisoindolin-2-yl)piperidine-2,6-dione;
3-(5-((4-(6,7-dihydro-5H-cyclopenta[4,5]thieno[2,3-d]pyrimidin-4-yl)piperazin-1-yl)methyl)-1-thioxoisoindolin-2-yl)piperidine-2,6-dione;
3-(5-((4-(2-methyl-5-phenylthieno[2,3-d]pyrimidin-4-yl)piperazin-1-yl)methyl)-1-thioxoisoindolin-2-yl)piperidine-2,6-dione;
3-(5-((4-(2-methyl-5,6,7,8-tetrahydrobenzo[4,5]thieno[2,3-d]pyrimidin-4-yl)piperazin-1-yl)methyl)-1-thioxoisoindolin-2-yl)piperidine-2,6-dione;
3-(5-((4-(2-methyl-6,7-dihydro-5H-cyclopenta[4,5]thieno[2,3-d]pyrimidin-4-yl)piperazin-1-yl)methyl)-1-thioxoisoindolin-2-yl)piperidine-2,6-dione;
3-(5-((4-(2-methylthieno[2,3-d]pyrimidin-4-yl)piperazin-1-yl)methyl)-1-thioxoisoindolin-2-yl)piperidine-2,6-dione;
3-(5-((4-(thieno[2,3-d]pyrimidin-2-yl)piperazin-1-yl)methyl)-1-thioxoisoindolin-2-yl)piperidine-2,6-dione;
3-(5-((4-(5,6-dimethylthieno[2,3-d]pyrimidin-4-yl)-1,4-diazepan-1-yl)methyl)-1-thioxoisoindolin-2-yl)piperidine-2,6-dione;
3-(5-((4-(thieno[2,3-d]pyrimidin-4-yl)piperidin-1-yl)methyl)-1-thioxoisoindolin-2-yl)piperidine-2,6-dione;
3-(5-((4-(6-methylthieno[2,3-d]pyrimidin-4-yl)piperidin-1-yl)methyl)-1-thioxoisoindolin-2-yl)piperidine-2,6-dione;
3-(5-((4-(5,6-dimethylthieno[2,3-d]pyrimidin-4-yl)piperidin-1-yl)methyl)-1-thioxoisoindolin-2-yl)piperidine-2,6-dione;
3-(5-((4-(6-phenylthieno[2,3-d]pyrimidin-4-yl)piperidin-1-yl)methyl)-1-thioxoisoindolin-2-yl)piperidine-2,6-dione;
3-(5-((4-(5-phenylthieno[2,3-d]pyrimidin-4-yl)piperidin-1-yl)methyl)-1-thioxoisoindolin-2-yl)piperidine-2,6-dione;
3-(5-((4-(5,6,7,8-tetrahydrobenzo[4,5]thieno[2,3-d]pyrimidin-4-yl)piperidin-1-yl)methyl)-1-thioxoisoindolin-2-yl)piperidine-2,6-dione;
3-(5-((4-(6,7-dihydro-5H-cyclopenta[4,5]thieno[2,3-d]pyrimidin-4-yl)piperidin-1-yl)methyl)-1-thioxoisoindolin-2-yl)piperidine-2,6-dione;
3-(5-((4-(2-methyl-5-phenylthieno[2,3-d]pyrimidin-4-yl)piperidin-1-yl)methyl)-1-thioxoisoindolin-2-yl)piperidine-2,6-dione;
3-(5-((4-(2-methyl-5,6,7,8-tetrahydrobenzo[4,5]thieno[2,3-d]pyrimidin-4-yl)piperidin-1-yl)methyl)-1-thioxoisoindolin-2-yl)piperidine-2,6-dione;
3-(5-((4-(2-methyl-6,7-dihydro-5H-cyclopenta[4,5]thieno[2,3-d]pyrimidin-4-yl)piperidin-1-yl)methyl)-1-thioxoisoindolin-2-yl)piperidine-2,6-dione;
3-(5-((4-(2-methylthieno[2,3-d]pyrimidin-4-yl)piperidin-1-yl)methyl)-1-thioxoisoindolin-2-yl)piperidine-2,6-dione;
3-(5-((4-(thieno[2,3-d]pyrimidin-2-yl)piperidin-1-yl)methyl)-1-thioxoisoindolin-2-yl)piperidine-2,6-dione;
3-(5-((4-(5-ethylpyrimidin-2-yl)piperidin-1-yl)methyl)-1-thioxoisoindolin-2-yl)piperidine-2,6-dione;
3-(5-((4-(thieno[2,3-d]pyrimidin-4-yl)-3,6-dihydropyridin-1(2H)-yl)methyl)-1-thioxoisoindolin-2-yl)piperidine-2,6-dione;
3-(5-((4-(5-methylthieno[2,3-d]pyrimidin-4-yl)-3,6-dihydropyridin-1(2H)-yl)methyl)-1-thioxoisoindolin-2-yl)piperidine-2,6-dione;
3-(5-((4-(6-methylthieno[2,3-d]pyrimidin-4-yl)-3,6-dihydropyridin-1(2H)-yl)methyl)-1-thioxoisoindolin-2-yl)piperidine-2,6-dione;
3-(5-((4-(6-isopropylthieno[2,3-d]pyrimidin-4-yl)-3,6-dihydropyridin-1(2H)-yl)methyl)-1-thioxoisoindolin-2-yl)piperidine-2,6-dione;
3-(5-((4-(5,6-dimethylthieno[2,3-d]pyrimidin-4-yl)-3,6-dihydropyridin-1(2H)-yl)methyl)-1-thioxoisoindolin-2-yl)piperidine-2,6-dione;
3-(5-((4-(6-phenylthieno[2,3-d]pyrimidin-4-yl)-3,6-dihydropyridin-1(2H)-yl)methyl)-1-thioxoisoindolin-2-yl)piperidine-2,6-dione;
3-(5-((4-(5-phenylthieno[2,3-d]pyrimidin-4-yl)-3,6-dihydropyridin-1(2H)-yl)methyl)-1-thioxoisoindolin-2-yl)piperidine-2,6-dione;
3-(5-((4-(5,6,7,8-tetrahydrobenzo[4,5]thieno[2,3-d]pyrimidin-4-yl)-3,6-dihydropyridin-1(2H)-yl)methyl)-1-thioxoisoindolin-2-yl)piperidine-2,6-dione;
3-(5-((4-(6,7-dihydro-5H-cyclopenta[4,5]thieno[2,3-d]pyrimidin-4-yl)-3,6-dihydropyridin-1(2H)-yl)methyl)-1-thioxoisoindolin-2-yl)piperidine-2,6-dione;
3-(5-((4-(2-methyl-5-phenylthieno [2,3-d]pyrimidin-4-yl)-3,6-dihydropyridin-1 (2H)-yl)methyl)-1-thioxoisoindolin-2-yl)piperidine-2,6-dione;
3-(5-((4-(2-methyl-5,6,7,8-tetrahydrobenzo[4,5]thieno[2,3-d]pyrimidin-4-yl)-3,6-dihydropyridin-1(2H)-yl)methyl)-1-thioxoisoindolin-2-yl)piperidine-2,6-dione;
3-(5-((4-(2-methyl-6,7-dihydro-5H-cyclopenta[4,5]thieno[2,3-d]pyrimidin-4-yl)-3,6-dihydropyridin-1(2H)-yl)methyl)-1-thioxoisoindolin-2-yl)piperidine-2,6-dione;
3-(5-((4-(2-methylthieno[2,3-d]pyrimidin-4-yl)-3,6-dihydropyridin-1(2H)-yl)methyl)-1-thioxoisoindolin-2-yl)piperidine-2,6-dione;
3-(5-((4-(thieno[2,3-d]pyrimidin-2-yl)-3,6-dihydropyridin-1(2H)-yl)methyl)-1-thioxoisoindolin-2-yl)piperidine-2,6-dione;
3-(5-((4-(2-chloro-6,7-dihydrothieno[3,2-d]pyrimidin-4-yl)piperazin-1-yl)methyl)-1-thioxoisoindolin-2-yl)piperidine-2,6-dione;
3-(5-((4-(2-morpholino-6,7-dihydrothieno[3,2-d]pyrimidin-4-yl)piperazin-1-yl)methyl)-1-thioxoisoindolin-2-yl)piperidine-2,6-dione;
3-(5-((4-(4-(piperazin-1-yl)-6,7-dihydrothieno[3,2-d]pyrimidin-2-yl)piperazin-1-yl)methyl)-1-thioxoisoindolin-2-yl)piperidine-2,6-dione;
3-(5-((4-(4-morpholino-6,7-dihydrothieno[3,2-d]pyrimidin-2-yl)piperazin-1-yl)methyl)-1-thioxoisoindolin-2-yl)piperidine-2,6-dione;
3-(5-((4-(2-(piperidin-1-yl)-6,7-dihydrothieno[3,2-d]pyrimidin-4-yl)piperazin-1-yl)methyl)-1-thioxoisoindolin-2-yl)piperidine-2,6-dione;
3-(5-((4-(2-(pyrrolidin-1-yl)-6,7-dihydrothieno[3,2-d]pyrimidin-4-yl)piperazin-1-yl)methyl)-1-thioxoisoindolin-2-yl)piperidine-2,6-dione;
3-(5-((4-(2-(azetidin-1-yl)-6,7-dihydrothieno[3,2-d]pyrimidin-4-yl)piperazin-1-yl)methyl)-1-thioxoisoindolin-2-yl)piperidine-2,6-dione;
3-(5-((4-(thieno[3,2-d]pyrimidin-4-yl)piperazin-1-yl)methyl)-1-thioxoisoindolin-2-yl)piperidine-2,6-dione;
3-(5-((4-(2-chlorothieno[3,2-d]pyrimidin-4-yl)piperazin-1-yl)methyl)-1-thioxoisoindolin-2-yl)piperidine-2,6-dione;
3-(5-((4-(2-morpholinothieno[3,2-d]pyrimidin-4-yl)piperazin-1-yl)methyl)-1-thioxoisoindolin-2-yl)piperidine-2,6-dione;
3-(5-((4-(4-(piperazin-1-yl)thieno[3,2-d]pyrimidin-2-yl)piperazin-1-yl)methyl)-1-thioxoisoindolin-2-yl)piperidine-2,6-dione;
3-(5-((4-(4-morpholinothieno[3,2-d]pyrimidin-2-yl)piperazin-1-yl)methyl)-1-thioxoisoindolin-2-yl)piperidine-2,6-dione;
3-(5-((4-(benzo[b]thiophen-4-yl)piperazin-1-yl)methyl)-1-thioxoisoindolin-2-yl)piperidine-2,6-dione;
3-(1-thioxo-5-((3-(trifluoromethyl)-5,6-dihydro-[1,2,4]triazolo[4,3-a]pyrazin-7(8H)-yl)methyl)isoindolin-2-yl)piperidine-2,6-dione;
3-(5-((4-(2-((2,4-dimethylphenyl)thio)phenyl)piperazin-1-yl)methyl)-1-thioxoisoindolin-2-yl)piperidine-2,6-dione;
3-(5-((6,7-dihydrothieno[3,2-c]pyridin-5(4H)-yl)methyl)-1-thioxoisoindolin-2-yl)piperidine-2,6-dione;
3-(5-((4-(benzo[d]isothiazol-3-yl)piperazin-1-yl)methyl)-1-thioxoisoindolin-2-yl)piperidine-2,6-dione;
3-(5-((4-(4-(adamantan-1-yl)benzyl)piperazin-1-yl)methyl)-1-thioxoisoindolin-2-yl)piperidine-2,6-dione;
3-(5-(((adamantan-1-yl)amino)methyl)-1-thioxoisoindolin-2-yl)piperidine-2,6-dione;
1-((1R,4R)-7,7-dimethyl-2-oxobicyclo[2.2.1]heptan-1-yl)-N-((2-(2,6-dioxopiperidin-3-yl)-1-thioxoisoindolin-5-yl)methyl)methanesulfonamide;
3-(5-((((1R,4S)-bicyclo[2.2.1]heptan-2-yl)oxy)methyl)-1-thioxoisoindolin-2-yl)piperidine-2,6-dione;
3-(6-((4-benzhydrylpiperazin-1-yl)methyl)-1-thioxoisoindolin-2-yl)piperidine-2,6-dione;
3-(6-((4-(phenyl(pyridin-2-yl)methyl)piperazin-1-yl)methyl)-1-thioxoisoindolin-2-yl)piperidine-2,6-dione;
3-(6-((4-(phenyl(pyridin-3-yl)methyl)piperazin-1-yl)methyl)-1-thioxoisoindolin-2-yl)piperidine-2,6-dione;
3-(6-((4-(phenyl(pyridin-4-yl)methyl)piperazin-1-yl)methyl)-1-thioxoisoindolin-2-yl)piperidine-2,6-dione;
3-(6-((4-(di(pyridin-2-yl)methyl)piperazin-1-yl)methyl)-1-thioxoisoindolin-2-yl)piperidine-2,6-dione;
3-(6-((4-(di(pyridin-3-yl)methyl)piperazin-1-yl)methyl)-1-thioxoisoindolin-2-yl)piperidine-2,6-dione;
3-(6-((4-(bis(3-fluorophenyl)methyl)piperazin-1-yl)methyl)-1-thioxoisoindolin-2-yl)piperidine-2,6-dione;
3-(6-((4-(bis(4-fluorophenyl)methyl)piperazin-1-yl)methyl)-1-thioxoisoindolin-2-yl)piperidine-2,6-dione;
3-(6-((4-(bis(4-chlorophenyl)methyl)piperazin-1-yl)methyl)-1-thioxoisoindolin-2-yl)piperidine-2,6-dione;
3-(6-((3-benzhydryl-3,6-diazabicyclo[3.1.1]heptan-6-yl)methyl)-1-thioxoisoindolin-2-yl)piperidine-2,6-dione;
3-(6-((5-benzhydryl-2,5-diazabicyclo[2.2.1]heptan-2-yl)methyl)-1-thioxoisoindolin-2-yl)piperidine-2,6-dione;
3-(6-((8-benzhydryl-3,8-diazabicyclo[3.2.1]octan-3-yl)methyl)-1-thioxoisoindolin-2-yl)piperidine-2,6-dione;
3-(6-((3-benzhydryl-3,8-diazabicyclo[3.2.1]octan-8-yl)methyl)-1-thioxoisoindolin-2-yl)piperidine-2,6-dione;
3-(6-((5-benzhydryl-2,5-diazabicyclo[2.2.2]octan-2-yl)methyl)-1-thioxoisoindolin-2-yl)piperidine-2,6-dione;

3-(6-((4-benzhydryl-1,4-diazepan-1-yl)methyl)-1-thioxoisoindolin-2-yl)piperidine-2,6-dione;
3-(6-((4-benzhydryl-3,5-dimethylpiperazin-1-yl)methyl)-1-thioxoisoindolin-2-yl)piperidine-2,6-dione;
3-(6-((4-benzhydryl-2,6-dimethylpiperazin-1-yl)methyl)-1-thioxoisoindolin-2-yl)piperidine-2,6-dione;
3-(6-((4-benzhydryl-3,3-dimethylpiperazin-1-yl)methyl)-1-thioxoisoindolin-2-yl)piperidine-2,6-dione;
3-(6-((4-benzhydryl-2-(trifluoromethyl)piperazin-1-yl)methyl)-1-thioxoisoindolin-2-yl)piperidine-2,6-dione;
3-(6-(((R)-4-benzhydryl-2-methylpiperazin-1-yl)methyl)-1-thioxoisoindolin-2-yl)piperidine-2,6-dione;
3-(6-((3-benzhydryl-2-oxoimidazolidin-1-yl)methyl)-1-thioxoisoindolin-2-yl)piperidine-2,6-dione;
3-(6-((4-(diphenylamino)piperidin-1-yl)methyl)-1-thioxoisoindolin-2-yl)piperidine-2,6-dione;
3-(6-((4-(phenyl(pyridin-2-yl)amino)piperidin-1-yl)methyl)-1-thioxoisoindolin-2-yl)piperidine-2,6-dione;
3-(6-((4-((4'-chloro-5,5-dimethyl-3,4,5,6-tetrahydro-[1,1'-biphenyl]-2-yl)methyl)piperazin-1-yl)methyl)-1-thioxoisoindolin-2-yl)piperidine-2,6-dione;
3-(6-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-thioxoisoindolin-2-yl)piperidine-2,6-dione;
3-(6-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-thioxoisoindolin-2-yl)piperidine-2,6-dione;
3-(6-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-thioxoisoindolin-2-yl)piperidine-2,6-dione;
3-(6-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-thioxoisoindolin-2-yl)piperidine-2,6-dione;
3-(6-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-thioxoisoindolin-2-yl)piperidine-2,6-dione;
3-(6-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-thioxoisoindolin-2-yl)piperidine-2,6-dione;
3-(6-((4-((4'-chloro-5,5-dimethyl-3,4,5,6-tetrahydro-[1,1 '-biphenyl]-2-yl)methyl)-2-(trifluoromethyl)piperazin-1-yl)methyl)-1-thioxoisoindolin-2-yl)piperidine-2,6-dione;
3-(6-((4-((4'-fluoro-3,4,5,6-tetrahydro-[1,1'-biphenyl]-2-yl)methyl)piperazin-1-yl)methyl)-1-thioxoisoindolin-2-yl)piperidine-2,6-dione;
3-(6-((4-(4'-fluoro-3,4,5,6-tetrahydro-[1,1'-biphenyl]-2-carbonyl)piperazin-1-yl)methyl)-1-thioxoisoindolin-2-yl)piperidine-2,6-dione;
3-(6-((3-(4'-fluoro-3,4,5,6-tetrahydro-[1,1'-biphenyl]-2-carbonyl)-3,6-diazabicyclo[3.1.1]heptan-6-yl)methyl)-1-thioxoisoindolin-2-yl)piperidine-2,6-dione;
3-(6-((4-((4'-chloro-[1,1'-biphenyl]-2-yl)amino)piperidin-1-yl)methyl)-1-thioxoisoindolin-2-yl)piperidine-2,6-dione;
3-(6-((4-((4'-chloro-[1,1'-biphenyl]-2-yl)methyl)piperazin-1-yl)methyl)-1-thioxoisoindolin-2-yl)piperidine-2,6-dione;
3-(6-((4-((4-(4-chlorophenyl)-6,6-dimethyl-5,6-dihydro-2H-pyran-3-yl)methyl)piperazin-1-yl)methyl)-1-thioxoisoindolin-2-yl)piperidine-2,6-dione;
3-(6-((4-(thiophen-2-yl)piperazin-1-yl)methyl)-1-thioxoisoindolin-2-yl)piperidine-2,6-dione;
3-(6-((4-(5-methylthiophen-2-yl)piperazin-1-yl)methyl)-1-thioxoisoindolin-2-yl)piperidine-2,6-dione;
3-(6-((4-(3-methylthiophen-2-yl)piperazin-1-yl)methyl)-1-thioxoisoindolin-2-yl)piperidine-2,6-dione;
3-(6-((4-(thiophen-3-yl)piperazin-1-yl)methyl)-1-thioxoisoindolin-2-yl)piperidine-2,6-dione;
3-(6-((4-(4-methylthiophen-3-yl)piperazin-1-yl)methyl)-1-thioxoisoindolin-2-yl)piperidine-2,6-dione;
3-(6-((4-(5-methylthiophen-3-yl)piperazin-1-yl)methyl)-1-thioxoisoindolin-2-yl)piperidine-2,6-dione;
3-(6-((4-(thiophen-2-yl)-3,6-dihydropyridin-1(2H)-yl)methyl)-1-thioxoisoindolin-2-yl)piperidine-2,6-dione;
3-(6-((4-(5-methylthiophen-2-yl)-3,6-dihydropyridin-1(2H)-yl)methyl)-1-thioxoisoindolin-2-yl)piperidine-2,6-dione;
3-(6-((4-(3-methylthiophen-2-yl)-3,6-dihydropyridin-1(2H)-yl)methyl)-1-thioxoisoindolin-2-yl)piperidine-2,6-dione;
3-(6-((4-(thiophen-3-yl)-3,6-dihydropyridin-1(2H)-yl)methyl)-1-thioxoisoindolin-2-yl)piperidine-2,6-dione;
3-(6-((4-(4-methylthiophen-3-yl)-3,6-dihydropyridin-1(2H)-yl)methyl)-1-thioxoisoindolin-2-yl)piperidine-2,6-dione;
3-(6-((4-(5-methylthiophen-3-yl)-3,6-dihydropyridin-1(2H)-yl)methyl)-1-thioxoisoindolin-2-yl)piperidine-2,6-dione;
3-(6-((4-(furan-2-yl)-3,6-dihydropyridin-1(2H)-yl)methyl)-1-thioxoisoindolin-2-yl)piperidine-2,6-dione;
3-(6-((4-(furan-3-yl)-3,6-dihydropyridin-1(2H)-yl)methyl)-1-thioxoisoindolin-2-yl)piperidine-2,6-dione;
3-(6-((4-(thiophen-2-yl)piperidin-1-yl)methyl)-1-thioxoisoindolin-2-yl)piperidine-2,6-dione;
3-(6-((4-(5-methylthiophen-2-yl)piperidin-1-yl)methyl)-1-thioxoisoindolin-2-yl)piperidine-2,6-dione;
3-(6-((4-(3-methylthiophen-2-yl)piperidin-1-yl)methyl)-1-thioxoisoindolin-2-yl)piperidine-2,6-dione;

3-(6-((4-(thiophen-3-yl)piperidin-1-yl)methyl)-1-thioxoisoindolin-2-yl)piperidine-2,6-dione;
3-(6-((4-(5-methylthiophen-3-yl)piperidin-1-yl)methyl)-1-thioxoisoindolin-2-yl)piperidine-2,6-dione;
3-(6-((4-(4-methylthiophen-3-yl)piperidin-1-yl)methyl)-1-thioxoisoindolin-2-yl)piperidine-2,6-dione;
3-(6-((4-(furan-2-yl)piperidin-1-yl)methyl)-1-thioxoisoindolin-2-yl)piperidine-2,6-dione;
3-(6-((4-(furan-3-yl)piperidin-1-yl)methyl)-1-thioxoisoindolin-2-yl)piperidine-2,6-dione;
3-(6-((4-(2,3-dichlorophenyl)piperazin-1-yl)methyl)-1-thioxoisoindolin-2-yl)piperidine-2,6-dione;
3-(6-((4-phenylpiperazin-1-yl)methyl)-1-thioxoisoindolin-2-yl)piperidine-2,6-dione;
3-(6-((4-(2-methoxyphenyl)piperazin-1-yl)methyl)-1-thioxoisoindolin-2-yl)piperidine-2,6-dione;
3-(6-((4-(3-methoxyphenyl)piperazin-1-yl)methyl)-1-thioxoisoindolin-2-yl)piperidine-2,6-dione;
3-(6-((4-(4-methoxyphenyl)piperazin-1-yl)methyl)-1-thioxoisoindolin-2-yl)piperidine-2,6-dione;
3-(1-thioxo-6-((4-(4-(trifluoromethyl)phenyl)piperazin-1-yl)methyl)isoindolin-2-yl)piperidine-2,6-dione;
3-(6-((4-(3-fluorophenyl)piperazin-1-yl)methyl)-1-thioxoisoindolin-2-yl)piperidine-2,6-dione;
3-(6-((4-(4-fluorophenyl)piperazin-1-yl)methyl)-1-thioxoisoindolin-2-yl)piperidine-2,6-dione;
3-(6-((4-(2-chlorophenyl)piperazin-1-yl)methyl)-1-thioxoisoindolin-2-yl)piperidine-2,6-dione;
3-(6-((4-(4-chlorophenyl)piperazin-1-yl)methyl)-1-thioxoisoindolin-2-yl)piperidine-2,6-dione;
3-(6-((4-(2-bromophenyl)piperazin-1-yl)methyl)-1-thioxoisoindolin-2-yl)piperidine-2,6-dione;
3-(6-((4-(3-bromophenyl)piperazin-1-yl)methyl)-1-thioxoisoindolin-2-yl)piperidine-2,6-dione;
3-(6-((4-(4-bromophenyl)piperazin-1-yl)methyl)-1-thioxoisoindolin-2-yl)piperidine-2,6-dione;
3-(1-thioxo-6-((4-(o-tolyl)piperazin-1-yl)methyl)isoindolin-2-yl)piperidine-2,6-dione;
3-(1-thioxo-6-((4-(m-tolyl)piperazin-1-yl)methyl)isoindolin-2-yl)piperidine-2,6-dione;
3-(1-thioxo-6-((4-(p-tolyl)piperazin-1-yl)methyl)isoindolin-2-yl)piperidine-2,6-dione;
3-(6-((4-(2,4-dichlorophenyl)piperazin-1-yl)methyl)-1-thioxoisoindolin-2-yl)piperidine-2,6-dione;
3-(6-((4-(2,5-dichlorophenyl)piperazin-1-yl)methyl)-1-thioxoisoindolin-2-yl)piperidine-2,6-dione;
3-(6-((4-(2,6-dichlorophenyl)piperazin-1-yl)methyl)-1-thioxoisoindolin-2-yl)piperidine-2,6-dione;
3-(6-((4-(3,4-dichlorophenyl)piperazin-1-yl)methyl)-1-thioxoisoindolin-2-yl)piperidine-2,6-dione;
3-(6-((4-(3,5-dichlorophenyl)piperazin-1-yl)methyl)-1-thioxoisoindolin-2-yl)piperidine-2,6-dione;
3-(6-((4-(2,3-difluorophenyl)piperazin-1-yl)methyl)-1-thioxoisoindolin-2-yl)piperidine-2,6-dione;
3-(6-((4-(2,4-difluorophenyl)piperazin-1-yl)methyl)-1-thioxoisoindolin-2-yl)piperidine-2,6-dione;
3-(6-((4-(2,5-difluorophenyl)piperazin-1-yl)methyl)-1-thioxoisoindolin-2-yl)piperidine-2,6-dione;
3-(6-((4-(2,6-difluorophenyl)piperazin-1-yl)methyl)-1-thioxoisoindolin-2-yl)piperidine-2,6-dione;
3-(6-((4-(3,4-difluorophenyl)piperazin-1-yl)methyl)-1-thioxoisoindolin-2-yl)piperidine-2,6-dione;
3-(6-((4-(3,5-difluorophenyl)piperazin-1-yl)methyl)-1-thioxoisoindolin-2-yl)piperidine-2,6-dione;
3-(6-((4-(2,4-dimethylphenyl)piperazin-1-yl)methyl)-1-thioxoisoindolin-2-yl)piperidine-2,6-dione;
3-(6-((4-(5-chloro-2-methylphenyl)piperazin-1-yl)methyl)-1-thioxoisoindolin-2-yl)piperidine-2,6-dione;
3-(6-((4-phenylpiperidin-1-yl)methyl)-1-thioxoisoindolin-2-yl)piperidine-2,6-dione;
3-(6-((4-(3-bromophenyl)piperidin-1-yl)methyl)-1-thioxoisoindolin-2-yl)piperidine-2,6-dione;
3-(6-((4-(4-bromophenyl)piperidin-1-yl)methyl)-1-thioxoisoindolin-2-yl)piperidine-2,6-dione;
3-(6-((4-(2-chlorophenyl)piperidin-1-yl)methyl)-1-thioxoisoindolin-2-yl)piperidine-2,6-dione;
3-(6-((4-(4-chlorophenyl)piperidin-1-yl)methyl)-1-thioxoisoindolin-2-yl)piperidine-2,6-dione;
3-(6-((4-(2-fluorophenyl)piperidin-1-yl)methyl)-1-thioxoisoindolin-2-yl)piperidine-2,6-dione;
3-(6-((4-(4-fluorophenyl)piperidin-1-yl)methyl)-1-thioxoisoindolin-2-yl)piperidine-2,6-dione;
3-(1-thioxo-6-((4-(2-(trifluoromethyl)phenyl)piperidin-1-yl)methyl)isoindolin-2-yl)piperidine-2,6-dione;
3-(1-thioxo-6-((4-(3-(trifluoromethyl)phenyl)piperidin-1-yl)methyl)isoindolin-2-yl)piperidine-2,6-dione;
3-(6-((4-(2,3-dichlorophenyl)piperidin-1-yl)methyl)-1-thioxoisoindolin-2-yl)piperidine-2,6-dione;
3-(6-((4-(2-chloro-3-fluorophenyl)piperidin-1-yl)methyl)-1-thioxoisoindolin-2-yl)piperidine-2,6-dione;
3-(6-((4-(2,3-difluorophenyl)piperidin-1-yl)methyl)-1-thioxoisoindolin-2-yl)piperidine-2,6-dione;
3-(6-((4-(2,3-dichlorophenyl)-3,6-dihydropyridin-1(2H)-yl)methyl)-1-thioxoisoindolin-2-yl)piperidine-2,6-dione;
3-(6-((4-(2-chloro-3-fluorophenyl)-3,6-dihydropyridin-1(2H)-yl)methyl)-1-thioxoisoindolin-2-yl)piperidine-2,6-dione;
3-(6-((4-(pyridin-2-yl)piperazin-1-yl)methyl)-1-thioxoisoindolin-2-yl)piperidine-2,6-dione;
3-(6-((4-(6-methylpyridin-2-yl)piperazin-1-yl)methyl)-1-thioxoisoindolin-2-yl)piperidine-2,6-dione;
3-(6-((4-(6-chloropyridin-2-yl)piperazin-1-yl)methyl)-1-thioxoisoindolin-2-yl)piperidine-2,6-dione;
3-(6-((4-(5-chloropyridin-2-yl)piperazin-1-yl)methyl)-1-thioxoisoindolin-2-yl)piperidine-2,6-dione;
3-(6-((4-(4-chloropyridin-2-yl)piperazin-1-yl)methyl)-1-thioxoisoindolin-2-yl)piperidine-2,6-dione;
1-(6-((4-(3-chloropyridin-2-yl)piperazin-1-yl)methyl)-1-oxoisoindolin-2-yl)dihydropyrimidine-2,4(1H,3H)-dione;
1-(6-((4-(3-fluoropyridin-2-yl)piperazin-1-yl)methyl)-1-oxoisoindolin-2-yl)dihydropyrimidine-2,4(1H,3H)-dione;
3-(6-((4-(6-fluoropyridin-2-yl)piperazin-1-yl)methyl)-1-thioxoisoindolin-2-yl)piperidine-2,6-dione;
3-(6-((4-(5-fluoropyridin-2-yl)piperazin-1-yl)methyl)-1-thioxoisoindolin-2-yl)piperidine-2,6-dione;

3-(1-thioxo-6-((4-(3-(trifluoromethyl)pyridin-2-yl)piperazin-1-yl)methyl)isoindolin-2-yl)piperidine-2,6-dione;
3-(1-thioxo-6-((4-(6-(trifluoromethyl)pyridin-2-yl)piperazin-1-yl)methyl)isoindolin-2-yl)piperidine-2,6-dione;
3-(1-thioxo-6-((4-(5-(trifluoromethyl)pyridin-2-yl)piperazin-1-yl)methyl)isoindolin-2-yl)piperidine-2,6-dione;
3-(6-((4-(3,4-dichloropyridin-2-yl)piperazin-1-yl)methyl)-1-thioxoisoindolin-2-yl)piperidine-2,6-dione;
3-(6-((4-(3,5-dichloropyridin-2-yl)piperazin-1-yl)methyl)-1-thioxoisoindolin-2-yl)piperidine-2,6-dione;
3-(6-((4-(4,5-dichloropyridin-2-yl)piperazin-1-yl)methyl)-1-thioxoisoindolin-2-yl)piperidine-2,6-dione;
3-(6-((4-(3,4-difluoropyridin-2-yl)piperazin-1-yl)methyl)-1-thioxoisoindolin-2-yl)piperidine-2,6-dione;
3-(6-((4-(4-chloro-3-fluoropyridin-2-yl)piperazin-1-yl)methyl)-1-thioxoisoindolin-2-yl)piperidine-2,6-dione;
3-(6-((4-(3-fluoro-4-iodopyridin-2-yl)piperazin-1-yl)methyl)-1-thioxoisoindolin-2-yl)piperidine-2,6-dione;
3-(6-((4-(3-bromo-4-chloropyridin-2-yl)piperazin-1-yl)methyl)-1-thioxoisoindolin-2-yl)piperidine-2,6-dione;
3-(6-((4-(3-chloro-5-(trifluoromethyl)pyridin-2-yl)piperazin-1-yl)methyl)-1-thioxoisoindolin-2-yl)piperidine-2,6-dione;
3-(6-((4-(4-chloro-6-methylpyridin-2-yl)piperazin-1-yl)methyl)-1-thioxoisoindolin-2-yl)piperidine-2,6-dione;
3-(6-((4-(pyridin-3-yl)piperazin-1-yl)methyl)-1-thioxoisoindolin-2-yl)piperidine-2,6-dione;
3-(6-((4-(6-chloropyridin-3-yl)piperazin-1-yl)methyl)-1-thioxoisoindolin-2-yl)piperidine-2,6-dione;
3-(6-((4-(4,5-dichloropyridin-3-yl)piperazin-1-yl)methyl)-1-thioxoisoindolin-2-yl)piperidine-2,6-dione;
3-(6-((4-(2,4-dichloropyridin-3-yl)piperazin-1-yl)methyl)-1-thioxoisoindolin-2-yl)piperidine-2,6-dione;
3-(6-((4-(pyridin-4-yl)piperazin-1-yl)methyl)-1-thioxoisoindolin-2-yl)piperidine-2,6-dione;
3-(6-((4-(2-methylpyridin-4-yl)piperazin-1-yl)methyl)-1-thioxoisoindolin-2-yl)piperidine-2,6-dione;
3-(6-((4-(3-bromopyridin-4-yl)piperazin-1-yl)methyl)-1-thioxoisoindolin-2-yl)piperidine-2,6-dione;
3-(6-((4-(3-fluoropyridin-4-yl)piperazin-1-yl)methyl)-1-thioxoisoindolin-2-yl)piperidine-2,6-dione;
3-(6-((4-(3-chloropyridin-4-yl)piperazin-1-yl)methyl)-1-thioxoisoindolin-2-yl)piperidine-2,6-dione;
3-(6-((4-(2-chloropyridin-4-yl)piperazin-1-yl)methyl)-1-thioxoisoindolin-2-yl)piperidine-2,6-dione;
3-(6-((4-(2-chloro-3-fluoropyridin-4-yl)piperazin-1-yl)methyl)-1-thioxoisoindolin-2-yl)piperidine-2,6-dione;
3-(6-((4-(2-bromo-3-fluoropyridin-4-yl)piperazin-1-yl)methyl)-1-thioxoisoindolin-2-yl)piperidine-2,6-dione;
3-(6-((4-(3-bromo-2-chloropyridin-4-yl)piperazin-1-yl)methyl)-1-thioxoisoindolin-2-yl)piperidine-2,6-dione;
3-(6-((4-(2,3-dichloropyridin-4-yl)piperazin-1-yl)methyl)-1-thioxoisoindolin-2-yl)piperidine-2,6-dione;
3-(6-((4-(2,3-difluoropyridin-4-yl)piperazin-1-yl)methyl)-1-thioxoisoindolin-2-yl)piperidine-2,6-dione;
3-(6-((4-(3-fluoro-2-hydroxypyridin-4-yl)piperazin-1-yl)methyl)-1-thioxoisoindolin-2-yl)piperidine-2,6-dione;
3-(6-((4-(3-bromo-5-chloropyridin-4-yl)piperazin-1-yl)methyl)-1-thioxoisoindolin-2-yl)piperidine-2,6-dione;
3-(6-((4-(3-chloro-5-fluoropyridin-4-yl)piperazin-1-yl)methyl)-1-thioxoisoindolin-2-yl)piperidine-2,6-dione;
3-(6-((4-(3,5-dichloropyridin-4-yl)piperazin-1-yl)methyl)-1-thioxoisoindolin-2-yl)piperidine-2,6-dione;
3-(6-((4-(2,6-dimethylpyridin-4-yl)piperazin-1-yl)methyl)-1-thioxoisoindolin-2-yl)piperidine-2,6-dione;
3-(6-((4-(perfluoropyridin-4-yl)piperazin-1-yl)methyl)-1-thioxoisoindolin-2-yl)piperidine-2,6-dione;
3-(6-((4-(pyridazin-3-yl)piperazin-1-yl)methyl)-1-thioxoisoindolin-2-yl)piperidine-2,6-dione;
3-(6-((4-(6-chloropyridazin-3-yl)piperazin-1-yl)methyl)-1-thioxoisoindolin-2-yl)piperidine-2,6-dione;
3-(6-((4-(pyrimidin-2-yl)piperazin-1-yl)methyl)-1-thioxoisoindolin-2-yl)piperidine-2,6-dione;
3-(6-((4-(pyrazin-2-yl)piperazin-1-yl)methyl)-1-thioxoisoindolin-2-yl)piperidine-2,6-dione;
3-(6-((4-(5,6-dichloropyrimidin-4-yl)piperazin-1-yl)methyl)-1-thioxoisoindolin-2-yl)piperidine-2,6-dione;
3-(6-((4-(3,6-dichloropyridazin-4-yl)piperazin-1-yl)methyl)-1-thioxoisoindolin-2-yl)piperidine-2,6-dione;
3-(6-((4-(5,6-dichloropyridazin-4-yl)piperazin-1-yl)methyl)-1-thioxoisoindolin-2-yl)piperidine-2,6-dione;
3-(6-((4-(1,3,5-triazin-2-yl)piperazin-1-yl)methyl)-1-thioxoisoindolin-2-yl)piperidine-2,6-dione;
3-(6-((4-(3,4-dichloropyridin-2-yl)piperidin-1-yl)methyl)-1-thioxoisoindolin-2-yl)piperidine-2,6-dione;
3-(6-((4-(2,3-dichloropyridin-4-yl)piperidin-1-yl)methyl)-1-thioxoisoindolin-2-yl)piperidine-2,6-dione;
3-(6-((4-(2,3-difluoropyridin-4-yl)piperidin-1-yl)methyl)-1-thioxoisoindolin-2-yl)piperidine-2,6-dione;
3-(6-([3,4'-bipiperidin]-1-ylmethyl)-1-thioxoisoindolin-2-yl)piperidine-2,6-dione;
3-(6-((3,4-dichloro-3',6'-dihydro-[2,4'-bipyridin]-1'(2'H)-yl)methyl)-1-thioxoisoindolin-2-yl)piperidine-2,6-dione;
3-(6-((4,5-dichloro-3',6'-dihydro-[3,4'-bipyridin]-1'(2'H)-yl)methyl)-1-thioxoisoindolin-2-yl)piperidine-2,6-dione;
3-(6-((5-chloro-3',6'-dihydro-[3,4'-bipyridin]-1'(2'H)-yl)methyl)-1-thioxoisoindolin-2-yl)piperidine-2,6-dione;
3-(6-((2',3'-dichloro-3,6-dihydro-[4,4'-bipyridin]-1(2H)-yl)methyl)-1-thioxoisoindolin-2-yl)piperidine-2,6-dione;
3-(6-((3-fluoro-3',6'-dihydro-[2,4'-bipyridin]-1'(2'H)-yl)methyl)-1-thioxoisoindolin-2-yl)piperidine-2,6-dione;
3-(6-((2',3'-difluoro-3,6-dihydro-[4,4'-bipyridin]-1(2H)-yl)methyl)-1-thioxoisoindolin-2-yl)piperidine-2,6-dione;
3-(6-((3-(2,3-dichlorophenyl)-3,6-diazabicyclo[3.1.1]heptan-6-yl)methyl)-1-thioxoisoindolin-2-yl)piperidine-2,6-dione;
3-(6-((6-(2,3-dichlorophenyl)-3,6-diazabicyclo[3.1.1]heptan-3-yl)methyl)-1-thioxoisoindolin-2-yl)piperidine-2,6-dione;
3-(6-(((1R,4R)-5-(2,3-dichlorophenyl)-2,5-diazabicyclo[2.2.1]heptan-2-yl)methyl)-1-thioxoisoindolin-2-yl)piperidine-2,6-dione;

3-(6-(((1S,4S)-5-(2,3-dichlorophenyl)-2,5-diazabicyclo[2.2.1]heptan-2-yl)methyl)-1-thioxoisoindolin-2-yl)piperidine-2,6-dione;
3-(6-((5-(2,3-dichlorophenyl)-2,5-diazabicyclo[2.2.2]octan-2-yl)methyl)-1-thioxoisoindolin-2-yl)piperidine-2,6-dione;
3-(6-((8-(2,3-dichlorophenyl)-3,8-diazabicyclo[3.2.1]octan-3-yl)methyl)-1-thioxoisoindolin-2-yl)piperidine-2,6-dione;
3-(6-((3-(2,3-dichlorophenyl)-3,8-diazabicyclo[3.2.1]octan-8-yl)methyl)-1-thioxoisoindolin-2-yl)piperidine-2,6-dione;
3-(6-((4-(2,3-dichlorophenyl)-1,4-diazepan-1-yl)methyl)-1-thioxoisoindolin-2-yl)piperidine-2,6-dione;
3-(6-((4-(6-fluorobenzo[d]isoxazol-3-yl)piperidin-1-yl)methyl)-1-thioxoisoindolin-2-yl)piperidine-2,6-dione;
3-(6-((4-(benzo[d]isoxazol-3-yl)piperidin-1-yl)methyl)-1-thioxoisoindolin-2-yl)piperidine-2,6-dione;
3-(6-((4-(isoxazol-3-yl)piperidin-1-yl)methyl)-1-thioxoisoindolin-2-yl)piperidine-2,6-dione;
3-(6-((4-(6-fluorobenzo[d]isoxazol-3-yl)piperazin-1-yl)methyl)-1-thioxoisoindolin-2-yl)piperidine-2,6-dione;
3-(6-((4-(isoxazolo[4,5-c]pyridin-3-yl)piperazin-1-yl)methyl)-1-thioxoisoindolin-2-yl)piperidine-2,6-dione;
3-(6-((4-(6-fluorobenzo[d]isothiazol-3-yl)piperazin-1-yl)methyl)-1-thioxoisoindolin-2-yl)piperidine-2,6-dione;
3-(6-((4-(6-fluoro-1H-indazol-3-yl)piperazin-1-yl)methyl)-1-thioxoisoindolin-2-yl)piperidine-2,6-dione;
3-(6-((4-(1H-pyrrolo[2,3-b]pyridin-3-yl)piperazin-1-yl)methyl)-1-thioxoisoindolin-2-yl)piperidine-2,6-dione;
3-(6-((4-(6-fluoro-1H-indol-3-yl)piperidin-1-yl)methyl)-1-thioxoisoindolin-2-yl)piperidine-2,6-dione;
3-(6-((4-(6-fluoro-1-methyl-1H-indol-3-yl)piperidin-1-yl)methyl)-1-thioxoisoindolin-2-yl)piperidine-2,6-dione;
3-(6-((4-(5-fluoroindolin-1-yl)piperidin-1-yl)methyl)-1-thioxoisoindolin-2-yl)piperidine-2,6-dione;
3-(6-((4-(5-fluoro-1H-benzo[d]imidazol-1-yl)piperidin-1-yl)methyl)-1-thioxoisoindolin-2-yl)piperidine-2,6-dione;
3-(6-((4-(5-fluoro-2-oxo-2,3-dihydro-1H-benzo[d]imidazol-1-yl)piperidin-1-yl)methyl)-1-thioxoisoindolin-2-yl)piperidine-2,6-dione;
3-(6-((4-(1H-pyrrolo[2,3-b]pyridin-3-yl)piperidin-1-yl)methyl)-1-thioxoisoindolin-2-yl)piperidine-2,6-dione;
3-(6-((4-(1H-pyrrolo[2,3-b]pyridin-3-yl)-3,6-dihydropyridin-1(2H)-yl)methyl)-1-thioxoisoindolin-2-yl)piperidine-2,6-dione;
3-(6-((4-(6-fluoro-1H-indol-3-yl)-3,6-dihydropyridin-1(2H)-yl)methyl)-1-thioxoisoindolin-2-yl)piperidine-2,6-dione;
3-(6-((4-(6-fluoro-1-methyl-1H-indol-3-yl)-3,6-dihydropyridin-1(2H)-yl)methyl)-1-thioxoisoindolin-2-yl)piperidine-2,6-dione;
3-(6-((4-(6-fluoro-3,4-dihydroquinolin-1(2H)-yl)piperidin-1-yl)methyl)-1-thioxoisoindolin-2-yl)piperidine-2,6-dione;
3-(6-((4-(7-fluoro-2,3-dihydro-4H-benzo[b][1,4]oxazin-4-yl)piperidin-1-yl)methyl)-1-thioxoisoindolin-2-yl)piperidine-2,6-dione;
3-(6-((4-(7-fluoro-3-oxo-2,3-dihydro-4H-benzo[b][1,4]oxazin-4-yl)piperidin-1-yl)methyl)-1-thioxoisoindolin-2-yl) piperidine-2,6-dione;
3-(6-((4-(7-fluorochroman-4-yl)piperidin-1-yl)methyl)-1-thioxoisoindolin-2-yl)piperidine-2,6-dione;
3-(6-((4-(7-fluorochroman-4-ylidene)piperidin-1-yl)methyl)-1-thioxoisoindolin-2-yl)piperidine-2,6-dione;
3-(6-((4-(5-fluoronaphthalen-1-yl)piperidin-1-yl)methyl)-1-thioxoisoindolin-2-yl)piperidine-2,6-dione;
3-(6-((4-(7-fluoroquinolin-4-yl)piperidin-1-yl)methyl)-1-thioxoisoindolin-2-yl)piperidine-2,6-dione;
3-(6-((4-(isoquinolin-5-yl)piperidin-1-yl)methyl)-1-thioxoisoindolin-2-yl)piperidine-2,6-dione;
3-(6-((4-(5-fluoronaphthalen-1-yl)-3,6-dihydropyridin-1(2H)-yl)methyl)-1-thioxoisoindolin-2-yl)piperidine-2,6-dione;
3-(6-((4-(isoquinolin-5-yl)-3,6-dihydropyridin-1(2H)-yl)methyl)-1-thioxoisoindolin-2-yl)piperidine-2,6-dione;
3-(6-((3-(7-fluorochroman-4-yl)azetidin-1-yl)methyl)-1-thioxoisoindolin-2-yl)piperidine-2,6-dione;
3-(6-((3-(7-fluorochroman-4-ylidene)azetidin-1-yl)methyl)-1-thioxoisoindolin-2-yl)piperidine-2,6-dione;
3-(6-((3-(7-fluoroquinolin-4-yl)azetidin-1-yl)methyl)-1-thioxoisoindolin-2-yl)piperidine-2,6-dione;
3-(6-((3-(5-fluoroindolin-1-yl)azetidin-1-yl)methyl)-1-thioxoisoindolin-2-yl)piperidine-2,6-dione;
3-(6-((3-(5-fluoro-1H-benzo[d]imidazol-1-yl)azetidin-1-yl)methyl)-1-thioxoisoindolin-2-yl)piperidine-2,6-dione;
3-(6-((4-(thieno[2,3-d]pyrimidin-4-yl)piperazin-1-yl)methyl)-1-thioxoisoindolin-2-yl)piperidine-2,6-dione;
3-(5-((4-(5-methylthieno[2,3-d]pyrimidin-4-yl)piperazin-1-yl)methyl)-1-thioxoisoindolin-2-yl)piperidine-2,6-dione;
3-(6-((4-(6-methylthieno[2,3-d]pyrimidin-4-yl)piperazin-1-yl)methyl)-1-thioxoisoindolin-2-yl)piperidine-2,6-dione;
3-(6-((4-(6-isopropylthieno[2,3-d]pyrimidin-4-yl)piperazin-1-yl)methyl)-1-thioxoisoindolin-2-yl)piperidine-2,6-dione;
3-(6-((4-(5,6-dimethylthieno[2,3-d]pyrimidin-4-yl)piperazin-1-yl)methyl)-1-thioxoisoindolin-2-yl)piperidine-2,6-dione;

3-(6-((4-(6-phenylthieno[2,3-d]pyrimidin-4-yl)piperazin-1-yl)methyl)-1-thioxoisoindolin-2-yl)piperidine-2,6-dione;
3-(6-((4-(6-bromothieno[2,3-d]pyrimidin-4-yl)piperazin-1-yl)methyl)-1-thioxoisoindolin-2-yl)piperidine-2,6-dione;
3-(6-((4-(5-phenylthieno[2,3-d]pyrimidin-4-yl)piperazin-1-yl)methyl)-1-thioxoisoindolin-2-yl)piperidine-2,6-dione;
3-(6-((4-(5,6,7,8-tetrahydrobenzo[4,5]thieno[2,3-d]pyrimidin-4-yl)piperazin-1-yl)methyl)-1-thioxoisoindolin-2-yl)piperidine-2,6-dione;
3-(6-((4-(6,7-dihydro-5H-cyclopenta[4,5]thieno[2,3-d]pyrimidin-4-yl)piperazin-1-yl)methyl)-1-thioxoisoindolin-2-yl)piperidine-2,6-dione;
3-(6-((4-(2-methyl-5-phenylthieno[2,3-d]pyrimidin-4-yl)piperazin-1-yl)methyl)-1-thioxoisoindolin-2-yl)piperidine-2,6-dione;
3-(6-((4-(2-methyl-5,6,7,8-tetrahydrobenzo[4,5]thieno[2,3-d]pyrimidin-4-yl)piperazin-1-yl)methyl)-1-thioxoisoindolin-2-yl)piperidine-2,6-dione;
3-(6-((4-(2-methyl-6,7-dihydro-5H-cyclopenta[4,5]thieno[2,3-d]pyrimidin-4-yl)piperazin-1-yl)methyl)-1-thioxoisoindolin-2-yl)piperidine-2,6-dione;
3-(6-((4-(2-methylthieno[2,3-d]pyrimidin-4-yl)piperazin-1-yl)methyl)-1-thioxoisoindolin-2-yl)piperidine-2,6-dione;
3-(6-((4-(thieno[2,3-d]pyrimidin-2-yl)piperazin-1-yl)methyl)-1-thioxoisoindolin-2-yl)piperidine-2,6-dione;
3-(6-((4-(5,6-dimethylthieno[2,3-d]pyrimidin-4-yl)-1,4-diazepan-1-yl)methyl)-1-thioxoisoindolin-2-yl)piperidine-2,6-dione;
3-(6-((4-(thieno[2,3-d]pyrimidin-4-yl)piperidin-1-yl)methyl)-1-thioxoisoindolin-2-yl)piperidine-2,6-dione;
3-(6-((4-(6-methylthieno[2,3-d]pyrimidin-4-yl)piperidin-1-yl)methyl)-1-thioxoisoindolin-2-yl)piperidine-2,6-dione;
3-(6-((4-(5,6-dimethylthieno[2,3-d]pyrimidin-4-yl)piperidin-1-yl)methyl)-1-thioxoisoindolin-2-yl)piperidine-2,6-dione;
3-(6-((4-(6-phenylthieno[2,3-d]pyrimidin-4-yl)piperidin-1-yl)methyl)-1-thioxoisoindolin-2-yl)piperidine-2,6-dione;
3-(6-((4-(5-phenylthieno[2,3-d]pyrimidin-4-yl)piperidin-1-yl)methyl)-1-thioxoisoindolin-2-yl)piperidine-2,6-dione;
3-(6-((4-(5,6,7,8-tetrahydrobenzo[4,5]thieno[2,3-d]pyrimidin-4-yl)piperidin-1-yl)methyl)-1-thioxoisoindolin-2-yl)piperidine-2,6-dione;
3-(6-((4-(6,7-dihydro-5H-cyclopenta[4,5]thieno[2,3-d]pyrimidin-4-yl)piperidin-1-yl)methyl)-1-thioxoisoindolin-2-yl)piperidine-2,6-dione;
3-(6-((4-(2-methyl-5-phenylthieno[2,3-d]pyrimidin-4-yl)piperidin-1-yl)methyl)-1-thioxoisoindolin-2-yl)piperidine-2,6-dione;
3-(6-((4-(2-methyl-5,6,7,8-tetrahydrobenzo[4,5]thieno[2,3-d]pyrimidin-4-yl)piperidin-1-yl)methyl)-1-thioxoisoindolin-2-yl)piperidine-2,6-dione;
3-(6-((4-(2-methyl-6,7-dihydro-5H-cyclopenta[4,5]thieno[2,3-d]pyrimidin-4-yl)piperidin-1-yl)methyl)-1-thioxoisoindolin-2-yl)piperidine-2,6-dione;
3-(6-((4-(2-methylthieno[2,3-d]pyrimidin-4-yl)piperidin-1-yl)methyl)-1-thioxoisoindolin-2-yl)piperidine-2,6-dione;
3-(6-((4-(thieno[2,3-d]pyrimidin-2-yl)piperidin-1-yl)methyl)-1-thioxoisoindolin-2-yl)piperidine-2,6-dione;
3-(6-((4-(5-ethylpyrimidin-2-yl)piperidin-1-yl)methyl)-1-thioxoisoindolin-2-yl)piperidine-2,6-dione;
3-(6-((4-(thieno[2,3-d]pyrimidin-4-yl)-3,6-dihydropyridin-1(2H)-yl)methyl)-1-thioxoisoindolin-2-yl)piperidine-2,6-dione;
3-(6-((4-(5-methylthieno[2,3-d]pyrimidin-4-yl)-3,6-dihydropyridin-1(2H)-yl)methyl)-1-thioxoisoindolin-2-yl)piperidine-2,6-dione;
3-(6-((4-(6-methylthieno[2,3-d]pyrimidin-4-yl)-3,6-dihydropyridin-1(2H)-yl)methyl)-1-thioxoisoindolin-2-yl)piperidine-2,6-dione;
3-(6-((4-(6-isopropylthieno[2,3-d]pyrimidin-4-yl)-3,6-dihydropyridin-1(2H)-yl)methyl)-1-thioxoisoindolin-2-yl)piperidine-2,6-dione;
3-(6-((4-(5,6-dimethylthieno[2,3-d]pyrimidin-4-yl)-3,6-dihydropyridin-1(2H)-yl)methyl)-1-thioxoisoindolin-2-yl)piperidine-2,6-dione;
3-(6-((4-(6-phenylthieno[2,3-d]pyrimidin-4-yl)-3,6-dihydropyridin-1(2H)-yl)methyl)-1-thioxoisoindolin-2-yl)piperidine-2,6-dione;
3-(6-((4-(5-phenylthieno[2,3-d]pyrimidin-4-yl)-3,6-dihydropyridin-1(2H)-yl)methyl)-1-thioxoisoindolin-2-yl)piperidine-2,6-dione;

3-(6-((4-(5,6,7,8-tetrahydrobenzo[4,5]thieno[2,3-d]pyrimidin-4-yl)-3,6-dihydropyridin-1(2H)-yl)methyl)-1-thioxoisoindolin-2-yl)piperidine-2,6-dione;
3-(6-((4-(6,7-dihydro-5H-cyclopenta[4,5]thieno[2,3-d]pyrimidin-4-yl)-3,6-dihydropyridin-1(2H)-yl)methyl)-1-thioxoisoindolin-2-yl)piperidine-2,6-dione;
3-(6-((4-(2-methyl-5-phenylthieno [2,3-d]pyrimidin-4-yl)-3,6-dihydropyridin-1 (2H)-yl)methyl)-1-thioxoisoindolin-2-yl)piperidine-2,6-dione;
3-(6-((4-(2-methyl-5,6,7,8-tetrahydrobenzo[4,5]thieno[2,3-d]pyrimidin-4-yl)-3,6-dihydropyridin-1(2H)-yl)methyl)-1-thioxoisoindolin-2-yl)piperidine-2,6-dione;
3-(6-((4-(2-methyl-6,7-dihydro-5H-cyclopenta[4,5]thieno[2,3-d]pyrimidin-4-yl)-3,6-dihydropyridin-1(2H)-yl)methyl)-1-thioxoisoindolin-2-yl)piperidine-2,6-dione;
3-(6-((4-(2-methylthieno[2,3-d]pyrimidin-4-yl)-3,6-dihydropyridin-1(2H)-yl)methyl)-1-thioxoisoindolin-2-yl)piperidine-2,6-dione;
3-(6-((4-(thieno[2,3-d]pyrimidin-2-yl)-3,6-dihydropyridin-1(2H)-yl)methyl)-1-thioxoisoindolin-2-yl)piperidine-2,6-dione;
3-(6-((4-(2-chloro-6,7-dihydrothieno[3,2-d]pyrimidin-4-yl)piperazin-1-yl)methyl)-1-thioxoisoindolin-2-yl)piperidine-2,6-dione;
3-(6-((4-(2-morpholino-6,7-dihydrothieno[3,2-d]pyrimidin-4-yl)piperazin-1-yl)methyl)-1-thioxoisoindolin-2-yl)piperidine-2,6-dione;
3-(6-((4-(4-(piperazin-1-yl)-6,7-dihydrothieno[3,2-d]pyrimidin-2-yl)piperazin-1-yl)methyl)-1-thioxoisoindolin-2-yl)piperidine-2,6-dione;
3-(6-((4-(4-morpholino-6,7-dihydrothieno[3,2-d]pyrimidin-2-yl)piperazin-1-yl)methyl)-1-thioxoisoindolin-2-yl)piperidine-2,6-dione;
3-(6-((4-(2-(piperidin-1-yl)-6,7-dihydrothieno[3,2-d]pyrimidin-4-yl)piperazin-1-yl)methyl)-1-thioxoisoindolin-2-yl)piperidine-2,6-dione;
3-(6-((4-(2-(pyrrolidin-1-yl)-6,7-dihydrothieno[3,2-d]pyrimidin-4-yl)piperazin-1-yl)methyl)-1-thioxoisoindolin-2-yl)piperidine-2,6-dione;
3-(6-((4-(2-(azetidin-1-yl)-6,7-dihydrothieno[3,2-d]pyrimidin-4-yl)piperazin-1-yl)methyl)-1-thioxoisoindolin-2-yl)piperidine-2,6-dione;
3-(6-((4-(thieno[3,2-d]pyrimidin-4-yl)piperazin-1-yl)methyl)-1-thioxoisoindolin-2-yl)piperidine-2,6-dione;
3-(6-((4-(2-chlorothieno[3,2-d]pyrimidin-4-yl)piperazin-1-yl)methyl)-1-thioxoisoindolin-2-yl)piperidine-2,6-dione;
3-(6-((4-(2-morpholinothieno[3,2-d]pyrimidin-4-yl)piperazin-1-yl)methyl)-1-thioxoisoindolin-2-yl)piperidine-2,6-dione;
3-(6-((4-(4-(piperazin-1-yl)thieno[3,2-d]pyrimidin-2-yl)piperazin-1-yl)methyl)-1-thioxoisoindolin-2-yl)piperidine-2,6-dione;
3-(6-((4-(4-morpholinothieno[3,2-d]pyrimidin-2-yl)piperazin-1-yl)methyl)-1-thioxoisoindolin-2-yl)piperidine-2,6-dione;
3-(6-((4-(benzo[b]thiophen-4-yl)piperazin-1-yl)methyl)-1-thioxoisoindolin-2-yl)piperidine-2,6-dione;
3-(1-thioxo-6-((3-(trifluoromethyl)-5,6-dihydro-[1,2,4]triazolo[4,3-a]pyrazin-7(8H)-yl)methyl)isoindolin-2-yl)piperidine-2,6-dione;
3-(6-((4-(2-((2,4-dimethylphenyl)thio)phenyl)piperazin-1-yl)methyl)-1-thioxoisoindolin-2-yl)piperidine-2,6-dione;
3-(6-((6,7-dihydrothieno[3,2-c]pyridin-5(4H)-yl)methyl)-1-thioxoisoindolin-2-yl)piperidine-2,6-dione;
3-(6-((4-(benzo[d]isothiazol-3-yl)piperazin-1-yl)methyl)-1-thioxoisoindolin-2-yl)piperidine-2,6-dione;
3-(6-((4-(4-(adamantan-1-yl)benzyl)piperazin-1-yl)methyl)-1-thioxoisoindolin-2-yl)piperidine-2,6-dione;
3-(6-((adamantan-1-yl)amino)methyl)-1-thioxoisoindolin-2-yl)piperidine-2,6-dione;
1-((1R,4R)-7,7-dimethyl-2-oxobicyclo[2.2.1]heptan-1-yl)-N-((2-(2,6-dioxopiperidin-3-yl)-3-thioxoisoindolin-5-yl)methyl)methanesulfonamide;
3-(6-((((1R,4S)-bicyclo[2.2.1]heptan-2-yl)oxy)methyl)-1-thioxoisoindolin-2-yl)piperidine-2,6-dione;
3-(4-((4-benzhydrylpiperazin-1-yl)methyl)-1-thioxoisoindolin-2-yl)piperidine-2,6-dione;
3-(4-((4-(phenyl(pyridin-2-yl)methyl)piperazin-1-yl)methyl)-1-thioxoisoindolin-2-yl)piperidine-2,6-dione;
3-(4-((4-(phenyl(pyridin-3-yl)methyl)piperazin-1-yl)methyl)-1-thioxoisoindolin-2-yl)piperidine-2,6-dione;
3-(4-((4-(phenyl(pyridin-4-yl)methyl)piperazin-1-yl)methyl)-1-thioxoisoindolin-2-yl)piperidine-2,6-dione;
3-(4-((4-(di(pyridin-2-yl)methyl)piperazin-1-yl)methyl)-1-thioxoisoindolin-2-yl)piperidine-2,6-dione;
3-(4-((4-(di(pyridin-3-yl)methyl)piperazin-1-yl)methyl)-1-thioxoisoindolin-2-yl)piperidine-2,6-dione;
3-(4-((4-(bis(3-fluorophenyl)methyl)piperazin-1-yl)methyl)-1-thioxoisoindolin-2-yl)piperidine-2,6-dione;
3-(4-((4-(bis(4-fluorophenyl)methyl)piperazin-1-yl)methyl)-1-thioxoisoindolin-2-yl)piperidine-2,6-dione;
3-(4-((4-(bis(4-chlorophenyl)methyl)piperazin-1-yl)methyl)-1-thioxoisoindolin-2-yl)piperidine-2,6-dione;

3-(4-((3-benzhydryl-3,6-diazabicyclo[3.1.1]heptan-6-yl)methyl)-1-thioxoisoindolin-2-yl)piperidine-2,6-dione;
3-(4-((5-benzhydryl-2,5-diazabicyclo[2.2.1]heptan-2-yl)methyl)-1-thioxoisoindolin-2-yl)piperidine-2,6-dione;
3-(4-(((1R,4R)-5-benzhydryl-2,5-diazabicyclo[2.2.1]heptan-2-yl)methyl)-1-thioxoisoindolin-2-yl)piperidine-2,6-dione;
3-(4-(((1S,4S)-5-benzhydryl-2,5-diazabicyclo[2.2.1]heptan-2-yl)methyl)-1-thioxoisoindolin-2-yl)piperidine-2,6-dione;
3-(4-((8-benzhydryl-3,8-diazabicyclo[3.2.1]octan-3-yl)methyl)-1-thioxoisoindolin-2-yl)piperidine-2,6-dione;
3-(4-((3-benzhydryl-3,8-diazabicyclo[3.2.1]octan-8-yl)methyl)-1-thioxoisoindolin-2-yl)piperidine-2,6-dione;
3-(4-((5-benzhydryl-2,5-diazabicyclo[2.2.2]octan-2-yl)methyl)-1-thioxoisoindolin-2-yl)piperidine-2,6-dione;
3-(4-((4-benzhydryl-1,4-diazepan-1-yl)methyl)-1-thioxoisoindolin-2-yl)piperidine-2,6-dione;
3-(4-((4-benzhydryl-3,5-dimethylpiperazin-1-yl)methyl)-1-thioxoisoindolin-2-yl)piperidine-2,6-dione;
3-(4-((4-benzhydryl-2,6-dimethylpiperazin-1-yl)methyl)-1-thioxoisoindolin-2-yl)piperidine-2,6-dione;
3-(4-((4-benzhydryl-3,3-dimethylpiperazin-1-yl)methyl)-1-thioxoisoindolin-2-yl)piperidine-2,6-dione;
3-(4-((4-benzhydryl-2-(trifluoromethyl)piperazin-1-yl)methyl)-1-thioxoisoindolin-2-yl)piperidine-2,6-dione;
3-(4-(((R)-4-benzhydryl-2-methylpiperazin-1-yl)methyl)-1-thioxoisoindolin-2-yl)piperidine-2,6-dione;
3-(4-((3-benzhydryl-2-oxoimidazolidin-1-yl)methyl)-1-thioxoisoindolin-2-yl)piperidine-2,6-dione;
3-(4-((4-(diphenylamino)piperidin-1-yl)methyl)-1-thioxoisoindolin-2-yl)piperidine-2,6-dione;
3-(4-((4-(phenyl(pyridin-2-yl)amino)piperidin-1-yl)methyl)-1-thioxoisoindolin-2-yl)piperidine-2,6-dione;
3-(4-((4-((4'-chloro-5,5-dimethyl-3,4,5,6-tetrahydro-[1,1'-biphenyl]-2-yl)methyl)piperazin-1-yl)methyl)-1-thioxoisoindolin-2-yl)piperidine-2,6-dione;
3-(4-((3-((4'-chloro-5,5-dimethyl-3,4,5,6-tetrahydro-[1,1'-biphenyl]-2-yl)methyl)-3,6-diazabicyclo[3.1.1]heptan-6-yl)methyl)-1-thioxoisoindolin-2-yl)piperidine-2,6-dione;
3-(4-((6-((4'-chloro-5,5-dimethyl-3,4,5,6-tetrahydro-[1,1'-biphenyl]-2-yl)methyl)-3,6-diazabicyclo[3.1.1]heptan-3-yl)methyl)-1-thioxoisoindolin-2-yl)piperidine-2,6-dione;
3-(4-((5-((4'-chloro-5,5-dimethyl-3,4,5,6-tetrahydro-[1,1'-biphenyl]-2-yl)methyl)-2,5-diazabicyclo[2.2.1]heptan-2-yl)methyl)-1-thioxoisoindolin-2-yl)piperidine-2,6-dione;
3-(4-((8-((4'-chloro-5,5-dimethyl-3,4,5,6-tetrahydro-[1,1'-biphenyl]-2-yl)methyl)-3,8-diazabicyclo[3.2.1]octan-3-yl)methyl)-1-thioxoisoindolin-2-yl)piperidine-2,6-dione;
3-(4-((3-((4'-chloro-5,5-dimethyl-3,4,5,6-tetrahydro-[1,1'-biphenyl]-2-yl)methyl)-3,8-diazabicyclo[3.2.1]octan-8-yl)methyl)-1-thioxoisoindolin-2-yl)piperidine-2,6-dione;
3-(4-((5-((4'-chloro-5,5-dimethyl-3,4,5,6-tetrahydro-[1,1'-biphenyl]-2-yl)methyl)-2,5-diazabicyclo[2.2.2]octan-2-yl)methyl)-1-thioxoisoindolin-2-yl)piperidine-2,6-dione;
3-(4-((4-((4'-chloro-5,5-dimethyl-3,4,5,6-tetrahydro-[1,1 '-biphenyl]-2-yl)methyl)-2-(trifluoromethyl)piperazin-1-yl)methyl)-1-thioxoisoindolin-2-yl)piperidine-2,6-dione;
3-(4-((4-((4'-fluoro-3,4,5,6-tetrahydro-[1,1'-biphenyl]-2-yl)methyl)piperazin-1-yl)methyl)-1-thioxoisoindolin-2-yl)piperidine-2,6-dione;
3-(4-((4-(4'-fluoro-3,4,5,6-tetrahydro-[1,1'-biphenyl]-2-carbonyl)piperazin-1-yl)methyl)-1-thioxoisoindolin-2-yl) piperidine-2,6-dione;
3-(4-((3-(4'-fluoro-3,4,5,6-tetrahydro-[1,1'-biphenyl]-2-carbonyl)-3,6-diazabicyclo[3.1.1]heptan-6-yl)methyl)-1-thioxoisoindolin-2-yl)piperidine-2,6-dione;
3-(4-((4-((4'-chloro-[1,1'-biphenyl]-2-yl)amino)piperidin-1-yl)methyl)-1-thioxoisoindolin-2-yl)piperidine-2,6-dione;
3-(4-((4-((4'-chloro-[1,1'-biphenyl]-2-yl)methyl)piperazin-1-yl)methyl)-1-thioxoisoindolin-2-yl)piperidine-2,6-dione;
3-(4-((4-((4-(4-chlorophenyl)-6,6-dimethyl-5,6-dihydro-2H-pyran-3-yl)methyl)piperazin-1-yl)methyl)-1-thioxoisoindolin-2-yl)piperidine-2,6-dione;
3-(4-((4-(thiophen-2-yl)piperazin-1-yl)methyl)-1-thioxoisoindolin-2-yl)piperidine-2,6-dione;
3-(4-((4-(5-methylthiophen-2-yl)piperazin-1-yl)methyl)-1-thioxoisoindolin-2-yl)piperidine-2,6-dione;
3-(4-((4-(3-methylthiophen-2-yl)piperazin-1-yl)methyl)-1-thioxoisoindolin-2-yl)piperidine-2,6-dione;
3-(4-((4-(thiophen-3-yl)piperazin-1-yl)methyl)-1-thioxoisoindolin-2-yl)piperidine-2,6-dione;
3-(4-((4-(4-methylthiophen-3-yl)piperazin-1-yl)methyl)-1-thioxoisoindolin-2-yl)piperidine-2,6-dione;
3-(4-((4-(5-methylthiophen-3-yl)piperazin-1-yl)methyl)-1-thioxoisoindolin-2-yl)piperidine-2,6-dione;
3-(4-((4-(thiophen-2-yl)-3,6-dihydropyridin-1(2H)-yl)methyl)-1-thioxoisoindolin-2-yl)piperidine-2,6-dione;
3-(4-((4-(5-methylthiophen-2-yl)-3,6-dihydropyridin-1(2H)-yl)methyl)-1-thioxoisoindolin-2-yl)piperidine-2,6-dione;
3-(4-((4-(3-methylthiophen-2-yl)-3,6-dihydropyridin-1(2H)-yl)methyl)-1-thioxoisoindolin-2-yl)piperidine-2,6-dione;
3-(4-((4-(thiophen-3-yl)-3,6-dihydropyridin-1(2H)-yl)methyl)-1-thioxoisoindolin-2-yl)piperidine-2,6-dione;

3-(4-((4-(4-methylthiophen-3-yl)-3,6-dihydropyridin-1(2H)-yl)methyl)-1-thioxoisoindolin-2-yl)piperidine-2,6-dione;
3-(4-((4-(5-methylthiophen-3-yl)-3,6-dihydropyridin-1(2H)-yl)methyl)-1-thioxoisoindolin-2-yl)piperidine-2,6-dione;
3-(4-((4-(furan-2-yl)-3,6-dihydropyridin-1(2H)-yl)methyl)-1-thioxoisoindolin-2-yl)piperidine-2,6-dione;
3-(4-((4-(furan-3-yl)-3,6-dihydropyridin-1(2H)-yl)methyl)-1-thioxoisoindolin-2-yl)piperidine-2,6-dione;
3-(4-((4-(thiophen-2-yl)piperidin-1-yl)methyl)-1-thioxoisoindolin-2-yl)piperidine-2,6-dione;
3-(4-((4-(5-methylthiophen-2-yl)piperidin-1-yl)methyl)-1-thioxoisoindolin-2-yl)piperidine-2,6-dione;
3-(4-((4-(3-methylthiophen-2-yl)piperidin-1-yl)methyl)-1-thioxoisoindolin-2-yl)piperidine-2,6-dione;
3-(4-((4-(thiophen-3-yl)piperidin-1-yl)methyl)-1-thioxoisoindolin-2-yl)piperidine-2,6-dione;
3-(4-((4-(5-methylthiophen-3-yl)piperidin-1-yl)methyl)-1-thioxoisoindolin-2-yl)piperidine-2,6-dione;
3-(4-((4-(4-methylthiophen-3-yl)piperidin-1-yl)methyl)-1-thioxoisoindolin-2-yl)piperidine-2,6-dione;
3-(4-((4-(furan-2-yl)piperidin-1-yl)methyl)-1-thioxoisoindolin-2-yl)piperidine-2,6-dione;
3-(4-((4-(furan-3-yl)piperidin-1-yl)methyl)-1-thioxoisoindolin-2-yl)piperidine-2,6-dione;
3-(4-((4-(2,3-dichlorophenyl)piperazin-1-yl)methyl)-1-thioxoisoindolin-2-yl)piperidine-2,6-dione;
3-(4-((4-phenylpiperazin-1-yl)methyl)-1-thioxoisoindolin-2-yl)piperidine-2,6-dione;
3-(4-((4-(2-methoxyphenyl)piperazin-1-yl)methyl)-1-thioxoisoindolin-2-yl)piperidine-2,6-dione;
3-(4-((4-(3-methoxyphenyl)piperazin-1-yl)methyl)-1-thioxoisoindolin-2-yl)piperidine-2,6-dione;
3-(4-((4-(4-methoxyphenyl)piperazin-1-yl)methyl)-1-thioxoisoindolin-2-yl)piperidine-2,6-dione;
3-(1-thioxo-4-((4-(4-(trifluoromethyl)phenyl)piperazin-1-yl)methyl)isoindolin-2-yl)piperidine-2,6-dione;
3-(4-((4-(3-fluorophenyl)piperazin-1-yl)methyl)-1-thioxoisoindolin-2-yl)piperidine-2,6-dione;
3-(4-((4-(4-fluorophenyl)piperazin-1-yl)methyl)-1-thioxoisoindolin-2-yl)piperidine-2,6-dione;
3-(4-((4-(2-chlorophenyl)piperazin-1-yl)methyl)-1-thioxoisoindolin-2-yl)piperidine-2,6-dione;
3-(4-((4-(4-chlorophenyl)piperazin-1-yl)methyl)-1-thioxoisoindolin-2-yl)piperidine-2,6-dione;
3-(4-((4-(2-bromophenyl)piperazin-1-yl)methyl)-1-thioxoisoindolin-2-yl)piperidine-2,6-dione;
3-(4-((4-(3-bromophenyl)piperazin-1-yl)methyl)-1-thioxoisoindolin-2-yl)piperidine-2,6-dione;
3-(4-((4-(4-bromophenyl)piperazin-1-yl)methyl)-1-thioxoisoindolin-2-yl)piperidine-2,6-dione;
3-(1-thioxo-4-((4-(o-tolyl)piperazin-1-yl)methyl)isoindolin-2-yl)piperidine-2,6-dione;
3-(1-thioxo-4-((4-(m-tolyl)piperazin-1-yl)methyl)isoindolin-2-yl)piperidine-2,6-dione;
3-(1-thioxo-4-((4-(p-tolyl)piperazin-1-yl)methyl)isoindolin-2-yl)piperidine-2,6-dione;
3-(4-((4-(2,4-dichlorophenyl)piperazin-1-yl)methyl)-1-thioxoisoindolin-2-yl)piperidine-2,6-dione;
3-(4-((4-(2,5-dichlorophenyl)piperazin-1-yl)methyl)-1-thioxoisoindolin-2-yl)piperidine-2,6-dione;
3-(4-((4-(2,6-dichlorophenyl)piperazin-1-yl)methyl)-1-thioxoisoindolin-2-yl)piperidine-2,6-dione;
3-(4-((4-(3,4-dichlorophenyl)piperazin-1-yl)methyl)-1-thioxoisoindolin-2-yl)piperidine-2,6-dione;
3-(4-((4-(3,5-dichlorophenyl)piperazin-1-yl)methyl)-1-thioxoisoindolin-2-yl)piperidine-2,6-dione;
3-(4-((4-(2,3-difluorophenyl)piperazin-1-yl)methyl)-1-thioxoisoindolin-2-yl)piperidine-2,6-dione;
3-(4-((4-(2,4-difluorophenyl)piperazin-1-yl)methyl)-1-thioxoisoindolin-2-yl)piperidine-2,6-dione;
3-(4-((4-(2,5-difluorophenyl)piperazin-1-yl)methyl)-1-thioxoisoindolin-2-yl)piperidine-2,6-dione;
3-(4-((4-(2,6-difluorophenyl)piperazin-1-yl)methyl)-1-thioxoisoindolin-2-yl)piperidine-2,6-dione;
3-(4-((4-(3,4-difluorophenyl)piperazin-1-yl)methyl)-1-thioxoisoindolin-2-yl)piperidine-2,6-dione;
3-(4-((4-(3,5-difluorophenyl)piperazin-1-yl)methyl)-1-thioxoisoindolin-2-yl)piperidine-2,6-dione;
3-(4-((4-(2,4-dimethylphenyl)piperazin-1-yl)methyl)-1-thioxoisoindolin-2-yl)piperidine-2,6-dione;
3-(4-((4-(5-chloro-2-methylphenyl)piperazin-1-yl)methyl)-1-thioxoisoindolin-2-yl)piperidine-2,6-dione;
3-(4-((4-phenylpiperidin-1-yl)methyl)-1-thioxoisoindolin-2-yl)piperidine-2,6-dione;
3-(4-((4-(3-bromophenyl)piperidin-1-yl)methyl)-1-thioxoisoindolin-2-yl)piperidine-2,6-dione;
3-(4-((4-(4-bromophenyl)piperidin-1-yl)methyl)-1-thioxoisoindolin-2-yl)piperidine-2,6-dione;
3-(4-((4-(2-chlorophenyl)piperidin-1-yl)methyl)-1-thioxoisoindolin-2-yl)piperidine-2,6-dione;
3-(4-((4-(4-chlorophenyl)piperidin-1-yl)methyl)-1-thioxoisoindolin-2-yl)piperidine-2,6-dione;
3-(4-((4-(2-fluorophenyl)piperidin-1-yl)methyl)-1-thioxoisoindolin-2-yl)piperidine-2,6-dione;
3-(4-((4-(4-fluorophenyl)piperidin-1-yl)methyl)-1-thioxoisoindolin-2-yl)piperidine-2,6-dione;
3-(1-thioxo-4-((4-(2-(trifluoromethyl)phenyl)piperidin-1-yl)methyl)isoindolin-2-yl)piperidine-2,6-dione;
3-(1-thioxo-4-((4-(3-(trifluoromethyl)phenyl)piperidin-1-yl)methyl)isoindolin-2-yl)piperidine-2,6-dione;
3-(4-((4-(2,3-dichlorophenyl)piperidin-1-yl)methyl)-1-thioxoisoindolin-2-yl)piperidine-2,6-dione;
3-(4-((4-(2-chloro-3-fluorophenyl)piperidin-1-yl)methyl)-1-thioxoisoindolin-2-yl)piperidine-2,6-dione;
3-(4-((4-(2,3-difluorophenyl)piperidin-1-yl)methyl)-1-thioxoisoindolin-2-yl)piperidine-2,6-dione;
3-(4-((4-(2,3-dichlorophenyl)-3,6-dihydropyridin-1(2H)-yl)methyl)-1-thioxoisoindolin-2-yl)piperidine-2,6-dione;
3-(4-((4-(2-chloro-3-fluorophenyl)-3,6-dihydropyridin-1(2H)-yl)methyl)-1-thioxoisoindolin-2-yl)piperidine-2,6-dione;

3-(4-((4-(pyridin-2-yl)piperazin-1-yl)methyl)-1-thioxoisoindolin-2-yl)piperidine-2,6-dione;
3-(4-((4-(6-methylpyridin-2-yl)piperazin-1-yl)methyl)-1-thioxoisoindolin-2-yl)piperidine-2,6-dione;
3-(4-((4-(6-chloropyridin-2-yl)piperazin-1-yl)methyl)-1-thioxoisoindolin-2-yl)piperidine-2,6-dione;
3-(4-((4-(5-chloropyridin-2-yl)piperazin-1-yl)methyl)-1-thioxoisoindolin-2-yl)piperidine-2,6-dione;
3-(4-((4-(4-chloropyridin-2-yl)piperazin-1-yl)methyl)-1-thioxoisoindolin-2-yl)piperidine-2,6-dione;
3-(4-((4-(3-chloropyridin-2-yl)piperazin-1-yl)methyl)-1-thioxoisoindolin-2-yl)piperidine-2,6-dione;
3-(4-((4-(3-fluoropyridin-2-yl)piperazin-1-yl)methyl)-1-thioxoisoindolin-2-yl)piperidine-2,6-dione;
3-(4-((4-(6-fluoropyridin-2-yl)piperazin-1-yl)methyl)-1-thioxoisoindolin-2-yl)piperidine-2,6-dione;
3-(4-((4-(5-fluoropyridin-2-yl)piperazin-1-yl)methyl)-1-thioxoisoindolin-2-yl)piperidine-2,6-dione;
3-(1-thioxo-4-((4-(3-(trifluoromethyl)pyridin-2-yl)piperazin-1-yl)methyl)isoindolin-2-yl)piperidine-2,6-dione;
3-(1-thioxo-4-((4-(6-(trifluoromethyl)pyridin-2-yl)piperazin-1-yl)methyl)isoindolin-2-yl)piperidine-2,6-dione;
3-(1-thioxo-4-((4-(5-(trifluoromethyl)pyridin-2-yl)piperazin-1-yl)methyl)isoindolin-2-yl)piperidine-2,6-dione;
3-(4-((4-(3,4-dichloropyridin-2-yl)piperazin-1-yl)methyl)-1-thioxoisoindolin-2-yl)piperidine-2,6-dione;
3-(4-((4-(3,5-dichloropyridin-2-yl)piperazin-1-yl)methyl)-1-thioxoisoindolin-2-yl)piperidine-2,6-dione;
3-(4-((4-(4,5-dichloropyridin-2-yl)piperazin-1-yl)methyl)-1-thioxoisoindolin-2-yl)piperidine-2,6-dione;
3-(4-((4-(3,4-difluoropyridin-2-yl)piperazin-1-yl)methyl)-1-thioxoisoindolin-2-yl)piperidine-2,6-dione;
3-(4-((4-(4-chloro-3-fluoropyridin-2-yl)piperazin-1-yl)methyl)-1-thioxoisoindolin-2-yl)piperidine-2,6-dione;
3-(4-((4-(3-fluoro-4-iodopyridin-2-yl)piperazin-1-yl)methyl)-1-thioxoisoindolin-2-yl)piperidine-2,6-dione;
3-(4-((4-(3-bromo-4-chloropyridin-2-yl)piperazin-1-yl)methyl)-1-thioxoisoindolin-2-yl)piperidine-2,6-dione;
3-(4-((4-(3-chloro-5-(trifluoromethyl)pyridin-2-yl)piperazin-1-yl)methyl)-1-thioxoisoindolin-2-yl)piperidine-2,6-dione;
3-(4-((4-(4-chloro-6-methylpyridin-2-yl)piperazin-1-yl)methyl)-1-thioxoisoindolin-2-yl)piperidine-2,6-dione;
3-(4-((4-(pyridin-3-yl)piperazin-1-yl)methyl)-1-thioxoisoindolin-2-yl)piperidine-2,6-dione;
3-(4-((4-(6-chloropyridin-3-yl)piperazin-1-yl)methyl)-1-thioxoisoindolin-2-yl)piperidine-2,6-dione;
3-(4-((4-(4,5-dichloropyridin-3-yl)piperazin-1-yl)methyl)-1-thioxoisoindolin-2-yl)piperidine-2,6-dione;
3-(4-((4-(2,4-dichloropyridin-3-yl)piperazin-1-yl)methyl)-1-thioxoisoindolin-2-yl)piperidine-2,6-dione;
3-(4-((4-(pyridin-4-yl)piperazin-1-yl)methyl)-1-thioxoisoindolin-2-yl)piperidine-2,6-dione;
3-(4-((4-(2-methylpyridin-4-yl)piperazin-1-yl)methyl)-1-thioxoisoindolin-2-yl)piperidine-2,6-dione;
3-(4-((4-(3-bromopyridin-4-yl)piperazin-1-yl)methyl)-1-thioxoisoindolin-2-yl)piperidine-2,6-dione;
3-(4-((4-(3-fluoropyridin-4-yl)piperazin-1-yl)methyl)-1-thioxoisoindolin-2-yl)piperidine-2,6-dione;
3-(4-((4-(3-chloropyridin-4-yl)piperazin-1-yl)methyl)-1-thioxoisoindolin-2-yl)piperidine-2,6-dione;
3-(4-((4-(2-chloropyridin-4-yl)piperazin-1-yl)methyl)-1-thioxoisoindolin-2-yl)piperidine-2,6-dione;
3-(4-((4-(2-chloro-3-fluoropyridin-4-yl)piperazin-1-yl)methyl)-1-thioxoisoindolin-2-yl)piperidine-2,6-dione;
3-(4-((4-(2-bromo-3-fluoropyridin-4-yl)piperazin-1-yl)methyl)-1-thioxoisoindolin-2-yl)piperidine-2,6-dione;
3-(4-((4-(3-bromo-2-chloropyridin-4-yl)piperazin-1-yl)methyl)-1-thioxoisoindolin-2-yl)piperidine-2,6-dione;
3-(4-((4-(2,3-dichloropyridin-4-yl)piperazin-1-yl)methyl)-1-thioxoisoindolin-2-yl)piperidine-2,6-dione;
3-(4-((4-(2,3-difluoropyridin-4-yl)piperazin-1-yl)methyl)-1-thioxoisoindolin-2-yl)piperidine-2,6-dione;
3-(4-((4-(3-fluoro-2-hydroxypyridin-4-yl)piperazin-1-yl)methyl)-1-thioxoisoindolin-2-yl)piperidine-2,6-dione;
3-(4-((4-(3-bromo-5-chloropyridin-4-yl)piperazin-1-yl)methyl)-1-thioxoisoindolin-2-yl)piperidine-2,6-dione;
3-(4-((4-(3-chloro-5-fluoropyridin-4-yl)piperazin-1-yl)methyl)-1-thioxoisoindolin-2-yl)piperidine-2,6-dione;
3-(4-((4-(3,5-dichloropyridin-4-yl)piperazin-1-yl)methyl)-1-thioxoisoindolin-2-yl)piperidine-2,6-dione;
3-(4-((4-(2,6-dimethylpyridin-4-yl)piperazin-1-yl)methyl)-1-thioxoisoindolin-2-yl)piperidine-2,6-dione;
3-(4-((4-(perfluoropyridin-4-yl)piperazin-1-yl)methyl)-1-thioxoisoindolin-2-yl)piperidine-2,6-dione;
3-(4-((4-(pyridazin-3-yl)piperazin-1-yl)methyl)-1-thioxoisoindolin-2-yl)piperidine-2,6-dione;
3-(4-((4-(6-chloropyridazin-3-yl)piperazin-1-yl)methyl)-1-thioxoisoindolin-2-yl)piperidine-2,6-dione;
3-(4-((4-(pyrimidin-2-yl)piperazin-1-yl)methyl)-1-thioxoisoindolin-2-yl)piperidine-2,6-dione;
3-(4-((4-(pyrazin-2-yl)piperazin-1-yl)methyl)-1-thioxoisoindolin-2-yl)piperidine-2,6-dione;
3-(4-((4-(5,6-dichloropyrimidin-4-yl)piperazin-1-yl)methyl)-1-thioxoisoindolin-2-yl)piperidine-2,6-dione;
3-(4-((4-(3,6-dichloropyridazin-4-yl)piperazin-1-yl)methyl)-1-thioxoisoindolin-2-yl)piperidine-2,6-dione;
3-(4-((4-(5,6-dichloropyridazin-4-yl)piperazin-1-yl)methyl)-1-thioxoisoindolin-2-yl)piperidine-2,6-dione;
3-(4-((4-(1,3,5-triazin-2-yl)piperazin-1-yl)methyl)-1-thioxoisoindolin-2-yl)piperidine-2,6-dione;
3-(4-((4-(3,4-dichloropyridin-2-yl)piperidin-1-yl)methyl)-1-thioxoisoindolin-2-yl)piperidine-2,6-dione;
3-(4-((4-(2,3-dichloropyridin-4-yl)piperidin-1-yl)methyl)-1-thioxoisoindolin-2-yl)piperidine-2,6-dione;
3-(4-((4-(2,3-difluoropyridin-4-yl)piperidin-1-yl)methyl)-1-thioxoisoindolin-2-yl)piperidine-2,6-dione;
3-(4-([3,4'-bipiperidin]-1-ylmethyl)-1-thioxoisoindolin-2-yl)piperidine-2,6-dione;
3-(4-((3,4-dichloro-3',6'-dihydro-[2,4'-bipyridin]-1'(2'H)-yl)methyl)-1-thioxoisoindolin-2-yl)piperidine-2,6-dione;
3-(4-((4,5-dichloro-3',6'-dihydro-[3,4'-bipyridin]-1'(2'H)-yl)methyl)-1-thioxoisoindolin-2-yl)piperidine-2,6-dione;
3-(4-((5-chloro-3',6'-dihydro-[3,4'-bipyridin]-1'(2'H)-yl)methyl)-1-thioxoisoindolin-2-yl)piperidine-2,6-dione;

3-(4-((2',3'-dichloro-3,6-dihydro-[4,4'-bipyridin]-1(2H)-yl)methyl)-1-thioxoisoindolin-2-yl)piperidine-2,6-dione;
3-(4-((3-fluoro-3',6'-dihydro-[2,4'-bipyridin]-1'(2'H)-yl)methyl)-1-thioxoisoindolin-2-yl)piperidine-2,6-dione;
3-(4-((2',3'-difluoro-3,6-dihydro-[4,4'-bipyridin]-1(2H)-yl)methyl)-1-thioxoisoindolin-2-yl)piperidine-2,6-dione;
3-(4-((3-(2,3-dichlorophenyl)-3,6-diazabicyclo[3.1.1]heptan-6-yl)methyl)-1-thioxoisoindolin-2-yl)piperidine-2,6-dione;
3-(4-((6-(2,3-dichlorophenyl)-3,6-diazabicyclo[3.1.1]heptan-3-yl)methyl)-1-thioxoisoindolin-2-yl)piperidine-2,6-dione;
3-(4-(((1R,4R)-5-(2,3-dichlorophenyl)-2,5-diazabicyclo[2.2.1]heptan-2-yl)methyl)-1-thioxoisoindolin-2-yl)piperidine-2,6-dione;
3-(4-(((1S,4S)-5-(2,3-dichlorophenyl)-2,5-diazabicyclo[2.2.1]heptan-2-yl)methyl)-1-thioxoisoindolin-2-yl)piperidine-2,6-dione;
3-(4-((5-(2,3-dichlorophenyl)-2,5-diazabicyclo[2.2.2]octan-2-yl)methyl)-1-thioxoisoindolin-2-yl)piperidine-2,6-dione;
3-(4-((8-(2,3-dichlorophenyl)-3,8-diazabicyclo[3.2.1]octan-3-yl)methyl)-1-thioxoisoindolin-2-yl)piperidine-2,6-dione;
3-(4-((3-(2,3-dichlorophenyl)-3,8-diazabicyclo[3.2.1]octan-8-yl)methyl)-1-thioxoisoindolin-2-yl)piperidine-2,6-dione;
3-(4-((4-(2,3-dichlorophenyl)-1,4-diazepan-1-yl)methyl)-1-thioxoisoindolin-2-yl)piperidine-2,6-dione;
3-(4-((4-(6-fluorobenzo[d]isoxazol-3-yl)piperidin-1-yl)methyl)-1-thioxoisoindolin-2-yl)piperidine-2,6-dione;
3-(4-((4-(benzo[d]isoxazol-3-yl)piperidin-1-yl)methyl)-1-thioxoisoindolin-2-yl)piperidine-2,6-dione;
3-(4-((4-(isoxazol-3-yl)piperidin-1-yl)methyl)-1-thioxoisoindolin-2-yl)piperidine-2,6-dione;
3-(4-((4-(6-fluorobenzo[d]isoxazol-3-yl)piperazin-1-yl)methyl)-1-thioxoisoindolin-2-yl)piperidine-2,6-dione;
3-(4-((4-(isoxazolo[4,5-c]pyridin-3-yl)piperazin-1-yl)methyl)-1-thioxoisoindolin-2-yl)piperidine-2,6-dione;
3-(4-((4-(6-fluorobenzo[d]isothiazol-3-yl)piperazin-1-yl)methyl)-1-thioxoisoindolin-2-yl)piperidine-2,6-dione;
3-(4-((4-(6-fluoro-1H-indazol-3-yl)piperazin-1-yl)methyl)-1-thioxoisoindolin-2-yl)piperidine-2,6-dione;
3-(4-((4-(1H-pyrrolo[2,3-b]pyridin-3-yl)piperazin-1-yl)methyl)-1-thioxoisoindolin-2-yl)piperidine-2,6-dione;
3-(4-((4-(6-fluoro-1H-indol-3-yl)piperidin-1-yl)methyl)-1-thioxoisoindolin-2-yl)piperidine-2,6-dione;
3-(4-((4-(6-fluoro-1-methyl-1H-indol-3-yl)piperidin-1-yl)methyl)-1-thioxoisoindolin-2-yl)piperidine-2,6-dione;
3-(4-((4-(6-fluoro-1H-indazol-3-yl)piperidin-1-yl)methyl)-1-thioxoisoindolin-2-yl)piperidine-2,6-dione;
3-(4-((4-(5-fluoroindolin-1-yl)piperidin-1-yl)methyl)-1-thioxoisoindolin-2-yl)piperidine-2,6-dione;
3-(4-((4-(5-fluoro-1H-benzo[d]imidazol-1-yl)piperidin-1-yl)methyl)-1-thioxoisoindolin-2-yl)piperidine-2,6-dione;
3-(4-((4-(5-fluoro-2-oxo-2,3-dihydro-1H-benzo[d]imidazol-1-yl)piperidin-1-yl)methyl)-1-thioxoisoindolin-2-yl)piperidine-2,6-dione;
3-(4-((4-(1H-pyrrolo[2,3-b]pyridin-3-yl)piperidin-1-yl)methyl)-1-thioxoisoindolin-2-yl)piperidine-2,6-dione;
3-(4-((4-(1H-pyrrolo[2,3-b]pyridin-3-yl)-3,6-dihydropyridin-1(2H)-yl)methyl)-1-thioxoisoindolin-2-yl)piperidine-2,6-dione;
3-(4-((4-(6-fluoro-1H-indol-3-yl)-3,6-dihydropyridin-1(2H)-yl)methyl)-1-thioxoisoindolin-2-yl)piperidine-2,6-dione;
3-(4-((4-(6-fluoro-1-methyl-1H-indol-3-yl)-3,6-dihydropyridin-1(2H)-yl)methyl)-1-thioxoisoindolin-2-yl)piperidine-2,6-dione;
3-(4-((4-(6-fluoro-1H-indazol-3-yl)-3,6-dihydropyridin-1(2H)-yl)methyl)-1-thioxoisoindolin-2-yl)piperidine-2,6-dione;
3-(4-((4-(6-fluoro-3,4-dihydroquinolin-1(2H)-yl)piperidin-1-yl)methyl)-1-thioxoisoindolin-2-yl)piperidine-2,6-dione;
3-(4-((4-(7-fluoro-2,3-dihydro-4H-benzo[b][1,4]oxazin-4-yl)piperidin-1-yl)methyl)-1-thioxoisoindolin-2-yl)piperidine-2,6-dione;
3-(4-((4-(7-fluoro-3-oxo-2,3-dihydro-4H-benzo[b][1,4]oxazin-4-yl)piperidin-1-yl)methyl)-1-thioxoisoindolin-2-yl)piperidine-2,6-dione;
3-(4-((4-(7-fluorochroman-4-yl)piperidin-1-yl)methyl)-1-thioxoisoindolin-2-yl)piperidine-2,6-dione;
3-(4-((4-(7-fluorochroman-4-ylidene)piperidin-1-yl)methyl)-1-thioxoisoindolin-2-yl)piperidine-2,6-dione;
3-(4-((4-(5-fluoronaphthalen-1-yl)piperidin-1-yl)methyl)-1-thioxoisoindolin-2-yl)piperidine-2,6-dione;
3-(4-((4-(5-fluoronaphthalen-1-yl)piperidin-1-yl)methyl)-1-thioxoisoindolin-2-yl)piperidine-2,6-dione;
3-(4-((4-(7-fluoroquinolin-4-yl)piperidin-1-yl)methyl)-1-thioxoisoindolin-2-yl)piperidine-2,6-dione;
3-(4-((4-(isoquinolin-5-yl)piperidin-1-yl)methyl)-1-thioxoisoindolin-2-yl)piperidine-2,6-dione;
3-(4-((4-(5-fluoronaphthalen-1-yl)-3,6-dihydropyridin-1(2H)-yl)methyl)-1-thioxoisoindolin-2-yl)piperidine-2,6-dione;
3-(4-((4-(7-fluoroquinolin-4-yl)-3,6-dihydropyridin-1(2H)-yl)methyl)-1-thioxoisoindolin-2-yl)piperidine-2,6-dione;

3-(4-((4-(isoquinolin-5-yl)-3,6-dihydropyridin-1(2H)-yl)methyl)-1-thioxoisoindolin-2-yl)piperidine-2,6-dione;
3-(4-((3-(7-fluorochroman-4-yl)azetidin-1-yl)methyl)-1-thioxoisoindolin-2-yl)piperidine-2,6-dione;
3-(4-((3-(7-fluorochroman-4-ylidene)azetidin-1-yl)methyl)-1-thioxoisoindolin-2-yl)piperidine-2,6-dione;
3-(4-((3-(7-fluoroquinolin-4-yl)azetidin-1-yl)methyl)-1-thioxoisoindolin-2-yl)piperidine-2,6-dione;
3-(4-((3-(5-fluoroindolin-1-yl)azetidin-1-yl)methyl)-1-thioxoisoindolin-2-yl)piperidine-2,6-dione;
3-(4-((3-(5-fluoro-1H-benzo[d]imidazol-1-yl)azetidin-1-yl)methyl)-1-thioxoisoindolin-2-yl)piperidine-2,6-dione;
3-(4-((4-(thieno[2,3-d]pyrimidin-4-yl)piperazin-1-yl)methyl)-1-thioxoisoindolin-2-yl)piperidine-2,6-dione;
3-(4-((4-(5-methylthieno[2,3-d]pyrimidin-4-yl)piperazin-1-yl)methyl)-1-thioxoisoindolin-2-yl)piperidine-2,6-dione;
3-(4-((4-(6-methylthieno[2,3-d]pyrimidin-4-yl)piperazin-1-yl)methyl)-1-thioxoisoindolin-2-yl)piperidine-2,6-dione;
3-(4-((4-(6-isopropylthieno[2,3-d]pyrimidin-4-yl)piperazin-1-yl)methyl)-1-thioxoisoindolin-2-yl)piperidine-2,6-dione;
3-(4-((4-(5,6-dimethylthieno[2,3-d]pyrimidin-4-yl)piperazin-1-yl)methyl)-1-thioxoisoindolin-2-yl)piperidine-2,6-dione;
3-(4-((4-(6-phenylthieno[2,3-d]pyrimidin-4-yl)piperazin-1-yl)methyl)-1-thioxoisoindolin-2-yl)piperidine-2,6-dione;
3-(4-((4-(6-bromothieno[2,3-d]pyrimidin-4-yl)piperazin-1-yl)methyl)-1-thioxoisoindolin-2-yl)piperidine-2,6-dione;
3-(4-((4-(5-phenylthieno[2,3-d]pyrimidin-4-yl)piperazin-1-yl)methyl)-1-thioxoisoindolin-2-yl)piperidine-2,6-dione;
3-(4-((4-(5,6,7,8-tetrahydrobenzo[4,5]thieno[2,3-d]pyrimidin-4-yl)piperazin-1-yl)methyl)-1-thioxoisoindolin-2-yl)piperidine-2,6-dione;
3-(4-((4-(6,7-dihydro-5H-cyclopenta[4,5]thieno[2,3-d]pyrimidin-4-yl)piperazin-1-yl)methyl)-1-thioxoisoindolin-2-yl)piperidine-2,6-dione;
3-(4-((4-(2-methyl-5-phenylthieno[2,3-d]pyrimidin-4-yl)piperazin-1-yl)methyl)-1-thioxoisoindolin-2-yl)piperidine-2,6-dione;
3-(4-((4-(2-methyl-5,6,7,8-tetrahydrobenzo[4,5]thieno[2,3-d]pyrimidin-4-yl)piperazin-1-yl)methyl)-1-thioxoisoindolin-2-yl)piperidine-2,6-dione;
3-(4-((4-(2-methyl-6,7-dihydro-5H-cyclopenta[4,5]thieno[2,3-d]pyrimidin-4-yl)piperazin-1-yl)methyl)-1-thioxoisoindolin-2-yl)piperidine-2,6-dione;
3-(4-((4-(2-methylthieno[2,3-d]pyrimidin-4-yl)piperazin-1-yl)methyl)-1-thioxoisoindolin-2-yl)piperidine-2,6-dione;
3-(4-((4-(thieno[2,3-d]pyrimidin-2-yl)piperazin-1-yl)methyl)-1-thioxoisoindolin-2-yl)piperidine-2,6-dione;
3-(4-((4-(5,6-dimethylthieno[2,3-d]pyrimidin-4-yl)-1,4-diazepan-1-yl)methyl)-1-thioxoisoindolin-2-yl)piperidine-2,6-dione;
3-(4-((4-(thieno[2,3-d]pyrimidin-4-yl)piperidin-1-yl)methyl)-1-thioxoisoindolin-2-yl)piperidine-2,6-dione;
3-(4-((4-(6-methylthieno[2,3-d]pyrimidin-4-yl)piperidin-1-yl)methyl)-1-thioxoisoindolin-2-yl)piperidine-2,6-dione;
3-(4-((4-(5,6-dimethylthieno[2,3-d]pyrimidin-4-yl)piperidin-1-yl)methyl)-1-thioxoisoindolin-2-yl)piperidine-2,6-dione;
3-(4-((4-(6-phenylthieno[2,3-d]pyrimidin-4-yl)piperidin-1-yl)methyl)-1-thioxoisoindolin-2-yl)piperidine-2,6-dione;
3-(4-((4-(5-phenylthieno[2,3-d]pyrimidin-4-yl)piperidin-1-yl)methyl)-1-thioxoisoindolin-2-yl)piperidine-2,6-dione;
3-(4-((4-(5,6,7,8-tetrahydrobenzo[4,5]thieno[2,3-d]pyrimidin-4-yl)piperidin-1-yl)methyl)-1-thioxoisoindolin-2-yl)piperidine-2,6-dione;
3-(4-((4-(6,7-dihydro-5H-cyclopenta[4,5]thieno[2,3-d]pyrimidin-4-yl)piperidin-1-yl)methyl)-1-thioxoisoindolin-2-yl)piperidine-2,6-dione;
3-(4-((4-(2-methyl-5-phenylthieno[2,3-d]pyrimidin-4-yl)piperidin-1-yl)methyl)-1-thioxoisoindolin-2-yl)piperidine-2,6-dione;
3-(4-((4-(2-methyl-5,6,7,8-tetrahydrobenzo[4,5]thieno[2,3-d]pyrimidin-4-yl)piperidin-1-yl)methyl)-1-thioxoisoindolin-2-yl)piperidine-2,6-dione;
3-(4-((4-(2-methyl-6,7-dihydro-5H-cyclopenta[4,5]thieno[2,3-d]pyrimidin-4-yl)piperidin-1-yl)methyl)-1-thioxoisoindolin-2-yl)piperidine-2,6-dione;
3-(4-((4-(2-methylthieno[2,3-d]pyrimidin-4-yl)piperidin-1-yl)methyl)-1-thioxoisoindolin-2-yl)piperidine-2,6-dione;
3-(4-((4-(thieno[2,3-d]pyrimidin-2-yl)piperidin-1-yl)methyl)-1-thioxoisoindolin-2-yl)piperidine-2,6-dione;

3-(4-((4-(5-ethylpyrimidin-2-yl)piperidin-1-yl)methyl)-1-thioxoisoindolin-2-yl)piperidine-2,6-dione;
3-(4-((4-(thieno[2,3-d]pyrimidin-4-yl)-3,6-dihydropyridin-1(2H)-yl)methyl)-1-thioxoisoindolin-2-yl)piperidine-2,6-dione;
3-(4-((4-(5-methylthieno[2,3-d]pyrimidin-4-yl)-3,6-dihydropyridin-1(2H)-yl)methyl)-1-thioxoisoindolin-2-yl)piperidine-2,6-dione;
3-(4-((4-(6-methylthieno[2,3-d]pyrimidin-4-yl)-3,6-dihydropyridin-1(2H)-yl)methyl)-1-thioxoisoindolin-2-yl)piperidine-2,6-dione;
3-(4-((4-(6-isopropylthieno[2,3-d]pyrimidin-4-yl)-3,6-dihydropyridin-1(2H)-yl)methyl)-1-thioxoisoindolin-2-yl)piperidine-2,6-dione;
3-(4-((4-(5,6-dimethylthieno[2,3-d]pyrimidin-4-yl)-3,6-dihydropyridin-1(2H)-yl)methyl)-1-thioxoisoindolin-2-yl) piperidine-2,6-dione;
3-(4-((4-(6-phenylthieno[2,3-d]pyrimidin-4-yl)-3,6-dihydropyridin-1(2H)-yl)methyl)-1-thioxoisoindolin-2-yl)piperidine-2,6-dione;
3-(4-((4-(5-phenylthieno[2,3-d]pyrimidin-4-yl)-3,6-dihydropyridin-1(2H)-yl)methyl)-1-thioxoisoindolin-2-yl)piperidine-2,6-dione;
3-(4-((4-(5,6,7,8-tetrahydrobenzo[4,5]thieno[2,3-d]pyrimidin-4-yl)-3,6-dihydropyridin-1(2H)-yl)methyl)-1-thioxoisoindolin-2-yl)piperidine-2,6-dione;
3-(4-((4-(6,7-dihydro-5H-cyclopenta[4,5]thieno[2,3-d]pyrimidin-4-yl)-3,6-dihydropyridin-1(2H)-yl)methyl)-1-thioxoisoindolin-2-yl)piperidine-2,6-dione;
3-(4-((4-(2-methyl-5-phenylthieno [2,3-d]pyrimidin-4-yl)-3,6-dihydropyridin-1 (2H)-yl)methyl)-1-thioxoisoindolin-2-yl)piperidine-2,6-dione;
3-(4-((4-(2-methyl-5,6,7,8-tetrahydrobenzo[4,5]thieno[2,3-d]pyrimidin-4-yl)-3,6-dihydropyridin-1(2H)-yl) methyl)-1-thioxoisoindolin-2-yl)piperidine-2,6-dione;
3-(4-((4-(2-methyl-6,7-dihydro-5H-cyclopenta[4,5]thieno[2,3-d]pyrimidin-4-yl)-3,6-dihydropyridin-1(2H)-yl) methyl)-1-thioxoisoindolin-2-yl)piperidine-2,6-dione;
3-(4-((4-(2-methylthieno[2,3-d]pyrimidin-4-yl)-3,6-dihydropyridin-1(2H)-yl)methyl)-1-thioxoisoindolin-2-yl)piperidine-2,6-dione;
3-(4-((4-(thieno[2,3-d]pyrimidin-2-yl)-3,6-dihydropyridin-1(2H)-yl)methyl)-1-thioxoisoindolin-2-yl)piperidine-2,6-dione;
3-(4-((4-(2-chloro-6,7-dihydrothieno[3,2-d]pyrimidin-4-yl)piperazin-1-yl)methyl)-1-thioxoisoindolin-2-yl)piperidine-2,6-dione;
3-(4-((4-(2-morpholino-6,7-dihydrothieno[3,2-d]pyrimidin-4-yl)piperazin-1-yl)methyl)-1-thioxoisoindolin-2-yl)piperidine-2,6-dione;
3-(4-((4-(4-(piperazin-1-yl)-6,7-dihydrothieno[3,2-d]pyrimidin-2-yl)piperazin-1-yl)methyl)-1-thioxoisoindolin-2-yl)piperidine-2,6-dione;
3-(4-((4-(4-morpholino-6,7-dihydrothieno[3,2-d]pyrimidin-2-yl)piperazin-1-yl)methyl)-1-thioxoisoindolin-2-yl)piperidine-2,6-dione;
3-(4-((4-(2-(piperidin-1-yl)-6,7-dihydrothieno[3,2-d]pyrimidin-4-yl)piperazin-1-yl)methyl)-1-thioxoisoindolin-2-yl)piperidine-2,6-dione;
3-(4-((4-(2-(pyrrolidin-1-yl)-6,7-dihydrothieno[3,2-d]pyrimidin-4-yl)piperazin-1-yl)methyl)-1-thioxoisoindolin-2-yl)piperidine-2,6-dione;
3-(4-((4-(2-(azetidin-1-yl)-6,7-dihydrothieno[3,2-d]pyrimidin-4-yl)piperazin-1-yl)methyl)-1-thioxoisoindolin-2-yl) piperidine-2,6-dione;
3-(4-((4-(thieno[3,2-d]pyrimidin-4-yl)piperazin-1-yl)methyl)-1-thioxoisoindolin-2-yl)piperidine-2,6-dione;
3-(4-((4-(2-chlorothieno[3,2-d]pyrimidin-4-yl)piperazin-1-yl)methyl)-1-thioxoisoindolin-2-yl)piperidine-2,6-dione;
3-(4-((4-(2-morpholinothieno[3,2-d]pyrimidin-4-yl)piperazin-1-yl)methyl)-1-thioxoisoindolin-2-yl)piperidine-2,6-dione;
3-(4-((4-(4-(piperazin-1-yl)thieno[3,2-d]pyrimidin-2-yl)piperazin-1-yl)methyl)-1-thioxoisoindolin-2-yl)piperidine-2,6-dione;
3-(4-((4-(4-morpholinothieno[3,2-d]pyrimidin-2-yl)piperazin-1-yl)methyl)-1-thioxoisoindolin-2-yl)piperidine-2,6-dione;
3-(4-((4-(benzo[b]thiophen-4-yl)piperazin-1-yl)methyl)-1-thioxoisoindolin-2-yl)piperidine-2,6-dione;
3-(1-thioxo-4-((3-(trifluoromethyl)-5,6-dihydro-[1,2,4]triazolo[4,3-a]pyrazin-7(8H)-yl)methyl)isoindolin-2-yl)piperidine-2,6-dione;
3-(4-((4-(2-((2,4-dimethylphenyl)thio)phenyl)piperazin-1-yl)methyl)-1-thioxoisoindolin-2-yl)piperidine-2,6-dione;
3-(4-((6,7-dihydrothieno[3,2-c]pyridin-5(4H)-yl)methyl)-1-thioxoisoindolin-2-yl)piperidine-2,6-dione;

3-(4-((4-(benzo[d]isothiazol-3-yl)piperazin-1-yl)methyl)-1-thioxoisoindolin-2-yl)piperidine-2,6-dione;
3-(4-((4-(4-(adamantan-1-yl)benzyl)piperazin-1-yl)methyl)-1-thioxoisoindolin-2-yl)piperidine-2,6-dione;
3-(4-(((adamantan-1-yl)amino)methyl)-1-thioxoisoindolin-2-yl)piperidine-2,6-dione;
1-((1R,4R)-7,7-dimethyl-2-oxobicyclo[2.2.1]heptan-1-yl)-N-((2-(2,6-dioxopiperidin-3-yl)-1-thioxoisoindolin-4-yl)methyl)methanesulfonamide;
3-(4-((((1R,4S)-bicyclo[2.2.1]heptan-2-yl)oxy)methyl)-1-thioxoisoindolin-2-yl)piperidine-2,6-dione;
3-(4-((4-((4'-chloro-[1,1'-biphenyl]-4-yl)methyl)piperazin-1-yl)methyl)-1-thioxoisoindolin-2-yl)piperidine-2,6-dione;
(S)-3-(4-((4-(morpholinomethyl)benzyl)oxy)-1-thioxoisoindolin-2-yl)piperidine-2,6-dione; and
(S)-4-(4-(4-(((2-(2,6-dioxopiperidin-3-yl)-1-thioxoisoindolin-4-yl)oxy)methyl)benzyl)piperazin-1-yl)-3-fluorobenzonitrile.

**12.** The compound of Formula (I) or salts, enantiomers, stereoisomers, solvates, or polymorphs thereof as claimed in any one of claims 1-11, which is hydrohalide (including hydrochloride, hydrobromide), sulfate, maleate, sulfonate, citrate, lactate, lactobionate, L-tartrate, fumarate, L-malate, L-lactate, α-ketoglutarate, hippurate, D-glucuronate, D-gluconate, α-D-glucoheptonate, glycolate, mucate, L-ascorbate, orotate, picrate, glycinate, alaninate, arginate, cinnamate, laurate, pamoate, sebacate, besylate, methanesulfonate, ethanesulfonate, edisylate,formate, acetate, 2,2-dichloroacetate, trimethylacetate, propionate, valerate, palmitate, triphenylacetate, 2-ethyl-butane-1,4-dioate, iodate, nicotinate, L-pyroglutamate, L-prolinate, ferulate, 2-hydroxyethanesulfonate, nitrate, gentisate, cholate, salicylate, terephthalate, glutarate, adipate, stearate, oleate, undecylenate, camphorate, camsylate, dodecylsulfate, phosphate, thiocyanate, dihydrogen phosphate, pyrophosphate, metaphosphate, oxalate, carbonate, malonate, benzoate, mandelate, succinate, pyruvate, 4-chlorobenzenesulfonate, 1,5-naphthalenedisulfonate, 3-hydroxy-2-naphthoate, 1-hydroxy-2-naphthoate, 2-naphthalenesulfonate, trifluoroacetate, glycolate, or 4-methylbenzenesulfonate of the compound of Formula (I).

**13.** A pharmaceutical composition, comprising: the compound of Formula (I) or pharmaceutically acceptable salts thereof as claimed in any one of claims 1-12, and at least one pharmaceutically acceptable carrier or excipient.

**14.** The pharmaceutical composition as claimed in claim 13, further comprising at least one additional therapeutic agent, e.g., an anticancer agent.

**15.** The compound of Formula (I) or pharmaceutically acceptable salts thereof as claimed in any one of claims 1-12, for use in the prevention or treatment of a disease or disorder selected from the group consisting of: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

**16.** The compound of Formula (I) or pharmaceutically acceptable salts thereof as claimed in claim 15, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, includingacute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, monocytic leukemia, myelomonocytic leukemia, T-lymphocytic leukemia, acute T-lymphocytic leukemia; lymphoma, including diffuse large B-cell lymphoma, non-Hodgkin' s lymphoma, Hodgkin' s lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer;

endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Unverricht' s syndrome; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

**17.** Use of the compound of Formula (I) or pharmaceutically acceptable salts thereof as claimed in any one of claims 1-12, or of the pharmaceutical composition as claimed in claim 13 or 14, for the manufacture of a medicament for the prevention or treatment of a disease or disorder selected from the group consisting of: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

**18.** A method for treating or preventing a disease or disorder in a subject, comprising administering to the subject a therapeutically effective amount of the compound of Formula (I) or pharmaceutically acceptable salts thereof as claimed in any one of claims 1-12, or the pharmaceutical composition as claimed in claim 13 or 14, wherein the disease or disorder is selected from the group consisting of: tumors, cancers, infectious diseases, inflammatory diseases, autoimmune diseases, anemia, hemorrhagic shock, transplant rejection, multiple organ dysfunction syndrome (MODS), sarcoidosis, adult respiratory distress syndrome, cardiovascular diseases, Richter syndrome (RS), acute liver failure, and diabetes.

**19.** The use as claimed in claim 17, or the method as claimed in claim 18, wherein the disease or disorder is selected from the group consisting of: myeloma, including multiple myeloma, plasma cell myeloma, smoldering myeloma, smoldering multiple myeloma; myelofibrosis; bone marrow disease; myelodysplastic syndrome (MDS); previously treated myelodysplastic syndrome; transplantation-related cancer; neutropenia; leukemia, includingacute myeloid leukemia (AML), chronic myeloid leukemia (CML), chronic myelogenous leukemia, acute lymphoblastic leukemia (ALL), chronic lymphocytic leukemia (CLL), B-cell chronic lymphocytic leukemia, leukemia-associated anemia, monocytic leukemia, myelomonocytic leukemia, T-lymphocytic leukemia, acute T-lymphocytic leukemia; lymphoma, including diffuse large B-cell lymphoma, non-Hodgkin's lymphoma, Hodgkin's lymphoma, anaplastic lymphoma, anaplastic large cell lymphoma, immunoblastic T-Cell lymphoma, CD20 positive lymphoma, mantle cell lymphoma, follicular lymphoma (FL), Burkitt's lymphoma, marginal zone lymphoma (MZL), primary lymphoma, B-cell lymphoma, recurrent B-cell non-Hodgkin's lymphoma, recurrent diffuse large B-cell lymphoma, recurrent mediastinal (thymic) large B-cell lymphoma, primary mediastinal (thymic) large B-cell lymphoma, recurrent transformed non-Hodgkin's lymphoma, refractory B-cell non-Hodgkin's lymphoma, refractory diffuse large B-cell lymphoma, refractory primary mediastinal (thymic) large B-cell lymphoma, refractory transformed non-Hodgkin's lymphoma; thyroid cancer; melanoma; lung cancer, including lung adenocarcinoma, lung squamous cell carcinoma, non-small cell lung cancer, and small cell lung cancer; inflammatory myofibroblastoma; colorectal cancer; intestinal cancer; brain glioma; astroblastoma; ovarian cancer; bronchial cancer; prostate cancer; breast cancer, including triple negative breast cancer, sporadic breast cancer, ductal carcinoma of the breast, and patients with Cowden syndrome; pancreatic cancer; central nervous system tumor; neuroblastoma; glioma; peripheral neuroepithelioma; extramedullary plasmacytoma; plasmacytoma; gastric cancer; gastrointestinal stromal tumors; esophageal cancer; colorectal adenocarcinoma; esophageal squamous cell carcinoma; liver cancer; renal cell carcinoma; bladder cancer; endometrial cancer; metrocarcinoma; head and neck cancer; brain cancer; oral cancer; sarcoma, including rhabdomyosarcoma, various lipogenic tumors, Ewing's sarcoma/primitive neuroectodermal tumors (Ewing/PNETs), and leiomyosarcoma; urothelial carcinoma; basal cell carcinoma; oral squamous cell carcinoma; cholangiocarcinoma; bone cancer; cervical cancer; skin cancer; Richter syndrome (RS); sepsis syndrome; autoimmune diseases, including rheumatoid arthritis, autoimmune encephalomyelitis, ankylosing spondylitis, psoriasis, systemic lupus erythematosus, multiple sclerosis, recurrent oral ulcers, Kawasaki disease, polymyositis/dermatomyositis, Sjogren's syndrome, and atopic dermatitis; keratoconjunctivitis; inflammatory diseases, including Crohn's disease and ulcerative colitis, pneumonia, osteoarthritis, synovitis, systemic inflammatory response syndrome, airway inflammation, bronchitis; cerebral malaria; infectious diseases, including viral pneumonia, Acquired immunodeficiency syndrome (AIDS), COVID-19 novel coronavirus infection, gram-negative bacteria infection, gram-positive bacteria infection, tuberculosis, etc; septic shock; tuberculosis; bacterial meningitis; chronic obstructive pulmonary disease; asthma; hemorrhagic shock; organ (including kidney, heart, lung) or tissue transplantation rejection; diabetes; sarcoidosis; adult respiratory distress syndrome; anemia; pediatric aplastic anemia; cardiovascular diseases (e.g., coronary heart disease, congestive heart failure, myocardial infarction, atherosclerosis); multiple organ dysfunction caused by cachexia and septic shock; and acute liver failure.

**20.** A method for preparing a compound of Formula (II), comprising reacting a compound of Formula (M1) with a compound of Formula (M2) to prepare the compound of Formula (II):

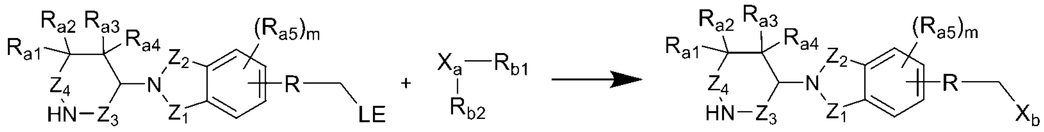

(M1) (M2) (II)

wherein the groups $R_{a1}$, $R_{a2}$, $R_{a3}$, $R_{a4}$, $(R_{a5})_m$, $Z_1$, $Z_2$, $Z_3$, $Z_4$, and R are as defined in claim 1; and wherein

(i) the group LE represents Cl, Br, I, mesyloxy, tosyloxy, or ortho-nitrobenzenesulfonyl; $R_{b1}$-$X_a$-$R_{b2}$ of Formula (M2) is $R_1$-NH-$R_2$; and $X_b$ of the compound of Formula (II) correspondingly represents $NR_1R_2$, wherein $R_1$ and $R_2$ are as defined in any one of claims 1-11; or

(ii) the group LE represents Cl, Br, I, mesyloxy, tosyloxy, or ortho-nitrobenzenesulfonyl; and the group $X_a$ represents NH; and the group $R_{b1}$ and the group $R_{b2}$ together with the nitrogen atom of $X_a$ to which they are attached form a nitrogen-containing heterocycle $A_a$ which is substituted by n1 substituent(s) $R_{d1}$, and a substituent represented by the formula of

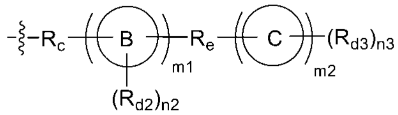

,

i.e., the compound of Formula (M2) has a structure represented by the following formula:

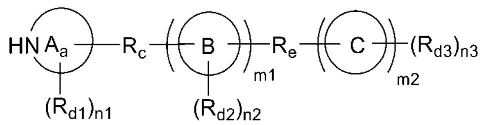

,

and $X_b$ of the compound of Formula (II) correspondingly represents a structure represented by the following general formula:

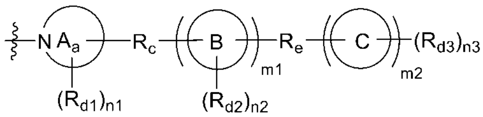

,

wherein the nitrogen-containing heterocycle $A_a$ represents 4- to 30-membered nitrogen-containing heterocyclyl (preferably, 4- to 20-membered nitrogen-containing heterocyclyl; more preferably, 4- to 15-membered nitrogen-containing heterocyclyl); and $(R_{d1})_{n1}$, n1, $R_c$, ring B, m1, $(R_{d2})_{n2}$, n2, $R_e$, ring C, m2, $(R_{d3})_{n3}$, and n3 are as defined in any one of claims 1-11; or

(iii) (a) the group LE represents C(O)Cl or COOH; $R_{b1}$-$X_a$-$R_{b2}$ of Formula (M2) represents $R_3$-NH-$R_4$; and $X_b$ of the compound of Formula (II) correspondingly represents $C(O)NR_3R_4$; or (b) the group LE represents

$S(O)_2Cl$; $R_{b1}$-$X_a$-$R_{b2}$ of Formula (M2) represents $R_{10}$-NH-$R_{11}$; and $X_b$ of the compound of Formula (II) correspondingly represents $S(O)_2NR_{10}R_{11}$, wherein $R_3$, $R_4$, $R_{10}$, and $R_{11}$ are as defined in any one of claims 1-11; or

(iv) the group LE represents $NH_2$; $R_{b1}$-$X_a$-$R_{b2}$ of Formula (M2) represents: $R_5$-C(O)-OH, $R_5$-C(O)-Cl, $R_{13}$-$S(O)_2$-OH, or $R_{13}$-$S(O)_2$-Cl; and $X_b$ of the compound of Formula (II) correspondingly represents $NHC(O)R_5$ or $NHS(O)_2R_{13}$, wherein $R_5$ and $R_{13}$ are as defined in any one of claims 1-11; or

(v) the group LE represents Cl, Br or I; $R_{b1}$-$X_a$-$R_{b2}$ of Formula (M2) represents: $R_8$-OH or $R_9$-SH; and $X_b$ of the compound of Formula (II) correspondingly represents $OR_8$ or $SR_9$, wherein $R_8$ and $R_9$ are as defined in any one of claims 1-11.

**21.** The method for preparing the compound of Formula (II) as claimed in claim 20, wherein when the group LE represents mesyloxy, the compound of Formula (M1) is prepared by reacting a compound of Formula (M3) with methanesulfonic anhydride or methanesulfonyl chloride,

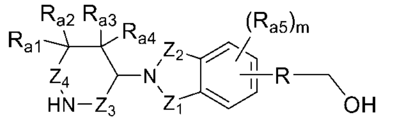

(M3)

wherein the groups $R_{a1}$, $R_{a2}$, $R_{a3}$, $R_{a4}$, $(R_{a5})_m$, $Z_1$, $Z_2$, $Z_3$, $Z_4$, and R are as defined in claim 1.

**22.** The method for preparing the compound of Formula (II) as claimed in claim 20 or 21, wherein, in the compounds of Formula (M1), Formula (II), and Formula (M3), R represents a bond.

**23.** The method for preparing the compound of Formula (II) as claimed in claim 22, wherein the compound of Formula (M3) is prepared by a coupling reaction of a compound of Formula (M4) with (tributylstannyl)methanol catalyzed by a palladium catalyst,

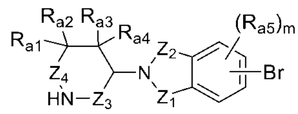

(M4)

wherein the groups $R_{a1}$, $R_{a2}$, $R_{a3}$, $R_{a4}$, $(R_{a5})_m$, $Z_1$, $Z_2$, $Z_3$, and $Z_4$ are as defined in claim 1.

**24.** The method for preparing the compound of Formula (II) as claimed in claim 23, wherein the thiocarbonyl compound of Formula (M4) is prepared by thionation of a carbonyl compound of Formula (M5) with a thionation reagent,

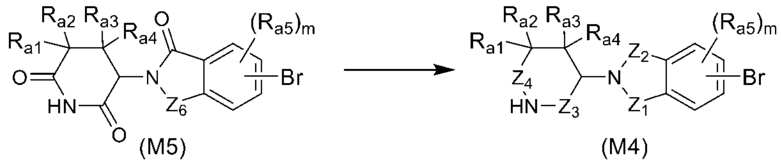

(M5) (M4)

wherein, in the carbonyl compound of Formula (M5), $Z_6$ represents C(O), $CH_2$ or $CD_2$;
in the thiocarbonyl compound of Formula (M4), $Z_1$ represents C(O), C(S), $CH_2$ or $CD_2$; $Z_2$, $Z_3$, and $Z_4$ each independently represent C(O) or C(S), wherein at least one of $Z_1$, $Z_2$, $Z_3$, and $Z_4$ represents C(S); and the groups $R_{a1}$, $R_{a2}$, $R_{a3}$, $R_{a4}$, and $(R_{a5})_m$ are as defined in claim 1.

**25.** The method for preparing the compound of Formula (II) as claimed in claim 24, wherein the thionation reagent comprises carbon disulfide, hexamethyldisilathiane, sulfur, thiourea, hydrogen sulfide, phosphorus pentasulfide, Lawesson's Reagent, Belleau's reagent, and Davy's reagent.

**26.** A method for preparing a compound of Formula (M4), comprising preparing the thiocarbonyl compound of Formula

(M4) by thionation of a carbonyl compound of Formula (M5) with a thionation reagent:

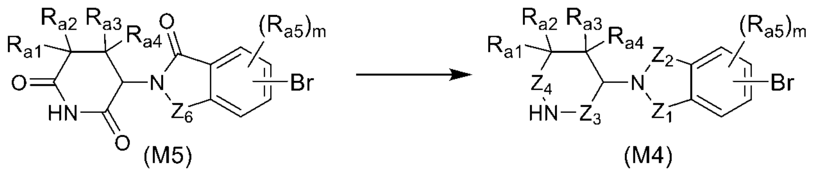

wherein, in the carbonyl compound of Formula (M5), $Z_6$ represents C(O), $CH_2$ or $CD_2$; and the groups $R_{a1}$, $R_{a2}$, $R_{a3}$, $R_{a4}$, $Z_1$, $Z_2$, $Z_3$, $Z_4$, and $(R_{a5})_m$ are as defined in claim 1.

**27.** The method for preparing the compound of Formula (M4) as claimed in claim 26, wherein the thionation reagent comprises carbon disulfide, hexamethyldisilathiane, sulfur, thiourea, hydrogen sulfide, phosphorus pentasulfide, Lawesson's Reagent, Belleau's reagent, and Davy's reagent.

**28.** A method for preparing a compound of Formula (M1),

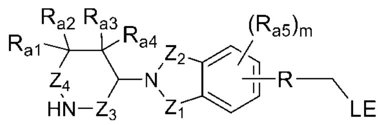

(M1)

wherein the group LE represents mesyloxy, and the method comprises reacting a compound of Formula (M3) with methanesulfonic anhydride or methanesulfonyl chloride to prepare the compound of Formula (M1),

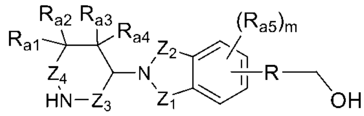

(M3)

wherein, in the compounds of Formula (M1) and Formula (M3), the groups $R_{a1}$, $R_{a2}$, $R_{a3}$, $R_{a4}$, R, $Z_1$, $Z_2$, $Z_3$, $Z_4$, and $(R_{a5})_m$ are as defined in claim 1.

**29.** The method for preparing the compound of Formula (M1) as claimed in claim 28, wherein, in the compounds of Formula (M1) and Formula (M3), R represents a bond.

**30.** The method for preparing the compound of Formula (M1) as claimed in claim 29, wherein the compound of Formula (M3) is prepared by a coupling reaction of a compound of Formula (M4) with (tributylstannyl)methanol,

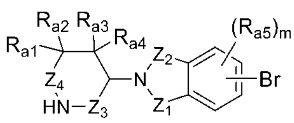

(M4)

wherein the groups $R_{a1}$, $R_{a2}$, $R_{a3}$, $R_{a4}$, $(R_{a5})_m$, $Z_1$, $Z_2$, $Z_3$, and $Z_4$ are as defined in claim 1.

**31.** The method for preparing the compound of Formula (M1) as claimed in claim 30, comprising preparing the thiocarbonyl compound of Formula (M4) by thionation of a carbonyl compound of Formula (M5) with a thionation reagent,

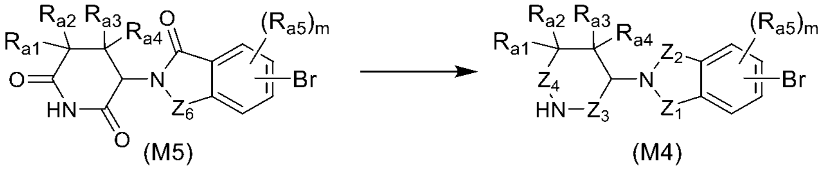

wherein, in the carbonyl compound of Formula (M5), $Z_6$ represents C(O), $CH_2$ or $CD_2$; and the groups $R_{a1}$, $R_{a2}$, $R_{a3}$, $R_{a4}$, $Z_1$, $Z_2$, $Z_3$, $Z_4$, and $(R_{a5})_m$ are as defined in claim 1.

**32.** The method for preparing the compound of Formula (M1) as claimed in claim 31, wherein the thionation reagent comprises carbon disulfide, hexamethyldisilathiane, sulfur, thiourea, hydrogen sulfide, phosphorus pentasulfide, Lawesson's Reagent, Belleau's reagent, and Davy's reagent.

**33.** A method for preparing a compound of Formula (I), comprising preparing the compound of Formula (I) by thionation of a compound of Formula (A) with a thionation reagent,

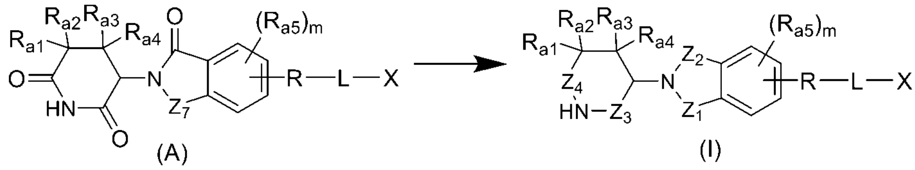

wherein $Z_7$ of the carbonyl compound of Formula (A) represents C(O), $CH_2$ or $CD_2$;
$Z_1$ of the compound of Formula (I) represents C(O), C(S), $CH_2$ or $CD_2$; $Z_2$, $Z_3$, and $Z_4$ each independently represent C(O) or C(S), wherein at least one of $Z_1$, $Z_2$, $Z_3$, and $Z_4$ represents C(S); and
wherein the groups $R_{a1}$, $R_{a2}$, $R_{a3}$, $R_{a4}$, $(R_{a5})_m$, R, L, and X are as defined in claim 1.

**34.** The method for preparing the compound of Formula (I) as claimed in claim 33, wherein the thionation reagent comprises carbon disulfide, hexamethyldisilathiane, sulfur, thiourea, hydrogen sulfide, phosphorus pentasulfide, Lawesson's Reagent, Belleau's reagent, and Davy's reagent.

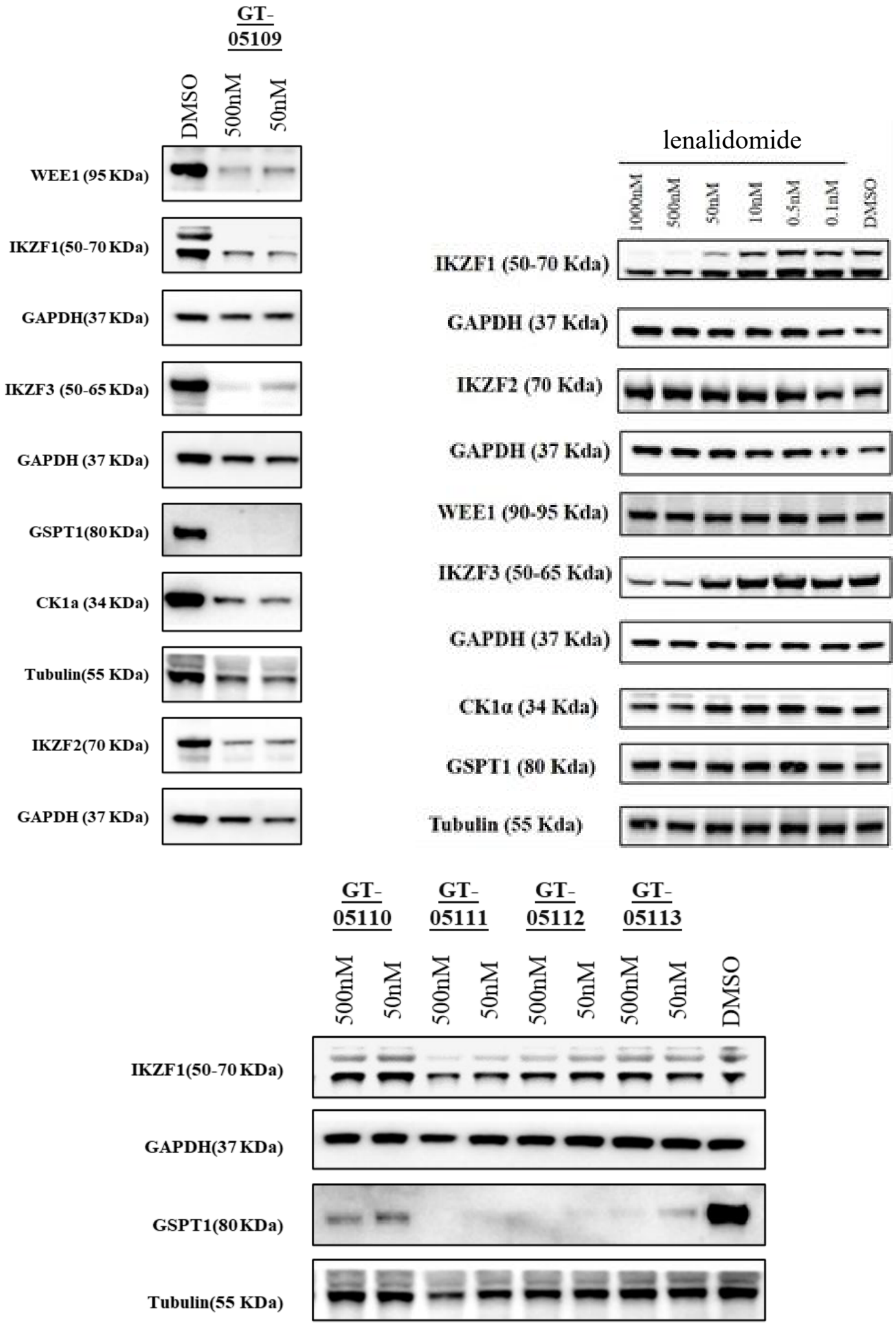
GT-05109
DMSO
500nM
50nM
WEE1 (95 KDa)
IKZF1(50-70 KDa)
GAPDH(37 KDa)
IKZF3 (50-65 KDa)
GAPDH (37 KDa)
GSPT1(80 KDa)
CK1a (34 KDa)
Tubulin(55 KDa)
IKZF2(70 KDa)
GAPDH (37 KDa)
lenalidomide
1000nM
500nM
50nM
10nM
0.5nM
0.1nM
DMSO
IKZF1 (50-70 Kda)
GAPDH (37 Kda)
IKZF2 (70 Kda)
GAPDH (37 Kda)
WEE1 (90-95 Kda)
IKZF3 (50-65 Kda)
GAPDH (37 Kda)
CK1α (34 Kda)
GSPT1 (80 Kda)
Tubulin (55 Kda)
GT-05110
GT-05111
GT-05112
GT-05113
500nM
50nM
500nM
50nM
500nM
50nM
500nM
50nM
DMSO
IKZF1(50-70 KDa)
GAPDH(37 KDa)
GSPT1(80 KDa)
Tubulin(55 KDa)

Figure 1

**INTERNATIONAL SEARCH REPORT**

International application No.
**PCT/CN2024/105405**

**A. CLASSIFICATION OF SUBJECT MATTER**

C07D401/14(2006.01)i; C07D413/14(2006.01)i; C07D417/14(2006.01)i; C07D 407/14(2006.01)i; C07D409/14(2006.01)i; A61K31/454(2006.01)i; A61K 31/4545(2006.01)i; A61K 31/453(2006.01)i; A61K 31/4535(2006.01)i; A61K31/496(2006.01)i; A61K31/506(2006.01)i; A61P35/00(2006.01)i; A61P35/02(2006.01)i; A61P 37/00(2006.01)i; A61P 29/00(2006.01)i; A61P 9/00(2006.01)i; A61P 3/10(2006.01)i

According to International Patent Classification (IPC) or to both national classification and IPC

**B. FIELDS SEARCHED**

Minimum documentation searched (classification system followed by classification symbols)

IPC: C07D, A61K, A61P

Documentation searched other than minimum documentation to the extent that such documents are included in the fields searched

Electronic data base consulted during the international search (name of data base and, where practicable, search terms used)

CNABS, CNTXT, ENTXT, ENTXTC, WPABS, CNKI, REGISTRY (STN), MARPAT (STN), CAPLUS (STN), 沙度利胺, 硫代, 类似物, 衍生物, 癌, 肿瘤, 炎症, 免疫, 心血管, thalidomide, thiothalidomide, analog+, derivative?, cancer?, tumour?, tumor?, inflammat+, +immune, cardiovascular, 结构式检索, structural formula search

**C. DOCUMENTS CONSIDERED TO BE RELEVANT**

| Category* | Citation of document, with indication, where appropriate, of the relevant passages | Relevant to claim No. |
|---|---|---|
| X | WO 2009139880 A1 (CELGENE CORPORATION et al.) 19 November 2009 (2009-11-19) claims 1, 9-15 and 24, embodiments 1-2, and description, paragraphs [21], [81]-[99], [108] and [110] | 1-4, 6-8, 12-19 |
| Y | WO 2009139880 A1 (CELGENE CORPORATION et al.) 19 November 2009 (2009-11-19) claims 1, 9-15 and 24, embodiments 1-2, and description, paragraphs [21], [81]-[99], [108] and [110] | 1-34 |
| X | US 2017182022 A1 (THE UNITED STATES OF AMERICA, AS REPRESENTED BY THE SECRETARY, DEPARTMENT OF HEALTH AND HUMAN SERVICES) 29 June 2017 (2017-06-29) claim 1, and description, paragraphs [0004], [0041], [0064]-[0068], [0076], [0077] and [0080]-[0083] | 1-4, 6-8, 12-19 |

☑ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **14 October 2024** | **21 October 2024** |

| Name and mailing address of the ISA/CN | Authorized officer |
|---|---|
| **China National Intellectual Property Administration (ISA/CN)**<br>**China No. 6, Xitucheng Road, Jimenqiao, Haidian District, Beijing 100088** | Telephone No. |

Form PCT/ISA/210 (second sheet) (July 2022)

**INTERNATIONAL SEARCH REPORT**

International application No.
**PCT/CN2024/105405**

**C. DOCUMENTS CONSIDERED TO BE RELEVANT**

| Category* | Citation of document, with indication, where appropriate, of the relevant passages | Relevant to claim No. |
|---|---|---|
| Y | US 2017182022 A1 (THE UNITED STATES OF AMERICA, AS REPRESENTED BY THE SECRETARY, DEPARTMENT OF HEALTH AND HUMAN SERVICES) 29 June 2017 (2017-06-29)<br>claim 1, and description, paragraphs [0004], [0041], [0064]-[0068], [0076], [0077] and [0080]-[0083] | 1-34 |
| X | WO 2017059062 A1 (THE UNITED STATES OF AMERICA, AS REPRESENTED BY THE SECRETARY, DEPARTMENT OF HEALTH AND HUMAN SERVICES) 06 April 2017 (2017-04-06)<br>claims 1, 7, 19 and 20, and description, pages 40-41 | 1-4, 6-8, 12-19 |
| Y | WO 2017059062 A1 (THE UNITED STATES OF AMERICA, AS REPRESENTED BY THE SECRETARY, DEPARTMENT OF HEALTH AND HUMAN SERVICES) 06 April 2017 (2017-04-06)<br>claims 1, 7, 19 and 20, and description, pages 40-41 | 1-34 |
| X | LUO, Weiming et al. "Syntheses of Aromatic Substituted 6'-Thiothalidomides" *Synthesis*, Vol. 2008, No. (21), 16 October 2008 (2008-10-16),<br>pp. 3415-3422 | 1-4, 6-8, 33-34 |
| Y | LUO, Weiming et al. "Syntheses of Aromatic Substituted 6'-Thiothalidomides" *Synthesis*, Vol. 2008, No. (21), 16 October 2008 (2008-10-16),<br>pp. 3415-3422 | 20-34 |
| Y | CN 111606883 A (SHANGHAITECH UNIVERSITY) 01 September 2020 (2020-09-01)<br>claims 1, 55, 72 and 73, and embodiments | 1-34 |
| Y | CN 1867331 A (THE GOVERNMENT OF UNITED STATES OF AMERICA, AS REPRESENTED BY THE SECRETARY OF THE DEPARTMENT OF HEALTH AND HUMAN SERVICES) 22 November 2006 (2006-11-22)<br>claims 1, 31 and 55-56, and description, page 37 | 1-34 |
| A | US 2018021315 A1 (WAYNE STATE UNIVERSITY et al.) 25 January 2018 (2018-01-25)<br>entire description | 1-34 |

Form PCT/ISA/210 (second sheet) (July 2022)

**INTERNATIONAL SEARCH REPORT**

International application No.
**PCT/CN2024/105405**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **18-19**
because they relate to subject matter not required to be searched by this Authority, namely:

The subject matter of claims 18-19 relates to a method for treatment of human diseases (PCT Rule 39.1(iv)). The present search is performed on the basis of the use of the compound or pharmaceutical composition in the preparation of a drug for treating corresponding diseases.

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2022)

**INTERNATIONAL SEARCH REPORT**
**Information on patent family members**

International application No.
**PCT/CN2024/105405**

| Patent document cited in search report | Publication date (day/month/year) | Patent family member(s) | Publication date (day/month/year) |
|---|---|---|---|
| WO 2009139880 A1 | 19 November 2009 | US 2009298882 A1 | 03 December 2009 |
| US 2017182022 A1 | 29 June 2017 | US 2015164877 A1 | 18 June 2015 |
| | | US 9084783 B2 | 21 July 2015 |
| | | US 10220028 B2 | 05 March 2019 |
| | | US 2015359784 A1 | 17 December 2015 |
| | | US 9623020 B2 | 18 April 2017 |
| | | US 2013143922 A1 | 06 June 2013 |
| | | US 8927725 B2 | 06 January 2015 |
| WO 2017059062 A1 | 06 April 2017 | US 2020325102 A1 | 15 October 2020 |
| | | US 10836721 B2 | 17 November 2020 |
| | | EP 3356346 A1 | 08 August 2018 |
| | | AU 2016330967 A1 | 10 May 2018 |
| | | AU 2016330967 B2 | 25 March 2021 |
| | | CA 3000661 A1 | 06 April 2017 |
| | | CA 3000661 C | 02 April 2024 |
| | | US 2021047267 A1 | 18 February 2021 |
| | | US 11440880 B2 | 13 September 2022 |
| | | US 2018273472 A1 | 27 September 2018 |
| | | US 10730835 B2 | 04 August 2020 |
| | | KR 20180088642 A | 06 August 2018 |
| CN 111606883 A | 01 September 2020 | AU 2020228803 A1 | 30 September 2021 |
| | | AU 2020228803 B2 | 18 May 2023 |
| | | WO 2020173426 A1 | 03 September 2020 |
| | | JP 2022521746 A | 12 April 2022 |
| | | JP 2024023405 A | 21 February 2024 |
| | | CA 3132308 A1 | 03 September 2020 |
| | | EP 3932922 A1 | 05 January 2022 |
| | | EP 3932922 A4 | 11 May 2022 |
| | | US 2022143002 A1 | 12 May 2022 |
| | | KR 20210142114 A | 24 November 2021 |
| CN 1867331 A | 22 November 2006 | WO 2005028436 A2 | 31 March 2005 |
| | | WO 2005028436 A3 | 19 May 2005 |
| | | BRPI 0414497 A | 14 November 2006 |
| | | CA 2808646 A1 | 31 March 2005 |
| | | CA 2808646 C | 23 August 2016 |
| | | CA 2538864 A1 | 31 March 2005 |
| | | CA 2538864 C | 07 May 2013 |
| | | US 2006211728 A1 | 21 September 2006 |
| | | US 7973057 B2 | 05 July 2011 |
| | | SG 133603 A1 | 30 July 2007 |
| | | US 2011245210 A1 | 06 October 2011 |
| | | US 8546430 B2 | 01 October 2013 |
| | | AU 2004274474 A1 | 31 March 2005 |
| | | AU 2004274474 B2 | 10 March 2011 |
| | | JP 2007505922 A | 15 March 2007 |
| | | JP 4943845 B2 | 30 May 2012 |
| | | EP 1663223 A2 | 07 June 2006 |
| | | EP 1663223 B1 | 01 January 2014 |
| US 2018021315 A1 | 25 January 2018 | None | |

Form PCT/ISA/210 (patent family annex) (July 2022)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- Clinical Pharmacology. People's Public Health Press, 2008
- *CHEMICAL ABSTRACTS*, 19172-47-5
- *CHEMICAL ABSTRACTS*, 88816-02-8
- *CHEMICAL ABSTRACTS*, 82737-61-9
- *CHEMICAL ABSTRACTS*, 768-94-5